# EUROPEAN PATENT SPECIFICATION

(11) **EP 4 122 934 B1**
(45) Date of publication and mention of the grant of the patent: **15.01.2025**
(21) Application number: 22209573.9
(22) Date of filing: 25.11.2022
(51) Int. Cl.: C07D 498/04, C07D 513/04, C07D 517/04, H10K 50/15

(54) **ORGANIC ELECTROLUMINESCENT MATERIAL AND DEVICE**
ORGANISCHE ELEKTROLUMINESZENZMATERIALIEN UND VORRICHTUNGEN
MATERIAUX ORGANIQUES ELECTROLUMINSECENTS ET DISPOSITIFS

(30) Priority: 25.11.2021 CN 202111410981; 30.09.2022 CN 202211206202
(43) Date of publication of application: 25.01.2023
(73) Proprietor: Beijing Summer Sprout Technology Co., Ltd., Beijing 102308 (CN)
(72) Inventor: CUI, Zhihao, Beijing, 102308 (CN); ZHENG, Renjie, Beijing, 102308 (CN); DING, Hualong, Beijing, 102308 (CN); HU, Juntao, Beijing, 102308 (CN); KWONG, Chi Yuen Raymond, Beijing, 102308 (CN); XIA, Chuanjun, Beijing, 102308 (CN)
(74) Representative: Ricker, Mathias

(56) References cited:
- DE-A1- 102020 104 604
- US-A1- 2020 062 778

## Description

### TECHNICAL FIELD

The present disclosure relates to compounds for organic electronic devices such as organic light-emitting devices. In particular, the present disclosure relates to a compound having an ortho-substituted dehydrogenated fused-ring structure, an organic electroluminescent device comprising the compound and a compound composition comprising the compound.

### BACKGROUND

Organic electronic devices include, but are not limited to, the following types: organic light-emitting diodes (OLEDs), organic field-effect transistors (O-FETs), organic light-emitting transistors (OLETs), organic photovoltaic devices (OPVs), dye-sensitized solar cells (DSSCs), organic optical detectors, organic photoreceptors, organic field-quench devices (OFQDs), light-emitting electrochemical cells (LECs), organic laser diodes and organic plasmon emitting devices.

In 1987, Tang and Van Slyke of Eastman Kodak reported a bilayer organic electroluminescent device, which comprises an arylamine hole transporting layer and a tris-8-hydroxyquinolato-aluminum layer as the electron and emitting layer (Applied Physics Letters, 1987, 51 (12): 913-915). Once a bias is applied to the device, green light was emitted from the device. This device laid the foundation for the development of modern organic light-emitting diodes (OLEDs). State-of-the-art OLEDs may comprise multiple layers such as charge injection and transporting layers, charge and exciton blocking layers, and one or multiple emissive layers between the cathode and anode. Since the OLED is a self-emitting solid state device, it offers tremendous potential for display and lighting applications. In addition, the inherent properties of organic materials, such as their flexibility, may make them well suited for particular applications such as fabrication on flexible substrates.

The OLED can be categorized as three different types according to its emitting mechanism. The OLED invented by Tang and van Slyke is a fluorescent OLED. It only utilizes singlet emission. The triplets generated in the device are wasted through nonradiative decay channels. Therefore, the internal quantum efficiency (IQE) of the fluorescent OLED is only 25%. This limitation hindered the commercialization of OLED. In 1997, Forrest and Thompson reported phosphorescent OLED, which uses triplet emission from heavy metal containing complexes as the emitter. As a result, both singlet and triplets can be harvested, achieving 100% IQE. The discovery and development of phosphorescent OLED contributed directly to the commercialization of active-matrix OLED (AMOLED) due to its high efficiency. Recently, Adachi achieved high efficiency through thermally activated delayed fluorescence (TADF) of organic compounds. These emitters have small singlet-triplet gap that makes the transition from triplet back to singlet possible. In the TADF device, the triplet excitons can go through reverse intersystem crossing to generate singlet excitons, resulting in high IQE.

OLEDs can also be classified as small molecule and polymer OLEDs according to the forms of the materials used. A small molecule refers to any organic or organometallic material that is not a polymer. The molecular weight of the small molecule can be large as long as it has well defined structure. Dendrimers with well-defined structures are considered as small molecules. Polymer OLEDs include conjugated polymers and non-conjugated polymers with pendant emitting groups. Small molecule OLED can become the polymer OLED if post polymerization occurred during the fabrication process.

There are various methods for OLED fabrication. Small molecule OLEDs are generally fabricated by vacuum thermal evaporation. Polymer OLEDs are fabricated by solution process such as spin-coating, inkjet printing, and slit printing. If the material can be dissolved or dispersed in a solvent, the small molecule OLED can also be produced by solution process.

The emitting color of the OLED can be achieved by emitter structural design. An OLED may comprise one emitting layer or a plurality of emitting layers to achieve desired spectrum. In the case of green, yellow, and red OLEDs, phosphorescent emitters have successfully reached commercialization. Blue phosphorescent device still suffers from non-saturated blue color, short device lifetime, and high operating voltage. Commercial full-color OLED displays normally adopt a hybrid strategy, using fluorescent blue and phosphorescent yellow, or red and green. At present, efficiency roll-off of phosphorescent OLEDs at high brightness remains a problem. In addition, it is desirable to have more saturated emitting color, higher efficiency, and longer device lifetime.

P-type doping materials have a very important role in OLED devices, and the reasonable use of the p-type doping materials can greatly improve device performance. For example, the applicant's previous patent applications DE102020104604A1 and US20200062778A1 disclose an organic compound having a dehydrobenzodioxazole, dehydrobenzodithiazole or dehydrobenzodiselenazole structure or a similar structure. Such compounds have a structure of Formula 1 in DE102020104604A1: 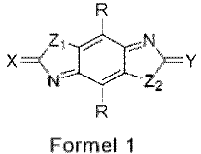 or Formula 1' in US 20200062778A1: 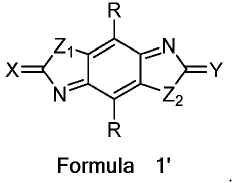 This application focuses on the use of such compounds as p-type doping materials with deep LUMO or similar materials in OLEDs and has not disclosed or taught a relationship between the molecular structure of such compounds and a sublimation yield.

The inventors have found through researches that such excellent p-type doping materials in the above literature need to have relatively deep LUMO energy levels and, therefore, their molecular structure is generally a conjugated system having one or more substituents with strong electron-withdrawing properties. Due to the structure, the compound is very deficient in electrons and thus is strong oxidative. Such p-type doping materials have a low sublimation yield due to extreme electron deficiency and a strong oxidative property. Organic small-molecule materials used in OLEDs must pass through a sublimation process before these materials are used for preparing devices. Moreover, the evaporation process of the organic small-molecule materials for preparing OLED devices is also a sublimation process. Thus, the sublimation yield of the organic small-molecule materials will directly affect the costs of production and application. Therefore, it is of great significance to study how to make such p-type doping materials have a high sublimation yield, which have excellent performance when used in devices.

### SUMMARY

The present disclosure aims to provide a series of compounds each having an ortho-substituted dehydrogenated fused-ring structure to solve at least part of the preceding problems. The compounds may be used as charge transporting materials, charge injection materials, and similar materials in organic electroluminescent devices. These novel compounds have an unexpected high sublimation yield due to a unique structural design. Additionally, these novel compounds can provide excellent performance for the organic electroluminescent devices, such as low voltage, high current efficiency (CE), high power efficiency (PE) and high external quantum efficiency (EQE).

According to an embodiment of the present disclosure, disclosed is a compound having a structure represented by any one of Formula 2-1 to Formula 2-10: 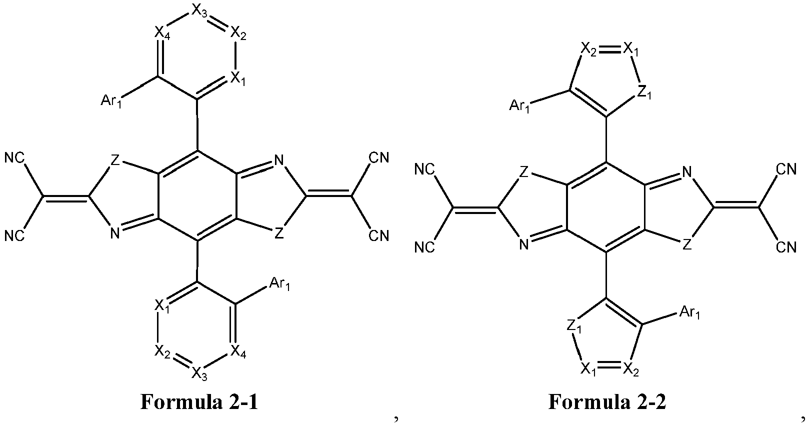 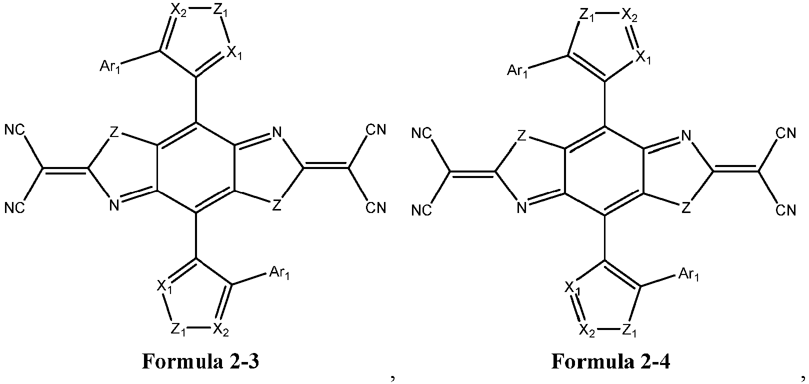 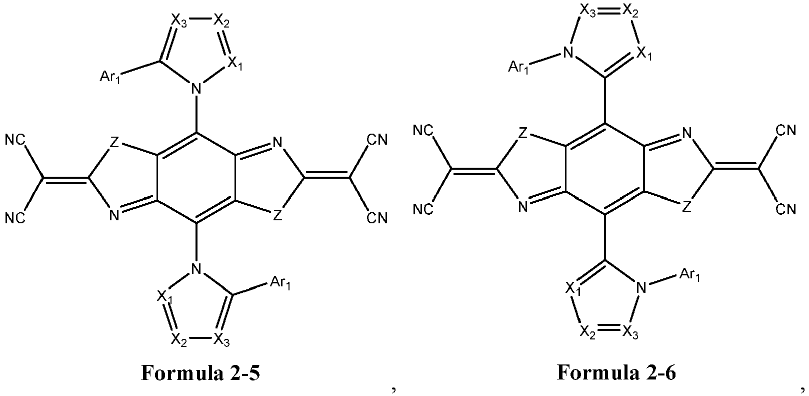 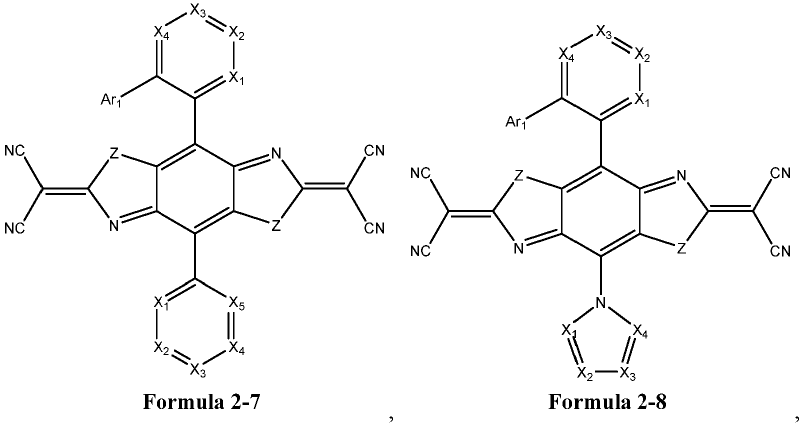 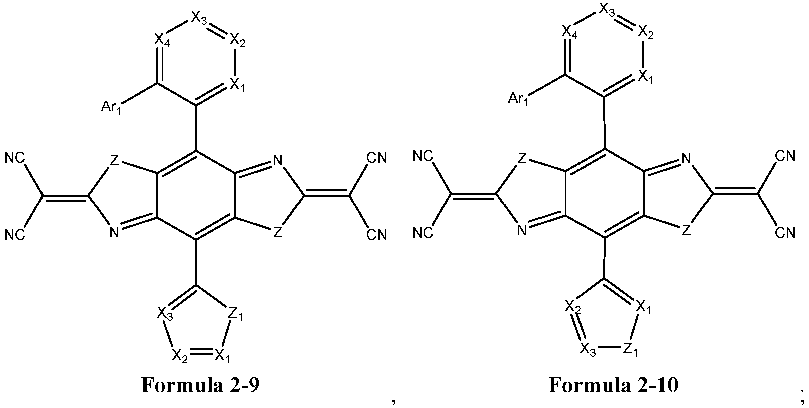
wherein Z is, at each occurrence identically or differently, selected from O, S or Se;
X₁ to X₅ are, at each occurrence identically or differently, selected from Nor CR₁;
Z₁ is, at each occurrence identically or differently, selected from O, S, Se or NR_{N1};
Ar₁ is, at each occurrence identically or differently, selected from the group consisting of: substituted or unsubstituted aryl having 6 to 30 carbon atoms, substituted or unsubstituted heteroaryl having 3 to 30 carbon atoms and combinations thereof; and when Ar₁ is selected from substituted aryl having 6 to 30 carbon atoms or substituted heteroaryl having 3 to 30 carbon atoms, the aryl or heteroaryl is substituted with one or more R₂;
R₁, R₂ and R_{N1} are, at each occurrence identically or differently, selected from the group consisting of: hydrogen, deuterium, a hydroxyl group, a sulfanyl group, halogen, a nitroso group, a nitro group, an acyl group, a carbonyl group, a carboxylic acid group, an ester group, a cyano group, an isocyano group, SCN, OCN, SF₅, a boranyl group, a sulfinyl group, a sulfonyl group, a phosphoroso group, substituted or unsubstituted alkyl having 1 to 20 carbon atoms, substituted or unsubstituted cycloalkyl having 3 to 20 ring carbon atoms, substituted or unsubstituted heteroalkyl having 1 to 20 carbon atoms, substituted or unsubstituted alkoxy having 1 to 20 carbon atoms, substituted or unsubstituted alkenyl having 2 to 20 carbon atoms, substituted or unsubstituted alkynyl having 2 to 20 carbon atoms, substituted or unsubstituted aryl having 6 to 30 carbon atoms, substituted or unsubstituted heteroaryl having 3 to 30 carbon atoms and combinations thereof;
at least one of the R₁ and R₂ is, at each occurrence identically or differently, selected from the group consisting of: a hydroxyl group, a sulfanyl group, halogen, a nitroso group, a nitro group, an acyl group, a carbonyl group, a carboxylic acid group, an ester group, a cyano group, an isocyano group, SCN, OCN, SF₅, a boranyl group, a sulfinyl group, a sulfonyl group, a phosphoroso group, substituted or unsubstituted alkyl having 1 to 20 carbon atoms, substituted or unsubstituted cycloalkyl having 3 to 20 ring carbon atoms, substituted or unsubstituted heteroalkyl having 1 to 20 carbon atoms, substituted or unsubstituted alkoxy having 1 to 20 carbon atoms, substituted or unsubstituted alkenyl having 2 to 20 carbon atoms, substituted or unsubstituted alkynyl having 2 to 20 carbon atoms, substituted or unsubstituted aryl having 6 to 30 carbon atoms, substituted or unsubstituted heteroaryl having 3 to 30 carbon atoms and combinations thereof; and
adjacent substituents R₁, R₂ can be optionally joined to form a ring.

According to another embodiment of the present disclosure, further disclosed is an electroluminescent device comprising an anode, a cathode and an organic layer disposed between the anode and the cathode, wherein the organic layer comprises a compound having a structure of any one of Formula 2-1 to Formula 2-10, and the specific structure of the compound is as shown in the preceding embodiment.

According to another embodiment of the present disclosure, further disclosed is a compound composition comprising a compound having a structure of any one of Formula 2-1 to Formula 2-10, and the specific structure of the compound is as shown in any one of the preceding embodiments.

The present disclosure discloses a novel compound having an ortho-substituted dehydrogenated fused-ring structure. The compound may be used as a charge transporting material, a charge injection material, and a similar material in an organic electroluminescent device. These novel compounds have an unexpected high sublimation yield due to their structural design. Additionally, these novel compounds can provide excellent performance for organic electroluminescent devices, such as low voltage and high efficiency.

### BRIEF DESCRIPTION OF DRAWINGS

FIG. 1 is a schematic diagram of an organic light-emitting apparatus that may contain a compound and a compound composition disclosed herein.
FIG. 2 is a schematic diagram of another organic light-emitting apparatus that may contain a compound and a compound composition disclosed herein.

### DETAILED DESCRIPTION

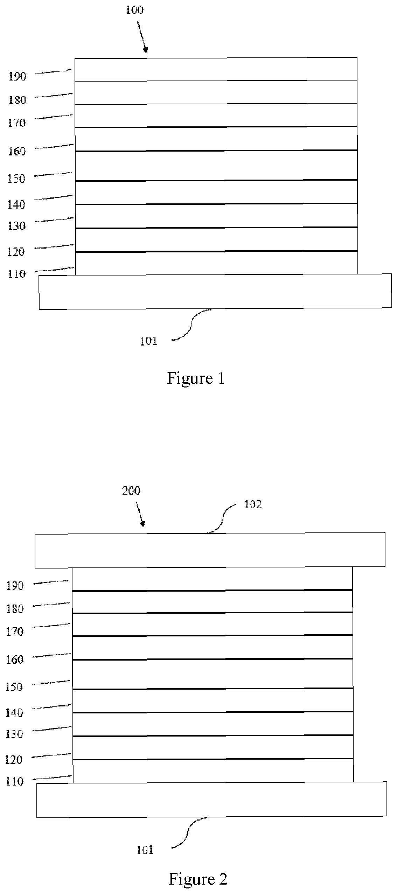

OLEDs can be fabricated on various types of substrates such as glass, plastic, and metal foil. FIG. 1 schematically shows an organic light-emitting device 100 without limitation. The figures are not necessarily drawn to scale. Some of the layers in the figures can also be omitted as needed. Device 100 may include a substrate 101, an anode 110, a hole injection layer 120, a hole transport layer 130, an electron blocking layer 140, an emissive layer 150, a hole blocking layer 160, an electron transport layer 170, an electron injection layer 180 and a cathode 190. Device 100 may be fabricated by depositing the layers described in order. The properties and functions of these various layers, as well as example materials, are described in more detail in U.S. Pat. No. 7,279,704 at cols. 6-10.

More examples for each of these layers are available. For example, a flexible and transparent substrate-anode combination is disclosed in U.S. Pat. No. 5,844,363. An example of a p-doped hole transport layer is m-MTDATA doped with F4-TCNQ at a molar ratio of 50:1, as disclosed in U.S. Patent Application Publication No. 2003/0230980. Examples of host materials are disclosed in U.S. Pat. No. 6,303,238 to Thompson et al.. An example of an n-doped electron transport layer is BPhen doped with Li at a molar ratio of 1:1, as disclosed in U.S. Patent Application Publication No. 2003/0230980. U.S. Pat. Nos. 5,703,436 and 5,707,745, disclose examples of cathodes including composite cathodes having a thin layer of metal such as Mg:Ag with an overlying transparent, electrically-conductive, sputter-deposited ITO layer. The theory and use of blocking layers are described in more detail in U.S. Pat. No. 6,097,147 and U.S. Patent Application Publication No. 2003/0230980. Examples of injection layers are provided in U.S. Patent Application Publication No. 2004/0174116. A description of protective layers may be found in U.S. Patent Application Publication No. 2004/0174116.

The layered structure described above is provided by way of non-limiting examples. Functional OLEDs may be achieved by combining the various layers described in different ways, or layers may be omitted entirely. It may also include other layers not specifically described. Within each layer, a single material or a mixture of multiple materials can be used to achieve optimum performance. Any functional layer may include several sublayers. For example, the emissive layer may have two layers of different emitting materials to achieve desired emission spectrum.

In one embodiment, an OLED may be described as having an "organic layer" disposed between a cathode and an anode. This organic layer may include a single layer or multiple layers.

An OLED can be encapsulated by a barrier layer. FIG. 2 schematically shows an organic light emitting device 200 without limitation. FIG. 2 differs from FIG. 1 in that the organic light emitting device include a barrier layer 102, which is above the cathode 190, to protect it from harmful species from the environment such as moisture and oxygen. Any material that can provide the barrier function can be used as the barrier layer such as glass or organic-inorganic hybrid layers. The barrier layer should be placed directly or indirectly outside of the OLED device. Multilayer thin film encapsulation was described in U.S. Pat. No. 7,968,146.

Devices fabricated in accordance with embodiments of the present disclosure can be incorporated into a wide variety of consumer products that have one or more of the electronic component modules (or units) incorporated therein. Some examples of such consumer products include flat panel displays, monitors, medical monitors, televisions, billboards, lights for interior or exterior illumination and/or signaling, heads-up displays, fully or partially transparent displays, flexible displays, smart phones, tablets, phablets, wearable devices, smart watches, laptop computers, digital cameras, camcorders, viewfinders, micro-displays, 3-D displays, vehicles displays, and vehicle tail lights.

The materials and structures described herein may be used in other organic electronic devices listed above.

As used herein, "top" means furthest away from the substrate, while "bottom" means closest to the substrate. Where a first layer is described as "disposed over" a second layer, the first layer is disposed further away from the substrate. There may be other layers between the first and second layers, unless it is specified that the first layer is "in contact with" the second layer. For example, a cathode may be described as "disposed over" an anode, even though there are various organic layers in between.

As used herein, "solution processible" means capable of being dissolved, dispersed, or transported in and/or deposited from a liquid medium, either in solution or suspension form.

A ligand may be referred to as "photoactive" when it is believed that the ligand directly contributes to the photoactive properties of an emissive material. A ligand may be referred to as "ancillary" when it is believed that the ligand does not contribute to the photoactive properties of an emissive material, although an ancillary ligand may alter the properties of a photoactive ligand.

It is believed that the internal quantum efficiency (IQE) of fluorescent OLEDs can exceed the 25% spin statistics limit through delayed fluorescence. As used herein, there are two types of delayed fluorescence, i.e. P-type delayed fluorescence and E-type delayed fluorescence. P-type delayed fluorescence is generated from triplet-triplet annihilation (TTA).

On the other hand, E-type delayed fluorescence does not rely on the collision of two triplets, but rather on the transition between the triplet states and the singlet excited states. Compounds that are capable of generating E-type delayed fluorescence are required to have very small singlet-triplet gaps to convert between energy states. Thermal energy can activate the transition from the triplet state back to the singlet state. This type of delayed fluorescence is also known as thermally activated delayed fluorescence (TADF). A distinctive feature of TADF is that the delayed component increases as temperature rises. If the reverse intersystem crossing (RISC) rate is fast enough to minimize the non-radiative decay from the triplet state, the fraction of back populated singlet excited states can potentially reach 75%. The total singlet fraction can be 100%, far exceeding 25% of the spin statistics limit for electrically generated excitons.

E-type delayed fluorescence characteristics can be found in an exciplex system or in a single compound. Without being bound by theory, it is believed that E-type delayed fluorescence requires the luminescent material to have a small singlet-triplet energy gap (ΔE_{S-T}). Organic, non-metal containing, donor-acceptor luminescent materials may be able to achieve this. The emission in these materials is generally characterized as a donor-acceptor charge-transfer (CT) type emission. The spatial separation of the HOMO and LUMO in these donor-acceptor type compounds generally results in small ΔE_{S-T}. These states may involve CT states. Generally, donor-acceptor luminescent materials are constructed by connecting an electron donor moiety such as amino- or carbazole-derivatives and an electron acceptor moiety such as N-containing six-membered aromatic rings.

### Definition of terms of substituents

Halogen or halide - as used herein includes fluorine, chlorine, bromine, and iodine.

Alkyl - as used herein includes both straight and branched chain alkyl groups. Alkyl may be alkyl having 1 to 20 carbon atoms, preferably alkyl having 1 to 12 carbon atoms, and more preferably alkyl having 1 to 6 carbon atoms. Examples of alkyl groups include a methyl group, an ethyl group, a propyl group, an isopropyl group, a n-butyl group, an s-butyl group, an isobutyl group, a t-butyl group, an n-pentyl group, an n-hexyl group, an n-heptyl group, an n-octyl group, an n-nonyl group, an n-decyl group, an n-undecyl group, an n-dodecyl group, an n-tridecyl group, an n-tetradecyl group, an n-pentadecyl group, an n-hexadecyl group, an n-heptadecyl group, an n-octadecyl group, a neopentyl group, a 1-methylpentyl group, a 2-methylpentyl group, a 1-pentylhexyl group, a 1-butylpentyl group, a 1-heptyloctyl group, and a 3-methylpentyl group. Of the above, preferred are a methyl group, an ethyl group, a propyl group, an isopropyl group, a n-butyl group, an s-butyl group, an isobutyl group, a t-butyl group, an n-pentyl group, a neopentyl group, and an n-hexyl group. Additionally, the alkyl group may be optionally substituted.

Cycloalkyl - as used herein includes cyclic alkyl groups. The cycloalkyl groups may be those having 3 to 20 ring carbon atoms, preferably those having 4 to 10 carbon atoms. Examples of cycloalkyl include cyclobutyl, cyclopentyl, cyclohexyl, 4-methylcyclohexyl, 4,4-dimethylcylcohexyl, 1-adamantyl, 2-adamantyl, 1-norbornyl, 2-norbornyl, and the like. Of the above, preferred are cyclopentyl, cyclohexyl, 4-methylcyclohexyl, and 4,4-dimethylcylcohexyl. Additionally, the cycloalkyl group may be optionally substituted.

Heteroalkyl - as used herein, includes a group formed by replacing one or more carbons in an alkyl chain with a hetero-atom(s) selected from the group consisting of a nitrogen atom, an oxygen atom, a sulfur atom, a selenium atom, a phosphorus atom, a silicon atom, a germanium atom, and a boron atom. Heteroalkyl may be those having 1 to 20 carbon atoms, preferably those having 1 to 10 carbon atoms, and more preferably those having 1 to 6 carbon atoms. Examples of heteroalkyl include methoxymethyl, ethoxymethyl, ethoxyethyl, methylthiomethyl, ethylthiomethyl, ethylthioethyl, methoxymethoxymethyl, ethoxymethoxymethyl, ethoxyethoxyethyl, hydroxymethyl, hydroxyethyl, hydroxypropyl, mercaptomethyl, mercaptoethyl, mercaptopropyl, aminomethyl, aminoethyl, aminopropyl, dimethylaminomethyl, trimethylgermanylmethyl, trimethylgermanylethyl, trimethylgermanylisopropyl, dimethylethylgermanylmethyl, dimethylisopropylgermanylmethyl, tert-butyldimethylgermanylmethyl, triethylgermanylmethyl, triethylgermanylethyl, triisopropylgermanylmethyl, triisopropylgermanylethyl, trimethylsilylmethyl, trimethylsilylethyl, trimethylsilylisopropyl, triisopropylsilylmethyl, and triisopropylsilylethyl. Additionally, the heteroalkyl group may be optionally substituted.

Alkenyl - as used herein includes straight chain, branched chain, and cyclic alkene groups. Alkenyl may be those having 2 to 20 carbon atoms, preferably those having 2 to 10 carbon atoms. Examples of alkenyl include vinyl, 1-propenyl group, 1-butenyl, 2-butenyl, 3-butenyl, 1,3-butandienyl, 1-methylvinyl, styryl, 2,2-diphenylvinyl, 1,2-diphenylvinyl, 1-methylallyl, 1,1-dimethylallyl, 2-methylallyl, 1-phenylallyl, 2-phenylallyl, 3-phenylallyl, 3,3-diphenylallyl, 1,2-dimethylallyl, 1-phenyl-1-butenyl, 3-phenyl-1-butenyl, cyclopentenyl, cyclopentadienyl, cyclohexenyl, cycloheptenyl, cycloheptatrienyl, cyclooctenyl, cyclooctatetraenyl, and norbornenyl. Additionally, the alkenyl group may be optionally substituted.

Alkynyl - as used herein includes straight chain alkynyl groups. Alkynyl may be those having 2 to 20 carbon atoms, preferably those having 2 to 10 carbon atoms. Examples of alkynyl groups include ethynyl, propynyl, propargyl, 1-butynyl, 2-butynyl, 3-butynyl, 1-pentynyl, 2-pentynyl, 3,3-dimethyl-1-butynyl, 3-ethyl-3-methyl-1-pentynyl, 3,3-diisopropyl-1-pentynyl, phenylethynyl, phenylpropynyl, etc. Of the above, preferred are ethynyl, propynyl, propargyl, 1-butynyl, 2-butynyl, 3-butynyl, 1-pentynyl, and phenylethynyl. Additionally, the alkynyl group may be optionally substituted.

Aryl or an aromatic group - as used herein includes non-condensed and condensed systems. Aryl may be those having 6 to 30 carbon atoms, preferably those having 6 to 20 carbon atoms, and more preferably those having 6 to 12 carbon atoms. Examples of aryl groups include phenyl, biphenyl, terphenyl, triphenylene, tetraphenylene, naphthalene, anthracene, phenalene, phenanthrene, fluorene, pyrene, chrysene, perylene, and azulene, preferably phenyl, biphenyl, terphenyl, triphenylene, fluorene, and naphthalene. Examples of non-condensed aryl groups include phenyl, biphenyl-2-yl, biphenyl-3-yl, biphenyl-4-yl, p-terphenyl-4-yl, p-terphenyl-3-yl, p-terphenyl-2-yl, m-terphenyl-4-yl, m-terphenyl-3-yl, m-terphenyl-2-yl, o-tolyl, m-tolyl, p-tolyl, p-(2-phenylpropyl)phenyl, 4'-methylbiphenylyl, 4"-t-butyl-p-terphenyl-4-yl, o-cumenyl, m-cumenyl, p-cumenyl, 2,3-xylyl, 3,4-xylyl, 2,5-xylyl, mesityl, and m-quarterphenyl. Additionally, the aryl group may be optionally substituted.

Heterocyclic groups or heterocycle - as used herein include non-aromatic cyclic groups. Non-aromatic heterocyclic groups include saturated heterocyclic groups having 3 to 20 ring atoms and unsaturated non-aromatic heterocyclic groups having 3 to 20 ring atoms, where at least one ring atom is selected from the group consisting of a nitrogen atom, an oxygen atom, a sulfur atom, a selenium atom, a silicon atom, a phosphorus atom, a germanium atom, and a boron atom. Preferred non-aromatic heterocyclic groups are those having 3 to 7 ring atoms, each of which includes at least one hetero-atom such as nitrogen, oxygen, silicon, or sulfur. Examples of non-aromatic heterocyclic groups include oxiranyl, oxetanyl, tetrahydrofuranyl, tetrahydropyranyl, dioxolanyl, dioxanyl, aziridinyl, dihydropyrrolyl, tetrahydropyrrolyl, piperidinyl, oxazolidinyl, morpholinyl, piperazinyl, oxepinyl, thiepinyl, azepinyl, and tetrahydrosilolyl. Additionally, the heterocyclic group may be optionally substituted.

Heteroaryl - as used herein, includes non-condensed and condensed hetero-aromatic groups having 1 to 5 hetero-atoms, where at least one hetero-atom is selected from the group consisting of a nitrogen atom, an oxygen atom, a sulfur atom, a selenium atom, a silicon atom, a phosphorus atom, a germanium atom, and a boron atom. A hetero-aromatic group is also referred to as heteroaryl. Heteroaryl may be those having 3 to 30 carbon atoms, preferably those having 3 to 20 carbon atoms, and more preferably those having 3 to 12 carbon atoms. Suitable heteroaryl groups include dibenzothiophene, dibenzofuran, dibenzoselenophene, furan, thiophene, benzofuran, benzothiophene, benzoselenophene, carbazole, indolocarbazole, pyridoindole, pyrrolodipyridine, pyrazole, imidazole, triazole, oxazole, thiazole, oxadiazole, oxatriazole, dioxazole, thiadiazole, pyridine, pyridazine, pyrimidine, pyrazine, triazine, oxazine, oxathiazine, oxadiazine, indole, benzimidazole, indazole, indoxazine, benzoxazole, benzisoxazole, benzothiazole, quinoline, isoquinoline, cinnoline, quinazoline, quinoxaline, naphthyridine, phthalazine, pteridine, xanthene, acridine, phenazine, phenothiazine, benzofuropyridine, furodipyridine, benzothienopyridine, thienodipyridine, benzoselenophenopyridine, and selenophenodipyridine, preferably dibenzothiophene, dibenzofuran, dibenzoselenophene, carbazole, indolocarbazole, imidazole, pyridine, triazine, benzimidazole, 1,2-azaborine, 1,3-azaborine, 1,4-azaborine, borazine, and aza-analogs thereof. Additionally, the heteroaryl group may be optionally substituted.

Alkoxy - as used herein, is represented by -O-alkyl, -O-cycloalkyl, -O-heteroalkyl, or - O-heterocyclic group. Examples and preferred examples of alkyl, cycloalkyl, heteroalkyl, and heterocyclic groups are the same as those described above. Alkoxy groups may be those having 1 to 20 carbon atoms, preferably those having 1 to 6 carbon atoms. Examples of alkoxy groups include methoxy, ethoxy, propoxy, butoxy, pentyloxy, hexyloxy, cyclopropyloxy, cyclobutyloxy, cyclopentyloxy, cyclohexyloxy, tetrahydrofuranyloxy, tetrahydropyranyloxy, methoxypropyloxy, ethoxyethyloxy, methoxymethyloxy, and ethoxymethyloxy. Additionally, the alkoxy group may be optionally substituted.

Aryloxy - as used herein, is represented by -O-aryl or -O-heteroaryl. Examples and preferred examples of aryl and heteroaryl are the same as those described above. Aryloxy groups may be those having 6 to 30 carbon atoms, preferably those having 6 to 20 carbon atoms. Examples of aryloxy groups include phenoxy and biphenyloxy. Additionally, the aryloxy group may be optionally substituted.

Arylalkyl - as used herein, contemplates alkyl substituted with an aryl group. Arylalkyl may be those having 7 to 30 carbon atoms, preferably those having 7 to 20 carbon atoms, and more preferably those having 7 to 13 carbon atoms. Examples of arylalkyl groups include benzyl, 1-phenylethyl, 2-phenylethyl, 1-phenylisopropyl, 2-phenylisopropyl, phenyl-t-butyl, alpha-naphthylmethyl, 1-alpha-naphthylethyl, 2-alpha-naphthylethyl, 1-alpha-naphthylisopropyl, 2-alpha-naphthylisopropyl, beta-naphthylmethyl, 1-beta-naphthylethyl, 2-beta-naphthylethyl, 1-beta-naphthylisopropyl, 2-beta-naphthylisopropyl, p-methylbenzyl, m-methylbenzyl, o-methylbenzyl, p-chlorobenzyl, m-chlorobenzyl, o-chlorobenzyl, p-bromobenzyl, m-bromobenzyl, o-bromobenzyl, p-iodobenzyl, m-iodobenzyl, o-iodobenzyl, p-hydroxybenzyl, m-hydroxybenzyl, o-hydroxybenzyl, p-aminobenzyl, m-aminobenzyl, o-aminobenzyl, p-nitrobenzyl, m-nitrobenzyl, o-nitrobenzyl, p-cyanobenzyl, m-cyanobenzyl, o-cyanobenzyl, 1-hydroxy-2-phenylisopropyl, and 1-chloro-2-phenylisopropyl. Of the above, preferred are benzyl, p-cyanobenzyl, m-cyanobenzyl, o-cyanobenzyl, 1-phenylethyl, 2-phenylethyl, 1-phenylisopropyl, and 2-phenylisopropyl. Additionally, the arylalkyl group may be optionally substituted.

Alkylsilyl - as used herein, contemplates a silyl group substituted with an alkyl group. Alkylsilyl groups may be those having 3 to 20 carbon atoms, preferably those having 3 to 10 carbon atoms. Examples of alkylsilyl groups include trimethylsilyl, triethylsilyl, methyldiethylsilyl, ethyldimethylsilyl, tripropylsilyl, tributylsilyl, triisopropylsilyl, methyldiisopropylsilyl, dimethylisopropylsilyl, tri-t-butylsilyl, triisobutylsilyl, dimethyl t-butylsilyl, and methyldi-t-butylsilyl. Additionally, the alkylsilyl group may be optionally substituted.

Arylsilyl - as used herein, contemplates a silyl group substituted with an aryl group. Arylsilyl groups may be those having 6 to 30 carbon atoms, preferably those having 8 to 20 carbon atoms. Examples of arylsilyl groups include triphenylsilyl, phenyldibiphenylylsilyl, diphenylbiphenylsilyl, phenyldiethylsilyl, diphenylethylsilyl, phenyldimethylsilyl, diphenylmethylsilyl, phenyldiisopropylsilyl, diphenylisopropylsilyl, diphenylbutylsilyl, diphenylisobutylsilyl, diphenyl t-butylsilyl. Additionally, the arylsilyl group may be optionally substituted.

Alkylgermanyl - as used herein contemplates a germanyl substituted with an alkyl group. The alkylgermanyl may be those having 3 to 20 carbon atoms, preferably those having 3 to 10 carbon atoms. Examples of alkylgermanyl include trimethylgermanyl, triethylgermanyl, methyldiethylgermanyl, ethyldimethylgermanyl, tripropylgermanyl, tributylgermanyl, triisopropylgermanyl, methyldiisopropylgermanyl, dimethylisopropylgermanyl, tri-t-butylgermanyl, triisobutylgermanyl, dimethyl-t-butylgermanyl, and methyldi-t-butylgermanyl. Additionally, the alkylgermanyl may be optionally substituted.

Arylgermanyl - as used herein contemplates a germanyl substituted with at least one aryl group or heteroaryl group. Arylgermanyl may be those having 6 to 30 carbon atoms, preferably those having 8 to 20 carbon atoms. Examples of arylgermanyl include triphenylgermanyl, phenyldibiphenylylgermanyl, diphenylbiphenylgermanyl, phenyldiethylgermanyl, diphenylethylgermanyl, phenyldimethylgermanyl, diphenylmethylgermanyl, phenyldiisopropylgermanyl, diphenylisopropylgermanyl, diphenylbutylgermanyl, diphenylisobutylgermanyl, and diphenyl-t-butylgermanyl. Additionally, the arylgermanyl may be optionally substituted.

The term "aza" in azadibenzofuran, azadibenzothiophene, etc. means that one or more of C-H groups in the respective aromatic fragment are replaced by a nitrogen atom. For example, azatriphenylene encompasses dibenzo[f,h]quinoxaline, dibenzo[f,h]quinoline and other analogs with two or more nitrogens in the ring system. One of ordinary skill in the art can readily envision other nitrogen analogs of the aza-derivatives described above, and all such analogs are intended to be encompassed by the terms as set forth herein.

In the present disclosure, unless otherwise defined, when any term of the group consisting of substituted alkyl, substituted cycloalkyl, substituted heteroalkyl, substituted heterocyclic group, substituted arylalkyl, substituted alkoxy, substituted aryloxy, substituted alkenyl, substituted alkynyl, substituted aryl, substituted heteroaryl, substituted alkylsilyl, substituted arylsilyl, substituted alkylgermanyl, substituted arylgermanyl, substituted amino, substituted acyl, substituted carbonyl, a substituted carboxylic acid group, a substituted ester group, substituted sulfinyl, substituted sulfonyl, and substituted phosphino is used, it means that any group of alkyl, cycloalkyl, heteroalkyl, heterocyclic group, arylalkyl, alkoxy, aryloxy, alkenyl, alkynyl, aryl, heteroaryl, alkylsilyl, arylsilyl, alkylgermanyl, arylgermanyl, amino, acyl, carbonyl, a carboxylic acid group, an ester group, sulfinyl, sulfonyl, and phosphino may be substituted with one or more groups selected from the group consisting of deuterium, halogen, unsubstituted alkyl having 1 to 20 carbon atoms, unsubstituted cycloalkyl having 3 to 20 ring carbon atoms, unsubstituted heteroalkyl having 1 to 20 carbon atoms, an unsubstituted heterocyclic group having 3 to 20 ring atoms, unsubstituted arylalkyl having 7 to 30 carbon atoms, unsubstituted alkoxy having 1 to 20 carbon atoms, unsubstituted aryloxy having 6 to 30 carbon atoms, unsubstituted alkenyl having 2 to 20 carbon atoms, unsubstituted alkynyl having 2 to 20 carbon atoms, unsubstituted aryl having 6 to 30 carbon atoms, unsubstituted heteroaryl having 3 to 30 carbon atoms, unsubstituted alkylsilyl having 3 to 20 carbon atoms, unsubstituted arylsilyl group having 6 to 20 carbon atoms, unsubstituted alkylgermanyl group having 3 to 20 carbon atoms, unsubstituted arylgermanyl group having 6 to 20 carbon atoms, unsubstituted amino having 0 to 20 carbon atoms, an acyl group, a carbonyl group, a carboxylic acid group, an ester group, a cyano group, an isocyano group, a hydroxyl group, a sulfanyl group, a sulfinyl group, a sulfonyl group, a phosphino group, and combinations thereof.

It is to be understood that when a molecular fragment is described as being a substituent or otherwise attached to another moiety, its name may be written as if it were a fragment (e.g. phenyl, phenylene, naphthyl, dibenzofuryl) or as if it were the whole molecule (e.g. benzene, naphthalene, dibenzofuran). As used herein, these different ways of designating a substituent or an attached fragment are considered to be equivalent.

In the compounds mentioned in the present disclosure, hydrogen atoms may be partially or fully replaced by deuterium. Other atoms such as carbon and nitrogen may also be replaced by their other stable isotopes. The replacement by other stable isotopes in the compounds may be preferred due to its enhancements of device efficiency and stability.

In the compounds mentioned in the present disclosure, multiple substitutions refer to a range that includes di-substitutions, up to the maximum available substitutions. When substitution in the compounds mentioned in the present disclosure represents multiple substitutions (including di-, tri-, and tetra-substitutions etc.), that means the substituent may exist at a plurality of available substitution positions on its linking structure, the substituents present at a plurality of available substitution positions may have the same structure or different structures.

In the compounds mentioned in the present disclosure, adjacent substituents in the compounds cannot be joined to form a ring unless otherwise explicitly defined, for example, adjacent substituents can be optionally joined to form a ring. In the compounds mentioned in the present disclosure, the expression that adjacent substituents can be optionally joined to form a ring includes a case where adjacent substituents may be joined to form a ring and a case where adjacent substituents are not joined to form a ring. When adjacent substituents can be optionally joined to form a ring, the ring formed may be monocyclic or polycyclic (including spirocyclic, endocyclic, fusedcyclic, and etc.), as well as alicyclic, heteroalicyclic, aromatic, or heteroaromatic. In such expression, adjacent substituents may refer to substituents bonded to the same atom, substituents bonded to carbon atoms which are directly bonded to each other, or substituents bonded to carbon atoms which are more distant from each other. Preferably, adjacent substituents refer to substituents bonded to the same carbon atom and substituents bonded to carbon atoms which are directly bonded to each other.

The expression that adjacent substituents can be optionally joined to form a ring is also intended to mean that two substituents bonded to the same carbon atom are joined to each other via a chemical bond to form a ring, which can be exemplified by the following formula:

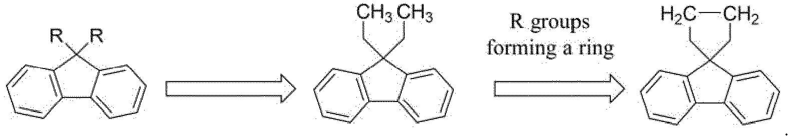

The expression that adjacent substituents can be optionally joined to form a ring is also intended to mean that two substituents bonded to carbon atoms which are directly bonded to each other are joined to each other via a chemical bond to form a ring, which can be exemplified by the following formula:

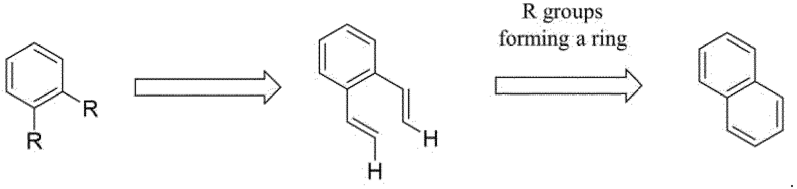

The expression that adjacent substituents can be optionally joined to form a ring is also intended to mean that two substituents bonded to further distant carbon atoms are joined to each other via a chemical bond to form a ring, which can be exemplified by the following formula:

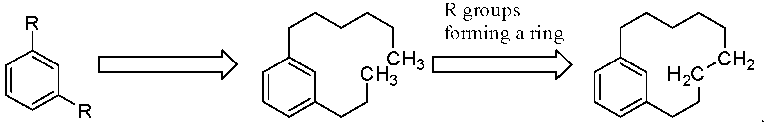

Furthermore, the expression that adjacent substituents can be optionally joined to form a ring is also intended to mean that, in the case where one of the two substituents bonded to carbon atoms which are directly bonded to each other represents hydrogen, the second substituent is bonded at a position at which the hydrogen atom is bonded, thereby forming a ring. This is exemplified by the following formula:

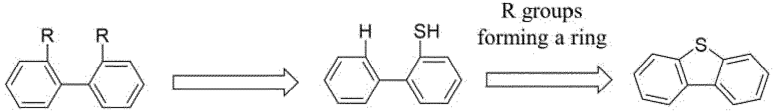

According to an embodiment of the present disclosure, disclosed is a compound having a structure represented by any one of Formula 2-1 to Formula 2-10: 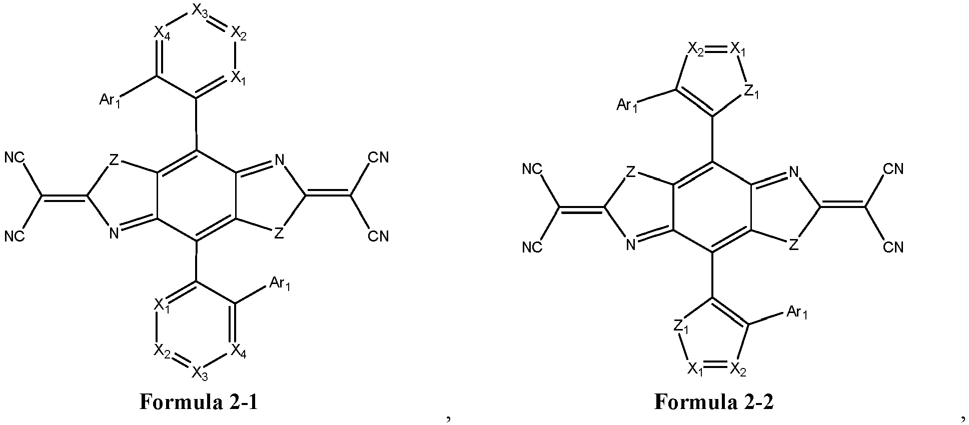 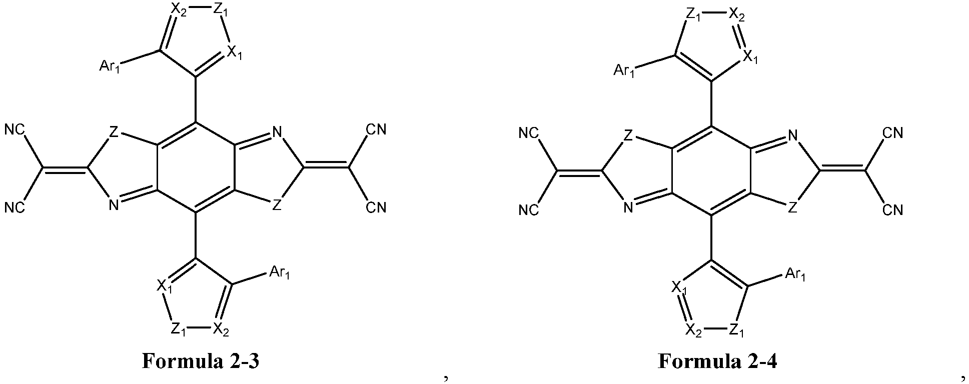 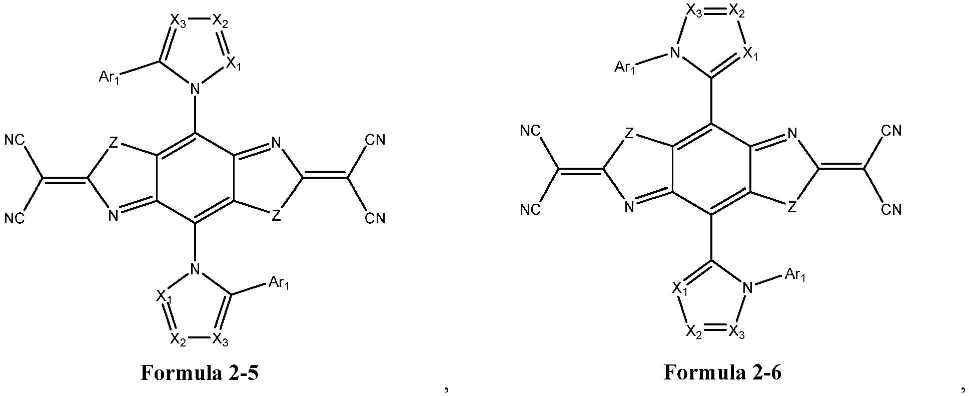 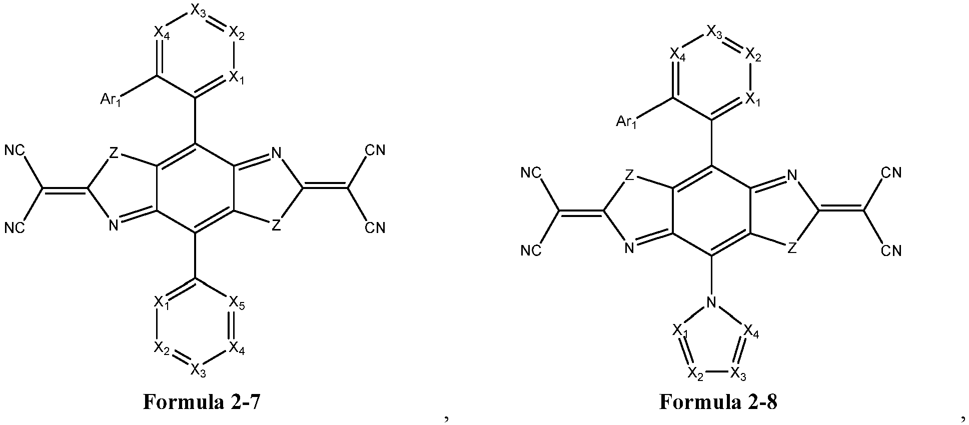 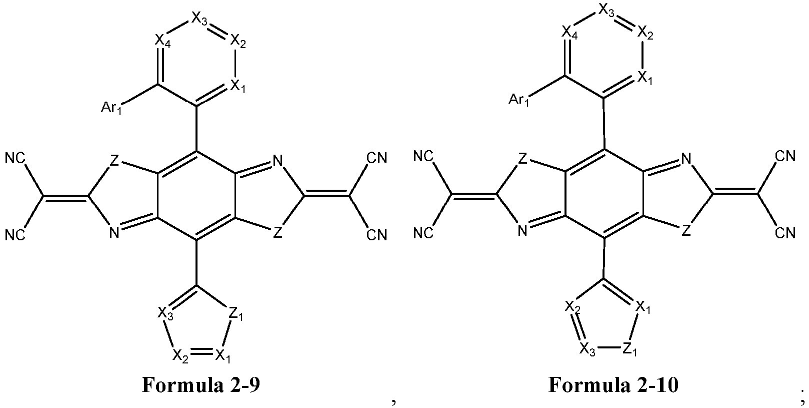
Z is, at each occurrence identically or differently, selected from O, S or Se;
X₁ to X₅ are, at each occurrence identically or differently, selected from N or CR₁;
Z₁ is, at each occurrence identically or differently, selected from O, S, Se or NR_{N1};
Ar₁ is, at each occurrence identically or differently, selected from the group consisting of: substituted or unsubstituted aryl having 6 to 30 carbon atoms, substituted or unsubstituted heteroaryl having 3 to 30 carbon atoms and combinations thereof; and when Ar₁ is selected from substituted aryl having 6 to 30 carbon atoms or substituted heteroaryl having 3 to 30 carbon atoms, the aryl or heteroaryl is substituted with one or more R₂;
R₁, R₂ and R_{N1} are, at each occurrence identically or differently, selected from the group consisting of: hydrogen, deuterium, a hydroxyl group, a sulfanyl group, halogen, a nitroso group, a nitro group, an acyl group, a carbonyl group, a carboxylic acid group, an ester group, a cyano group, an isocyano group, SCN, OCN, SF₅, a boranyl group, a sulfinyl group, a sulfonyl group, a phosphoroso group, substituted or unsubstituted alkyl having 1 to 20 carbon atoms, substituted or unsubstituted cycloalkyl having 3 to 20 ring carbon atoms, substituted or unsubstituted heteroalkyl having 1 to 20 carbon atoms, substituted or unsubstituted alkoxy having 1 to 20 carbon atoms, substituted or unsubstituted alkenyl having 2 to 20 carbon atoms, substituted or unsubstituted alkynyl having 2 to 20 carbon atoms, substituted or unsubstituted aryl having 6 to 30 carbon atoms, substituted or unsubstituted heteroaryl having 3 to 30 carbon atoms and combinations thereof;
at least one of R₁ and R₂ is, at each occurrence identically or differently, selected from the group consisting of: a hydroxyl group, a sulfanyl group, halogen, a nitroso group, a nitro group, an acyl group, a carbonyl group, a carboxylic acid group, an ester group, a cyano group, an isocyano group, SCN, OCN, SF₅, a boranyl group, a sulfinyl group, a sulfonyl group, a phosphoroso group, substituted or unsubstituted alkyl having 1 to 20 carbon atoms, substituted or unsubstituted cycloalkyl having 3 to 20 ring carbon atoms, substituted or unsubstituted heteroalkyl having 1 to 20 carbon atoms, substituted or unsubstituted alkoxy having 1 to 20 carbon atoms, substituted or unsubstituted alkenyl having 2 to 20 carbon atoms, substituted or unsubstituted alkynyl having 2 to 20 carbon atoms, substituted or unsubstituted aryl having 6 to 30 carbon atoms, substituted or unsubstituted heteroaryl having 3 to 30 carbon atoms and combinations thereof; and
adjacent substituents R₁, R₂ can be optionally joined to form a ring.

In the present disclosure, the expression that adjacent substituents R₁, R₂ can be optionally joined to form a ring is intended to mean that any one or more of groups of adjacent substituents, such as adjacent substituents R₁, adjacent substituents R₂, and adjacent substituents R₁ and R₂, can be joined to form a ring. Obviously, it is possible that none of these groups of adjacent substituents are joined to form a ring.

According to an embodiment of the present disclosure, wherein, X₁ is, at each occurrence identically or differently, selected from CR₁.

According to an embodiment of the present disclosure, wherein, the compound has a structure represented by Formula 3-1: 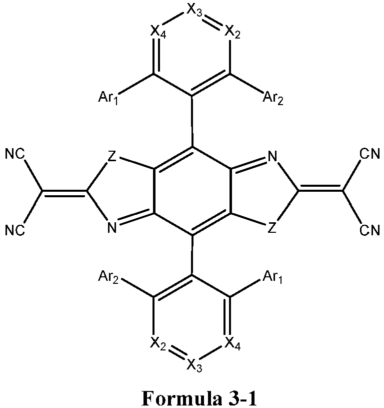 wherein in Formula 3-1,
Z is, at each occurrence identically or differently, selected from O, S or Se;
X₂ to X₄ are, at each occurrence identically or differently, selected from N or CR₁;
Ar₁ and Ar₂ are, at each occurrence identically or differently, selected from the group consisting of: substituted or unsubstituted aryl having 6 to 30 carbon atoms, substituted or unsubstituted heteroaryl having 3 to 30 carbon atoms and combinations thereof; and when Ar₁ and Ar₂ are selected from substituted aryl having 6 to 30 carbon atoms or substituted heteroaryl having 3 to 30 carbon atoms, the aryl or heteroaryl is substituted with one or more R₂;
R₁ and R₂ are, at each occurrence identically or differently, selected from the group consisting of: hydrogen, deuterium, halogen, a hydroxyl group, a sulfanyl group, a nitroso group, a nitro group, an acyl group, a carbonyl group, a carboxylic acid group, an ester group, a cyano group, an isocyano group, SCN, OCN, SF₅, a boranyl group, a sulfinyl group, a sulfonyl group, a phosphoroso group, substituted or unsubstituted alkyl having 1 to 20 carbon atoms, substituted or unsubstituted cycloalkyl having 3 to 20 ring carbon atoms, substituted or unsubstituted heteroalkyl having 1 to 20 carbon atoms, substituted or unsubstituted alkoxy having 1 to 20 carbon atoms, substituted or unsubstituted alkenyl having 2 to 20 carbon atoms, substituted or unsubstituted alkynyl having 2 to 20 carbon atoms, substituted or unsubstituted aryl having 6 to 30 carbon atoms, substituted or unsubstituted heteroaryl having 3 to 30 carbon atoms and combinations thereof;
at least one of the R₁ and R₂ is, at each occurrence identically or differently, selected from the group consisting of: halogen, a hydroxyl group, a sulfanyl group, a nitroso group, a nitro group, an acyl group, a carbonyl group, a carboxylic acid group, an ester group, a cyano group, an isocyano group, SCN, OCN, SF₅, a boranyl group, a sulfinyl group, a sulfonyl group, a phosphoroso group, substituted or unsubstituted alkyl having 1 to 20 carbon atoms, substituted or unsubstituted cycloalkyl having 3 to 20 ring carbon atoms, substituted or unsubstituted heteroalkyl having 1 to 20 carbon atoms, substituted or unsubstituted alkoxy having 1 to 20 carbon atoms, substituted or unsubstituted alkenyl having 2 to 20 carbon atoms, substituted or unsubstituted alkynyl having 2 to 20 carbon atoms, substituted or unsubstituted aryl having 6 to 30 carbon atoms, substituted or unsubstituted heteroaryl having 3 to 30 carbon atoms and combinations thereof; and
adjacent substituents R₁, R₂ can be optionally joined to form a ring.

According to an embodiment of the present disclosure, wherein, Z is, at each occurrence identically or differently, selected from O or S.

According to an embodiment of the present disclosure, wherein, Z is selected from O.

According to an embodiment of the present disclosure, wherein, in Formula 2-1 to Formula 2-10, at least one of X₁ to X₅ is, at each occurrence identically or differently, selected from CR₁.

According to an embodiment of the present disclosure, wherein, in Formula 2-1 to Formula 2-10, X₁ to X₅ are, at each occurrence identically or differently, selected from CR₁.

According to an embodiment of the present disclosure, wherein, in Formula 2-1 to Formula 2-10, at least one of X₁ to X₅ is selected from N.

According to an embodiment of the present disclosure, wherein, in Formula 2-1 to Formula 2-10, at least two of X₁ to X₅ are selected from N.

According to an embodiment of the present disclosure, wherein, in Formula 2-1 to Formula 2-10, two of X₁ to X₅ are selected from N.

According to an embodiment of the present disclosure, wherein, the R₁ and R₂ are, at each occurrence identically or differently, selected from the group consisting of: hydrogen, deuterium, halogen, cyano, isocyano, SCN, OCN, SF₅, boranyl, substituted or unsubstituted alkyl having 1 to 20 carbon atoms, substituted or unsubstituted heteroalkyl having 1 to 20 carbon atoms, substituted or unsubstituted alkoxy having 1 to 20 carbon atoms, substituted or unsubstituted aryl having 6 to 30 carbon atoms, substituted or unsubstituted heteroaryl having 3 to 30 carbon atoms and combinations thereof;
at least one of the R₁ and R₂ is, at each occurrence identically or differently, selected from the group consisting of: halogen, cyano, isocyano, SCN, OCN, SF₅, boranyl, substituted or unsubstituted alkyl having 1 to 20 carbon atoms, substituted or unsubstituted heteroalkyl having 1 to 20 carbon atoms, substituted or unsubstituted alkoxy having 1 to 20 carbon atoms, substituted or unsubstituted aryl having 6 to 30 carbon atoms, substituted or unsubstituted heteroaryl having 3 to 30 carbon atoms and combinations thereof; and
adjacent substituents R₁, R₂ can be optionally joined to form a ring.

According to an embodiment of the present disclosure, wherein, the R₁ and R₂ are, at each occurrence identically or differently, selected from the group consisting of: hydrogen, deuterium, halogen, cyano, isocyano, SCN, OCN, SF₅, boranyl, substituted or unsubstituted alkyl having 1 to 20 carbon atoms, substituted or unsubstituted heteroalkyl having 1 to 20 carbon atoms, substituted or unsubstituted alkoxy having 1 to 20 carbon atoms, substituted or unsubstituted aryl having 6 to 30 carbon atoms, substituted or unsubstituted heteroaryl having 3 to 30 carbon atoms and combinations thereof;
at least one of the R₁ and R₂ is, at each occurrence identically or differently, selected from the group consisting of: fluorine, SF₅, cyano, substituted alkyl having 1 to 20 carbon atoms, substituted heteroalkyl having 1 to 20 carbon atoms, substituted alkoxy having 1 to 20 carbon atoms, substituted aryl having 6 to 30 carbon atoms, substituted heteroaryl having 3 to 30 carbon atoms and combinations thereof; and a substituent in the substituted alkyl, substituted heteroalkyl, substituted alkoxy, substituted aryl or substituted heteroaryl comprises at least one electron-withdrawing group; and
adjacent substituents R₁, R₂ can be optionally joined to form a ring.

According to an embodiment of the present disclosure, wherein, a Hammett constant of the electron-withdrawing group is greater than or equal to 0.05, preferably greater than or equal to 0.3, and more preferably greater than or equal to 0.5.

In the present disclosure, the electron-withdrawing group has a Hammett constant greater than or equal to 0.05. The relatively strong electron-withdrawing ability can significantly reduce the LUMO energy level of the compound and improve charge mobility.

It is to be noted that the Hammett constant includes a Hammett para constant and/or a Hammett meta constant, and as long as one of the para constant and the meta constant is greater than or equal to 0.05, the substituent can be used as the preferred electron-withdrawing group of the present disclosure.

According to an embodiment of the present disclosure, wherein, the electron-withdrawing group is selected from the group consisting of: halogen, a nitroso group, a nitro group, an acyl group, a carbonyl group, a carboxylic acid group, an ester group, a cyano group, an isocyano group, SCN, OCN, SF₅, a boranyl group, a sulfinyl group, a sulfonyl group, a phosphoroso group, an aza-aromatic ring group and any one of the following groups substituted with one or more of halogen, a nitroso group, a nitro group, an acyl group, a carbonyl group, a carboxylic acid group, an ester group, a cyano group, an isocyano group, SCN, OCN, SF₅, a boranyl group, a sulfinyl group, a sulfonyl group, a phosphoroso group and an aza-aromatic ring group: alkyl having 1 to 20 carbon atoms, cycloalkyl having 3 to 20 ring carbon atoms, heteroalkyl having 1 to 20 carbon atoms, arylalkyl having 7 to 30 carbon atoms, alkoxy having 1 to 20 carbon atoms, aryloxy having 6 to 30 carbon atoms, alkenyl having 2 to 20 carbon atoms, alkynyl having 2 to 20 carbon atoms, aryl having 6 to 30 carbon atoms, heteroaryl having 3 to 30 carbon atoms, alkylsilyl having 3 to 20 carbon atoms, arylsilyl having 6 to 20 carbon atoms, alkylgermanyl having 3 to 20 carbon atoms, arylgermanyl having 6 to 20 carbon atoms and combinations thereof.

According to an embodiment of the present disclosure, wherein, the electron-withdrawing group is selected from the group consisting of: F, CF₃, OCF₃, SF₅, SO₂CF₃, cyano, isocyano, SCN, OCN, pyrimidinyl, triazinyl and combinations thereof.

According to an embodiment of the present disclosure, wherein, the R₁ and R₂ are, at each occurrence identically or differently, selected from the group consisting of: hydrogen, deuterium, halogen, cyano, isocyano, SCN, OCN, SF₅, boranyl, substituted or unsubstituted alkyl having 1 to 20 carbon atoms, substituted or unsubstituted heteroalkyl having 1 to 20 carbon atoms, substituted or unsubstituted alkoxy having 1 to 20 carbon atoms, substituted or unsubstituted aryl having 6 to 30 carbon atoms, substituted or unsubstituted heteroaryl having 3 to 30 carbon atoms and combinations thereof; and
at least one of the R₁ and R₂ is, at each occurrence identically or differently, selected from the group consisting of: fluorine, SF₅, cyano, substituted alkyl having 1 to 20 carbon atoms, substituted heteroalkyl having 1 to 20 carbon atoms, substituted alkoxy having 1 to 20 carbon atoms, substituted aryl having 6 to 30 carbon atoms, substituted heteroaryl having 3 to 30 carbon atoms and combinations thereof; and a substituent in the substituted alkyl, substituted heteroalkyl, substituted alkoxy, substituted aryl or substituted heteroaryl comprises at least one fluorine atom and/or at least one cyano group.

According to an embodiment of the present disclosure, wherein, R₁ and R₂ are, at each occurrence identically or differently, selected from the group consisting of: hydrogen, deuterium, fluorine, cyano, methyl, ethyl, isopropyl, n-propyl, t-butyl, -CF₃, -C₂F₅, -C₃F₇, -C₄F₉, -C₅F₁₁, - C₆F₁₃, -C₇F₁₅, -OCF₃, -OC₂F₅, -OC₃F₇, -OC₄F₉, -OC₅F₁₁, -OC₆F₁₃, -OC₇F₁₅, -SCF₃, -SC₂F₅, - SC₃F₇, -SC₄F₉, -SC₅F₁₁, -SC₆F₁₃, -SC₇F₁₅, -SF₅ and any one of the following groups substituted with one or more groups selected from the group consisting of: fluorine, cyano, methyl, ethyl, isopropyl, n-propyl, t-butyl, -CF₃, -C₂F₅, -C₃F₇, -C₄F₉, -C₅F₁₁, -C₆F₁₃, -C₇F₁₅, -OCF₃, -OC₂F₅, - OC₃F₇, -OC₄F₉, -OC₅F₁₁, -OC₆F₁₃, -OC₇F₁₅, -SCF₃, -SC₂F₅, -SC₃F₇, -SC₄F₉, -SC₅F₁₁, -SC₆F₁₃, - SC₇F₁₅ and -SF₅: aryl having 6 to 30 carbon atoms or heteroaryl having 3 to 30 carbon atoms;
at least one of the R₁ and R₂ is, at each occurrence identically or differently, selected from the group consisting of: fluorine, cyano, -CF₃, -C₂F₅, -C₃F₇, -C₄F₉, -C₅F₁₁, -C₆F₁₃, -C₇F₁₅, -OCF₃, -OC₂F₅, -OC₃F₇, -OC₄F₉, -OC₅F₁₁, -OC₆F₁₃, -OC₇F₁₅, -SCF₃, -SC₂F₅, -SC₃F₇, -SC₄F₉, -SC₅F₁₁, - SC₆F₁₃, -SC₇F₁₅, -SF₅ and any one of the following groups substituted with one or more groups selected from the group consisting of: fluorine, cyano, -CF₃, -C₂F₅, -C₃F₇, -C₄F₉, -C₅F₁₁, -C₆F₁₃, -C₇F₁₅, -OCF₃, -OC₂F₅, -OC₃F₇, -OC₄F₉, -OC₅F₁₁, -OC₆F₁₃, -OC₇F₁₅, -SCF₃, -SC₂F₅, -SC₃F₇, - SC₄F₉, -SC₅F₁₁, -SC₆F₁₃, -SC₇F₁₅ and -SF₅: aryl having 6 to 30 carbon atoms or heteroaryl having 3 to 30 carbon atoms; and
adjacent substituents R₁, R₂ can be optionally joined to form a ring.

In the present disclosure, -CₙF₂ₙ₊₁ (n is selected from 1 to 20, for example, -C₃F₇, -C₄F₉, -C₅F₁₁, -C₆F₁₃, -C₇F₁₅) represents perfluoro-substituted alkyl having n carbon atoms and comprises all straight-chain and branched-chain isomers that can exist. For example, -C₃F₇ represents -CF₂CF₂CF₃ and -CF(CF₃)CF₃. Similarly, -OCₙF₂ₙ₊₁ (n is selected from 1 to 20, for example, -OC₃F₇, -OC₄F₉, -OC₅F₁₁, -OC₆F₁₃, -OC₇F₁₅) represents perfluoro-substituted alkoxy having n carbon atoms and comprises all straight-chain and branched-chain isomers that can exist. For example, -OC₃F₇ represents -OCF₂CF₂CF₃ and -OCF(CF₃)CF₃. Similarly, -SCₙF₂ₙ₊₁ (n is selected from 1 to 20, for example, -SC₃F₇, -SC₄F₉, -SC₅F₁₁, -SC₆F₁₃, -SC₇F₁₅) represents perfluoro-substituted alkylthio having n carbon atoms and comprises all straight-chain and branched-chain isomers that can exist. For example, -SC₃F₇ represents -SCF₂CF₂CF₃ and - SCF(CF₃)CF₃. Obviously, it will be appreciated by those skilled in the art that -*n*CₙF₂ₙ₊₁ (for example, -*n*C₃F₇, -*n*C₄F₉, -*n*C₅F₁₁, -*n*C₆F₁₃, -*n*C₇F₁₅) represents only perfluoro-substituted normal alkyl (straight-chain alkyl) having n carbon atoms. For example, -*n*C₃F₇ represents only - CF₂CF₂CF₃; similarly, -O-*n*C*ₙ*F₂ₙ₊₁ (for example, -O-*n*C₃F₇, -O-*n*C₄F₉, -O-*n*C₅F₁₁, -O-*n*C₆F₁₃, - O-*n*C₇F₁₅) represents only perfluoro-substituted normal alkoxy (straight-chain alkoxy) having n carbon atoms. For example, -O-*n*C₃F₇ represents only -O-CF₂CF₂CF₃; similarly, -S-*n*CₙF₂ₙ₊₁ (for example, -S-*n*C₃F₇, -S-*n*C₄F₉, -S-*n*C₅F₁₁, -S-*n*C₆F₁₃, -S-*n*C₇F₁₅) represents only perfluoro-substituted normal alkylthio (straight-chain alkylthio) having n carbon atoms. For example, -S-*n*C₃F₇ represents only -S-CF₂CF₂CF₃.

According to an embodiment of the present disclosure, wherein, R₁ and R₂ are, at each occurrence identically or differently, selected from the group consisting of: hydrogen, deuterium, fluorine, cyano, methyl, ethyl, isopropyl, n-propyl, t-butyl, -CF₃, -C₂F₅, -C₃F₇, -C₄F₉, -C₅F₁₁, - C₆F₁₃, -C₇F₁₅, -OCF₃, -OC₂F₅, -OC₃F₇, -OC₄F₉, -OC₅F₁₁, -OC₆F₁₃, -OC₇F₁₅, -SCF₃, -SC₂F₅, - SC₃F₇, -SC₄F₉, -SC₅F₁₁, -SC₆F₁₃, -SC₇F₁₅, -SF₅ and any one of the following groups substituted with one or more groups selected from the group consisting of: F, OCF₃, CN and CF₃: phenyl, biphenyl, pyridyl, pyrimidinyl, triazinyl, oxaboraanthryl and combinations thereof;
at least one of R₁ and R₂ is, at each occurrence identically or differently, selected from the group consisting of: fluorine, cyano, -CF₃, -C₂F₅, -C₃F₇, -C₄F₉, -C₅F₁₁, -C₆F₁₃, -C₇F₁₅, -OCF₃, - OC₂F₅, -OC₃F₇, -OC₄F₉, -OC₅F₁₁, -OC₆F₁₃, -OC₇F₁₅, -SCF₃, -SC₂F₅, -SC₃F₇, -SC₄F₉, -SC₅F₁₁, - SC₆F₁₃, -SC₇F₁₅, -SF₅, phenyl or biphenyl substituted with one or more of F, OCF₃, CN or CF₃, pyridyl, pyrimidinyl, triazinyl, oxaboraanthryl and combinations thereof; and
adjacent substituents R₁, R₂ can be optionally joined to form a ring.

According to an embodiment of the present disclosure, wherein, R₁ and R₂ are, at each occurrence identically or differently, selected from the group consisting of A1 to A19 and B1 to B314. For the specific structures of A1 to A19 and B1 to B314, refer to claim 7.

According to an embodiment of the present disclosure, wherein, Ar₁ and Ar₂ are, at each occurrence identically or differently, selected from the group consisting of B1 to B314. For the specific structures of B1 to B314, refer to claim 7.

According to an embodiment of the present disclosure, wherein, the compound is selected from the group consisting of Compound H1-1 to Compound H1-2449, Compound H2-1 to Compound H2-138, Compound H3-1 to Compound H3-92, Compound H4-1 to Compound H4-92, Compound H5-1 to Compound H5-115, Compound H6-1 to Compound H6-115, Compound H7-1 to Compound H7-203 and Compound H8-7 to Compound H8-54, Compound H8-61 to Compound H8-108, Compound H8-115 to Compound H8-162, Compound H8-169 to Compound H8-216, Compound H8-223 to Compound H8-270, Compound H8-277 to Compound H8-324, Compound H8-331 to Compound H8-378, Compound H8-385 to Compound H8-432, Compound H8-439 to Compound H8-486, Compound H8-493 to Compound H8-540, Compound H8-547 to Compound H8-594, Compound H8-601 to Compound H8-648, Compound H8-655 to Compound H8-702, Compound H8-709 to Compound H8-756, Compound H8-763 to Compound H8-810, Compound H8-817 to Compound H8-864, Compound H8-871 to Compound H8-918, Compound H8-925 to Compound H8-972, Compound H8-979 to Compound H8-1026, Compound H8-1033 to Compound H8-1080, Compound H8-1087 to Compound H8-1134, Compound H8-1141 to Compound H8-1188, Compound H8-1195 to Compound H8-1242, Compound H8-1249 to Compound H8-1296, Compound H8-1303 to Compound H8-1350, Compound H8-1357 to Compound H8-1404, Compound H8-1411 to Compound H8-1458, Compound H8-1465 to Compound H8-1512. For the specific structures of Compound H1-1 to Compound H1-2449, Compound H2-1 to Compound H2-138, Compound H3-1 to Compound H3-92, Compound H4-1 to Compound H4-92, Compound H5-1 to Compound H5-115, Compound H6-1 to Compound H6-115, Compound H7-1 to Compound H7-203 and Compound H8-7 to Compound H8-54, Compound H8-61 to Compound H8-108, Compound H8-115 to Compound H8-162, Compound H8-169 to Compound H8-216, Compound H8-223 to Compound H8-270, Compound H8-277 to Compound H8-324, Compound H8-331 to Compound H8-378, Compound H8-385 to Compound H8-432, Compound H8-439 to Compound H8-486, Compound H8-493 to Compound H8-540, Compound H8-547 to Compound H8-594, Compound H8-601 to Compound H8-648, Compound H8-655 to Compound H8-702, Compound H8-709 to Compound H8-756, Compound H8-763 to Compound H8-810, Compound H8-817 to Compound H8-864, Compound H8-871 to Compound H8-918, Compound H8-925 to Compound H8-972, Compound H8-979 to Compound H8-1026, Compound H8-1033 to Compound H8-1080, Compound H8-1087 to Compound H8-1134, Compound H8-1141 to Compound H8-1188, Compound H8-1195 to Compound H8-1242, Compound H8-1249 to Compound H8-1296, Compound H8-1303 to Compound H8-1350, Compound H8-1357 to Compound H8-1404, Compound H8-1411 to Compound H8-1458, Compound H8-1465 to Compound H8-1512, refer to claim 8.

According to an embodiment of the present disclosure, further disclosed is an electroluminescent device, which comprises:
an anode,
a cathode and
an organic layer disposed between the anode and the cathode, wherein the organic layer comprises a compound having a structure of any one of Formula 2-1 to Formula 2-10, and the specific structure of the compound is shown in any one of the preceding embodiments.

According to an embodiment of the present disclosure, wherein, the organic layer is a hole injection layer or a hole transporting layer, and the hole injection layer or the hole transporting layer is formed by the compound alone.

According to an embodiment of the present disclosure, wherein, the organic layer is a hole injection layer or a hole transporting layer, and the hole injection layer or the hole transporting layer further comprises at least one hole transporting material; wherein a molar doping ratio of the compound to the at least one hole transporting material is 10000: 1 to 1: 10000.

According to an embodiment of the present disclosure, wherein, the organic layer is a hole injection layer or a hole transporting layer, and the hole injection layer or the hole transporting layer further comprises at least one hole transporting material; wherein a molar doping ratio of the compound to the at least one hole transporting material is 10: 1 to 1:100.

According to an embodiment of the present disclosure, wherein, the electroluminescent device comprises at least two light-emitting units and the organic layer is a charge generation layer and disposed between the at least two light-emitting units, wherein the charge generation layer comprises a p-type charge generation layer and an n-type charge generation layer.

According to an embodiment of the present disclosure, wherein, the p-type charge generation layer comprises the compound.

According to an embodiment of the present disclosure, wherein, the p-type charge generation layer further comprises at least one hole transporting material, wherein a molar doping ratio of the compound to the at least one hole transporting material is 10000: 1 to 1: 10000.

According to an embodiment of the present disclosure, wherein, the p-type charge generation layer further comprises at least one hole transporting material, wherein a molar doping ratio of the compound to the at least one hole transporting material is 10: 1 to 1:100.

According to an embodiment of the present disclosure, wherein, the hole transporting material is selected from a compound having a triarylamine unit, a spirobifluorene compound, a pentacene compound, an oligothiophene compound, an oligophenyl compound, an oligophenylenevinylene compound, an oligofluorene compound, a porphyrin complex or a metallic phthalocyanine complex.

According to an embodiment of the present disclosure, wherein, the charge generation layer further comprises a buffer layer disposed between the p-type charge generation layer and the n-type charge generation layer, and the buffer layer comprises the compound.

According to an embodiment of the present disclosure, further disclosed is a compound composition comprising the compound in any one of the preceding embodiments.

US20200062778A1 discloses a series of compounds each having a structure represented by general Formula 1' 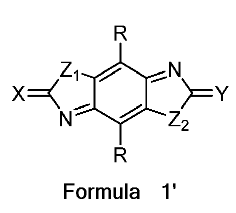 and specific compounds. The present disclosure discloses a compound having a structure represented by any one of Formula 2-1 to Formula 2-10 and specific compounds. These compounds are each a p-dopant with excellent performance. Such compounds may be applied to various different types of OLED device. For example, from the perspective of device structure, such compounds may be applied to single-stack devices, stacked devices (tandem devices), devices with P-I-N structures or inverted devices. Such compounds may be applied to transparent devices or opaque devices.

The compound having the structure of Formula 1' and the compound of the present disclosure having the structure of any one of Formula 2-1 to Formula 2-10 may each be used in a charge generation layer of a stacked device, especially a p-type charge generation layer. When the compound is used in the p-type charge generation layer of the stacked device, the p-type charge generation layer may be formed by the compound alone or may be formed by the compound doped in a conventional hole transporting material. In this case, the weight ratio of the compound to the hole transporting material is 1:99 to 99:1. Preferably, the weight ratio of the compound to the hole transporting material is 5:95 to 95:5. More preferably, the weight ratio of the compound to the hole transporting material is 10:90 to 90:10. An increase of a weight ratio of the compound having the structure of Formula 1' or the compound of the present disclosure having the structure of any one of Formula 2-1 to Formula 2-10 is conducive to improving the conductive performance (for example, conductivity) of the p-type charge generation layer formed after doping, which may improve the performance of the tandem device.

From the perspective of a light-emitting mechanism of a device, the compound having the structure of Formula 1' and the compound of the present disclosure having the structure of any one of Formula 2-1 to Formula 2-10 may each be applied to OLED devices with various different light-emitting mechanisms, such as a fluorescent device, a phosphorescent device, a TADF device, a hyper-fluorescence device, a thermally activated sensitized fluorescence (TASF) device and a triplet-triplet annihilation upconversion (TTA-UC) device. From the perspective of an emission wavelength of a device, the compounds may each be applied to a device emitting light in a UV waveband, a device emitting light in a visible light waveband and a device emitting light in a near-infrared waveband and may also be applied to a device emitting white light. Additionally, the OLED devices may be used in combination with color filters, quantum dots, polarizers, etc. The OLED devices may have polarized light emission characteristics.

From the perspective of a backplane technique, the compound having the structure of Formula 1' and the compound of the present disclosure having the structure of any one of Formula 2-1 to Formula 2-10 may each be applied to display devices or displays using different backplane techniques, such as a low temperature polysilicon (LTPS) thin-film transistor (TFT), a low temperature polycrystalline oxide (LTPO) TFT, an indium gallium zinc oxide (IGZO) TFT or an organic thin-film transistor (OTFT). The application scenarios of OLED devices include, but are not limited to, small-, medium- and large-sized displays, such as watches, mobile phones, tablets and televisions. The OLED devices may be applied to lighting systems, medical beauty facilities or phototherapy facilities and have been widely applied to taillights and internal display devices of automobiles in recent years. An OLED device may be a hard screen or a flexible screen. When made into the flexible screen, the OLED device is foldable or rollable and can provide users with more novel experience while having a wider design space.

Due to its strong oxidative property, the p-dopant, when doped, can significantly increase the concentration of majority carriers (such as holes) to improve device performance. Therefore, the p-dopant may be applied to transistors, capacitors and photovoltaic devices in addition to various different types of OLED device. The OLED device may include charge injection and transporting layers such as a hole transporting layer, an electron transporting layer and an electron injection layer. The OLED device may further include an emissive layer which generally includes at least one light-emitting dopant and at least one host compound. The light-emitting dopant may be a fluorescent light-emitting dopant and/or a phosphorescent light-emitting dopant. The OLED device may further include a blocking layer such as a hole blocking layer and an electron blocking layer.

The OLED device may include the hole transporting layer or the electron blocking layer. Hole transporting materials or electron blocking materials may use conventional hole transporting materials or electron blocking materials in the related art. These materials are described in detail in patent applications or patents such as US20060232198A1, US20100141119A1, US20100001636A1, US20090284140A1, US20090160323A1, US20090066225A1, US20080303417A1, US20080220285A1, US20080124572A1, US20070215867A1, US20120061714A1, US20100301744A1, CN104137288A, US20140231774A1, US20150115239A1, US20150333276A1, US20160118591A1, US20160301005A1, US20170162798A1, US9972786B2, US20180123043A1, US20180190904A1, US10069076B2, US20190288208A1, US10497880B2, US10629830B2, US20200235305A1, US20200321526A1, US20200313090A1, US11094888B2, US20210305515A1, US20210376247A1 and CN114085182A. The hole transporting layer or the electron blocking layer may typically include the following materials without limitation: 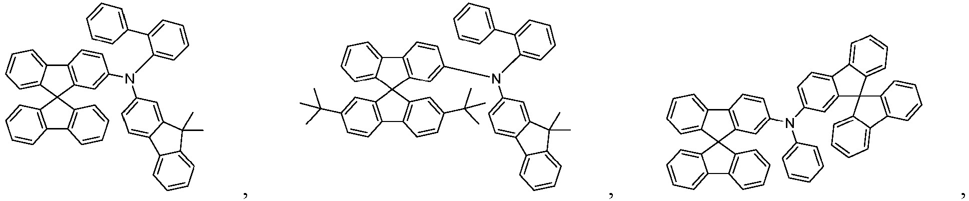 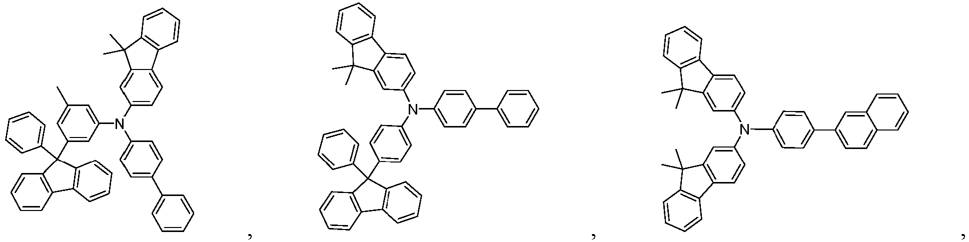 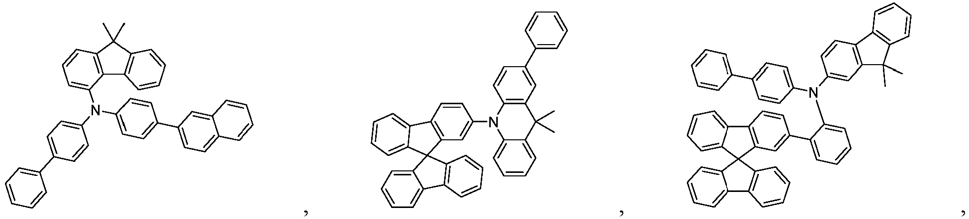 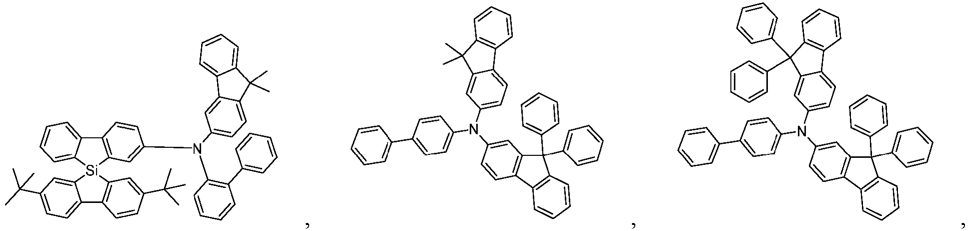 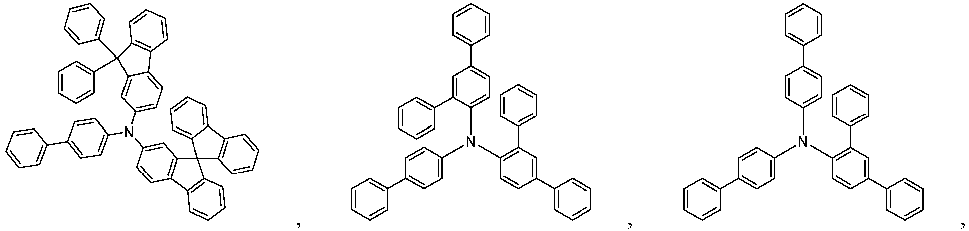 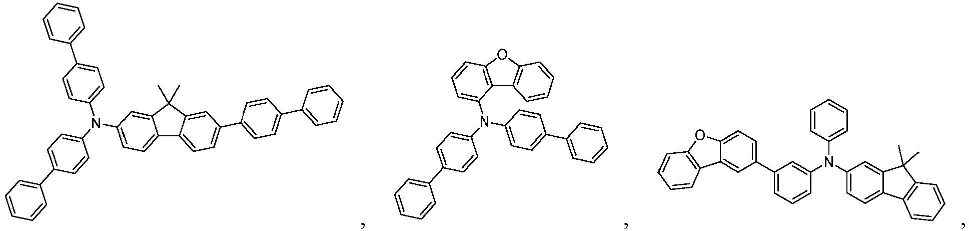 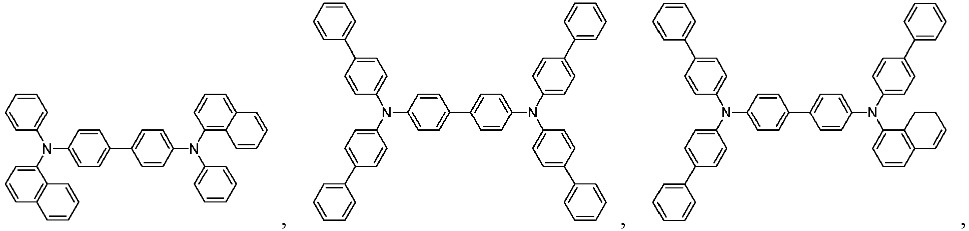 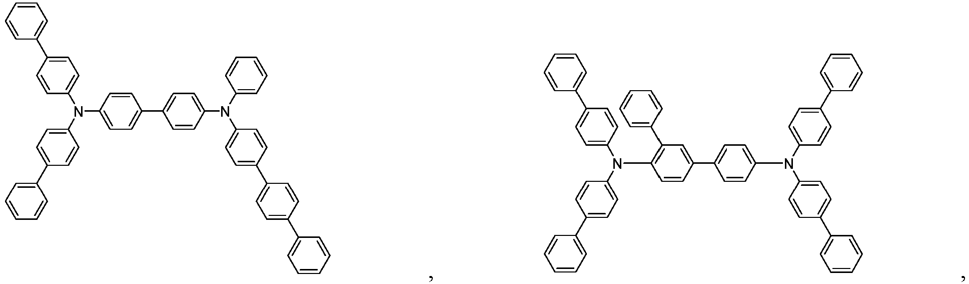 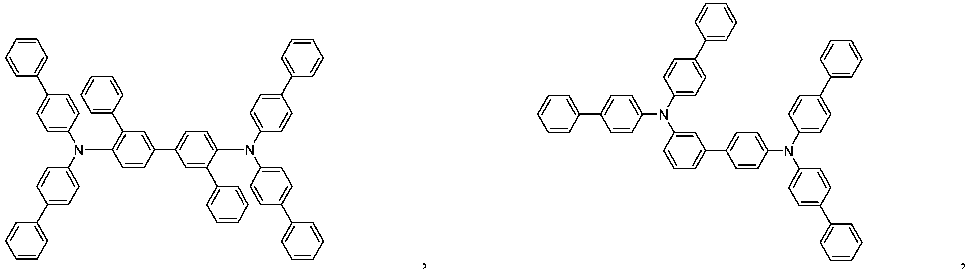 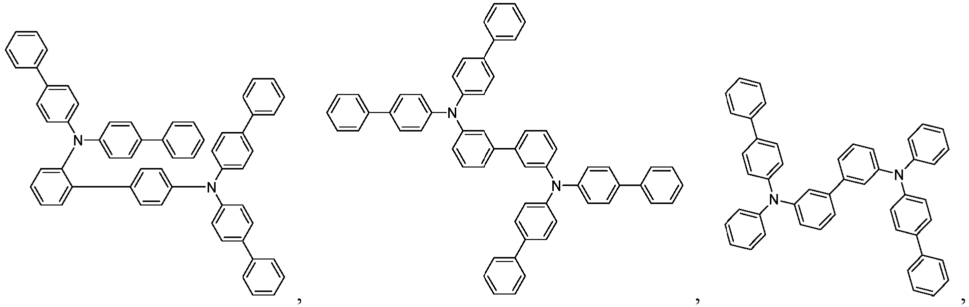 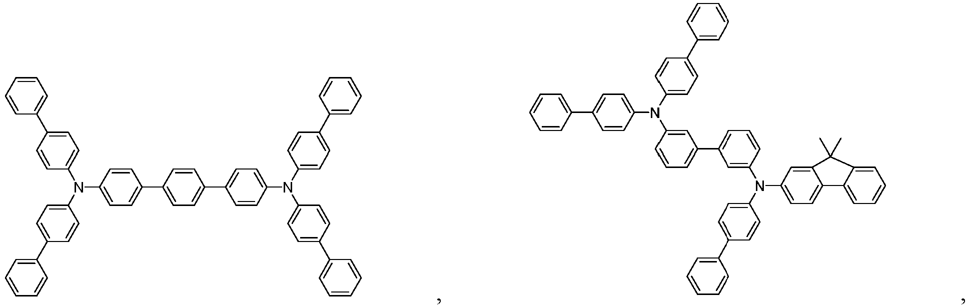 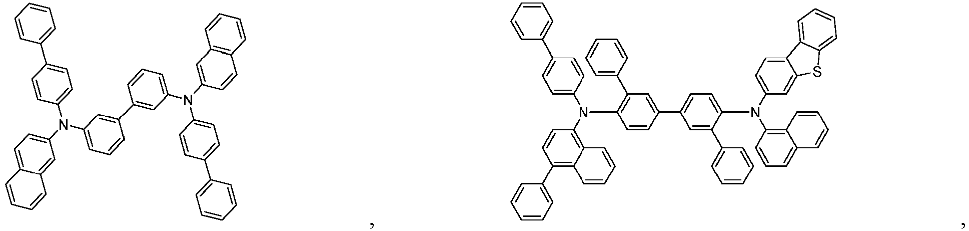 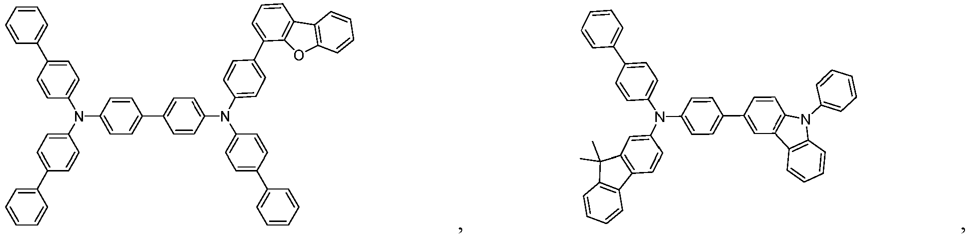 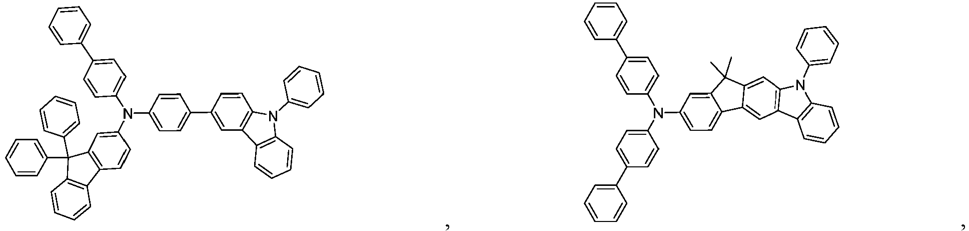 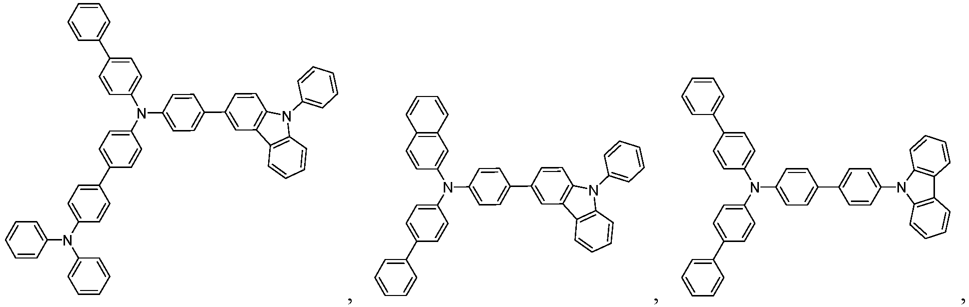 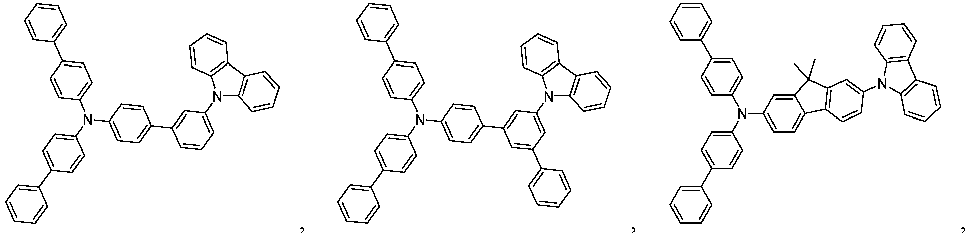 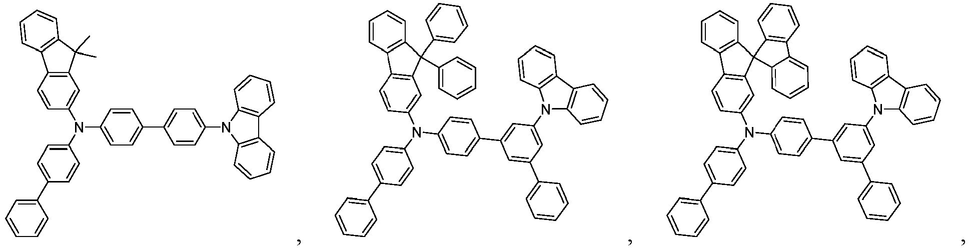 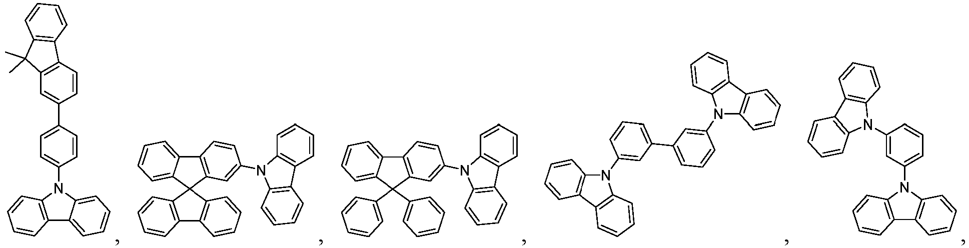 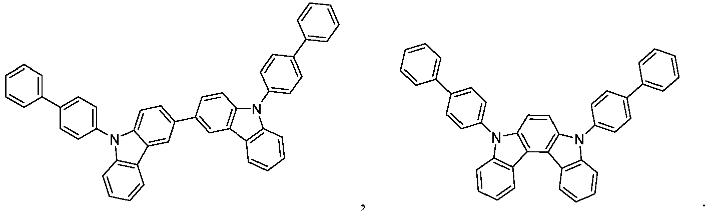 The preceding hole transporting materials disclosed in the related art may also be used as matrix materials in the hole injection layer, the p-type charge generation layer or another p-type doping layer. For example, when the compound having the structure of Formula 1' 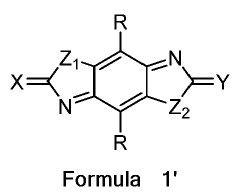 and the compound of the present disclosure having the structure of any one of Formula 2-1 to Formula 2-10 are doped in the preceding hole transporting materials disclosed in the related art as the hole injection layer, the p-type charge generation layer or another p-type doping layer, they can effectively regulate the conductivity or hole injection ability of the hole injection layer, the p-type charge generation layer or another p-type doping layer so that the device can have better performance.

The OLED device may include one or more of the hole blocking layer, the electron transporting layer or the electron injection layer. The hole blocking layer, the electron transporting layer or the electron injection layer includes a material selected from a metal or a metal compound. The "metal compound" includes an organic metal complex, an organic metal salt and an inorganic metal salt, a metal oxide and a metal halide. These materials are described in detail in patent applications or patents, US20100289009A1, US20150214483A1, CN102643296A, CN102675352A, KR1020140121991A, KR1020140140298A, WO2016172414A1, US9991451B2, US20180175307A1, CN108368058A, KR102204963B1, CN110551043A, CN110551015A, KR102051910B1 and KR1020190122547A. The hole blocking layer, the electron transporting layer or the electron injection layer may typically include the following materials without limitation: LiF, Liq, CsF, CsCO₃, 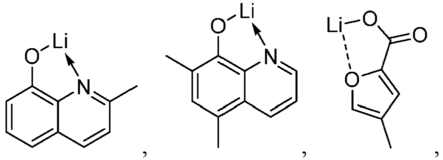 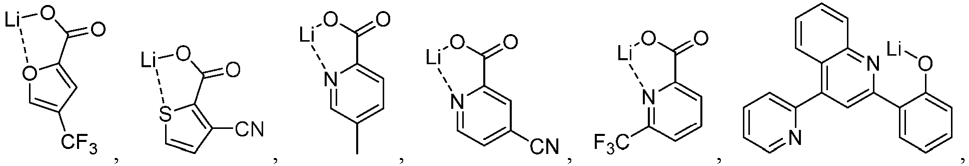 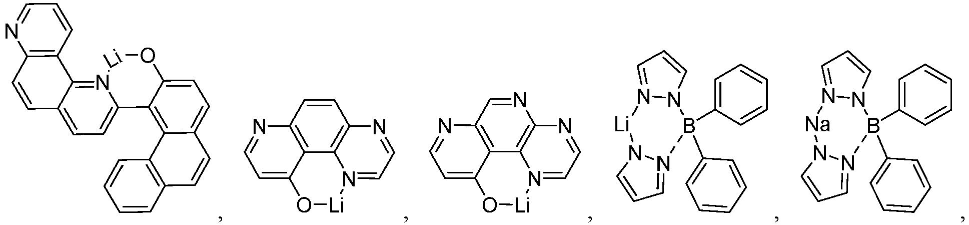 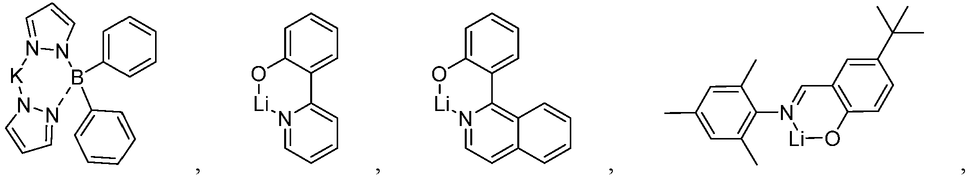 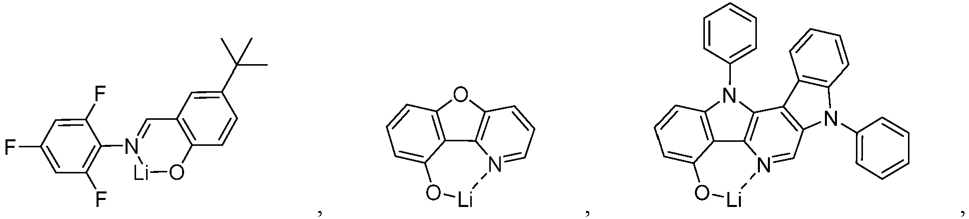 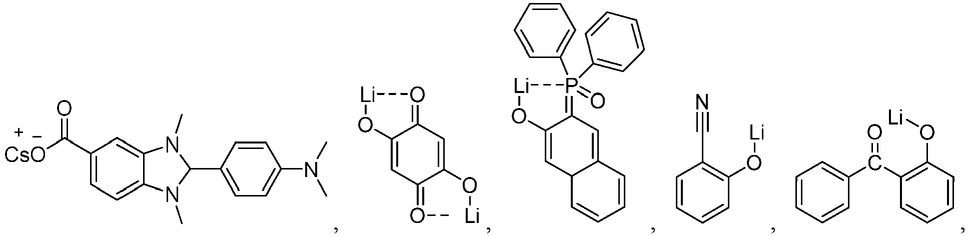 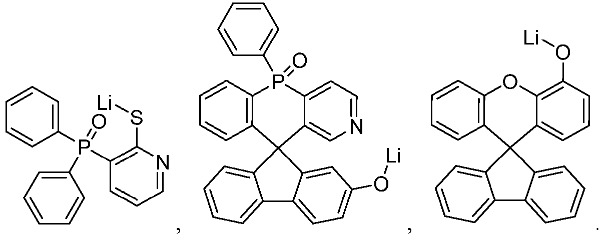 In the hole blocking layer, electron transporting layer or electron injection layer including a metal, Yb, Li, Na, K, Rb, Cs, Be, Mg, Al, Ca, Sr, Ba, La, Ce, Pr, Nd, Sm, Eu, Tb, Dy, Ho, Er, Em, Gd, Lu, Y, Mn, Ag or a compound thereof is especially useful.

The hole blocking material and/or the electron transporting layer and/or the electron injection layer may also include an n-type organic dopant and an electron transporting material. The n-type organic dopant is selected from an organic molecule, a neutral radical compound or a mixture of an organic molecule and a neutral radical compound, and the HOMO energy level of the n-type organic dopant is greater than -3.5 eV, preferably greater than -3.0 eV, and more preferably greater than -2.6 eV The electron transporting material includes any one or more chemical structural units selected from the group consisting of: anthracene, phenanthrene, phenanthroline, dibenzothiophene, dibenzofuran, dibenzoselenophene, furan, thiophene, benzofuran, benzothiophene, benzoselenophene, carbazole, indolocarbazole, pyridoindole, pyrrolopyridine, pyrazole, imidazole, triazole, oxazole, thiazole, oxadiazole, oxatriazole, dioxazole, thiadiazole, pyridine, pyridazine, pyrimidine, pyrazine, triazine, oxazine, oxathiazine, oxadiazine, indole, benzimidazole, indazole, indoxazine, benzoxazole, benzisoxazole, benzothiazole, quinoline, isoquinoline, cinnoline, quinazoline, quinoxaline, naphthyridine, phthalazine, pteridine, xanthene, acridine, phenazine, phenothiazine, benzofuropyridine, furodipyridine, benzothienopyridine, thienodipyridine, benzoselenophenopyridine and selenophenodipyridine. The n-type organic dopant compound is selected from a hybrid radical compound, a diradical compound, a dimer, an oligomer, a polymer, a bis-spiro compound or a polycyclic compound. These materials are described in detail in US patent applications, US20070252140A1, US20110108772A1, US20100233844A1 and US20100187515A1. The n-type organic dopant is selected from the group consisting of the following structures: 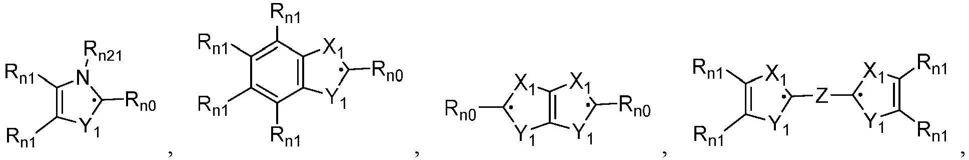 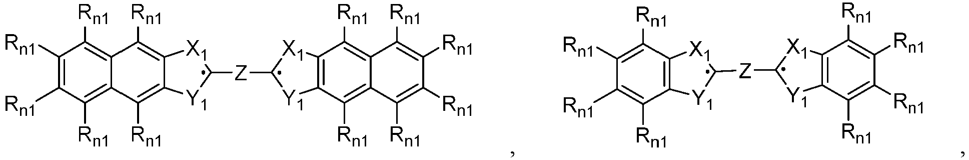 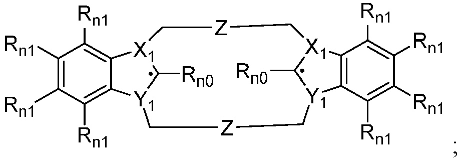
wherein the bridge Z may be independently selected from alkylene, alkenylene, alkynylene, cycloalkylene, silylene, diazoline, disulfanylene, heterocyclylene, dialkylene ether, polyether or imino;
X₁ and Y₁ may be O, S, N, NRₙ₂₁, P or PRₙ₂₁;
Rₙ₀, Rₙ₁ and Rₙ₂₁ are, at each occurrence identically or differently, selected from the group consisting of: hydrogen, deuterium, halogen, substituted or unsubstituted alkyl having 1 to 20 carbon atoms, substituted or unsubstituted cycloalkyl having 3 to 20 ring carbon atoms, substituted or unsubstituted heteroalkyl having 1 to 20 carbon atoms, substituted or unsubstituted arylalkyl having 7 to 30 carbon atoms, substituted or unsubstituted alkoxy having 1 to 20 carbon atoms, substituted or unsubstituted aryloxy having 6 to 30 carbon atoms, substituted or unsubstituted alkenyl having 2 to 20 carbon atoms, substituted or unsubstituted aryl having 6 to 30 carbon atoms, substituted or unsubstituted heteroaryl having 3 to 30 carbon atoms, substituted or unsubstituted alkylsilyl having 3 to 20 carbon atoms, substituted or unsubstituted arylsilyl having 6 to 20 carbon atoms, substituted or unsubstituted alkylgermanyl having 3 to 20 carbon atoms, substituted or unsubstituted arylgermanyl having 6 to 20 carbon atoms, substituted or unsubstituted amino having 0 to 20 carbon atoms, an acyl group, a carbonyl group, a carboxylic acid group, an ester group, a cyano group, an isocyano group, a hydroxyl group, a sulfanyl group, a sulfinyl group, a sulfonyl group, a phosphino group and combinations thereof; and
adjacent substituents Rₙ₀, Rₙ₁, Rₙ₂₁ can be optionally joined to form a ring.

The n-type organic dopant may be produced by a precursor during a layer formation (deposition) process or in a post process of layer formation. Examples of suitable precursors of the n-type organic dopant include:

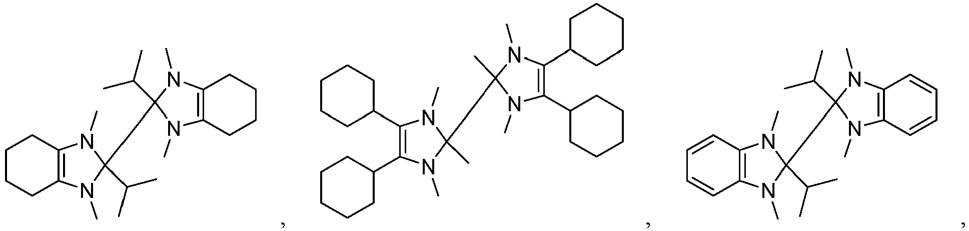

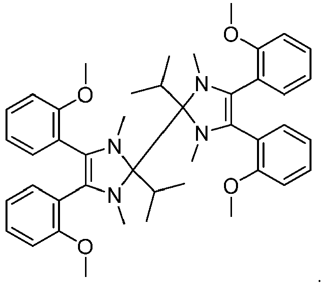

The electron transporting materials are described in detail in patent applications, JP2022110006A, US20220209137A1, US11362280B2, CN114591314A, US20220149291A1, US11314007B2, US20220077399A1, US20220064153A1, CN114085182A, US20220052273A1, US20220052268A1, US11251376B2, JP2022000427A, US20210384441A1, US20210371427A1, US20210367158A1, US20210359226A1, CN113631550A, US20210336155A1, US20210320263A1, US11145826B2, US20210313520A1, US20210313518A1, US20210305516A1, US11133473B2, US20210288264A1, US20210288260A1, US20210280794A1, US20210277305A1, CN107311987B, US20210253542A1, US11088331B2, US11069862B2 and US20210119133A1. The electron transporting materials may be typically selected from the following structures without limitation:

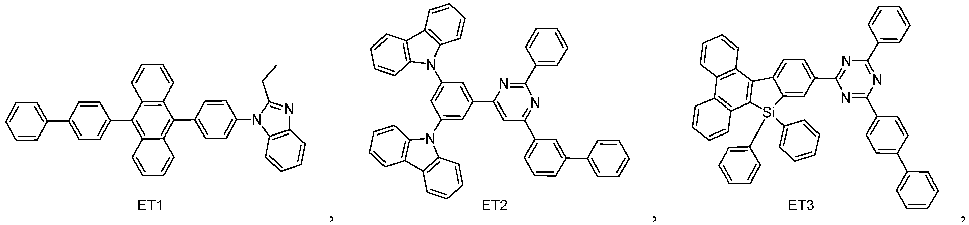

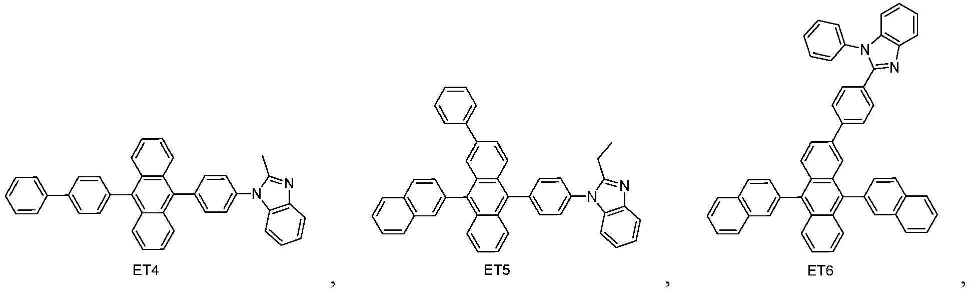

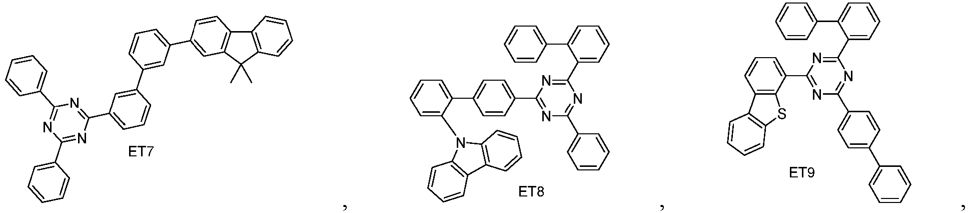

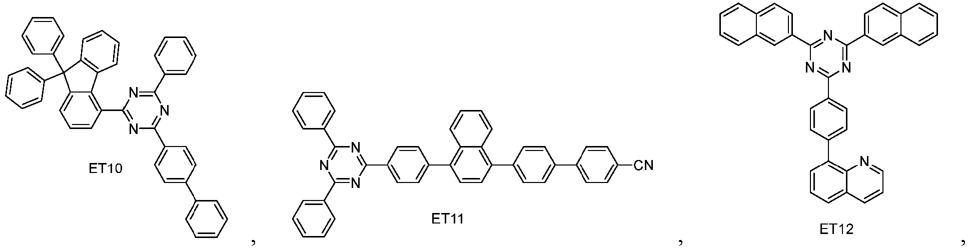

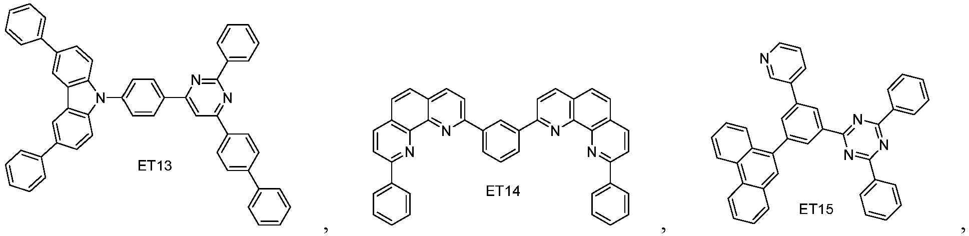

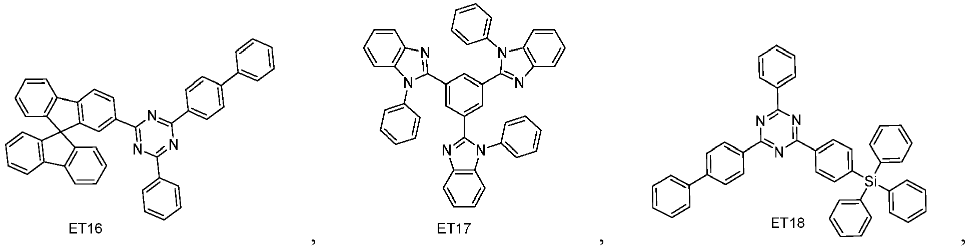

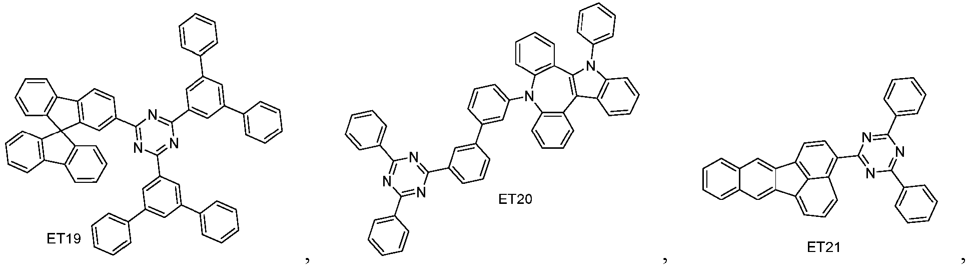

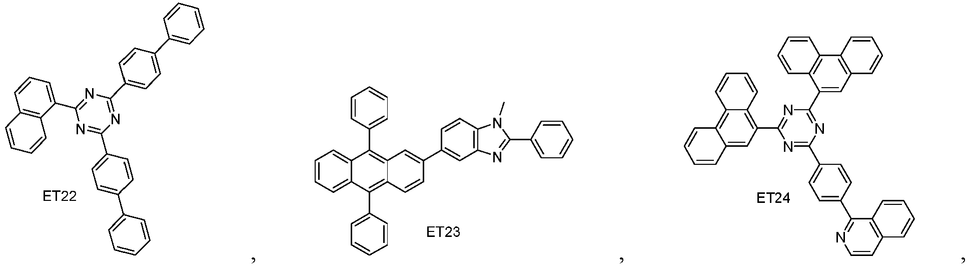

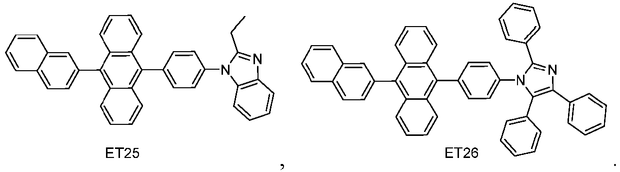

The OLED device may include a capping layer (CPL) to improve light extraction efficiency. CPL materials are described in detail in patent applications, US20220209127A1, TW202222769A, WO2022118910A1, WO2022100099A1, US20220119360A1, US20220056050A1, WO2022004555A1, US20210226132A1, US20200172558A1, KR102077776B1, CN105633118A and US20200395549A1. The CPL materials may be typically selected from the following materials without limitation:

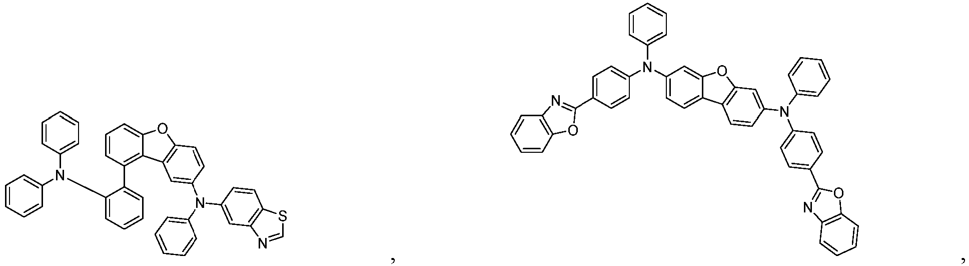

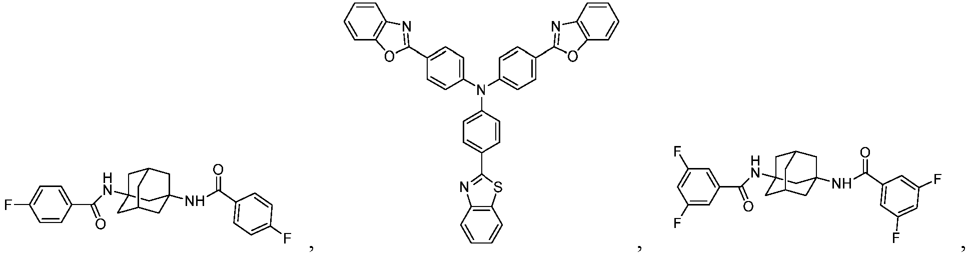

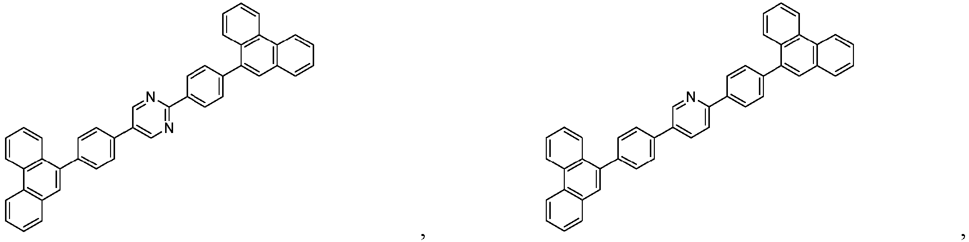

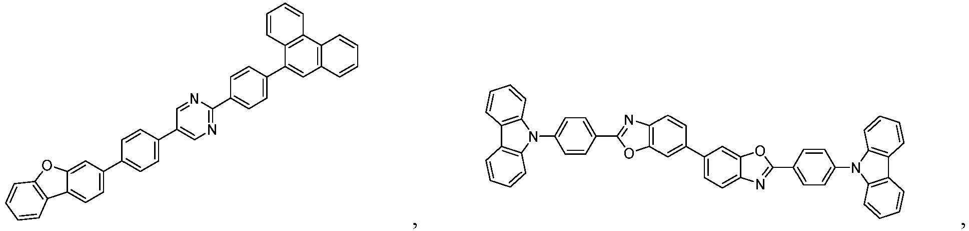

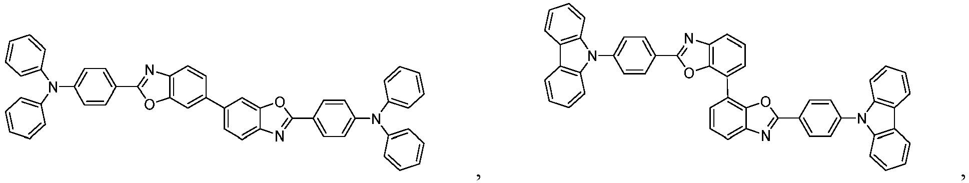

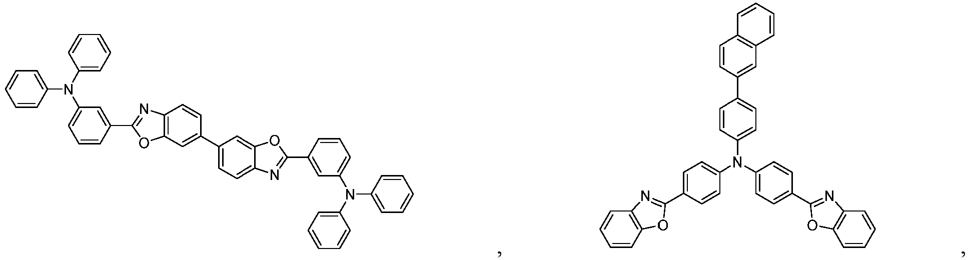

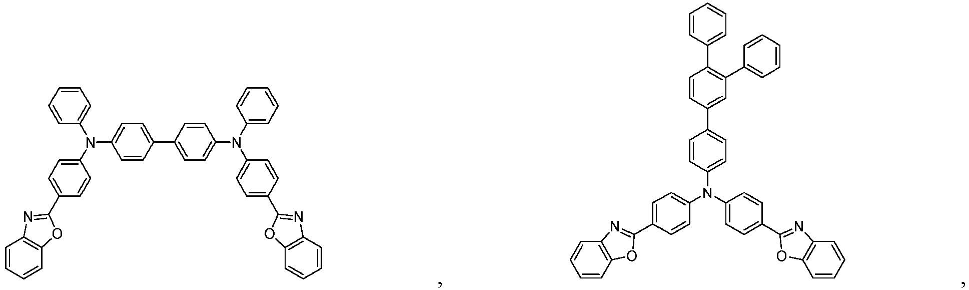

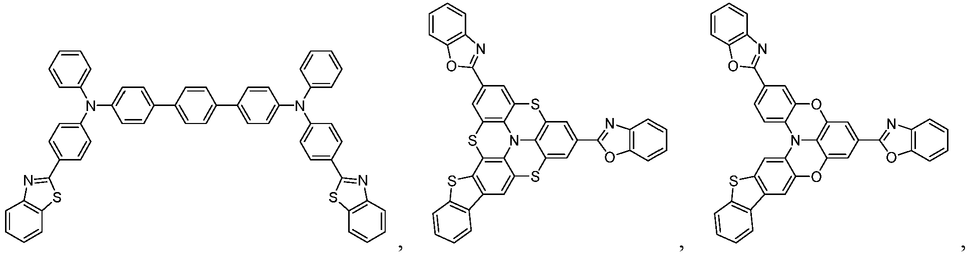

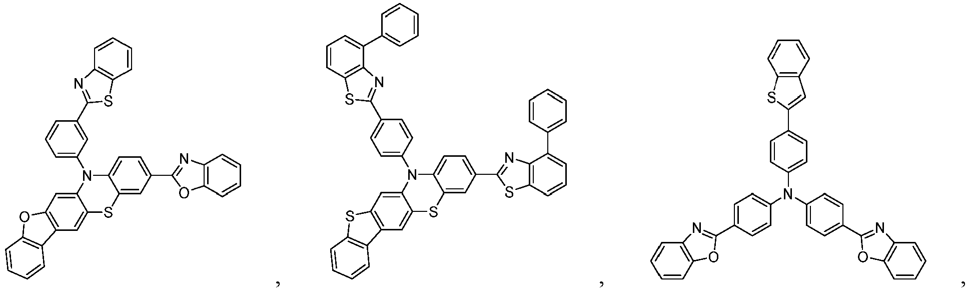

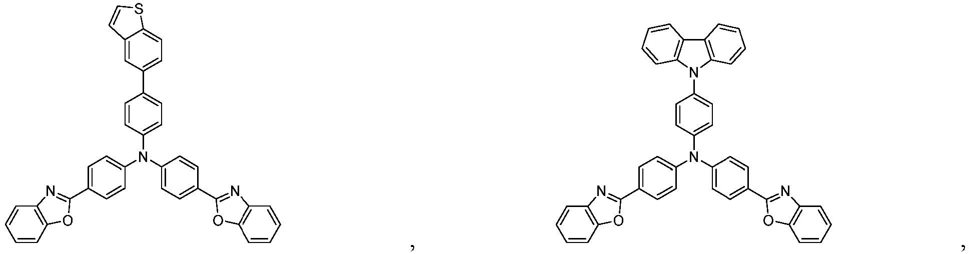

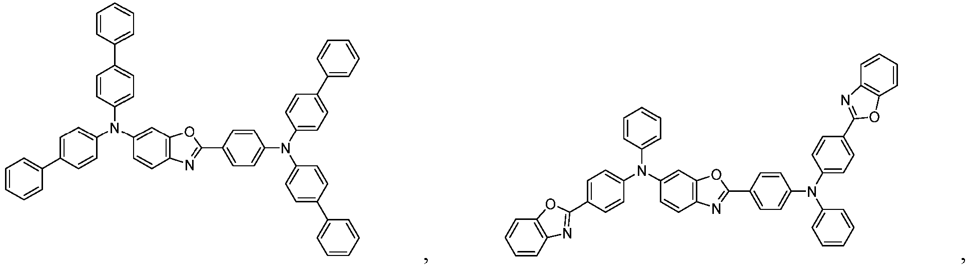

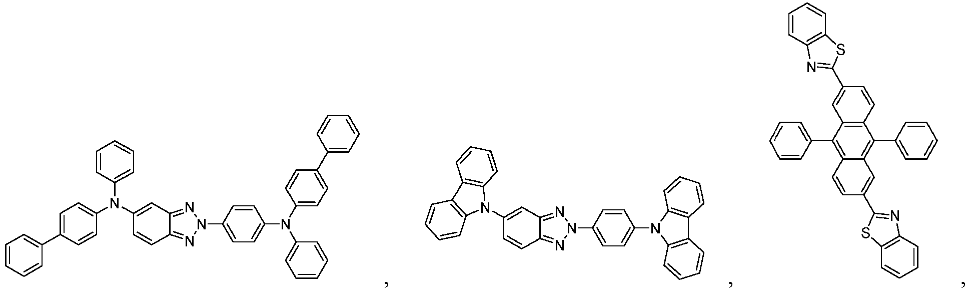

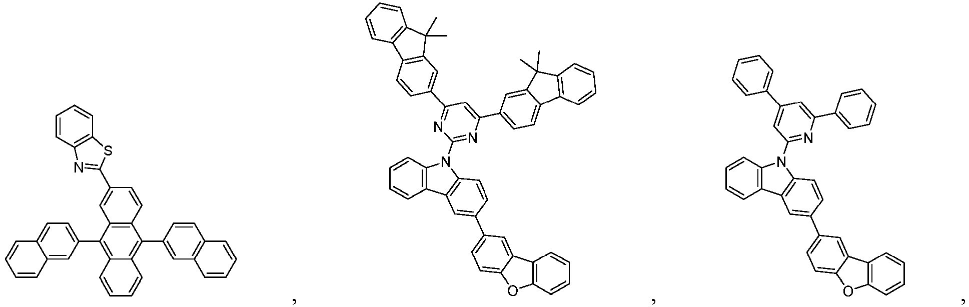

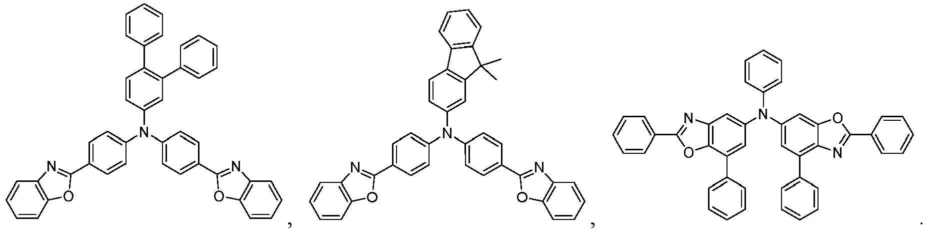

Conventional light-emitting materials and host materials in the related art may be used in the emissive layer. The emissive layer may be a fluorescent emissive layer or a phosphorescent emissive layer according to different light-emitting mechanism of light-emitting dopants included in the emissive layer. Fluorescent light-emitting materials may be used in the fluorescent emissive layer, such as those described in CN101910147A, WO2018235953A1, CN102232068A, WO2019088194A1, WO2018194035A1, WO2016017919, US2012181520, US20110006289, CN105431439A, CN106905367A, CN110692146A, CN111253421A, CN111933810A, CN102232068B, CN104795495B, US8431250B2, US9166179B2, US9466800B2, US9741938B2, WO2021200252A1 and WO2021131766A1. The fluorescent light-emitting materials may be typically selected from the following materials without limitation:

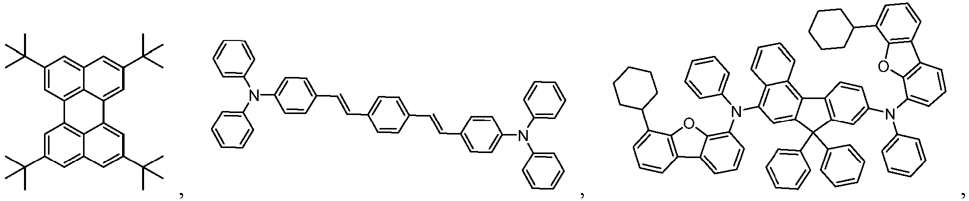

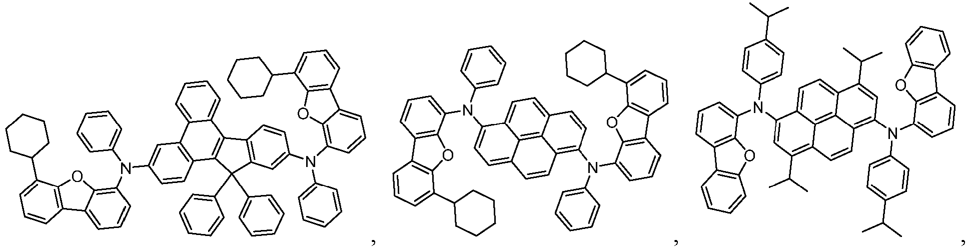

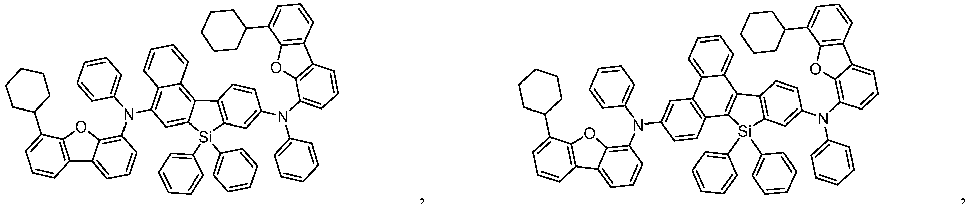

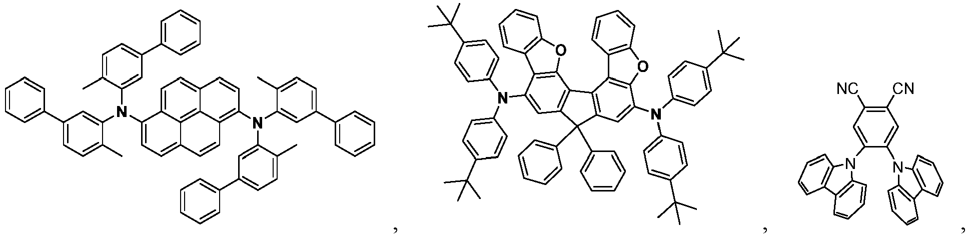

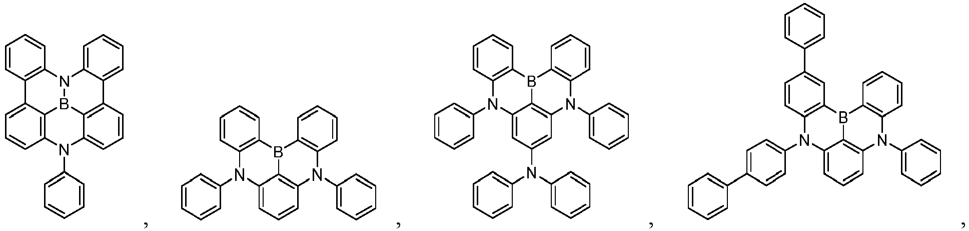

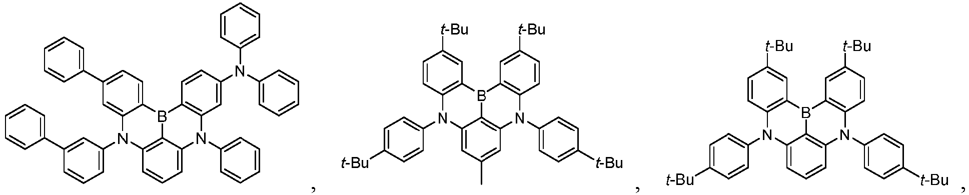

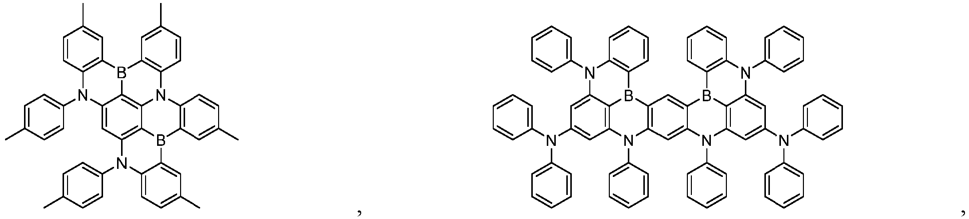

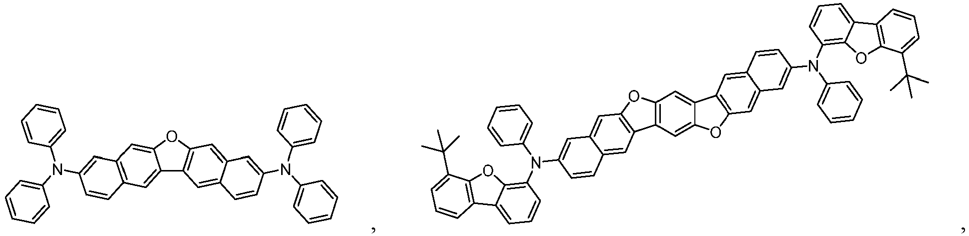

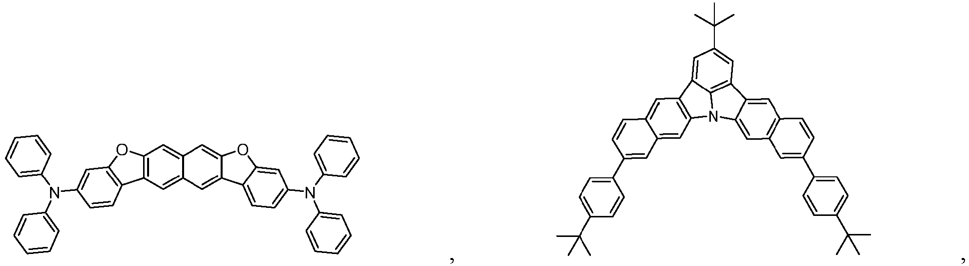

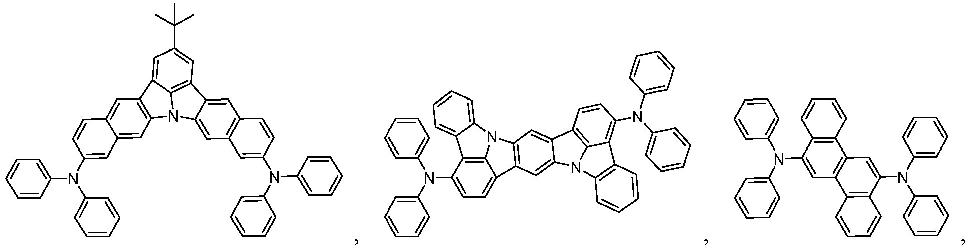

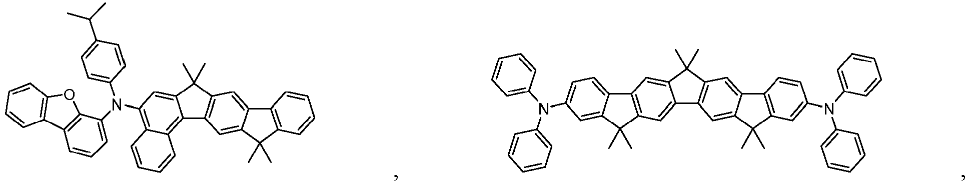

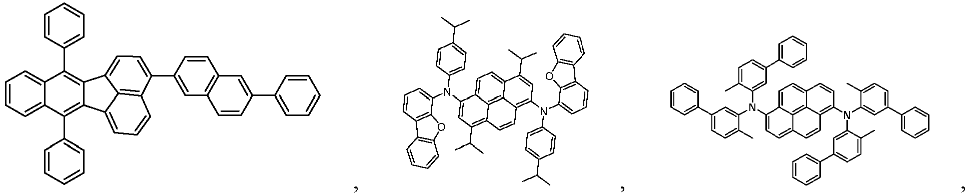

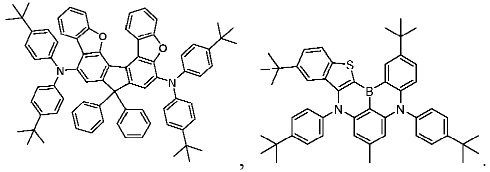

The fluorescent emissive layer may also include fluorescent host materials, such as those described in KR1020170044001A, CN109790462A, CN109804043A, WO2008062773A1, WO2009102054A1, WO2009102026A1, WO2009107596A1, US20200190103A1, US20200343457A1 and US20210210700A1. The fluorescent host materials may be typically selected from the following materials without limitation:

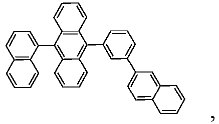

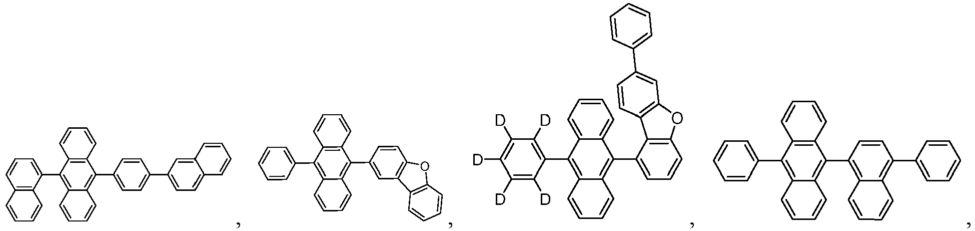

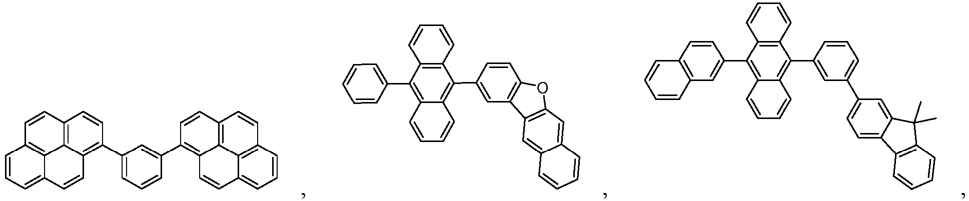

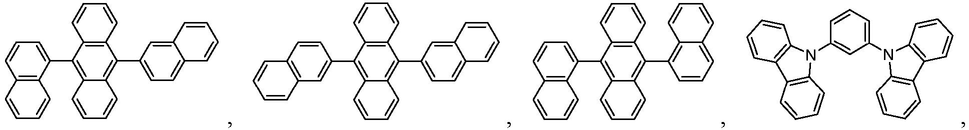

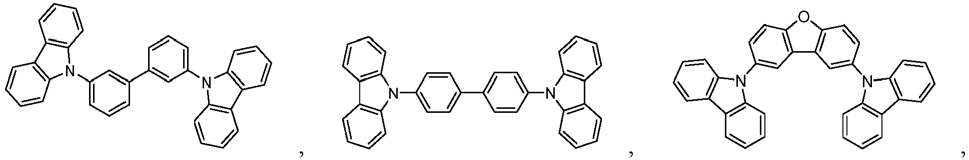

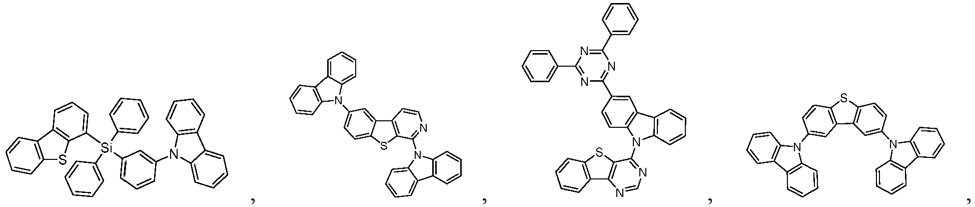

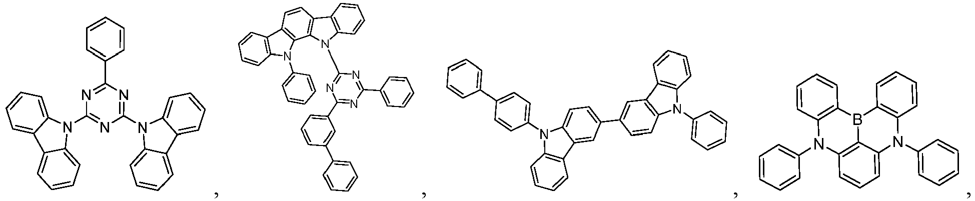

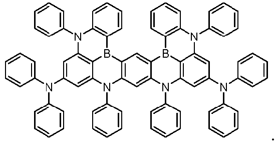

The phosphorescent emissive layer generally includes at least one phosphorescent host material and at least one phosphorescent light-emitting material.

The emissive layer may include conventional red phosphorescent materials in the related art, such as those red light materials disclosed in US2018097179A1, US2021002311A1, US2020308205A1, US2019165283A1, US2020308201A1, US20200227659A1, US20220109118A1, US20210380618A1, US20210242411A1 and US20200358011A1. Additionally, the emissive layer may typically include the following red phosphorescent light-emitting materials without limitation:

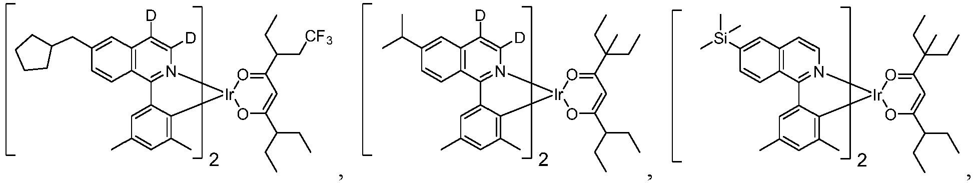

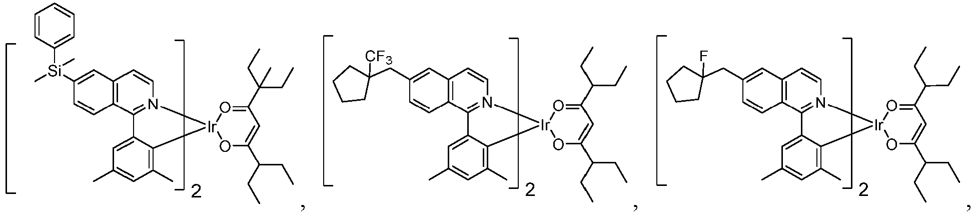

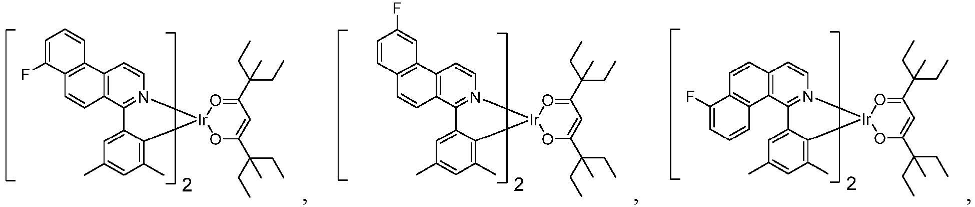

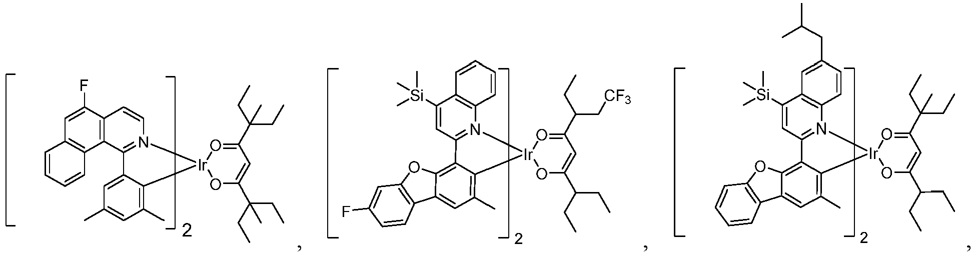

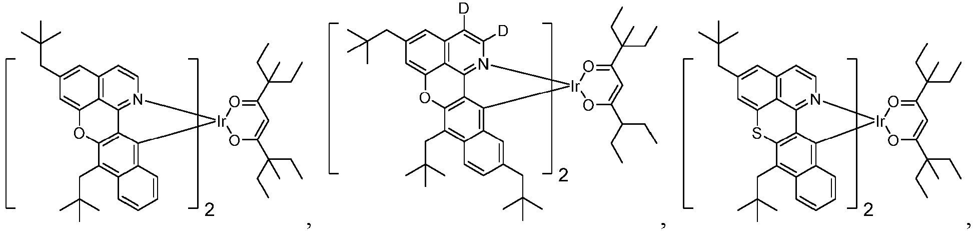

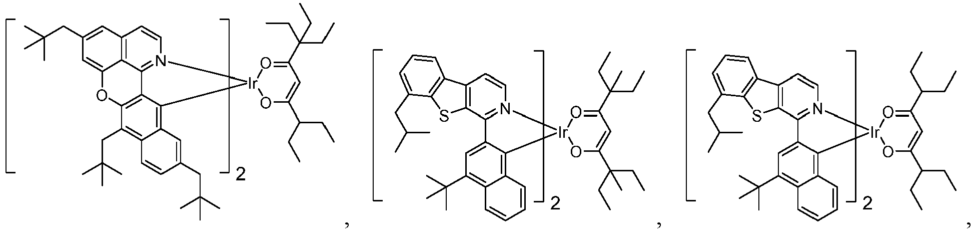

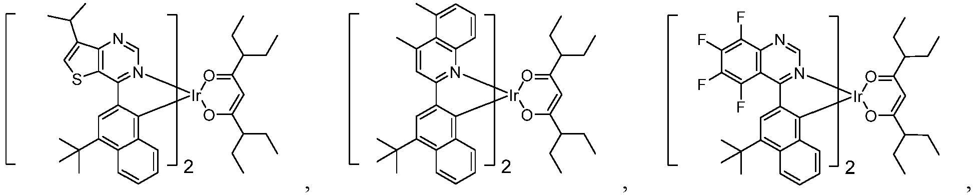

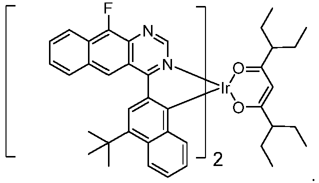

The emissive layer may include conventional blue phosphorescent materials in the related art, such as those blue phosphorescent materials disclosed in WO2002002714, CN101180304A, US2011057559A1, US2014110691, CN109111487A, US20200140471A1 and EP3750900A1. Additionally, the emissive layer may typically include the following blue phosphorescent light-emitting materials without limitation:

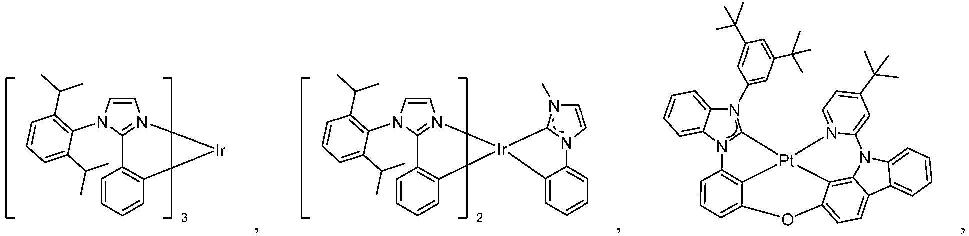

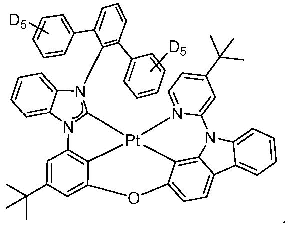

The emissive layer may include conventional green phosphorescent light-emitting materials or yellow phosphorescent light-emitting materials in the related art, such as those green or yellow phosphorescent materials disclosed in US20130119354A1, US20200091442A1, US2020251666A1, US20220140259A1, US20140131676A1, US20210024557A1, US20220153769A1, US20190036045A1, US20170194577A1 and US20170294597A1. Additionally, the emissive layer may typically include the following green phosphorescent light-emitting materials or yellow phosphorescent light-emitting materials without limitation:

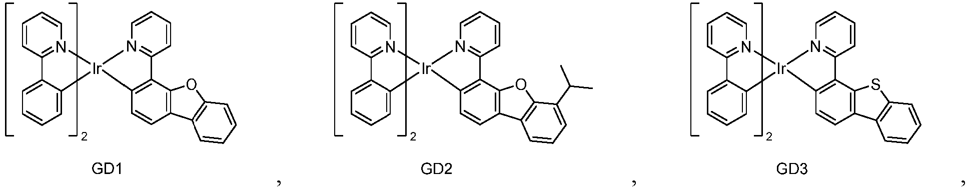

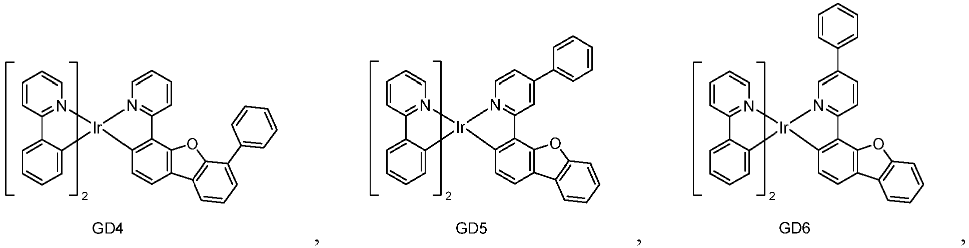

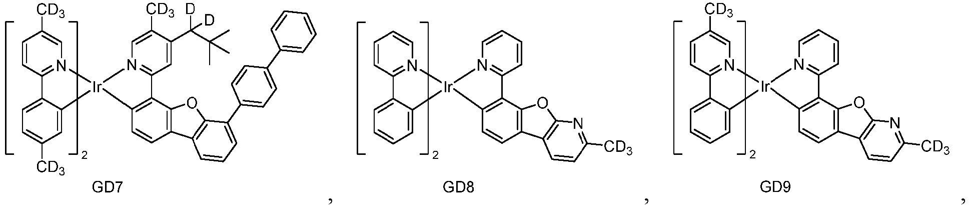

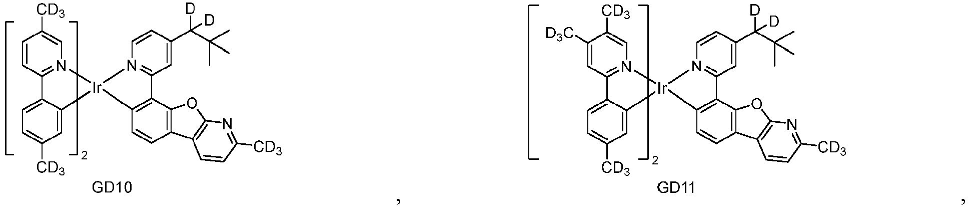

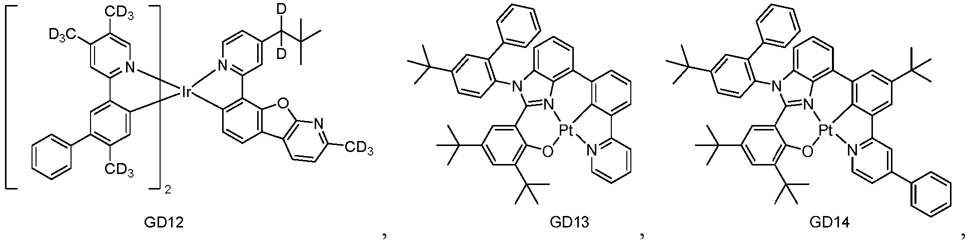

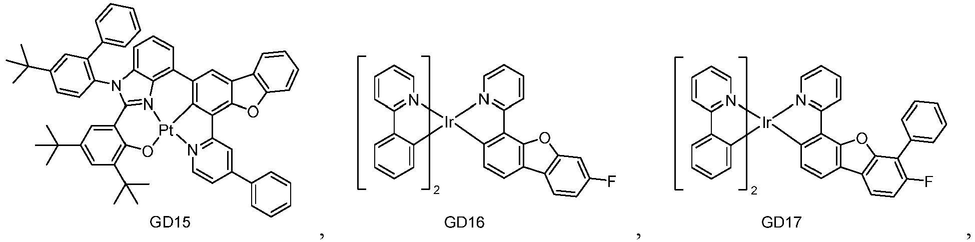

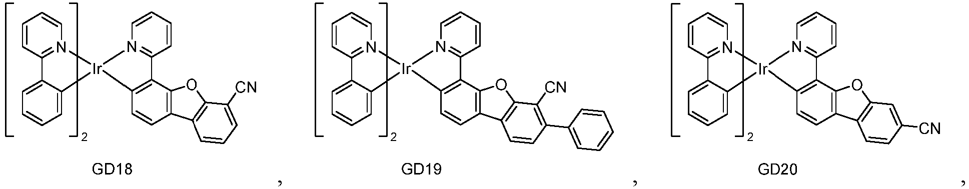

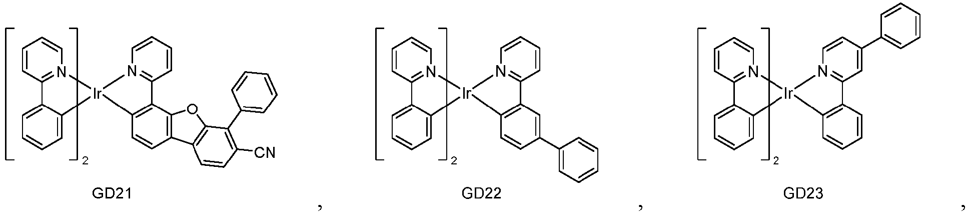

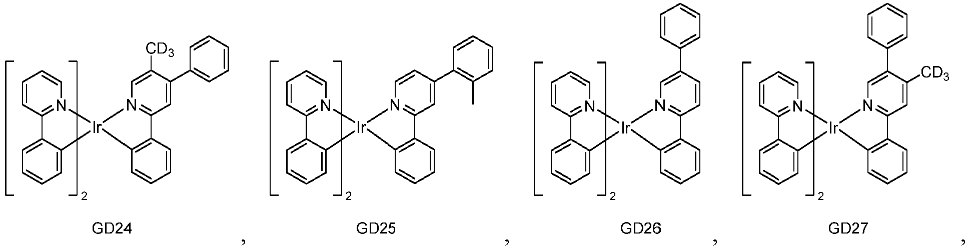

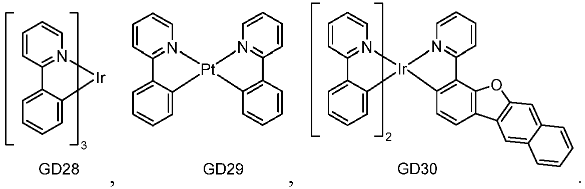

The emissive layer may include conventional red light host materials in the related art, such as those red light single-host materials disclosed in CN114249738A, US20220186114A1, WO2012121561A1, US20220162210A1, CN113614203A and US20200013964A1, those red light multi-host materials disclosed in CN114613924A, US20220165956A1, WO2020022769A1 and WO2019066315A2, and those red light multi-host materials suitable for premix evaporation which are disclosed in US20210359216A1, US20210328154A1, JP2021125684A, CN114479835A and CN113277988A. Additionally, the emissive layer may typically include the following host materials without limitation:

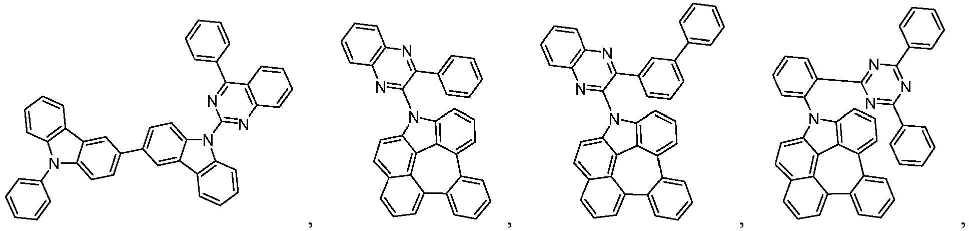

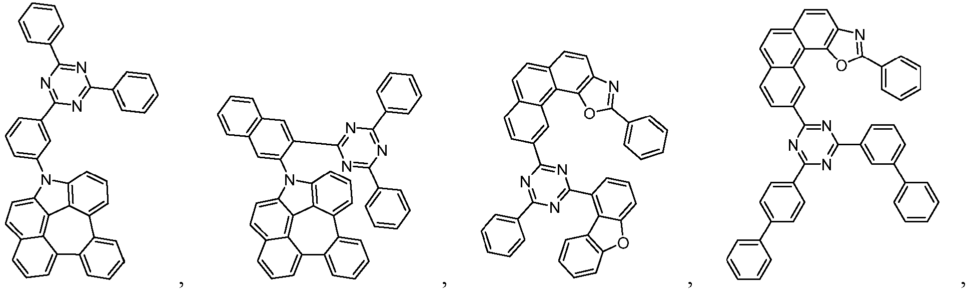

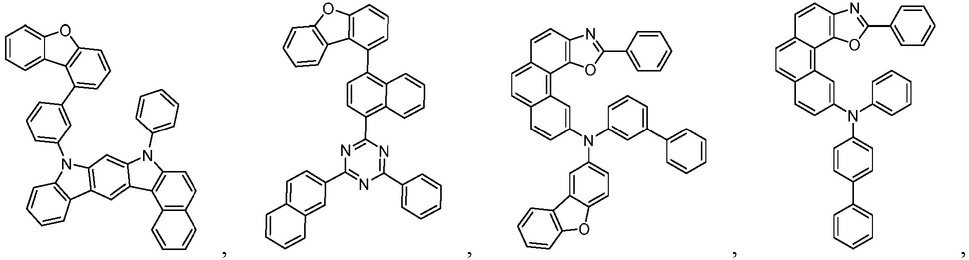

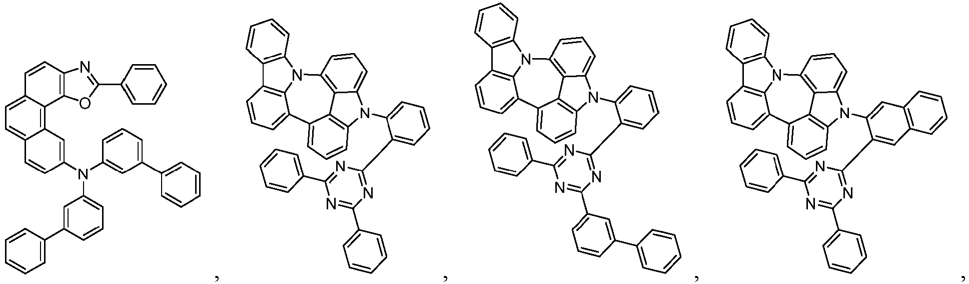

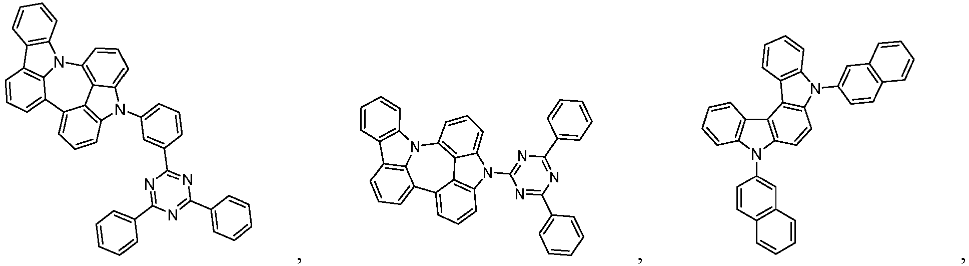

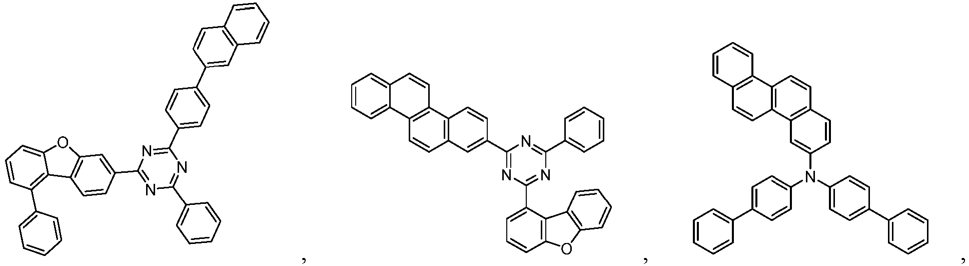

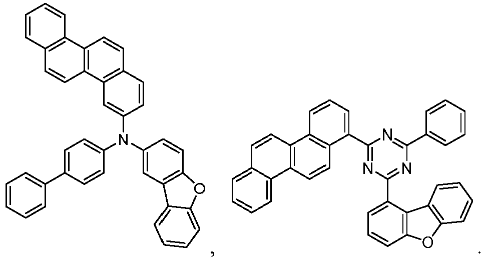

The emissive layer may include conventional green light host materials in the related art, such as those green light host materials disclosed in CN101511834A, CN105143398A, CN106459018A, US2015171340A1, CN110540536A, US20220213116A1, CN114621199A, WO2018016742A1, WO2019231210A1, WO2019132374A1 and CN110437213A. The green light host materials may also be materials suitable for premix evaporation. Additionally, the emissive layer may typically include the following green light host materials without limitation:

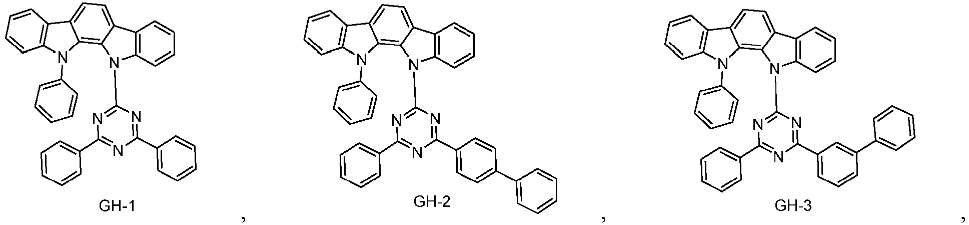

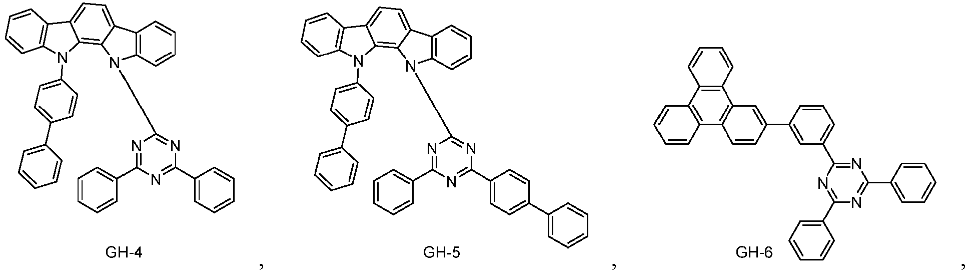

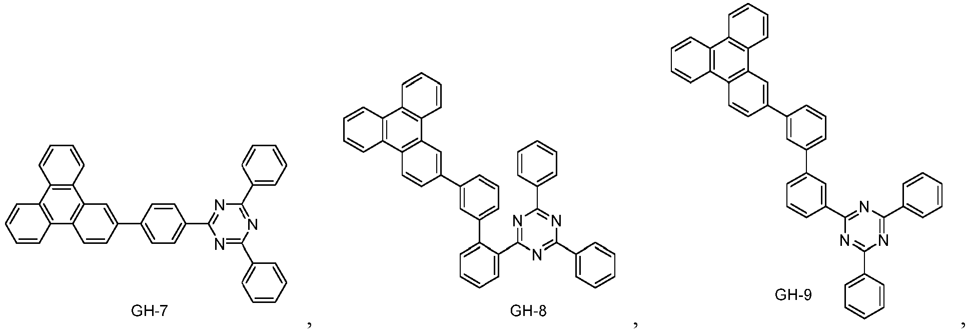

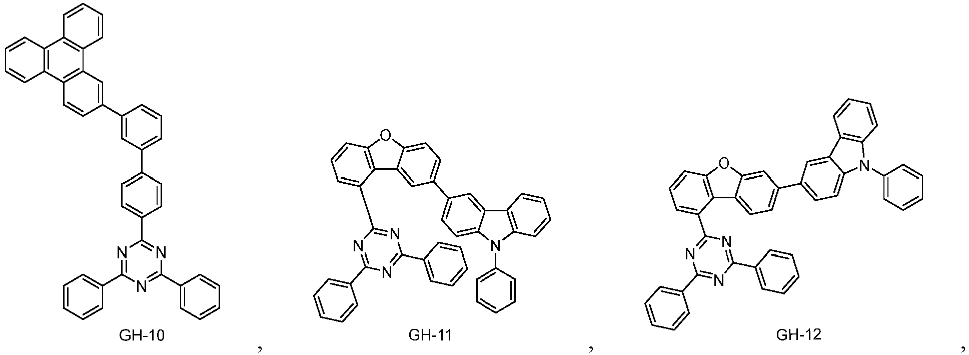

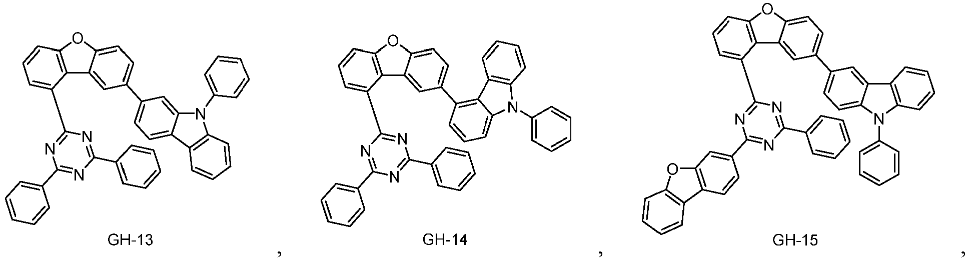

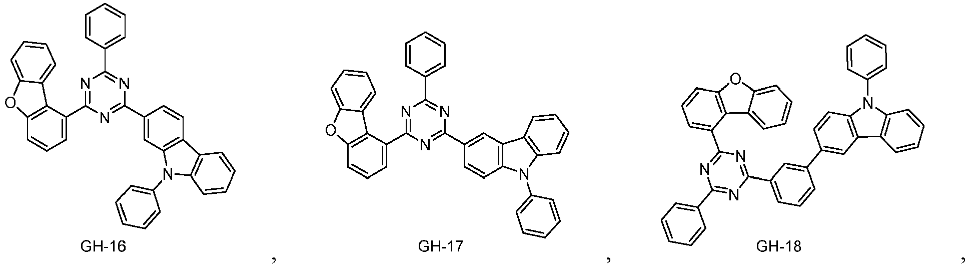

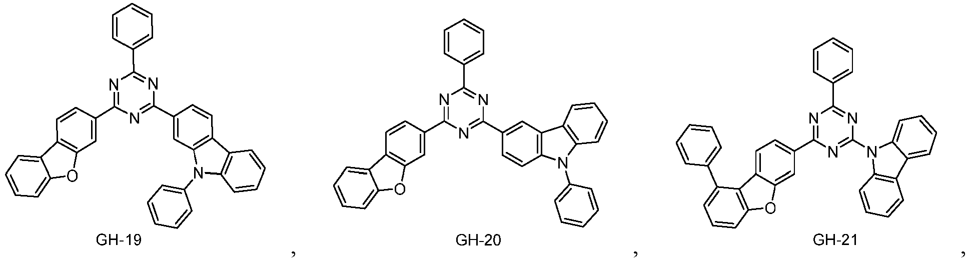

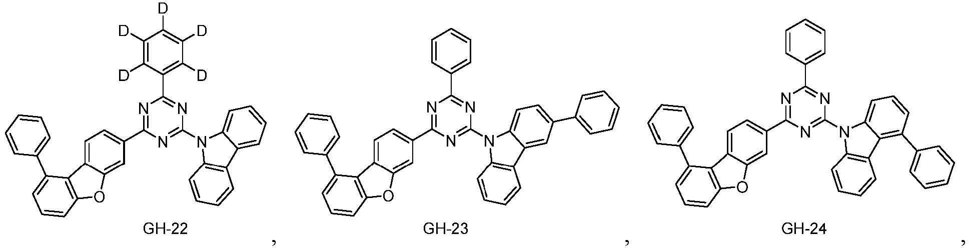

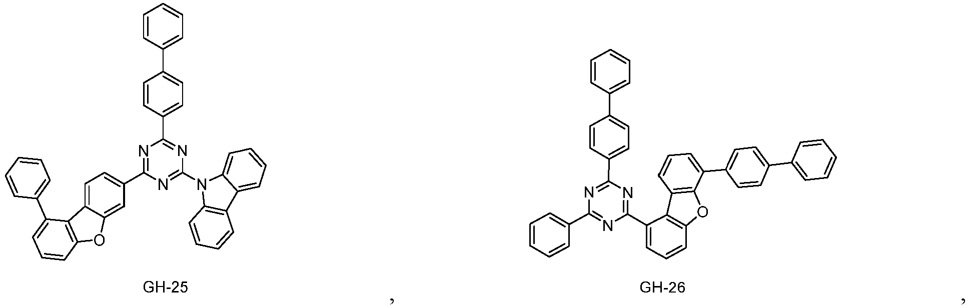

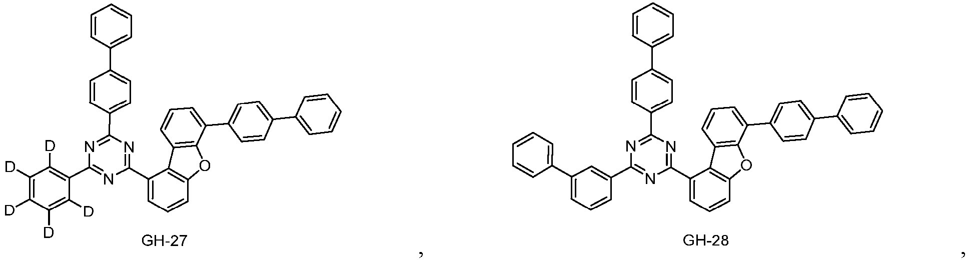

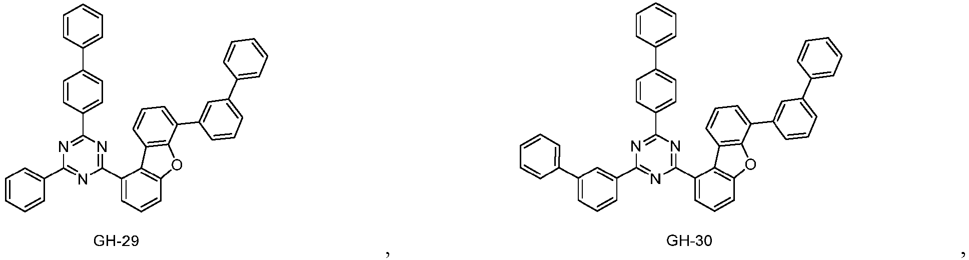

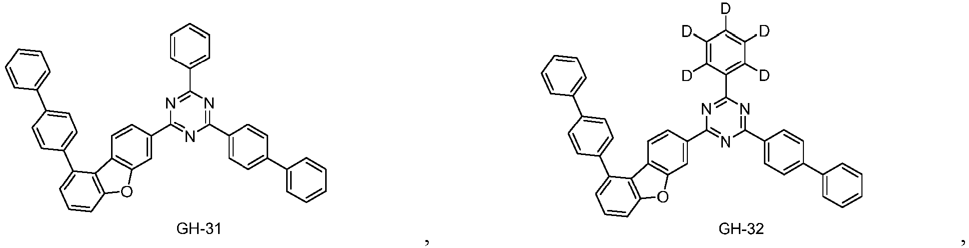

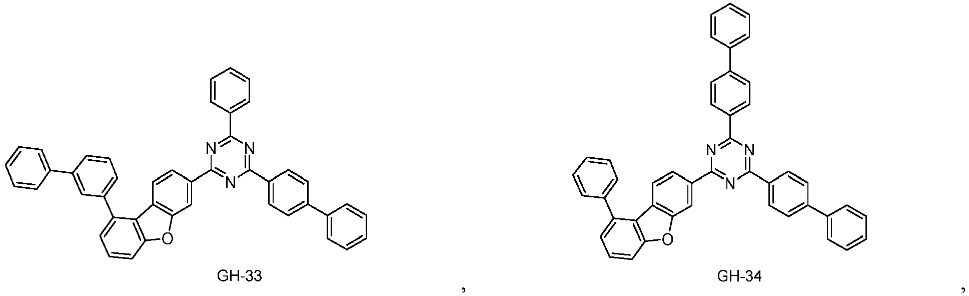

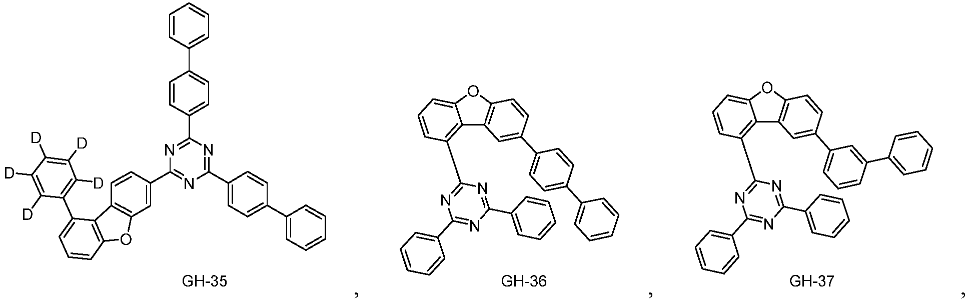

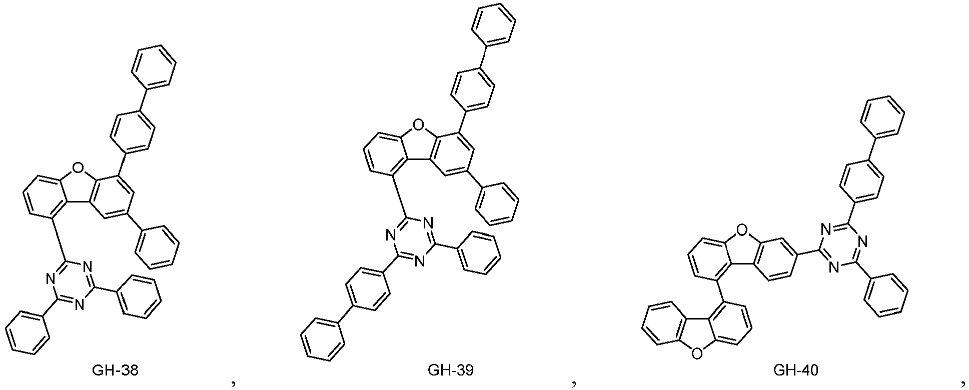

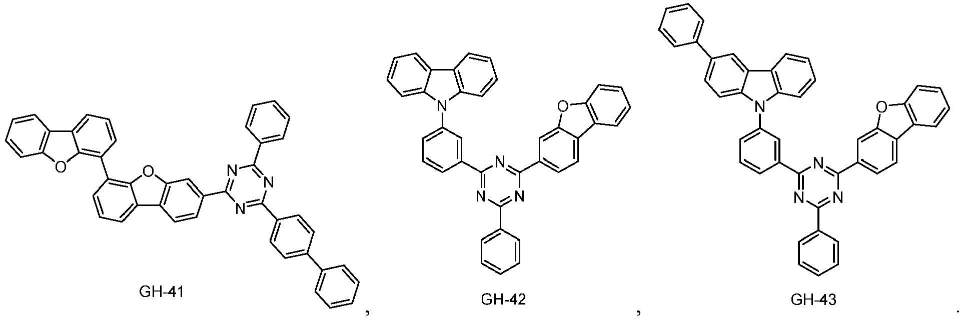

### Combination with Other Materials

The materials described in the present disclosure for a particular layer in an organic light emitting device can be used in combination with various other materials present in the device. The combinations of these materials are described in more detail in U.S. Pat. App. No. 20160359122 at paragraphs 0132-0161. The materials described or referred to the disclosure are non-limiting examples of materials that may be useful in combination with the compounds disclosed herein, and one of skill in the art can readily consult the literature to identify other materials that may be useful in combination.

The materials described herein as useful for a particular layer in an organic light emitting device may be used in combination with a variety of other materials present in the device. For example, compounds disclosed herein may be used in combination with a wide variety of dopants, transport layers, blocking layers, injection layers, electrodes and other layers that may be present. The combination of these materials is described in detail in paragraphs 0080-0101 of U.S. Pat. App. No. 20150349273. The materials described or referred to the disclosure are non-limiting examples of materials that may be useful in combination with the compounds disclosed herein, and one of skill in the art can readily consult the literature to identify other materials that may be useful in combination.

In the embodiments of material synthesis, all reactions were performed under nitrogen protection unless otherwise stated. All reaction solvents were anhydrous and used as received from commercial sources. Synthetic products were structurally confirmed and tested for properties using one or more conventional equipment in the art (including, but not limited to, nuclear magnetic resonance instrument produced by BRUKER, liquid chromatograph produced by SHIMADZU, liquid chromatograph-mass spectrometry produced by SHIMADZU, gas chromatograph-mass spectrometry produced by SHIMADZU, differential Scanning calorimeters produced by SHIMADZU, fluorescence spectrophotometer produced by SHANGHAI LENGGUANG TECH., electrochemical workstation produced by WUHAN CORRTEST, and sublimation apparatus produced by ANHUI BEQ, etc.) by methods well known to the persons skilled in the art. In the embodiments of the device, the characteristics of the device were also tested using conventional equipment in the art (including, but not limited to, evaporator produced by ANGSTROM ENGINEERING, optical testing system produced by SUZHOU FSTAR, life testing system produced by SUZHOU FSTAR, and ellipsometer produced by BEIJING ELLITOP, etc.) by methods well known to the persons skilled in the art. As the persons skilled in the art are aware of the above-mentioned equipment use, test methods and other related contents, the inherent data of the sample can be obtained with certainty and without influence, so the above related contents are not further described in this present disclosure.

### Material Synthesis Example

The method for preparing a compound in the present disclosure is not limited herein. Typically, the following compounds are used as examples without limitation, and synthesis routes and preparation methods thereof are described below.

### Synthesis Example 1: Synthesis of Compound H1-20

### Step 1: Synthesis of Intermediate H1-20-A

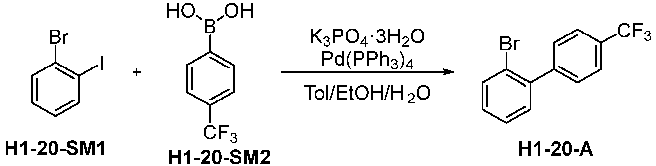

In a 2 L two-necked flask, H1-20-SM1 (60 g, 212.08 mmol), H1-20-SM2 (42.3 g, 222.684 mmol), potassium phosphate trihydrate (141.2 g, 530.2 mmol) and Pd(PPh₃)₄ (4.9 g, 4.24 mmol) were added in sequence. Toluene (800 mL), ethanol (100 mL) and water (100 mL) were added under nitrogen protection, heated to 90 °C and reacted overnight. After the reaction was completed, the system was cooled, extracted with ethyl acetate, dried over anhydrous magnesium sulfate, concentrated and purified through column chromatography to obtain Intermediate H1-20-A (63 g, with a yield of 99%).

### Step 2: Synthesis of Intermediate H1-20-B

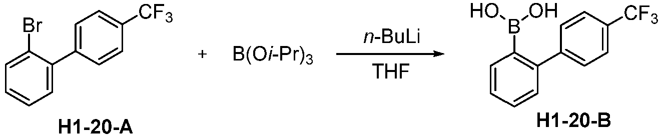

In a 2 L two-necked flask, Intermediate H1-20-A (63 g, 209.23 mmol) was added, added with ultra-dry THF (800 mL) under nitrogen protection and cooled to -78 °C. n-Butyl lithium (2.5 M, 230 mmol, 92 mL) was slowly added dropwise and stirred for 1 h at this temperature. Then, triisopropyl borate (55.42 g, 284.68 mmol) was added and slowly heated to room temperature, and then 3 N dilute hydrochloric acid was added and stirred for 2 h at room temperature. After it was monitored that the reaction was completed, ethyl acetate was added for extraction, and organic phases were isolated, dried, concentrated and purified through column chromatography to obtain Intermediate H1-20-B (34.1 g, with a yield of 61%).

### Step 3: Synthesis of Intermediate H1-20-C

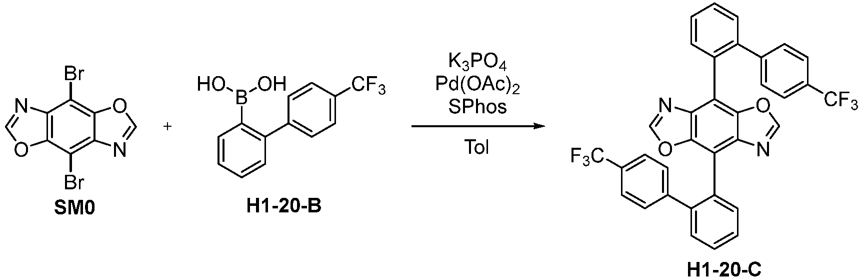

In a 1 L two-necked flask, Intermediate SM0 (13.54 g, 42.6 mmol), Intermediate H1-20-B (34 g, 127.81 mmol), potassium phosphate (54.3 g, 255.61 mmol), palladium acetate (191 mg, 0.852 mmol) and 2-dicyclohexylphosphino-2',6'-dimethoxybiphenyl (SPhos) (874 mg, 2.13 mmol) were added in sequence, purged with nitrogen three times, added with toluene (800 mL) under nitrogen protection, heated to 100 °C and reacted overnight. After the reaction was completed, the system was cooled, extracted with an appropriate amount of tetrahydrofuran, dried over anhydrous magnesium sulfate, concentrated and purified through column chromatography to obtain Intermediate H1-20-C as a white solid (9.2 g, with a yield of 36%).

### Step 4: Synthesis of Intermediate H1-20-D

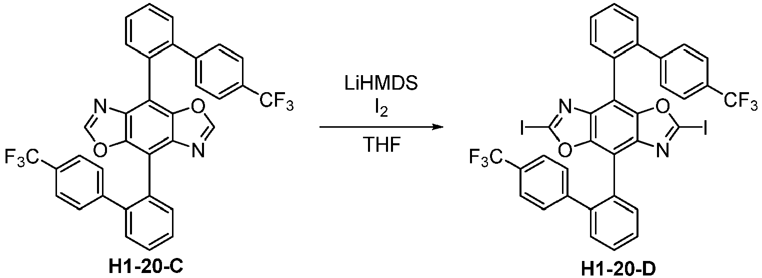

In a 2 L two-necked flask, Intermediate H1-20-C (9.2 g, 15.32 mmol) was added, added with ultra-dry THF (800 mL) under nitrogen protection and cooled to -20 °C. Then, lithium bis(trimethylsilyl)amide (LiHMDS) (34 mL, 34 mmol) was added dropwise and reacted for 2 h at this temperature, and then iodine (15.6 g, 61.28 mmol) was added, heated to room temperature and reacted for 0.5 h. The reaction was quenched with a saturated solution of sodium sulfite, extracted with an appropriate amount of ethyl acetate, dried over anhydrous magnesium sulfate, concentrated and purified through column chromatography to obtain Intermediate H1-20-D as a white solid (11 g, with a yield of 84%).

### Step 5: Synthesis of Intermediate H1-20-E

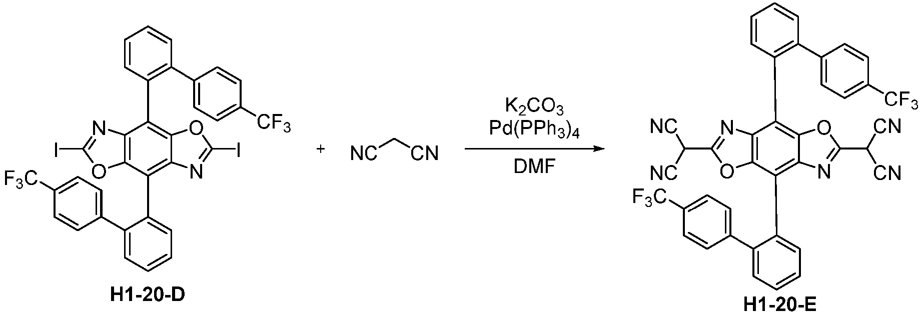

In a 2 L two-necked flask, Intermediate H1-20-D (11 g, 12.906 mmol), malononitrile (3.41 g, 51.62 mmol), potassium carbonate (10.7 g, 77.44 mmol) and Pd(PPh₃)₄ (298 mg, 0.258 mmol) were added in sequence, added with DMF (300 mL) under nitrogen protection, heated to 80 °C and reacted overnight. After the reaction was completed, the system was cooled to room temperature, cooled in an ice water bath, an appropriate amount of concentrated hydrochloric acid was slowly added dropwise, and an appropriate amount of water was added dropwise to precipitate a large amount of yellow solids. The solids were filtered to obtain a crude product, and the crude product was crystallized to obtain Intermediate H1-20-E as a white solid (5.4 g, with a yield of 57%).

### Step 6: Synthesis of Compound H1-20

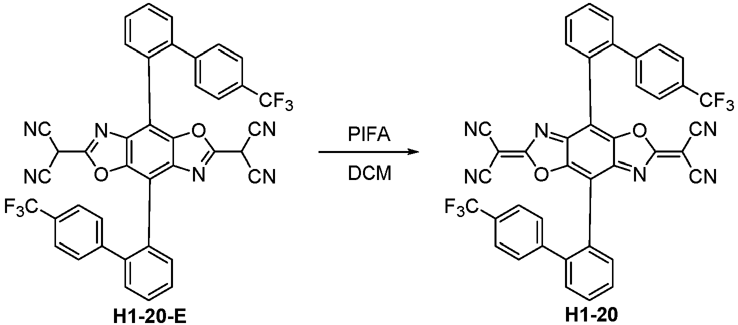

Under a nitrogen atmosphere, Intermediate H1-20-E (5.4 g, 7.41 mmol) was added in a 3 L single-necked flask, 500 mL of DCM was added, and bis(trifluoroacetoxy)iodobenzene (PIFA) (6.37 g, 14.82 mmol) was added portion-wise at room temperature. The reaction solution was stirred at room temperature for 2 days, concentrated to an appropriate volume and filtered to obtain Compound H1-20 as a black-gold solid (4.6 g, with a yield of 85%). The product was confirmed as the target product with a molecular weight of 726.1.

### Synthesis Example 2: Synthesis of Compound H1-68

### Step 1: Synthesis of Intermediate H1-68-A

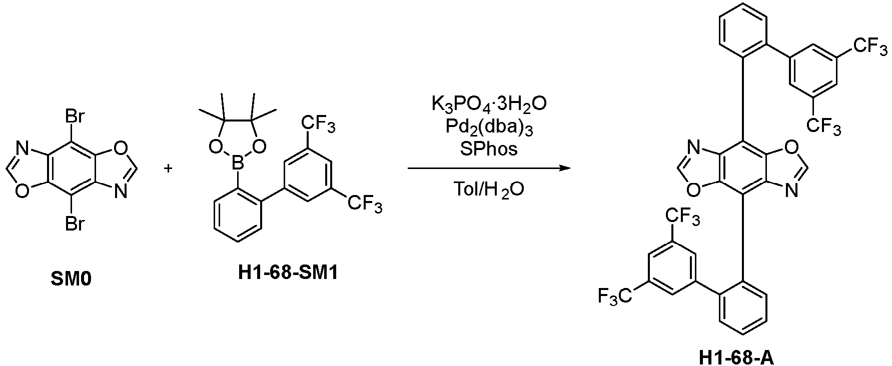

In a 1 L two-necked flask, Intermediate SM0 (13.3 g, 41.83 mmol), Intermediate H1-68-SM1 (47 g, 112.94 mmol), potassium phosphate trihydrate (66.8 g, 250.97 mmol), Pd₂(dba)₃ (766 mg, 0.836 mmol) and SPhos (1.03 g, 2.51 mmol) were added in sequence. Toluene (250 mL) and water (125 mL) were added under nitrogen protection, heated to 95 °C and reacted overnight. After the reaction was completed, the system was cooled, extracted with an appropriate amount of THF, dried over anhydrous magnesium sulfate, concentrated and purified through column chromatography to obtain Intermediate H1-68-A as a white solid (18 g, with a yield of 58%).

### Step 2: Synthesis of Intermediate H1-68-B

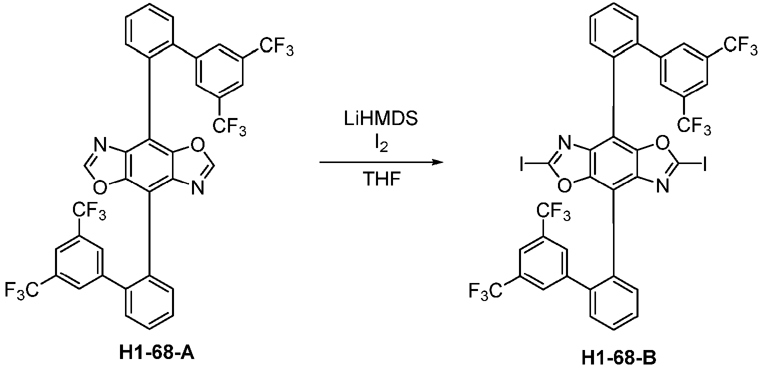

In a 2 L two-necked flask, Intermediate H1-68-A (18 g, 24.44 mmol) was added, purged with nitrogen three times, added with ultra-dry THF (1 L) and cooled to -30 °C. Then, lithium bis(trimethylsilyl)amide (54 mL, 54 mmol) was added dropwise and reacted for 1 h at this temperature, and then iodine (25 g, 97.76 mmol) was added, heated to room temperature and reacted for 0.5 h. The reaction was quenched with a saturated solution of sodium sulfite, extracted with an appropriate amount of ethyl acetate, dried over anhydrous magnesium sulfate, concentrated and purified through column chromatography to obtain Intermediate H1-68-B as a white solid (22.2 g, with a yield of 92%).

### Step 3: Synthesis of Intermediate H1-68-C

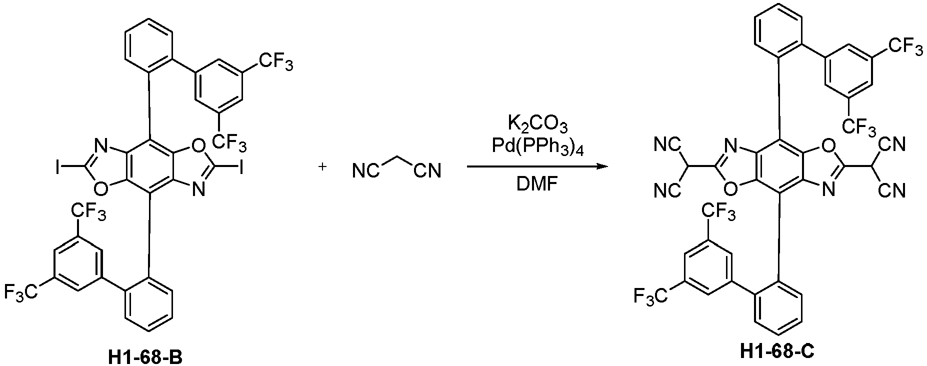

In a 2 L two-necked flask, Intermediate H1-68-B (22 g, 22.26 mmol), malononitrile (5.88 g, 89.04 mmol), potassium carbonate (18.5 g, 133.56 mmol) and Pd(PPh₃)₄ (0.51 g, 0.445 mmol) were added in sequence, purged with nitrogen three times, added with DMF (500 mL) under nitrogen protection, heated to 80 °C and reacted overnight. After the reaction was completed, the system was cooled to room temperature, cooled in an ice water bath, an appropriate amount of concentrated hydrochloric acid was slowly added dropwise, and an appropriate amount of water was added dropwise to precipitate a large amount of yellow solids. The solids were filtered to obtain a crude product, and the crude product was crystallized to obtain Intermediate H1-68-C as a white solid (17 g, with a yield of 88%).

### Step 4: Synthesis of Compound H1-68

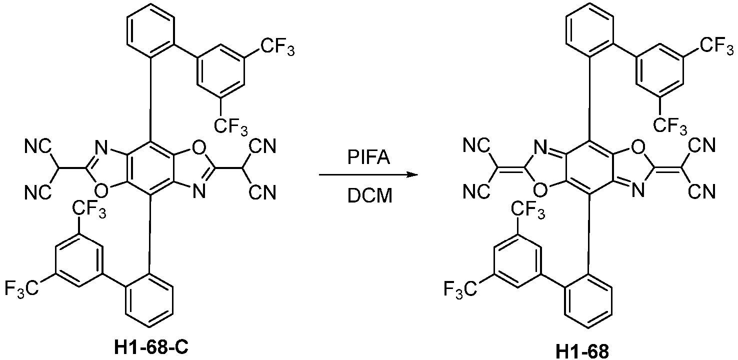

Under a nitrogen atmosphere, Intermediate H1-68-C (15 g, 17.35 mmol) was added in a 3 L single-necked flask, 2 L of DCM was added, and PIFA (14.9 g, 34.7 mmol) was added portion-wise at room temperature. The reaction solution was stirred at room temperature for 3 days, concentrated to about 500 mL and filtered to obtain Compound H1-68 as a black-gold solid (11.9 g, with a yield of 79%). The product was confirmed as the target product with a molecular weight of 862.1.

### Synthesis Example 3: Synthesis of Compound H1-70

### Step 1: Synthesis of Intermediate H1-70-A

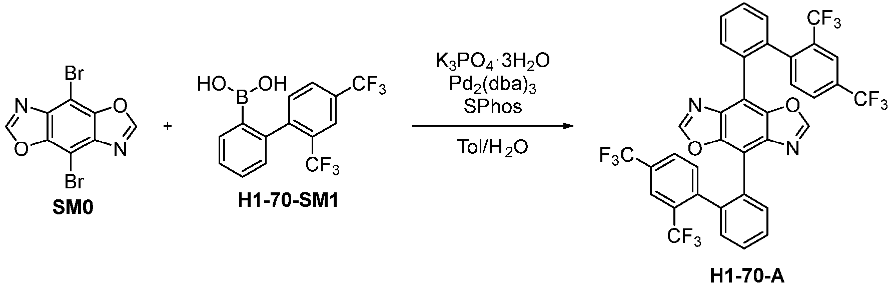

In a 2 L two-necked flask, Intermediate SM0 (17.92 g, 56.36 mmol), Intermediate H1-70-SM1 (47 g, 140.88 mmol), potassium phosphate trihydrate (90 g, 338.12 mmol), Pd₂(dba)₃ (1.5 g, 1.69 mmol) and SPhos (2.78 g, 6.77 mmol) were added in sequence. Toluene (600 mL) and water (300 mL) were added under nitrogen protection, heated to 95 °C and reacted overnight. After the reaction was completed, the system was cooled, extracted with an appropriate amount of THF, dried over anhydrous magnesium sulfate, concentrated and purified through column chromatography to obtain Intermediate H1-70-A as a white solid (28.6 g, with a yield of 69%).

### Step 2: Synthesis of Intermediate H1-70-B

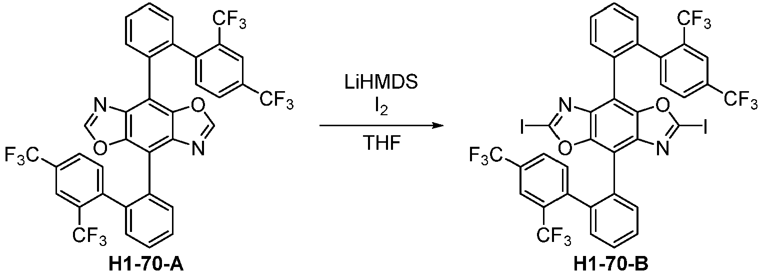

In a 2 L two-necked flask, Intermediate H1-70-A (23.8 g, 32.31 mmol) was added, added with ultra-dry THF (1 L) under nitrogen protection and cooled to -30 °C. Then, lithium bis(trimethylsilyl)amide (1.0 M, 68 mL, 68 mmol) was added dropwise and reacted for 2 h at this temperature, and then iodine (32.8 g, 129.24 mmol) was added, heated to room temperature and reacted for 0.5 h. The reaction was quenched with a saturated solution of sodium sulfite, extracted with an appropriate amount of ethyl acetate, dried over anhydrous magnesium sulfate, concentrated and purified through column chromatography to obtain Intermediate H1-70-B as a white solid (22 g, with a yield of 69%).

### Step 3: Synthesis of Intermediate H1-70-C

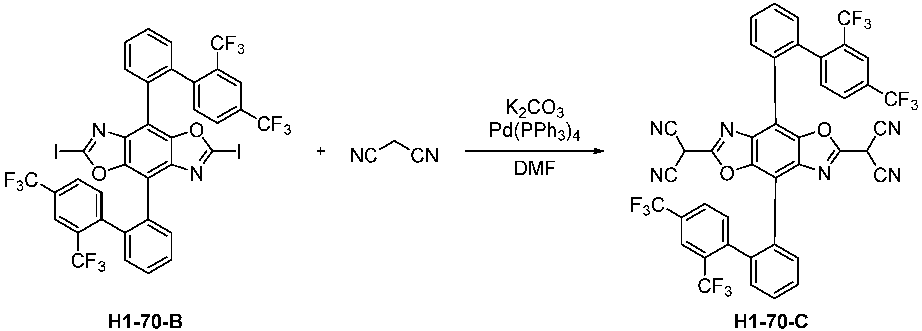

In a 2 L two-necked flask, Intermediate H1-70-B (22 g, 22.26 mmol), malononitrile (5.9 g, 89.04 mmol), potassium carbonate (18.5 g, 133.56 mmol) and Pd(PPh₃)₄ (0.51 g, 0.445 mmol) were added in sequence, added with DMF (500 mL) under nitrogen protection, heated to 80 °C and reacted overnight. After the reaction was completed, the system was cooled to room temperature, cooled in an ice water bath, an appropriate amount of concentrated hydrochloric acid was slowly added dropwise, and an appropriate amount of water was added dropwise to precipitate a large amount of yellow solids. The solids were filtered to obtain a crude product, and the crude product was crystallized to obtain Intermediate H1-70-C as a white solid (13 g, with a yield of 68%).

### Step 4: Synthesis of Compound H1-70

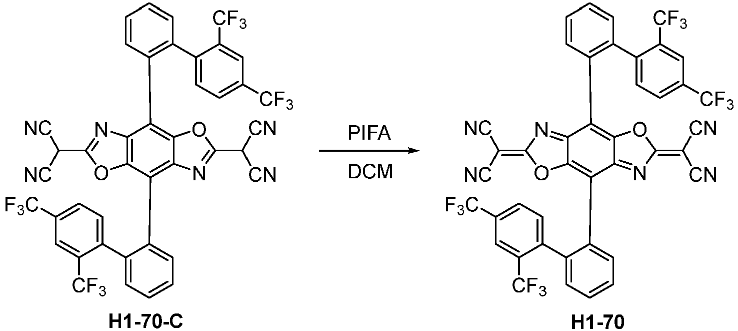

Under a nitrogen atmosphere, Intermediate H1-70-C (13 g, 15.04 mmol) was added in a 3 L single-necked flask, 1.5 L of DCM was added, and PIFA (13 g, 30.07 mmol) was added portion-wise at room temperature. The reaction solution was purple black after stirred at room temperature for 2 days. The reaction solution was concentrated to 500 mL and filtered to obtain Compound H1-70 as a black-gold solid (10 g, with a yield of 77%). The product was confirmed as the target product with a molecular weight of 862.1.

### Synthesis Example 4: Synthesis of Compound H1-22

### Step 1: Synthesis of Intermediate H1-22-A

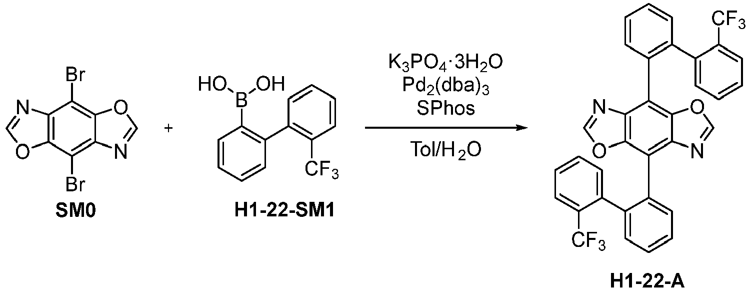

In a 1 L two-necked flask, Intermediate SM0 (11.2 g, 35.08 mmol), Intermediate H1-22-SM1 (28 g, 105.25 mmol), potassium phosphate trihydrate (46.7 g, 175.42 mmol), Pd₂(dba)₃ (643 mg, 0.702 mmol) and SPhos (1.44 g, 3.508 mmol) were added in sequence. Toluene (1 L) and water (200 mL) were added under nitrogen protection, heated to 90 °C and reacted overnight. After the reaction was completed, the system was cooled, extracted with an appropriate amount of tetrahydrofuran, dried over anhydrous magnesium sulfate, concentrated and purified through column chromatography to obtain Intermediate H1-22-A as a white solid (19.1 g, with a yield of 91%).

### Step 2: Synthesis of Intermediate H1-22-B

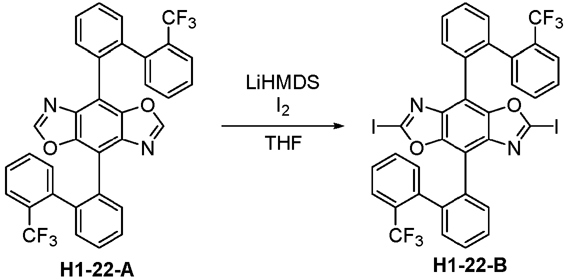

In a 2 L two-necked flask, Intermediate H1-22-A (19 g, 31.64 mmol) was added, added with ultra-dry THF (500 mL) and cooled to -20 °C. Then, lithium bis(trimethylsilyl)amide (67 mL, 66.44 mmol) was added dropwise and reacted for 2 h at this temperature, and then iodine (32.1 g, 126.56 mmol) was added, heated to room temperature and reacted for 0.5 h. The reaction was quenched with a saturated solution of sodium sulfite, extracted with an appropriate amount of ethyl acetate, dried over anhydrous magnesium sulfate, concentrated and purified through column chromatography to obtain Intermediate H1-22-B as a white solid (16 g, with a yield of 60%).

### Step 3: Synthesis of Intermediate H1-22-C

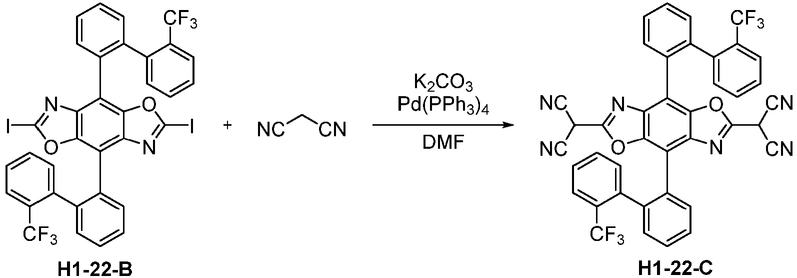

In a 2 L two-necked flask, Intermediate H1-22-B (16 g, 18.77 mmol), malononitrile (4.96 g, 75.09 mmol), potassium carbonate (15.6 g, 112.64 mmol) and Pd(PPh₃)₄ (0.43 g, 0.375 mmol) were added in sequence, added with DMF (300 mL) under nitrogen protection, heated to 80 °C and reacted overnight. After the reaction was completed, the system was cooled to room temperature, cooled in an ice water bath, an appropriate amount of concentrated hydrochloric acid was slowly added dropwise, and an appropriate amount of water was added dropwise to precipitate a large amount of yellow solids. The solids were filtered to obtain a crude product, and the crude product was crystallized to obtain Intermediate H1-22-C as a white solid (9.6 g, with a yield of 70%).

### Step 4: Synthesis of Compound H1-22

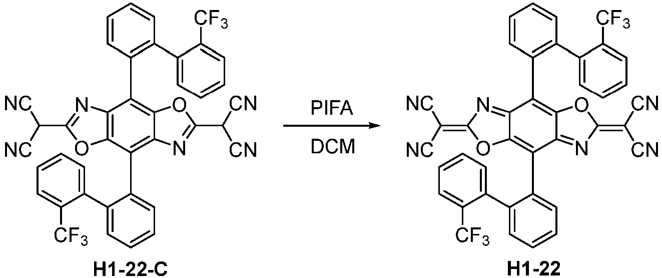

Under a nitrogen atmosphere, Intermediate H1-22-C (9.6 g, 13.18 mmol) was added in a 2 L single-necked flask, 1 L of DCM was added, and bis(trifluoroacetoxy)iodobenzene (11.3 g, 26.35 mmol) was added portion-wise at room temperature. The reaction solution was purple black after stirred at room temperature for 2 days. The reaction solution was concentrated to about 100 mL, added with an appropriate amount of petroleum ether and filtered to obtain Compound H1-22 as a yellow-brown solid (6.5 g, with a yield of 68%). The product was confirmed as the target product with a molecular weight of 726.1.

### Synthesis Example 5: Synthesis of Compound H1-1518

### Step 1: Synthesis of Intermediate H1-1518-A

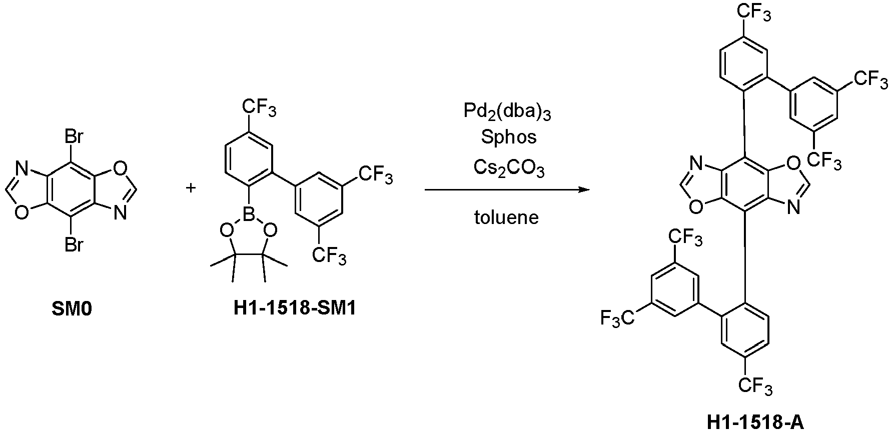

In a 500 mL two-necked round-bottom flask, Intermediate SM0 (15.5 g, 32 mmol), Intermediate H1-1518-SM1 (2.04 g, 6.4 mmol), cesium carbonate (14.6 g, 44.8 mmol), Pd₂(dba)₃ (293 mg, 0.32 mmol), SPhos (0.53 g, 1.28 mmol) and toluene (120 mL) were added in sequence under a N₂ atmosphere, heated to 115 °C and reacted overnight. After TLC showed that the reaction was completed, the reaction solution was cooled to room temperature and filtered through Celite, and the filtrate was concentrated and purified through column chromatography to obtain Intermediate H1-1518-A as a white solid (3.3 g, with a yield of 12%).

### Step 2: Synthesis of Intermediate H1-1518-B

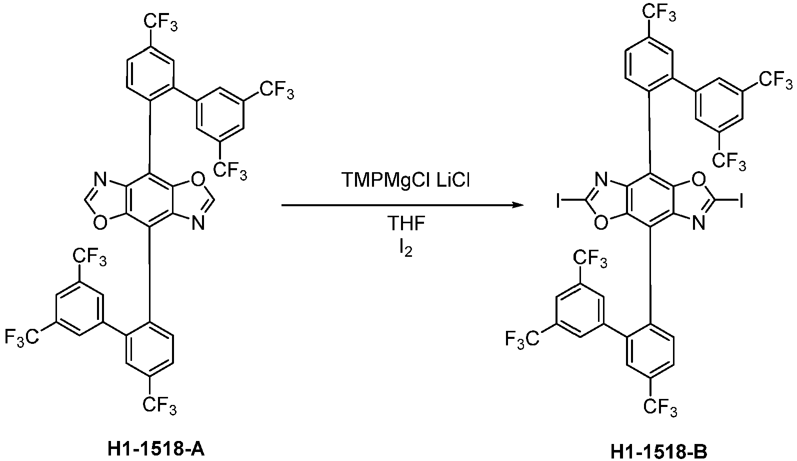

In a 500 mL two-necked round-bottom flask, Intermediate H1-1518-A (3.3 g, 3.8 mmol) and tetrahydrofuran (190 mL) were added under a N₂ atmosphere and cooled to -30 °C. 2,2,6,6-tetramethylpiperidylmagnesium chloride lithium chloride complex (TMPMgCl LiCl) (9.12 mL, 9.12 mmol) was added dropwise, stirred for 1 h at -30 °C, slowly heated to -20 °C and reacted for 2 h at this temperature. Iodine (3.86 g, 15.2 mmol) was added, heated to room temperature and reacted for 2 h. After TLC showed that the reaction was completed, the reaction was quenched with a saturated aqueous solution of sodium sulfite, extracted with dichloromethane, concentrated and purified through column chromatography to obtain Intermediate H1-1518-B as a white solid (3.0 g, with a yield of 70%).

### Step 3: Synthesis of Intermediate H1-1518-C

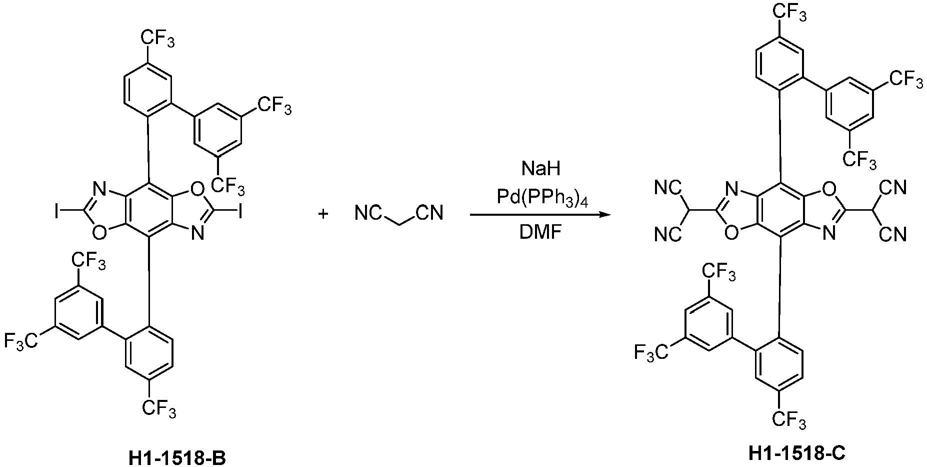

In a 150 mL two-necked round-bottom flask, malononitrile (1.06 g, 16 mmol) and N,N-dimethylformamide (50 mL) were added in sequence under a N₂ atmosphere and cooled to 0 °C. NaH (60% by weight) (0.64 g, 16 mmol) was added portion-wise and stirred for 10 min at 0 °C. Intermediate H1-1518-B (3.0 g, 2.67 mmol) and Pd(PPh₃)₄ (247 mg, 0.214 mmol) were added, heated to 90 °C and reacted for 36 h. After HPLC showed that the reaction was completed, the reaction system was cooled to room temperature, poured into dilute hydrochloric acid to precipitate a large amount of yellow solids, and filtered. The filter cake was washed with water and an appropriate amount of acetonitrile to obtain Intermediate H1-1518-C as an off-white solid (2.65 g, with a yield of 99.6%).

### Step 4: Synthesis of Compound H1-1518

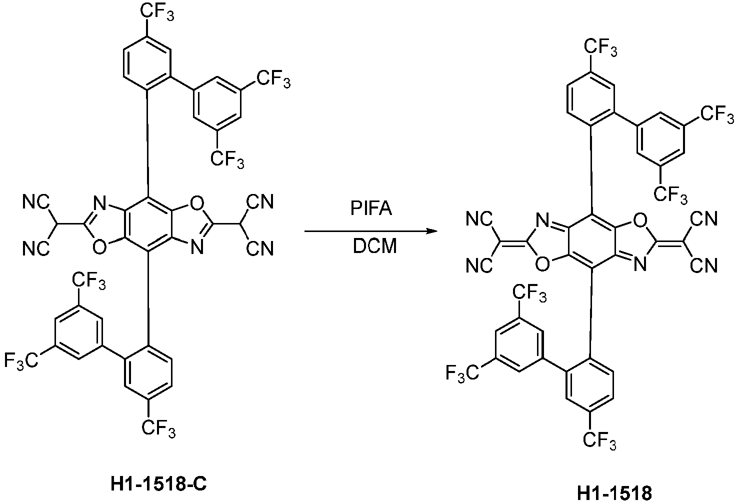

In a 150 mL two-necked round-bottom flask, Intermediate H1-1518-C (2.6 g, 2.6 mmol) and dichloromethane (80 mL) were added in sequence under a N₂ atmosphere. PIFA (1.67 g, 5.2 mmol) was added portion-wise and stirred at room temperature for 3 days. The reaction solution was concentrated to an appropriate volume, added with n-hexane and filtered to obtain Compound H1-1518 as a purple-black solid (2.0 g, with a yield of 77%). The product was confirmed as the target product with a molecular weight of 998.1.

### Synthesis Example 6: Synthesis of Compound H1-428

### Step 1: Synthesis of Intermediate H1-428-A

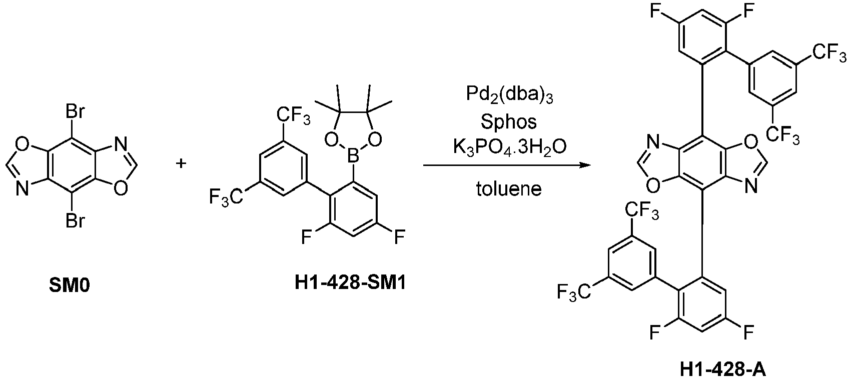

In a 2 L two-necked round-bottom flask, Intermediate H1-428-SM1 (22 g, 48.8 mmol), Intermediate SM0 (3.1 g, 9.75 mmol), potassium phosphate trihydrate (21.6 g, 68.3 mmol), Pd₂(dba)₃ (358 mg, 0.39 mmol), SPhos (0.72 g, 1.75 mmol) and toluene (500 mL) were added in sequence under a N₂ atmosphere, heated to 105 °C and reacted overnight. After TLC showed that the reaction was completed, the reaction solution was cooled to room temperature, filtered through Celite, concentrated and purified through column chromatography to obtain Intermediate H1-428-A as a white solid (6.0 g, with a yield of 76%).

### Step 2: Synthesis of Intermediate H1-428-B

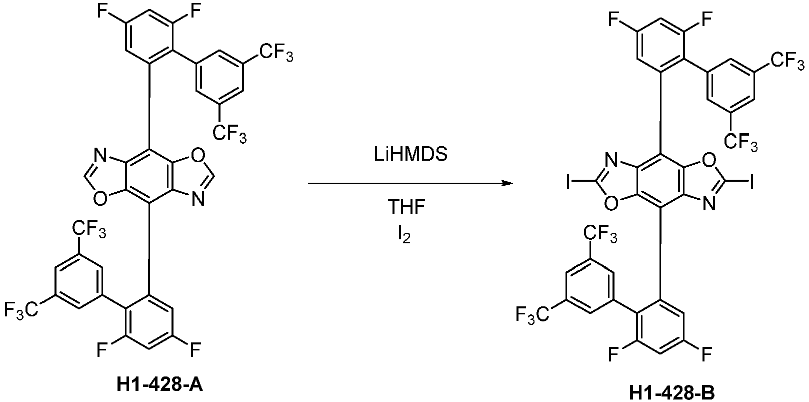

In a 500 mL two-necked round-bottom flask, Intermediate H1-428-A (5 g, 6.19 mmol) and tetrahydrofuran (120 mL) were added under a N₂ atmosphere and cooled to -30 °C. LiHMDS (13 mL, 13 mmol) was added dropwise and stirred for 1 h at -30 °C. Iodine (4.72 g, 18.6 mmol) was added, heated to room temperature and reacted for 2 h. After TLC showed that the reaction was completed, the reaction was quenched with a saturated aqueous solution of sodium sulfite until the reaction solution was colorless. The reaction solution was extracted with dichloromethane, concentrated and purified through column chromatography to obtain Intermediate H1-428-B as a white solid (5.1 g, with a yield of 78%).

### Step 3: Synthesis of Intermediate H1-428-C

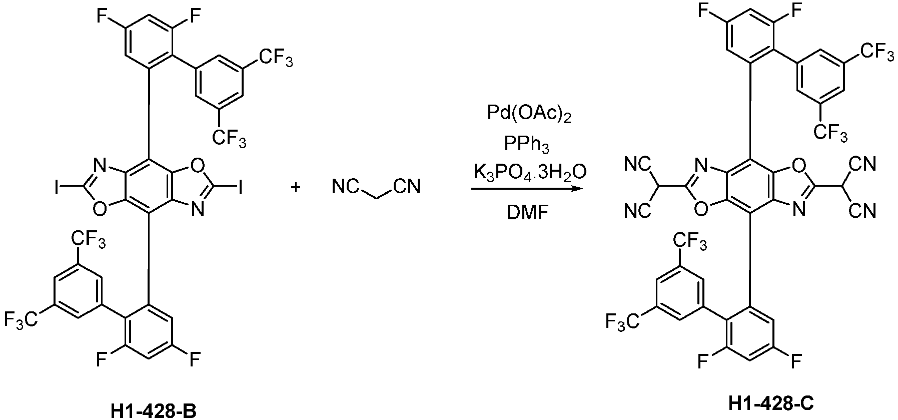

In a 500 mL two-necked round-bottom flask, Intermediate H1-428-B (3.9 g, 3.68 mmol), malononitrile (1.45 g, 22.08 mmol), potassium phosphate trihydrate (5.85 g, 22.08 mmol), Pd(OAc)₂ (33.1 mg, 0.147 mmol), triphenylphosphine (PPh₃) (130 mg, 0.368 mmol) and N,N-dimethylformamide (310 mL) were added in sequence under a N₂ atmosphere, heated to 80 °C and reacted for 36 h. After HPLC showed that the reaction was completed, the reaction system was cooled to room temperature, poured into dilute hydrochloric acid to precipitate a large amount of yellow solids, and filtered. The filter cake was washed with water and an appropriate amount of acetonitrile to obtain Intermediate H1-428-C as an off-white solid (3.3 g, with a yield of 95%).

### Step 4: Synthesis of Compound H1-428

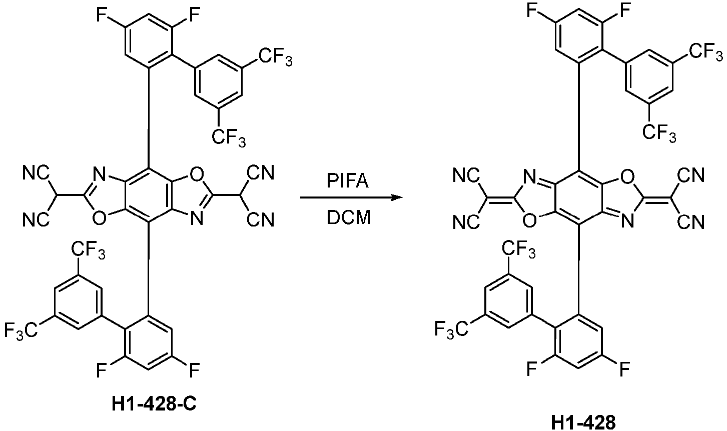

In a 2 L two-necked round-bottom flask, Intermediate H1-428-C (3.3 g, 3.5 mmol) and dichloromethane (1 L) were added in sequence under a N₂ atmosphere. PIFA (2.26 g, 5.25 mmol) was added portion-wise and stirred at room temperature for 3 days. The reaction solution was concentrated to an appropriate volume, washed with n-hexane and filtered to obtain Compound H1-428 as a purple-black solid (2.26 g, with a yield of 68.5%). The product was confirmed as the target product with a molecular weight of 934.1.

### Synthesis Example 7: Synthesis of Compound H1-32

### Step 1: Synthesis of Intermediate H1-32-A

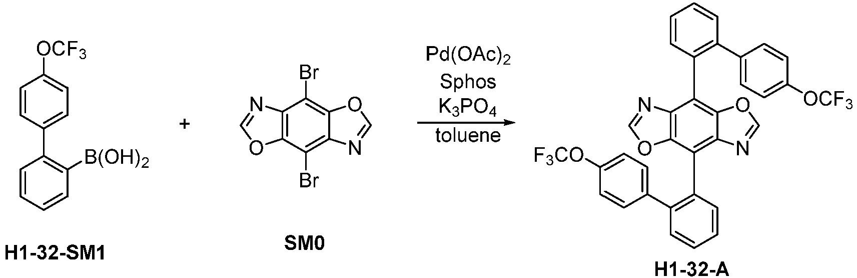

In a 1 L two-necked round-bottom flask, Intermediate H1-32-SM1 (19 g, 67.4 mmol), Intermediate SM0 (5.4 g, 16.8 mmol), potassium phosphate (21.6 g, 101.9 mmol), Pd(OAc)₂ (76 mg, 0.34 mmol), SPhos (0.7 g, 1.7 mmol) and toluene (320 mL) were added in sequence under a N₂ atmosphere, heated to 110 °C and reacted overnight. After TLC showed that the reaction was completed, the reaction solution was cooled to room temperature and filtered through Celite, and the filtrate was concentrated to dryness, washed with heptane and filtered to obtain Intermediate H1-32-A as a white solid (9.5 g, with a yield of 89%).

### Step 2: Synthesis of Intermediate H1-32-B

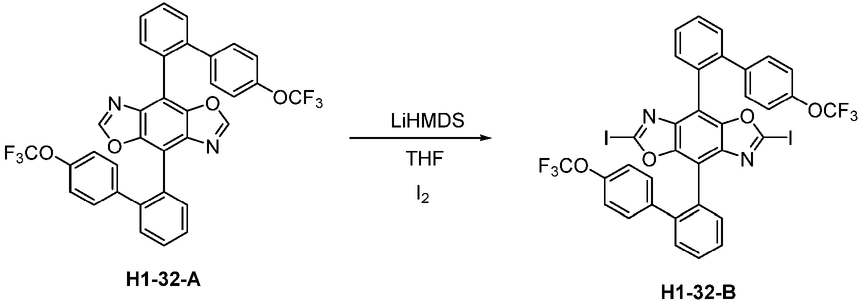

In a 1 L two-necked round-bottom flask, Intermediate H1-32-A (9.5 g, 15 mmol) and tetrahydrofuran (300 mL) were added under a N₂ atmosphere and cooled to -30 °C. LiHMDS (38 mL, 37.5 mmol) was added dropwise and stirred for 1 h at-30 °C. I₂ (15.2 g, 60 mmol) was added, heated to room temperature and reacted for 0.5 h. After TLC showed that the reaction was completed, the reaction was quenched with a saturated aqueous solution of sodium sulfite, extracted with dichloromethane, concentrated and purified through column chromatography to obtain Intermediate H1-32-B as a white solid (11 g, with a yield of 82.7%).

### Step 3: Synthesis of Intermediate H1-32-C

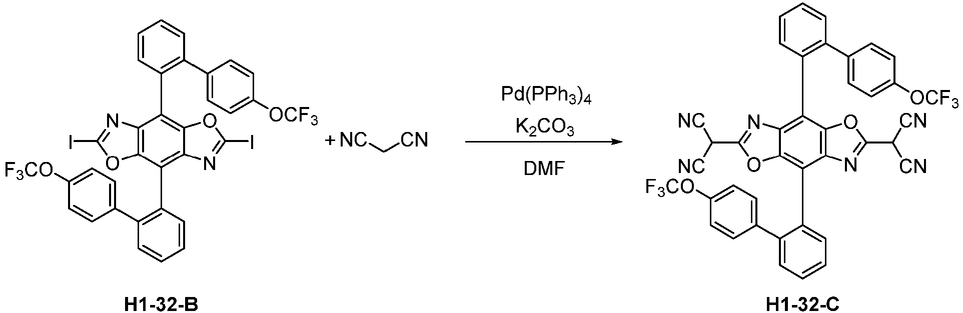

In a 1 L two-necked round-bottom flask, Intermediate H1-32-B (11 g, 12.4 mmol), malononitrile (4.93 g, 74.6 mmol), Pd(PPh₃)₄ (0.29 g, 0.25 mmol), potassium carbonate (10.3 g, 74.6 mmol) and N,N-dimethylformamide (310 mL) were added in sequence under a N₂ atmosphere, heated to 65 °C and reacted overnight. After HPLC showed that the reaction was completed, the reaction system was cooled to room temperature, poured into dilute hydrochloric acid to precipitate a large amount of yellow solids, and suction filtered. The filter cake was washed with water and an appropriate amount of acetonitrile to obtain Intermediate H1-32-C as an off-white solid (9.4 g, with a yield of 100%).

### Step 4: Synthesis of Compound H1-32

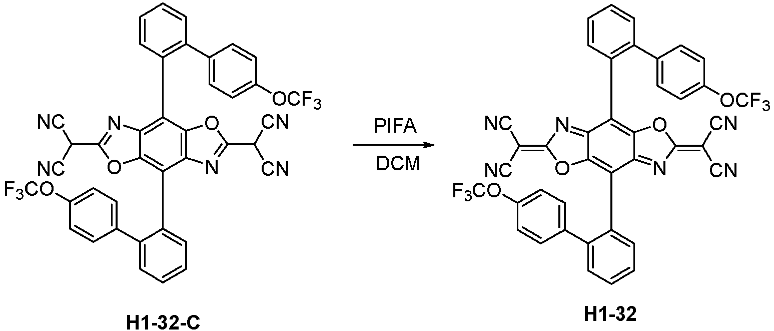

In a 2 L two-necked round-bottom flask, Intermediate H1-32-C (9.4 g, 12.4 mmol) and DCM (1.5 L) were added in sequence under a N₂ atmosphere. PIFA (12.5 g, 29 mmol) was added portion-wise and stirred at room temperature for 3 days. The reaction solution was concentrated to an appropriate volume and filtered to obtain Compound H1-32 as a brown-yellow solid (5.08 g, with a yield of 54%). The product was confirmed as the target product with a molecular weight of758.1.

### Synthesis Example 8: Synthesis of Compound H1-258

### Step 1: Synthesis of Intermediate H1-258-A

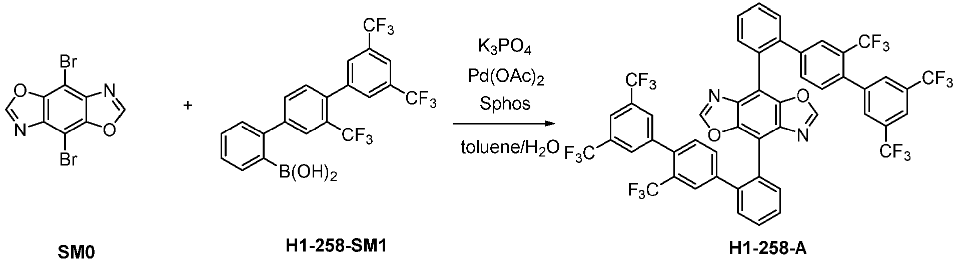

In a 1 L two-necked round-bottom flask, Intermediate SM0 (4.98 g, 15.67 mmol), Intermediate H1-258-SM1 (21.0 g, 43.9 mmol), potassium phosphate (13.3 g, 62.7 mmol), Pd(OAc)₂ (70.5 mg, 0.314 mmol), SPhos (0.32 g, 0.785 mmol), toluene (350 mL) and water (50 mL) were added in sequence under a N₂ atmosphere, heated to 90 °C and reacted overnight at this temperature. After TLC showed that the reaction was completed, the system was cooled to room temperature, layers were separated, and the aqueous phase was extracted three times with toluene. Organic phases were combined, concentrated, and purified through column chromatography to obtain Intermediate H1-258-A as a white solid (16.0 g, with a yield of 99%).

### Step 2: Synthesis of Intermediate H1-258-B

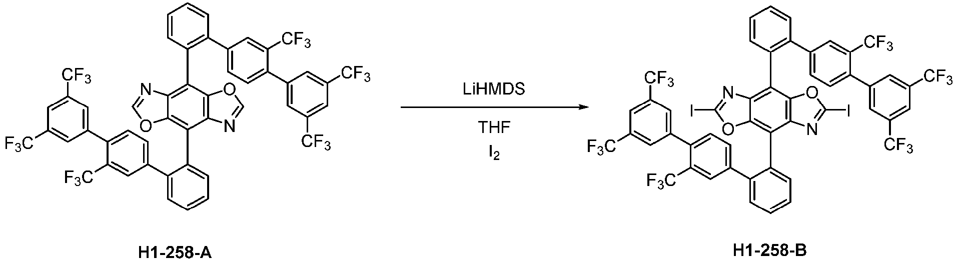

In a 1 L two-necked round-bottom flask, Intermediate H1-258-A (16 g, 15.6 mmol) and tetrahydrofuran (380 mL) were added under a N₂ atmosphere and cooled to -50 °C. LiHMDS (39 mL, 39 mmol) was slowly added dropwise, heated to -35 °C and stirred for 2 h at this temperature. I₂ (16.9 g, 66.5 mmol) was added, heated to room temperature and reacted for 2 h at room temperature. After TLC showed that the reaction was completed, the reaction was quenched with a saturated aqueous solution of sodium sulfite, extracted with dichloromethane, concentrated and purified through column chromatography to obtain Intermediate H1-258-B as a white solid (16.8 g, with a yield of 84%).

### Step 3: Synthesis of Intermediate H1-258-C

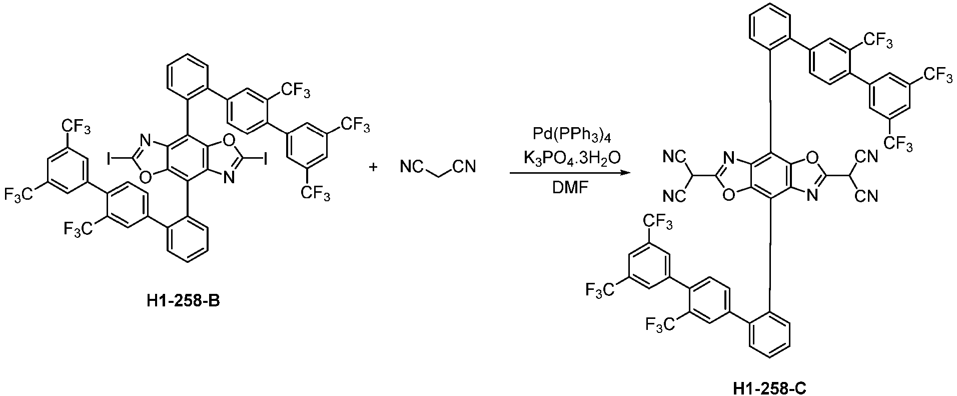

In a 1 L two-necked round-bottom flask, Intermediate H1-258-B (16.8 g, 13.1 mmol), malononitrile (3.46 g, 52.4 mmol), potassium phosphate trihydrate (14.1 g, 53 mmol), Pd(PPh₃)₄ (454 mg, 0.39 mmol) and N,N-dimethylformamide (436 mL) were added in sequence under a N₂ atmosphere, heated to 90 °C and reacted for 16 h at this temperature. After HPLC showed that the reaction was completed, the reaction system was cooled to room temperature, poured into dilute hydrochloric acid to precipitate a large amount of yellow solids, and suction filtered. The filter cake was washed with water and an appropriate amount of acetonitrile to obtain Intermediate H1-258-C as an off-white solid (13 g, with a yield of 85.5%).

### Step 4: Synthesis of Compound H1-258

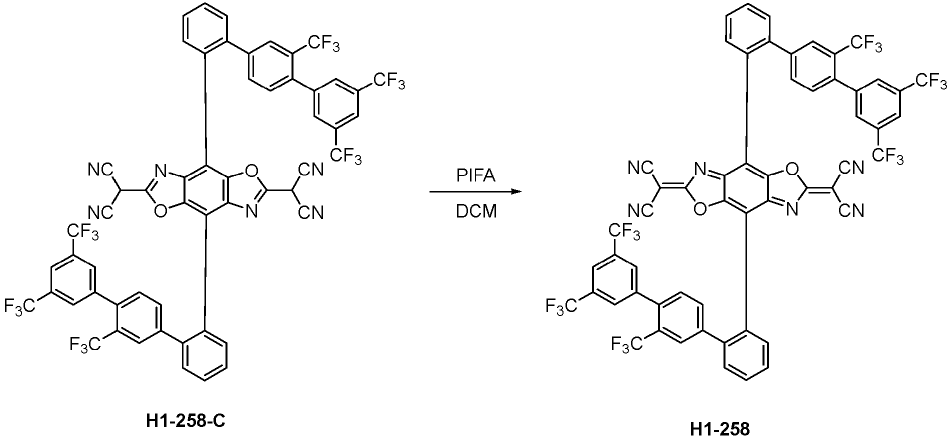

In a 3 L single-necked round-bottom flask, Intermediate H1-258-C (13 g, 11.2 mmol) and dichloromethane (1.5 L) were added under a N₂ atmosphere. PIFA (7.22 g, 16.8 mmol) was added portion-wise and reacted at room temperature for 2 days. The reaction solution was concentrated to an appropriate volume and filtered to obtain Compound H1-258 as a black solid (9 g, with a yield of 70%). The product was confirmed as the target product with a molecular weight of 1150.1.

### Synthesis Example 9: Synthesis of Compound H1-194

### Step 1: Synthesis of Intermediate H1-194-A

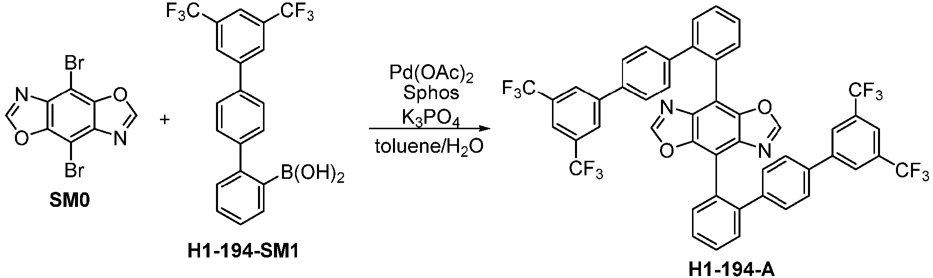

In a 2 L two-necked round-bottom flask, Intermediate H1-194-SM1 (32 g, 78 mmol), Intermediate SM0 (8.3 g, 26 mmol), potassium phosphate (22 g, 104 mmol), Pd(OAc)₂ (0.12 g, 0.52 mmol), SPhos (0.53 g, 1.3 mmol), toluene (600 mL) and water (60 mL) were added in sequence under a N₂ atmosphere, heated to 110 °C and reacted overnight. After TLC showed that the reaction was completed, the system was cooled to room temperature, added with water to precipitate a gray solid and filtered, and the filter cake was purified through column chromatography (eluted with tetrahydrofuran) to obtain Intermediate H1-194-A as a white solid (22 g, with a yield of 95%).

### Step 2: Synthesis of Intermediate H1-194-B

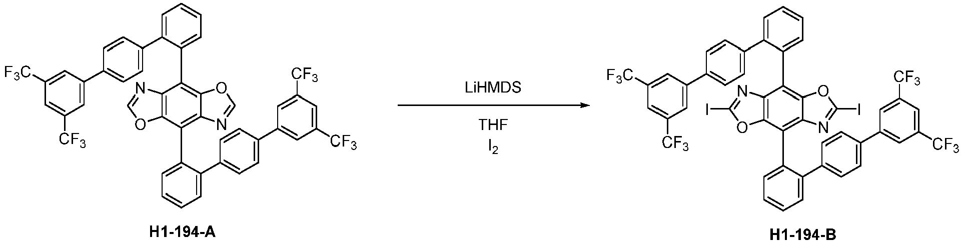

In a 2 L two-necked round-bottom flask, Intermediate H1-194-A (12 g, 13.5 mmol) and tetrahydrofuran (700 mL) were added under a N₂ atmosphere and cooled to -30 °C. LiHMDS (34 mL, 34 mmol) was added dropwise and stirred for 1 h at -30 °C. I₂ (13.7 g, 54 mmol) was added, heated to room temperature and reacted for 1 h. After TLC showed that the reaction was completed, the reaction was quenched with a saturated aqueous solution of sodium sulfite, extracted with dichloromethane, dried over anhydrous magnesium sulfate, concentrated and washed with acetonitrile to obtain Intermediate H1-194-B as a white solid (13.4 g, with a yield of 87%).

### Step 3: Synthesis of Intermediate H1-194-C

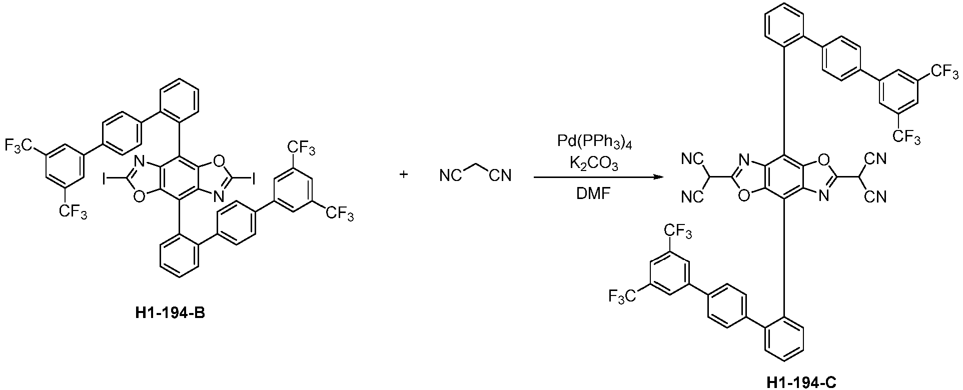

In a 1 L two-necked round-bottom flask, Intermediate H1-194-B (13.4 g, 11.8 mmol), malononitrile (5.3 g, 80 mmol), Pd(PPh₃)₄ (0.29 g, 0.3 mmol), potassium carbonate (11 g, 80 mmol) and N,N-dimethylformamide (400 mL) were added in sequence under a N₂ atmosphere, heated to 65 °C and reacted overnight. After HPLC showed that the reaction was completed, the reaction system was cooled to room temperature, poured into dilute hydrochloric acid to precipitate a large amount of yellow solids, and filtered. The filter cake was washed with water and an appropriate amount of acetonitrile to obtain Intermediate H1-194-C as an off-white solid (9.1 g, with a yield of 75%).

### Step 4: Synthesis of Compound H1-194

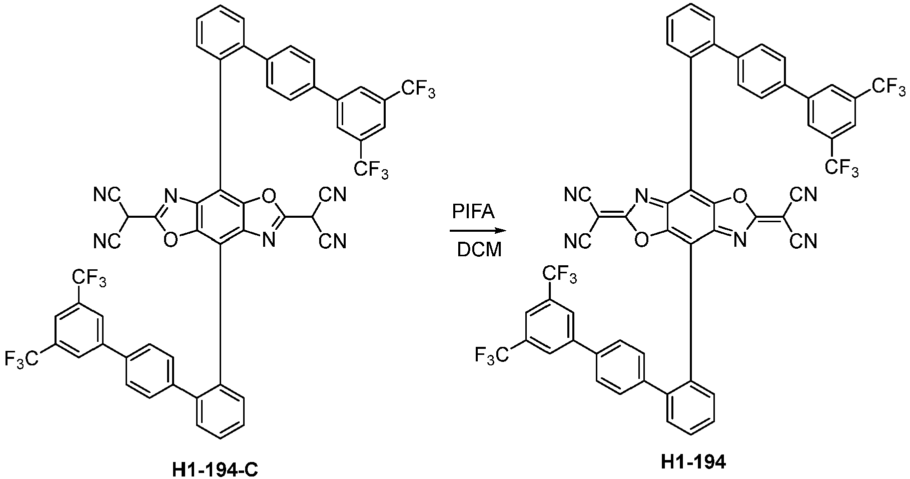

In a 1 L two-necked round-bottom flask, Intermediate H1-194-C (9.1 g, 8.9 mmol) and dichloromethane (600 mL) were added in sequence under a N₂ atmosphere. PIFA (7.7 g, 13.2 mmol) was added portion-wise and stirred at room temperature for 2 days. The reaction solution was concentrated to an appropriate volume and filtered to obtain Compound H1-194 as a purple-black solid (4.0 g, with a yield of 44%). The product was confirmed as the target product with a molecular weight of 1014.2.

### Synthesis Example 10: Synthesis of Compound H1-250

### Step 1: Synthesis of Intermediate H1-250-A

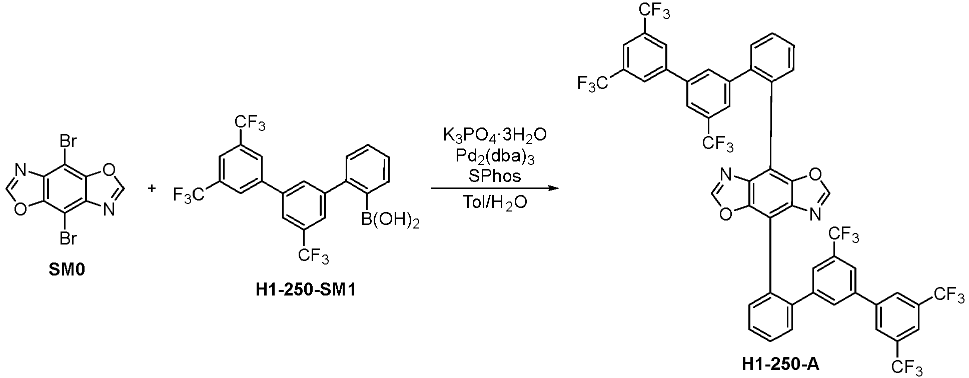

In a 2 L two-necked flask, Intermediate SM0 (12.2 g, 38.4 mmol), Intermediate H1-250-SM1 (55 g, 115.1 mmol), potassium phosphate trihydrate (51.1 g, 191.9 mmol), Pd₂(dba)₃ (703 mg, 0.768 mmol) and SPhos (1.57 g, 3.84 mmol) were added in sequence. Toluene (1 L) and water (200 mL) were added under nitrogen protection, heated to 90 °C and reacted overnight. After the reaction was completed, the system was cooled, extracted with an appropriate amount of tetrahydrofuran, dried over anhydrous magnesium sulfate, concentrated and purified through column chromatography to obtain Intermediate H1-250-A as a white solid (36.9 g, with a yield of 94%).

### Step 2: Synthesis of Intermediate H1-250-B

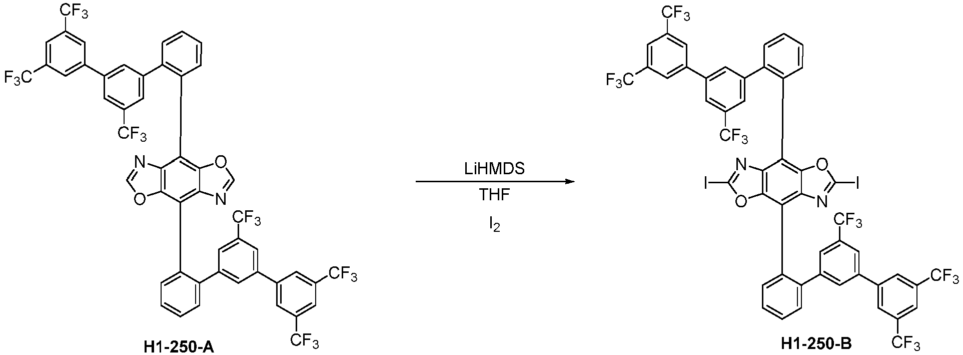

In a 2 L two-necked flask, Intermediate H1-250-A (36.9 g, 36.01 mmol) was added under nitrogen protection, added with ultra-dry THF (800 mL) and cooled to -20 °C. Then, LiHMDS (1.0 M, 76 mL, 76 mmol) was added dropwise and reacted for 2 h at this temperature, and then iodine (36.6 g, 144.04 mmol) was added, heated to room temperature and reacted for 0.5 h. The reaction was quenched with a saturated solution of sodium sulfite and extracted with an appropriate amount of ethyl acetate. The organic phase was dried over anhydrous magnesium sulfate and purified through column chromatography to obtain Intermediate H1-250-B as a white solid (40 g, with a yield of 87%).

### Step 3: Synthesis of Intermediate H1-250-C

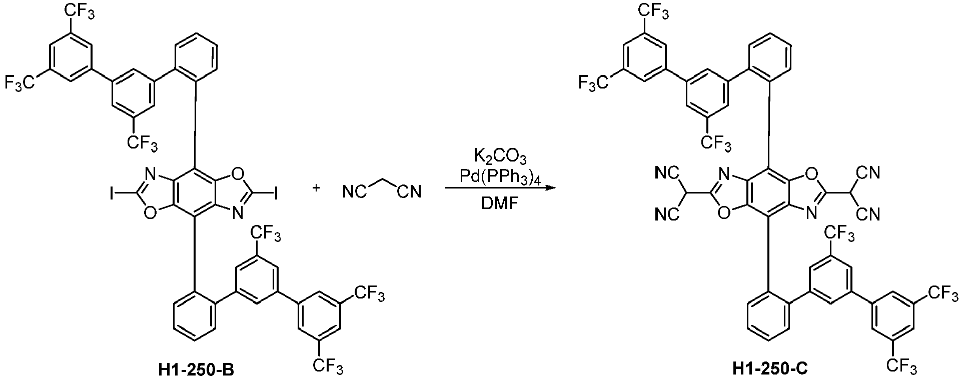

In a 2 L two-necked flask, Intermediate H1-250-B (40 g, 31.34 mmol), malononitrile (8.28 g, 125.36 mmol), potassium carbonate (26 g, 188.04 mmol) and Pd(PPh₃)₄ (0.724 g, 0.627 mmol) were added in sequence, added with DMF (500 mL) under nitrogen protection, heated to 80 °C and reacted overnight. After the reaction was completed, the system was cooled to room temperature, cooled in an ice water bath, concentrated hydrochloric acid was slowly added dropwise, and 600 mL of water was added dropwise to precipitate a large amount of yellow solids. The solids were filtered to obtain a crude product, and the crude product was crystallized to obtain Intermediate H1-250-C as a white solid (33 g, with a yield of 91%).

### Step 4: Synthesis of Compound H1-250

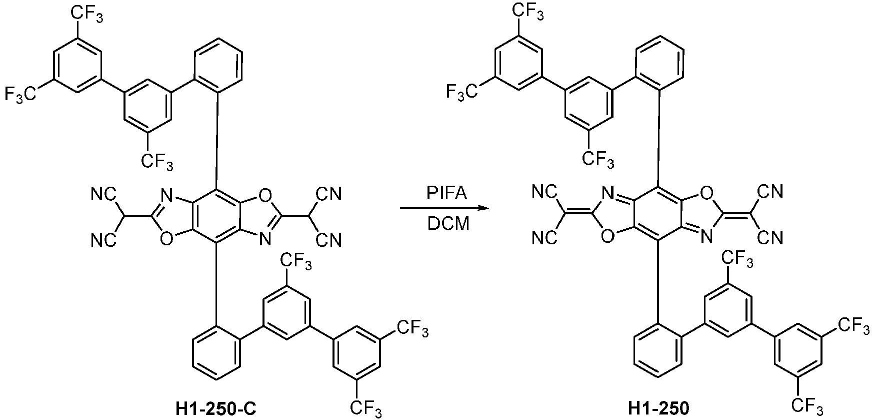

Under a nitrogen atmosphere, Intermediate H1-250-C (33 g, 28.63 mmol) was added in a 3 L single-necked flask, 2 L of DCM was added, and bis(trifluoroacetoxy)iodobenzene (24.6 g, 57.26 mmol) was added portion-wise at room temperature. The reaction solution was purple black after stirred at room temperature for 3 days. The reaction solution was concentrated to about 300 mL and filtered to obtain Compound H1-250 as a black-gold solid (21.8 g, with a yield of 66%). The product was confirmed as the target product with a molecular weight of 1150.1.

### Synthesis Example 11: Synthesis of Compound H1-1455

### Step 1: Synthesis of Intermediate H1-1455-A

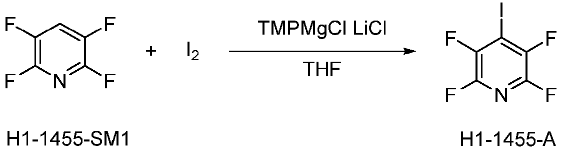

Under a nitrogen atmosphere, 2,3,5,6-tetrafluoropyridine (H1-1455-SM1) (30.2 g, 200 mmol) and THF (800 mL) were added in sequence to a 2 L two-necked flask and cooled to - 60 °C. A solution of 2,2,6,6-tetramethylpiperidylmagnesium chloride lithium chloride complex in tetrahydrofuran (TMPMgCl LiCl, 240 mL, 240 mmol) was slowly added dropwise, heated to -45 °C and stirred for 1 h at -45 °C. Iodine (71.12 g, 280 mmol) was added, slowly heated to room temperature and stirred overnight. After GCMS showed that the reaction was completed, the reaction was quenched with a saturated aqueous solution of ammonium chloride, extracted three times with ethyl acetate, concentrated and purified through column chromatography to obtain Intermediate H1-1455-A as a white solid (26 g, with a yield of 47.3%).

### Step 2: Synthesis of Intermediate H1-1455-B

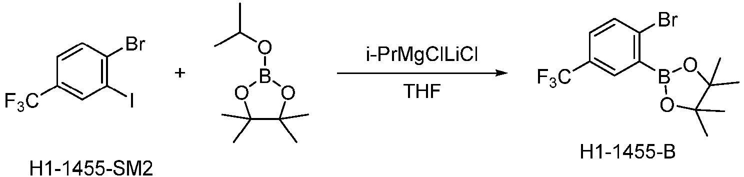

Under a nitrogen atmosphere, Intermediate H1-1455-SM2 (75 g, 214 mmol), isopropoxyboronic acid pinacol ester (iPrOBpin) (47.8 g, 257 mmol) and THF (800 mL) were added in sequence to a 2 L two-necked flask and cooled to 0 °C. A solution of isopropylmagnesium chloride lithium chloride complex in tetrahydrofuran (i-PrMgCl·LiCl, 120 mL, 156 mmol) was slowly added dropwise, heated to room temperature and stirred for 2 h at room temperature. After TLC showed that the reaction was completed, the reaction solution was poured into dilute hydrochloric acid, extracted twice with DCM and AcOEt respectively, concentrated and purified through column chromatography to obtain oily Intermediate H1-1455-B (60 g, with a yield of 80%).

### Step 3: Synthesis of Intermediate H1-1455-C

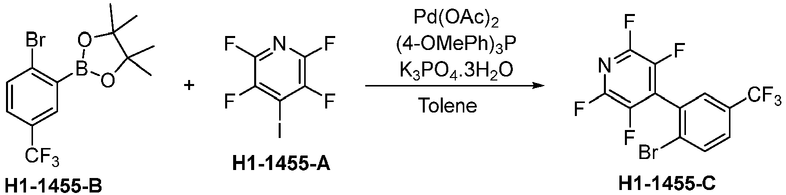

Under a nitrogen atmosphere, toluene (1000 mL), Pd(OAc)₂ (413 mg, 1.83 mmol), (4-OMePh)₃P (1.93 g, 5.49 mmol), Intermediate H1-1455-B (41.8 g, 119.2 mmol), Intermediate H1-1455-A (25.4 g, 91.7 mmol) and tripotassium phosphate trihydrate (48.8 g, 183 mmol) were added in sequence to a 2 L two-necked flask, heated to 100 °C and stirred for 15 h at 100 °C. After GCMS showed that the reaction was completed, the reaction solution was cooled to room temperature and filtered through Celite, the filter cake was fully washed with dichloromethane, and the filtrate was concentrated and purified through column chromatography to obtain oily Intermediate H1-1455-C (29.1 g, with a yield of 85%).

### Step 4: Synthesis of Intermediate H1-1455-D

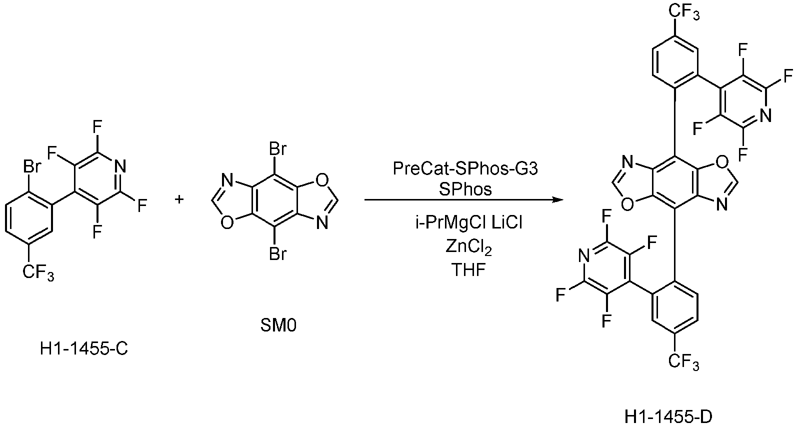

Under a nitrogen atmosphere, Intermediate H1-1455-C (15.5 g, 41.4 mmol) and THF (500 mL) were added to a 1 L two-necked reaction flask and cooled to -10 °C. A solution of isopropylmagnesium chloride lithium chloride in tetrahydrofuran (31.85 mL, 41.4 mmol) was added dropwise to the reaction solution, restored to room temperature and reacted for 3 h at room temperature. A solution of zinc chloride in 2-methyltetrahydrofuran (22.75 mL, 45.5 mmol) was slowly added dropwise to the reaction solution and reacted for 2 h at room temperature. Intermediate SM0 (3.18 g, 10 mmol), (2-dicyclohexylphosphino-2',6'-dimethoxy-1,1'-biphenyl)(2'-amino-1,1'-biphen-2-yl)palladium(II) methanesulfonate (PreCat-Sphos-G3, SPhos Pd G3, CAS No.: 1445085-82-4, 155 mg, 0.2 mmol) and SPhos (164 mg, 0.4 mmol) were added to the reaction solution, heated to 48 °C and stirred overnight at this temperature. After TLC showed that the reaction was completed, the reaction was cooled to room temperature, quenched with saturated ammonium chloride, dried over sodium sulfate, filtered through Celite, concentrated and purified through column chromatography to obtain Intermediate H1-1455-D as a white solid (6.19 g, with a yield of 83%).

### Step 5: Synthesis of Intermediate H1-1455-E

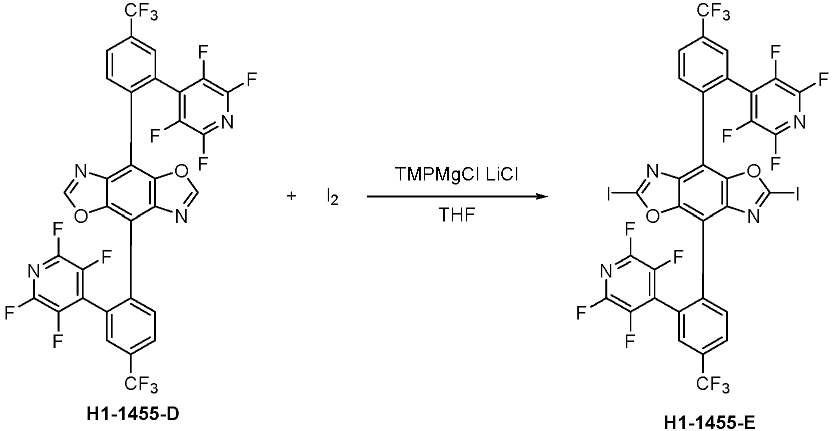

Under a nitrogen atmosphere, Intermediate H1-1455-D (6 g, 8.04 mmol) and THF (212 mL) were added to a 1 L two-necked flask and cooled to -60 °C. A solution of 2,2,6,6-tetramethylpiperidylmagnesium chloride lithium chloride complex in tetrahydrofuran (48 mL, 48 mmol) was slowly added dropwise and stirred for 5 min at -60 °C. Iodine (20 g, 80 mmol) was added, slowly heated to room temperature and stirred for 1 h at room temperature. The reaction was quenched with a small amount of a saturated aqueous solution of ammonium chloride, dried over anhydrous MgSO₄, concentrated and purified through column chromatography to obtain Intermediate H1-1455-E as a white solid (5 g, with a yield of 63%).

### Step 6: Synthesis of Intermediate H1-1455-F

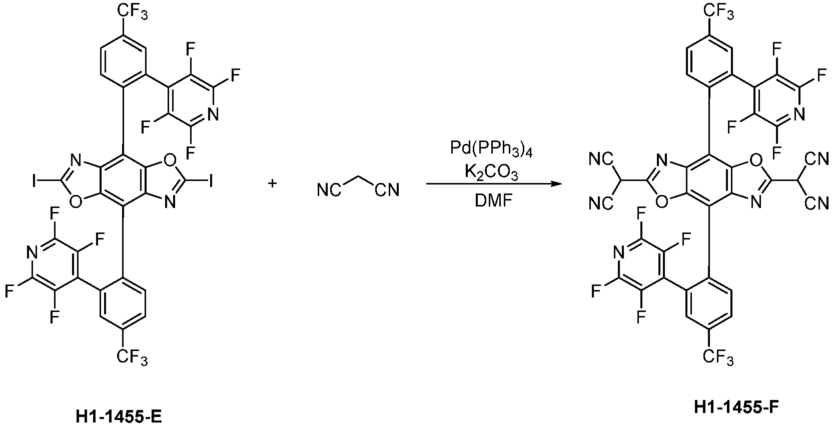

Under a nitrogen atmosphere, Intermediate H1-1455-E (5 g, 5.01 mmol), malononitrile (677 mg, 10.27 mmol), potassium carbonate (2.07 g, 15 mmol), anhydrous DMF (100 mL) and Pd(PPh₄)₃ (174 mg, 0.15 mmol) were added in sequence to a 250 mL two-necked flask and reacted overnight at room temperature. After HPLC showed that the reaction was completed, the reaction solution was poured into ice water, adjusted with dilute hydrochloric acid to pH = 1, fully stirred and filtered to obtain a yellow solid. The solid was washed with acetonitrile to obtain Intermediate H1-1455-F as a white solid (3.8 g, with a yield of 87%).

### Step 7: Synthesis of Compound H1-1455

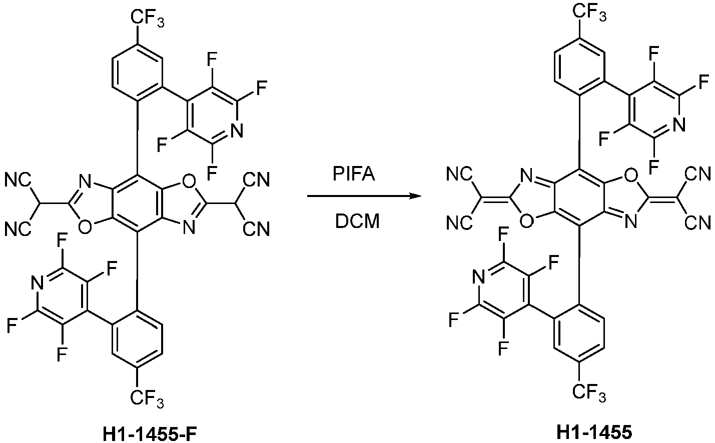

Under a nitrogen atmosphere, Intermediate H1-1455-F (3.8 g, 4.3 mmol) and DCM (1.5 L) were added to a 3 L single-necked flask and cooled to 0 °C. PIFA (3.7 g, 8.6 mmol) was added and stirred at room temperature for 1 day. The reaction solution was concentrated to about 50 mL, added with n-hexane (450 mL), stirred for 10 min and filtered to obtain a dark purple solid. The solid was washed with DCM to obtain Compound H1-1455 as a dark purple solid (3.2 g, with a yield of 85.3%). The product was confirmed as the target product with a molecular weight of 872.0.

### Synthesis Example 12: Synthesis of Compound H8-281

### Step 1: Synthesis of Intermediate H8-281-A

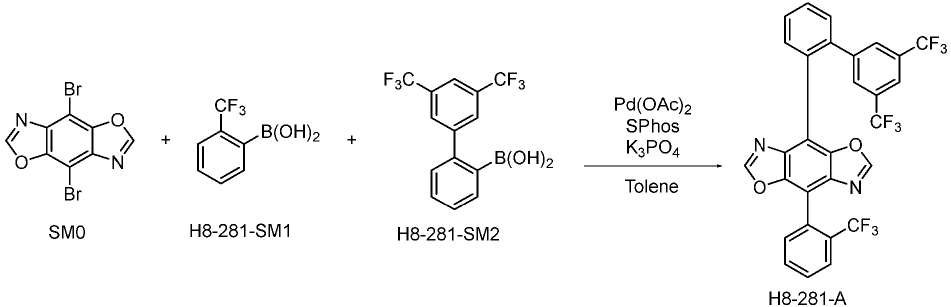

Under a N₂ atmosphere, SM0 (11.9 g, 37.4 mmol), toluene (900 mL), Compound H8-281-SM1 (14.2 g, 74.9 mmol), Compound H8-281-SM2 (25 g, 74.9 mmol), K₃PO₄ (31.7 g, 149.7 mmol), Pd(OAc)₂ (89 mg, 0.4 mmol) and SPhos (0.81 g, 2.0 mmol) were added in sequence to a 2 L two-necked flask, heated to 80 °C and reacted for 15 h at this temperature. After GCMS showed that the reaction was completed, the reaction solution was cooled to room temperature, filtered through Celite, concentrated and purified through column chromatography to obtain Intermediate H8-281-A as a white solid (8 g, with a yield of 36%).

### Step 2: Synthesis of Intermediate H8-281-B

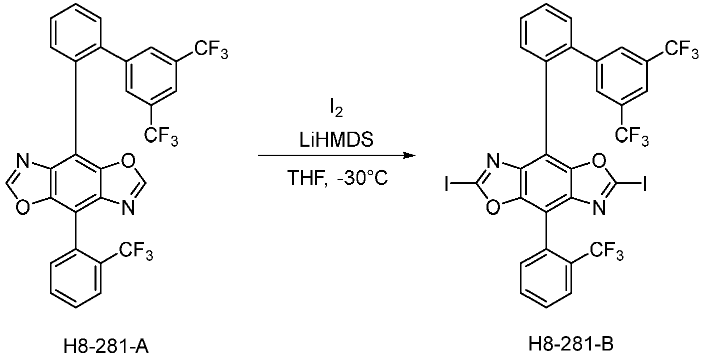

Under a N₂ atmosphere, Intermediate H8-281-A (8 g, 13.5 mmol) and THF (640 mL) were added to a 2 L two-necked flask and cooled to -30 °C. LiHMDS (34 mL, 34 mmol) was added dropwise and stirred for 1 h at -30 °C. Iodine (13.7 g, 54 mmol) was added, restored to room temperature and stirred for 1 h at room temperature. After TLC showed that the reaction was completed, the reaction was quenched with an aqueous solution of sodium sulfite, extracted once with DCM, concentrated to dryness to obtain a red solid and washed with acetonitrile to obtain Intermediate H8-281-B as a white solid (11.4 g, with a yield of 100%).

### Step 3: Synthesis of Intermediate H8-281-C

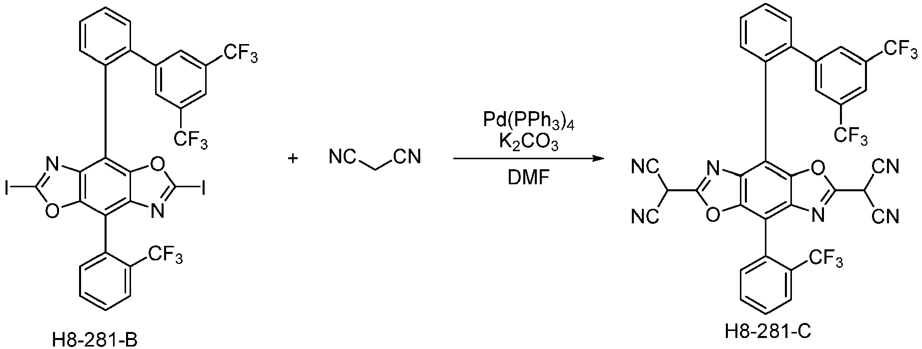

Under a N₂ atmosphere, Intermediate H8-281-B (11.4 g, 13.5 mmol), malononitrile (5.4 g, 81 mmol), Pd(PPh₃)₄ (0.34 g, 0.3 mmol), potassium carbonate (12.4 g, 90.2 mmol) and DMF (360 mL) were added in sequence to a 1 L two-necked flask, heated to 70 °C and stirred for 15 h at this temperature. After HPLC showed that the reaction was completed, the reaction system was cooled to room temperature, and the reaction solution was poured into dilute hydrochloric acid, filtered to obtain a yellow solid and washed with MeCN to obtain Intermediate H8-281-C as a white solid (9.8 g, with a yield of 100%).

### Step 4: Synthesis of Compound H8-281

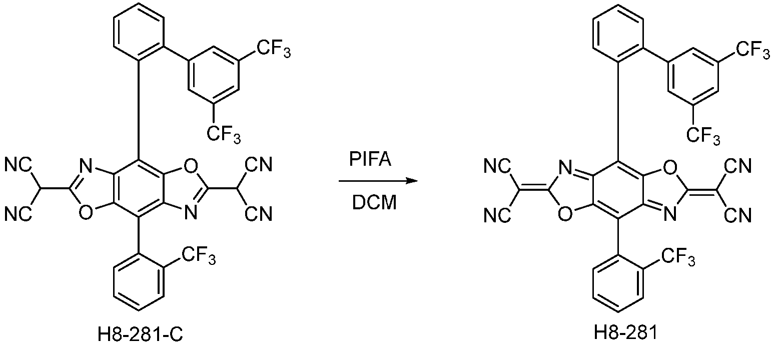

Under a N₂ atmosphere, DCM (1.5 L) and Intermediate H8-281-C (9.8 g, 13.6 mmol) were added to a 2 L two-necked flask. PIFA (11.7 g, 27.2 mmol) was added portion-wise and stirred at room temperature for 3 days. The reaction solution was concentrated to about 100 mL, added with 100 mL of heptane and filtered to obtain Compound H8-281 as a dark green solid (6.3 g, with a yield of 64%). The product was confirmed as the target product with a molecular weight of718.1.

### Synthesis Example 13: Synthesis of Compound H8-389

### Step 1: Synthesis of Intermediate H8-389-A

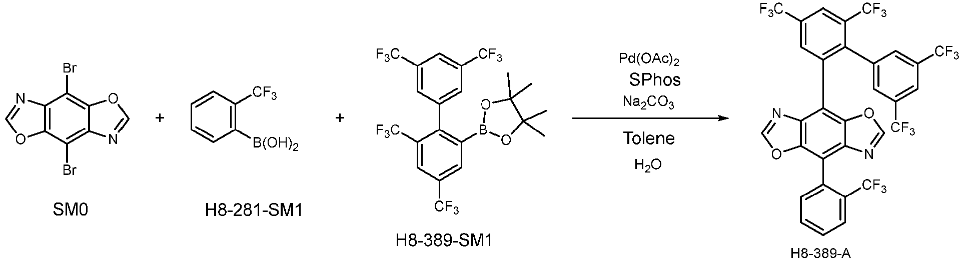

Under a N₂ atmosphere, Intermediate SM0 (10 g, 31.4 mmol), toluene (256 mL), H₂O (64 mL), Intermediate H8-281-SM1 (8.9 g, 47.2 mmol), Intermediate H8-389-SM1 (25 g, 74.9 mmol), Na₂CO₃ (13.3 g, 125.8 mmol), Pd(OAc)₂ (140 mg, 0.63 mmol) and SPhos (1.3 g, 3.1 mmol) were added in sequence to a 1 L two-necked flask, heated to 80 °C and reacted for 15 h at this temperature. After GCMS showed that the reaction was completed, the reaction solution was cooled to room temperature and stood still to separate layers. The organic phase was concentrated to dryness and purified through column chromatography to obtain Intermediate H8-389-A as a white solid (6.4 g, with a yield of 27.9%).

### Step 2: Synthesis of Intermediate H8-389-B

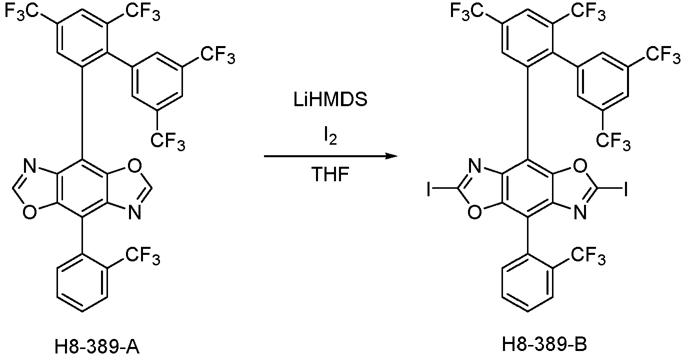

Under a N₂ atmosphere, Intermediate H8-389-A (6.1 g, 8.4 mmol) and THF (85 mL) were added to a 500 mL two-necked flask and cooled to -30 °C. LiHMDS (21 mL, 29.4 mmol) was added dropwise and stirred for 1 h at -30 °C. Iodine (8.5 g, 33.5 mmol) was added, restored to room temperature and stirred for 1 h at room temperature. After TLC showed that the reaction was completed, the reaction was quenched with an aqueous solution of sodium sulfite and extracted once with DCM. Organic phases were combined, concentrated to obtain a red solid and washed with acetonitrile to obtain Intermediate H8-389-B as a pale yellow solid (7.4 g, with a yield of 90.5%).

### Step 3: Synthesis of Intermediate H8-389-C

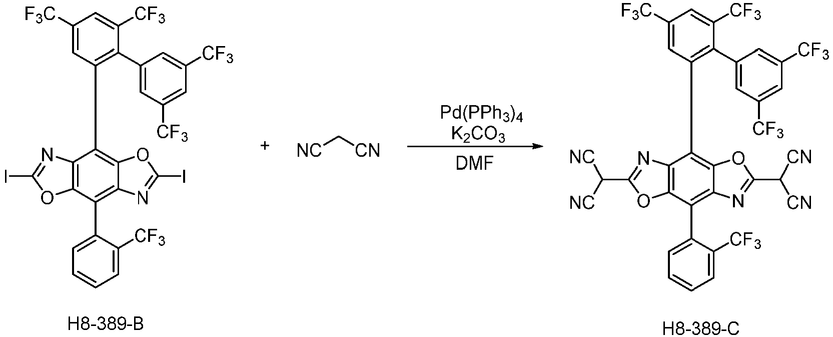

Under a N₂ atmosphere, Intermediate H8-389-B (7.4 g, 7.6 mmol), malononitrile (3 g, 45.3 mmol), Pd(PPh₃)₄ (0.18 g, 0.15 mmol), potassium carbonate (6.3 g, 45.3 mmol) and DMF (130 mL) were added in sequence to a 500 mL two-necked flask, heated to 70 °C and stirred for 15 h at this temperature. After HPLC showed that the reaction was completed, the reaction system was cooled to room temperature, and the reaction solution was poured into dilute hydrochloric acid, filtered to obtain a yellow solid and washed with acetonitrile to obtain Intermediate H8-389-C as a white solid (5.5 g, with a yield of 84%).

### Step 4: Synthesis of Compound H8-389

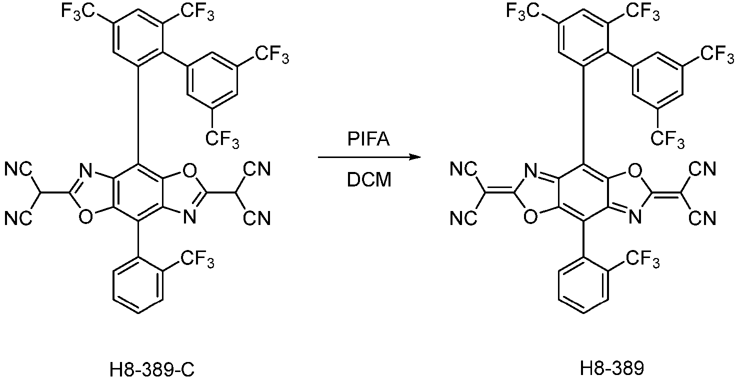

Under a N₂ atmosphere, DCM (600 mL) and Intermediate H8-389-C (5.5 g, 6.4 mmol) were added to a 2 L two-necked flask. PIFA (5.5 g, 12.8 mmol) was added portion-wise and stirred at room temperature for 3 days. The reaction solution was concentrated to 30 mL and filtered to obtain Compound H8-389 as a dark green solid (3.9 g, with a yield of 70.9%). The product was confirmed as the target product with a molecular weight of 854.1.

### Synthesis Example 14: Synthesis of Compound H8-551

### Step 1: Synthesis of Intermediate H8-551-A

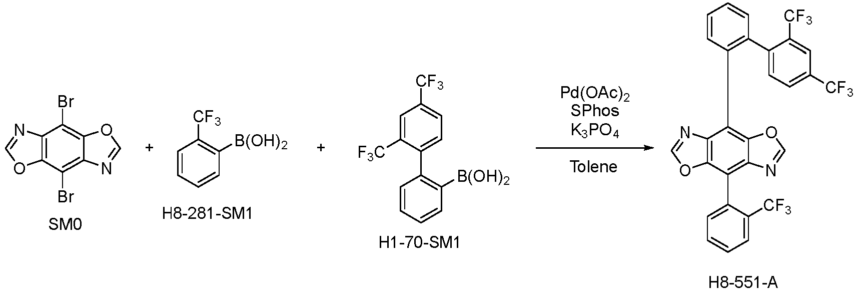

Under a N₂ atmosphere, SM0 (11.9 g, 37.4 mmol), toluene (900 mL), Intermediate H8-281-SM1 (14.2 g, 74.9 mmol), Intermediate H1-70-SM1 (25 g, 74.9 mmol), K₃PO₄ (31.7 g, 149.7 mmol), Pd(OAc)₂ (89 mg, 0.4 mmol) and SPhos (0.81 g, 2.0 mmol) were added in sequence to a 2 L two-necked flask, heated to 80 °C and reacted for 15 h at this temperature. After GCMS showed that the reaction was completed, the reaction solution was cooled to room temperature and filtered through Celite, the filter cake was fully washed with DCM, and the filtrate was concentrated and purified through column chromatography to obtain Intermediate H8-551-A as a white solid (5 g, with a yield of 23%).

### Step 2: Synthesis of Intermediate H8-551-B

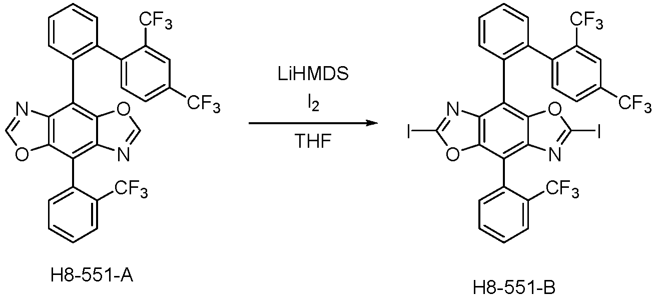

Under a N₂ atmosphere, Intermediate H8-551-A (5 g, 8.44 mmol) and THF (150 mL) were added to a 500 mL two-necked flask and cooled to -30 °C. LiHMDS (21 mL, 21 mmol) was added dropwise and stirred for 1 h at -30 °C. Iodine (8.6 g, 33.8 mmol) was added, restored to room temperature and stirred for 1 h at room temperature. After TLC showed that the reaction was completed, the reaction was quenched with an aqueous solution of sodium sulfite and extracted once with DCM, and the organic phase was concentrated to dryness to obtain a red solid and washed with acetonitrile to obtain Intermediate H8-551-B as a white solid (6 g, with a yield of 84%).

### Step 3: Synthesis of Intermediate H8-551-C

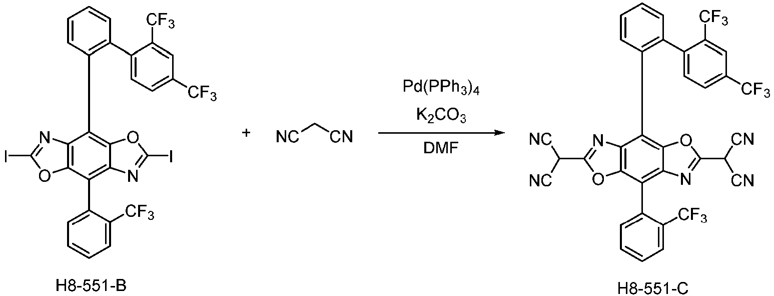

Under a N₂ atmosphere, Intermediate H8-551-B (6.0 g, 7.1 mmol), malononitrile (2.8 g, 42.7 mmol), Pd(PPh₃)₄ (0.16 g, 0.14 mmol), potassium carbonate (5.9 g, 42.7 mmol) and DMF (360 mL) were added in sequence to a 1 L two-necked flask, heated to 70 °C and stirred for 15 h at this temperature. After HPLC showed that the reaction was completed, the reaction system was cooled to room temperature, poured into dilute hydrochloric acid, filtered to obtain a yellow solid and washed with acetonitrile to obtain Intermediate H8-551-C as a white solid (3 g, with a yield of 56.3%).

### Step 4: Synthesis of Compound H8-551

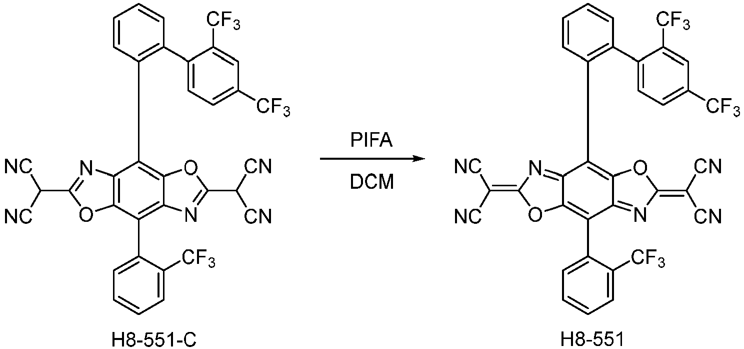

Under a N₂ atmosphere, DCM (1.5 L) and Intermediate H8-551-C (3 g, 4.0 mmol) were added to a 2 L two-necked flask. PIFA (3.5 g, 8 mmol) was added portion-wise and stirred at room temperature for 3 days. The reaction solution was concentrated to 30 mL and filtered to obtain Compound H8-551 as a dark green solid (1.5 g, with a yield of 50%). The product was confirmed as the target product with a molecular weight of 718.1.

Those skilled in the art will appreciate that the above preparation methods are merely exemplary. Those skilled in the art can obtain other compound structures of the present disclosure through the modifications of the preparation methods.

### Sublimation experiment

To verify the excellent characteristic of a high sublimation yield of the compound of the present disclosure, Compounds H1-1518, H1-428, H1-68, H1-70, H1-22, H1-20, H1-32, H1-258, H1-194, H1-250, H1-1455, H8-281, H8-389 and H8-551 of the present disclosure and Comparative Compounds PD-1 to PD-5 having similar structures were selected for the sublimation experiment.

In the sublimation experiment, an apophorometer (model No. BOF-A-3-100) purchased from Anhui BEQ Equipment technology Co., Ltd. was uniformly used, with rated power of 11 kW, a highest heating temperature of 800 °C and a K-type thermocouple. All the compounds of the present application and the comparative compounds were sublimated at a vacuum degree of about 2×10⁻⁴ Pa. The apophorometer included three temperature zones: a first temperature zone, which was a heating zone where containers holding materials to be sublimated were placed and which was used for heating the materials in the containers to sublimate the materials; a second temperature zone, which was a deposition zone, where the temperature was set to be about 60-80 °C lower than that of the first temperature zone; and a third temperature zone, which was a non-heating zone for collecting impurities with high volatility. Sublimation steps are as follows: a crude material with a mass of M₀ was placed in a sublimation boat and the sublimation boat was placed in the middle of the first temperature zone of the apophorometer; the vacuum system was turned on, and when the vacuum degree of the system reached about 2.0×10⁻⁴ Pa, the first temperature zone was turned on to heat until the material started sublimation, and the temperature of the first temperature zone at this moment was the sublimation temperature T; the temperature of the first temperature zone was maintained at T until the material was no longer sublimated; then the sublimation was stopped, the temperature was lowered, and all the material deposited in the second temperature zone was collected, which weighted as a mass of M₁. The sublimation yield was calculated as W, where W = M₁/M₀ × 100%.

Relevant compounds for the sublimation experiment have the following structures:

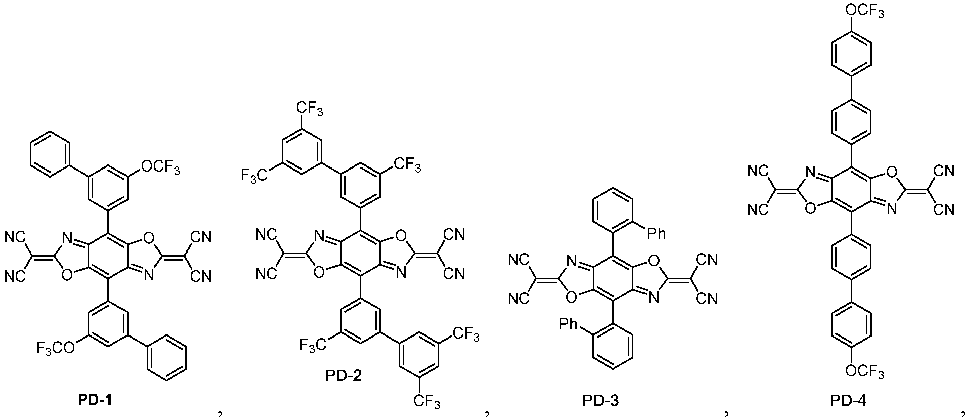

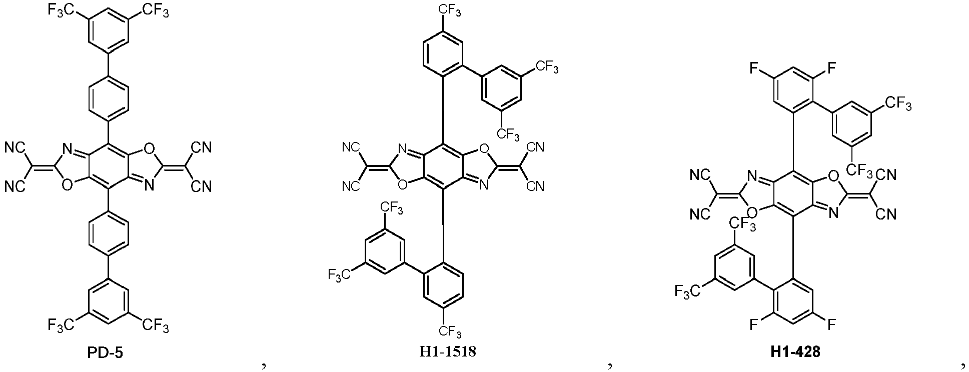

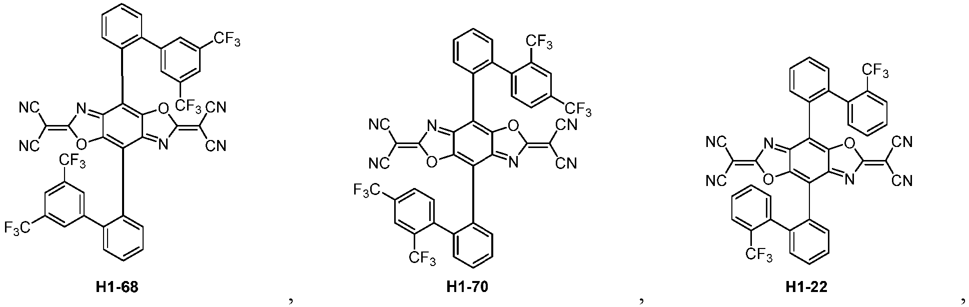

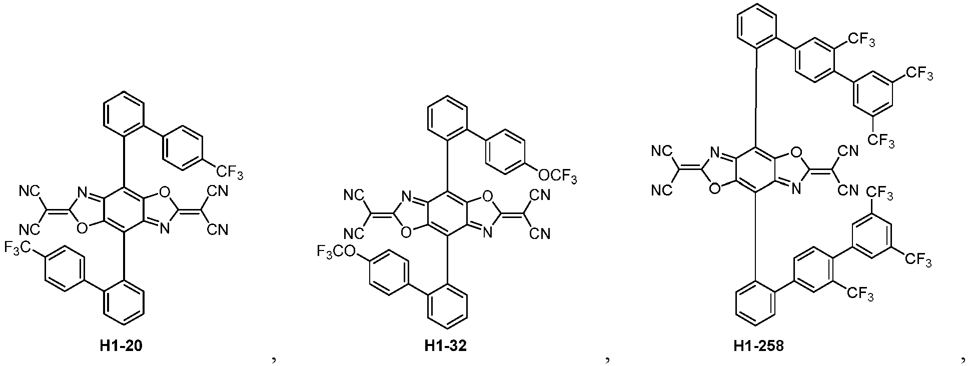

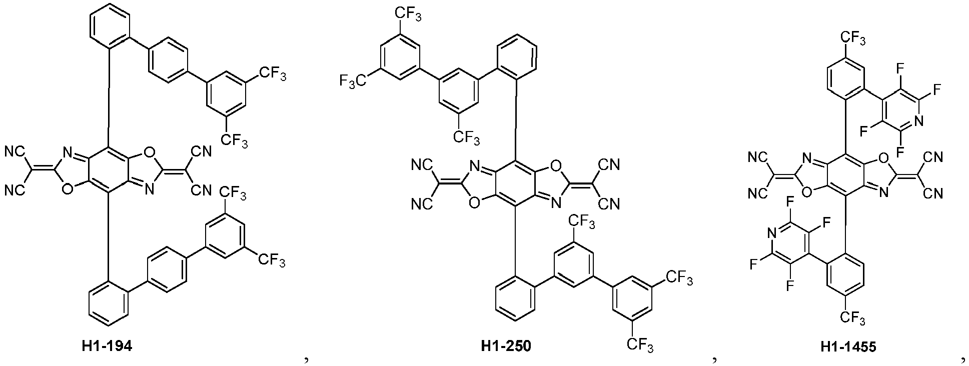

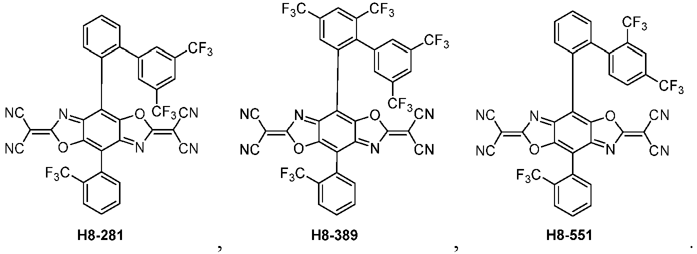

The sublimation yields, sublimation temperatures and molecular weights of the compounds of the present disclosure and the comparative compounds for the sublimation experiment are recorded and shown in Table 1.

**Table 1 Sublimation yields, sublimation temperatures and molecular weights**

| | Compound No. | Sublimation Yield (%) | Sublimation Temperature T (°C) | Molecular Weight (g/mol) |
|---|---|---|---|---|
| Comparative compounds | PD-1 | 10 | 320 | 758 |
| | PD-2 | 13 | 310 | 998 |
| | PD-3 | 10 | 300 | 590 |
| | PD-4 | 6 | 320 | 758 |
| | PD-5 | 0.8 | 310 | 862 |
| Compounds of the present disclosure | H1-1518 | 65 | 280 | 998 |
| | H1-428 | 61 | 250 | 934 |
| | H1-68 | 83 | 270 | 862 |
| | H1-70 | 80 | 280 | 862 |
| | H1-22 | 71 | 280 | 726 |
| | H1-20 | 87 | 280 | 726 |
| | H1-32 | 71 | 280 | 758 |
| | H1-258 | 72 | 300 | 1150 |
| | H1-194 | 73 | 300 | 1014 |
| | H1-250 | 57 | 300 | 1150 |
| | H1-1455 | 25 | 290 | 872 |
| | H8-281 | 67 | 250 | 718 |
| | H8-389 | 73 | 270 | 854 |
| | H8-551 | 73 | 270 | 718 |

As can be seen from the data in Table 1, the sublimation yields of the compounds of the present disclosure are at unexpected high levels and are all above 25% and mostly above 50%. However, the sublimation yields of Comparative Compounds PD-1 to PD-5 are all relatively low, the highest sublimation yield is only 13%, some sublimation yield is even lower than 1%, and the sublimation yields are at very low levels.

Compounds H1-22, H1-20, H1-32 and H1-194 of the present disclosure and Comparative Compound PD-3 all have phenyl substituted at an ortho position (corresponding to Ar₁ in any one of Formula 2-1 to Formula 2-10) in structure and differ only in that Compounds H1-22, H1-20, H1-32 and H1-194 of the present disclosure additionally introduce substituents (-CF₃, -OCF₃, 3,5-bis(trifluoromethyl)phenyl) into the phenyl substituted at the ortho position. As can be seen from the data in Table 1, Compounds H1-22, H1-20, H1-32 and H1-194 of the present disclosure having higher molecular weights all have sublimation yields which are more than 7 times higher than that of Comparative Compound PD-3 due to the additionally introduced substituents.

Compound H1-32 of the present disclosure and Comparative Compound PD-4 differ in structure only in that 4-trifluoromethoxyphenyl is substituted at an ortho or para position. However, as can be seen from the data in Table 1, the sublimation yield of Compound H1-32 of the present disclosure is more than 10 times higher than that of PD-4. Compound H1-68 of the present disclosure and Comparative Compound PD-5 differ in structure only in that 3,5-bis(trifluoromethyl)phenyl is substituted at an ortho or para position. However, as can be seen from the data in Table 1, the sublimation yield of Compound H1-68 of the present disclosure having a particular Ar₁ substituent substituted at the ortho position is more than 100 times higher than that of PD-5 having Ar₁ substituted at the para position.

Additionally, Compound H1-1518 of the present disclosure and Comparative Compound PD-2 differ in structure only in that substituents such as trifluoromethyl and 3,5-bis(trifluoromethyl)phenyl are substituted at different positions. However, the sublimation yield of Compound H1-1518 of the present disclosure having a particular Ar₁ substituent substituted at the ortho position is as high as 65%, which is 5 times of that of Comparative Compound PD-2. Compound H1-70 of the present disclosure and Comparative Compound PD-5 are isomers with the same parent core and differ only in that bis(trifluoromethyl)phenyl is substituted at different positions. However, the sublimation yield of Compound H1-70 of the present disclosure having a particular Ar₁ substituent substituted at the ortho position is as high as 80%, which is 100 times of that of Comparative Compound PD-5 having a similar substituent substituted at the para position. Similarly, Compound H1-32 of the present disclosure and Comparative Compound PD-1 are isomers with the same parent core and differ in molecular structure only in that substituents such as trifluoromethoxy and phenyl are substituted at different positions. However, the sublimation yield of Compound H1-32 of the present disclosure having a particular Ar₁ substituent substituted at the ortho position is as high as 71%, which is more than 7 times of that of Comparative Compound PD-1 having substituents substituted at meta positions. Compared with Comparative Compound PD-3, Compounds H1-258 and H1-250 of the present disclosure that introduce additional substituents (-CF₃, 3,5-bis(trifluoromethyl)phenyl) into the phenyl substituted at the ortho position have the same sublimation temperature and several times higher sublimation yields though their molecular weights are nearly twice that of PD-3; Compound H1-428 of the present disclosure that introduces additional substituents (-CF₃, F) has a greatly reduced sublimation temperature and a several times higher sublimation yield though its molecular weight is nearly 60% higher than that of PD-3. Compared with Comparative Compound PD-3, Compound H1-1455 of the present disclosure that introduces a heteroaryl substituent at an ortho position of the phenyl substituent has a significantly reduced sublimation temperature and a 1.5 times higher sublimation yield though its molecular weight is nearly 50% higher than that of PD-3. Additionally, compared with Comparative Compound PD-3, Compounds H8-281, H8-389 and H8-551 of the present disclosure, each of which has an upper and lower asymmetric structure, introduce more substituents into biphenyl at the ortho position on one side and replace the phenyl substituted at the ortho position with trifluoromethyl on the other side, and they each have a greatly reduced sublimation temperature and a several times higher sublimation yield though their molecular weights are at least 20% higher than that of PD-3.

Generally, isomers and analogs with the same parent core structure and different substituents tend to have the same or similar physical characteristics. However, the compounds of the present disclosure particularly select the substituent Ar₁ substituted at the ortho position and additionally introduce R₁ and/or R₂ substituents that are not hydrogen or deuterium so as to obtain sublimation yields that are several times higher than that of the comparative compound having the same parent core structure. Such huge improvements are unexpected.

To conclude, the compounds of the present disclosure particularly select the group Ar₁ fixedly substituted at the ortho position and additionally introduce R₁ and/or R₂ substituents that are not hydrogen or deuterium to obtain greatly improved sublimation yields, achieving completely unexpected excellent effects and reflecting the excellent characteristics and great application prospects of the compounds of the present disclosure.

Additionally, the compounds of the present disclosure have relatively low sublimation temperatures than the comparative compounds, which is conducive to reducing energy consumption and costs.

As can be seen from the data in Table 1, the sublimation temperatures of the compounds of the present disclosure are not higher than 300 °C, while the sublimation temperatures of the comparative compounds are not lower than 300 °C. It can be found through the detailed comparison of the sublimation temperatures of the compounds of the present disclosure and the comparative compounds measured under the same conditions that the sublimation temperature of Compound H1-1518 of the present disclosure is 280 °C, which is 30 °C lower than that of Comparative Compound PD-2, an isomer having the same number of substituents, the same types of substituent and the same parent core; the sublimation temperature of Compound H1-32 of the present disclosure is 280 °C, which is 40 °C lower than those of PD-1 and PD-4, isomers having the same parent core; similarly, the sublimation temperatures of Compounds H1-68 and H1-70 of the present disclosure are 270 °C and 280 °C, which are 40 °C and 30 °C lower than that of PD-5, an isomer having the same parent core, respectively. It can be seen that the compounds of the present disclosure particularly select the group Ar₁ fixedly substituted at the ortho position and additionally introduce R₁ and/or R₂ substituents that are not hydrogen or deuterium to obtain greatly reduced sublimation temperatures (which are more than 30 °C lower than those of the comparative compounds on average), which can significantly reduce the energy consumption and costs in the industrial production of OLEDs on a large scale and proves again that the compounds of the present disclosure have unexpected excellent characteristics and huge application prospects.

The compounds of the present disclosure have not only the unexpected excellent characteristics of high sublimation yields but also excellent device performance. The excellent performance of the compounds of the present disclosure in devices is verified below through device examples.

### Device Example

### Example 1

Firstly, a glass substrate having a thickness of 0.7 mm, on which an indium tin oxide (ITO) anode with a thickness of 800 Å had been patterned, was washed with deionized water and a detergent, and then the ITO surface was treated with oxygen plasma and UV ozone. Then, the substrate was dried in a glovebox to remove moisture, mounted on a support and transferred into a vacuum chamber. The organic layers specified below were sequentially deposited on the anode layer through vacuum thermal evaporation at a rate of 0.01-10 Å/s and at a vacuum degree of about 10⁻⁶ torr. Compound HT and Compound H1-1518 of the present disclosure were co-deposited for use as a hole injection layer (HIL, 97:3, 100 Å). Compound HT was deposited for use as a hole transporting layer (HTL, 1650 Å). Compound EB was deposited for use as an electron blocking layer (EBL, 50 Å). On the EBL, Compound BH and Compound BD were co-deposited for use as an emissive layer (EML, 96:4, 250 Å). Compound HB was deposited for use as a hole blocking layer (HBL, 50 Å). On the HBL, Compound ET and Liq were co-deposited for use as an electron transporting layer (ETL, 40:60, 300 Å). Liq was deposited for use as an electron injection layer (EIL) with a thickness of 10 Å. Finally, a metal aluminum was deposited for use as a cathode (1200 Å). The device was transferred back to the glovebox and encapsulated with a glass lid to complete the device.

Example 2: This example was prepared by the same method as Example 1 except that Compound HT and Compound H1-68 of the present disclosure were deposited for use as a hole injection layer (HIL, 97:3, 100 Å).

Example 3: This example was prepared by the same method as Example 1 except that Compound HT and Compound H1-70 of the present disclosure were deposited for use as a hole injection layer (HIL, 97:3, 100 Å).

Example 4: This example was prepared by the same method as Example 1 except that Compound HT and Compound H1-32 of the present disclosure were deposited for use as a hole injection layer (HIL, 97:3, 100 Å).

Example 5: This example was prepared by the same method as Example 1 except that Compound HT and Compound H1-428 of the present disclosure were deposited for use as a hole injection layer (HIL, 97:3, 100 Å).

Example 6: This example was prepared by the same method as Example 1 except that Compound HT and Compound H1-20 of the present disclosure were deposited for use as a hole injection layer (HIL, 97:3, 100 Å).

Example 7: This example was prepared by the same method as Example 1 except that Compound HT and Compound H1-258 of the present disclosure were deposited for use as a hole injection layer (HIL, 97:3, 100 Å).

Example 8: This example was prepared by the same method as Example 1 except that Compound HT and Compound H1-250 of the present disclosure were deposited for use as a hole injection layer (HIL, 97:3, 100 Å) and Compound BH-1 and Compound BD were co-deposited for use as an emissive layer (EML, 96:4, 250 Å).

Example 9: This example was prepared by the same method as Example 1 except that Compound HT and Compound H1-1455 of the present disclosure were deposited for use as a hole injection layer (HIL, 97:3, 100 Å) and Compound BH-1 and Compound BD were co-deposited for use as an emissive layer (EML, 96:4, 250 Å).

Example 10: This example was prepared by the same method as Example 1 except that Compound HT and Compound H8-281 of the present disclosure were deposited for use as a hole injection layer (HIL, 97:3, 100 Å) and Compound BH-1 and Compound BD were co-deposited for use as an emissive layer (EML, 96:4, 250 Å).

Example 11: This example was prepared by the same method as Example 1 except that Compound HT and Compound H8-389 of the present disclosure were deposited for use as a hole injection layer (HIL, 97:3, 100 Å) and Compound BH-1 and Compound BD were co-deposited for use as an emissive layer (EML, 96:4, 250 Å).

Example 12: This example was prepared by the same method as Example 1 except that Compound HT and Compound H8-551 of the present disclosure were deposited for use as a hole injection layer (HIL, 97:3, 100 Å) and Compound BH-1 and Compound BD were co-deposited for use as an emissive layer (EML, 96:4, 250 Å).

Comparative Example 1: This comparative example was prepared by the same method as Example 1 except that Compound HT and Compound PD-2 were deposited for use as a hole injection layer (HIL, 97:3, 100 Å).

Comparative Example 2: This comparative example was prepared by the same method as Example 1 except that Compound HT and Compound PD-5 were deposited for use as a hole injection layer (HIL, 97:3, 100 Å).

Comparative Example 3: This comparative example was prepared by the same method as Example 1 except that Compound HT and Compound PD-1 were deposited for use as a hole injection layer (HIL, 97:3, 100 Å).

Comparative Example 4: This comparative example was prepared by the same method as Example 1 except that Compound HT and Compound PD-4 were deposited for use as a hole injection layer (HIL, 97:3, 100 Å).

A functional layer using more than one material is obtained by doping different compounds at their weight ratio as recorded. The structures and thicknesses of HILs of the devices are shown in the following table.

**Table 2 HIL structures of device examples and comparative examples**

| Device No. | HIL | EML |
|---|---|---|
| Example 1 | Compound H1-1518:Compound HT (3:97) (100 Å) | Compound BH:Compound BD (96:4) (250 Å) |
| Example 2 | Compound H1-68:Compound HT (3:97) (100 Å) | Compound BH:Compound BD (96:4) (250 Å) |
| Example 3 | Compound H1-70:Compound HT (3:97) (100 Å) | Compound BH:Compound BD (96:4) (250 Å) |
| Example 4 | Compound H1-32:Compound HT (3:97) (100 Å) | Compound BH:Compound BD (96:4) (250 Å) |
| Example 5 | Compound H1-428:Compound HT (3:97) (100 Å) | Compound BH:Compound BD (96:4) (250 Å) |
| Example 6 | Compound H1-20:Compound HT (3:97) (100 Å) | Compound BH:Compound BD (96:4) (250 Å) |
| Example 7 | Compound H1-258:Compound HT (3:97) (100 Å) | Compound BH:Compound BD (96:4) (250 Å) |
| Example 8 | Compound H1-250:Compound HT (3:97) (100 Å) | Compound BH-1:Compound BD (96:4) (250 Å) |
| Example 9 | Compound H1-1455:Compound HT (3:97) (100 Å) | Compound BH-1:Compound BD (96:4) (250 Å) |
| Example 10 | Compound H8-281:Compound HT (3:97) (100 Å) | Compound BH-1:Compound BD (96:4) (250 Å) |
| Example 11 | Compound H8-389:Compound HT (3:97) (100 Å) | Compound BH-1:Compound BD (96:4) (250 Å) |
| Example 12 | Compound H8-551:Compound HT (3:97) (100 Å) | Compound BH-1:Compound BD (96:4) (250 Å) |
| Comparative Example 1 | Compound PD-2:Compound HT (3:97) (100 Å) | Compound BH:Compound BD (96:4) (250 Å) |
| Comparative Example 2 | Compound PD-5:Compound HT (3:97) (100 Å) | Compound BH:Compound BD (96:4) (250 Å) |
| Comparative Example 3 | Compound PD-1:Compound HT (3:97) (100 Å) | Compound BH:Compound BD (96:4) (250 Å) |
| Comparative Example 4 | Compound PD-4:Compound HT (3:97) (100 Å) | Compound BH:Compound BD (96:4) (250 Å) |

The materials used in the devices have the following structures:

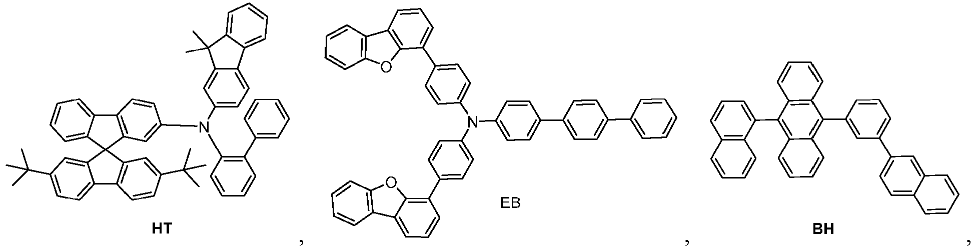

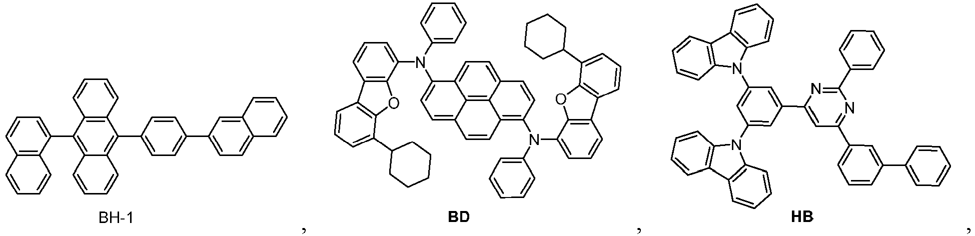

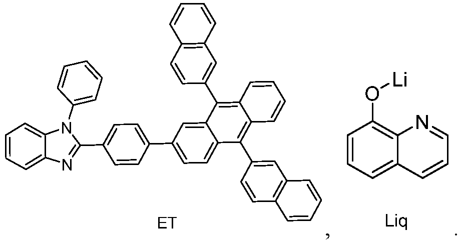

The current-voltage-luminance (IVL) characteristics of the devices were measured. Voltage, current efficiency (CE), power efficiency (PE) and external quantum efficiency (EQE) were measured at a current density of 10 mA/cm².

**Table 3 Device data of Examples 1 to 12 and Comparative Examples 1 to 4**

| Device No. | Voltage (V) | CE (cd/A) | PE (lm/W) | EQE (%) |
|---|---|---|---|---|
| Example 1 | 4.19 | 5.98 | 4.48 | 7.15 |
| Comparative Example 1 | 4.22 | 5.86 | 4.36 | 7.05 |
| Example 2 | 4.14 | 7.08 | 5.37 | 7.72 |
| Example 3 | 4.09 | 7.07 | 5.43 | 7.77 |
| Comparative Example 2 | 4.28 | 6.01 | 4.41 | 7.18 |
| Example 4 | 4.18 | 6.82 | 5.13 | 8.08 |
| Comparative Example 3 | 4.26 | 6.17 | 4.55 | 7.42 |
| Comparative Example 4 | 4.21 | 6.40 | 4.77 | 7.77 |
| Example 5 | 4.00 | 7.55 | 5.93 | 8.31 |
| Example 6 | 4.06 | 6.09 | 4.70 | 7.34 |
| Example 7 | 4.26 | 6.82 | 5.04 | 7.85 |
| Example 8 | 4.05 | 6.05 | 4.69 | 7.46 |
| Example 9 | 4.10 | 6.09 | 4.66 | 7.42 |
| Example 10 | 4.01 | 5.83 | 4.57 | 7.08 |
| Example 11 | 4.04 | 5.77 | 4.49 | 7.00 |
| Example 12 | 4.18 | 5.88 | 4.42 | 7.09 |

As can be seen from the device data in Table 3, the device performance of Comparative Examples 1 to 4 has reached a relatively high level in the industry, but Device Examples 1 to 4 using the compounds of the present disclosure have further improved device performance. Specifically, Compound H1-1518 of the present disclosure and Comparative Compound PD-2, which are used in Example 1 and Comparative Example 1 respectively, are isomers in which only substituents are connected at different positions, but Example 1 exhibits higher levels than Comparative Example 1 in terms of voltage, current efficiency (CE), power efficiency (PE) and external quantum efficiency (EQE). Similarly, the p-dopants used in Example 2 and Example 3 are Compounds H1-68 and H1-70 of the present disclosure, respectively. These compounds and Comparative Compound PD-5 used in Comparative Example 2 are isomers in which only substituents are connected at different positions, but Example 2 and Example 3 both reach higher levels than Comparative Example 2 in terms of voltage, current efficiency (CE), power efficiency (PE) and external quantum efficiency (EQE). Similarly, the p-dopants used in Example 4, Comparative Example 3 and Comparative Example 4 are Compound H1-32 of the present disclosure, Comparative Compound PD-1 and Comparative Compound PD-4 respectively, which are isomers having the same parent core, but Example 4 exhibits higher levels than Comparative Examples 3 and 4 in terms of voltage, current efficiency (CE), power efficiency (PE) and external quantum efficiency (EQE). Additionally, the HILs of the devices in Example 5 to Example 9 use Compound H1-428, Compound H1-20, Compound H1-258, Compound H1-250 and Compound H1-1455 of the present disclosure respectively, and the devices exhibit very high levels similar to those of Examples 1 to 4 in terms of voltage, current efficiency (CE), power efficiency (PE) and external quantum efficiency (EQE). Particularly, the device in Example 5 reaches extremely high levels in voltage and efficiency. It is worth noting that the HILs of the devices in Example 10, Example 11 and Example 12 use Compound H8-281, Compound H8-389 and Compound H8-551 of the present disclosure respectively, which have upper and lower asymmetric structures, and the devices also exhibit excellent performance similar to those of Examples 1 to 4 in terms of voltage, current efficiency (CE), power efficiency (PE) and external quantum efficiency (EQE). To conclude, the compounds of the present disclosure used in the examples particularly select the group Ar₁ fixedly substituted at the ortho position and additionally introduce R₁ and/or R₂ substituents that are not hydrogen or deuterium to achieve improved overall device performance including voltage, current efficiency (CE), power efficiency (PE) and external quantum efficiency (EQE). The comparison of these data fully proves that the compounds of the present disclosure have not only the unexpected excellent characteristics of high sublimation yields but also excellent device performance, which proves that the compounds of the present disclosure have excellent characteristics and huge application prospects.

Additionally, the LUMO energy levels of the compounds of the present disclosure and the comparative compounds were tested by a cyclic voltammetry method, which further verified the excellent performance of the compounds of the present disclosure.

Cyclic voltammetry (CV) tests were conducted using a CorrTest CS120 electrochemical workstation produced by WUHAN CORRTEST INSTRUMENTS CORP., LTD. A three-electrode working system was used: a platinum disk electrode served as a working electrode, a Ag/AgNO₃ electrode served as a reference electrode, and a platinum wire electrode served as an auxiliary electrode; 0.1 mol/L tetrabutylammonium hexafluorophosphate was used as a supporting electrolyte; anhydrous DCM was used as a solvent, a compound to be tested was prepared into a solution of 10⁻³ mol/L; and nitrogen was introduced into the solution for 10 min for oxygen removal before the tests. The parameters of the instrument were set as follows: a scan rate of 100 mV/s, a potential interval of 0.5 mV and a test window of 1 V to -0.5 V The related data are shown in Table 4.

**Table 4 Data on the LUMO energy levels of the compounds of the present disclosure and the comparative compounds**

| Compound No. | LUMO (eV) |
|---|---|
| H1-1518 | -5.23 |
| H1-428 | -5.26 |
| H1-68 | -5.16 |
| H1-70 | -5.09 |
| H1-22 | -5.05 |
| H1-20 | -5.08 |
| H1-32 | -5.06 |
| H1-258 | -5.13 |
| H1-194 | -5.08 |
| H1-250 | -5.17 |
| H1-1455 | -5.28 |
| H8-281 | -5.11 |
| H8-389 | -5.20 |
| H8-551 | -5.12 |
| PD-1 | -5.12 |
| PD-2 | -5.17 |
| PD-3 | -5.03 |
| PD-4 | -5.10 |
| PD-5 | -5.13 |

As can be seen from Table 4, the compounds of the present disclosure all have the characteristics of deep LUMOs (which are all less than or equal to -5.05 eV). On the one hand, the compounds of the present disclosure have strong oxidative properties similar to those of the comparative compounds, but the compounds of the present disclosure have completely unexpected high sublimation yields. On the other hand, as can be seen from the excellent device performance shown in Table 3, which are exhibited by Device Examples 1 to 12 using the compounds of the present disclosure in combination with a commercially available hole transporting material Compound HT, efficient charge transfer is generated between the compounds of the present disclosure with Compound HT. Therefore, the compounds of the present disclosure have the excellent performance of high doping efficiency and are very good charge transfer materials and p-type doping materials, which proves that the compounds of the present disclosure have excellent characteristics and huge application prospects.

To conclude, the compounds of the present disclosure have the unexpected excellent characteristics of high sublimation yields and can greatly reduce the cost of industrialization, and the compounds are very good charge transfer materials and p-type doping materials and have very broad industrial application prospects.

## Claims

1. A compound having a structure represented by any one of Formula 2-1 to Formula 2-10: 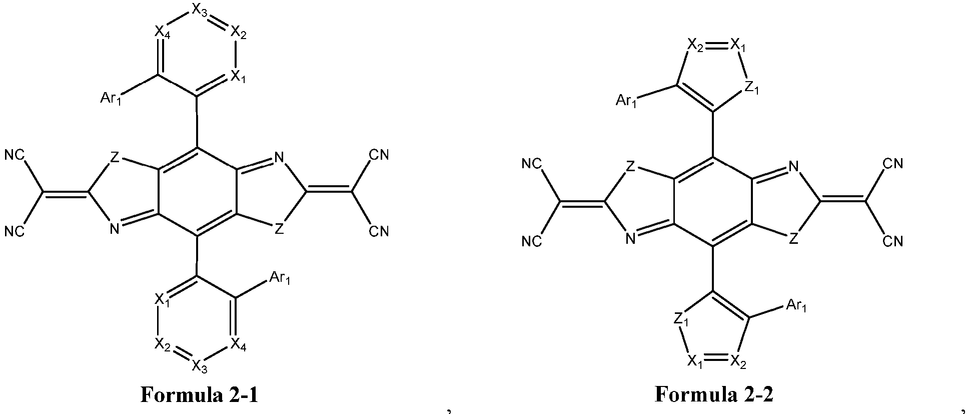 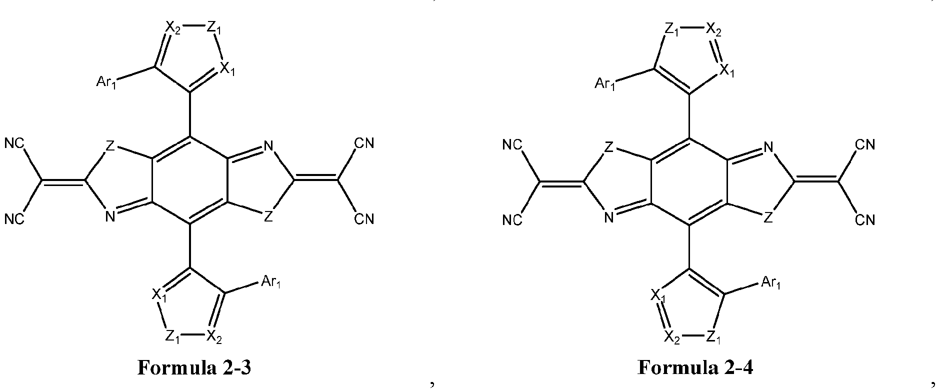 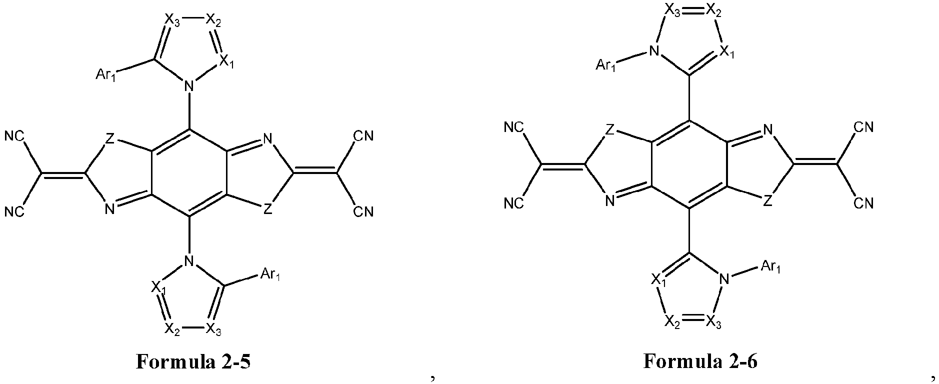 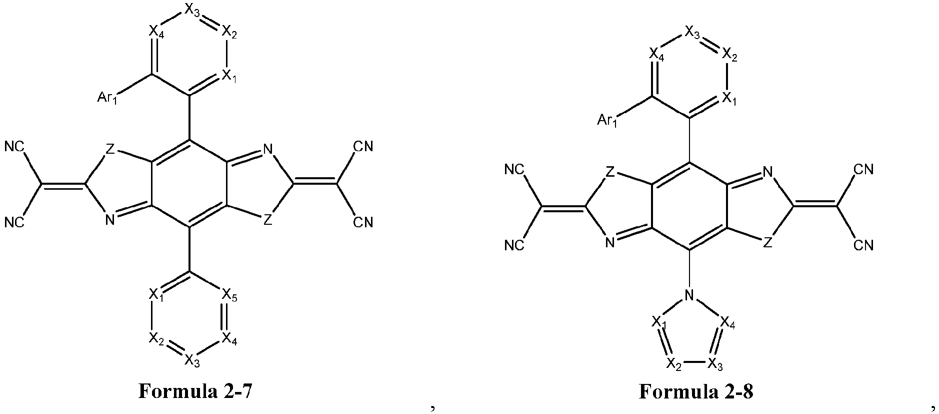 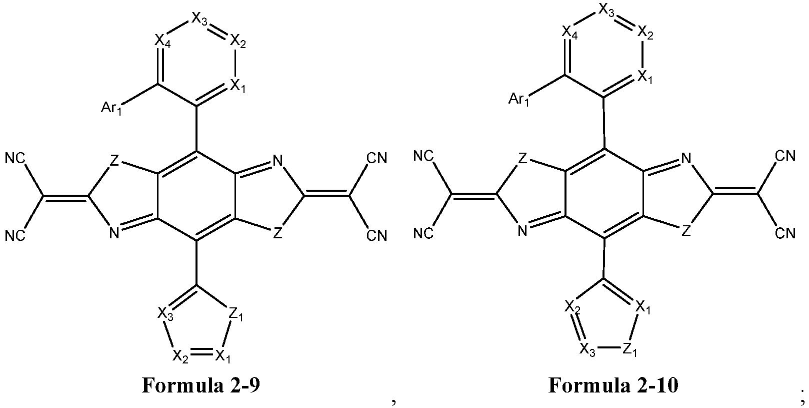
Z is, at each occurrence identically or differently, selected from O, S or Se;
X₁ to X₅ are, at each occurrence identically or differently, selected from N or CR₁;
Z₁ is, at each occurrence identically or differently, selected from O, S, Se or NR_{N1};
Ar₁ is, at each occurrence identically or differently, selected from the group consisting of: substituted or unsubstituted aryl having 6 to 20 carbon atoms, substituted or unsubstituted heteroaryl having 3 to 20 carbon atoms and combinations thereof; and when Ar₁ is selected from substituted aryl having 6 to 20 carbon atoms or substituted heteroaryl having 3 to 20 carbon atoms, the aryl or heteroaryl is substituted with one or more R₂;
Ri, R₂ and R_{N1} are, at each occurrence identically or differently, selected from the group consisting of: hydrogen, deuterium, a hydroxyl group, a sulfanyl group, halogen, a nitroso group, a nitro group, an acyl group, a carbonyl group, a carboxylic acid group, an ester group, a cyano group, an isocyano group, SCN, OCN, SF₅, a boranyl group, a sulfinyl group, a sulfonyl group, a phosphoroso group, substituted or unsubstituted alkyl having 1 to 20 carbon atoms, substituted or unsubstituted cycloalkyl having 3 to 20 ring carbon atoms, substituted or unsubstituted heteroalkyl having 1 to 20 carbon atoms, substituted or unsubstituted alkoxy having 1 to 20 carbon atoms, substituted or unsubstituted alkenyl having 2 to 20 carbon atoms, substituted or unsubstituted alkynyl having 2 to 20 carbon atoms, substituted or unsubstituted aryl having 6 to 30 carbon atoms, substituted or unsubstituted heteroaryl having 3 to 30 carbon atoms and combinations thereof;
at least one of the R₁ and R₂ is, at each occurrence identically or differently, selected from the group consisting of: a hydroxyl group, a sulfanyl group, halogen, a nitroso group, a nitro group, an acyl group, a carbonyl group, a carboxylic acid group, an ester group, a cyano group, an isocyano group, SCN, OCN, SF₅, a boranyl group, a sulfinyl group, a sulfonyl group, a phosphoroso group, substituted or unsubstituted alkyl having 1 to 20 carbon atoms, substituted or unsubstituted cycloalkyl having 3 to 20 ring carbon atoms, substituted or unsubstituted heteroalkyl having 1 to 20 carbon atoms, substituted or unsubstituted alkoxy having 1 to 20 carbon atoms, substituted or unsubstituted alkenyl having 2 to 20 carbon atoms, substituted or unsubstituted alkynyl having 2 to 20 carbon atoms, substituted or unsubstituted aryl having 6 to 30 carbon atoms, substituted or unsubstituted heteroaryl having 3 to 30 carbon atoms and combinations thereof; and
adjacent substituents Ri, R₂ can be optionally joined to form a ring.

2. The compound according to claim 1, wherein X₁ is, at each occurrence identically or differently, selected from CR₁.

3. The compound according to any one of claims 1 to 2, wherein the compound has a structure represented by Formula 3-1: 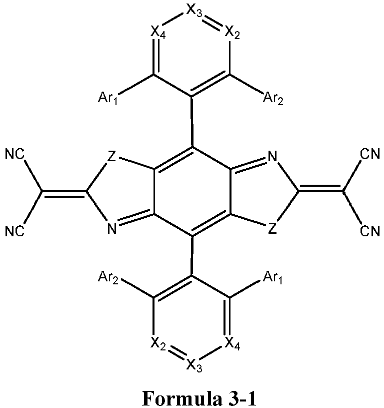 wherein in Formula 3-1,
Z is, at each occurrence identically or differently, selected from O, S or Se;
X₂ to X₄ are, at each occurrence identically or differently, selected from N or CR₁;
Ar₁ and Ar₂ are, at each occurrence identically or differently, selected from the group consisting of: substituted or unsubstituted aryl having 6 to 20 carbon atoms, substituted or unsubstituted heteroaryl having 3 to 20 carbon atoms and combinations thereof; and when Ar₁ and Ar₂ are selected from substituted aryl having 6 to 20 carbon atoms or substituted heteroaryl having 3 to 20 carbon atoms, the aryl or heteroaryl is substituted with one or more R₂;
R₁ and R₂ are, at each occurrence identically or differently, selected from the group consisting of: hydrogen, deuterium, a hydroxyl group, a sulfanyl group, halogen, a nitroso group, a nitro group, an acyl group, a carbonyl group, a carboxylic acid group, an ester group, a cyano group, an isocyano group, SCN, OCN, SF₅, a boranyl group, a sulfinyl group, a sulfonyl group, a phosphoroso group, substituted or unsubstituted alkyl having 1 to 20 carbon atoms, substituted or unsubstituted cycloalkyl having 3 to 20 ring carbon atoms, substituted or unsubstituted heteroalkyl having 1 to 20 carbon atoms, substituted or unsubstituted alkoxy having 1 to 20 carbon atoms, substituted or unsubstituted alkenyl having 2 to 20 carbon atoms, substituted or unsubstituted alkynyl having 2 to 20 carbon atoms, substituted or unsubstituted aryl having 6 to 30 carbon atoms, substituted or unsubstituted heteroaryl having 3 to 30 carbon atoms and combinations thereof;
at least one of the R₁ and R₂ is, at each occurrence identically or differently, selected from the group consisting of: a hydroxyl group, a sulfanyl group, halogen, a nitroso group, a nitro group, an acyl group, a carbonyl group, a carboxylic acid group, an ester group, a cyano group, an isocyano group, SCN, OCN, SF₅, a boranyl group, a sulfinyl group, a sulfonyl group, a phosphoroso group, substituted or unsubstituted alkyl having 1 to 20 carbon atoms, substituted or unsubstituted cycloalkyl having 3 to 20 ring carbon atoms, substituted or unsubstituted heteroalkyl having 1 to 20 carbon atoms, substituted or unsubstituted alkoxy having 1 to 20 carbon atoms, substituted or unsubstituted alkenyl having 2 to 20 carbon atoms, substituted or unsubstituted alkynyl having 2 to 20 carbon atoms, substituted or unsubstituted aryl having 6 to 30 carbon atoms, substituted or unsubstituted heteroaryl having 3 to 30 carbon atoms and combinations thereof; and
adjacent substituents Ri, R₂ can be optionally joined to form a ring.

4. The compound according to any one of claims 1 to 3, wherein Z is, at each occurrence identically or differently, selected from O or S; preferably, Z is selected from O.

5. The compound according to claim 1, wherein in Formula 2-1 to Formula 2-10, at least one of X₁ to X₅ is, at each occurrence identically or differently, selected from CR₁; preferably, X₁ to X₅ are, at each occurrence identically or differently, selected from CR₁.

6. The compound according to any one of claims 1 to 5, wherein the R₁ and R₂ are, at each occurrence identically or differently, selected from the group consisting of: hydrogen, deuterium, halogen, cyano, isocyano, SCN, OCN, SF₅, boranyl, substituted or unsubstituted alkyl having 1 to 20 carbon atoms, substituted or unsubstituted heteroalkyl having 1 to 20 carbon atoms, substituted or unsubstituted alkoxy having 1 to 20 carbon atoms, substituted or unsubstituted aryl having 6 to 30 carbon atoms, substituted or unsubstituted heteroaryl having 3 to 30 carbon atoms and combinations thereof;
at least one of the R₁ and R₂ is, at each occurrence identically or differently, selected from the group consisting of: halogen, cyano, isocyano, SCN, OCN, SF₅, boranyl, substituted or unsubstituted alkyl having 1 to 20 carbon atoms, substituted or unsubstituted heteroalkyl having 1 to 20 carbon atoms, substituted or unsubstituted alkoxy having 1 to 20 carbon atoms, substituted or unsubstituted aryl having 6 to 30 carbon atoms, substituted or unsubstituted heteroaryl having 3 to 30 carbon atoms and combinations thereof; and
adjacent substituents Ri, R₂ can be optionally joined to form a ring;
preferably, at least one of the R₁ and R₂ is, at each occurrence identically or differently, selected from the group consisting of: fluorine, SF₅, cyano, substituted alkyl having 1 to 20 carbon atoms, substituted heteroalkyl having 1 to 20 carbon atoms, substituted alkoxy having 1 to 20 carbon atoms, substituted aryl having 6 to 30 carbon atoms, substituted heteroaryl having 3 to 30 carbon atoms and combinations thereof; and a substituent in the substituted alkyl, substituted heteroalkyl, substituted alkoxy, substituted aryl or substituted heteroaryl comprises at least one electron-withdrawing group;
more preferably, at least one of the R₁ and R₂ is, at each occurrence identically or differently, selected from the group consisting of: fluorine, SF₅, cyano, substituted alkyl having 1 to 20 carbon atoms, substituted heteroalkyl having 1 to 20 carbon atoms, substituted alkoxy having 1 to 20 carbon atoms, substituted aryl having 6 to 30 carbon atoms, substituted heteroaryl having 3 to 30 carbon atoms and combinations thereof; and a substituent in the substituted alkyl, substituted heteroalkyl, substituted alkoxy, substituted aryl or substituted heteroaryl comprises at least one fluorine atom and/or at least one cyano group.

7. The compound according to any one of claims 1 to 6, wherein R₁ and R₂ are, at each occurrence identically or differently, selected from the group consisting of A1 to A19 and B1 to B314: 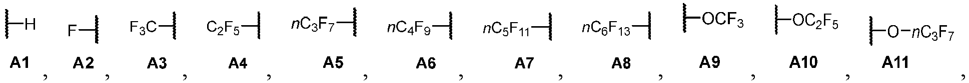 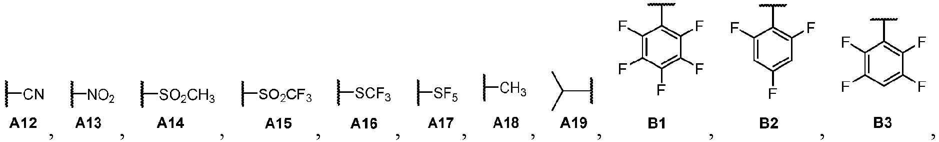 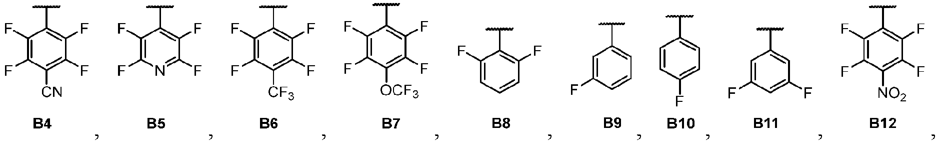 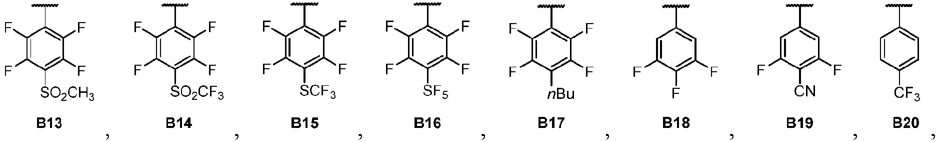 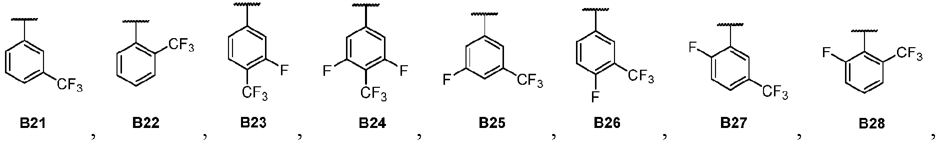 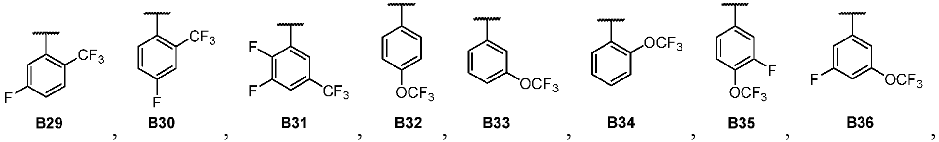 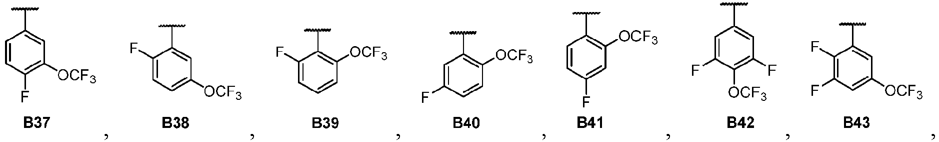 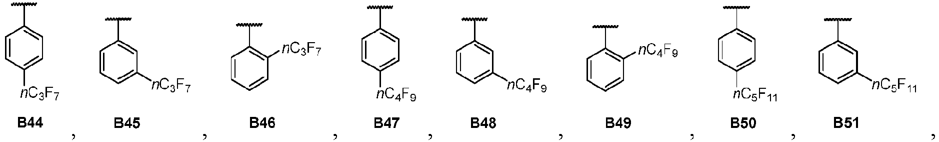 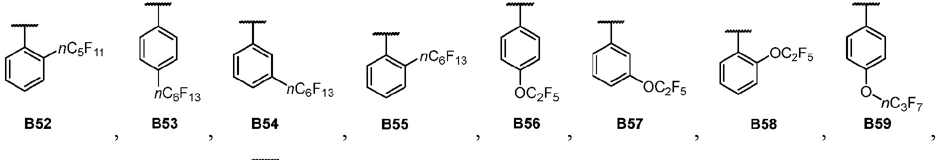 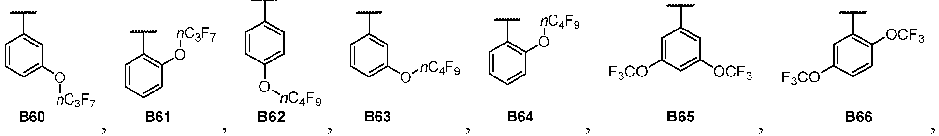 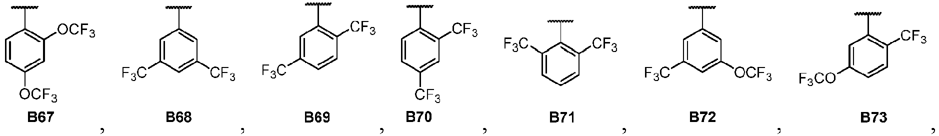 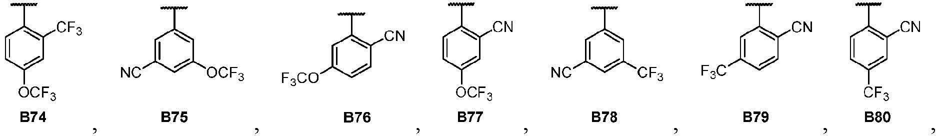 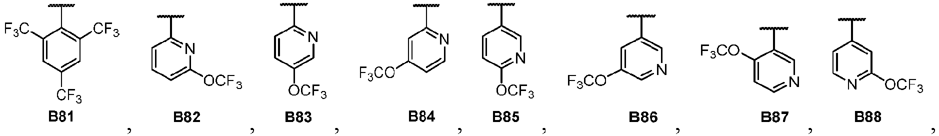 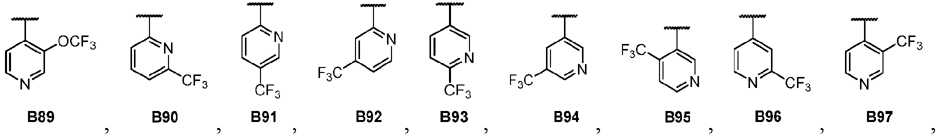 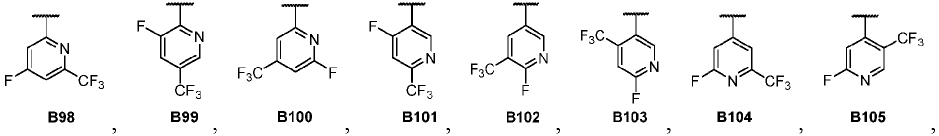 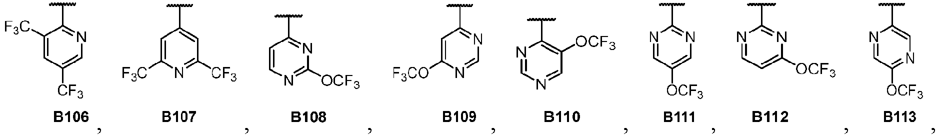 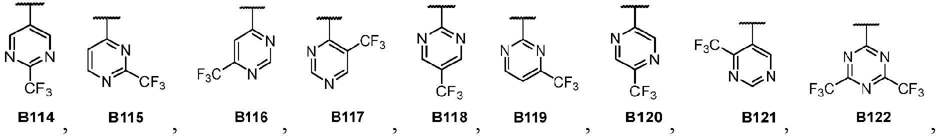 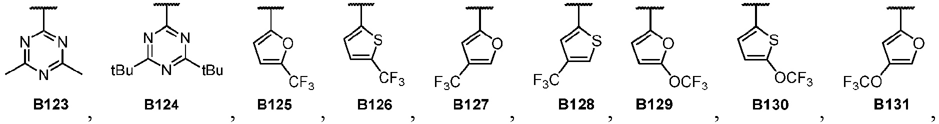 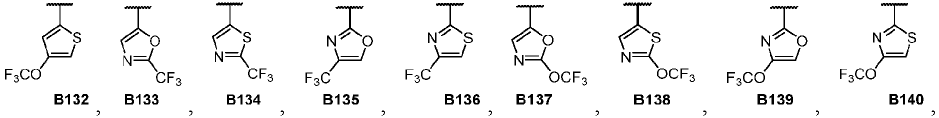 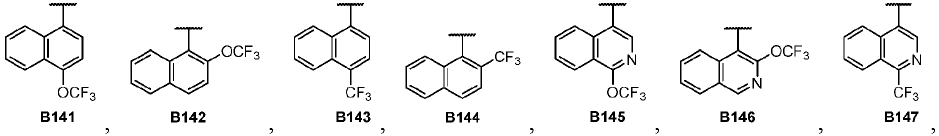 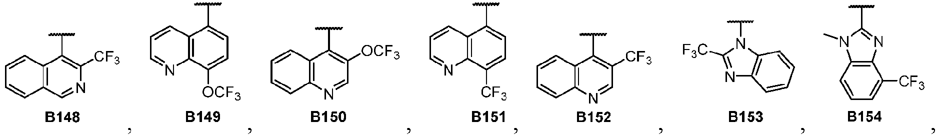 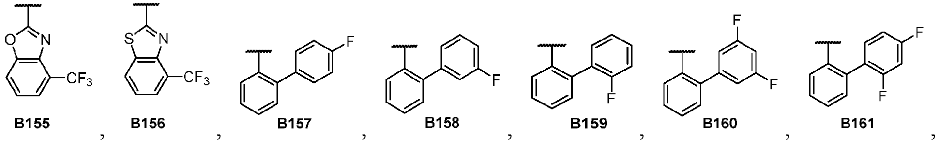 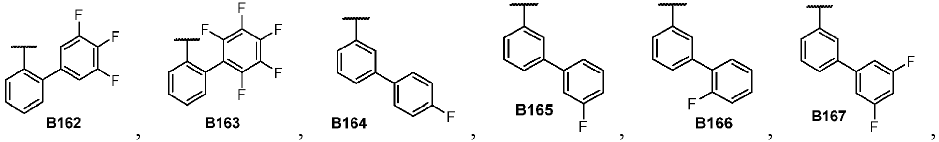 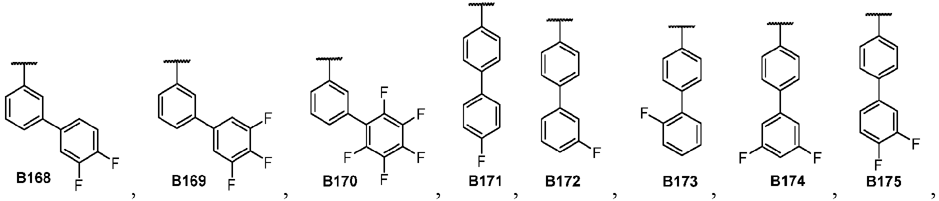 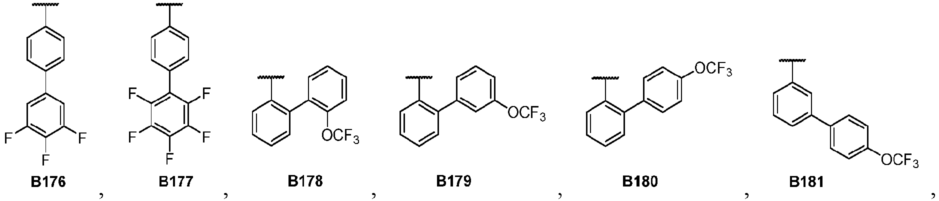 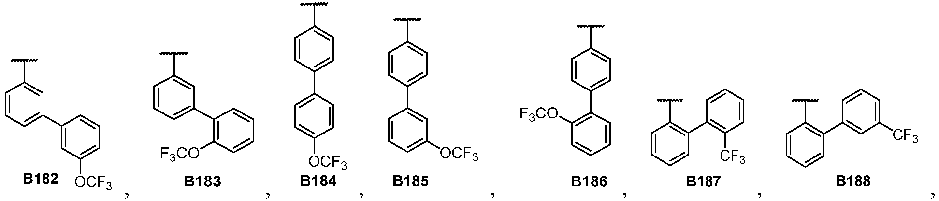 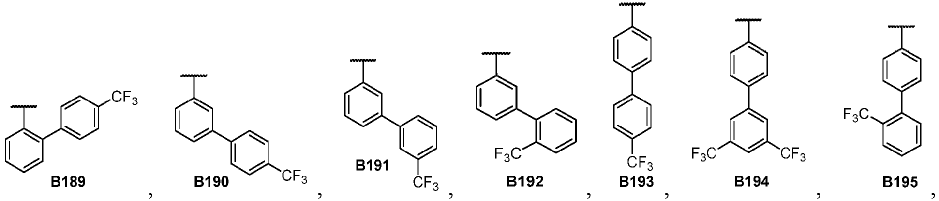 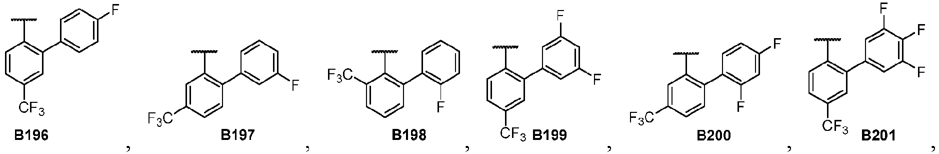 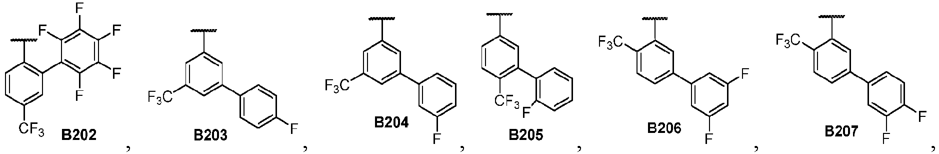 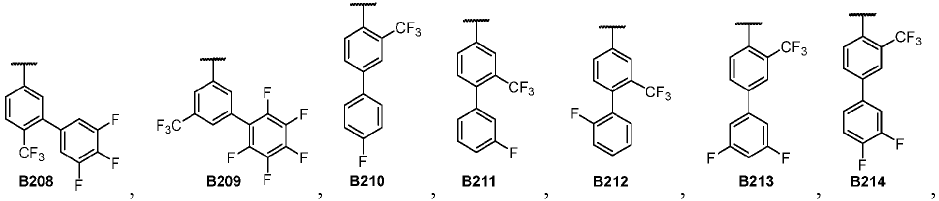 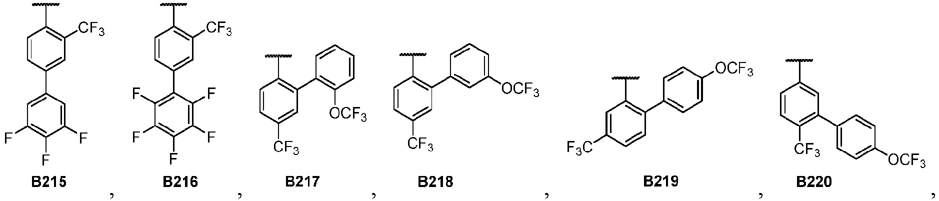 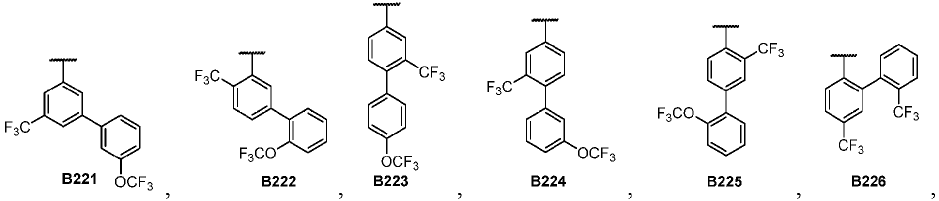 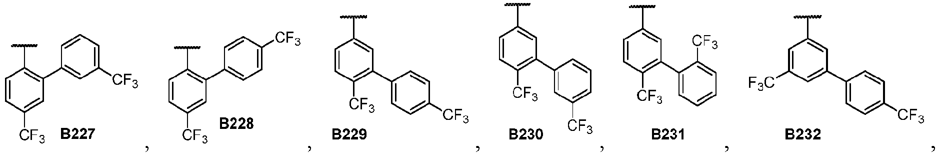 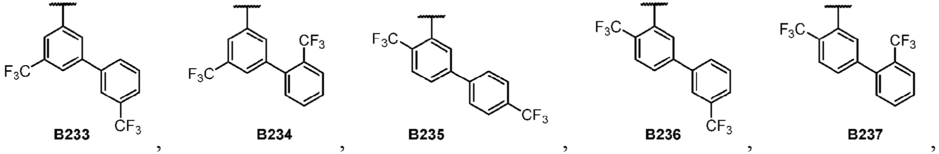 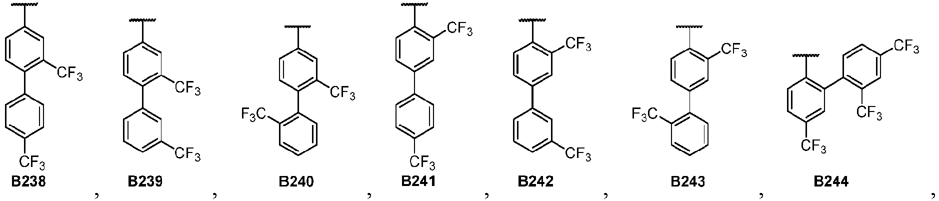 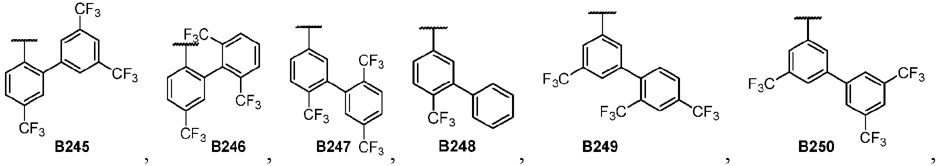 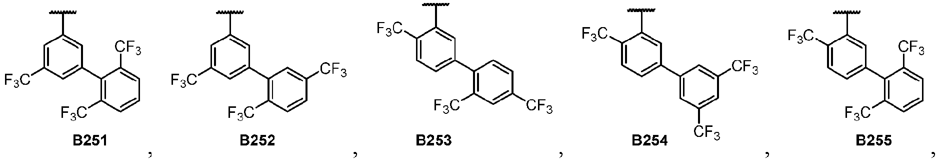 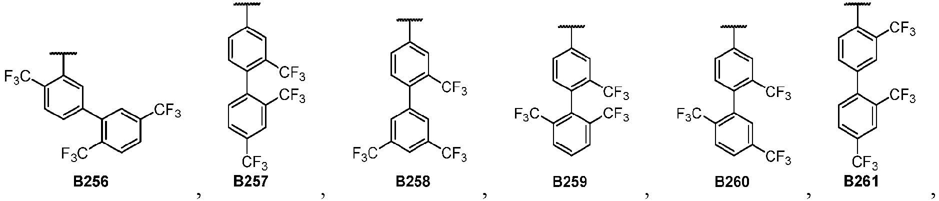 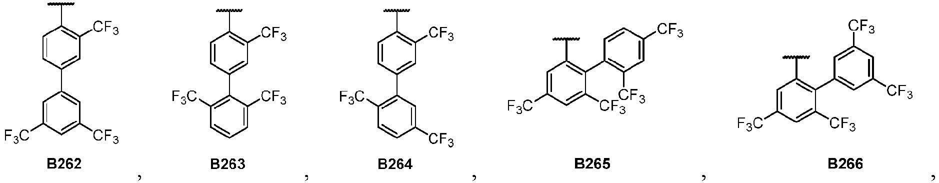 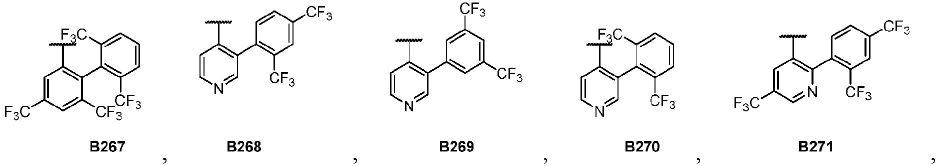 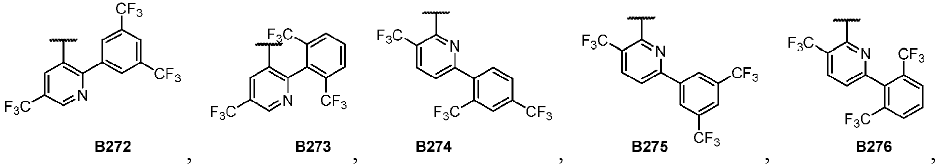 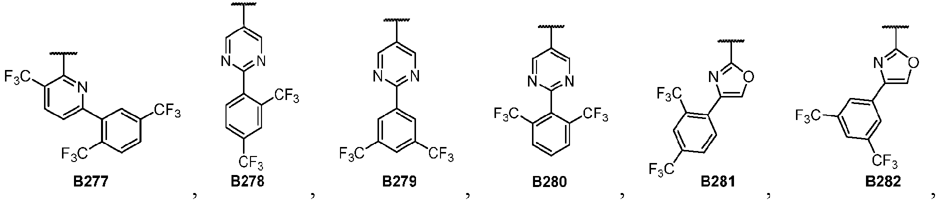 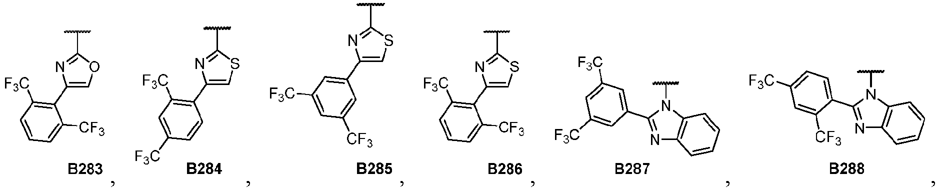 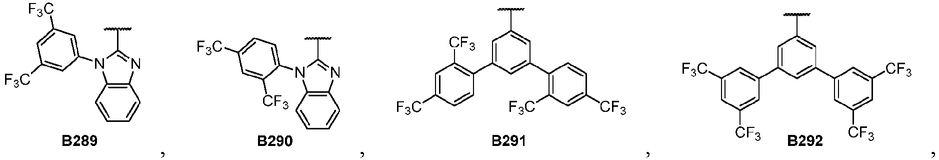 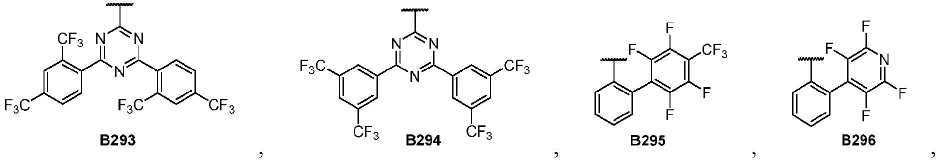 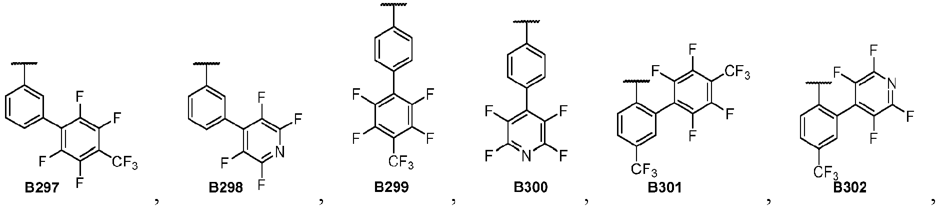 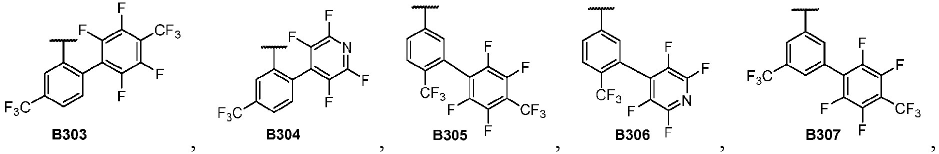 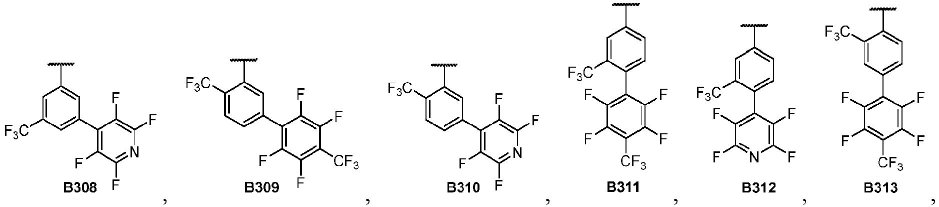 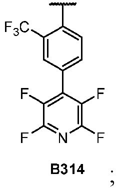 and/or Ar₁ and Ar₂ are, at each occurrence identically or differently, selected from the group consisting of B1 to B314.

8. The compound according to claim 7, wherein the compound is selected from the group consisting of Compound H1-1 to Compound H1-2449, Compound H2-1 to Compound H2-138, Compound H3-1 to Compound H3-92, Compound H4-1 to Compound H4-92, Compound H5-1 to Compound H5-115, Compound H6-1 to Compound H6-115, Compound H7-1 to Compound H7-203 and Compound H8-7 to Compound H8-54, Compound H8-61 to Compound H8-108, Compound H8-115 to Compound H8-162, Compound H8-169 to Compound H8-216, Compound H8-223 to Compound H8-270, Compound H8-277 to Compound H8-324, Compound H8-331 to Compound H8-378, Compound H8-385 to Compound H8-432, Compound H8-439 to Compound H8-486, Compound H8-493 to Compound H8-540, Compound H8-547 to Compound H8-594, Compound H8-601 to Compound H8-648, Compound H8-655 to Compound H8-702, Compound H8-709 to Compound H8-756, Compound H8-763 to Compound H8-810, Compound H8-817 to Compound H8-864, Compound H8-871 to Compound H8-918, Compound H8-925 to Compound H8-972, Compound H8-979 to Compound H8-1026, Compound H8-1033 to Compound H8-1080, Compound H8-1087 to Compound H8-1134, Compound H8-1141 to Compound H8-1188, Compound H8-1195 to Compound H8-1242, Compound H8-1249 to Compound H8-1296, Compound H8-1303 to Compound H8-1350, Compound H8-1357 to Compound H8-1404, Compound H8-1411 to Compound H8-1458, Compound H8-1465 to Compound H8-1512;
wherein Compound H1-1 to Compound H1-2449 each have a structure represented by Formula 2-1: 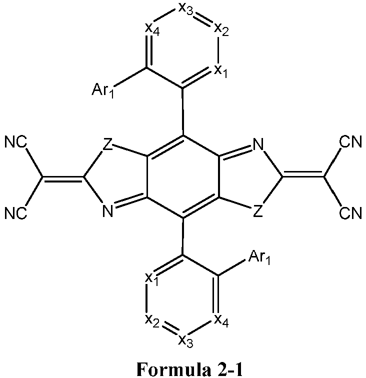
in Formula 2-1, two Z are the same, two X₁ are the same, two X₂ are the same, two X₃ are the same, two X₄ are the same, two Ar₁ are the same, and Z, X₁, X₂, X₃, X₄ and Ar₁ are selected from atoms or groups in the following table, respectively: 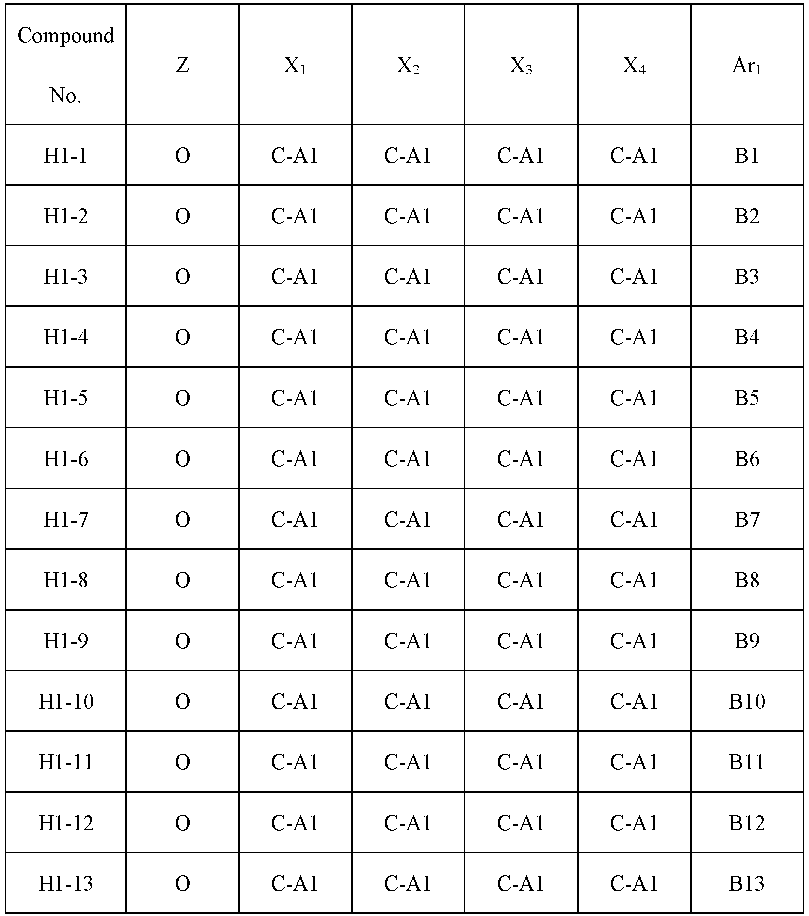 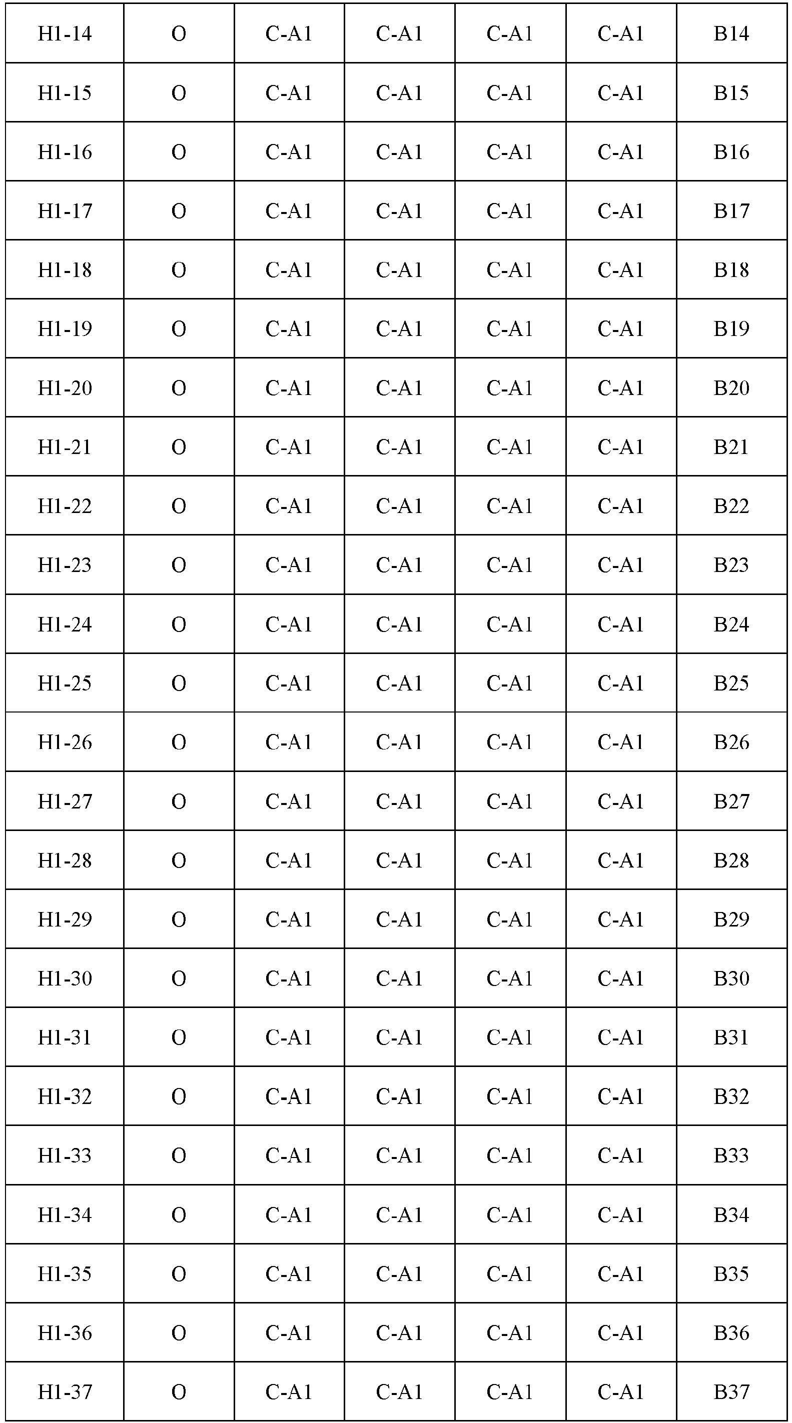 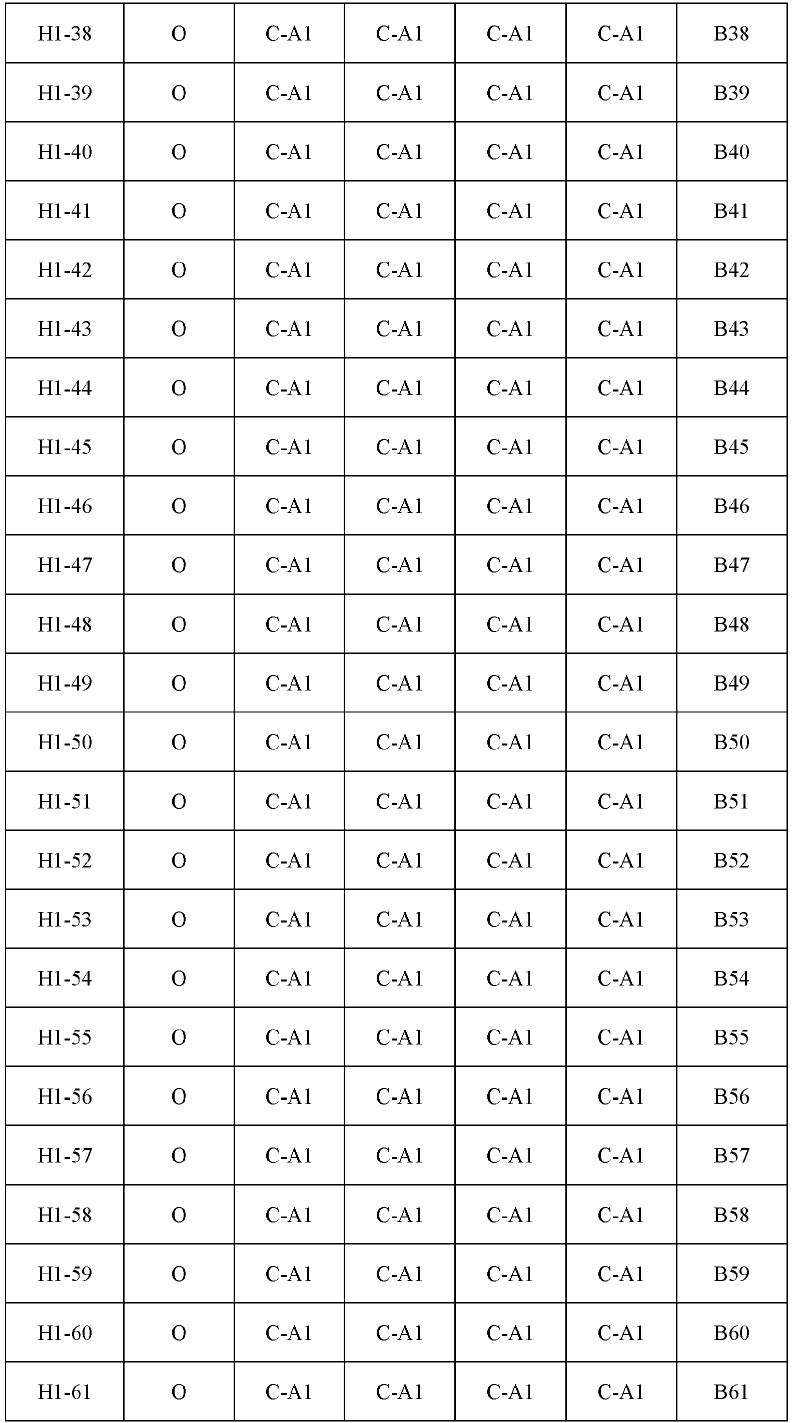 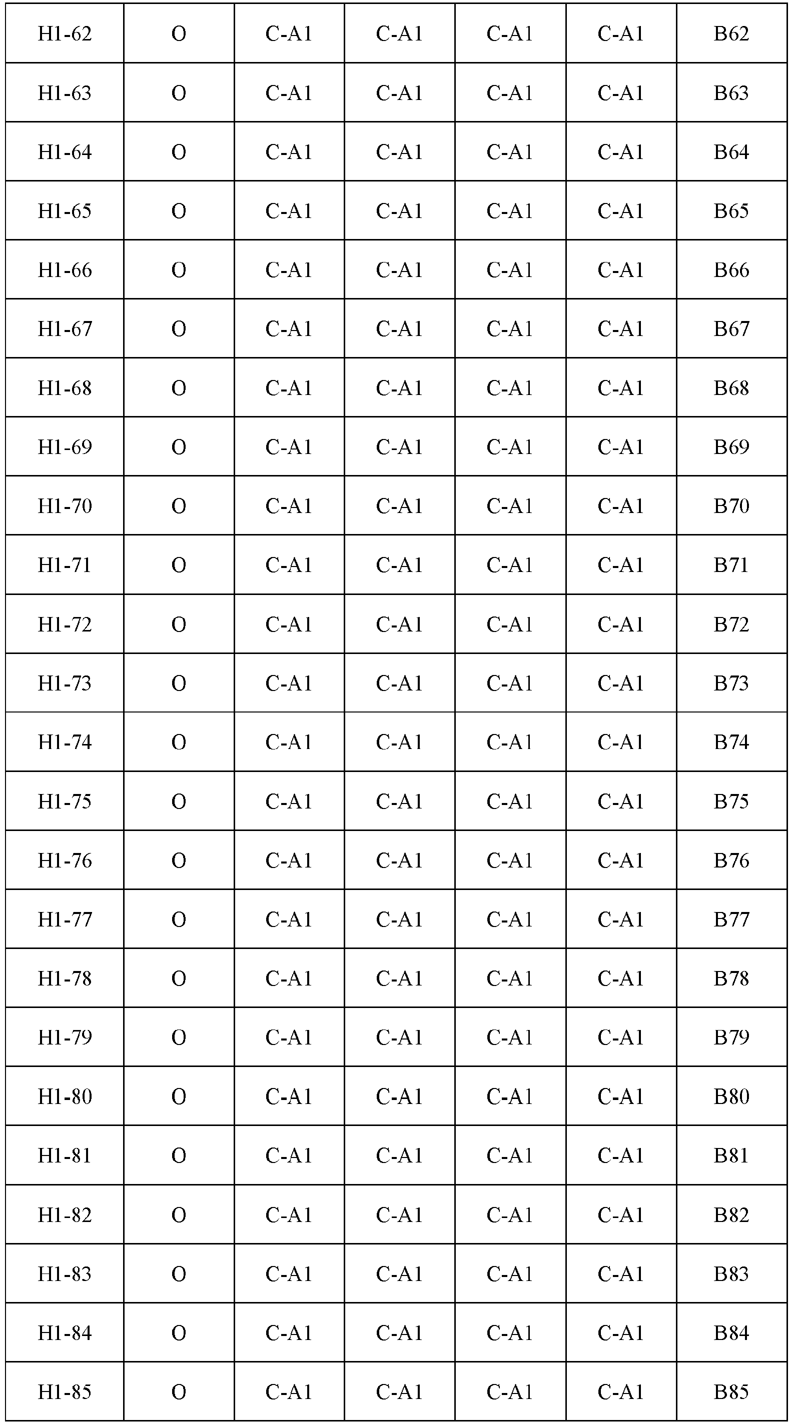 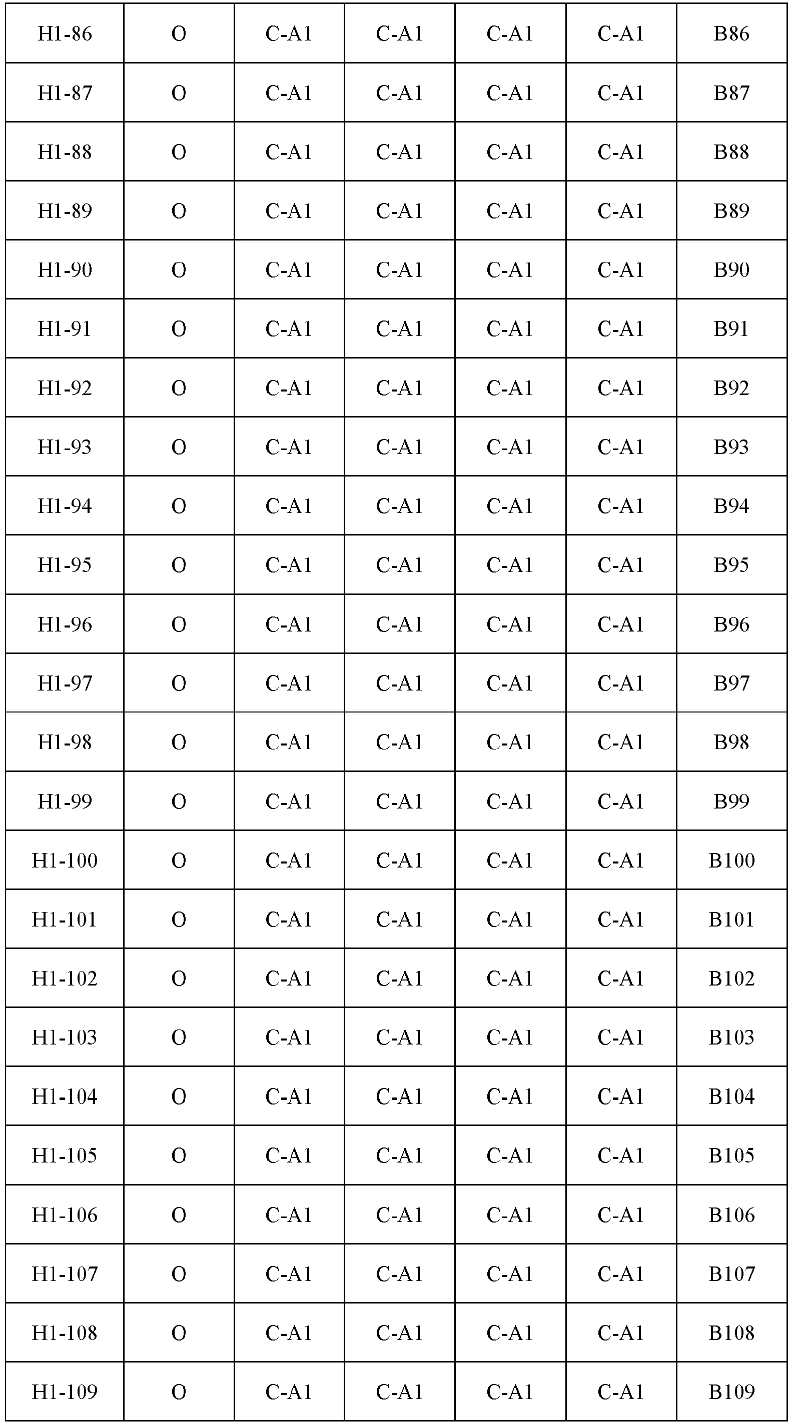 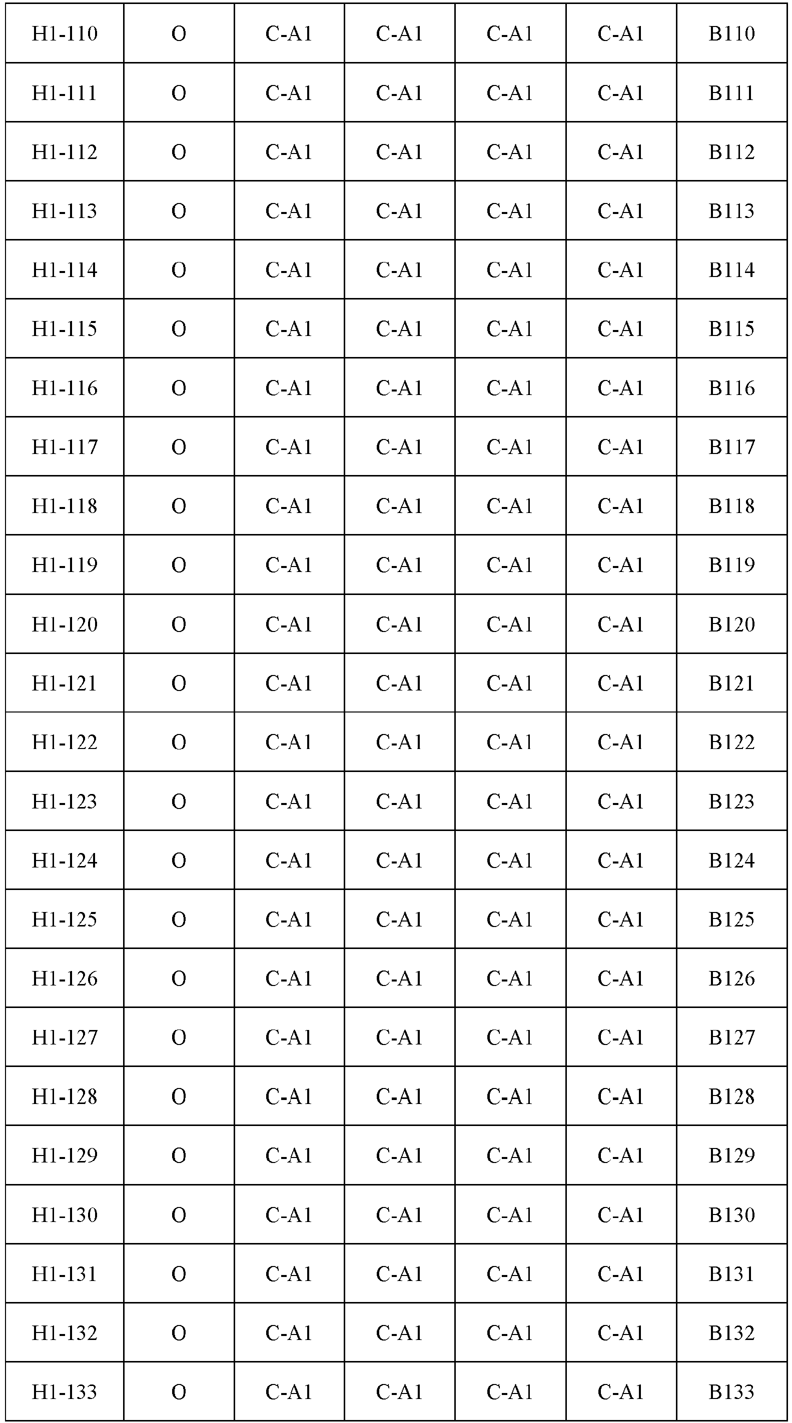 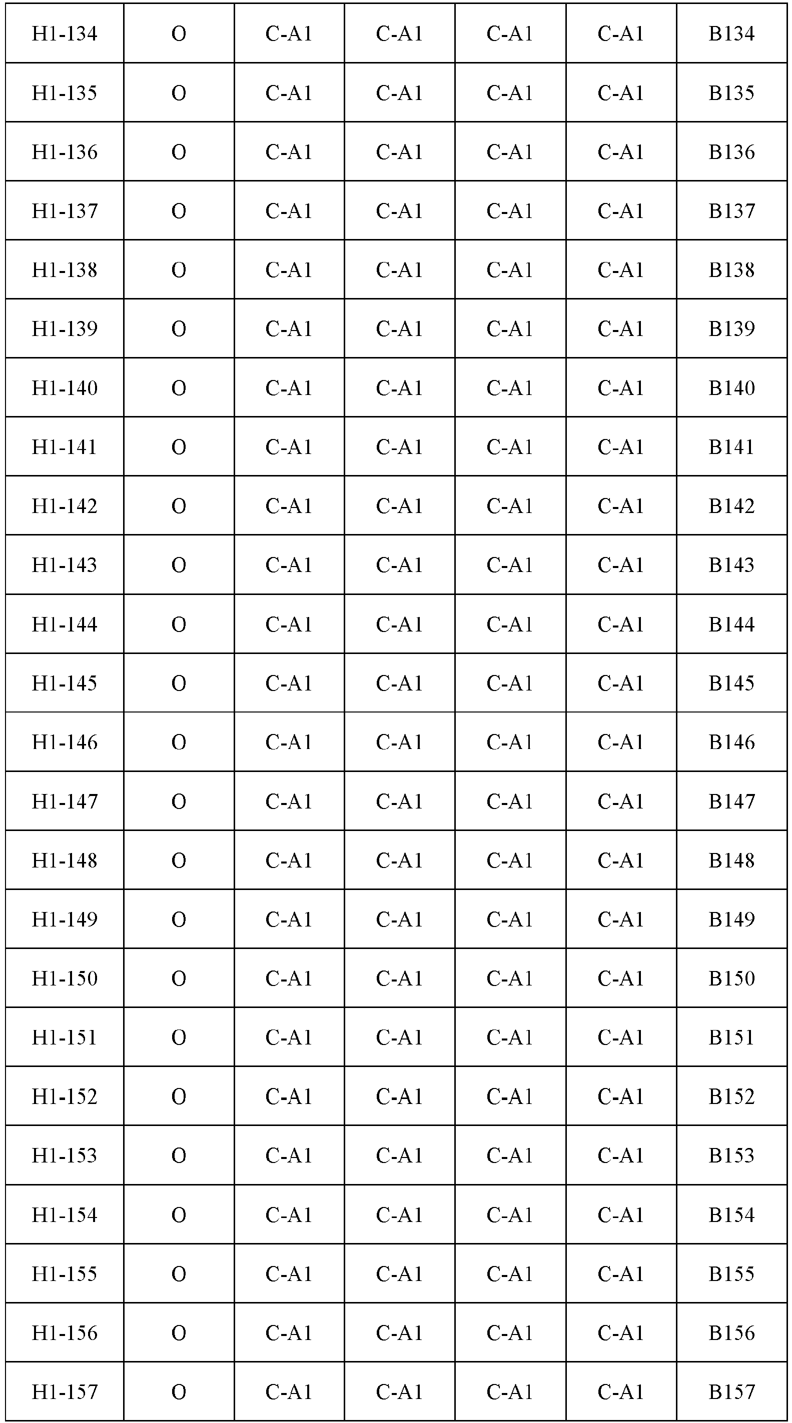 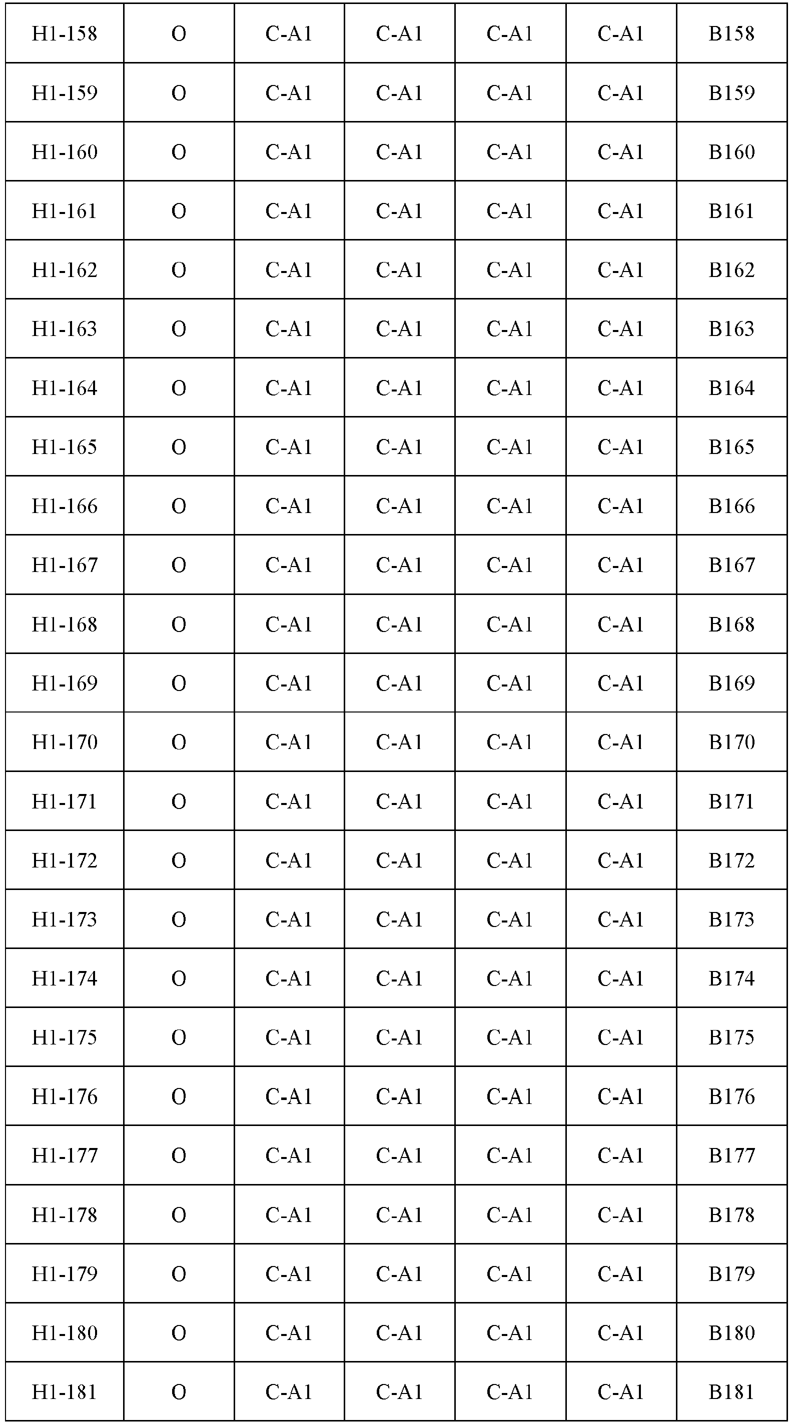 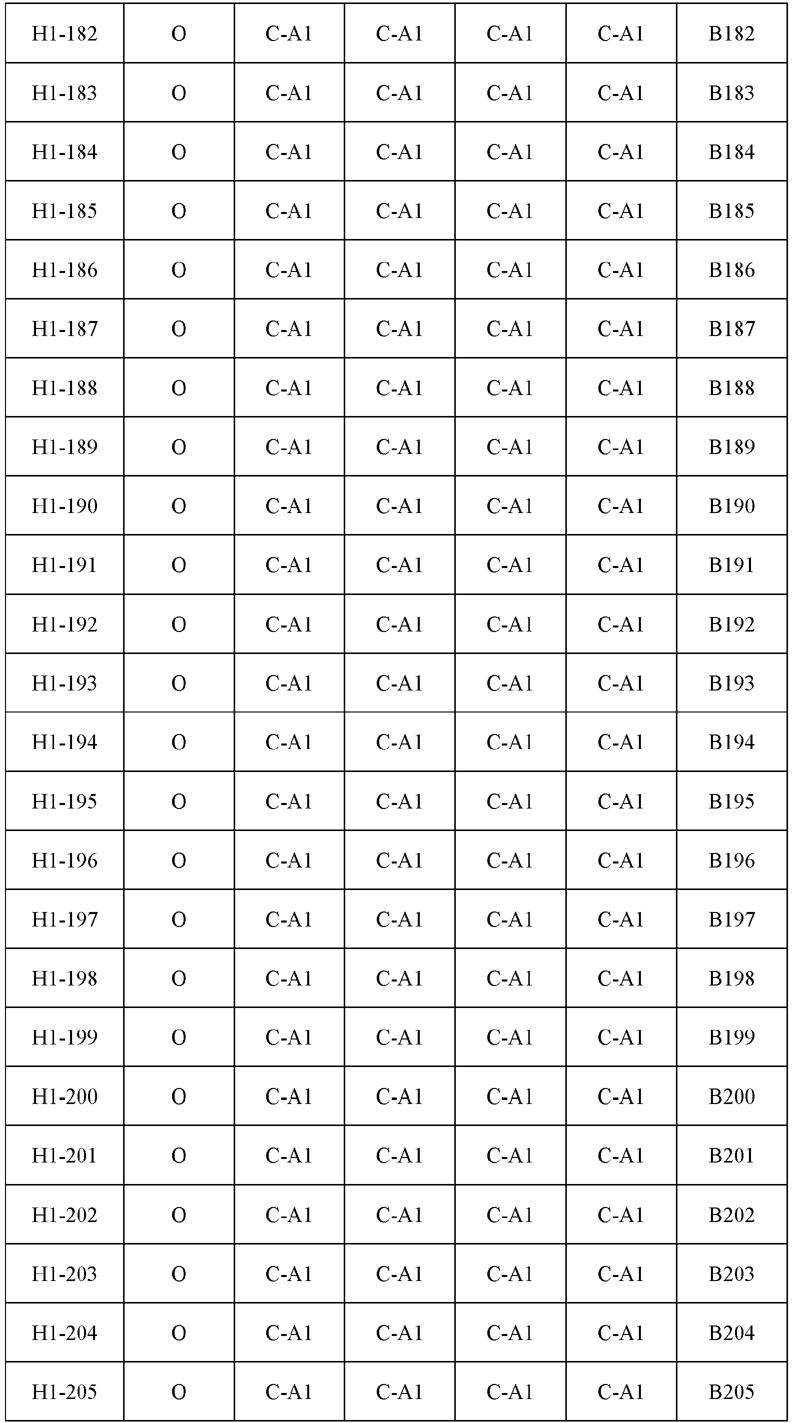 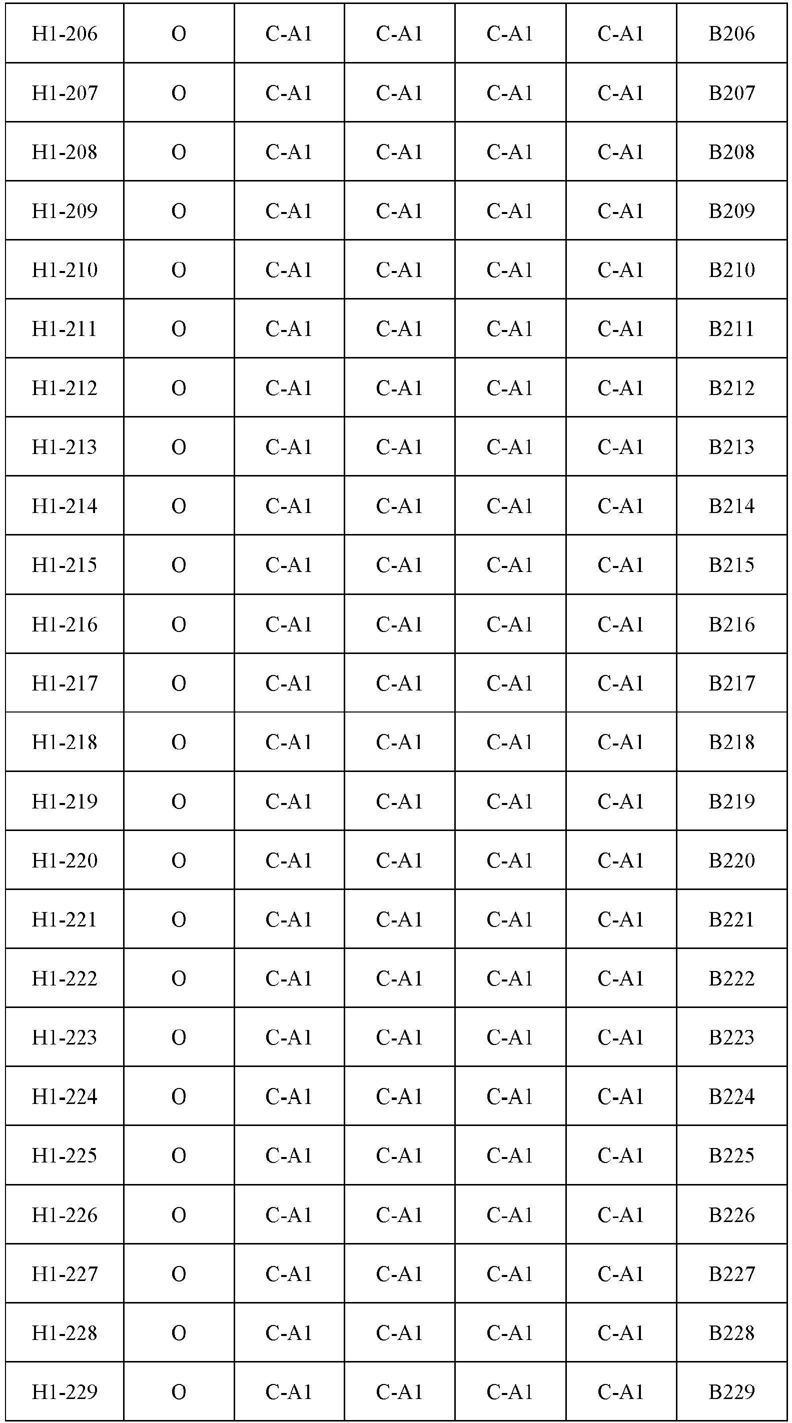 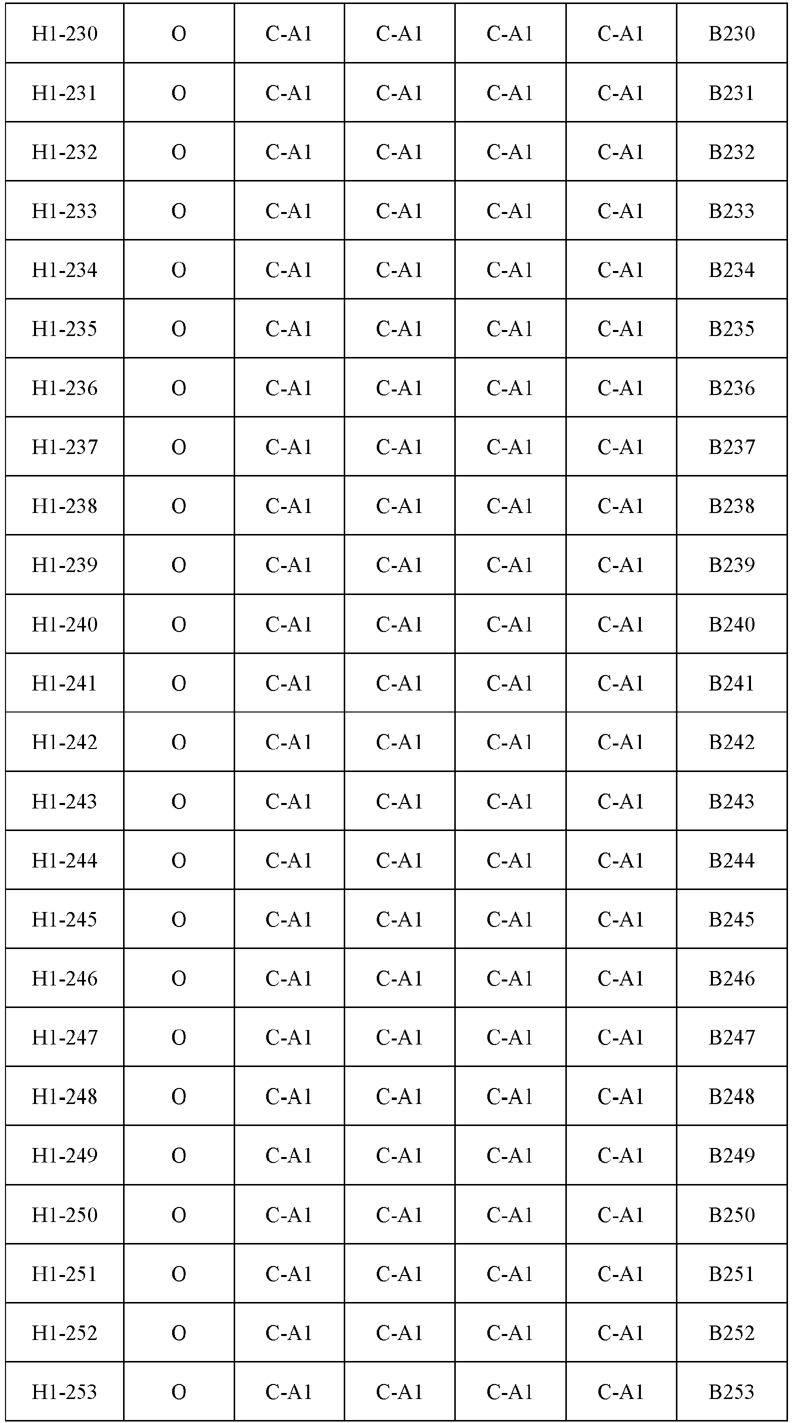 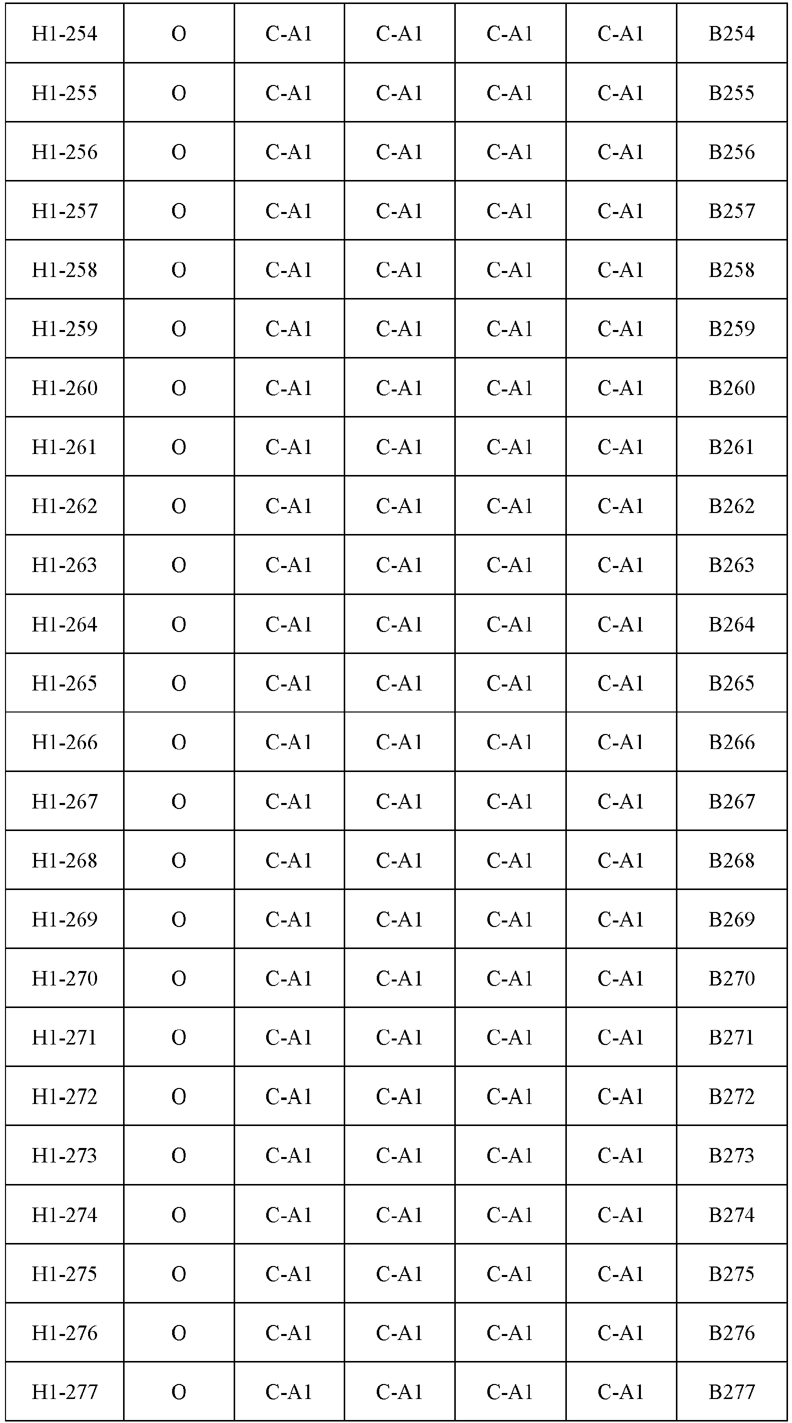 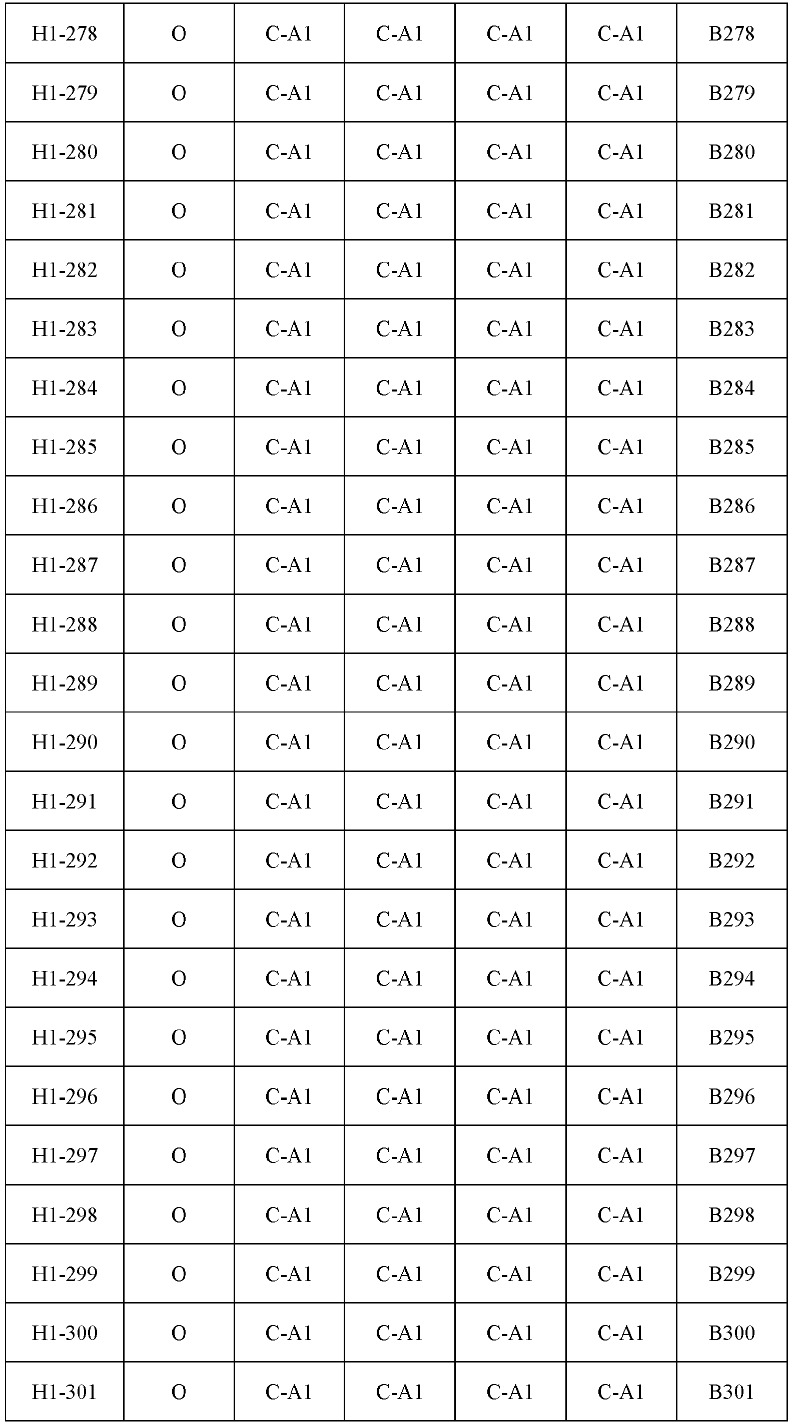 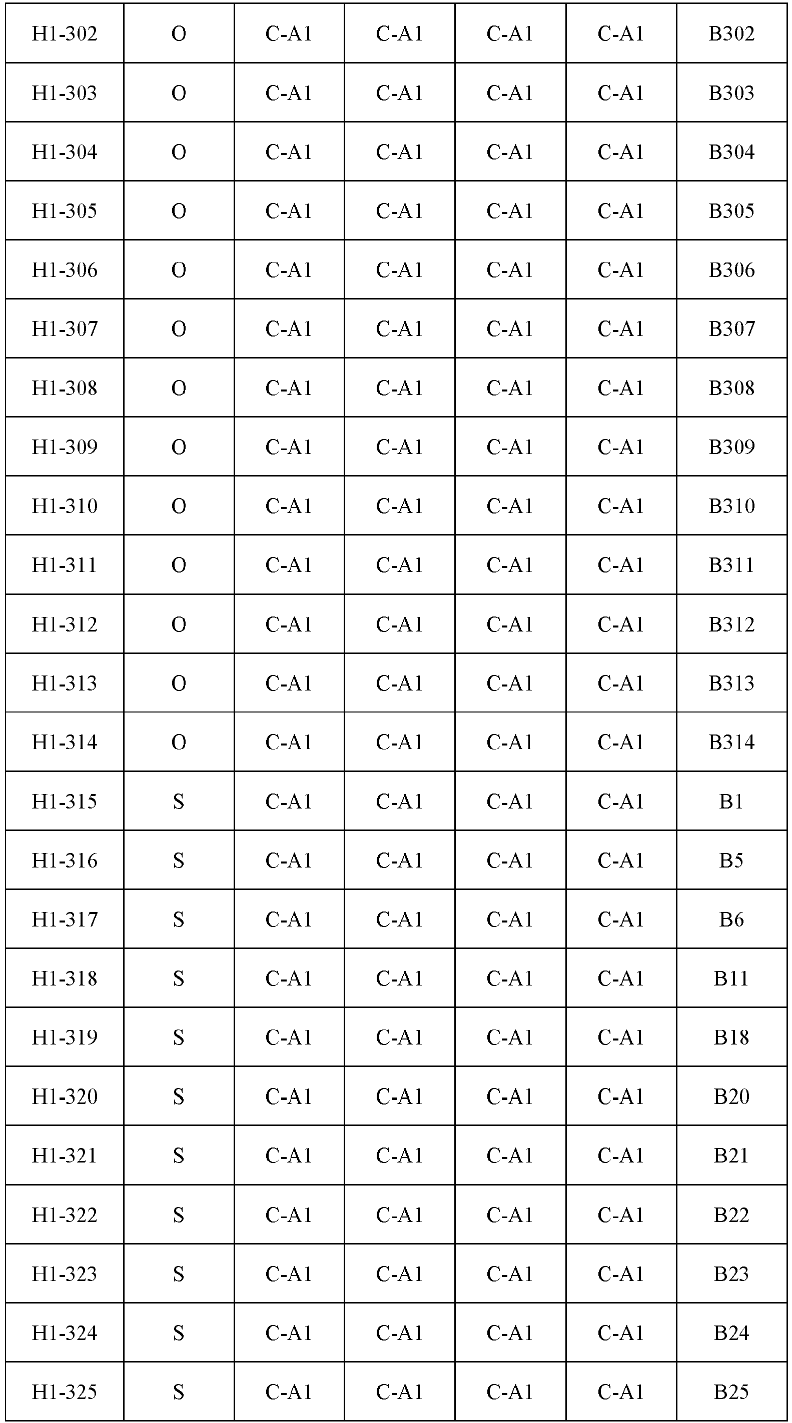 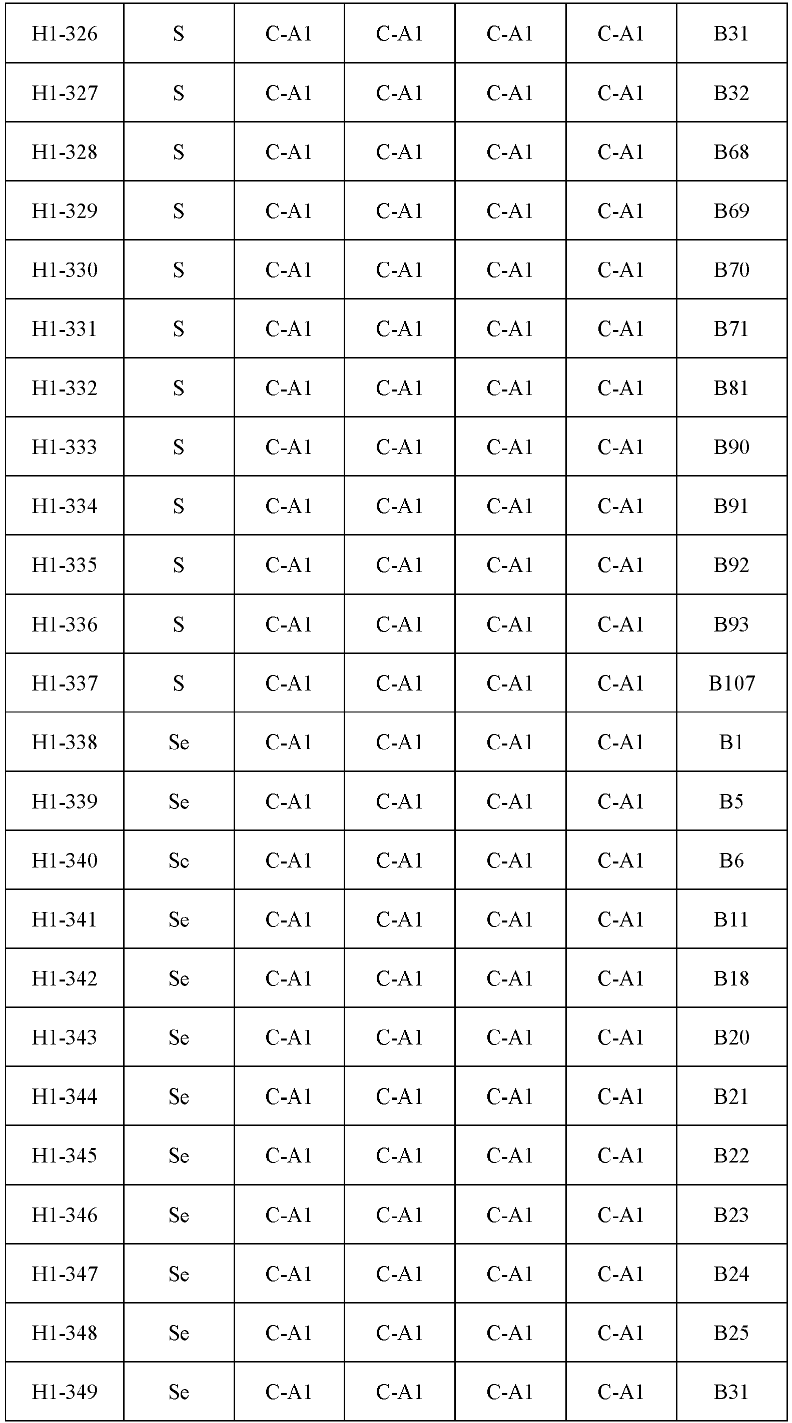 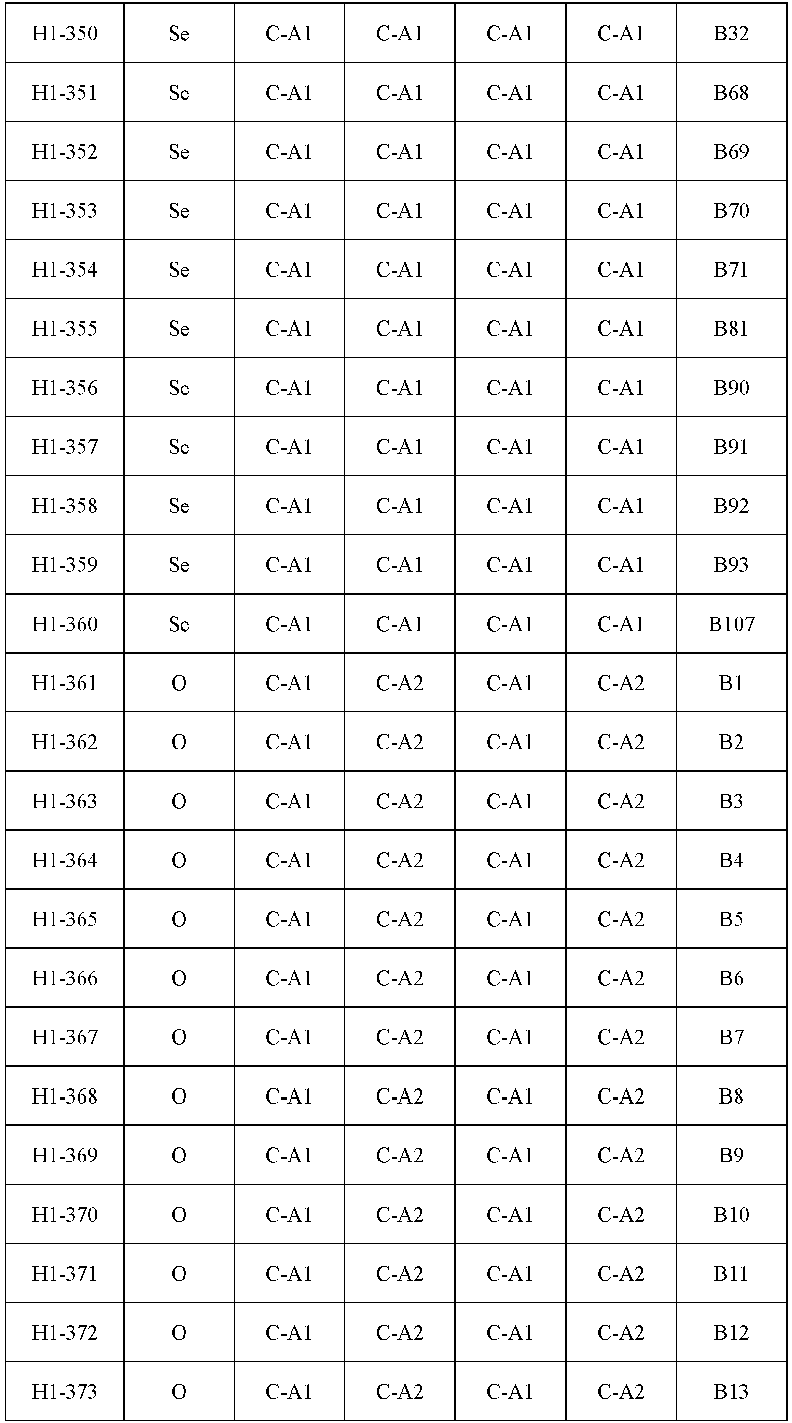 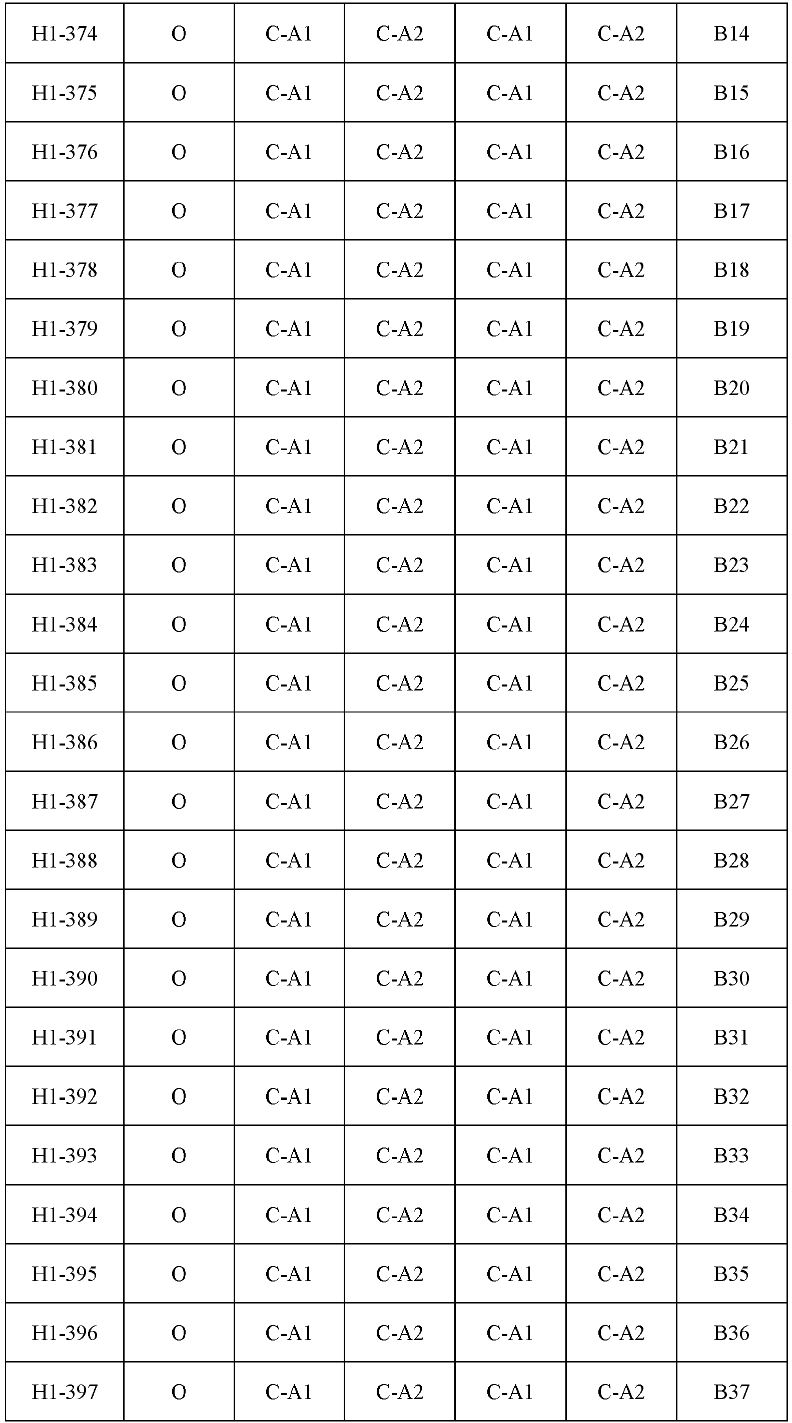 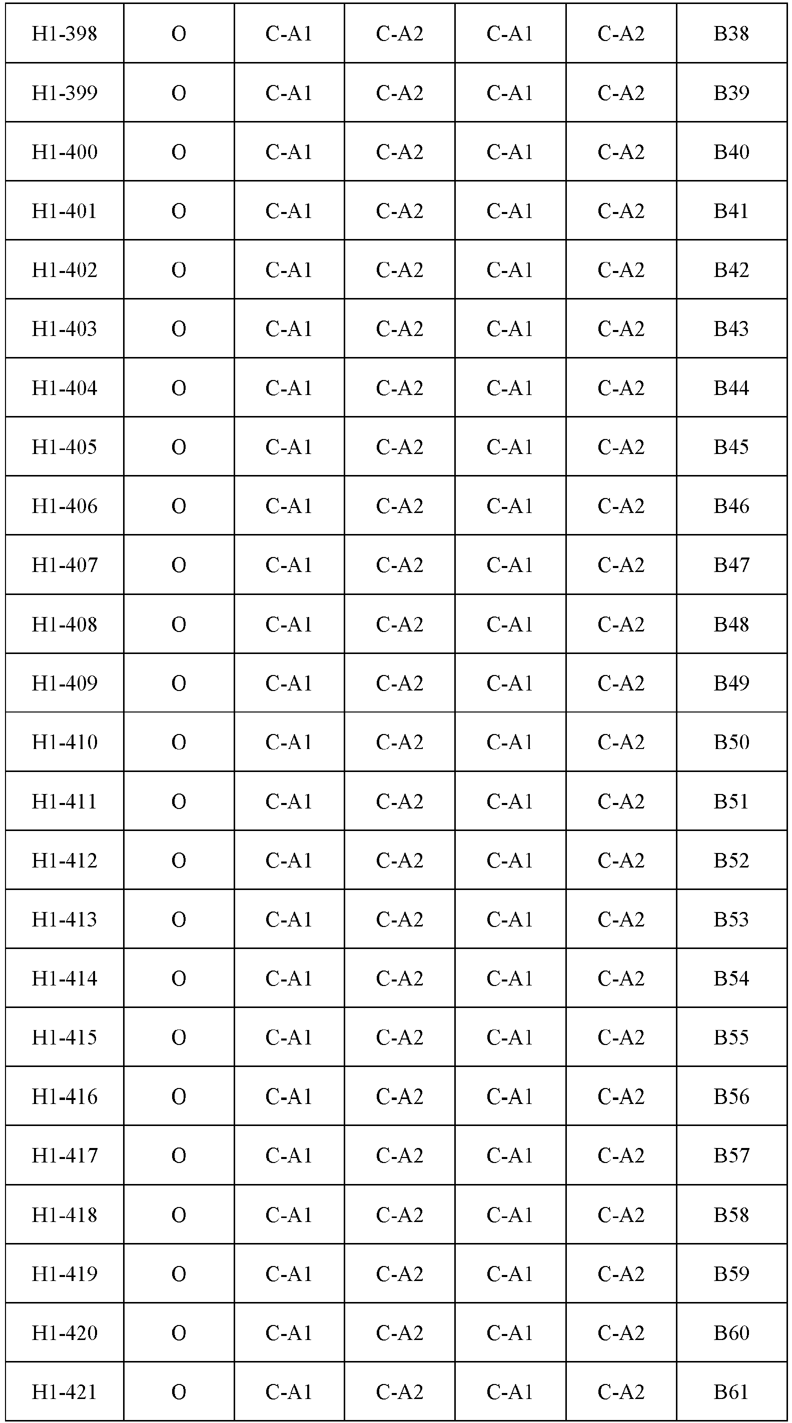 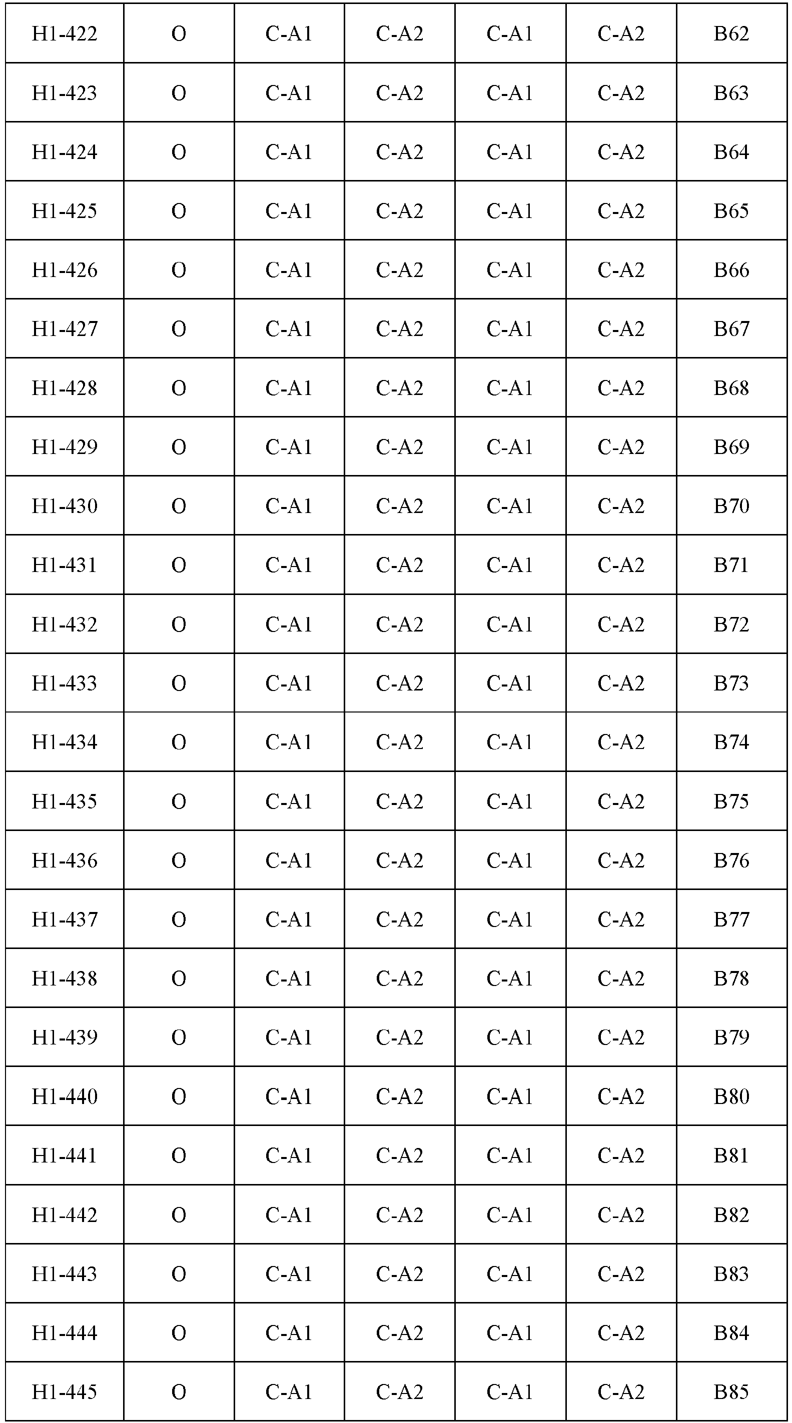 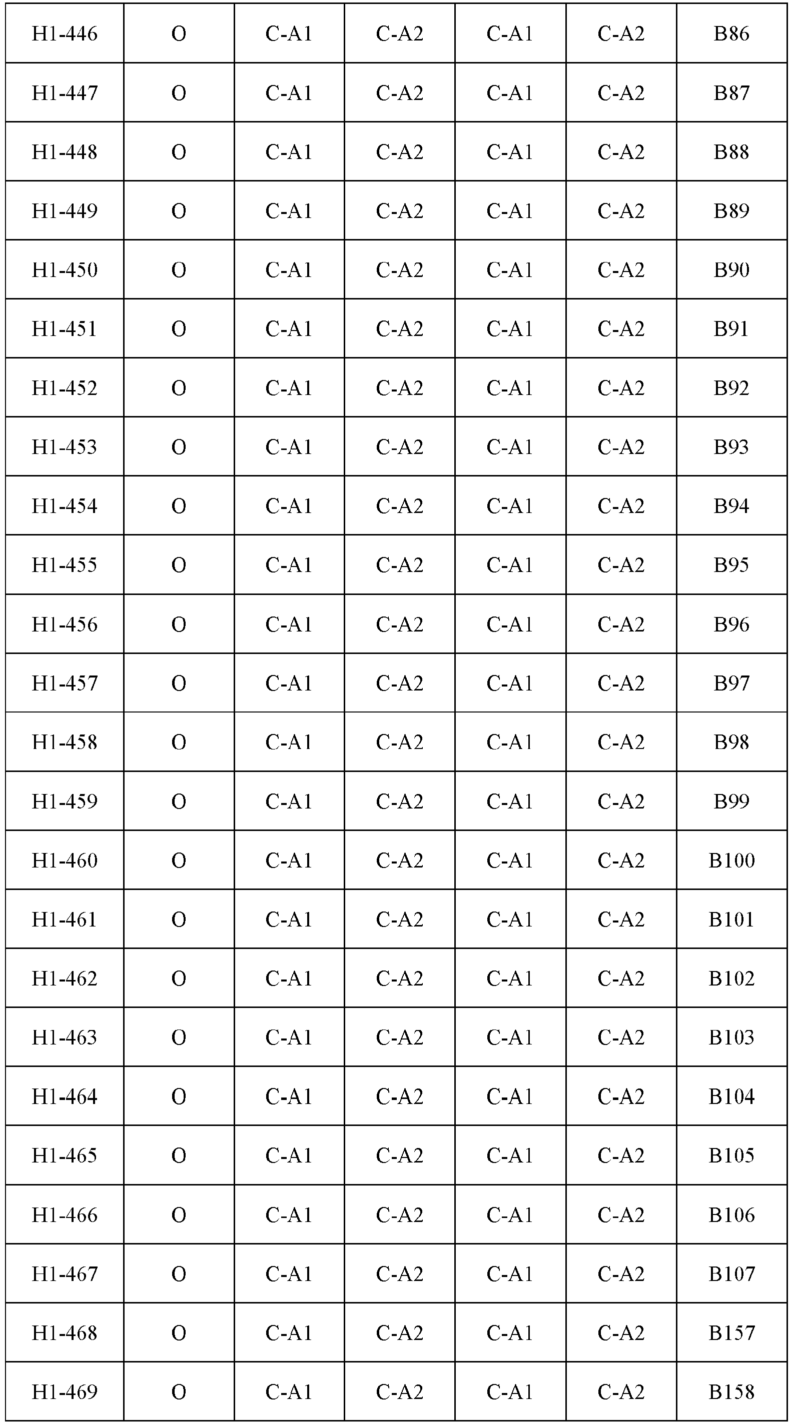 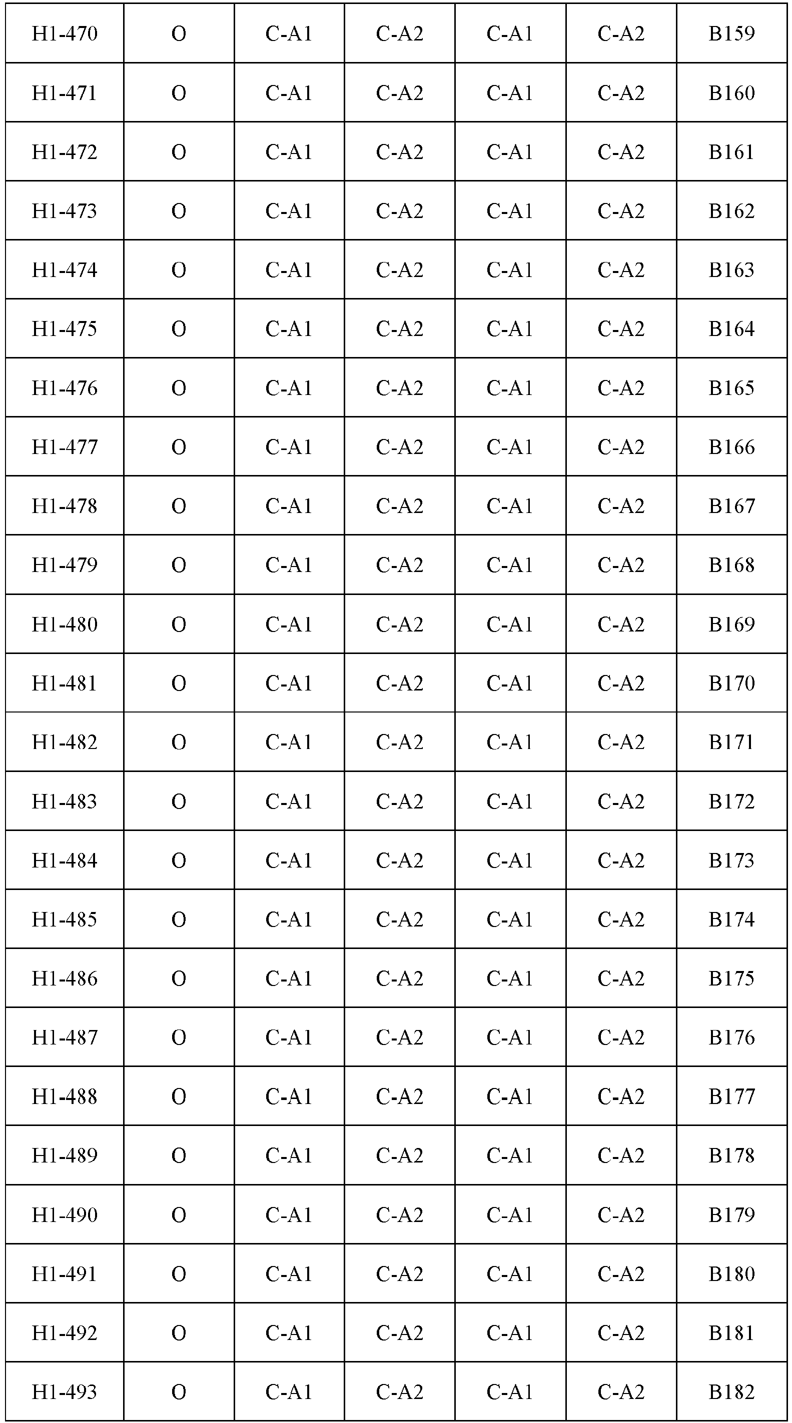 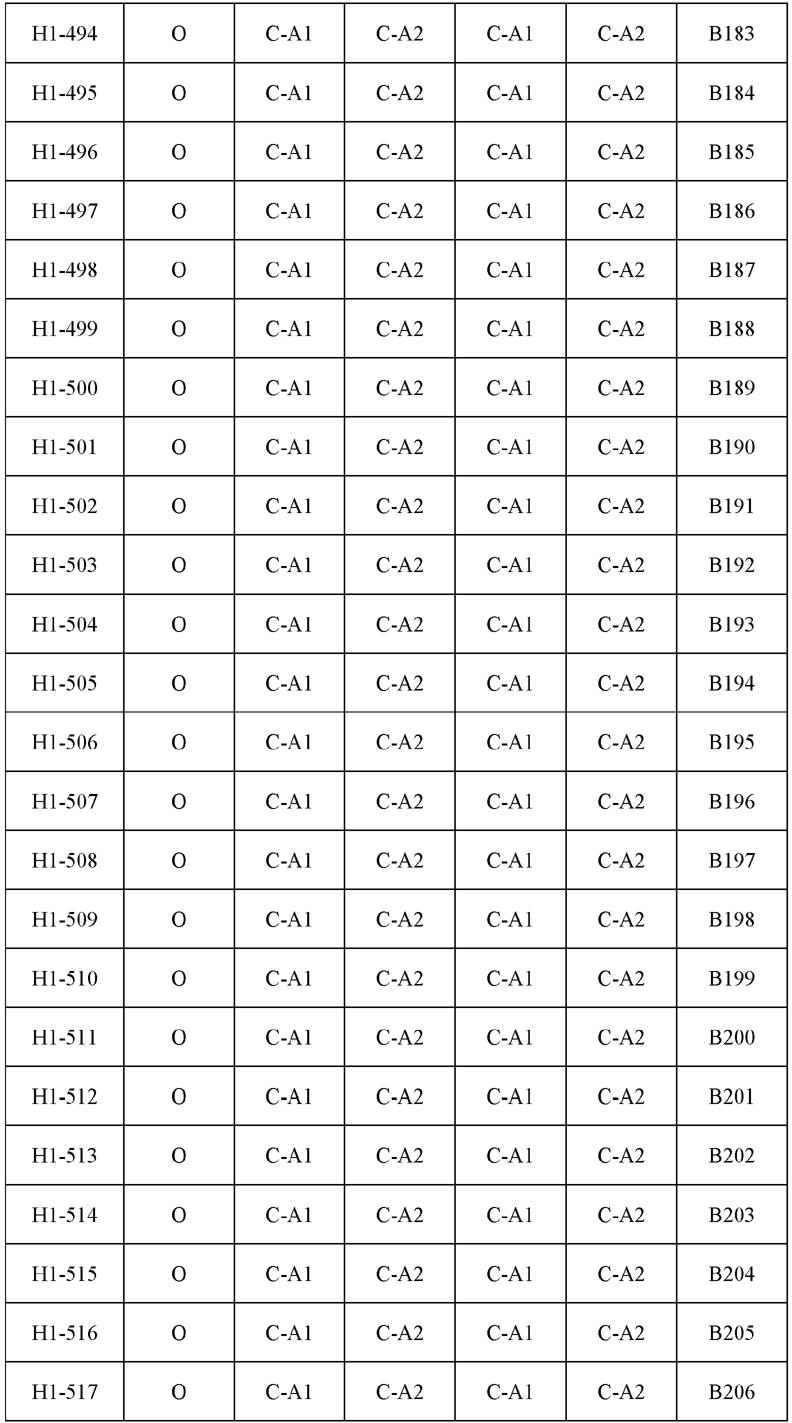 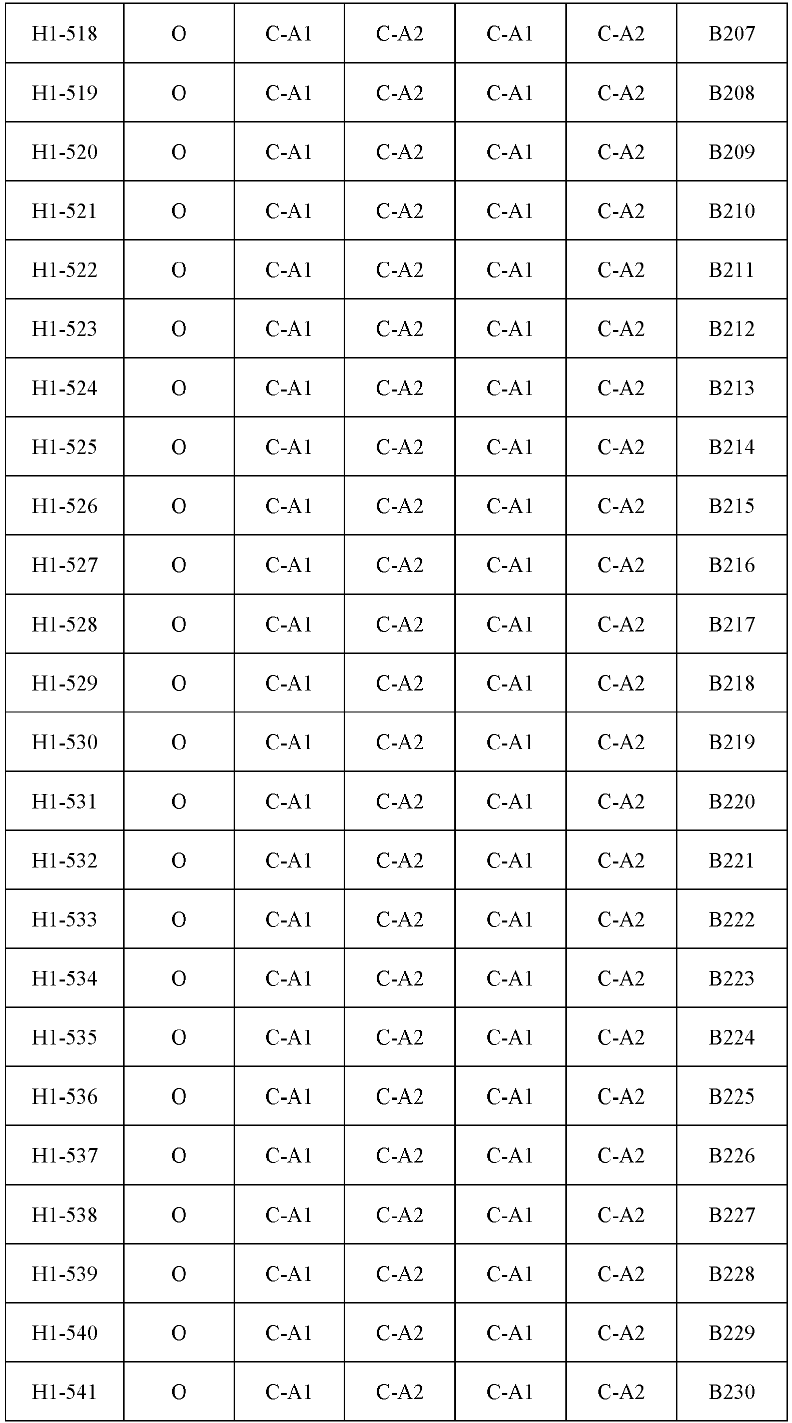 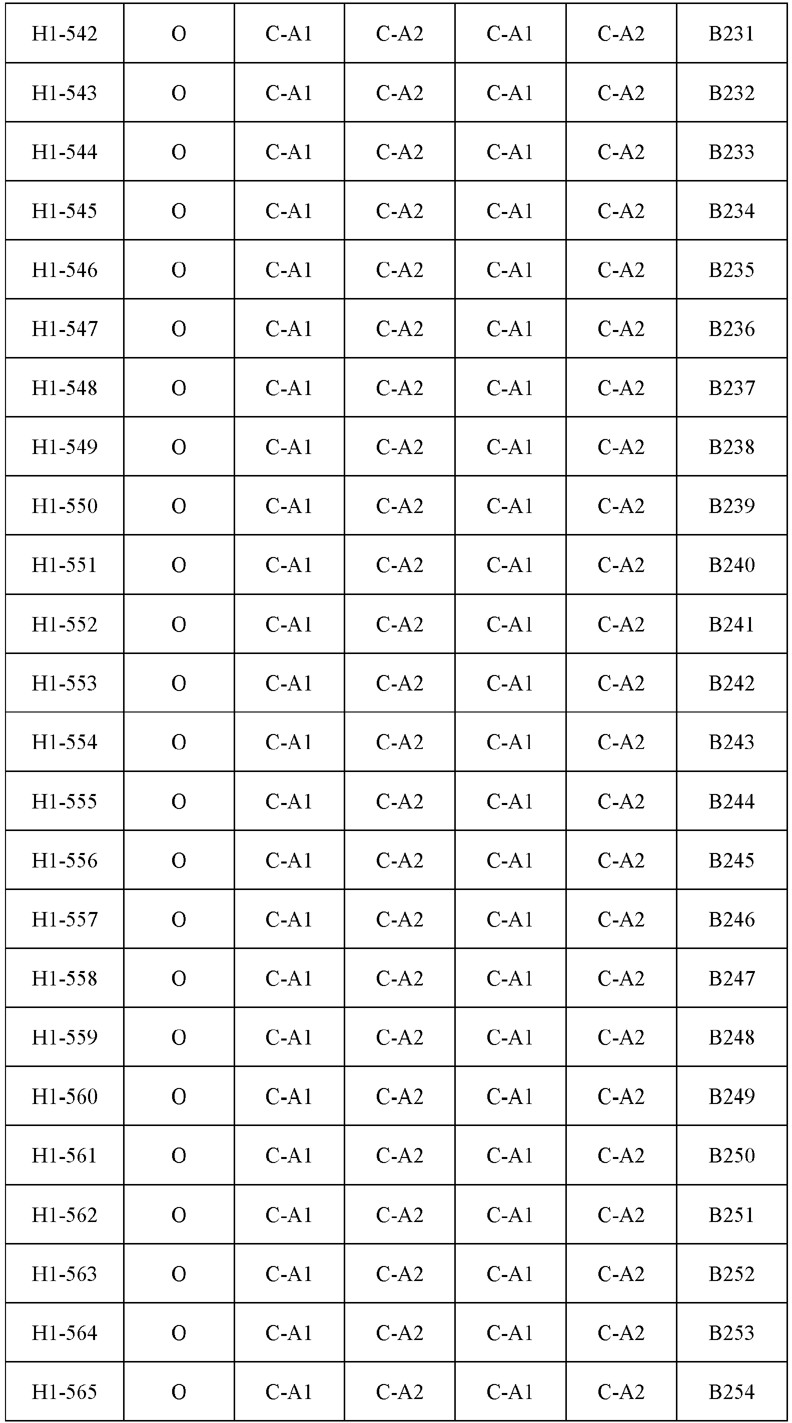 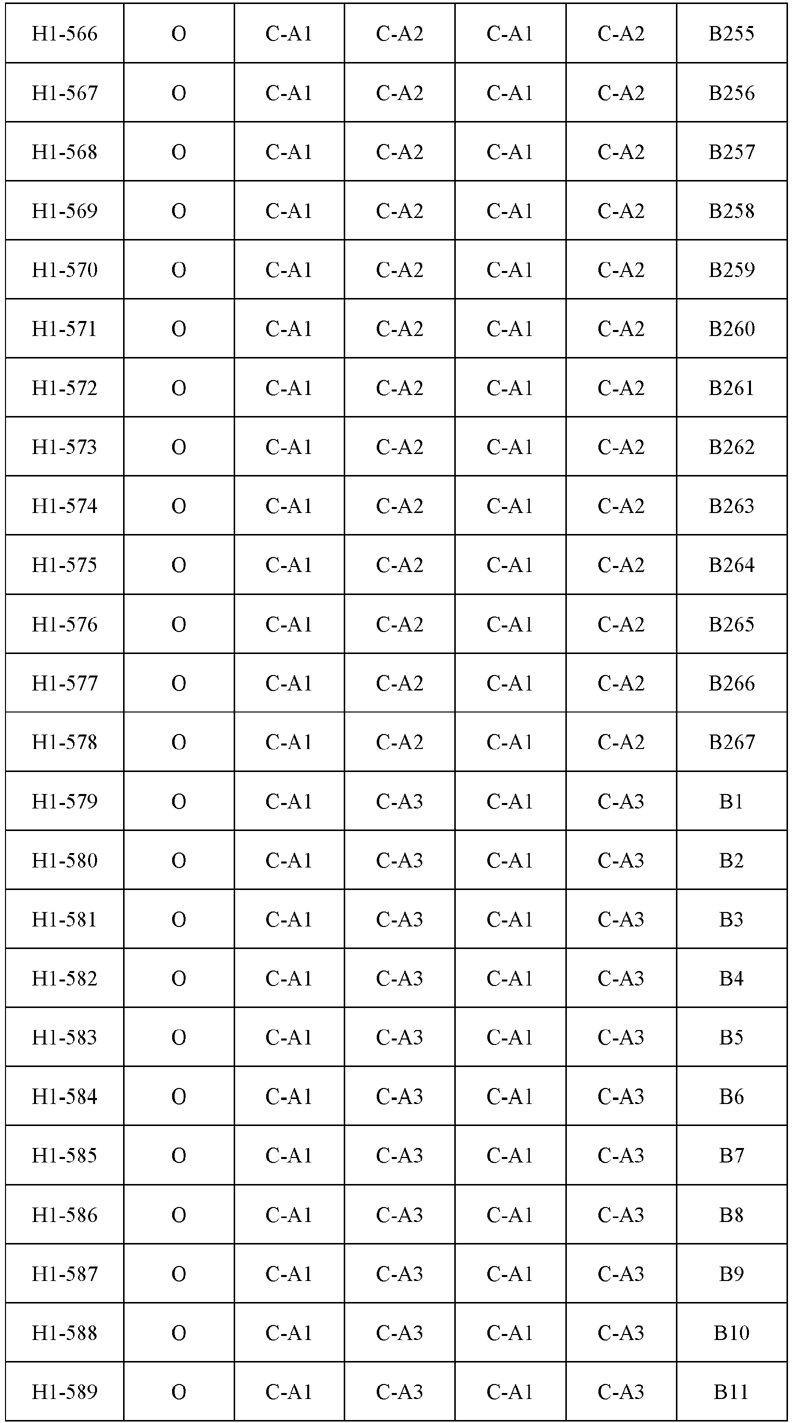 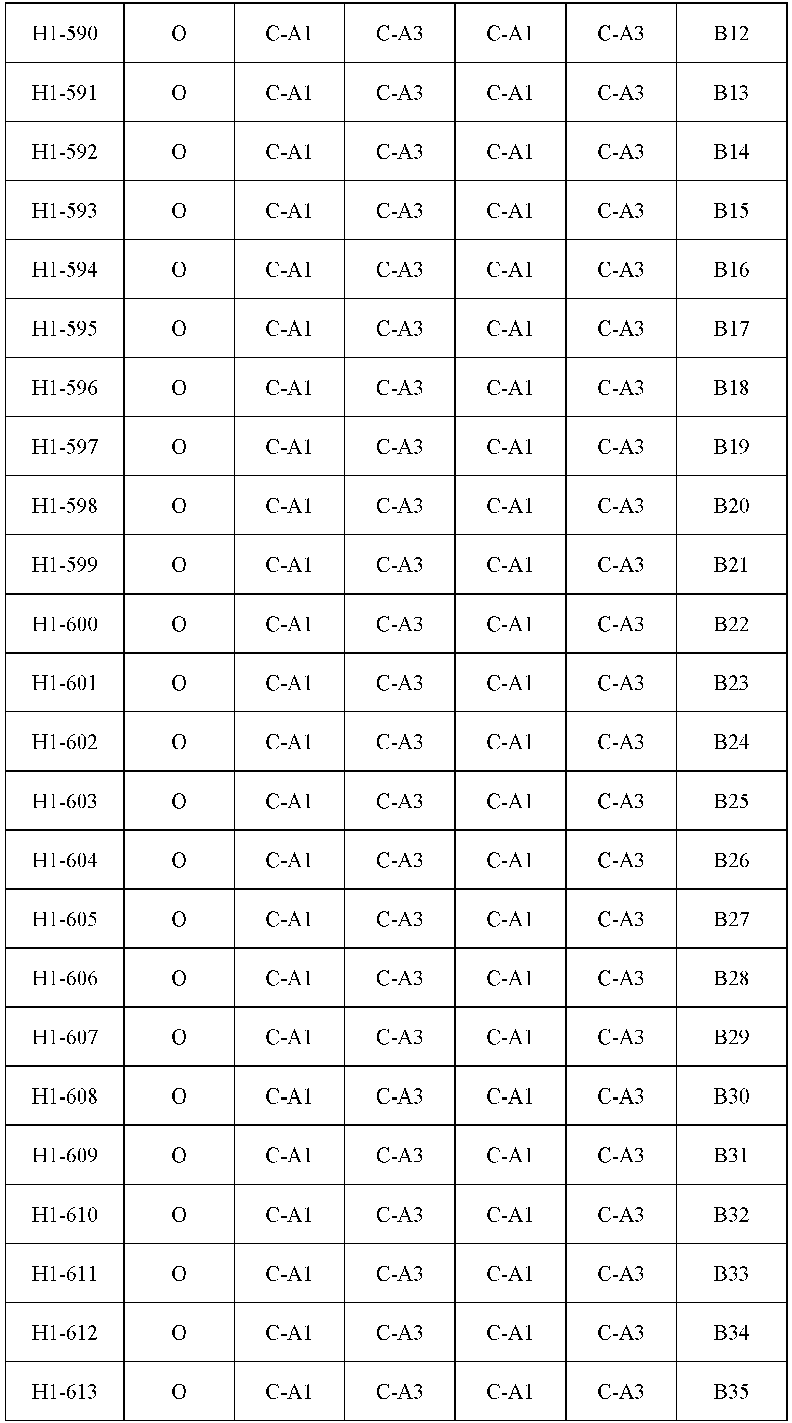 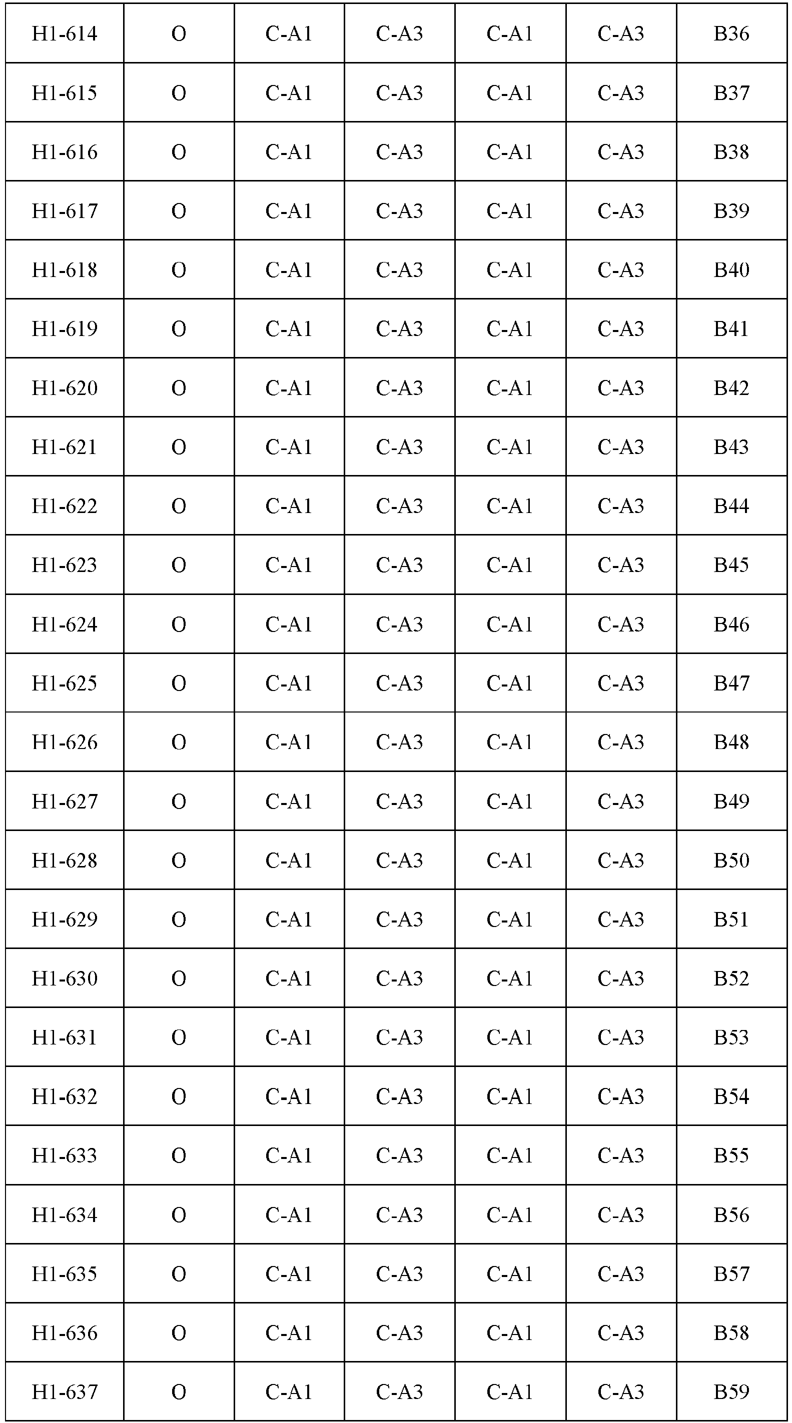 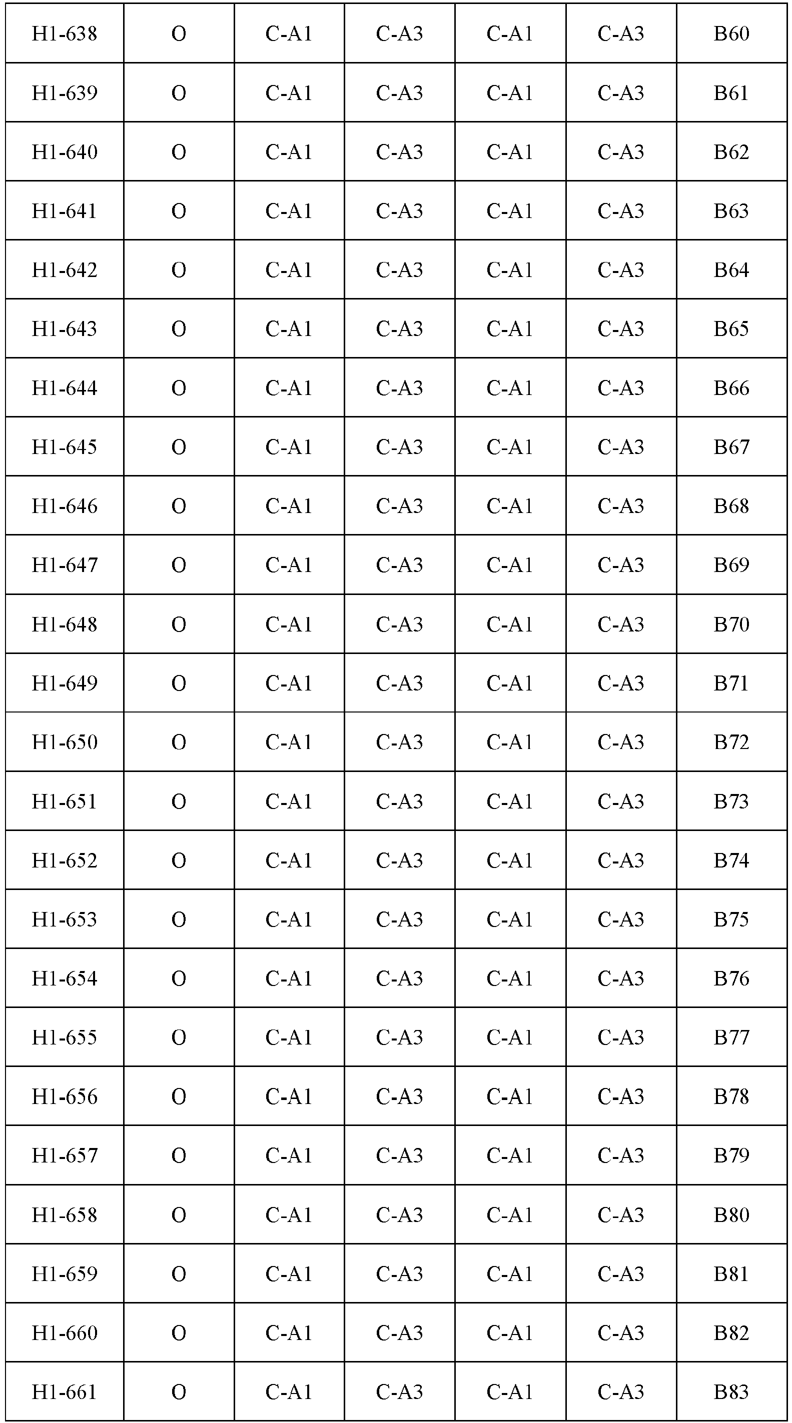 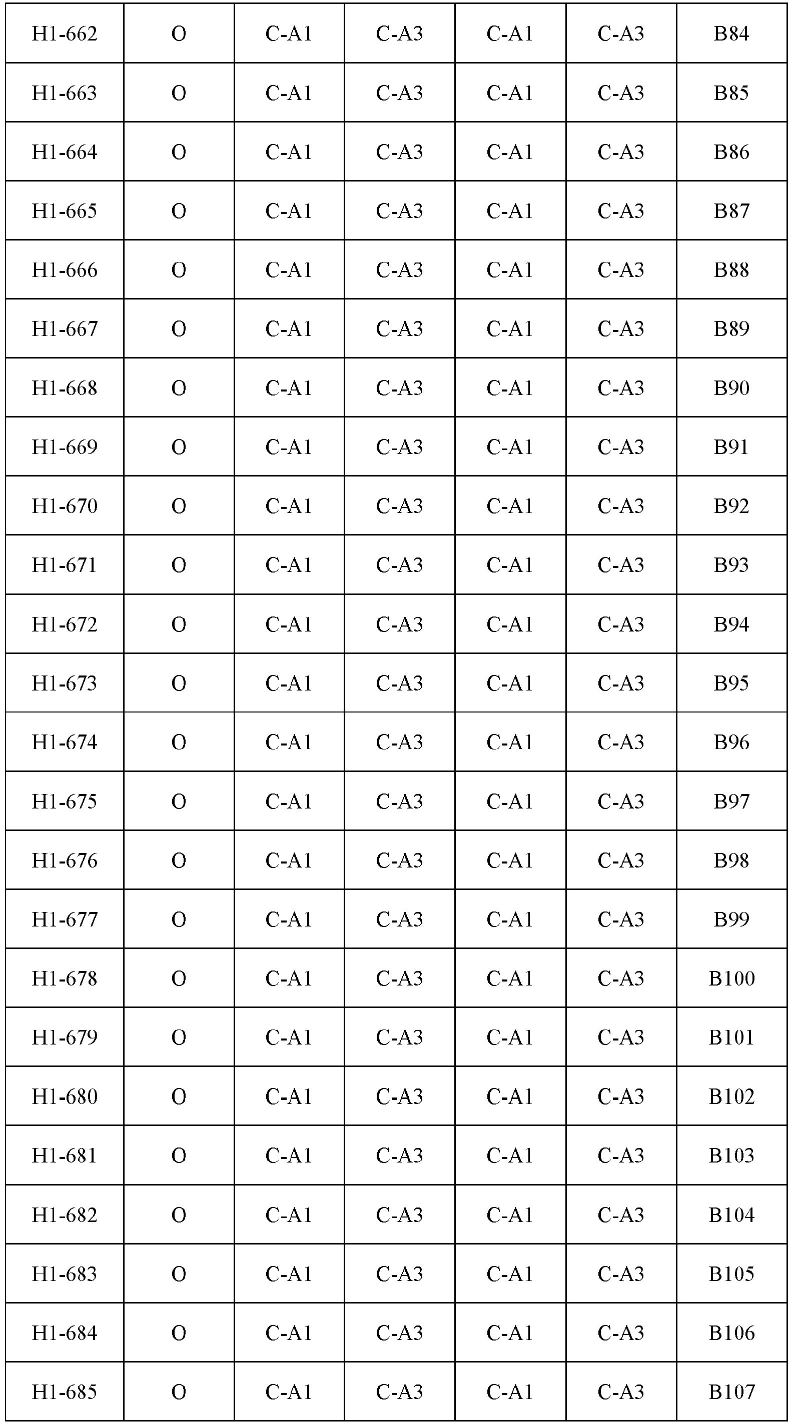 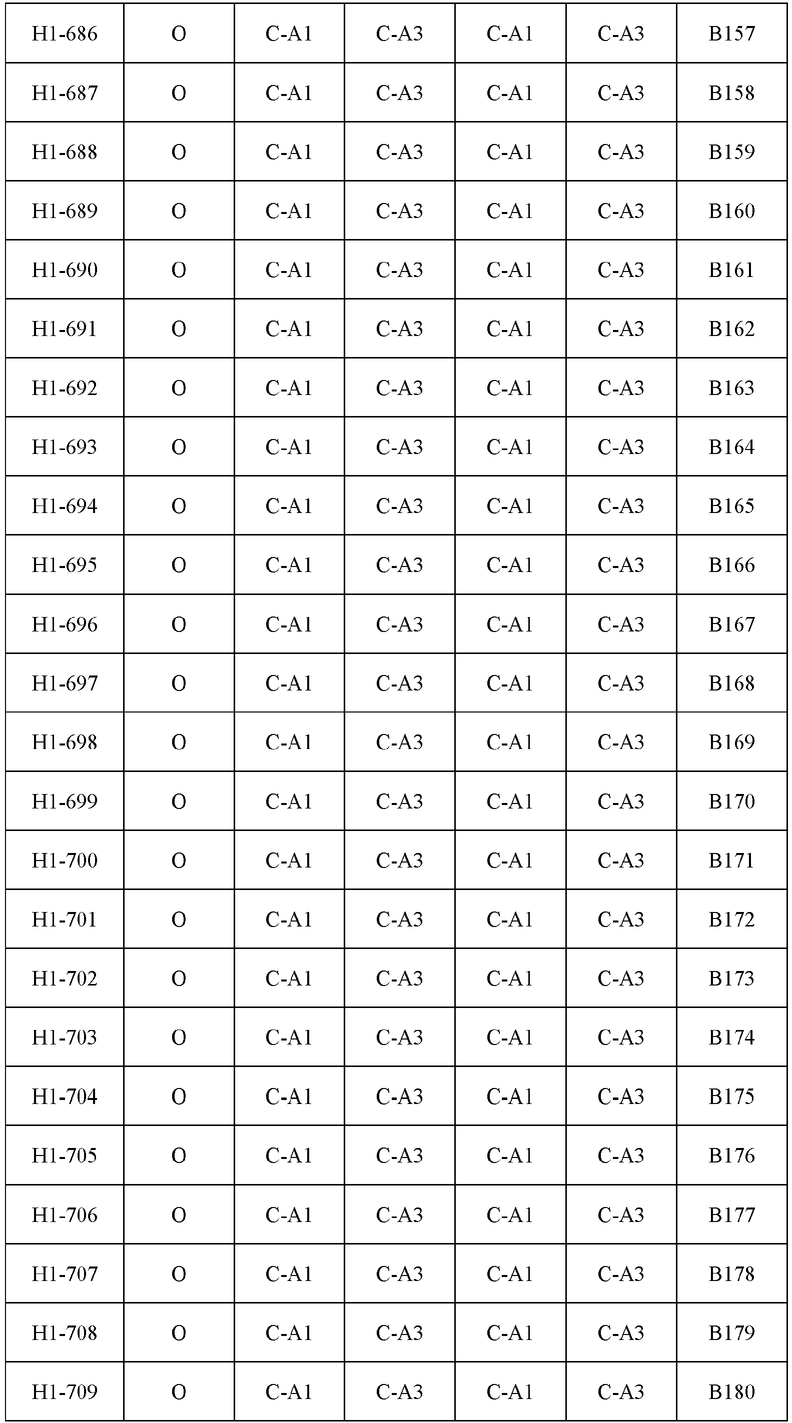 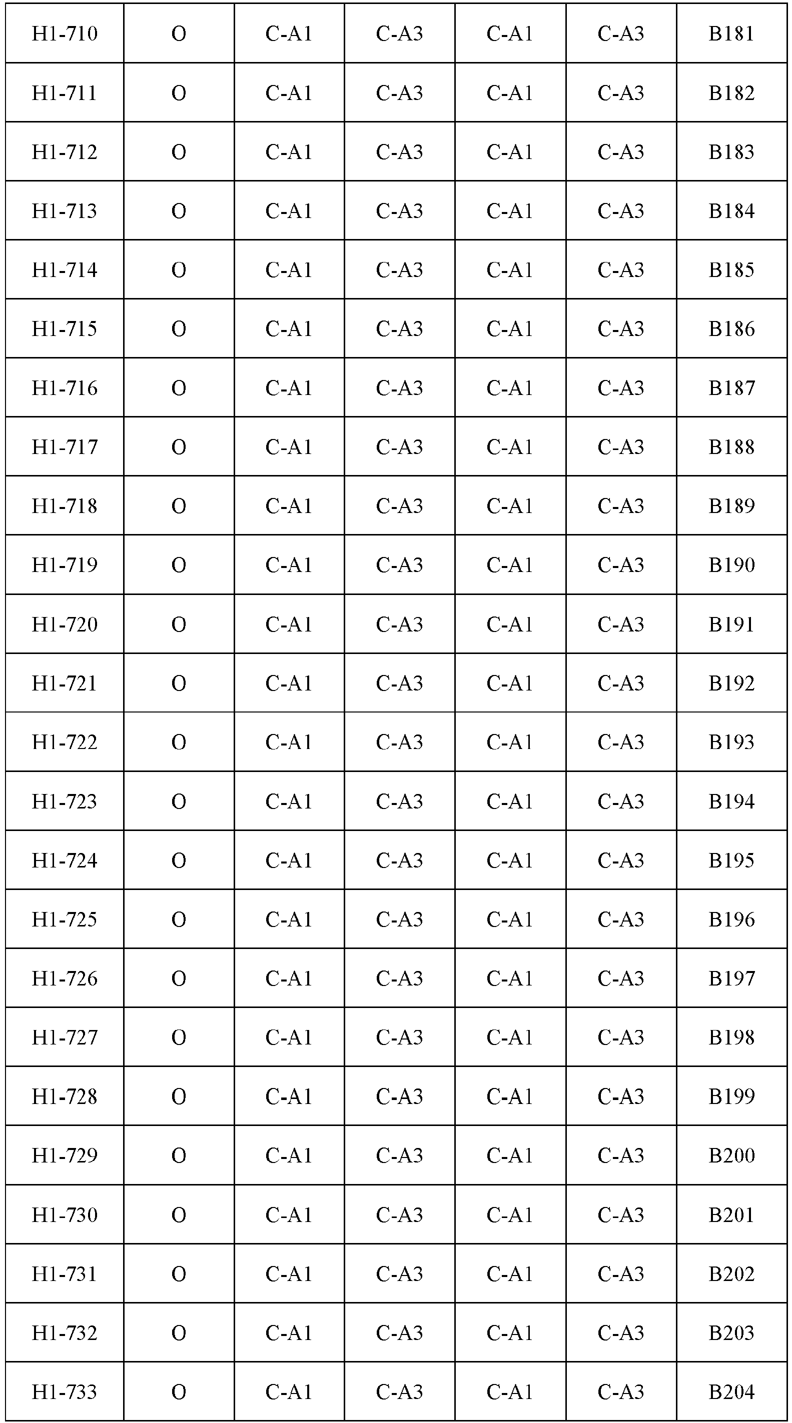 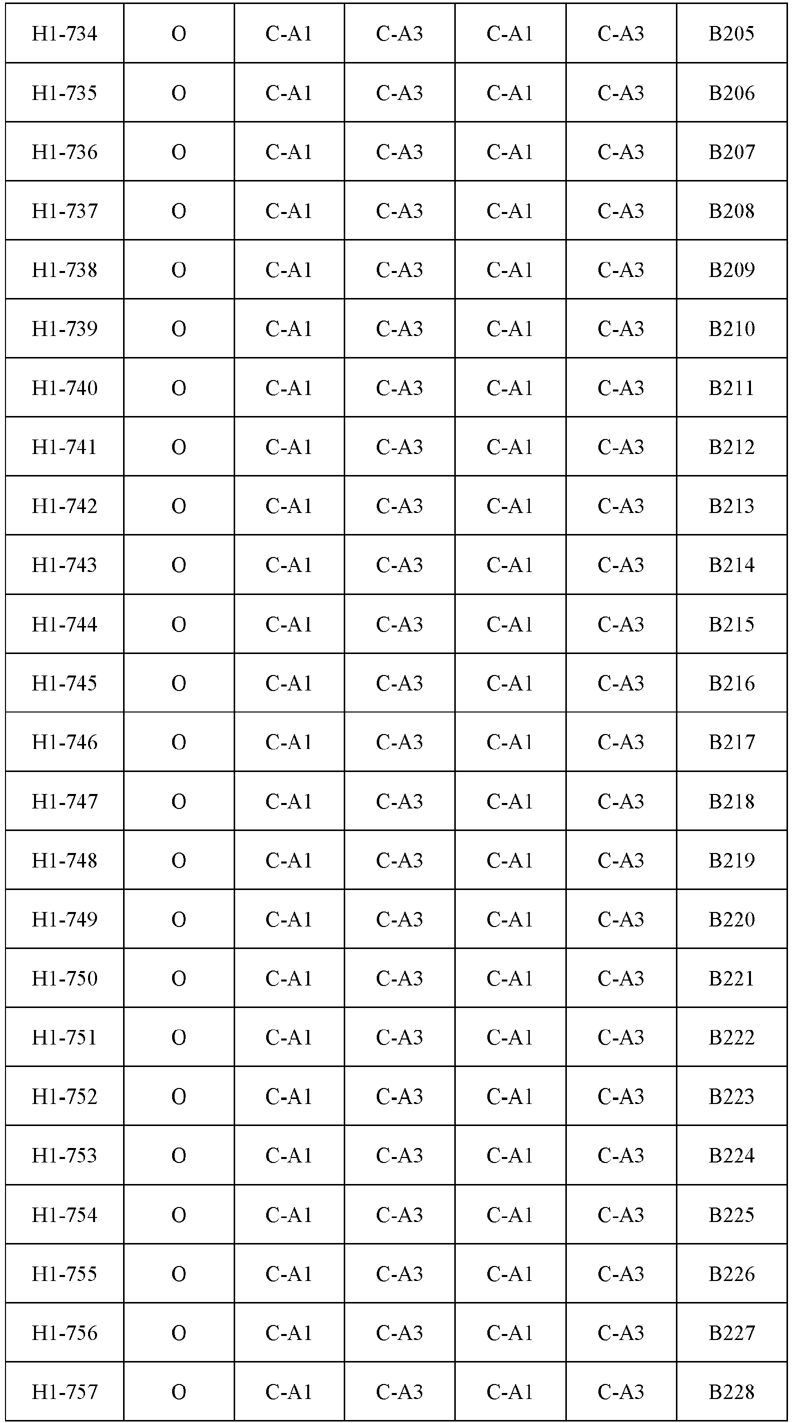 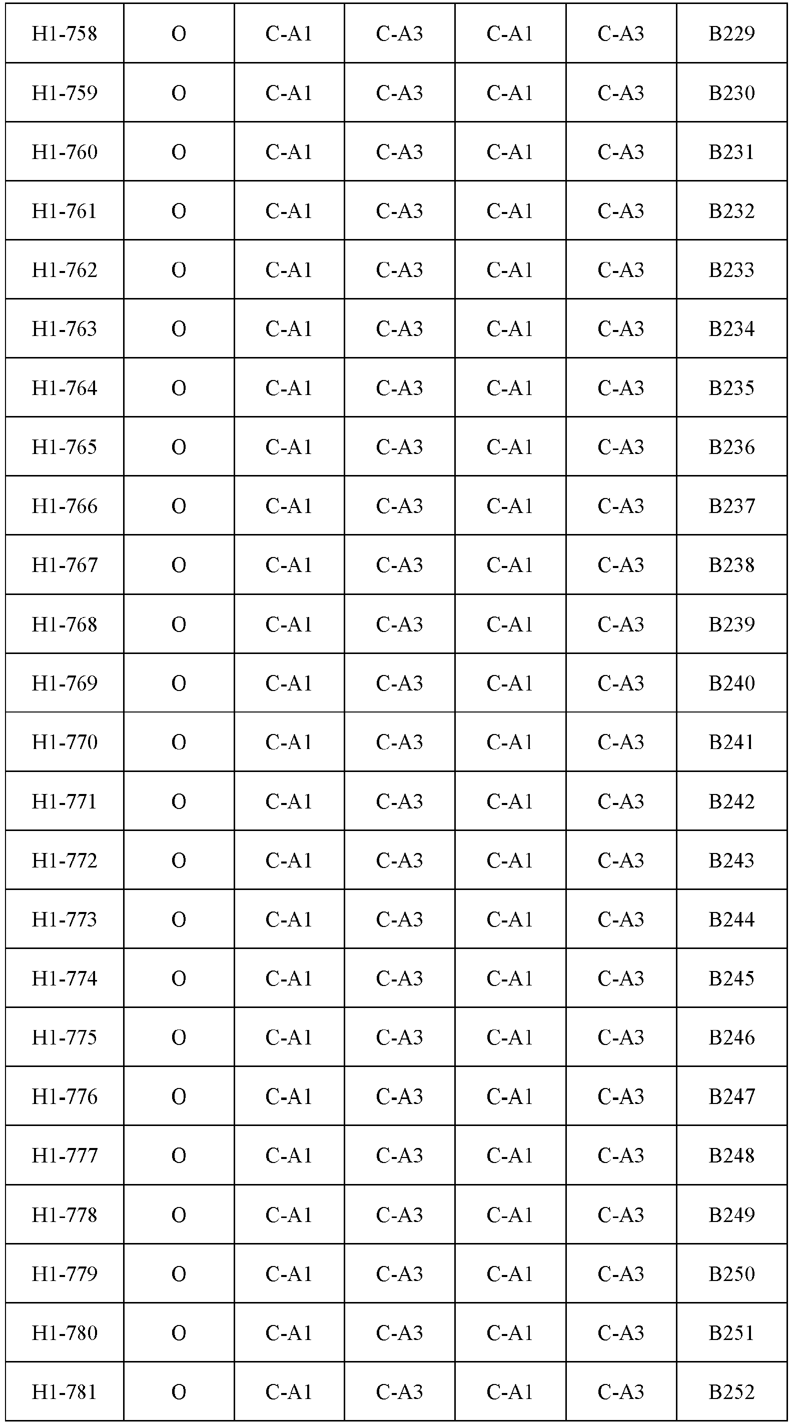 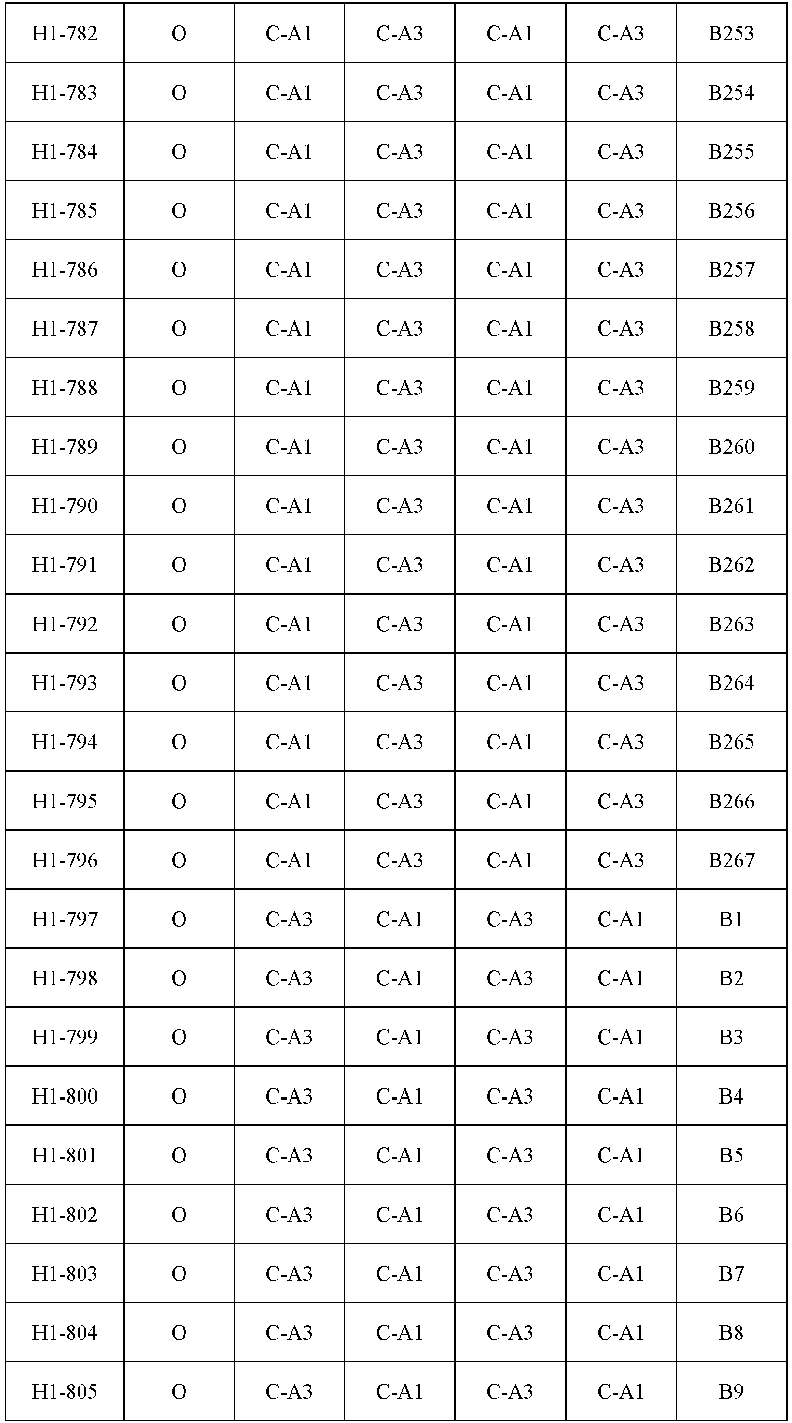 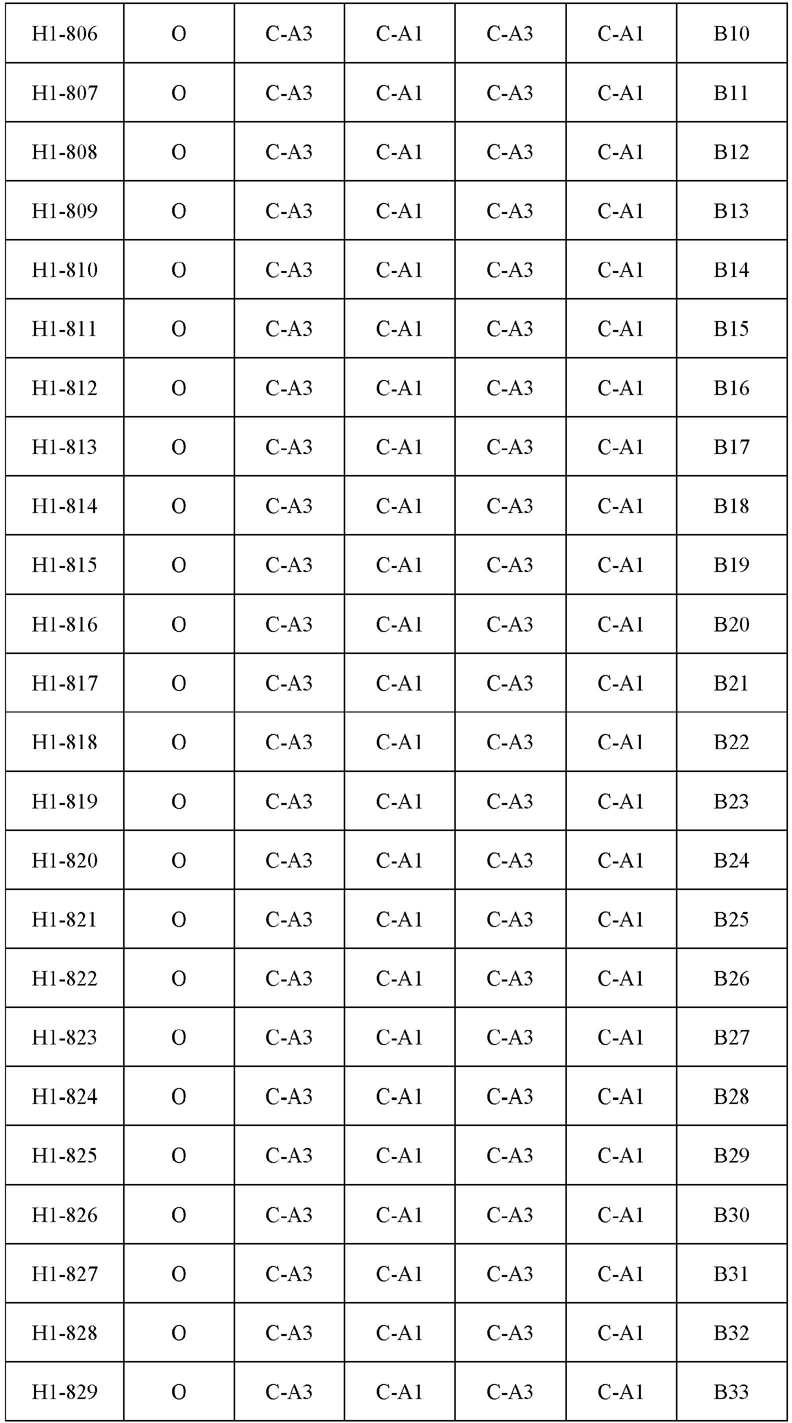 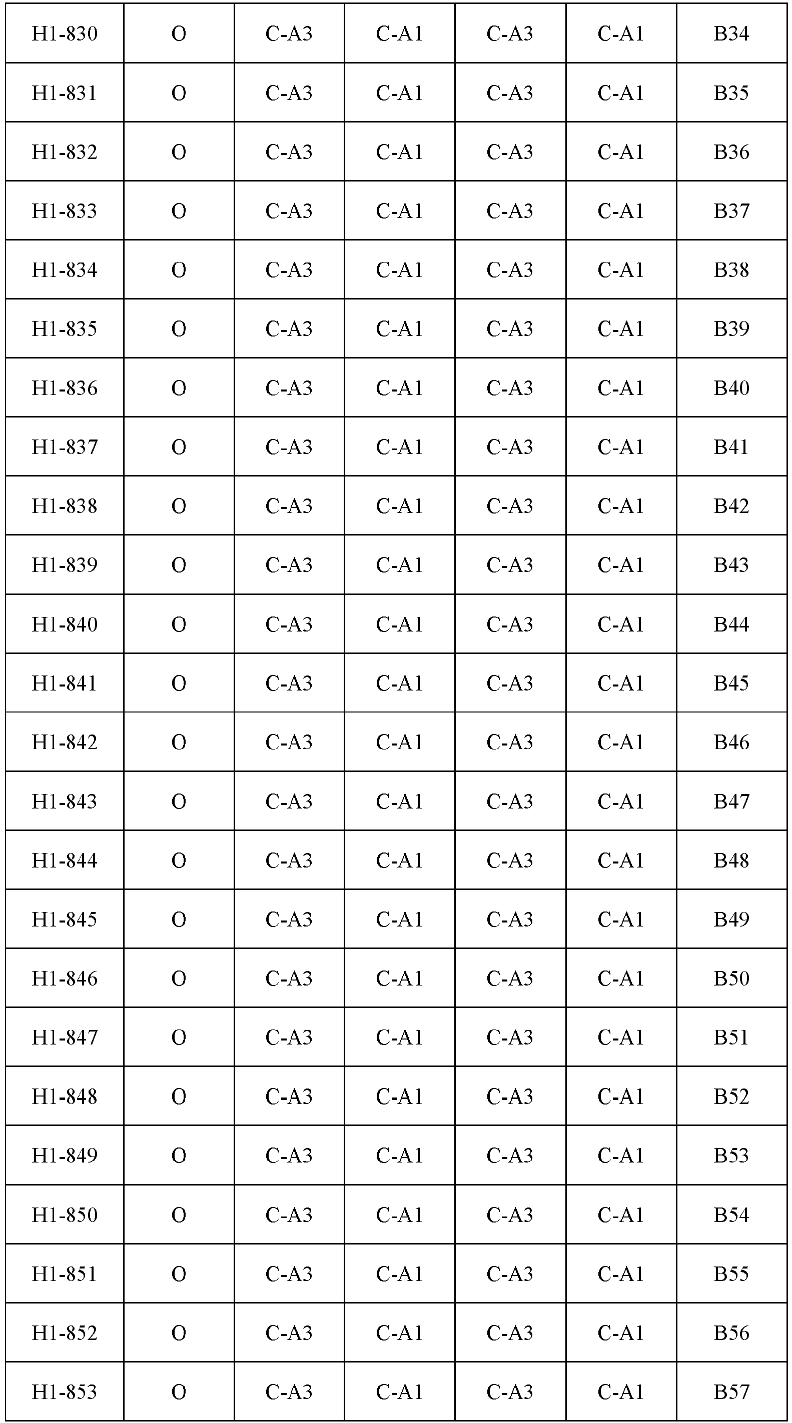 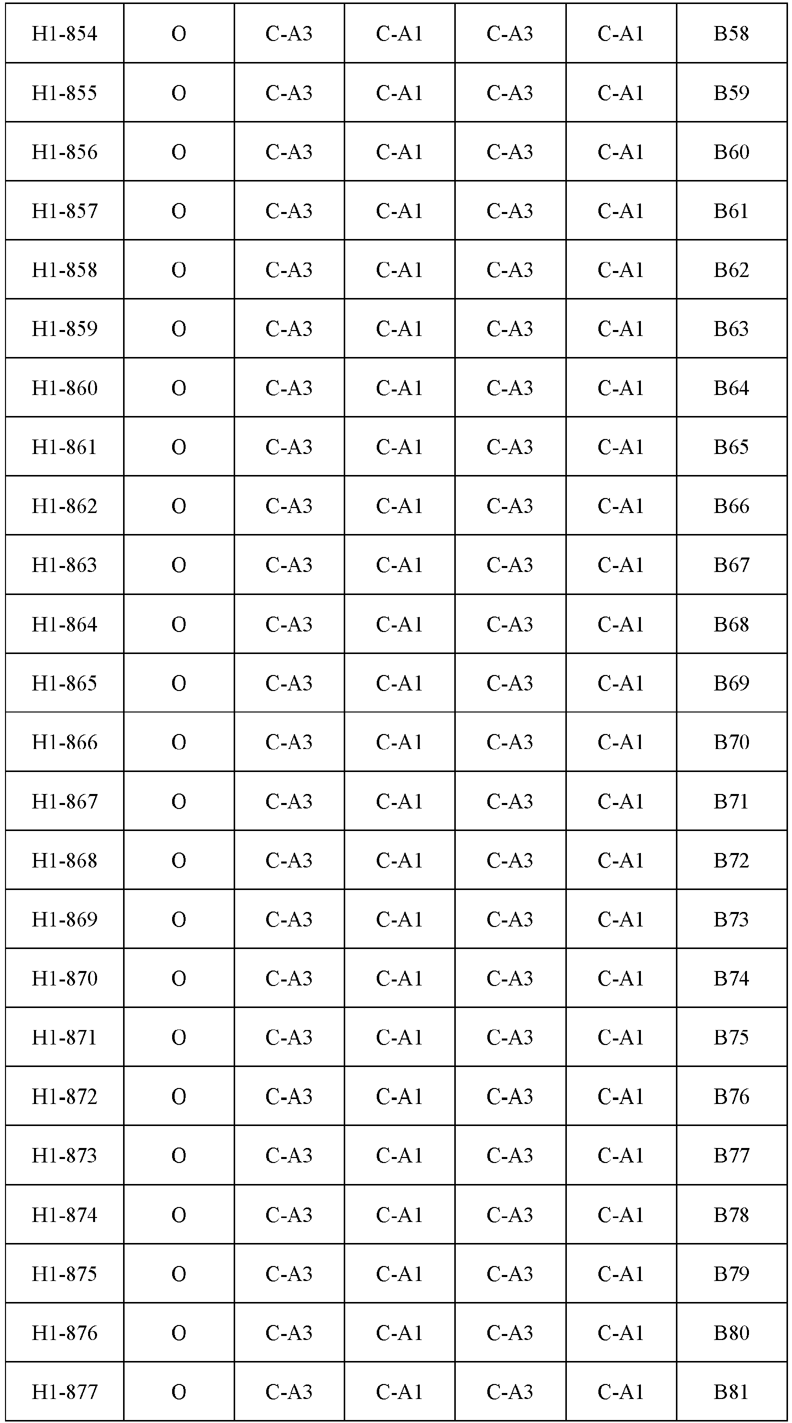 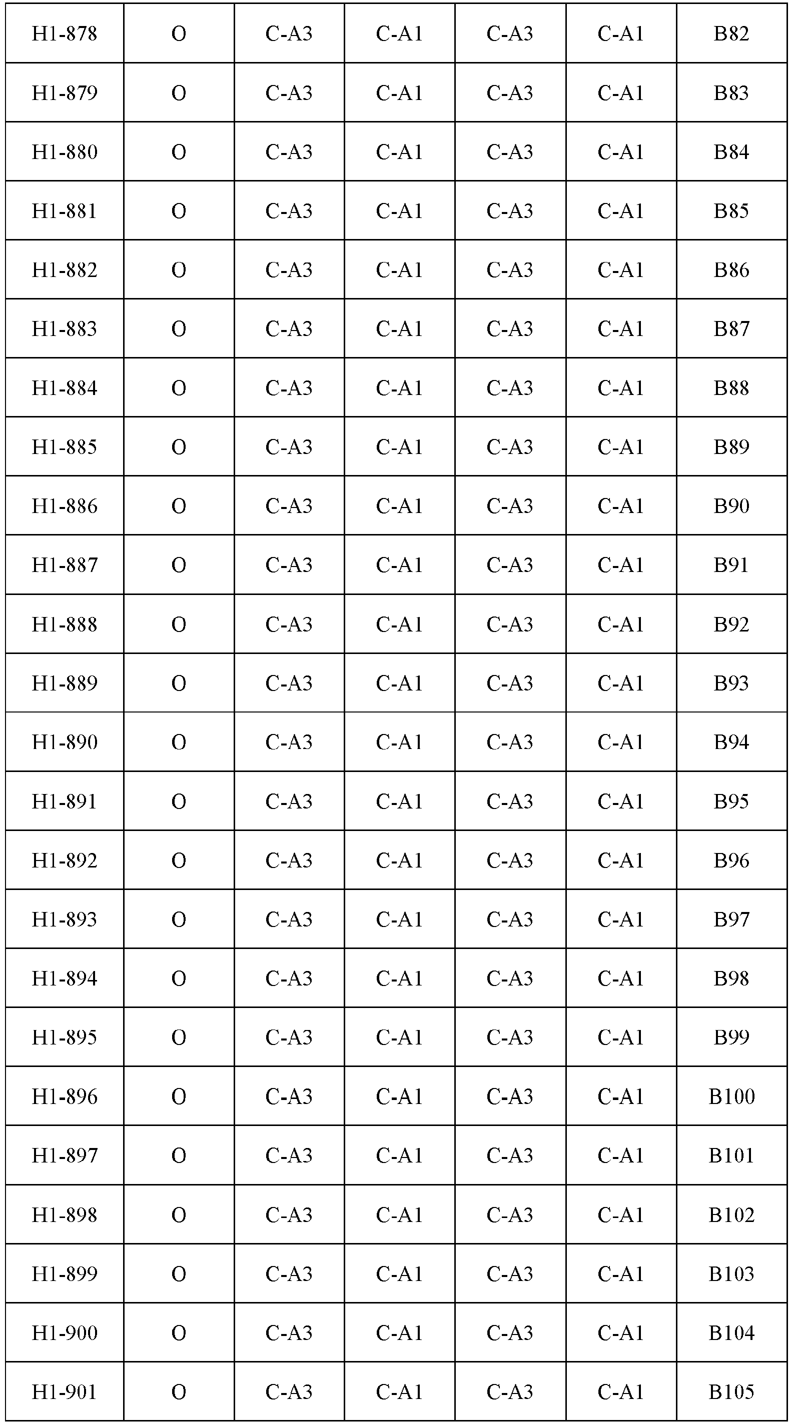 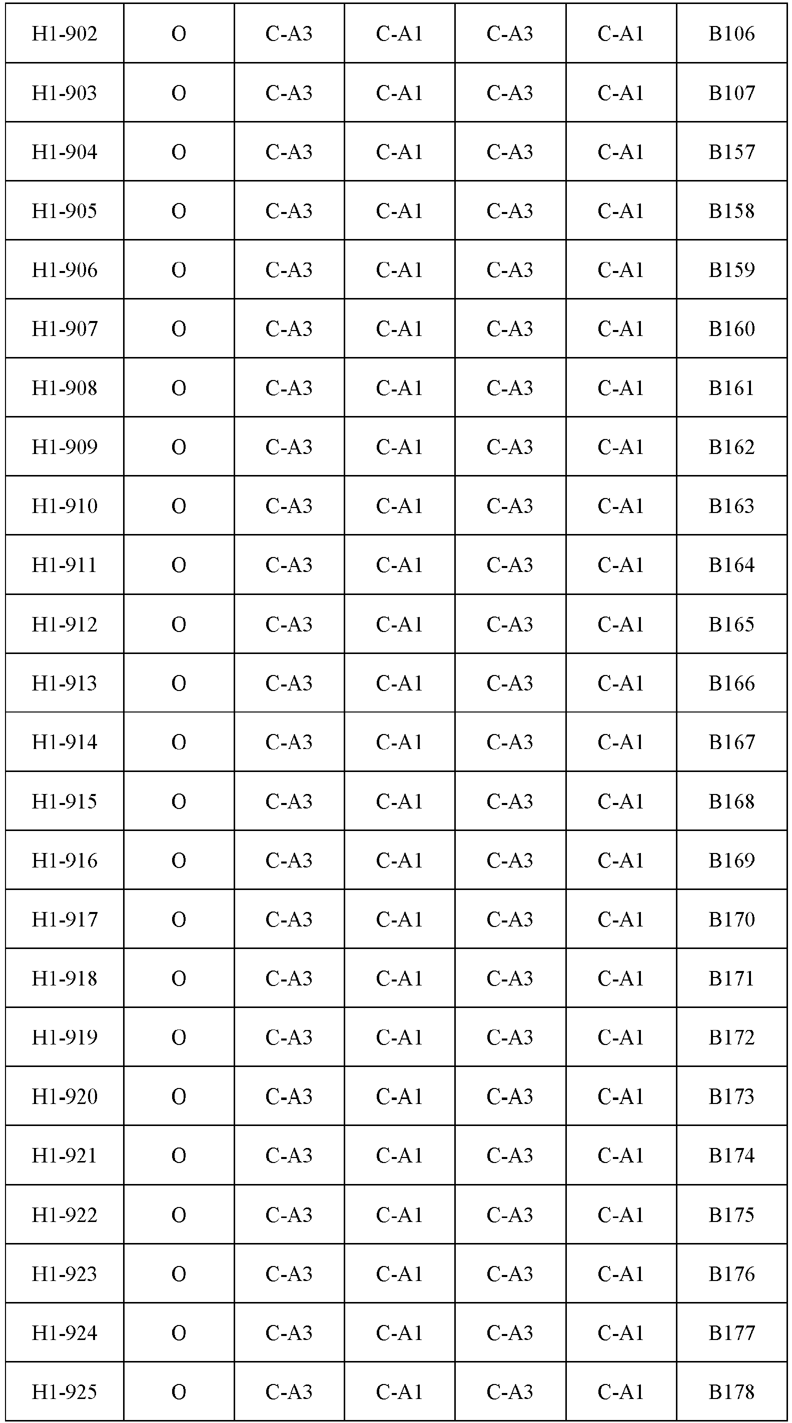 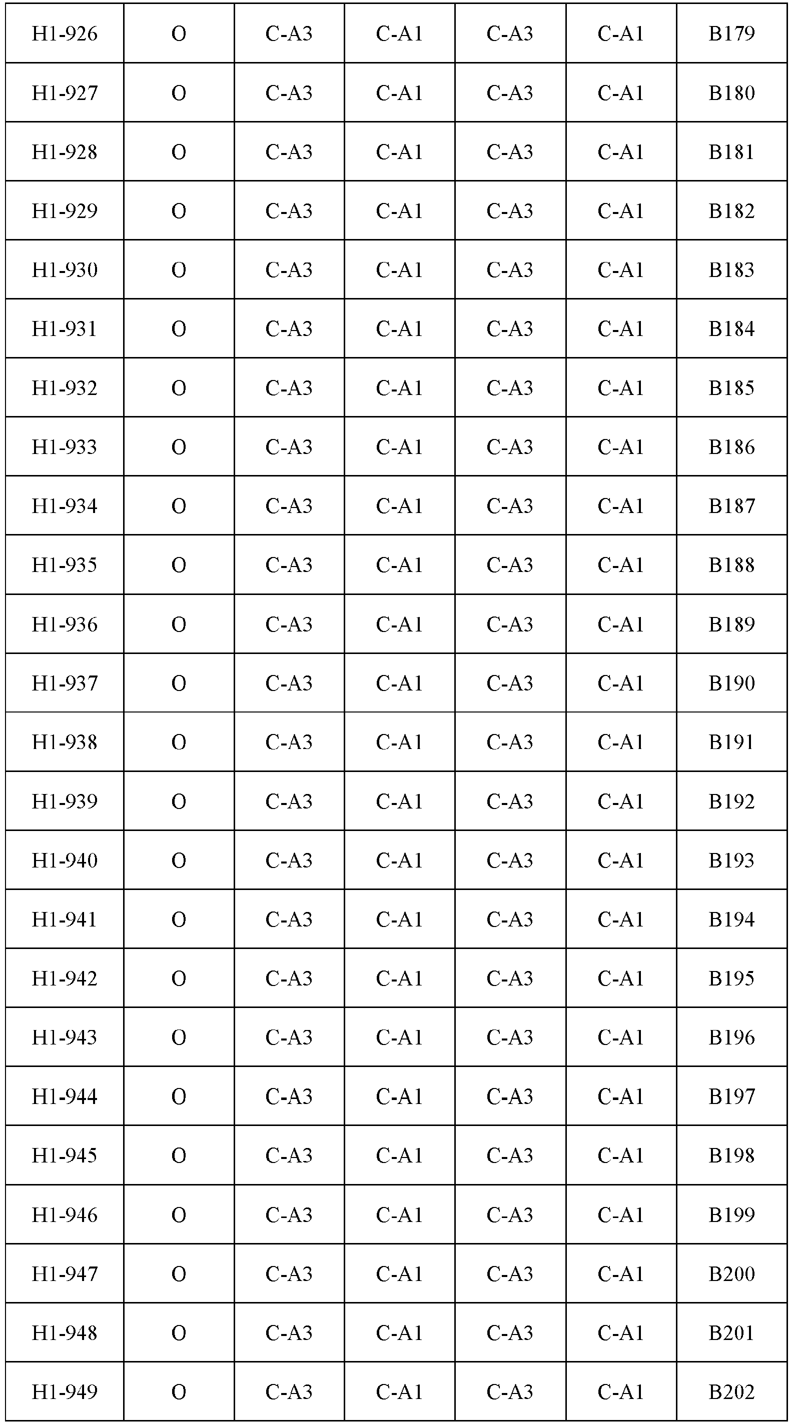 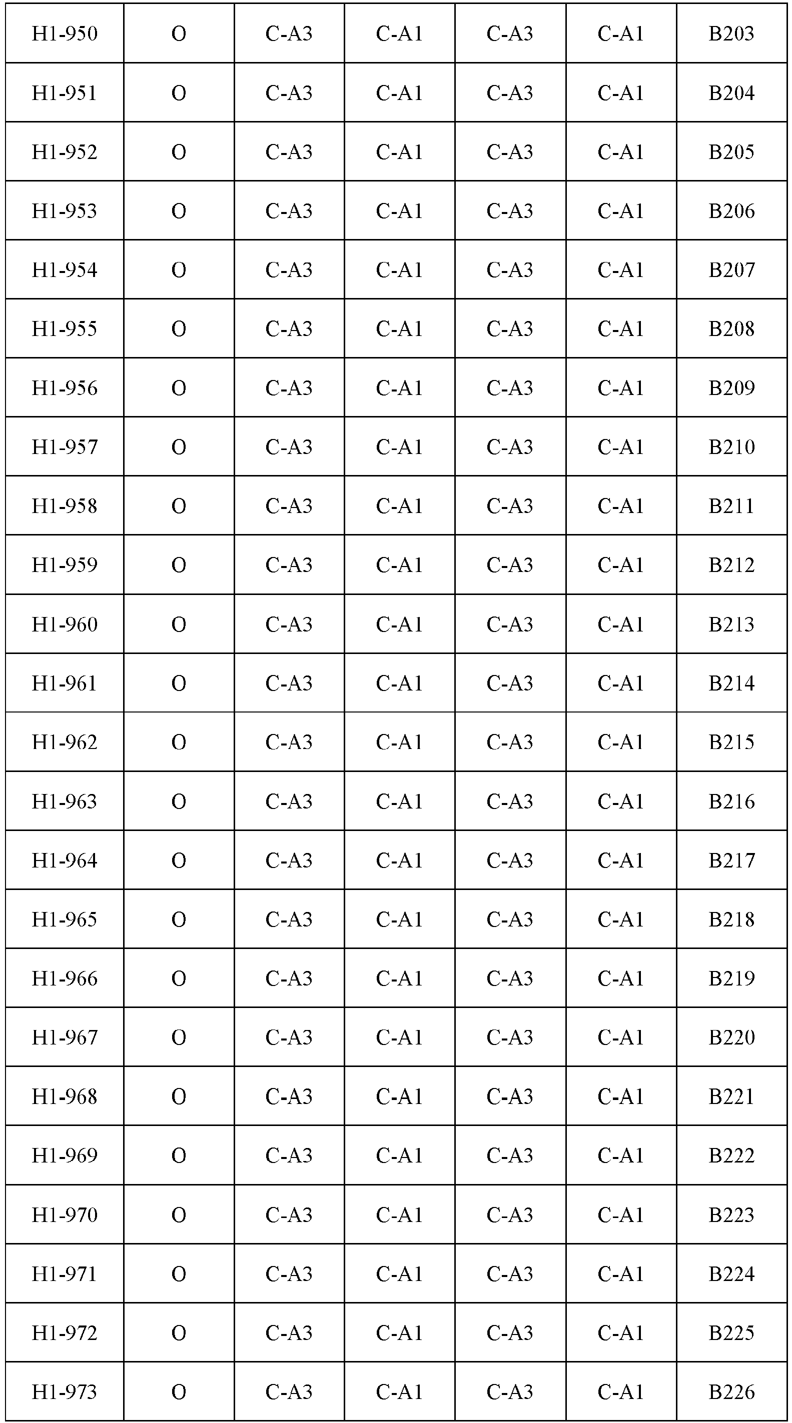 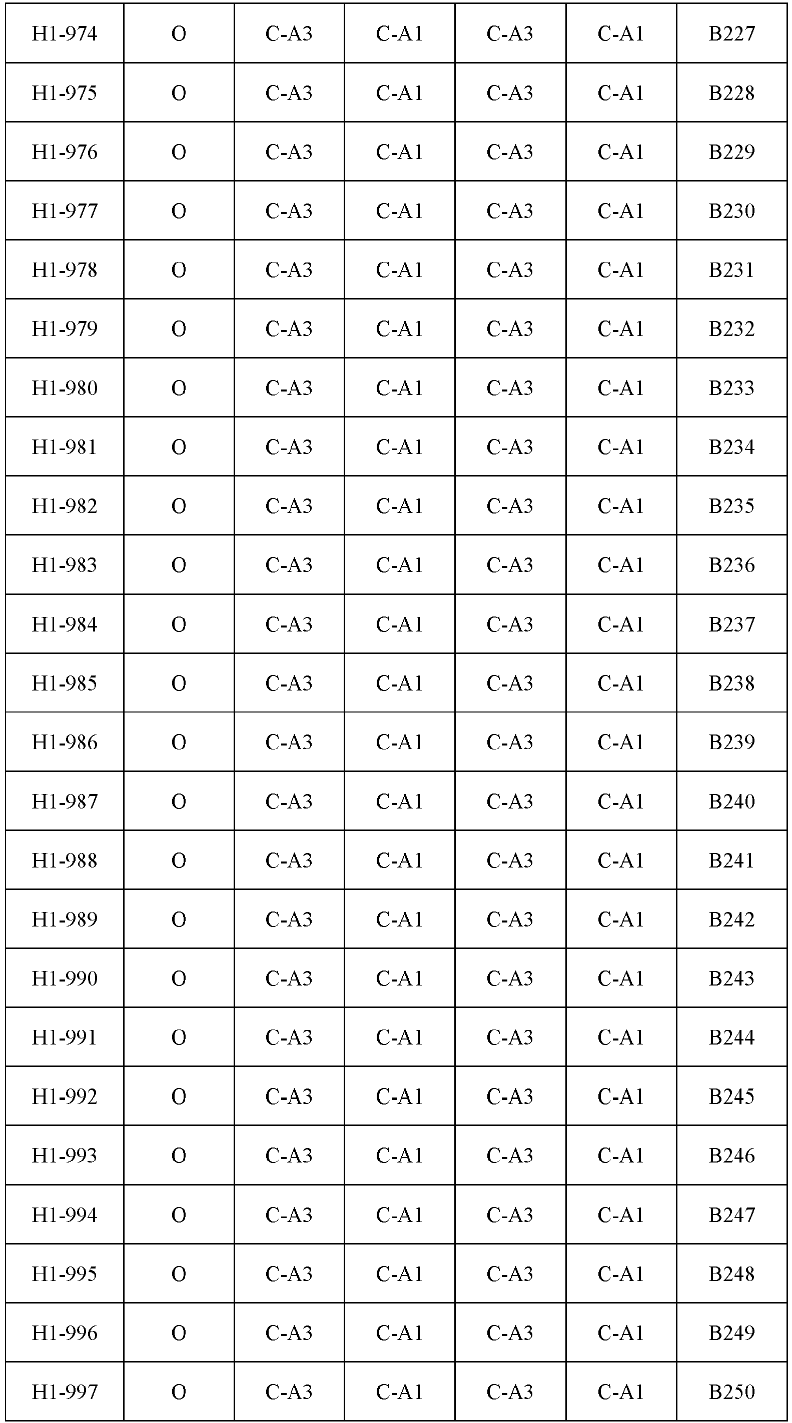 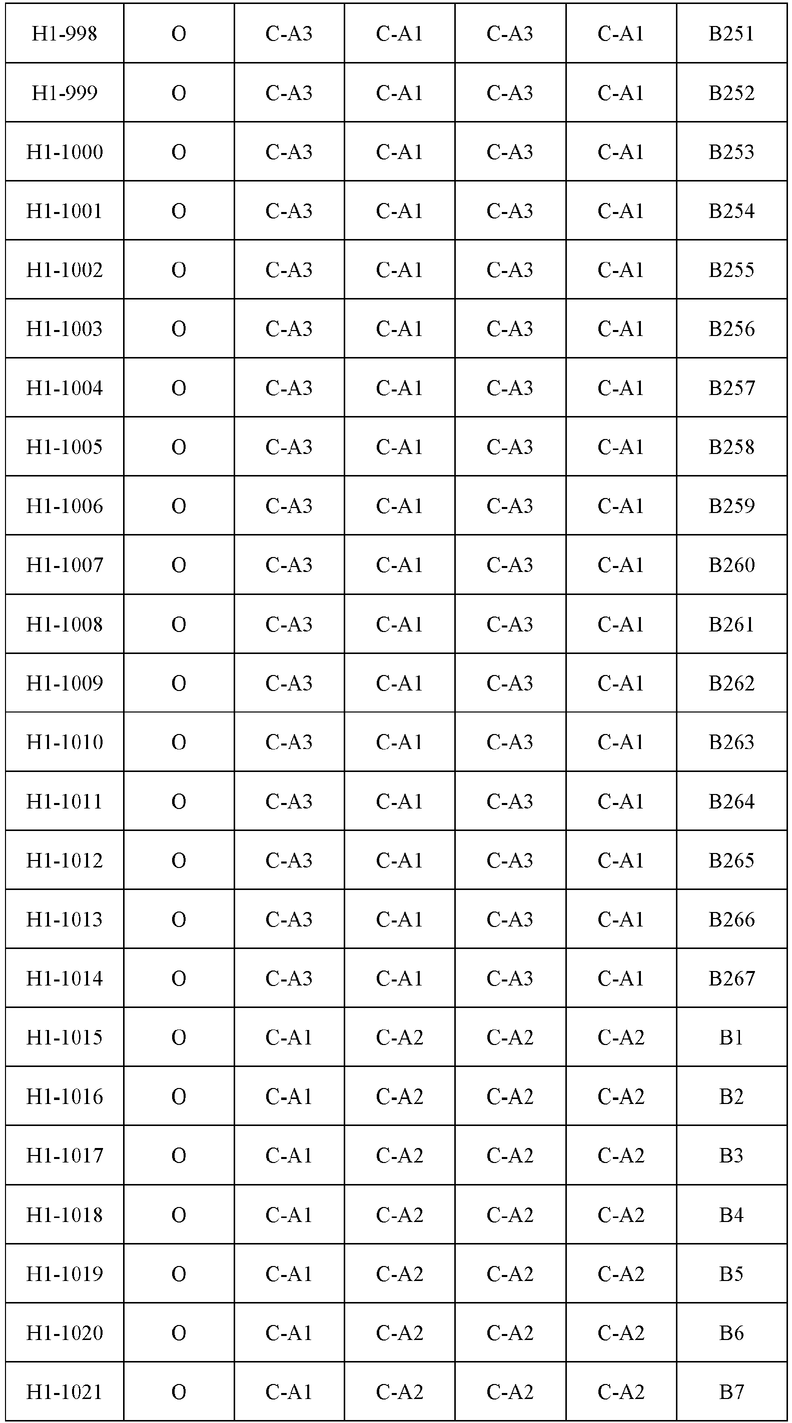 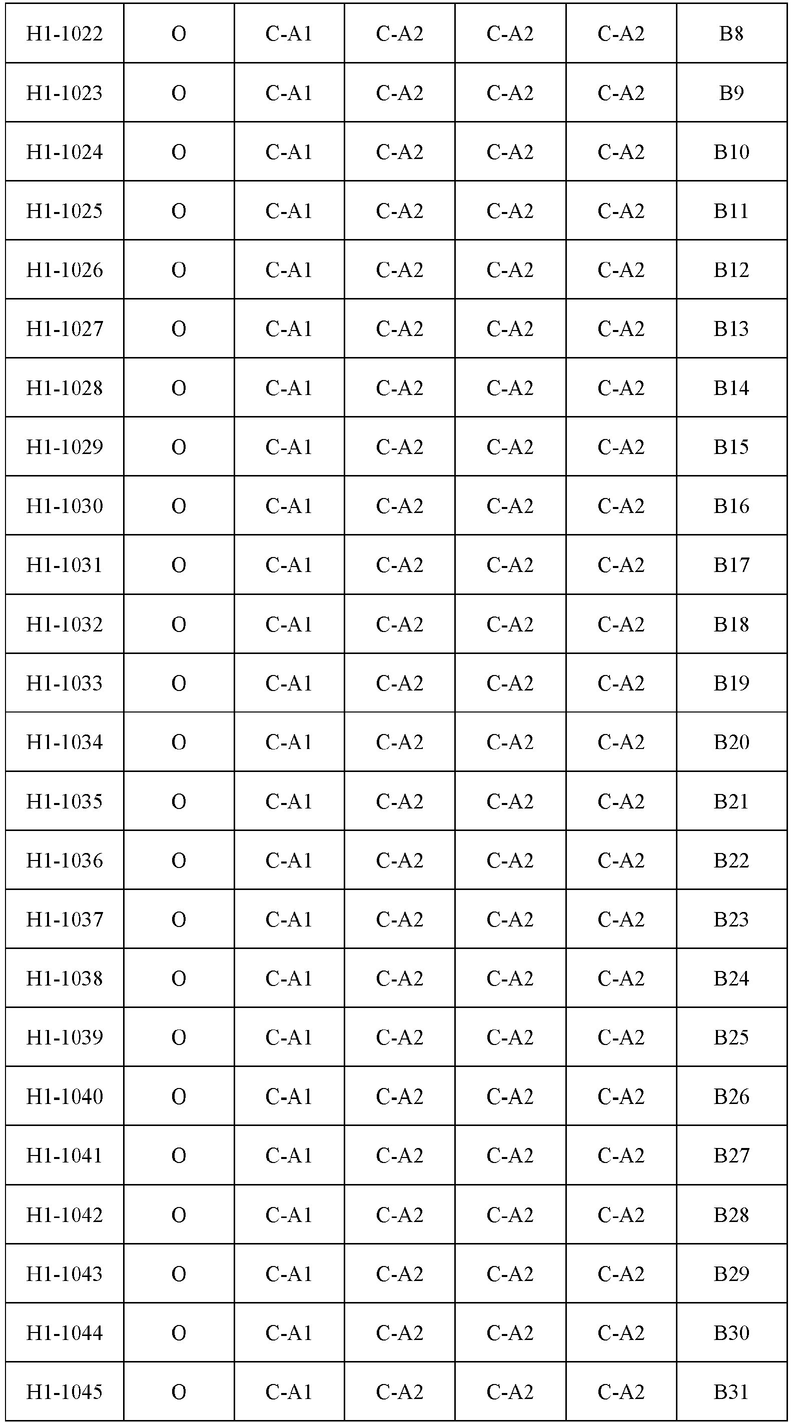 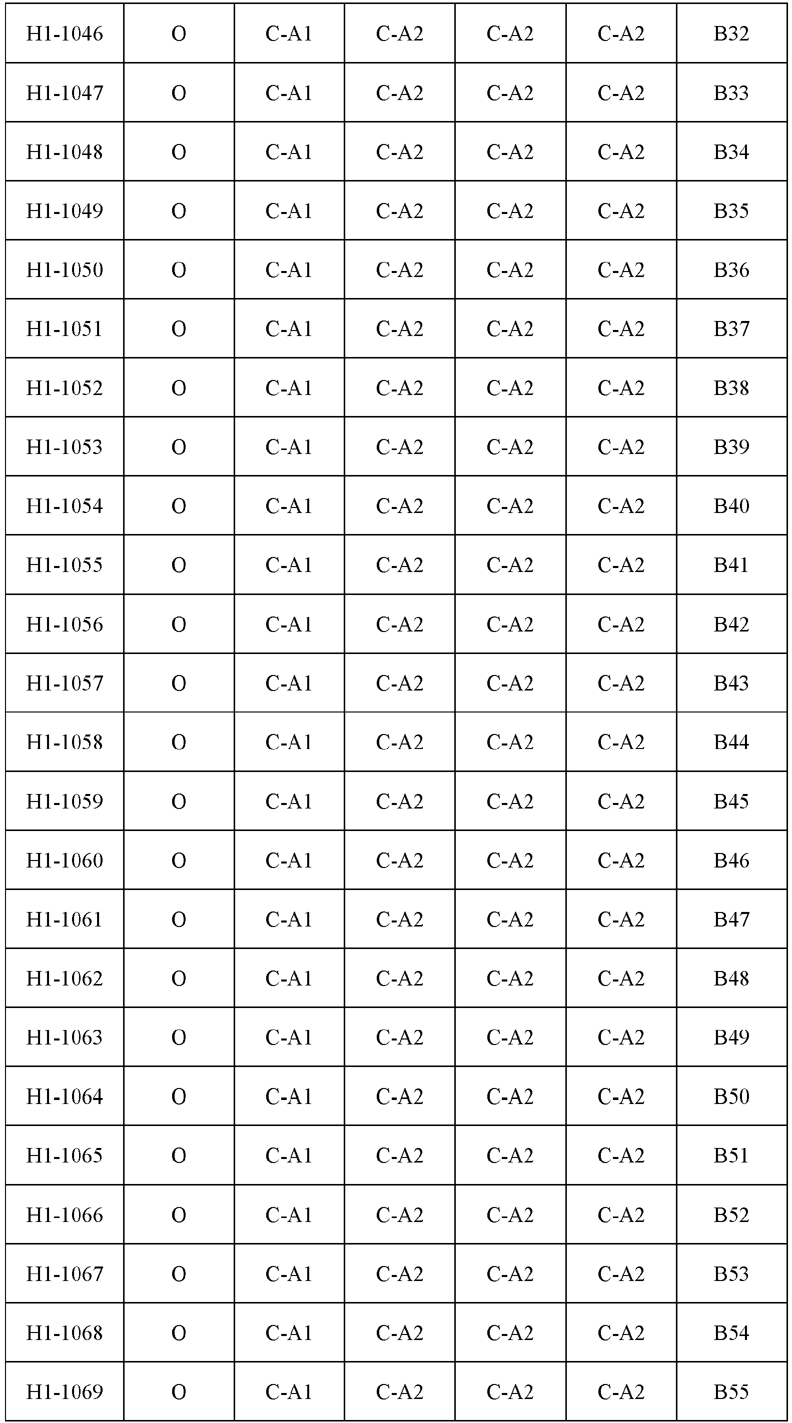 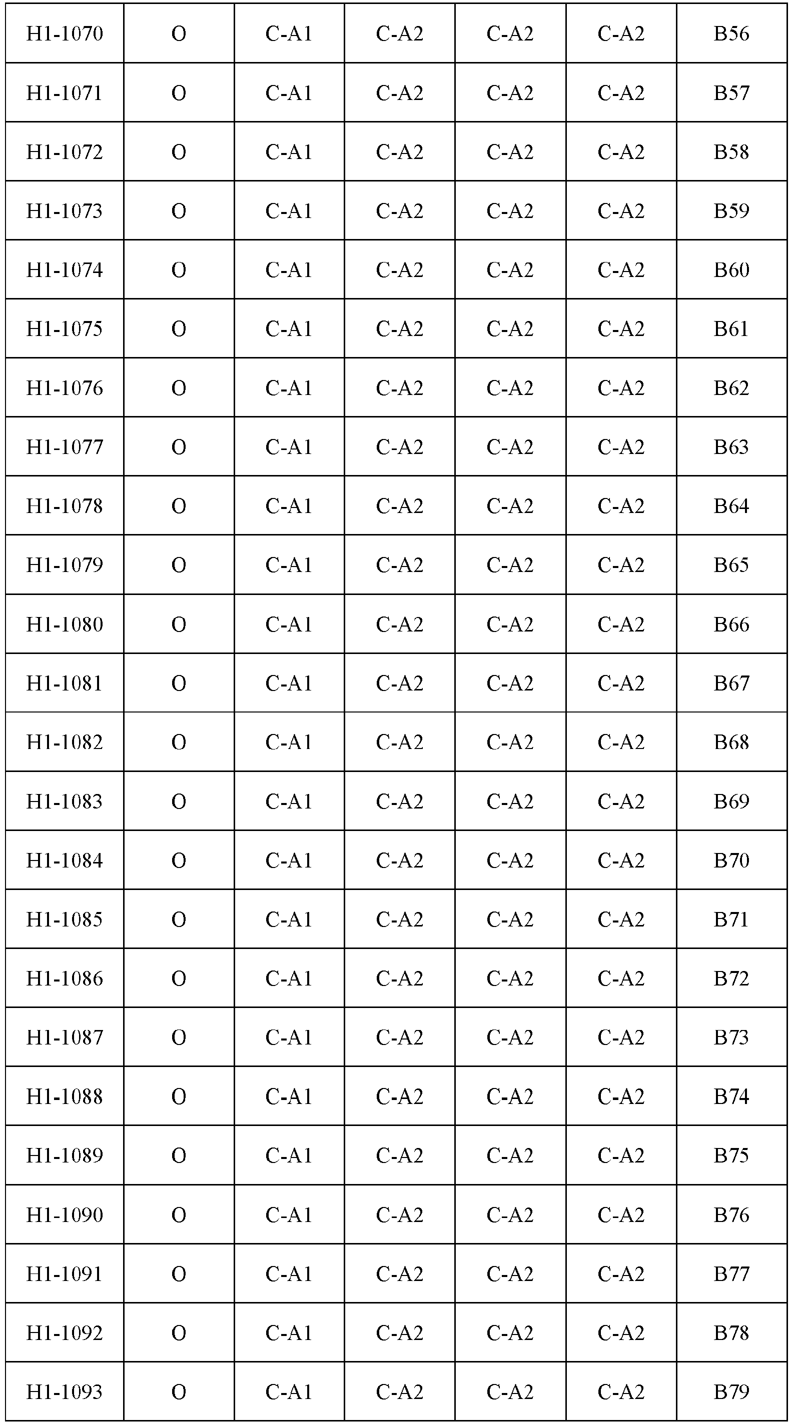 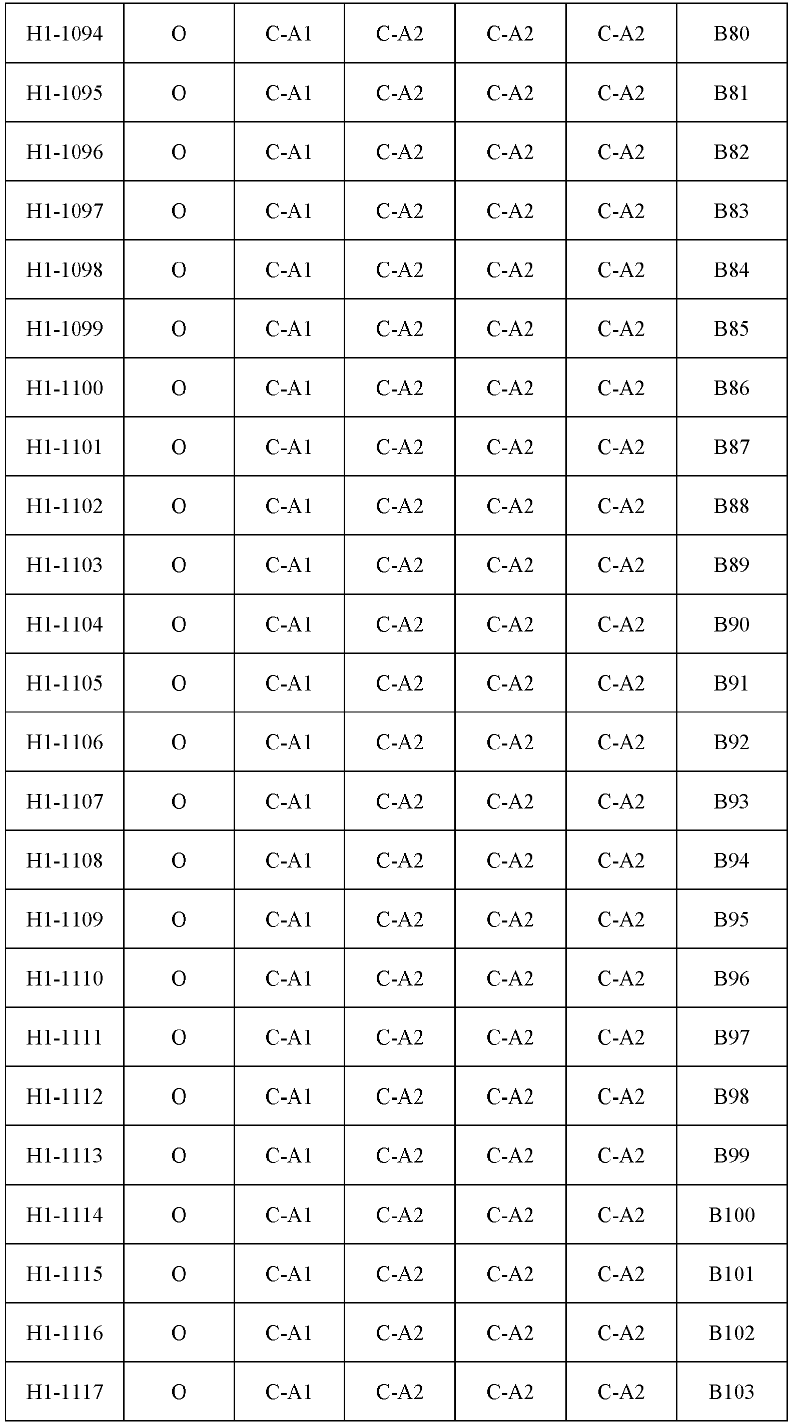 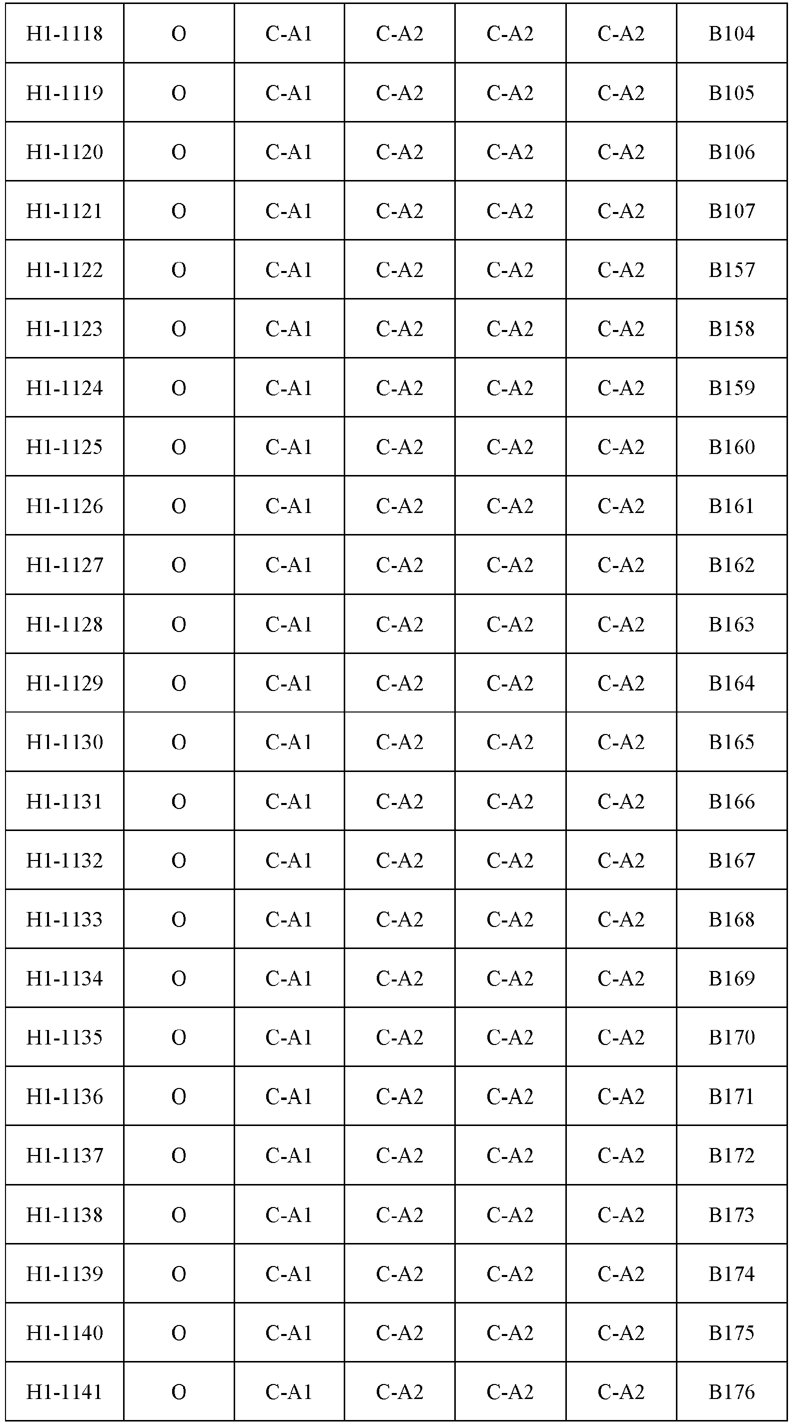 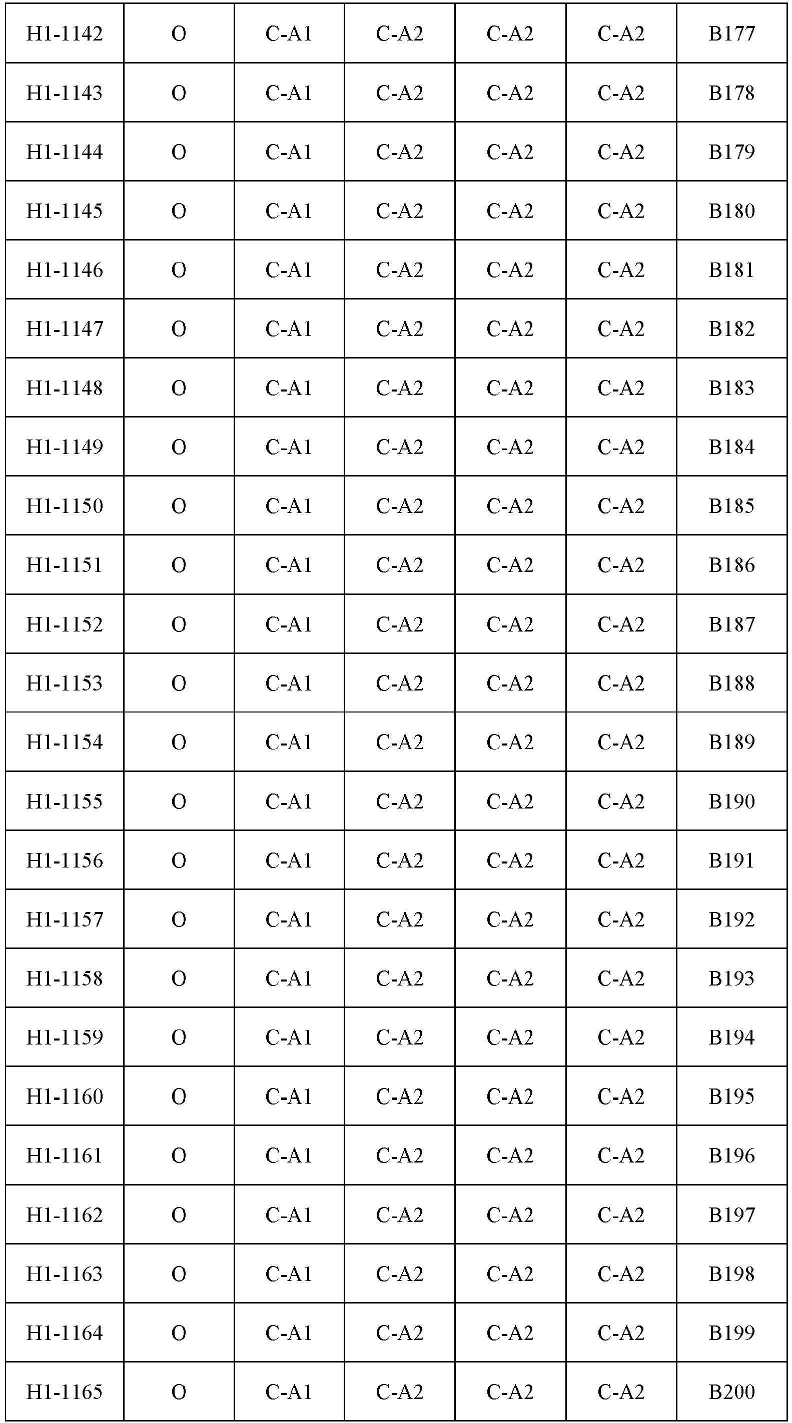 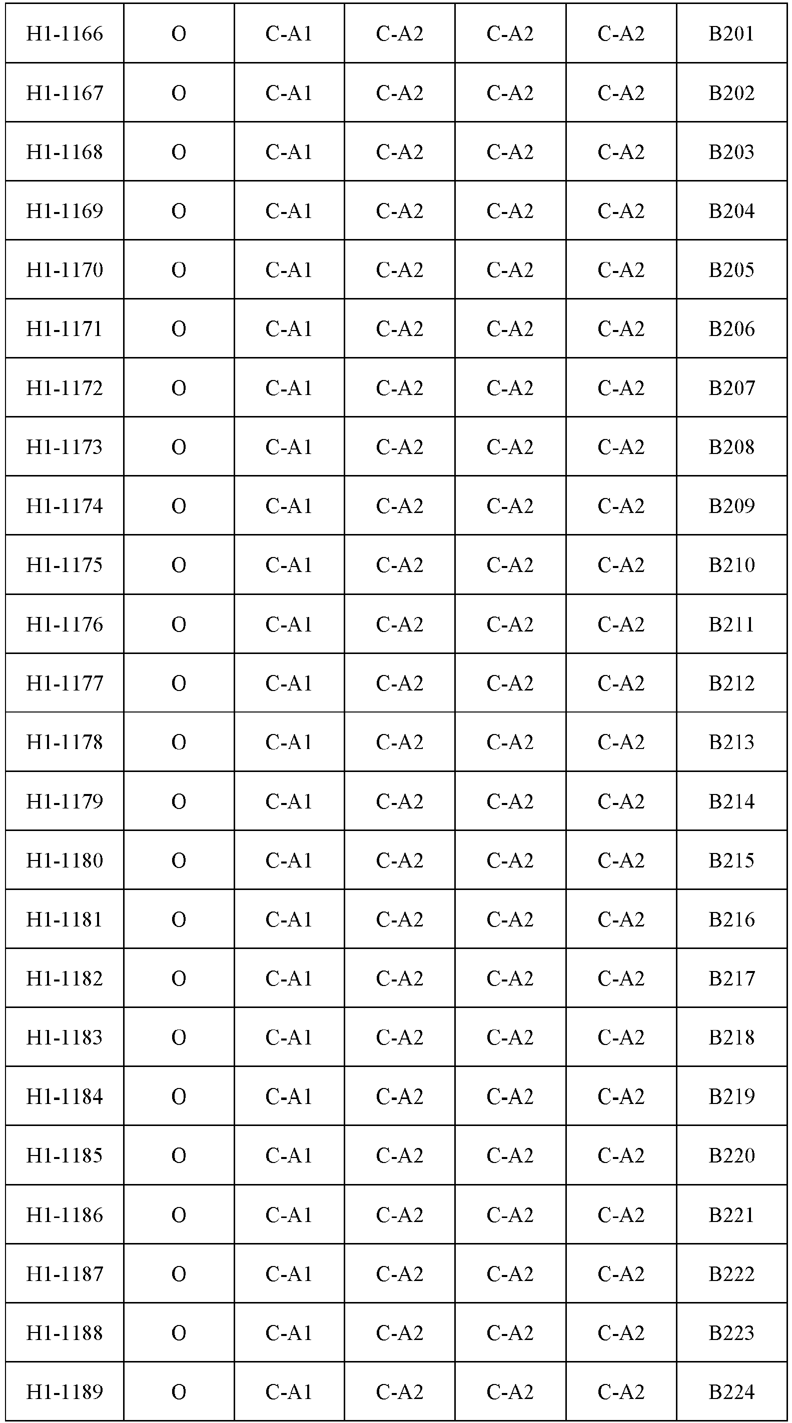 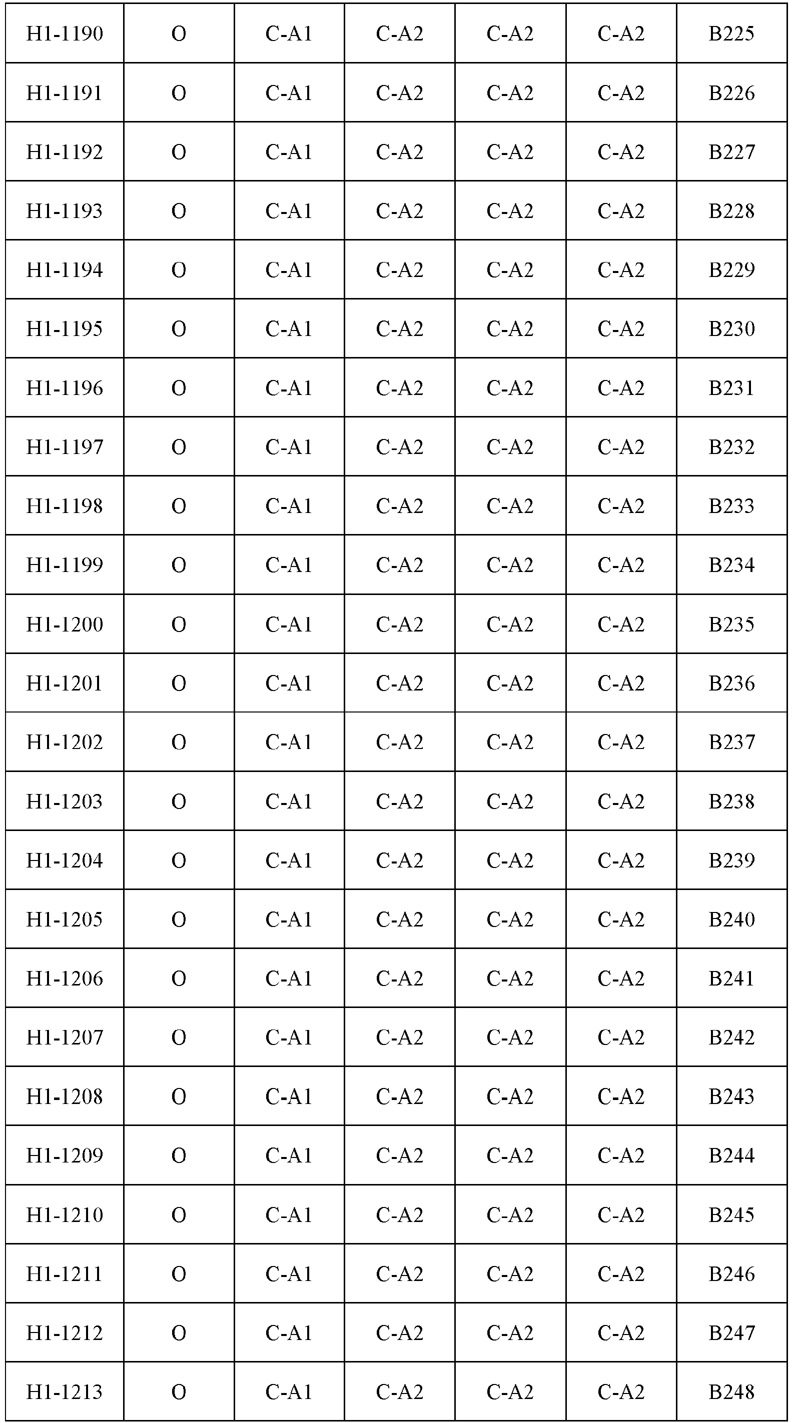 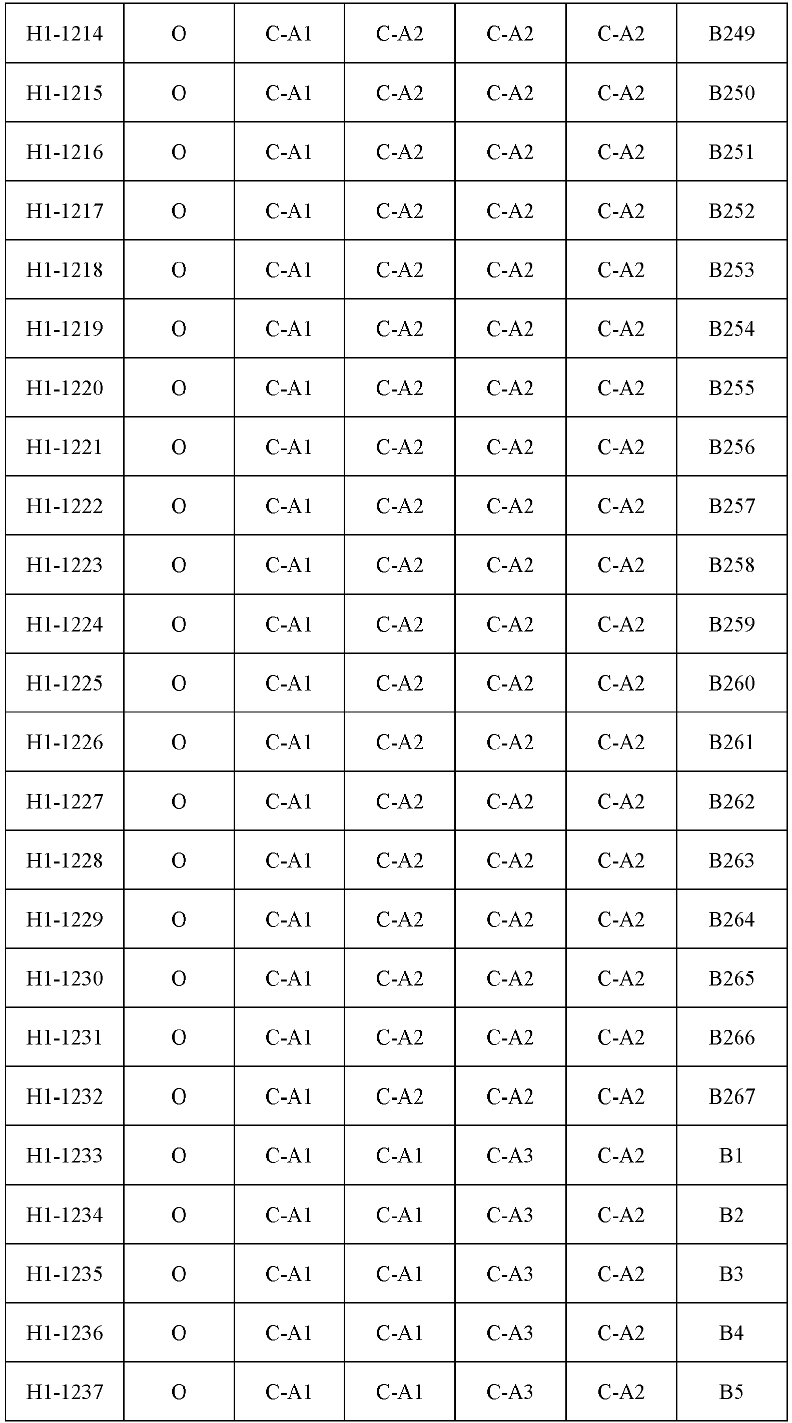 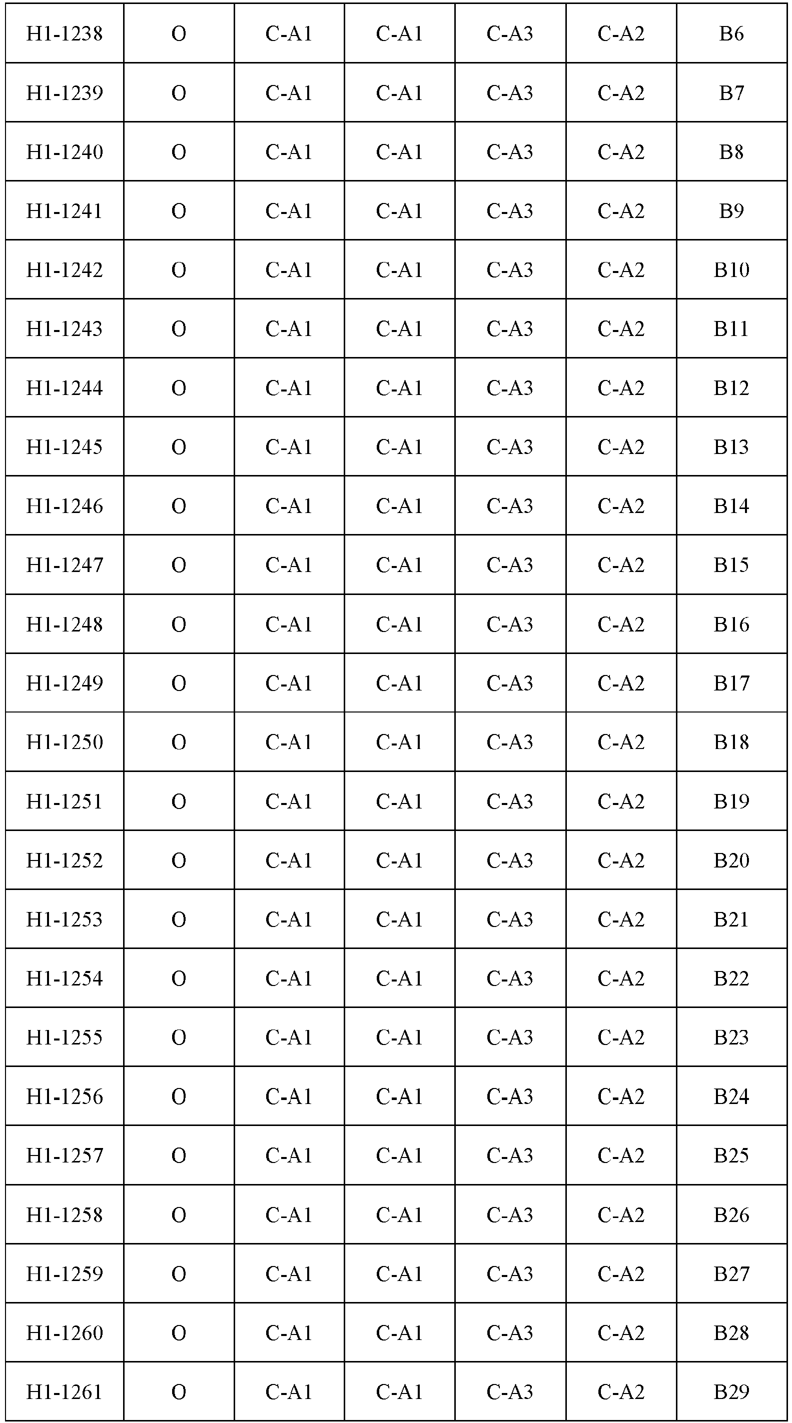 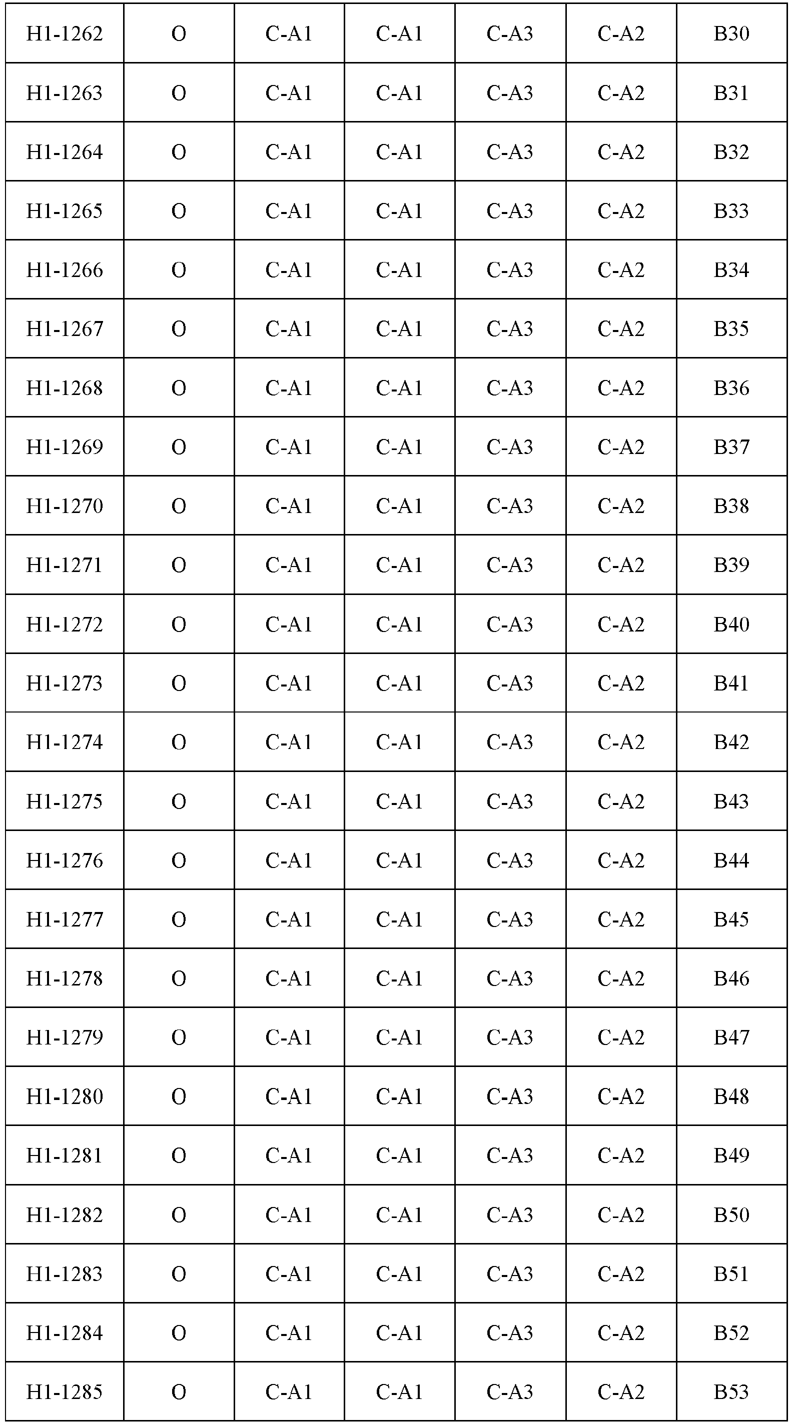 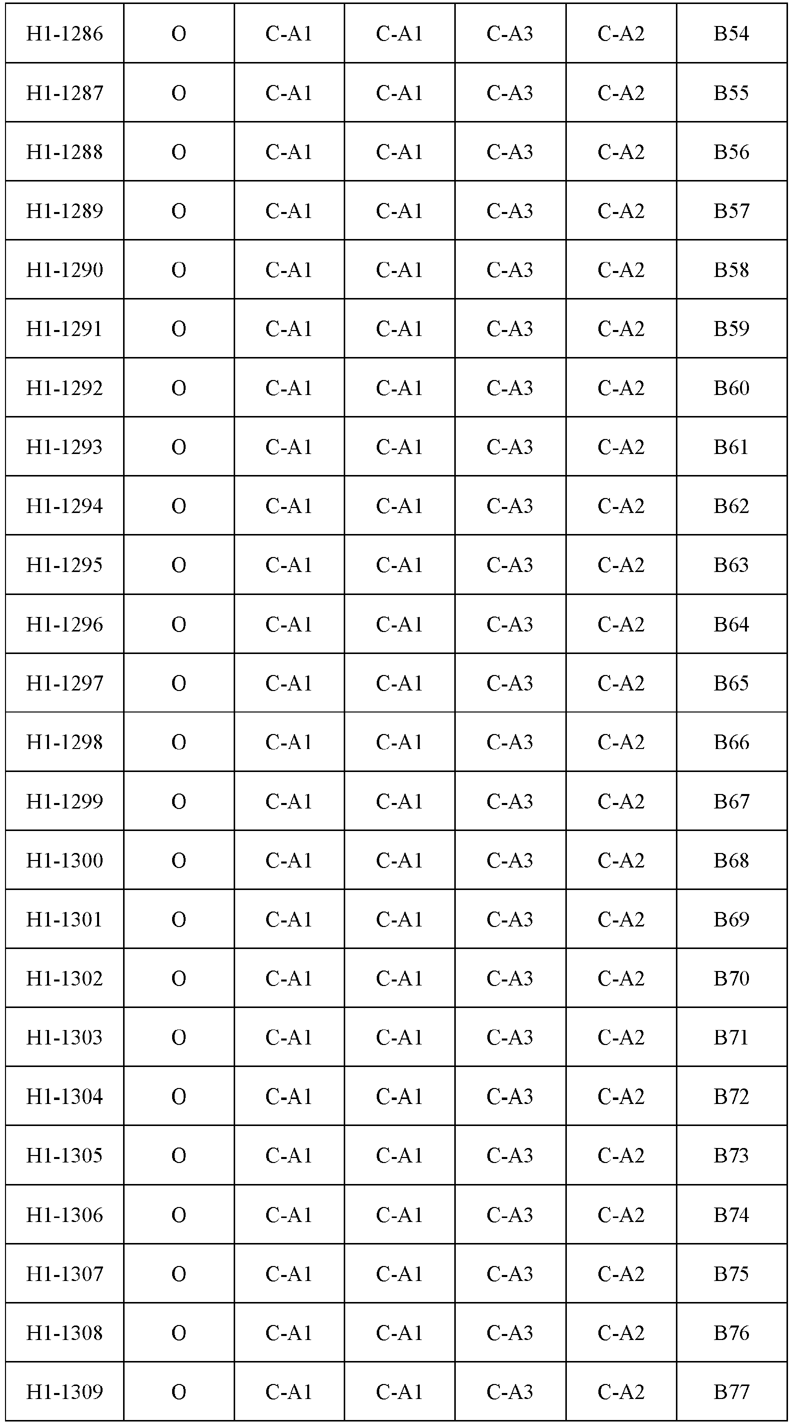 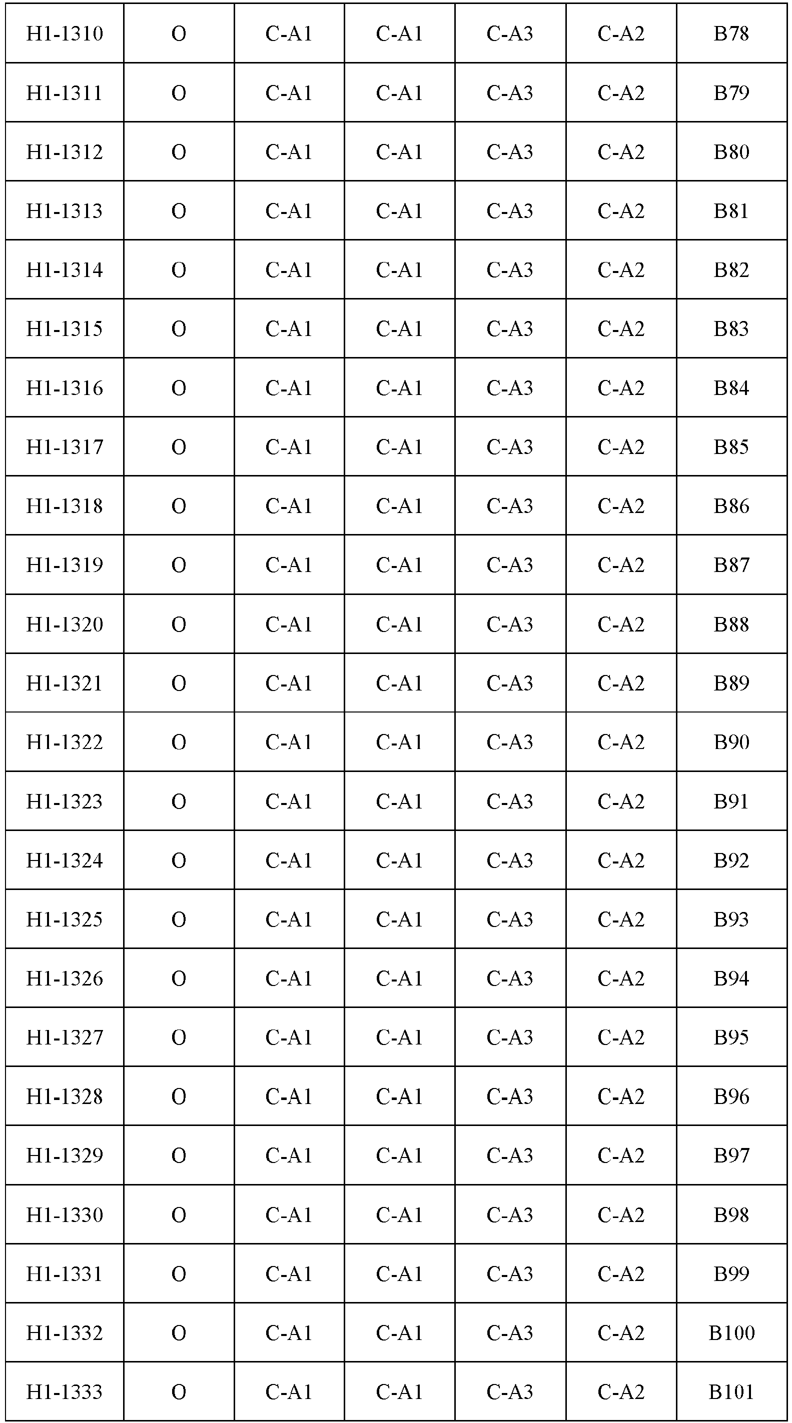 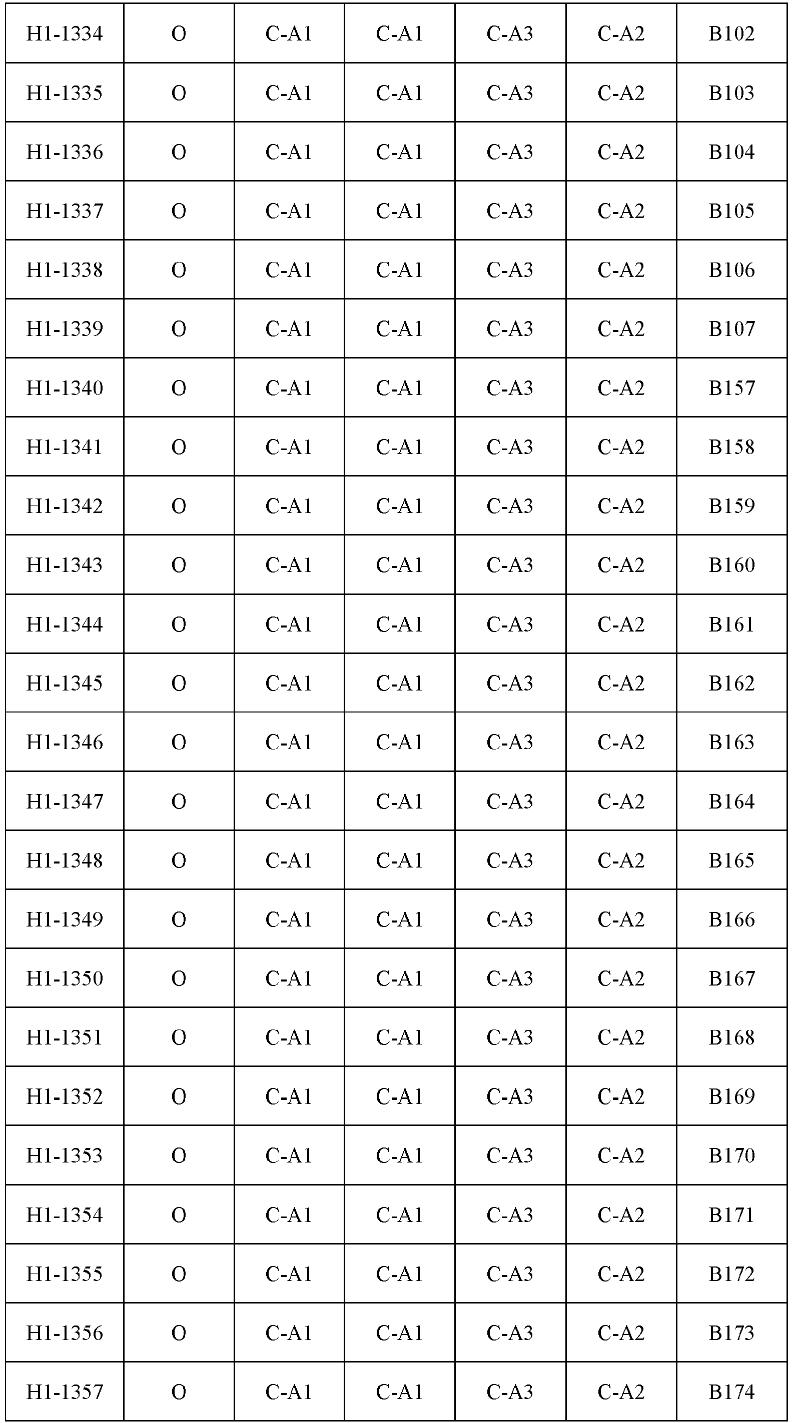 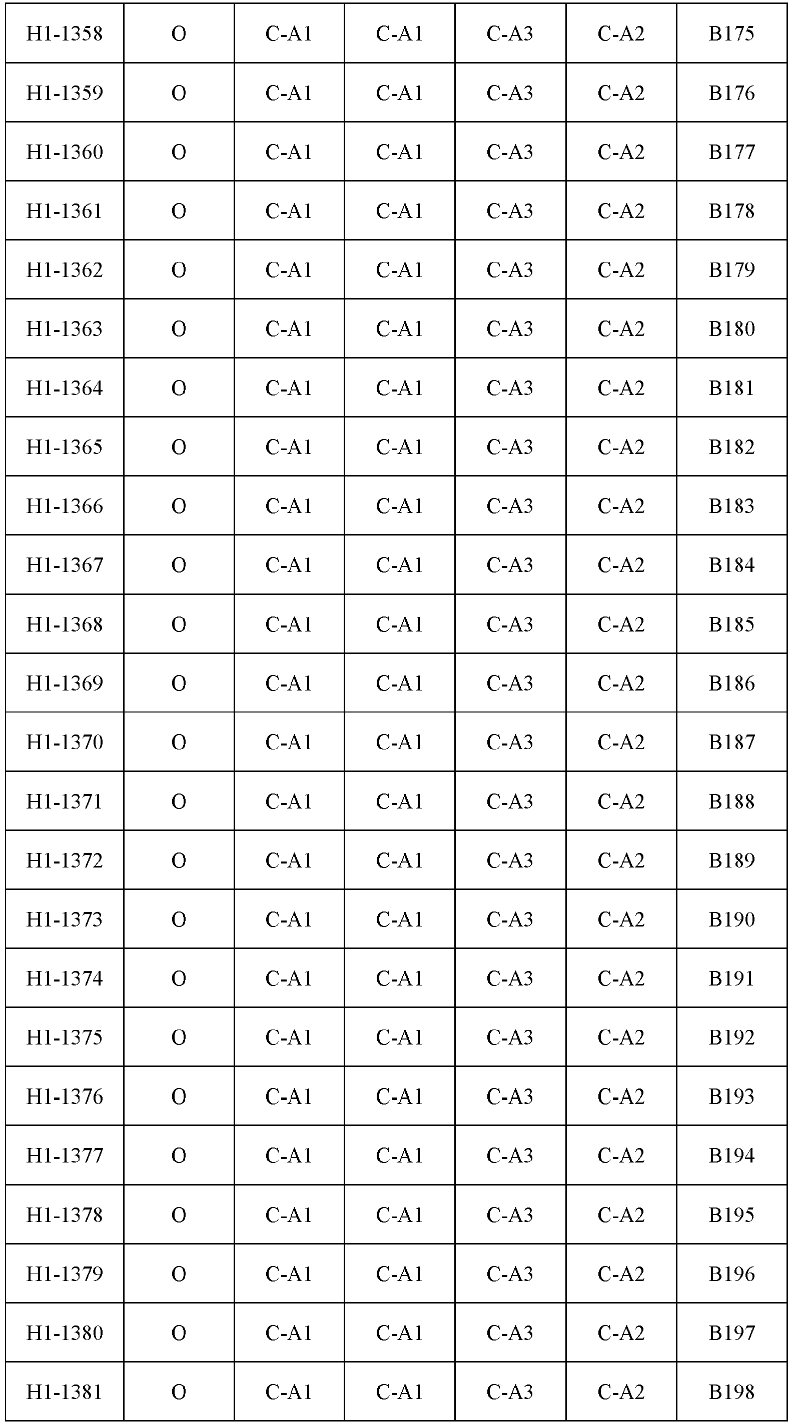 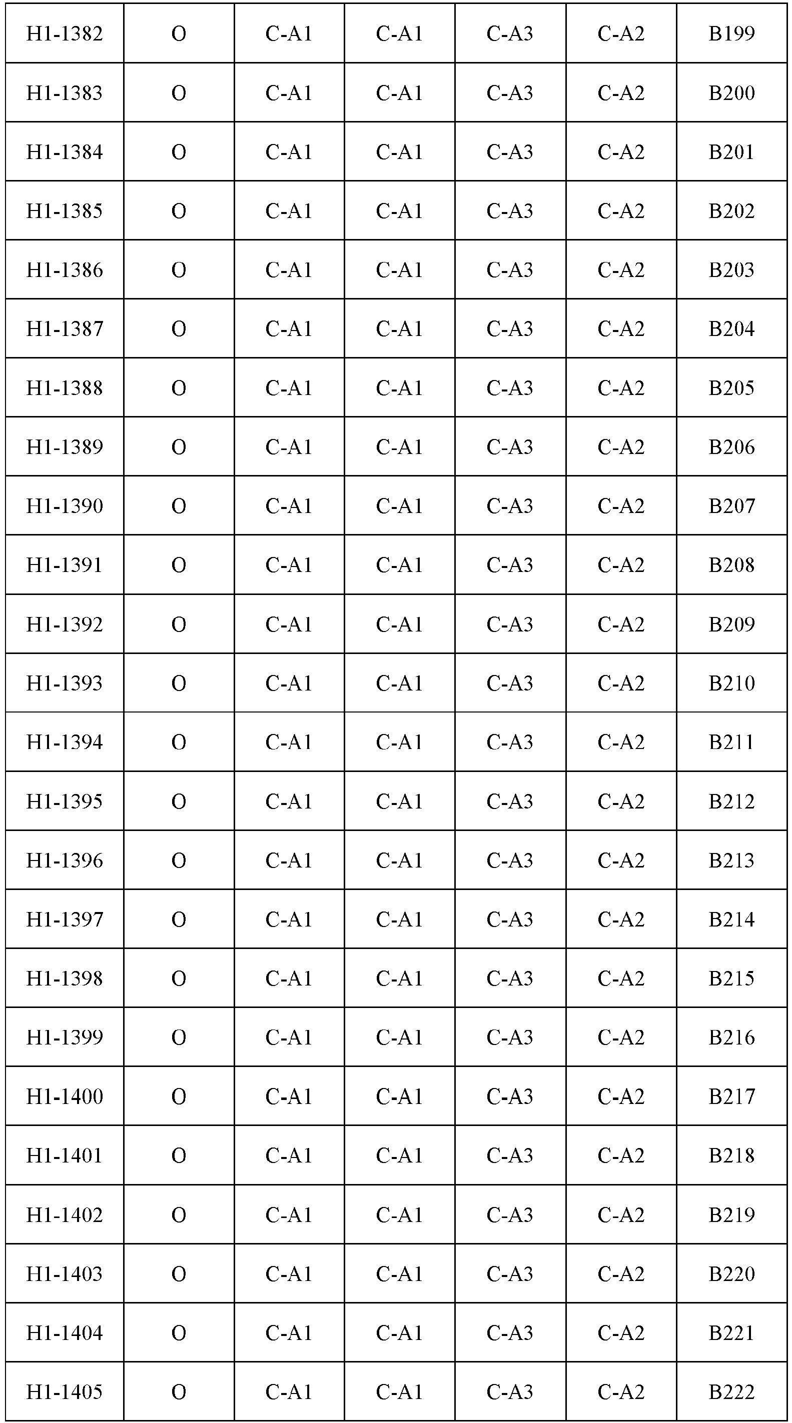 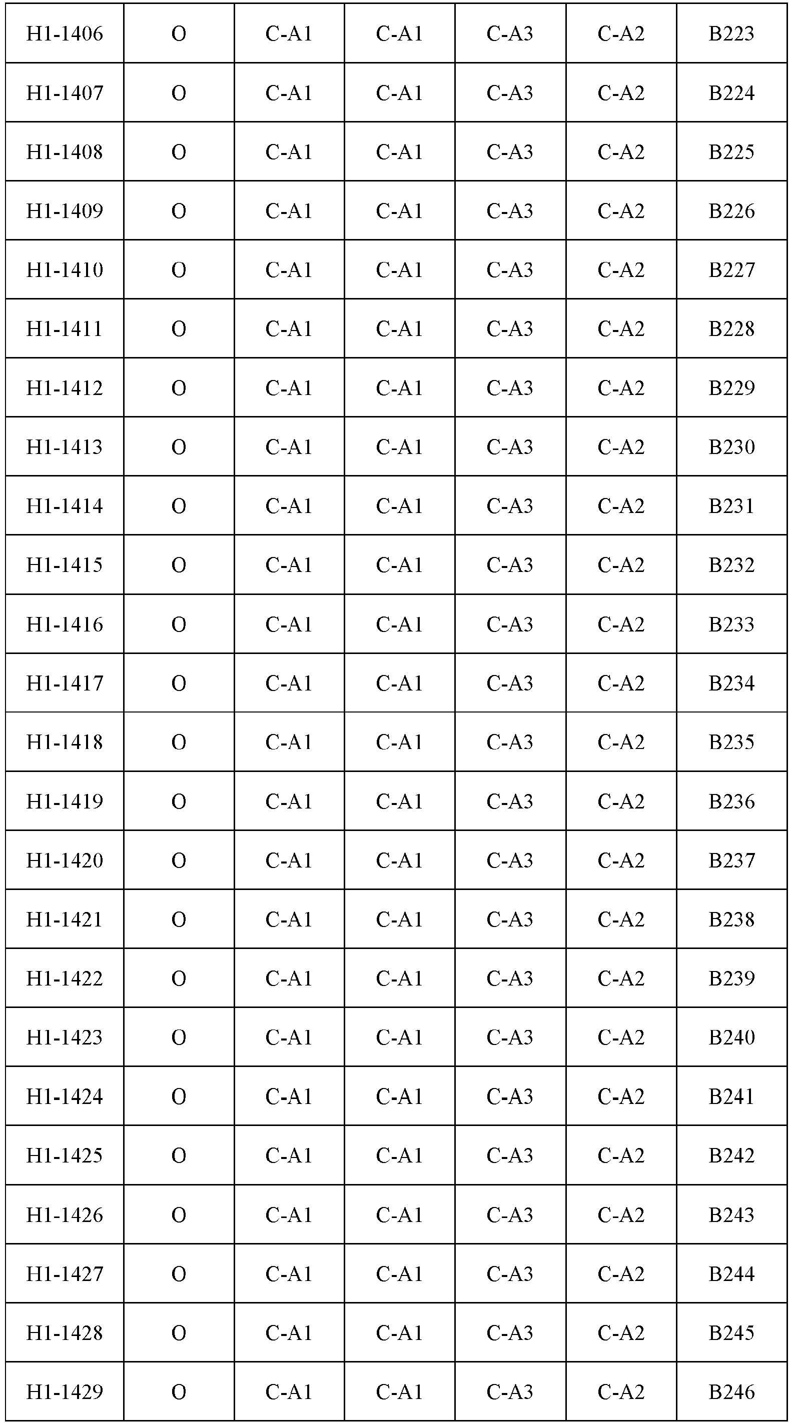 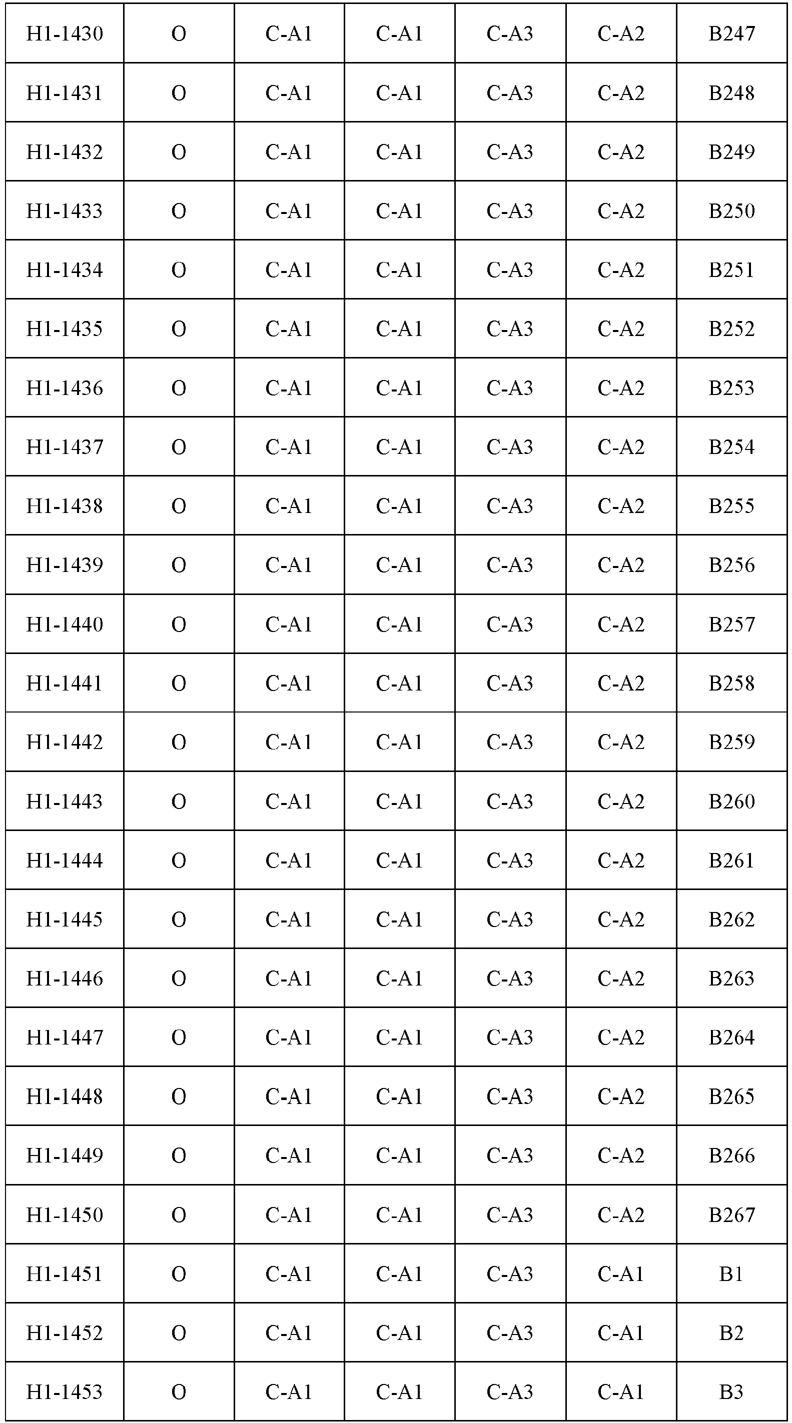 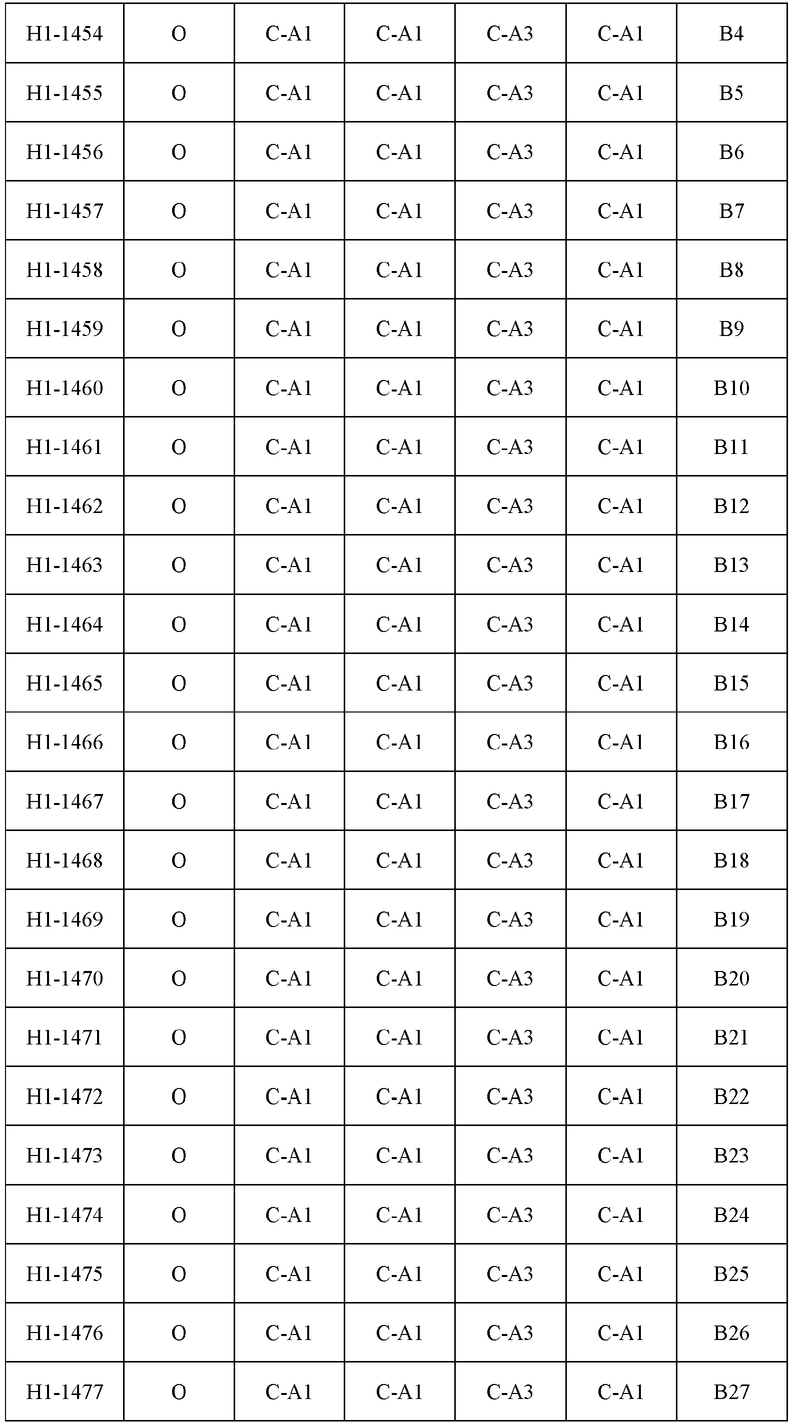 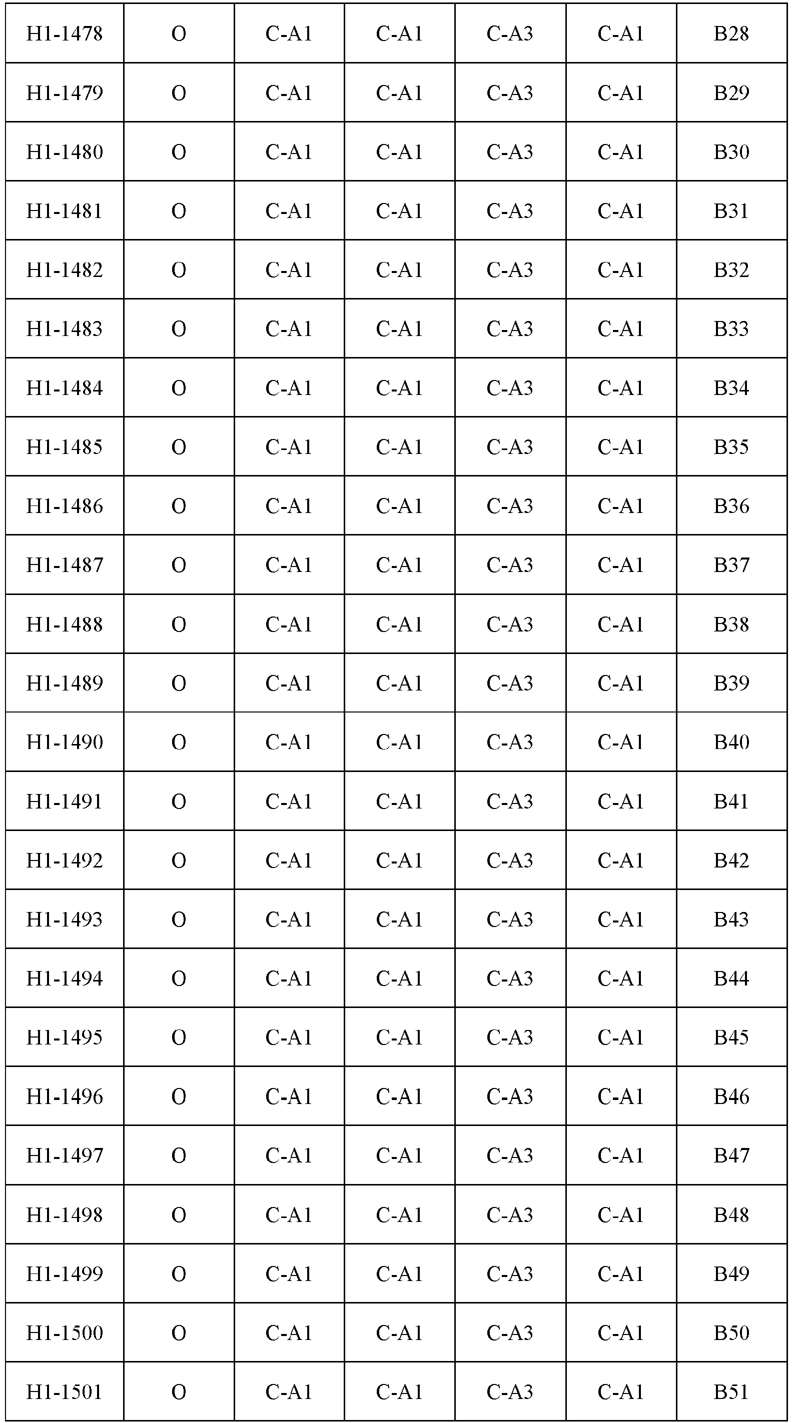 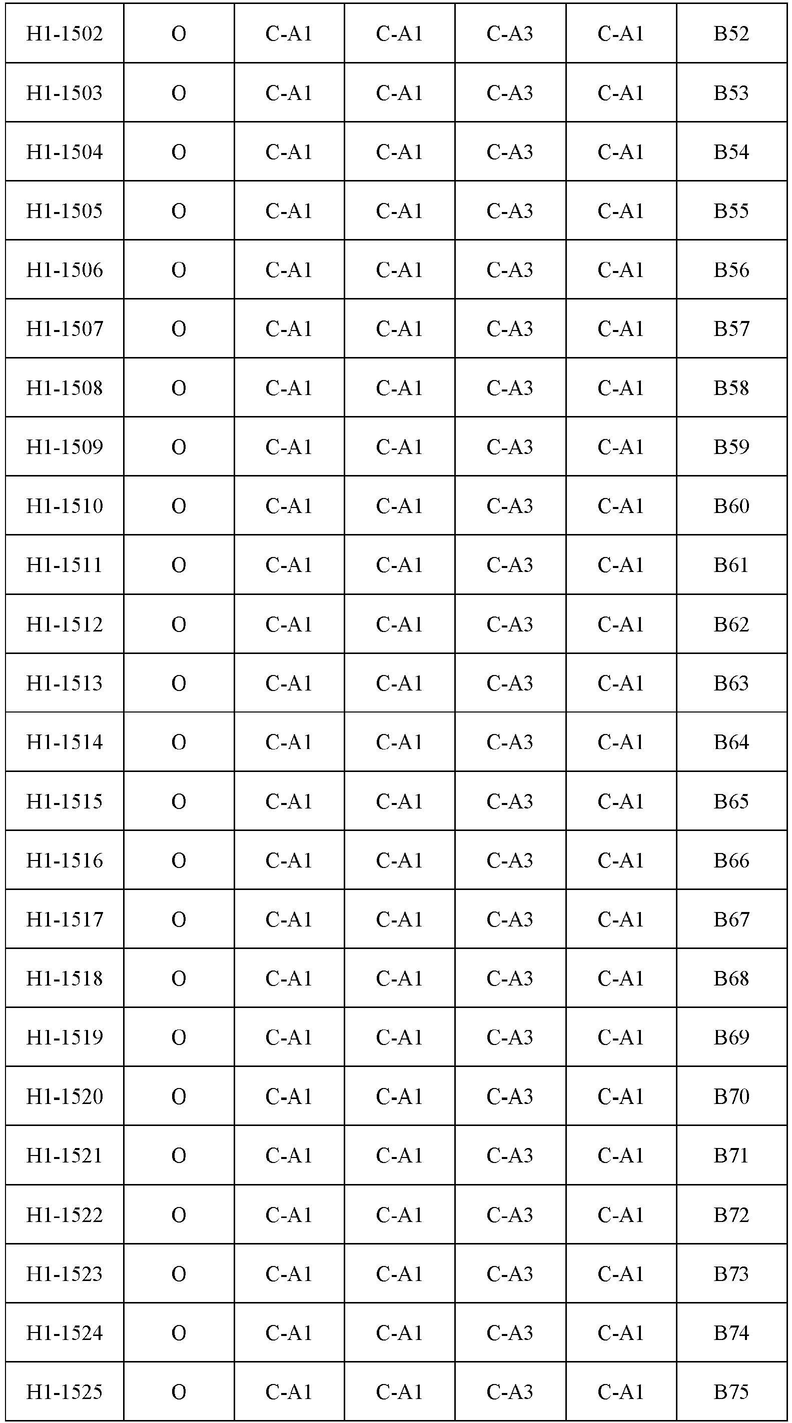 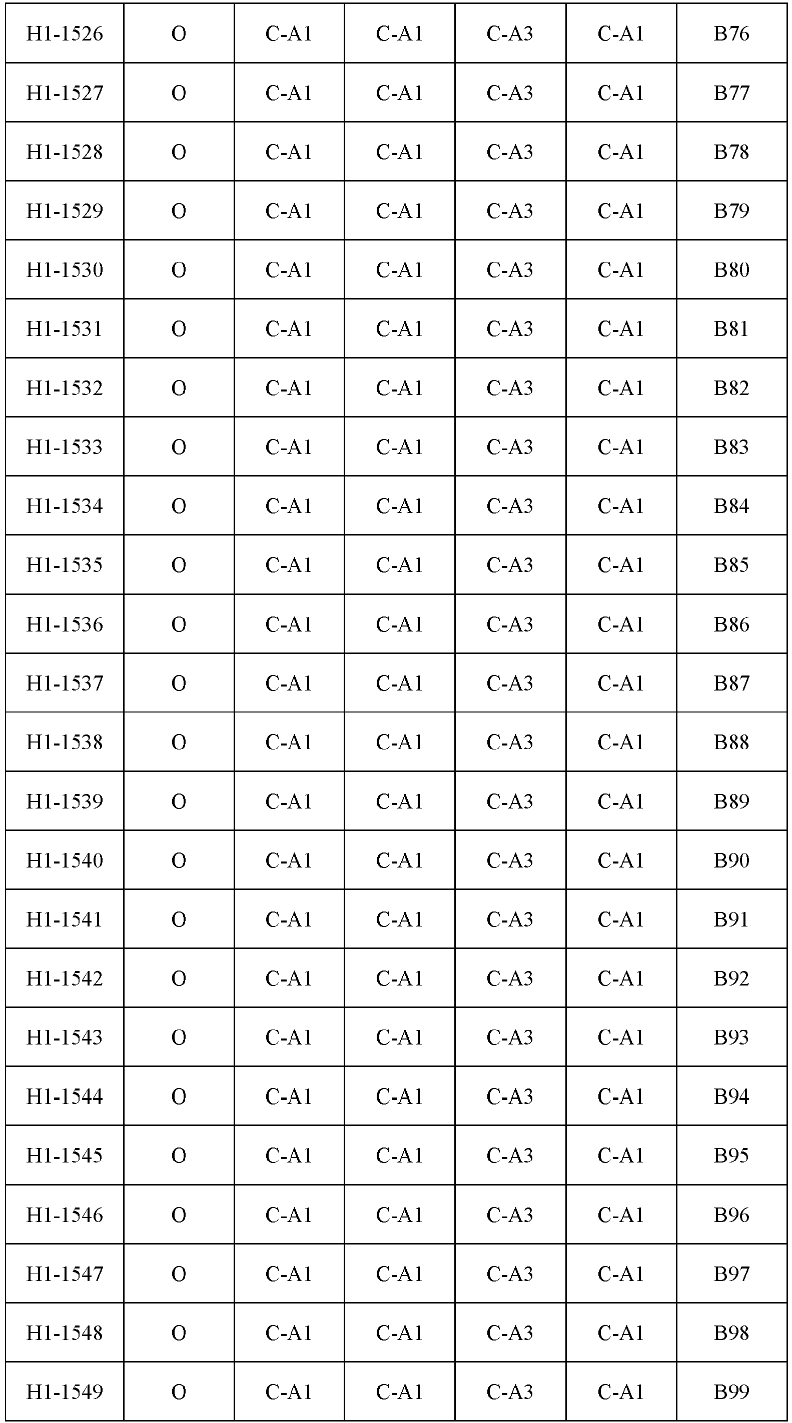 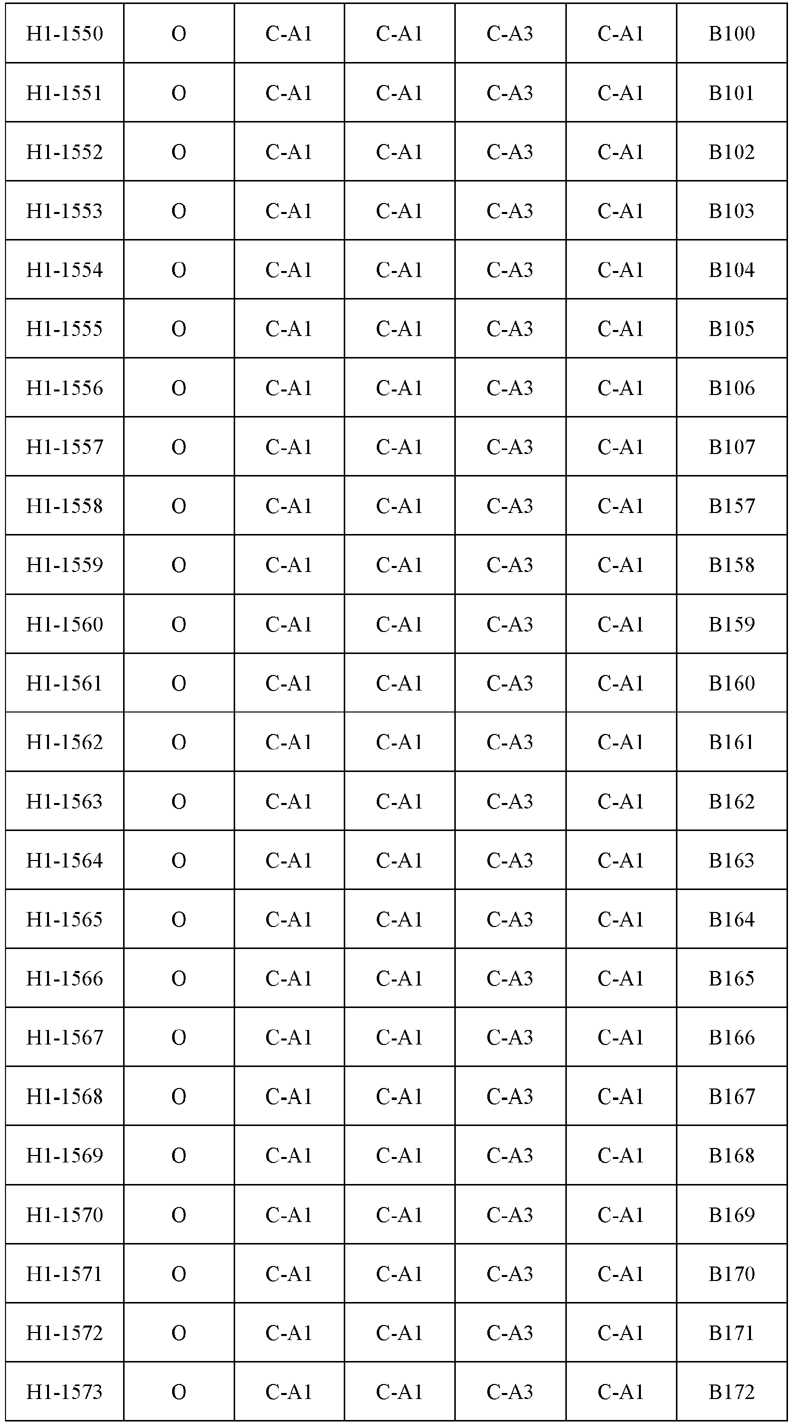 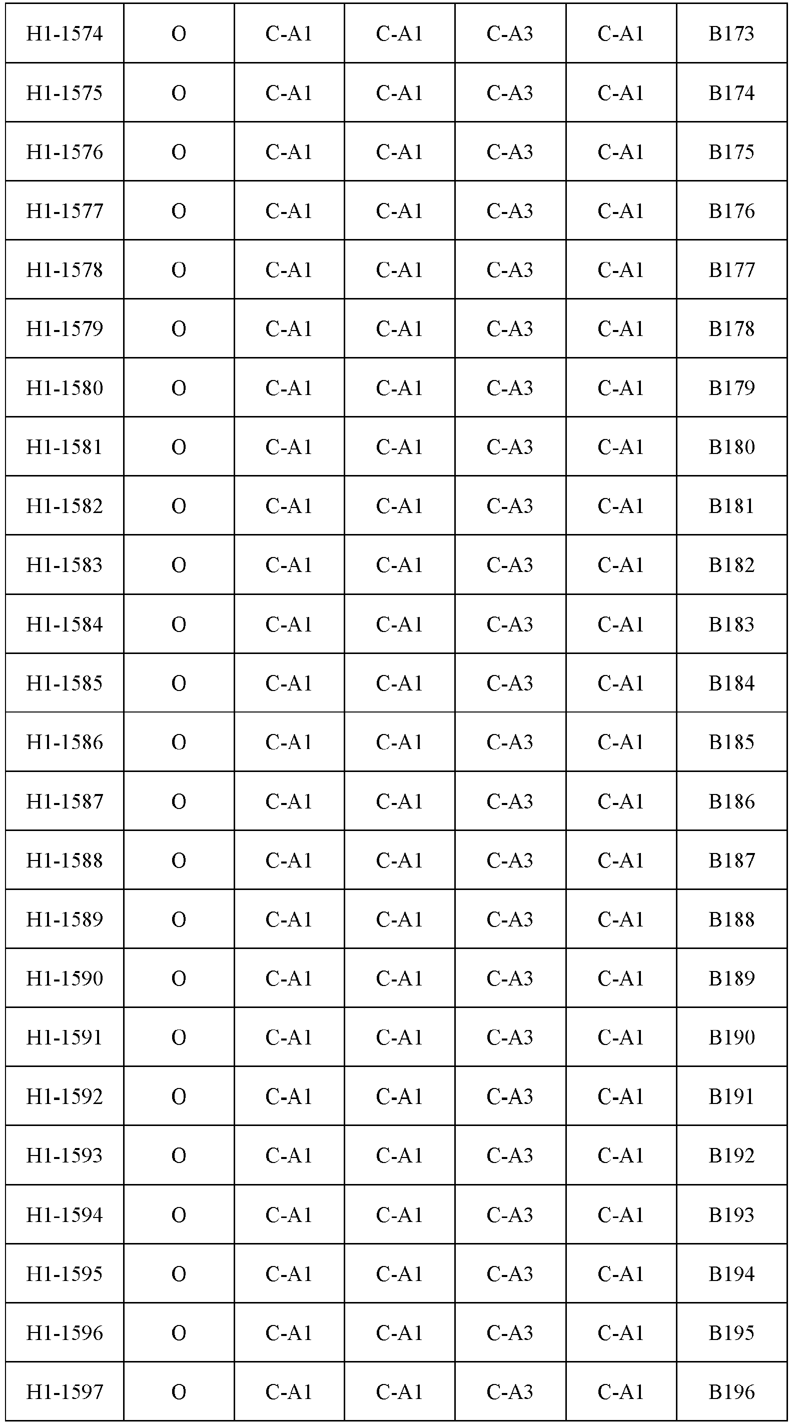 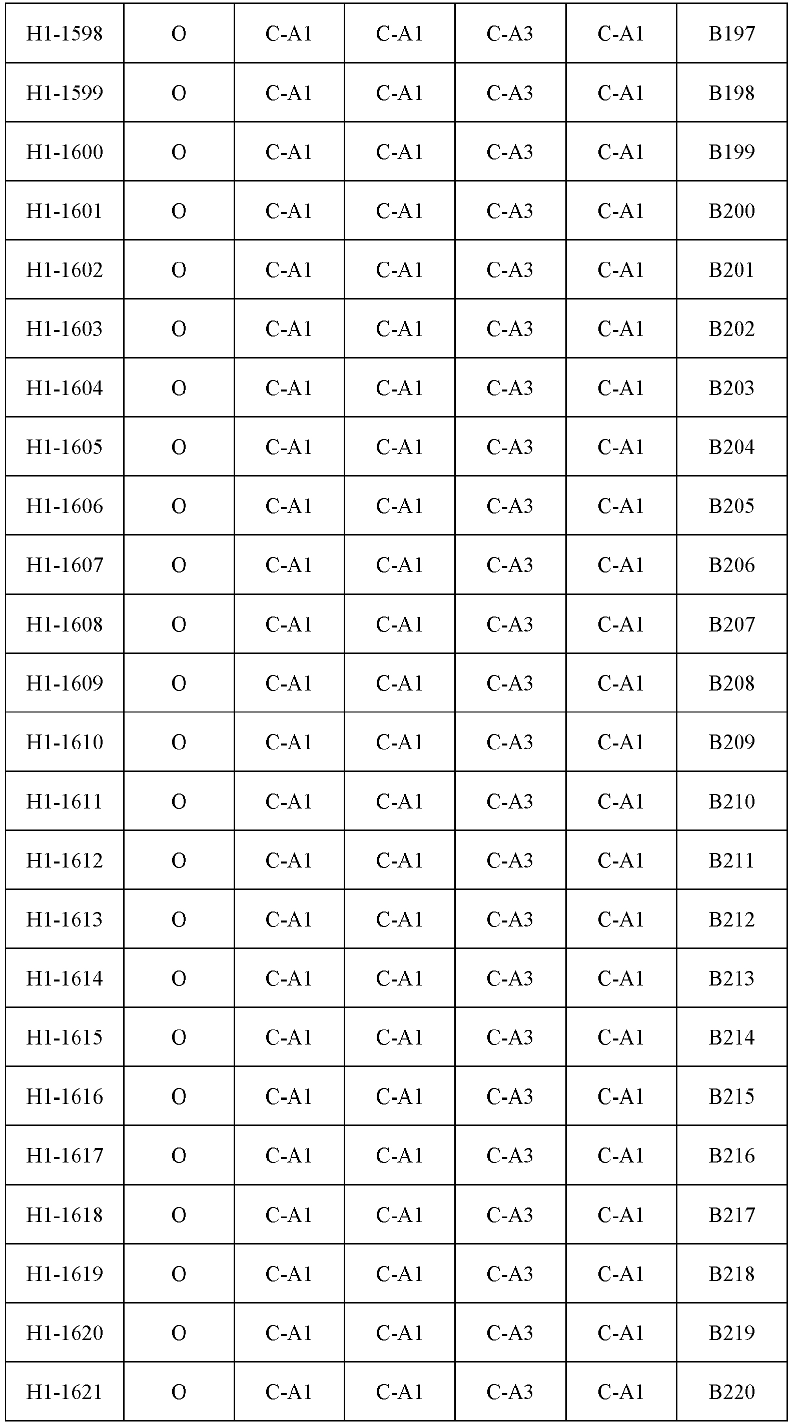 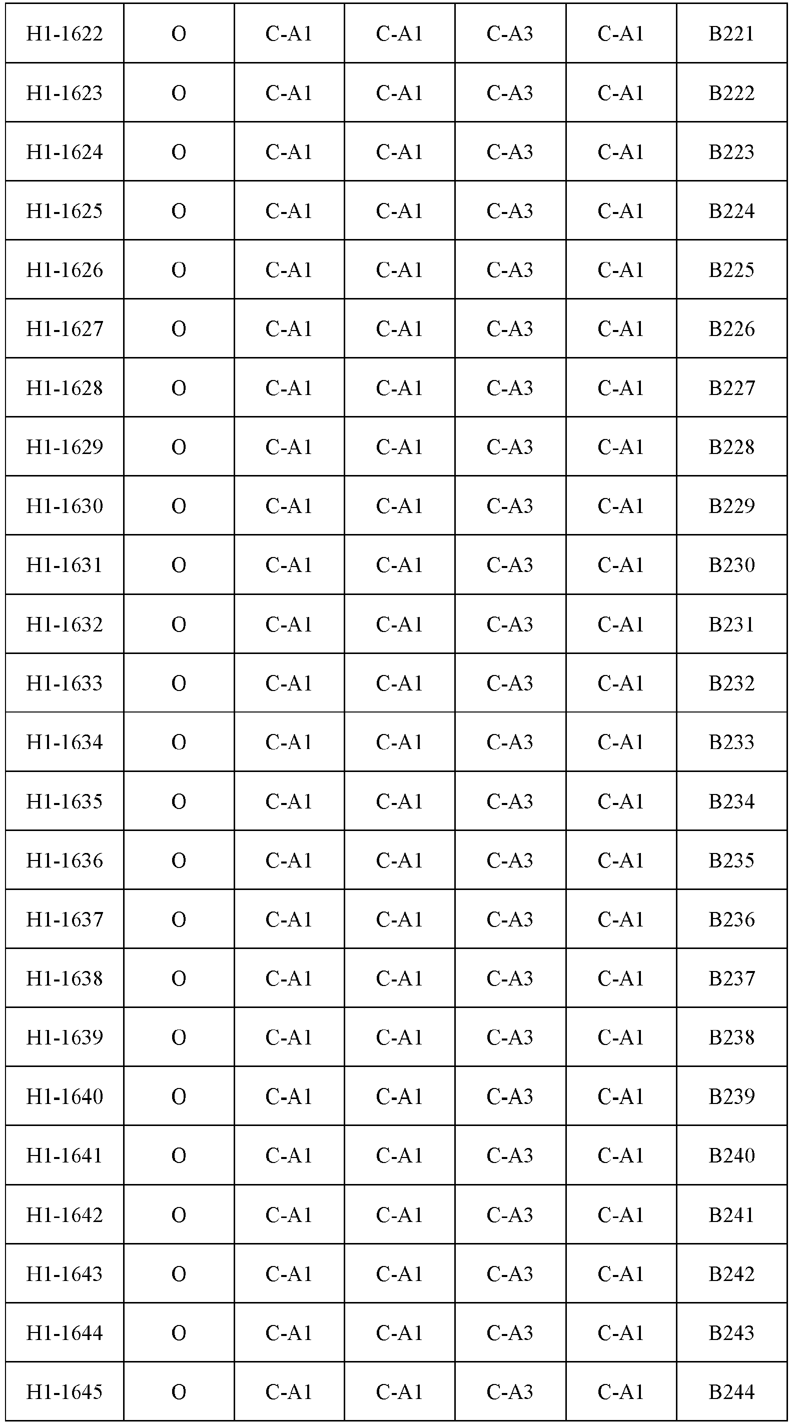 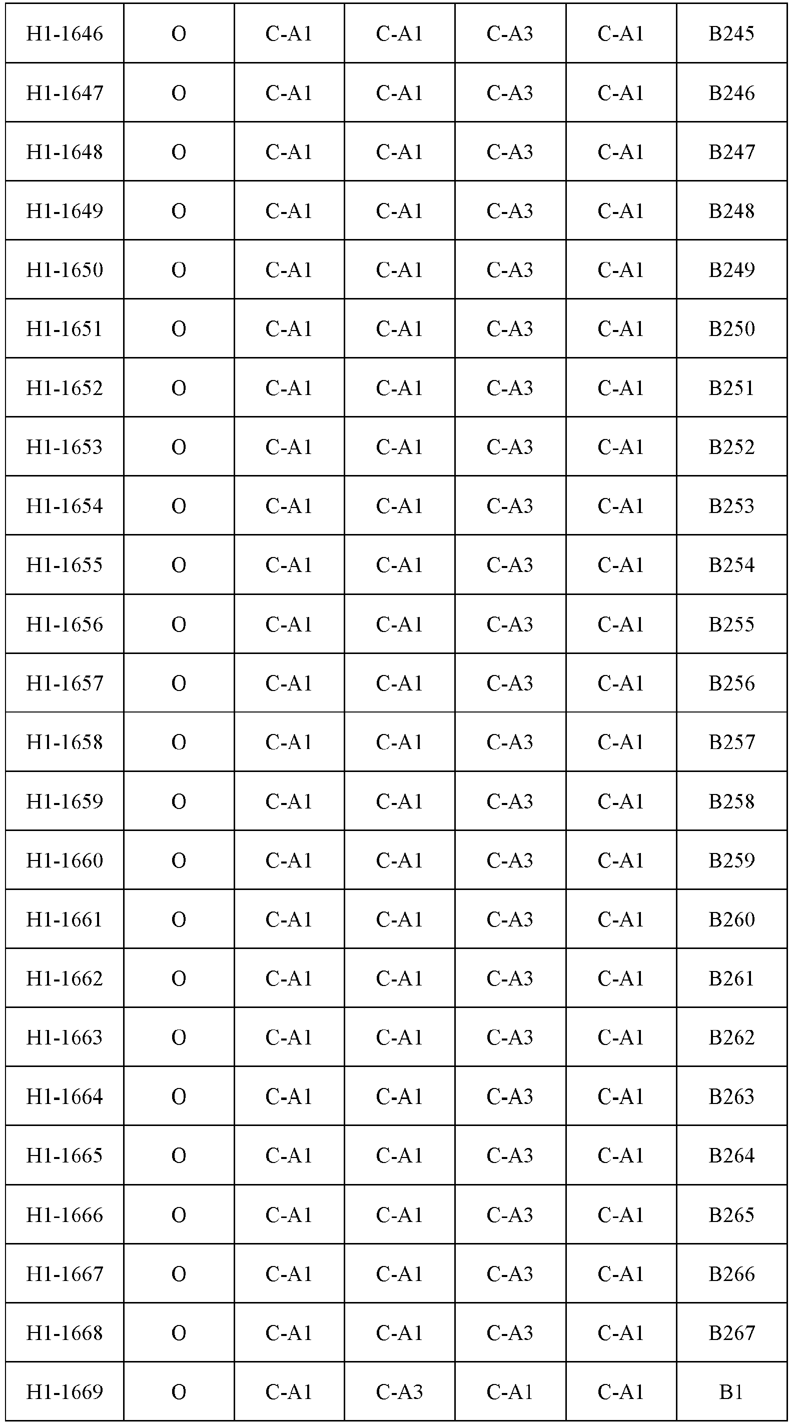 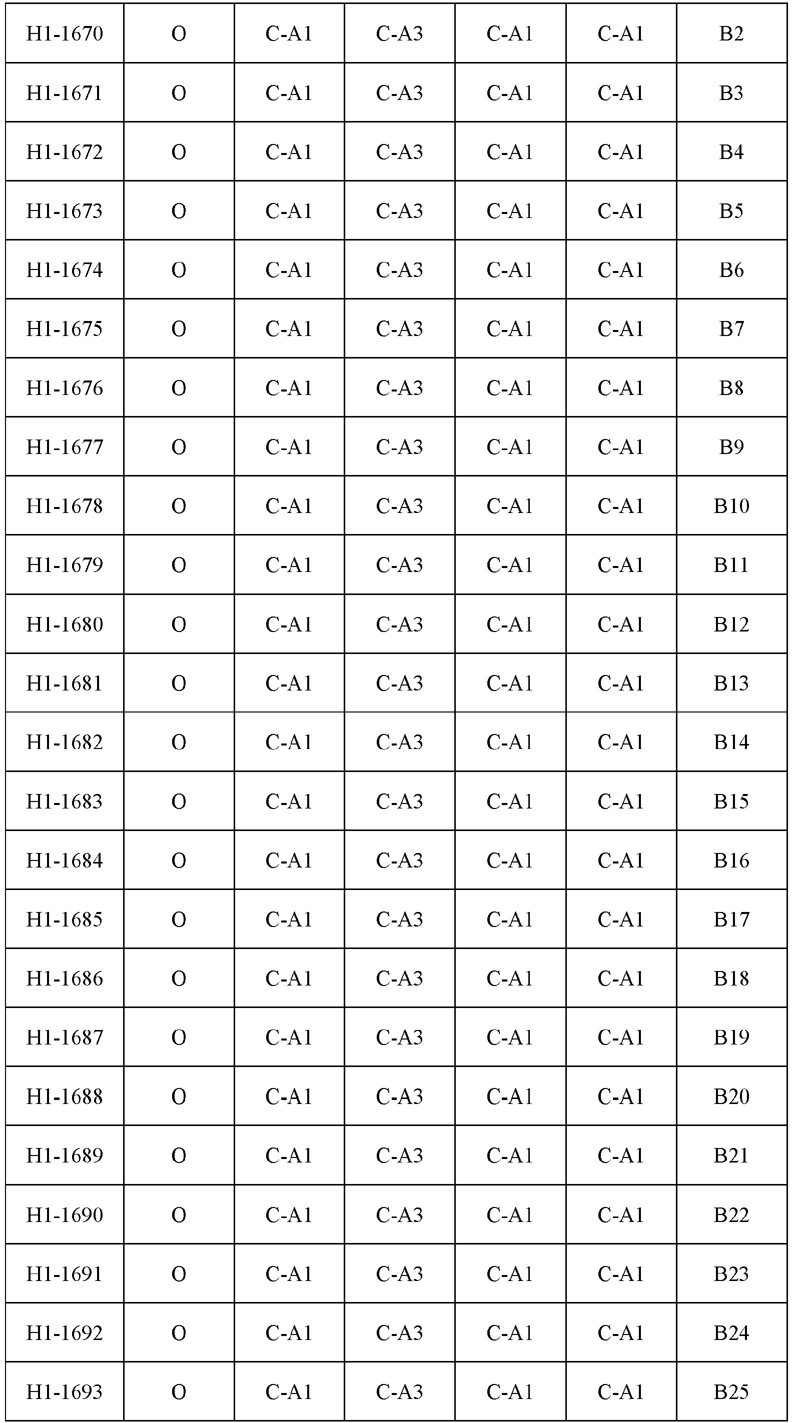 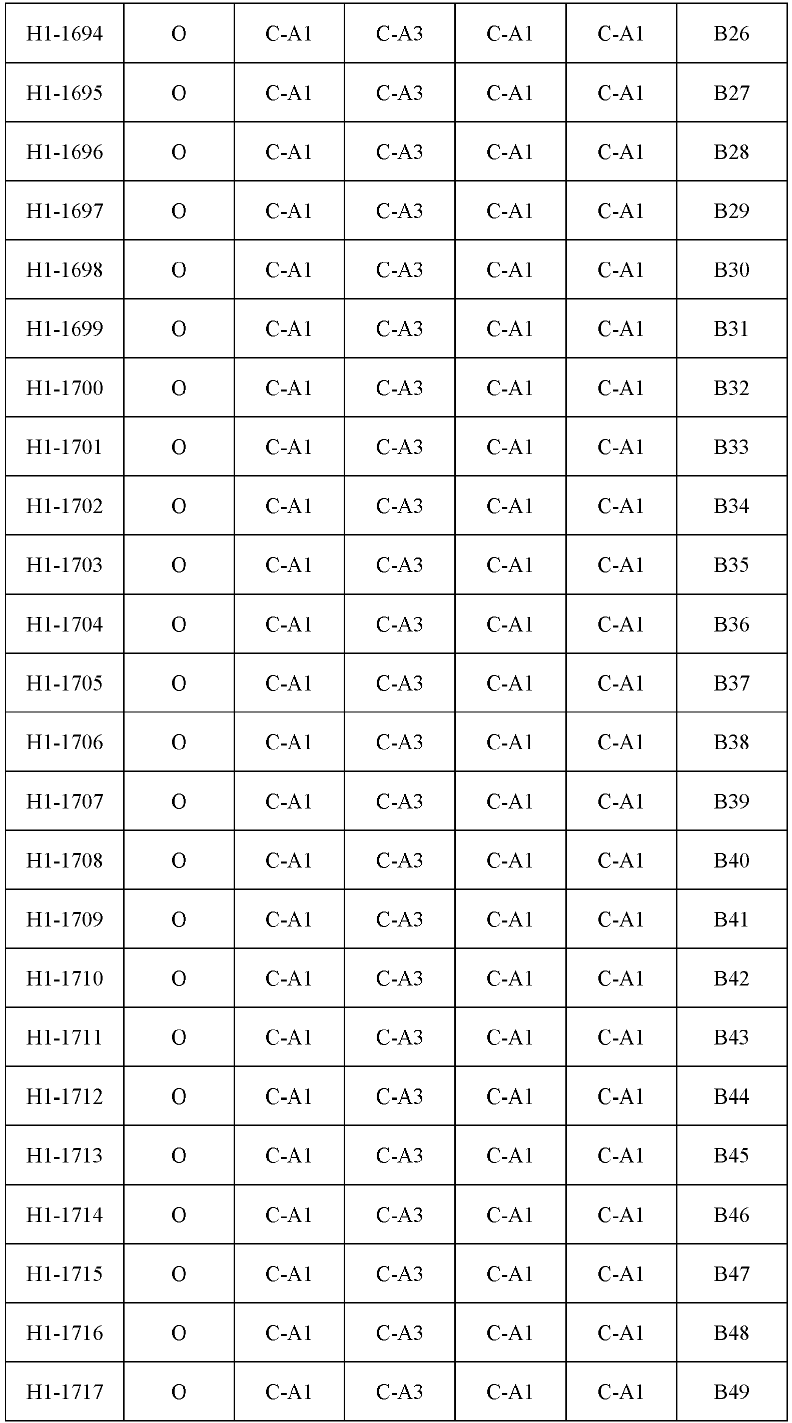 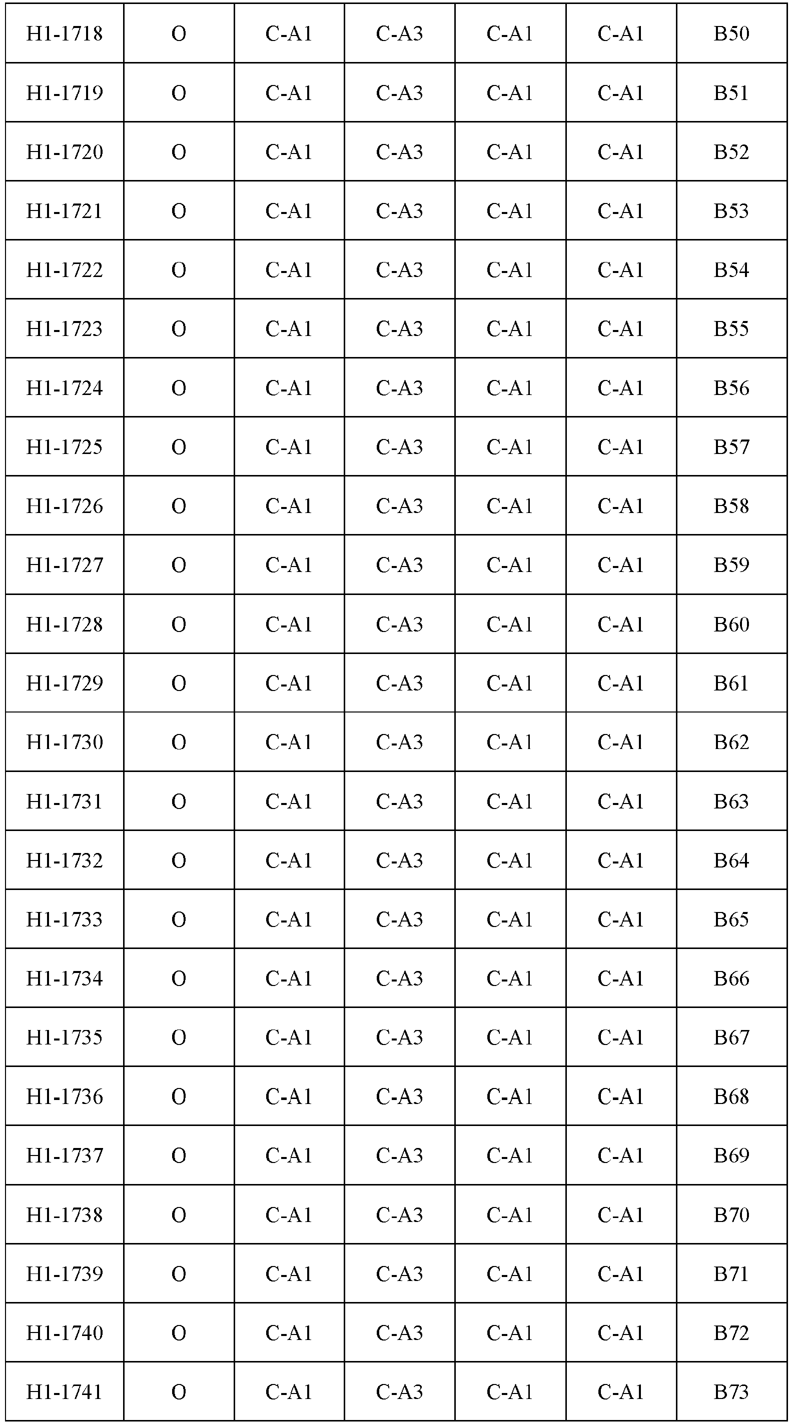 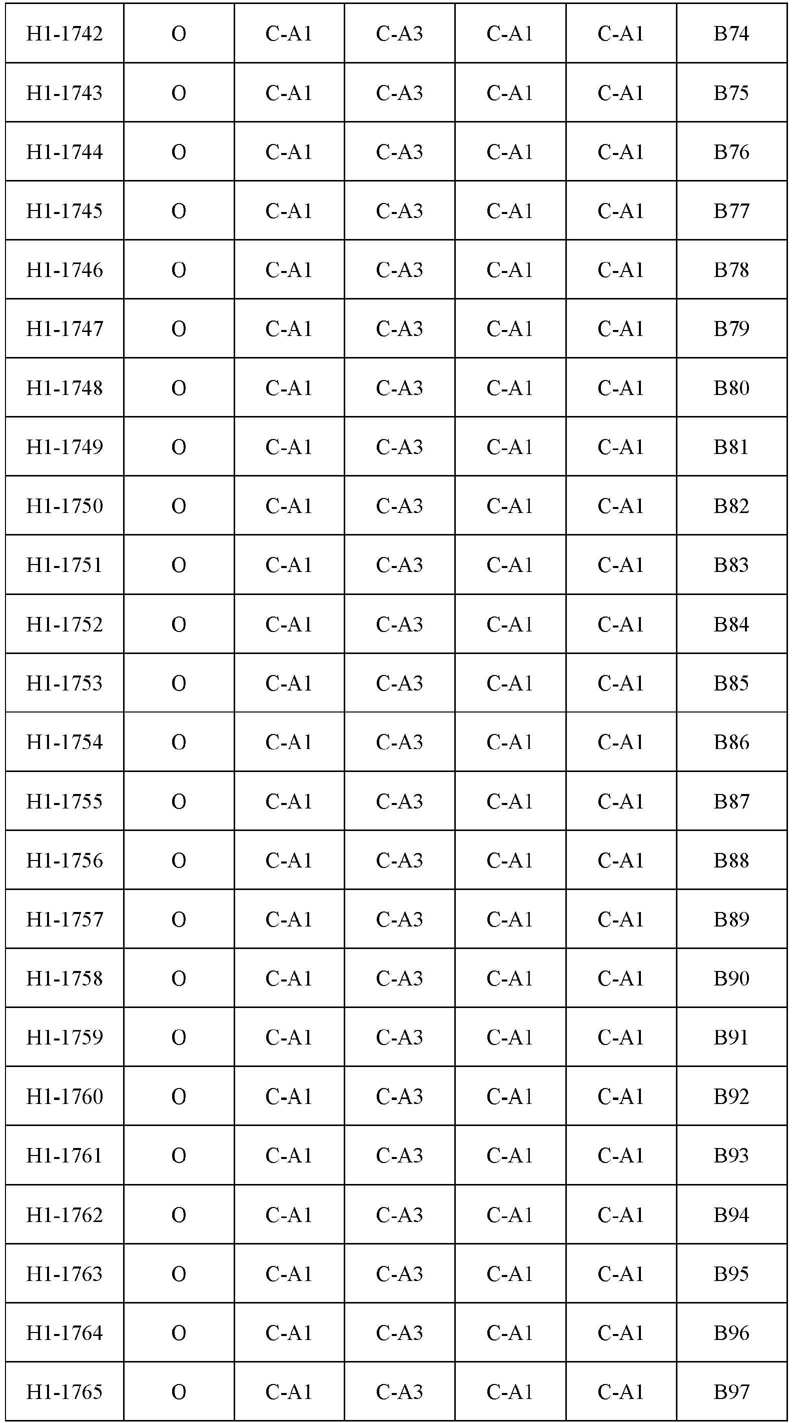 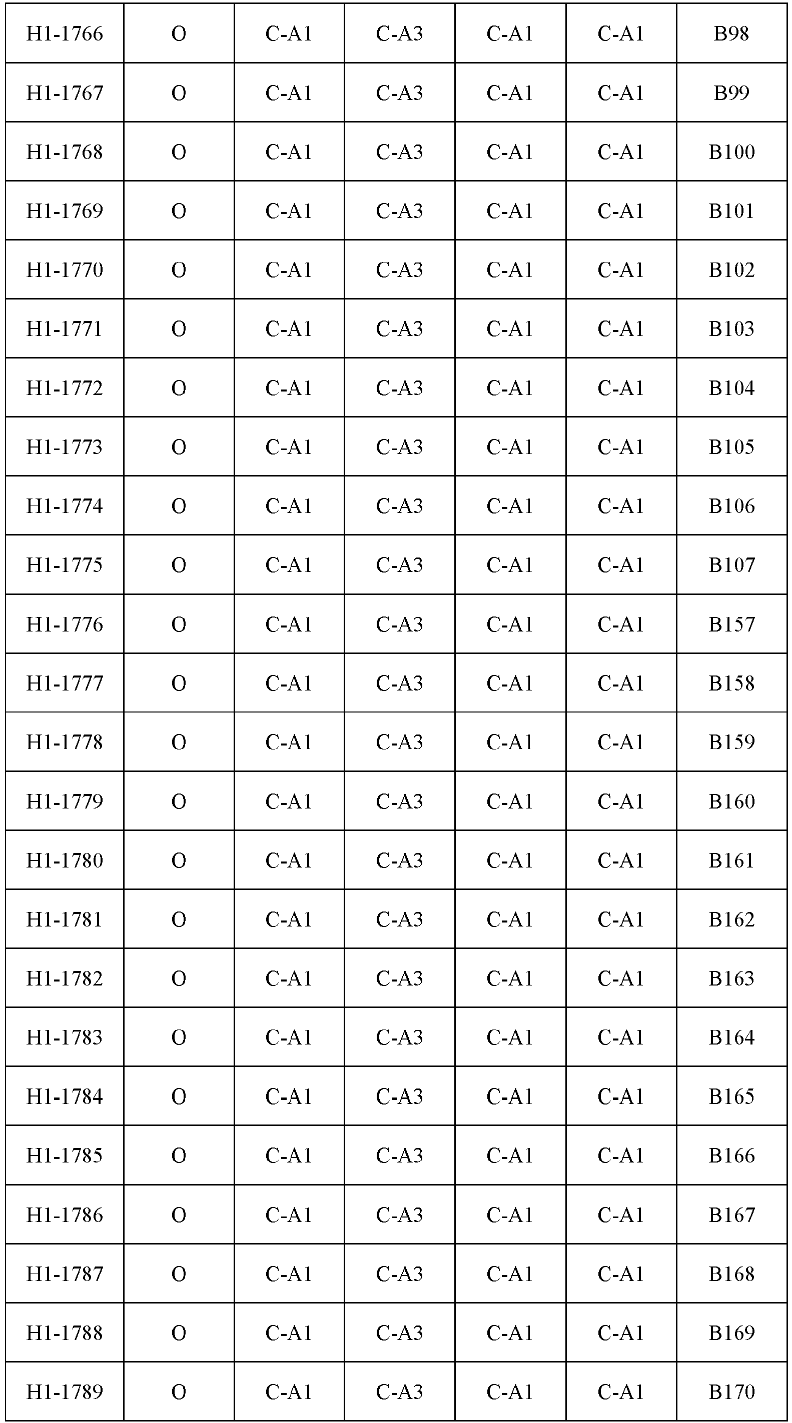 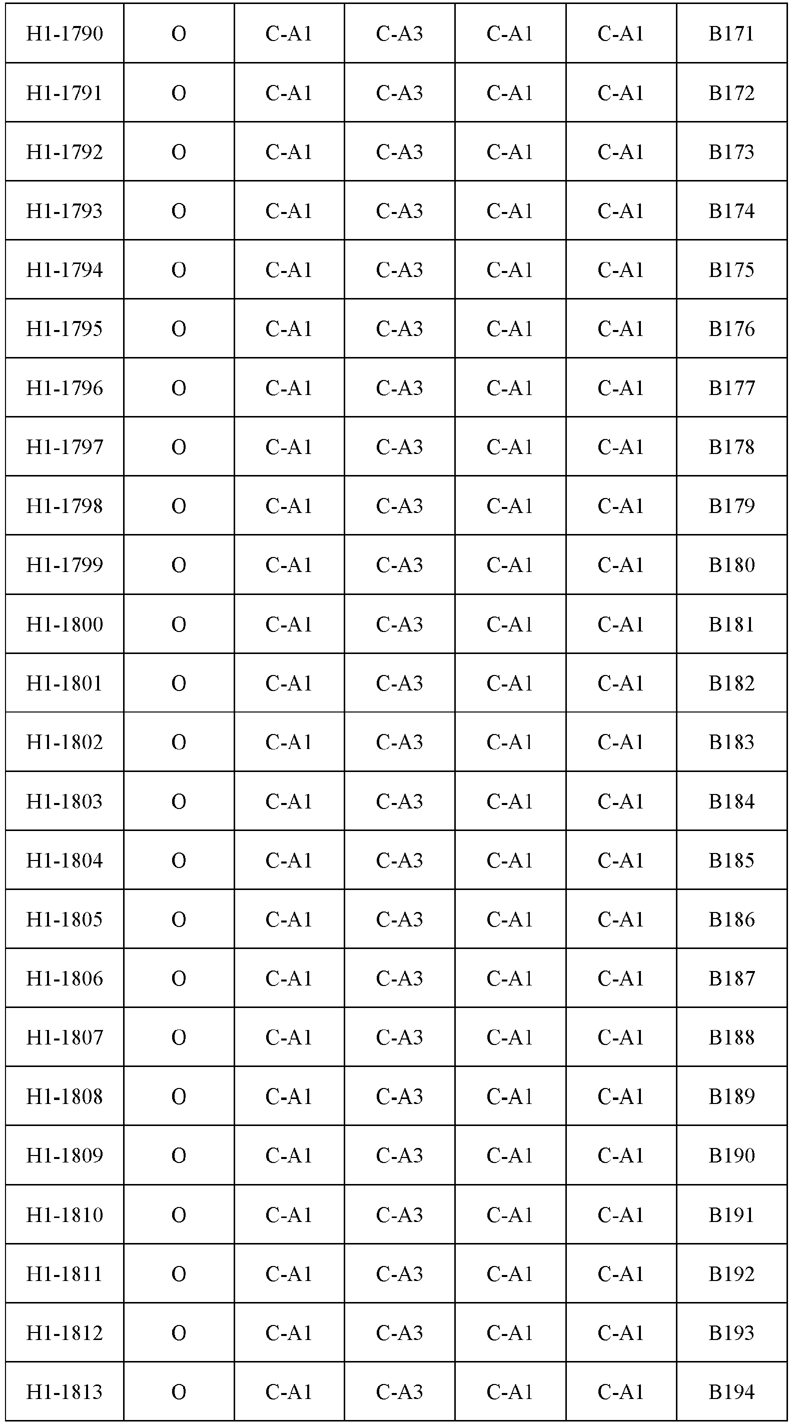 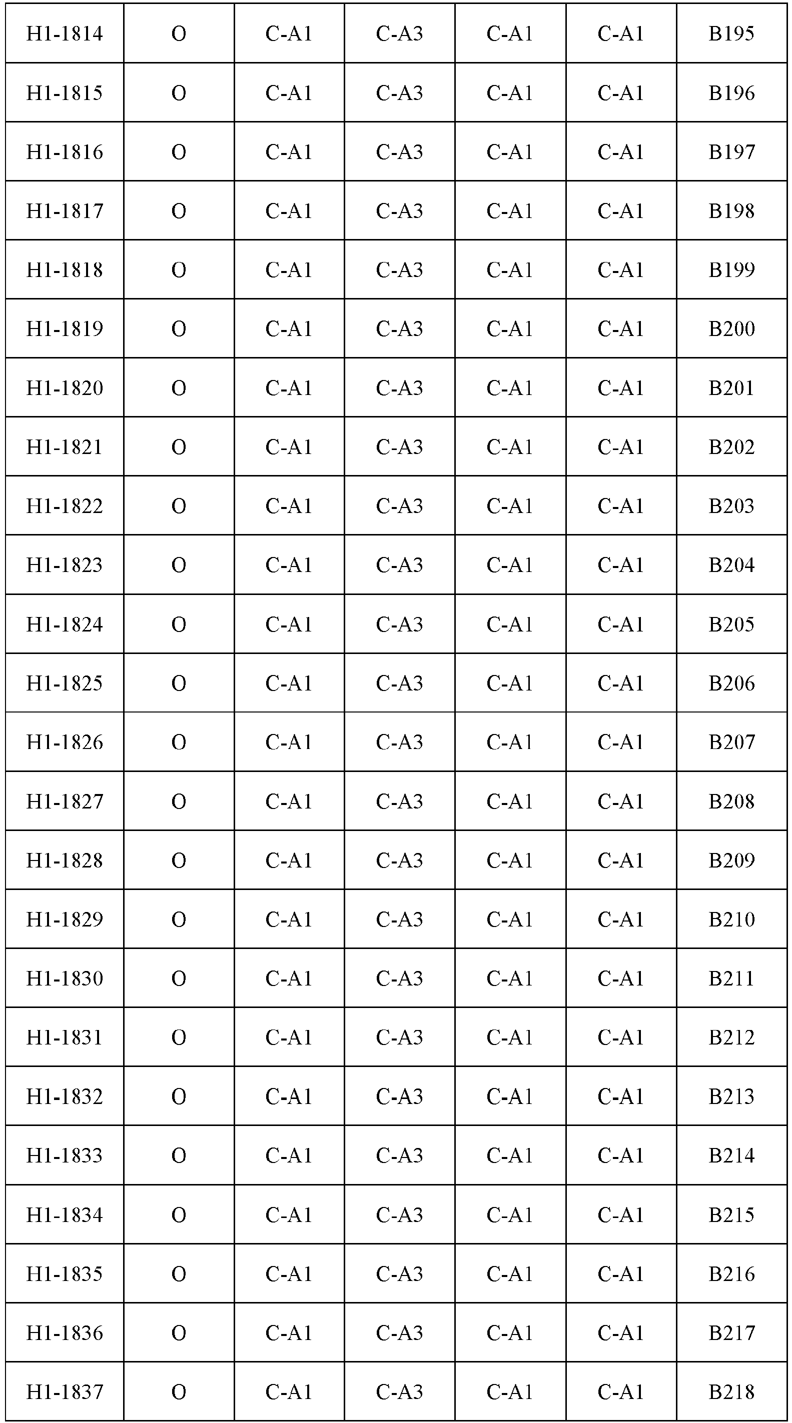 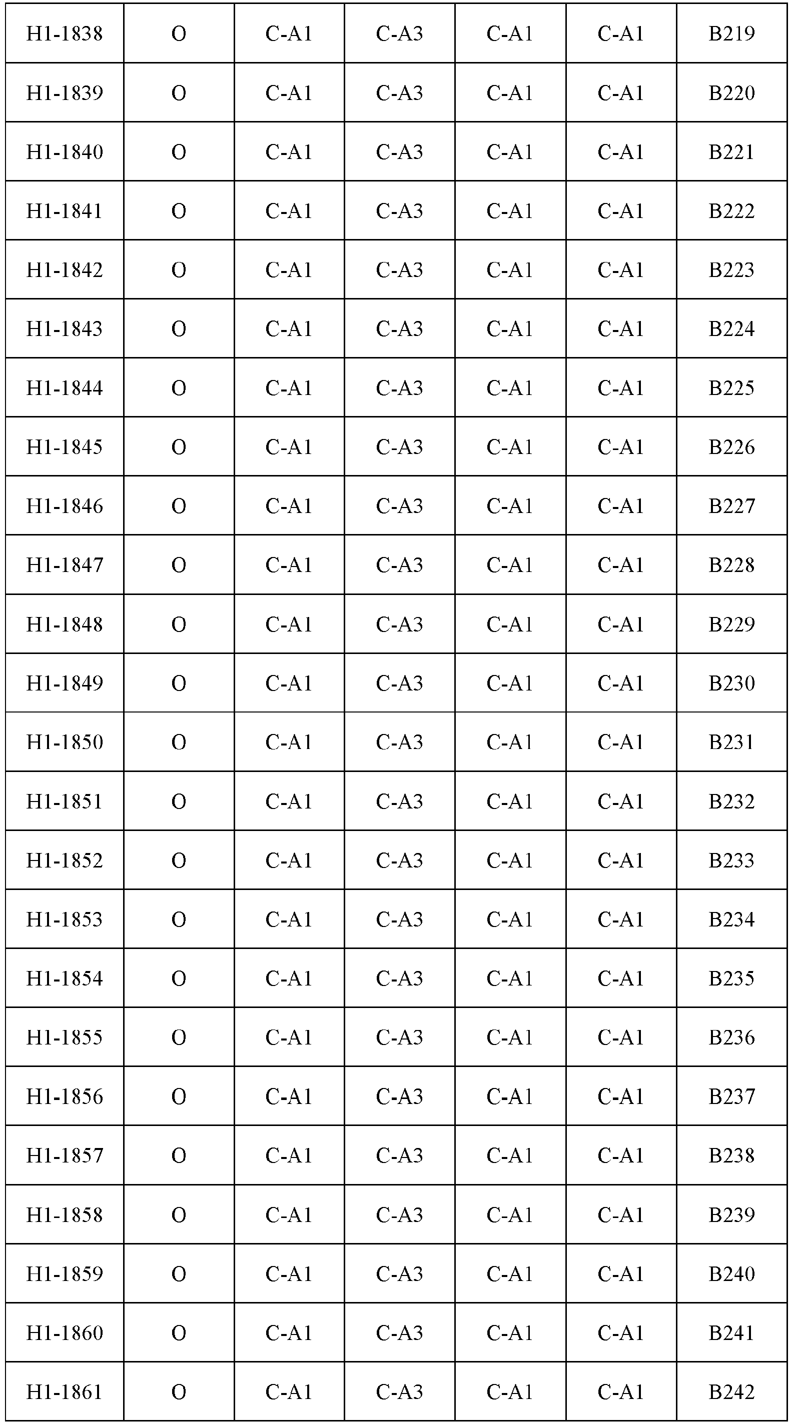 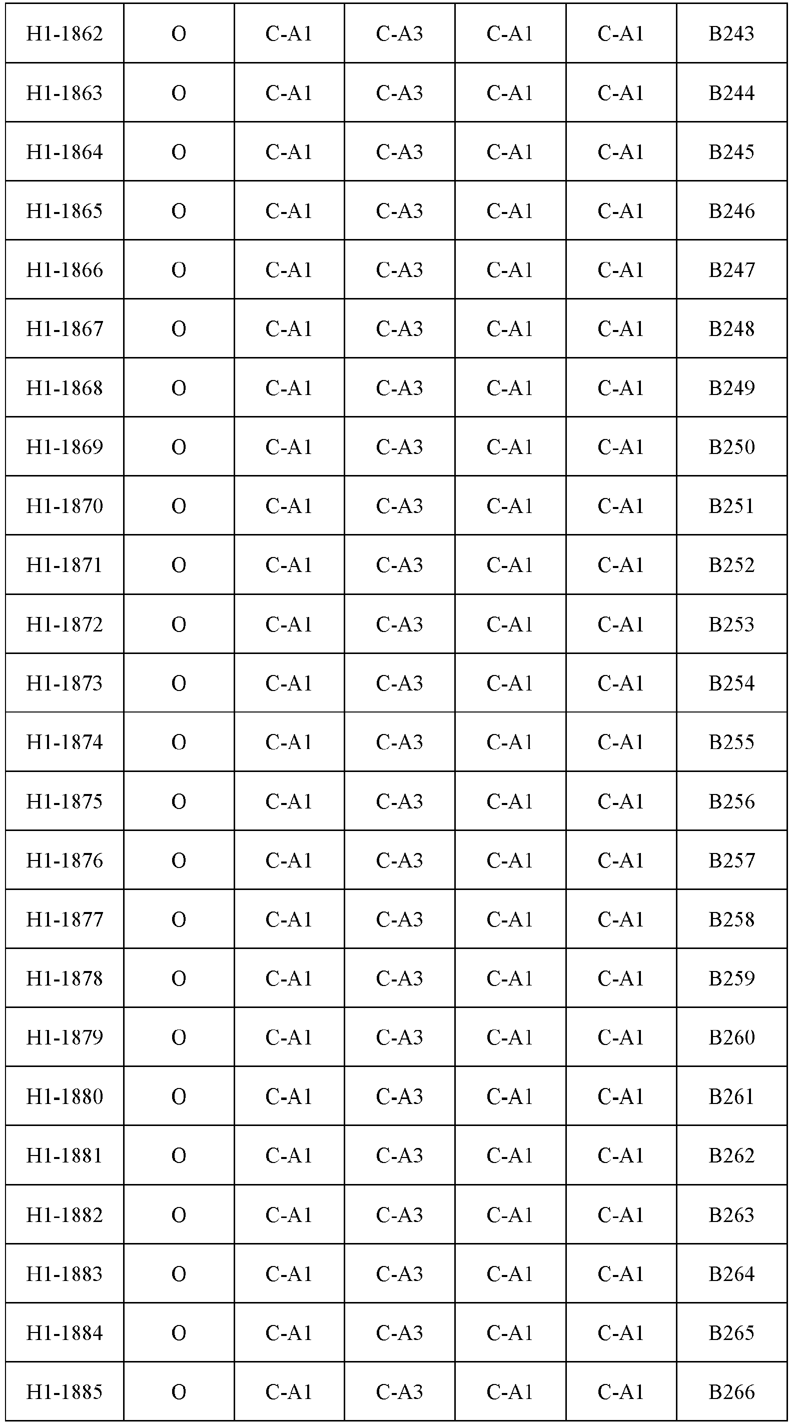 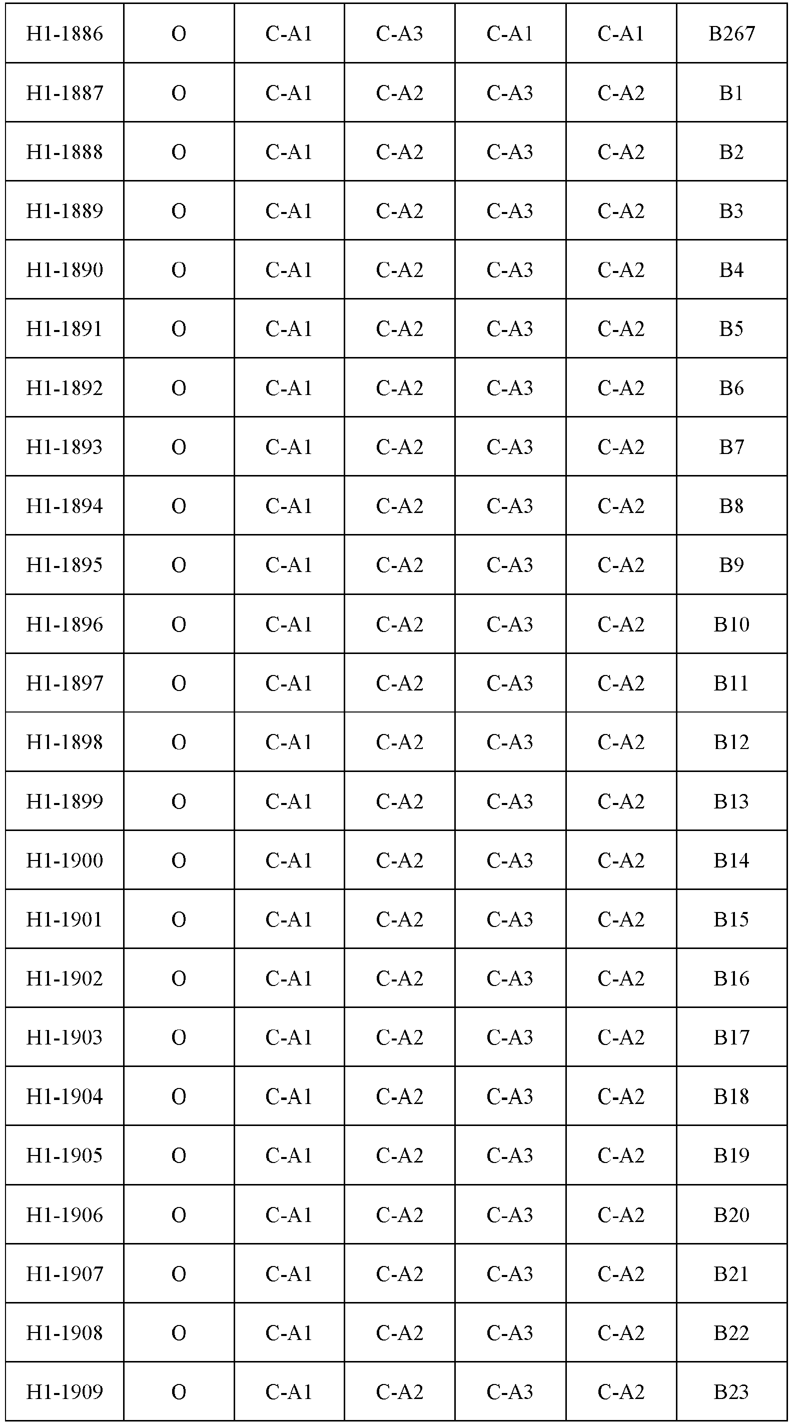 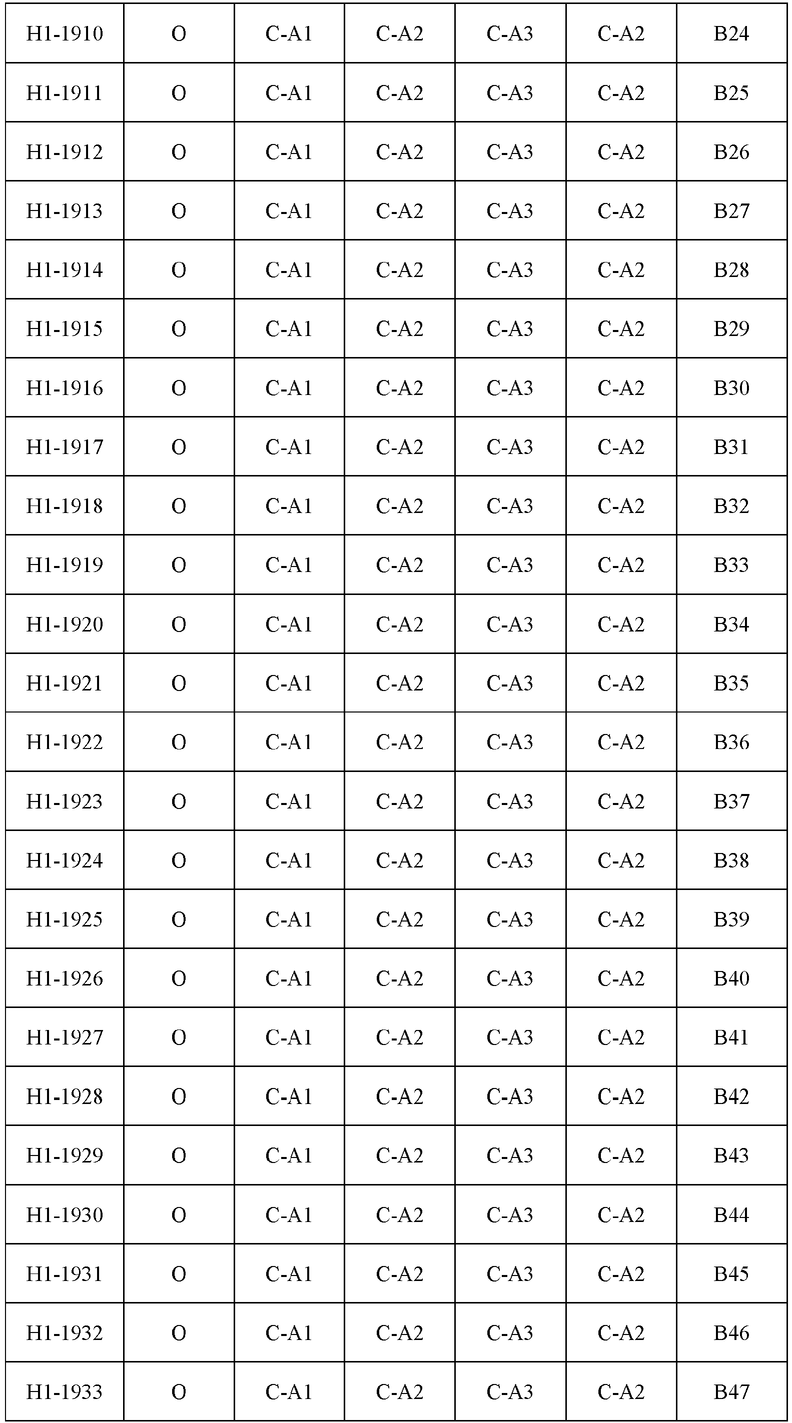 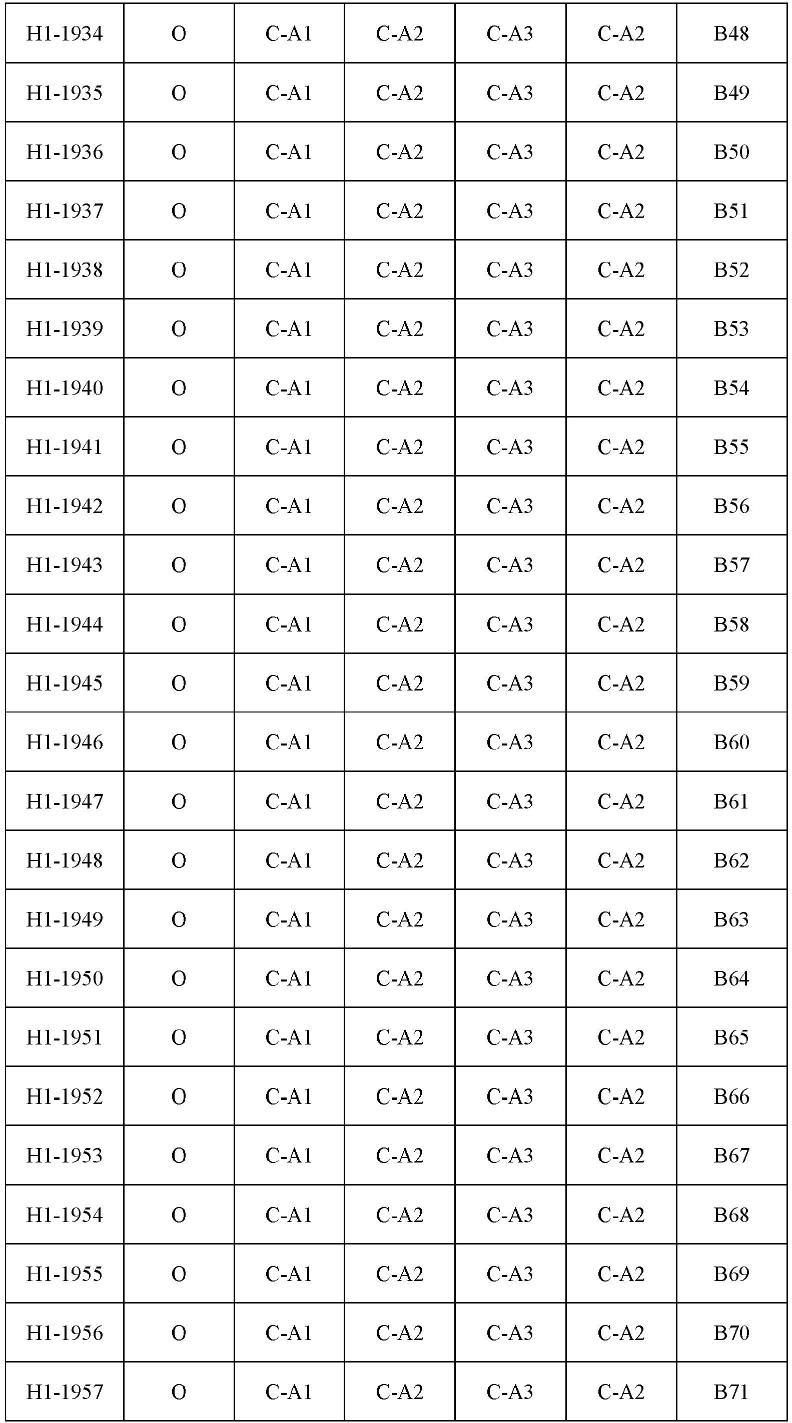 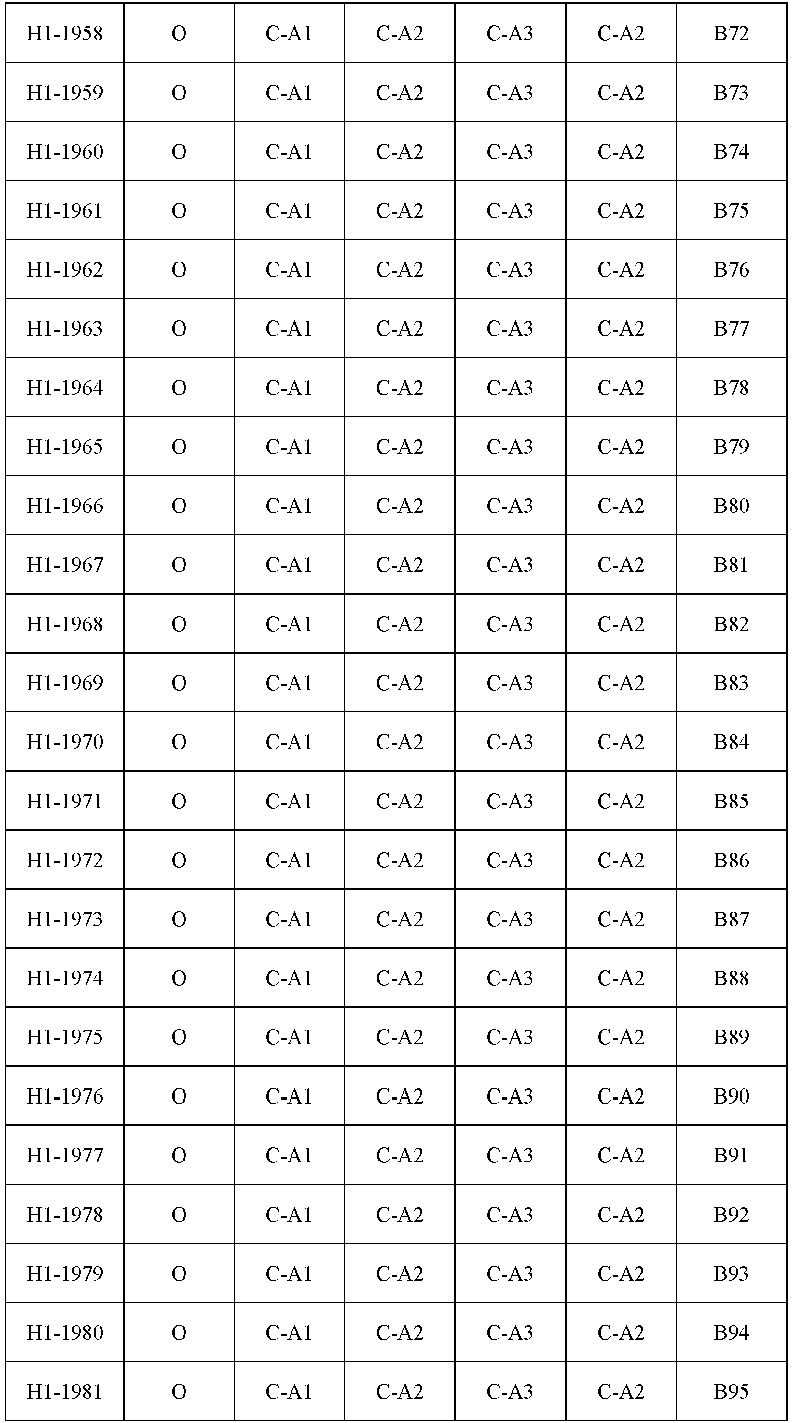 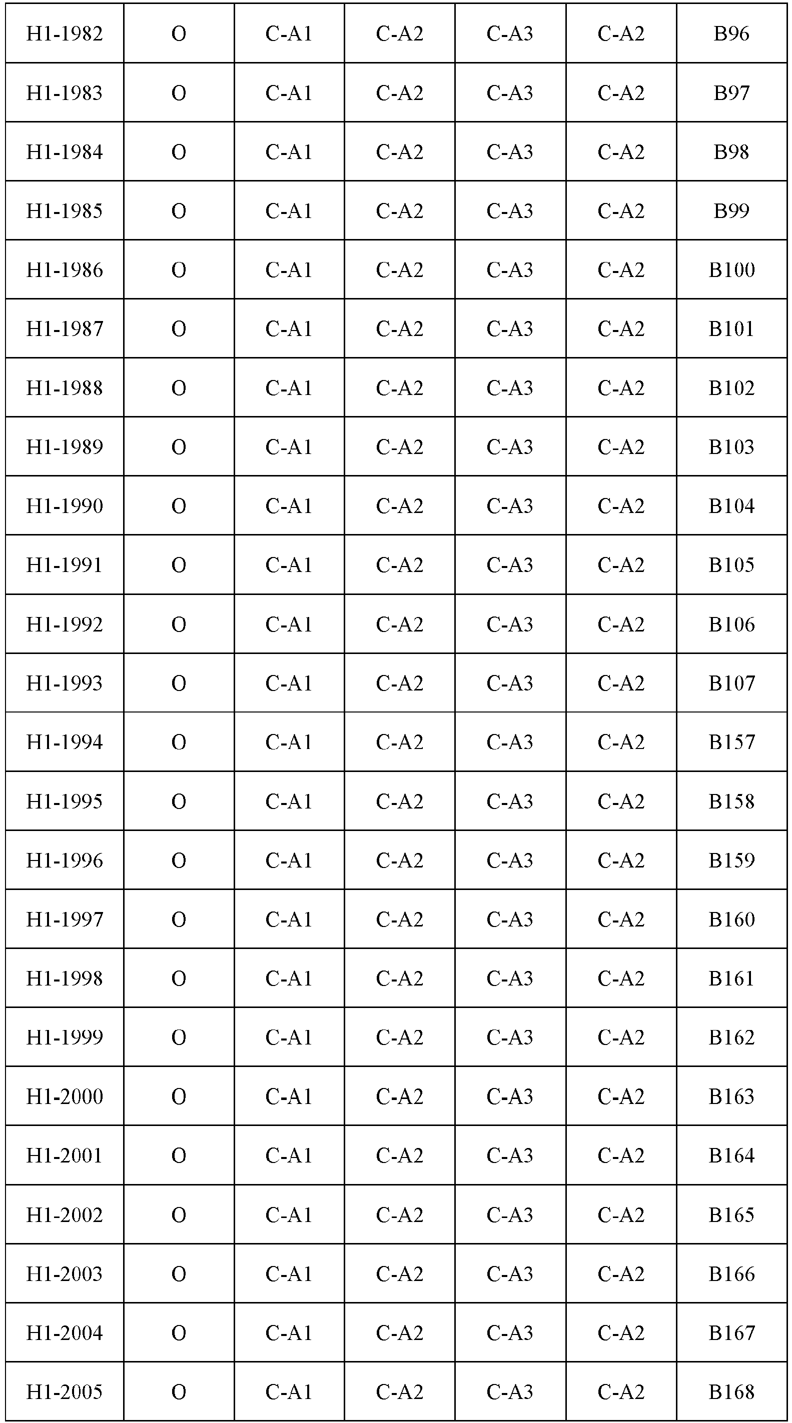 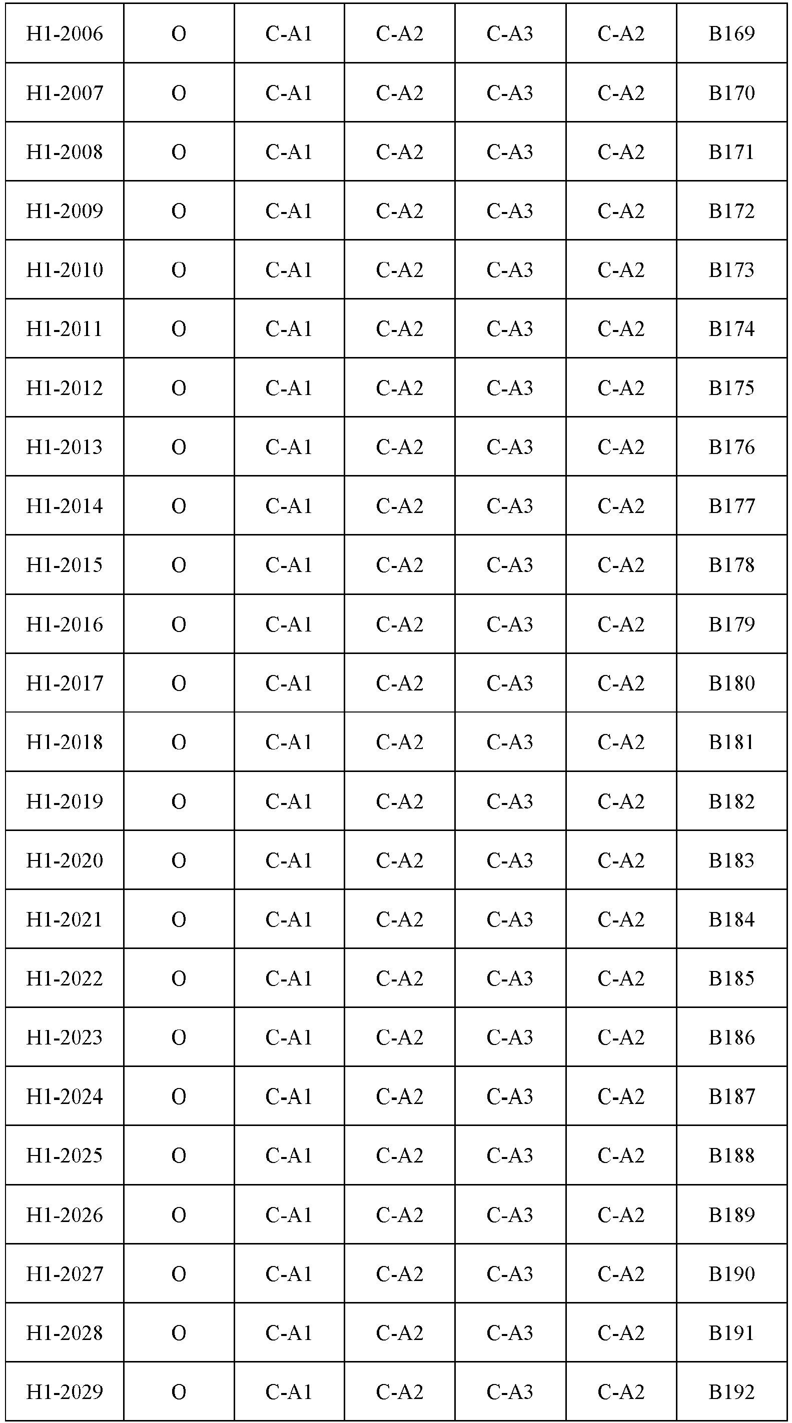 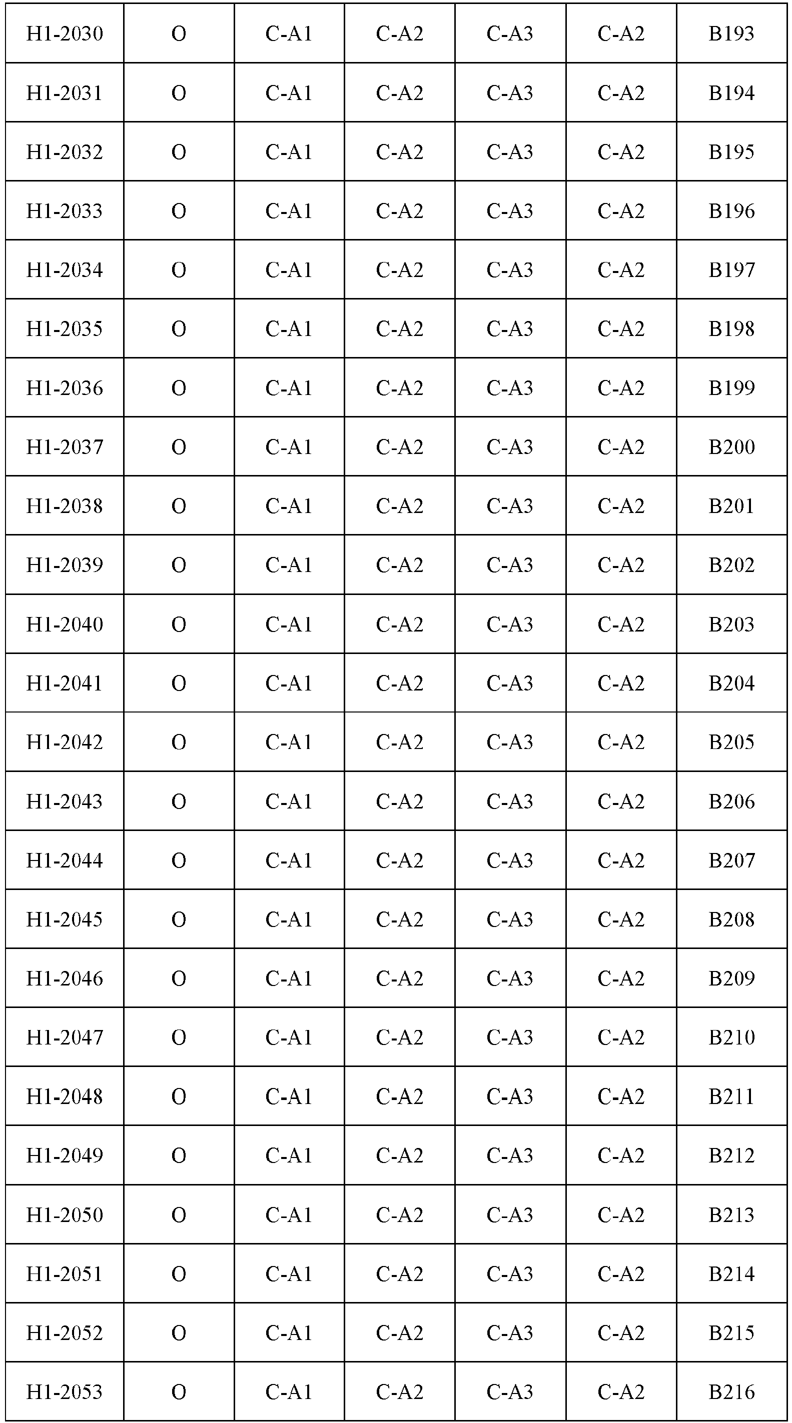 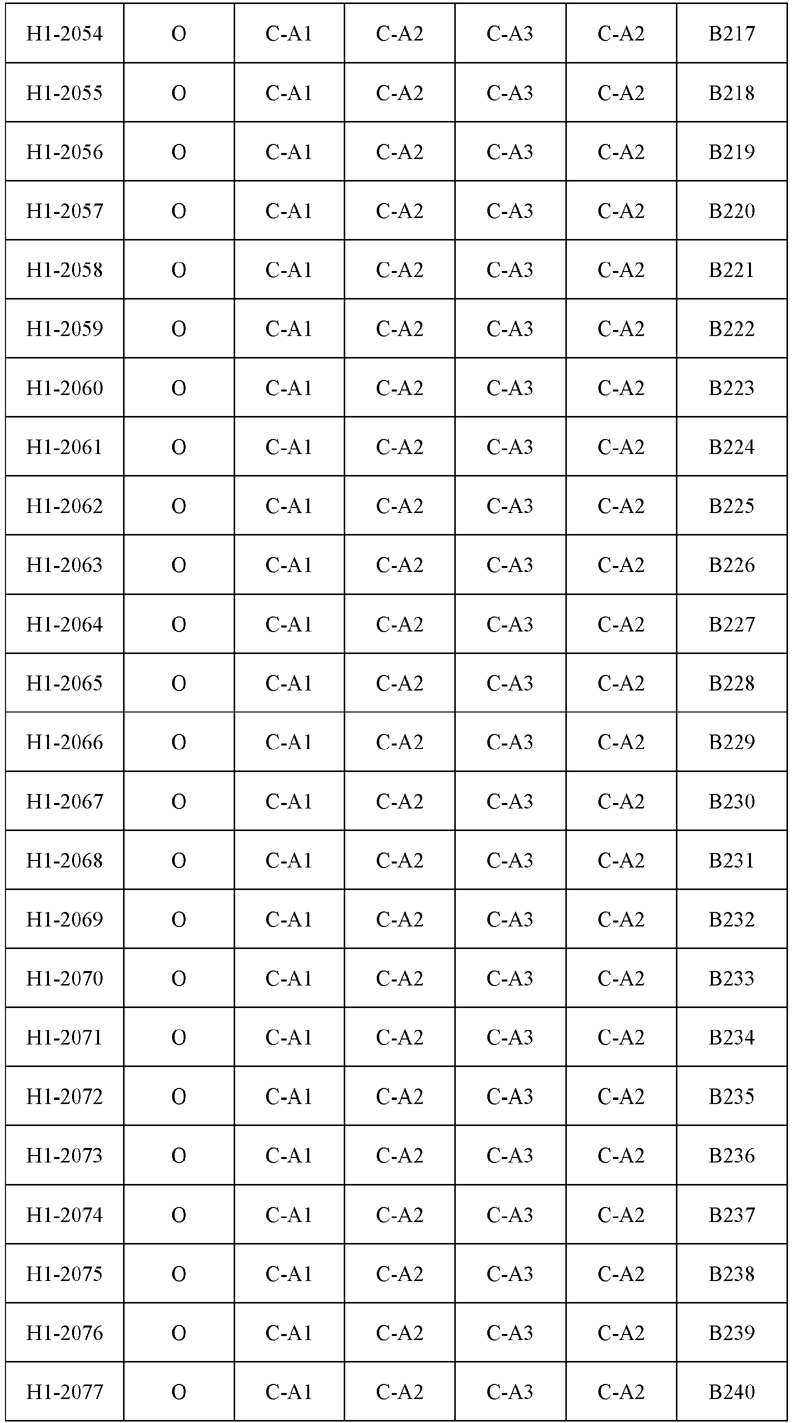 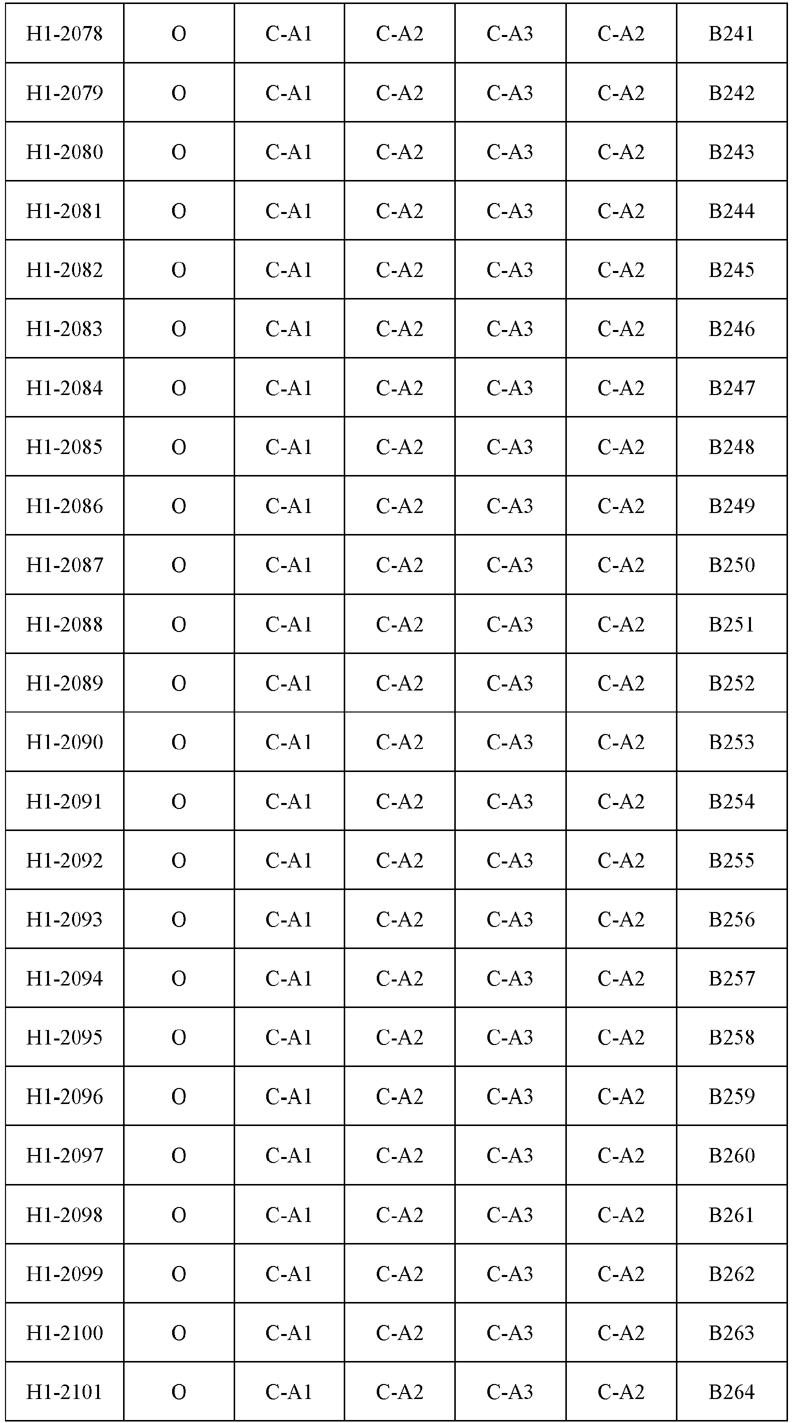 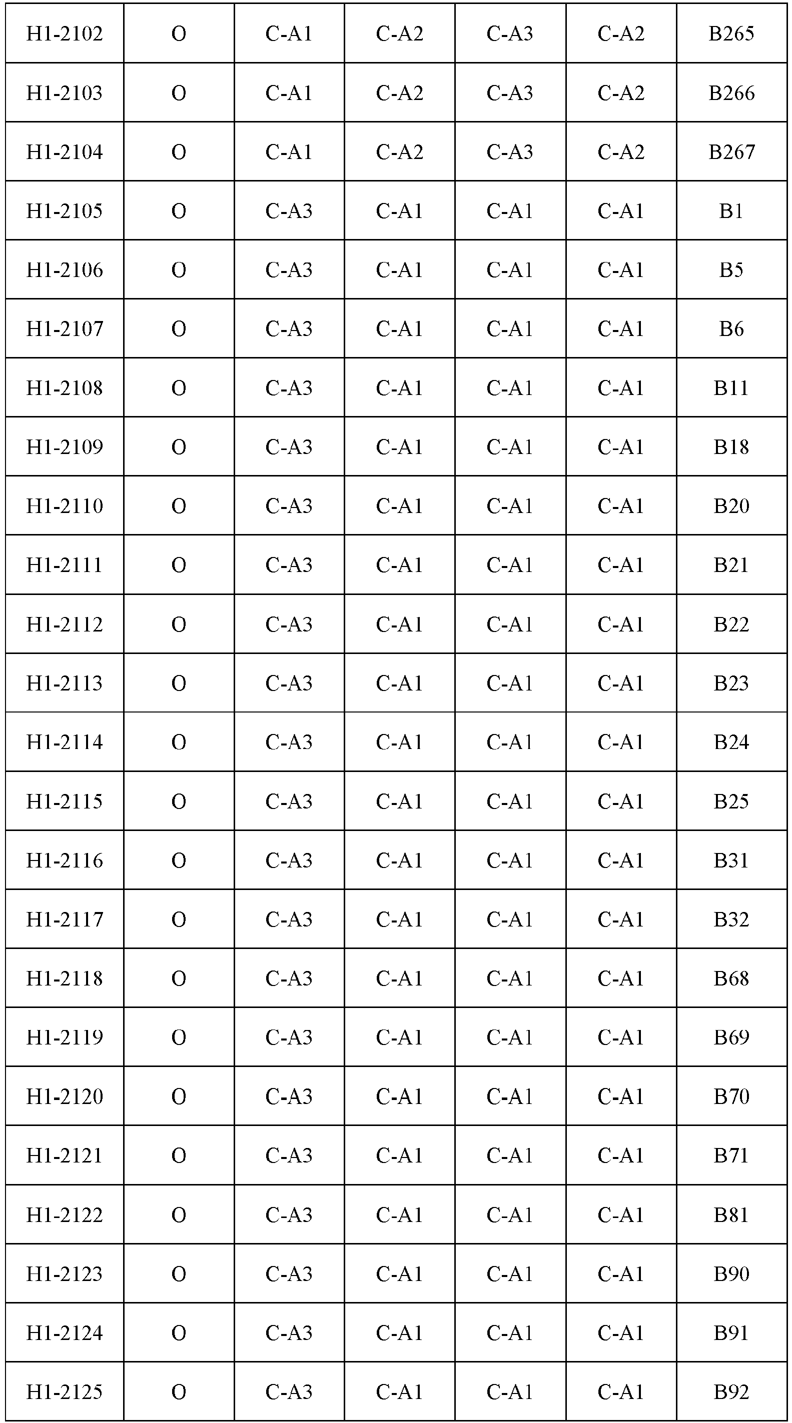 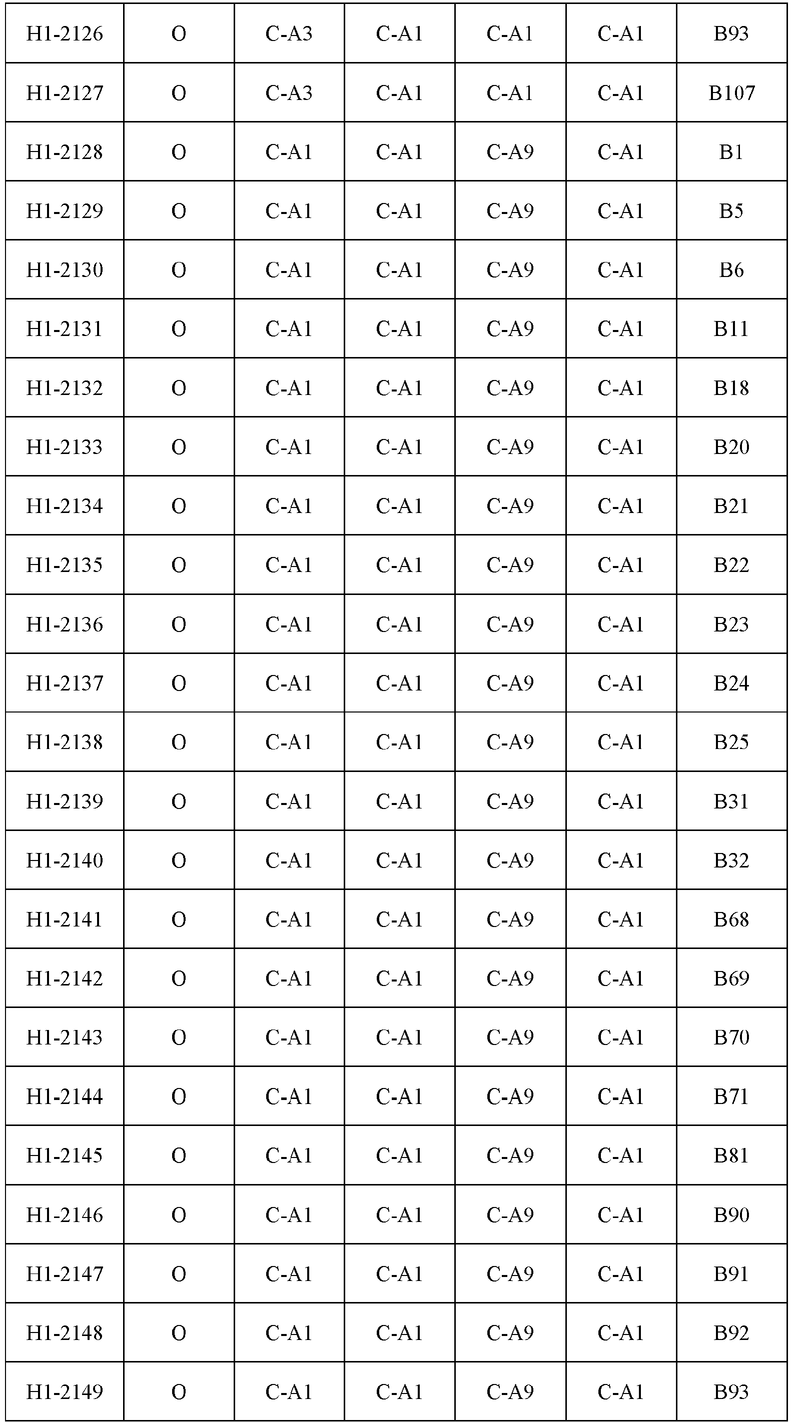 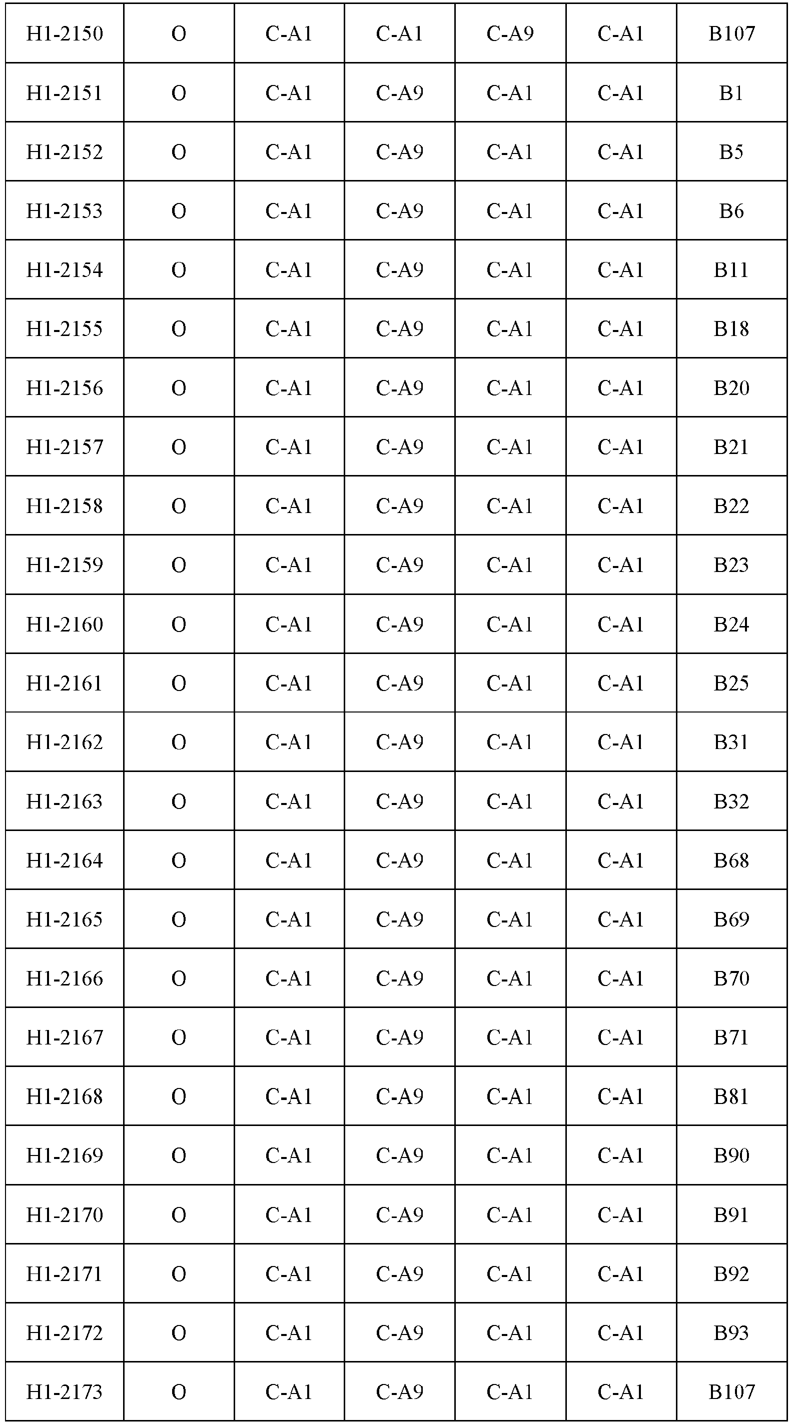 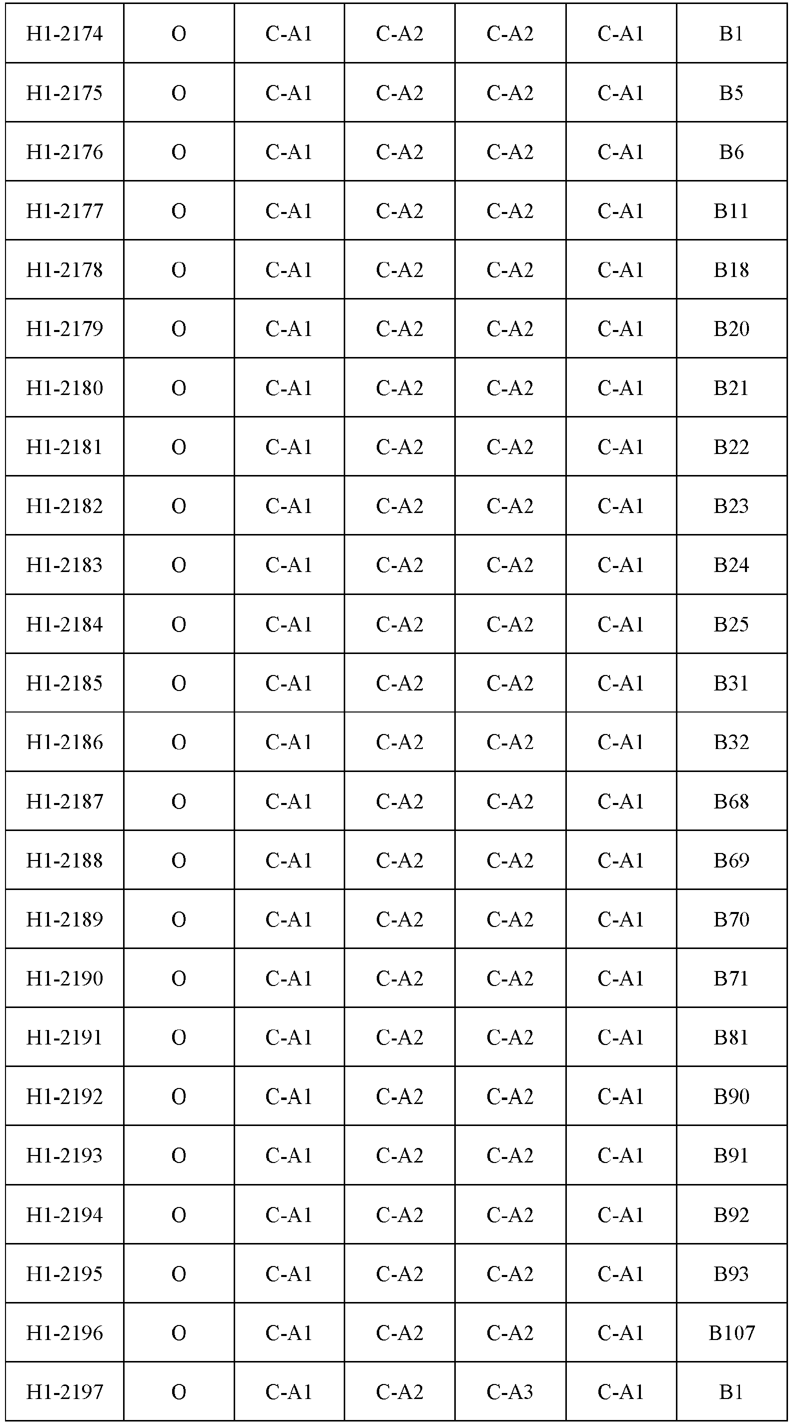 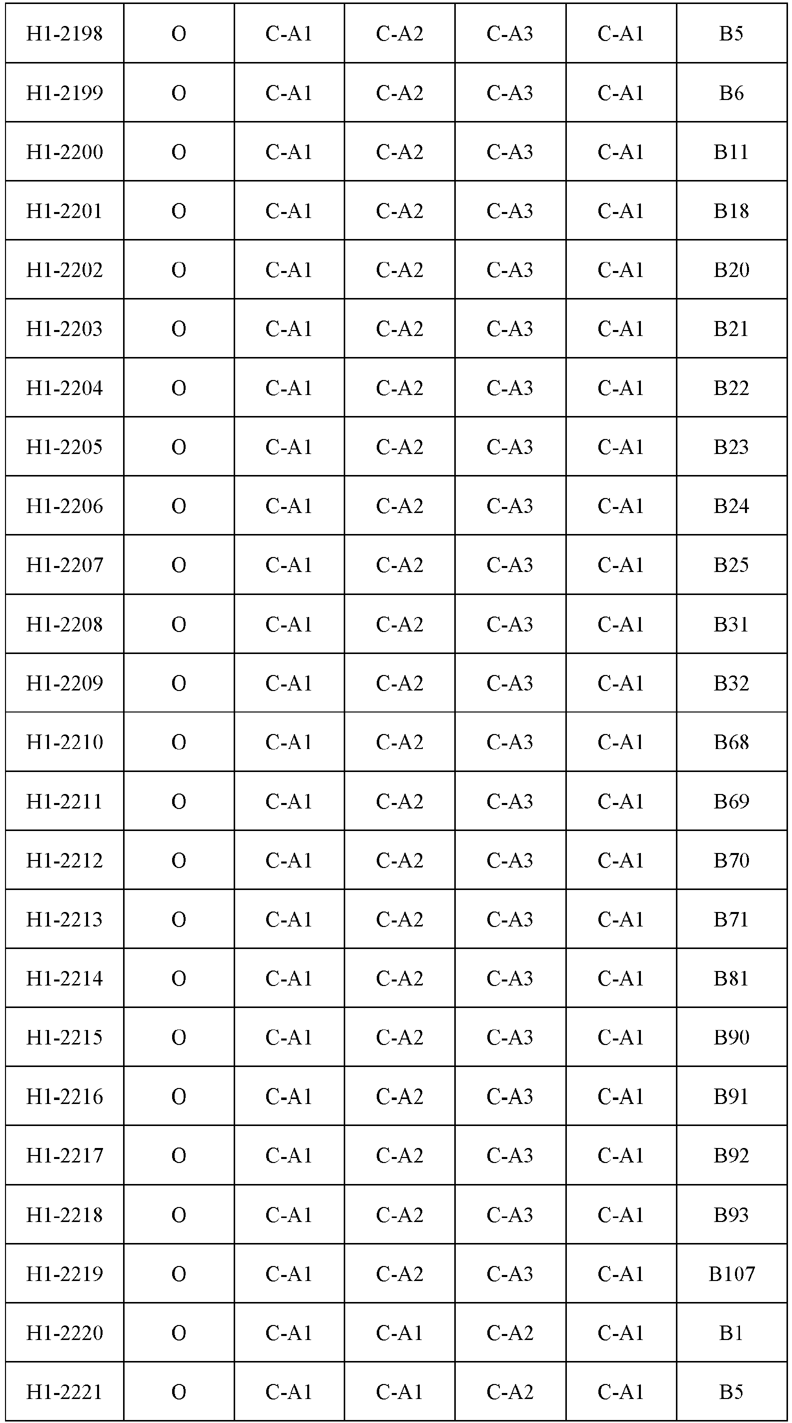 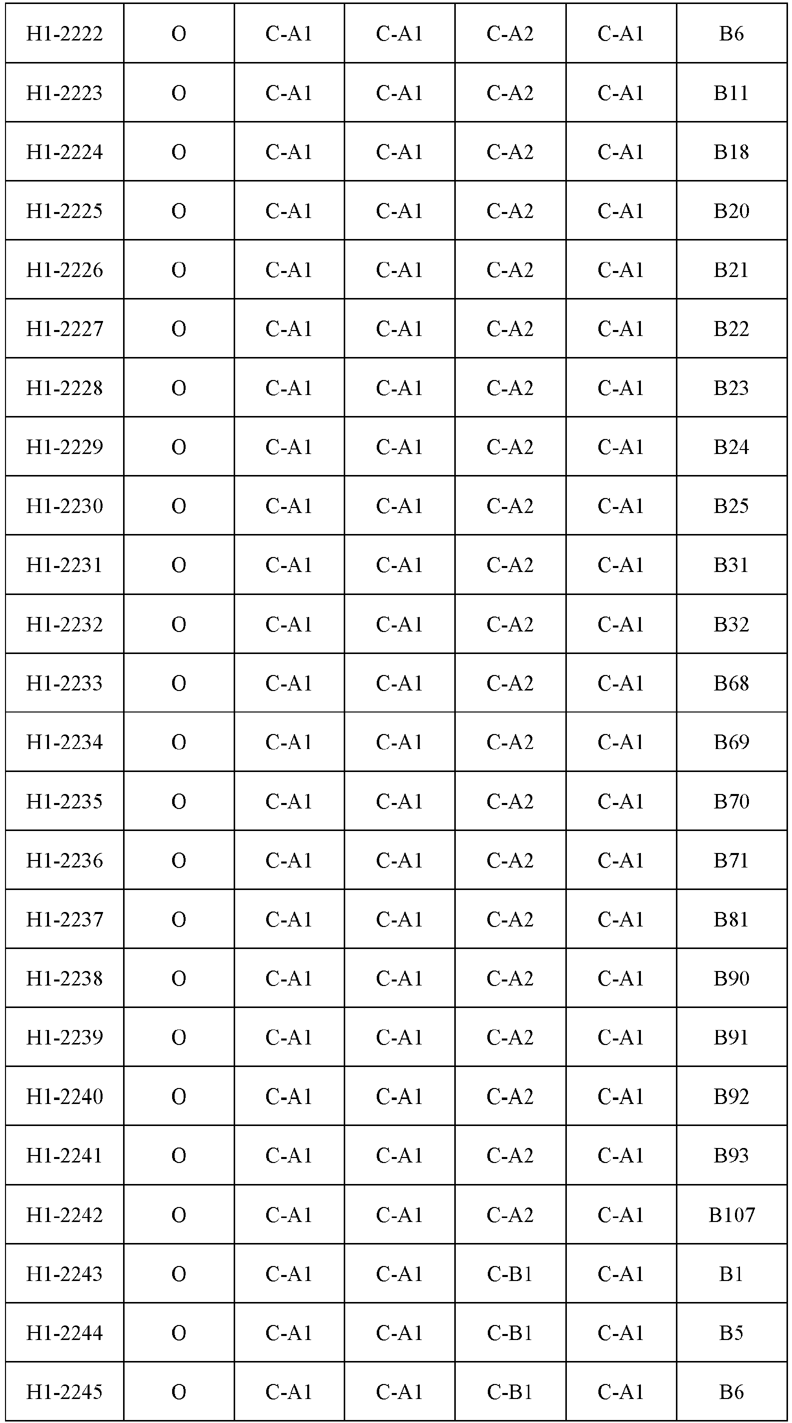 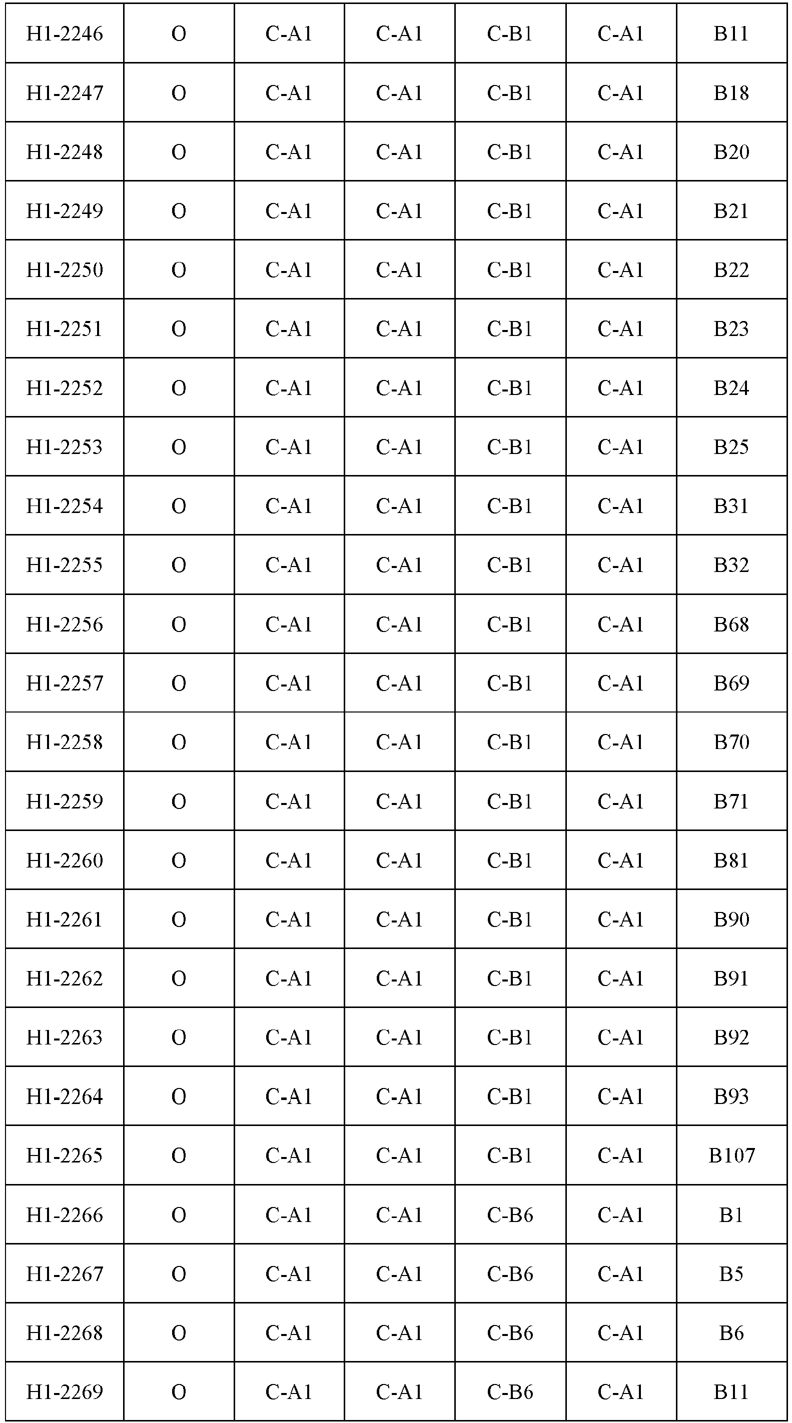 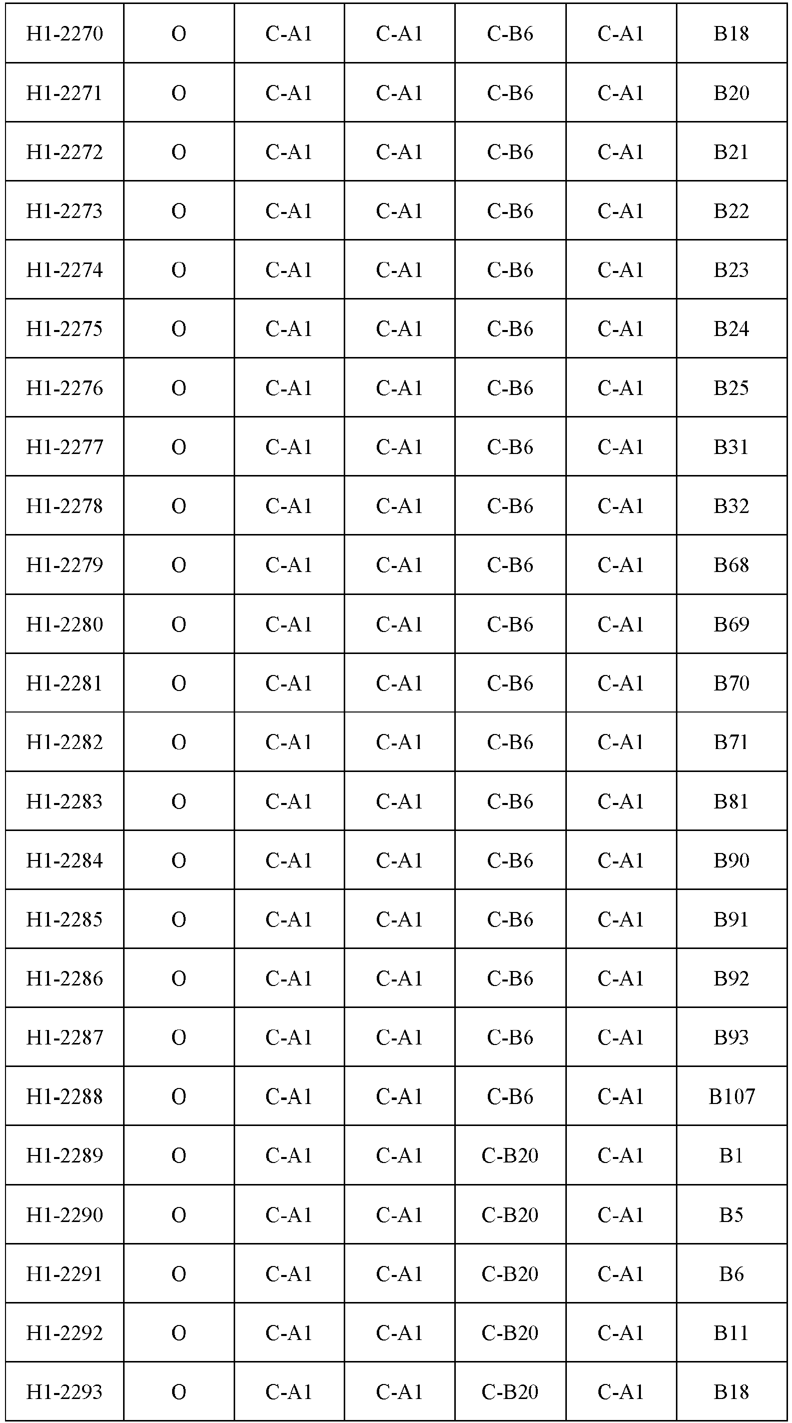 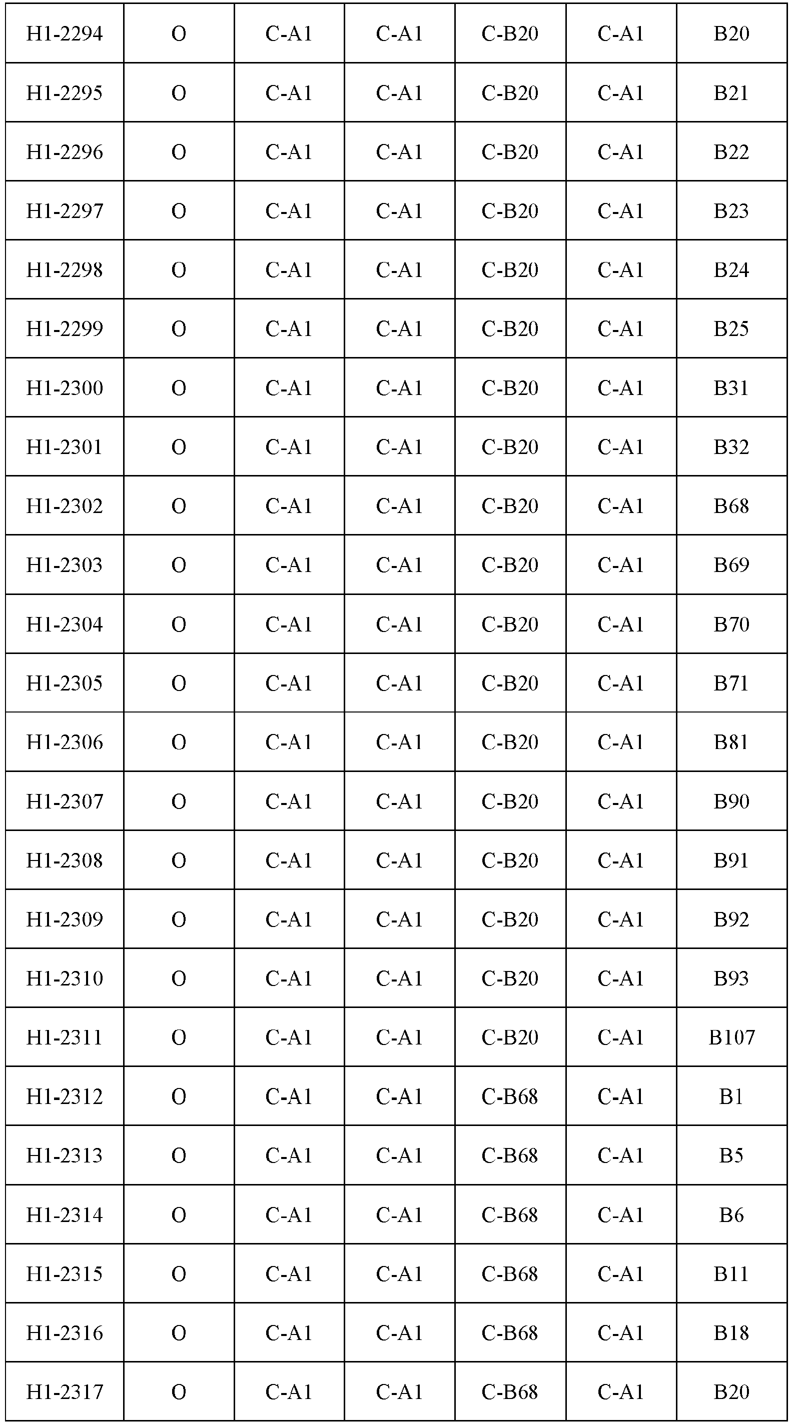 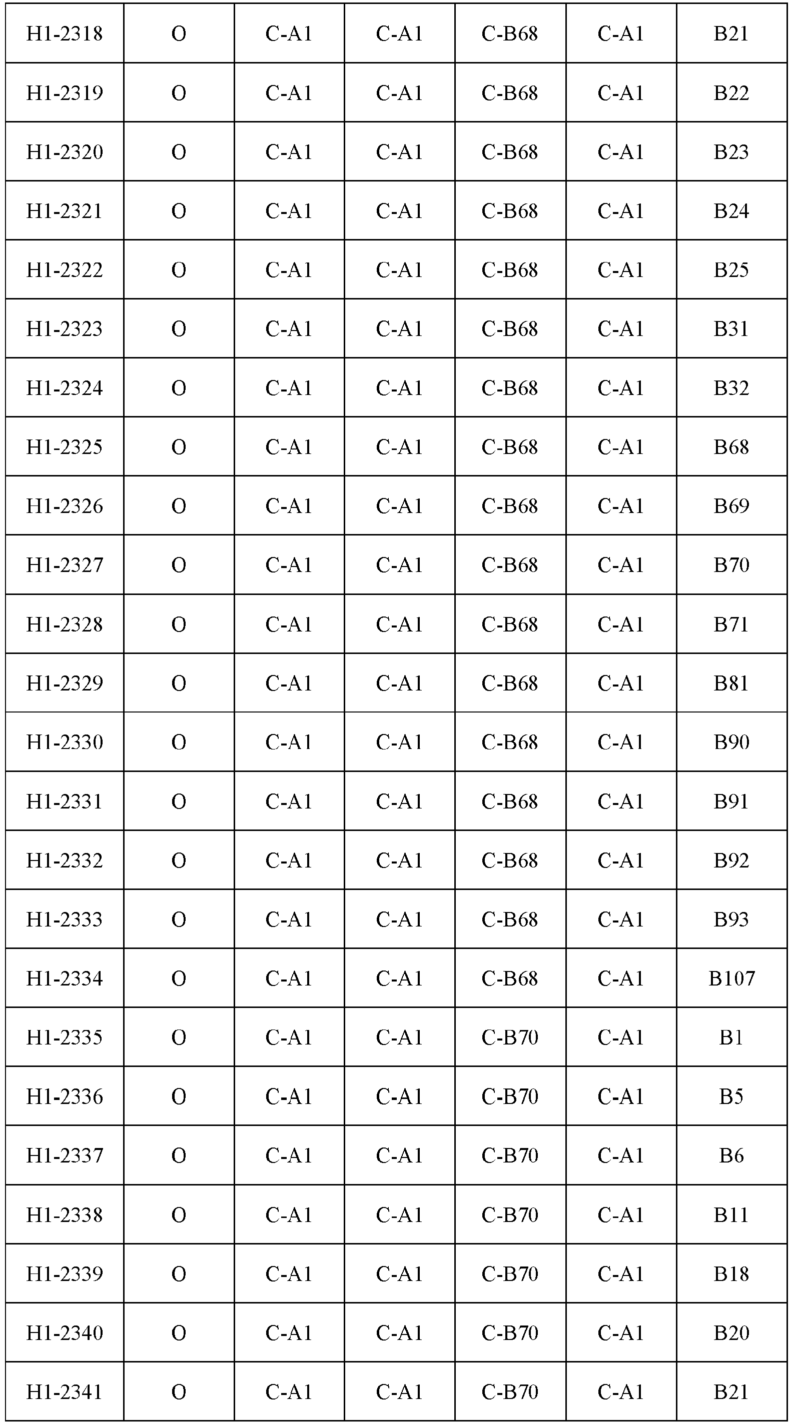 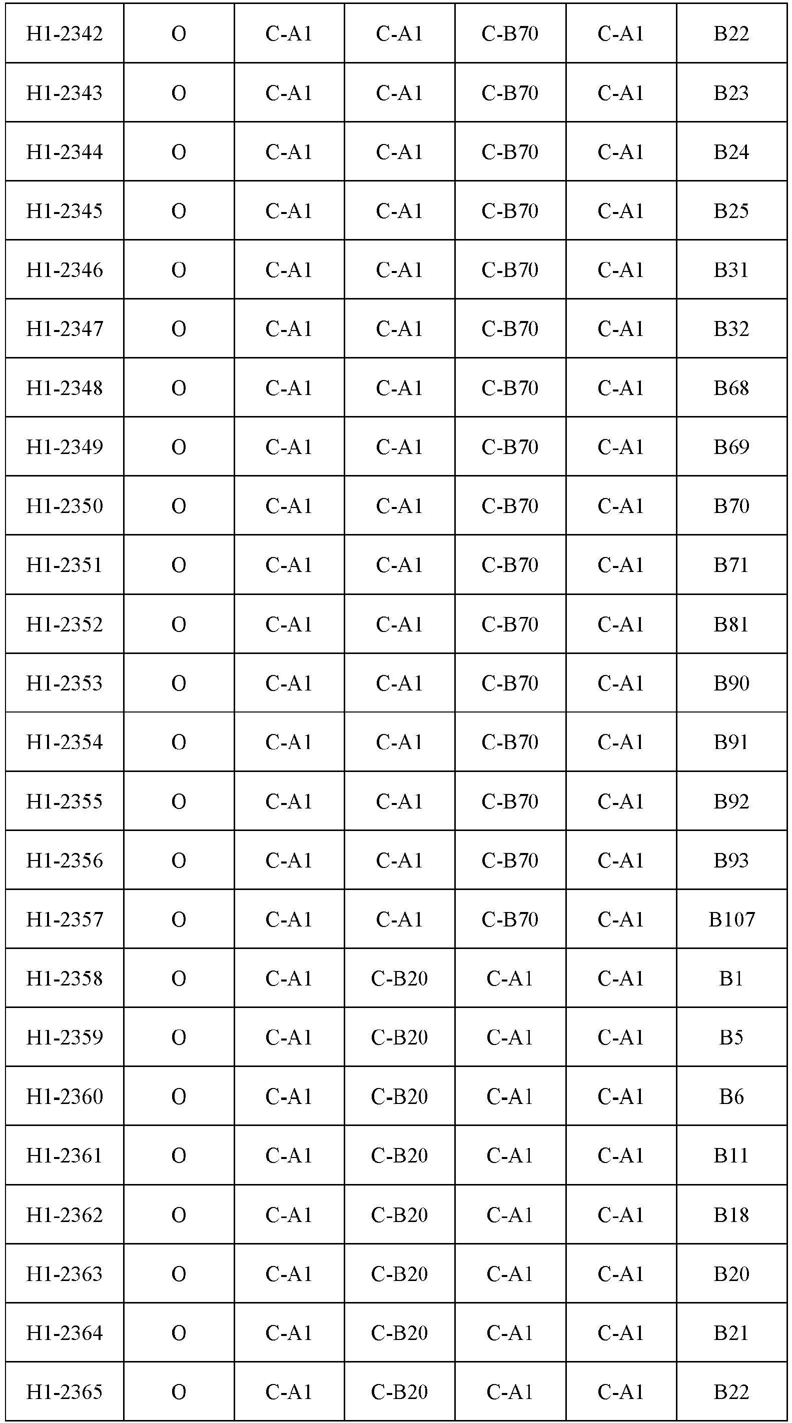 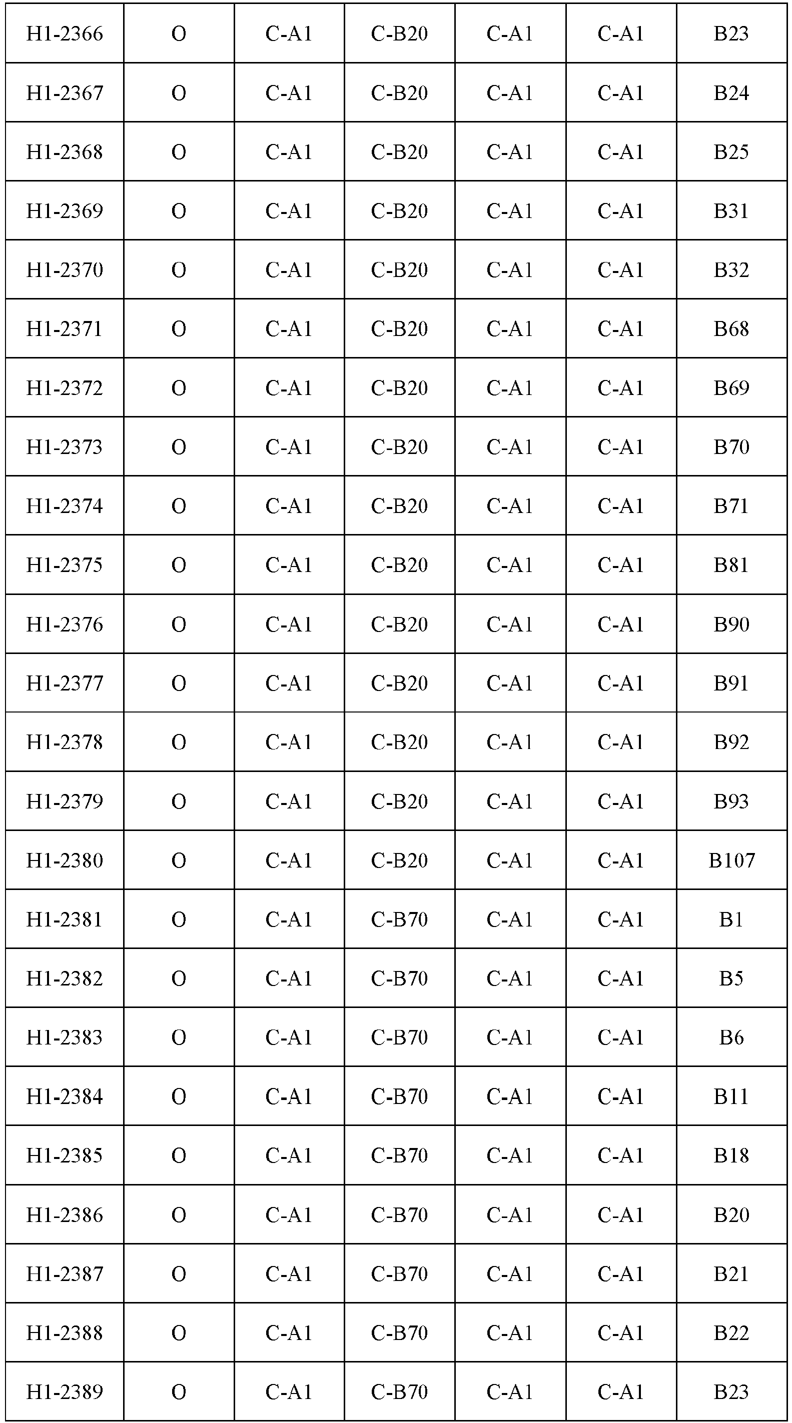 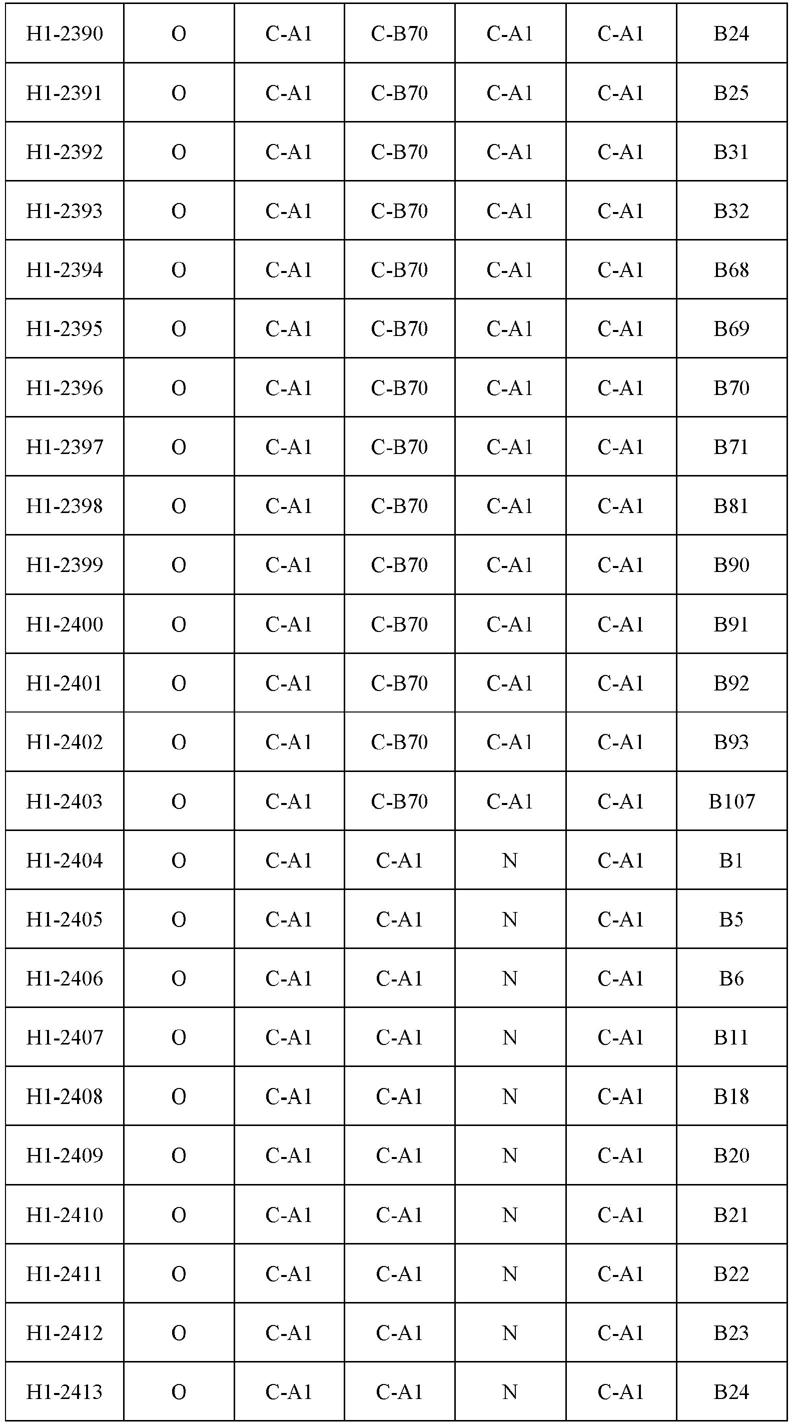 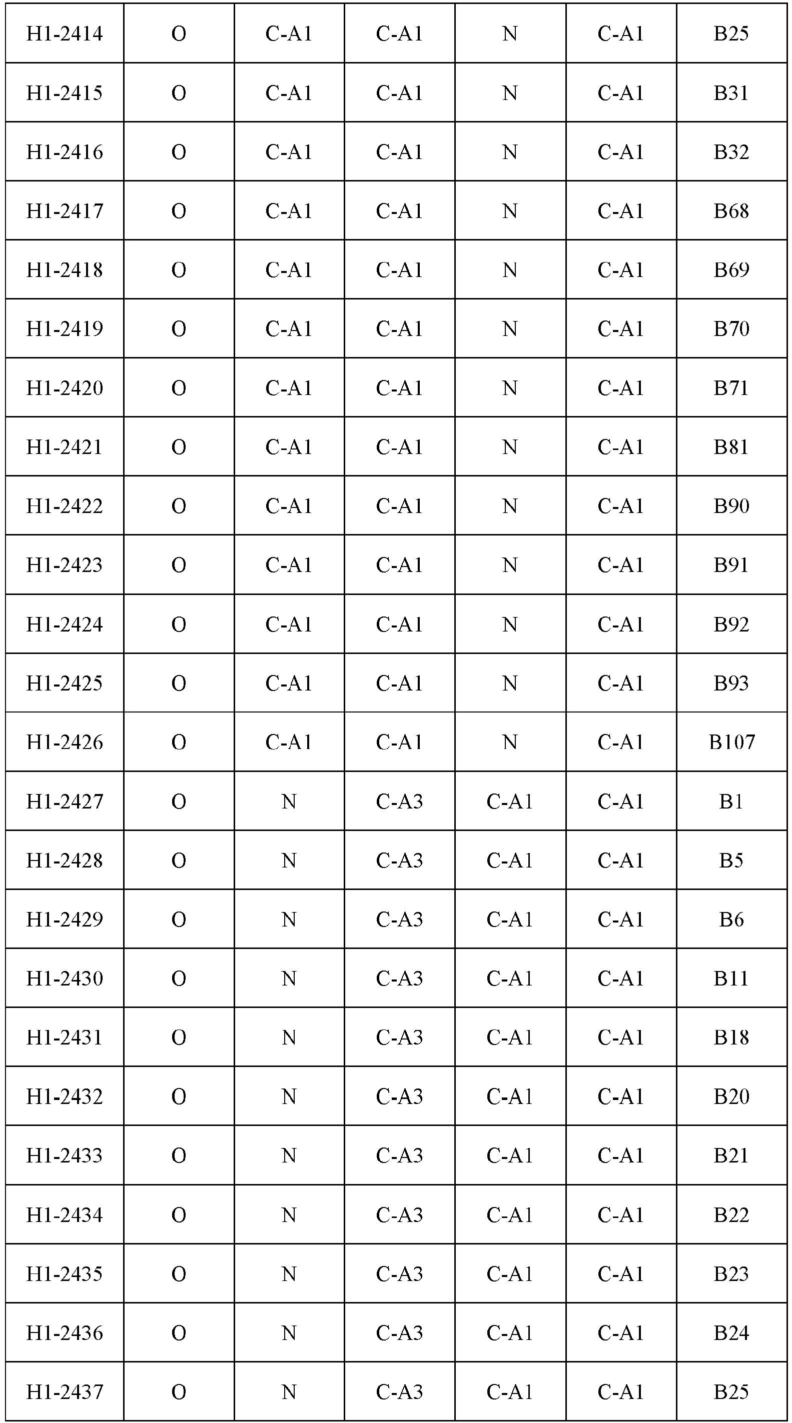 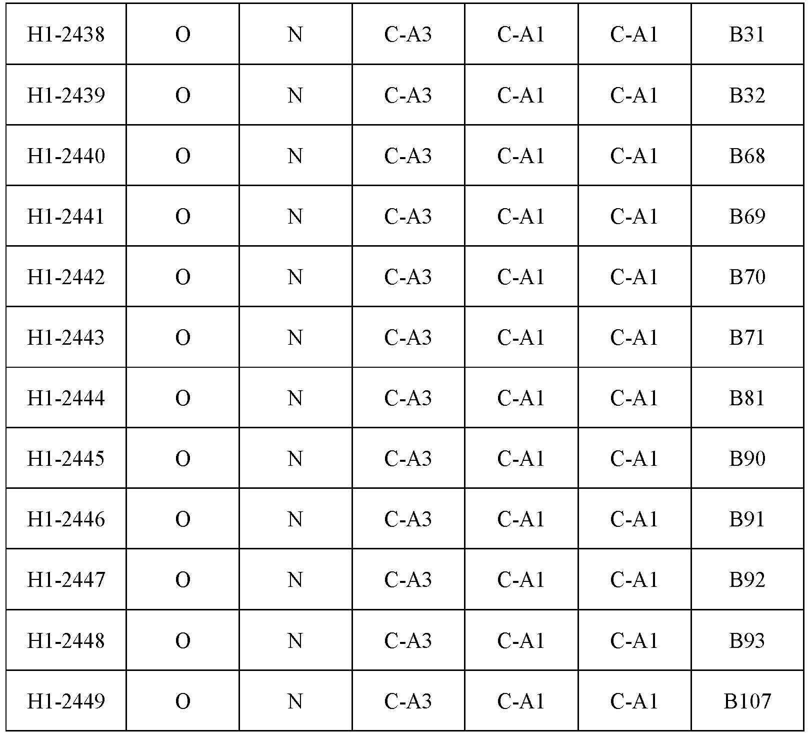
wherein Compound H2-1 to Compound H2-138 each have a structure represented by Formula 2-2-1: 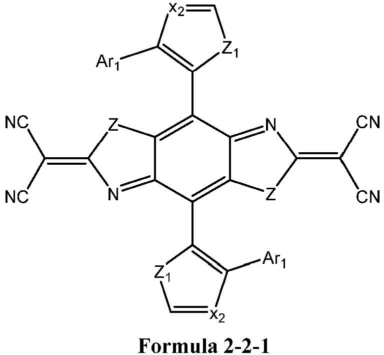
in Formula 2-2-1, two Z are the same, two Z₁ are the same, two X₂ are the same, two Ar₁ are the same, and Z, Z₁, X₂ and Ar₁ are selected from atoms or groups in the following table, respectively:
| Compound No. | Z | Z₁ | X₂ | Ar₁ |
|---|---|---|---|---|
| H2-1 | O | O | C-A1 | B1 |
| H2-2 | O | O | C-A1 | B5 |
| H2-3 | O | O | C-A1 | B6 |
| H2-4 | O | O | C-A1 | B11 |
| H2-5 | O | O | C-A1 | B18 |
| H2-6 | O | O | C-A1 | B20 |
| H2-7 | O | O | C-A1 | B21 |
| H2-8 | O | O | C-A1 | B22 |
| H2-9 | O | O | C-A1 | B23 |
| H2-10 | O | O | C-A1 | B24 |
| H2-11 | O | O | C-A1 | B25 |
| H2-12 | O | O | C-A1 | B31 |
| H2-13 | O | O | C-A1 | B32 |
| H2-14 | O | O | C-A1 | B68 |
| H2-15 | O | O | C-A1 | B69 |
| H2-16 | O | O | C-A1 | B70 |
| H2-17 | O | O | C-A1 | B71 |
| H2-18 | O | O | C-A1 | B81 |
| H2-19 | O | O | C-A1 | B90 |
| H2-20 | O | O | C-A1 | B91 |
| H2-21 | O | O | C-A1 | B92 |
| H2-22 | O | O | C-A1 | B93 |
| H2-23 | O | O | C-A1 | B107 |
| H2-24 | S | O | C-A1 | B1 |
| H2-25 | S | O | C-A1 | B5 |
| H2-26 | S | O | C-A1 | B6 |
| H2-27 | S | O | C-A1 | B11 |
| H2-28 | S | O | C-A1 | B18 |
| H2-29 | S | O | C-A1 | B20 |
| H2-30 | S | O | C-A1 | B21 |
| H2-31 | S | O | C-A1 | B22 |
| H2-32 | S | O | C-A1 | B23 |
| H2-33 | S | O | C-A1 | B24 |
| H2-34 | S | O | C-A1 | B25 |
| H2-35 | S | O | C-A1 | B31 |
| H2-36 | S | O | C-A1 | B32 |
| H2-37 | S | O | C-A1 | B68 |
| H2-38 | S | O | C-A1 | B69 |
| H2-39 | S | O | C-A1 | B70 |
| H2-40 | S | O | C-A1 | B71 |
| H2-41 | S | O | C-A1 | B81 |
| H2-42 | S | O | C-A1 | B90 |
| H2-43 | S | O | C-A1 | B91 |
| H2-44 | S | O | C-A1 | B92 |
| H2-45 | S | O | C-A1 | B93 |
| H2-46 | S | O | C-A1 | B107 |
| H2-47 | Se | O | C-A1 | B1 |
| H2-48 | Se | O | C-A1 | B5 |
| H2-49 | Se | O | C-A1 | B6 |
| H2-50 | Se | O | C-A1 | B11 |
| H2-51 | Se | O | C-A1 | B18 |
| H2-52 | Se | O | C-A1 | B20 |
| H2-53 | Se | O | C-A1 | B21 |
| H2-54 | Se | O | C-A1 | B22 |
| H2-55 | Se | O | C-A1 | B23 |
| H2-56 | Se | O | C-A1 | B24 |
| H2-57 | Se | O | C-A1 | B25 |
| H2-58 | Se | O | C-A1 | B31 |
| H2-59 | Se | O | C-A1 | B32 |
| H2-60 | Se | O | C-A1 | B68 |
| H2-61 | Se | O | C-A1 | B69 |
| H2-62 | Se | O | C-A1 | B70 |
| H2-63 | Se | O | C-A1 | B71 |
| H2-64 | Se | O | C-A1 | B81 |
| H2-65 | Se | O | C-A1 | B90 |
| H2-66 | Se | O | C-A1 | B91 |
| H2-67 | Se | O | C-A1 | B92 |
| H2-68 | Se | O | C-A1 | B93 |
| H2-69 | Se | O | C-A1 | B107 |
| H2-70 | O | S | C-A1 | B1 |
| H2-71 | O | S | C-A1 | B5 |
| H2-72 | O | S | C-A1 | B6 |
| H2-73 | O | S | C-A1 | B11 |
| H2-74 | O | S | C-A1 | B18 |
| H2-75 | O | S | C-A1 | B20 |
| H2-76 | O | S | C-A1 | B21 |
| H2-77 | O | S | C-A1 | B22 |
| H2-78 | O | S | C-A1 | B23 |
| H2-79 | O | S | C-A1 | B24 |
| H2-80 | O | S | C-A1 | B25 |
| H2-81 | O | S | C-A1 | B31 |
| H2-82 | O | S | C-A1 | B32 |
| H2-83 | O | S | C-A1 | B68 |
| H2-84 | O | S | C-A1 | B69 |
| H2-85 | O | S | C-A1 | B70 |
| H2-86 | O | S | C-A1 | B71 |
| H2-87 | O | S | C-A1 | B81 |
| H2-88 | O | S | C-A1 | B90 |
| H2-89 | O | S | C-A1 | B91 |
| H2-90 | O | S | C-A1 | B92 |
| H2-91 | O | S | C-A1 | B93 |
| H2-92 | O | S | C-A1 | B107 |
| H2-93 | O | O | N | B1 |
| H2-94 | O | O | N | B5 |
| H2-95 | O | O | N | B6 |
| H2-96 | O | O | N | B11 |
| H2-97 | O | O | N | B18 |
| H2-98 | O | O | N | B20 |
| H2-99 | O | O | N | B21 |
| H2-100 | O | O | N | B22 |
| H2-101 | O | O | N | B23 |
| H2-102 | O | O | N | B24 |
| H2-103 | O | O | N | B25 |
| H2-104 | O | O | N | B31 |
| H2-105 | O | O | N | B32 |
| H2-106 | O | O | N | B68 |
| H2-107 | O | O | N | B69 |
| H2-108 | O | O | N | B70 |
| H2-109 | O | O | N | B71 |
| H2-110 | O | O | N | B81 |
| H2-111 | O | O | N | B90 |
| H2-112 | O | O | N | B91 |
| H2-113 | O | O | N | B92 |
| H2-114 | O | O | N | B93 |
| H2-115 | O | O | N | B107 |
| H2-116 | O | S | N | B1 |
| H2-117 | O | S | N | B5 |
| H2-118 | O | S | N | B6 |
| H2-119 | O | S | N | B11 |
| H2-120 | O | S | N | B18 |
| H2-121 | O | S | N | B20 |
| H2-122 | O | S | N | B21 |
| H2-123 | O | S | N | B22 |
| H2-124 | O | S | N | B23 |
| H2-125 | O | S | N | B24 |
| H2-126 | O | S | N | B25 |
| H2-127 | O | S | N | B31 |
| H2-128 | O | S | N | B32 |
| H2-129 | O | S | N | B68 |
| H2-130 | O | S | N | B69 |
| H2-131 | O | S | N | B70 |
| H2-132 | O | S | N | B71 |
| H2-133 | O | S | N | B81 |
| H2-134 | O | S | N | B90 |
| H2-135 | O | S | N | B91 |
| H2-136 | O | S | N | B92 |
| H2-137 | O | S | N | B93 |
| H2-138 | O | S | N | B107 |
wherein Compound H3-1 to Compound H3-92 each have a structure represented by Formula 2-3-1: 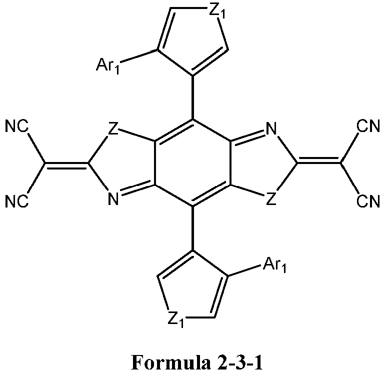
in Formula 2-3-1, two Z are the same, two Z₁ are the same, two Ar₁ are the same, and Z, Z₁ and Ar₁ are selected from atoms or groups in the following table, respectively:
| Compound No. | Z | Z₁ | Ar₁ |
|---|---|---|---|
| H3-1 | O | O | B1 |
| H3-2 | O | O | B5 |
| H3-3 | O | O | B6 |
| H3-4 | O | O | B11 |
| H3-5 | O | O | B18 |
| H3-6 | O | O | B20 |
| H3-7 | O | O | B21 |
| H3-8 | O | O | B22 |
| H3-9 | O | O | B23 |
| H3-10 | O | O | B24 |
| H3-11 | O | O | B25 |
| H3-12 | O | O | B31 |
| H3-13 | O | O | B32 |
| H3-14 | O | O | B68 |
| H3-15 | O | O | B69 |
| H3-16 | O | O | B70 |
| H3-17 | O | O | B71 |
| H3-18 | O | O | B81 |
| H3-19 | O | O | B90 |
| H3-20 | O | O | B91 |
| H3-21 | O | O | B92 |
| H3-22 | O | O | B93 |
| H3-23 | O | O | B107 |
| H3-24 | S | O | B1 |
| H3-25 | S | O | B5 |
| H3-26 | S | O | B6 |
| H3-27 | S | O | B11 |
| H3-28 | S | O | B18 |
| H3-29 | S | O | B20 |
| H3-30 | S | O | B21 |
| H3-31 | S | O | B22 |
| H3-32 | S | O | B23 |
| H3-33 | S | O | B24 |
| H3-34 | S | O | B25 |
| H3-35 | S | O | B31 |
| H3-36 | S | O | B32 |
| H3-37 | S | O | B68 |
| H3-38 | S | O | B69 |
| H3-39 | S | O | B70 |
| H3-40 | S | O | B71 |
| H3-41 | S | O | B81 |
| H3-42 | S | O | B90 |
| H3-43 | S | O | B91 |
| H3-44 | S | O | B92 |
| H3-45 | S | O | B93 |
| H3-46 | S | O | B107 |
| H3-47 | Se | O | B1 |
| H3-48 | Se | O | B5 |
| H3-49 | Se | O | B6 |
| H3-50 | Se | O | B11 |
| H3-51 | Se | O | B18 |
| H3-52 | Se | O | B20 |
| H3-53 | Se | O | B21 |
| H3-54 | Se | O | B22 |
| H3-55 | Se | O | B23 |
| H3-56 | Se | O | B24 |
| H3-57 | Se | O | B25 |
| H3-58 | Se | O | B31 |
| H3-59 | Se | O | B32 |
| H3-60 | Se | O | B68 |
| H3-61 | Se | O | B69 |
| H3-62 | Se | O | B70 |
| H3-63 | Se | O | B71 |
| H3-64 | Se | O | B81 |
| H3-65 | Se | O | B90 |
| H3-66 | Se | O | B91 |
| H3-67 | Se | O | B92 |
| H3-68 | Se | O | B93 |
| H3-69 | Se | O | B107 |
| H3-70 | O | S | B1 |
| H3-71 | O | S | B5 |
| H3-72 | O | S | B6 |
| H3-73 | O | S | B11 |
| H3-74 | O | S | B18 |
| H3-75 | O | S | B20 |
| H3-76 | O | S | B21 |
| H3-77 | O | S | B22 |
| H3-78 | O | S | B23 |
| H3-79 | O | S | B24 |
| H3-80 | O | S | B25 |
| H3-81 | O | S | B31 |
| H3-82 | O | S | B32 |
| H3-83 | O | S | B68 |
| H3-84 | O | S | B69 |
| H3-85 | O | S | B70 |
| H3-86 | O | S | B71 |
| H3-87 | O | S | B81 |
| H3-88 | O | S | B90 |
| H3-89 | O | S | B91 |
| H3-90 | O | S | B92 |
| H3-91 | O | S | B93 |
| H3-92 | O | S | B107 |
wherein Compound H4-1 to Compound H4-92 each have a structure represented by Formula 2-4-1: 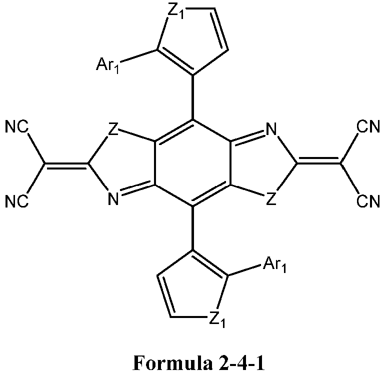
in Formula 2-4-1, two Z are the same, two Z₁ are the same, two Ar₁ are the same, and Z, Z₁ and Ar₁ are selected from atoms or groups in the following table, respectively:
| Compound No. | Z | Z₁ | Ar₁ |
|---|---|---|---|
| H4-1 | O | O | B1 |
| H4-2 | O | O | B5 |
| H4-3 | O | O | B6 |
| H4-4 | O | O | B11 |
| H4-5 | O | O | B18 |
| H4-6 | O | O | B20 |
| H4-7 | O | O | B21 |
| H4-8 | O | O | B22 |
| H4-9 | O | O | B23 |
| H4-10 | O | O | B24 |
| H4-11 | O | O | B25 |
| H4-12 | O | O | B31 |
| H4-13 | O | O | B32 |
| H4-14 | O | O | B68 |
| H4-15 | O | O | B69 |
| H4-16 | O | O | B70 |
| H4-17 | O | O | B71 |
| H4-18 | O | O | B81 |
| H4-19 | O | O | B90 |
| H4-20 | O | O | B91 |
| H4-21 | O | O | B92 |
| H4-22 | O | O | B93 |
| H4-23 | O | O | B107 |
| H4-24 | S | O | B1 |
| H4-25 | S | O | B5 |
| H4-26 | S | O | B6 |
| H4-27 | S | O | B11 |
| H4-28 | S | O | B18 |
| H4-29 | S | O | B20 |
| H4-30 | S | O | B21 |
| H4-31 | S | O | B22 |
| H4-32 | S | O | B23 |
| H4-33 | S | O | B24 |
| H4-34 | S | O | B25 |
| H4-35 | S | O | B31 |
| H4-36 | S | O | B32 |
| H4-37 | S | O | B68 |
| H4-38 | S | O | B69 |
| H4-39 | S | O | B70 |
| H4-40 | S | O | B71 |
| H4-41 | S | O | B81 |
| H4-42 | S | O | B90 |
| H4-43 | S | O | B91 |
| H4-44 | S | O | B92 |
| H4-45 | S | O | B93 |
| H4-46 | S | O | B107 |
| H4-47 | Se | O | B1 |
| H4-48 | Se | O | B5 |
| H4-49 | Se | O | B6 |
| H4-50 | Se | O | B11 |
| H4-51 | Se | O | B18 |
| H4-52 | Se | O | B20 |
| H4-53 | Se | O | B21 |
| H4-54 | Se | O | B22 |
| H4-55 | Se | O | B23 |
| H4-56 | Se | O | B24 |
| H4-57 | Se | O | B25 |
| H4-58 | Se | O | B31 |
| H4-59 | Se | O | B32 |
| H4-60 | Se | O | B68 |
| H4-61 | Se | O | B69 |
| H4-62 | Se | O | B70 |
| H4-63 | Se | O | B71 |
| H4-64 | Se | O | B81 |
| H4-65 | Se | O | B90 |
| H4-66 | Se | O | B91 |
| H4-67 | Se | O | B92 |
| H4-68 | Se | O | B93 |
| H4-69 | Se | O | B107 |
| H4-70 | O | S | B1 |
| H4-71 | O | S | B5 |
| H4-72 | O | S | B6 |
| H4-73 | O | S | B11 |
| H4-74 | O | S | B18 |
| H4-75 | O | S | B20 |
| H4-76 | O | S | B21 |
| H4-77 | O | S | B22 |
| H4-78 | O | S | B23 |
| H4-79 | O | S | B24 |
| H4-80 | O | S | B25 |
| H4-81 | O | S | B31 |
| H4-82 | O | S | B32 |
| H4-83 | O | S | B68 |
| H4-84 | O | S | B69 |
| H4-85 | O | S | B70 |
| H4-86 | O | S | B71 |
| H4-87 | O | S | B81 |
| H4-88 | O | S | B90 |
| H4-89 | O | S | B91 |
| H4-90 | O | S | B92 |
| H4-91 | O | S | B93 |
| H4-92 | O | S | B107 |
wherein Compound H5-1 to Compound H5-115 each have a structure represented by Formula 2-5: 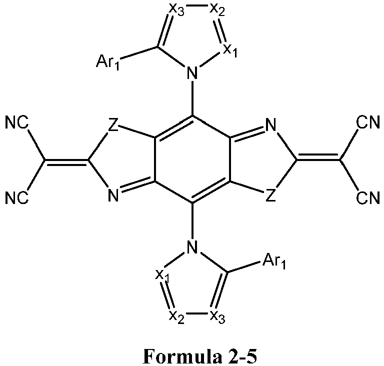
in Formula 2-5, two Z are the same, two X₁ are the same, two X₂ are the same, two X₃ are the same, two Ar₁ are the same, and Z, X₁, X₂, X₃ and Ar₁ are selected from atoms or groups in the following table, respectively:
| Compound No. | Z | X₁ | X₂ | X₃ | Ar₁ |
|---|---|---|---|---|---|
| H5-1 | O | C-A1 | C-A1 | C-A1 | B1 |
| H5-2 | O | C-A1 | C-A1 | C-A1 | B5 |
| H5-3 | O | C-A1 | C-A1 | C-A1 | B6 |
| H5-4 | O | C-A1 | C-A1 | C-A1 | B11 |
| H5-5 | O | C-A1 | C-A1 | C-A1 | B18 |
| H5-6 | O | C-A1 | C-A1 | C-A1 | B20 |
| H5-7 | O | C-A1 | C-A1 | C-A1 | B21 |
| H5-8 | O | C-A1 | C-A1 | C-A1 | B22 |
| H5-9 | O | C-A1 | C-A1 | C-A1 | B23 |
| H5-10 | O | C-A1 | C-A1 | C-A1 | B24 |
| H5-11 | O | C-A1 | C-A1 | C-A1 | B25 |
| H5-12 | O | C-A1 | C-A1 | C-A1 | B31 |
| H5-13 | O | C-A1 | C-A1 | C-A1 | B32 |
| H5-14 | O | C-A1 | C-A1 | C-A1 | B68 |
| H5-15 | O | C-A1 | C-A1 | C-A1 | B69 |
| H5-16 | O | C-A1 | C-A1 | C-A1 | B70 |
| H5-17 | O | C-A1 | C-A1 | C-A1 | B71 |
| H5-18 | O | C-A1 | C-A1 | C-A1 | B81 |
| H5-19 | O | C-A1 | C-A1 | C-A1 | B90 |
| H5-20 | O | C-A1 | C-A1 | C-A1 | B91 |
| H5-21 | O | C-A1 | C-A1 | C-A1 | B92 |
| H5-22 | O | C-A1 | C-A1 | C-A1 | B93 |
| H5-23 | O | C-A1 | C-A1 | C-A1 | B107 |
| H5-24 | S | C-A1 | C-A1 | C-A1 | B1 |
| H5-25 | S | C-A1 | C-A1 | C-A1 | B5 |
| H5-26 | S | C-A1 | C-A1 | C-A1 | B6 |
| H5-27 | S | C-A1 | C-A1 | C-A1 | B11 |
| H5-28 | S | C-A1 | C-A1 | C-A1 | B18 |
| H5-29 | S | C-A1 | C-A1 | C-A1 | B20 |
| H5-30 | S | C-A1 | C-A1 | C-A1 | B21 |
| H5-31 | S | C-A1 | C-A1 | C-A1 | B22 |
| H5-32 | S | C-A1 | C-A1 | C-A1 | B23 |
| H5-33 | S | C-A1 | C-A1 | C-A1 | B24 |
| H5-34 | S | C-A1 | C-A1 | C-A1 | B25 |
| H5-35 | S | C-A1 | C-A1 | C-A1 | B31 |
| H5-36 | S | C-A1 | C-A1 | C-A1 | B32 |
| H5-37 | S | C-A1 | C-A1 | C-A1 | B68 |
| H5-38 | S | C-A1 | C-A1 | C-A1 | B69 |
| H5-39 | S | C-A1 | C-A1 | C-A1 | B70 |
| H5-40 | S | C-A1 | C-A1 | C-A1 | B71 |
| H5-41 | S | C-A1 | C-A1 | C-A1 | B81 |
| H5-42 | S | C-A1 | C-A1 | C-A1 | B90 |
| H5-43 | S | C-A1 | C-A1 | C-A1 | B91 |
| H5-44 | S | C-A1 | C-A1 | C-A1 | B92 |
| H5-45 | S | C-A1 | C-A1 | C-A1 | B93 |
| H5-46 | S | C-A1 | C-A1 | C-A1 | B107 |
| H5-47 | Se | C-A1 | C-A1 | C-A1 | B1 |
| H5-48 | Se | C-A1 | C-A1 | C-A1 | B5 |
| H5-49 | Se | C-A1 | C-A1 | C-A1 | B6 |
| H5-50 | Se | C-A1 | C-A1 | C-A1 | B11 |
| H5-51 | Se | C-A1 | C-A1 | C-A1 | B18 |
| H5-52 | Se | C-A1 | C-A1 | C-A1 | B20 |
| H5-53 | Se | C-A1 | C-A1 | C-A1 | B21 |
| H5-54 | Se | C-A1 | C-A1 | C-A1 | B22 |
| H5-55 | Se | C-A1 | C-A1 | C-A1 | B23 |
| H5-56 | Se | C-A1 | C-A1 | C-A1 | B24 |
| H5-57 | Se | C-A1 | C-A1 | C-A1 | B25 |
| H5-58 | Se | C-A1 | C-A1 | C-A1 | B31 |
| H5-59 | Se | C-A1 | C-A1 | C-A1 | B32 |
| H5-60 | Se | C-A1 | C-A1 | C-A1 | B68 |
| H5-61 | Se | C-A1 | C-A1 | C-A1 | B69 |
| H5-62 | Se | C-A1 | C-A1 | C-A1 | B70 |
| H5-63 | Se | C-A1 | C-A1 | C-A1 | B71 |
| H5-64 | Se | C-A1 | C-A1 | C-A1 | B81 |
| H5-65 | Se | C-A1 | C-A1 | C-A1 | B90 |
| H5-66 | Se | C-A1 | C-A1 | C-A1 | B91 |
| H5-67 | Se | C-A1 | C-A1 | C-A1 | B92 |
| H5-68 | Se | C-A1 | C-A1 | C-A1 | B93 |
| H5-69 | Se | C-A1 | C-A1 | C-A1 | B107 |
| H5-70 | O | C-A1 | C-A1 | N | B1 |
| H5-71 | O | C-A1 | C-A1 | N | B5 |
| H5-72 | O | C-A1 | C-A1 | N | B6 |
| H5-73 | O | C-A1 | C-A1 | N | B11 |
| H5-74 | O | C-A1 | C-A1 | N | B18 |
| H5-75 | O | C-A1 | C-A1 | N | B20 |
| H5-76 | O | C-A1 | C-A1 | N | B21 |
| H5-77 | O | C-A1 | C-A1 | N | B22 |
| H5-78 | O | C-A1 | C-A1 | N | B23 |
| H5-79 | O | C-A1 | C-A1 | N | B24 |
| H5-80 | O | C-A1 | C-A1 | N | B25 |
| H5-81 | O | C-A1 | C-A1 | N | B31 |
| H5-82 | O | C-A1 | C-A1 | N | B32 |
| H5-83 | O | C-A1 | C-A1 | N | B68 |
| H5-84 | O | C-A1 | C-A1 | N | B69 |
| H5-85 | O | C-A1 | C-A1 | N | B70 |
| H5-86 | O | C-A1 | C-A1 | N | B71 |
| H5-87 | O | C-A1 | C-A1 | N | B81 |
| H5-88 | O | C-A1 | C-A1 | N | B90 |
| H5-89 | O | C-A1 | C-A1 | N | B91 |
| H5-90 | O | C-A1 | C-A1 | N | B92 |
| H5-91 | O | C-A1 | C-A1 | N | B93 |
| H5-92 | O | C-A1 | C-A1 | N | B107 |
| H5-93 | O | C-A1 | N | C-A1 | B1 |
| H5-94 | O | C-A1 | N | C-A1 | B5 |
| H5-95 | O | C-A1 | N | C-A1 | B6 |
| H5-96 | O | C-A1 | N | C-A1 | B11 |
| H5-97 | O | C-A1 | N | C-A1 | B18 |
| H5-98 | O | C-A1 | N | C-A1 | B20 |
| H5-99 | O | C-A1 | N | C-A1 | B21 |
| H5-100 | O | C-A1 | N | C-A1 | B22 |
| H5-101 | O | C-A1 | N | C-A1 | B23 |
| H5-102 | O | C-A1 | N | C-A1 | B24 |
| H5-103 | O | C-A1 | N | C-A1 | B25 |
| H5-104 | O | C-A1 | N | C-A1 | B31 |
| H5-105 | O | C-A1 | N | C-A1 | B32 |
| H5-106 | O | C-A1 | N | C-A1 | B68 |
| H5-107 | O | C-A1 | N | C-A1 | B69 |
| H5-108 | O | C-A1 | N | C-A1 | B70 |
| H5-109 | O | C-A1 | N | C-A1 | B71 |
| HS-110 | O | C-A1 | N | C-A1 | B81 |
| H5-111 | O | C-A1 | N | C-A1 | B90 |
| H5-112 | O | C-A1 | N | C-A1 | B91 |
| H5-113 | O | C-A1 | N | C-A1 | B92 |
| H5-114 | O | C-A1 | N | C-A1 | B93 |
| H5-115 | O | C-A1 | N | C-A1 | B107 |
wherein Compound H6-1 to Compound H6-115 each have a structure represented by Formula 2-6: 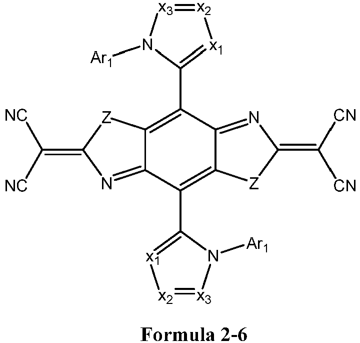
in Formula 2-6, two Z are the same, two X₁ are the same, two X₂ are the same, two X₃ are the same, two Ar₁ are the same, and Z, X₁, X₂, X₃ and Ar₁ are selected from atoms or groups in the following table, respectively:
| Compound No. | Z | X₁ | X₂ | X₃ | Ar₁ |
|---|---|---|---|---|---|
| H6-1 | O | C-A1 | C-A1 | C-A1 | B1 |
| H6-2 | O | C-A1 | C-A1 | C-A1 | B5 |
| H6-3 | O | C-A1 | C-A1 | C-A1 | B6 |
| H6-4 | O | C-A1 | C-A1 | C-A1 | B11 |
| H6-5 | O | C-A1 | C-A1 | C-A1 | B18 |
| H6-6 | O | C-A1 | C-A1 | C-A1 | B20 |
| H6-7 | O | C-A1 | C-A1 | C-A1 | B21 |
| H6-8 | O | C-A1 | C-A1 | C-A1 | B22 |
| H6-9 | O | C-A1 | C-A1 | C-A1 | B23 |
| H6-10 | O | C-A1 | C-A1 | C-A1 | B24 |
| H6-11 | O | C-A1 | C-A1 | C-A1 | B25 |
| H6-12 | O | C-A1 | C-A1 | C-A1 | B31 |
| H6-13 | O | C-A1 | C-A1 | C-A1 | B32 |
| H6-14 | O | C-A1 | C-A1 | C-A1 | B68 |
| H6-15 | O | C-A1 | C-A1 | C-A1 | B69 |
| H6-16 | O | C-A1 | C-A1 | C-A1 | B70 |
| H6-17 | O | C-A1 | C-A1 | C-A1 | B71 |
| H6-18 | O | C-A1 | C-A1 | C-A1 | B81 |
| H6-19 | O | C-A1 | C-A1 | C-A1 | B90 |
| H6-20 | O | C-A1 | C-A1 | C-A1 | B91 |
| H6-21 | O | C-A1 | C-A1 | C-A1 | B92 |
| H6-22 | O | C-A1 | C-A1 | C-A1 | B93 |
| H6-23 | O | C-A1 | C-A1 | C-A1 | B107 |
| H6-24 | S | C-A1 | C-A1 | C-A1 | B1 |
| H6-25 | S | C-A1 | C-A1 | C-A1 | B5 |
| H6-26 | S | C-A1 | C-A1 | C-A1 | B6 |
| H6-27 | S | C-A1 | C-A1 | C-A1 | B11 |
| H6-28 | S | C-A1 | C-A1 | C-A1 | B18 |
| H6-29 | S | C-A1 | C-A1 | C-A1 | B20 |
| H6-30 | S | C-A1 | C-A1 | C-A1 | B21 |
| H6-31 | S | C-A1 | C-A1 | C-A1 | B22 |
| H6-32 | S | C-A1 | C-A1 | C-A1 | B23 |
| H6-33 | S | C-A1 | C-A1 | C-A1 | B24 |
| H6-34 | S | C-A1 | C-A1 | C-A1 | B25 |
| H6-35 | S | C-A1 | C-A1 | C-A1 | B31 |
| H6-36 | S | C-A1 | C-A1 | C-A1 | B32 |
| H6-37 | S | C-A1 | C-A1 | C-A1 | B68 |
| H6-38 | S | C-A1 | C-A1 | C-A1 | B69 |
| H6-39 | S | C-A1 | C-A1 | C-A1 | B70 |
| H6-40 | S | C-A1 | C-A1 | C-A1 | B71 |
| H6-41 | S | C-A1 | C-A1 | C-A1 | B81 |
| H6-42 | S | C-A1 | C-A1 | C-A1 | B90 |
| H6-43 | S | C-A1 | C-A1 | C-A1 | B91 |
| H6-44 | S | C-A1 | C-A1 | C-A1 | B92 |
| H6-45 | S | C-A1 | C-A1 | C-A1 | B93 |
| H6-46 | S | C-A1 | C-A1 | C-A1 | B107 |
| H6-47 | Se | C-A1 | C-A1 | C-A1 | B1 |
| H6-48 | Se | C-A1 | C-A1 | C-A1 | B5 |
| H6-49 | Se | C-A1 | C-A1 | C-A1 | B6 |
| H6-50 | Se | C-A1 | C-A1 | C-A1 | B11 |
| H6-51 | Se | C-A1 | C-A1 | C-A1 | B18 |
| H6-52 | Se | C-A1 | C-A1 | C-A1 | B20 |
| H6-53 | Se | C-A1 | C-A1 | C-A1 | B21 |
| H6-54 | Se | C-A1 | C-A1 | C-A1 | B22 |
| H6-55 | Se | C-A1 | C-A1 | C-A1 | B23 |
| H6-56 | Se | C-A1 | C-A1 | C-A1 | B24 |
| H6-57 | Se | C-A1 | C-A1 | C-A1 | B25 |
| H6-58 | Se | C-A1 | C-A1 | C-A1 | B31 |
| H6-59 | Se | C-A1 | C-A1 | C-A1 | B32 |
| H6-60 | Se | C-A1 | C-A1 | C-A1 | B68 |
| H6-61 | Se | C-A1 | C-A1 | C-A1 | B69 |
| H6-62 | Se | C-A1 | C-A1 | C-A1 | B70 |
| H6-63 | Se | C-A1 | C-A1 | C-A1 | B71 |
| H6-64 | Se | C-A1 | C-A1 | C-A1 | B81 |
| H6-65 | Se | C-A1 | C-A1 | C-A1 | B90 |
| H6-66 | Se | C-A1 | C-A1 | C-A1 | B91 |
| H6-67 | Se | C-A1 | C-A1 | C-A1 | B92 |
| H6-68 | Se | C-A1 | C-A1 | C-A1 | B93 |
| H6-69 | Se | C-A1 | C-A1 | C-A1 | B107 |
| H6-70 | O | N | C-A1 | C-A1 | B1 |
| H6-71 | O | N | C-A1 | C-A1 | B5 |
| H6-72 | O | N | C-A1 | C-A1 | B6 |
| H6-73 | O | N | C-A1 | C-A1 | B11 |
| H6-74 | O | N | C-A1 | C-A1 | B18 |
| H6-75 | O | N | C-A1 | C-A1 | B20 |
| H6-76 | O | N | C-A1 | C-A1 | B21 |
| H6-77 | O | N | C-A1 | C-A1 | B22 |
| H6-78 | O | N | C-A1 | C-A1 | B23 |
| H6-79 | O | N | C-A1 | C-A1 | B24 |
| H6-80 | O | N | C-A1 | C-A1 | B25 |
| H6-81 | O | N | C-A1 | C-A1 | B31 |
| H6-82 | O | N | C-A1 | C-A1 | B32 |
| H6-83 | O | N | C-A1 | C-A1 | B68 |
| H6-84 | O | N | C-A1 | C-A1 | B69 |
| H6-85 | O | N | C-A1 | C-A1 | B70 |
| H6-86 | O | N | C-A1 | C-A1 | B71 |
| H6-87 | O | N | C-A1 | C-A1 | B81 |
| H6-88 | O | N | C-A1 | C-A1 | B90 |
| H6-89 | O | N | C-A1 | C-A1 | B91 |
| H6-90 | O | N | C-A1 | C-A1 | B92 |
| H6-91 | O | N | C-A1 | C-A1 | B93 |
| H6-92 | O | N | C-A1 | C-A1 | B107 |
| H6-93 | O | C-A1 | N | C-A1 | B1 |
| H6-94 | O | C-A1 | N | C-A1 | B5 |
| H6-95 | O | C-A1 | N | C-A1 | B6 |
| H6-96 | O | C-A1 | N | C-A1 | B11 |
| H6-97 | O | C-A1 | N | C-A1 | B18 |
| H6-98 | O | C-A1 | N | C-A1 | B20 |
| H6-99 | O | C-A1 | N | C-A1 | B21 |
| H6-100 | O | C-A1 | N | C-A1 | B22 |
| H6-101 | O | C-A1 | N | C-A1 | B23 |
| H6-102 | O | C-A1 | N | C-A1 | B24 |
| H6-103 | O | C-A1 | N | C-A1 | B25 |
| H6-104 | O | C-A1 | N | C-A1 | B31 |
| H6-105 | O | C-A1 | N | C-A1 | B32 |
| H6-106 | O | C-A1 | N | C-A1 | B68 |
| H6-107 | O | C-A1 | N | C-A1 | B69 |
| H6-108 | O | C-A1 | N | C-A1 | B70 |
| H6-109 | O | C-A1 | N | C-A1 | B71 |
| H6-110 | O | C-A1 | N | C-A1 | B81 |
| H6-111 | O | C-A1 | N | C-A1 | B90 |
| H6-112 | O | C-A1 | N | C-A1 | B91 |
| H6-113 | O | C-A1 | N | C-A1 | B92 |
| H6-114 | O | C-A1 | N | C-A1 | B93 |
| H6-115 | O | C-A1 | N | C-A1 | B107 |
wherein Compound H7-1 to Compound H7-203 each have a structure represented by Formula 3-1: 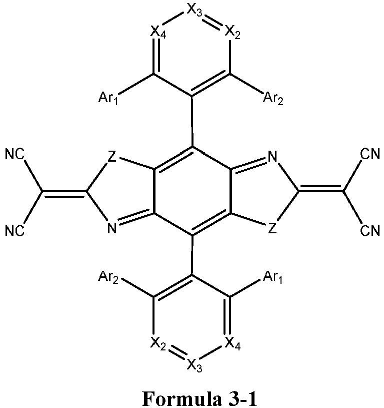
in Formula 3-1, two Z are the same, two X₂ are the same, two X₃ are the same, two X₄ are the same, two Ar₁ are the same, two Ar₂ are the same, and Z, X₂, X₃, X₄, Ar₁ and Ar₂ are selected from atoms or groups in the following table, respectively:
| Compound No. | Z | X₂ | X₃ | X₄ | Ar₁ | Ar₂ |
|---|---|---|---|---|---|---|
| H7-1 | O | C-A1 | C-A1 | C-A1 | B1 | B1 |
| H7-2 | O | C-A1 | C-A1 | C-A1 | B5 | B5 |
| H7-3 | O | C-A1 | C-A1 | C-A1 | B6 | B6 |
| H7-4 | O | C-A1 | C-A1 | C-A1 | B11 | B11 |
| H7-5 | O | C-A1 | C-A1 | C-A1 | B18 | B18 |
| H7-6 | O | C-A1 | C-A1 | C-A1 | B20 | B20 |
| H7-7 | O | C-A1 | C-A1 | C-A1 | B21 | B21 |
| H7-8 | O | C-A1 | C-A1 | C-A1 | B22 | B22 |
| H7-9 | O | C-A1 | C-A1 | C-A1 | B23 | B23 |
| H7-10 | O | C-A1 | C-A1 | C-A1 | B24 | B24 |
| H7-11 | O | C-A1 | C-A1 | C-A1 | B25 | B25 |
| H7-12 | O | C-A1 | C-A1 | C-A1 | B31 | B31 |
| H7-13 | O | C-A1 | C-A1 | C-A1 | B32 | B32 |
| H7-14 | O | C-A1 | C-A1 | C-A1 | B68 | B68 |
| H7-15 | O | C-A1 | C-A1 | C-A1 | B69 | B69 |
| H7-16 | O | C-A1 | C-A1 | C-A1 | B70 | B70 |
| H7-17 | O | C-A1 | C-A1 | C-A1 | B71 | B71 |
| H7-18 | O | C-A1 | C-A1 | C-A1 | B81 | B81 |
| H7-19 | O | C-A1 | C-A1 | C-A1 | B90 | B90 |
| H7-20 | O | C-A1 | C-A1 | C-A1 | B91 | B91 |
| H7-21 | O | C-A1 | C-A1 | C-A1 | B92 | B92 |
| H7-22 | O | C-A1 | C-A1 | C-A1 | B93 | B93 |
| H7-23 | O | C-A1 | C-A1 | C-A1 | B107 | B107 |
| H7-24 | O | C-A1 | C-A3 | C-A1 | B1 | B1 |
| H7-25 | O | C-A1 | C-A3 | C-A1 | B5 | B5 |
| H7-26 | O | C-A1 | C-A3 | C-A1 | B6 | B6 |
| H7-27 | O | C-A1 | C-A3 | C-A1 | B11 | B11 |
| H7-28 | O | C-A1 | C-A3 | C-A1 | B18 | B18 |
| H7-29 | O | C-A1 | C-A3 | C-A1 | B20 | B20 |
| H7-30 | O | C-A1 | C-A3 | C-A1 | B21 | B21 |
| H7-31 | O | C-A1 | C-A3 | C-A1 | B22 | B22 |
| H7-32 | O | C-A1 | C-A3 | C-A1 | B23 | B23 |
| H7-33 | O | C-A1 | C-A3 | C-A1 | B24 | B24 |
| H7-34 | O | C-A1 | C-A3 | C-A1 | B25 | B25 |
| H7-35 | O | C-A1 | C-A3 | C-A1 | B31 | B31 |
| H7-36 | O | C-A1 | C-A3 | C-A1 | B32 | B32 |
| H7-37 | O | C-A1 | C-A3 | C-A1 | B68 | B68 |
| H7-38 | O | C-A1 | C-A3 | C-A1 | B69 | B69 |
| H7-39 | O | C-A1 | C-A3 | C-A1 | B70 | B70 |
| H7-40 | O | C-A1 | C-A3 | C-A1 | B71 | B71 |
| H7-41 | O | C-A1 | C-A3 | C-A1 | B81 | B81 |
| H7-42 | O | C-A1 | C-A3 | C-A1 | B90 | B90 |
| H7-43 | O | C-A1 | C-A3 | C-A1 | B91 | B91 |
| H7-44 | O | C-A1 | C-A3 | C-A1 | B92 | B92 |
| H7-45 | O | C-A1 | C-A3 | C-A1 | B93 | B93 |
| H7-46 | O | C-A1 | C-A3 | C-A1 | B107 | B107 |
| H7-47 | O | C-A1 | C-A9 | C-A1 | B1 | B1 |
| H7-48 | O | C-A1 | C-A9 | C-A1 | B5 | B5 |
| H7-49 | O | C-A1 | C-A9 | C-A1 | B6 | B6 |
| H7-50 | O | C-A1 | C-A9 | C-A1 | B11 | B11 |
| H7-51 | O | C-A1 | C-A9 | C-A1 | B18 | B18 |
| H7-52 | O | C-A1 | C-A9 | C-A1 | B20 | B20 |
| H7-53 | O | C-A1 | C-A9 | C-A1 | B21 | B21 |
| H7-54 | O | C-A1 | C-A9 | C-A1 | B22 | B22 |
| H7-55 | O | C-A1 | C-A9 | C-A1 | B23 | B23 |
| H7-56 | O | C-A1 | C-A9 | C-A1 | B24 | B24 |
| H7-57 | O | C-A1 | C-A9 | C-A1 | B25 | B25 |
| H7-58 | O | C-A1 | C-A9 | C-A1 | B31 | B31 |
| H7-59 | O | C-A1 | C-A9 | C-A1 | B32 | B32 |
| H7-60 | O | C-A1 | C-A9 | C-A1 | B68 | B68 |
| H7-61 | O | C-A1 | C-A9 | C-A1 | B69 | B69 |
| H7-62 | O | C-A1 | C-A9 | C-A1 | B70 | B70 |
| H7-63 | O | C-A1 | C-A9 | C-A1 | B71 | B71 |
| H7-64 | O | C-A1 | C-A9 | C-A1 | B81 | B81 |
| H7-65 | O | C-A1 | C-A9 | C-A1 | B90 | B90 |
| H7-66 | O | C-A1 | C-A9 | C-A1 | B91 | B91 |
| H7-67 | O | C-A1 | C-A9 | C-A1 | B92 | B92 |
| H7-68 | O | C-A1 | C-A9 | C-A1 | B93 | B93 |
| H7-69 | O | C-A1 | C-A9 | C-A1 | B107 | B107 |
| H7-70 | S | C-A1 | C-A1 | C-A1 | B1 | B1 |
| H7-71 | S | C-A1 | C-A1 | C-A1 | B5 | B5 |
| H7-72 | S | C-A1 | C-A1 | C-A1 | B6 | B6 |
| H7-73 | S | C-A1 | C-A1 | C-A1 | B11 | B11 |
| H7-74 | S | C-A1 | C-A1 | C-A1 | B18 | B18 |
| H7-75 | S | C-A1 | C-A1 | C-A1 | B20 | B20 |
| H7-76 | S | C-A1 | C-A1 | C-A1 | B21 | B21 |
| H7-77 | S | C-A1 | C-A1 | C-A1 | B22 | B22 |
| H7-78 | S | C-A1 | C-A1 | C-A1 | B23 | B23 |
| H7-79 | S | C-A1 | C-A1 | C-A1 | B24 | B24 |
| H7-80 | S | C-A1 | C-A1 | C-A1 | B25 | B25 |
| H7-81 | S | C-A1 | C-A1 | C-A1 | B31 | B31 |
| H7-82 | S | C-A1 | C-A1 | C-A1 | B32 | B32 |
| H7-83 | S | C-A1 | C-A1 | C-A1 | B68 | B68 |
| H7-84 | S | C-A1 | C-A1 | C-A1 | B69 | B69 |
| H7-85 | S | C-A1 | C-A1 | C-A1 | B70 | B70 |
| H7-86 | S | C-A1 | C-A1 | C-A1 | B71 | B71 |
| H7-87 | S | C-A1 | C-A1 | C-A1 | B81 | B81 |
| H7-88 | S | C-A1 | C-A1 | C-A1 | B90 | B90 |
| H7-89 | S | C-A1 | C-A1 | C-A1 | B91 | B91 |
| H7-90 | S | C-A1 | C-A1 | C-A1 | B92 | B92 |
| H7-91 | S | C-A1 | C-A1 | C-A1 | B93 | B93 |
| H7-92 | S | C-A1 | C-A1 | C-A1 | B107 | B107 |
| H7-93 | Se | C-A1 | C-A1 | C-A1 | B1 | B1 |
| H7-94 | Se | C-A1 | C-A1 | C-A1 | B5 | B5 |
| H7-95 | Se | C-A1 | C-A1 | C-A1 | B6 | B6 |
| H7-96 | Se | C-A1 | C-A1 | C-A1 | B11 | B11 |
| H7-97 | Se | C-A1 | C-A1 | C-A1 | B18 | B18 |
| H7-98 | Se | C-A1 | C-A1 | C-A1 | B20 | B20 |
| H7-99 | Se | C-A1 | C-A1 | C-A1 | B21 | B21 |
| H7-100 | Se | C-A1 | C-A1 | C-A1 | B22 | B22 |
| H7-101 | Se | C-A1 | C-A1 | C-A1 | B23 | B23 |
| H7-102 | Se | C-A1 | C-A1 | C-A1 | B24 | B24 |
| H7-103 | Se | C-A1 | C-A1 | C-A1 | B25 | B25 |
| H7-104 | Se | C-A1 | C-A1 | C-A1 | B31 | B31 |
| H7-105 | Se | C-A1 | C-A1 | C-A1 | B32 | B32 |
| H7-106 | Se | C-A1 | C-A1 | C-A1 | B68 | B68 |
| H7-107 | Se | C-A1 | C-A1 | C-A1 | B69 | B69 |
| H7-108 | Se | C-A1 | C-A1 | C-A1 | B70 | B70 |
| H7-109 | Se | C-A1 | C-A1 | C-A1 | B71 | B71 |
| H7-110 | Se | C-A1 | C-A1 | C-A1 | B81 | B81 |
| H7-111 | Se | C-A1 | C-A1 | C-A1 | B90 | B90 |
| H7-112 | Se | C-A1 | C-A1 | C-A1 | B91 | B91 |
| H7-113 | Se | C-A1 | C-A1 | C-A1 | B92 | B92 |
| H7-114 | Se | C-A1 | C-A1 | C-A1 | B93 | B93 |
| H7-115 | Se | C-A1 | C-A1 | C-A1 | B107 | B107 |
| H7-116 | O | C-A1 | C-A1 | C-A1 | B1 | B5 |
| H7-117 | O | C-A1 | C-A1 | C-A1 | B1 | B6 |
| H7-118 | O | C-A1 | C-A1 | C-A1 | B1 | B11 |
| H7-119 | O | C-A1 | C-A1 | C-A1 | B1 | B18 |
| H7-120 | O | C-A1 | C-A1 | C-A1 | B1 | B20 |
| H7-121 | O | C-A1 | C-A1 | C-A1 | B1 | B21 |
| H7-122 | O | C-A1 | C-A1 | C-A1 | B1 | B22 |
| H7-123 | O | C-A1 | C-A1 | C-A1 | B1 | B23 |
| H7-124 | O | C-A1 | C-A1 | C-A1 | B1 | B24 |
| H7-125 | O | C-A1 | C-A1 | C-A1 | B1 | B25 |
| H7-126 | O | C-A1 | C-A1 | C-A1 | B1 | B31 |
| H7-127 | O | C-A1 | C-A1 | C-A1 | B1 | B32 |
| H7-128 | O | C-A1 | C-A1 | C-A1 | B1 | B68 |
| H7-129 | O | C-A1 | C-A1 | C-A1 | B1 | B69 |
| H7-130 | O | C-A1 | C-A1 | C-A1 | B1 | B70 |
| H7-131 | O | C-A1 | C-A1 | C-A1 | B1 | B71 |
| H7-132 | O | C-A1 | C-A1 | C-A1 | B1 | B81 |
| H7-133 | O | C-A1 | C-A1 | C-A1 | B1 | B90 |
| H7-134 | O | C-A1 | C-A1 | C-A1 | B1 | B91 |
| H7-135 | O | C-A1 | C-A1 | C-A1 | B1 | B92 |
| H7-136 | O | C-A1 | C-A1 | C-A1 | B1 | B93 |
| H7-137 | O | C-A1 | C-A1 | C-A1 | B1 | B107 |
| H7-138 | O | C-A1 | C-A1 | C-A1 | B20 | B1 |
| H7-139 | O | C-A1 | C-A1 | C-A1 | B20 | B5 |
| H7-140 | O | C-A1 | C-A1 | C-A1 | B20 | B6 |
| H7-141 | O | C-A1 | C-A1 | C-A1 | B20 | B11 |
| H7-142 | O | C-A1 | C-A1 | C-A1 | B20 | B18 |
| H7-143 | O | C-A1 | C-A1 | C-A1 | B20 | B21 |
| H7-144 | O | C-A1 | C-A1 | C-A1 | B20 | B22 |
| H7-145 | O | C-A1 | C-A1 | C-A1 | B20 | B23 |
| H7-146 | O | C-A1 | C-A1 | C-A1 | B20 | B24 |
| H7-147 | O | C-A1 | C-A1 | C-A1 | B20 | B25 |
| H7-148 | O | C-A1 | C-A1 | C-A1 | B20 | B31 |
| H7-149 | O | C-A1 | C-A1 | C-A1 | B20 | B32 |
| H7-150 | O | C-A1 | C-A1 | C-A1 | B20 | B68 |
| H7-151 | O | C-A1 | C-A1 | C-A1 | B20 | B69 |
| H7-152 | O | C-A1 | C-A1 | C-A1 | B20 | B70 |
| H7-153 | O | C-A1 | C-A1 | C-A1 | B20 | B71 |
| H7-154 | O | C-A1 | C-A1 | C-A1 | B20 | B81 |
| H7-155 | O | C-A1 | C-A1 | C-A1 | B20 | B90 |
| H7-156 | O | C-A1 | C-A1 | C-A1 | B20 | B91 |
| H7-157 | O | C-A1 | C-A1 | C-A1 | B20 | B92 |
| H7-158 | O | C-A1 | C-A1 | C-A1 | B20 | B93 |
| H7-159 | O | C-A1 | C-A1 | C-A1 | B20 | B107 |
| H7-160 | O | C-A1 | C-A1 | C-A1 | B68 | B1 |
| H7-161 | O | C-A1 | C-A1 | C-A1 | B68 | B5 |
| H7-162 | O | C-A1 | C-A1 | C-A1 | B68 | B6 |
| H7-163 | O | C-A1 | C-A1 | C-A1 | B68 | B11 |
| H7-164 | O | C-A1 | C-A1 | C-A1 | B68 | B18 |
| H7-165 | O | C-A1 | C-A1 | C-A1 | B68 | B20 |
| H7-166 | O | C-A1 | C-A1 | C-A1 | B68 | B21 |
| H7-167 | O | C-A1 | C-A1 | C-A1 | B68 | B22 |
| H7-168 | O | C-A1 | C-A1 | C-A1 | B68 | B23 |
| H7-169 | O | C-A1 | C-A1 | C-A1 | B68 | B24 |
| H7-170 | O | C-A1 | C-A1 | C-A1 | B68 | B25 |
| H7-171 | O | C-A1 | C-A1 | C-A1 | B68 | B31 |
| H7-172 | O | C-A1 | C-A1 | C-A1 | B68 | B32 |
| H7-173 | O | C-A1 | C-A1 | C-A1 | B68 | B69 |
| H7-174 | O | C-A1 | C-A1 | C-A1 | B68 | B70 |
| H7-175 | O | C-A1 | C-A1 | C-A1 | B68 | B71 |
| H7-176 | O | C-A1 | C-A1 | C-A1 | B68 | B81 |
| H7-177 | O | C-A1 | C-A1 | C-A1 | B68 | B90 |
| H7-178 | O | C-A1 | C-A1 | C-A1 | B68 | B91 |
| H7-179 | O | C-A1 | C-A1 | C-A1 | B68 | B92 |
| H7-180 | O | C-A1 | C-A1 | C-A1 | B68 | B93 |
| H7-181 | O | C-A1 | C-A1 | C-A1 | B68 | B107 |
| H7-182 | O | C-A1 | C-A1 | C-A1 | B70 | B1 |
| H7-183 | O | C-A1 | C-A1 | C-A1 | B70 | B5 |
| H7-184 | O | C-A1 | C-A1 | C-A1 | B70 | B6 |
| H7-185 | O | C-A1 | C-A1 | C-A1 | B70 | B11 |
| H7-186 | O | C-A1 | C-A1 | C-A1 | B70 | B18 |
| H7-187 | O | C-A1 | C-A1 | C-A1 | B70 | B20 |
| H7-188 | O | C-A1 | C-A1 | C-A1 | B70 | B21 |
| H7-189 | O | C-A1 | C-A1 | C-A1 | B70 | B22 |
| H7-190 | O | C-A1 | C-A1 | C-A1 | B70 | B23 |
| H7-191 | O | C-A1 | C-A1 | C-A1 | B70 | B24 |
| H7-192 | O | C-A1 | C-A1 | C-A1 | B70 | B25 |
| H7-193 | O | C-A1 | C-A1 | C-A1 | B70 | B31 |
| H7-194 | O | C-A1 | C-A1 | C-A1 | B70 | B32 |
| H7-195 | O | C-A1 | C-A1 | C-A1 | B70 | B68 |
| H7-196 | O | C-A1 | C-A1 | C-A1 | B70 | B69 |
| H7-197 | O | C-A1 | C-A1 | C-A1 | B70 | B71 |
| H7-198 | O | C-A1 | C-A1 | C-A1 | B70 | B81 |
| H7-199 | O | C-A1 | C-A1 | C-A1 | B70 | B90 |
| H7-200 | O | C-A1 | C-A1 | C-A1 | B70 | B91 |
| H7-201 | O | C-A1 | C-A1 | C-A1 | B70 | B92 |
| H7-202 | O | C-A1 | C-A1 | C-A1 | B70 | B93 |
| H7-203 | O | C-A1 | C-A1 | C-A1 | B70 | B107 |
wherein Compound H8-7 to Compound H8-54, Compound H8-61 to Compound H8-108, Compound H8-115 to Compound H8-162, Compound H8-169 to Compound H8-216, Compound H8-223 to Compound H8-270, Compound H8-277 to Compound H8-324, Compound H8-331 to Compound H8-378, Compound H8-385 to Compound H8-432, Compound H8-439 to Compound H8-486, Compound H8-493 to Compound H8-540, Compound H8-547 to Compound H8-594, Compound H8-601 to Compound H8-648, Compound H8-655 to Compound H8-702, Compound H8-709 to Compound H8-756, Compound H8-763 to Compound H8-810, Compound H8-817 to Compound H8-864, Compound H8-871 to Compound H8-918, Compound H8-925 to Compound H8-972, Compound H8-979 to Compound H8-1026, Compound H8-1033 to Compound H8-1080, Compound H8-1087 to Compound H8-1134, Compound H8-1141 to Compound H8-1188, Compound H8-1195 to Compound H8-1242, Compound H8-1249 to Compound H8-1296, Compound H8-1303 to Compound H8-1350, Compound H8-1357 to Compound H8-1404, Compound H8-1411 to Compound H8-1458, Compound H8-1465 to Compound H8-1512 each have a structure represented by Formula 3-2: 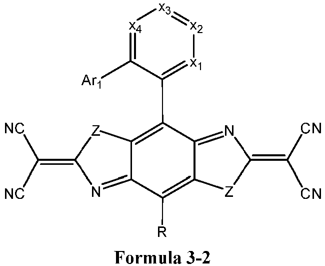
in Formula 3-2, two Z are the same and Z, X₁, X₂, X₃, X₄, Ar₁ and R are selected from atoms or groups in the following table, respectively:
| Compound No. | Z | X₁ | X₂ | X₃ | X₄ | Ar₁ | R |
|---|---|---|---|---|---|---|---|
| H8-7 | O | C-A1 | C-A1 | C-A1 | C-A1 | B20 | B1 |
| H8-8 | O | C-A1 | C-A1 | C-A1 | C-A1 | B20 | B6 |
| H8-9 | O | C-A1 | C-A1 | C-A1 | C-A1 | B20 | B20 |
| H8-10 | O | C-A1 | C-A1 | C-A1 | C-A1 | B20 | B21 |
| H8-11 | O | C-A1 | C-A1 | C-A1 | C-A1 | B20 | B22 |
| H8-12 | O | C-A1 | C-A1 | C-A1 | C-A1 | B20 | B32 |
| H8-13 | O | C-A1 | C-A1 | C-A1 | C-A1 | B20 | B33 |
| H8-14 | O | C-A1 | C-A1 | C-A1 | C-A1 | B20 | B34 |
| H8-15 | O | C-A1 | C-A1 | C-A1 | C-A1 | B20 | B68 |
| H8-16 | O | C-A1 | C-A1 | C-A1 | C-A1 | B20 | B69 |
| H8-17 | O | C-A1 | C-A1 | C-A1 | C-A1 | B20 | B70 |
| H8-18 | O | C-A1 | C-A1 | C-A1 | C-A1 | B20 | B71 |
| H8-19 | O | C-A1 | C-A1 | C-A1 | C-A1 | B20 | B81 |
| H8-20 | O | C-A1 | C-A1 | C-A1 | C-A1 | B20 | B90 |
| H8-21 | O | C-A1 | C-A1 | C-A1 | C-A1 | B20 | B91 |
| H8-22 | O | C-A1 | C-A1 | C-A1 | C-A1 | B20 | B92 |
| H8-23 | O | C-A1 | C-A1 | C-A1 | C-A1 | B20 | B93 |
| H8-24 | O | C-A1 | C-A1 | C-A1 | C-A1 | B20 | B94 |
| H8-25 | O | C-A1 | C-A1 | C-A1 | C-A1 | B20 | B95 |
| H8-26 | O | C-A1 | C-A1 | C-A1 | C-A1 | B20 | B96 |
| H8-27 | O | C-A1 | C-A1 | C-A1 | C-A1 | B20 | B97 |
| H8-28 | O | C-A1 | C-A1 | C-A1 | C-A1 | B20 | B106 |
| H8-29 | O | C-A1 | C-A1 | C-A1 | C-A1 | B20 | B107 |
| H8-30 | O | C-A1 | C-A1 | C-A1 | C-A1 | B20 | B114 |
| H8-31 | O | C-A1 | C-A1 | C-A1 | C-A1 | B20 | B115 |
| H8-32 | O | C-A1 | C-A1 | C-A1 | C-A1 | B20 | B116 |
| H8-33 | O | C-A1 | C-A1 | C-A1 | C-A1 | B20 | B117 |
| H8-34 | O | C-A1 | C-A1 | C-A1 | C-A1 | B20 | B118 |
| H8-35 | O | C-A1 | C-A1 | C-A1 | C-A1 | B20 | B119 |
| H8-36 | O | C-A1 | C-A1 | C-A1 | C-A1 | B20 | B120 |
| H8-37 | O | C-A1 | C-A1 | C-A1 | C-A1 | B20 | B121 |
| H8-38 | O | C-A1 | C-A1 | C-A1 | C-A1 | B20 | B122 |
| H8-39 | O | C-A1 | C-A1 | C-A1 | C-A1 | B20 | B125 |
| H8-40 | O | C-A1 | C-A1 | C-A1 | C-A1 | B20 | B126 |
| H8-41 | O | C-A1 | C-A1 | C-A1 | C-A1 | B20 | B127 |
| H8-42 | O | C-A1 | C-A1 | C-A1 | C-A1 | B20 | B128 |
| H8-43 | O | C-A1 | C-A1 | C-A1 | C-A1 | B20 | B129 |
| H8-44 | O | C-A1 | C-A1 | C-A1 | C-A1 | B20 | B130 |
| H8-45 | O | C-A1 | C-A1 | C-A1 | C-A1 | B20 | B131 |
| H8-46 | O | C-A1 | C-A1 | C-A1 | C-A1 | B20 | B132 |
| H8-47 | O | C-A1 | C-A1 | C-A1 | C-A1 | B20 | B133 |
| H8-48 | O | C-A1 | C-A1 | C-A1 | C-A1 | B20 | B134 |
| H8-49 | O | C-A1 | C-A1 | C-A1 | C-A1 | B20 | B135 |
| H8-50 | O | C-A1 | C-A1 | C-A1 | C-A1 | B20 | B136 |
| H8-51 | O | C-A1 | C-A1 | C-A1 | C-A1 | B20 | B137 |
| H8-52 | O | C-A1 | C-A1 | C-A1 | C-A1 | B20 | B138 |
| H8-53 | O | C-A1 | C-A1 | C-A1 | C-A1 | B20 | B139 |
| H8-54 | O | C-A1 | C-A1 | C-A1 | C-A1 | B20 | B140 |
| H8-61 | O | C-A1 | C-A2 | C-A1 | C-A2 | B20 | B1 |
| H8-62 | O | C-A1 | C-A2 | C-A1 | C-A2 | B20 | B6 |
| H8-63 | O | C-A1 | C-A2 | C-A1 | C-A2 | B20 | B20 |
| H8-64 | O | C-A1 | C-A2 | C-A1 | C-A2 | B20 | B21 |
| H8-65 | O | C-A1 | C-A2 | C-A1 | C-A2 | B20 | B22 |
| H8-66 | O | C-A1 | C-A2 | C-A1 | C-A2 | B20 | B32 |
| H8-67 | O | C-A1 | C-A2 | C-A1 | C-A2 | B20 | B33 |
| H8-68 | O | C-A1 | C-A2 | C-A1 | C-A2 | B20 | B34 |
| H8-69 | O | C-A1 | C-A2 | C-A1 | C-A2 | B20 | B68 |
| H8-70 | O | C-A1 | C-A2 | C-A1 | C-A2 | B20 | B69 |
| H8-71 | O | C-A1 | C-A2 | C-A1 | C-A2 | B20 | B70 |
| H8-72 | O | C-A1 | C-A2 | C-A1 | C-A2 | B20 | B71 |
| H8-73 | O | C-A1 | C-A2 | C-A1 | C-A2 | B20 | B81 |
| H8-74 | O | C-A1 | C-A2 | C-A1 | C-A2 | B20 | B90 |
| H8-75 | O | C-A1 | C-A2 | C-A1 | C-A2 | B20 | B91 |
| H8-76 | O | C-A1 | C-A2 | C-A1 | C-A2 | B20 | B92 |
| H8-77 | O | C-A1 | C-A2 | C-A1 | C-A2 | B20 | B93 |
| H8-78 | O | C-A1 | C-A2 | C-A1 | C-A2 | B20 | B94 |
| H8-79 | O | C-A1 | C-A2 | C-A1 | C-A2 | B20 | B95 |
| H8-80 | O | C-A1 | C-A2 | C-A1 | C-A2 | B20 | B96 |
| H8-81 | O | C-A1 | C-A2 | C-A1 | C-A2 | B20 | B97 |
| H8-82 | O | C-A1 | C-A2 | C-A1 | C-A2 | B20 | B106 |
| H8-83 | O | C-A1 | C-A2 | C-A1 | C-A2 | B20 | B107 |
| H8-84 | O | C-A1 | C-A2 | C-A1 | C-A2 | B20 | B114 |
| H8-85 | O | C-A1 | C-A2 | C-A1 | C-A2 | B20 | B115 |
| H8-86 | O | C-A1 | C-A2 | C-A1 | C-A2 | B20 | B116 |
| H8-87 | O | C-A1 | C-A2 | C-A1 | C-A2 | B20 | B117 |
| H8-88 | O | C-A1 | C-A2 | C-A1 | C-A2 | B20 | B118 |
| H8-89 | O | C-A1 | C-A2 | C-A1 | C-A2 | B20 | B119 |
| H8-90 | O | C-A1 | C-A2 | C-A1 | C-A2 | B20 | B120 |
| H8-91 | O | C-A1 | C-A2 | C-A1 | C-A2 | B20 | B121 |
| H8-92 | O | C-A1 | C-A2 | C-A1 | C-A2 | B20 | B122 |
| H8-93 | O | C-A1 | C-A2 | C-A1 | C-A2 | B20 | B125 |
| H8-94 | O | C-A1 | C-A2 | C-A1 | C-A2 | B20 | B126 |
| H8-95 | O | C-A1 | C-A2 | C-A1 | C-A2 | B20 | B127 |
| H8-96 | O | C-A1 | C-A2 | C-A1 | C-A2 | B20 | B128 |
| H8-97 | O | C-A1 | C-A2 | C-A1 | C-A2 | B20 | B129 |
| H8-98 | O | C-A1 | C-A2 | C-A1 | C-A2 | B20 | B130 |
| H8-99 | O | C-A1 | C-A2 | C-A1 | C-A2 | B20 | B131 |
| H8-100 | O | C-A1 | C-A2 | C-A1 | C-A2 | B20 | B132 |
| H8-101 | O | C-A1 | C-A2 | C-A1 | C-A2 | B20 | B133 |
| H8-102 | O | C-A1 | C-A2 | C-A1 | C-A2 | B20 | B134 |
| H8-103 | O | C-A1 | C-A2 | C-A1 | C-A2 | B20 | B135 |
| H8-104 | O | C-A1 | C-A2 | C-A1 | C-A2 | B20 | B136 |
| H8-105 | O | C-A1 | C-A2 | C-A1 | C-A2 | B20 | B137 |
| H8-106 | O | C-A1 | C-A2 | C-A1 | C-A2 | B20 | B138 |
| H8-107 | O | C-A1 | C-A2 | C-A1 | C-A2 | B20 | B139 |
| H8-108 | O | C-A1 | C-A2 | C-A1 | C-A2 | B20 | B140 |
| H8-115 | O | C-A1 | C-A3 | C-A1 | C-A3 | B20 | B1 |
| H8-116 | O | C-A1 | C-A3 | C-A1 | C-A3 | B20 | B6 |
| H8-117 | O | C-A1 | C-A3 | C-A1 | C-A3 | B20 | B20 |
| H8-118 | O | C-A1 | C-A3 | C-A1 | C-A3 | B20 | B21 |
| H8-119 | O | C-A1 | C-A3 | C-A1 | C-A3 | B20 | B22 |
| H8-120 | O | C-A1 | C-A3 | C-A1 | C-A3 | B20 | B32 |
| H8-121 | O | C-A1 | C-A3 | C-A1 | C-A3 | B20 | B33 |
| H8-122 | O | C-A1 | C-A3 | C-A1 | C-A3 | B20 | B34 |
| H8-123 | O | C-A1 | C-A3 | C-A1 | C-A3 | B20 | B68 |
| H8-124 | O | C-A1 | C-A3 | C-A1 | C-A3 | B20 | B69 |
| H8-125 | O | C-A1 | C-A3 | C-A1 | C-A3 | B20 | B70 |
| H8-126 | O | C-A1 | C-A3 | C-A1 | C-A3 | B20 | B71 |
| H8-127 | O | C-A1 | C-A3 | C-A1 | C-A3 | B20 | B81 |
| H8-128 | O | C-A1 | C-A3 | C-A1 | C-A3 | B20 | B90 |
| H8-129 | O | C-A1 | C-A3 | C-A1 | C-A3 | B20 | B91 |
| H8-130 | O | C-A1 | C-A3 | C-A1 | C-A3 | B20 | B92 |
| H8-131 | O | C-A1 | C-A3 | C-A1 | C-A3 | B20 | B93 |
| H8-132 | O | C-A1 | C-A3 | C-A1 | C-A3 | B20 | B94 |
| H8-133 | O | C-A1 | C-A3 | C-A1 | C-A3 | B20 | B95 |
| H8-134 | O | C-A1 | C-A3 | C-A1 | C-A3 | B20 | B96 |
| H8-135 | O | C-A1 | C-A3 | C-A1 | C-A3 | B20 | B97 |
| H8-136 | O | C-A1 | C-A3 | C-A1 | C-A3 | B20 | B106 |
| H8-137 | O | C-A1 | C-A3 | C-A1 | C-A3 | B20 | B107 |
| H8-138 | O | C-A1 | C-A3 | C-A1 | C-A3 | B20 | B114 |
| H8-139 | O | C-A1 | C-A3 | C-A1 | C-A3 | B20 | B115 |
| H8-140 | O | C-A1 | C-A3 | C-A1 | C-A3 | B20 | B116 |
| H8-141 | O | C-A1 | C-A3 | C-A1 | C-A3 | B20 | B117 |
| H8-142 | O | C-A1 | C-A3 | C-A1 | C-A3 | B20 | B118 |
| H8-143 | O | C-A1 | C-A3 | C-A1 | C-A3 | B20 | B119 |
| H8-144 | O | C-A1 | C-A3 | C-A1 | C-A3 | B20 | B120 |
| H8-145 | O | C-A1 | C-A3 | C-A1 | C-A3 | B20 | B121 |
| H8-146 | O | C-A1 | C-A3 | C-A1 | C-A3 | B20 | B122 |
| H8-147 | O | C-A1 | C-A3 | C-A1 | C-A3 | B20 | B125 |
| H8-148 | O | C-A1 | C-A3 | C-A1 | C-A3 | B20 | B126 |
| H8-149 | O | C-A1 | C-A3 | C-A1 | C-A3 | B20 | B127 |
| H8-150 | O | C-A1 | C-A3 | C-A1 | C-A3 | B20 | B128 |
| H8-151 | O | C-A1 | C-A3 | C-A1 | C-A3 | B20 | B129 |
| H8-152 | O | C-A1 | C-A3 | C-A1 | C-A3 | B20 | B130 |
| H8-153 | O | C-A1 | C-A3 | C-A1 | C-A3 | B20 | B131 |
| H8-154 | O | C-A1 | C-A3 | C-A1 | C-A3 | B20 | B132 |
| H8-155 | O | C-A1 | C-A3 | C-A1 | C-A3 | B20 | B133 |
| H8-156 | O | C-A1 | C-A3 | C-A1 | C-A3 | B20 | B134 |
| H8-157 | O | C-A1 | C-A3 | C-A1 | C-A3 | B20 | B135 |
| H8-158 | O | C-A1 | C-A3 | C-A1 | C-A3 | B20 | B136 |
| H8-159 | O | C-A1 | C-A3 | C-A1 | C-A3 | B20 | B137 |
| H8-160 | O | C-A1 | C-A3 | C-A1 | C-A3 | B20 | B138 |
| H8-161 | O | C-A1 | C-A3 | C-A1 | C-A3 | B20 | B139 |
| H8-162 | O | C-A1 | C-A3 | C-A1 | C-A3 | B20 | B140 |
| H8-169 | O | C-A3 | C-A1 | C-A3 | C-A1 | B20 | B1 |
| H8-170 | O | C-A3 | C-A1 | C-A3 | C-A1 | B20 | B6 |
| H8-171 | O | C-A3 | C-A1 | C-A3 | C-A1 | B20 | B20 |
| H8-172 | O | C-A3 | C-A1 | C-A3 | C-A1 | B20 | B21 |
| H8-173 | O | C-A3 | C-A1 | C-A3 | C-A1 | B20 | B22 |
| H8-174 | O | C-A3 | C-A1 | C-A3 | C-A1 | B20 | B32 |
| H8-175 | O | C-A3 | C-A1 | C-A3 | C-A1 | B20 | B33 |
| H8-176 | O | C-A3 | C-A1 | C-A3 | C-A1 | B20 | B34 |
| H8-177 | O | C-A3 | C-A1 | C-A3 | C-A1 | B20 | B68 |
| H8-178 | O | C-A3 | C-A1 | C-A3 | C-A1 | B20 | B69 |
| H8-179 | O | C-A3 | C-A1 | C-A3 | C-A1 | B20 | B70 |
| H8-180 | O | C-A3 | C-A1 | C-A3 | C-A1 | B20 | B71 |
| H8-181 | O | C-A3 | C-A1 | C-A3 | C-A1 | B20 | B81 |
| H8-182 | O | C-A3 | C-A1 | C-A3 | C-A1 | B20 | B90 |
| H8-183 | O | C-A3 | C-A1 | C-A3 | C-A1 | B20 | B91 |
| H8-184 | O | C-A3 | C-A1 | C-A3 | C-A1 | B20 | B92 |
| H8-185 | O | C-A3 | C-A1 | C-A3 | C-A1 | B20 | B93 |
| H8-186 | O | C-A3 | C-A1 | C-A3 | C-A1 | B20 | B94 |
| H8-187 | O | C-A3 | C-A1 | C-A3 | C-A1 | B20 | B95 |
| H8-188 | O | C-A3 | C-A1 | C-A3 | C-A1 | B20 | B96 |
| H8-189 | O | C-A3 | C-A1 | C-A3 | C-A1 | B20 | B97 |
| H8-190 | O | C-A3 | C-A1 | C-A3 | C-A1 | B20 | B106 |
| H8-191 | O | C-A3 | C-A1 | C-A3 | C-A1 | B20 | B107 |
| H8-192 | O | C-A3 | C-A1 | C-A3 | C-A1 | B20 | B114 |
| H8-193 | O | C-A3 | C-A1 | C-A3 | C-A1 | B20 | B115 |
| H8-194 | O | C-A3 | C-A1 | C-A3 | C-A1 | B20 | B116 |
| H8-195 | O | C-A3 | C-A1 | C-A3 | C-A1 | B20 | B117 |
| H8-196 | O | C-A3 | C-A1 | C-A3 | C-A1 | B20 | B118 |
| H8-197 | O | C-A3 | C-A1 | C-A3 | C-A1 | B20 | B119 |
| H8-198 | O | C-A3 | C-A1 | C-A3 | C-A1 | B20 | B120 |
| H8-199 | O | C-A3 | C-A1 | C-A3 | C-A1 | B20 | B121 |
| H8-200 | O | C-A3 | C-A1 | C-A3 | C-A1 | B20 | B122 |
| H8-201 | O | C-A3 | C-A1 | C-A3 | C-A1 | B20 | B125 |
| H8-202 | O | C-A3 | C-A1 | C-A3 | C-A1 | B20 | B126 |
| H8-203 | O | C-A3 | C-A1 | C-A3 | C-A1 | B20 | B127 |
| H8-204 | O | C-A3 | C-A1 | C-A3 | C-A1 | B20 | B128 |
| H8-205 | O | C-A3 | C-A1 | C-A3 | C-A1 | B20 | B129 |
| H8-206 | O | C-A3 | C-A1 | C-A3 | C-A1 | B20 | B130 |
| H8-207 | O | C-A3 | C-A1 | C-A3 | C-A1 | B20 | B131 |
| H8-208 | O | C-A3 | C-A1 | C-A3 | C-A1 | B20 | B132 |
| H8-209 | O | C-A3 | C-A1 | C-A3 | C-A1 | B20 | B133 |
| H8-210 | O | C-A3 | C-A1 | C-A3 | C-A1 | B20 | B134 |
| H8-211 | O | C-A3 | C-A1 | C-A3 | C-A1 | B20 | B135 |
| H8-212 | O | C-A3 | C-A1 | C-A3 | C-A1 | B20 | B136 |
| H8-213 | O | C-A3 | C-A1 | C-A3 | C-A1 | B20 | B137 |
| H8-214 | O | C-A3 | C-A1 | C-A3 | C-A1 | B20 | B138 |
| H8-215 | O | C-A3 | C-A1 | C-A3 | C-A1 | B20 | B139 |
| H8-216 | O | C-A3 | C-A1 | C-A3 | C-A1 | B20 | B140 |
| H8-223 | O | C-A1 | C-A1 | C-A3 | C-A1 | B20 | B1 |
| H8-224 | O | C-A1 | C-A1 | C-A3 | C-A1 | B20 | B6 |
| H8-225 | O | C-A1 | C-A1 | C-A3 | C-A1 | B20 | B20 |
| H8-226 | O | C-A1 | C-A1 | C-A3 | C-A1 | B20 | B21 |
| H8-227 | O | C-A1 | C-A1 | C-A3 | C-A1 | B20 | B22 |
| H8-228 | O | C-A1 | C-A1 | C-A3 | C-A1 | B20 | B32 |
| H8-229 | O | C-A1 | C-A1 | C-A3 | C-A1 | B20 | B33 |
| H8-230 | O | C-A1 | C-A1 | C-A3 | C-A1 | B20 | B34 |
| H8-231 | O | C-A1 | C-A1 | C-A3 | C-A1 | B20 | B68 |
| H8-232 | O | C-A1 | C-A1 | C-A3 | C-A1 | B20 | B69 |
| H8-233 | O | C-A1 | C-A1 | C-A3 | C-A1 | B20 | B70 |
| H8-234 | O | C-A1 | C-A1 | C-A3 | C-A1 | B20 | B71 |
| H8-235 | O | C-A1 | C-A1 | C-A3 | C-A1 | B20 | B81 |
| H8-236 | O | C-A1 | C-A1 | C-A3 | C-A1 | B20 | B90 |
| H8-237 | O | C-A1 | C-A1 | C-A3 | C-A1 | B20 | B91 |
| H8-238 | O | C-A1 | C-A1 | C-A3 | C-A1 | B20 | B92 |
| H8-239 | O | C-A1 | C-A1 | C-A3 | C-A1 | B20 | B93 |
| H8-240 | O | C-A1 | C-A1 | C-A3 | C-A1 | B20 | B94 |
| H8-241 | O | C-A1 | C-A1 | C-A3 | C-A1 | B20 | B95 |
| H8-242 | O | C-A1 | C-A1 | C-A3 | C-A1 | B20 | B96 |
| H8-243 | O | C-A1 | C-A1 | C-A3 | C-A1 | B20 | B97 |
| H8-244 | O | C-A1 | C-A1 | C-A3 | C-A1 | B20 | B106 |
| H8-245 | O | C-A1 | C-A1 | C-A3 | C-A1 | B20 | B107 |
| H8-246 | O | C-A1 | C-A1 | C-A3 | C-A1 | B20 | B114 |
| H8-247 | O | C-A1 | C-A1 | C-A3 | C-A1 | B20 | B115 |
| H8-248 | O | C-A1 | C-A1 | C-A3 | C-A1 | B20 | B116 |
| H8-249 | O | C-A1 | C-A1 | C-A3 | C-A1 | B20 | B117 |
| H8-250 | O | C-A1 | C-A1 | C-A3 | C-A1 | B20 | B118 |
| H8-251 | O | C-A1 | C-A1 | C-A3 | C-A1 | B20 | B119 |
| H8-252 | O | C-A1 | C-A1 | C-A3 | C-A1 | B20 | B120 |
| H8-253 | O | C-A1 | C-A1 | C-A3 | C-A1 | B20 | B121 |
| H8-254 | O | C-A1 | C-A1 | C-A3 | C-A1 | B20 | B122 |
| H8-255 | O | C-A1 | C-A1 | C-A3 | C-A1 | B20 | B125 |
| H8-256 | O | C-A1 | C-A1 | C-A3 | C-A1 | B20 | B126 |
| H8-257 | O | C-A1 | C-A1 | C-A3 | C-A1 | B20 | B127 |
| H8-258 | O | C-A1 | C-A1 | C-A3 | C-A1 | B20 | B128 |
| H8-259 | O | C-A1 | C-A1 | C-A3 | C-A1 | B20 | B129 |
| H8-260 | O | C-A1 | C-A1 | C-A3 | C-A1 | B20 | B130 |
| H8-261 | O | C-A1 | C-A1 | C-A3 | C-A1 | B20 | B131 |
| H8-262 | O | C-A1 | C-A1 | C-A3 | C-A1 | B20 | B132 |
| H8-263 | O | C-A1 | C-A1 | C-A3 | C-A1 | B20 | B133 |
| H8-264 | O | C-A1 | C-A1 | C-A3 | C-A1 | B20 | B134 |
| H8-265 | O | C-A1 | C-A1 | C-A3 | C-A1 | B20 | B135 |
| H8-266 | O | C-A1 | C-A1 | C-A3 | C-A1 | B20 | B136 |
| H8-267 | O | C-A1 | C-A1 | C-A3 | C-A1 | B20 | B137 |
| H8-268 | O | C-A1 | C-A1 | C-A3 | C-A1 | B20 | B138 |
| H8-269 | O | C-A1 | C-A1 | C-A3 | C-A1 | B20 | B139 |
| H8-270 | O | C-A1 | C-A1 | C-A3 | C-A1 | B20 | B140 |
| H8-277 | O | C-A1 | C-A1 | C-A1 | C-A1 | B68 | B1 |
| H8-278 | O | C-A1 | C-A1 | C-A1 | C-A1 | B68 | B6 |
| H8-279 | O | C-A1 | C-A1 | C-A1 | C-A1 | B68 | B20 |
| H8-280 | O | C-A1 | C-A1 | C-A1 | C-A1 | B68 | B21 |
| H8-281 | O | C-A1 | C-A1 | C-A1 | C-A1 | B68 | B22 |
| H8-282 | O | C-A1 | C-A1 | C-A1 | C-A1 | B68 | B32 |
| H8-283 | O | C-A1 | C-A1 | C-A1 | C-A1 | B68 | B33 |
| H8-284 | O | C-A1 | C-A1 | C-A1 | C-A1 | B68 | B34 |
| H8-285 | O | C-A1 | C-A1 | C-A1 | C-A1 | B68 | B68 |
| H8-286 | O | C-A1 | C-A1 | C-A1 | C-A1 | B68 | B69 |
| H8-287 | O | C-A1 | C-A1 | C-A1 | C-A1 | B68 | B70 |
| H8-288 | O | C-A1 | C-A1 | C-A1 | C-A1 | B68 | B71 |
| H8-289 | O | C-A1 | C-A1 | C-A1 | C-A1 | B68 | B81 |
| H8-290 | O | C-A1 | C-A1 | C-A1 | C-A1 | B68 | B90 |
| H8-291 | O | C-A1 | C-A1 | C-A1 | C-A1 | B68 | B91 |
| H8-292 | O | C-A1 | C-A1 | C-A1 | C-A1 | B68 | B92 |
| H8-293 | O | C-A1 | C-A1 | C-A1 | C-A1 | B68 | B93 |
| H8-294 | O | C-A1 | C-A1 | C-A1 | C-A1 | B68 | B94 |
| H8-295 | O | C-A1 | C-A1 | C-A1 | C-A1 | B68 | B95 |
| H8-296 | O | C-A1 | C-A1 | C-A1 | C-A1 | B68 | B96 |
| H8-297 | O | C-A1 | C-A1 | C-A1 | C-A1 | B68 | B97 |
| H8-298 | O | C-A1 | C-A1 | C-A1 | C-A1 | B68 | B106 |
| H8-299 | O | C-A1 | C-A1 | C-A1 | C-A1 | B68 | B107 |
| H8-300 | O | C-A1 | C-A1 | C-A1 | C-A1 | B68 | B114 |
| H8-301 | O | C-A1 | C-A1 | C-A1 | C-A1 | B68 | B115 |
| H8-302 | O | C-A1 | C-A1 | C-A1 | C-A1 | B68 | B116 |
| H8-303 | O | C-A1 | C-A1 | C-A1 | C-A1 | B68 | B117 |
| H8-304 | O | C-A1 | C-A1 | C-A1 | C-A1 | B68 | B118 |
| H8-305 | O | C-A1 | C-A1 | C-A1 | C-A1 | B68 | B119 |
| H8-306 | O | C-A1 | C-A1 | C-A1 | C-A1 | B68 | B120 |
| H8-307 | O | C-A1 | C-A1 | C-A1 | C-A1 | B68 | B121 |
| H8-308 | O | C-A1 | C-A1 | C-A1 | C-A1 | B68 | B122 |
| H8-309 | O | C-A1 | C-A1 | C-A1 | C-A1 | B68 | B125 |
| H8-310 | O | C-A1 | C-A1 | C-A1 | C-A1 | B68 | B126 |
| H8-311 | O | C-A1 | C-A1 | C-A1 | C-A1 | B68 | B127 |
| H8-312 | O | C-A1 | C-A1 | C-A1 | C-A1 | B68 | B128 |
| H8-313 | O | C-A1 | C-A1 | C-A1 | C-A1 | B68 | B129 |
| H8-314 | O | C-A1 | C-A1 | C-A1 | C-A1 | B68 | B130 |
| H8-315 | O | C-A1 | C-A1 | C-A1 | C-A1 | B68 | B131 |
| H8-316 | O | C-A1 | C-A1 | C-A1 | C-A1 | B68 | B132 |
| H8-317 | O | C-A1 | C-A1 | C-A1 | C-A1 | B68 | B133 |
| H8-318 | O | C-A1 | C-A1 | C-A1 | C-A1 | B68 | B134 |
| H8-319 | O | C-A1 | C-A1 | C-A1 | C-A1 | B68 | B135 |
| H8-320 | O | C-A1 | C-A1 | C-A1 | C-A1 | B68 | B136 |
| H8-321 | O | C-A1 | C-A1 | C-A1 | C-A1 | B68 | B137 |
| H8-322 | O | C-A1 | C-A1 | C-A1 | C-A1 | B68 | B138 |
| H8-323 | O | C-A1 | C-A1 | C-A1 | C-A1 | B68 | B139 |
| H8-324 | O | C-A1 | C-A1 | C-A1 | C-A1 | B68 | B140 |
| H8-331 | O | C-A1 | C-A2 | C-A1 | C-A2 | B68 | B1 |
| H8-332 | O | C-A1 | C-A2 | C-A1 | C-A2 | B68 | B6 |
| H8-333 | O | C-A1 | C-A2 | C-A1 | C-A2 | B68 | B20 |
| H8-334 | O | C-A1 | C-A2 | C-A1 | C-A2 | B68 | B21 |
| H8-335 | O | C-A1 | C-A2 | C-A1 | C-A2 | B68 | B22 |
| H8-336 | O | C-A1 | C-A2 | C-A1 | C-A2 | B68 | B32 |
| H8-337 | O | C-A1 | C-A2 | C-A1 | C-A2 | B68 | B33 |
| H8-338 | O | C-A1 | C-A2 | C-A1 | C-A2 | B68 | B34 |
| H8-339 | O | C-A1 | C-A2 | C-A1 | C-A2 | B68 | B68 |
| H8-340 | O | C-A1 | C-A2 | C-A1 | C-A2 | B68 | B69 |
| H8-341 | O | C-A1 | C-A2 | C-A1 | C-A2 | B68 | B70 |
| H8-342 | O | C-A1 | C-A2 | C-A1 | C-A2 | B68 | B71 |
| H8-343 | O | C-A1 | C-A2 | C-A1 | C-A2 | B68 | B81 |
| H8-344 | O | C-A1 | C-A2 | C-A1 | C-A2 | B68 | B90 |
| H8-345 | O | C-A1 | C-A2 | C-A1 | C-A2 | B68 | B91 |
| H8-346 | O | C-A1 | C-A2 | C-A1 | C-A2 | B68 | B92 |
| H8-347 | O | C-A1 | C-A2 | C-A1 | C-A2 | B68 | B93 |
| H8-348 | O | C-A1 | C-A2 | C-A1 | C-A2 | B68 | B94 |
| H8-349 | O | C-A1 | C-A2 | C-A1 | C-A2 | B68 | B95 |
| H8-350 | O | C-A1 | C-A2 | C-A1 | C-A2 | B68 | B96 |
| H8-351 | O | C-A1 | C-A2 | C-A1 | C-A2 | B68 | B97 |
| H8-352 | O | C-A1 | C-A2 | C-A1 | C-A2 | B68 | B106 |
| H8-353 | O | C-A1 | C-A2 | C-A1 | C-A2 | B68 | B107 |
| H8-354 | O | C-A1 | C-A2 | C-A1 | C-A2 | B68 | B114 |
| H8-355 | O | C-A1 | C-A2 | C-A1 | C-A2 | B68 | B115 |
| H8-356 | O | C-A1 | C-A2 | C-A1 | C-A2 | B68 | B116 |
| H8-357 | O | C-A1 | C-A2 | C-A1 | C-A2 | B68 | B117 |
| H8-358 | O | C-A1 | C-A2 | C-A1 | C-A2 | B68 | B118 |
| H8-359 | O | C-A1 | C-A2 | C-A1 | C-A2 | B68 | B119 |
| H8-360 | O | C-A1 | C-A2 | C-A1 | C-A2 | B68 | B120 |
| H8-361 | O | C-A1 | C-A2 | C-A1 | C-A2 | B68 | B121 |
| H8-362 | O | C-A1 | C-A2 | C-A1 | C-A2 | B68 | B122 |
| H8-363 | O | C-A1 | C-A2 | C-A1 | C-A2 | B68 | B125 |
| H8-364 | O | C-A1 | C-A2 | C-A1 | C-A2 | B68 | B126 |
| H8-365 | O | C-A1 | C-A2 | C-A1 | C-A2 | B68 | B127 |
| H8-366 | O | C-A1 | C-A2 | C-A1 | C-A2 | B68 | B128 |
| H8-367 | O | C-A1 | C-A2 | C-A1 | C-A2 | B68 | B129 |
| H8-368 | O | C-A1 | C-A2 | C-A1 | C-A2 | B68 | B130 |
| H8-369 | O | C-A1 | C-A2 | C-A1 | C-A2 | B68 | B131 |
| H8-370 | O | C-A1 | C-A2 | C-A1 | C-A2 | B68 | B132 |
| H8-371 | O | C-A1 | C-A2 | C-A1 | C-A2 | B68 | B133 |
| H8-372 | O | C-A1 | C-A2 | C-A1 | C-A2 | B68 | B134 |
| H8-373 | O | C-A1 | C-A2 | C-A1 | C-A2 | B68 | B135 |
| H8-374 | O | C-A1 | C-A2 | C-A1 | C-A2 | B68 | B136 |
| H8-375 | O | C-A1 | C-A2 | C-A1 | C-A2 | B68 | B137 |
| H8-376 | O | C-A1 | C-A2 | C-A1 | C-A2 | B68 | B138 |
| H8-377 | O | C-A1 | C-A2 | C-A1 | C-A2 | B68 | B139 |
| H8-378 | O | C-A1 | C-A2 | C-A1 | C-A2 | B68 | B140 |
| H8-385 | O | C-A1 | C-A3 | C-A1 | C-A3 | B68 | B1 |
| H8-386 | O | C-A1 | C-A3 | C-A1 | C-A3 | B68 | B6 |
| H8-387 | O | C-A1 | C-A3 | C-A1 | C-A3 | B68 | B20 |
| H8-388 | O | C-A1 | C-A3 | C-A1 | C-A3 | B68 | B21 |
| H8-389 | O | C-A1 | C-A3 | C-A1 | C-A3 | B68 | B22 |
| H8-390 | O | C-A1 | C-A3 | C-A1 | C-A3 | B68 | B32 |
| H8-391 | O | C-A1 | C-A3 | C-A1 | C-A3 | B68 | B33 |
| H8-392 | O | C-A1 | C-A3 | C-A1 | C-A3 | B68 | B34 |
| H8-393 | O | C-A1 | C-A3 | C-A1 | C-A3 | B68 | B68 |
| H8-394 | O | C-A1 | C-A3 | C-A1 | C-A3 | B68 | B69 |
| H8-395 | O | C-A1 | C-A3 | C-A1 | C-A3 | B68 | B70 |
| H8-396 | O | C-A1 | C-A3 | C-A1 | C-A3 | B68 | B71 |
| H8-397 | O | C-A1 | C-A3 | C-A1 | C-A3 | B68 | B81 |
| H8-398 | O | C-A1 | C-A3 | C-A1 | C-A3 | B68 | B90 |
| H8-399 | O | C-A1 | C-A3 | C-A1 | C-A3 | B68 | B91 |
| H8-400 | O | C-A1 | C-A3 | C-A1 | C-A3 | B68 | B92 |
| H8-401 | O | C-A1 | C-A3 | C-A1 | C-A3 | B68 | B93 |
| H8-402 | O | C-A1 | C-A3 | C-A1 | C-A3 | B68 | B94 |
| H8-403 | O | C-A1 | C-A3 | C-A1 | C-A3 | B68 | B95 |
| H8-404 | O | C-A1 | C-A3 | C-A1 | C-A3 | B68 | B96 |
| H8-405 | O | C-A1 | C-A3 | C-A1 | C-A3 | B68 | B97 |
| H8-406 | O | C-A1 | C-A3 | C-A1 | C-A3 | B68 | B106 |
| H8-407 | O | C-A1 | C-A3 | C-A1 | C-A3 | B68 | B107 |
| H8-408 | O | C-A1 | C-A3 | C-A1 | C-A3 | B68 | B114 |
| H8-409 | O | C-A1 | C-A3 | C-A1 | C-A3 | B68 | B115 |
| H8-410 | O | C-A1 | C-A3 | C-A1 | C-A3 | B68 | B116 |
| H8-411 | O | C-A1 | C-A3 | C-A1 | C-A3 | B68 | B117 |
| H8-412 | O | C-A1 | C-A3 | C-A1 | C-A3 | B68 | B118 |
| H8-413 | O | C-A1 | C-A3 | C-A1 | C-A3 | B68 | B119 |
| H8-414 | O | C-A1 | C-A3 | C-A1 | C-A3 | B68 | B120 |
| H8-415 | O | C-A1 | C-A3 | C-A1 | C-A3 | B68 | B121 |
| H8-416 | O | C-A1 | C-A3 | C-A1 | C-A3 | B68 | B122 |
| H8-417 | O | C-A1 | C-A3 | C-A1 | C-A3 | B68 | B125 |
| H8-418 | O | C-A1 | C-A3 | C-A1 | C-A3 | B68 | B126 |
| H8-419 | O | C-A1 | C-A3 | C-A1 | C-A3 | B68 | B127 |
| H8-420 | O | C-A1 | C-A3 | C-A1 | C-A3 | B68 | B128 |
| H8-421 | O | C-A1 | C-A3 | C-A1 | C-A3 | B68 | B129 |
| H8-422 | O | C-A1 | C-A3 | C-A1 | C-A3 | B68 | B130 |
| H8-423 | O | C-A1 | C-A3 | C-A1 | C-A3 | B68 | B131 |
| H8-424 | O | C-A1 | C-A3 | C-A1 | C-A3 | B68 | B132 |
| H8-425 | O | C-A1 | C-A3 | C-A1 | C-A3 | B68 | B133 |
| H8-426 | O | C-A1 | C-A3 | C-A1 | C-A3 | B68 | B134 |
| H8-427 | O | C-A1 | C-A3 | C-A1 | C-A3 | B68 | B135 |
| H8-428 | O | C-A1 | C-A3 | C-A1 | C-A3 | B68 | B136 |
| H8-429 | O | C-A1 | C-A3 | C-A1 | C-A3 | B68 | B137 |
| H8-430 | O | C-A1 | C-A3 | C-A1 | C-A3 | B68 | B138 |
| H8-431 | O | C-A1 | C-A3 | C-A1 | C-A3 | B68 | B139 |
| H8-432 | O | C-A1 | C-A3 | C-A1 | C-A3 | B68 | B140 |
| H8-439 | O | C-A3 | C-A1 | C-A3 | C-A1 | B68 | B1 |
| H8-440 | O | C-A3 | C-A1 | C-A3 | C-A1 | B68 | B6 |
| H8-441 | O | C-A3 | C-A1 | C-A3 | C-A1 | B68 | B20 |
| H8-442 | O | C-A3 | C-A1 | C-A3 | C-A1 | B68 | B21 |
| H8-443 | O | C-A3 | C-A1 | C-A3 | C-A1 | B68 | B22 |
| H8-444 | O | C-A3 | C-A1 | C-A3 | C-A1 | B68 | B32 |
| H8-445 | O | C-A3 | C-A1 | C-A3 | C-A1 | B68 | B33 |
| H8-446 | O | C-A3 | C-A1 | C-A3 | C-A1 | B68 | B34 |
| H8-447 | O | C-A3 | C-A1 | C-A3 | C-A1 | B68 | B68 |
| H8-448 | O | C-A3 | C-A1 | C-A3 | C-A1 | B68 | B69 |
| H8-449 | O | C-A3 | C-A1 | C-A3 | C-A1 | B68 | B70 |
| H8-450 | O | C-A3 | C-A1 | C-A3 | C-A1 | B68 | B71 |
| H8-451 | O | C-A3 | C-A1 | C-A3 | C-A1 | B68 | B81 |
| H8-452 | O | C-A3 | C-A1 | C-A3 | C-A1 | B68 | B90 |
| H8-453 | O | C-A3 | C-A1 | C-A3 | C-A1 | B68 | B91 |
| H8-454 | O | C-A3 | C-A1 | C-A3 | C-A1 | B68 | B92 |
| H8-455 | O | C-A3 | C-A1 | C-A3 | C-A1 | B68 | B93 |
| H8-456 | O | C-A3 | C-A1 | C-A3 | C-A1 | B68 | B94 |
| H8-457 | O | C-A3 | C-A1 | C-A3 | C-A1 | B68 | B95 |
| H8-458 | O | C-A3 | C-A1 | C-A3 | C-A1 | B68 | B96 |
| H8-459 | O | C-A3 | C-A1 | C-A3 | C-A1 | B68 | B97 |
| H8-460 | O | C-A3 | C-A1 | C-A3 | C-A1 | B68 | B106 |
| H8-461 | O | C-A3 | C-A1 | C-A3 | C-A1 | B68 | B107 |
| H8-462 | O | C-A3 | C-A1 | C-A3 | C-A1 | B68 | B114 |
| H8-463 | O | C-A3 | C-A1 | C-A3 | C-A1 | B68 | B115 |
| H8-464 | O | C-A3 | C-A1 | C-A3 | C-A1 | B68 | B116 |
| H8-465 | O | C-A3 | C-A1 | C-A3 | C-A1 | B68 | B117 |
| H8-466 | O | C-A3 | C-A1 | C-A3 | C-A1 | B68 | B118 |
| H8-467 | O | C-A3 | C-A1 | C-A3 | C-A1 | B68 | B119 |
| H8-468 | O | C-A3 | C-A1 | C-A3 | C-A1 | B68 | B120 |
| H8-469 | O | C-A3 | C-A1 | C-A3 | C-A1 | B68 | B121 |
| H8-470 | O | C-A3 | C-A1 | C-A3 | C-A1 | B68 | B122 |
| H8-471 | O | C-A3 | C-A1 | C-A3 | C-A1 | B68 | B125 |
| H8-472 | O | C-A3 | C-A1 | C-A3 | C-A1 | B68 | B126 |
| H8-473 | O | C-A3 | C-A1 | C-A3 | C-A1 | B68 | B127 |
| H8-474 | O | C-A3 | C-A1 | C-A3 | C-A1 | B68 | B128 |
| H8-475 | O | C-A3 | C-A1 | C-A3 | C-A1 | B68 | B129 |
| H8-476 | O | C-A3 | C-A1 | C-A3 | C-A1 | B68 | B130 |
| H8-477 | O | C-A3 | C-A1 | C-A3 | C-A1 | B68 | B131 |
| H8-478 | O | C-A3 | C-A1 | C-A3 | C-A1 | B68 | B132 |
| H8-479 | O | C-A3 | C-A1 | C-A3 | C-A1 | B68 | B133 |
| H8-480 | O | C-A3 | C-A1 | C-A3 | C-A1 | B68 | B134 |
| H8-481 | O | C-A3 | C-A1 | C-A3 | C-A1 | B68 | B135 |
| H8-482 | O | C-A3 | C-A1 | C-A3 | C-A1 | B68 | B136 |
| H8-483 | O | C-A3 | C-A1 | C-A3 | C-A1 | B68 | B137 |
| H8-484 | O | C-A3 | C-A1 | C-A3 | C-A1 | B68 | B138 |
| H8-485 | O | C-A3 | C-A1 | C-A3 | C-A1 | B68 | B139 |
| H8-486 | O | C-A3 | C-A1 | C-A3 | C-A1 | B68 | B140 |
| H8-493 | O | C-A1 | C-A1 | C-A3 | C-A1 | B68 | B1 |
| H8-494 | O | C-A1 | C-A1 | C-A3 | C-A1 | B68 | B6 |
| H8-495 | O | C-A1 | C-A1 | C-A3 | C-A1 | B68 | B20 |
| H8-496 | O | C-A1 | C-A1 | C-A3 | C-A1 | B68 | B21 |
| H8-497 | O | C-A1 | C-A1 | C-A3 | C-A1 | B68 | B22 |
| H8-498 | O | C-A1 | C-A1 | C-A3 | C-A1 | B68 | B32 |
| H8-499 | O | C-A1 | C-A1 | C-A3 | C-A1 | B68 | B33 |
| H8-500 | O | C-A1 | C-A1 | C-A3 | C-A1 | B68 | B34 |
| H8-501 | O | C-A1 | C-A1 | C-A3 | C-A1 | B68 | B68 |
| H8-502 | O | C-A1 | C-A1 | C-A3 | C-A1 | B68 | B69 |
| H8-503 | O | C-A1 | C-A1 | C-A3 | C-A1 | B68 | B70 |
| H8-504 | O | C-A1 | C-A1 | C-A3 | C-A1 | B68 | B71 |
| H8-505 | O | C-A1 | C-A1 | C-A3 | C-A1 | B68 | B81 |
| H8-506 | O | C-A1 | C-A1 | C-A3 | C-A1 | B68 | B90 |
| H8-507 | O | C-A1 | C-A1 | C-A3 | C-A1 | B68 | B91 |
| H8-508 | O | C-A1 | C-A1 | C-A3 | C-A1 | B68 | B92 |
| H8-509 | O | C-A1 | C-A1 | C-A3 | C-A1 | B68 | B93 |
| H8-510 | O | C-A1 | C-A1 | C-A3 | C-A1 | B68 | B94 |
| H8-511 | O | C-A1 | C-A1 | C-A3 | C-A1 | B68 | B95 |
| H8-512 | O | C-A1 | C-A1 | C-A3 | C-A1 | B68 | B96 |
| H8-513 | O | C-A1 | C-A1 | C-A3 | C-A1 | B68 | B97 |
| H8-514 | O | C-A1 | C-A1 | C-A3 | C-A1 | B68 | B106 |
| H8-515 | O | C-A1 | C-A1 | C-A3 | C-A1 | B68 | B107 |
| H8-516 | O | C-A1 | C-A1 | C-A3 | C-A1 | B68 | B114 |
| H8-517 | O | C-A1 | C-A1 | C-A3 | C-A1 | B68 | B115 |
| H8-518 | O | C-A1 | C-A1 | C-A3 | C-A1 | B68 | B116 |
| H8-519 | O | C-A1 | C-A1 | C-A3 | C-A1 | B68 | B117 |
| H8-520 | O | C-A1 | C-A1 | C-A3 | C-A1 | B68 | B118 |
| H8-521 | O | C-A1 | C-A1 | C-A3 | C-A1 | B68 | B119 |
| H8-522 | O | C-A1 | C-A1 | C-A3 | C-A1 | B68 | B120 |
| H8-523 | O | C-A1 | C-A1 | C-A3 | C-A1 | B68 | B121 |
| H8-524 | O | C-A1 | C-A1 | C-A3 | C-A1 | B68 | B122 |
| H8-525 | O | C-A1 | C-A1 | C-A3 | C-A1 | B68 | B125 |
| H8-526 | O | C-A1 | C-A1 | C-A3 | C-A1 | B68 | B126 |
| H8-527 | O | C-A1 | C-A1 | C-A3 | C-A1 | B68 | B127 |
| H8-528 | O | C-A1 | C-A1 | C-A3 | C-A1 | B68 | B128 |
| H8-529 | O | C-A1 | C-A1 | C-A3 | C-A1 | B68 | B129 |
| H8-530 | O | C-A1 | C-A1 | C-A3 | C-A1 | B68 | B130 |
| H8-531 | O | C-A1 | C-A1 | C-A3 | C-A1 | B68 | B131 |
| H8-532 | O | C-A1 | C-A1 | C-A3 | C-A1 | B68 | B132 |
| H8-533 | O | C-A1 | C-A1 | C-A3 | C-A1 | B68 | B133 |
| H8-534 | O | C-A1 | C-A1 | C-A3 | C-A1 | B68 | B134 |
| H8-535 | O | C-A1 | C-A1 | C-A3 | C-A1 | B68 | B135 |
| H8-536 | O | C-A1 | C-A1 | C-A3 | C-A1 | B68 | B136 |
| H8-537 | O | C-A1 | C-A1 | C-A3 | C-A1 | B68 | B137 |
| H8-538 | O | C-A1 | C-A1 | C-A3 | C-A1 | B68 | B138 |
| H8-539 | O | C-A1 | C-A1 | C-A3 | C-A1 | B68 | B139 |
| H8-540 | O | C-A1 | C-A1 | C-A3 | C-A1 | B68 | B140 |
| H8-547 | O | C-A1 | C-A1 | C-A1 | C-A1 | B70 | B1 |
| H8-548 | O | C-A1 | C-A1 | C-A1 | C-A1 | B70 | B6 |
| H8-549 | O | C-A1 | C-A1 | C-A1 | C-A1 | B70 | B20 |
| H8-550 | O | C-A1 | C-A1 | C-A1 | C-A1 | B70 | B21 |
| H8-551 | O | C-A1 | C-A1 | C-A1 | C-A1 | B70 | B22 |
| H8-552 | O | C-A1 | C-A1 | C-A1 | C-A1 | B70 | B32 |
| H8-553 | O | C-A1 | C-A1 | C-A1 | C-A1 | B70 | B33 |
| H8-554 | O | C-A1 | C-A1 | C-A1 | C-A1 | B70 | B34 |
| H8-555 | O | C-A1 | C-A1 | C-A1 | C-A1 | B70 | B68 |
| H8-556 | O | C-A1 | C-A1 | C-A1 | C-A1 | B70 | B69 |
| H8-557 | O | C-A1 | C-A1 | C-A1 | C-A1 | B70 | B70 |
| H8-558 | O | C-A1 | C-A1 | C-A1 | C-A1 | B70 | B71 |
| H8-559 | O | C-A1 | C-A1 | C-A1 | C-A1 | B70 | B81 |
| H8-560 | O | C-A1 | C-A1 | C-A1 | C-A1 | B70 | B90 |
| H8-561 | O | C-A1 | C-A1 | C-A1 | C-A1 | B70 | B91 |
| H8-562 | O | C-A1 | C-A1 | C-A1 | C-A1 | B70 | B92 |
| H8-563 | O | C-A1 | C-A1 | C-A1 | C-A1 | B70 | B93 |
| H8-564 | O | C-A1 | C-A1 | C-A1 | C-A1 | B70 | B94 |
| H8-565 | O | C-A1 | C-A1 | C-A1 | C-A1 | B70 | B95 |
| H8-566 | O | C-A1 | C-A1 | C-A1 | C-A1 | B70 | B96 |
| H8-567 | O | C-A1 | C-A1 | C-A1 | C-A1 | B70 | B97 |
| H8-568 | O | C-A1 | C-A1 | C-A1 | C-A1 | B70 | B106 |
| H8-569 | O | C-A1 | C-A1 | C-A1 | C-A1 | B70 | B107 |
| H8-570 | O | C-A1 | C-A1 | C-A1 | C-A1 | B70 | B114 |
| H8-571 | O | C-A1 | C-A1 | C-A1 | C-A1 | B70 | B115 |
| H8-572 | O | C-A1 | C-A1 | C-A1 | C-A1 | B70 | B116 |
| H8-573 | O | C-A1 | C-A1 | C-A1 | C-A1 | B70 | B117 |
| H8-574 | O | C-A1 | C-A1 | C-A1 | C-A1 | B70 | B118 |
| H8-575 | O | C-A1 | C-A1 | C-A1 | C-A1 | B70 | B119 |
| H8-576 | O | C-A1 | C-A1 | C-A1 | C-A1 | B70 | B120 |
| H8-577 | O | C-A1 | C-A1 | C-A1 | C-A1 | B70 | B121 |
| H8-578 | O | C-A1 | C-A1 | C-A1 | C-A1 | B70 | B122 |
| H8-579 | O | C-A1 | C-A1 | C-A1 | C-A1 | B70 | B125 |
| H8-580 | O | C-A1 | C-A1 | C-A1 | C-A1 | B70 | B126 |
| H8-581 | O | C-A1 | C-A1 | C-A1 | C-A1 | B70 | B127 |
| H8-582 | O | C-A1 | C-A1 | C-A1 | C-A1 | B70 | B128 |
| H8-583 | O | C-A1 | C-A1 | C-A1 | C-A1 | B70 | B129 |
| H8-584 | O | C-A1 | C-A1 | C-A1 | C-A1 | B70 | B130 |
| H8-585 | O | C-A1 | C-A1 | C-A1 | C-A1 | B70 | B131 |
| H8-586 | O | C-A1 | C-A1 | C-A1 | C-A1 | B70 | B132 |
| H8-587 | O | C-A1 | C-A1 | C-A1 | C-A1 | B70 | B133 |
| H8-588 | O | C-A1 | C-A1 | C-A1 | C-A1 | B70 | B134 |
| H8-589 | O | C-A1 | C-A1 | C-A1 | C-A1 | B70 | B135 |
| H8-590 | O | C-A1 | C-A1 | C-A1 | C-A1 | B70 | B136 |
| H8-591 | O | C-A1 | C-A1 | C-A1 | C-A1 | B70 | B137 |
| H8-592 | O | C-A1 | C-A1 | C-A1 | C-A1 | B70 | B138 |
| H8-593 | O | C-A1 | C-A1 | C-A1 | C-A1 | B70 | B139 |
| H8-594 | O | C-A1 | C-A1 | C-A1 | C-A1 | B70 | B140 |
| H8-601 | O | C-A1 | C-A2 | C-A1 | C-A2 | B70 | B1 |
| H8-602 | O | C-A1 | C-A2 | C-A1 | C-A2 | B70 | B6 |
| H8-603 | O | C-A1 | C-A2 | C-A1 | C-A2 | B70 | B20 |
| H8-604 | O | C-A1 | C-A2 | C-A1 | C-A2 | B70 | B21 |
| H8-605 | O | C-A1 | C-A2 | C-A1 | C-A2 | B70 | B22 |
| H8-606 | O | C-A1 | C-A2 | C-A1 | C-A2 | B70 | B32 |
| H8-607 | O | C-A1 | C-A2 | C-A1 | C-A2 | B70 | B33 |
| H8-608 | O | C-A1 | C-A2 | C-A1 | C-A2 | B70 | B34 |
| H8-609 | O | C-A1 | C-A2 | C-A1 | C-A2 | B70 | B68 |
| H8-610 | O | C-A1 | C-A2 | C-A1 | C-A2 | B70 | B69 |
| H8-611 | O | C-A1 | C-A2 | C-A1 | C-A2 | B70 | B70 |
| H8-612 | O | C-A1 | C-A2 | C-A1 | C-A2 | B70 | B71 |
| H8-613 | O | C-A1 | C-A2 | C-A1 | C-A2 | B70 | B81 |
| H8-614 | O | C-A1 | C-A2 | C-A1 | C-A2 | B70 | B90 |
| H8-615 | O | C-A1 | C-A2 | C-A1 | C-A2 | B70 | B91 |
| H8-616 | O | C-A1 | C-A2 | C-A1 | C-A2 | B70 | B92 |
| H8-617 | O | C-A1 | C-A2 | C-A1 | C-A2 | B70 | B93 |
| H8-618 | O | C-A1 | C-A2 | C-A1 | C-A2 | B70 | B94 |
| H8-619 | O | C-A1 | C-A2 | C-A1 | C-A2 | B70 | B95 |
| H8-620 | O | C-A1 | C-A2 | C-A1 | C-A2 | B70 | B96 |
| H8-621 | O | C-A1 | C-A2 | C-A1 | C-A2 | B70 | B97 |
| H8-622 | O | C-A1 | C-A2 | C-A1 | C-A2 | B70 | B106 |
| H8-623 | O | C-A1 | C-A2 | C-A1 | C-A2 | B70 | B107 |
| H8-624 | O | C-A1 | C-A2 | C-A1 | C-A2 | B70 | B114 |
| H8-625 | O | C-A1 | C-A2 | C-A1 | C-A2 | B70 | B115 |
| H8-626 | O | C-A1 | C-A2 | C-A1 | C-A2 | B70 | B116 |
| H8-627 | O | C-A1 | C-A2 | C-A1 | C-A2 | B70 | B117 |
| H8-628 | O | C-A1 | C-A2 | C-A1 | C-A2 | B70 | B118 |
| H8-629 | O | C-A1 | C-A2 | C-A1 | C-A2 | B70 | B119 |
| H8-630 | O | C-A1 | C-A2 | C-A1 | C-A2 | B70 | B120 |
| H8-631 | O | C-A1 | C-A2 | C-A1 | C-A2 | B70 | B121 |
| H8-632 | O | C-A1 | C-A2 | C-A1 | C-A2 | B70 | B122 |
| H8-633 | O | C-A1 | C-A2 | C-A1 | C-A2 | B70 | B125 |
| H8-634 | O | C-A1 | C-A2 | C-A1 | C-A2 | B70 | B126 |
| H8-635 | O | C-A1 | C-A2 | C-A1 | C-A2 | B70 | B127 |
| H8-636 | O | C-A1 | C-A2 | C-A1 | C-A2 | B70 | B128 |
| H8-637 | O | C-A1 | C-A2 | C-A1 | C-A2 | B70 | B129 |
| H8-638 | O | C-A1 | C-A2 | C-A1 | C-A2 | B70 | B130 |
| H8-639 | O | C-A1 | C-A2 | C-A1 | C-A2 | B70 | B131 |
| H8-640 | O | C-A1 | C-A2 | C-A1 | C-A2 | B70 | B132 |
| H8-641 | O | C-A1 | C-A2 | C-A1 | C-A2 | B70 | B133 |
| H8-642 | O | C-A1 | C-A2 | C-A1 | C-A2 | B70 | B134 |
| H8-643 | O | C-A1 | C-A2 | C-A1 | C-A2 | B70 | B135 |
| H8-644 | O | C-A1 | C-A2 | C-A1 | C-A2 | B70 | B136 |
| H8-645 | O | C-A1 | C-A2 | C-A1 | C-A2 | B70 | B137 |
| H8-646 | O | C-A1 | C-A2 | C-A1 | C-A2 | B70 | B138 |
| H8-647 | O | C-A1 | C-A2 | C-A1 | C-A2 | B70 | B139 |
| H8-648 | O | C-A1 | C-A2 | C-A1 | C-A2 | B70 | B140 |
| H8-655 | O | C-A1 | C-A3 | C-A1 | C-A3 | B70 | B1 |
| H8-656 | O | C-A1 | C-A3 | C-A1 | C-A3 | B70 | B6 |
| H8-657 | O | C-A1 | C-A3 | C-A1 | C-A3 | B70 | B20 |
| H8-658 | O | C-A1 | C-A3 | C-A1 | C-A3 | B70 | B21 |
| H8-659 | O | C-A1 | C-A3 | C-A1 | C-A3 | B70 | B22 |
| H8-660 | O | C-A1 | C-A3 | C-A1 | C-A3 | B70 | B32 |
| H8-661 | O | C-A1 | C-A3 | C-A1 | C-A3 | B70 | B33 |
| H8-662 | O | C-A1 | C-A3 | C-A1 | C-A3 | B70 | B34 |
| H8-663 | O | C-A1 | C-A3 | C-A1 | C-A3 | B70 | B68 |
| H8-664 | O | C-A1 | C-A3 | C-A1 | C-A3 | B70 | B69 |
| H8-665 | O | C-A1 | C-A3 | C-A1 | C-A3 | B70 | B70 |
| H8-666 | O | C-A1 | C-A3 | C-A1 | C-A3 | B70 | B71 |
| H8-667 | O | C-A1 | C-A3 | C-A1 | C-A3 | B70 | B81 |
| H8-668 | O | C-A1 | C-A3 | C-A1 | C-A3 | B70 | B90 |
| H8-669 | O | C-A1 | C-A3 | C-A1 | C-A3 | B70 | B91 |
| H8-670 | O | C-A1 | C-A3 | C-A1 | C-A3 | B70 | B92 |
| H8-671 | O | C-A1 | C-A3 | C-A1 | C-A3 | B70 | B93 |
| H8-672 | O | C-A1 | C-A3 | C-A1 | C-A3 | B70 | B94 |
| H8-673 | O | C-A1 | C-A3 | C-A1 | C-A3 | B70 | B95 |
| H8-674 | O | C-A1 | C-A3 | C-A1 | C-A3 | B70 | B96 |
| H8-675 | O | C-A1 | C-A3 | C-A1 | C-A3 | B70 | B97 |
| H8-676 | O | C-A1 | C-A3 | C-A1 | C-A3 | B70 | B106 |
| H8-677 | O | C-A1 | C-A3 | C-A1 | C-A3 | B70 | B107 |
| H8-678 | O | C-A1 | C-A3 | C-A1 | C-A3 | B70 | B114 |
| H8-679 | O | C-A1 | C-A3 | C-A1 | C-A3 | B70 | B115 |
| H8-680 | O | C-A1 | C-A3 | C-A1 | C-A3 | B70 | B116 |
| H8-681 | O | C-A1 | C-A3 | C-A1 | C-A3 | B70 | B117 |
| H8-682 | O | C-A1 | C-A3 | C-A1 | C-A3 | B70 | B118 |
| H8-683 | O | C-A1 | C-A3 | C-A1 | C-A3 | B70 | B119 |
| H8-684 | O | C-A1 | C-A3 | C-A1 | C-A3 | B70 | B120 |
| H8-685 | O | C-A1 | C-A3 | C-A1 | C-A3 | B70 | B121 |
| H8-686 | O | C-A1 | C-A3 | C-A1 | C-A3 | B70 | B122 |
| H8-687 | O | C-A1 | C-A3 | C-A1 | C-A3 | B70 | B125 |
| H8-688 | O | C-A1 | C-A3 | C-A1 | C-A3 | B70 | B126 |
| H8-689 | O | C-A1 | C-A3 | C-A1 | C-A3 | B70 | B127 |
| H8-690 | O | C-A1 | C-A3 | C-A1 | C-A3 | B70 | B128 |
| H8-691 | O | C-A1 | C-A3 | C-A1 | C-A3 | B70 | B129 |
| H8-692 | O | C-A1 | C-A3 | C-A1 | C-A3 | B70 | B130 |
| H8-693 | O | C-A1 | C-A3 | C-A1 | C-A3 | B70 | B131 |
| H8-694 | O | C-A1 | C-A3 | C-A1 | C-A3 | B70 | B132 |
| H8-695 | O | C-A1 | C-A3 | C-A1 | C-A3 | B70 | B133 |
| H8-696 | O | C-A1 | C-A3 | C-A1 | C-A3 | B70 | B134 |
| H8-697 | O | C-A1 | C-A3 | C-A1 | C-A3 | B70 | B135 |
| H8-698 | O | C-A1 | C-A3 | C-A1 | C-A3 | B70 | B136 |
| H8-699 | O | C-A1 | C-A3 | C-A1 | C-A3 | B70 | B137 |
| H8-700 | O | C-A1 | C-A3 | C-A1 | C-A3 | B70 | B138 |
| H8-701 | O | C-A1 | C-A3 | C-A1 | C-A3 | B70 | B139 |
| H8-702 | O | C-A1 | C-A3 | C-A1 | C-A3 | B70 | B140 |
| H8-709 | O | C-A3 | C-A1 | C-A3 | C-A1 | B70 | B1 |
| H8-710 | O | C-A3 | C-A1 | C-A3 | C-A1 | B70 | B6 |
| H8-711 | O | C-A3 | C-A1 | C-A3 | C-A1 | B70 | B20 |
| H8-712 | O | C-A3 | C-A1 | C-A3 | C-A1 | B70 | B21 |
| H8-713 | O | C-A3 | C-A1 | C-A3 | C-A1 | B70 | B22 |
| H8-714 | O | C-A3 | C-A1 | C-A3 | C-A1 | B70 | B32 |
| H8-715 | O | C-A3 | C-A1 | C-A3 | C-A1 | B70 | B33 |
| H8-716 | O | C-A3 | C-A1 | C-A3 | C-A1 | B70 | B34 |
| H8-717 | O | C-A3 | C-A1 | C-A3 | C-A1 | B70 | B68 |
| H8-718 | O | C-A3 | C-A1 | C-A3 | C-A1 | B70 | B69 |
| H8-719 | O | C-A3 | C-A1 | C-A3 | C-A1 | B70 | B70 |
| H8-720 | O | C-A3 | C-A1 | C-A3 | C-A1 | B70 | B71 |
| H8-721 | O | C-A3 | C-A1 | C-A3 | C-A1 | B70 | B81 |
| H8-722 | O | C-A3 | C-A1 | C-A3 | C-A1 | B70 | B90 |
| H8-723 | O | C-A3 | C-A1 | C-A3 | C-A1 | B70 | B91 |
| H8-724 | O | C-A3 | C-A1 | C-A3 | C-A1 | B70 | B92 |
| H8-725 | O | C-A3 | C-A1 | C-A3 | C-A1 | B70 | B93 |
| H8-726 | O | C-A3 | C-A1 | C-A3 | C-A1 | B70 | B94 |
| H8-727 | O | C-A3 | C-A1 | C-A3 | C-A1 | B70 | B95 |
| H8-728 | O | C-A3 | C-A1 | C-A3 | C-A1 | B70 | B96 |
| H8-729 | O | C-A3 | C-A1 | C-A3 | C-A1 | B70 | B97 |
| H8-730 | O | C-A3 | C-A1 | C-A3 | C-A1 | B70 | B106 |
| H8-731 | O | C-A3 | C-A1 | C-A3 | C-A1 | B70 | B107 |
| H8-732 | O | C-A3 | C-A1 | C-A3 | C-A1 | B70 | B114 |
| H8-733 | O | C-A3 | C-A1 | C-A3 | C-A1 | B70 | B115 |
| H8-734 | O | C-A3 | C-A1 | C-A3 | C-A1 | B70 | B116 |
| H8-735 | O | C-A3 | C-A1 | C-A3 | C-A1 | B70 | B117 |
| H8-736 | O | C-A3 | C-A1 | C-A3 | C-A1 | B70 | B118 |
| H8-737 | O | C-A3 | C-A1 | C-A3 | C-A1 | B70 | B119 |
| H8-738 | O | C-A3 | C-A1 | C-A3 | C-A1 | B70 | B120 |
| H8-739 | O | C-A3 | C-A1 | C-A3 | C-A1 | B70 | B121 |
| H8-740 | O | C-A3 | C-A1 | C-A3 | C-A1 | B70 | B122 |
| H8-741 | O | C-A3 | C-A1 | C-A3 | C-A1 | B70 | B125 |
| H8-742 | O | C-A3 | C-A1 | C-A3 | C-A1 | B70 | B126 |
| H8-743 | O | C-A3 | C-A1 | C-A3 | C-A1 | B70 | B127 |
| H8-744 | O | C-A3 | C-A1 | C-A3 | C-A1 | B70 | B128 |
| H8-745 | O | C-A3 | C-A1 | C-A3 | C-A1 | B70 | B129 |
| H8-746 | O | C-A3 | C-A1 | C-A3 | C-A1 | B70 | B130 |
| H8-747 | O | C-A3 | C-A1 | C-A3 | C-A1 | B70 | B131 |
| H8-748 | O | C-A3 | C-A1 | C-A3 | C-A1 | B70 | B132 |
| H8-749 | O | C-A3 | C-A1 | C-A3 | C-A1 | B70 | B133 |
| H8-750 | O | C-A3 | C-A1 | C-A3 | C-A1 | B70 | B134 |
| H8-751 | O | C-A3 | C-A1 | C-A3 | C-A1 | B70 | B135 |
| H8-752 | O | C-A3 | C-A1 | C-A3 | C-A1 | B70 | B136 |
| H8-753 | O | C-A3 | C-A1 | C-A3 | C-A1 | B70 | B137 |
| H8-754 | O | C-A3 | C-A1 | C-A3 | C-A1 | B70 | B138 |
| H8-755 | O | C-A3 | C-A1 | C-A3 | C-A1 | B70 | B139 |
| H8-756 | O | C-A3 | C-A1 | C-A3 | C-A1 | B70 | B140 |
| H8-763 | O | C-A1 | C-A1 | C-A3 | C-A1 | B70 | B1 |
| H8-764 | O | C-A1 | C-A1 | C-A3 | C-A1 | B70 | B6 |
| H8-765 | O | C-A1 | C-A1 | C-A3 | C-A1 | B70 | B20 |
| H8-766 | O | C-A1 | C-A1 | C-A3 | C-A1 | B70 | B21 |
| H8-767 | O | C-A1 | C-A1 | C-A3 | C-A1 | B70 | B22 |
| H8-768 | O | C-A1 | C-A1 | C-A3 | C-A1 | B70 | B32 |
| H8-769 | O | C-A1 | C-A1 | C-A3 | C-A1 | B70 | B33 |
| H8-770 | O | C-A1 | C-A1 | C-A3 | C-A1 | B70 | B34 |
| H8-771 | O | C-A1 | C-A1 | C-A3 | C-A1 | B70 | B68 |
| H8-772 | O | C-A1 | C-A1 | C-A3 | C-A1 | B70 | B69 |
| H8-773 | O | C-A1 | C-A1 | C-A3 | C-A1 | B70 | B70 |
| H8-774 | O | C-A1 | C-A1 | C-A3 | C-A1 | B70 | B71 |
| H8-775 | O | C-A1 | C-A1 | C-A3 | C-A1 | B70 | B81 |
| H8-776 | O | C-A1 | C-A1 | C-A3 | C-A1 | B70 | B90 |
| H8-777 | O | C-A1 | C-A1 | C-A3 | C-A1 | B70 | B91 |
| H8-778 | O | C-A1 | C-A1 | C-A3 | C-A1 | B70 | B92 |
| H8-779 | O | C-A1 | C-A1 | C-A3 | C-A1 | B70 | B93 |
| H8-780 | O | C-A1 | C-A1 | C-A3 | C-A1 | B70 | B94 |
| H8-781 | O | C-A1 | C-A1 | C-A3 | C-A1 | B70 | B95 |
| H8-782 | O | C-A1 | C-A1 | C-A3 | C-A1 | B70 | B96 |
| H8-783 | O | C-A1 | C-A1 | C-A3 | C-A1 | B70 | B97 |
| H8-784 | O | C-A1 | C-A1 | C-A3 | C-A1 | B70 | B106 |
| H8-785 | O | C-A1 | C-A1 | C-A3 | C-A1 | B70 | B107 |
| H8-786 | O | C-A1 | C-A1 | C-A3 | C-A1 | B70 | B114 |
| H8-787 | O | C-A1 | C-A1 | C-A3 | C-A1 | B70 | B115 |
| H8-788 | O | C-A1 | C-A1 | C-A3 | C-A1 | B70 | B116 |
| H8-789 | O | C-A1 | C-A1 | C-A3 | C-A1 | B70 | B117 |
| H8-790 | O | C-A1 | C-A1 | C-A3 | C-A1 | B70 | B118 |
| H8-791 | O | C-A1 | C-A1 | C-A3 | C-A1 | B70 | B119 |
| H8-792 | O | C-A1 | C-A1 | C-A3 | C-A1 | B70 | B120 |
| H8-793 | O | C-A1 | C-A1 | C-A3 | C-A1 | B70 | B121 |
| H8-794 | O | C-A1 | C-A1 | C-A3 | C-A1 | B70 | B122 |
| H8-795 | O | C-A1 | C-A1 | C-A3 | C-A1 | B70 | B125 |
| H8-796 | O | C-A1 | C-A1 | C-A3 | C-A1 | B70 | B126 |
| H8-797 | O | C-A1 | C-A1 | C-A3 | C-A1 | B70 | B127 |
| H8-798 | O | C-A1 | C-A1 | C-A3 | C-A1 | B70 | B128 |
| H8-799 | O | C-A1 | C-A1 | C-A3 | C-A1 | B70 | B129 |
| H8-800 | O | C-A1 | C-A1 | C-A3 | C-A1 | B70 | B130 |
| H8-801 | O | C-A1 | C-A1 | C-A3 | C-A1 | B70 | B131 |
| H8-802 | O | C-A1 | C-A1 | C-A3 | C-A1 | B70 | B132 |
| H8-803 | O | C-A1 | C-A1 | C-A3 | C-A1 | B70 | B133 |
| H8-804 | O | C-A1 | C-A1 | C-A3 | C-A1 | B70 | B134 |
| H8-805 | O | C-A1 | C-A1 | C-A3 | C-A1 | B70 | B135 |
| H8-806 | O | C-A1 | C-A1 | C-A3 | C-A1 | B70 | B136 |
| H8-807 | O | C-A1 | C-A1 | C-A3 | C-A1 | B70 | B137 |
| H8-808 | O | C-A1 | C-A1 | C-A3 | C-A1 | B70 | B138 |
| H8-809 | O | C-A1 | C-A1 | C-A3 | C-A1 | B70 | B139 |
| H8-810 | O | C-A1 | C-A1 | C-A3 | C-A1 | B70 | B140 |
| H8-817 | O | C-A1 | C-A1 | C-A1 | C-A1 | B22 | B1 |
| H8-818 | O | C-A1 | C-A1 | C-A1 | C-A1 | B22 | B6 |
| H8-819 | O | C-A1 | C-A1 | C-A1 | C-A1 | B22 | B20 |
| H8-820 | O | C-A1 | C-A1 | C-A1 | C-A1 | B22 | B21 |
| H8-821 | O | C-A1 | C-A1 | C-A1 | C-A1 | B22 | B22 |
| H8-822 | O | C-A1 | C-A1 | C-A1 | C-A1 | B22 | B32 |
| H8-823 | O | C-A1 | C-A1 | C-A1 | C-A1 | B22 | B33 |
| H8-824 | O | C-A1 | C-A1 | C-A1 | C-A1 | B22 | B34 |
| H8-825 | O | C-A1 | C-A1 | C-A1 | C-A1 | B22 | B68 |
| H8-826 | O | C-A1 | C-A1 | C-A1 | C-A1 | B22 | B69 |
| H8-827 | O | C-A1 | C-A1 | C-A1 | C-A1 | B22 | B70 |
| H8-828 | O | C-A1 | C-A1 | C-A1 | C-A1 | B22 | B71 |
| H8-829 | O | C-A1 | C-A1 | C-A1 | C-A1 | B22 | B81 |
| H8-830 | O | C-A1 | C-A1 | C-A1 | C-A1 | B22 | B90 |
| H8-831 | O | C-A1 | C-A1 | C-A1 | C-A1 | B22 | B91 |
| H8-832 | O | C-A1 | C-A1 | C-A1 | C-A1 | B22 | B92 |
| H8-833 | O | C-A1 | C-A1 | C-A1 | C-A1 | B22 | B93 |
| H8-834 | O | C-A1 | C-A1 | C-A1 | C-A1 | B22 | B94 |
| H8-835 | O | C-A1 | C-A1 | C-A1 | C-A1 | B22 | B95 |
| H8-836 | O | C-A1 | C-A1 | C-A1 | C-A1 | B22 | B96 |
| H8-837 | O | C-A1 | C-A1 | C-A1 | C-A1 | B22 | B97 |
| H8-838 | O | C-A1 | C-A1 | C-A1 | C-A1 | B22 | B106 |
| H8-839 | O | C-A1 | C-A1 | C-A1 | C-A1 | B22 | B107 |
| H8-840 | O | C-A1 | C-A1 | C-A1 | C-A1 | B22 | B114 |
| H8-841 | O | C-A1 | C-A1 | C-A1 | C-A1 | B22 | B115 |
| H8-842 | O | C-A1 | C-A1 | C-A1 | C-A1 | B22 | B116 |
| H8-843 | O | C-A1 | C-A1 | C-A1 | C-A1 | B22 | B117 |
| H8-844 | O | C-A1 | C-A1 | C-A1 | C-A1 | B22 | B118 |
| H8-845 | O | C-A1 | C-A1 | C-A1 | C-A1 | B22 | B119 |
| H8-846 | O | C-A1 | C-A1 | C-A1 | C-A1 | B22 | B120 |
| H8-847 | O | C-A1 | C-A1 | C-A1 | C-A1 | B22 | B121 |
| H8-848 | O | C-A1 | C-A1 | C-A1 | C-A1 | B22 | B122 |
| H8-849 | O | C-A1 | C-A1 | C-A1 | C-A1 | B22 | B125 |
| H8-850 | O | C-A1 | C-A1 | C-A1 | C-A1 | B22 | B126 |
| H8-851 | O | C-A1 | C-A1 | C-A1 | C-A1 | B22 | B127 |
| H8-852 | O | C-A1 | C-A1 | C-A1 | C-A1 | B22 | B128 |
| H8-853 | O | C-A1 | C-A1 | C-A1 | C-A1 | B22 | B129 |
| H8-854 | O | C-A1 | C-A1 | C-A1 | C-A1 | B22 | B130 |
| H8-855 | O | C-A1 | C-A1 | C-A1 | C-A1 | B22 | B131 |
| H8-856 | O | C-A1 | C-A1 | C-A1 | C-A1 | B22 | B132 |
| H8-857 | O | C-A1 | C-A1 | C-A1 | C-A1 | B22 | B133 |
| H8-858 | O | C-A1 | C-A1 | C-A1 | C-A1 | B22 | B134 |
| H8-859 | O | C-A1 | C-A1 | C-A1 | C-A1 | B22 | B135 |
| H8-860 | O | C-A1 | C-A1 | C-A1 | C-A1 | B22 | B136 |
| H8-861 | O | C-A1 | C-A1 | C-A1 | C-A1 | B22 | B137 |
| H8-862 | O | C-A1 | C-A1 | C-A1 | C-A1 | B22 | B138 |
| H8-863 | O | C-A1 | C-A1 | C-A1 | C-A1 | B22 | B139 |
| H8-864 | O | C-A1 | C-A1 | C-A1 | C-A1 | B22 | B140 |
| H8-871 | O | C-A1 | C-A2 | C-A1 | C-A2 | B22 | B1 |
| H8-872 | O | C-A1 | C-A2 | C-A1 | C-A2 | B22 | B6 |
| H8-873 | O | C-A1 | C-A2 | C-A1 | C-A2 | B22 | B20 |
| H8-874 | O | C-A1 | C-A2 | C-A1 | C-A2 | B22 | B21 |
| H8-875 | O | C-A1 | C-A2 | C-A1 | C-A2 | B22 | B22 |
| H8-876 | O | C-A1 | C-A2 | C-A1 | C-A2 | B22 | B32 |
| H8-877 | O | C-A1 | C-A2 | C-A1 | C-A2 | B22 | B33 |
| H8-878 | O | C-A1 | C-A2 | C-A1 | C-A2 | B22 | B34 |
| H8-879 | O | C-A1 | C-A2 | C-A1 | C-A2 | B22 | B68 |
| H8-880 | O | C-A1 | C-A2 | C-A1 | C-A2 | B22 | B69 |
| H8-881 | O | C-A1 | C-A2 | C-A1 | C-A2 | B22 | B70 |
| H8-882 | O | C-A1 | C-A2 | C-A1 | C-A2 | B22 | B71 |
| H8-883 | O | C-A1 | C-A2 | C-A1 | C-A2 | B22 | B81 |
| H8-884 | O | C-A1 | C-A2 | C-A1 | C-A2 | B22 | B90 |
| H8-885 | O | C-A1 | C-A2 | C-A1 | C-A2 | B22 | B91 |
| H8-886 | O | C-A1 | C-A2 | C-A1 | C-A2 | B22 | B92 |
| H8-887 | O | C-A1 | C-A2 | C-A1 | C-A2 | B22 | B93 |
| H8-888 | O | C-A1 | C-A2 | C-A1 | C-A2 | B22 | B94 |
| H8-889 | O | C-A1 | C-A2 | C-A1 | C-A2 | B22 | B95 |
| H8-890 | O | C-A1 | C-A2 | C-A1 | C-A2 | B22 | B96 |
| H8-891 | O | C-A1 | C-A2 | C-A1 | C-A2 | B22 | B97 |
| H8-892 | O | C-A1 | C-A2 | C-A1 | C-A2 | B22 | B106 |
| H8-893 | O | C-A1 | C-A2 | C-A1 | C-A2 | B22 | B107 |
| H8-894 | O | C-A1 | C-A2 | C-A1 | C-A2 | B22 | B114 |
| H8-895 | O | C-A1 | C-A2 | C-A1 | C-A2 | B22 | B115 |
| H8-896 | O | C-A1 | C-A2 | C-A1 | C-A2 | B22 | B116 |
| H8-897 | O | C-A1 | C-A2 | C-A1 | C-A2 | B22 | B117 |
| H8-898 | O | C-A1 | C-A2 | C-A1 | C-A2 | B22 | B118 |
| H8-899 | O | C-A1 | C-A2 | C-A1 | C-A2 | B22 | B119 |
| H8-900 | O | C-A1 | C-A2 | C-A1 | C-A2 | B22 | B120 |
| H8-901 | O | C-A1 | C-A2 | C-A1 | C-A2 | B22 | B121 |
| H8-902 | O | C-A1 | C-A2 | C-A1 | C-A2 | B22 | B122 |
| H8-903 | O | C-A1 | C-A2 | C-A1 | C-A2 | B22 | B125 |
| H8-904 | O | C-A1 | C-A2 | C-A1 | C-A2 | B22 | B126 |
| H8-905 | O | C-A1 | C-A2 | C-A1 | C-A2 | B22 | B127 |
| H8-906 | O | C-A1 | C-A2 | C-A1 | C-A2 | B22 | B128 |
| H8-907 | O | C-A1 | C-A2 | C-A1 | C-A2 | B22 | B129 |
| H8-908 | O | C-A1 | C-A2 | C-A1 | C-A2 | B22 | B130 |
| H8-909 | O | C-A1 | C-A2 | C-A1 | C-A2 | B22 | B131 |
| H8-910 | O | C-A1 | C-A2 | C-A1 | C-A2 | B22 | B132 |
| H8-911 | O | C-A1 | C-A2 | C-A1 | C-A2 | B22 | B133 |
| H8-912 | O | C-A1 | C-A2 | C-A1 | C-A2 | B22 | B134 |
| H8-913 | O | C-A1 | C-A2 | C-A1 | C-A2 | B22 | B135 |
| H8-914 | O | C-A1 | C-A2 | C-A1 | C-A2 | B22 | B136 |
| H8-915 | O | C-A1 | C-A2 | C-A1 | C-A2 | B22 | B137 |
| H8-916 | O | C-A1 | C-A2 | C-A1 | C-A2 | B22 | B138 |
| H8-917 | O | C-A1 | C-A2 | C-A1 | C-A2 | B22 | B139 |
| H8-918 | O | C-A1 | C-A2 | C-A1 | C-A2 | B22 | B140 |
| H8-925 | O | C-A1 | C-A3 | C-A1 | C-A3 | B22 | B1 |
| H8-926 | O | C-A1 | C-A3 | C-A1 | C-A3 | B22 | B6 |
| H8-927 | O | C-A1 | C-A3 | C-A1 | C-A3 | B22 | B20 |
| H8-928 | O | C-A1 | C-A3 | C-A1 | C-A3 | B22 | B21 |
| H8-929 | O | C-A1 | C-A3 | C-A1 | C-A3 | B22 | B22 |
| H8-930 | O | C-A1 | C-A3 | C-A1 | C-A3 | B22 | B32 |
| H8-931 | O | C-A1 | C-A3 | C-A1 | C-A3 | B22 | B33 |
| H8-932 | O | C-A1 | C-A3 | C-A1 | C-A3 | B22 | B34 |
| H8-933 | O | C-A1 | C-A3 | C-A1 | C-A3 | B22 | B68 |
| H8-934 | O | C-A1 | C-A3 | C-A1 | C-A3 | B22 | B69 |
| H8-935 | O | C-A1 | C-A3 | C-A1 | C-A3 | B22 | B70 |
| H8-936 | O | C-A1 | C-A3 | C-A1 | C-A3 | B22 | B71 |
| H8-937 | O | C-A1 | C-A3 | C-A1 | C-A3 | B22 | B81 |
| H8-938 | O | C-A1 | C-A3 | C-A1 | C-A3 | B22 | B90 |
| H8-939 | O | C-A1 | C-A3 | C-A1 | C-A3 | B22 | B91 |
| H8-940 | O | C-A1 | C-A3 | C-A1 | C-A3 | B22 | B92 |
| H8-941 | O | C-A1 | C-A3 | C-A1 | C-A3 | B22 | B93 |
| H8-942 | O | C-A1 | C-A3 | C-A1 | C-A3 | B22 | B94 |
| H8-943 | O | C-A1 | C-A3 | C-A1 | C-A3 | B22 | B95 |
| H8-944 | O | C-A1 | C-A3 | C-A1 | C-A3 | B22 | B96 |
| H8-945 | O | C-A1 | C-A3 | C-A1 | C-A3 | B22 | B97 |
| H8-946 | O | C-A1 | C-A3 | C-A1 | C-A3 | B22 | B106 |
| H8-947 | O | C-A1 | C-A3 | C-A1 | C-A3 | B22 | B107 |
| H8-948 | O | C-A1 | C-A3 | C-A1 | C-A3 | B22 | B114 |
| H8-949 | O | C-A1 | C-A3 | C-A1 | C-A3 | B22 | B115 |
| H8-950 | O | C-A1 | C-A3 | C-A1 | C-A3 | B22 | B116 |
| H8-951 | O | C-A1 | C-A3 | C-A1 | C-A3 | B22 | B117 |
| H8-952 | O | C-A1 | C-A3 | C-A1 | C-A3 | B22 | B118 |
| H8-953 | O | C-A1 | C-A3 | C-A1 | C-A3 | B22 | B119 |
| H8-954 | O | C-A1 | C-A3 | C-A1 | C-A3 | B22 | B120 |
| H8-955 | O | C-A1 | C-A3 | C-A1 | C-A3 | B22 | B121 |
| H8-956 | O | C-A1 | C-A3 | C-A1 | C-A3 | B22 | B122 |
| H8-957 | O | C-A1 | C-A3 | C-A1 | C-A3 | B22 | B125 |
| H8-958 | O | C-A1 | C-A3 | C-A1 | C-A3 | B22 | B126 |
| H8-959 | O | C-A1 | C-A3 | C-A1 | C-A3 | B22 | B127 |
| H8-960 | O | C-A1 | C-A3 | C-A1 | C-A3 | B22 | B128 |
| H8-961 | O | C-A1 | C-A3 | C-A1 | C-A3 | B22 | B129 |
| H8-962 | O | C-A1 | C-A3 | C-A1 | C-A3 | B22 | B130 |
| H8-963 | O | C-A1 | C-A3 | C-A1 | C-A3 | B22 | B131 |
| H8-964 | O | C-A1 | C-A3 | C-A1 | C-A3 | B22 | B132 |
| H8-965 | O | C-A1 | C-A3 | C-A1 | C-A3 | B22 | B133 |
| H8-966 | O | C-A1 | C-A3 | C-A1 | C-A3 | B22 | B134 |
| H8-967 | O | C-A1 | C-A3 | C-A1 | C-A3 | B22 | B135 |
| H8-968 | O | C-A1 | C-A3 | C-A1 | C-A3 | B22 | B136 |
| H8-969 | O | C-A1 | C-A3 | C-A1 | C-A3 | B22 | B137 |
| H8-970 | O | C-A1 | C-A3 | C-A1 | C-A3 | B22 | B138 |
| H8-971 | O | C-A1 | C-A3 | C-A1 | C-A3 | B22 | B139 |
| H8-972 | O | C-A1 | C-A3 | C-A1 | C-A3 | B22 | B140 |
| H8-979 | O | C-A3 | C-A1 | C-A3 | C-A1 | B22 | B1 |
| H8-980 | O | C-A3 | C-A1 | C-A3 | C-A1 | B22 | B6 |
| H8-981 | O | C-A3 | C-A1 | C-A3 | C-A1 | B22 | B20 |
| H8-982 | O | C-A3 | C-A1 | C-A3 | C-A1 | B22 | B21 |
| H8-983 | O | C-A3 | C-A1 | C-A3 | C-A1 | B22 | B22 |
| H8-984 | O | C-A3 | C-A1 | C-A3 | C-A1 | B22 | B32 |
| H8-985 | O | C-A3 | C-A1 | C-A3 | C-A1 | B22 | B33 |
| H8-986 | O | C-A3 | C-A1 | C-A3 | C-A1 | B22 | B34 |
| H8-987 | O | C-A3 | C-A1 | C-A3 | C-A1 | B22 | B68 |
| H8-988 | O | C-A3 | C-A1 | C-A3 | C-A1 | B22 | B69 |
| H8-989 | O | C-A3 | C-A1 | C-A3 | C-A1 | B22 | B70 |
| H8-990 | O | C-A3 | C-A1 | C-A3 | C-A1 | B22 | B71 |
| H8-991 | O | C-A3 | C-A1 | C-A3 | C-A1 | B22 | B81 |
| H8-992 | O | C-A3 | C-A1 | C-A3 | C-A1 | B22 | B90 |
| H8-993 | O | C-A3 | C-A1 | C-A3 | C-A1 | B22 | B91 |
| H8-994 | O | C-A3 | C-A1 | C-A3 | C-A1 | B22 | B92 |
| H8-995 | O | C-A3 | C-A1 | C-A3 | C-A1 | B22 | B93 |
| H8-996 | O | C-A3 | C-A1 | C-A3 | C-A1 | B22 | B94 |
| H8-997 | O | C-A3 | C-A1 | C-A3 | C-A1 | B22 | B95 |
| H8-998 | O | C-A3 | C-A1 | C-A3 | C-A1 | B22 | B96 |
| H8-999 | O | C-A3 | C-A1 | C-A3 | C-A1 | B22 | B97 |
| H8-1000 | O | C-A3 | C-A1 | C-A3 | C-A1 | B22 | B106 |
| H8-1001 | O | C-A3 | C-A1 | C-A3 | C-A1 | B22 | B107 |
| H8-1002 | O | C-A3 | C-A1 | C-A3 | C-A1 | B22 | B114 |
| H8-1003 | O | C-A3 | C-A1 | C-A3 | C-A1 | B22 | B115 |
| H8-1004 | O | C-A3 | C-A1 | C-A3 | C-A1 | B22 | B116 |
| H8-1005 | O | C-A3 | C-A1 | C-A3 | C-A1 | B22 | B117 |
| H8-1006 | O | C-A3 | C-A1 | C-A3 | C-A1 | B22 | B118 |
| H8-1007 | O | C-A3 | C-A1 | C-A3 | C-A1 | B22 | B119 |
| H8-1008 | O | C-A3 | C-A1 | C-A3 | C-A1 | B22 | B120 |
| H8-1009 | O | C-A3 | C-A1 | C-A3 | C-A1 | B22 | B121 |
| H8-1010 | O | C-A3 | C-A1 | C-A3 | C-A1 | B22 | B122 |
| H8-1011 | O | C-A3 | C-A1 | C-A3 | C-A1 | B22 | B125 |
| H8-1012 | O | C-A3 | C-A1 | C-A3 | C-A1 | B22 | B126 |
| H8-1013 | O | C-A3 | C-A1 | C-A3 | C-A1 | B22 | B127 |
| H8-1014 | O | C-A3 | C-A1 | C-A3 | C-A1 | B22 | B128 |
| H8-1015 | O | C-A3 | C-A1 | C-A3 | C-A1 | B22 | B129 |
| H8-1016 | O | C-A3 | C-A1 | C-A3 | C-A1 | B22 | B130 |
| H8-1017 | O | C-A3 | C-A1 | C-A3 | C-A1 | B22 | B131 |
| H8-1018 | O | C-A3 | C-A1 | C-A3 | C-A1 | B22 | B132 |
| H8-1019 | O | C-A3 | C-A1 | C-A3 | C-A1 | B22 | B133 |
| H8-1020 | O | C-A3 | C-A1 | C-A3 | C-A1 | B22 | B134 |
| H8-1021 | O | C-A3 | C-A1 | C-A3 | C-A1 | B22 | B135 |
| H8-1022 | O | C-A3 | C-A1 | C-A3 | C-A1 | B22 | B136 |
| H8-1023 | O | C-A3 | C-A1 | C-A3 | C-A1 | B22 | B137 |
| H8-1024 | O | C-A3 | C-A1 | C-A3 | C-A1 | B22 | B138 |
| H8-1025 | O | C-A3 | C-A1 | C-A3 | C-A1 | B22 | B139 |
| H8-1026 | O | C-A3 | C-A1 | C-A3 | C-A1 | B22 | B140 |
| H8-1033 | O | C-A1 | C-A1 | C-A3 | C-A1 | B22 | B1 |
| H8-1034 | O | C-A1 | C-A1 | C-A3 | C-A1 | B22 | B6 |
| H8-1035 | O | C-A1 | C-A1 | C-A3 | C-A1 | B22 | B20 |
| H8-1036 | O | C-A1 | C-A1 | C-A3 | C-A1 | B22 | B21 |
| H8-1037 | O | C-A1 | C-A1 | C-A3 | C-A1 | B22 | B22 |
| H8-1038 | O | C-A1 | C-A1 | C-A3 | C-A1 | B22 | B32 |
| H8-1039 | O | C-A1 | C-A1 | C-A3 | C-A1 | B22 | B33 |
| H8-1040 | O | C-A1 | C-A1 | C-A3 | C-A1 | B22 | B34 |
| H8-1041 | O | C-A1 | C-A1 | C-A3 | C-A1 | B22 | B68 |
| H8-1042 | O | C-A1 | C-A1 | C-A3 | C-A1 | B22 | B69 |
| H8-1043 | O | C-A1 | C-A1 | C-A3 | C-A1 | B22 | B70 |
| H8-1044 | O | C-A1 | C-A1 | C-A3 | C-A1 | B22 | B71 |
| H8-1045 | O | C-A1 | C-A1 | C-A3 | C-A1 | B22 | B81 |
| H8-1046 | O | C-A1 | C-A1 | C-A3 | C-A1 | B22 | B90 |
| H8-1047 | O | C-A1 | C-A1 | C-A3 | C-A1 | B22 | B91 |
| H8-1048 | O | C-A1 | C-A1 | C-A3 | C-A1 | B22 | B92 |
| H8-1049 | O | C-A1 | C-A1 | C-A3 | C-A1 | B22 | B93 |
| H8-1050 | O | C-A1 | C-A1 | C-A3 | C-A1 | B22 | B94 |
| H8-1051 | O | C-A1 | C-A1 | C-A3 | C-A1 | B22 | B95 |
| H8-1052 | O | C-A1 | C-A1 | C-A3 | C-A1 | B22 | B96 |
| H8-1053 | O | C-A1 | C-A1 | C-A3 | C-A1 | B22 | B97 |
| H8-1054 | O | C-A1 | C-A1 | C-A3 | C-A1 | B22 | B106 |
| H8-1055 | O | C-A1 | C-A1 | C-A3 | C-A1 | B22 | B107 |
| H8-1056 | O | C-A1 | C-A1 | C-A3 | C-A1 | B22 | B114 |
| H8-1057 | O | C-A1 | C-A1 | C-A3 | C-A1 | B22 | B115 |
| H8-1058 | O | C-A1 | C-A1 | C-A3 | C-A1 | B22 | B116 |
| H8-1059 | O | C-A1 | C-A1 | C-A3 | C-A1 | B22 | B117 |
| H8-1060 | O | C-A1 | C-A1 | C-A3 | C-A1 | B22 | B118 |
| H8-1061 | O | C-A1 | C-A1 | C-A3 | C-A1 | B22 | B119 |
| H8-1062 | O | C-A1 | C-A1 | C-A3 | C-A1 | B22 | B120 |
| H8-1063 | O | C-A1 | C-A1 | C-A3 | C-A1 | B22 | B121 |
| H8-1064 | O | C-A1 | C-A1 | C-A3 | C-A1 | B22 | B122 |
| H8-1065 | O | C-A1 | C-A1 | C-A3 | C-A1 | B22 | B125 |
| H8-1066 | O | C-A1 | C-A1 | C-A3 | C-A1 | B22 | B126 |
| H8-1067 | O | C-A1 | C-A1 | C-A3 | C-A1 | B22 | B127 |
| H8-1068 | O | C-A1 | C-A1 | C-A3 | C-A1 | B22 | B128 |
| H8-1069 | O | C-A1 | C-A1 | C-A3 | C-A1 | B22 | B129 |
| H8-1070 | O | C-A1 | C-A1 | C-A3 | C-A1 | B22 | B130 |
| H8-1071 | O | C-A1 | C-A1 | C-A3 | C-A1 | B22 | B131 |
| H8-1072 | O | C-A1 | C-A1 | C-A3 | C-A1 | B22 | B132 |
| H8-1073 | O | C-A1 | C-A1 | C-A3 | C-A1 | B22 | B133 |
| H8-1074 | O | C-A1 | C-A1 | C-A3 | C-A1 | B22 | B134 |
| H8-1075 | O | C-A1 | C-A1 | C-A3 | C-A1 | B22 | B135 |
| H8-1076 | O | C-A1 | C-A1 | C-A3 | C-A1 | B22 | B136 |
| H8-1077 | O | C-A1 | C-A1 | C-A3 | C-A1 | B22 | B137 |
| H8-1078 | O | C-A1 | C-A1 | C-A3 | C-A1 | B22 | B138 |
| H8-1079 | O | C-A1 | C-A1 | C-A3 | C-A1 | B22 | B139 |
| H8-1080 | O | C-A1 | C-A1 | C-A3 | C-A1 | B22 | B140 |
| H8-1087 | O | C-A1 | C-A1 | C-A1 | C-A1 | B32 | B1 |
| H8-1088 | O | C-A1 | C-A1 | C-A1 | C-A1 | B32 | B6 |
| H8-1089 | O | C-A1 | C-A1 | C-A1 | C-A1 | B32 | B20 |
| H8-1090 | O | C-A1 | C-A1 | C-A1 | C-A1 | B32 | B21 |
| H8-1091 | O | C-A1 | C-A1 | C-A1 | C-A1 | B32 | B22 |
| H8-1092 | O | C-A1 | C-A1 | C-A1 | C-A1 | B32 | B32 |
| H8-1093 | O | C-A1 | C-A1 | C-A1 | C-A1 | B32 | B33 |
| H8-1094 | O | C-A1 | C-A1 | C-A1 | C-A1 | B32 | B34 |
| H8-1095 | O | C-A1 | C-A1 | C-A1 | C-A1 | B32 | B68 |
| H8-1096 | O | C-A1 | C-A1 | C-A1 | C-A1 | B32 | B69 |
| H8-1097 | O | C-A1 | C-A1 | C-A1 | C-A1 | B32 | B70 |
| H8-1098 | O | C-A1 | C-A1 | C-A1 | C-A1 | B32 | B71 |
| H8-1099 | O | C-A1 | C-A1 | C-A1 | C-A1 | B32 | B81 |
| H8-1100 | O | C-A1 | C-A1 | C-A1 | C-A1 | B32 | B90 |
| H8-1101 | O | C-A1 | C-A1 | C-A1 | C-A1 | B32 | B91 |
| H8-1102 | O | C-A1 | C-A1 | C-A1 | C-A1 | B32 | B92 |
| H8-1103 | O | C-A1 | C-A1 | C-A1 | C-A1 | B32 | B93 |
| H8-1104 | O | C-A1 | C-A1 | C-A1 | C-A1 | B32 | B94 |
| H8-1105 | O | C-A1 | C-A1 | C-A1 | C-A1 | B32 | B95 |
| H8-1106 | O | C-A1 | C-A1 | C-A1 | C-A1 | B32 | B96 |
| H8-1107 | O | C-A1 | C-A1 | C-A1 | C-A1 | B32 | B97 |
| H8-1108 | O | C-A1 | C-A1 | C-A1 | C-A1 | B32 | B106 |
| H8-1109 | O | C-A1 | C-A1 | C-A1 | C-A1 | B32 | B107 |
| H8-1110 | O | C-A1 | C-A1 | C-A1 | C-A1 | B32 | B114 |
| H8-1111 | O | C-A1 | C-A1 | C-A1 | C-A1 | B32 | B115 |
| H8-1112 | O | C-A1 | C-A1 | C-A1 | C-A1 | B32 | B116 |
| H8-1113 | O | C-A1 | C-A1 | C-A1 | C-A1 | B32 | B117 |
| H8-1114 | O | C-A1 | C-A1 | C-A1 | C-A1 | B32 | B118 |
| H8-1115 | O | C-A1 | C-A1 | C-A1 | C-A1 | B32 | B119 |
| H8-1116 | O | C-A1 | C-A1 | C-A1 | C-A1 | B32 | B120 |
| H8-1117 | O | C-A1 | C-A1 | C-A1 | C-A1 | B32 | B121 |
| H8-1118 | O | C-A1 | C-A1 | C-A1 | C-A1 | B32 | B122 |
| H8-1119 | O | C-A1 | C-A1 | C-A1 | C-A1 | B32 | B125 |
| H8-1120 | O | C-A1 | C-A1 | C-A1 | C-A1 | B32 | B126 |
| H8-1121 | O | C-A1 | C-A1 | C-A1 | C-A1 | B32 | B127 |
| H8-1122 | O | C-A1 | C-A1 | C-A1 | C-A1 | B32 | B128 |
| H8-1123 | O | C-A1 | C-A1 | C-A1 | C-A1 | B32 | B129 |
| H8-1124 | O | C-A1 | C-A1 | C-A1 | C-A1 | B32 | B130 |
| H8-1125 | O | C-A1 | C-A1 | C-A1 | C-A1 | B32 | B131 |
| H8-1126 | O | C-A1 | C-A1 | C-A1 | C-A1 | B32 | B132 |
| H8-1127 | O | C-A1 | C-A1 | C-A1 | C-A1 | B32 | B133 |
| H8-1128 | O | C-A1 | C-A1 | C-A1 | C-A1 | B32 | B134 |
| H8-1129 | O | C-A1 | C-A1 | C-A1 | C-A1 | B32 | B135 |
| H8-1130 | O | C-A1 | C-A1 | C-A1 | C-A1 | B32 | B136 |
| H8-1131 | O | C-A1 | C-A1 | C-A1 | C-A1 | B32 | B137 |
| H8-1132 | O | C-A1 | C-A1 | C-A1 | C-A1 | B32 | B138 |
| H8-1133 | O | C-A1 | C-A1 | C-A1 | C-A1 | B32 | B139 |
| H8-1134 | O | C-A1 | C-A1 | C-A1 | C-A1 | B32 | B140 |
| H8-1141 | O | C-A1 | C-A2 | C-A1 | C-A2 | B32 | B1 |
| H8-1142 | O | C-A1 | C-A2 | C-A1 | C-A2 | B32 | B6 |
| H8-1143 | O | C-A1 | C-A2 | C-A1 | C-A2 | B32 | B20 |
| H8-1144 | O | C-A1 | C-A2 | C-A1 | C-A2 | B32 | B21 |
| H8-1145 | O | C-A1 | C-A2 | C-A1 | C-A2 | B32 | B22 |
| H8-1146 | O | C-A1 | C-A2 | C-A1 | C-A2 | B32 | B32 |
| H8-1147 | O | C-A1 | C-A2 | C-A1 | C-A2 | B32 | B33 |
| H8-1148 | O | C-A1 | C-A2 | C-A1 | C-A2 | B32 | B34 |
| H8-1149 | O | C-A1 | C-A2 | C-A1 | C-A2 | B32 | B68 |
| H8-1150 | O | C-A1 | C-A2 | C-A1 | C-A2 | B32 | B69 |
| H8-1151 | O | C-A1 | C-A2 | C-A1 | C-A2 | B32 | B70 |
| H8-1152 | O | C-A1 | C-A2 | C-A1 | C-A2 | B32 | B71 |
| H8-1153 | O | C-A1 | C-A2 | C-A1 | C-A2 | B32 | B81 |
| H8-1154 | O | C-A1 | C-A2 | C-A1 | C-A2 | B32 | B90 |
| H8-1155 | O | C-A1 | C-A2 | C-A1 | C-A2 | B32 | B91 |
| H8-1156 | O | C-A1 | C-A2 | C-A1 | C-A2 | B32 | B92 |
| H8-1157 | O | C-A1 | C-A2 | C-A1 | C-A2 | B32 | B93 |
| H8-1158 | O | C-A1 | C-A2 | C-A1 | C-A2 | B32 | B94 |
| H8-1159 | O | C-A1 | C-A2 | C-A1 | C-A2 | B32 | B95 |
| H8-1160 | O | C-A1 | C-A2 | C-A1 | C-A2 | B32 | B96 |
| H8-1161 | O | C-A1 | C-A2 | C-A1 | C-A2 | B32 | B97 |
| H8-1162 | O | C-A1 | C-A2 | C-A1 | C-A2 | B32 | B106 |
| H8-1163 | O | C-A1 | C-A2 | C-A1 | C-A2 | B32 | B107 |
| H8-1164 | O | C-A1 | C-A2 | C-A1 | C-A2 | B32 | B114 |
| H8-1165 | O | C-A1 | C-A2 | C-A1 | C-A2 | B32 | B115 |
| H8-1166 | O | C-A1 | C-A2 | C-A1 | C-A2 | B32 | B116 |
| H8-1167 | O | C-A1 | C-A2 | C-A1 | C-A2 | B32 | B117 |
| H8-1168 | O | C-A1 | C-A2 | C-A1 | C-A2 | B32 | B118 |
| H8-1169 | O | C-A1 | C-A2 | C-A1 | C-A2 | B32 | B119 |
| H8-1170 | O | C-A1 | C-A2 | C-A1 | C-A2 | B32 | B120 |
| H8-1171 | O | C-A1 | C-A2 | C-A1 | C-A2 | B32 | B121 |
| H8-1172 | O | C-A1 | C-A2 | C-A1 | C-A2 | B32 | B122 |
| H8-1173 | O | C-A1 | C-A2 | C-A1 | C-A2 | B32 | B125 |
| H8-1174 | O | C-A1 | C-A2 | C-A1 | C-A2 | B32 | B126 |
| H8-1175 | O | C-A1 | C-A2 | C-A1 | C-A2 | B32 | B127 |
| H8-1176 | O | C-A1 | C-A2 | C-A1 | C-A2 | B32 | B128 |
| H8-1177 | O | C-A1 | C-A2 | C-A1 | C-A2 | B32 | B129 |
| H8-1178 | O | C-A1 | C-A2 | C-A1 | C-A2 | B32 | B130 |
| H8-1179 | O | C-A1 | C-A2 | C-A1 | C-A2 | B32 | B131 |
| H8-1180 | O | C-A1 | C-A2 | C-A1 | C-A2 | B32 | B132 |
| H8-1181 | O | C-A1 | C-A2 | C-A1 | C-A2 | B32 | B133 |
| H8-1182 | O | C-A1 | C-A2 | C-A1 | C-A2 | B32 | B134 |
| H8-1183 | O | C-A1 | C-A2 | C-A1 | C-A2 | B32 | B135 |
| H8-1184 | O | C-A1 | C-A2 | C-A1 | C-A2 | B32 | B136 |
| H8-1185 | O | C-A1 | C-A2 | C-A1 | C-A2 | B32 | B137 |
| H8-1186 | O | C-A1 | C-A2 | C-A1 | C-A2 | B32 | B138 |
| H8-1187 | O | C-A1 | C-A2 | C-A1 | C-A2 | B32 | B139 |
| H8-1188 | O | C-A1 | C-A2 | C-A1 | C-A2 | B32 | B140 |
| H8-1195 | O | C-A1 | C-A3 | C-A1 | C-A3 | B32 | B1 |
| H8-1196 | O | C-A1 | C-A3 | C-A1 | C-A3 | B32 | B6 |
| H8-1197 | O | C-A1 | C-A3 | C-A1 | C-A3 | B32 | B20 |
| H8-1198 | O | C-A1 | C-A3 | C-A1 | C-A3 | B32 | B21 |
| H8-1199 | O | C-A1 | C-A3 | C-A1 | C-A3 | B32 | B22 |
| H8-1200 | O | C-A1 | C-A3 | C-A1 | C-A3 | B32 | B32 |
| H8-1201 | O | C-A1 | C-A3 | C-A1 | C-A3 | B32 | B33 |
| H8-1202 | O | C-A1 | C-A3 | C-A1 | C-A3 | B32 | B34 |
| H8-1203 | O | C-A1 | C-A3 | C-A1 | C-A3 | B32 | B68 |
| H8-1204 | O | C-A1 | C-A3 | C-A1 | C-A3 | B32 | B69 |
| H8-1205 | O | C-A1 | C-A3 | C-A1 | C-A3 | B32 | B70 |
| H8-1206 | O | C-A1 | C-A3 | C-A1 | C-A3 | B32 | B71 |
| H8-1207 | O | C-A1 | C-A3 | C-A1 | C-A3 | B32 | B81 |
| H8-1208 | O | C-A1 | C-A3 | C-A1 | C-A3 | B32 | B90 |
| H8-1209 | O | C-A1 | C-A3 | C-A1 | C-A3 | B32 | B91 |
| H8-1210 | O | C-A1 | C-A3 | C-A1 | C-A3 | B32 | B92 |
| H8-1211 | O | C-A1 | C-A3 | C-A1 | C-A3 | B32 | B93 |
| H8-1212 | O | C-A1 | C-A3 | C-A1 | C-A3 | B32 | B94 |
| H8-1213 | O | C-A1 | C-A3 | C-A1 | C-A3 | B32 | B95 |
| H8-1214 | O | C-A1 | C-A3 | C-A1 | C-A3 | B32 | B96 |
| H8-1215 | O | C-A1 | C-A3 | C-A1 | C-A3 | B32 | B97 |
| H8-1216 | O | C-A1 | C-A3 | C-A1 | C-A3 | B32 | B106 |
| H8-1217 | O | C-A1 | C-A3 | C-A1 | C-A3 | B32 | B107 |
| H8-1218 | O | C-A1 | C-A3 | C-A1 | C-A3 | B32 | B114 |
| H8-1219 | O | C-A1 | C-A3 | C-A1 | C-A3 | B32 | B115 |
| H8-1220 | O | C-A1 | C-A3 | C-A1 | C-A3 | B32 | B116 |
| H8-1221 | O | C-A1 | C-A3 | C-A1 | C-A3 | B32 | B117 |
| H8-1222 | O | C-A1 | C-A3 | C-A1 | C-A3 | B32 | B118 |
| H8-1223 | O | C-A1 | C-A3 | C-A1 | C-A3 | B32 | B119 |
| H8-1224 | O | C-A1 | C-A3 | C-A1 | C-A3 | B32 | B120 |
| H8-1225 | O | C-A1 | C-A3 | C-A1 | C-A3 | B32 | B121 |
| H8-1226 | O | C-A1 | C-A3 | C-A1 | C-A3 | B32 | B122 |
| H8-1227 | O | C-A1 | C-A3 | C-A1 | C-A3 | B32 | B125 |
| H8-1228 | O | C-A1 | C-A3 | C-A1 | C-A3 | B32 | B126 |
| H8-1229 | O | C-A1 | C-A3 | C-A1 | C-A3 | B32 | B127 |
| H8-1230 | O | C-A1 | C-A3 | C-A1 | C-A3 | B32 | B128 |
| H8-1231 | O | C-A1 | C-A3 | C-A1 | C-A3 | B32 | B129 |
| H8-1232 | O | C-A1 | C-A3 | C-A1 | C-A3 | B32 | B130 |
| H8-1233 | O | C-A1 | C-A3 | C-A1 | C-A3 | B32 | B131 |
| H8-1234 | O | C-A1 | C-A3 | C-A1 | C-A3 | B32 | B132 |
| H8-1235 | O | C-A1 | C-A3 | C-A1 | C-A3 | B32 | B133 |
| H8-1236 | O | C-A1 | C-A3 | C-A1 | C-A3 | B32 | B134 |
| H8-1237 | O | C-A1 | C-A3 | C-A1 | C-A3 | B32 | B135 |
| H8-1238 | O | C-A1 | C-A3 | C-A1 | C-A3 | B32 | B136 |
| H8-1239 | O | C-A1 | C-A3 | C-A1 | C-A3 | B32 | B137 |
| H8-1240 | O | C-A1 | C-A3 | C-A1 | C-A3 | B32 | B138 |
| H8-1241 | O | C-A1 | C-A3 | C-A1 | C-A3 | B32 | B139 |
| H8-1242 | O | C-A1 | C-A3 | C-A1 | C-A3 | B32 | B140 |
| H8-1249 | O | C-A3 | C-A1 | C-A3 | C-A1 | B32 | B1 |
| H8-1250 | O | C-A3 | C-A1 | C-A3 | C-A1 | B32 | B6 |
| H8-1251 | O | C-A3 | C-A1 | C-A3 | C-A1 | B32 | B20 |
| H8-1252 | O | C-A3 | C-A1 | C-A3 | C-A1 | B32 | B21 |
| H8-1253 | O | C-A3 | C-A1 | C-A3 | C-A1 | B32 | B22 |
| H8-1254 | O | C-A3 | C-A1 | C-A3 | C-A1 | B32 | B32 |
| H8-1255 | O | C-A3 | C-A1 | C-A3 | C-A1 | B32 | B33 |
| H8-1256 | O | C-A3 | C-A1 | C-A3 | C-A1 | B32 | B34 |
| H8-1257 | O | C-A3 | C-A1 | C-A3 | C-A1 | B32 | B68 |
| H8-1258 | O | C-A3 | C-A1 | C-A3 | C-A1 | B32 | B69 |
| H8-1259 | O | C-A3 | C-A1 | C-A3 | C-A1 | B32 | B70 |
| H8-1260 | O | C-A3 | C-A1 | C-A3 | C-A1 | B32 | B71 |
| H8-1261 | O | C-A3 | C-A1 | C-A3 | C-A1 | B32 | B81 |
| H8-1262 | O | C-A3 | C-A1 | C-A3 | C-A1 | B32 | B90 |
| H8-1263 | O | C-A3 | C-A1 | C-A3 | C-A1 | B32 | B91 |
| H8-1264 | O | C-A3 | C-A1 | C-A3 | C-A1 | B32 | B92 |
| H8-1265 | O | C-A3 | C-A1 | C-A3 | C-A1 | B32 | B93 |
| H8-1266 | O | C-A3 | C-A1 | C-A3 | C-A1 | B32 | B94 |
| H8-1267 | O | C-A3 | C-A1 | C-A3 | C-A1 | B32 | B95 |
| H8-1268 | O | C-A3 | C-A1 | C-A3 | C-A1 | B32 | B96 |
| H8-1269 | O | C-A3 | C-A1 | C-A3 | C-A1 | B32 | B97 |
| H8-1270 | O | C-A3 | C-A1 | C-A3 | C-A1 | B32 | B106 |
| H8-1271 | O | C-A3 | C-A1 | C-A3 | C-A1 | B32 | B107 |
| H8-1272 | O | C-A3 | C-A1 | C-A3 | C-A1 | B32 | B114 |
| H8-1273 | O | C-A3 | C-A1 | C-A3 | C-A1 | B32 | B115 |
| H8-1274 | O | C-A3 | C-A1 | C-A3 | C-A1 | B32 | B116 |
| H8-1275 | O | C-A3 | C-A1 | C-A3 | C-A1 | B32 | B117 |
| H8-1276 | O | C-A3 | C-A1 | C-A3 | C-A1 | B32 | B118 |
| H8-1277 | O | C-A3 | C-A1 | C-A3 | C-A1 | B32 | B119 |
| H8-1278 | O | C-A3 | C-A1 | C-A3 | C-A1 | B32 | B 20 |
| H8-1279 | O | C-A3 | C-A1 | C-A3 | C-A1 | B32 | B121 |
| H8-1280 | O | C-A3 | C-A1 | C-A3 | C-A1 | B32 | B122 |
| H8-1281 | O | C-A3 | C-A1 | C-A3 | C-A1 | B32 | B125 |
| H8-1282 | O | C-A3 | C-A1 | C-A3 | C-A1 | B32 | B126 |
| H8-1283 | O | C-A3 | C-A1 | C-A3 | C-A1 | B32 | B127 |
| H8-1284 | O | C-A3 | C-A1 | C-A3 | C-A1 | B32 | B128 |
| H8-1285 | O | C-A3 | C-A1 | C-A3 | C-A1 | B32 | B129 |
| H8-1286 | O | C-A3 | C-A1 | C-A3 | C-A1 | B32 | B130 |
| H8-1287 | O | C-A3 | C-A1 | C-A3 | C-A1 | B32 | B131 |
| H8-1288 | O | C-A3 | C-A1 | C-A3 | C-A1 | B32 | B132 |
| H8-1289 | O | C-A3 | C-A1 | C-A3 | C-A1 | B32 | B133 |
| H8-1290 | O | C-A3 | C-A1 | C-A3 | C-A1 | B32 | B134 |
| H8-1291 | O | C-A3 | C-A1 | C-A3 | C-A1 | B32 | B135 |
| H8-1292 | O | C-A3 | C-A1 | C-A3 | C-A1 | B32 | B136 |
| H8-1293 | O | C-A3 | C-A1 | C-A3 | C-A1 | B32 | B137 |
| H8-1294 | O | C-A3 | C-A1 | C-A3 | C-A1 | B32 | B138 |
| H8-1295 | O | C-A3 | C-A1 | C-A3 | C-A1 | B32 | B139 |
| H8-1296 | O | C-A3 | C-A1 | C-A3 | C-A1 | B32 | B140 |
| H8-1303 | O | C-A1 | C-A1 | C-A3 | C-A1 | B32 | B1 |
| H8-1304 | O | C-A1 | C-A1 | C-A3 | C-A1 | B32 | B6 |
| H8-1305 | O | C-A1 | C-A1 | C-A3 | C-A1 | B32 | B20 |
| H8-1306 | O | C-A1 | C-A1 | C-A3 | C-A1 | B32 | B21 |
| H8-1307 | O | C-A1 | C-A1 | C-A3 | C-A1 | B32 | B22 |
| H8-1308 | O | C-A1 | C-A1 | C-A3 | C-A1 | B32 | B32 |
| H8-1309 | O | C-A1 | C-A1 | C-A3 | C-A1 | B32 | B33 |
| H8-1310 | O | C-A1 | C-A1 | C-A3 | C-A1 | B32 | B34 |
| H8-1311 | O | C-A1 | C-A1 | C-A3 | C-A1 | B32 | B68 |
| H8-1312 | O | C-A1 | C-A1 | C-A3 | C-A1 | B32 | B69 |
| H8-1313 | O | C-A1 | C-A1 | C-A3 | C-A1 | B32 | B70 |
| H8-1314 | O | C-A1 | C-A1 | C-A3 | C-A1 | B32 | B71 |
| H8-1315 | O | C-A1 | C-A1 | C-A3 | C-A1 | B32 | B81 |
| H8-1316 | O | C-A1 | C-A1 | C-A3 | C-A1 | B32 | B90 |
| H8-1317 | O | C-A1 | C-A1 | C-A3 | C-A1 | B32 | B91 |
| H8-1318 | O | C-A1 | C-A1 | C-A3 | C-A1 | B32 | B92 |
| H8-1319 | O | C-A1 | C-A1 | C-A3 | C-A1 | B32 | B93 |
| H8-1320 | O | C-A1 | C-A1 | C-A3 | C-A1 | B32 | B94 |
| H8-1321 | O | C-A1 | C-A1 | C-A3 | C-A1 | B32 | B95 |
| H8-1322 | O | C-A1 | C-A1 | C-A3 | C-A1 | B32 | B96 |
| H8-1323 | O | C-A1 | C-A1 | C-A3 | C-A1 | B32 | B97 |
| H8-1324 | O | C-A1 | C-A1 | C-A3 | C-A1 | B32 | B106 |
| H8-1325 | O | C-A1 | C-A1 | C-A3 | C-A1 | B32 | B107 |
| H8-1326 | O | C-A1 | C-A1 | C-A3 | C-A1 | B32 | B114 |
| H8-1327 | O | C-A1 | C-A1 | C-A3 | C-A1 | B32 | B115 |
| H8-1328 | O | C-A1 | C-A1 | C-A3 | C-A1 | B32 | B116 |
| H8-1329 | O | C-A1 | C-A1 | C-A3 | C-A1 | B32 | B117 |
| H8-1330 | O | C-A1 | C-A1 | C-A3 | C-A1 | B32 | B118 |
| H8-1331 | O | C-A1 | C-A1 | C-A3 | C-A1 | B32 | B119 |
| H8-1332 | O | C-A1 | C-A1 | C-A3 | C-A1 | B32 | B120 |
| H8-1333 | O | C-A1 | C-A1 | C-A3 | C-A1 | B32 | B121 |
| H8-1334 | O | C-A1 | C-A1 | C-A3 | C-A1 | B32 | B122 |
| H8-1335 | O | C-A1 | C-A1 | C-A3 | C-A1 | B32 | B125 |
| H8-1336 | O | C-A1 | C-A1 | C-A3 | C-A1 | B32 | B126 |
| H8-1337 | O | C-A1 | C-A1 | C-A3 | C-A1 | B32 | B127 |
| H8-1338 | O | C-A1 | C-A1 | C-A3 | C-A1 | B32 | B128 |
| H8-1339 | O | C-A1 | C-A1 | C-A3 | C-A1 | B32 | B129 |
| H8-1340 | O | C-A1 | C-A1 | C-A3 | C-A1 | B32 | B130 |
| H8-1341 | O | C-A1 | C-A1 | C-A3 | C-A1 | B32 | B131 |
| H8-1342 | O | C-A1 | C-A1 | C-A3 | C-A1 | B32 | B132 |
| H8-1343 | O | C-A1 | C-A1 | C-A3 | C-A1 | B32 | B133 |
| H8-1344 | O | C-A1 | C-A1 | C-A3 | C-A1 | B32 | B134 |
| H8-1345 | O | C-A1 | C-A1 | C-A3 | C-A1 | B32 | B135 |
| H8-1346 | O | C-A1 | C-A1 | C-A3 | C-A1 | B32 | B136 |
| H8-1347 | O | C-A1 | C-A1 | C-A3 | C-A1 | B32 | B137 |
| H8-1348 | O | C-A1 | C-A1 | C-A3 | C-A1 | B32 | B138 |
| H8-1349 | O | C-A1 | C-A1 | C-A3 | C-A1 | B32 | B139 |
| H8-1350 | O | C-A1 | C-A1 | C-A3 | C-A1 | B32 | B140 |
| H8-1357 | S | C-A1 | C-A1 | C-A1 | C-A1 | B20 | B1 |
| H8-1358 | S | C-A1 | C-A1 | C-A1 | C-A1 | B20 | B6 |
| H8-1359 | S | C-A1 | C-A1 | C-A1 | C-A1 | B20 | B20 |
| H8-1360 | S | C-A1 | C-A1 | C-A1 | C-A1 | B20 | B21 |
| H8-1361 | S | C-A1 | C-A1 | C-A1 | C-A1 | B20 | B22 |
| H8-1362 | S | C-A1 | C-A1 | C-A1 | C-A1 | B20 | B32 |
| H8-1363 | S | C-A1 | C-A1 | C-A1 | C-A1 | B20 | B33 |
| H8-1364 | S | C-A1 | C-A1 | C-A1 | C-A1 | B20 | B34 |
| H8-1365 | S | C-A1 | C-A1 | C-A1 | C-A1 | B20 | B68 |
| H8-1366 | S | C-A1 | C-A1 | C-A1 | C-A1 | B20 | B69 |
| H8-1367 | S | C-A1 | C-A1 | C-A1 | C-A1 | B20 | B70 |
| H8-1368 | S | C-A1 | C-A1 | C-A1 | C-A1 | B20 | B71 |
| H8-1369 | S | C-A1 | C-A1 | C-A1 | C-A1 | B20 | B81 |
| H8-1370 | S | C-A1 | C-A1 | C-A1 | C-A1 | B20 | B90 |
| H8-1371 | S | C-A1 | C-A1 | C-A1 | C-A1 | B20 | B91 |
| H8-1372 | S | C-A1 | C-A1 | C-A1 | C-A1 | B20 | B92 |
| H8-1373 | S | C-A1 | C-A1 | C-A1 | C-A1 | B20 | B93 |
| H8-1374 | S | C-A1 | C-A1 | C-A1 | C-A1 | B20 | B94 |
| H8-1375 | S | C-A1 | C-A1 | C-A1 | C-A1 | B20 | B95 |
| H8-1376 | S | C-A1 | C-A1 | C-A1 | C-A1 | B20 | B96 |
| H8-1377 | S | C-A1 | C-A1 | C-A1 | C-A1 | B20 | B97 |
| H8-1378 | S | C-A1 | C-A1 | C-A1 | C-A1 | B20 | B106 |
| H8-1379 | S | C-A1 | C-A1 | C-A1 | C-A1 | B20 | B107 |
| H8-1380 | S | C-A1 | C-A1 | C-A1 | C-A1 | B20 | B114 |
| H8-1381 | S | C-A1 | C-A1 | C-A1 | C-A1 | B20 | B115 |
| H8-1382 | S | C-A1 | C-A1 | C-A1 | C-A1 | B20 | B116 |
| H8-1383 | S | C-A1 | C-A1 | C-A1 | C-A1 | B20 | B117 |
| H8-1384 | S | C-A1 | C-A1 | C-A1 | C-A1 | B20 | B118 |
| H8-1385 | S | C-A1 | C-A1 | C-A1 | C-A1 | B20 | B119 |
| H8-1386 | S | C-A1 | C-A1 | C-A1 | C-A1 | B20 | B120 |
| H8-1387 | S | C-A1 | C-A1 | C-A1 | C-A1 | B20 | B121 |
| H8-1388 | S | C-A1 | C-A1 | C-A1 | C-A1 | B20 | B122 |
| H8-1389 | S | C-A1 | C-A1 | C-A1 | C-A1 | B20 | B125 |
| H8-1390 | S | C-A1 | C-A1 | C-A1 | C-A1 | B20 | B126 |
| H8-1391 | S | C-A1 | C-A1 | C-A1 | C-A1 | B20 | B127 |
| H8-1392 | S | C-A1 | C-A1 | C-A1 | C-A1 | B20 | B128 |
| H8-1393 | S | C-A1 | C-A1 | C-A1 | C-A1 | B20 | B129 |
| H8-1394 | S | C-A1 | C-A1 | C-A1 | C-A1 | B20 | B130 |
| H8-1395 | S | C-A1 | C-A1 | C-A1 | C-A1 | B20 | B131 |
| H8-1396 | S | C-A1 | C-A1 | C-A1 | C-A1 | B20 | B132 |
| H8-1397 | S | C-A1 | C-A1 | C-A1 | C-A1 | B20 | B133 |
| H8-1398 | S | C-A1 | C-A1 | C-A1 | C-A1 | B20 | B134 |
| H8-1399 | S | C-A1 | C-A1 | C-A1 | C-A1 | B20 | B135 |
| H8-1400 | S | C-A1 | C-A1 | C-A1 | C-A1 | B20 | B136 |
| H8-1401 | S | C-A1 | C-A1 | C-A1 | C-A1 | B20 | B137 |
| H8-1402 | S | C-A1 | C-A1 | C-A1 | C-A1 | B20 | B138 |
| H8-1403 | S | C-A1 | C-A1 | C-A1 | C-A1 | B20 | B139 |
| H8-1404 | S | C-A1 | C-A1 | C-A1 | C-A1 | B20 | B140 |
| H8-1411 | S | C-A1 | C-A1 | C-A1 | C-A1 | B68 | B1 |
| H8-1412 | S | C-A1 | C-A1 | C-A1 | C-A1 | B68 | B6 |
| H8-1413 | S | C-A1 | C-A1 | C-A1 | C-A1 | B68 | B20 |
| H8-1414 | S | C-A1 | C-A1 | C-A1 | C-A1 | B68 | B21 |
| H8-1415 | S | C-A1 | C-A1 | C-A1 | C-A1 | B68 | B22 |
| H8-1416 | S | C-A1 | C-A1 | C-A1 | C-A1 | B68 | B32 |
| H8-1417 | S | C-A1 | C-A1 | C-A1 | C-A1 | B68 | B33 |
| H8-1418 | S | C-A1 | C-A1 | C-A1 | C-A1 | B68 | B34 |
| H8-1419 | S | C-A1 | C-A1 | C-A1 | C-A1 | B68 | B68 |
| H8-1420 | S | C-A1 | C-A1 | C-A1 | C-A1 | B68 | B69 |
| H8-1421 | S | C-A1 | C-A1 | C-A1 | C-A1 | B68 | B70 |
| H8-1422 | S | C-A1 | C-A1 | C-A1 | C-A1 | B68 | B71 |
| H8-1423 | S | C-A1 | C-A1 | C-A1 | C-A1 | B68 | B81 |
| H8-1424 | S | C-A1 | C-A1 | C-A1 | C-A1 | B68 | B90 |
| H8-1425 | S | C-A1 | C-A1 | C-A1 | C-A1 | B68 | B91 |
| H8-1426 | S | C-A1 | C-A1 | C-A1 | C-A1 | B68 | B92 |
| H8-1427 | S | C-A1 | C-A1 | C-A1 | C-A1 | B68 | B93 |
| H8-1428 | S | C-A1 | C-A1 | C-A1 | C-A1 | B68 | B94 |
| H8-1429 | S | C-A1 | C-A1 | C-A1 | C-A1 | B68 | B95 |
| H8-1430 | S | C-A1 | C-A1 | C-A1 | C-A1 | B68 | B96 |
| H8-1431 | S | C-A1 | C-A1 | C-A1 | C-A1 | B68 | B97 |
| H8-1432 | S | C-A1 | C-A1 | C-A1 | C-A1 | B68 | B106 |
| H8-1433 | S | C-A1 | C-A1 | C-A1 | C-A1 | B68 | B107 |
| H8-1434 | S | C-A1 | C-A1 | C-A1 | C-A1 | B68 | B114 |
| H8-1435 | S | C-A1 | C-A1 | C-A1 | C-A1 | B68 | B115 |
| H8-1436 | S | C-A1 | C-A1 | C-A1 | C-A1 | B68 | B116 |
| H8-1437 | S | C-A1 | C-A1 | C-A1 | C-A1 | B68 | B117 |
| H8-1438 | S | C-A1 | C-A1 | C-A1 | C-A1 | B68 | B118 |
| H8-1439 | S | C-A1 | C-A1 | C-A1 | C-A1 | B68 | B119 |
| H8-1440 | S | C-A1 | C-A1 | C-A1 | C-A1 | B68 | B120 |
| H8-1441 | S | C-A1 | C-A1 | C-A1 | C-A1 | B68 | B121 |
| H8-1442 | S | C-A1 | C-A1 | C-A1 | C-A1 | B68 | B122 |
| H8-1443 | S | C-A1 | C-A1 | C-A1 | C-A1 | B68 | B125 |
| H8-1444 | S | C-A1 | C-A1 | C-A1 | C-A1 | B68 | B126 |
| H8-1445 | S | C-A1 | C-A1 | C-A1 | C-A1 | B68 | B127 |
| H8-1446 | S | C-A1 | C-A1 | C-A1 | C-A1 | B68 | B128 |
| H8-1447 | S | C-A1 | C-A1 | C-A1 | C-A1 | B68 | B129 |
| H8-1448 | S | C-A1 | C-A1 | C-A1 | C-A1 | B68 | B130 |
| H8-1449 | S | C-A1 | C-A1 | C-A1 | C-A1 | B68 | B131 |
| H8-1450 | S | C-A1 | C-A1 | C-A1 | C-A1 | B68 | B132 |
| H8-1451 | S | C-A1 | C-A1 | C-A1 | C-A1 | B68 | B133 |
| H8-1452 | S | C-A1 | C-A1 | C-A1 | C-A1 | B68 | B134 |
| H8-1453 | S | C-A1 | C-A1 | C-A1 | C-A1 | B68 | B135 |
| H8-1454 | S | C-A1 | C-A1 | C-A1 | C-A1 | B68 | B136 |
| H8-1455 | S | C-A1 | C-A1 | C-A1 | C-A1 | B68 | B137 |
| H8-1456 | S | C-A1 | C-A1 | C-A1 | C-A1 | B68 | B138 |
| H8-1457 | S | C-A1 | C-A1 | C-A1 | C-A1 | B68 | B139 |
| H8-1458 | S | C-A1 | C-A1 | C-A1 | C-A1 | B68 | B140 |
| H8-1465 | S | C-A1 | C-A1 | C-A1 | C-A1 | B70 | B1 |
| H8-1466 | S | C-A1 | C-A1 | C-A1 | C-A1 | B70 | B6 |
| H8-1467 | S | C-A1 | C-A1 | C-A1 | C-A1 | B70 | B20 |
| H8-1468 | S | C-A1 | C-A1 | C-A1 | C-A1 | B70 | B21 |
| H8-1469 | S | C-A1 | C-A1 | C-A1 | C-A1 | B70 | B22 |
| H8-1470 | S | C-A1 | C-A1 | C-A1 | C-A1 | B70 | B32 |
| H8-1471 | S | C-A1 | C-A1 | C-A1 | C-A1 | B70 | B33 |
| H8-1472 | S | C-A1 | C-A1 | C-A1 | C-A1 | B70 | B34 |
| H8-1473 | S | C-A1 | C-A1 | C-A1 | C-A1 | B70 | B68 |
| H8-1474 | S | C-A1 | C-A1 | C-A1 | C-A1 | B70 | B69 |
| H8-1475 | S | C-A1 | C-A1 | C-A1 | C-A1 | B70 | B70 |
| H8-1476 | S | C-A1 | C-A1 | C-A1 | C-A1 | B70 | B71 |
| H8-1477 | S | C-A1 | C-A1 | C-A1 | C-A1 | B70 | B81 |
| H8-1478 | S | C-A1 | C-A1 | C-A1 | C-A1 | B70 | B90 |
| H8-1479 | S | C-A1 | C-A1 | C-A1 | C-A1 | B70 | B91 |
| H8-1480 | S | C-A1 | C-A1 | C-A1 | C-A1 | B70 | B92 |
| H8-1481 | S | C-A1 | C-A1 | C-A1 | C-A1 | B70 | B93 |
| H8-1482 | S | C-A1 | C-A1 | C-A1 | C-A1 | B70 | B94 |
| H8-1483 | S | C-A1 | C-A1 | C-A1 | C-A1 | B70 | B95 |
| H8-1484 | S | C-A1 | C-A1 | C-A1 | C-A1 | B70 | B96 |
| H8-1485 | S | C-A1 | C-A1 | C-A1 | C-A1 | B70 | B97 |
| H8-1486 | S | C-A1 | C-A1 | C-A1 | C-A1 | B70 | B106 |
| H8-1487 | S | C-A1 | C-A1 | C-A1 | C-A1 | B70 | B107 |
| H8-1488 | S | C-A1 | C-A1 | C-A1 | C-A1 | B70 | B114 |
| H8-1489 | S | C-A1 | C-A1 | C-A1 | C-A1 | B70 | B115 |
| H8-1490 | S | C-A1 | C-A1 | C-A1 | C-A1 | B70 | B116 |
| H8-1491 | S | C-A1 | C-A1 | C-A1 | C-A1 | B70 | B117 |
| H8-1492 | S | C-A1 | C-A1 | C-A1 | C-A1 | B70 | B118 |
| H8-1493 | S | C-A1 | C-A1 | C-A1 | C-A1 | B70 | B119 |
| H8-1494 | S | C-A1 | C-A1 | C-A1 | C-A1 | B70 | B120 |
| H8-1495 | S | C-A1 | C-A1 | C-A1 | C-A1 | B70 | B121 |
| H8-1496 | S | C-A1 | C-A1 | C-A1 | C-A1 | B70 | B122 |
| H8-1497 | S | C-A1 | C-A1 | C-A1 | C-A1 | B70 | B125 |
| H8-1498 | S | C-A1 | C-A1 | C-A1 | C-A1 | B70 | B126 |
| H8-1499 | S | C-A1 | C-A1 | C-A1 | C-A1 | B70 | B127 |
| H8-1500 | S | C-A1 | C-A1 | C-A1 | C-A1 | B70 | B128 |
| H8-1501 | S | C-A1 | C-A1 | C-A1 | C-A1 | B70 | B129 |
| H8-1502 | S | C-A1 | C-A1 | C-A1 | C-A1 | B70 | B130 |
| H8-1503 | S | C-A1 | C-A1 | C-A1 | C-A1 | B70 | B131 |
| H8-1504 | S | C-A1 | C-A1 | C-A1 | C-A1 | B70 | B132 |
| H8-1505 | S | C-A1 | C-A1 | C-A1 | C-A1 | B70 | B133 |
| H8-1506 | S | C-A1 | C-A1 | C-A1 | C-A1 | B70 | B134 |
| H8-1507 | S | C-A1 | C-A1 | C-A1 | C-A1 | B70 | B135 |
| H8-1508 | S | C-A1 | C-A1 | C-A1 | C-A1 | B70 | B136 |
| H8-1509 | S | C-A1 | C-A1 | C-A1 | C-A1 | B70 | B137 |
| H8-1510 | S | C-A1 | C-A1 | C-A1 | C-A1 | B70 | B138 |
| H8-1511 | S | C-A1 | C-A1 | C-A1 | C-A1 | B70 | B139 |
| H8-1512 | S | C-A1 | C-A1 | C-A1 | C-A1 | B70 | B140 |

9. An electroluminescent device, comprising:
an anode,
a cathode and
an organic layer disposed between the anode and the cathode, wherein the organic layer comprises the compound according to any one of claims 1 to 8.

10. The electroluminescent device according to claim 9, wherein the organic layer is a hole injection layer or a hole transporting layer, and the hole injection layer or the hole transporting layer is formed by the compound alone.

11. The electroluminescent device according to claim 9, wherein the organic layer is a hole injection layer or a hole transporting layer, and the hole injection layer or the hole transporting layer further comprises at least one hole transporting material; wherein a molar doping ratio of the compound to the at least one hole transporting material is 10000: 1 to 1:10000;
preferably, the molar doping ratio of the compound to the at least one hole transporting material is 10:1 to 1:100.

12. The electroluminescent device according to claim 9, wherein the electroluminescent device comprises at least two light-emitting units and the organic layer is a charge generation layer and disposed between the at least two light-emitting units, wherein the charge generation layer comprises a p-type charge generation layer and an n-type charge generation layer;
preferably, the p-type charge generation layer comprises the compound;
more preferably, the p-type charge generation layer further comprises at least one hole transporting material, wherein a molar doping ratio of the compound to the at least one hole transporting material is 10000:1 to 1:10000; most preferably, the molar doping ratio of the compound to the at least one hole transporting material is 10:1 to 1:100.

13. The electroluminescent device according to claim 11 or 12, wherein the hole transporting material is selected from a compound having a triarylamine unit, a spirobifluorene compound, a pentacene compound, an oligothiophene compound, an oligophenyl compound, an oligophenylenevinylene compound, an oligofluorene compound, a porphyrin complex or a metallic phthalocyanine complex.

14. The electroluminescent device according to claim 12, wherein the charge generation layer further comprises a buffer layer disposed between the p-type charge generation layer and the n-type charge generation layer, and the buffer layer comprises the compound.

15. A compound composition, comprising the compound according to any one of claims 1 to 8.

## Patentansprüche

1. Eine Verbindung, die eine durch die Formeln 2-1 bis 2-10 dargestellte Struktur hat: 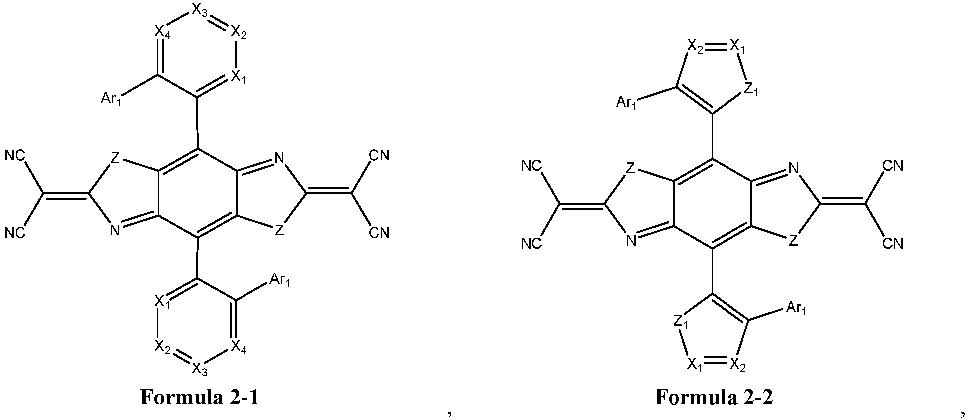 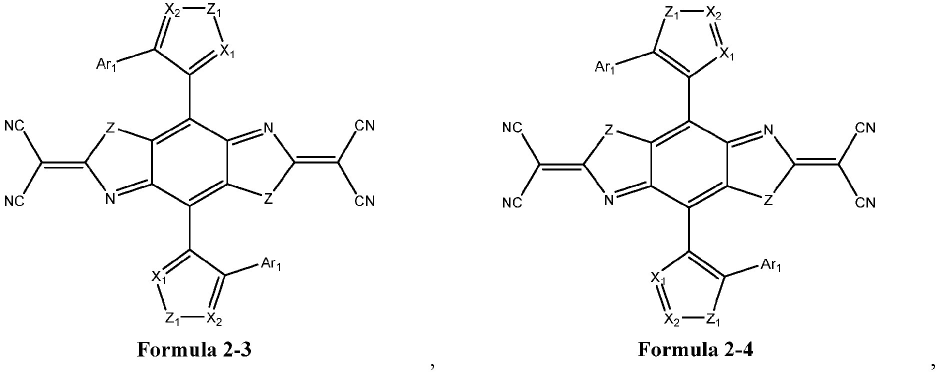 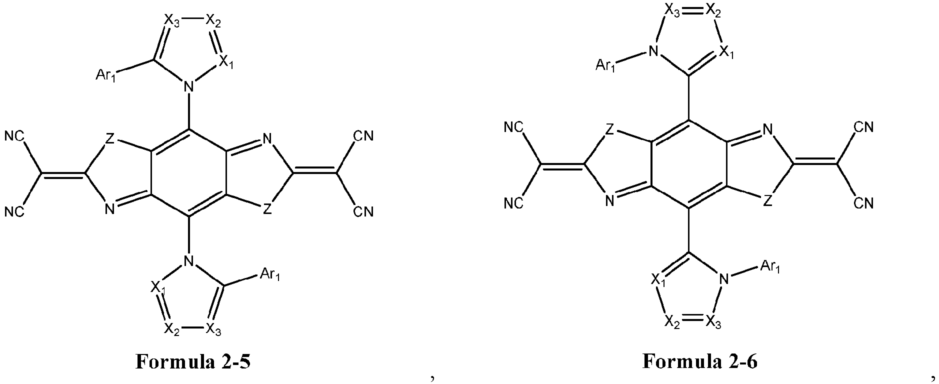 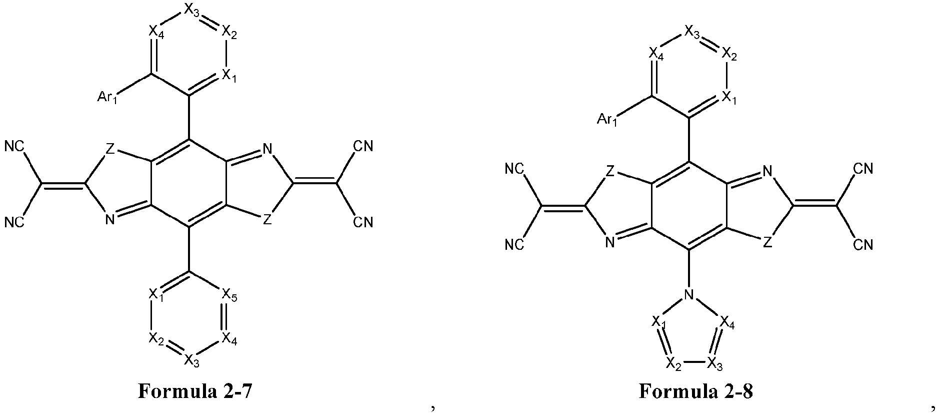 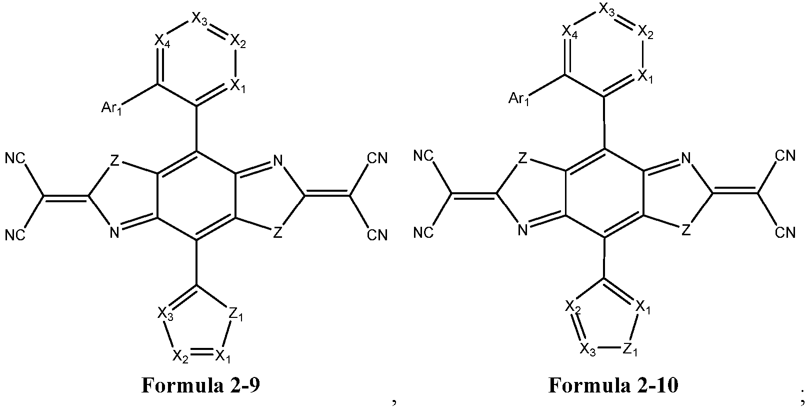
Z ist bei jedem Auftreten identisch oder unterschiedlich ausgewählt aus O, S oder Se;
X₁ bis X₅ sind bei jedem Auftreten identisch oder unterschiedlich ausgewählt aus N oder CR₁,
Z₁ ist bei jedem Auftreten identisch oder unterschiedlich ausgewählt aus O, S, Se oder NR_{N1};
Ar₁ ist bei jedem Auftreten identisch oder unterschiedlich ausgewählt aus der Gruppe bestehend aus: substituiertem oder unsubstituiertem Aryl mit 6 bis 20 Kohlenstoffatomen, substituiertem oder unsubstituiertem Heteroaryl mit 3 bis 20 Kohlenstoffatomen und Kombinationen davon; und wenn Ar₁ ausgewählt ist aus substituiertem Aryl mit 6 bis 20 Kohlenstoffatomen oder substituiertem Heteroaryl mit 3 bis 20 Kohlenstoffatomen, ist das Aryl oder Heteroaryl mit einem oder mehreren R₂ substituiert;
R₁ , R₂ und R_{N1} sind bei jedem Auftreten identisch oder unterschiedlich ausgewählt aus der Gruppe bestehend aus: Wasserstoff, Deuterium, einer Hydroxylgruppe, einer Sulfanylgruppe, Halogen, einer Nitrosogruppe, einer Nitrogruppe, einer Acylgruppe, einer Carbonylgruppe, einer Carbonsäuregruppe, einer Estergruppe, einer Cyanogruppe, einer Isocyanogruppe, SCN, OCN, SF₅, einer Boranylgruppe, einer Sulfinylgruppe, einer Sulfonylgruppe, einer Phosphorosogruppe, substituiertem oder unsubstituiertem Alkyl mit 1 bis 20 Kohlenstoffatomen, substituiertem oder unsubstituiertem Cycloalkyl mit 3 bis 20 Ringkohlenstoffatomen, substituiertem oder unsubstituiertem Heteroalkyl mit 1 bis 20 Kohlenstoffatomen, substituiertem oder unsubstituiertem Alkoxy mit 1 bis 20 Kohlenstoffatomen, substituiertem oder unsubstituiertem Alkenyl mit 2 bis 20 Kohlenstoffatomen, substituiertem oder unsubstituiertem Alkinyl mit 2 bis 20 Kohlenstoffatomen, substituiertem oder unsubstituiertem Aryl mit 6 bis 30 Kohlenstoffatomen, substituiertem oder unsubstituiertem Heteroaryl mit 3 bis 30 Kohlenstoffatomen und Kombinationen davon;
mindestens eines der R₁ und R₂ ist bei jedem Auftreten identisch oder unterschiedlich ausgewählt aus der Gruppe bestehend aus: einer Hydroxylgruppe, einer Sulfanylgruppe, Halogen, einer Nitrosogruppe, einer Nitrogruppe, einer Acylgruppe, einer Carbonylgruppe, einer Carbonsäuregruppe, einer Estergruppe, einer Cyanogruppe, einer Isocyanogruppe, SCN, OCN, SF₅, einer Boranylgruppe, einer Sulfinylgruppe, einer Sulfonylgruppe, einer Phosphorosogruppe, substituiertem oder unsubstituiertem Alkyl mit 1 bis 20 Kohlenstoffatomen, substituiertem oder unsubstituiertem Cycloalkyl mit 3 bis 20 Ringkohlenstoffatomen, substituiertem oder unsubstituiertem Heteroalkyl mit 1 bis 20 Kohlenstoffatomen, substituiertem oder unsubstituiertem Alkoxy mit 1 bis 20 Kohlenstoffatomen, substituiertem oder unsubstituiertem Alkenyl mit 2 bis 20 Kohlenstoffatomen, substituiertem oder unsubstituiertem Alkinyl mit 2 bis 20 Kohlenstoffatomen, substituiertem oder unsubstituiertem Aryl mit 6 bis 30 Kohlenstoffatomen, substituiertem oder unsubstituiertem Heteroaryl mit 3 bis 30 Kohlenstoffatomen und Kombinationen davon; und
benachbarte Substituenten Ri, R₂ können optional zu einem Ring verbunden sein.

2. Verbindung nach Anspruch 1, wobei X₁ bei jedem Auftreten identisch oder unterschiedlich ausgewählt ist aus CR₁.

3. Verbindung nach einem der Ansprüche 1 bis 2, wobei die Verbindung eine durch Formel 3-1 dargestellte Struktur hat: 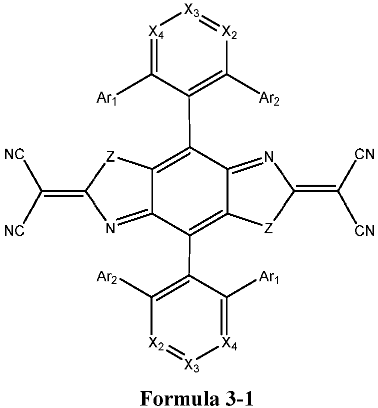 wobei in Formel 3-1,
Z bei jedem Auftreten identisch oder unterschiedlich ausgewählt ist aus O, S oder Se;
X₂ bis X₄ bei jedem Auftreten identisch oder unterschiedlich ausgewählt sind aus N oder CR₁;
Ar₁ und Ar₂ bei jedem Auftreten identisch oder unterschiedlich ausgewählt sind aus der Gruppe bestehend aus: substituiertem oder unsubstituiertem Aryl mit 6 bis 20 Kohlenstoffatomen, substituiertem oder unsubstituiertem Heteroaryl mit 3 bis 20 Kohlenstoffatomen und Kombinationen davon; und wenn Ar₁ und Ar₂ ausgewählt sind aus substituiertem Aryl mit 6 bis 20 Kohlenstoffatomen oder substituiertem Heteroaryl mit 3 bis 20 Kohlenstoffatomen, ist das Aryl oder Heteroaryl mit einem oder mehreren R₂ substituiert;
R₁ und R₂ sind bei jedem Auftreten identisch oder unterschiedlich ausgewählt aus der Gruppe bestehend aus: Wasserstoff, Deuterium, einer Hydroxylgruppe, einer Sulfanylgruppe, Halogen, einer Nitrosogruppe, einer Nitrogruppe, einer Acylgruppe, einer Carbonylgruppe, einer Carbonsäuregruppe, einer Estergruppe, einer Cyanogruppe, einer Isocyanogruppe, SCN, OCN, SF₅, einer Boranylgruppe, einer Sulfinylgruppe, einer Sulfonylgruppe, einer Phosphorosogruppe, substituiertem oder unsubstituiertem Alkyl mit 1 bis 20 Kohlenstoffatomen, substituiertem oder unsubstituiertem Cycloalkyl mit 3 bis 20 Ringkohlenstoffatomen, substituiertem oder unsubstituiertem Heteroalkyl mit 1 bis 20 Kohlenstoffatomen, substituiertem oder unsubstituiertem Alkoxy mit 1 bis 20 Kohlenstoffatomen, substituiertem oder unsubstituiertem Alkenyl mit 2 bis 20 Kohlenstoffatomen, substituiertem oder unsubstituiertem Alkinyl mit 2 bis 20 Kohlenstoffatomen, substituiertem oder unsubstituiertem Aryl mit 6 bis 30 Kohlenstoffatomen, substituiertem oder unsubstituiertem Heteroaryl mit 3 bis 30 Kohlenstoffatomen und Kombinationen davon;
mindestens eines der R₁ und R₂ ist bei jedem Auftreten identisch oder unterschiedlich ausgewählt aus der Gruppe bestehend aus: einer Hydroxylgruppe, einer Sulfanylgruppe, Halogen, einer Nitrosogruppe, einer Nitrogruppe, einer Acylgruppe, einer Carbonylgruppe, einer Carbonsäuregruppe, einer Estergruppe, einer Cyanogruppe, einer Isocyanogruppe, SCN, OCN, SF₅, einer Boranylgruppe, einer Sulfinylgruppe, einer Sulfonylgruppe, einer Phosphorosogruppe, substituiertem oder unsubstituiertem Alkyl mit 1 bis 20 Kohlenstoffatomen, substituiertem oder unsubstituiertem Cycloalkyl mit 3 bis 20 Ringkohlenstoffatomen, substituiertem oder unsubstituiertem Heteroalkyl mit 1 bis 20 Kohlenstoffatomen, substituiertem oder unsubstituiertem Alkoxy mit 1 bis 20 Kohlenstoffatomen, substituiertem oder unsubstituiertem Alkenyl mit 2 bis 20 Kohlenstoffatomen, substituiertem oder unsubstituiertem Alkinyl mit 2 bis 20 Kohlenstoffatomen, substituiertem oder unsubstituiertem Aryl mit 6 bis 30 Kohlenstoffatomen, substituiertem oder unsubstituiertem Heteroaryl mit 3 bis 30 Kohlenstoffatomen und Kombinationen davon; und
benachbarte Substituenten R₁, R₂ können optional zu einem Ring verbunden sein.

4. Verbindung nach einem der Ansprüche 1 bis 3, wobei Z bei jedem Auftreten identisch oder unterschiedlich ausgewählt ist aus O oder S; bevorzugt ist Z aus O ausgewählt.

5. Verbindung nach Anspruch 1, wobei in den Formeln 2-1 bis 2-10 mindestens eines von X₁ bis X₅ bei jedem Auftreten identisch oder unterschiedlich ausgewählt ist aus CR₁; bevorzugt sind X₁ bis X₅ bei jedem Auftreten identisch oder unterschiedlich ausgewählt aus CRi.

6. Verbindung nach einem der Ansprüche 1 bis 5, wobei R₁ und R₂ bei jedem Auftreten identisch oder unterschiedlich ausgewählt sind aus der Gruppe bestehend aus: Wasserstoff, Deuterium, Halogen, Cyano, Isocyano, SCN, OCN, SF₅, Boranyl, substituiertem oder unsubstituiertem Alkyl mit 1 bis 20 Kohlenstoffatomen, substituiertem oder unsubstituiertem Heteroalkyl mit 1 bis 20 Kohlenstoffatomen, substituiertem oder unsubstituiertem Alkoxy mit 1 bis 20 Kohlenstoffatomen, substituiertem oder unsubstituiertem Aryl mit 6 bis 30 Kohlenstoffatomen, substituiertem oder unsubstituiertem Heteroaryl mit 3 bis 30 Kohlenstoffatomen und Kombinationen davon;
mindestens eines der R₁ und R₂ ist bei jedem Auftreten identisch oder unterschiedlich ausgewählt aus der Gruppe bestehend aus: Halogen, Cyano, Isocyano, SCN, OCN, SF₅, Boranyl, substituiertem oder unsubstituiertem Alkyl mit 1 bis 20 Kohlenstoffatomen, substituiertem oder unsubstituiertem Heteroalkyl mit 1 bis 20 Kohlenstoffatomen, substituiertem oder unsubstituiertem Alkoxy mit 1 bis 20 Kohlenstoffatomen, substituiertem oder unsubstituiertem Aryl mit 6 bis 30 Kohlenstoffatomen, substituiertem oder unsubstituiertem Heteroaryl mit 3 bis 30 Kohlenstoffatomen und Kombinationen davon; und
benachbarte Substituenten R₁, R₂ können optional zu einem Ring verbunden sein;
bevorzugt ist mindestens eines der R₁ und R₂ bei jedem Auftreten identisch oder unterschiedlich ausgewählt aus der Gruppe bestehend aus: Fluor, SF₅, Cyano, substituiertem Alkyl mit 1 bis 20 Kohlenstoffatomen, substituiertem Heteroalkyl mit 1 bis 20 Kohlenstoffatomen, substituiertem Alkoxy mit 1 bis 20 Kohlenstoffatomen, substituiertem Aryl mit 6 bis 30 Kohlenstoffatomen, substituiertem Heteroaryl mit 3 bis 30 Kohlenstoffatomen und Kombinationen davon; und ein Substituent in dem substituierten Alkyl, substituierten Heteroalkyl, substituierten Alkoxy, substituierten Aryl oder substituierten Heteroaryl umfasst mindestens eine elektronenziehende Gruppe;
besonders bevorzugt ist mindestens eines der R₁ und R₂ bei jedem Auftreten identisch oder unterschiedlich ausgewählt aus der Gruppe bestehend aus: Fluor, SF₅, Cyano, substituiertem Alkyl mit 1 bis 20 Kohlenstoffatomen, substituiertem Heteroalkyl mit 1 bis 20 Kohlenstoffatomen, substituiertem Alkoxy mit 1 bis 20 Kohlenstoffatomen, substituiertem Aryl mit 6 bis 30 Kohlenstoffatomen, substituiertem Heteroaryl mit 3 bis 30 Kohlenstoffatomen und Kombinationen davon; und ein Substituent in dem substituierten Alkyl, substituierten Heteroalkyl, substituierten Alkoxy, substituierten Aryl oder substituierten Heteroaryl umfasst mindestens ein Fluoratom und/oder mindestens eine Cyanogruppe.

7. Verbindung nach einem der Ansprüche 1 bis 6, wobei R₁ und R₂ bei jedem Auftreten identisch oder unterschiedlich ausgewählt sind aus der Gruppe bestehend aus A1 bis A19 und B1 bis B314: 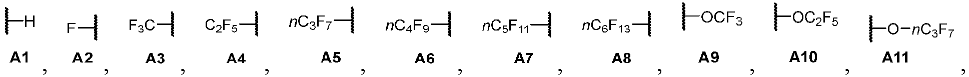 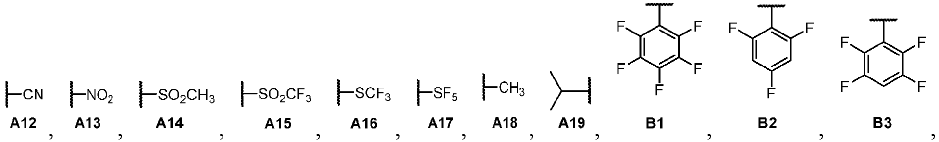 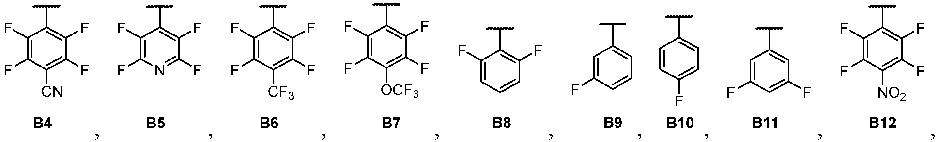 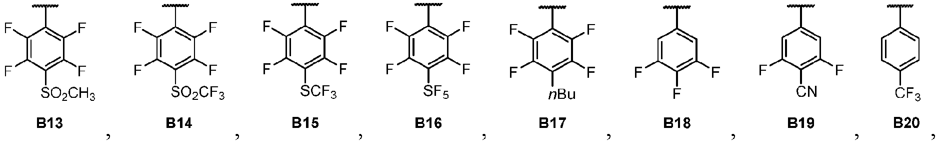 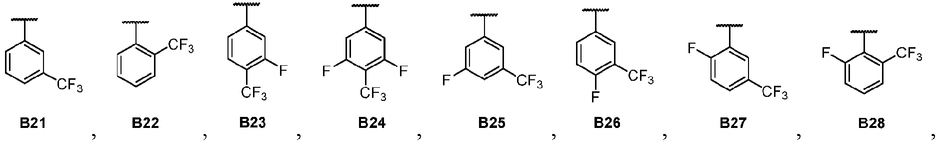 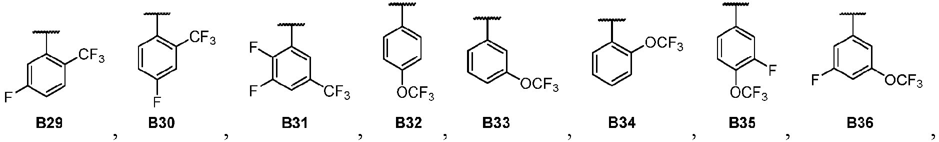 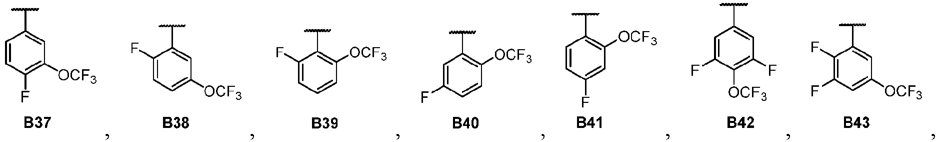 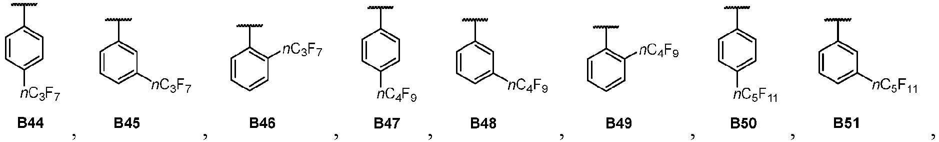 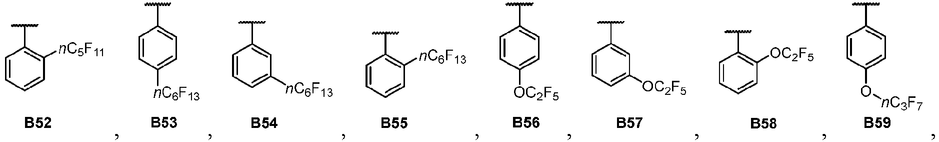 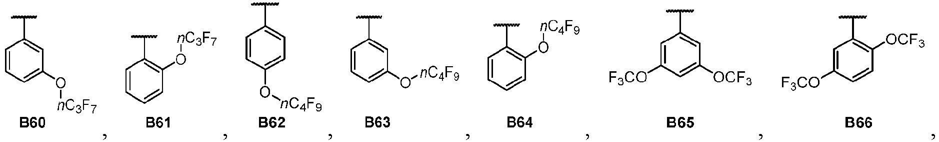 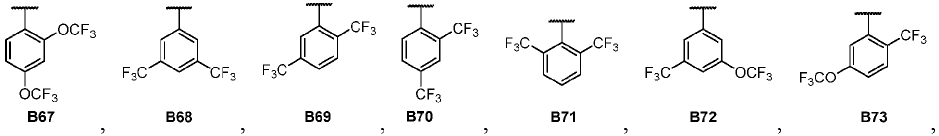 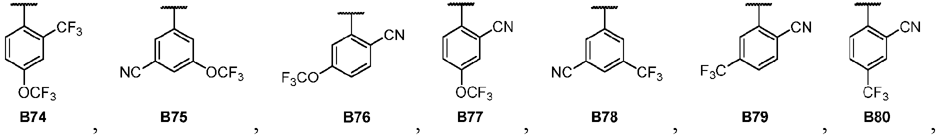 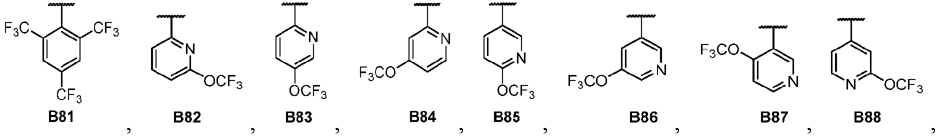 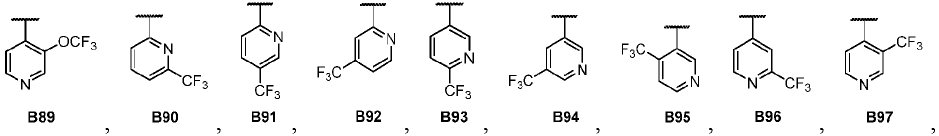 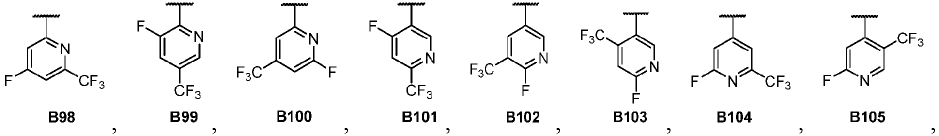 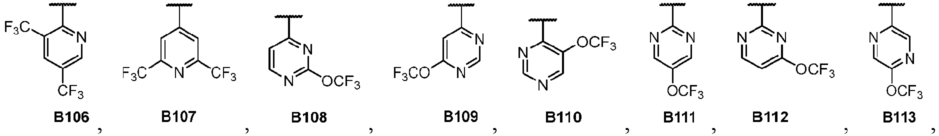 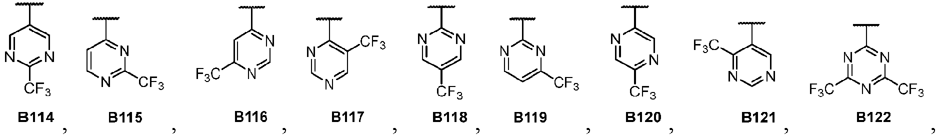 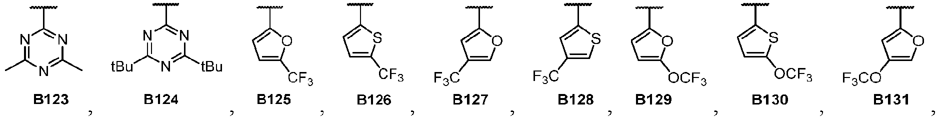 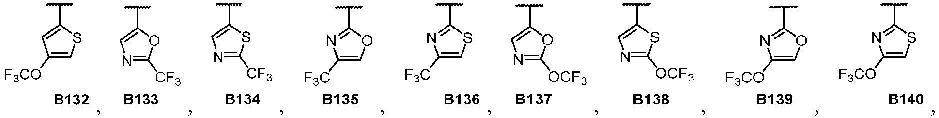 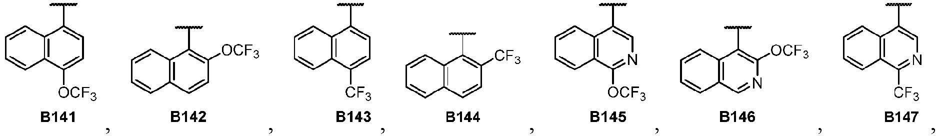 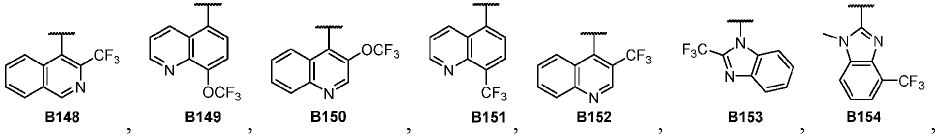 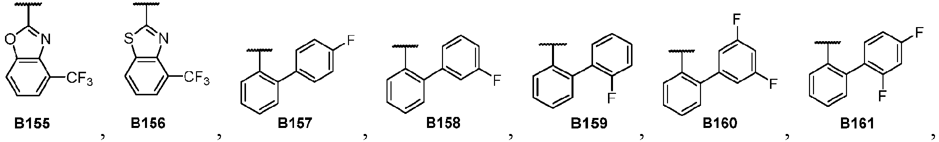 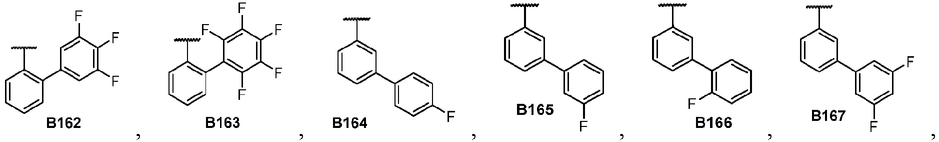 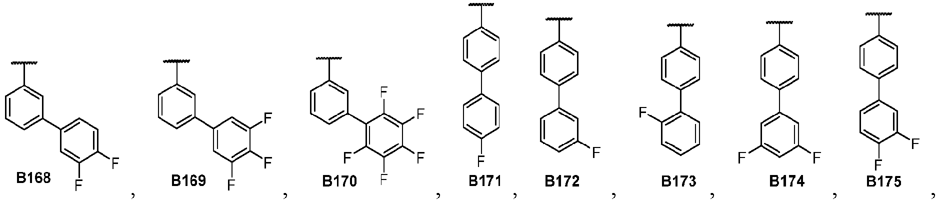 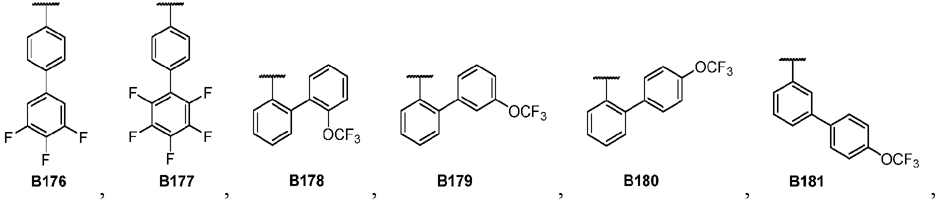 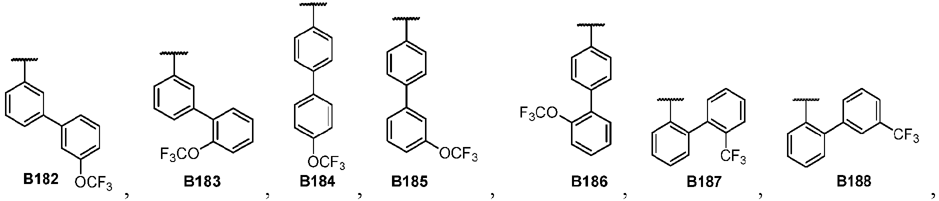 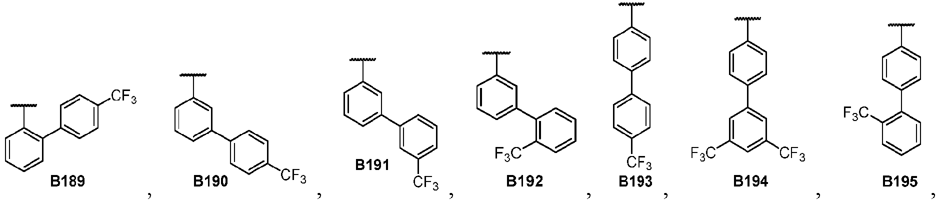 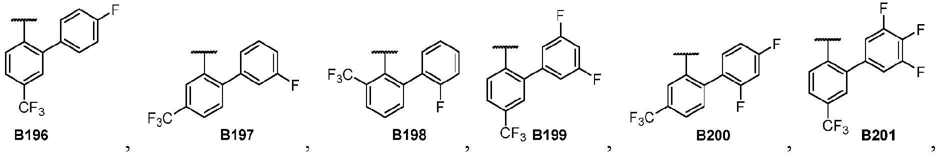 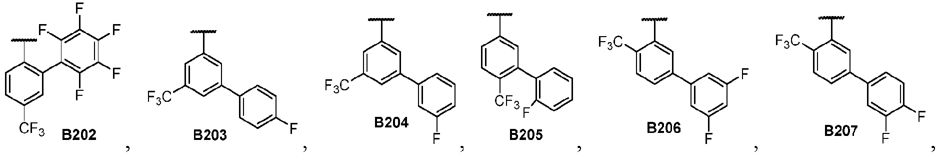 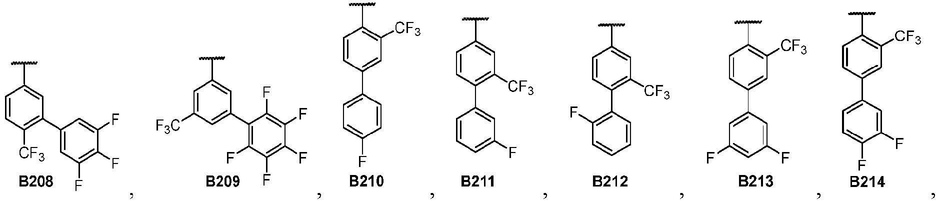 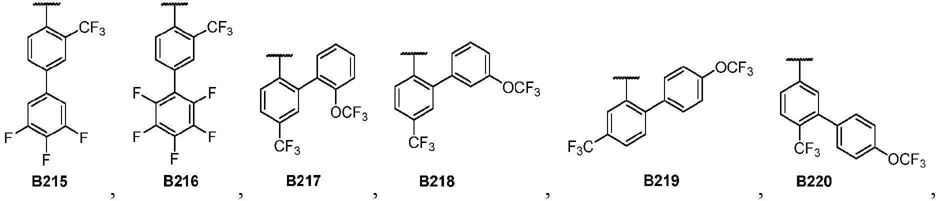 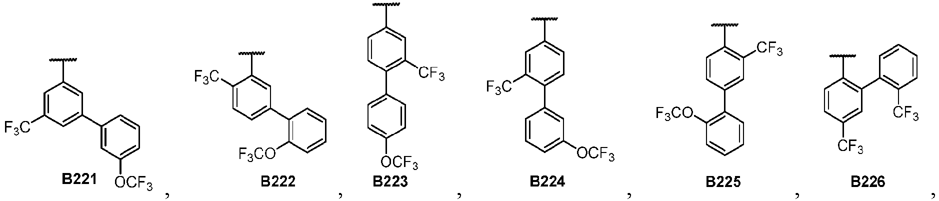 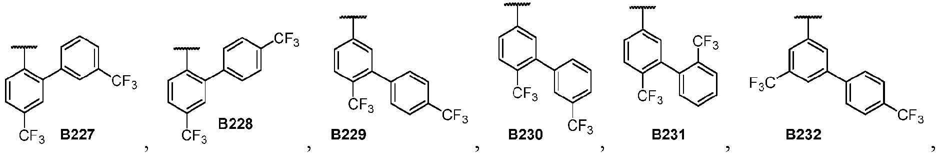 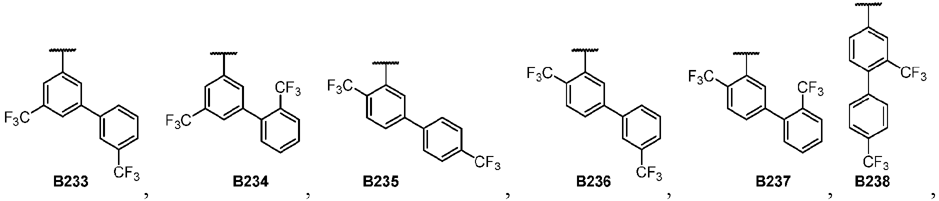 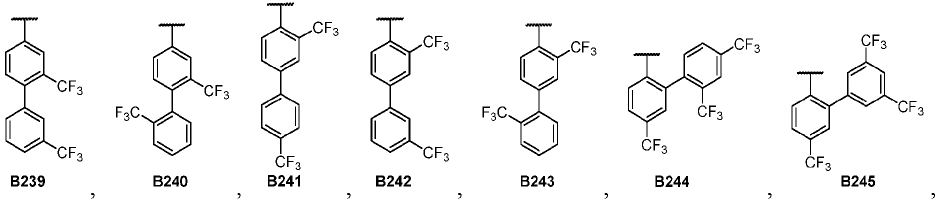 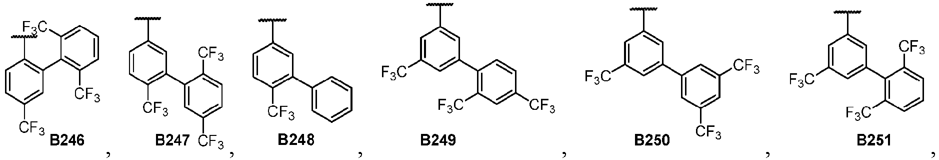 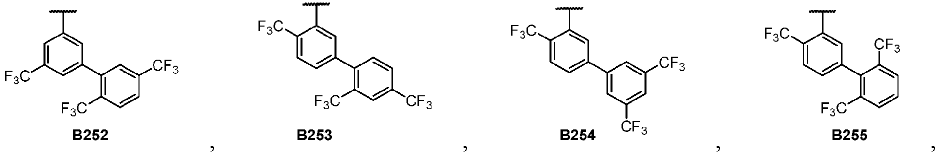 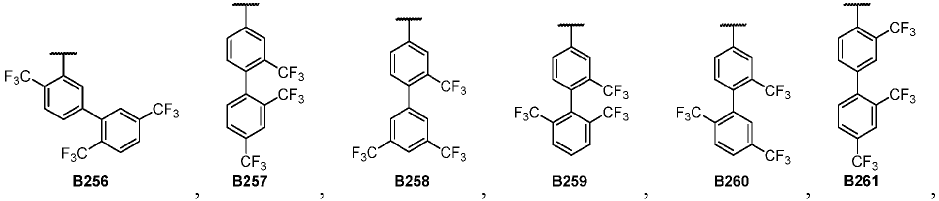 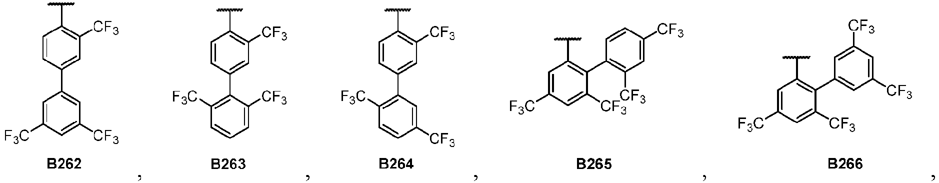 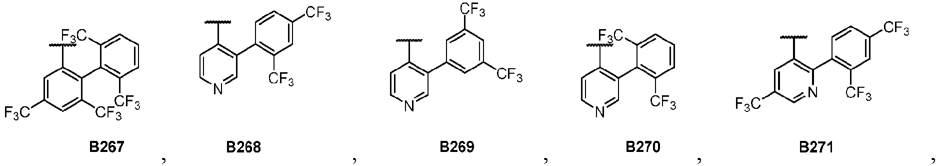 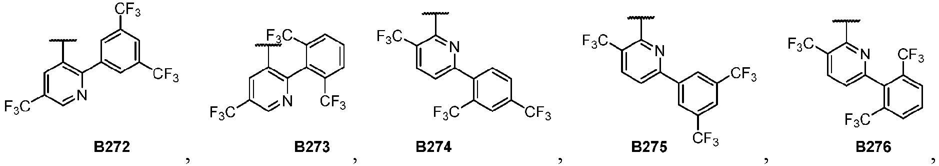 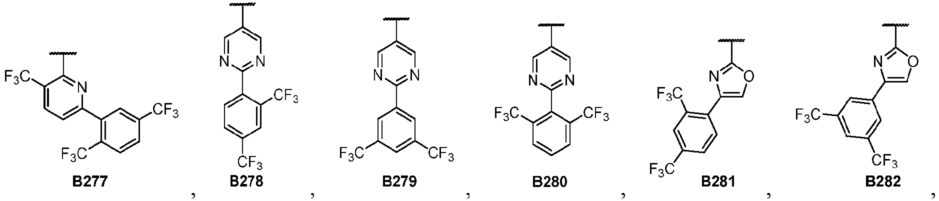 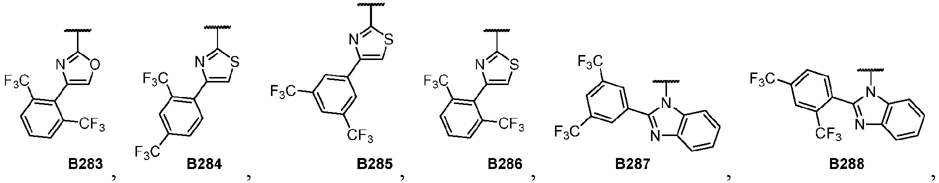 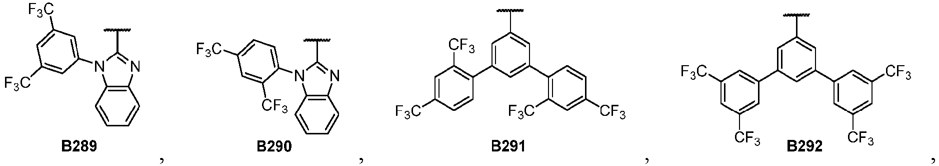 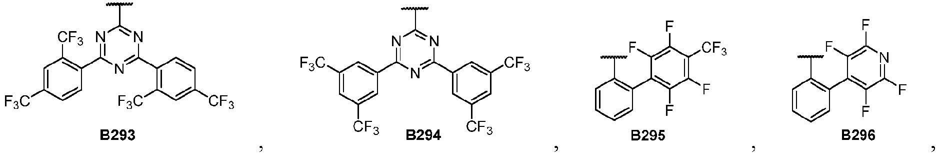 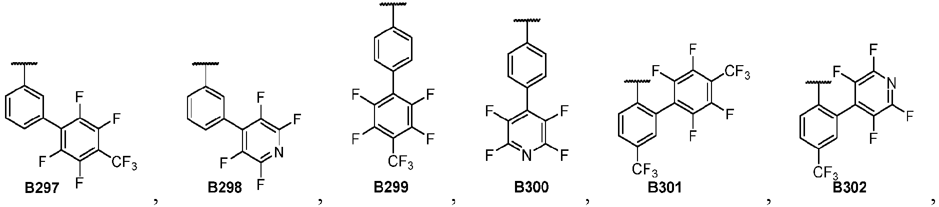 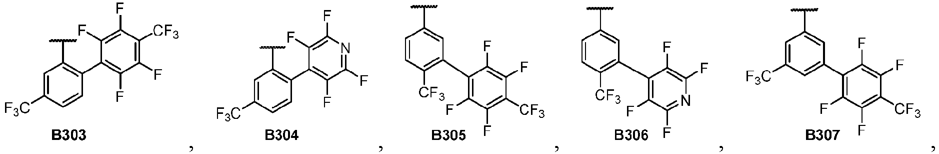 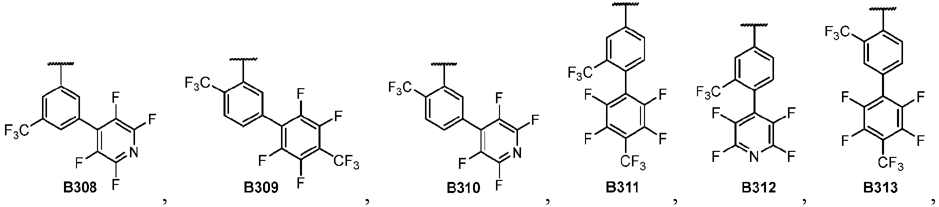 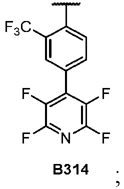 und/oder Ar₁ und Ar₂ bei jedem Auftreten identisch oder unterschiedlich ausgewählt sind aus der Gruppe bestehend aus B1 bis B314.

8. Verbindung nach Anspruch 7, wobei die Verbindung ausgewählt ist aus der Gruppe bestehend aus Verbindung H1-1 bis Verbindung H1-2449, Verbindung H2-1 bis Verbindung H2-138, Verbindung H3-1 bis Verbindung H3-92, Verbindung H4-1 bis Verbindung H4-92, Verbindung H5-1 bis Verbindung H5-115, Verbindung H6-1 bis Verbindung H6-115, Verbindung H7-1 bis Verbindung H7-203 und Verbindung H8-7 bis Verbindung H8-54, Verbindung H8-61 bis Verbindung H8-108, Verbindung H8-115 bis Verbindung H8-162, Verbindung H8-169 bis Verbindung H8-216, Verbindung H8-223 bis Verbindung H8-270, Verbindung H8-277 bis Verbindung H8-324, Verbindung H8-331 bis Verbindung H8-378, Verbindung H8-385 bis Verbindung H8-432, Verbindung H8-439 bis Verbindung H8-486, Verbindung H8-493 bis Verbindung H8-540, Verbindung H8-547 bis Verbindung H8-594, Verbindung H8-601 bis Verbindung H8-648, Verbindung H8-655 bis Verbindung H8-702, Verbindung H8-709 bis Verbindung H8-756, Verbindung H8-763 bis Verbindung H8-810, Verbindung H8-817 bis Verbindung H8-864, Verbindung H8-871 bis Verbindung H8-918, Verbindung H8-925 bis Verbindung H8-972, Verbindung H8-979 bis Verbindung H8-1026, Verbindung H8-1033 bis Verbindung H8-1080, Verbindung H8-1087 bis Verbindung H8-1134, Verbindung H8-1141 bis Verbindung H8-1188, Verbindung H8-1195 bis Verbindung H8-1242, Verbindung H8-1249 bis Verbindung H8-1296, Verbindung H8-1303 bis Verbindung H8-1350, Verbindung H8-1357 bis Verbindung H8-1404, Verbindung H8-1411 bis Verbindung H8-1458, Verbindung H8-1465 bis Verbindung H8-1512;
wobei Verbindung H1-1 bis Verbindung H1-2449 jeweils eine durch die Formel 2-1 dargestellte Struktur haben: 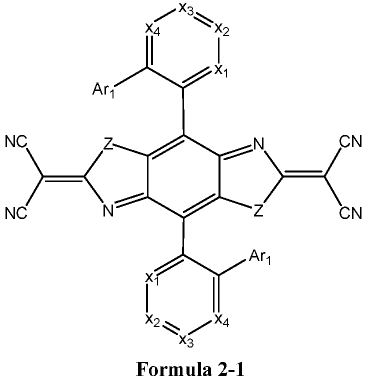
in Formel 2-1 zwei Z gleich sind, zwei X₁ gleich sind, zwei X₂ gleich sind, zwei X₃ gleich sind, zwei X₄ gleich sind, zwei Ar₁ gleich sind, und Z, X₁, X₂, X₃, X₄ und Ar₁ jeweils aus den Atomen oder Gruppen in der folgenden Tabelle ausgewählt sind: 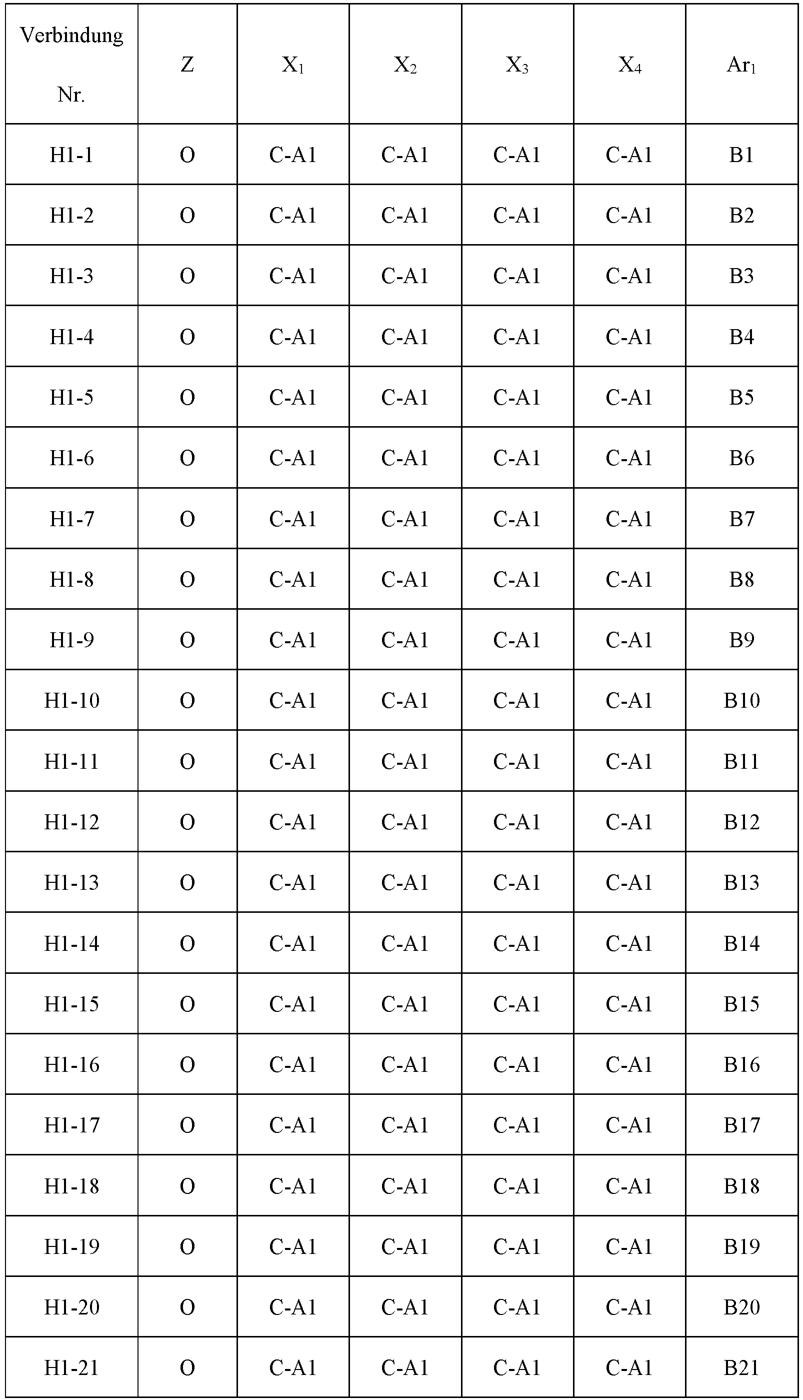 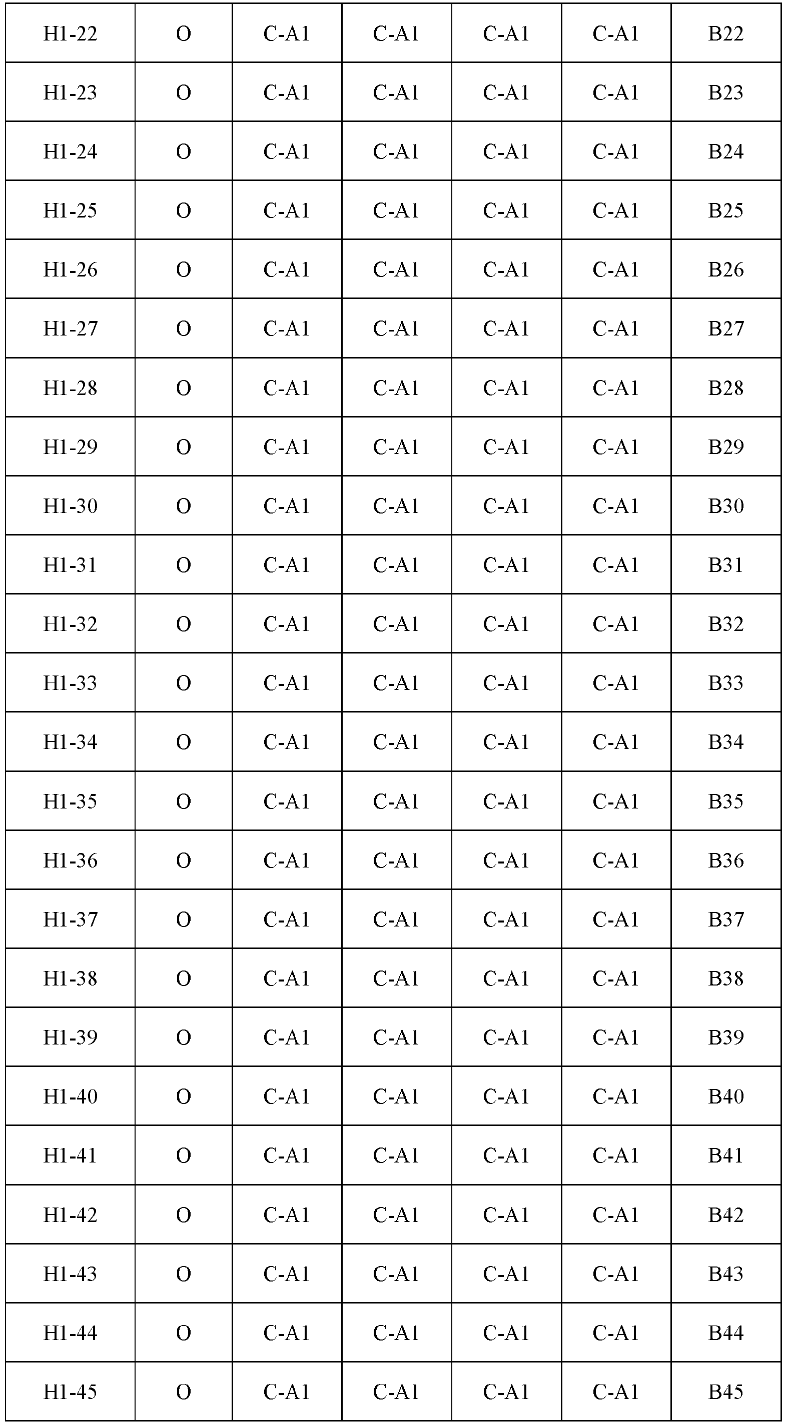 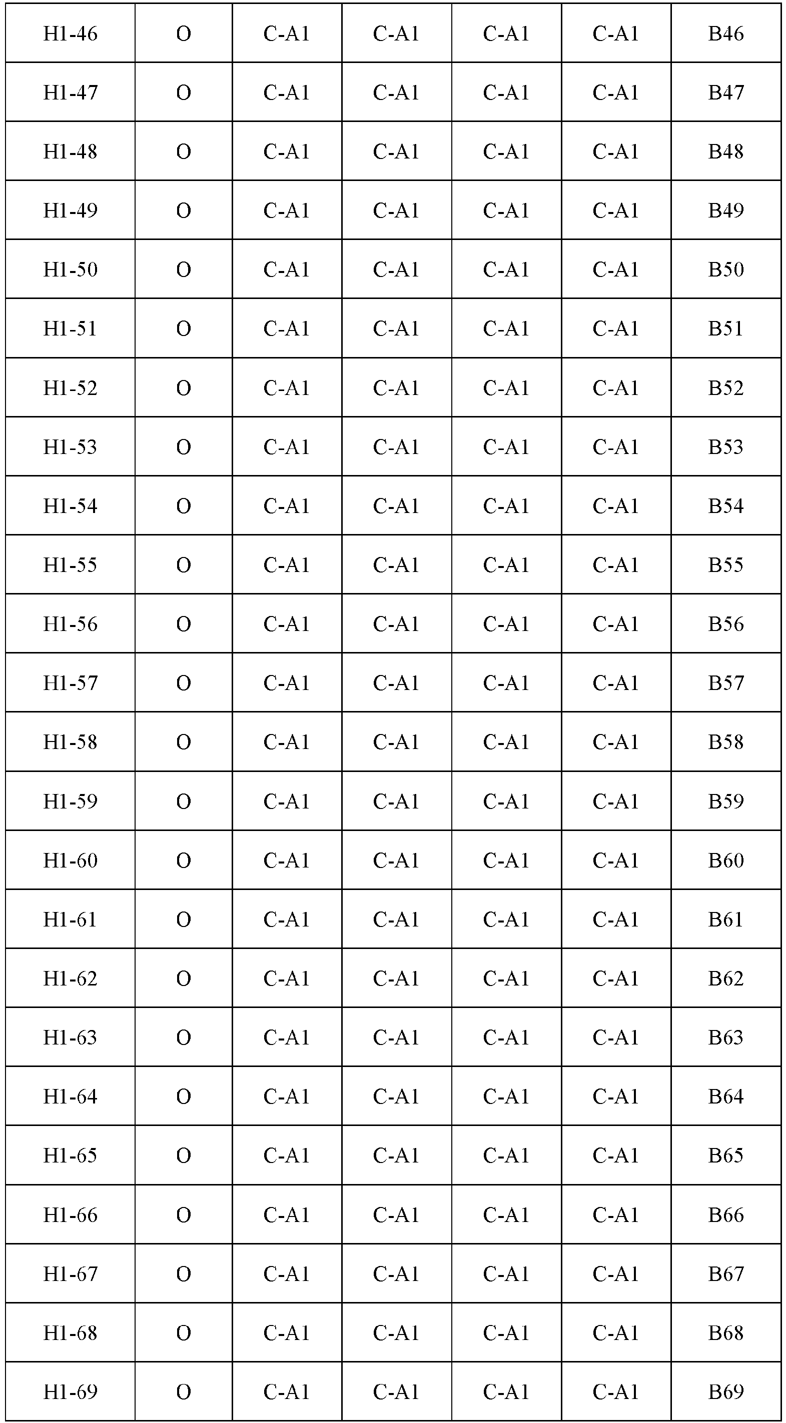 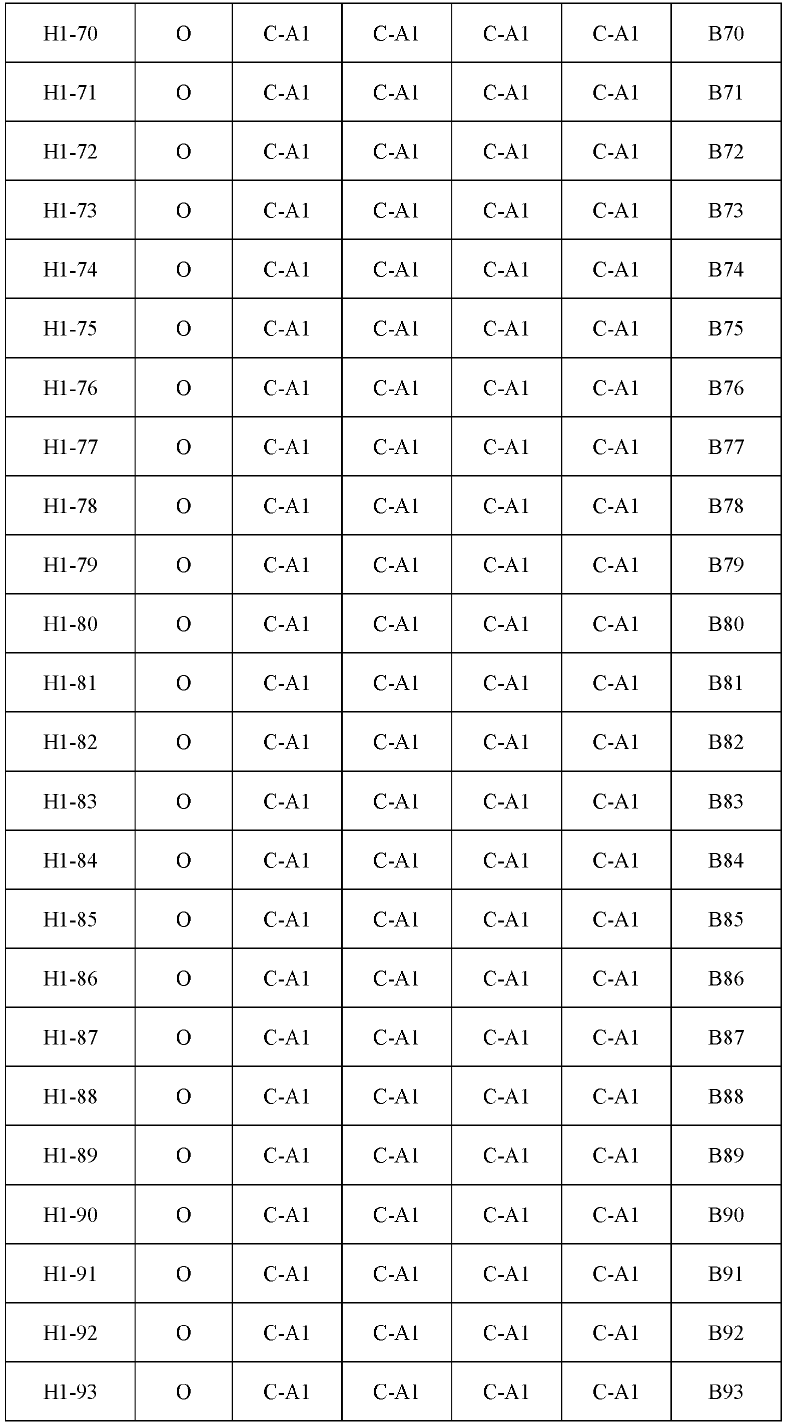 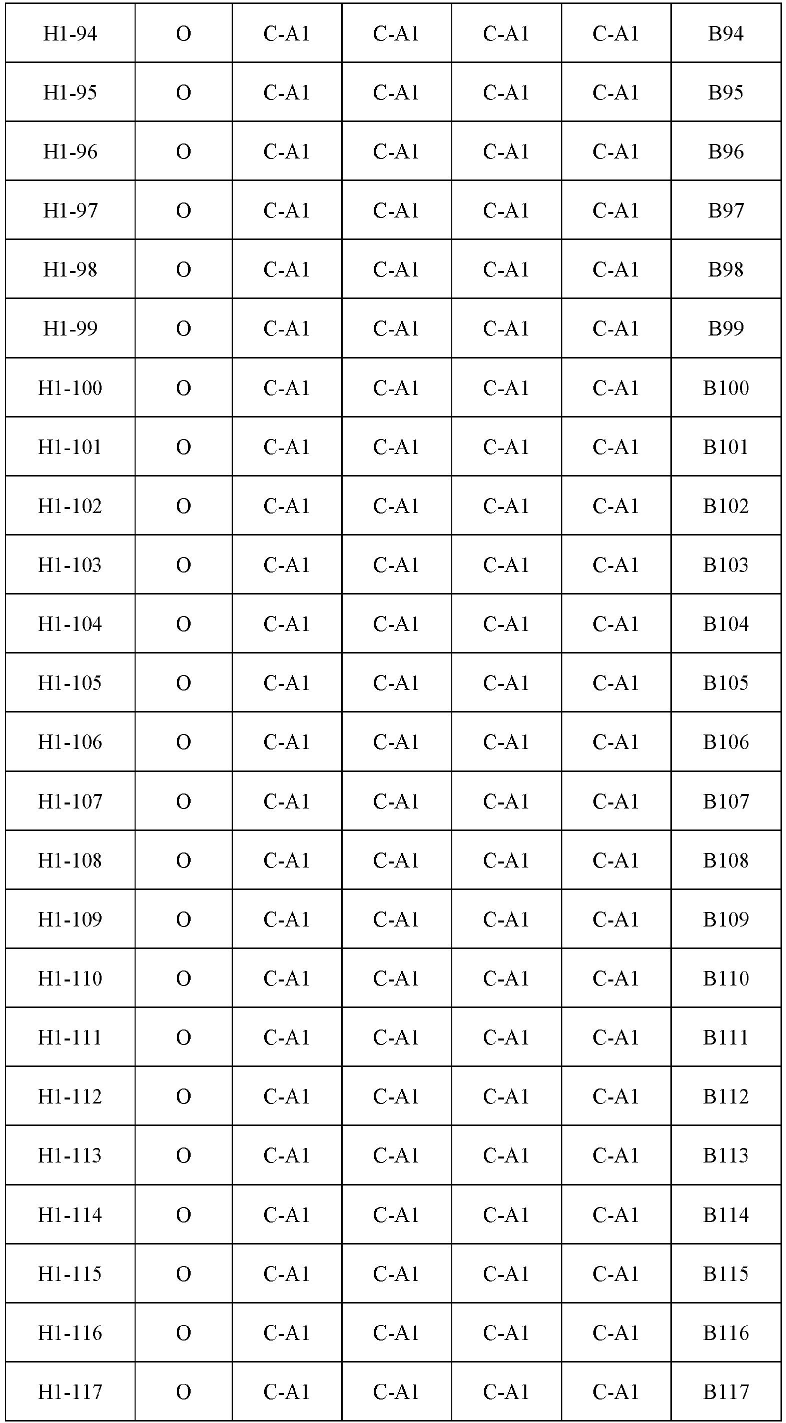 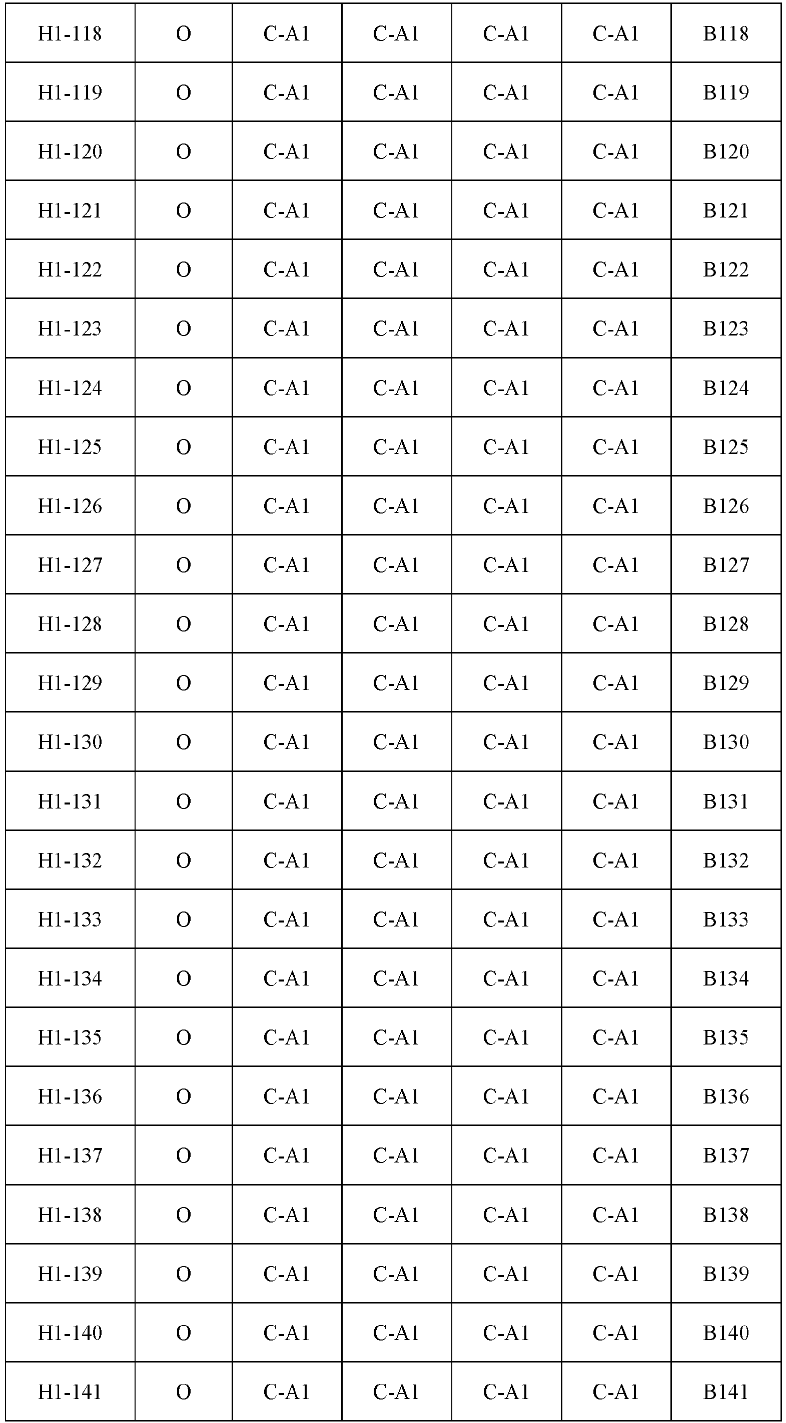 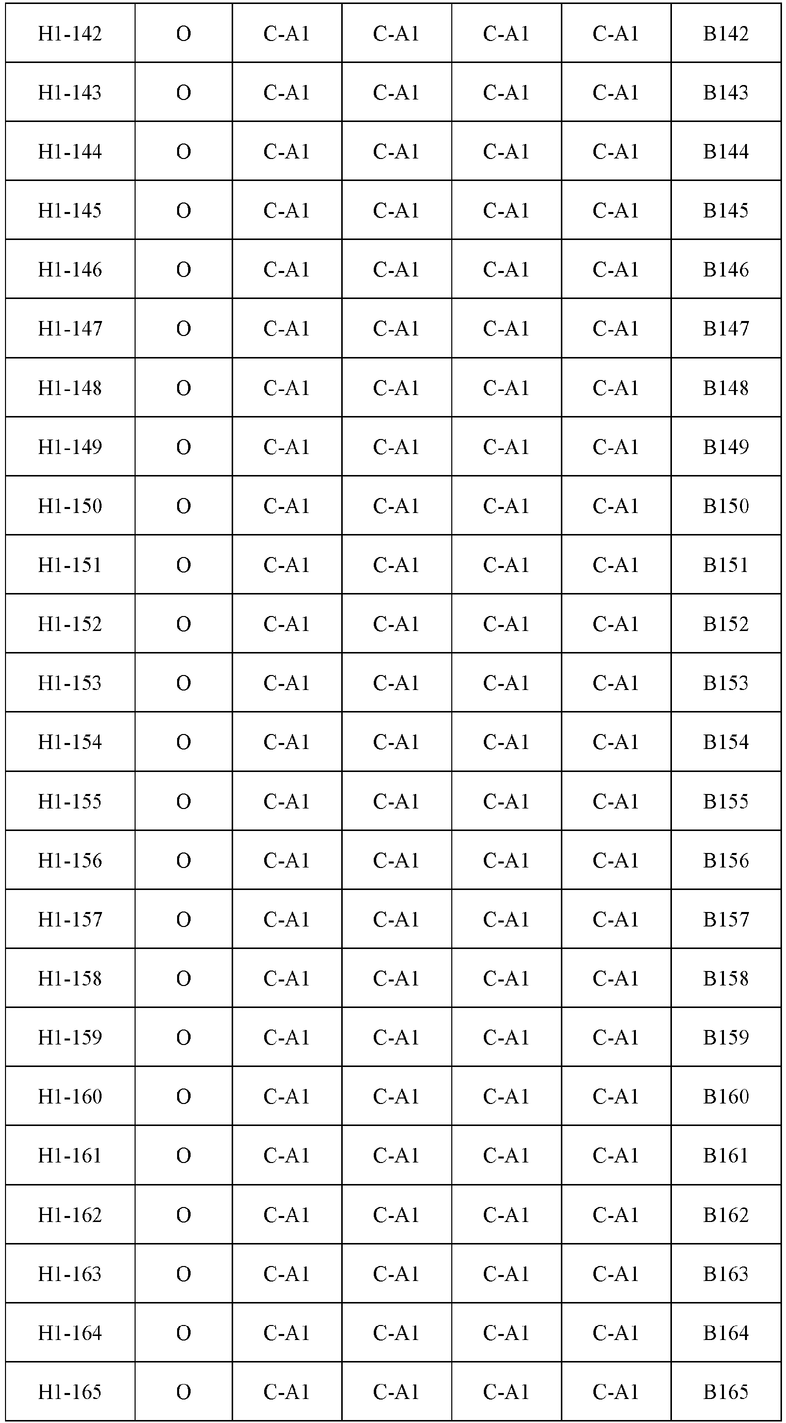 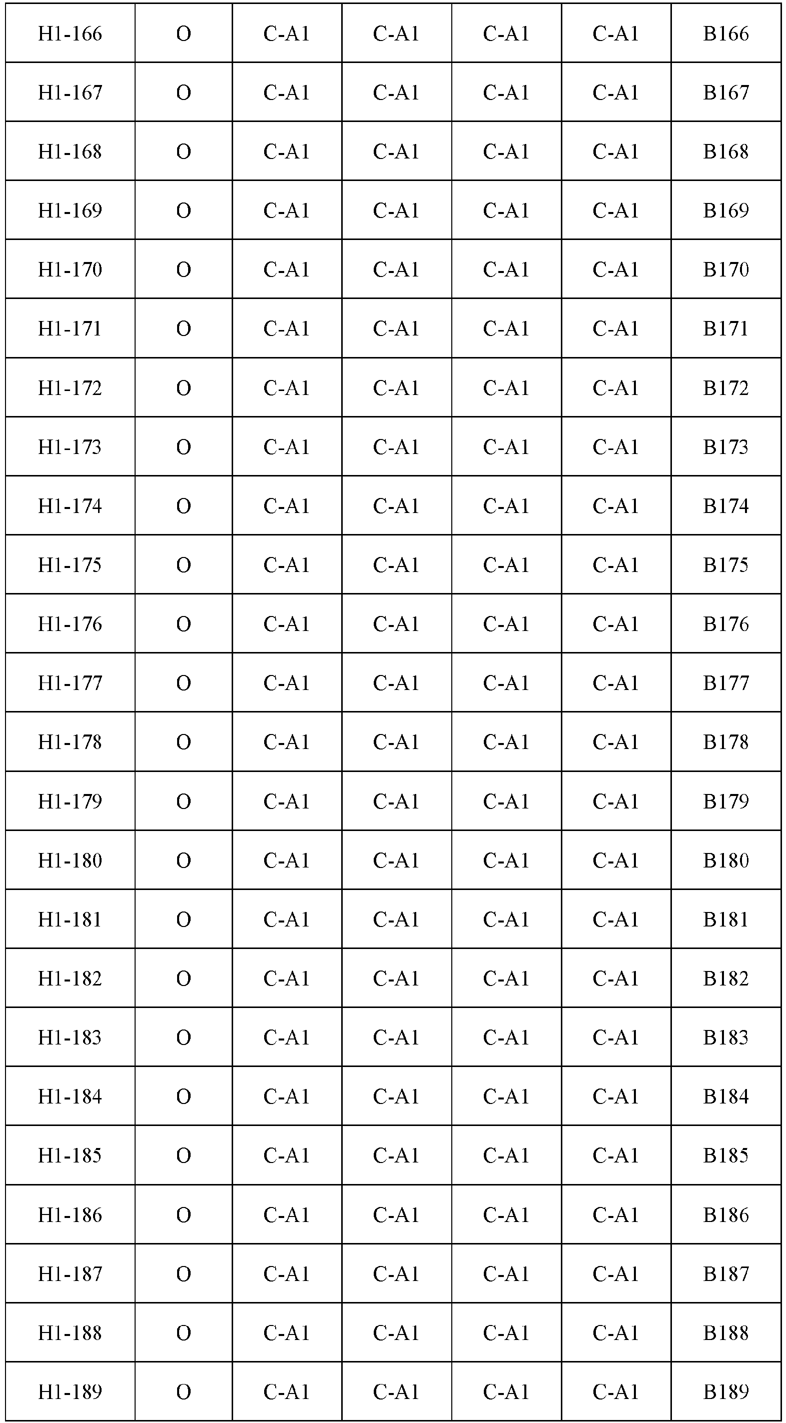 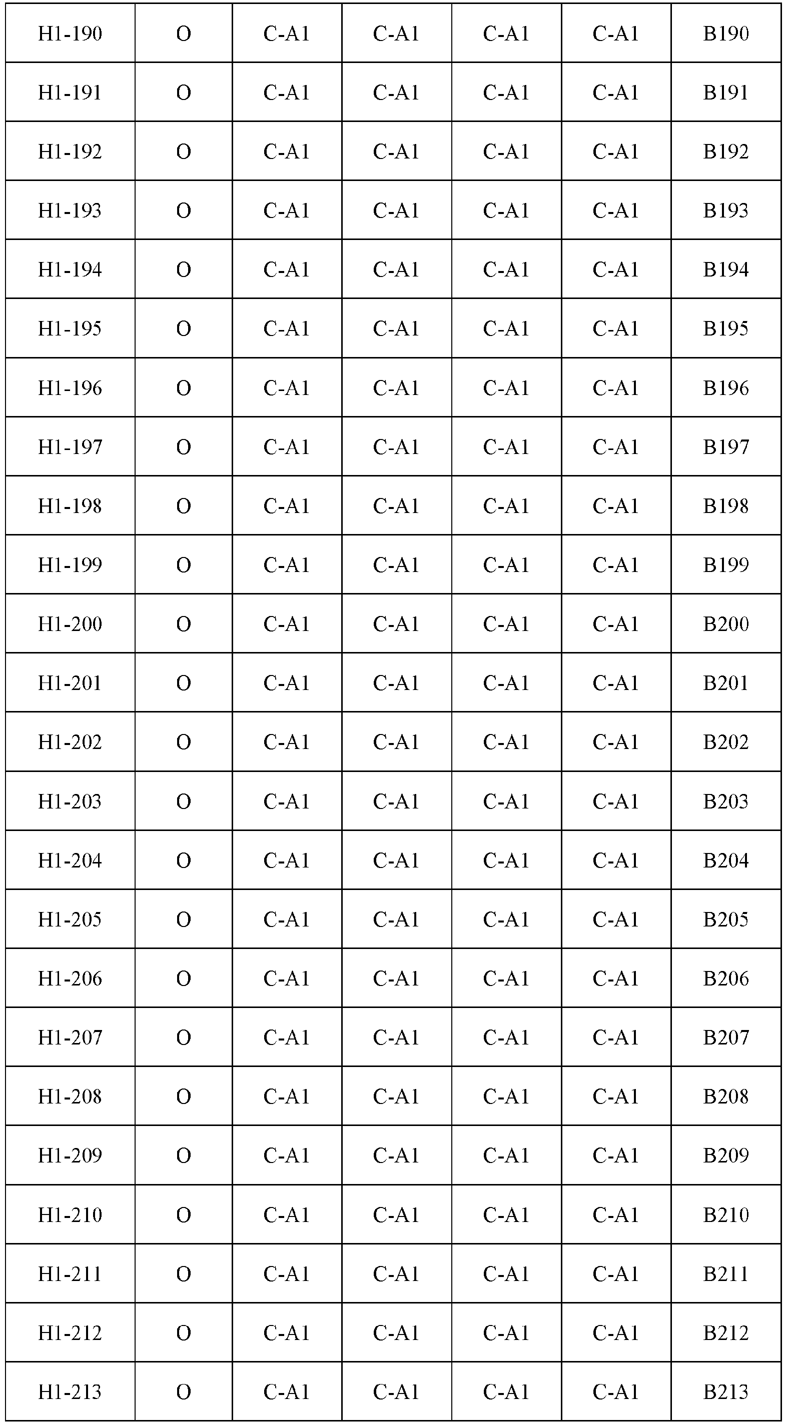 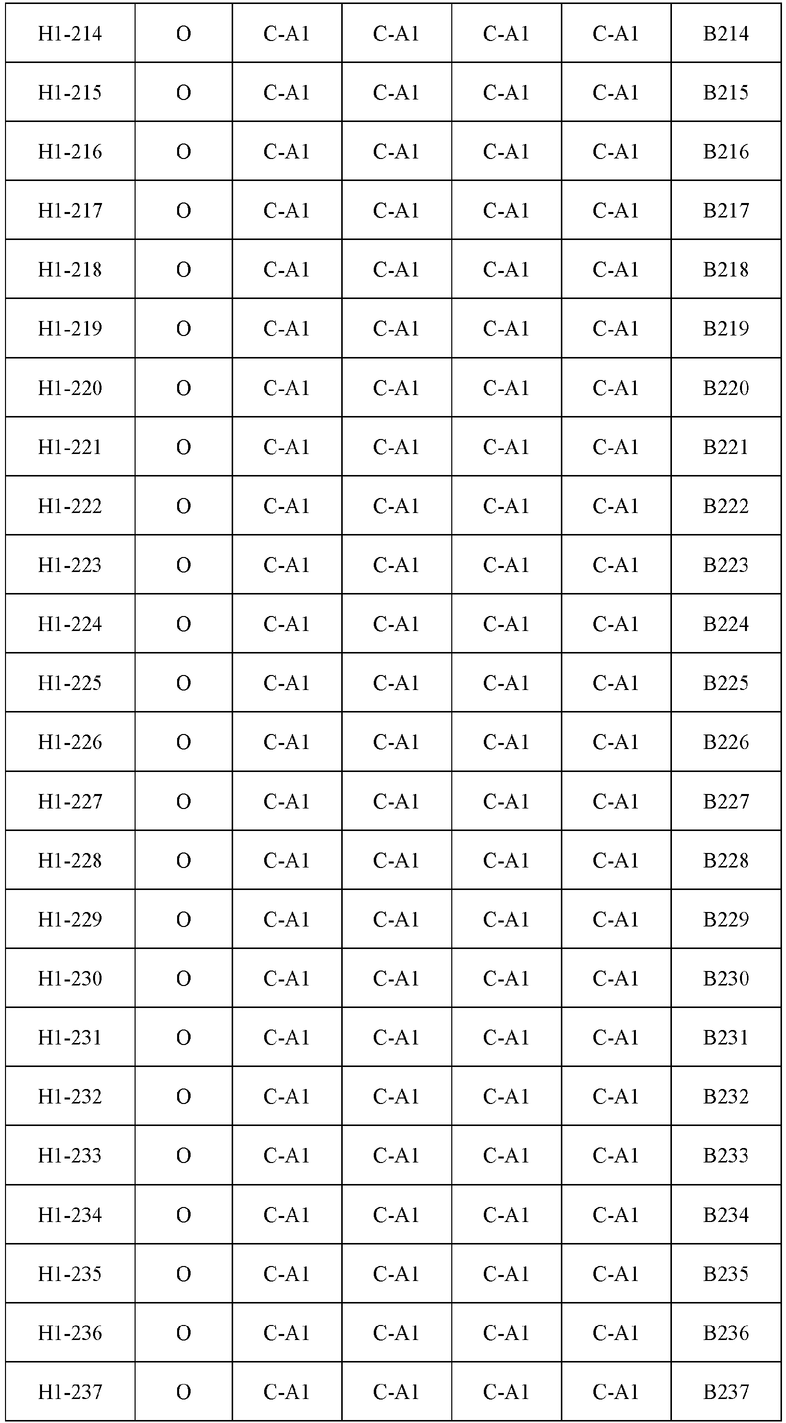 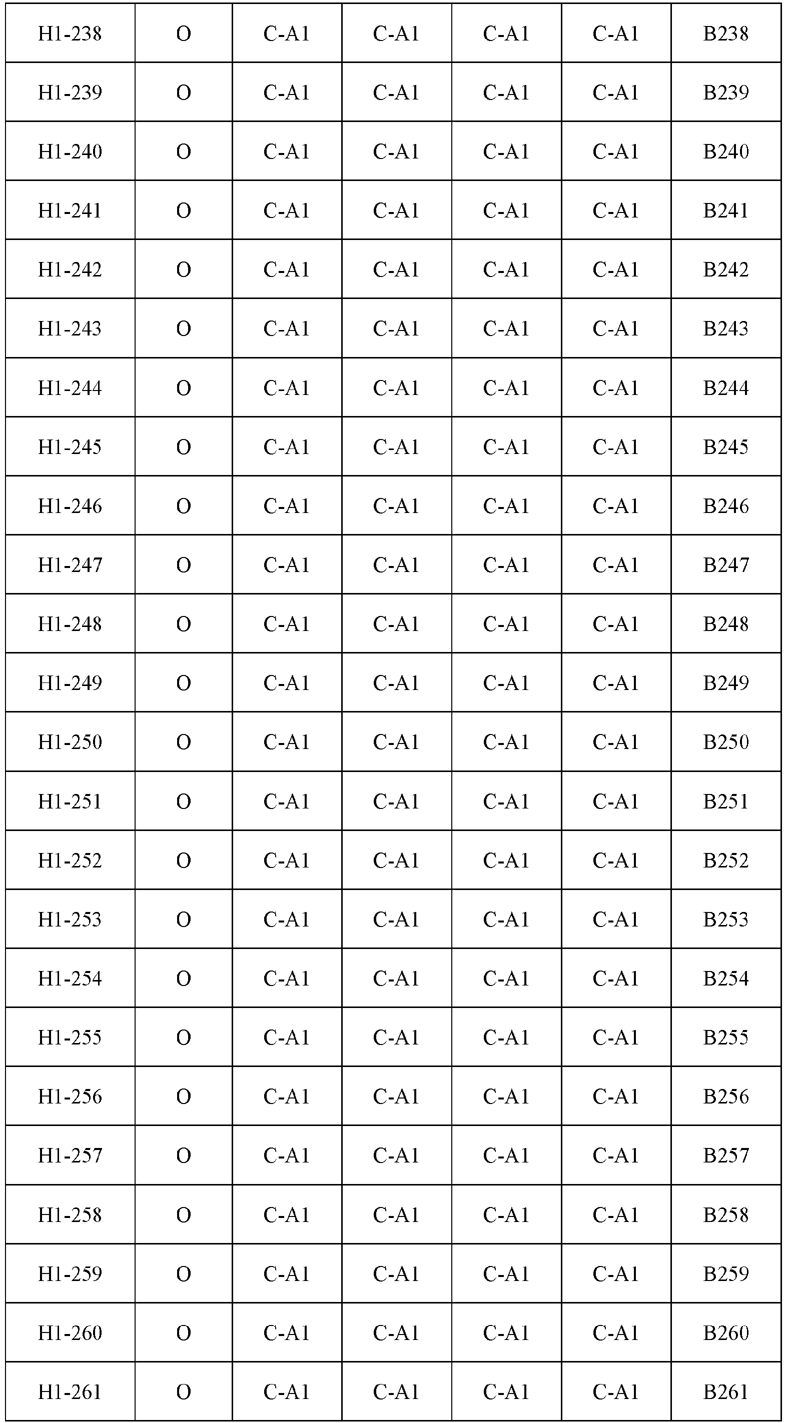 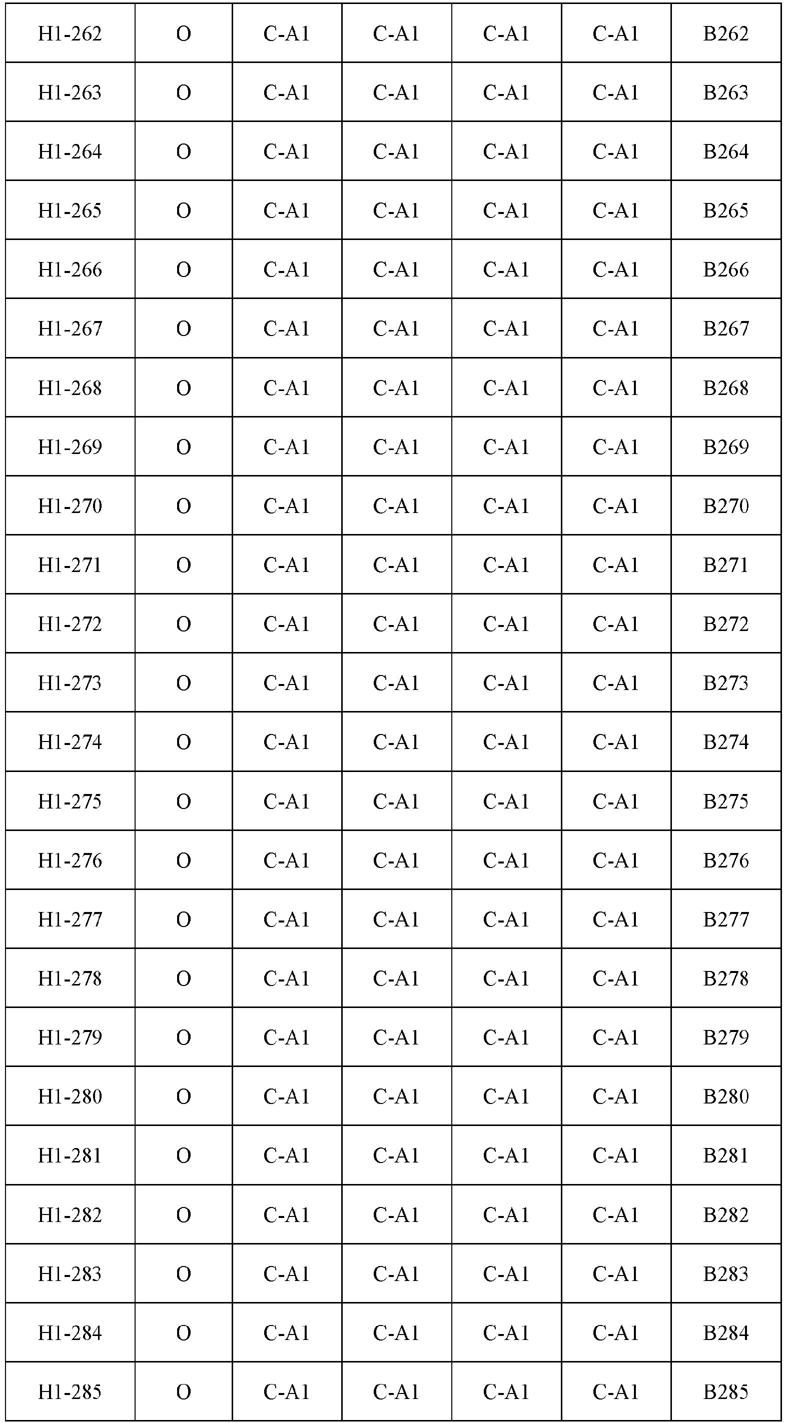 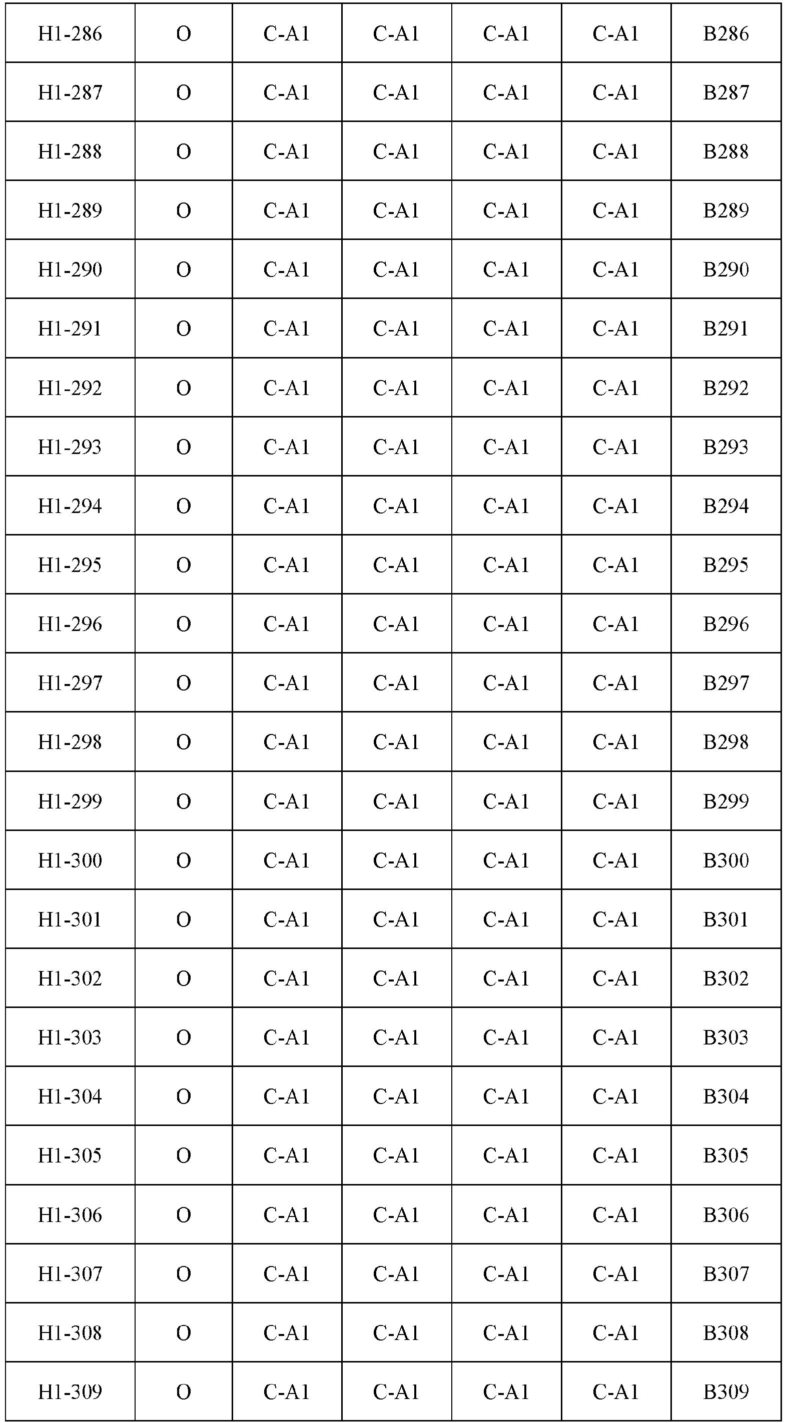 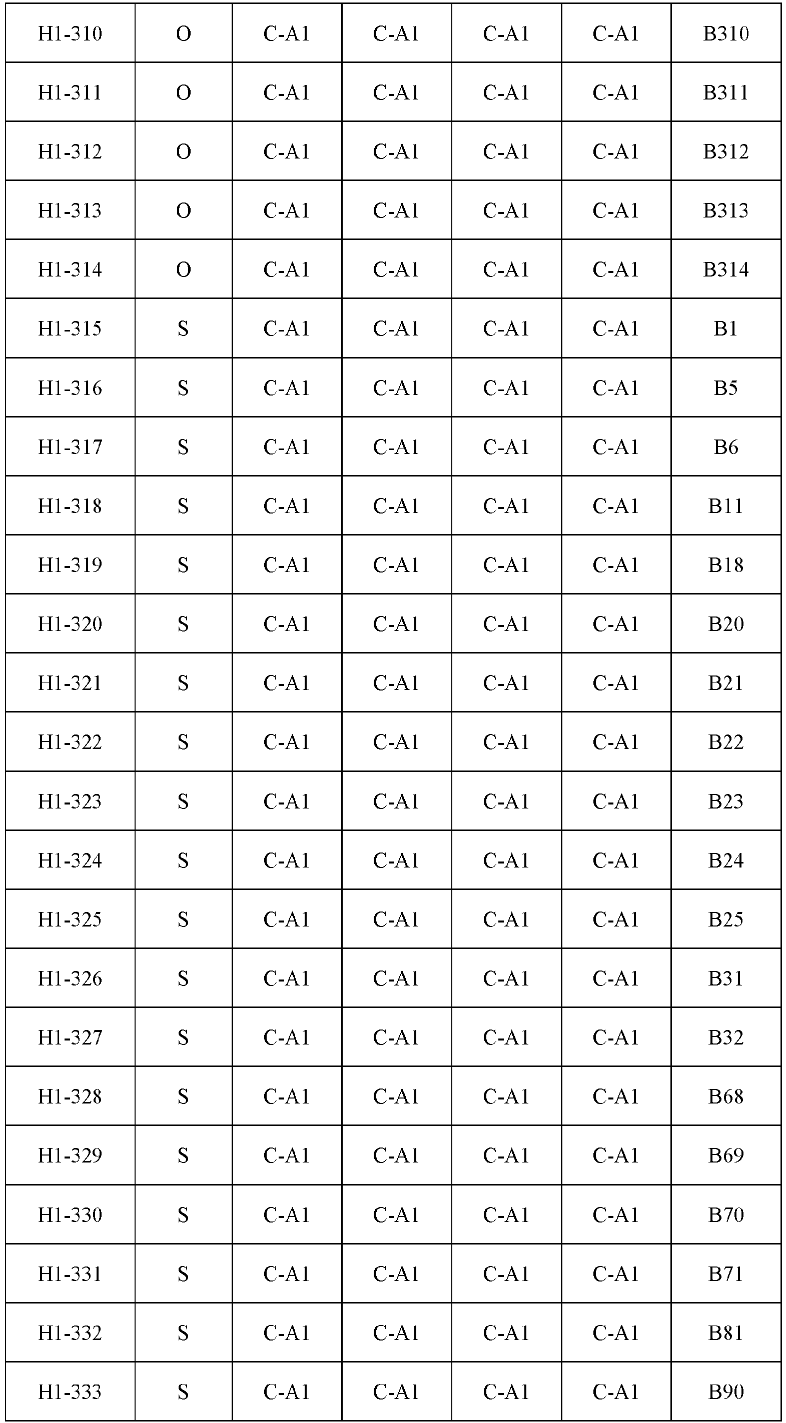 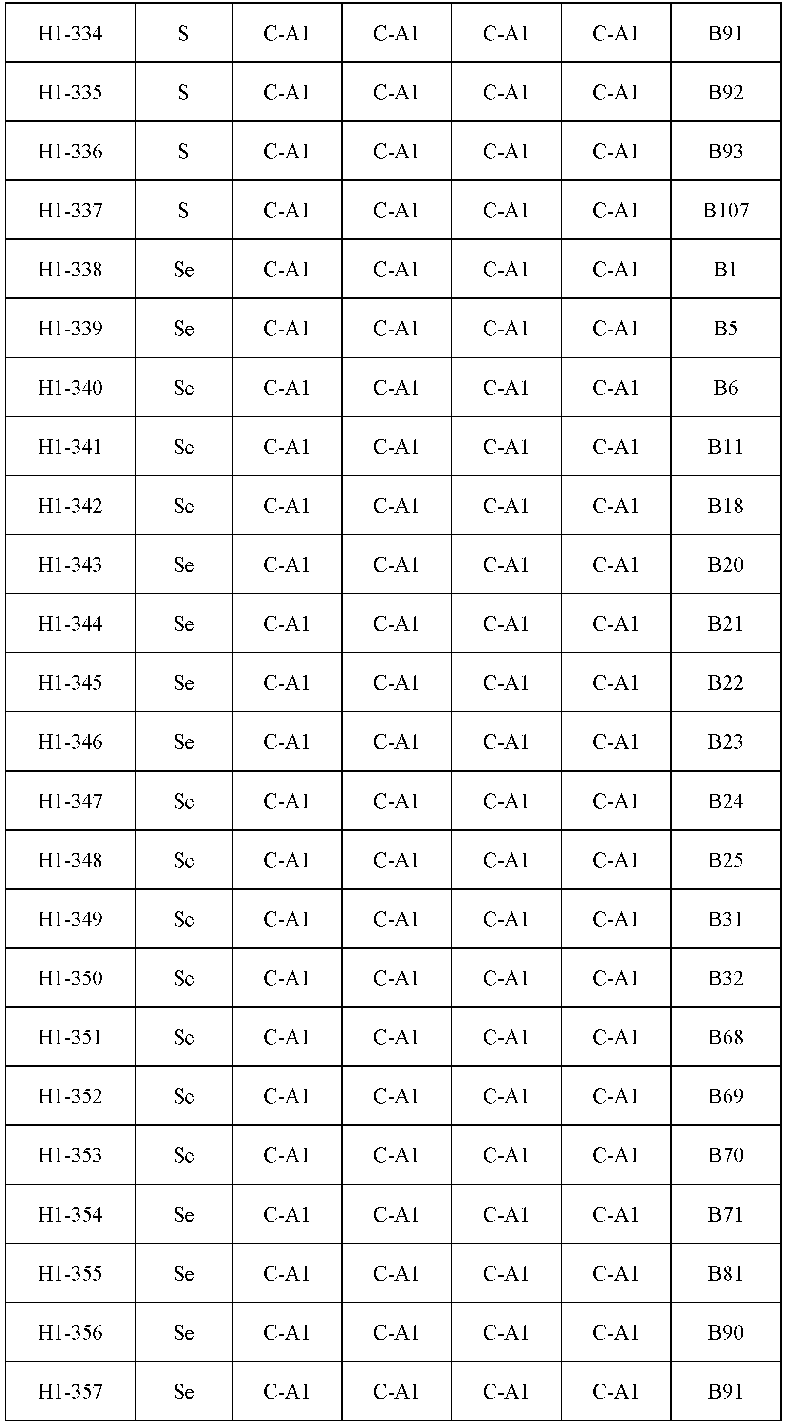 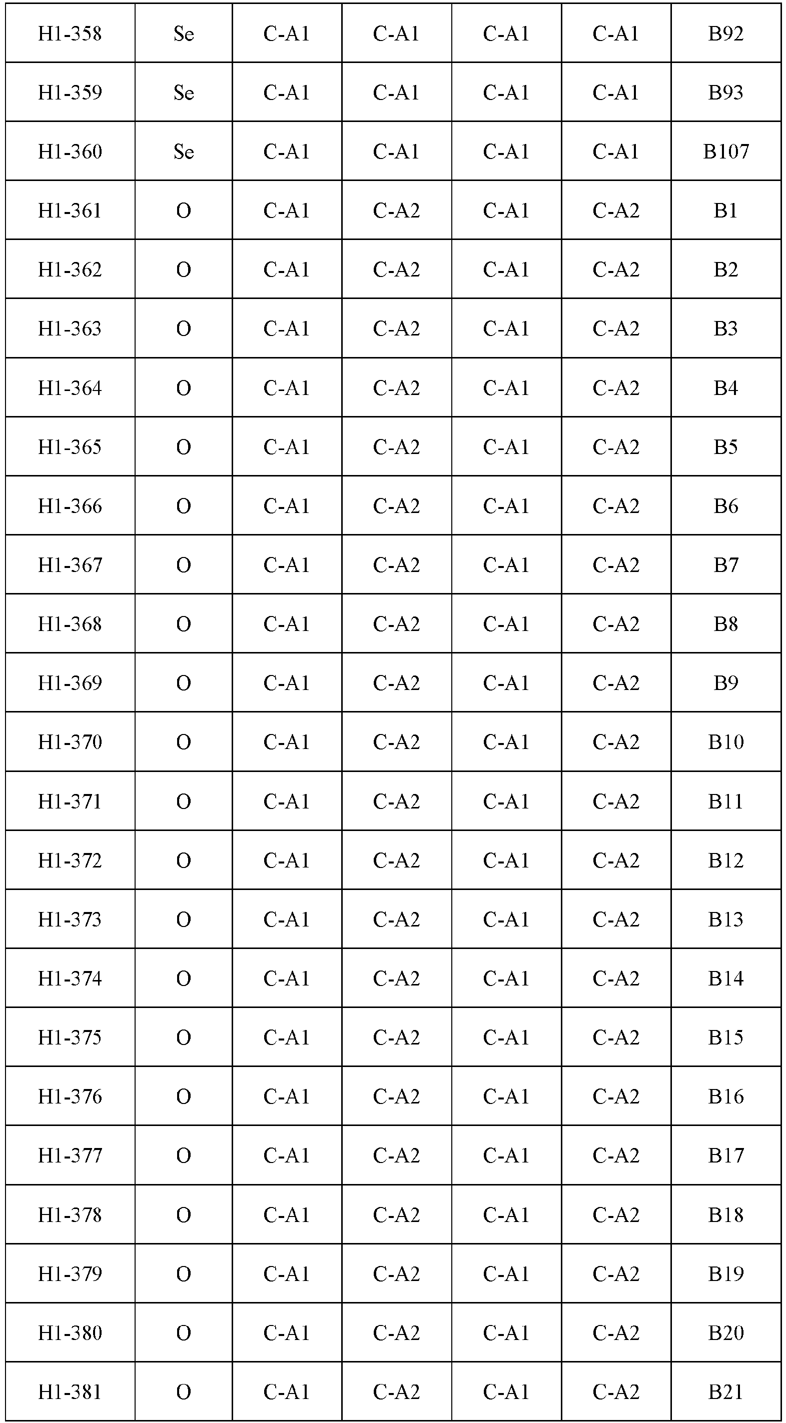 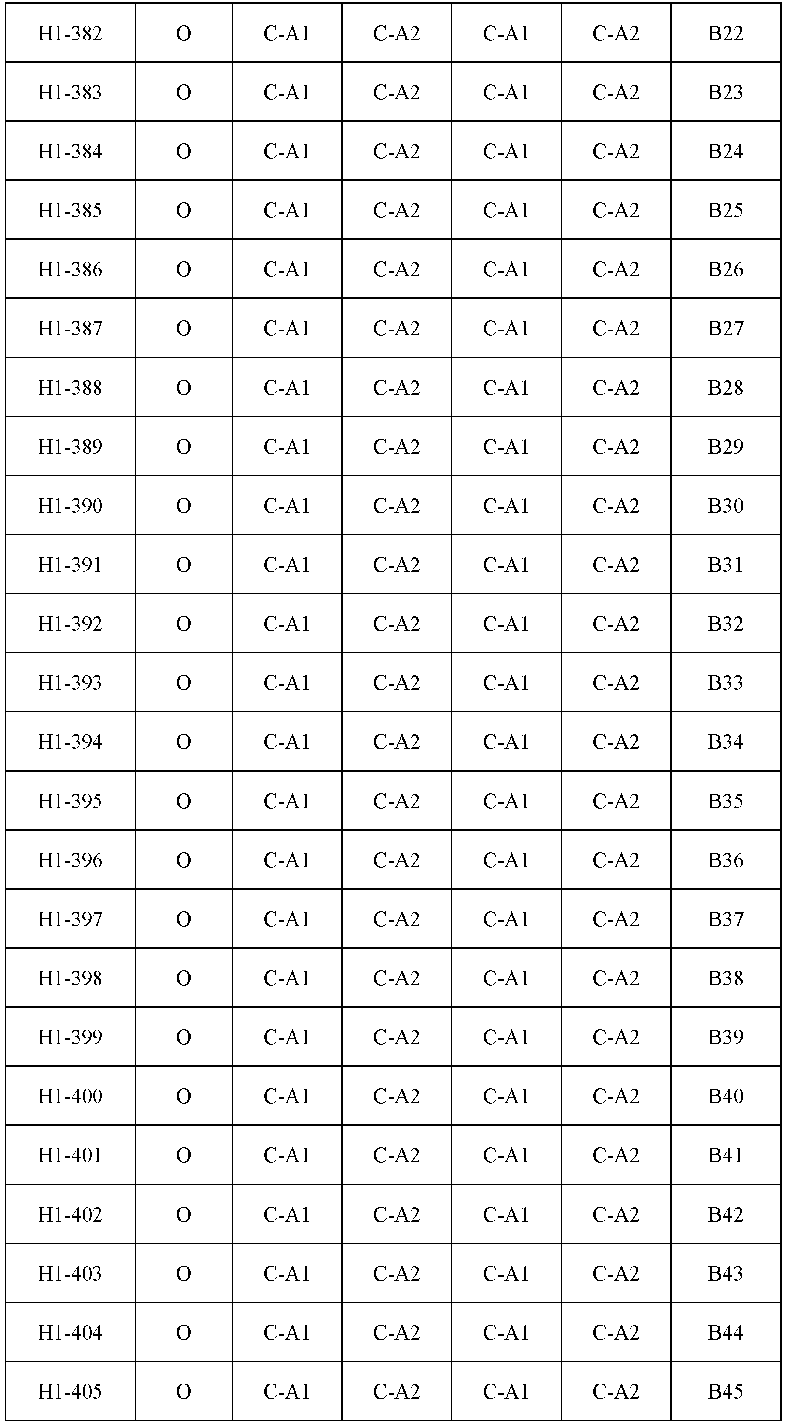 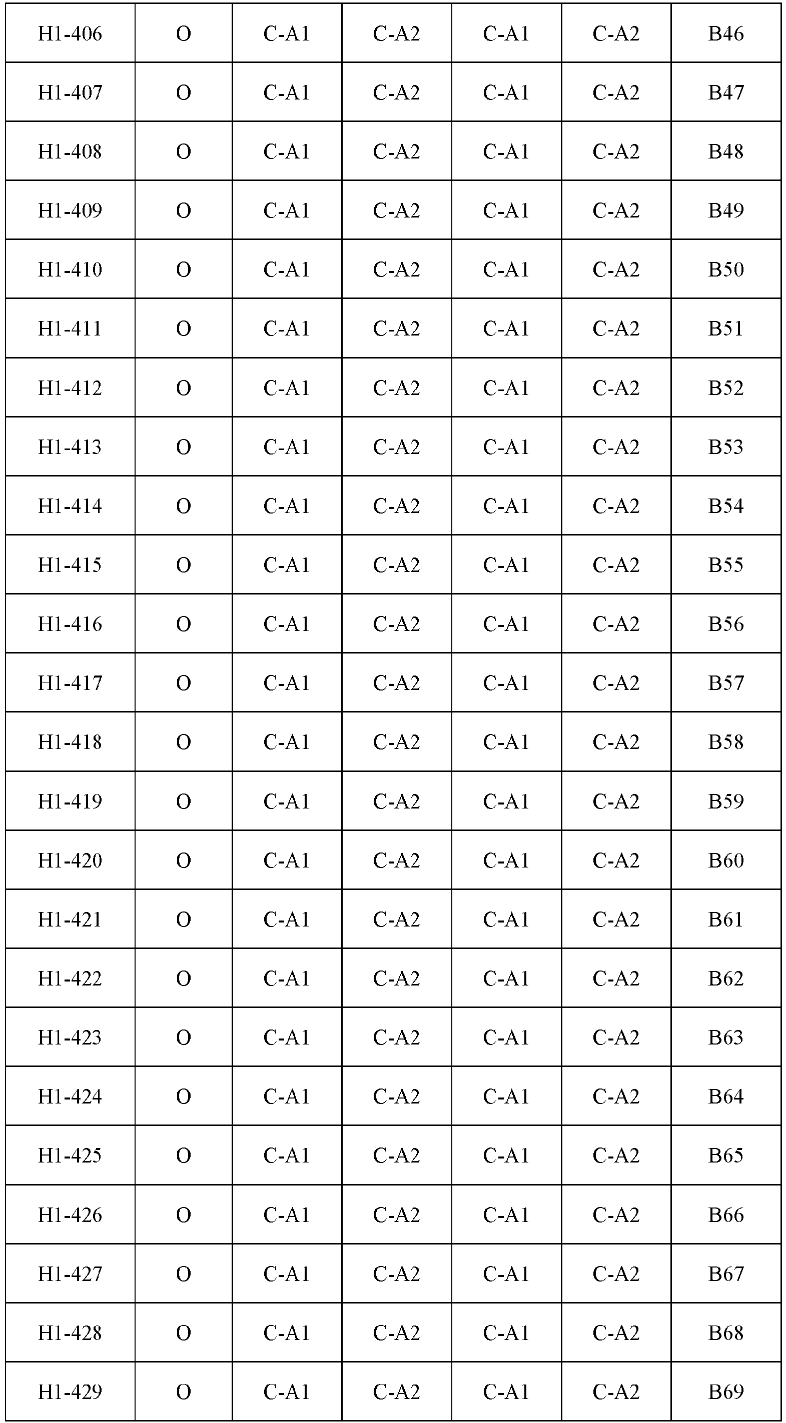 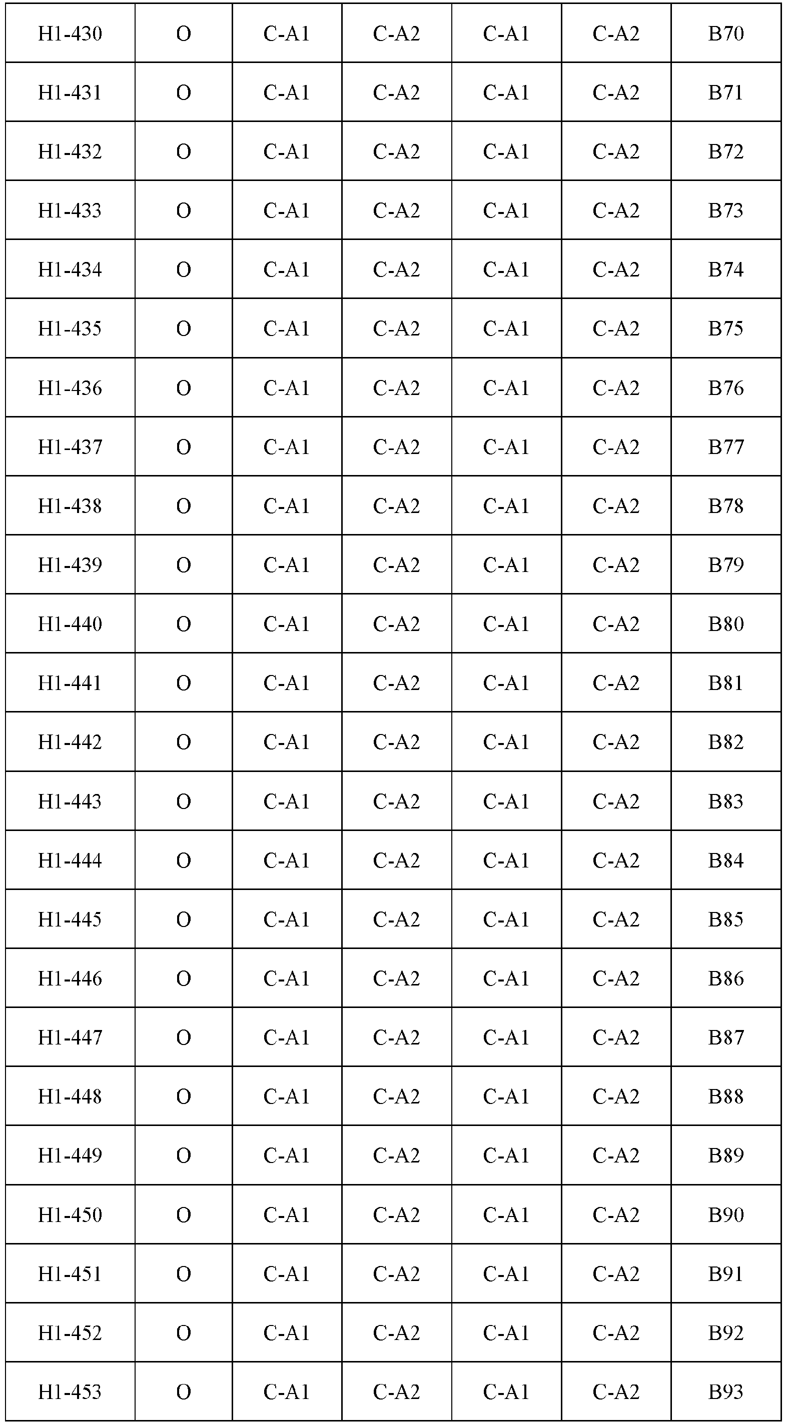 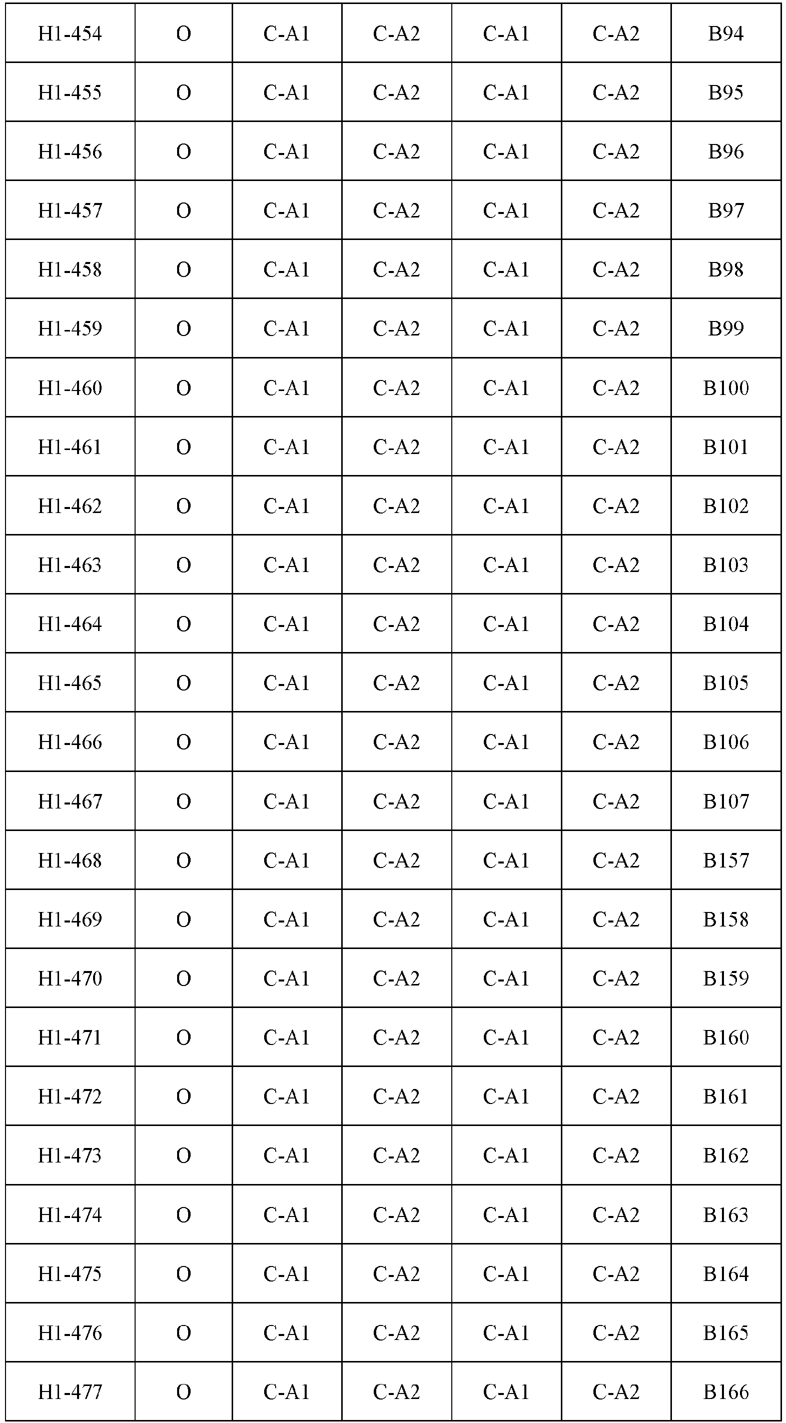 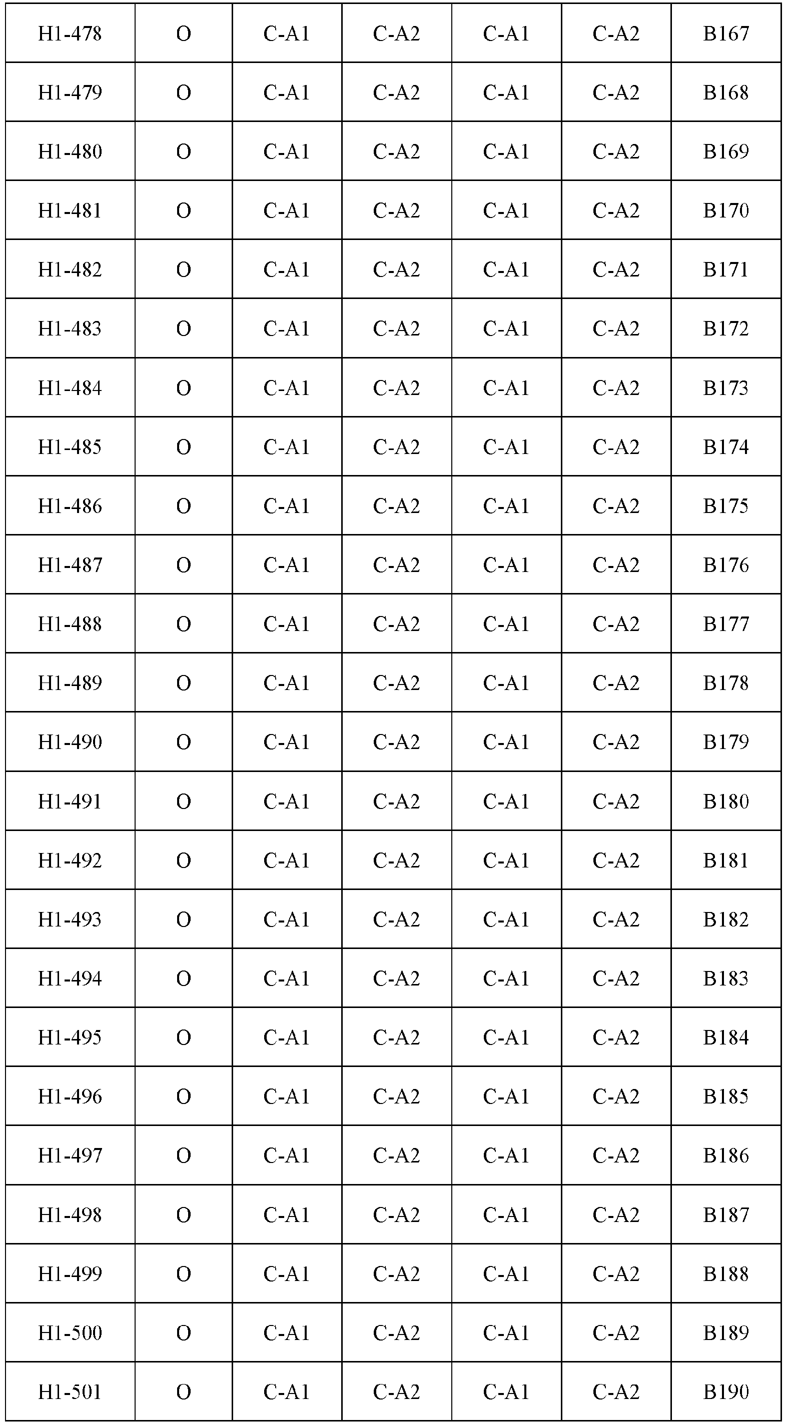 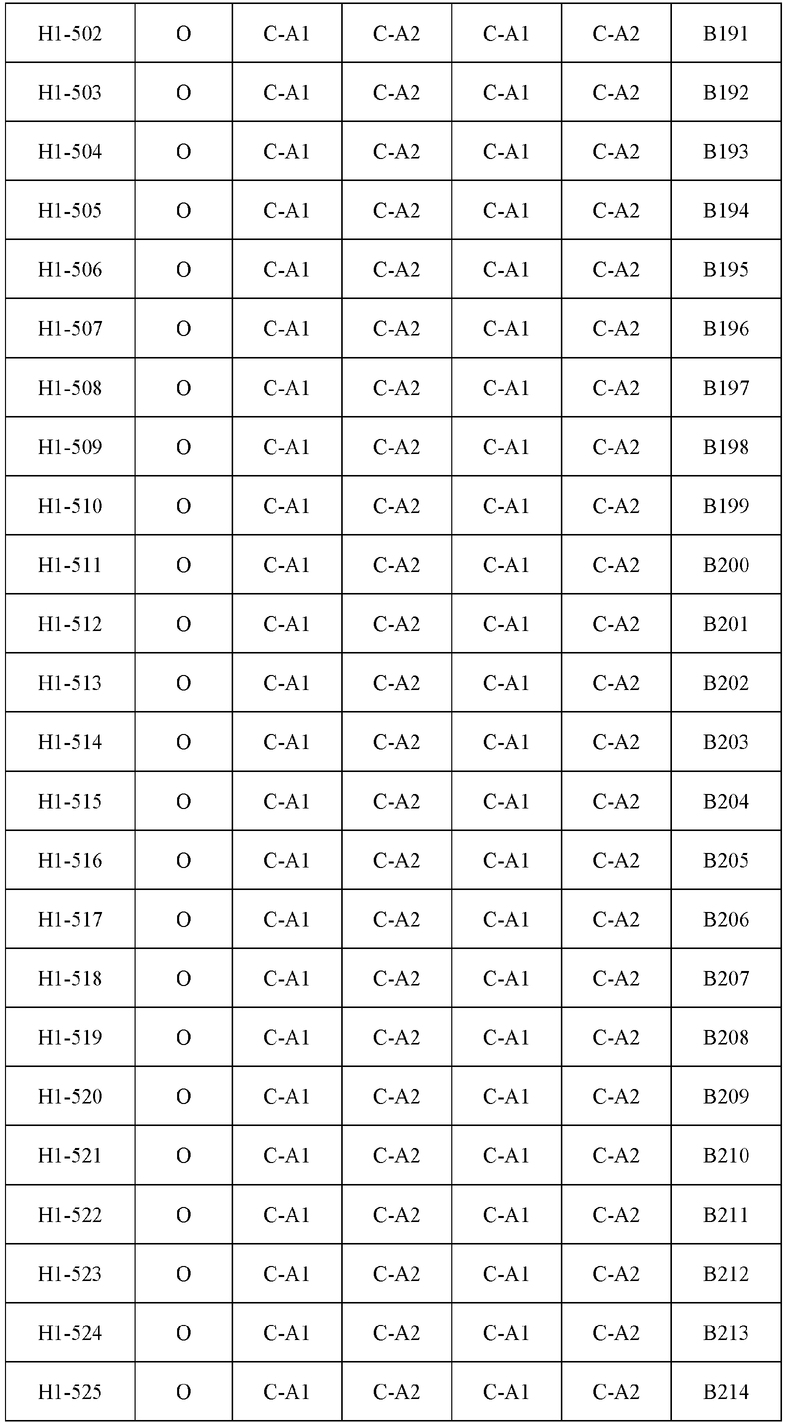 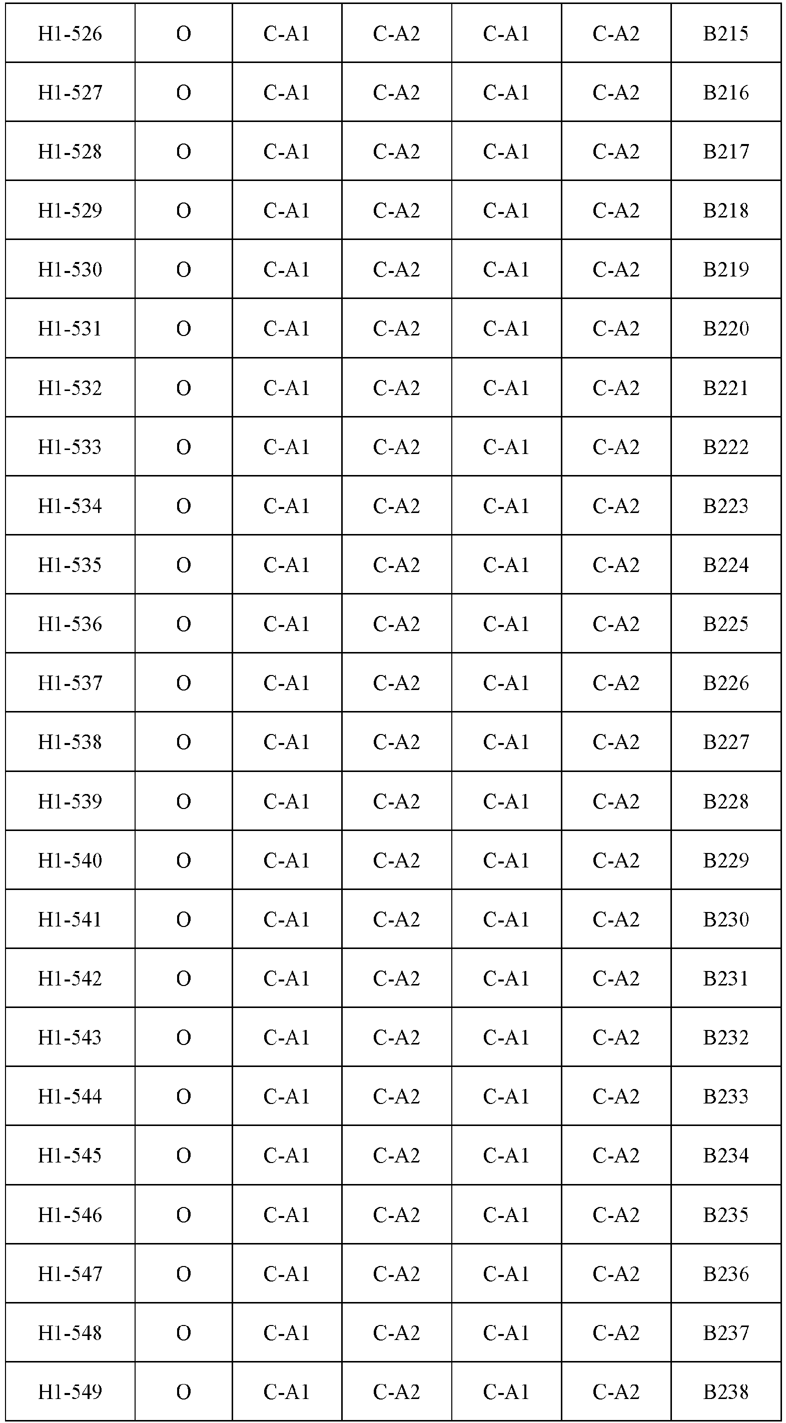 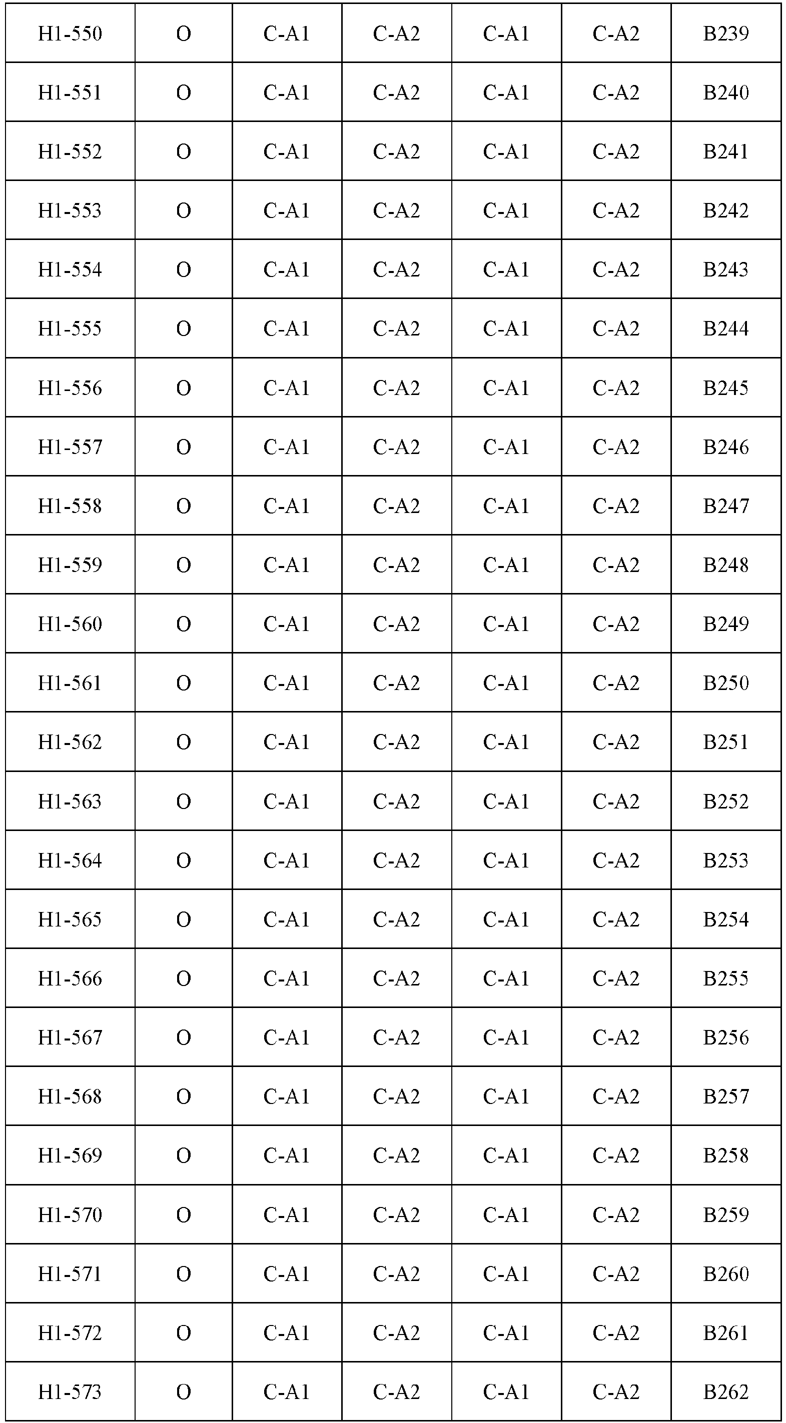 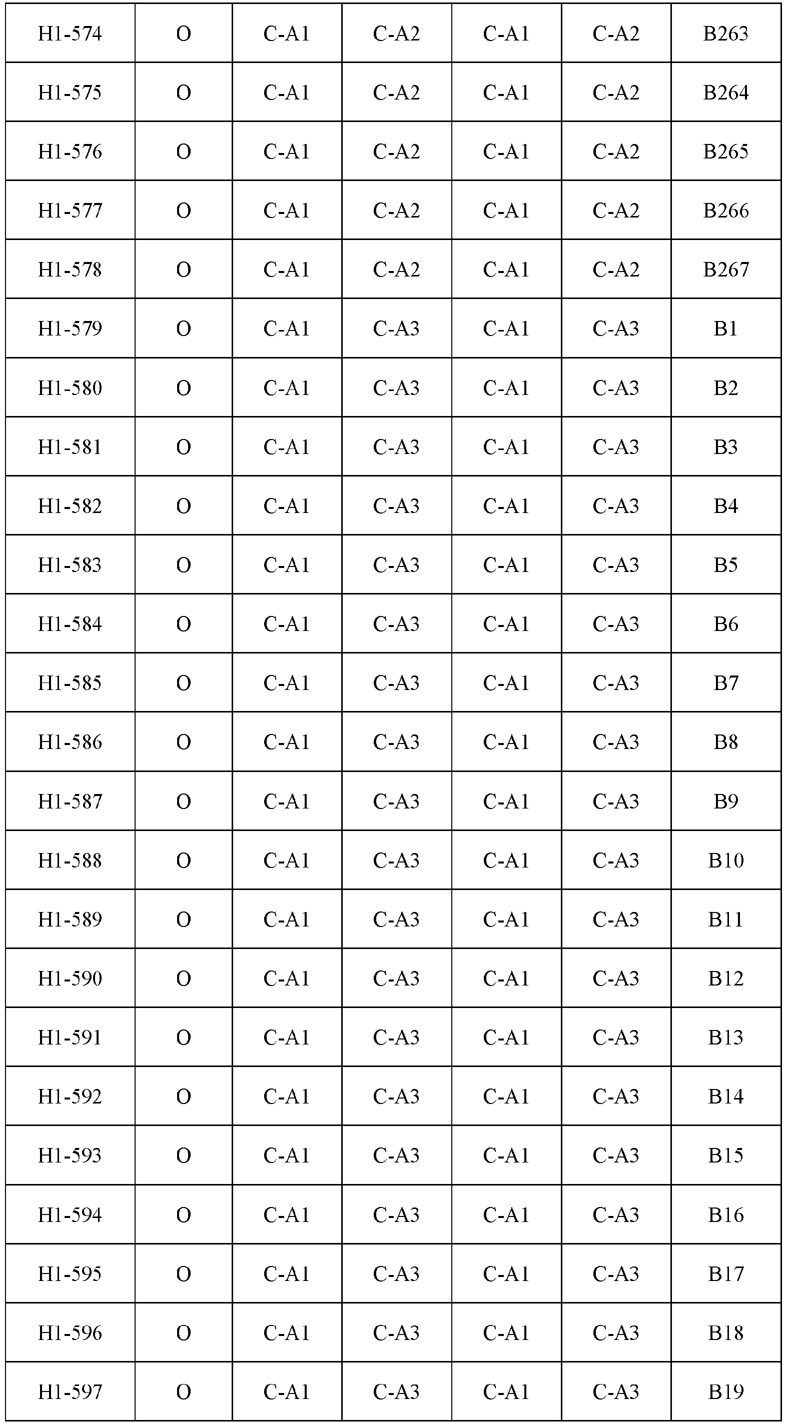 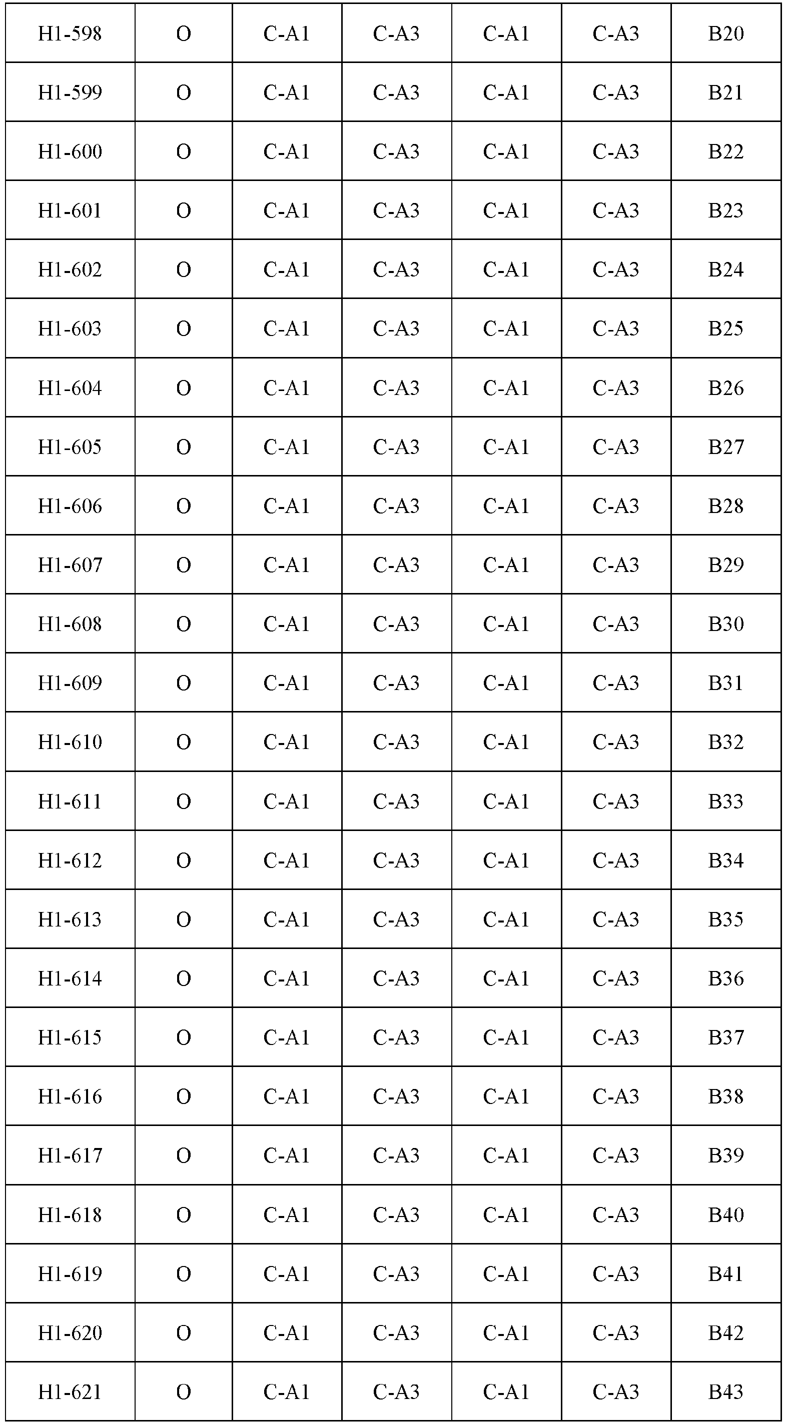 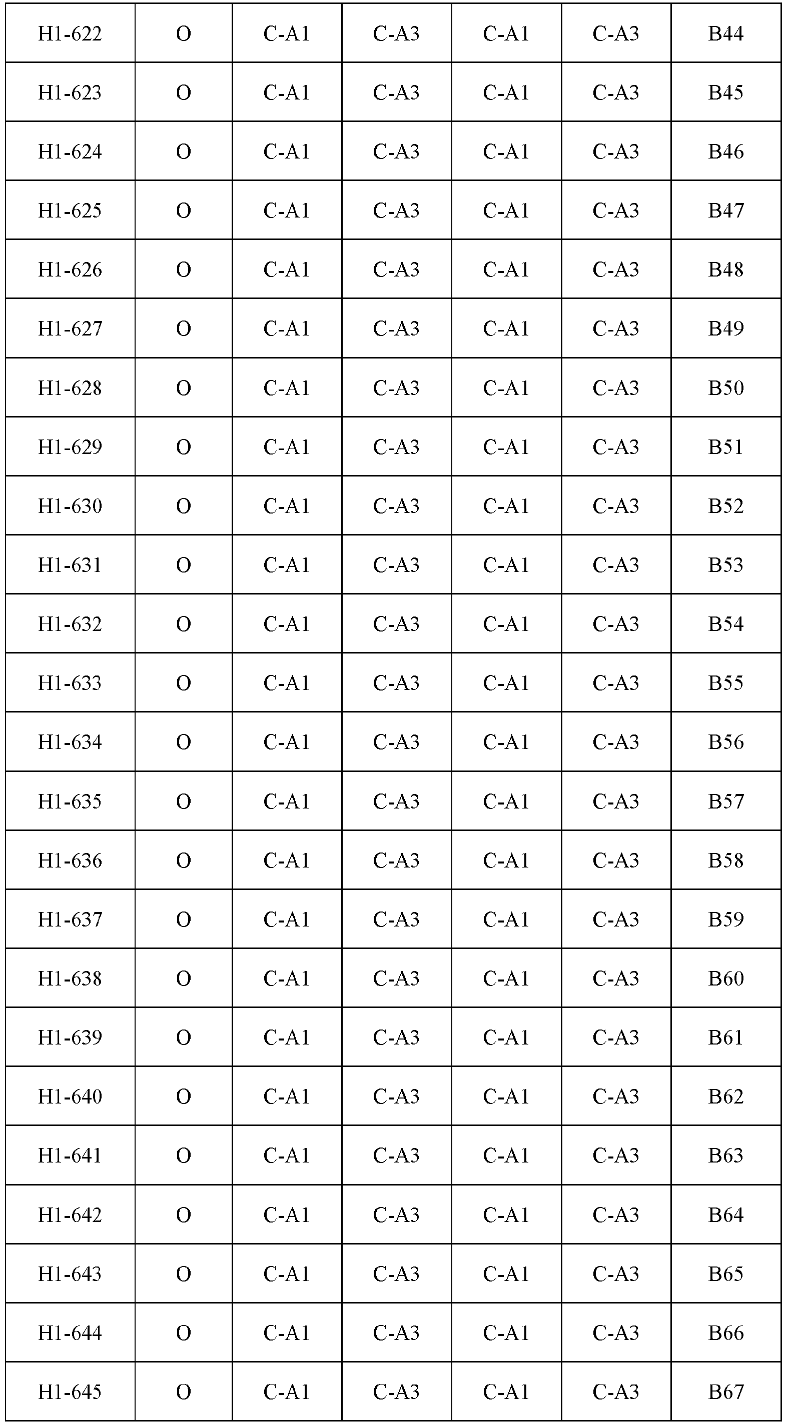 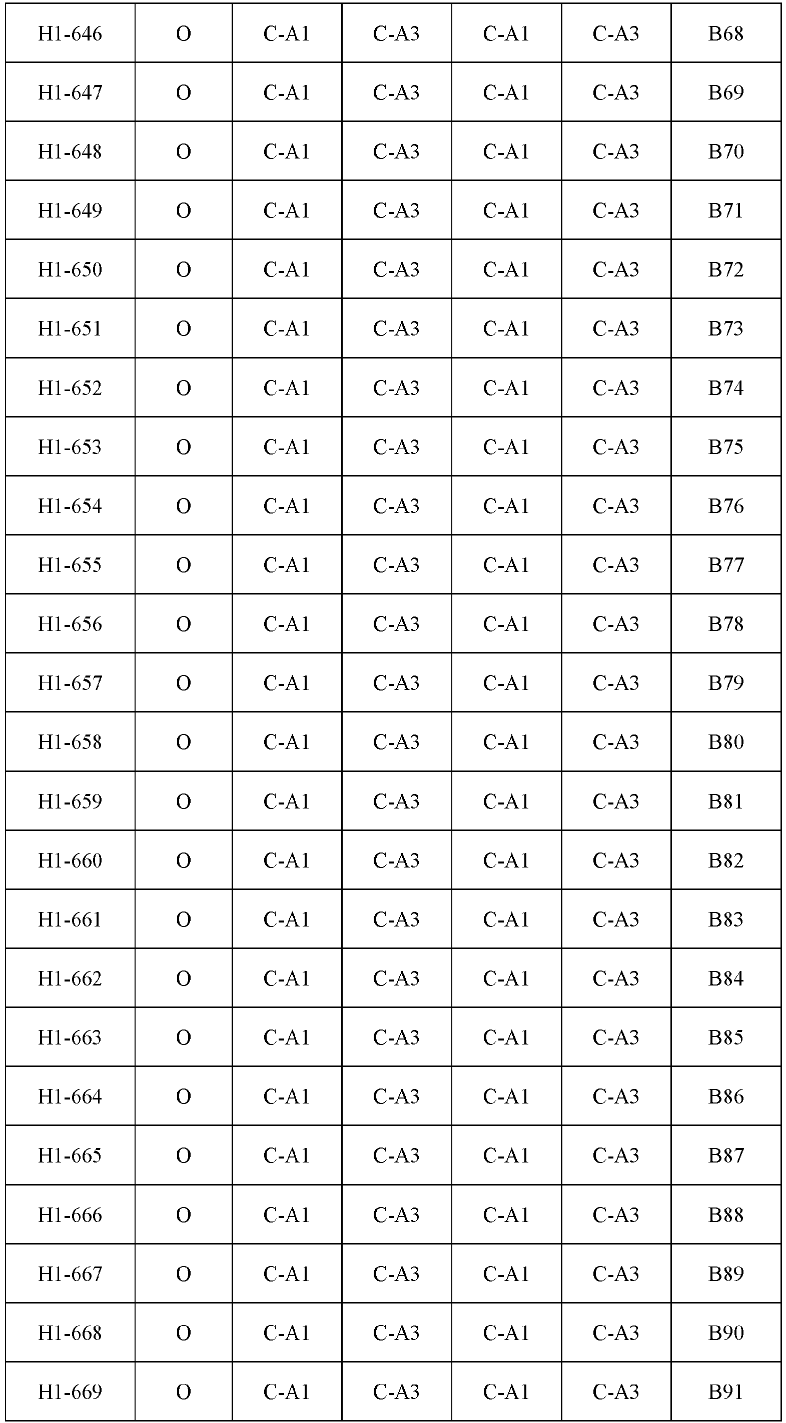 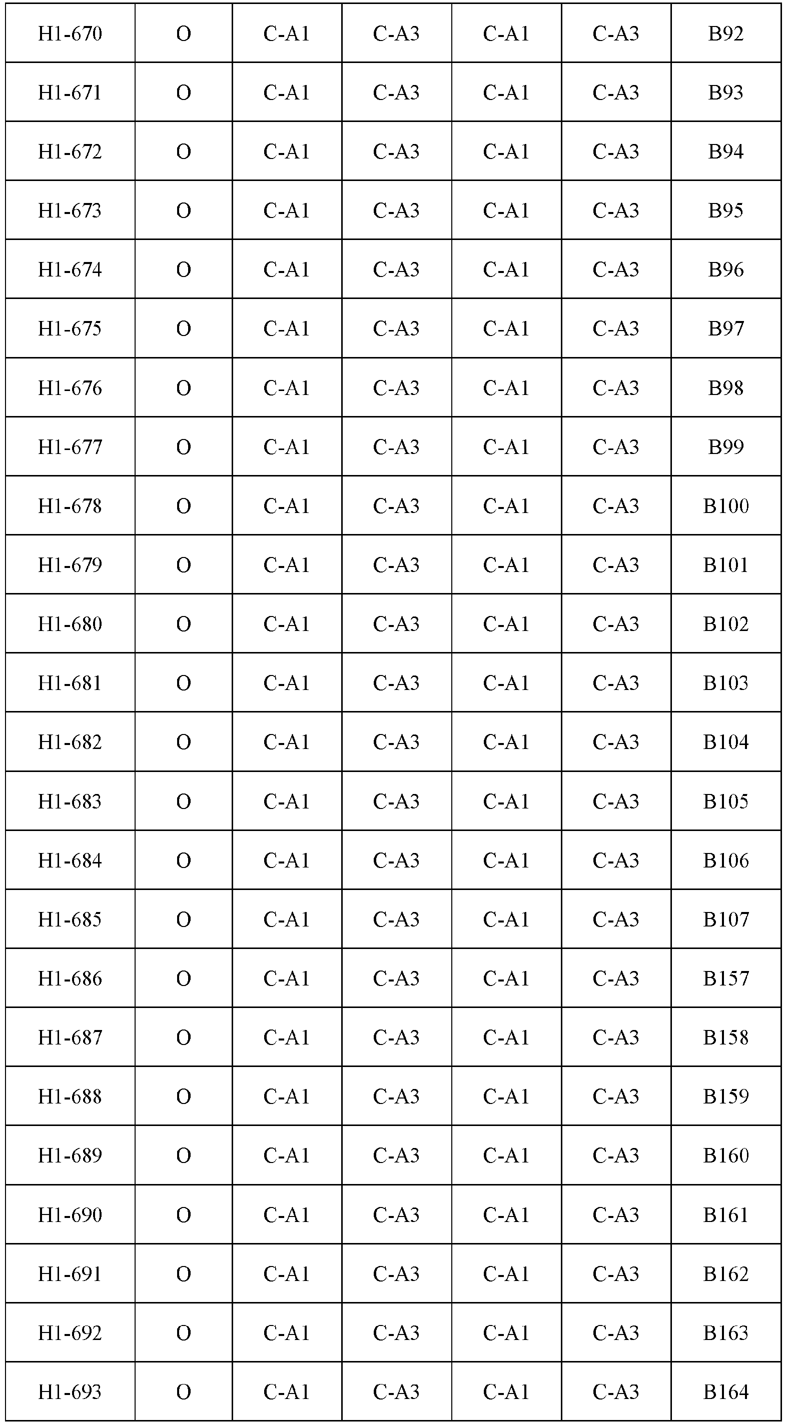 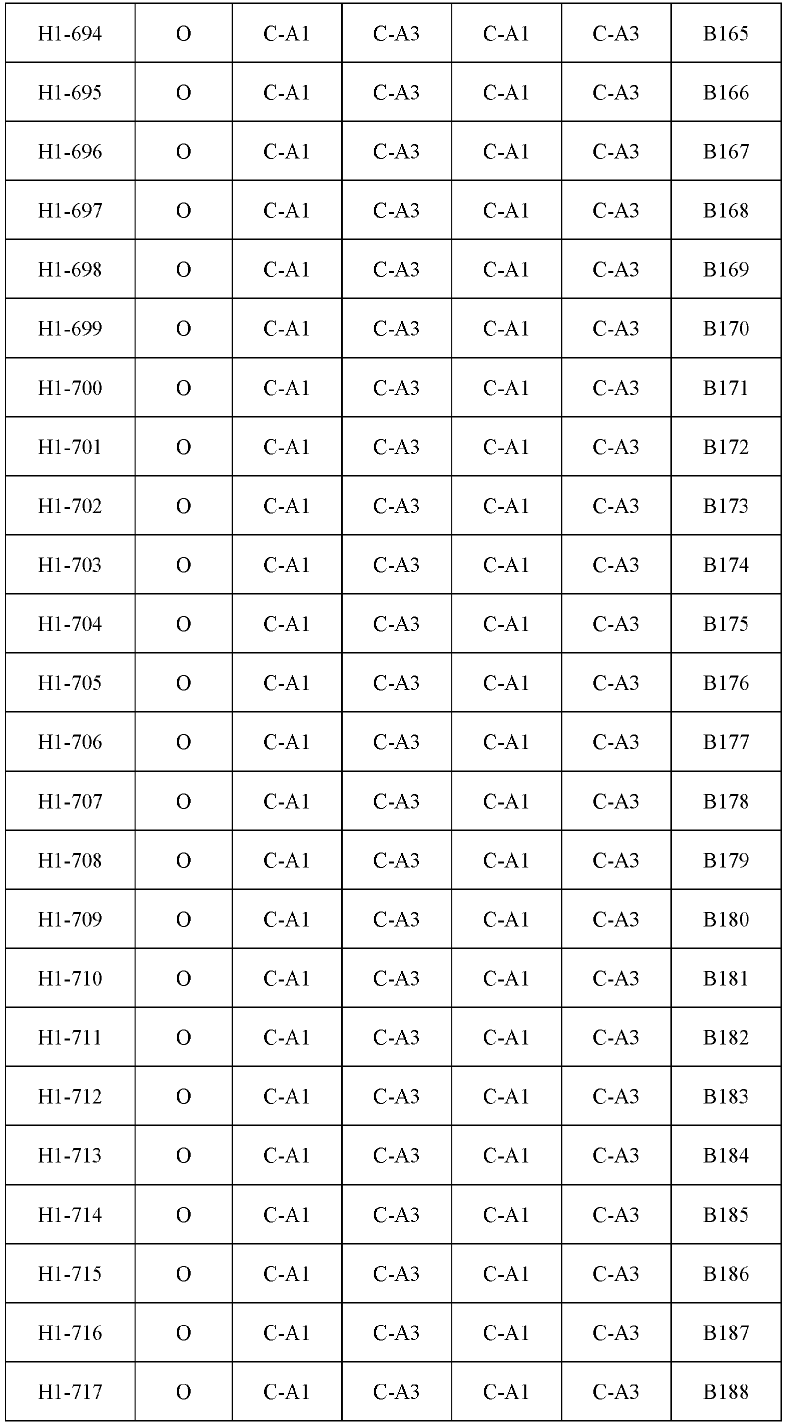 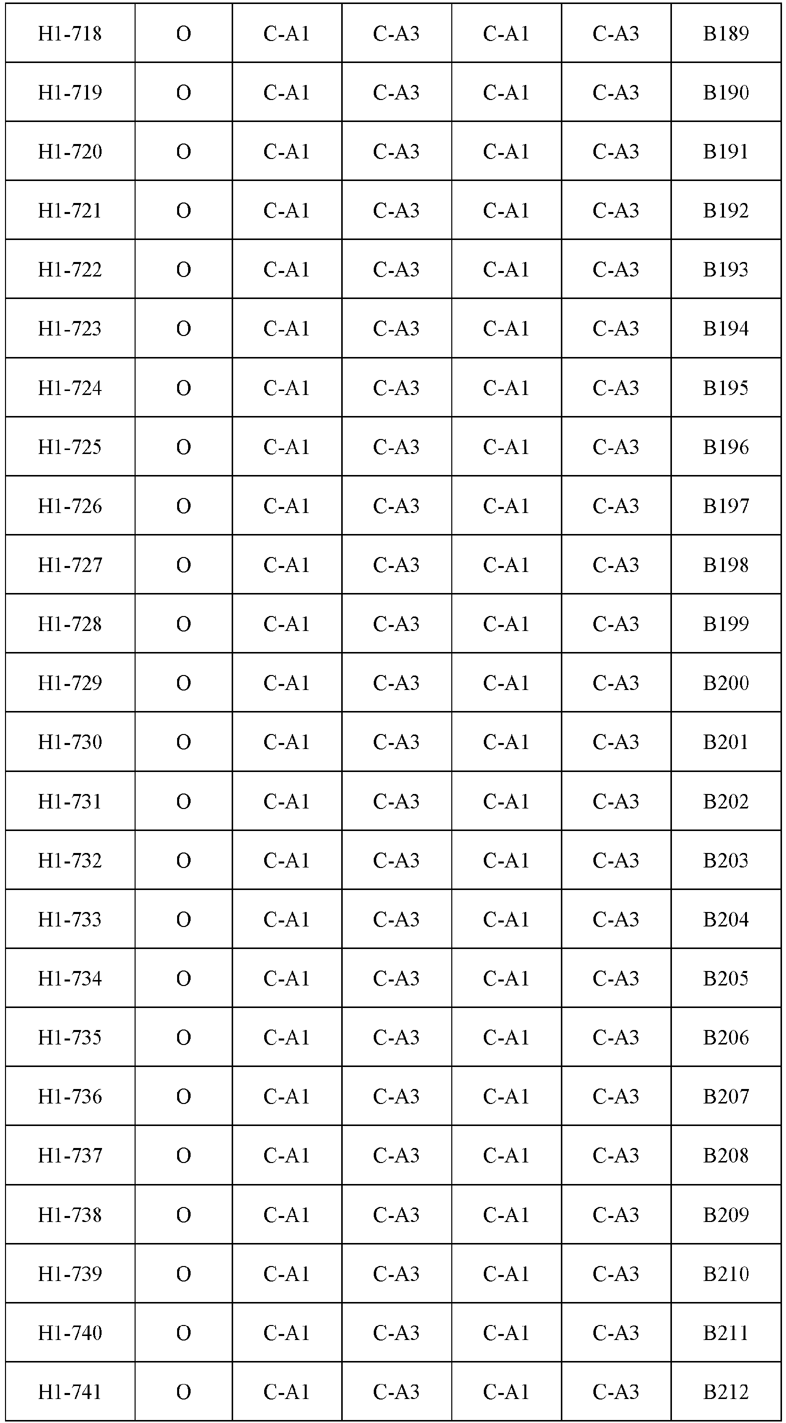 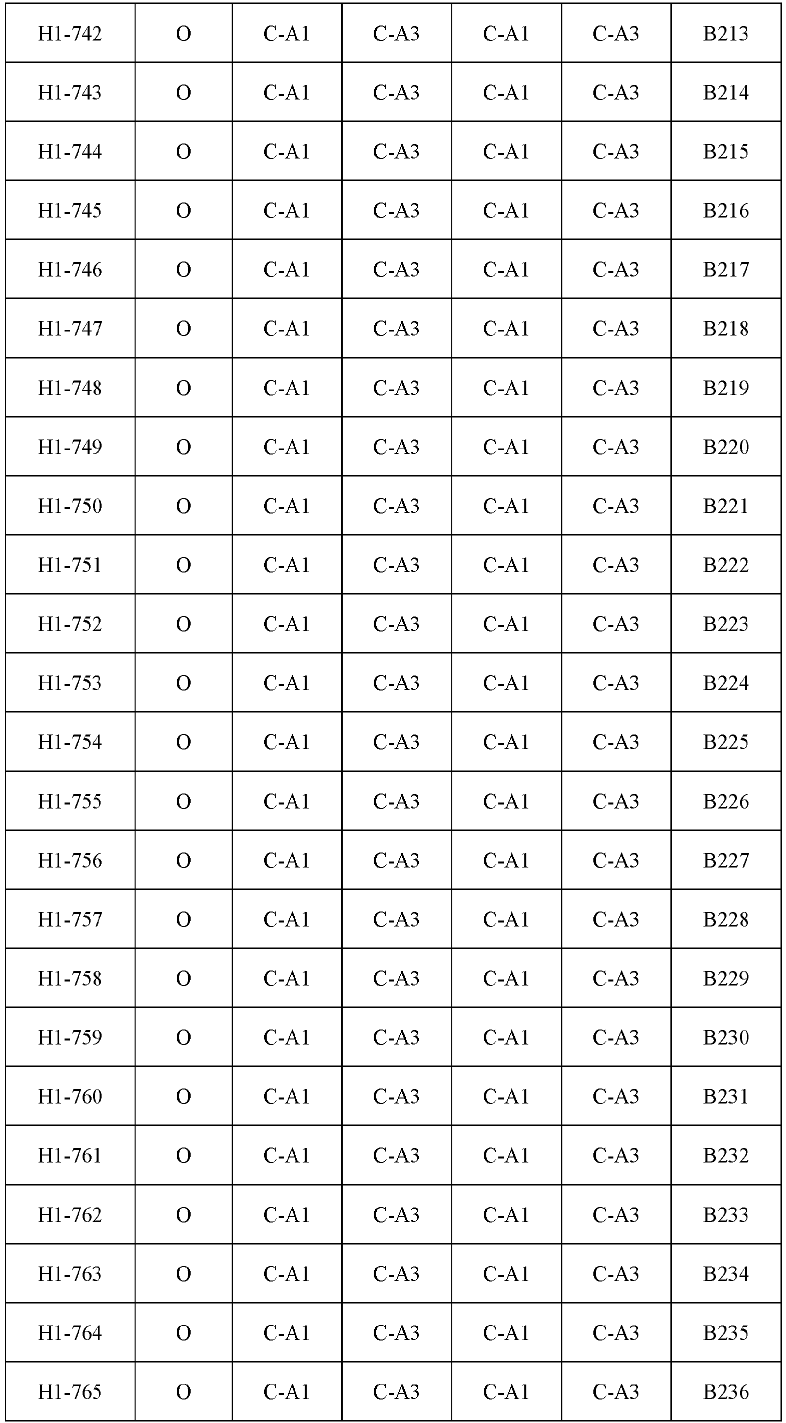 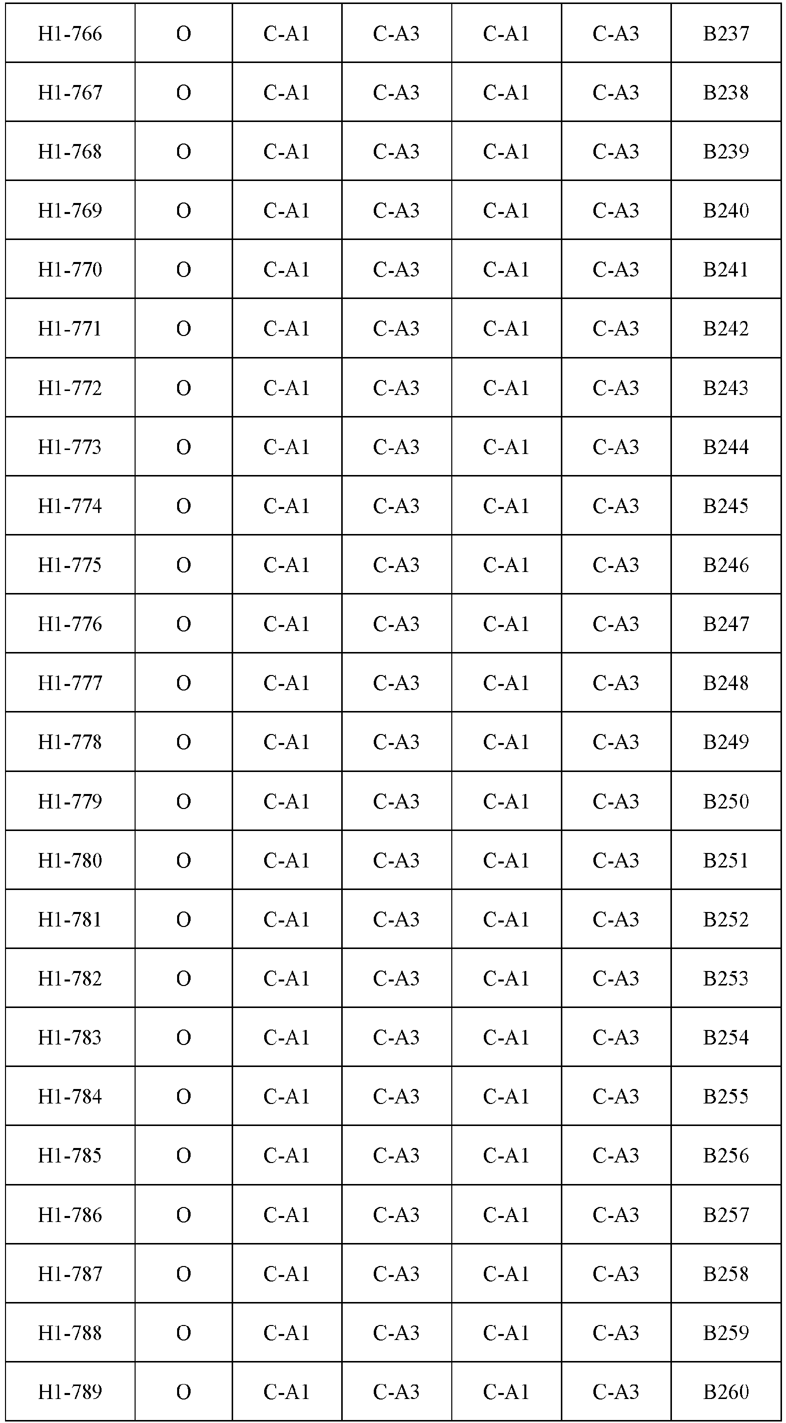 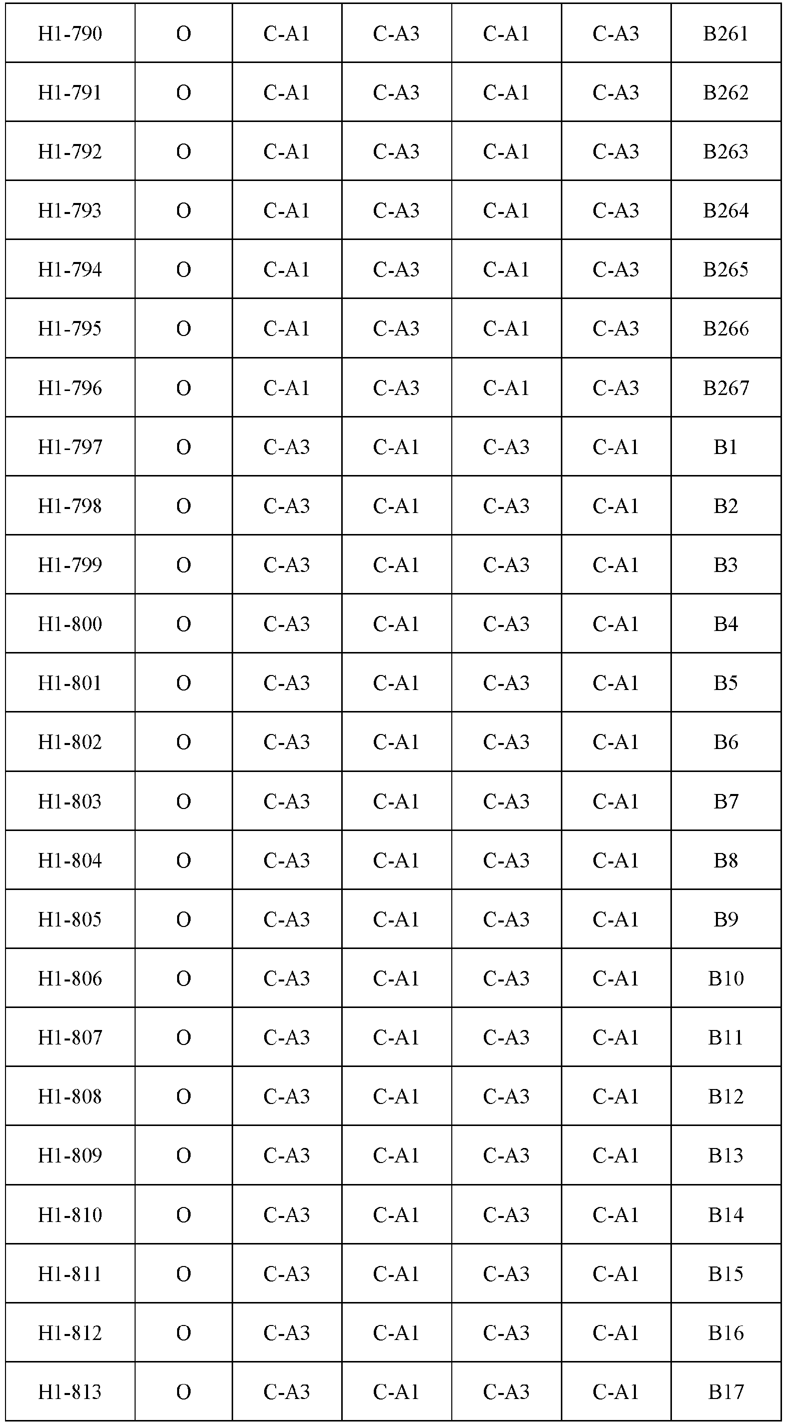 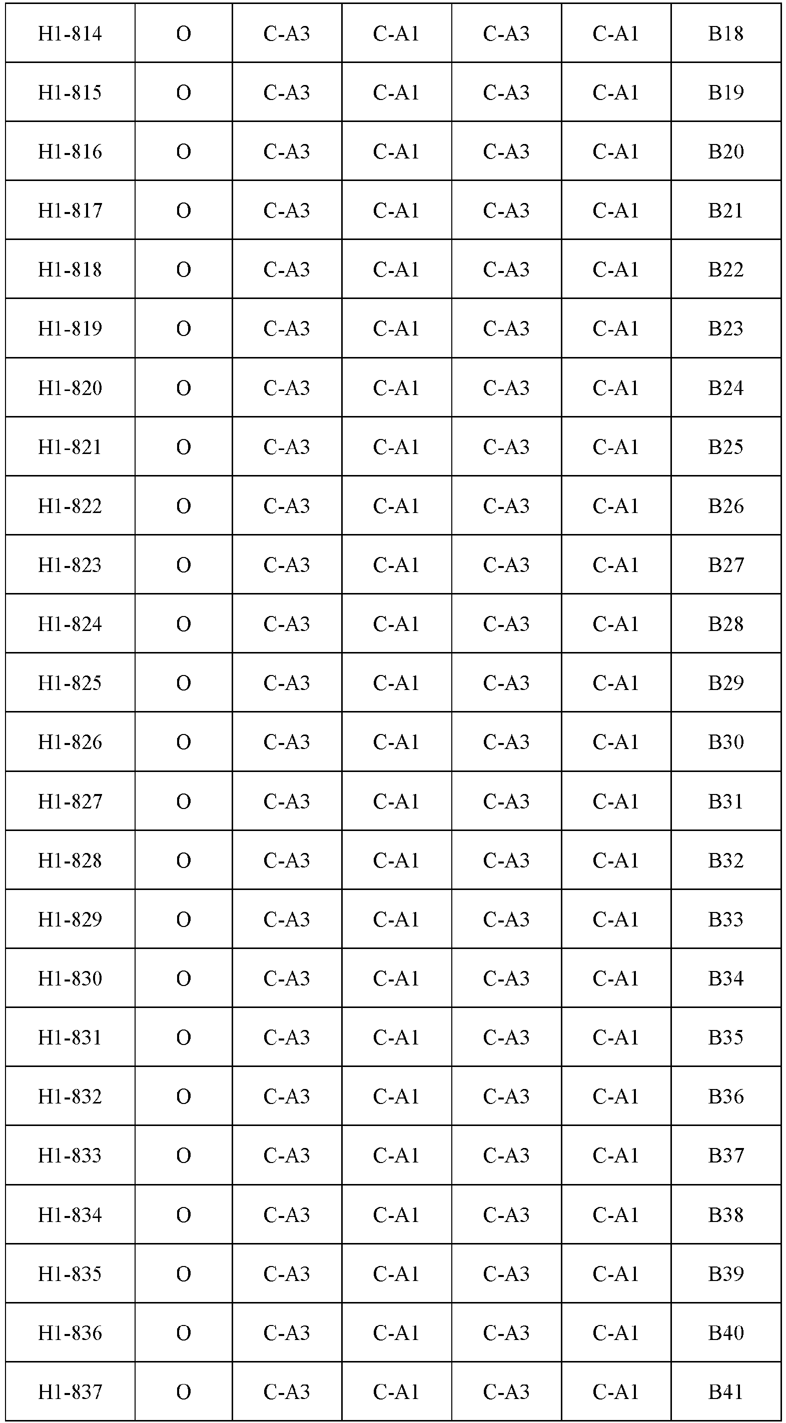 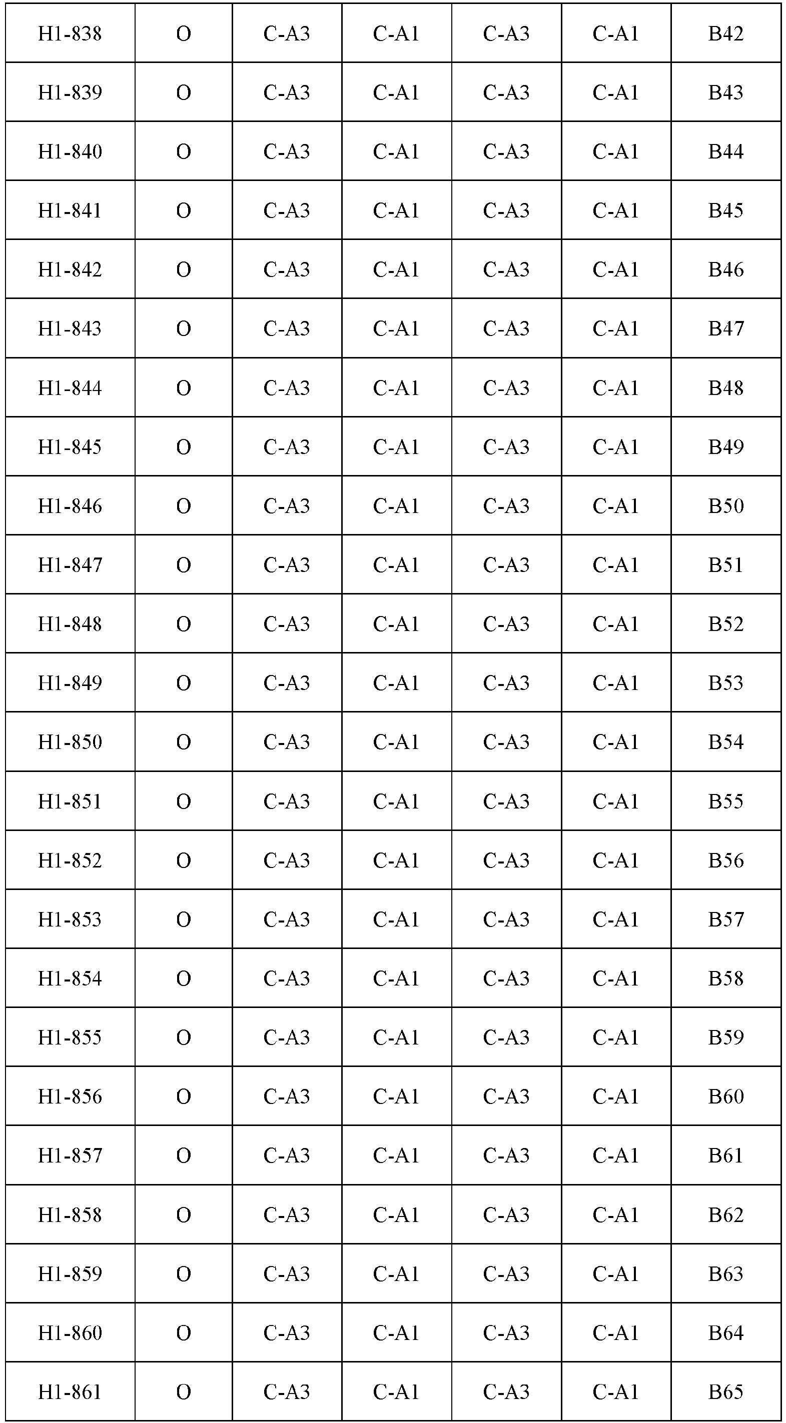 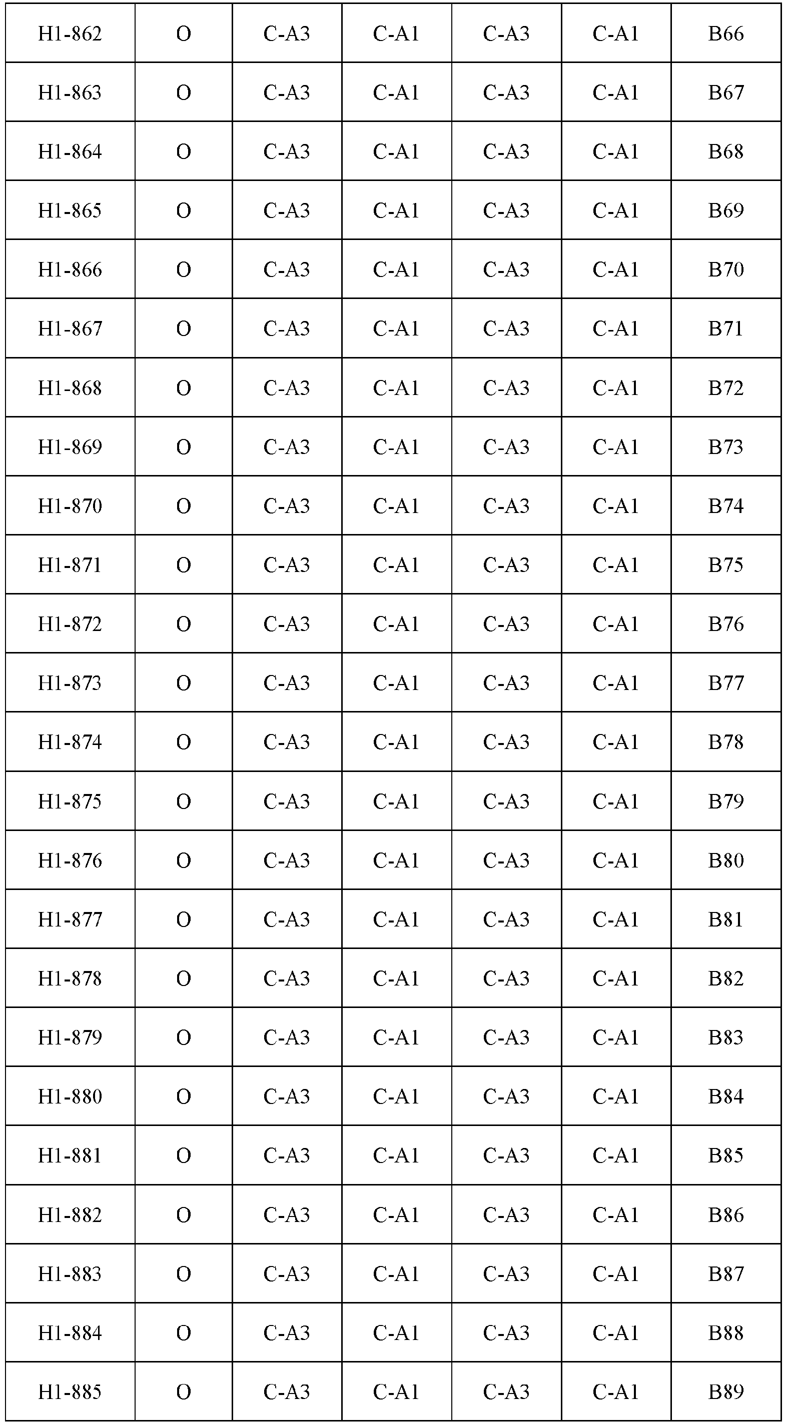 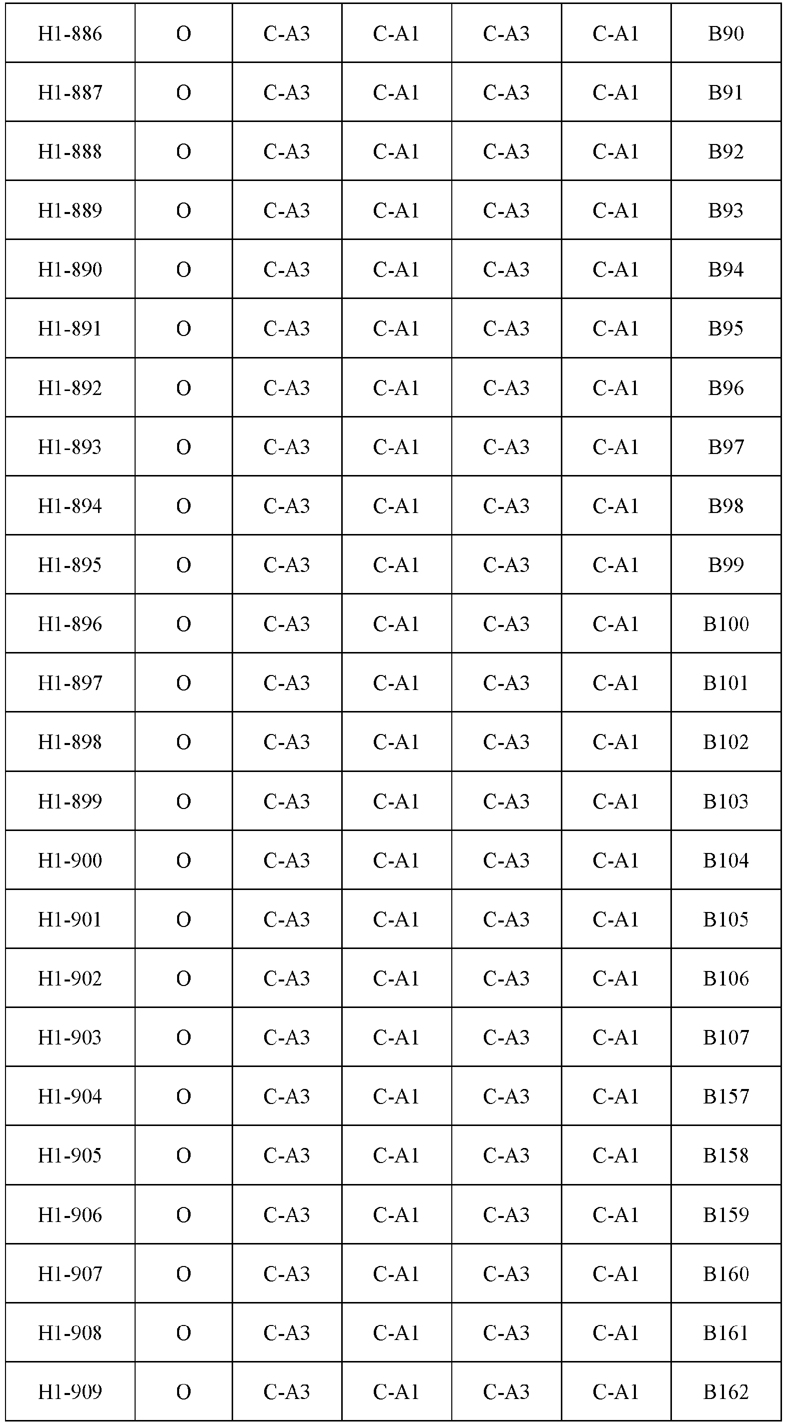 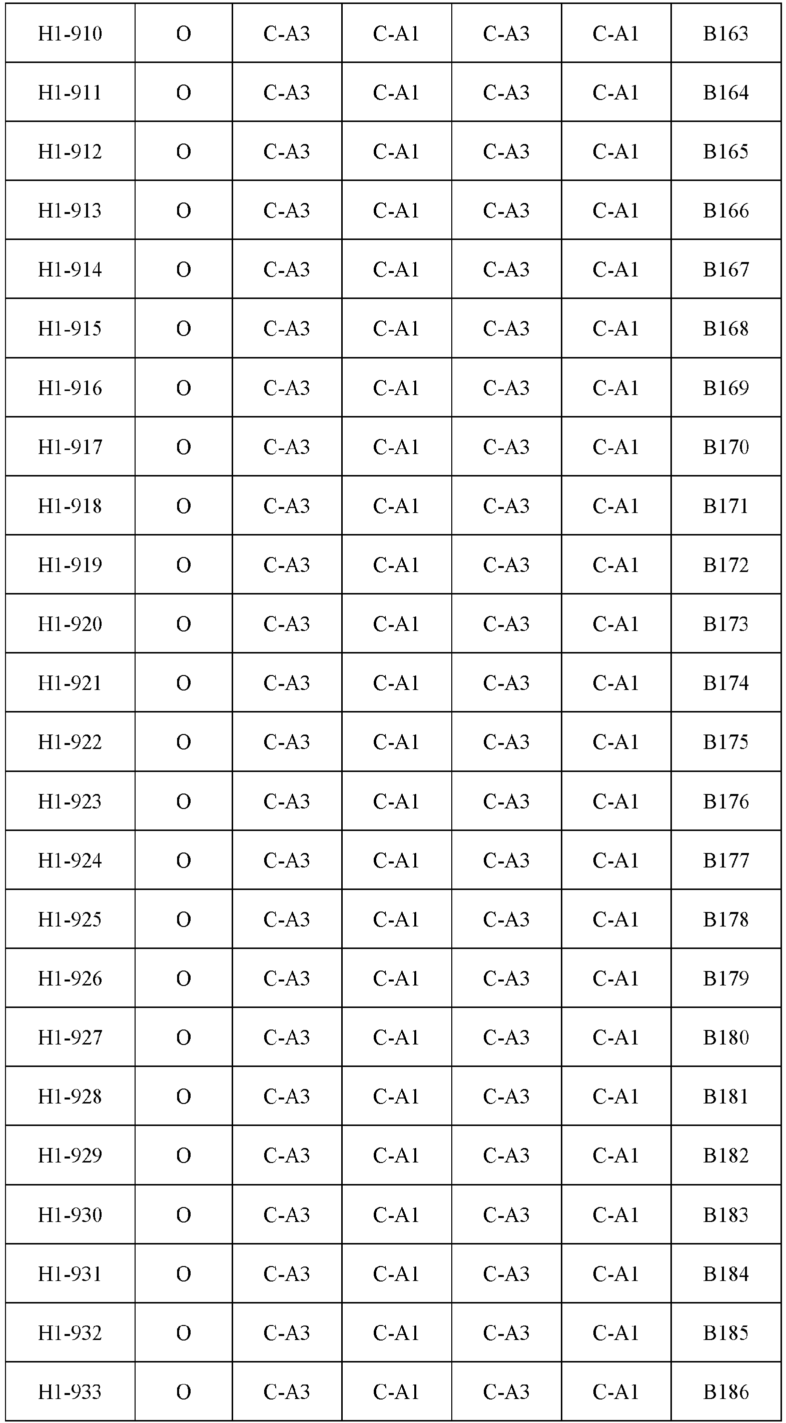 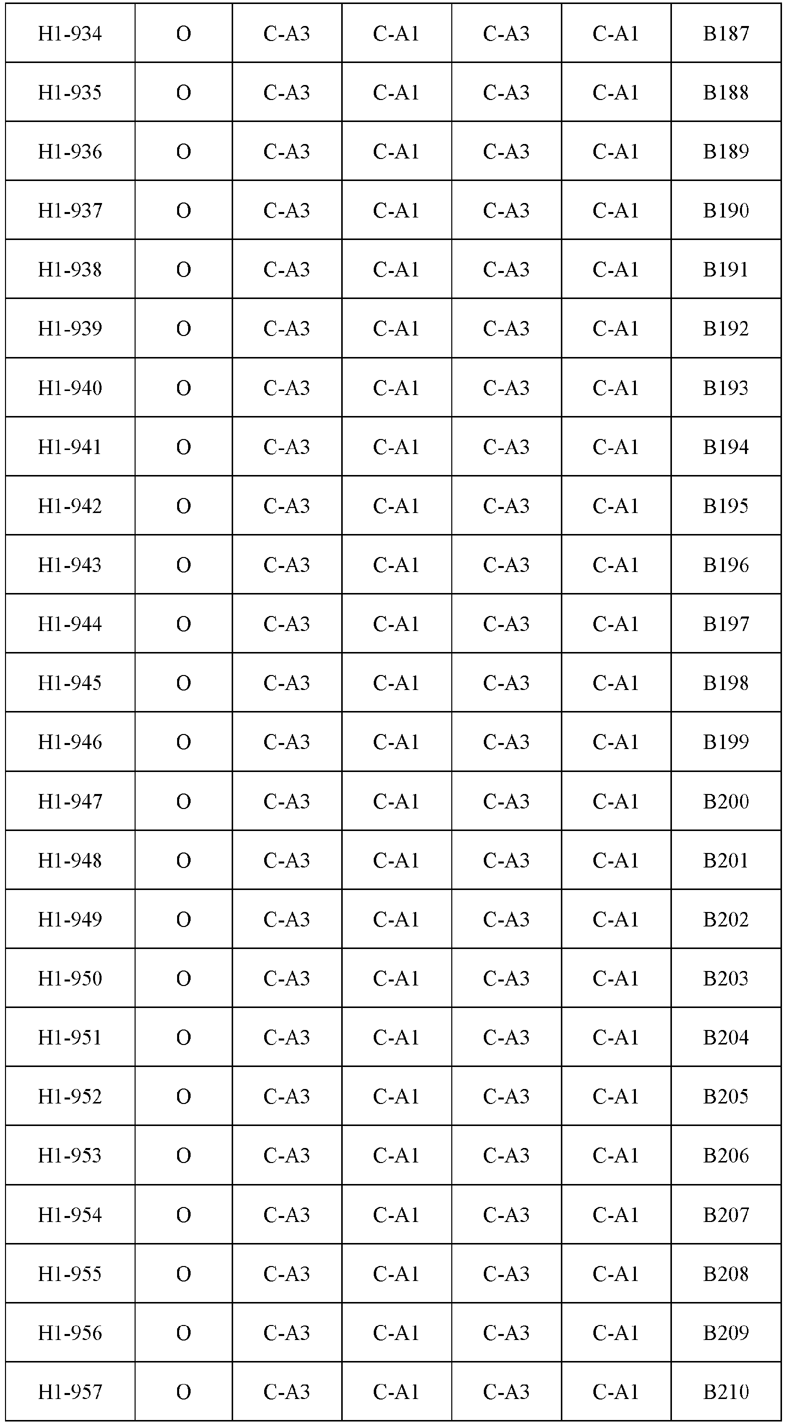 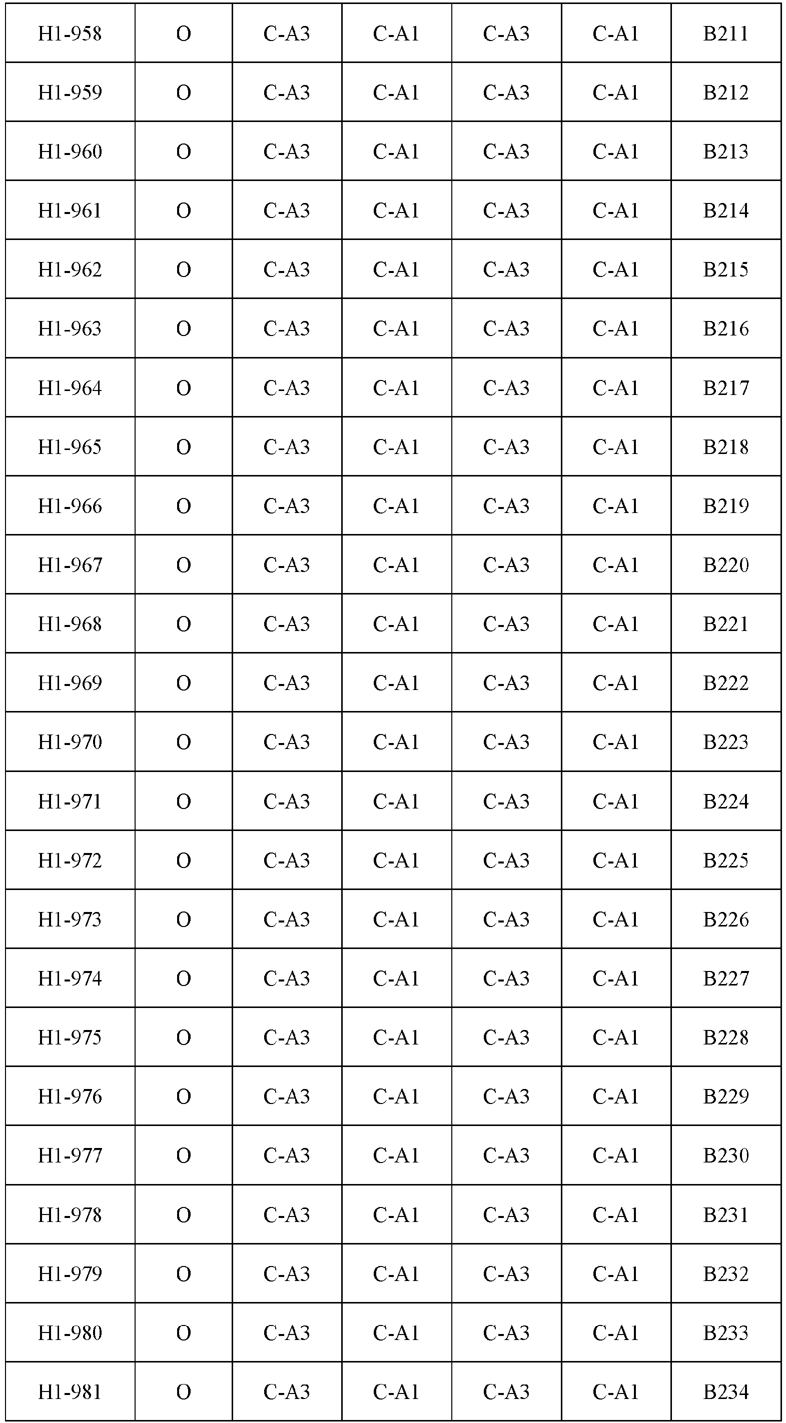 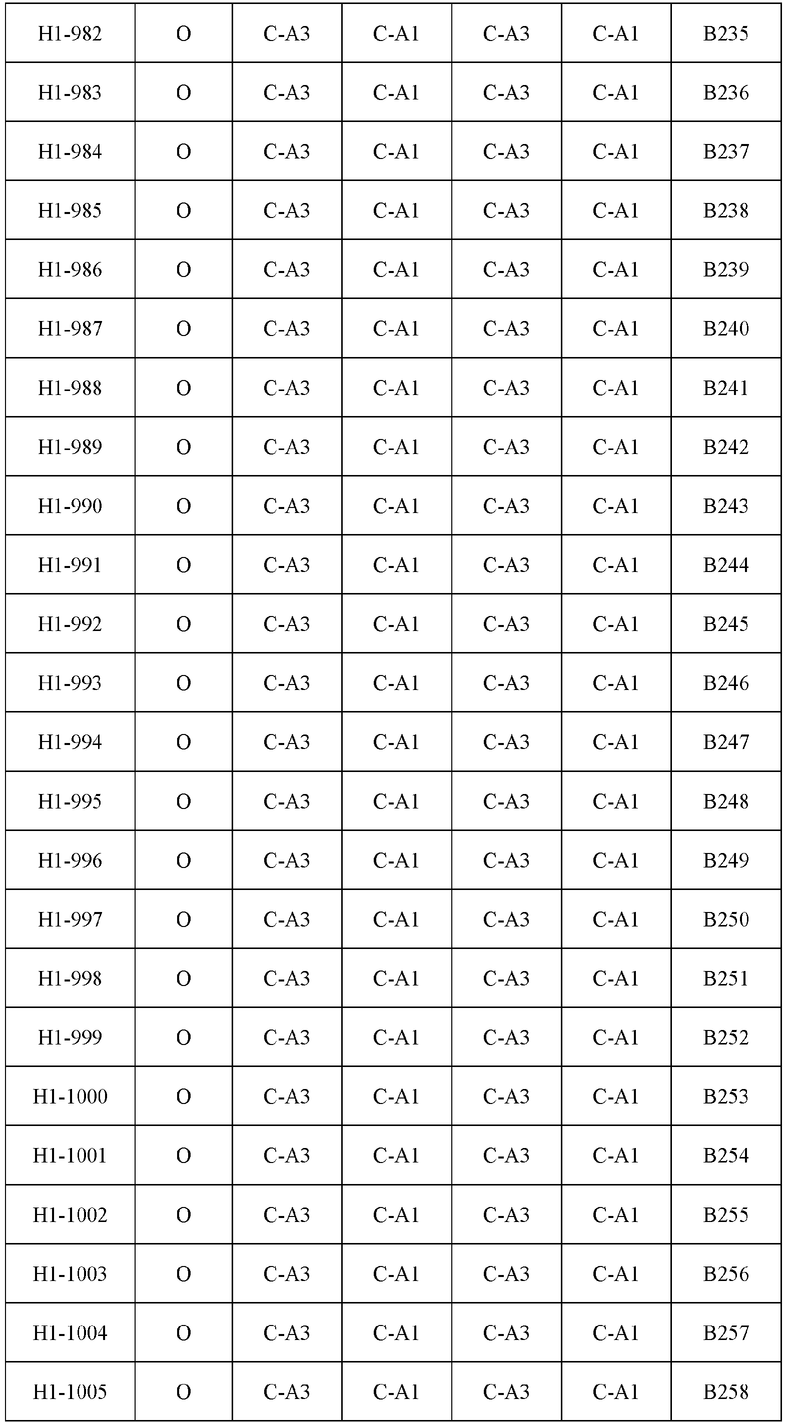 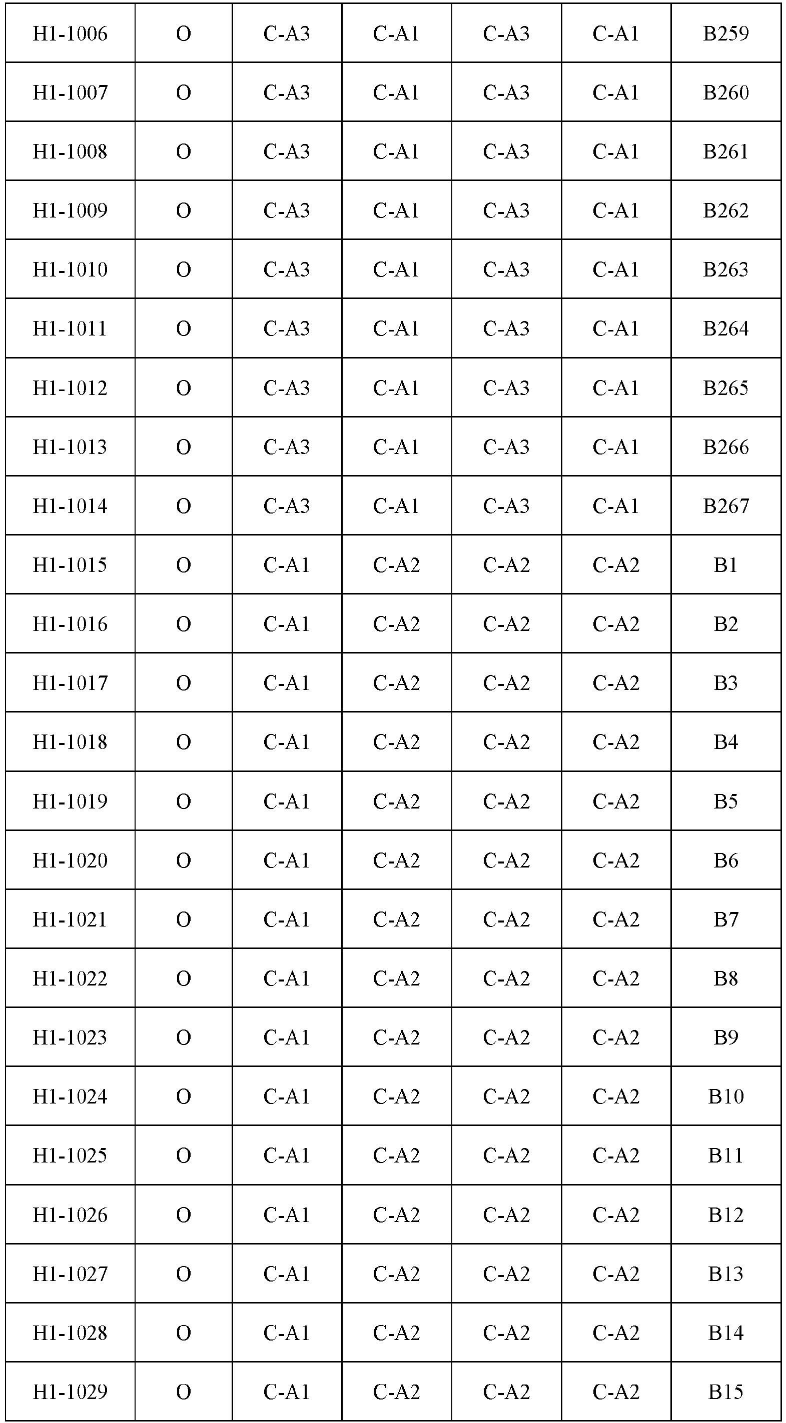 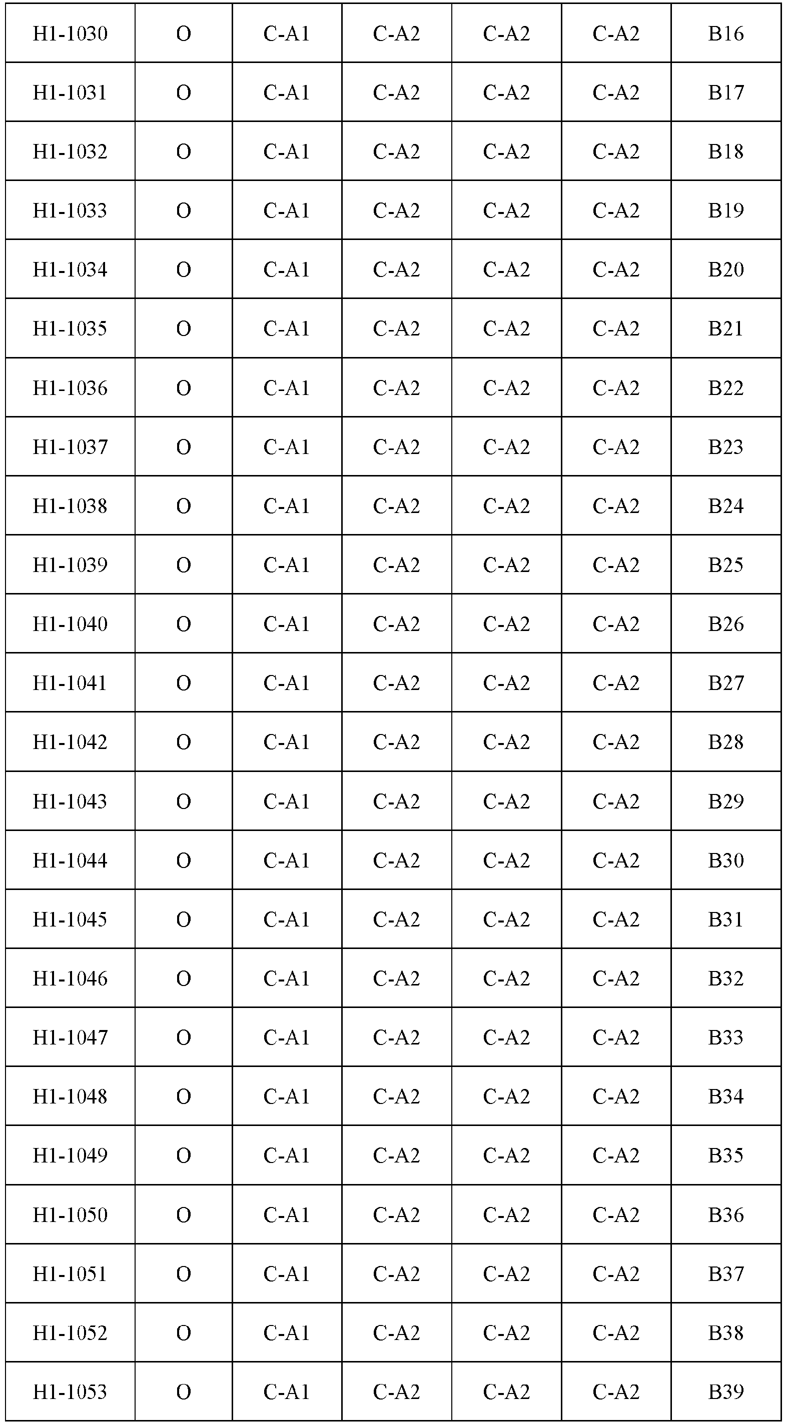 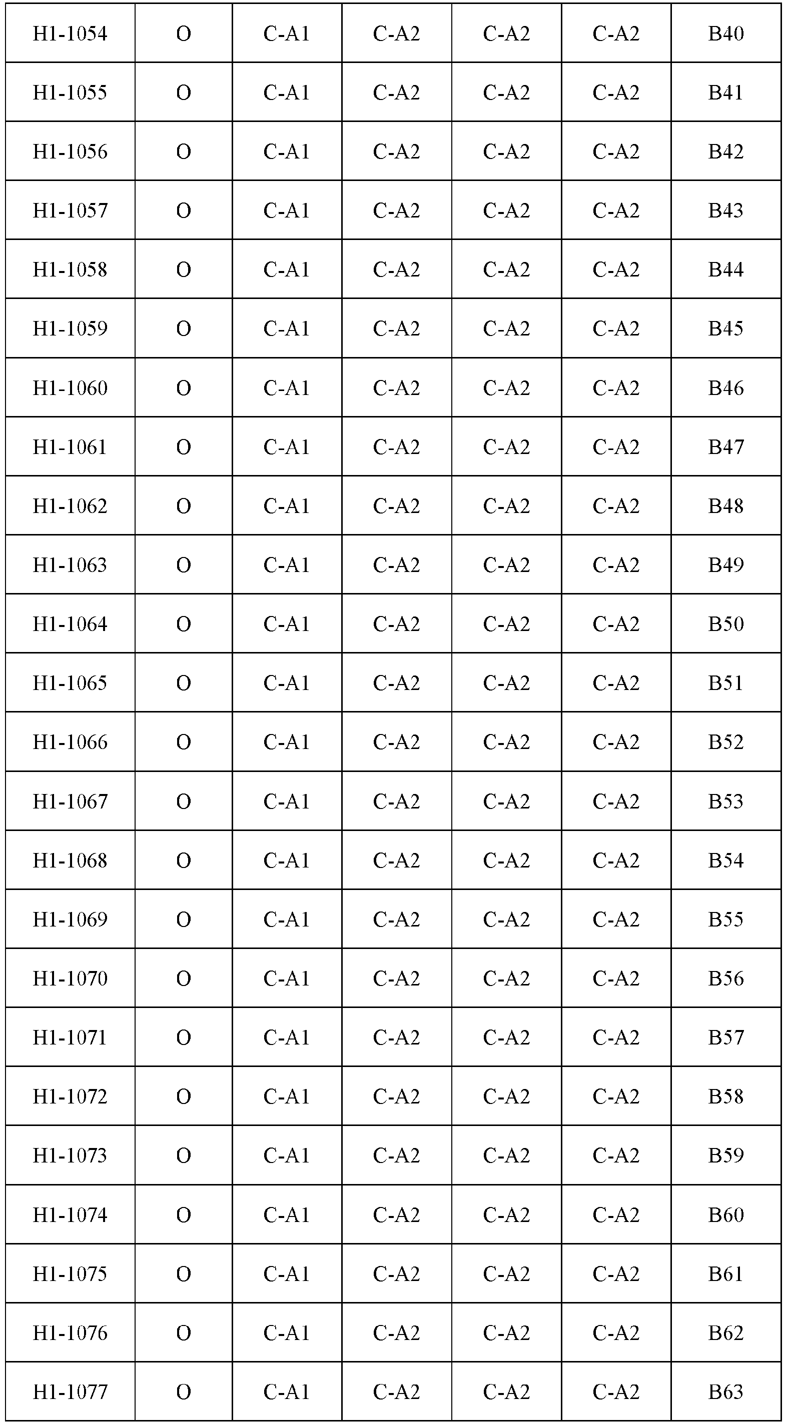 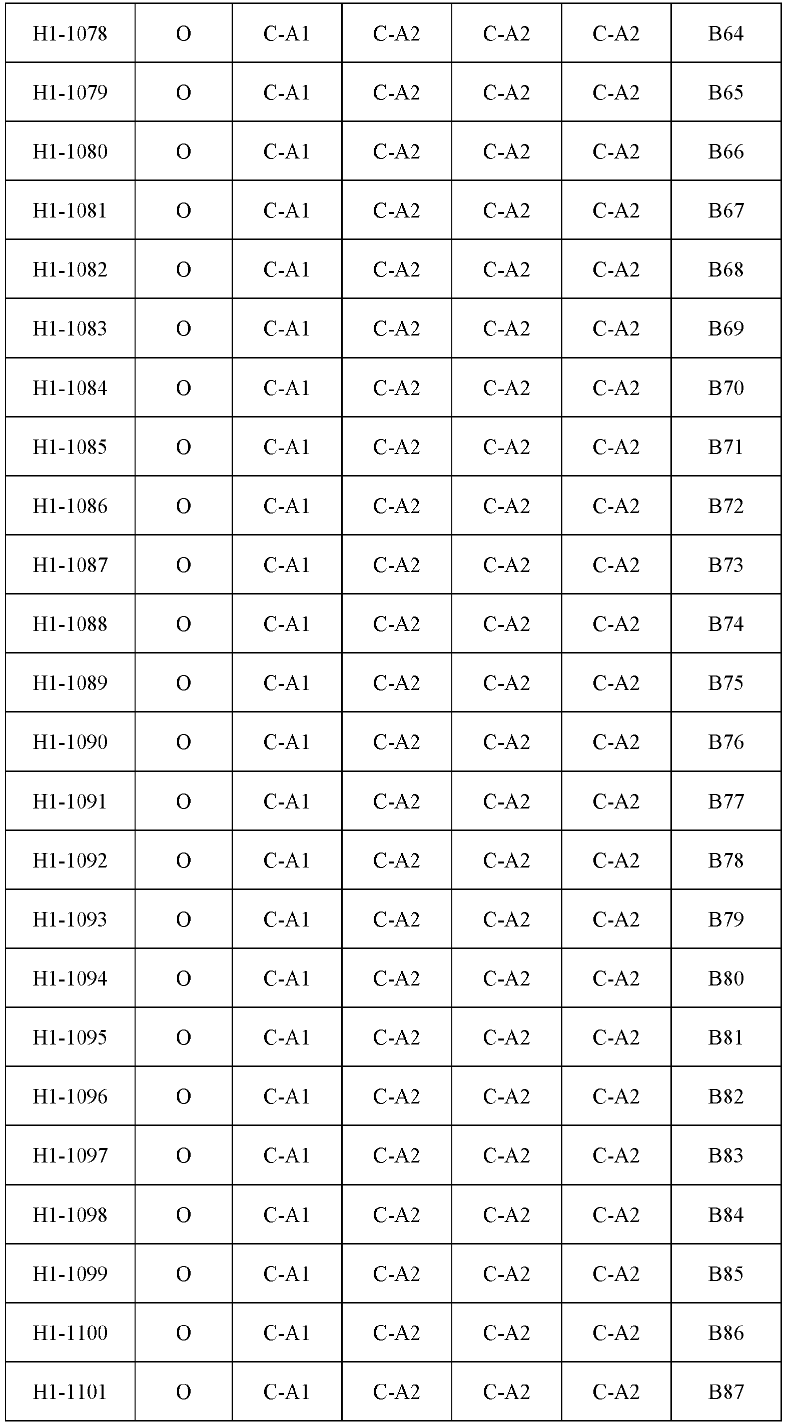 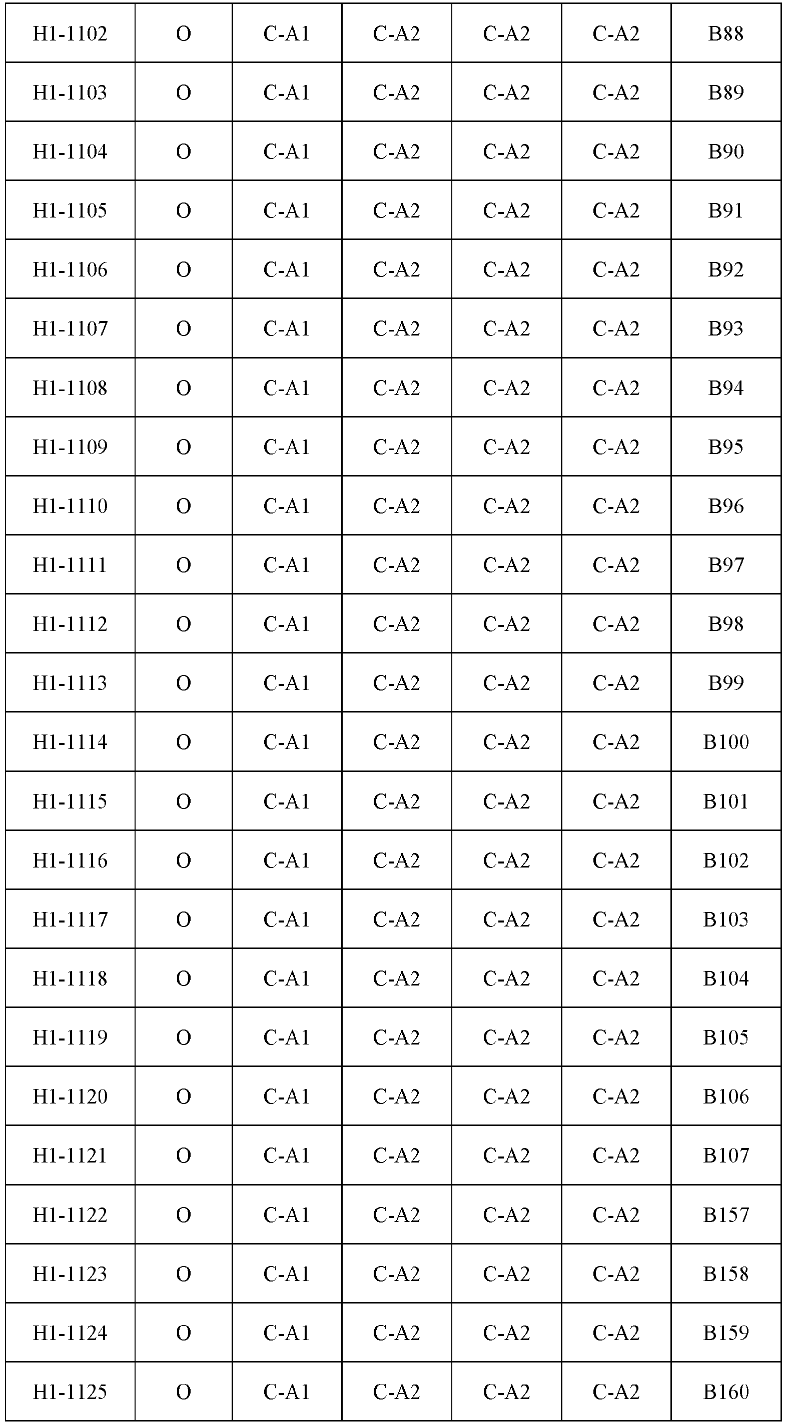 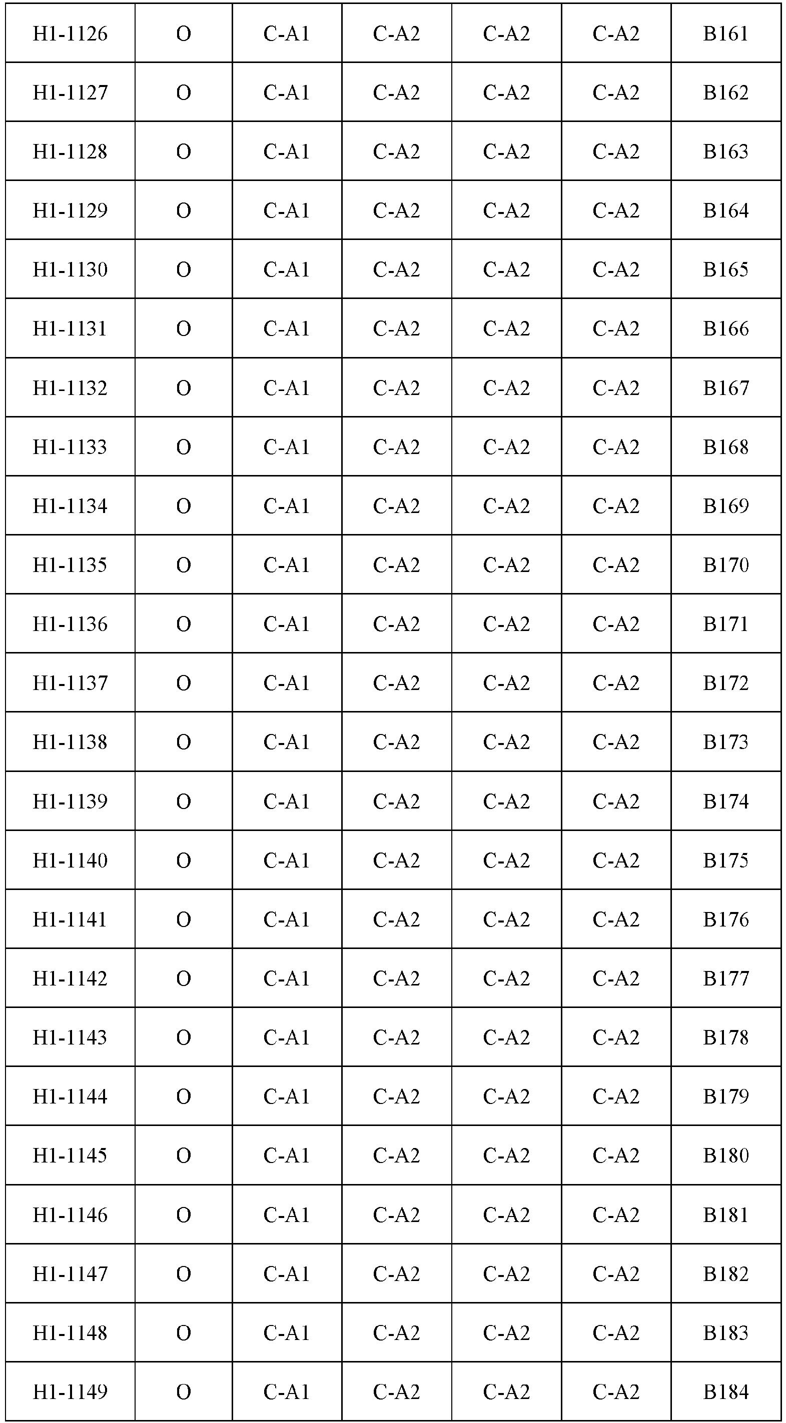 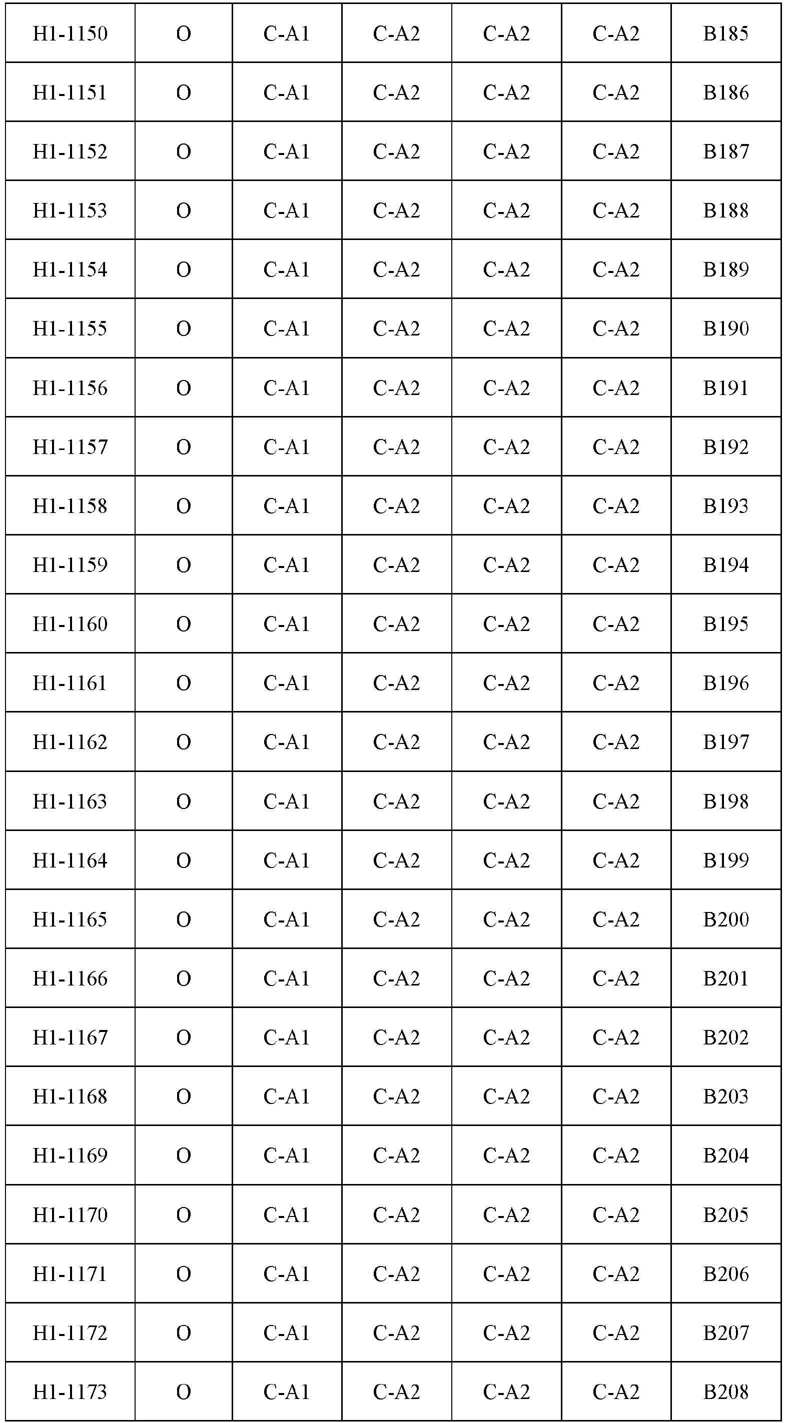 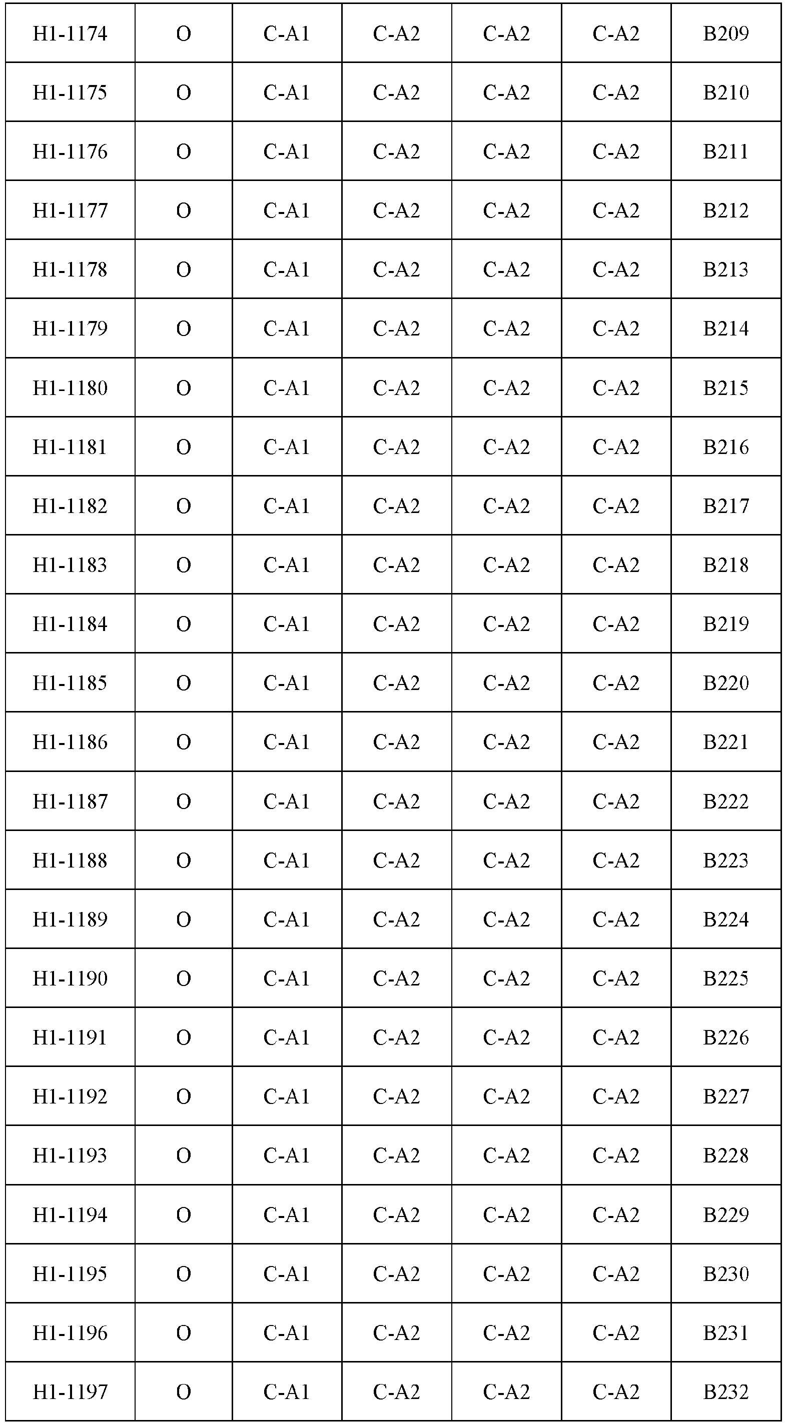 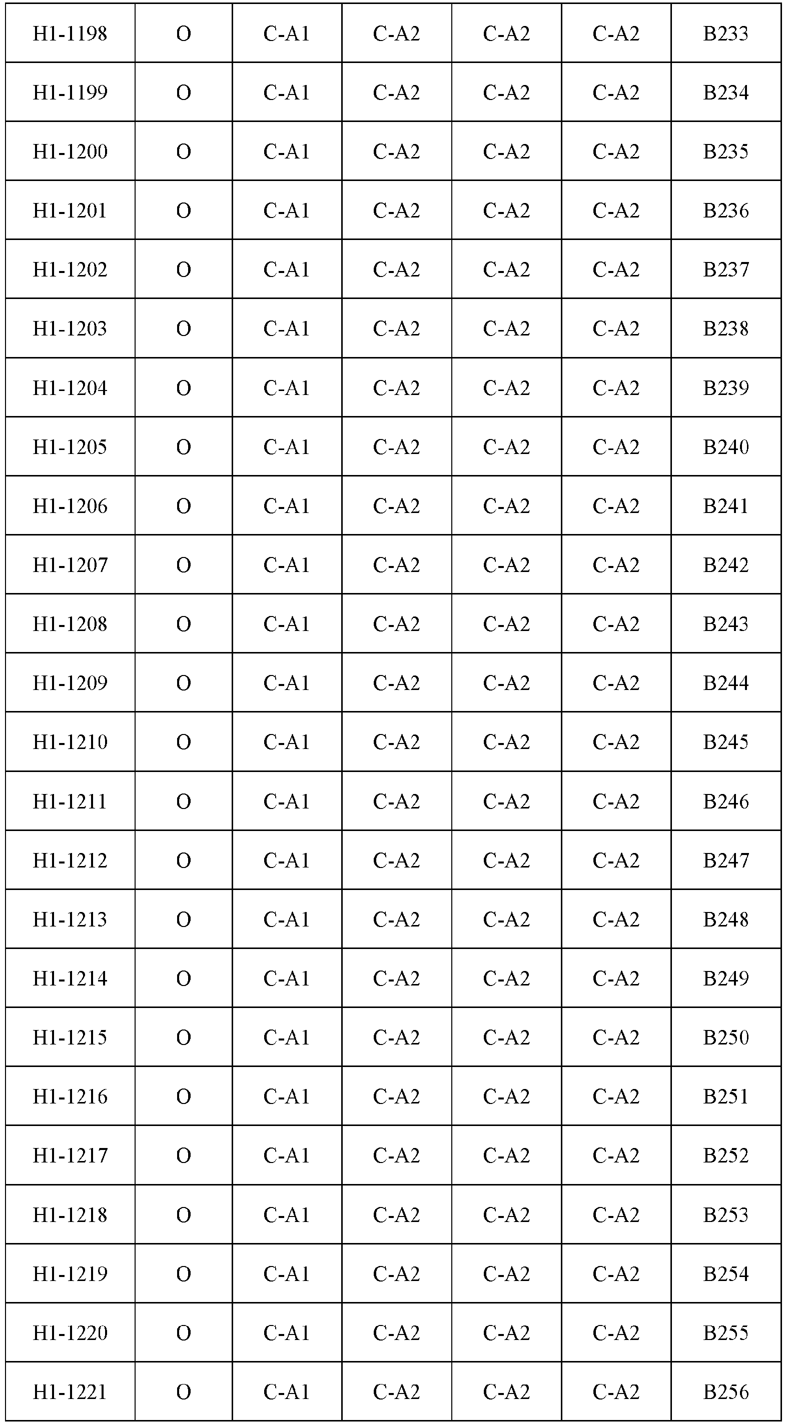 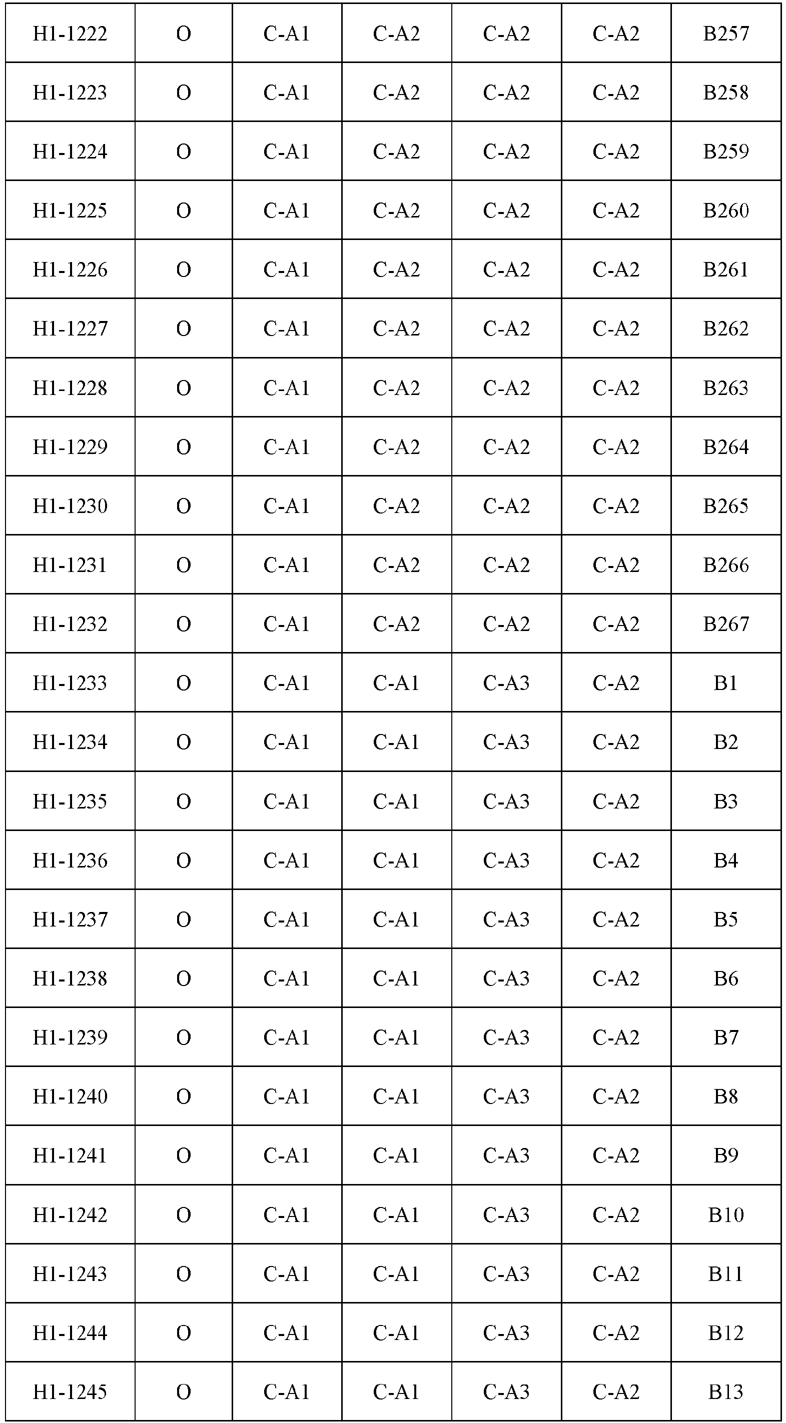 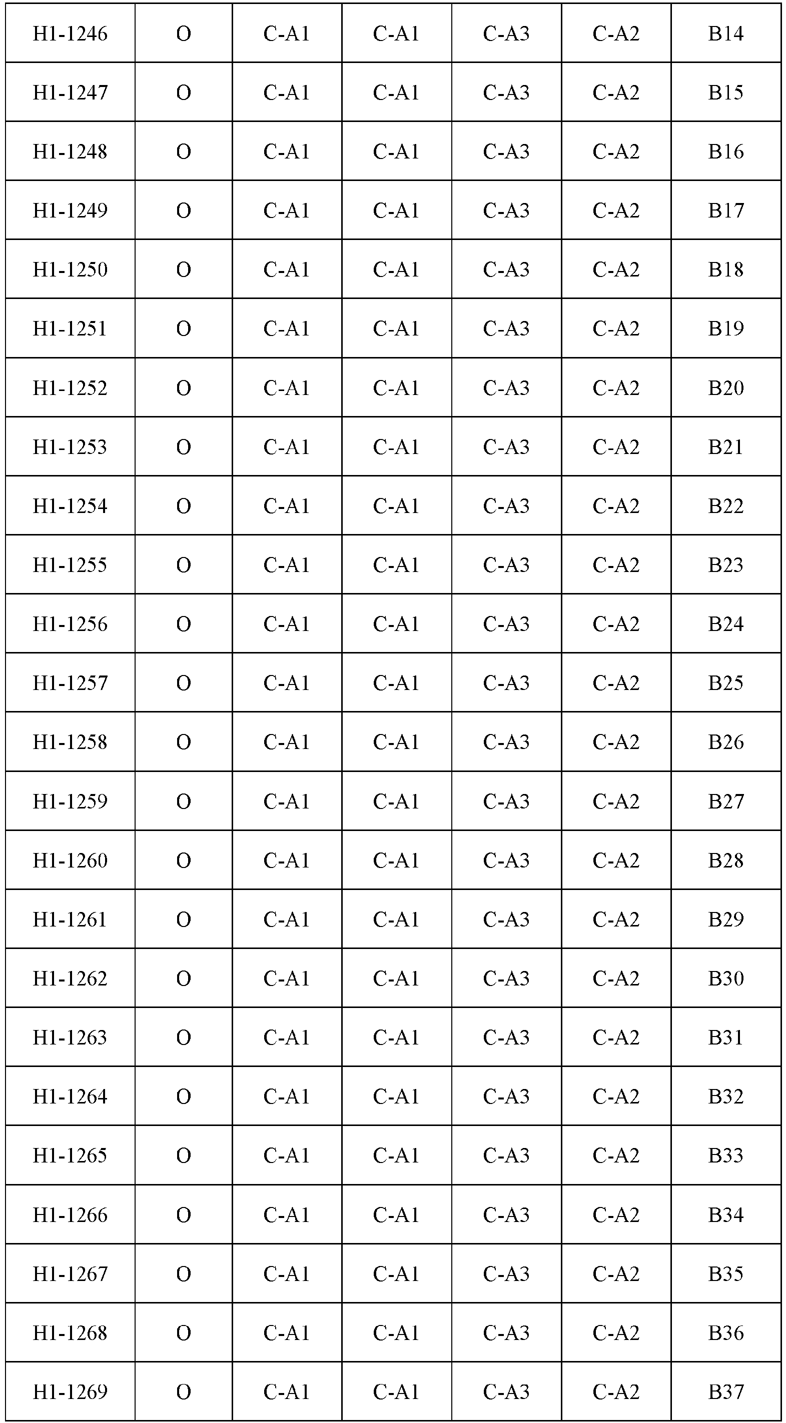 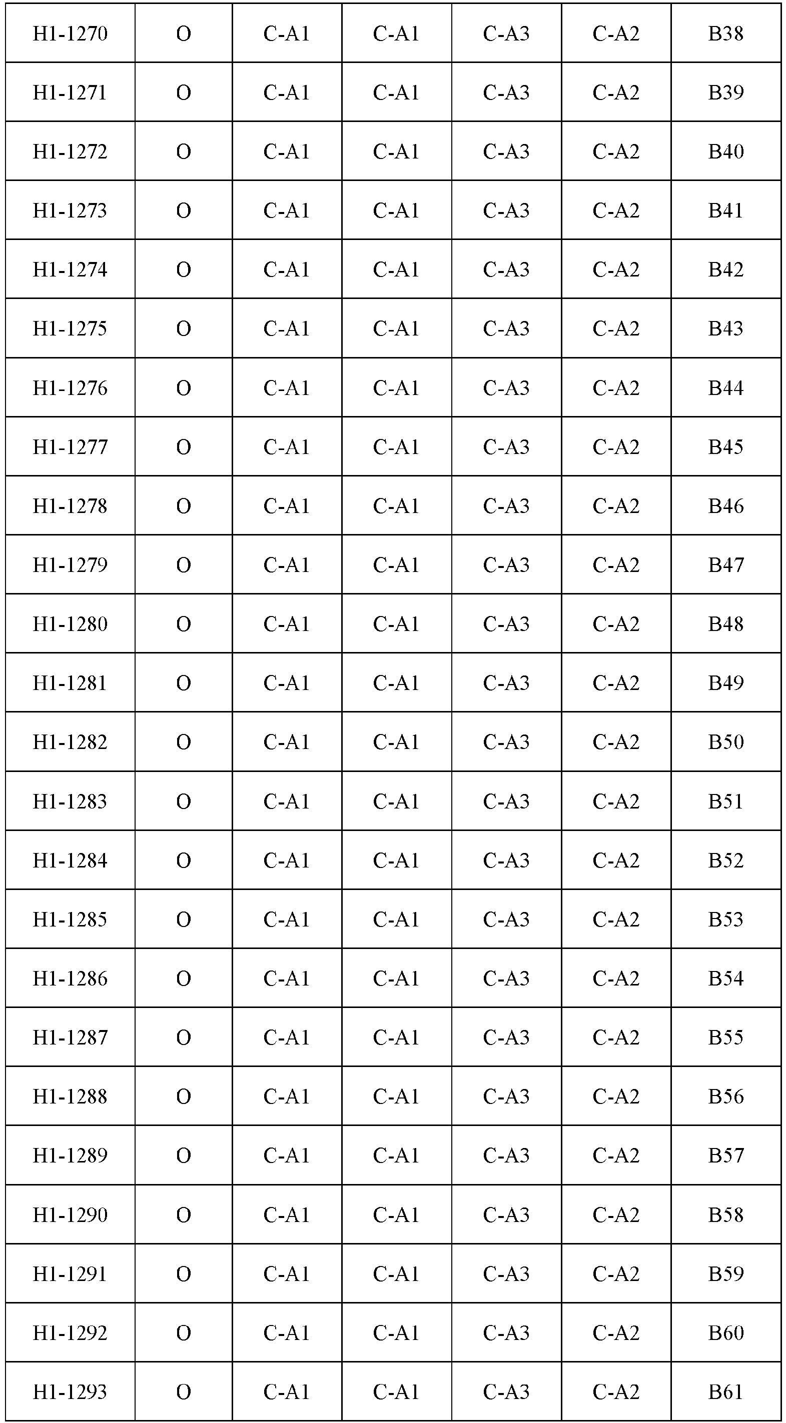 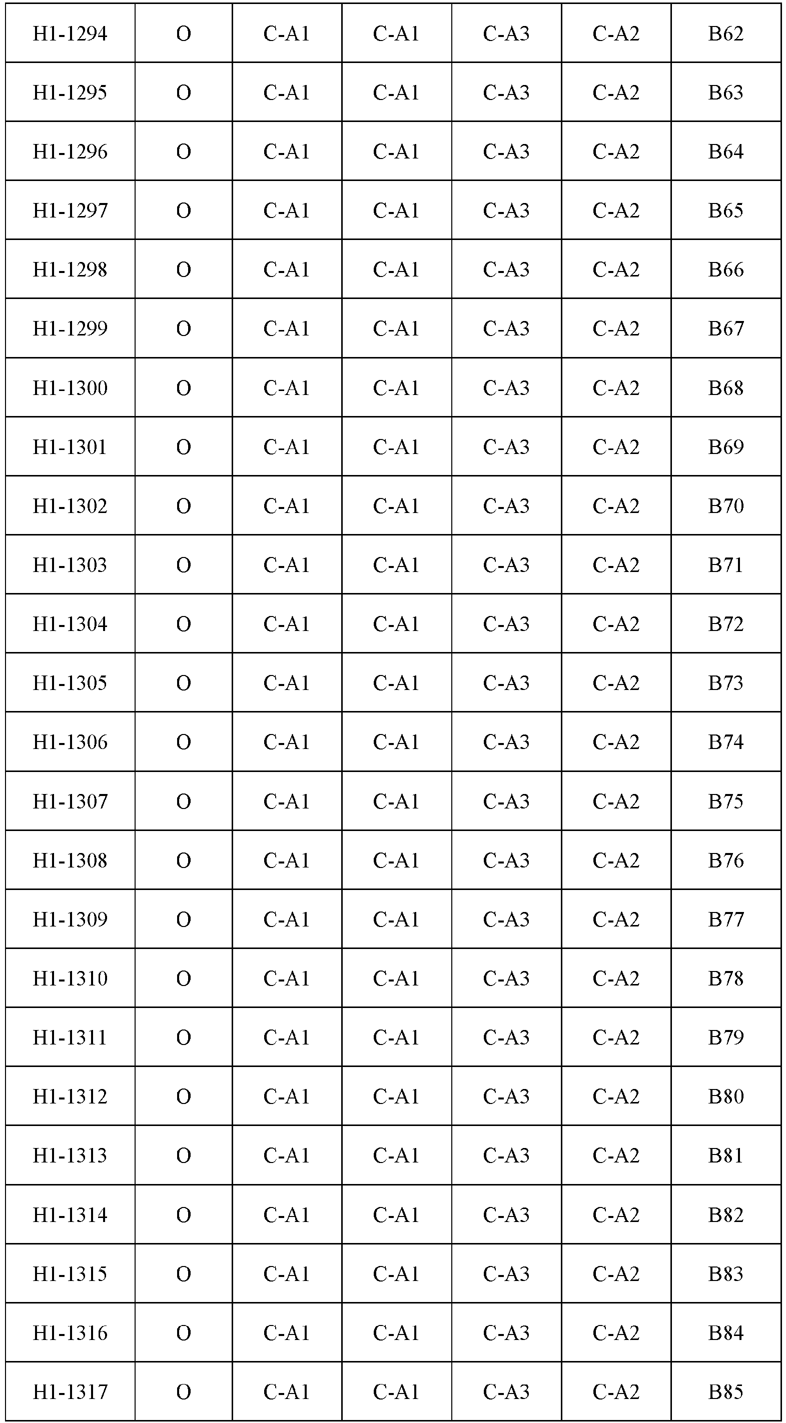 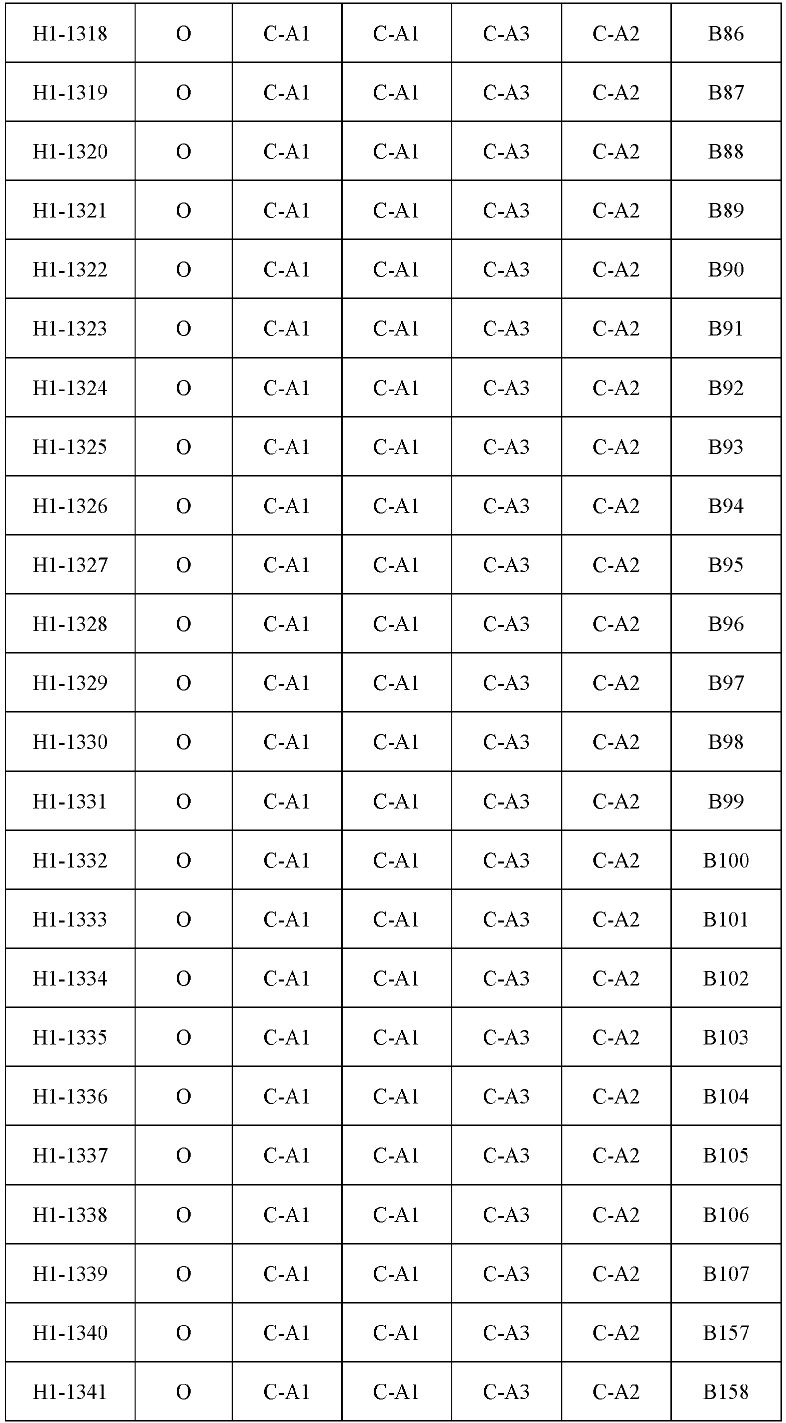 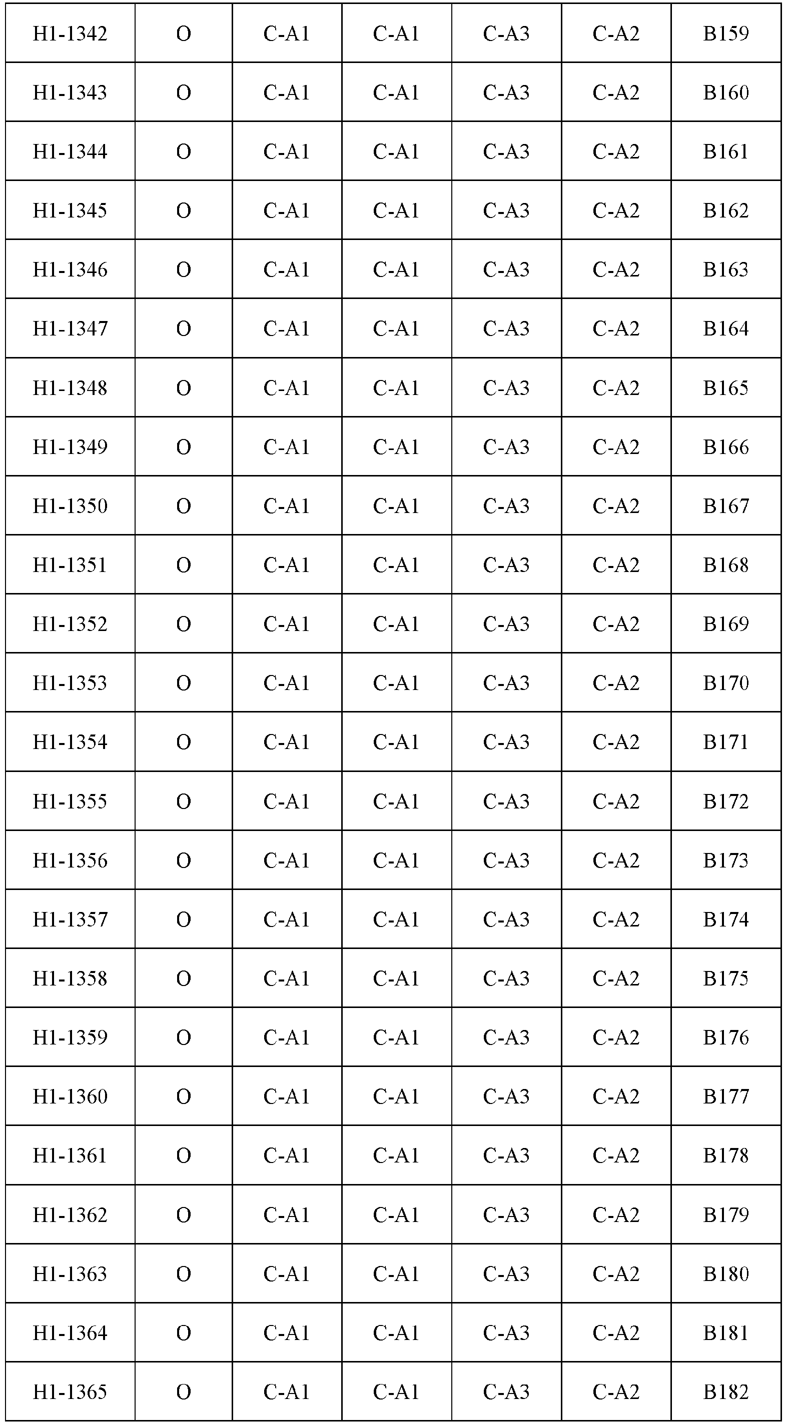 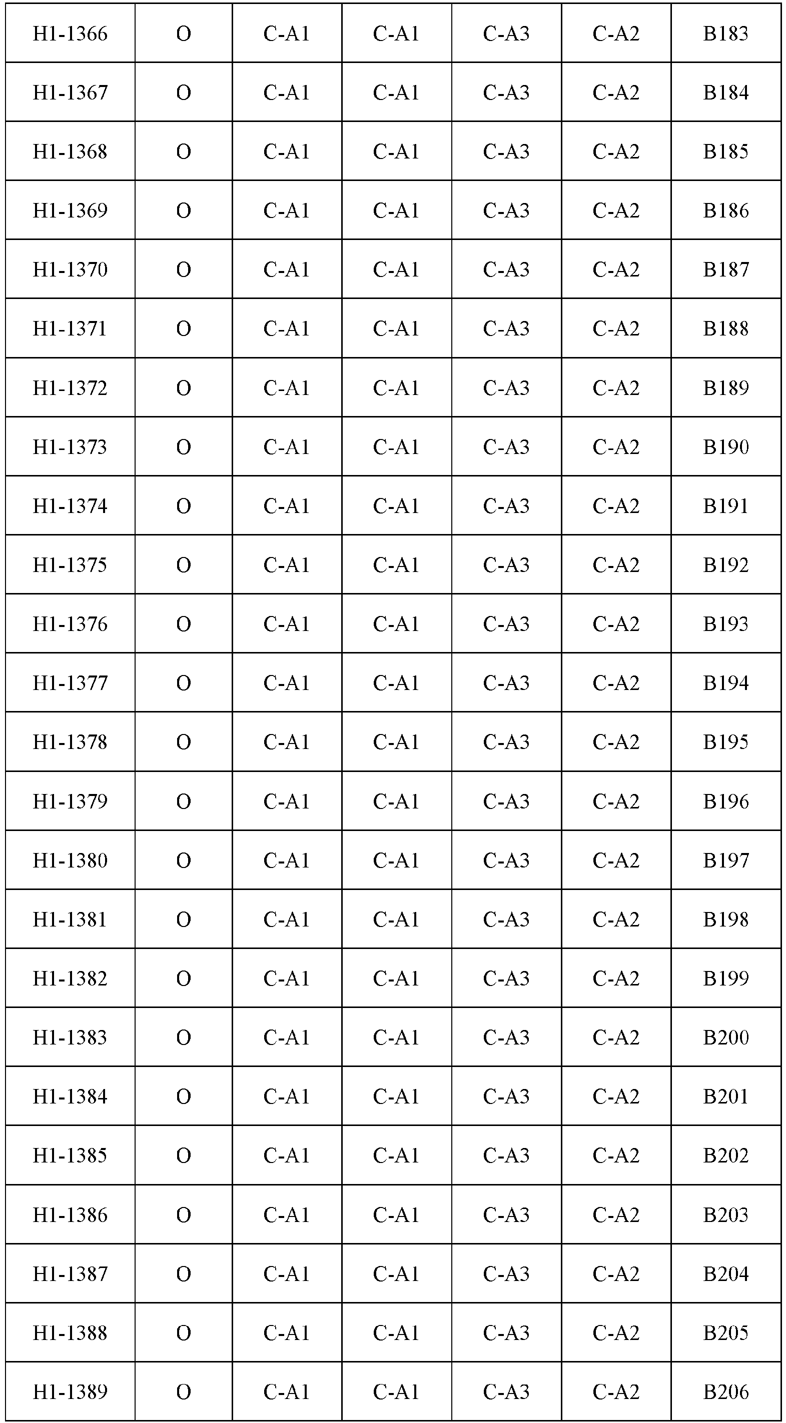 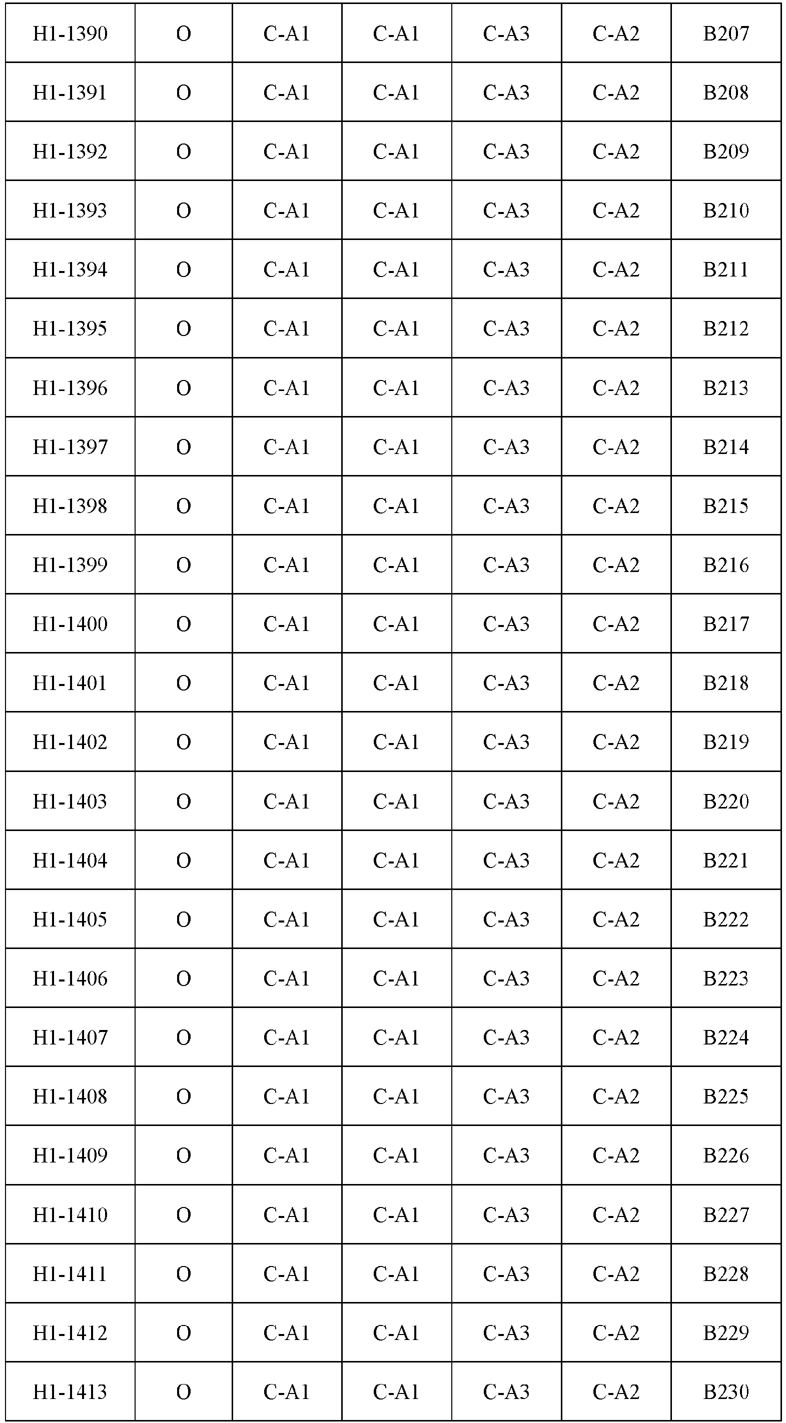 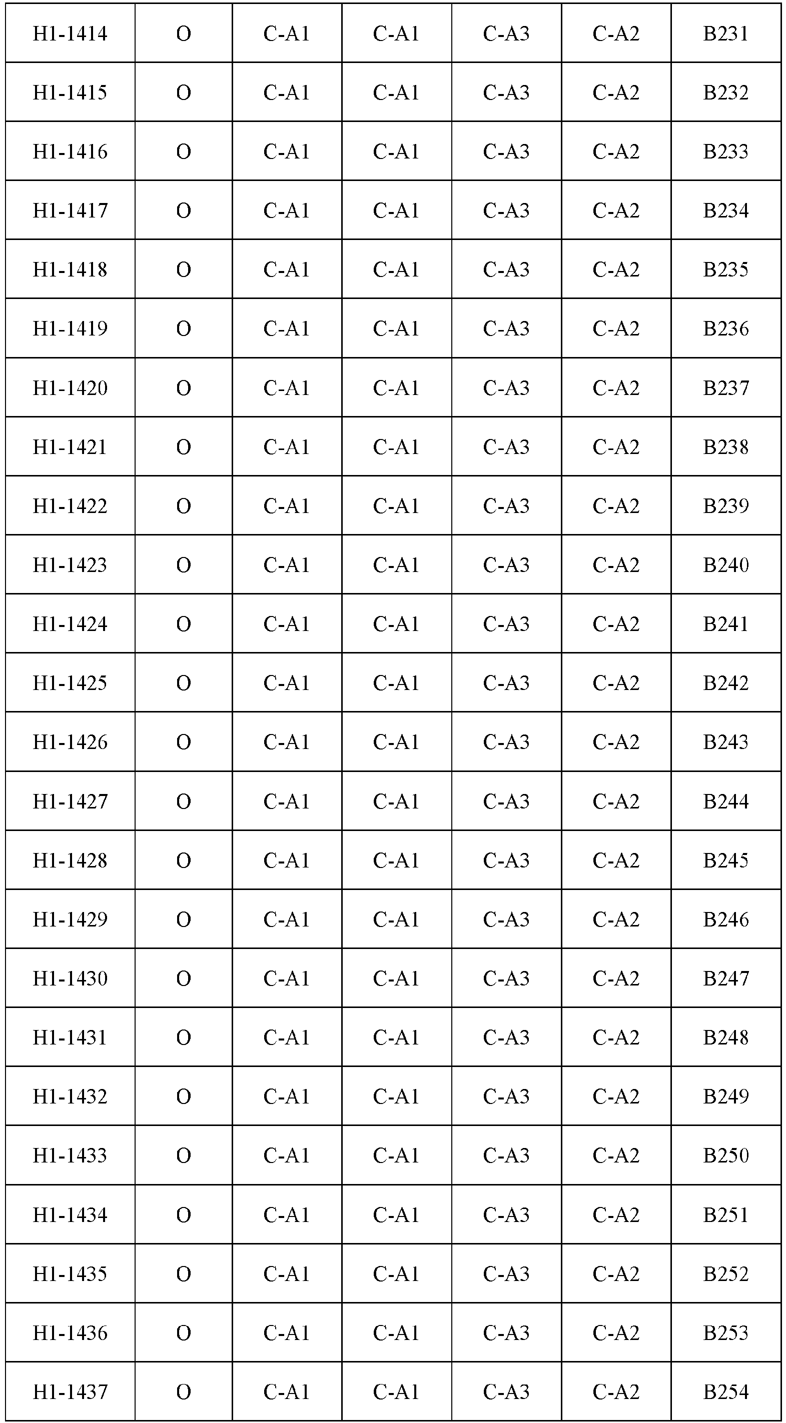 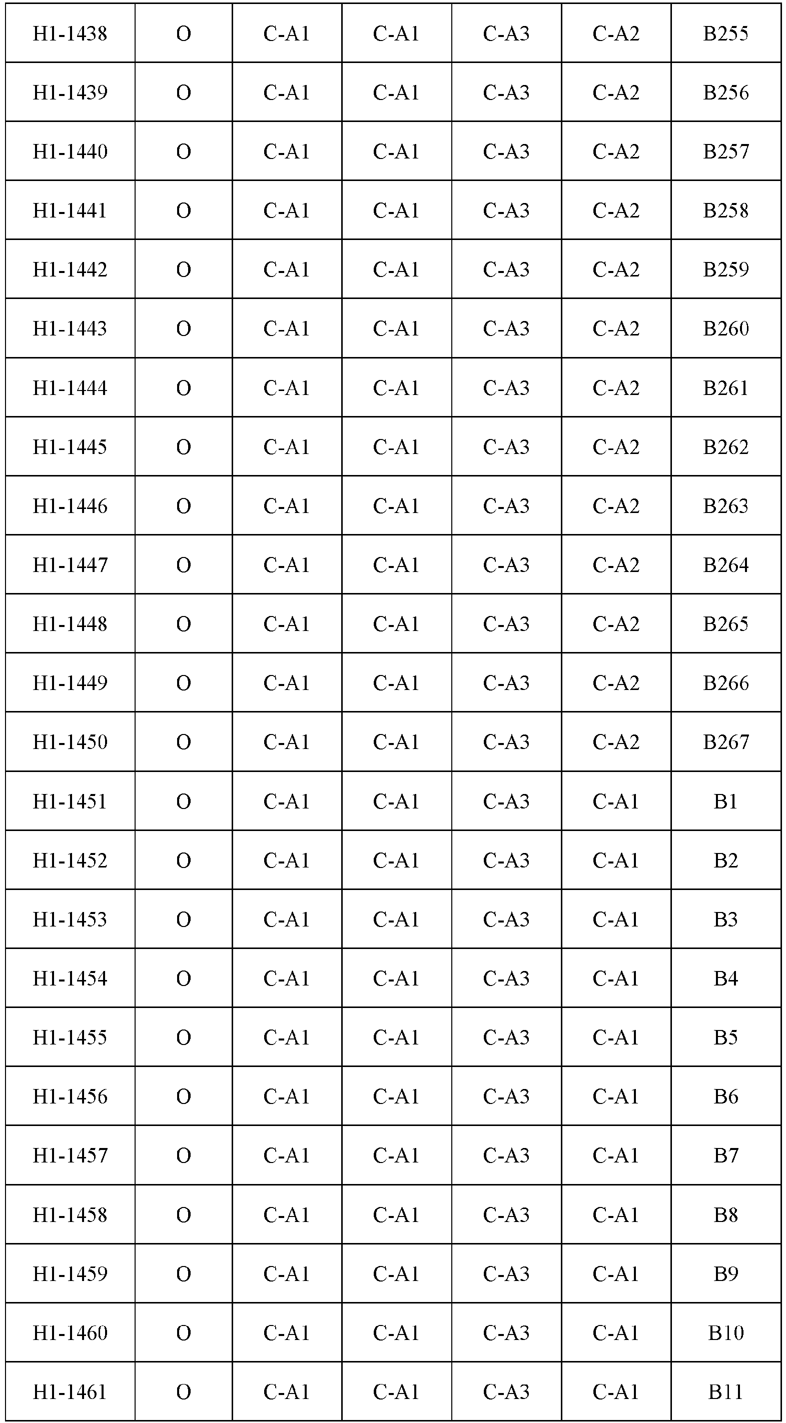 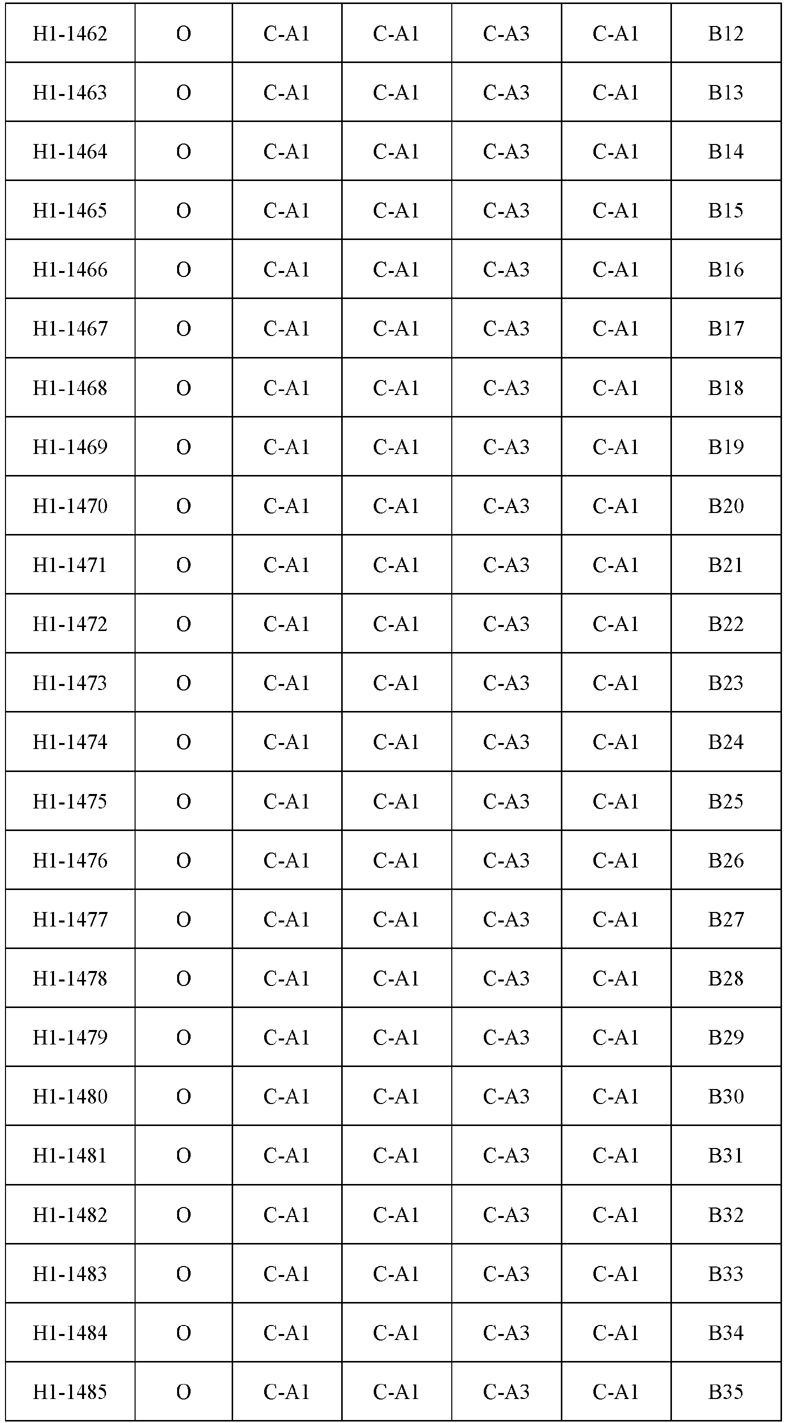 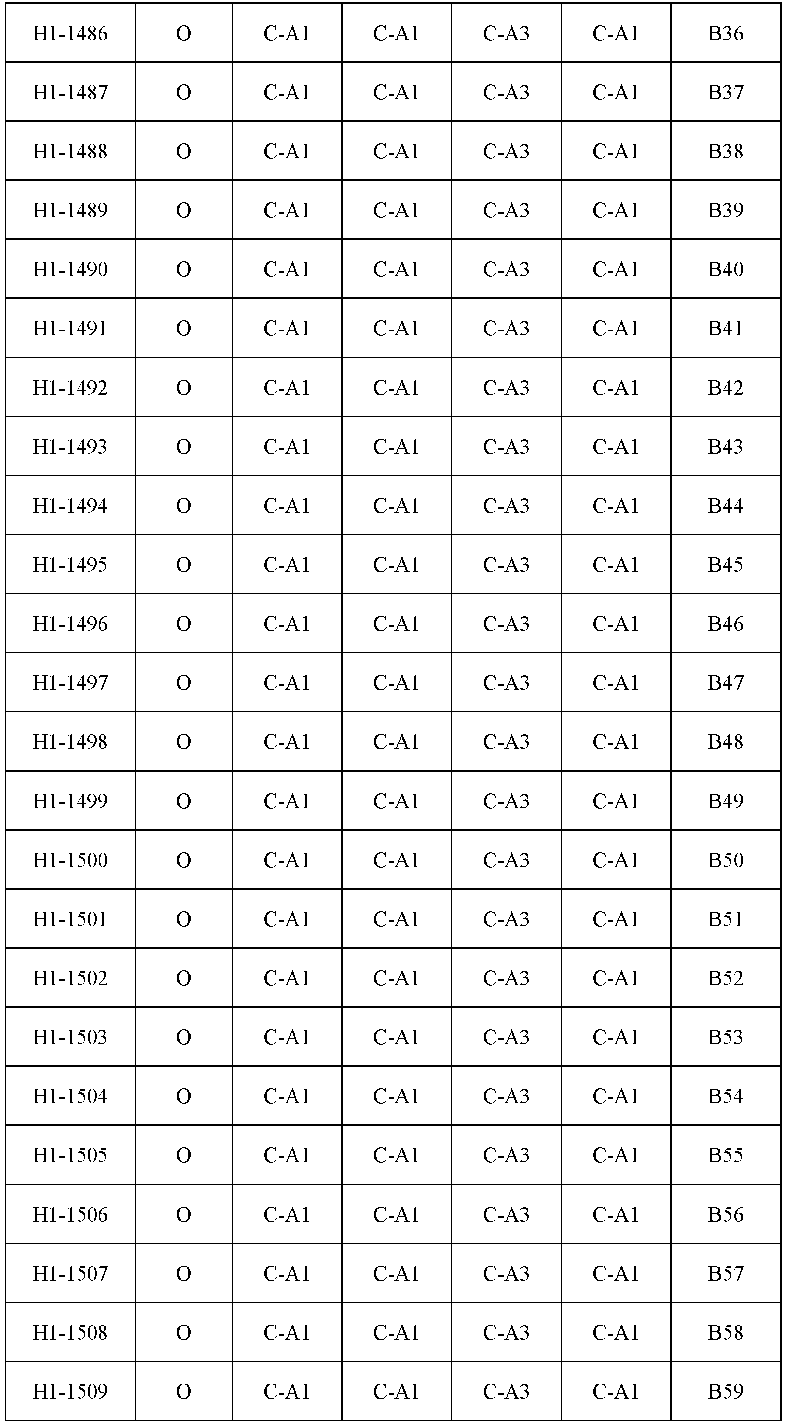 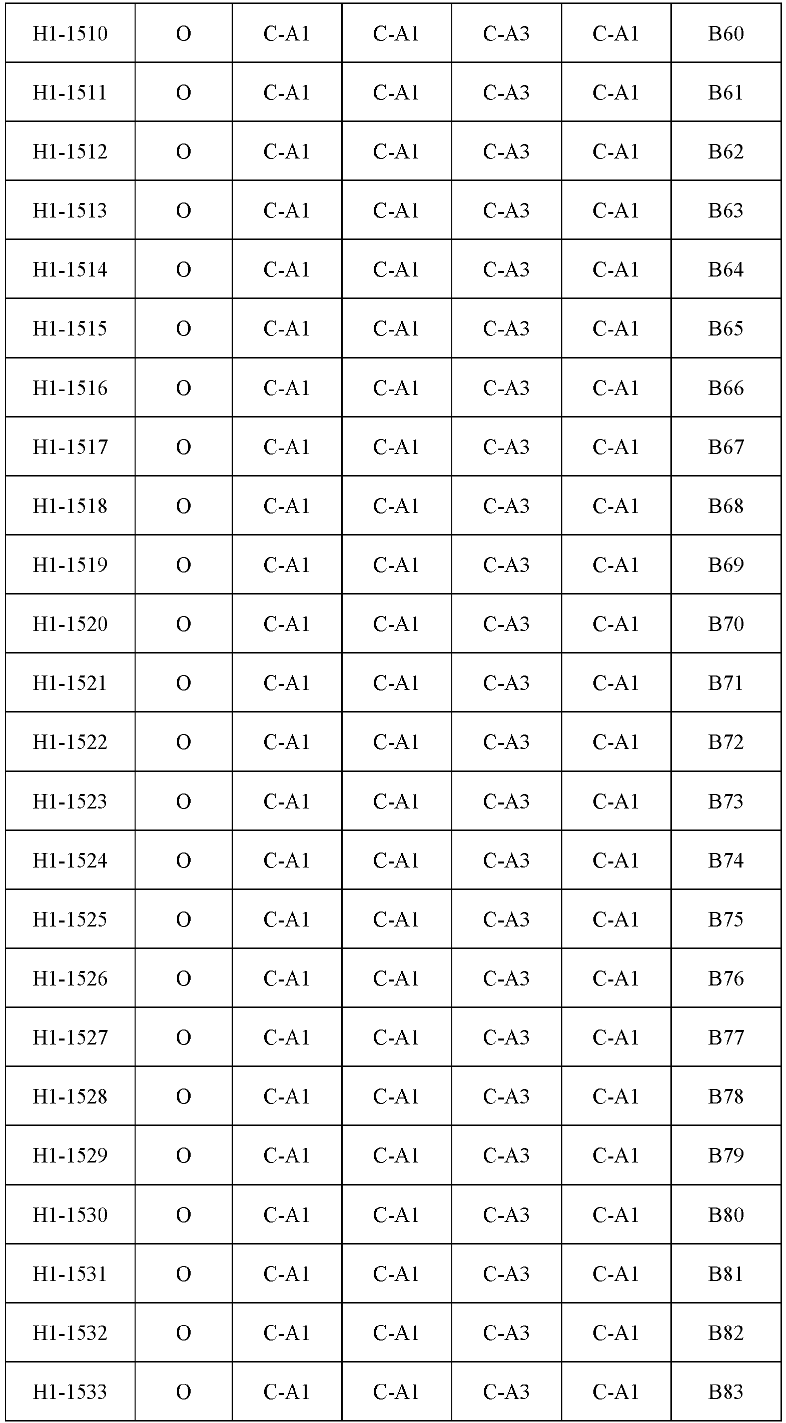 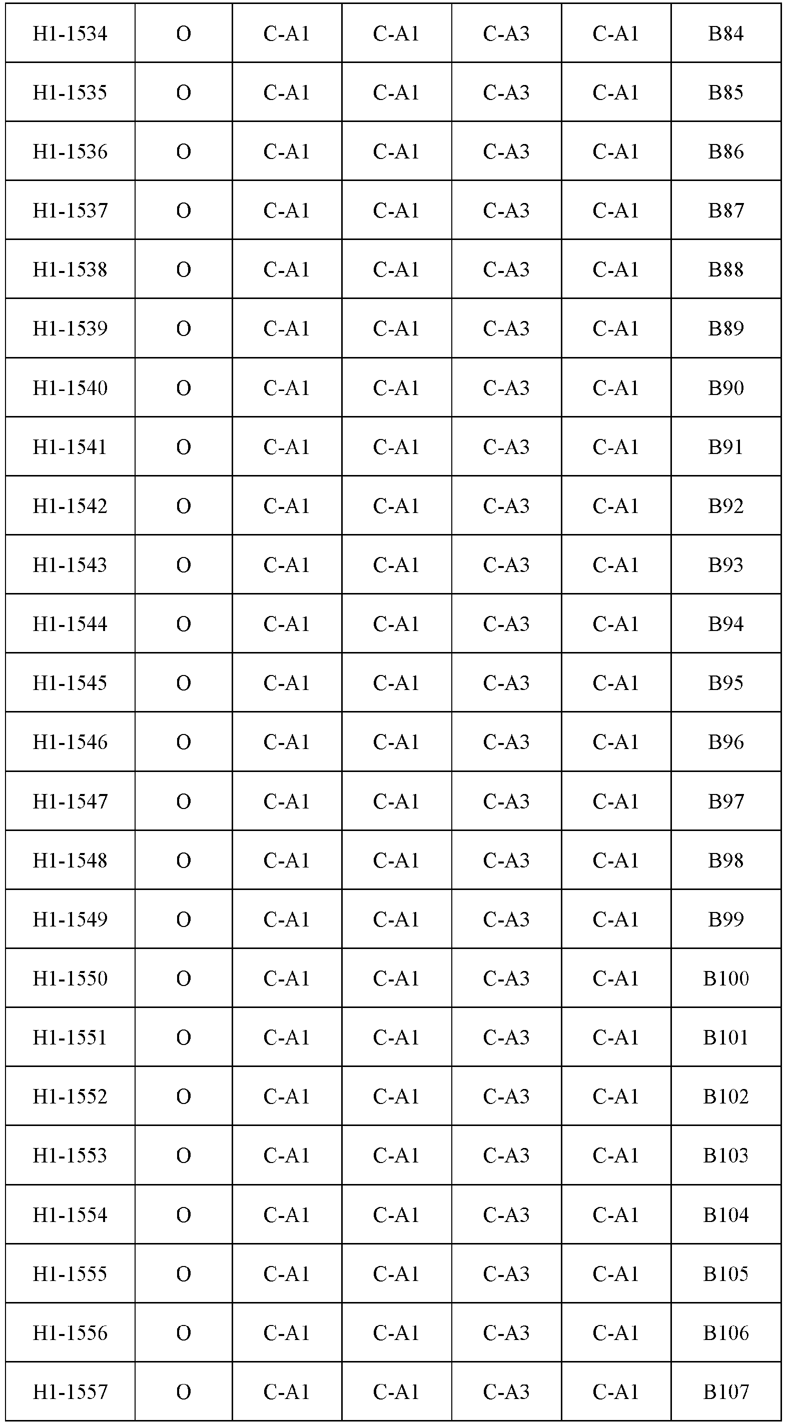 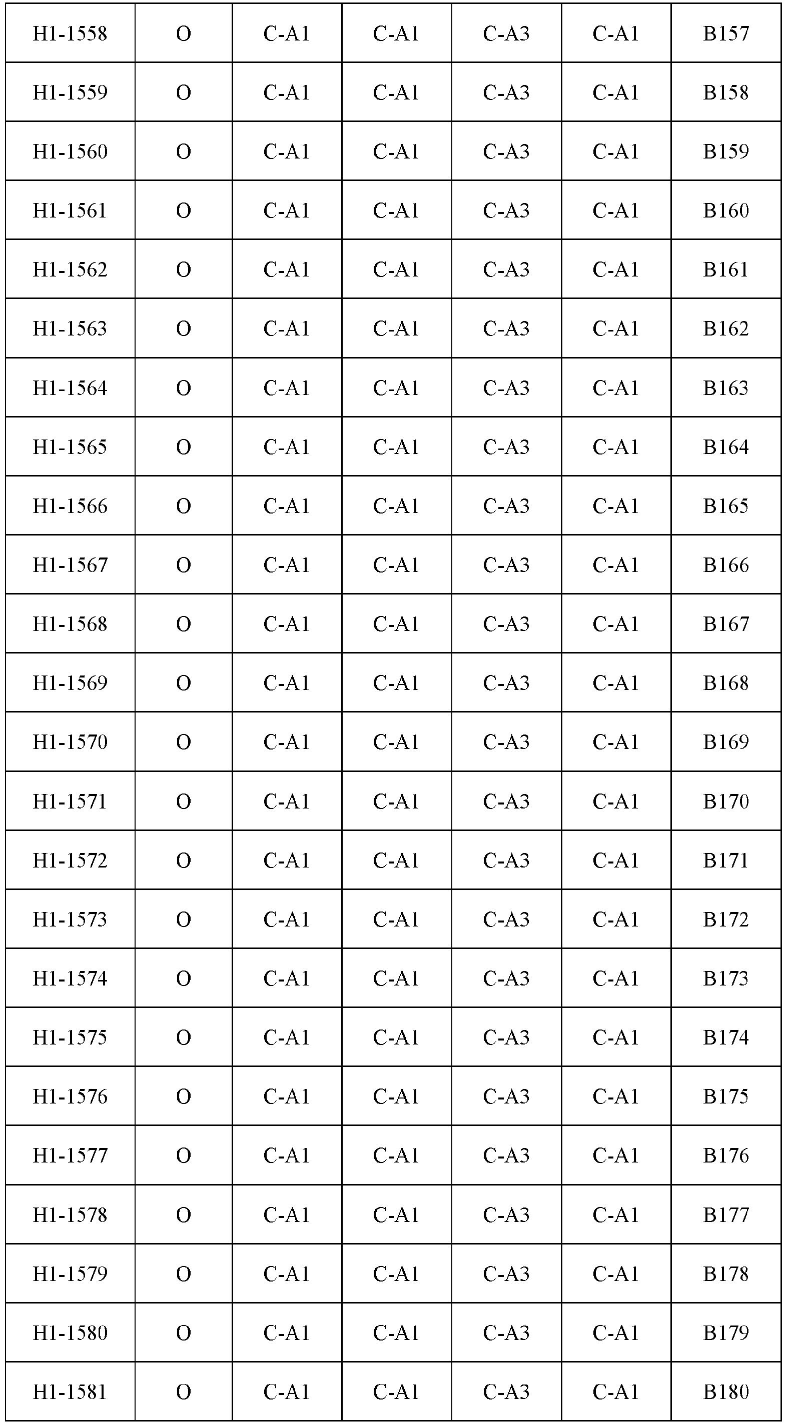 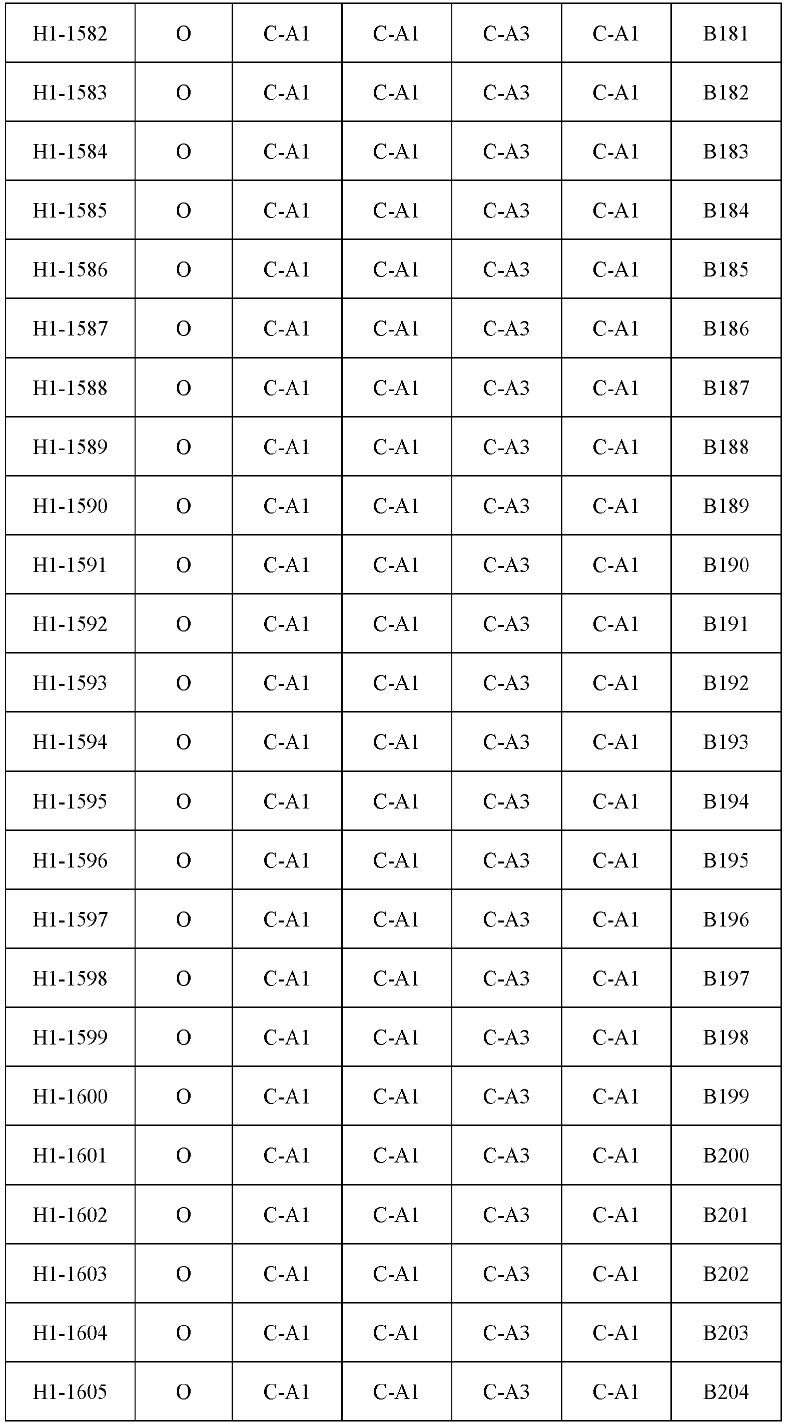 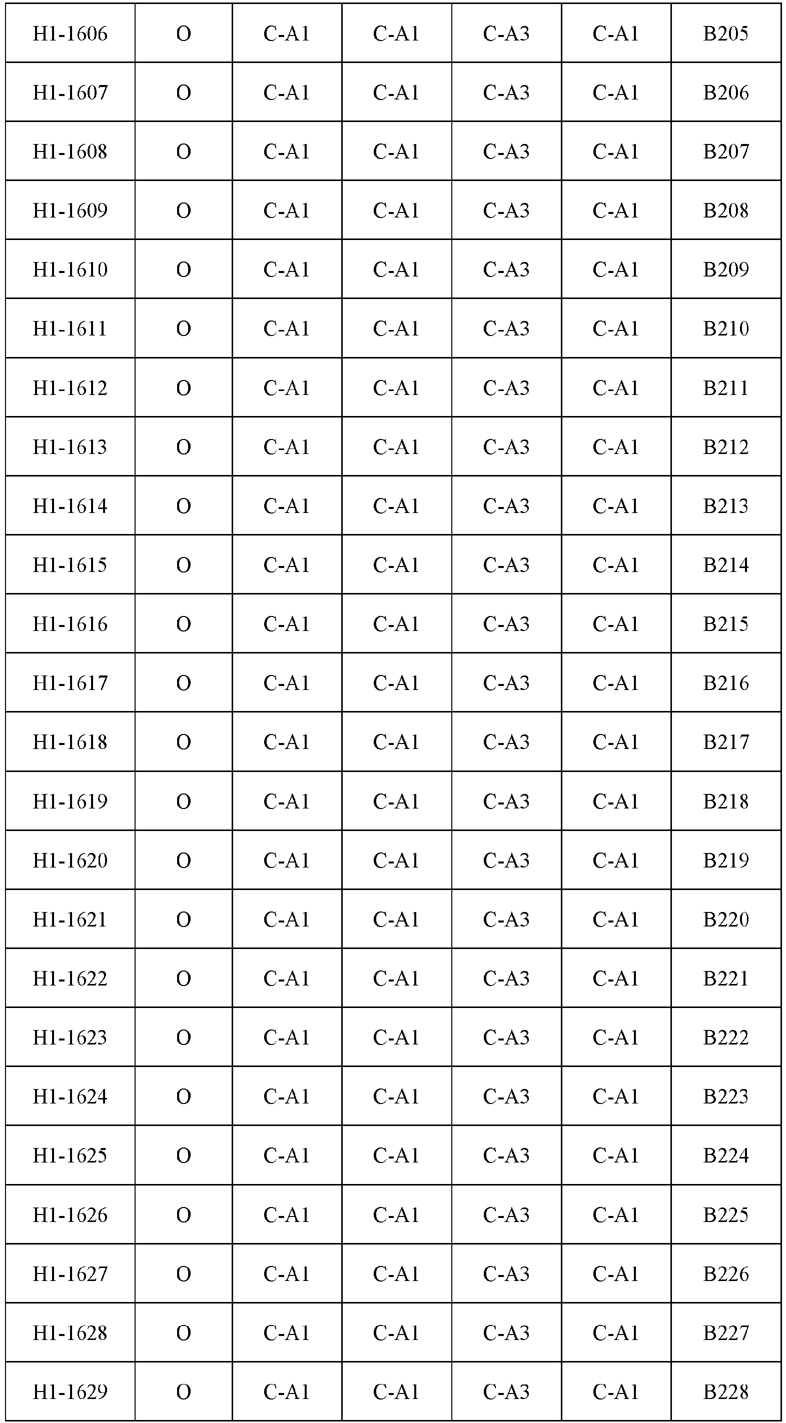 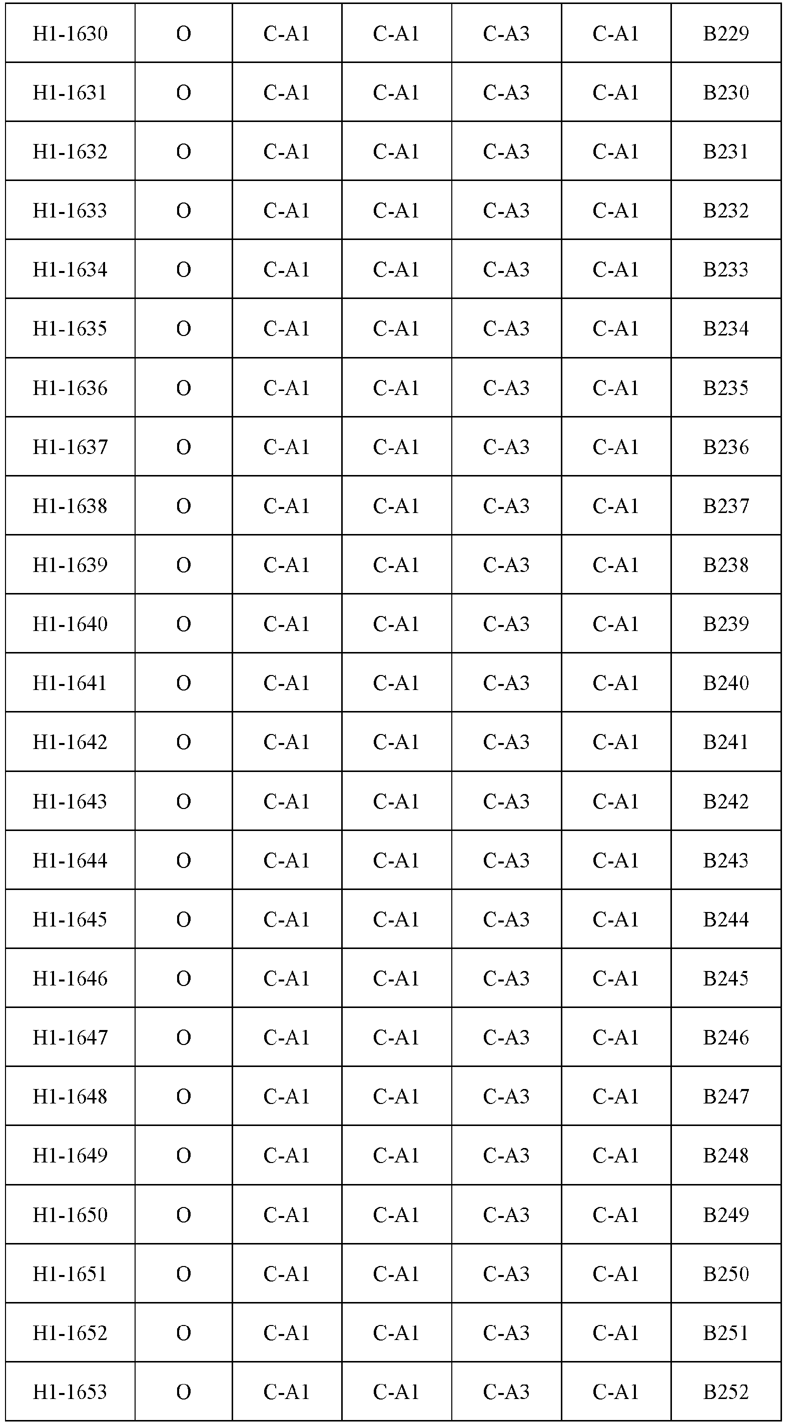 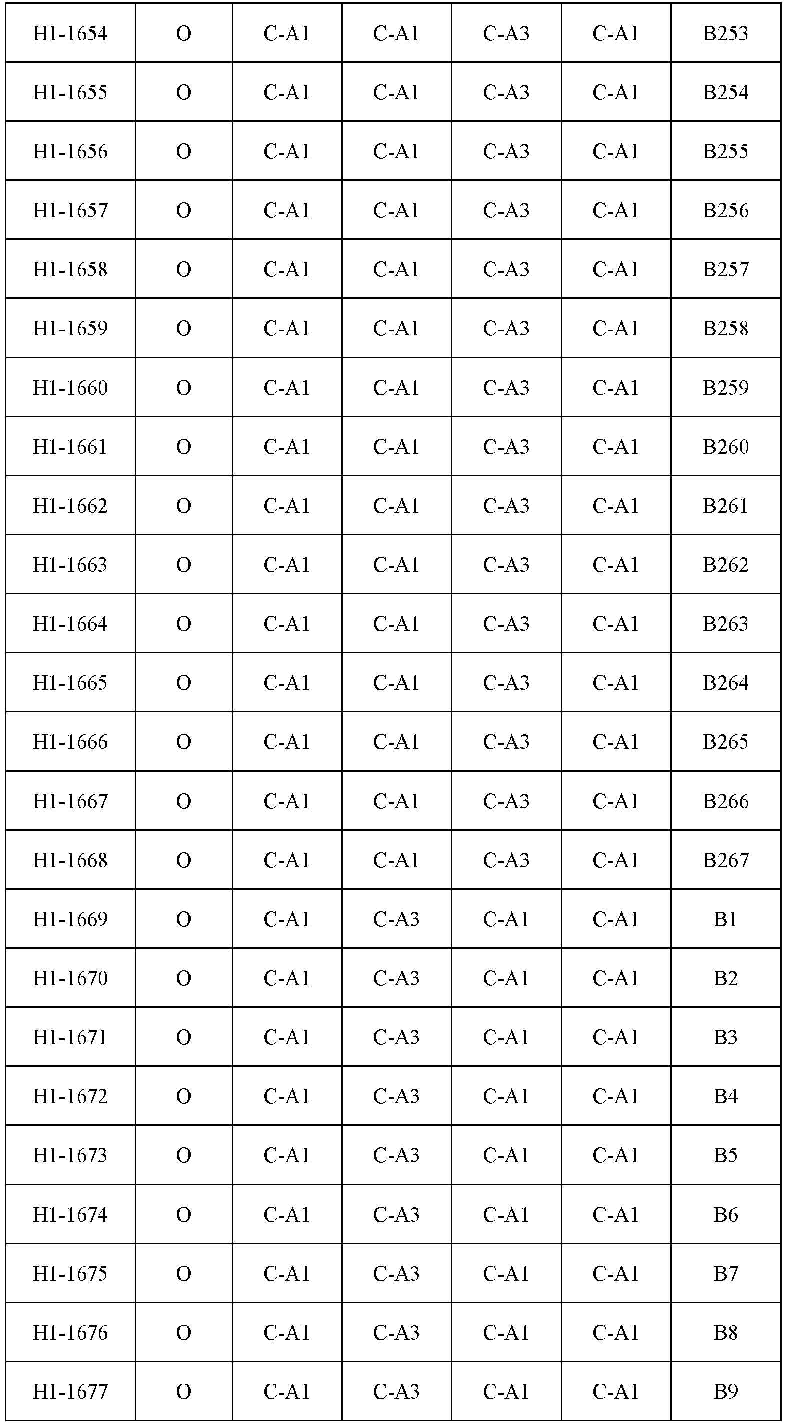 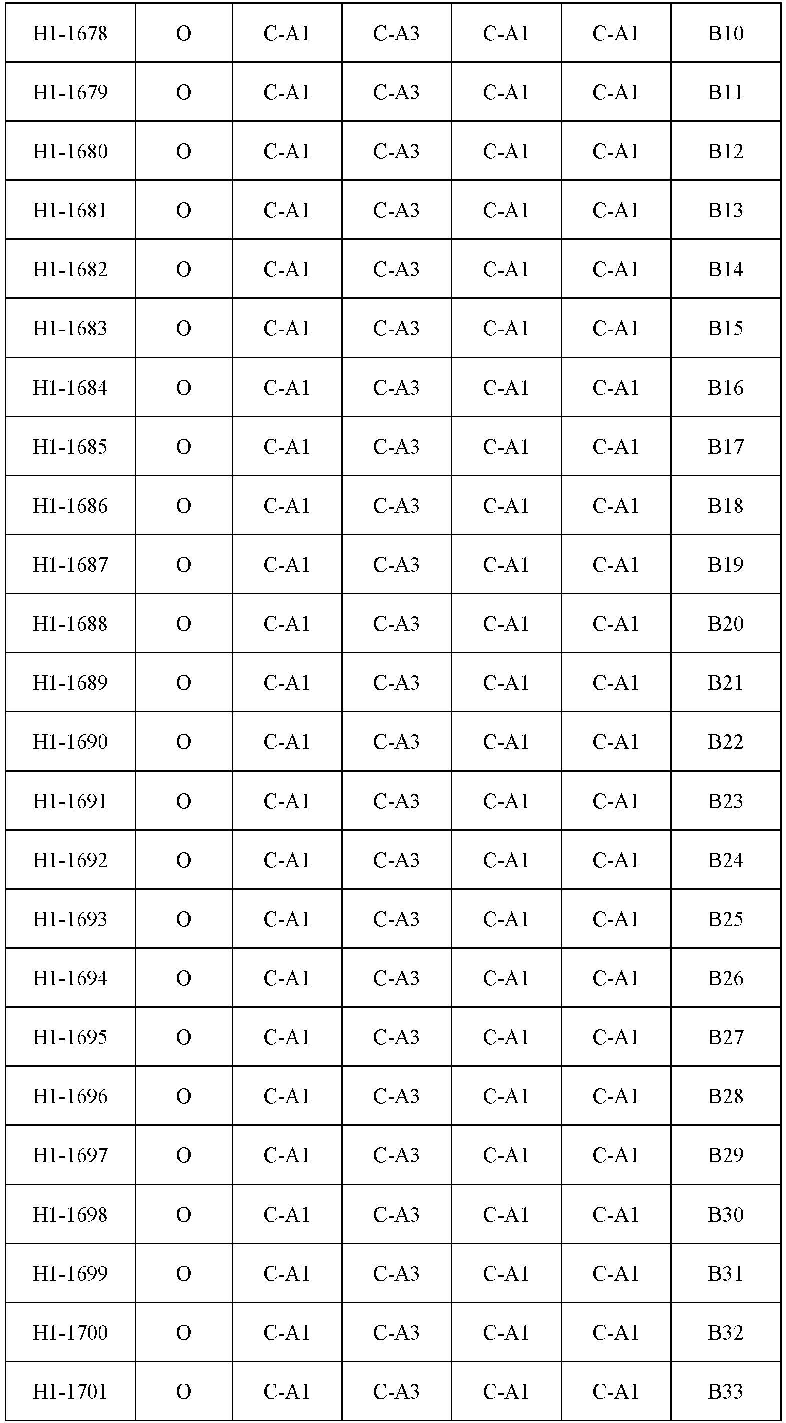 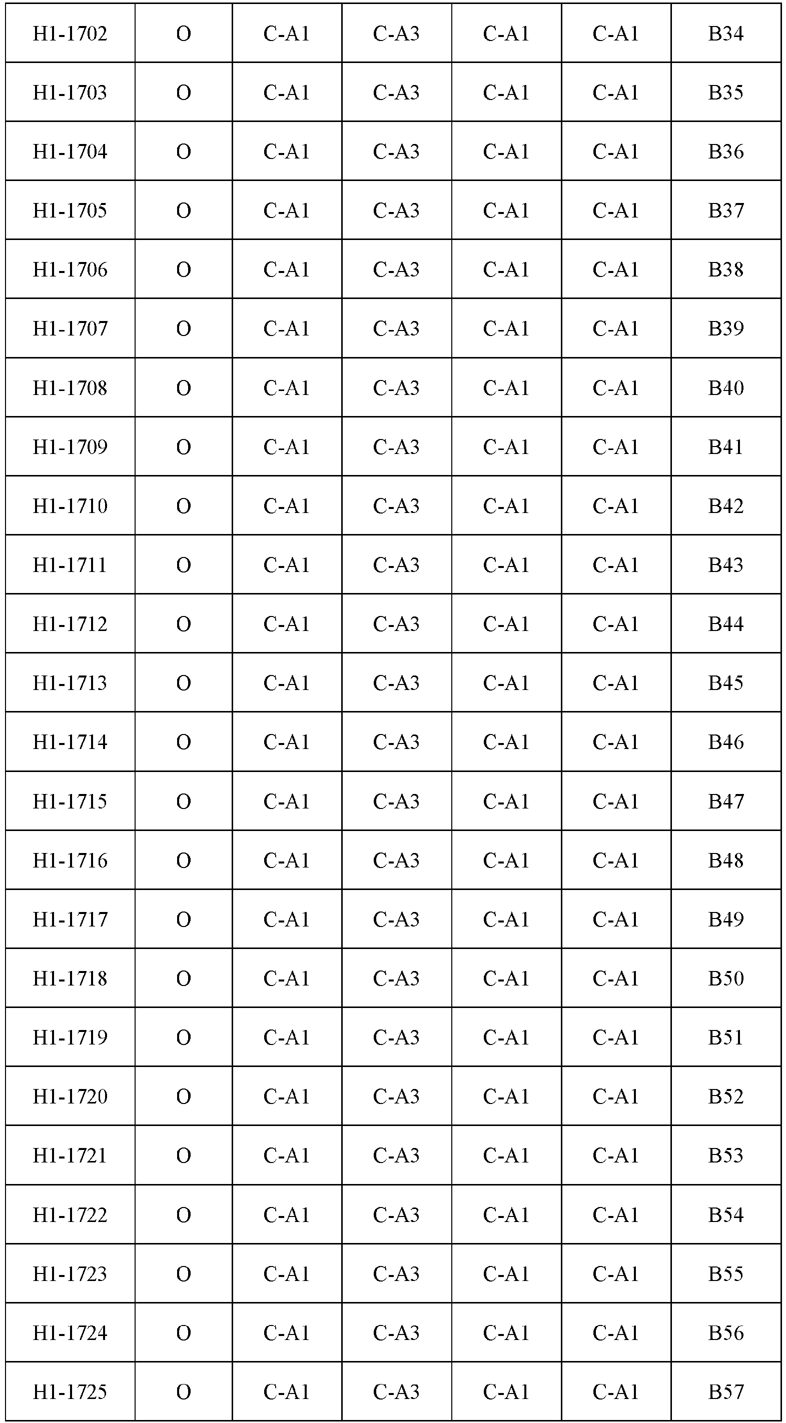 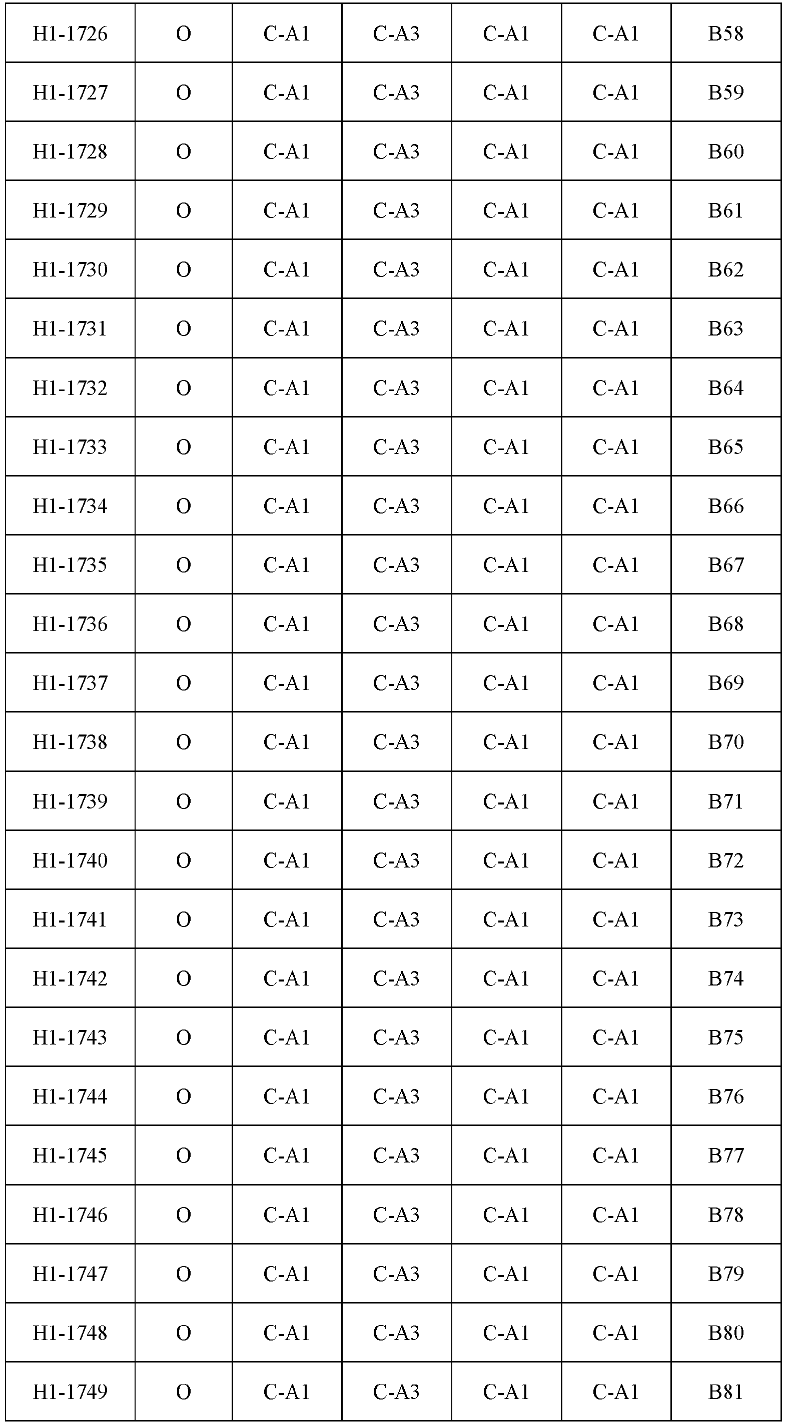 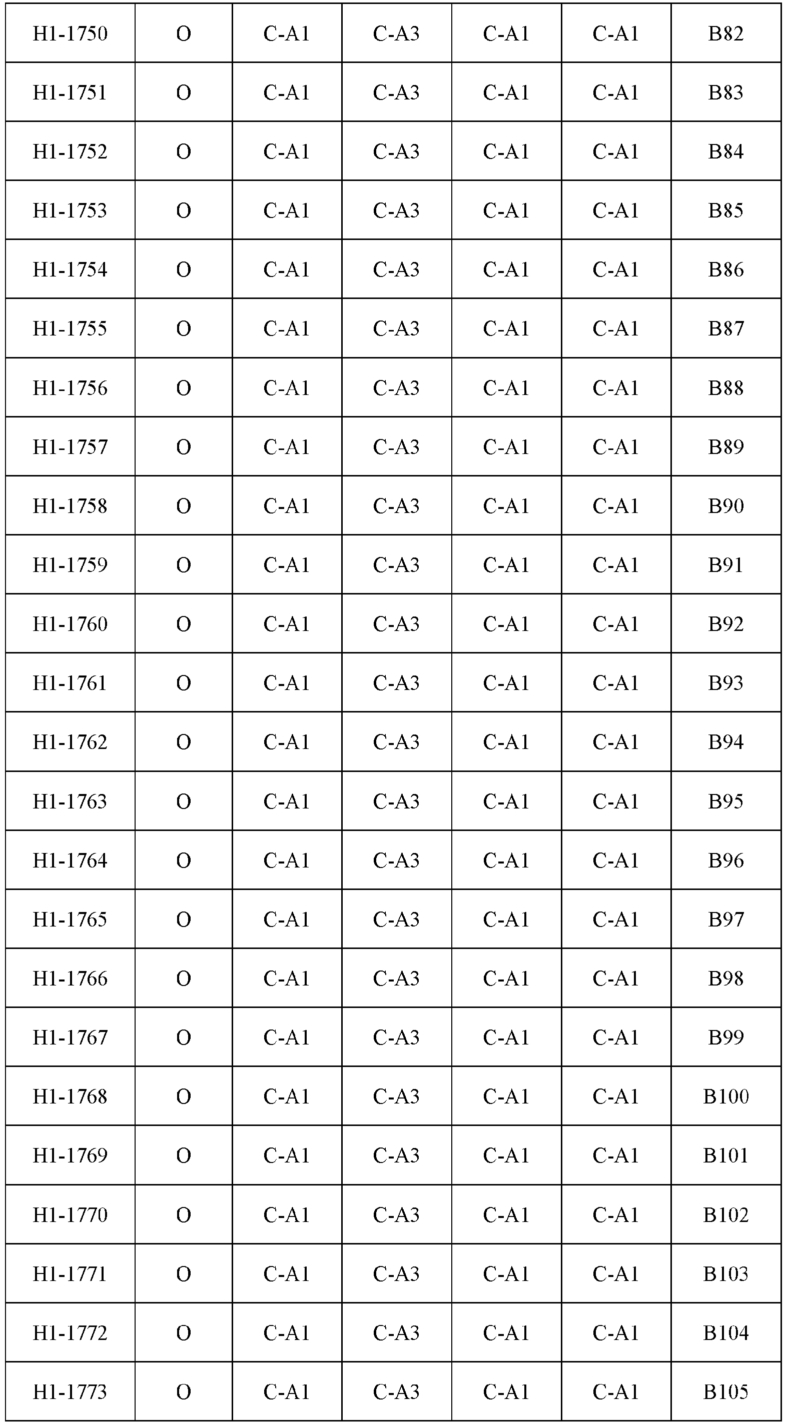 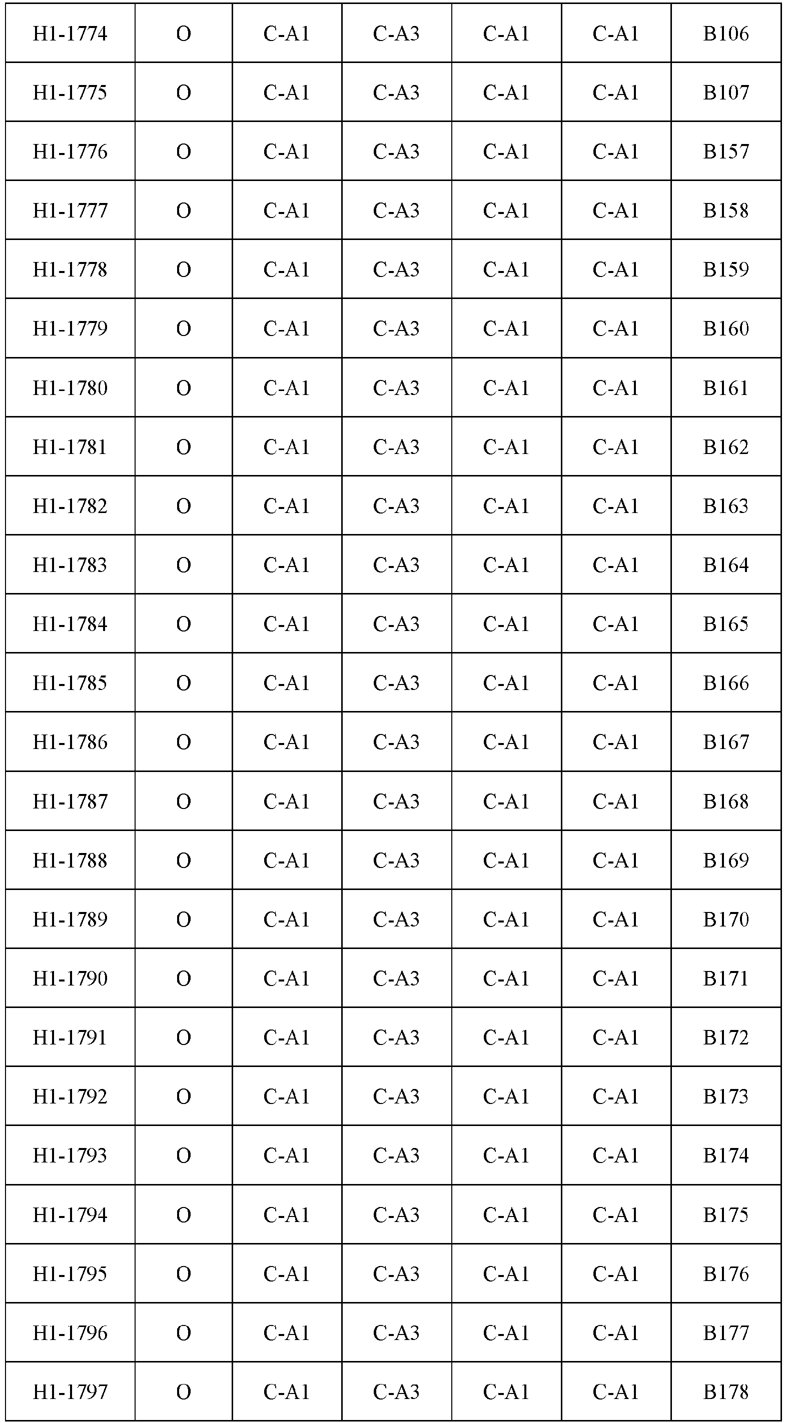 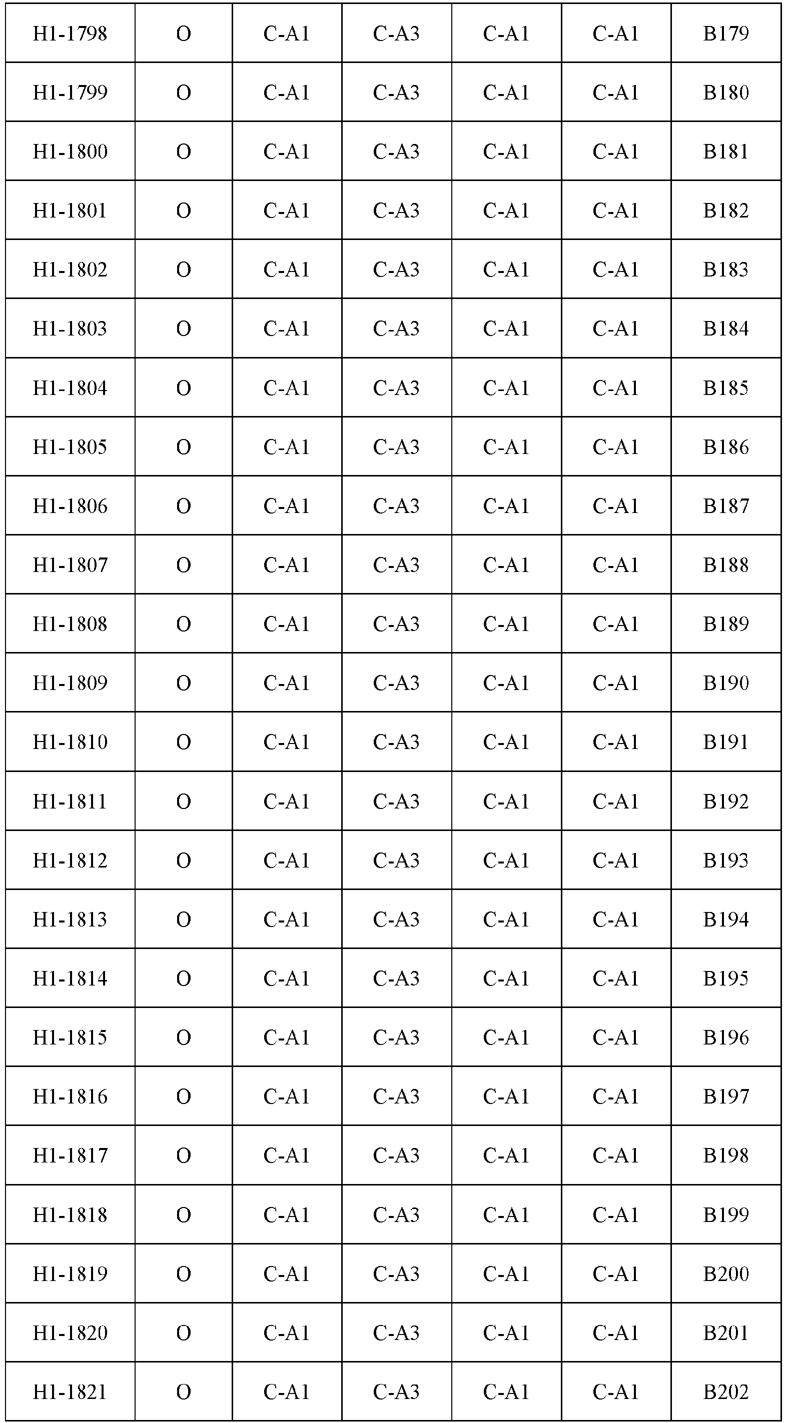 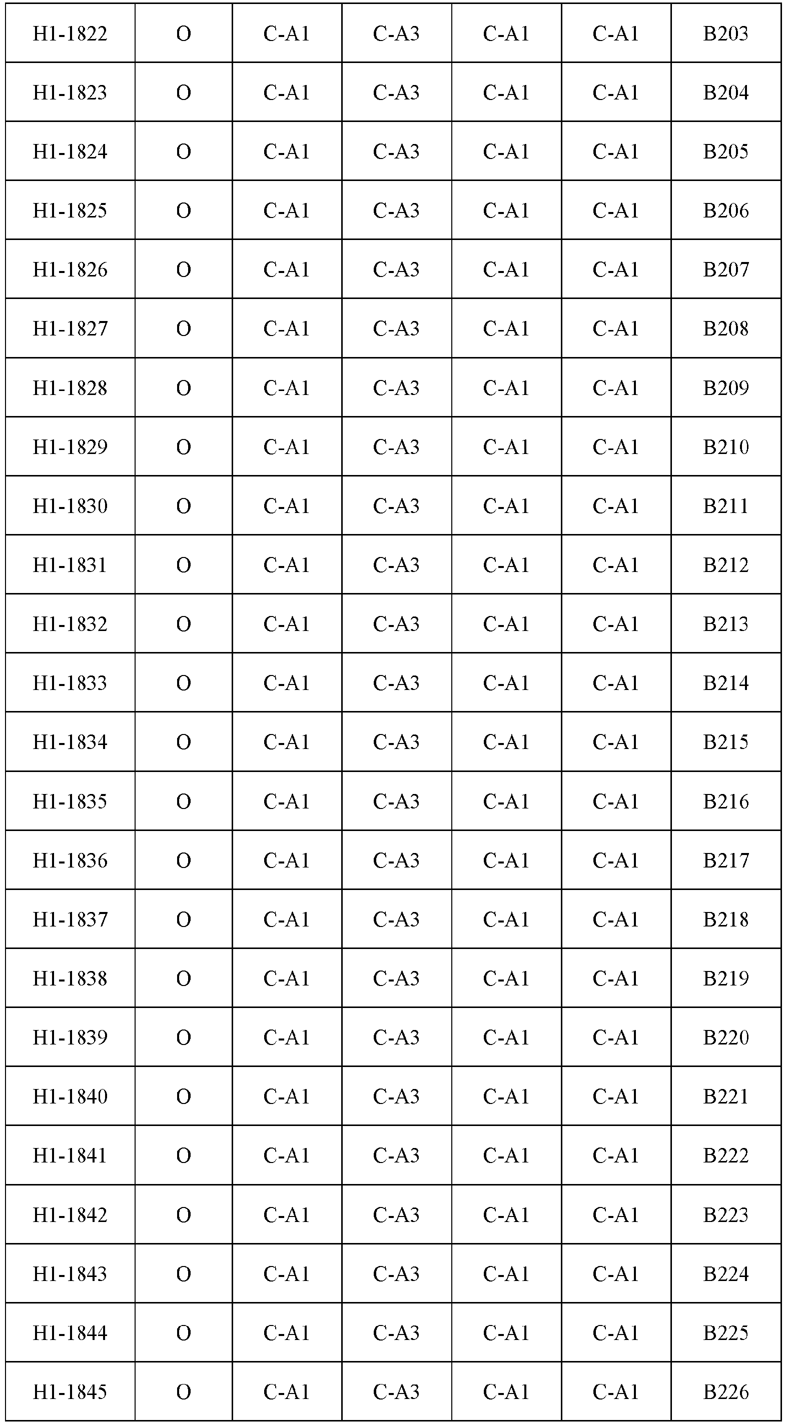 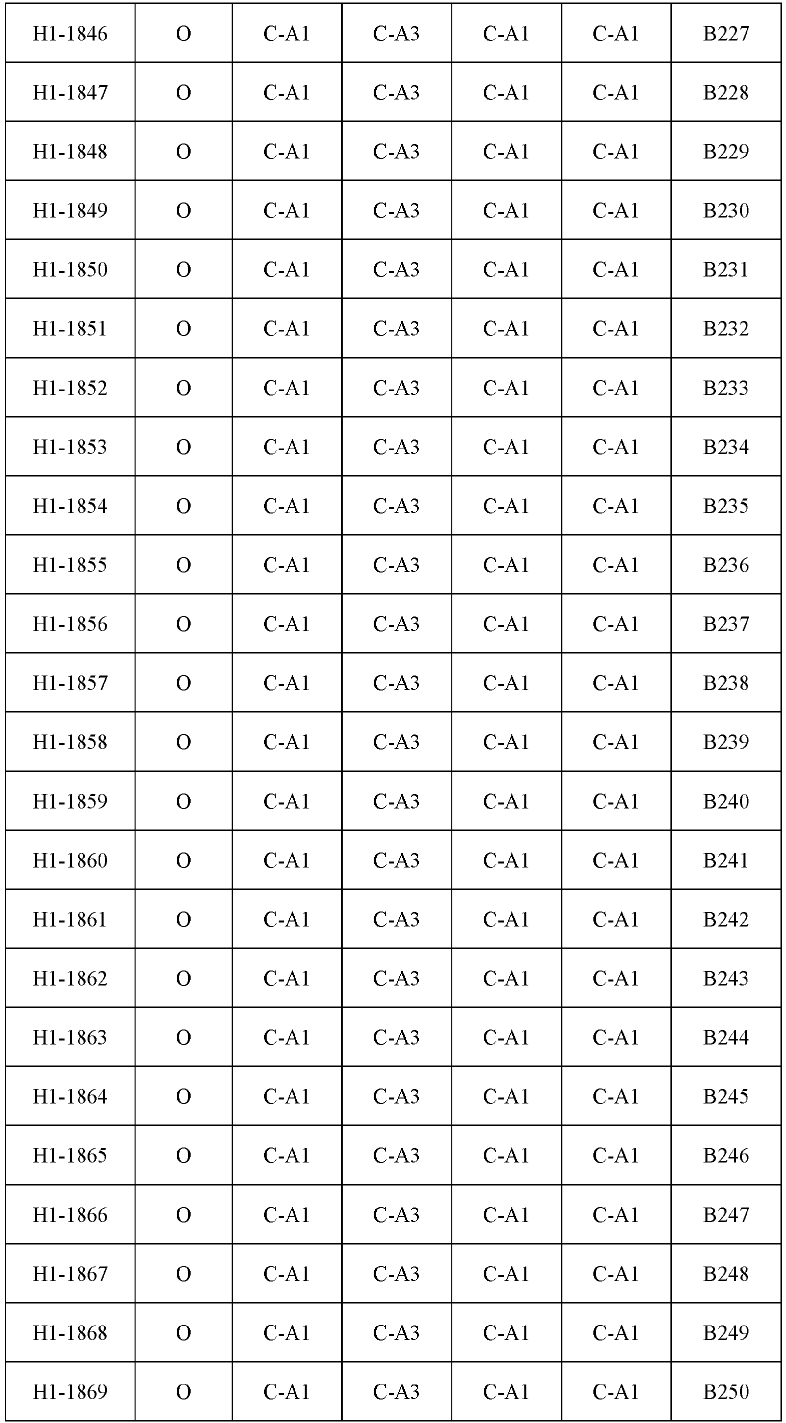 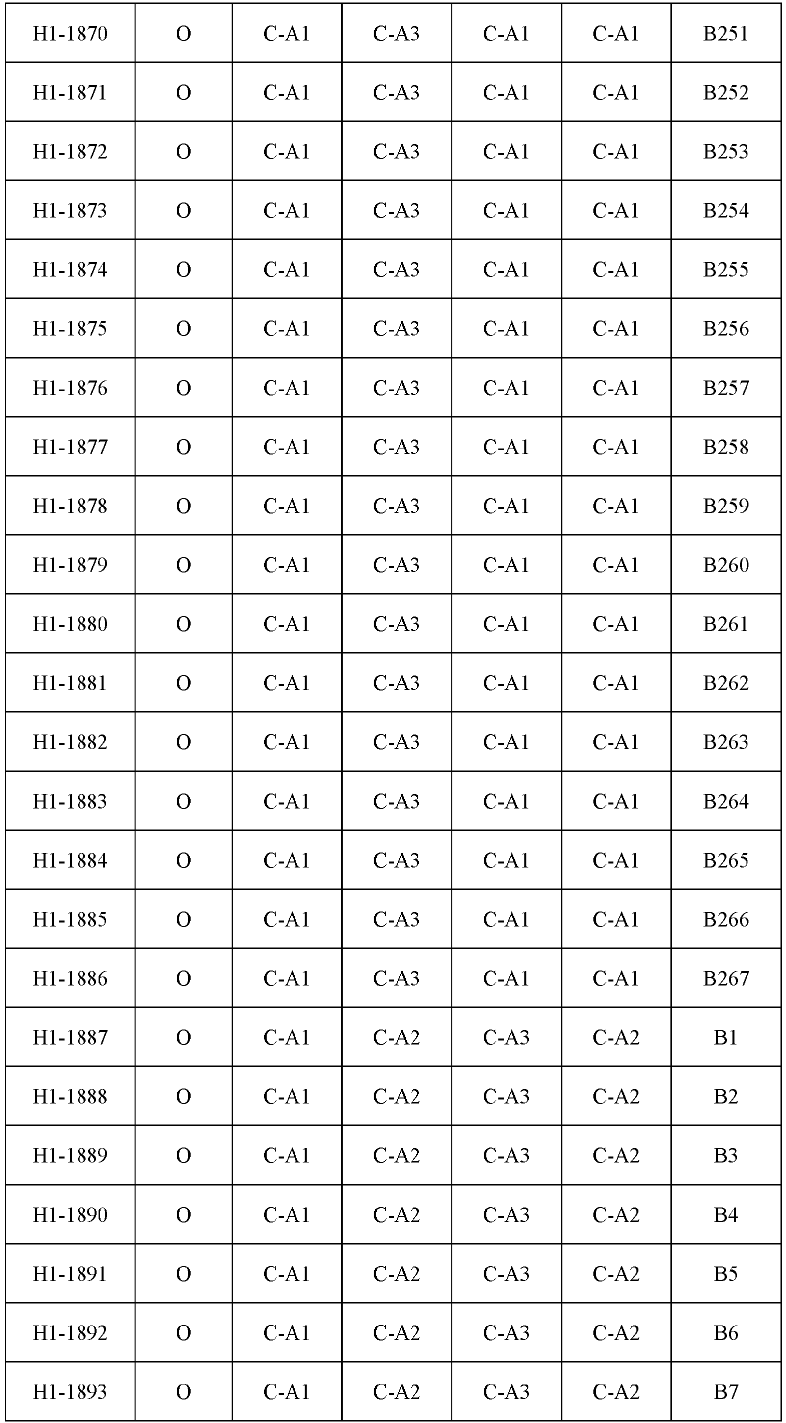 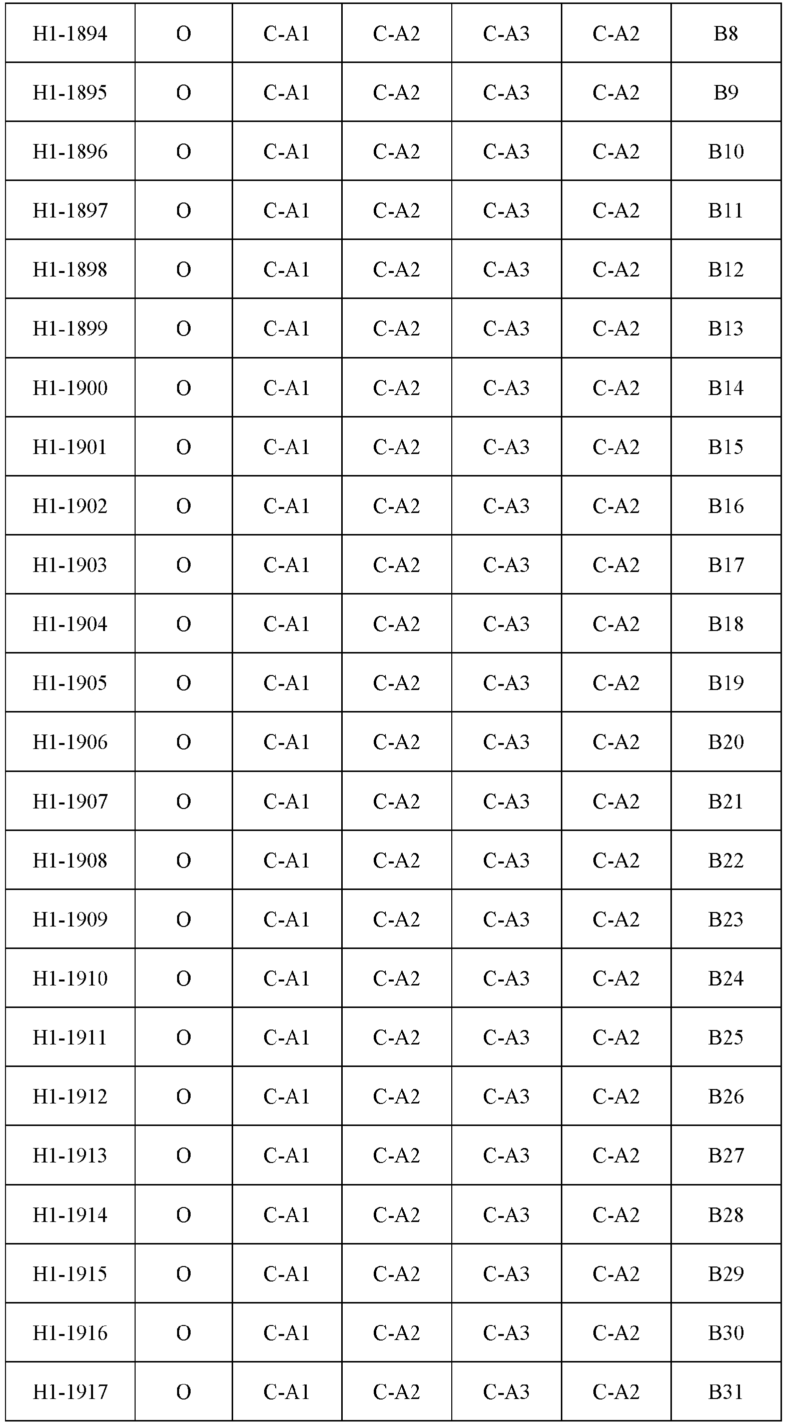 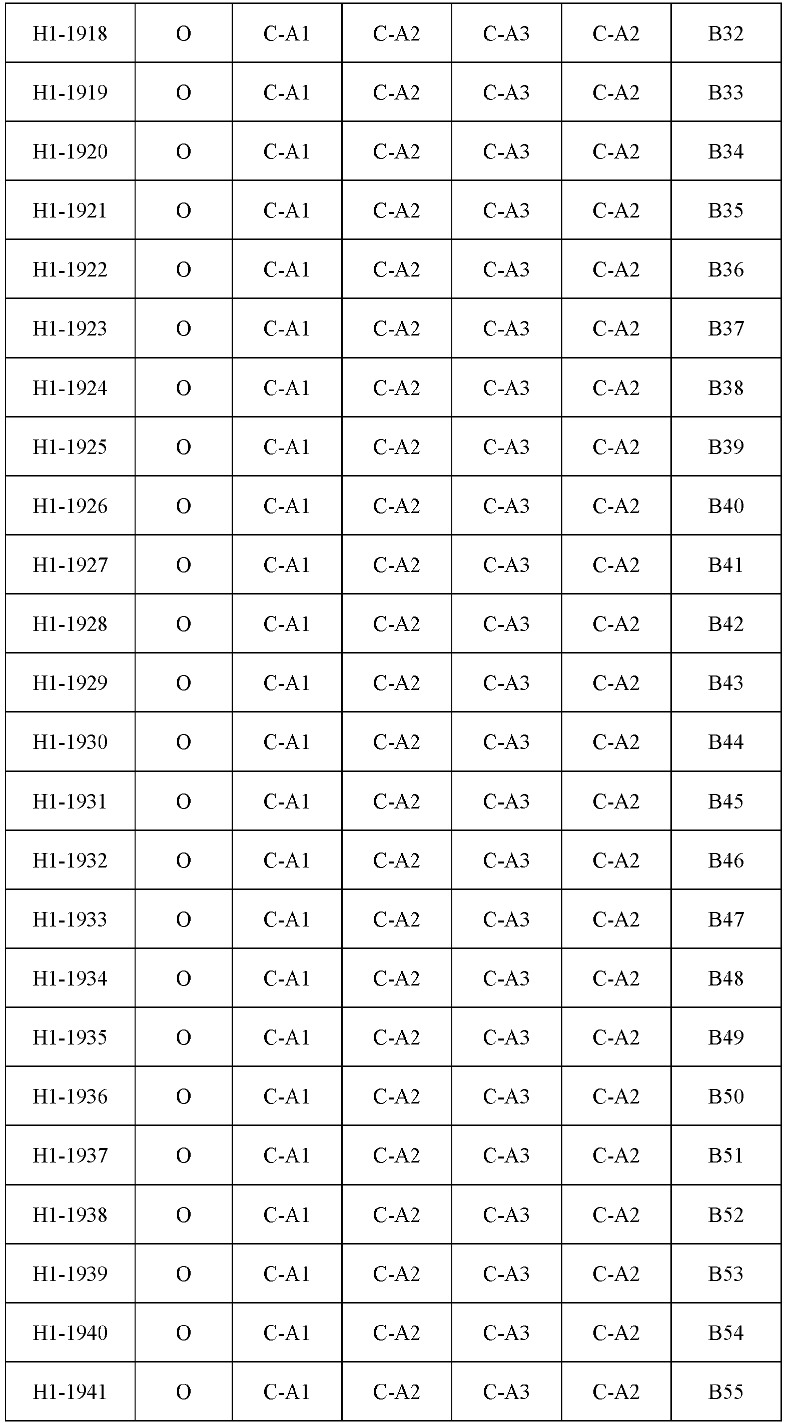 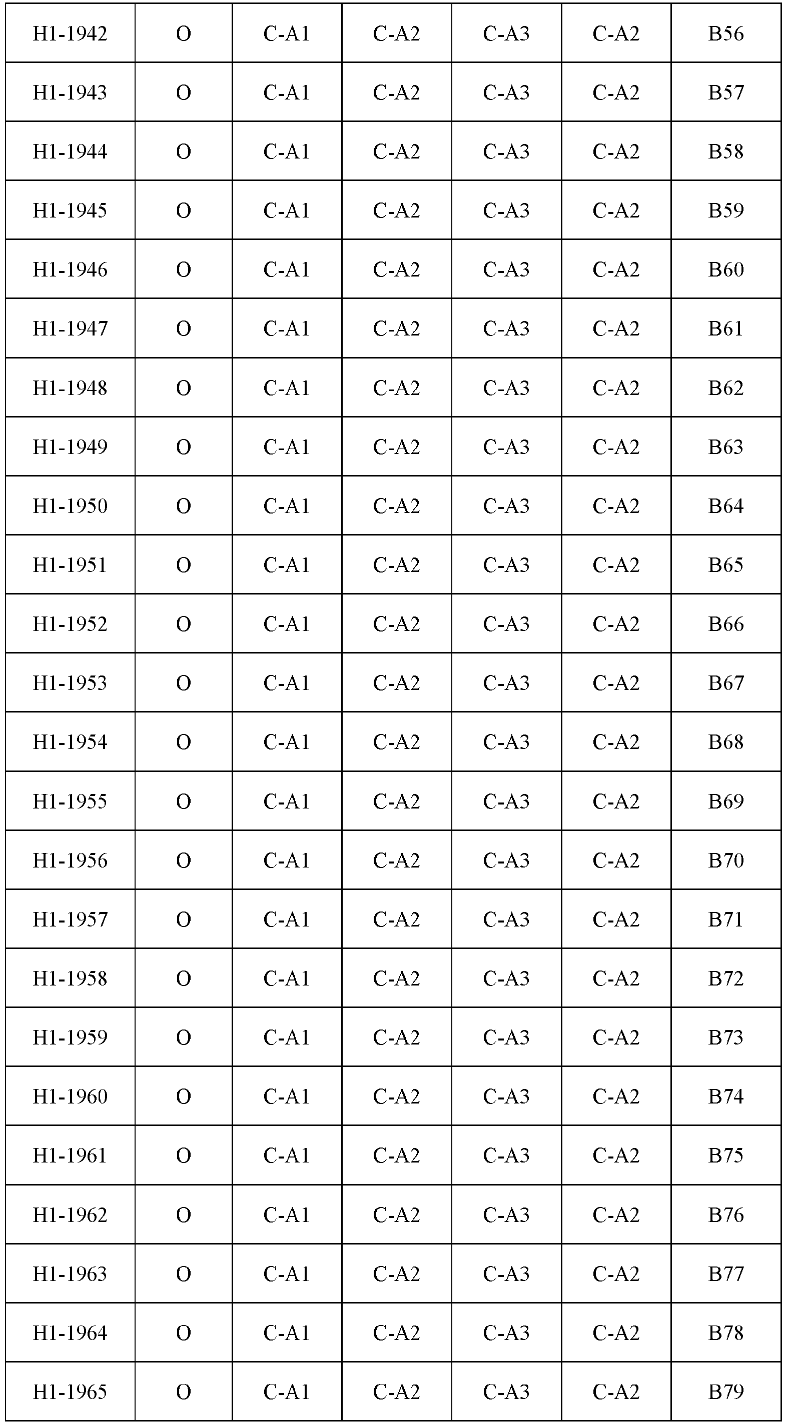 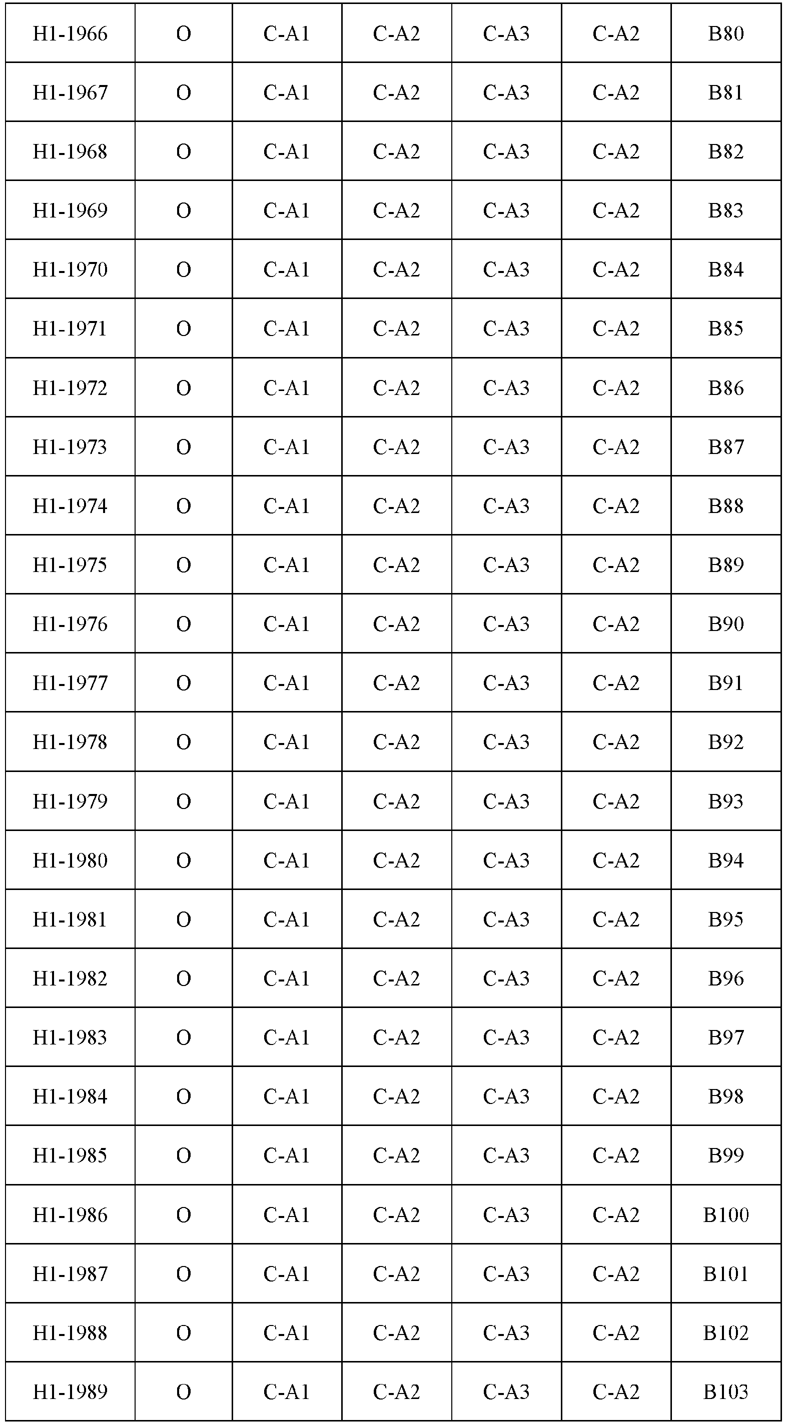 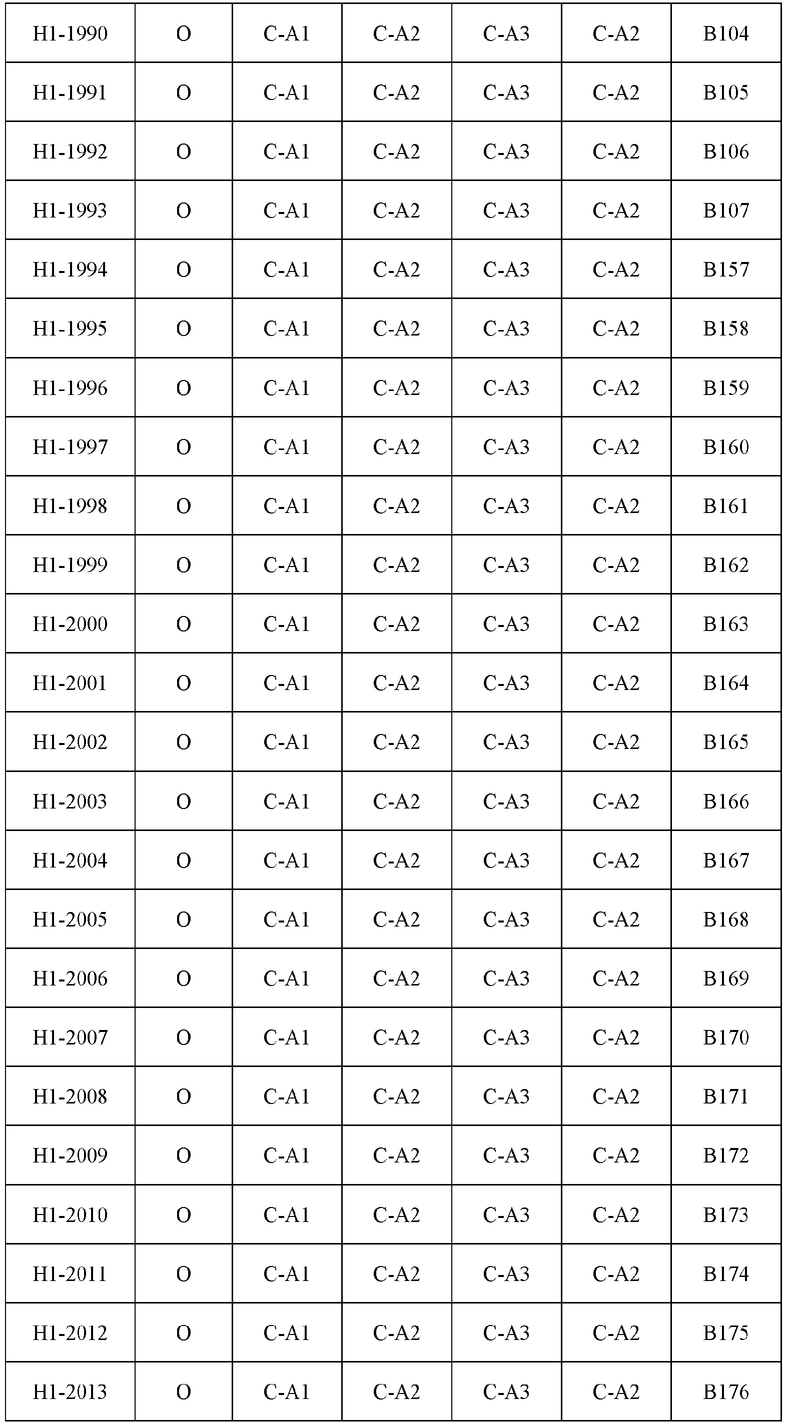 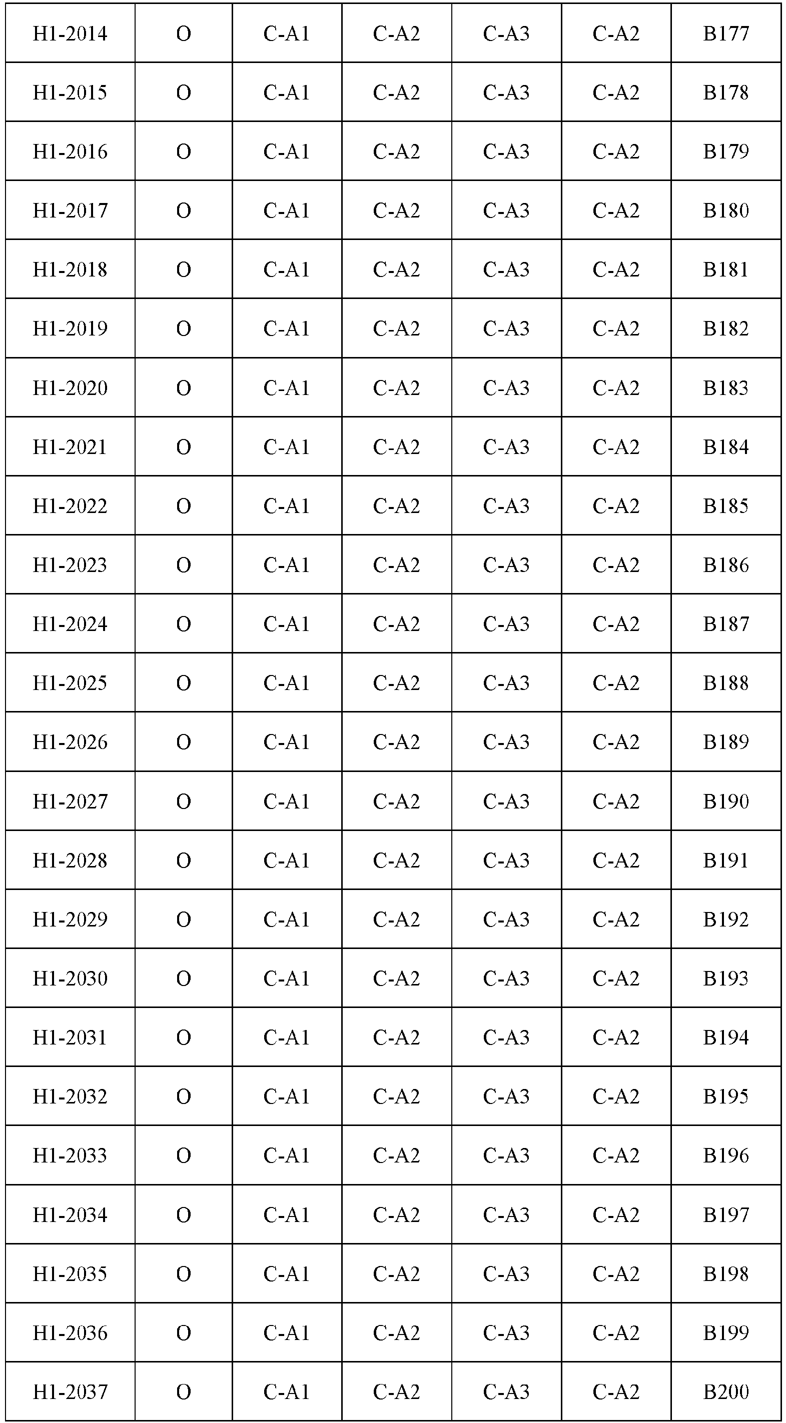 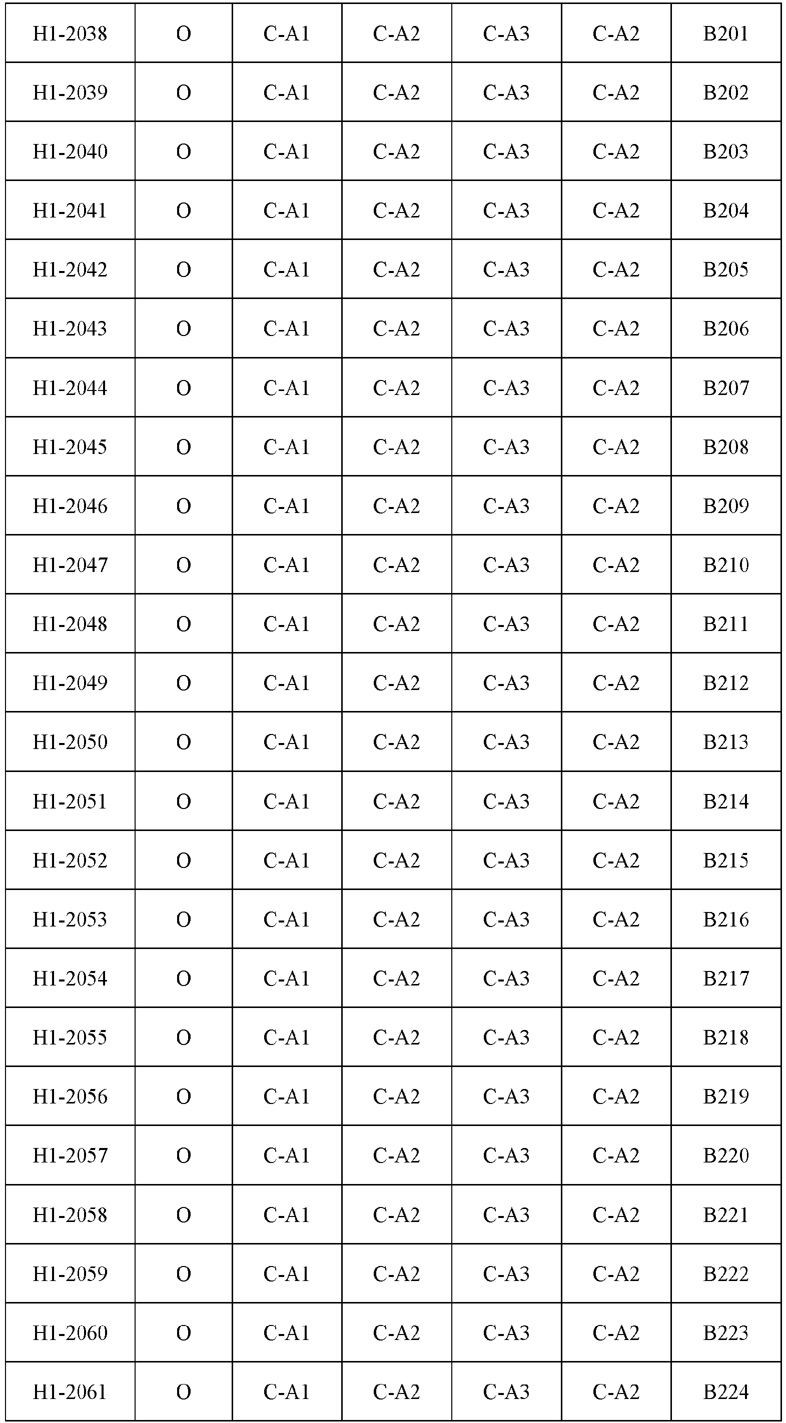 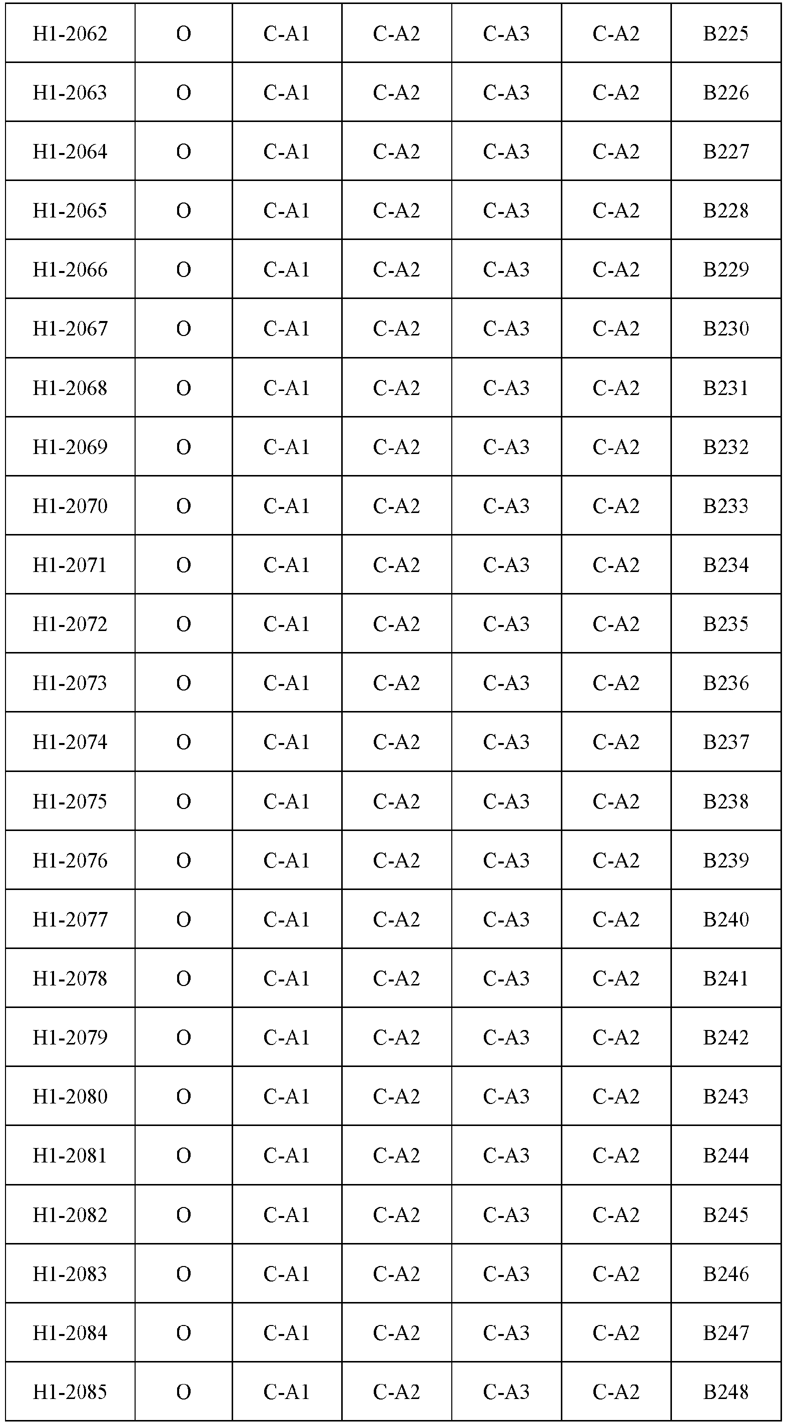 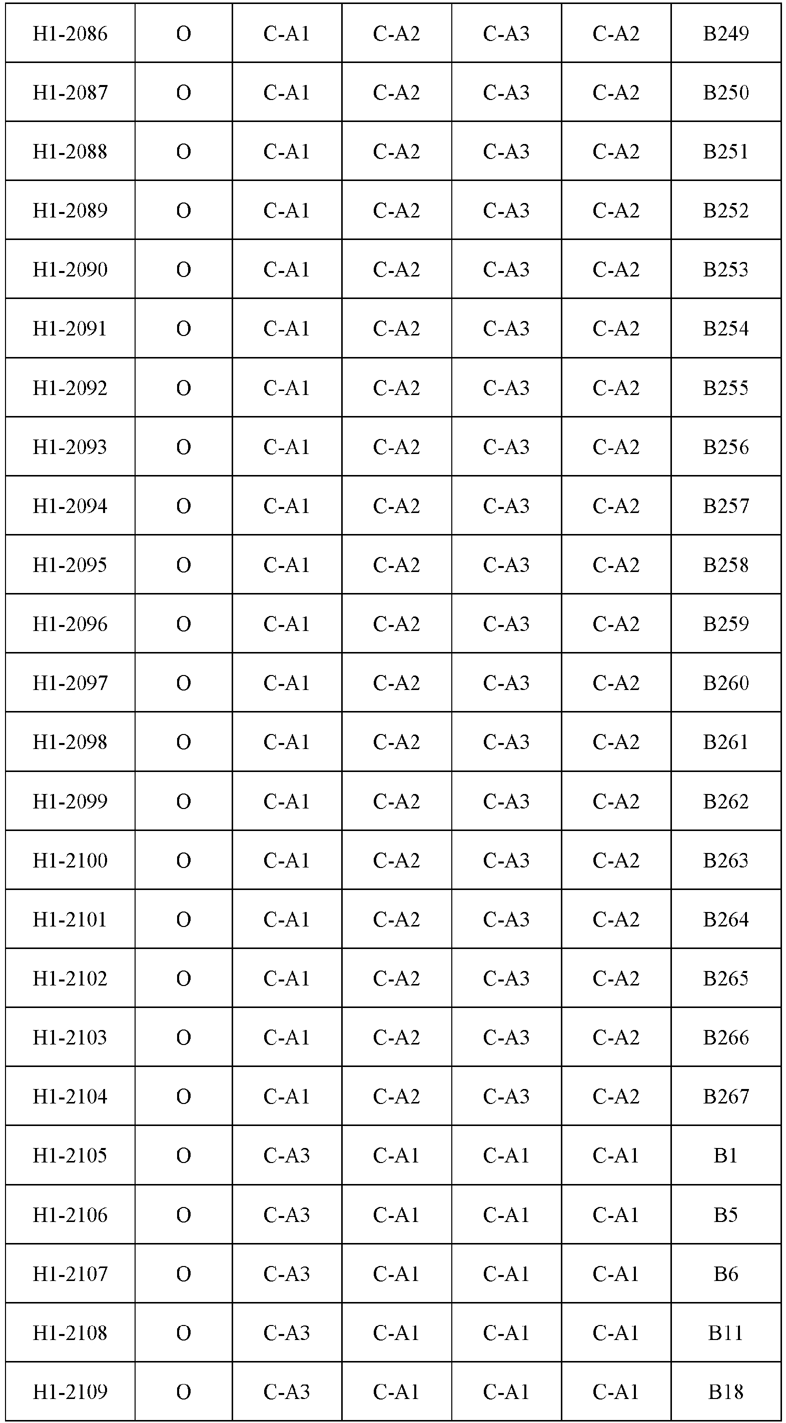 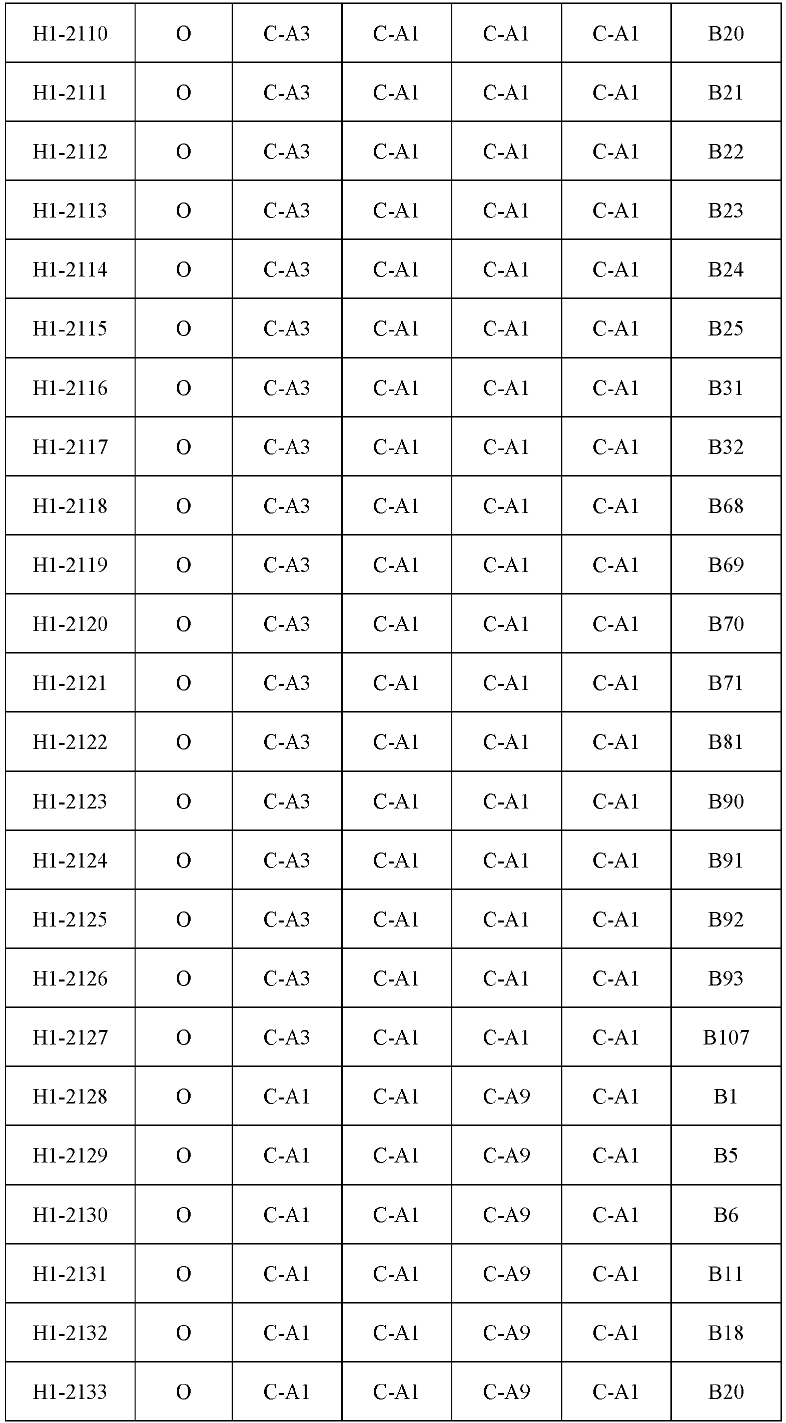 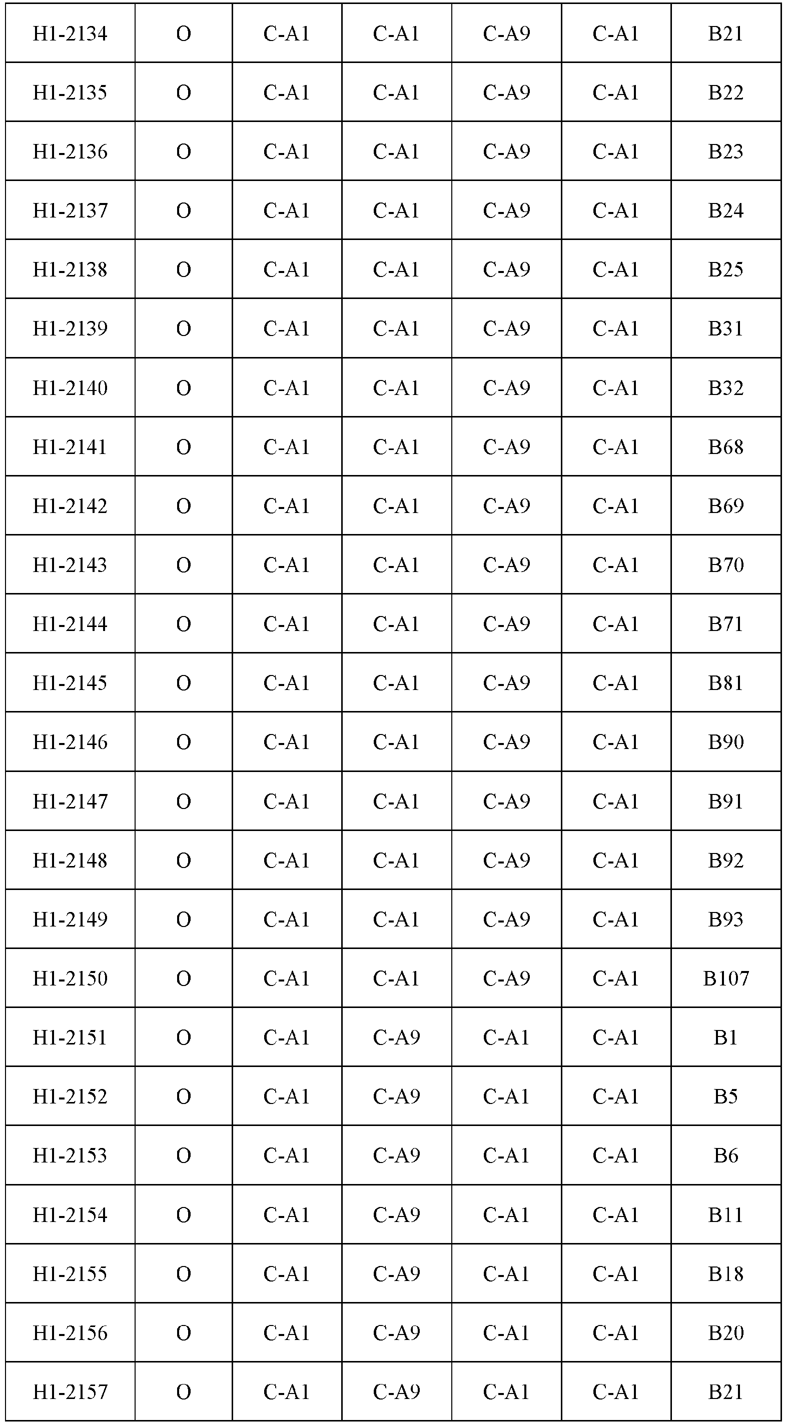 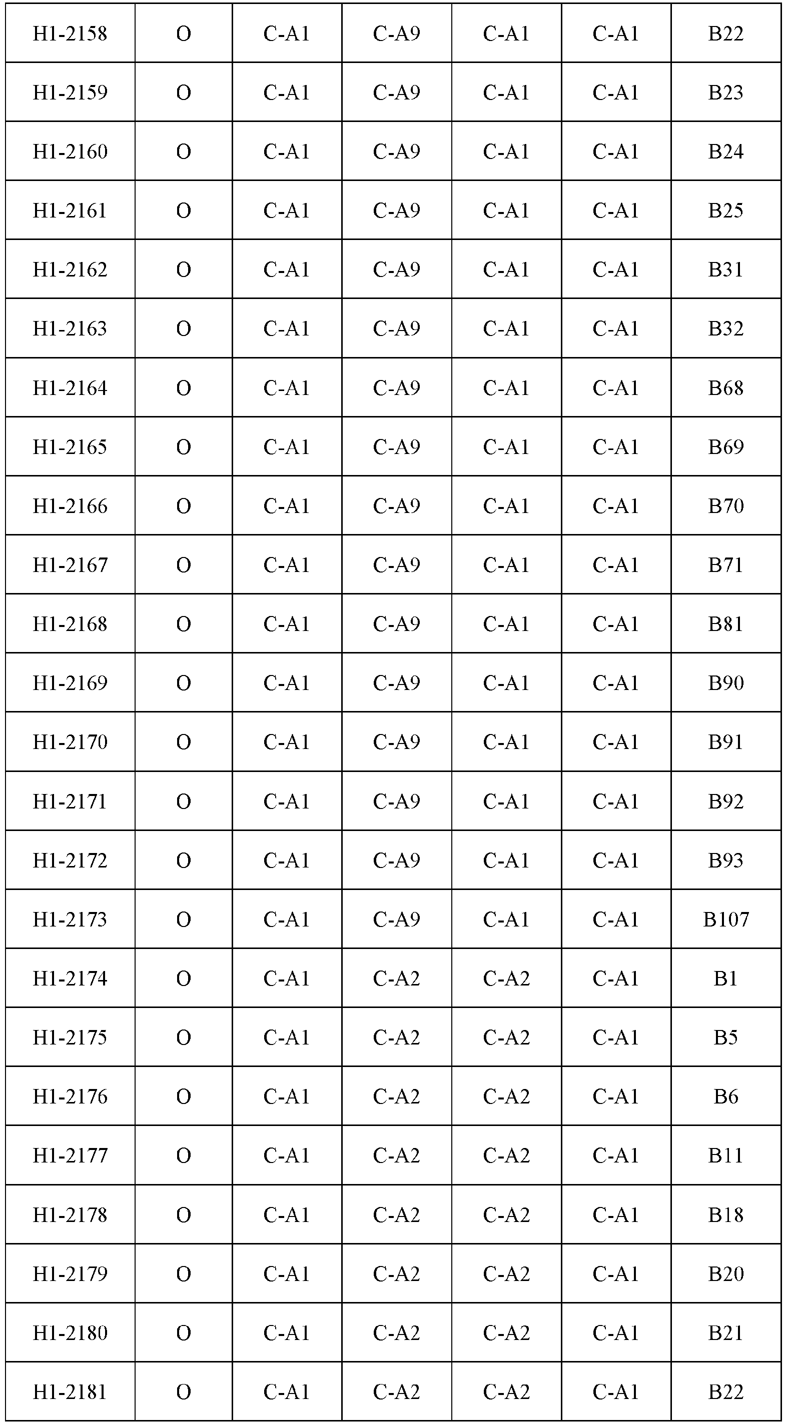 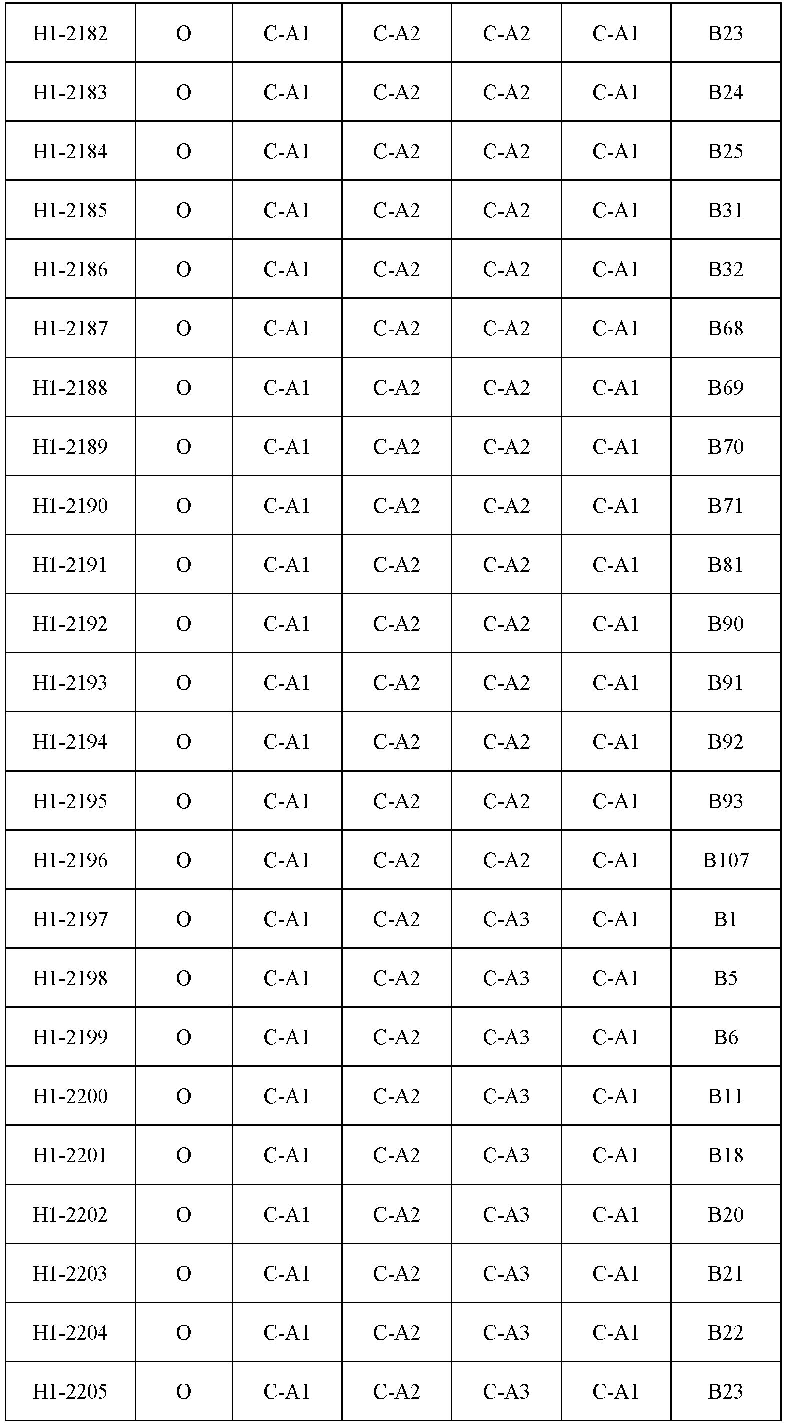 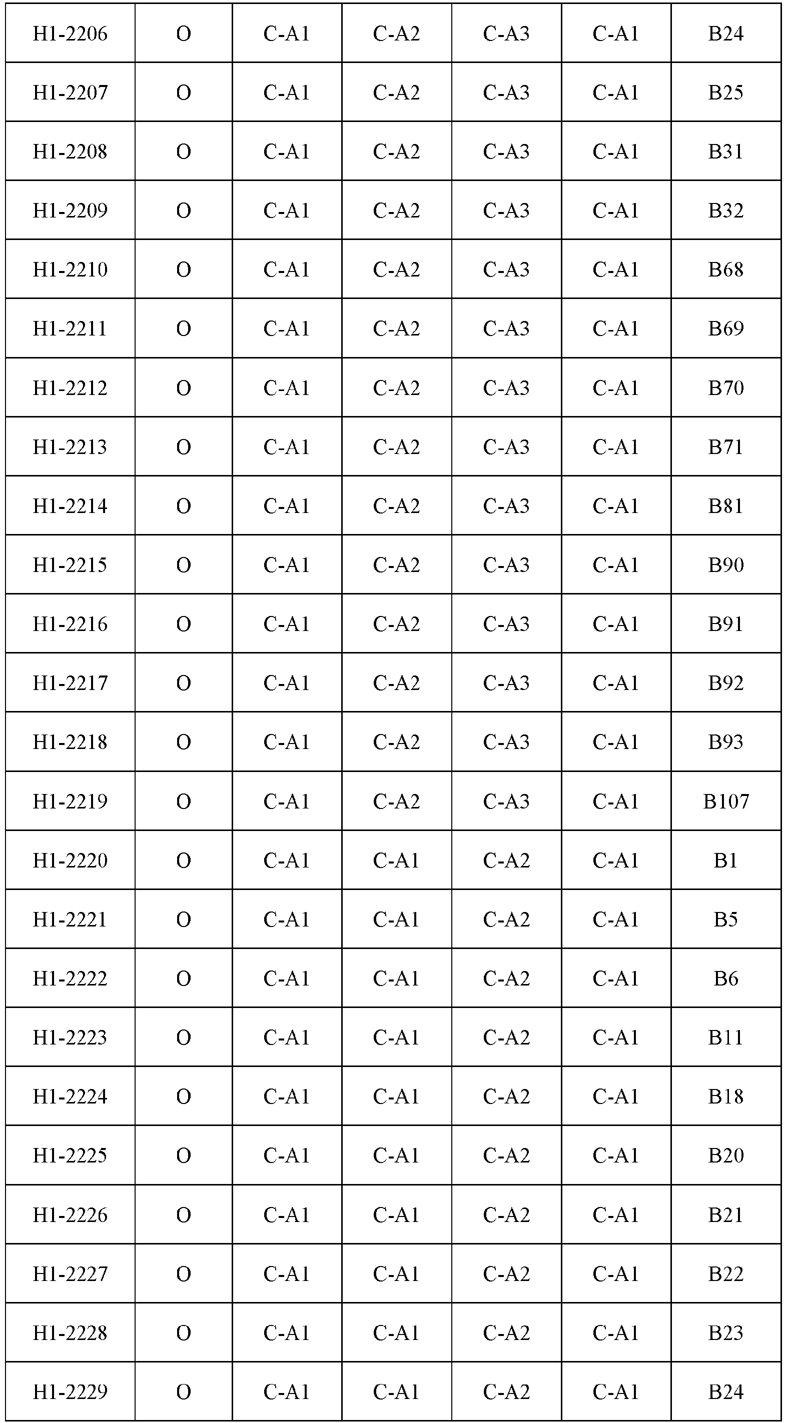 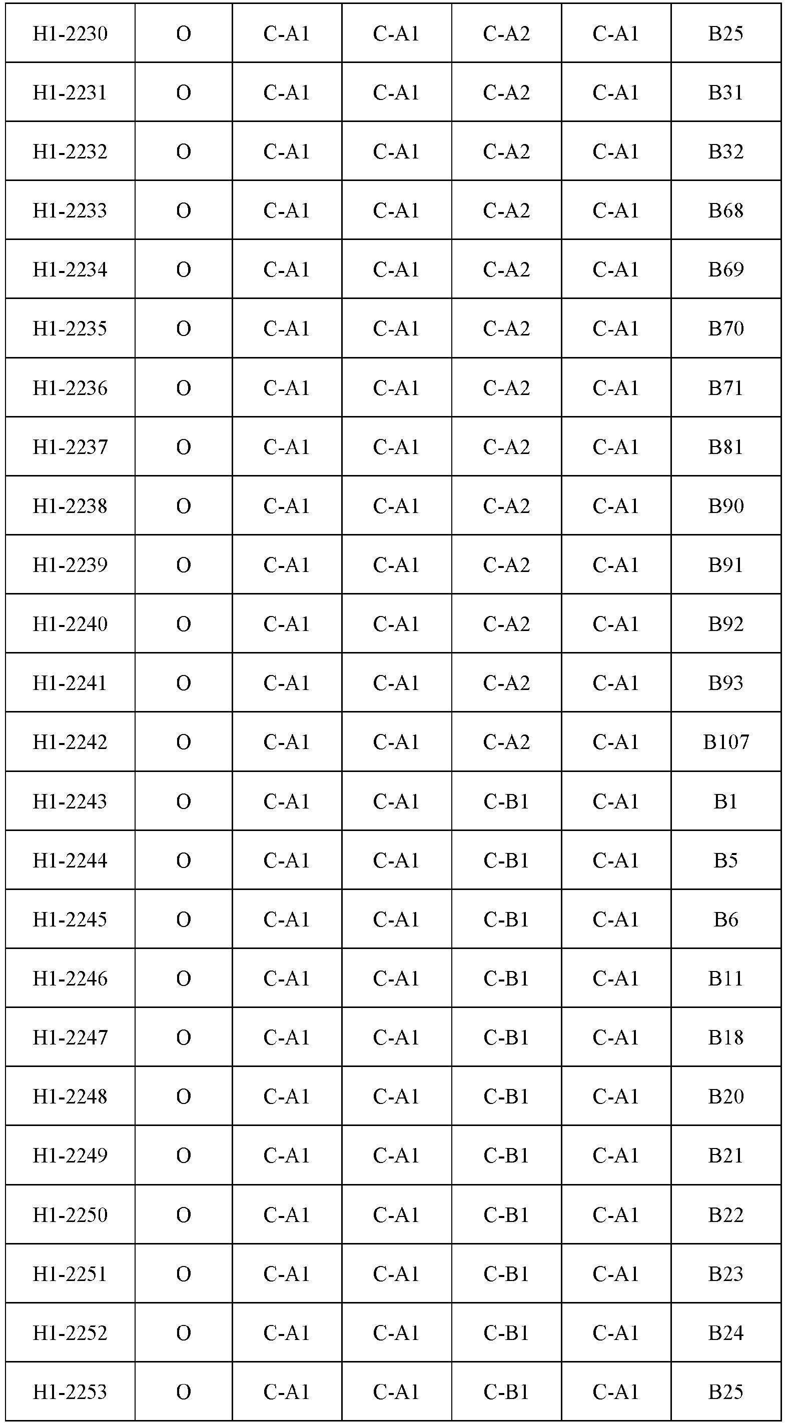 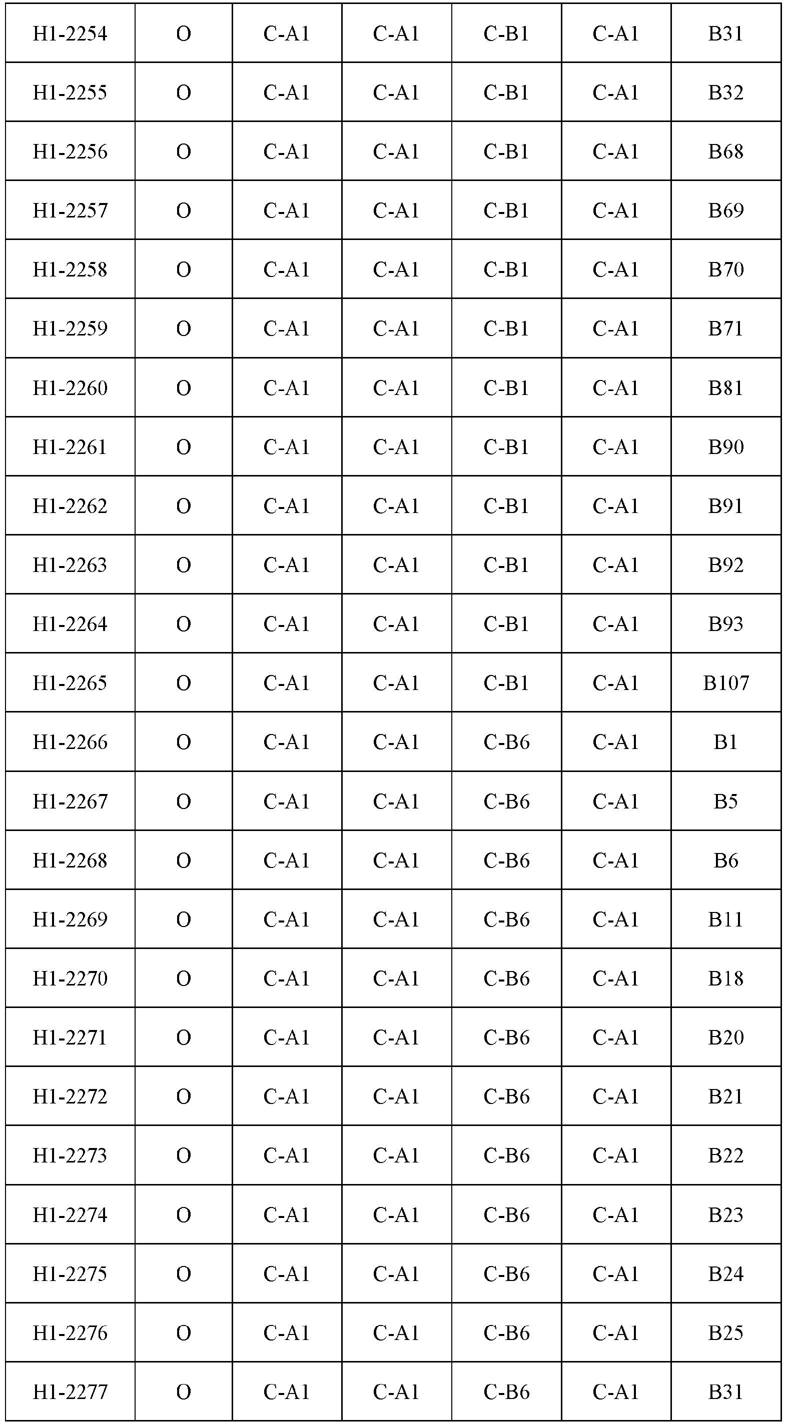 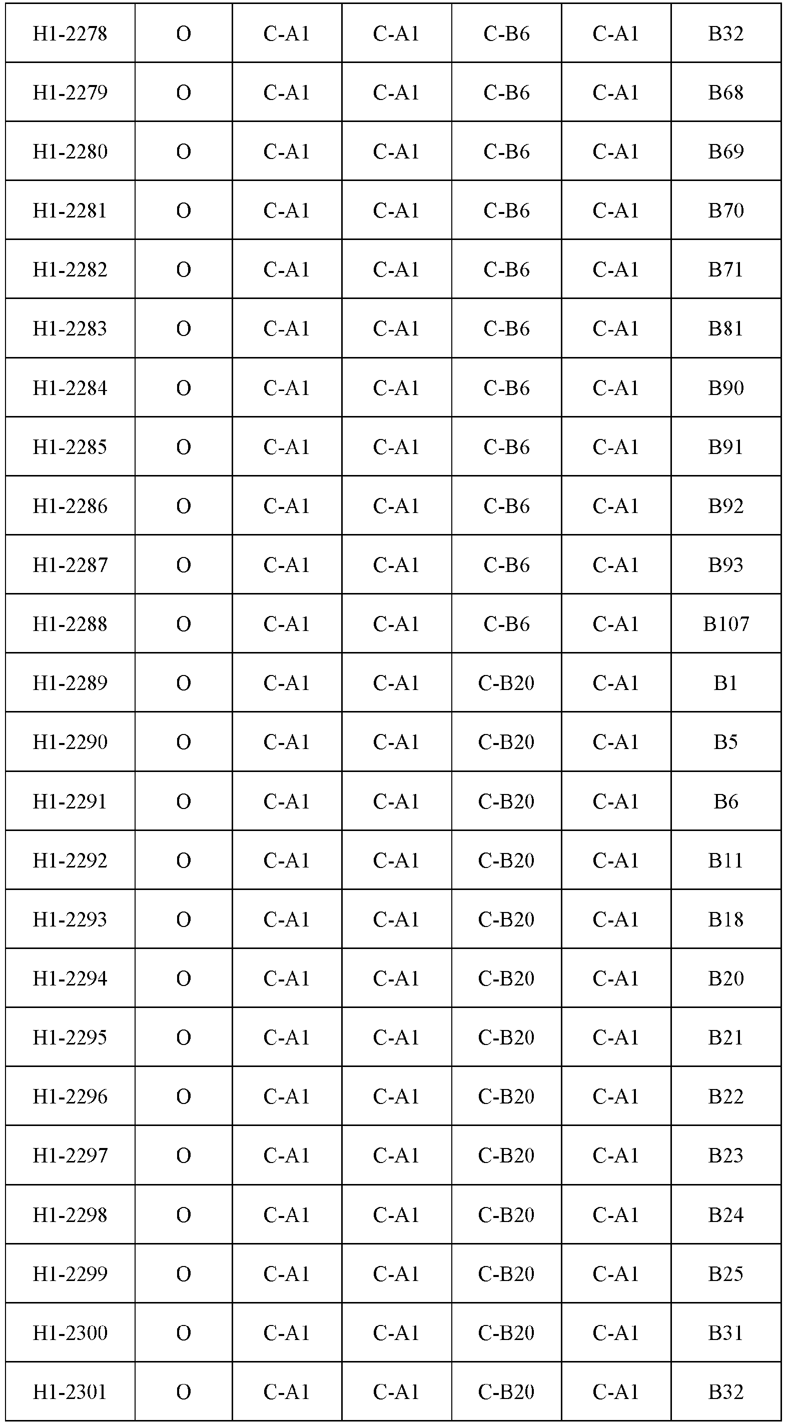 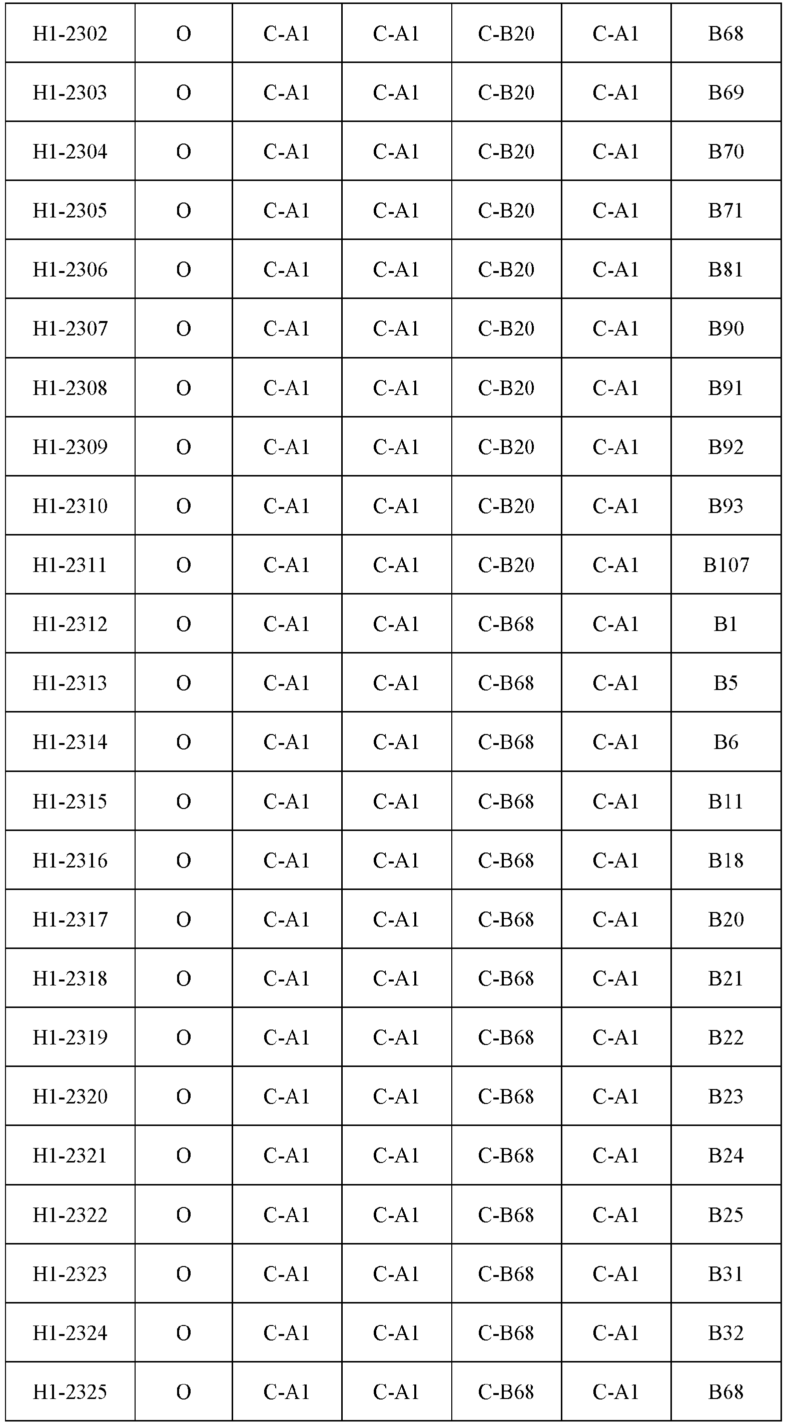 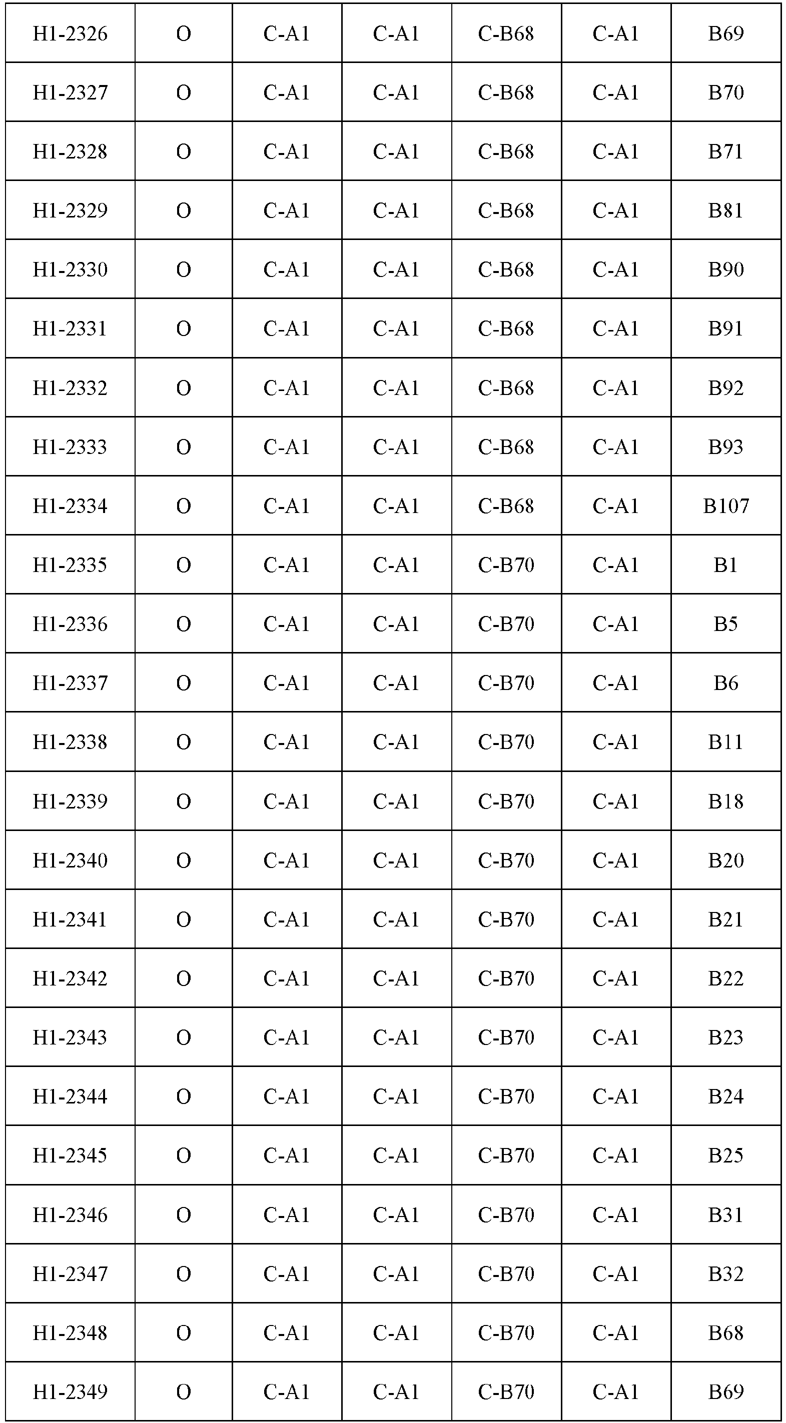 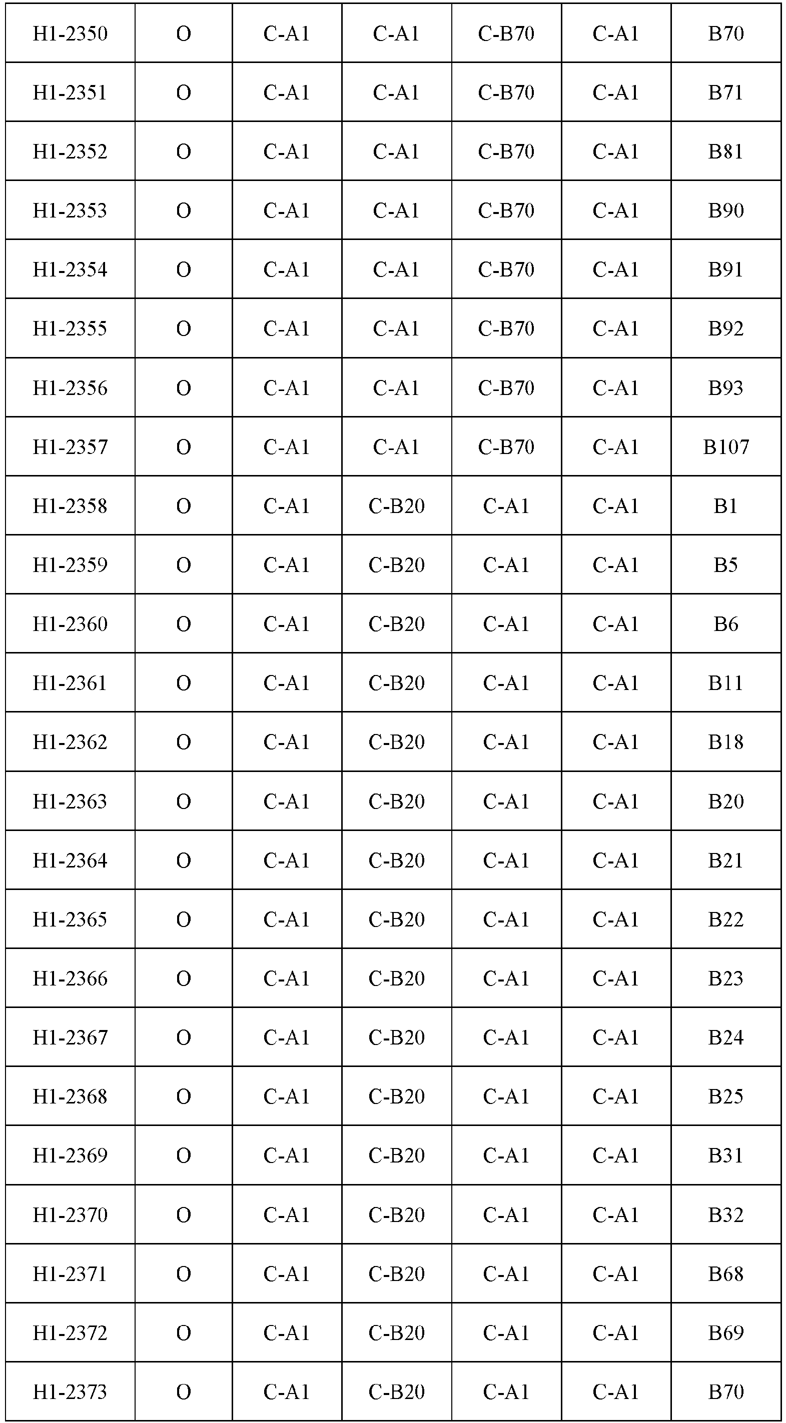 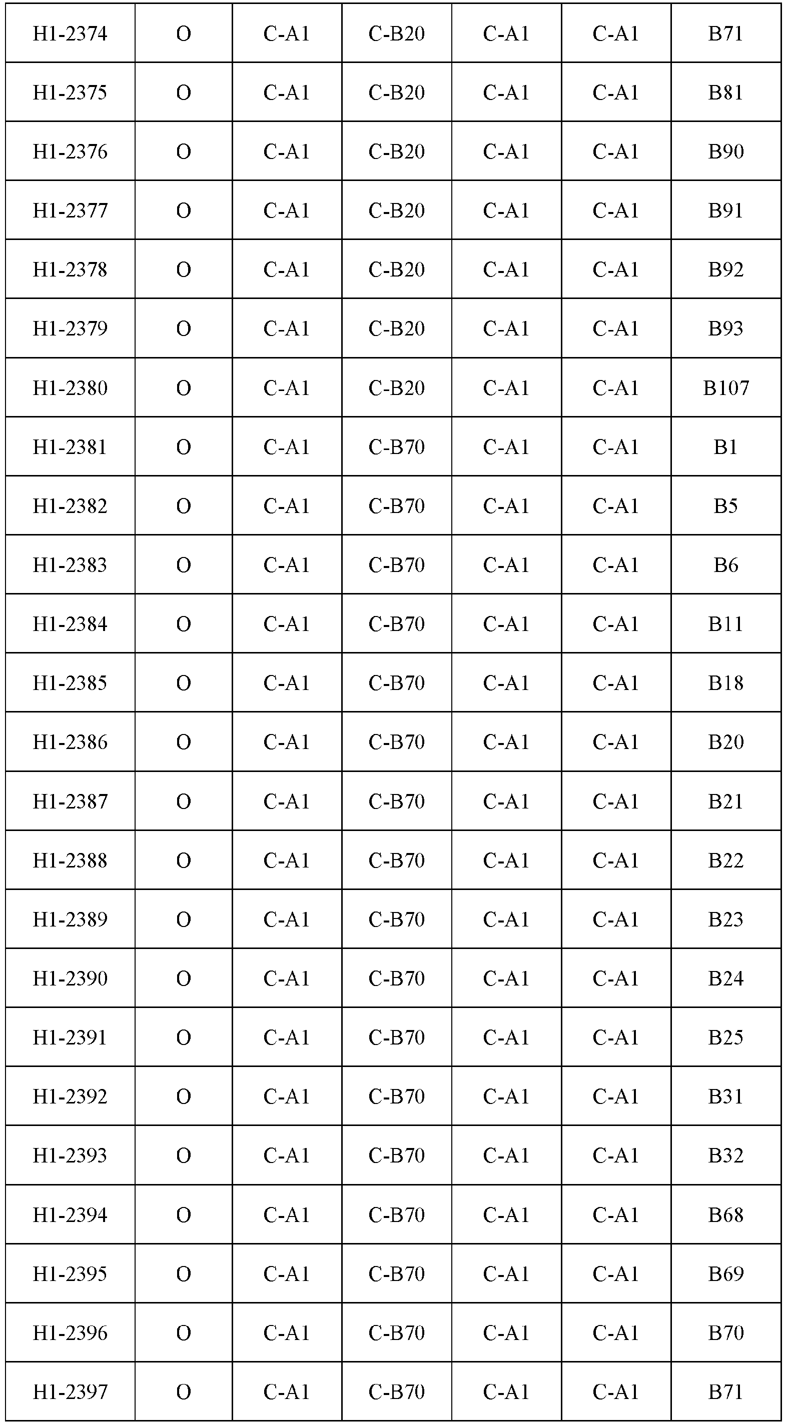 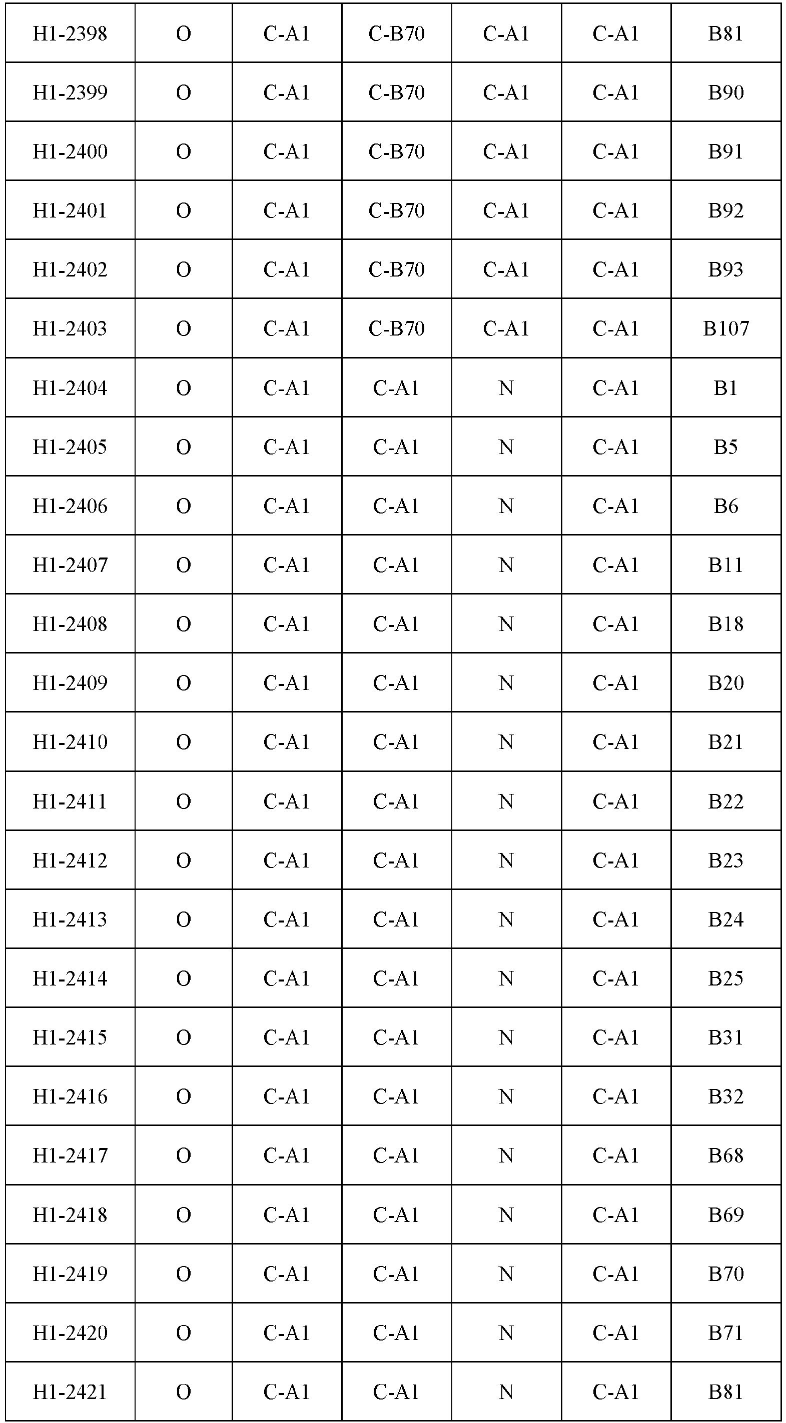 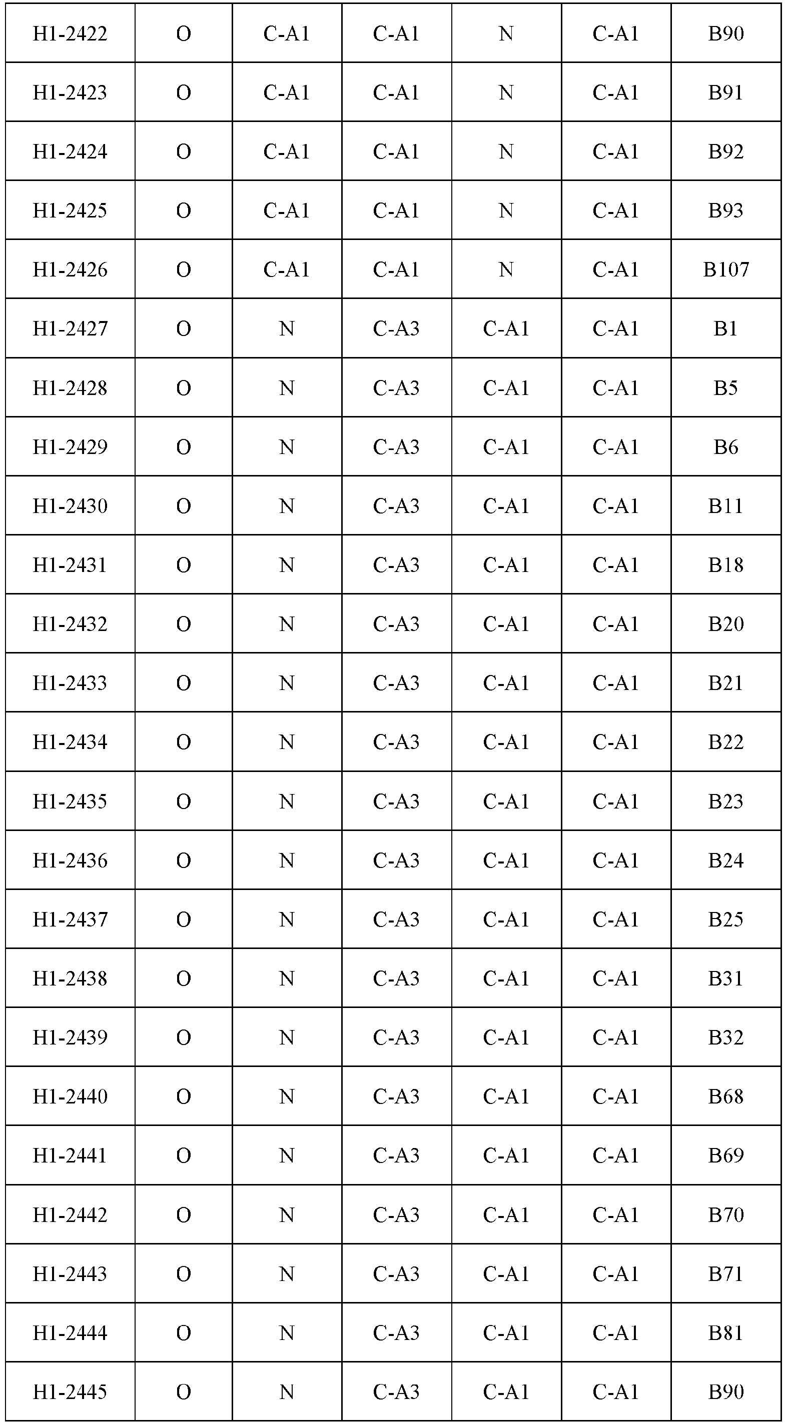 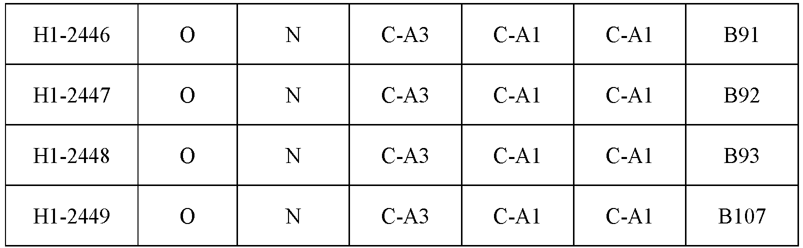
wobei Verbindung H2-1 bis Verbindung H2-138 jeweils eine durch die Formel 2-2-1 dargestellte Struktur haben: 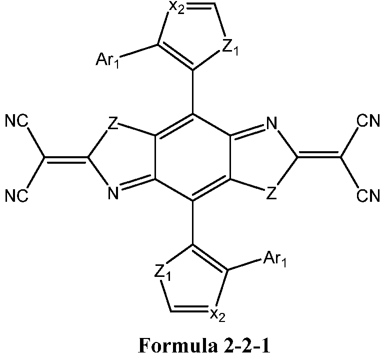
in Formel 2-2-1 zwei Z gleich sind, zwei Z₁ gleich sind, zwei X₂ gleich sind, zwei An gleich sind und Z, Z₁, X₂ und Ar₁ jeweils aus den Atomen oder Gruppen in der folgenden Tabelle ausgewählt sind:
| Verbindung Nr. | Z | Z₁ | X₂ | Ar₁ |
|---|---|---|---|---|
| H2-1 | O | O | C-A1 | B1 |
| H2-2 | O | O | C-A1 | B5 |
| H2-3 | O | O | C-A1 | B6 |
| H2-4 | O | O | C-A1 | B11 |
| H2-5 | O | O | C-A1 | B18 |
| H2-6 | O | O | C-A1 | B20 |
| H2-7 | O | O | C-A1 | B21 |
| H2-8 | O | O | C-A1 | B22 |
| H2-9 | O | O | C-A1 | B23 |
| H2-10 | O | O | C-A1 | B24 |
| H2-11 | O | O | C-A1 | B25 |
| H2-12 | O | O | C-A1 | B31 |
| H2-13 | O | O | C-A1 | B32 |
| H2-14 | O | O | C-A1 | B68 |
| H2-15 | O | O | C-A1 | B69 |
| H2-16 | O | O | C-A1 | B70 |
| H2-17 | O | O | C-A1 | B71 |
| H2-18 | O | O | C-A1 | B81 |
| H2-19 | O | O | C-A1 | B90 |
| H2-20 | O | O | C-A1 | B91 |
| H2-21 | O | O | C-A1 | B92 |
| H2-22 | O | O | C-A1 | B93 |
| H2-23 | O | O | C-A1 | B107 |
| H2-24 | S | O | C-A1 | B1 |
| H2-25 | S | O | C-A1 | B5 |
| H2-26 | S | O | C-A1 | B6 |
| H2-27 | S | O | C-A1 | B11 |
| H2-28 | S | O | C-A1 | B18 |
| H2-29 | S | O | C-A1 | B20 |
| H2-30 | S | O | C-A1 | B21 |
| H2-31 | S | O | C-A1 | B22 |
| H2-32 | S | O | C-A1 | B23 |
| H2-33 | S | O | C-A1 | B24 |
| H2-34 | S | O | C-A1 | B25 |
| H2-35 | S | O | C-A1 | B31 |
| H2-36 | S | O | C-A1 | B32 |
| H2-37 | S | O | C-A1 | B68 |
| H2-38 | S | O | C-A1 | B69 |
| H2-39 | S | O | C-A1 | B70 |
| H2-40 | S | O | C-A1 | B71 |
| H2-41 | S | O | C-A1 | B81 |
| H2-42 | S | O | C-A1 | B90 |
| H2-43 | S | O | C-A1 | B91 |
| H2-44 | S | O | C-A1 | B92 |
| H2-45 | S | O | C-A1 | B93 |
| H2-46 | S | O | C-A1 | B107 |
| H2-47 | Se | O | C-A1 | B1 |
| H2-48 | Se | O | C-A1 | B5 |
| H2-49 | Se | O | C-A1 | B6 |
| H2-50 | Se | O | C-A1 | B11 |
| H2-51 | Se | O | C-A1 | B18 |
| H2-52 | Se | O | C-A1 | B20 |
| H2-53 | Se | O | C-A1 | B21 |
| H2-54 | Se | O | C-A1 | B22 |
| H2-55 | Se | O | C-A1 | B23 |
| H2-56 | Se | O | C-A1 | B24 |
| H2-57 | Se | O | C-A1 | B25 |
| H2-58 | Se | O | C-A1 | B31 |
| H2-59 | Se | O | C-A1 | B32 |
| H2-60 | Se | O | C-A1 | B68 |
| H2-61 | Se | O | C-A1 | B69 |
| H2-62 | Se | O | C-A1 | B70 |
| H2-63 | Se | O | C-A1 | B71 |
| H2-64 | Se | O | C-A1 | B81 |
| H2-65 | Se | O | C-A1 | B90 |
| H2-66 | Se | O | C-A1 | B91 |
| H2-67 | Se | O | C-A1 | B92 |
| H2-68 | Se | O | C-A1 | B93 |
| H2-69 | Se | O | C-A1 | B107 |
| H2-70 | O | S | C-A1 | B1 |
| H2-71 | O | S | C-A1 | B5 |
| H2-72 | O | S | C-A1 | B6 |
| H2-73 | O | S | C-A1 | B11 |
| H2-74 | O | S | C-A1 | B18 |
| H2-75 | O | S | C-A1 | B20 |
| H2-76 | O | S | C-A1 | B21 |
| H2-77 | O | S | C-A1 | B22 |
| H2-78 | O | S | C-A1 | B23 |
| H2-79 | O | S | C-A1 | B24 |
| H2-80 | O | S | C-A1 | B25 |
| H2-81 | O | S | C-A1 | B31 |
| H2-82 | O | S | C-A1 | B32 |
| H2-83 | O | S | C-A1 | B68 |
| H2-84 | O | S | C-A1 | B69 |
| H2-85 | O | S | C-A1 | B70 |
| H2-86 | O | S | C-A1 | B71 |
| H2-87 | O | S | C-A1 | B81 |
| H2-88 | O | S | C-A1 | B90 |
| H2-89 | O | S | C-A1 | B91 |
| H2-90 | O | S | C-A1 | B92 |
| H2-91 | O | S | C-A1 | B93 |
| H2-92 | O | S | C-A1 | B107 |
| H2-93 | O | O | N | B1 |
| H2-94 | O | O | N | B5 |
| H2-95 | O | O | N | B6 |
| H2-96 | O | O | N | B11 |
| H2-97 | O | O | N | B18 |
| H2-98 | O | O | N | B20 |
| H2-99 | O | O | N | B21 |
| H2-100 | O | O | N | B22 |
| H2-101 | O | O | N | B23 |
| H2-102 | O | O | N | B24 |
| H2-103 | O | O | N | B25 |
| H2-104 | O | O | N | B31 |
| H2-105 | O | O | N | B32 |
| H2-106 | O | O | N | B68 |
| H2-107 | O | O | N | B69 |
| H2-108 | O | O | N | B70 |
| H2-109 | O | O | N | B71 |
| H2-110 | O | O | N | B81 |
| H2-111 | O | O | N | B90 |
| H2-112 | O | O | N | B91 |
| H2-113 | O | O | N | B92 |
| H2-114 | O | O | N | B93 |
| H2-115 | O | O | N | B107 |
| H2-116 | O | S | N | B1 |
| H2-117 | O | S | N | B5 |
| H2-118 | O | S | N | B6 |
| H2-119 | O | S | N | B11 |
| H2-120 | O | S | N | B18 |
| H2-121 | O | S | N | B20 |
| H2-122 | O | S | N | B21 |
| H2-123 | O | S | N | B22 |
| H2-124 | O | S | N | B23 |
| H2-125 | O | S | N | B24 |
| H2-126 | O | S | N | B25 |
| H2-127 | O | S | N | B31 |
| H2-128 | O | S | N | B32 |
| H2-129 | O | S | N | B68 |
| H2-130 | O | S | N | B69 |
| H2-131 | O | S | N | B70 |
| H2-132 | O | S | N | B71 |
| H2-133 | O | S | N | B81 |
| H2-134 | O | S | N | B90 |
| H2-135 | O | S | N | B91 |
| H2-136 | O | S | N | B92 |
| H2-137 | O | S | N | B93 |
| H2-138 | O | S | N | B107 |
wobei Verbindung H3-1 bis Verbindung H3-92 jeweils eine durch die Formel 2-3-1 dargestellte Struktur haben: 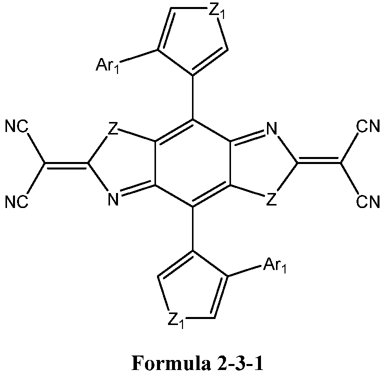
in Formel 2-3-1 zwei Z gleich sind, zwei Z₁ gleich sind, zwei Ar₁ gleich sind und Z, Z₁ und Ar₁ jeweils aus den Atomen oder Gruppen in der folgenden Tabelle ausgewählt sind:
| Verbindung Nr. | Z | Z₁ | Ar₁ |
|---|---|---|---|
| H3-1 | O | O | B1 |
| H3-2 | O | O | B5 |
| H3-3 | O | O | B6 |
| H3-4 | O | O | B11 |
| H3-5 | O | O | B18 |
| H3-6 | O | O | B20 |
| H3-7 | O | O | B21 |
| H3-8 | O | O | B22 |
| H3-9 | O | O | B23 |
| H3-10 | O | O | B24 |
| H3-11 | O | O | B25 |
| H3-12 | O | O | B31 |
| H3-13 | O | O | B32 |
| H3-14 | O | O | B68 |
| H3-15 | O | O | B69 |
| H3-16 | O | O | B70 |
| H3-17 | O | O | B71 |
| H3-18 | O | O | B81 |
| H3-19 | O | O | B90 |
| H3-20 | O | O | B91 |
| H3-21 | O | O | B92 |
| H3-22 | O | O | B93 |
| H3-23 | O | O | B107 |
| H3-24 | S | O | B1 |
| H3-25 | S | O | B5 |
| H3-26 | S | O | B6 |
| H3-27 | S | O | B11 |
| H3-28 | S | O | B18 |
| H3-29 | S | O | B20 |
| H3-30 | S | O | B21 |
| H3-31 | S | O | B22 |
| H3-32 | S | O | B23 |
| H3-33 | S | O | B24 |
| H3-34 | S | O | B25 |
| H3-35 | S | O | B31 |
| H3-36 | S | O | B32 |
| H3-37 | S | O | B68 |
| H3-38 | S | O | B69 |
| H3-39 | S | O | B70 |
| H3-40 | S | O | B71 |
| H3-41 | S | O | B81 |
| H3-42 | S | O | B90 |
| H3-43 | S | O | B91 |
| H3-44 | S | O | B92 |
| H3-45 | S | O | B93 |
| H3-46 | S | O | B107 |
| H3-47 | Se | O | B1 |
| H3-48 | Se | O | B5 |
| H3-49 | Se | O | B6 |
| H3-50 | Se | O | B11 |
| H3-51 | Se | O | B18 |
| H3-52 | Se | O | B20 |
| H3-53 | Se | O | B21 |
| H3-54 | Se | O | B22 |
| H3-55 | Se | O | B23 |
| H3-56 | Se | O | B24 |
| H3-57 | Se | O | B25 |
| H3-58 | Se | O | B31 |
| H3-59 | Se | O | B32 |
| H3-60 | Se | O | B68 |
| H3-61 | Se | O | B69 |
| H3-62 | Se | O | B70 |
| H3-63 | Se | O | B71 |
| H3-64 | Se | O | B81 |
| H3-65 | Se | O | B90 |
| H3-66 | Se | O | B91 |
| H3-67 | Se | O | B92 |
| H3-68 | Se | O | B93 |
| H3-69 | Se | O | B107 |
| H3-70 | O | S | B1 |
| H3-71 | O | S | B5 |
| H3-72 | O | S | B6 |
| H3-73 | O | S | B11 |
| H3-74 | O | S | B18 |
| H3-75 | O | S | B20 |
| H3-76 | O | S | B21 |
| H3-77 | O | S | B22 |
| H3-78 | O | S | B23 |
| H3-79 | O | S | B24 |
| H3-80 | O | S | B25 |
| H3-81 | O | S | B31 |
| H3-82 | O | S | B32 |
| H3-83 | O | S | B68 |
| H3-84 | O | S | B69 |
| H3-85 | O | S | B70 |
| H3-86 | O | S | B71 |
| H3-87 | O | S | B81 |
| H3-88 | O | S | B90 |
| H3-89 | O | S | B91 |
| H3-90 | O | S | B92 |
| H3-91 | O | S | B93 |
| H3-92 | O | S | B107 |
wobei Verbindung H4-1 bis Verbindung H4-92 jeweils eine durch die Formel 2-4-1 dargestellte Struktur haben: 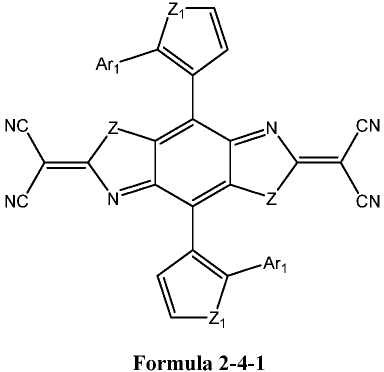
in Formel 2-4-1 zwei Z gleich sind, zwei Z₁ gleich sind, zwei Ar₁ gleich sind, und Z, Z₁ und Ar₁ jeweils aus den Atomen oder Gruppen in der folgenden Tabelle ausgewählt sind:
| Verbindung Nr. | Z | Z₁ | Ar₁ |
|---|---|---|---|
| H4-1 | O | O | B1 |
| H4-2 | O | O | B5 |
| H4-3 | O | O | B6 |
| H4-4 | O | O | B11 |
| H4-5 | O | O | B18 |
| H4-6 | O | O | B20 |
| H4-7 | O | O | B21 |
| H4-8 | O | O | B22 |
| H4-9 | O | O | B23 |
| H4-10 | O | O | B24 |
| H4-11 | O | O | B25 |
| H4-12 | O | O | B31 |
| H4-13 | O | O | B32 |
| H4-14 | O | O | B68 |
| H4-15 | O | O | B69 |
| H4-16 | O | O | B70 |
| H4-17 | O | O | B71 |
| H4-18 | O | O | B81 |
| H4-19 | O | O | B90 |
| H4-20 | O | O | B91 |
| H4-21 | O | O | B92 |
| H4-22 | O | O | B93 |
| H4-23 | O | O | B107 |
| H4-24 | S | O | B1 |
| H4-25 | S | O | B5 |
| H4-26 | S | O | B6 |
| H4-27 | S | O | B11 |
| H4-28 | S | O | B18 |
| H4-29 | S | O | B20 |
| H4-30 | S | O | B21 |
| H4-31 | S | O | B22 |
| H4-32 | S | O | B23 |
| H4-33 | S | O | B24 |
| H4-34 | S | O | B25 |
| H4-35 | S | O | B31 |
| H4-36 | S | O | B32 |
| H4-37 | S | O | B68 |
| H4-38 | S | O | B69 |
| H4-39 | S | O | B70 |
| H4-40 | S | O | B71 |
| H4-41 | S | O | B81 |
| H4-42 | S | O | B90 |
| H4-43 | S | O | B91 |
| H4-44 | S | O | B92 |
| H4-45 | S | O | B93 |
| H4-46 | S | O | B107 |
| H4-47 | Se | O | B1 |
| H4-48 | Se | O | B5 |
| H4-49 | Se | O | B6 |
| H4-50 | Se | O | B11 |
| H4-51 | Se | O | B18 |
| H4-52 | Se | O | B20 |
| H4-53 | Se | O | B21 |
| H4-54 | Se | O | B22 |
| H4-55 | Se | O | B23 |
| H4-56 | Se | O | B24 |
| H4-57 | Se | O | B25 |
| H4-58 | Se | O | B31 |
| H4-59 | Se | O | B32 |
| H4-60 | Se | O | B68 |
| H4-61 | Se | O | B69 |
| H4-62 | Se | O | B70 |
| H4-63 | Se | O | B71 |
| H4-64 | Se | O | B81 |
| H4-65 | Se | O | B90 |
| H4-66 | Se | O | B91 |
| H4-67 | Se | O | B92 |
| H4-68 | Se | O | B93 |
| H4-69 | Se | O | B107 |
| H4-70 | O | S | B1 |
| H4-71 | O | S | B5 |
| H4-72 | O | S | B6 |
| H4-73 | O | S | B11 |
| H4-74 | O | S | B18 |
| H4-75 | O | S | B20 |
| H4-76 | O | S | B21 |
| H4-77 | O | S | B22 |
| H4-78 | O | S | B23 |
| H4-79 | O | S | B24 |
| H4-80 | O | S | B25 |
| H4-81 | O | S | B31 |
| H4-82 | O | S | B32 |
| H4-83 | O | S | B68 |
| H4-84 | O | S | B69 |
| H4-85 | O | S | B70 |
| H4-86 | O | S | B71 |
| H4-87 | O | S | B81 |
| H4-88 | O | S | B90 |
| H4-89 | O | S | B91 |
| H4-90 | O | S | B92 |
| H4-91 | O | S | B93 |
| H4-92 | O | S | B107 |
wobei Verbindung H5-1 bis Verbindung H5-115 jeweils eine durch die Formel 2-5 dargestellte Struktur haben: 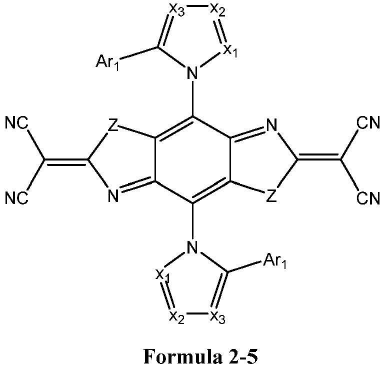
in Formel 2-5 zwei Z gleich sind, zwei X₁ gleich sind, zwei X₂ gleich sind, zwei X₃ gleich sind, zwei Ar₁ gleich sind, und Z, X₁, X₂, X₃ und An jeweils aus den Atomen oder Gruppen in der folgenden Tabelle ausgewählt sind:
| Verbindung Nr. | Z | X₁ | X₂ | X3 | Ar₁ |
|---|---|---|---|---|---|
| H5-1 | O | C-A1 | C-A1 | C-A1 | B1 |
| H5-2 | O | C-A1 | C-A1 | C-A1 | B5 |
| H5-3 | O | C-A1 | C-A1 | C-A1 | B6 |
| H5-4 | O | C-A1 | C-A1 | C-A1 | B11 |
| H5-5 | O | C-A1 | C-A1 | C-A1 | B18 |
| H5-6 | O | C-A1 | C-A1 | C-A1 | B20 |
| H5-7 | O | C-A1 | C-A1 | C-A1 | B21 |
| H5-8 | O | C-A1 | C-A1 | C-A1 | B22 |
| H5-9 | O | C-A1 | C-A1 | C-A1 | B23 |
| H5-10 | O | C-A1 | C-A1 | C-A1 | B24 |
| H5-11 | O | C-A1 | C-A1 | C-A1 | B25 |
| H5-12 | O | C-A1 | C-A1 | C-A1 | B31 |
| H5-13 | O | C-A1 | C-A1 | C-A1 | B32 |
| H5-14 | O | C-A1 | C-A1 | C-A1 | B68 |
| H5-15 | O | C-A1 | C-A1 | C-A1 | B69 |
| H5-16 | O | C-A1 | C-A1 | C-A1 | B70 |
| H5-17 | O | C-A1 | C-A1 | C-A1 | B71 |
| H5-18 | O | C-A1 | C-A1 | C-A1 | B81 |
| H5-19 | O | C-A1 | C-A1 | C-A1 | B90 |
| H5-20 | O | C-A1 | C-A1 | C-A1 | B91 |
| H5-21 | O | C-A1 | C-A1 | C-A1 | B92 |
| H5-22 | O | C-A1 | C-A1 | C-A1 | B93 |
| H5-23 | O | C-A1 | C-A1 | C-A1 | B107 |
| H5-24 | S | C-A1 | C-A1 | C-A1 | B1 |
| H5-25 | S | C-A1 | C-A1 | C-A1 | B5 |
| H5-26 | S | C-A1 | C-A1 | C-A1 | B6 |
| H5-27 | S | C-A1 | C-A1 | C-A1 | B11 |
| H5-28 | S | C-A1 | C-A1 | C-A1 | B18 |
| H5-29 | S | C-A1 | C-A1 | C-A1 | B20 |
| H5-30 | S | C-A1 | C-A1 | C-A1 | B21 |
| H5-31 | S | C-A1 | C-A1 | C-A1 | B22 |
| H5-32 | S | C-A1 | C-A1 | C-A1 | B23 |
| H5-33 | S | C-A1 | C-A1 | C-A1 | B24 |
| H5-34 | S | C-A1 | C-A1 | C-A1 | B25 |
| H5-35 | S | C-A1 | C-A1 | C-A1 | B31 |
| H5-36 | S | C-A1 | C-A1 | C-A1 | B32 |
| H5-37 | S | C-A1 | C-A1 | C-A1 | B68 |
| H5-38 | S | C-A1 | C-A1 | C-A1 | B69 |
| H5-39 | S | C-A1 | C-A1 | C-A1 | B70 |
| H5-40 | S | C-A1 | C-A1 | C-A1 | B71 |
| H5-41 | S | C-A1 | C-A1 | C-A1 | B81 |
| H5-42 | S | C-A1 | C-A1 | C-A1 | B90 |
| H5-43 | S | C-A1 | C-A1 | C-A1 | B91 |
| H5-44 | S | C-A1 | C-A1 | C-A1 | B92 |
| H5-45 | S | C-A1 | C-A1 | C-A1 | B93 |
| H5-46 | S | C-A1 | C-A1 | C-A1 | B107 |
| H5-47 | Se | C-A1 | C-A1 | C-A1 | B1 |
| H5-48 | Se | C-A1 | C-A1 | C-A1 | B5 |
| H5-49 | Se | C-A1 | C-A1 | C-A1 | B6 |
| H5-50 | Se | C-A1 | C-A1 | C-A1 | B11 |
| H5-51 | Se | C-A1 | C-A1 | C-A1 | B18 |
| H5-52 | Se | C-A1 | C-A1 | C-A1 | B20 |
| H5-53 | Se | C-A1 | C-A1 | C-A1 | B21 |
| H5-54 | Se | C-A1 | C-A1 | C-A1 | B22 |
| H5-55 | Se | C-A1 | C-A1 | C-A1 | B23 |
| H5-56 | Se | C-A1 | C-A1 | C-A1 | B24 |
| H5-57 | Se | C-A1 | C-A1 | C-A1 | B25 |
| H5-58 | Se | C-A1 | C-A1 | C-A1 | B31 |
| H5-59 | Se | C-A1 | C-A1 | C-A1 | B32 |
| H5-60 | Se | C-A1 | C-A1 | C-A1 | B68 |
| H5-61 | Se | C-A1 | C-A1 | C-A1 | B69 |
| H5-62 | Se | C-A1 | C-A1 | C-A1 | B70 |
| H5-63 | Se | C-A1 | C-A1 | C-A1 | B71 |
| H5-64 | Se | C-A1 | C-A1 | C-A1 | B81 |
| H5-65 | Se | C-A1 | C-A1 | C-A1 | B90 |
| H5-66 | Se | C-A1 | C-A1 | C-A1 | B91 |
| H5-67 | Se | C-A1 | C-A1 | C-A1 | B92 |
| H5-68 | Se | C-A1 | C-A1 | C-A1 | B93 |
| H5-69 | Se | C-A1 | C-A1 | C-A1 | B107 |
| H5-70 | O | C-A1 | C-A1 | N | B1 |
| H5-71 | O | C-A1 | C-A1 | N | B5 |
| H5-72 | O | C-A1 | C-A1 | N | B6 |
| H5-73 | O | C-A1 | C-A1 | N | B11 |
| H5-74 | O | C-A1 | C-A1 | N | B18 |
| H5-75 | O | C-A1 | C-A1 | N | B20 |
| H5-76 | O | C-A1 | C-A1 | N | B21 |
| H5-77 | O | C-A1 | C-A1 | N | B22 |
| H5-78 | O | C-A1 | C-A1 | N | B23 |
| H5-79 | O | C-A1 | C-A1 | N | B24 |
| H5-80 | O | C-A1 | C-A1 | N | B25 |
| H5-81 | O | C-A1 | C-A1 | N | B31 |
| H5-82 | O | C-A1 | C-A1 | N | B32 |
| H5-83 | O | C-A1 | C-A1 | N | B68 |
| H5-84 | O | C-A1 | C-A1 | N | B69 |
| H5-85 | O | C-A1 | C-A1 | N | B70 |
| H5-86 | O | C-A1 | C-A1 | N | B71 |
| H5-87 | O | C-A1 | C-A1 | N | B81 |
| H5-88 | O | C-A1 | C-A1 | N | B90 |
| H5-89 | O | C-A1 | C-A1 | N | B91 |
| H5-90 | O | C-A1 | C-A1 | N | B92 |
| H5-91 | O | C-A1 | C-A1 | N | B93 |
| H5-92 | O | C-A1 | C-A1 | N | B107 |
| H5-93 | O | C-A1 | N | C-A1 | B1 |
| H5-94 | O | C-A1 | N | C-A1 | B5 |
| H5-95 | O | C-A1 | N | C-A1 | B6 |
| H5-96 | O | C-A1 | N | C-A1 | B11 |
| H5-97 | O | C-A1 | N | C-A1 | B18 |
| H5-98 | O | C-A1 | N | C-A1 | B20 |
| H5-99 | O | C-A1 | N | C-A1 | B21 |
| H5-100 | O | C-A1 | N | C-A1 | B22 |
| H5-101 | O | C-A1 | N | C-A1 | B23 |
| H5-102 | O | C-A1 | N | C-A1 | B24 |
| H5-103 | O | C-A1 | N | C-A1 | B25 |
| H5-104 | O | C-A1 | N | C-A1 | B31 |
| H5-105 | O | C-A1 | N | C-A1 | B32 |
| H5-106 | O | C-A1 | N | C-A1 | B68 |
| H5-107 | O | C-A1 | N | C-A1 | B69 |
| H5-108 | O | C-A1 | N | C-A1 | B70 |
| H5-109 | O | C-A1 | N | C-A1 | B71 |
| H5-110 | O | C-A1 | N | C-A1 | B81 |
| H5-111 | O | C-A1 | N | C-A1 | B90 |
| H5-112 | O | C-A1 | N | C-A1 | B91 |
| H5-113 | O | C-A1 | N | C-A1 | B92 |
| H5-114 | O | C-A1 | N | C-A1 | B93 |
| H5-115 | O | C-A1 | N | C-A1 | B107 |
wobei Verbindung H6-1 bis Verbindung H6-115 jeweils eine durch die Formel 2-6 dargestellte Struktur haben: 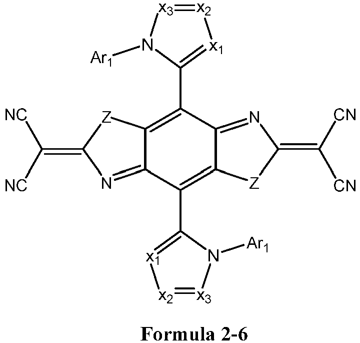
in Formel 2-6 zwei Z gleich sind, zwei X₁ gleich sind, zwei X₂ gleich sind, zwei X₃ gleich sind, zwei Ar₁ gleich sind, und Z, X₁, X₂, X₃ und Ar₁ jeweils aus den Atomen oder Gruppen in der folgenden Tabelle ausgewählt sind:
| Verbindung Nr. | Z | X₁ | X₂ | X₃ | Ar₁ |
|---|---|---|---|---|---|
| H6-1 | O | C-A1 | C-A1 | C-A1 | B1 |
| H6-2 | O | C-A1 | C-A1 | C-A1 | B5 |
| H6-3 | O | C-A1 | C-A1 | C-A1 | B6 |
| H6-4 | O | C-A1 | C-A1 | C-A1 | B11 |
| H6-5 | O | C-A1 | C-A1 | C-A1 | B18 |
| H6-6 | O | C-A1 | C-A1 | C-A1 | B20 |
| H6-7 | O | C-A1 | C-A1 | C-A1 | B21 |
| H6-8 | O | C-A1 | C-A1 | C-A1 | B22 |
| H6-9 | O | C-A1 | C-A1 | C-A1 | B23 |
| H6-10 | O | C-A1 | C-A1 | C-A1 | B24 |
| H6-11 | O | C-A1 | C-A1 | C-A1 | B25 |
| H6-12 | O | C-A1 | C-A1 | C-A1 | B31 |
| H6-13 | O | C-A1 | C-A1 | C-A1 | B32 |
| H6-14 | O | C-A1 | C-A1 | C-A1 | B68 |
| H6-15 | O | C-A1 | C-A1 | C-A1 | B69 |
| H6-16 | O | C-A1 | C-A1 | C-A1 | B70 |
| H6-17 | O | C-A1 | C-A1 | C-A1 | B71 |
| H6-18 | O | C-A1 | C-A1 | C-A1 | B81 |
| H6-19 | O | C-A1 | C-A1 | C-A1 | B90 |
| H6-20 | O | C-A1 | C-A1 | C-A1 | B91 |
| H6-21 | O | C-A1 | C-A1 | C-A1 | B92 |
| H6-22 | O | C-A1 | C-A1 | C-A1 | B93 |
| H6-23 | O | C-A1 | C-A1 | C-A1 | B107 |
| H6-24 | S | C-A1 | C-A1 | C-A1 | B1 |
| H6-25 | S | C-A1 | C-A1 | C-A1 | B5 |
| H6-26 | S | C-A1 | C-A1 | C-A1 | B6 |
| H6-27 | S | C-A1 | C-A1 | C-A1 | B11 |
| H6-28 | S | C-A1 | C-A1 | C-A1 | B18 |
| H6-29 | S | C-A1 | C-A1 | C-A1 | B20 |
| H6-30 | S | C-A1 | C-A1 | C-A1 | B21 |
| H6-31 | S | C-A1 | C-A1 | C-A1 | B22 |
| H6-32 | S | C-A1 | C-A1 | C-A1 | B23 |
| H6-33 | S | C-A1 | C-A1 | C-A1 | B24 |
| H6-34 | S | C-A1 | C-A1 | C-A1 | B25 |
| H6-35 | S | C-A1 | C-A1 | C-A1 | B31 |
| H6-36 | S | C-A1 | C-A1 | C-A1 | B32 |
| H6-37 | S | C-A1 | C-A1 | C-A1 | B68 |
| H6-38 | S | C-A1 | C-A1 | C-A1 | B69 |
| H6-39 | S | C-A1 | C-A1 | C-A1 | B70 |
| H6-40 | S | C-A1 | C-A1 | C-A1 | B71 |
| H6-41 | S | C-A1 | C-A1 | C-A1 | B81 |
| H6-42 | S | C-A1 | C-A1 | C-A1 | B90 |
| H6-43 | S | C-A1 | C-A1 | C-A1 | B91 |
| H6-44 | S | C-A1 | C-A1 | C-A1 | B92 |
| H6-45 | S | C-A1 | C-A1 | C-A1 | B93 |
| H6-46 | S | C-A1 | C-A1 | C-A1 | B107 |
| H6-47 | Se | C-A1 | C-A1 | C-A1 | B1 |
| H6-48 | Se | C-A1 | C-A1 | C-A1 | B5 |
| H6-49 | Se | C-A1 | C-A1 | C-A1 | B6 |
| H6-50 | Se | C-A1 | C-A1 | C-A1 | B11 |
| H6-51 | Se | C-A1 | C-A1 | C-A1 | B18 |
| H6-52 | Se | C-A1 | C-A1 | C-A1 | B20 |
| H6-53 | Se | C-A1 | C-A1 | C-A1 | B21 |
| H6-54 | Se | C-A1 | C-A1 | C-A1 | B22 |
| H6-55 | Se | C-A1 | C-A1 | C-A1 | B23 |
| H6-56 | Se | C-A1 | C-A1 | C-A1 | B24 |
| H6-57 | Se | C-A1 | C-A1 | C-A1 | B25 |
| H6-58 | Se | C-A1 | C-A1 | C-A1 | B31 |
| H6-59 | Se | C-A1 | C-A1 | C-A1 | B32 |
| H6-60 | Se | C-A1 | C-A1 | C-A1 | B68 |
| H6-61 | Se | C-A1 | C-A1 | C-A1 | B69 |
| H6-62 | Se | C-A1 | C-A1 | C-A1 | B70 |
| H6-63 | Se | C-A1 | C-A1 | C-A1 | B71 |
| H6-64 | Se | C-A1 | C-A1 | C-A1 | B81 |
| H6-65 | Se | C-A1 | C-A1 | C-A1 | B90 |
| H6-66 | Se | C-A1 | C-A1 | C-A1 | B91 |
| H6-67 | Se | C-A1 | C-A1 | C-A1 | B92 |
| H6-68 | Se | C-A1 | C-A1 | C-A1 | B93 |
| H6-69 | Se | C-A1 | C-A1 | C-A1 | B107 |
| H6-70 | O | N | C-A1 | C-A1 | B1 |
| H6-71 | O | N | C-A1 | C-A1 | B5 |
| H6-72 | O | N | C-A1 | C-A1 | B6 |
| H6-73 | O | N | C-A1 | C-A1 | B11 |
| H6-74 | O | N | C-A1 | C-A1 | B18 |
| H6-75 | O | N | C-A1 | C-A1 | B20 |
| H6-76 | O | N | C-A1 | C-A1 | B21 |
| H6-77 | O | N | C-A1 | C-A1 | B22 |
| H6-78 | O | N | C-A1 | C-A1 | B23 |
| H6-79 | O | N | C-A1 | C-A1 | B24 |
| H6-80 | O | N | C-A1 | C-A1 | B25 |
| H6-81 | O | N | C-A1 | C-A1 | B31 |
| H6-82 | O | N | C-A1 | C-A1 | B32 |
| H6-83 | O | N | C-A1 | C-A1 | B68 |
| H6-84 | O | N | C-A1 | C-A1 | B69 |
| H6-85 | O | N | C-A1 | C-A1 | B70 |
| H6-86 | O | N | C-A1 | C-A1 | B71 |
| H6-87 | O | N | C-A1 | C-A1 | B81 |
| H6-88 | O | N | C-A1 | C-A1 | B90 |
| H6-89 | O | N | C-A1 | C-A1 | B91 |
| H6-90 | O | N | C-A1 | C-A1 | B92 |
| H6-91 | O | N | C-A1 | C-A1 | B93 |
| H6-92 | O | N | C-A1 | C-A1 | B107 |
| H6-93 | O | C-A1 | N | C-A1 | B1 |
| H6-94 | O | C-A1 | N | C-A1 | B5 |
| H6-95 | O | C-A1 | N | C-A1 | B6 |
| H6-96 | O | C-A1 | N | C-A1 | B11 |
| H6-97 | O | C-A1 | N | C-A1 | B18 |
| H6-98 | O | C-A1 | N | C-A1 | B20 |
| H6-99 | O | C-A1 | N | C-A1 | B21 |
| H6-100 | O | C-A1 | N | C-A1 | B22 |
| H6-101 | O | C-A1 | N | C-A1 | B23 |
| H6-102 | O | C-A1 | N | C-A1 | B24 |
| H6-103 | O | C-A1 | N | C-A1 | B25 |
| H6-104 | O | C-A1 | N | C-A1 | B31 |
| H6-105 | O | C-A1 | N | C-A1 | B32 |
| H6-106 | O | C-A1 | N | C-A1 | B68 |
| H6-107 | O | C-A1 | N | C-A1 | B69 |
| H6-108 | O | C-A1 | N | C-A1 | B70 |
| H6-109 | O | C-A1 | N | C-A1 | B71 |
| H6-110 | O | C-A1 | N | C-A1 | B81 |
| H6-111 | O | C-A1 | N | C-A1 | B90 |
| H6-112 | O | C-A1 | N | C-A1 | B91 |
| H6-113 | O | C-A1 | N | C-A1 | B92 |
| H6-114 | O | C-A1 | N | C-A1 | B93 |
| H6-115 | O | C-A1 | N | C-A1 | B107 |
wobei Verbindung H7-1 bis Verbindung H7-203 jeweils eine durch die Formel 3-1 dargestellte Struktur haben: 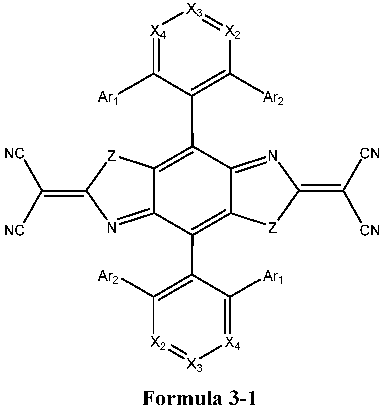
in Formel 3-1 zwei Z gleich sind, zwei X₂ gleich sind, zwei X₃ gleich sind, zwei X₄ gleich sind, zwei Ar₁ gleich sind, zwei Ar₂ gleich sind, und Z, X₂, X₃, X₄, Ar₁ und Ar₂ jeweils aus den Atomen oder Gruppen in der folgenden Tabelle ausgewählt sind:
| Verbindung Nr. | Z | X₂ | X₃ | X₄ | Ar₁ | Ar₂ |
|---|---|---|---|---|---|---|
| H7-1 | O | C-A1 | C-A1 | C-A1 | B1 | B1 |
| H7-2 | O | C-A1 | C-A1 | C-A1 | B5 | B5 |
| H7-3 | O | C-A1 | C-A1 | C-A1 | B6 | B6 |
| H7-4 | O | C-A1 | C-A1 | C-A1 | B11 | B11 |
| H7-5 | O | C-A1 | C-A1 | C-A1 | B18 | B18 |
| H7-6 | O | C-A1 | C-A1 | C-A1 | B20 | B20 |
| H7-7 | O | C-A1 | C-A1 | C-A1 | B21 | B21 |
| H7-8 | O | C-A1 | C-A1 | C-A1 | B22 | B22 |
| H7-9 | O | C-A1 | C-A1 | C-A1 | B23 | B23 |
| H7-10 | O | C-A1 | C-A1 | C-A1 | B24 | B24 |
| H7-11 | O | C-A1 | C-A1 | C-A1 | B25 | B25 |
| H7-12 | O | C-A1 | C-A1 | C-A1 | B31 | B31 |
| H7-13 | O | C-A1 | C-A1 | C-A1 | B32 | B32 |
| H7-14 | O | C-A1 | C-A1 | C-A1 | B68 | B68 |
| H7-15 | O | C-A1 | C-A1 | C-A1 | B69 | B69 |
| H7-16 | O | C-A1 | C-A1 | C-A1 | B70 | B70 |
| H7-17 | O | C-A1 | C-A1 | C-A1 | B71 | B71 |
| H7-18 | O | C-A1 | C-A1 | C-A1 | B81 | B81 |
| H7-19 | O | C-A1 | C-A1 | C-A1 | B90 | B90 |
| H7-20 | O | C-A1 | C-A1 | C-A1 | B91 | B91 |
| H7-21 | O | C-A1 | C-A1 | C-A1 | B92 | B92 |
| H7-22 | O | C-A1 | C-A1 | C-A1 | B93 | B93 |
| H7-23 | O | C-A1 | C-A1 | C-A1 | B107 | B107 |
| H7-24 | O | C-A1 | C-A3 | C-A1 | B1 | B1 |
| H7-25 | O | C-A1 | C-A3 | C-A1 | B5 | B5 |
| H7-26 | O | C-A1 | C-A3 | C-A1 | B6 | B6 |
| H7-27 | O | C-A1 | C-A3 | C-A1 | B11 | B11 |
| H7-28 | O | C-A1 | C-A3 | C-A1 | B18 | B18 |
| H7-29 | O | C-A1 | C-A3 | C-A1 | B20 | B20 |
| H7-30 | O | C-A1 | C-A3 | C-A1 | B21 | B21 |
| H7-31 | O | C-A1 | C-A3 | C-A1 | B22 | B22 |
| H7-32 | O | C-A1 | C-A3 | C-A1 | B23 | B23 |
| H7-33 | O | C-A1 | C-A3 | C-A1 | B24 | B24 |
| H7-34 | O | C-A1 | C-A3 | C-A1 | B25 | B25 |
| H7-35 | O | C-A1 | C-A3 | C-A1 | B31 | B31 |
| H7-36 | O | C-A1 | C-A3 | C-A1 | B32 | B32 |
| H7-37 | O | C-A1 | C-A3 | C-A1 | B68 | B68 |
| H7-38 | O | C-A1 | C-A3 | C-A1 | B69 | B69 |
| H7-39 | O | C-A1 | C-A3 | C-A1 | B70 | B70 |
| H7-40 | O | C-A1 | C-A3 | C-A1 | B71 | B71 |
| H7-41 | O | C-A1 | C-A3 | C-A1 | B81 | B81 |
| H7-42 | O | C-A1 | C-A3 | C-A1 | B90 | B90 |
| H7-43 | O | C-A1 | C-A3 | C-A1 | B91 | B91 |
| H7-44 | O | C-A1 | C-A3 | C-A1 | B92 | B92 |
| H7-45 | O | C-A1 | C-A3 | C-A1 | B93 | B93 |
| H7-46 | O | C-A1 | C-A3 | C-A1 | B107 | B107 |
| H7-47 | O | C-A1 | C-A9 | C-A1 | B1 | B1 |
| H7-48 | O | C-A1 | C-A9 | C-A1 | B5 | B5 |
| H7-49 | O | C-A1 | C-A9 | C-A1 | B6 | B6 |
| H7-50 | O | C-A1 | C-A9 | C-A1 | B11 | B11 |
| H7-51 | O | C-A1 | C-A9 | C-A1 | B18 | B18 |
| H7-52 | O | C-A1 | C-A9 | C-A1 | B20 | B20 |
| H7-53 | O | C-A1 | C-A9 | C-A1 | B21 | B21 |
| H7-54 | O | C-A1 | C-A9 | C-A1 | B22 | B22 |
| H7-55 | O | C-A1 | C-A9 | C-A1 | B23 | B23 |
| H7-56 | O | C-A1 | C-A9 | C-A1 | B24 | B24 |
| H7-57 | O | C-A1 | C-A9 | C-A1 | B25 | B25 |
| H7-58 | O | C-A1 | C-A9 | C-A1 | B31 | B31 |
| H7-59 | O | C-A1 | C-A9 | C-A1 | B32 | B32 |
| H7-60 | O | C-A1 | C-A9 | C-A1 | B68 | B68 |
| H7-61 | O | C-A1 | C-A9 | C-A1 | B69 | B69 |
| H7-62 | O | C-A1 | C-A9 | C-A1 | B70 | B70 |
| H7-63 | O | C-A1 | C-A9 | C-A1 | B71 | B71 |
| H7-64 | O | C-A1 | C-A9 | C-A1 | B81 | B81 |
| H7-65 | O | C-A1 | C-A9 | C-A1 | B90 | B90 |
| H7-66 | O | C-A1 | C-A9 | C-A1 | B91 | B91 |
| H7-67 | O | C-A1 | C-A9 | C-A1 | B92 | B92 |
| H7-68 | O | C-A1 | C-A9 | C-A1 | B93 | B93 |
| H7-69 | O | C-A1 | C-A9 | C-A1 | B107 | B107 |
| H7-70 | S | C-A1 | C-A1 | C-A1 | B1 | B1 |
| H7-71 | S | C-A1 | C-A1 | C-A1 | B5 | B5 |
| H7-72 | S | C-A1 | C-A1 | C-A1 | B6 | B6 |
| H7-73 | S | C-A1 | C-A1 | C-A1 | B11 | B11 |
| H7-74 | S | C-A1 | C-A1 | C-A1 | B18 | B18 |
| H7-75 | S | C-A1 | C-A1 | C-A1 | B20 | B20 |
| H7-76 | S | C-A1 | C-A1 | C-A1 | B21 | B21 |
| H7-77 | S | C-A1 | C-A1 | C-A1 | B22 | B22 |
| H7-78 | S | C-A1 | C-A1 | C-A1 | B23 | B23 |
| H7-79 | S | C-A1 | C-A1 | C-A1 | B24 | B24 |
| H7-80 | S | C-A1 | C-A1 | C-A1 | B25 | B25 |
| H7-81 | S | C-A1 | C-A1 | C-A1 | B31 | B31 |
| H7-82 | S | C-A1 | C-A1 | C-A1 | B32 | B32 |
| H7-83 | S | C-A1 | C-A1 | C-A1 | B68 | B68 |
| H7-84 | S | C-A1 | C-A1 | C-A1 | B69 | B69 |
| H7-85 | S | C-A1 | C-A1 | C-A1 | B70 | B70 |
| H7-86 | S | C-A1 | C-A1 | C-A1 | B71 | B71 |
| H7-87 | S | C-A1 | C-A1 | C-A1 | B81 | B81 |
| H7-88 | S | C-A1 | C-A1 | C-A1 | B90 | B90 |
| H7-89 | S | C-A1 | C-A1 | C-A1 | B91 | B91 |
| H7-90 | S | C-A1 | C-A1 | C-A1 | B92 | B92 |
| H7-91 | S | C-A1 | C-A1 | C-A1 | B93 | B93 |
| H7-92 | S | C-A1 | C-A1 | C-A1 | B107 | B107 |
| H7-93 | Se | C-A1 | C-A1 | C-A1 | B1 | B1 |
| H7-94 | Se | C-A1 | C-A1 | C-A1 | B5 | B5 |
| H7-95 | Se | C-A1 | C-A1 | C-A1 | B6 | B6 |
| H7-96 | Se | C-A1 | C-A1 | C-A1 | B11 | B11 |
| H7-97 | Se | C-A1 | C-A1 | C-A1 | B18 | B18 |
| H7-98 | Se | C-A1 | C-A1 | C-A1 | B20 | B20 |
| H7-99 | Se | C-A1 | C-A1 | C-A1 | B21 | B21 |
| H7-100 | Se | C-A1 | C-A1 | C-A1 | B22 | B22 |
| H7-101 | Se | C-A1 | C-A1 | C-A1 | B23 | B23 |
| H7-102 | Se | C-A1 | C-A1 | C-A1 | B24 | B24 |
| H7-103 | Se | C-A1 | C-A1 | C-A1 | B25 | B25 |
| H7-104 | Se | C-A1 | C-A1 | C-A1 | B31 | B31 |
| H7-105 | Se | C-A1 | C-A1 | C-A1 | B32 | B32 |
| H7-106 | Se | C-A1 | C-A1 | C-A1 | B68 | B68 |
| H7-107 | Se | C-A1 | C-A1 | C-A1 | B69 | B69 |
| H7-108 | Se | C-A1 | C-A1 | C-A1 | B70 | B70 |
| H7-109 | Se | C-A1 | C-A1 | C-A1 | B71 | B71 |
| H7-110 | Se | C-A1 | C-A1 | C-A1 | B81 | B81 |
| H7-111 | Se | C-A1 | C-A1 | C-A1 | B90 | B90 |
| H7-112 | Se | C-A1 | C-A1 | C-A1 | B91 | B91 |
| H7-113 | Se | C-A1 | C-A1 | C-A1 | B92 | B92 |
| H7-114 | Se | C-A1 | C-A1 | C-A1 | B93 | B93 |
| H7-115 | Se | C-A1 | C-A1 | C-A1 | B107 | B107 |
| H7-116 | O | C-A1 | C-A1 | C-A1 | B1 | B5 |
| H7-117 | O | C-A1 | C-A1 | C-A1 | B1 | B6 |
| H7-118 | O | C-A1 | C-A1 | C-A1 | B1 | B11 |
| H7-119 | O | C-A1 | C-A1 | C-A1 | B1 | B18 |
| H7-120 | O | C-A1 | C-A1 | C-A1 | B1 | B20 |
| H7-121 | O | C-A1 | C-A1 | C-A1 | B1 | B21 |
| H7-122 | O | C-A1 | C-A1 | C-A1 | B1 | B22 |
| H7-123 | O | C-A1 | C-A1 | C-A1 | B1 | B23 |
| H7-124 | O | C-A1 | C-A1 | C-A1 | B1 | B24 |
| H7-125 | O | C-A1 | C-A1 | C-A1 | B1 | B25 |
| H7-126 | O | C-A1 | C-A1 | C-A1 | B1 | B31 |
| H7-127 | O | C-A1 | C-A1 | C-A1 | B1 | B32 |
| H7-128 | O | C-A1 | C-A1 | C-A1 | B1 | B68 |
| H7-129 | O | C-A1 | C-A1 | C-A1 | B1 | B69 |
| H7-130 | O | C-A1 | C-A1 | C-A1 | B1 | B70 |
| H7-131 | O | C-A1 | C-A1 | C-A1 | B1 | B71 |
| H7-132 | O | C-A1 | C-A1 | C-A1 | B1 | B81 |
| H7-133 | O | C-A1 | C-A1 | C-A1 | B1 | B90 |
| H7-134 | O | C-A1 | C-A1 | C-A1 | B1 | B91 |
| H7-135 | O | C-A1 | C-A1 | C-A1 | B1 | B92 |
| H7-136 | O | C-A1 | C-A1 | C-A1 | B1 | B93 |
| H7-137 | O | C-A1 | C-A1 | C-A1 | B1 | B107 |
| H7-138 | O | C-A1 | C-A1 | C-A1 | B20 | B1 |
| H7-139 | O | C-A1 | C-A1 | C-A1 | B20 | B5 |
| H7-140 | O | C-A1 | C-A1 | C-A1 | B20 | B6 |
| H7-141 | O | C-A1 | C-A1 | C-A1 | B20 | B11 |
| H7-142 | O | C-A1 | C-A1 | C-A1 | B20 | B18 |
| H7-143 | O | C-A1 | C-A1 | C-A1 | B20 | B21 |
| H7-144 | O | C-A1 | C-A1 | C-A1 | B20 | B22 |
| H7-145 | O | C-A1 | C-A1 | C-A1 | B20 | B23 |
| H7-146 | O | C-A1 | C-A1 | C-A1 | B20 | B24 |
| H7-147 | O | C-A1 | C-A1 | C-A1 | B20 | B25 |
| H7-148 | O | C-A1 | C-A1 | C-A1 | B20 | B31 |
| H7-149 | O | C-A1 | C-A1 | C-A1 | B20 | B32 |
| H7-150 | O | C-A1 | C-A1 | C-A1 | B20 | B68 |
| H7-151 | O | C-A1 | C-A1 | C-A1 | B20 | B69 |
| H7-152 | O | C-A1 | C-A1 | C-A1 | B20 | B70 |
| H7-153 | O | C-A1 | C-A1 | C-A1 | B20 | B71 |
| H7-154 | O | C-A1 | C-A1 | C-A1 | B20 | B81 |
| H7-155 | O | C-A1 | C-A1 | C-A1 | B20 | B90 |
| H7-156 | O | C-A1 | C-A1 | C-A1 | B20 | B91 |
| H7-157 | O | C-A1 | C-A1 | C-A1 | B20 | B92 |
| H7-158 | O | C-A1 | C-A1 | C-A1 | B20 | B93 |
| H7-159 | O | C-A1 | C-A1 | C-A1 | B20 | B107 |
| H7-160 | O | C-A1 | C-A1 | C-A1 | B68 | B1 |
| H7-161 | O | C-A1 | C-A1 | C-A1 | B68 | B5 |
| H7-162 | O | C-A1 | C-A1 | C-A1 | B68 | B6 |
| H7-163 | O | C-A1 | C-A1 | C-A1 | B68 | B11 |
| H7-164 | O | C-A1 | C-A1 | C-A1 | B68 | B18 |
| H7-165 | O | C-A1 | C-A1 | C-A1 | B68 | B20 |
| H7-166 | O | C-A1 | C-A1 | C-A1 | B68 | B21 |
| H7-167 | O | C-A1 | C-A1 | C-A1 | B68 | B22 |
| H7-168 | O | C-A1 | C-A1 | C-A1 | B68 | B23 |
| H7-169 | O | C-A1 | C-A1 | C-A1 | B68 | B24 |
| H7-170 | O | C-A1 | C-A1 | C-A1 | B68 | B25 |
| H7-171 | O | C-A1 | C-A1 | C-A1 | B68 | B31 |
| H7-172 | O | C-A1 | C-A1 | C-A1 | B68 | B32 |
| H7-173 | O | C-A1 | C-A1 | C-A1 | B68 | B69 |
| H7-174 | O | C-A1 | C-A1 | C-A1 | B68 | B70 |
| H7-175 | O | C-A1 | C-A1 | C-A1 | B68 | B71 |
| H7-176 | O | C-A1 | C-A1 | C-A1 | B68 | B81 |
| H7-177 | O | C-A1 | C-A1 | C-A1 | B68 | B90 |
| H7-178 | O | C-A1 | C-A1 | C-A1 | B68 | B91 |
| H7-179 | O | C-A1 | C-A1 | C-A1 | B68 | B92 |
| H7-180 | O | C-A1 | C-A1 | C-A1 | B68 | B93 |
| H7-181 | O | C-A1 | C-A1 | C-A1 | B68 | B107 |
| H7-182 | O | C-A1 | C-A1 | C-A1 | B70 | B1 |
| H7-183 | O | C-A1 | C-A1 | C-A1 | B70 | B5 |
| H7-184 | O | C-A1 | C-A1 | C-A1 | B70 | B6 |
| H7-185 | O | C-A1 | C-A1 | C-A1 | B70 | B11 |
| H7-186 | O | C-A1 | C-A1 | C-A1 | B70 | B18 |
| H7-187 | O | C-A1 | C-A1 | C-A1 | B70 | B20 |
| H7-188 | O | C-A1 | C-A1 | C-A1 | B70 | B21 |
| H7-189 | O | C-A1 | C-A1 | C-A1 | B70 | B22 |
| H7-190 | O | C-A1 | C-A1 | C-A1 | B70 | B23 |
| H7-191 | O | C-A1 | C-A1 | C-A1 | B70 | B24 |
| H7-192 | O | C-A1 | C-A1 | C-A1 | B70 | B25 |
| H7-193 | O | C-A1 | C-A1 | C-A1 | B70 | B31 |
| H7-194 | O | C-A1 | C-A1 | C-A1 | B70 | B32 |
| H7-195 | O | C-A1 | C-A1 | C-A1 | B70 | B68 |
| H7-196 | O | C-A1 | C-A1 | C-A1 | B70 | B69 |
| H7-197 | O | C-A1 | C-A1 | C-A1 | B70 | B71 |
| H7-198 | O | C-A1 | C-A1 | C-A1 | B70 | B81 |
| H7-199 | O | C-A1 | C-A1 | C-A1 | B70 | B90 |
| H7-200 | O | C-A1 | C-A1 | C-A1 | B70 | B91 |
| H7-201 | O | C-A1 | C-A1 | C-A1 | B70 | B92 |
| H7-202 | O | C-A1 | C-A1 | C-A1 | B70 | B93 |
| H7-203 | O | C-A1 | C-A1 | C-A1 | B70 | B107 |
wobei Verbindung H8-7 bis Verbindung H8-54, Verbindung H8-61 bis Verbindung H8-108, Verbindung H8-115 bis Verbindung H8-162, Verbindung H8-169 bis Verbindung H8-216, Verbindung H8-223 bis Verbindung H8-270, Verbindung H8-277 bis Verbindung H8-324, Verbindung H8-331 bis Verbindung H8-378, Verbindung H8-385 bis Verbindung H8-432, Verbindung H8-439 bis Verbindung H8-486, Verbindung H8-493 bis Verbindung H8-540, Verbindung H8-547 bis Verbindung H8-594, Verbindung H8-601 bis Verbindung H8-648, Verbindung H8-655 bis Verbindung H8-702, Verbindung H8-709 bis Verbindung H8-756, Verbindung H8-763 bis Verbindung H8-810, Verbindung H8-817 bis Verbindung H8-864, Verbindung H8-871 bis Verbindung H8-918, Verbindung H8-925 bis Verbindung H8-972, Verbindung H8-979 bis Verbindung H8-1026, Verbindung H8-1033 bis Verbindung H8-1080, Verbindung H8-1087 bis Verbindung H8-1134, Verbindung H8-1141 bis Verbindung H8-1188, Verbindung H8-1195 bis Verbindung H8-1242, Verbindung H8-1249 bis Verbindung H8-1296, Verbindung H8-1303 bis Verbindung H8-1350, Verbindung H8-1357 bis Verbindung H8-1404, Verbindung H8-1411 bis Verbindung H8-1458, Verbindung H8-1465 bis Verbindung H8-1512 jeweils eine durch Formel 3-2 dargestellte Struktur haben: 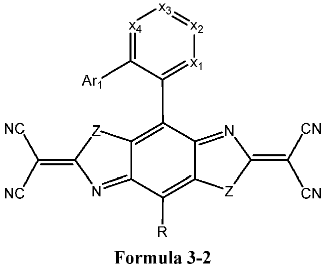
in Formel 3-2 zwei Z gleich sind und Z, X₁, X₂, X₃, X₄, Ar₁ und R jeweils aus den Atomen oder Gruppen in der folgenden Tabelle ausgewählt sind:
| Verbindung Nr. | Z | X₁ | X₂ | X₃ | X₄ | Ar₁ | R |
|---|---|---|---|---|---|---|---|
| H8-7 | O | C-A1 | C-A1 | C-A1 | C-A1 | B20 | B1 |
| H8-8 | O | C-A1 | C-A1 | C-A1 | C-A1 | B20 | B6 |
| H8-9 | O | C-A1 | C-A1 | C-A1 | C-A1 | B20 | B20 |
| H8-10 | O | C-A1 | C-A1 | C-A1 | C-A1 | B20 | B21 |
| H8-11 | O | C-A1 | C-A1 | C-A1 | C-A1 | B20 | B22 |
| H8-12 | O | C-A1 | C-A1 | C-A1 | C-A1 | B20 | B32 |
| H8-13 | O | C-A1 | C-A1 | C-A1 | C-A1 | B20 | B33 |
| H8-14 | O | C-A1 | C-A1 | C-A1 | C-A1 | B20 | B34 |
| H8-15 | O | C-A1 | C-A1 | C-A1 | C-A1 | B20 | B68 |
| H8-16 | O | C-A1 | C-A1 | C-A1 | C-A1 | B20 | B69 |
| H8-17 | O | C-A1 | C-A1 | C-A1 | C-A1 | B20 | B70 |
| H8-18 | O | C-A1 | C-A1 | C-A1 | C-A1 | B20 | B71 |
| H8-19 | O | C-A1 | C-A1 | C-A1 | C-A1 | B20 | B81 |
| H8-20 | O | C-A1 | C-A1 | C-A1 | C-A1 | B20 | B90 |
| H8-21 | O | C-A1 | C-A1 | C-A1 | C-A1 | B20 | B91 |
| H8-22 | O | C-A1 | C-A1 | C-A1 | C-A1 | B20 | B92 |
| H8-23 | O | C-A1 | C-A1 | C-A1 | C-A1 | B20 | B93 |
| H8-24 | O | C-A1 | C-A1 | C-A1 | C-A1 | B20 | B94 |
| H8-25 | O | C-A1 | C-A1 | C-A1 | C-A1 | B20 | B95 |
| H8-26 | O | C-A1 | C-A1 | C-A1 | C-A1 | B20 | B96 |
| H8-27 | O | C-A1 | C-A1 | C-A1 | C-A1 | B20 | B97 |
| H8-28 | O | C-A1 | C-A1 | C-A1 | C-A1 | B20 | B106 |
| H8-29 | O | C-A1 | C-A1 | C-A1 | C-A1 | B20 | B107 |
| H8-30 | O | C-A1 | C-A1 | C-A1 | C-A1 | B20 | B114 |
| H8-31 | O | C-A1 | C-A1 | C-A1 | C-A1 | B20 | B115 |
| H8-32 | O | C-A1 | C-A1 | C-A1 | C-A1 | B20 | B116 |
| H8-33 | O | C-A1 | C-A1 | C-A1 | C-A1 | B20 | B117 |
| H8-34 | O | C-A1 | C-A1 | C-A1 | C-A1 | B20 | B118 |
| H8-35 | O | C-A1 | C-A1 | C-A1 | C-A1 | B20 | B119 |
| H8-36 | O | C-A1 | C-A1 | C-A1 | C-A1 | B20 | B120 |
| H8-37 | O | C-A1 | C-A1 | C-A1 | C-A1 | B20 | B121 |
| H8-38 | O | C-A1 | C-A1 | C-A1 | C-A1 | B20 | B122 |
| H8-39 | O | C-A1 | C-A1 | C-A1 | C-A1 | B20 | B125 |
| H8-40 | O | C-A1 | C-A1 | C-A1 | C-A1 | B20 | B126 |
| H8-41 | O | C-A1 | C-A1 | C-A1 | C-A1 | B20 | B127 |
| H8-42 | O | C-A1 | C-A1 | C-A1 | C-A1 | B20 | B128 |
| H8-43 | O | C-A1 | C-A1 | C-A1 | C-A1 | B20 | B129 |
| H8-44 | O | C-A1 | C-A1 | C-A1 | C-A1 | B20 | B130 |
| H8-45 | O | C-A1 | C-A1 | C-A1 | C-A1 | B20 | B131 |
| H8-46 | O | C-A1 | C-A1 | C-A1 | C-A1 | B20 | B132 |
| H8-47 | O | C-A1 | C-A1 | C-A1 | C-A1 | B20 | B133 |
| H8-48 | O | C-A1 | C-A1 | C-A1 | C-A1 | B20 | B134 |
| H8-49 | O | C-A1 | C-A1 | C-A1 | C-A1 | B20 | B135 |
| H8-50 | O | C-A1 | C-A1 | C-A1 | C-A1 | B20 | B136 |
| H8-51 | O | C-A1 | C-A1 | C-A1 | C-A1 | B20 | B137 |
| H8-52 | O | C-A1 | C-A1 | C-A1 | C-A1 | B20 | B138 |
| H8-53 | O | C-A1 | C-A1 | C-A1 | C-A1 | B20 | B139 |
| H8-54 | O | C-A1 | C-A1 | C-A1 | C-A1 | B20 | B140 |
| H8-61 | O | C-A1 | C-A2 | C-A1 | C-A2 | B20 | B1 |
| H8-62 | O | C-A1 | C-A2 | C-A1 | C-A2 | B20 | B6 |
| H8-63 | O | C-A1 | C-A2 | C-A1 | C-A2 | B20 | B20 |
| H8-64 | O | C-A1 | C-A2 | C-A1 | C-A2 | B20 | B21 |
| H8-65 | O | C-A1 | C-A2 | C-A1 | C-A2 | B20 | B22 |
| H8-66 | O | C-A1 | C-A2 | C-A1 | C-A2 | B20 | B32 |
| H8-67 | O | C-A1 | C-A2 | C-A1 | C-A2 | B20 | B33 |
| H8-68 | O | C-A1 | C-A2 | C-A1 | C-A2 | B20 | B34 |
| H8-69 | O | C-A1 | C-A2 | C-A1 | C-A2 | B20 | B68 |
| H8-70 | O | C-A1 | C-A2 | C-A1 | C-A2 | B20 | B69 |
| H8-71 | O | C-A1 | C-A2 | C-A1 | C-A2 | B20 | B70 |
| H8-72 | O | C-A1 | C-A2 | C-A1 | C-A2 | B20 | B71 |
| H8-73 | O | C-A1 | C-A2 | C-A1 | C-A2 | B20 | B81 |
| H8-74 | O | C-A1 | C-A2 | C-A1 | C-A2 | B20 | B90 |
| H8-75 | O | C-A1 | C-A2 | C-A1 | C-A2 | B20 | B91 |
| H8-76 | O | C-A1 | C-A2 | C-A1 | C-A2 | B20 | B92 |
| H8-77 | O | C-A1 | C-A2 | C-A1 | C-A2 | B20 | B93 |
| H8-78 | O | C-A1 | C-A2 | C-A1 | C-A2 | B20 | B94 |
| H8-79 | O | C-A1 | C-A2 | C-A1 | C-A2 | B20 | B95 |
| H8-80 | O | C-A1 | C-A2 | C-A1 | C-A2 | B20 | B96 |
| H8-81 | O | C-A1 | C-A2 | C-A1 | C-A2 | B20 | B97 |
| H8-82 | O | C-A1 | C-A2 | C-A1 | C-A2 | B20 | B106 |
| H8-83 | O | C-A1 | C-A2 | C-A1 | C-A2 | B20 | B107 |
| H8-84 | O | C-A1 | C-A2 | C-A1 | C-A2 | B20 | B114 |
| H8-85 | O | C-A1 | C-A2 | C-A1 | C-A2 | B20 | B115 |
| H8-86 | O | C-A1 | C-A2 | C-A1 | C-A2 | B20 | B116 |
| H8-87 | O | C-A1 | C-A2 | C-A1 | C-A2 | B20 | B117 |
| H8-88 | O | C-A1 | C-A2 | C-A1 | C-A2 | B20 | B118 |
| H8-89 | O | C-A1 | C-A2 | C-A1 | C-A2 | B20 | B119 |
| H8-90 | O | C-A1 | C-A2 | C-A1 | C-A2 | B20 | B120 |
| H8-91 | O | C-A1 | C-A2 | C-A1 | C-A2 | B20 | B121 |
| H8-92 | O | C-A1 | C-A2 | C-A1 | C-A2 | B20 | B122 |
| H8-93 | O | C-A1 | C-A2 | C-A1 | C-A2 | B20 | B125 |
| H8-94 | O | C-A1 | C-A2 | C-A1 | C-A2 | B20 | B126 |
| H8-95 | O | C-A1 | C-A2 | C-A1 | C-A2 | B20 | B127 |
| H8-96 | O | C-A1 | C-A2 | C-A1 | C-A2 | B20 | B128 |
| H8-97 | O | C-A1 | C-A2 | C-A1 | C-A2 | B20 | B129 |
| H8-98 | O | C-A1 | C-A2 | C-A1 | C-A2 | B20 | B130 |
| H8-99 | O | C-A1 | C-A2 | C-A1 | C-A2 | B20 | B131 |
| H8-100 | O | C-A1 | C-A2 | C-A1 | C-A2 | B20 | B132 |
| H8-101 | O | C-A1 | C-A2 | C-A1 | C-A2 | B20 | B133 |
| H8-102 | O | C-A1 | C-A2 | C-A1 | C-A2 | B20 | B134 |
| H8-103 | O | C-A1 | C-A2 | C-A1 | C-A2 | B20 | B135 |
| H8-104 | O | C-A1 | C-A2 | C-A1 | C-A2 | B20 | B136 |
| H8-105 | O | C-A1 | C-A2 | C-A1 | C-A2 | B20 | B137 |
| H8-106 | O | C-A1 | C-A2 | C-A1 | C-A2 | B20 | B138 |
| H8-107 | O | C-A1 | C-A2 | C-A1 | C-A2 | B20 | B139 |
| H8-108 | O | C-A1 | C-A2 | C-A1 | C-A2 | B20 | B140 |
| H8-115 | O | C-A1 | C-A3 | C-A1 | C-A3 | B20 | B1 |
| H8-116 | O | C-A1 | C-A3 | C-A1 | C-A3 | B20 | B6 |
| H8-117 | O | C-A1 | C-A3 | C-A1 | C-A3 | B20 | B20 |
| H8-118 | O | C-A1 | C-A3 | C-A1 | C-A3 | B20 | B21 |
| H8-119 | O | C-A1 | C-A3 | C-A1 | C-A3 | B20 | B22 |
| H8-120 | O | C-A1 | C-A3 | C-A1 | C-A3 | B20 | B32 |
| H8-121 | O | C-A1 | C-A3 | C-A1 | C-A3 | B20 | B33 |
| H8-122 | O | C-A1 | C-A3 | C-A1 | C-A3 | B20 | B34 |
| H8-123 | O | C-A1 | C-A3 | C-A1 | C-A3 | B20 | B68 |
| H8-124 | O | C-A1 | C-A3 | C-A1 | C-A3 | B20 | B69 |
| H8-125 | O | C-A1 | C-A3 | C-A1 | C-A3 | B20 | B70 |
| H8-126 | O | C-A1 | C-A3 | C-A1 | C-A3 | B20 | B71 |
| H8-127 | O | C-A1 | C-A3 | C-A1 | C-A3 | B20 | B81 |
| H8-128 | O | C-A1 | C-A3 | C-A1 | C-A3 | B20 | B90 |
| H8-129 | O | C-A1 | C-A3 | C-A1 | C-A3 | B20 | B91 |
| H8-130 | O | C-A1 | C-A3 | C-A1 | C-A3 | B20 | B92 |
| H8-131 | O | C-A1 | C-A3 | C-A1 | C-A3 | B20 | B93 |
| H8-132 | O | C-A1 | C-A3 | C-A1 | C-A3 | B20 | B94 |
| H8-133 | O | C-A1 | C-A3 | C-A1 | C-A3 | B20 | B95 |
| H8-134 | O | C-A1 | C-A3 | C-A1 | C-A3 | B20 | B96 |
| H8-135 | O | C-A1 | C-A3 | C-A1 | C-A3 | B20 | B97 |
| H8-136 | O | C-A1 | C-A3 | C-A1 | C-A3 | B20 | B106 |
| H8-137 | O | C-A1 | C-A3 | C-A1 | C-A3 | B20 | B107 |
| H8-138 | O | C-A1 | C-A3 | C-A1 | C-A3 | B20 | B114 |
| H8-139 | O | C-A1 | C-A3 | C-A1 | C-A3 | B20 | B115 |
| H8-140 | O | C-A1 | C-A3 | C-A1 | C-A3 | B20 | B116 |
| H8-141 | O | C-A1 | C-A3 | C-A1 | C-A3 | B20 | B117 |
| H8-142 | O | C-A1 | C-A3 | C-A1 | C-A3 | B20 | B118 |
| H8-143 | O | C-A1 | C-A3 | C-A1 | C-A3 | B20 | B119 |
| H8-144 | O | C-A1 | C-A3 | C-A1 | C-A3 | B20 | B 120 |
| H8-145 | O | C-A1 | C-A3 | C-A1 | C-A3 | B20 | B 121 |
| H8-146 | O | C-A1 | C-A3 | C-A1 | C-A3 | B20 | B122 |
| H8-147 | O | C-A1 | C-A3 | C-A1 | C-A3 | B20 | B125 |
| H8-148 | O | C-A1 | C-A3 | C-A1 | C-A3 | B20 | B126 |
| H8-149 | O | C-A1 | C-A3 | C-A1 | C-A3 | B20 | B127 |
| H8-150 | O | C-A1 | C-A3 | C-A1 | C-A3 | B20 | B128 |
| H8-151 | O | C-A1 | C-A3 | C-A1 | C-A3 | B20 | B129 |
| H8-152 | O | C-A1 | C-A3 | C-A1 | C-A3 | B20 | B130 |
| H8-153 | O | C-A1 | C-A3 | C-A1 | C-A3 | B20 | B131 |
| H8-154 | O | C-A1 | C-A3 | C-A1 | C-A3 | B20 | B132 |
| H8-155 | O | C-A1 | C-A3 | C-A1 | C-A3 | B20 | B133 |
| H8-156 | O | C-A1 | C-A3 | C-A1 | C-A3 | B20 | B134 |
| H8-157 | O | C-A1 | C-A3 | C-A1 | C-A3 | B20 | B135 |
| H8-158 | O | C-A1 | C-A3 | C-A1 | C-A3 | B20 | B136 |
| H8-159 | O | C-A1 | C-A3 | C-A1 | C-A3 | B20 | B137 |
| H8-160 | O | C-A1 | C-A3 | C-A1 | C-A3 | B20 | B138 |
| H8-161 | O | C-A1 | C-A3 | C-A1 | C-A3 | B20 | B139 |
| H8-162 | O | C-A1 | C-A3 | C-A1 | C-A3 | B20 | B140 |
| H8-169 | O | C-A3 | C-A1 | C-A3 | C-A1 | B20 | B1 |
| H8-170 | O | C-A3 | C-A1 | C-A3 | C-A1 | B20 | B6 |
| H8-171 | O | C-A3 | C-A1 | C-A3 | C-A1 | B20 | B20 |
| H8-172 | O | C-A3 | C-A1 | C-A3 | C-A1 | B20 | B21 |
| H8-173 | O | C-A3 | C-A1 | C-A3 | C-A1 | B20 | B22 |
| H8-174 | O | C-A3 | C-A1 | C-A3 | C-A1 | B20 | B32 |
| H8-175 | O | C-A3 | C-A1 | C-A3 | C-A1 | B20 | B33 |
| H8-176 | O | C-A3 | C-A1 | C-A3 | C-A1 | B20 | B34 |
| H8-177 | O | C-A3 | C-A1 | C-A3 | C-A1 | B20 | B68 |
| H8-178 | O | C-A3 | C-A1 | C-A3 | C-A1 | B20 | B69 |
| H8-179 | O | C-A3 | C-A1 | C-A3 | C-A1 | B20 | B70 |
| H8-180 | O | C-A3 | C-A1 | C-A3 | C-A1 | B20 | B71 |
| H8-181 | O | C-A3 | C-A1 | C-A3 | C-A1 | B20 | B81 |
| H8-182 | O | C-A3 | C-A1 | C-A3 | C-A1 | B20 | B90 |
| H8-183 | O | C-A3 | C-A1 | C-A3 | C-A1 | B20 | B91 |
| H8-184 | O | C-A3 | C-A1 | C-A3 | C-A1 | B20 | B92 |
| H8-185 | O | C-A3 | C-A1 | C-A3 | C-A1 | B20 | B93 |
| H8-186 | O | C-A3 | C-A1 | C-A3 | C-A1 | B20 | B94 |
| H8-187 | O | C-A3 | C-A1 | C-A3 | C-A1 | B20 | B95 |
| H8-188 | O | C-A3 | C-A1 | C-A3 | C-A1 | B20 | B96 |
| H8-189 | O | C-A3 | C-A1 | C-A3 | C-A1 | B20 | B97 |
| H8-190 | O | C-A3 | C-A1 | C-A3 | C-A1 | B20 | B106 |
| H8-191 | O | C-A3 | C-A1 | C-A3 | C-A1 | B20 | B107 |
| H8-192 | O | C-A3 | C-A1 | C-A3 | C-A1 | B20 | B114 |
| H8-193 | O | C-A3 | C-A1 | C-A3 | C-A1 | B20 | B115 |
| H8-194 | O | C-A3 | C-A1 | C-A3 | C-A1 | B20 | B116 |
| H8-195 | O | C-A3 | C-A1 | C-A3 | C-A1 | B20 | B117 |
| H8-196 | O | C-A3 | C-A1 | C-A3 | C-A1 | B20 | B118 |
| H8-197 | O | C-A3 | C-A1 | C-A3 | C-A1 | B20 | B119 |
| H8-198 | O | C-A3 | C-A1 | C-A3 | C-A1 | B20 | B 120 |
| H8-199 | O | C-A3 | C-A1 | C-A3 | C-A1 | B20 | B121 |
| H8-200 | O | C-A3 | C-A1 | C-A3 | C-A1 | B20 | B122 |
| H8-201 | O | C-A3 | C-A1 | C-A3 | C-A1 | B20 | B125 |
| H8-202 | O | C-A3 | C-A1 | C-A3 | C-A1 | B20 | B126 |
| H8-203 | O | C-A3 | C-A1 | C-A3 | C-A1 | B20 | B127 |
| H8-204 | O | C-A3 | C-A1 | C-A3 | C-A1 | B20 | B128 |
| H8-205 | O | C-A3 | C-A1 | C-A3 | C-A1 | B20 | B129 |
| H8-206 | O | C-A3 | C-A1 | C-A3 | C-A1 | B20 | B130 |
| H8-207 | O | C-A3 | C-A1 | C-A3 | C-A1 | B20 | B131 |
| H8-208 | O | C-A3 | C-A1 | C-A3 | C-A1 | B20 | B132 |
| H8-209 | O | C-A3 | C-A1 | C-A3 | C-A1 | B20 | B133 |
| H8-210 | O | C-A3 | C-A1 | C-A3 | C-A1 | B20 | B134 |
| H8-211 | O | C-A3 | C-A1 | C-A3 | C-A1 | B20 | B135 |
| H8-212 | O | C-A3 | C-A1 | C-A3 | C-A1 | B20 | B136 |
| H8-213 | O | C-A3 | C-A1 | C-A3 | C-A1 | B20 | B137 |
| H8-214 | O | C-A3 | C-A1 | C-A3 | C-A1 | B20 | B138 |
| H8-215 | O | C-A3 | C-A1 | C-A3 | C-A1 | B20 | B139 |
| H8-216 | O | C-A3 | C-A1 | C-A3 | C-A1 | B20 | B140 |
| H8-223 | O | C-A1 | C-A1 | C-A3 | C-A1 | B20 | B1 |
| H8-224 | O | C-A1 | C-A1 | C-A3 | C-A1 | B20 | B6 |
| H8-225 | O | C-A1 | C-A1 | C-A3 | C-A1 | B20 | B20 |
| H8-226 | O | C-A1 | C-A1 | C-A3 | C-A1 | B20 | B21 |
| H8-227 | O | C-A1 | C-A1 | C-A3 | C-A1 | B20 | B22 |
| H8-228 | O | C-A1 | C-A1 | C-A3 | C-A1 | B20 | B32 |
| H8-229 | O | C-A1 | C-A1 | C-A3 | C-A1 | B20 | B33 |
| H8-230 | O | C-A1 | C-A1 | C-A3 | C-A1 | B20 | B34 |
| H8-231 | O | C-A1 | C-A1 | C-A3 | C-A1 | B20 | B68 |
| H8-232 | O | C-A1 | C-A1 | C-A3 | C-A1 | B20 | B69 |
| H8-233 | O | C-A1 | C-A1 | C-A3 | C-A1 | B20 | B70 |
| H8-234 | O | C-A1 | C-A1 | C-A3 | C-A1 | B20 | B71 |
| H8-235 | O | C-A1 | C-A1 | C-A3 | C-A1 | B20 | B81 |
| H8-236 | O | C-A1 | C-A1 | C-A3 | C-A1 | B20 | B90 |
| H8-237 | O | C-A1 | C-A1 | C-A3 | C-A1 | B20 | B91 |
| H8-238 | O | C-A1 | C-A1 | C-A3 | C-A1 | B20 | B92 |
| H8-239 | O | C-A1 | C-A1 | C-A3 | C-A1 | B20 | B93 |
| H8-240 | O | C-A1 | C-A1 | C-A3 | C-A1 | B20 | B94 |
| H8-241 | O | C-A1 | C-A1 | C-A3 | C-A1 | B20 | B95 |
| H8-242 | O | C-A1 | C-A1 | C-A3 | C-A1 | B20 | B96 |
| H8-243 | O | C-A1 | C-A1 | C-A3 | C-A1 | B20 | B97 |
| H8-244 | O | C-A1 | C-A1 | C-A3 | C-A1 | B20 | B106 |
| H8-245 | O | C-A1 | C-A1 | C-A3 | C-A1 | B20 | B107 |
| H8-246 | O | C-A1 | C-A1 | C-A3 | C-A1 | B20 | B114 |
| H8-247 | O | C-A1 | C-A1 | C-A3 | C-A1 | B20 | B115 |
| H8-248 | O | C-A1 | C-A1 | C-A3 | C-A1 | B20 | B116 |
| H8-249 | O | C-A1 | C-A1 | C-A3 | C-A1 | B20 | B117 |
| H8-250 | O | C-A1 | C-A1 | C-A3 | C-A1 | B20 | B118 |
| H8-251 | O | C-A1 | C-A1 | C-A3 | C-A1 | B20 | B119 |
| H8-252 | O | C-A1 | C-A1 | C-A3 | C-A1 | B20 | B 120 |
| H8-253 | O | C-A1 | C-A1 | C-A3 | C-A1 | B20 | B 121 |
| H8-254 | O | C-A1 | C-A1 | C-A3 | C-A1 | B20 | B122 |
| H8-255 | O | C-A1 | C-A1 | C-A3 | C-A1 | B20 | B125 |
| H8-256 | O | C-A1 | C-A1 | C-A3 | C-A1 | B20 | B126 |
| H8-257 | O | C-A1 | C-A1 | C-A3 | C-A1 | B20 | B127 |
| H8-258 | O | C-A1 | C-A1 | C-A3 | C-A1 | B20 | B128 |
| H8-259 | O | C-A1 | C-A1 | C-A3 | C-A1 | B20 | B129 |
| H8-260 | O | C-A1 | C-A1 | C-A3 | C-A1 | B20 | B130 |
| H8-261 | O | C-A1 | C-A1 | C-A3 | C-A1 | B20 | B131 |
| H8-262 | O | C-A1 | C-A1 | C-A3 | C-A1 | B20 | B132 |
| H8-263 | O | C-A1 | C-A1 | C-A3 | C-A1 | B20 | B133 |
| H8-264 | O | C-A1 | C-A1 | C-A3 | C-A1 | B20 | B134 |
| H8-265 | O | C-A1 | C-A1 | C-A3 | C-A1 | B20 | B135 |
| H8-266 | O | C-A1 | C-A1 | C-A3 | C-A1 | B20 | B136 |
| H8-267 | O | C-A1 | C-A1 | C-A3 | C-A1 | B20 | B137 |
| H8-268 | O | C-A1 | C-A1 | C-A3 | C-A1 | B20 | B138 |
| H8-269 | O | C-A1 | C-A1 | C-A3 | C-A1 | B20 | B139 |
| H8-270 | O | C-A1 | C-A1 | C-A3 | C-A1 | B20 | B140 |
| H8-277 | O | C-A1 | C-A1 | C-A1 | C-A1 | B68 | B1 |
| H8-278 | O | C-A1 | C-A1 | C-A1 | C-A1 | B68 | B6 |
| H8-279 | O | C-A1 | C-A1 | C-A1 | C-A1 | B68 | B20 |
| H8-280 | O | C-A1 | C-A1 | C-A1 | C-A1 | B68 | B21 |
| H8-281 | O | C-A1 | C-A1 | C-A1 | C-A1 | B68 | B22 |
| H8-282 | O | C-A1 | C-A1 | C-A1 | C-A1 | B68 | B32 |
| H8-283 | O | C-A1 | C-A1 | C-A1 | C-A1 | B68 | B33 |
| H8-284 | O | C-A1 | C-A1 | C-A1 | C-A1 | B68 | B34 |
| H8-285 | O | C-A1 | C-A1 | C-A1 | C-A1 | B68 | B68 |
| H8-286 | O | C-A1 | C-A1 | C-A1 | C-A1 | B68 | B69 |
| H8-287 | O | C-A1 | C-A1 | C-A1 | C-A1 | B68 | B70 |
| H8-288 | O | C-A1 | C-A1 | C-A1 | C-A1 | B68 | B71 |
| H8-289 | O | C-A1 | C-A1 | C-A1 | C-A1 | B68 | B81 |
| H8-290 | O | C-A1 | C-A1 | C-A1 | C-A1 | B68 | B90 |
| H8-291 | O | C-A1 | C-A1 | C-A1 | C-A1 | B68 | B91 |
| H8-292 | O | C-A1 | C-A1 | C-A1 | C-A1 | B68 | B92 |
| H8-293 | O | C-A1 | C-A1 | C-A1 | C-A1 | B68 | B93 |
| H8-294 | O | C-A1 | C-A1 | C-A1 | C-A1 | B68 | B94 |
| H8-295 | O | C-A1 | C-A1 | C-A1 | C-A1 | B68 | B95 |
| H8-296 | O | C-A1 | C-A1 | C-A1 | C-A1 | B68 | B96 |
| H8-297 | O | C-A1 | C-A1 | C-A1 | C-A1 | B68 | B97 |
| H8-298 | O | C-A1 | C-A1 | C-A1 | C-A1 | B68 | B106 |
| H8-299 | O | C-A1 | C-A1 | C-A1 | C-A1 | B68 | B107 |
| H8-300 | O | C-A1 | C-A1 | C-A1 | C-A1 | B68 | B114 |
| H8-301 | O | C-A1 | C-A1 | C-A1 | C-A1 | B68 | B115 |
| H8-302 | O | C-A1 | C-A1 | C-A1 | C-A1 | B68 | B116 |
| H8-303 | O | C-A1 | C-A1 | C-A1 | C-A1 | B68 | B117 |
| H8-304 | O | C-A1 | C-A1 | C-A1 | C-A1 | B68 | B118 |
| H8-305 | O | C-A1 | C-A1 | C-A1 | C-A1 | B68 | B119 |
| H8-306 | O | C-A1 | C-A1 | C-A1 | C-A1 | B68 | B120 |
| H8-307 | O | C-A1 | C-A1 | C-A1 | C-A1 | B68 | B121 |
| H8-308 | O | C-A1 | C-A1 | C-A1 | C-A1 | B68 | B122 |
| H8-309 | O | C-A1 | C-A1 | C-A1 | C-A1 | B68 | B125 |
| H8-310 | O | C-A1 | C-A1 | C-A1 | C-A1 | B68 | B126 |
| H8-311 | O | C-A1 | C-A1 | C-A1 | C-A1 | B68 | B127 |
| H8-312 | O | C-A1 | C-A1 | C-A1 | C-A1 | B68 | B128 |
| H8-313 | O | C-A1 | C-A1 | C-A1 | C-A1 | B68 | B129 |
| H8-314 | O | C-A1 | C-A1 | C-A1 | C-A1 | B68 | B130 |
| H8-315 | O | C-A1 | C-A1 | C-A1 | C-A1 | B68 | B131 |
| H8-316 | O | C-A1 | C-A1 | C-A1 | C-A1 | B68 | B132 |
| H8-317 | O | C-A1 | C-A1 | C-A1 | C-A1 | B68 | B133 |
| H8-318 | O | C-A1 | C-A1 | C-A1 | C-A1 | B68 | B134 |
| H8-319 | O | C-A1 | C-A1 | C-A1 | C-A1 | B68 | B135 |
| H8-320 | O | C-A1 | C-A1 | C-A1 | C-A1 | B68 | B136 |
| H8-321 | O | C-A1 | C-A1 | C-A1 | C-A1 | B68 | B137 |
| H8-322 | O | C-A1 | C-A1 | C-A1 | C-A1 | B68 | B138 |
| H8-323 | O | C-A1 | C-A1 | C-A1 | C-A1 | B68 | B139 |
| H8-324 | O | C-A1 | C-A1 | C-A1 | C-A1 | B68 | B140 |
| H8-331 | O | C-A1 | C-A2 | C-A1 | C-A2 | B68 | B1 |
| H8-332 | O | C-A1 | C-A2 | C-A1 | C-A2 | B68 | B6 |
| H8-333 | O | C-A1 | C-A2 | C-A1 | C-A2 | B68 | B20 |
| H8-334 | O | C-A1 | C-A2 | C-A1 | C-A2 | B68 | B21 |
| H8-335 | O | C-A1 | C-A2 | C-A1 | C-A2 | B68 | B22 |
| H8-336 | O | C-A1 | C-A2 | C-A1 | C-A2 | B68 | B32 |
| H8-337 | O | C-A1 | C-A2 | C-A1 | C-A2 | B68 | B33 |
| H8-338 | O | C-A1 | C-A2 | C-A1 | C-A2 | B68 | B34 |
| H8-339 | O | C-A1 | C-A2 | C-A1 | C-A2 | B68 | B68 |
| H8-340 | O | C-A1 | C-A2 | C-A1 | C-A2 | B68 | B69 |
| H8-341 | O | C-A1 | C-A2 | C-A1 | C-A2 | B68 | B70 |
| H8-342 | O | C-A1 | C-A2 | C-A1 | C-A2 | B68 | B71 |
| H8-343 | O | C-A1 | C-A2 | C-A1 | C-A2 | B68 | B81 |
| H8-344 | O | C-A1 | C-A2 | C-A1 | C-A2 | B68 | B90 |
| H8-345 | O | C-A1 | C-A2 | C-A1 | C-A2 | B68 | B91 |
| H8-346 | O | C-A1 | C-A2 | C-A1 | C-A2 | B68 | B92 |
| H8-347 | O | C-A1 | C-A2 | C-A1 | C-A2 | B68 | B93 |
| H8-348 | O | C-A1 | C-A2 | C-A1 | C-A2 | B68 | B94 |
| H8-349 | O | C-A1 | C-A2 | C-A1 | C-A2 | B68 | B95 |
| H8-350 | O | C-A1 | C-A2 | C-A1 | C-A2 | B68 | B96 |
| H8-351 | O | C-A1 | C-A2 | C-A1 | C-A2 | B68 | B97 |
| H8-352 | O | C-A1 | C-A2 | C-A1 | C-A2 | B68 | B106 |
| H8-353 | O | C-A1 | C-A2 | C-A1 | C-A2 | B68 | B107 |
| H8-354 | O | C-A1 | C-A2 | C-A1 | C-A2 | B68 | B114 |
| H8-355 | O | C-A1 | C-A2 | C-A1 | C-A2 | B68 | B115 |
| H8-356 | O | C-A1 | C-A2 | C-A1 | C-A2 | B68 | B116 |
| H8-357 | O | C-A1 | C-A2 | C-A1 | C-A2 | B68 | B117 |
| H8-358 | O | C-A1 | C-A2 | C-A1 | C-A2 | B68 | B118 |
| H8-359 | O | C-A1 | C-A2 | C-A1 | C-A2 | B68 | B119 |
| H8-360 | O | C-A1 | C-A2 | C-A1 | C-A2 | B68 | B120 |
| H8-361 | O | C-A1 | C-A2 | C-A1 | C-A2 | B68 | B121 |
| H8-362 | O | C-A1 | C-A2 | C-A1 | C-A2 | B68 | B122 |
| H8-363 | O | C-A1 | C-A2 | C-A1 | C-A2 | B68 | B125 |
| H8-364 | O | C-A1 | C-A2 | C-A1 | C-A2 | B68 | B126 |
| H8-365 | O | C-A1 | C-A2 | C-A1 | C-A2 | B68 | B127 |
| H8-366 | O | C-A1 | C-A2 | C-A1 | C-A2 | B68 | B128 |
| H8-367 | O | C-A1 | C-A2 | C-A1 | C-A2 | B68 | B129 |
| H8-368 | O | C-A1 | C-A2 | C-A1 | C-A2 | B68 | B130 |
| H8-369 | O | C-A1 | C-A2 | C-A1 | C-A2 | B68 | B131 |
| H8-370 | O | C-A1 | C-A2 | C-A1 | C-A2 | B68 | B132 |
| H8-371 | O | C-A1 | C-A2 | C-A1 | C-A2 | B68 | B133 |
| H8-372 | O | C-A1 | C-A2 | C-A1 | C-A2 | B68 | B134 |
| H8-373 | O | C-A1 | C-A2 | C-A1 | C-A2 | B68 | B135 |
| H8-374 | O | C-A1 | C-A2 | C-A1 | C-A2 | B68 | B136 |
| H8-375 | O | C-A1 | C-A2 | C-A1 | C-A2 | B68 | B137 |
| H8-376 | O | C-A1 | C-A2 | C-A1 | C-A2 | B68 | B138 |
| H8-377 | O | C-A1 | C-A2 | C-A1 | C-A2 | B68 | B139 |
| H8-378 | O | C-A1 | C-A2 | C-A1 | C-A2 | B68 | B140 |
| H8-385 | O | C-A1 | C-A3 | C-A1 | C-A3 | B68 | B1 |
| H8-386 | O | C-A1 | C-A3 | C-A1 | C-A3 | B68 | B6 |
| H8-387 | O | C-A1 | C-A3 | C-A1 | C-A3 | B68 | B20 |
| H8-388 | O | C-A1 | C-A3 | C-A1 | C-A3 | B68 | B21 |
| H8-389 | O | C-A1 | C-A3 | C-A1 | C-A3 | B68 | B22 |
| H8-390 | O | C-A1 | C-A3 | C-A1 | C-A3 | B68 | B32 |
| H8-391 | O | C-A1 | C-A3 | C-A1 | C-A3 | B68 | B33 |
| H8-392 | O | C-A1 | C-A3 | C-A1 | C-A3 | B68 | B34 |
| H8-393 | O | C-A1 | C-A3 | C-A1 | C-A3 | B68 | B68 |
| H8-394 | O | C-A1 | C-A3 | C-A1 | C-A3 | B68 | B69 |
| H8-395 | O | C-A1 | C-A3 | C-A1 | C-A3 | B68 | B70 |
| H8-396 | O | C-A1 | C-A3 | C-A1 | C-A3 | B68 | B71 |
| H8-397 | O | C-A1 | C-A3 | C-A1 | C-A3 | B68 | B81 |
| H8-398 | O | C-A1 | C-A3 | C-A1 | C-A3 | B68 | B90 |
| H8-399 | O | C-A1 | C-A3 | C-A1 | C-A3 | B68 | B91 |
| H8-400 | O | C-A1 | C-A3 | C-A1 | C-A3 | B68 | B92 |
| H8-401 | O | C-A1 | C-A3 | C-A1 | C-A3 | B68 | B93 |
| H8-402 | O | C-A1 | C-A3 | C-A1 | C-A3 | B68 | B94 |
| H8-403 | O | C-A1 | C-A3 | C-A1 | C-A3 | B68 | B95 |
| H8-404 | O | C-A1 | C-A3 | C-A1 | C-A3 | B68 | B96 |
| H8-405 | O | C-A1 | C-A3 | C-A1 | C-A3 | B68 | B97 |
| H8-406 | O | C-A1 | C-A3 | C-A1 | C-A3 | B68 | B106 |
| H8-407 | O | C-A1 | C-A3 | C-A1 | C-A3 | B68 | B107 |
| H8-408 | O | C-A1 | C-A3 | C-A1 | C-A3 | B68 | B114 |
| H8-409 | O | C-A1 | C-A3 | C-A1 | C-A3 | B68 | B115 |
| H8-410 | O | C-A1 | C-A3 | C-A1 | C-A3 | B68 | B116 |
| H8-411 | O | C-A1 | C-A3 | C-A1 | C-A3 | B68 | B117 |
| H8-412 | O | C-A1 | C-A3 | C-A1 | C-A3 | B68 | B118 |
| H8-413 | O | C-A1 | C-A3 | C-A1 | C-A3 | B68 | B119 |
| H8-414 | O | C-A1 | C-A3 | C-A1 | C-A3 | B68 | B120 |
| H8-415 | O | C-A1 | C-A3 | C-A1 | C-A3 | B68 | B121 |
| H8-416 | O | C-A1 | C-A3 | C-A1 | C-A3 | B68 | B122 |
| H8-417 | O | C-A1 | C-A3 | C-A1 | C-A3 | B68 | B125 |
| H8-418 | O | C-A1 | C-A3 | C-A1 | C-A3 | B68 | B126 |
| H8-419 | O | C-A1 | C-A3 | C-A1 | C-A3 | B68 | B 127 |
| H8-420 | O | C-A1 | C-A3 | C-A1 | C-A3 | B68 | B128 |
| H8-421 | O | C-A1 | C-A3 | C-A1 | C-A3 | B68 | B129 |
| H8-422 | O | C-A1 | C-A3 | C-A1 | C-A3 | B68 | B130 |
| H8-423 | O | C-A1 | C-A3 | C-A1 | C-A3 | B68 | B131 |
| H8-424 | O | C-A1 | C-A3 | C-A1 | C-A3 | B68 | B132 |
| H8-425 | O | C-A1 | C-A3 | C-A1 | C-A3 | B68 | B133 |
| H8-426 | O | C-A1 | C-A3 | C-A1 | C-A3 | B68 | B134 |
| H8-427 | O | C-A1 | C-A3 | C-A1 | C-A3 | B68 | B135 |
| H8-428 | O | C-A1 | C-A3 | C-A1 | C-A3 | B68 | B136 |
| H8-429 | O | C-A1 | C-A3 | C-A1 | C-A3 | B68 | B137 |
| H8-430 | O | C-A1 | C-A3 | C-A1 | C-A3 | B68 | B138 |
| H8-431 | O | C-A1 | C-A3 | C-A1 | C-A3 | B68 | B139 |
| H8-432 | O | C-A1 | C-A3 | C-A1 | C-A3 | B68 | B140 |
| H8-439 | O | C-A3 | C-A1 | C-A3 | C-A1 | B68 | B1 |
| H8-440 | O | C-A3 | C-A1 | C-A3 | C-A1 | B68 | B6 |
| H8-441 | O | C-A3 | C-A1 | C-A3 | C-A1 | B68 | B20 |
| H8-442 | O | C-A3 | C-A1 | C-A3 | C-A1 | B68 | B21 |
| H8-443 | O | C-A3 | C-A1 | C-A3 | C-A1 | B68 | B22 |
| H8-444 | O | C-A3 | C-A1 | C-A3 | C-A1 | B68 | B32 |
| H8-445 | O | C-A3 | C-A1 | C-A3 | C-A1 | B68 | B33 |
| H8-446 | O | C-A3 | C-A1 | C-A3 | C-A1 | B68 | B34 |
| H8-447 | O | C-A3 | C-A1 | C-A3 | C-A1 | B68 | B68 |
| H8-448 | O | C-A3 | C-A1 | C-A3 | C-A1 | B68 | B69 |
| H8-449 | O | C-A3 | C-A1 | C-A3 | C-A1 | B68 | B70 |
| H8-450 | O | C-A3 | C-A1 | C-A3 | C-A1 | B68 | B71 |
| H8-451 | O | C-A3 | C-A1 | C-A3 | C-A1 | B68 | B81 |
| H8-452 | O | C-A3 | C-A1 | C-A3 | C-A1 | B68 | B90 |
| H8-453 | O | C-A3 | C-A1 | C-A3 | C-A1 | B68 | B91 |
| H8-454 | O | C-A3 | C-A1 | C-A3 | C-A1 | B68 | B92 |
| H8-455 | O | C-A3 | C-A1 | C-A3 | C-A1 | B68 | B93 |
| H8-456 | O | C-A3 | C-A1 | C-A3 | C-A1 | B68 | B94 |
| H8-457 | O | C-A3 | C-A1 | C-A3 | C-A1 | B68 | B95 |
| H8-458 | O | C-A3 | C-A1 | C-A3 | C-A1 | B68 | B96 |
| H8-459 | O | C-A3 | C-A1 | C-A3 | C-A1 | B68 | B97 |
| H8-460 | O | C-A3 | C-A1 | C-A3 | C-A1 | B68 | B106 |
| H8-461 | O | C-A3 | C-A1 | C-A3 | C-A1 | B68 | B107 |
| H8-462 | O | C-A3 | C-A1 | C-A3 | C-A1 | B68 | B114 |
| H8-463 | O | C-A3 | C-A1 | C-A3 | C-A1 | B68 | B115 |
| H8-464 | O | C-A3 | C-A1 | C-A3 | C-A1 | B68 | B116 |
| H8-465 | O | C-A3 | C-A1 | C-A3 | C-A1 | B68 | B117 |
| H8-466 | O | C-A3 | C-A1 | C-A3 | C-A1 | B68 | B118 |
| H8-467 | O | C-A3 | C-A1 | C-A3 | C-A1 | B68 | B119 |
| H8-468 | O | C-A3 | C-A1 | C-A3 | C-A1 | B68 | B120 |
| H8-469 | O | C-A3 | C-A1 | C-A3 | C-A1 | B68 | B121 |
| H8-470 | O | C-A3 | C-A1 | C-A3 | C-A1 | B68 | B122 |
| H8-471 | O | C-A3 | C-A1 | C-A3 | C-A1 | B68 | B125 |
| H8-472 | O | C-A3 | C-A1 | C-A3 | C-A1 | B68 | B126 |
| H8-473 | O | C-A3 | C-A1 | C-A3 | C-A1 | B68 | B127 |
| H8-474 | O | C-A3 | C-A1 | C-A3 | C-A1 | B68 | B128 |
| H8-475 | O | C-A3 | C-A1 | C-A3 | C-A1 | B68 | B129 |
| H8-476 | O | C-A3 | C-A1 | C-A3 | C-A1 | B68 | B130 |
| H8-477 | O | C-A3 | C-A1 | C-A3 | C-A1 | B68 | B131 |
| H8-478 | O | C-A3 | C-A1 | C-A3 | C-A1 | B68 | B132 |
| H8-479 | O | C-A3 | C-A1 | C-A3 | C-A1 | B68 | B133 |
| H8-480 | O | C-A3 | C-A1 | C-A3 | C-A1 | B68 | B134 |
| H8-481 | O | C-A3 | C-A1 | C-A3 | C-A1 | B68 | B135 |
| H8-482 | O | C-A3 | C-A1 | C-A3 | C-A1 | B68 | B136 |
| H8-483 | O | C-A3 | C-A1 | C-A3 | C-A1 | B68 | B137 |
| H8-484 | O | C-A3 | C-A1 | C-A3 | C-A1 | B68 | B138 |
| H8-485 | O | C-A3 | C-A1 | C-A3 | C-A1 | B68 | B139 |
| H8-486 | O | C-A3 | C-A1 | C-A3 | C-A1 | B68 | B140 |
| H8-493 | O | C-A1 | C-A1 | C-A3 | C-A1 | B68 | B1 |
| H8-494 | O | C-A1 | C-A1 | C-A3 | C-A1 | B68 | B6 |
| H8-495 | O | C-A1 | C-A1 | C-A3 | C-A1 | B68 | B20 |
| H8-496 | O | C-A1 | C-A1 | C-A3 | C-A1 | B68 | B21 |
| H8-497 | O | C-A1 | C-A1 | C-A3 | C-A1 | B68 | B22 |
| H8-498 | O | C-A1 | C-A1 | C-A3 | C-A1 | B68 | B32 |
| H8-499 | O | C-A1 | C-A1 | C-A3 | C-A1 | B68 | B33 |
| H8-500 | O | C-A1 | C-A1 | C-A3 | C-A1 | B68 | B34 |
| H8-501 | O | C-A1 | C-A1 | C-A3 | C-A1 | B68 | B68 |
| H8-502 | O | C-A1 | C-A1 | C-A3 | C-A1 | B68 | B69 |
| H8-503 | O | C-A1 | C-A1 | C-A3 | C-A1 | B68 | B70 |
| H8-504 | O | C-A1 | C-A1 | C-A3 | C-A1 | B68 | B71 |
| H8-505 | O | C-A1 | C-A1 | C-A3 | C-A1 | B68 | B81 |
| H8-506 | O | C-A1 | C-A1 | C-A3 | C-A1 | B68 | B90 |
| H8-507 | O | C-A1 | C-A1 | C-A3 | C-A1 | B68 | B91 |
| H8-508 | O | C-A1 | C-A1 | C-A3 | C-A1 | B68 | B92 |
| H8-509 | O | C-A1 | C-A1 | C-A3 | C-A1 | B68 | B93 |
| H8-510 | O | C-A1 | C-A1 | C-A3 | C-A1 | B68 | B94 |
| H8-511 | O | C-A1 | C-A1 | C-A3 | C-A1 | B68 | B95 |
| H8-512 | O | C-A1 | C-A1 | C-A3 | C-A1 | B68 | B96 |
| H8-513 | O | C-A1 | C-A1 | C-A3 | C-A1 | B68 | B97 |
| H8-514 | O | C-A1 | C-A1 | C-A3 | C-A1 | B68 | B106 |
| H8-515 | O | C-A1 | C-A1 | C-A3 | C-A1 | B68 | B107 |
| H8-516 | O | C-A1 | C-A1 | C-A3 | C-A1 | B68 | B114 |
| H8-517 | O | C-A1 | C-A1 | C-A3 | C-A1 | B68 | B115 |
| H8-518 | O | C-A1 | C-A1 | C-A3 | C-A1 | B68 | B116 |
| H8-519 | O | C-A1 | C-A1 | C-A3 | C-A1 | B68 | B117 |
| H8-520 | O | C-A1 | C-A1 | C-A3 | C-A1 | B68 | B118 |
| H8-521 | O | C-A1 | C-A1 | C-A3 | C-A1 | B68 | B119 |
| H8-522 | O | C-A1 | C-A1 | C-A3 | C-A1 | B68 | B120 |
| H8-523 | O | C-A1 | C-A1 | C-A3 | C-A1 | B68 | B121 |
| H8-524 | O | C-A1 | C-A1 | C-A3 | C-A1 | B68 | B122 |
| H8-525 | O | C-A1 | C-A1 | C-A3 | C-A1 | B68 | B125 |
| H8-526 | O | C-A1 | C-A1 | C-A3 | C-A1 | B68 | B126 |
| H8-527 | O | C-A1 | C-A1 | C-A3 | C-A1 | B68 | B127 |
| H8-528 | O | C-A1 | C-A1 | C-A3 | C-A1 | B68 | B128 |
| H8-529 | O | C-A1 | C-A1 | C-A3 | C-A1 | B68 | B129 |
| H8-530 | O | C-A1 | C-A1 | C-A3 | C-A1 | B68 | B130 |
| H8-531 | O | C-A1 | C-A1 | C-A3 | C-A1 | B68 | B131 |
| H8-532 | O | C-A1 | C-A1 | C-A3 | C-A1 | B68 | B132 |
| H8-533 | O | C-A1 | C-A1 | C-A3 | C-A1 | B68 | B133 |
| H8-534 | O | C-A1 | C-A1 | C-A3 | C-A1 | B68 | B134 |
| H8-535 | O | C-A1 | C-A1 | C-A3 | C-A1 | B68 | B135 |
| H8-536 | O | C-A1 | C-A1 | C-A3 | C-A1 | B68 | B136 |
| H8-537 | O | C-A1 | C-A1 | C-A3 | C-A1 | B68 | B137 |
| H8-538 | O | C-A1 | C-A1 | C-A3 | C-A1 | B68 | B138 |
| H8-539 | O | C-A1 | C-A1 | C-A3 | C-A1 | B68 | B139 |
| H8-540 | O | C-A1 | C-A1 | C-A3 | C-A1 | B68 | B140 |
| H8-547 | O | C-A1 | C-A1 | C-A1 | C-A1 | B70 | B1 |
| H8-548 | O | C-A1 | C-A1 | C-A1 | C-A1 | B70 | B6 |
| H8-549 | O | C-A1 | C-A1 | C-A1 | C-A1 | B70 | B20 |
| H8-550 | O | C-A1 | C-A1 | C-A1 | C-A1 | B70 | B21 |
| H8-551 | O | C-A1 | C-A1 | C-A1 | C-A1 | B70 | B22 |
| H8-552 | O | C-A1 | C-A1 | C-A1 | C-A1 | B70 | B32 |
| H8-553 | O | C-A1 | C-A1 | C-A1 | C-A1 | B70 | B33 |
| H8-554 | O | C-A1 | C-A1 | C-A1 | C-A1 | B70 | B34 |
| H8-555 | O | C-A1 | C-A1 | C-A1 | C-A1 | B70 | B68 |
| H8-556 | O | C-A1 | C-A1 | C-A1 | C-A1 | B70 | B69 |
| H8-557 | O | C-A1 | C-A1 | C-A1 | C-A1 | B70 | B70 |
| H8-558 | O | C-A1 | C-A1 | C-A1 | C-A1 | B70 | B71 |
| H8-559 | O | C-A1 | C-A1 | C-A1 | C-A1 | B70 | B81 |
| H8-560 | O | C-A1 | C-A1 | C-A1 | C-A1 | B70 | B90 |
| H8-561 | O | C-A1 | C-A1 | C-A1 | C-A1 | B70 | B91 |
| H8-562 | O | C-A1 | C-A1 | C-A1 | C-A1 | B70 | B92 |
| H8-563 | O | C-A1 | C-A1 | C-A1 | C-A1 | B70 | B93 |
| H8-564 | O | C-A1 | C-A1 | C-A1 | C-A1 | B70 | B94 |
| H8-565 | O | C-A1 | C-A1 | C-A1 | C-A1 | B70 | B95 |
| H8-566 | O | C-A1 | C-A1 | C-A1 | C-A1 | B70 | B96 |
| H8-567 | O | C-A1 | C-A1 | C-A1 | C-A1 | B70 | B97 |
| H8-568 | O | C-A1 | C-A1 | C-A1 | C-A1 | B70 | B106 |
| H8-569 | O | C-A1 | C-A1 | C-A1 | C-A1 | B70 | B107 |
| H8-570 | O | C-A1 | C-A1 | C-A1 | C-A1 | B70 | B114 |
| H8-571 | O | C-A1 | C-A1 | C-A1 | C-A1 | B70 | B115 |
| H8-572 | O | C-A1 | C-A1 | C-A1 | C-A1 | B70 | B116 |
| H8-573 | O | C-A1 | C-A1 | C-A1 | C-A1 | B70 | B117 |
| H8-574 | O | C-A1 | C-A1 | C-A1 | C-A1 | B70 | B118 |
| H8-575 | O | C-A1 | C-A1 | C-A1 | C-A1 | B70 | B119 |
| H8-576 | O | C-A1 | C-A1 | C-A1 | C-A1 | B70 | B120 |
| H8-577 | O | C-A1 | C-A1 | C-A1 | C-A1 | B70 | B121 |
| H8-578 | O | C-A1 | C-A1 | C-A1 | C-A1 | B70 | B122 |
| H8-579 | O | C-A1 | C-A1 | C-A1 | C-A1 | B70 | B125 |
| H8-580 | O | C-A1 | C-A1 | C-A1 | C-A1 | B70 | B126 |
| H8-581 | O | C-A1 | C-A1 | C-A1 | C-A1 | B70 | B127 |
| H8-582 | O | C-A1 | C-A1 | C-A1 | C-A1 | B70 | B128 |
| H8-583 | O | C-A1 | C-A1 | C-A1 | C-A1 | B70 | B129 |
| H8-584 | O | C-A1 | C-A1 | C-A1 | C-A1 | B70 | B130 |
| H8-585 | O | C-A1 | C-A1 | C-A1 | C-A1 | B70 | B131 |
| H8-586 | O | C-A1 | C-A1 | C-A1 | C-A1 | B70 | B132 |
| H8-587 | O | C-A1 | C-A1 | C-A1 | C-A1 | B70 | B133 |
| H8-588 | O | C-A1 | C-A1 | C-A1 | C-A1 | B70 | B134 |
| H8-589 | O | C-A1 | C-A1 | C-A1 | C-A1 | B70 | B135 |
| H8-590 | O | C-A1 | C-A1 | C-A1 | C-A1 | B70 | B136 |
| H8-591 | O | C-A1 | C-A1 | C-A1 | C-A1 | B70 | B137 |
| H8-592 | O | C-A1 | C-A1 | C-A1 | C-A1 | B70 | B138 |
| H8-593 | O | C-A1 | C-A1 | C-A1 | C-A1 | B70 | B139 |
| H8-594 | O | C-A1 | C-A1 | C-A1 | C-A1 | B70 | B140 |
| H8-601 | O | C-A1 | C-A2 | C-A1 | C-A2 | B70 | B1 |
| H8-602 | O | C-A1 | C-A2 | C-A1 | C-A2 | B70 | B6 |
| H8-603 | O | C-A1 | C-A2 | C-A1 | C-A2 | B70 | B20 |
| H8-604 | O | C-A1 | C-A2 | C-A1 | C-A2 | B70 | B21 |
| H8-605 | O | C-A1 | C-A2 | C-A1 | C-A2 | B70 | B22 |
| H8-606 | O | C-A1 | C-A2 | C-A1 | C-A2 | B70 | B32 |
| H8-607 | O | C-A1 | C-A2 | C-A1 | C-A2 | B70 | B33 |
| H8-608 | O | C-A1 | C-A2 | C-A1 | C-A2 | B70 | B34 |
| H8-609 | O | C-A1 | C-A2 | C-A1 | C-A2 | B70 | B68 |
| H8-610 | O | C-A1 | C-A2 | C-A1 | C-A2 | B70 | B69 |
| H8-611 | O | C-A1 | C-A2 | C-A1 | C-A2 | B70 | B70 |
| H8-612 | O | C-A1 | C-A2 | C-A1 | C-A2 | B70 | B71 |
| H8-613 | O | C-A1 | C-A2 | C-A1 | C-A2 | B70 | B81 |
| H8-614 | O | C-A1 | C-A2 | C-A1 | C-A2 | B70 | B90 |
| H8-615 | O | C-A1 | C-A2 | C-A1 | C-A2 | B70 | B91 |
| H8-616 | O | C-A1 | C-A2 | C-A1 | C-A2 | B70 | B92 |
| H8-617 | O | C-A1 | C-A2 | C-A1 | C-A2 | B70 | B93 |
| H8-618 | O | C-A1 | C-A2 | C-A1 | C-A2 | B70 | B94 |
| H8-619 | O | C-A1 | C-A2 | C-A1 | C-A2 | B70 | B95 |
| H8-620 | O | C-A1 | C-A2 | C-A1 | C-A2 | B70 | B96 |
| H8-621 | O | C-A1 | C-A2 | C-A1 | C-A2 | B70 | B97 |
| H8-622 | O | C-A1 | C-A2 | C-A1 | C-A2 | B70 | B106 |
| H8-623 | O | C-A1 | C-A2 | C-A1 | C-A2 | B70 | B107 |
| H8-624 | O | C-A1 | C-A2 | C-A1 | C-A2 | B70 | B114 |
| H8-625 | O | C-A1 | C-A2 | C-A1 | C-A2 | B70 | B115 |
| H8-626 | O | C-A1 | C-A2 | C-A1 | C-A2 | B70 | B116 |
| H8-627 | O | C-A1 | C-A2 | C-A1 | C-A2 | B70 | B117 |
| H8-628 | O | C-A1 | C-A2 | C-A1 | C-A2 | B70 | B118 |
| H8-629 | O | C-A1 | C-A2 | C-A1 | C-A2 | B70 | B119 |
| H8-630 | O | C-A1 | C-A2 | C-A1 | C-A2 | B70 | B120 |
| H8-631 | O | C-A1 | C-A2 | C-A1 | C-A2 | B70 | B121 |
| H8-632 | O | C-A1 | C-A2 | C-A1 | C-A2 | B70 | B122 |
| H8-633 | O | C-A1 | C-A2 | C-A1 | C-A2 | B70 | B125 |
| H8-634 | O | C-A1 | C-A2 | C-A1 | C-A2 | B70 | B126 |
| H8-635 | O | C-A1 | C-A2 | C-A1 | C-A2 | B70 | B127 |
| H8-636 | O | C-A1 | C-A2 | C-A1 | C-A2 | B70 | B128 |
| H8-637 | O | C-A1 | C-A2 | C-A1 | C-A2 | B70 | B129 |
| H8-638 | O | C-A1 | C-A2 | C-A1 | C-A2 | B70 | B130 |
| H8-639 | O | C-A1 | C-A2 | C-A1 | C-A2 | B70 | B131 |
| H8-640 | O | C-A1 | C-A2 | C-A1 | C-A2 | B70 | B132 |
| H8-641 | O | C-A1 | C-A2 | C-A1 | C-A2 | B70 | B133 |
| H8-642 | O | C-A1 | C-A2 | C-A1 | C-A2 | B70 | B134 |
| H8-643 | O | C-A1 | C-A2 | C-A1 | C-A2 | B70 | B135 |
| H8-644 | O | C-A1 | C-A2 | C-A1 | C-A2 | B70 | B136 |
| H8-645 | O | C-A1 | C-A2 | C-A1 | C-A2 | B70 | B137 |
| H8-646 | O | C-A1 | C-A2 | C-A1 | C-A2 | B70 | B138 |
| H8-647 | O | C-A1 | C-A2 | C-A1 | C-A2 | B70 | B139 |
| H8-648 | O | C-A1 | C-A2 | C-A1 | C-A2 | B70 | B140 |
| H8-655 | O | C-A1 | C-A3 | C-A1 | C-A3 | B70 | B1 |
| H8-656 | O | C-A1 | C-A3 | C-A1 | C-A3 | B70 | B6 |
| H8-657 | O | C-A1 | C-A3 | C-A1 | C-A3 | B70 | B20 |
| H8-658 | O | C-A1 | C-A3 | C-A1 | C-A3 | B70 | B21 |
| H8-659 | O | C-A1 | C-A3 | C-A1 | C-A3 | B70 | B22 |
| H8-660 | O | C-A1 | C-A3 | C-A1 | C-A3 | B70 | B32 |
| H8-661 | O | C-A1 | C-A3 | C-A1 | C-A3 | B70 | B33 |
| H8-662 | O | C-A1 | C-A3 | C-A1 | C-A3 | B70 | B34 |
| H8-663 | O | C-A1 | C-A3 | C-A1 | C-A3 | B70 | B68 |
| H8-664 | O | C-A1 | C-A3 | C-A1 | C-A3 | B70 | B69 |
| H8-665 | O | C-A1 | C-A3 | C-A1 | C-A3 | B70 | B70 |
| H8-666 | O | C-A1 | C-A3 | C-A1 | C-A3 | B70 | B71 |
| H8-667 | O | C-A1 | C-A3 | C-A1 | C-A3 | B70 | B81 |
| H8-668 | O | C-A1 | C-A3 | C-A1 | C-A3 | B70 | B90 |
| H8-669 | O | C-A1 | C-A3 | C-A1 | C-A3 | B70 | B91 |
| H8-670 | O | C-A1 | C-A3 | C-A1 | C-A3 | B70 | B92 |
| H8-671 | O | C-A1 | C-A3 | C-A1 | C-A3 | B70 | B93 |
| H8-672 | O | C-A1 | C-A3 | C-A1 | C-A3 | B70 | B94 |
| H8-673 | O | C-A1 | C-A3 | C-A1 | C-A3 | B70 | B95 |
| H8-674 | O | C-A1 | C-A3 | C-A1 | C-A3 | B70 | B96 |
| H8-675 | O | C-A1 | C-A3 | C-A1 | C-A3 | B70 | B97 |
| H8-676 | O | C-A1 | C-A3 | C-A1 | C-A3 | B70 | B106 |
| H8-677 | O | C-A1 | C-A3 | C-A1 | C-A3 | B70 | B107 |
| H8-678 | O | C-A1 | C-A3 | C-A1 | C-A3 | B70 | B114 |
| H8-679 | O | C-A1 | C-A3 | C-A1 | C-A3 | B70 | B115 |
| H8-680 | O | C-A1 | C-A3 | C-A1 | C-A3 | B70 | B116 |
| H8-681 | O | C-A1 | C-A3 | C-A1 | C-A3 | B70 | B117 |
| H8-682 | O | C-A1 | C-A3 | C-A1 | C-A3 | B70 | B118 |
| H8-683 | O | C-A1 | C-A3 | C-A1 | C-A3 | B70 | B119 |
| H8-684 | O | C-A1 | C-A3 | C-A1 | C-A3 | B70 | B120 |
| H8-685 | O | C-A1 | C-A3 | C-A1 | C-A3 | B70 | B121 |
| H8-686 | O | C-A1 | C-A3 | C-A1 | C-A3 | B70 | B122 |
| H8-687 | O | C-A1 | C-A3 | C-A1 | C-A3 | B70 | B125 |
| H8-688 | O | C-A1 | C-A3 | C-A1 | C-A3 | B70 | B126 |
| H8-689 | O | C-A1 | C-A3 | C-A1 | C-A3 | B70 | B127 |
| H8-690 | O | C-A1 | C-A3 | C-A1 | C-A3 | B70 | B128 |
| H8-691 | O | C-A1 | C-A3 | C-A1 | C-A3 | B70 | B129 |
| H8-692 | O | C-A1 | C-A3 | C-A1 | C-A3 | B70 | B130 |
| H8-693 | O | C-A1 | C-A3 | C-A1 | C-A3 | B70 | B131 |
| H8-694 | O | C-A1 | C-A3 | C-A1 | C-A3 | B70 | B132 |
| H8-695 | O | C-A1 | C-A3 | C-A1 | C-A3 | B70 | B133 |
| H8-696 | O | C-A1 | C-A3 | C-A1 | C-A3 | B70 | B134 |
| H8-697 | O | C-A1 | C-A3 | C-A1 | C-A3 | B70 | B135 |
| H8-698 | O | C-A1 | C-A3 | C-A1 | C-A3 | B70 | B136 |
| H8-699 | O | C-A1 | C-A3 | C-A1 | C-A3 | B70 | B137 |
| H8-700 | O | C-A1 | C-A3 | C-A1 | C-A3 | B70 | B138 |
| H8-701 | O | C-A1 | C-A3 | C-A1 | C-A3 | B70 | B139 |
| H8-702 | O | C-A1 | C-A3 | C-A1 | C-A3 | B70 | B140 |
| H8-709 | O | C-A3 | C-A1 | C-A3 | C-A1 | B70 | B1 |
| H8-710 | O | C-A3 | C-A1 | C-A3 | C-A1 | B70 | B6 |
| H8-711 | O | C-A3 | C-A1 | C-A3 | C-A1 | B70 | B20 |
| H8-712 | O | C-A3 | C-A1 | C-A3 | C-A1 | B70 | B21 |
| H8-713 | O | C-A3 | C-A1 | C-A3 | C-A1 | B70 | B22 |
| H8-714 | O | C-A3 | C-A1 | C-A3 | C-A1 | B70 | B32 |
| H8-715 | O | C-A3 | C-A1 | C-A3 | C-A1 | B70 | B33 |
| H8-716 | O | C-A3 | C-A1 | C-A3 | C-A1 | B70 | B34 |
| H8-717 | O | C-A3 | C-A1 | C-A3 | C-A1 | B70 | B68 |
| H8-718 | O | C-A3 | C-A1 | C-A3 | C-A1 | B70 | B69 |
| H8-719 | O | C-A3 | C-A1 | C-A3 | C-A1 | B70 | B70 |
| H8-720 | O | C-A3 | C-A1 | C-A3 | C-A1 | B70 | B71 |
| H8-721 | O | C-A3 | C-A1 | C-A3 | C-A1 | B70 | B81 |
| H8-722 | O | C-A3 | C-A1 | C-A3 | C-A1 | B70 | B90 |
| H8-723 | O | C-A3 | C-A1 | C-A3 | C-A1 | B70 | B91 |
| H8-724 | O | C-A3 | C-A1 | C-A3 | C-A1 | B70 | B92 |
| H8-725 | O | C-A3 | C-A1 | C-A3 | C-A1 | B70 | B93 |
| H8-726 | O | C-A3 | C-A1 | C-A3 | C-A1 | B70 | B94 |
| H8-727 | O | C-A3 | C-A1 | C-A3 | C-A1 | B70 | B95 |
| H8-728 | O | C-A3 | C-A1 | C-A3 | C-A1 | B70 | B96 |
| H8-729 | O | C-A3 | C-A1 | C-A3 | C-A1 | B70 | B97 |
| H8-730 | O | C-A3 | C-A1 | C-A3 | C-A1 | B70 | B106 |
| H8-731 | O | C-A3 | C-A1 | C-A3 | C-A1 | B70 | B107 |
| H8-732 | O | C-A3 | C-A1 | C-A3 | C-A1 | B70 | B114 |
| H8-733 | O | C-A3 | C-A1 | C-A3 | C-A1 | B70 | B115 |
| H8-734 | O | C-A3 | C-A1 | C-A3 | C-A1 | B70 | B116 |
| H8-735 | O | C-A3 | C-A1 | C-A3 | C-A1 | B70 | B117 |
| H8-736 | O | C-A3 | C-A1 | C-A3 | C-A1 | B70 | B118 |
| H8-737 | O | C-A3 | C-A1 | C-A3 | C-A1 | B70 | B119 |
| H8-738 | O | C-A3 | C-A1 | C-A3 | C-A1 | B70 | B120 |
| H8-739 | O | C-A3 | C-A1 | C-A3 | C-A1 | B70 | B121 |
| H8-740 | O | C-A3 | C-A1 | C-A3 | C-A1 | B70 | B122 |
| H8-741 | O | C-A3 | C-A1 | C-A3 | C-A1 | B70 | B125 |
| H8-742 | O | C-A3 | C-A1 | C-A3 | C-A1 | B70 | B126 |
| H8-743 | O | C-A3 | C-A1 | C-A3 | C-A1 | B70 | B127 |
| H8-744 | O | C-A3 | C-A1 | C-A3 | C-A1 | B70 | B128 |
| H8-745 | O | C-A3 | C-A1 | C-A3 | C-A1 | B70 | B129 |
| H8-746 | O | C-A3 | C-A1 | C-A3 | C-A1 | B70 | B130 |
| H8-747 | O | C-A3 | C-A1 | C-A3 | C-A1 | B70 | B131 |
| H8-748 | O | C-A3 | C-A1 | C-A3 | C-A1 | B70 | B132 |
| H8-749 | O | C-A3 | C-A1 | C-A3 | C-A1 | B70 | B133 |
| H8-750 | O | C-A3 | C-A1 | C-A3 | C-A1 | B70 | B134 |
| H8-751 | O | C-A3 | C-A1 | C-A3 | C-A1 | B70 | B135 |
| H8-752 | O | C-A3 | C-A1 | C-A3 | C-A1 | B70 | B136 |
| H8-753 | O | C-A3 | C-A1 | C-A3 | C-A1 | B70 | B137 |
| H8-754 | O | C-A3 | C-A1 | C-A3 | C-A1 | B70 | B138 |
| H8-755 | O | C-A3 | C-A1 | C-A3 | C-A1 | B70 | B139 |
| H8-756 | O | C-A3 | C-A1 | C-A3 | C-A1 | B70 | B140 |
| H8-763 | O | C-A1 | C-A1 | C-A3 | C-A1 | B70 | B1 |
| H8-764 | O | C-A1 | C-A1 | C-A3 | C-A1 | B70 | B6 |
| H8-765 | O | C-A1 | C-A1 | C-A3 | C-A1 | B70 | B20 |
| H8-766 | O | C-A1 | C-A1 | C-A3 | C-A1 | B70 | B21 |
| H8-767 | O | C-A1 | C-A1 | C-A3 | C-A1 | B70 | B22 |
| H8-768 | O | C-A1 | C-A1 | C-A3 | C-A1 | B70 | B32 |
| H8-769 | O | C-A1 | C-A1 | C-A3 | C-A1 | B70 | B33 |
| H8-770 | O | C-A1 | C-A1 | C-A3 | C-A1 | B70 | B34 |
| H8-771 | O | C-A1 | C-A1 | C-A3 | C-A1 | B70 | B68 |
| H8-772 | O | C-A1 | C-A1 | C-A3 | C-A1 | B70 | B69 |
| H8-773 | O | C-A1 | C-A1 | C-A3 | C-A1 | B70 | B70 |
| H8-774 | O | C-A1 | C-A1 | C-A3 | C-A1 | B70 | B71 |
| H8-775 | O | C-A1 | C-A1 | C-A3 | C-A1 | B70 | B81 |
| H8-776 | O | C-A1 | C-A1 | C-A3 | C-A1 | B70 | B90 |
| H8-777 | O | C-A1 | C-A1 | C-A3 | C-A1 | B70 | B91 |
| H8-778 | O | C-A1 | C-A1 | C-A3 | C-A1 | B70 | B92 |
| H8-779 | O | C-A1 | C-A1 | C-A3 | C-A1 | B70 | B93 |
| H8-780 | O | C-A1 | C-A1 | C-A3 | C-A1 | B70 | B94 |
| H8-781 | O | C-A1 | C-A1 | C-A3 | C-A1 | B70 | B95 |
| H8-782 | O | C-A1 | C-A1 | C-A3 | C-A1 | B70 | B96 |
| H8-783 | O | C-A1 | C-A1 | C-A3 | C-A1 | B70 | B97 |
| H8-784 | O | C-A1 | C-A1 | C-A3 | C-A1 | B70 | B106 |
| H8-785 | O | C-A1 | C-A1 | C-A3 | C-A1 | B70 | B107 |
| H8-786 | O | C-A1 | C-A1 | C-A3 | C-A1 | B70 | B114 |
| H8-787 | O | C-A1 | C-A1 | C-A3 | C-A1 | B70 | B115 |
| H8-788 | O | C-A1 | C-A1 | C-A3 | C-A1 | B70 | B116 |
| H8-789 | O | C-A1 | C-A1 | C-A3 | C-A1 | B70 | B117 |
| H8-790 | O | C-A1 | C-A1 | C-A3 | C-A1 | B70 | B118 |
| H8-791 | O | C-A1 | C-A1 | C-A3 | C-A1 | B70 | B119 |
| H8-792 | O | C-A1 | C-A1 | C-A3 | C-A1 | B70 | B120 |
| H8-793 | O | C-A1 | C-A1 | C-A3 | C-A1 | B70 | B121 |
| H8-794 | O | C-A1 | C-A1 | C-A3 | C-A1 | B70 | B122 |
| H8-795 | O | C-A1 | C-A1 | C-A3 | C-A1 | B70 | B125 |
| H8-796 | O | C-A1 | C-A1 | C-A3 | C-A1 | B70 | B126 |
| H8-797 | O | C-A1 | C-A1 | C-A3 | C-A1 | B70 | B127 |
| H8-798 | O | C-A1 | C-A1 | C-A3 | C-A1 | B70 | B128 |
| H8-799 | O | C-A1 | C-A1 | C-A3 | C-A1 | B70 | B129 |
| H8-800 | O | C-A1 | C-A1 | C-A3 | C-A1 | B70 | B130 |
| H8-801 | O | C-A1 | C-A1 | C-A3 | C-A1 | B70 | B131 |
| H8-802 | O | C-A1 | C-A1 | C-A3 | C-A1 | B70 | B132 |
| H8-803 | O | C-A1 | C-A1 | C-A3 | C-A1 | B70 | B133 |
| H8-804 | O | C-A1 | C-A1 | C-A3 | C-A1 | B70 | B134 |
| H8-805 | O | C-A1 | C-A1 | C-A3 | C-A1 | B70 | B135 |
| H8-806 | O | C-A1 | C-A1 | C-A3 | C-A1 | B70 | B136 |
| H8-807 | O | C-A1 | C-A1 | C-A3 | C-A1 | B70 | B137 |
| H8-808 | O | C-A1 | C-A1 | C-A3 | C-A1 | B70 | B138 |
| H8-809 | O | C-A1 | C-A1 | C-A3 | C-A1 | B70 | B139 |
| H8-810 | O | C-A1 | C-A1 | C-A3 | C-A1 | B70 | B140 |
| H8-817 | O | C-A1 | C-A1 | C-A1 | C-A1 | B22 | B1 |
| H8-818 | O | C-A1 | C-A1 | C-A1 | C-A1 | B22 | B6 |
| H8-819 | O | C-A1 | C-A1 | C-A1 | C-A1 | B22 | B20 |
| H8-820 | O | C-A1 | C-A1 | C-A1 | C-A1 | B22 | B21 |
| H8-821 | O | C-A1 | C-A1 | C-A1 | C-A1 | B22 | B22 |
| H8-822 | O | C-A1 | C-A1 | C-A1 | C-A1 | B22 | B32 |
| H8-823 | O | C-A1 | C-A1 | C-A1 | C-A1 | B22 | B33 |
| H8-824 | O | C-A1 | C-A1 | C-A1 | C-A1 | B22 | B34 |
| H8-825 | O | C-A1 | C-A1 | C-A1 | C-A1 | B22 | B68 |
| H8-826 | O | C-A1 | C-A1 | C-A1 | C-A1 | B22 | B69 |
| H8-827 | O | C-A1 | C-A1 | C-A1 | C-A1 | B22 | B70 |
| H8-828 | O | C-A1 | C-A1 | C-A1 | C-A1 | B22 | B71 |
| H8-829 | O | C-A1 | C-A1 | C-A1 | C-A1 | B22 | B81 |
| H8-830 | O | C-A1 | C-A1 | C-A1 | C-A1 | B22 | B90 |
| H8-831 | O | C-A1 | C-A1 | C-A1 | C-A1 | B22 | B91 |
| H8-832 | O | C-A1 | C-A1 | C-A1 | C-A1 | B22 | B92 |
| H8-833 | O | C-A1 | C-A1 | C-A1 | C-A1 | B22 | B93 |
| H8-834 | O | C-A1 | C-A1 | C-A1 | C-A1 | B22 | B94 |
| H8-835 | O | C-A1 | C-A1 | C-A1 | C-A1 | B22 | B95 |
| H8-836 | O | C-A1 | C-A1 | C-A1 | C-A1 | B22 | B96 |
| H8-837 | O | C-A1 | C-A1 | C-A1 | C-A1 | B22 | B97 |
| H8-838 | O | C-A1 | C-A1 | C-A1 | C-A1 | B22 | B106 |
| H8-839 | O | C-A1 | C-A1 | C-A1 | C-A1 | B22 | B107 |
| H8-840 | O | C-A1 | C-A1 | C-A1 | C-A1 | B22 | B114 |
| H8-841 | O | C-A1 | C-A1 | C-A1 | C-A1 | B22 | B115 |
| H8-842 | O | C-A1 | C-A1 | C-A1 | C-A1 | B22 | B116 |
| H8-843 | O | C-A1 | C-A1 | C-A1 | C-A1 | B22 | B117 |
| H8-844 | O | C-A1 | C-A1 | C-A1 | C-A1 | B22 | B118 |
| H8-845 | O | C-A1 | C-A1 | C-A1 | C-A1 | B22 | B119 |
| H8-846 | O | C-A1 | C-A1 | C-A1 | C-A1 | B22 | B120 |
| H8-847 | O | C-A1 | C-A1 | C-A1 | C-A1 | B22 | B121 |
| H8-848 | O | C-A1 | C-A1 | C-A1 | C-A1 | B22 | B122 |
| H8-849 | O | C-A1 | C-A1 | C-A1 | C-A1 | B22 | B125 |
| H8-850 | O | C-A1 | C-A1 | C-A1 | C-A1 | B22 | B126 |
| H8-851 | O | C-A1 | C-A1 | C-A1 | C-A1 | B22 | B 127 |
| H8-852 | O | C-A1 | C-A1 | C-A1 | C-A1 | B22 | B128 |
| H8-853 | O | C-A1 | C-A1 | C-A1 | C-A1 | B22 | B129 |
| H8-854 | O | C-A1 | C-A1 | C-A1 | C-A1 | B22 | B130 |
| H8-855 | O | C-A1 | C-A1 | C-A1 | C-A1 | B22 | B131 |
| H8-856 | O | C-A1 | C-A1 | C-A1 | C-A1 | B22 | B132 |
| H8-857 | O | C-A1 | C-A1 | C-A1 | C-A1 | B22 | B133 |
| H8-858 | O | C-A1 | C-A1 | C-A1 | C-A1 | B22 | B134 |
| H8-859 | O | C-A1 | C-A1 | C-A1 | C-A1 | B22 | B135 |
| H8-860 | O | C-A1 | C-A1 | C-A1 | C-A1 | B22 | B136 |
| H8-861 | O | C-A1 | C-A1 | C-A1 | C-A1 | B22 | B137 |
| H8-862 | O | C-A1 | C-A1 | C-A1 | C-A1 | B22 | B138 |
| H8-863 | O | C-A1 | C-A1 | C-A1 | C-A1 | B22 | B139 |
| H8-864 | O | C-A1 | C-A1 | C-A1 | C-A1 | B22 | B140 |
| H8-871 | O | C-A1 | C-A2 | C-A1 | C-A2 | B22 | B1 |
| H8-872 | O | C-A1 | C-A2 | C-A1 | C-A2 | B22 | B6 |
| H8-873 | O | C-A1 | C-A2 | C-A1 | C-A2 | B22 | B20 |
| H8-874 | O | C-A1 | C-A2 | C-A1 | C-A2 | B22 | B21 |
| H8-875 | O | C-A1 | C-A2 | C-A1 | C-A2 | B22 | B22 |
| H8-876 | O | C-A1 | C-A2 | C-A1 | C-A2 | B22 | B32 |
| H8-877 | O | C-A1 | C-A2 | C-A1 | C-A2 | B22 | B33 |
| H8-878 | O | C-A1 | C-A2 | C-A1 | C-A2 | B22 | B34 |
| H8-879 | O | C-A1 | C-A2 | C-A1 | C-A2 | B22 | B68 |
| H8-880 | O | C-A1 | C-A2 | C-A1 | C-A2 | B22 | B69 |
| H8-881 | O | C-A1 | C-A2 | C-A1 | C-A2 | B22 | B70 |
| H8-882 | O | C-A1 | C-A2 | C-A1 | C-A2 | B22 | B71 |
| H8-883 | O | C-A1 | C-A2 | C-A1 | C-A2 | B22 | B81 |
| H8-884 | O | C-A1 | C-A2 | C-A1 | C-A2 | B22 | B90 |
| H8-885 | O | C-A1 | C-A2 | C-A1 | C-A2 | B22 | B91 |
| H8-886 | O | C-A1 | C-A2 | C-A1 | C-A2 | B22 | B92 |
| H8-887 | O | C-A1 | C-A2 | C-A1 | C-A2 | B22 | B93 |
| H8-888 | O | C-A1 | C-A2 | C-A1 | C-A2 | B22 | B94 |
| H8-889 | O | C-A1 | C-A2 | C-A1 | C-A2 | B22 | B95 |
| H8-890 | O | C-A1 | C-A2 | C-A1 | C-A2 | B22 | B96 |
| H8-891 | O | C-A1 | C-A2 | C-A1 | C-A2 | B22 | B97 |
| H8-892 | O | C-A1 | C-A2 | C-A1 | C-A2 | B22 | B106 |
| H8-893 | O | C-A1 | C-A2 | C-A1 | C-A2 | B22 | B107 |
| H8-894 | O | C-A1 | C-A2 | C-A1 | C-A2 | B22 | B114 |
| H8-895 | O | C-A1 | C-A2 | C-A1 | C-A2 | B22 | B115 |
| H8-896 | O | C-A1 | C-A2 | C-A1 | C-A2 | B22 | B116 |
| H8-897 | O | C-A1 | C-A2 | C-A1 | C-A2 | B22 | B117 |
| H8-898 | O | C-A1 | C-A2 | C-A1 | C-A2 | B22 | B118 |
| H8-899 | O | C-A1 | C-A2 | C-A1 | C-A2 | B22 | B119 |
| H8-900 | O | C-A1 | C-A2 | C-A1 | C-A2 | B22 | B 120 |
| H8-901 | O | C-A1 | C-A2 | C-A1 | C-A2 | B22 | B121 |
| H8-902 | O | C-A1 | C-A2 | C-A1 | C-A2 | B22 | B122 |
| H8-903 | O | C-A1 | C-A2 | C-A1 | C-A2 | B22 | B125 |
| H8-904 | O | C-A1 | C-A2 | C-A1 | C-A2 | B22 | B126 |
| H8-905 | O | C-A1 | C-A2 | C-A1 | C-A2 | B22 | B127 |
| H8-906 | O | C-A1 | C-A2 | C-A1 | C-A2 | B22 | B128 |
| H8-907 | O | C-A1 | C-A2 | C-A1 | C-A2 | B22 | B129 |
| H8-908 | O | C-A1 | C-A2 | C-A1 | C-A2 | B22 | B130 |
| H8-909 | O | C-A1 | C-A2 | C-A1 | C-A2 | B22 | B131 |
| H8-910 | O | C-A1 | C-A2 | C-A1 | C-A2 | B22 | B132 |
| H8-911 | O | C-A1 | C-A2 | C-A1 | C-A2 | B22 | B133 |
| H8-912 | O | C-A1 | C-A2 | C-A1 | C-A2 | B22 | B134 |
| H8-913 | O | C-A1 | C-A2 | C-A1 | C-A2 | B22 | B135 |
| H8-914 | O | C-A1 | C-A2 | C-A1 | C-A2 | B22 | B136 |
| H8-915 | O | C-A1 | C-A2 | C-A1 | C-A2 | B22 | B137 |
| H8-916 | O | C-A1 | C-A2 | C-A1 | C-A2 | B22 | B138 |
| H8-917 | O | C-A1 | C-A2 | C-A1 | C-A2 | B22 | B139 |
| H8-918 | O | C-A1 | C-A2 | C-A1 | C-A2 | B22 | B140 |
| H8-925 | O | C-A1 | C-A3 | C-A1 | C-A3 | B22 | B1 |
| H8-926 | O | C-A1 | C-A3 | C-A1 | C-A3 | B22 | B6 |
| H8-927 | O | C-A1 | C-A3 | C-A1 | C-A3 | B22 | B20 |
| H8-928 | O | C-A1 | C-A3 | C-A1 | C-A3 | B22 | B21 |
| H8-929 | O | C-A1 | C-A3 | C-A1 | C-A3 | B22 | B22 |
| H8-930 | O | C-A1 | C-A3 | C-A1 | C-A3 | B22 | B32 |
| H8-931 | O | C-A1 | C-A3 | C-A1 | C-A3 | B22 | B33 |
| H8-932 | O | C-A1 | C-A3 | C-A1 | C-A3 | B22 | B34 |
| H8-933 | O | C-A1 | C-A3 | C-A1 | C-A3 | B22 | B68 |
| H8-934 | O | C-A1 | C-A3 | C-A1 | C-A3 | B22 | B69 |
| H8-935 | O | C-A1 | C-A3 | C-A1 | C-A3 | B22 | B70 |
| H8-936 | O | C-A1 | C-A3 | C-A1 | C-A3 | B22 | B71 |
| H8-937 | O | C-A1 | C-A3 | C-A1 | C-A3 | B22 | B81 |
| H8-938 | O | C-A1 | C-A3 | C-A1 | C-A3 | B22 | B90 |
| H8-939 | O | C-A1 | C-A3 | C-A1 | C-A3 | B22 | B91 |
| H8-940 | O | C-A1 | C-A3 | C-A1 | C-A3 | B22 | B92 |
| H8-941 | O | C-A1 | C-A3 | C-A1 | C-A3 | B22 | B93 |
| H8-942 | O | C-A1 | C-A3 | C-A1 | C-A3 | B22 | B94 |
| H8-943 | O | C-A1 | C-A3 | C-A1 | C-A3 | B22 | B95 |
| H8-944 | O | C-A1 | C-A3 | C-A1 | C-A3 | B22 | B96 |
| H8-945 | O | C-A1 | C-A3 | C-A1 | C-A3 | B22 | B97 |
| H8-946 | O | C-A1 | C-A3 | C-A1 | C-A3 | B22 | B106 |
| H8-947 | O | C-A1 | C-A3 | C-A1 | C-A3 | B22 | B107 |
| H8-948 | O | C-A1 | C-A3 | C-A1 | C-A3 | B22 | B114 |
| H8-949 | O | C-A1 | C-A3 | C-A1 | C-A3 | B22 | B115 |
| H8-950 | O | C-A1 | C-A3 | C-A1 | C-A3 | B22 | B116 |
| H8-951 | O | C-A1 | C-A3 | C-A1 | C-A3 | B22 | B117 |
| H8-952 | O | C-A1 | C-A3 | C-A1 | C-A3 | B22 | B118 |
| H8-953 | O | C-A1 | C-A3 | C-A1 | C-A3 | B22 | B119 |
| H8-954 | O | C-A1 | C-A3 | C-A1 | C-A3 | B22 | B120 |
| H8-955 | O | C-A1 | C-A3 | C-A1 | C-A3 | B22 | B121 |
| H8-956 | O | C-A1 | C-A3 | C-A1 | C-A3 | B22 | B122 |
| H8-957 | O | C-A1 | C-A3 | C-A1 | C-A3 | B22 | B125 |
| H8-958 | O | C-A1 | C-A3 | C-A1 | C-A3 | B22 | B126 |
| H8-959 | O | C-A1 | C-A3 | C-A1 | C-A3 | B22 | B127 |
| H8-960 | O | C-A1 | C-A3 | C-A1 | C-A3 | B22 | B128 |
| H8-961 | O | C-A1 | C-A3 | C-A1 | C-A3 | B22 | B129 |
| H8-962 | O | C-A1 | C-A3 | C-A1 | C-A3 | B22 | B130 |
| H8-963 | O | C-A1 | C-A3 | C-A1 | C-A3 | B22 | B131 |
| H8-964 | O | C-A1 | C-A3 | C-A1 | C-A3 | B22 | B132 |
| H8-965 | O | C-A1 | C-A3 | C-A1 | C-A3 | B22 | B133 |
| H8-966 | O | C-A1 | C-A3 | C-A1 | C-A3 | B22 | B134 |
| H8-967 | O | C-A1 | C-A3 | C-A1 | C-A3 | B22 | B135 |
| H8-968 | O | C-A1 | C-A3 | C-A1 | C-A3 | B22 | B136 |
| H8-969 | O | C-A1 | C-A3 | C-A1 | C-A3 | B22 | B137 |
| H8-970 | O | C-A1 | C-A3 | C-A1 | C-A3 | B22 | B138 |
| H8-971 | O | C-A1 | C-A3 | C-A1 | C-A3 | B22 | B139 |
| H8-972 | O | C-A1 | C-A3 | C-A1 | C-A3 | B22 | B140 |
| H8-979 | O | C-A3 | C-A1 | C-A3 | C-A1 | B22 | B1 |
| H8-980 | O | C-A3 | C-A1 | C-A3 | C-A1 | B22 | B6 |
| H8-981 | O | C-A3 | C-A1 | C-A3 | C-A1 | B22 | B20 |
| H8-982 | O | C-A3 | C-A1 | C-A3 | C-A1 | B22 | B21 |
| H8-983 | O | C-A3 | C-A1 | C-A3 | C-A1 | B22 | B22 |
| H8-984 | O | C-A3 | C-A1 | C-A3 | C-A1 | B22 | B32 |
| H8-985 | O | C-A3 | C-A1 | C-A3 | C-A1 | B22 | B33 |
| H8-986 | O | C-A3 | C-A1 | C-A3 | C-A1 | B22 | B34 |
| H8-987 | O | C-A3 | C-A1 | C-A3 | C-A1 | B22 | B68 |
| H8-988 | O | C-A3 | C-A1 | C-A3 | C-A1 | B22 | B69 |
| H8-989 | O | C-A3 | C-A1 | C-A3 | C-A1 | B22 | B70 |
| H8-990 | O | C-A3 | C-A1 | C-A3 | C-A1 | B22 | B71 |
| H8-991 | O | C-A3 | C-A1 | C-A3 | C-A1 | B22 | B81 |
| H8-992 | O | C-A3 | C-A1 | C-A3 | C-A1 | B22 | B90 |
| H8-993 | O | C-A3 | C-A1 | C-A3 | C-A1 | B22 | B91 |
| H8-994 | O | C-A3 | C-A1 | C-A3 | C-A1 | B22 | B92 |
| H8-995 | O | C-A3 | C-A1 | C-A3 | C-A1 | B22 | B93 |
| H8-996 | O | C-A3 | C-A1 | C-A3 | C-A1 | B22 | B94 |
| H8-997 | O | C-A3 | C-A1 | C-A3 | C-A1 | B22 | B95 |
| H8-998 | O | C-A3 | C-A1 | C-A3 | C-A1 | B22 | B96 |
| H8-999 | O | C-A3 | C-A1 | C-A3 | C-A1 | B22 | B97 |
| H8-1000 | O | C-A3 | C-A1 | C-A3 | C-A1 | B22 | B106 |
| H8-1001 | O | C-A3 | C-A1 | C-A3 | C-A1 | B22 | B107 |
| H8-1002 | O | C-A3 | C-A1 | C-A3 | C-A1 | B22 | B114 |
| H8-1003 | O | C-A3 | C-A1 | C-A3 | C-A1 | B22 | B115 |
| H8-1004 | O | C-A3 | C-A1 | C-A3 | C-A1 | B22 | B116 |
| H8-1005 | O | C-A3 | C-A1 | C-A3 | C-A1 | B22 | B117 |
| H8-1006 | O | C-A3 | C-A1 | C-A3 | C-A1 | B22 | B118 |
| H8-1007 | O | C-A3 | C-A1 | C-A3 | C-A1 | B22 | B119 |
| H8-1008 | O | C-A3 | C-A1 | C-A3 | C-A1 | B22 | B120 |
| H8-1009 | O | C-A3 | C-A1 | C-A3 | C-A1 | B22 | B121 |
| H8-1010 | O | C-A3 | C-A1 | C-A3 | C-A1 | B22 | B122 |
| H8-1011 | O | C-A3 | C-A1 | C-A3 | C-A1 | B22 | B125 |
| H8-1012 | O | C-A3 | C-A1 | C-A3 | C-A1 | B22 | B126 |
| H8-1013 | O | C-A3 | C-A1 | C-A3 | C-A1 | B22 | B127 |
| H8-1014 | O | C-A3 | C-A1 | C-A3 | C-A1 | B22 | B128 |
| H8-1015 | O | C-A3 | C-A1 | C-A3 | C-A1 | B22 | B129 |
| H8-1016 | O | C-A3 | C-A1 | C-A3 | C-A1 | B22 | B130 |
| H8-1017 | O | C-A3 | C-A1 | C-A3 | C-A1 | B22 | B131 |
| H8-1018 | O | C-A3 | C-A1 | C-A3 | C-A1 | B22 | B132 |
| H8-1019 | O | C-A3 | C-A1 | C-A3 | C-A1 | B22 | B133 |
| H8-1020 | O | C-A3 | C-A1 | C-A3 | C-A1 | B22 | B134 |
| H8-1021 | O | C-A3 | C-A1 | C-A3 | C-A1 | B22 | B135 |
| H8-1022 | O | C-A3 | C-A1 | C-A3 | C-A1 | B22 | B136 |
| H8-1023 | O | C-A3 | C-A1 | C-A3 | C-A1 | B22 | B137 |
| H8-1024 | O | C-A3 | C-A1 | C-A3 | C-A1 | B22 | B138 |
| H8-1025 | O | C-A3 | C-A1 | C-A3 | C-A1 | B22 | B139 |
| H8-1026 | O | C-A3 | C-A1 | C-A3 | C-A1 | B22 | B140 |
| H8-1033 | O | C-A1 | C-A1 | C-A3 | C-A1 | B22 | B1 |
| H8-1034 | O | C-A1 | C-A1 | C-A3 | C-A1 | B22 | B6 |
| H8-1035 | O | C-A1 | C-A1 | C-A3 | C-A1 | B22 | B20 |
| H8-1036 | O | C-A1 | C-A1 | C-A3 | C-A1 | B22 | B21 |
| H8-1037 | O | C-A1 | C-A1 | C-A3 | C-A1 | B22 | B22 |
| H8-1038 | O | C-A1 | C-A1 | C-A3 | C-A1 | B22 | B32 |
| H8-1039 | O | C-A1 | C-A1 | C-A3 | C-A1 | B22 | B33 |
| H8-1040 | O | C-A1 | C-A1 | C-A3 | C-A1 | B22 | B34 |
| H8-1041 | O | C-A1 | C-A1 | C-A3 | C-A1 | B22 | B68 |
| H8-1042 | O | C-A1 | C-A1 | C-A3 | C-A1 | B22 | B69 |
| H8-1043 | O | C-A1 | C-A1 | C-A3 | C-A1 | B22 | B70 |
| H8-1044 | O | C-A1 | C-A1 | C-A3 | C-A1 | B22 | B71 |
| H8-1045 | O | C-A1 | C-A1 | C-A3 | C-A1 | B22 | B81 |
| H8-1046 | O | C-A1 | C-A1 | C-A3 | C-A1 | B22 | B90 |
| H8-1047 | O | C-A1 | C-A1 | C-A3 | C-A1 | B22 | B91 |
| H8-1048 | O | C-A1 | C-A1 | C-A3 | C-A1 | B22 | B92 |
| H8-1049 | O | C-A1 | C-A1 | C-A3 | C-A1 | B22 | B93 |
| H8-1050 | O | C-A1 | C-A1 | C-A3 | C-A1 | B22 | B94 |
| H8-1051 | O | C-A1 | C-A1 | C-A3 | C-A1 | B22 | B95 |
| H8-1052 | O | C-A1 | C-A1 | C-A3 | C-A1 | B22 | B96 |
| H8-1053 | O | C-A1 | C-A1 | C-A3 | C-A1 | B22 | B97 |
| H8-1054 | O | C-A1 | C-A1 | C-A3 | C-A1 | B22 | B106 |
| H8-1055 | O | C-A1 | C-A1 | C-A3 | C-A1 | B22 | B107 |
| H8-1056 | O | C-A1 | C-A1 | C-A3 | C-A1 | B22 | B114 |
| H8-1057 | O | C-A1 | C-A1 | C-A3 | C-A1 | B22 | B115 |
| H8-1058 | O | C-A1 | C-A1 | C-A3 | C-A1 | B22 | B116 |
| H8-1059 | O | C-A1 | C-A1 | C-A3 | C-A1 | B22 | B117 |
| H8-1060 | O | C-A1 | C-A1 | C-A3 | C-A1 | B22 | B118 |
| H8-1061 | O | C-A1 | C-A1 | C-A3 | C-A1 | B22 | B119 |
| H8-1062 | O | C-A1 | C-A1 | C-A3 | C-A1 | B22 | B120 |
| H8-1063 | O | C-A1 | C-A1 | C-A3 | C-A1 | B22 | B121 |
| H8-1064 | O | C-A1 | C-A1 | C-A3 | C-A1 | B22 | B122 |
| H8-1065 | O | C-A1 | C-A1 | C-A3 | C-A1 | B22 | B125 |
| H8-1066 | O | C-A1 | C-A1 | C-A3 | C-A1 | B22 | B126 |
| H8-1067 | O | C-A1 | C-A1 | C-A3 | C-A1 | B22 | B127 |
| H8-1068 | O | C-A1 | C-A1 | C-A3 | C-A1 | B22 | B128 |
| H8-1069 | O | C-A1 | C-A1 | C-A3 | C-A1 | B22 | B129 |
| H8-1070 | O | C-A1 | C-A1 | C-A3 | C-A1 | B22 | B130 |
| H8-1071 | O | C-A1 | C-A1 | C-A3 | C-A1 | B22 | B131 |
| H8-1072 | O | C-A1 | C-A1 | C-A3 | C-A1 | B22 | B132 |
| H8-1073 | O | C-A1 | C-A1 | C-A3 | C-A1 | B22 | B133 |
| H8-1074 | O | C-A1 | C-A1 | C-A3 | C-A1 | B22 | B134 |
| H8-1075 | O | C-A1 | C-A1 | C-A3 | C-A1 | B22 | B135 |
| H8-1076 | O | C-A1 | C-A1 | C-A3 | C-A1 | B22 | B136 |
| H8-1077 | O | C-A1 | C-A1 | C-A3 | C-A1 | B22 | B137 |
| H8-1078 | O | C-A1 | C-A1 | C-A3 | C-A1 | B22 | B138 |
| H8-1079 | O | C-A1 | C-A1 | C-A3 | C-A1 | B22 | B139 |
| H8-1080 | O | C-A1 | C-A1 | C-A3 | C-A1 | B22 | B140 |
| H8-1087 | O | C-A1 | C-A1 | C-A1 | C-A1 | B32 | B1 |
| H8-1088 | O | C-A1 | C-A1 | C-A1 | C-A1 | B32 | B6 |
| H8-1089 | O | C-A1 | C-A1 | C-A1 | C-A1 | B32 | B20 |
| H8-1090 | O | C-A1 | C-A1 | C-A1 | C-A1 | B32 | B21 |
| H8-1091 | O | C-A1 | C-A1 | C-A1 | C-A1 | B32 | B22 |
| H8-1092 | O | C-A1 | C-A1 | C-A1 | C-A1 | B32 | B32 |
| H8-1093 | O | C-A1 | C-A1 | C-A1 | C-A1 | B32 | B33 |
| H8-1094 | O | C-A1 | C-A1 | C-A1 | C-A1 | B32 | B34 |
| H8-1095 | O | C-A1 | C-A1 | C-A1 | C-A1 | B32 | B68 |
| H8-1096 | O | C-A1 | C-A1 | C-A1 | C-A1 | B32 | B69 |
| H8-1097 | O | C-A1 | C-A1 | C-A1 | C-A1 | B32 | B70 |
| H8-1098 | O | C-A1 | C-A1 | C-A1 | C-A1 | B32 | B71 |
| H8-1099 | O | C-A1 | C-A1 | C-A1 | C-A1 | B32 | B81 |
| H8-1100 | O | C-A1 | C-A1 | C-A1 | C-A1 | B32 | B90 |
| H8-1101 | O | C-A1 | C-A1 | C-A1 | C-A1 | B32 | B91 |
| H8-1102 | O | C-A1 | C-A1 | C-A1 | C-A1 | B32 | B92 |
| H8-1103 | O | C-A1 | C-A1 | C-A1 | C-A1 | B32 | B93 |
| H8-1104 | O | C-A1 | C-A1 | C-A1 | C-A1 | B32 | B94 |
| H8-1105 | O | C-A1 | C-A1 | C-A1 | C-A1 | B32 | B95 |
| H8-1106 | O | C-A1 | C-A1 | C-A1 | C-A1 | B32 | B96 |
| H8-1107 | O | C-A1 | C-A1 | C-A1 | C-A1 | B32 | B97 |
| H8-1108 | O | C-A1 | C-A1 | C-A1 | C-A1 | B32 | B106 |
| H8-1109 | O | C-A1 | C-A1 | C-A1 | C-A1 | B32 | B107 |
| H8-1110 | O | C-A1 | C-A1 | C-A1 | C-A1 | B32 | B114 |
| H8-1111 | O | C-A1 | C-A1 | C-A1 | C-A1 | B32 | B115 |
| H8-1112 | O | C-A1 | C-A1 | C-A1 | C-A1 | B32 | B116 |
| H8-1113 | O | C-A1 | C-A1 | C-A1 | C-A1 | B32 | B117 |
| H8-1114 | O | C-A1 | C-A1 | C-A1 | C-A1 | B32 | B118 |
| H8-1115 | O | C-A1 | C-A1 | C-A1 | C-A1 | B32 | B119 |
| H8-1116 | O | C-A1 | C-A1 | C-A1 | C-A1 | B32 | B120 |
| H8-1117 | O | C-A1 | C-A1 | C-A1 | C-A1 | B32 | B121 |
| H8-1118 | O | C-A1 | C-A1 | C-A1 | C-A1 | B32 | B122 |
| H8-1119 | O | C-A1 | C-A1 | C-A1 | C-A1 | B32 | B125 |
| H8-1120 | O | C-A1 | C-A1 | C-A1 | C-A1 | B32 | B126 |
| H8-1121 | O | C-A1 | C-A1 | C-A1 | C-A1 | B32 | B127 |
| H8-1122 | O | C-A1 | C-A1 | C-A1 | C-A1 | B32 | B128 |
| H8-1123 | O | C-A1 | C-A1 | C-A1 | C-A1 | B32 | B129 |
| H8-1124 | O | C-A1 | C-A1 | C-A1 | C-A1 | B32 | B130 |
| H8-1125 | O | C-A1 | C-A1 | C-A1 | C-A1 | B32 | B131 |
| H8-1126 | O | C-A1 | C-A1 | C-A1 | C-A1 | B32 | B132 |
| H8-1127 | O | C-A1 | C-A1 | C-A1 | C-A1 | B32 | B133 |
| H8-1128 | O | C-A1 | C-A1 | C-A1 | C-A1 | B32 | B134 |
| H8-1129 | O | C-A1 | C-A1 | C-A1 | C-A1 | B32 | B135 |
| H8-1130 | O | C-A1 | C-A1 | C-A1 | C-A1 | B32 | B136 |
| H8-1131 | O | C-A1 | C-A1 | C-A1 | C-A1 | B32 | B137 |
| H8-1132 | O | C-A1 | C-A1 | C-A1 | C-A1 | B32 | B138 |
| H8-1133 | O | C-A1 | C-A1 | C-A1 | C-A1 | B32 | B139 |
| H8-1134 | O | C-A1 | C-A1 | C-A1 | C-A1 | B32 | B140 |
| H8-1141 | O | C-A1 | C-A2 | C-A1 | C-A2 | B32 | B1 |
| H8-1142 | O | C-A1 | C-A2 | C-A1 | C-A2 | B32 | B6 |
| H8-1143 | O | C-A1 | C-A2 | C-A1 | C-A2 | B32 | B20 |
| H8-1144 | O | C-A1 | C-A2 | C-A1 | C-A2 | B32 | B21 |
| H8-1145 | O | C-A1 | C-A2 | C-A1 | C-A2 | B32 | B22 |
| H8-1146 | O | C-A1 | C-A2 | C-A1 | C-A2 | B32 | B32 |
| H8-1147 | O | C-A1 | C-A2 | C-A1 | C-A2 | B32 | B33 |
| H8-1148 | O | C-A1 | C-A2 | C-A1 | C-A2 | B32 | B34 |
| H8-1149 | O | C-A1 | C-A2 | C-A1 | C-A2 | B32 | B68 |
| H8-1150 | O | C-A1 | C-A2 | C-A1 | C-A2 | B32 | B69 |
| H8-1151 | O | C-A1 | C-A2 | C-A1 | C-A2 | B32 | B70 |
| H8-1152 | O | C-A1 | C-A2 | C-A1 | C-A2 | B32 | B71 |
| H8-1153 | O | C-A1 | C-A2 | C-A1 | C-A2 | B32 | B81 |
| H8-1154 | O | C-A1 | C-A2 | C-A1 | C-A2 | B32 | B90 |
| H8-1155 | O | C-A1 | C-A2 | C-A1 | C-A2 | B32 | B91 |
| H8-1156 | O | C-A1 | C-A2 | C-A1 | C-A2 | B32 | B92 |
| H8-1157 | O | C-A1 | C-A2 | C-A1 | C-A2 | B32 | B93 |
| H8-1158 | O | C-A1 | C-A2 | C-A1 | C-A2 | B32 | B94 |
| H8-1159 | O | C-A1 | C-A2 | C-A1 | C-A2 | B32 | B95 |
| H8-1160 | O | C-A1 | C-A2 | C-A1 | C-A2 | B32 | B96 |
| H8-1161 | O | C-A1 | C-A2 | C-A1 | C-A2 | B32 | B97 |
| H8-1162 | O | C-A1 | C-A2 | C-A1 | C-A2 | B32 | B106 |
| H8-1163 | O | C-A1 | C-A2 | C-A1 | C-A2 | B32 | B107 |
| H8-1164 | O | C-A1 | C-A2 | C-A1 | C-A2 | B32 | B114 |
| H8-1165 | O | C-A1 | C-A2 | C-A1 | C-A2 | B32 | B115 |
| H8-1166 | O | C-A1 | C-A2 | C-A1 | C-A2 | B32 | B116 |
| H8-1167 | O | C-A1 | C-A2 | C-A1 | C-A2 | B32 | B117 |
| H8-1168 | O | C-A1 | C-A2 | C-A1 | C-A2 | B32 | B118 |
| H8-1169 | O | C-A1 | C-A2 | C-A1 | C-A2 | B32 | B119 |
| H8-1170 | O | C-A1 | C-A2 | C-A1 | C-A2 | B32 | B120 |
| H8-1171 | O | C-A1 | C-A2 | C-A1 | C-A2 | B32 | B121 |
| H8-1172 | O | C-A1 | C-A2 | C-A1 | C-A2 | B32 | B122 |
| H8-1173 | O | C-A1 | C-A2 | C-A1 | C-A2 | B32 | B125 |
| H8-1174 | O | C-A1 | C-A2 | C-A1 | C-A2 | B32 | B126 |
| H8-1175 | O | C-A1 | C-A2 | C-A1 | C-A2 | B32 | B127 |
| H8-1176 | O | C-A1 | C-A2 | C-A1 | C-A2 | B32 | B128 |
| H8-1177 | O | C-A1 | C-A2 | C-A1 | C-A2 | B32 | B129 |
| H8-1178 | O | C-A1 | C-A2 | C-A1 | C-A2 | B32 | B130 |
| H8-1179 | O | C-A1 | C-A2 | C-A1 | C-A2 | B32 | B131 |
| H8-1180 | O | C-A1 | C-A2 | C-A1 | C-A2 | B32 | B132 |
| H8-1181 | O | C-A1 | C-A2 | C-A1 | C-A2 | B32 | B133 |
| H8-1182 | O | C-A1 | C-A2 | C-A1 | C-A2 | B32 | B134 |
| H8-1183 | O | C-A1 | C-A2 | C-A1 | C-A2 | B32 | B135 |
| H8-1184 | O | C-A1 | C-A2 | C-A1 | C-A2 | B32 | B136 |
| H8-1185 | O | C-A1 | C-A2 | C-A1 | C-A2 | B32 | B137 |
| H8-1186 | O | C-A1 | C-A2 | C-A1 | C-A2 | B32 | B138 |
| H8-1187 | O | C-A1 | C-A2 | C-A1 | C-A2 | B32 | B139 |
| H8-1188 | O | C-A1 | C-A2 | C-A1 | C-A2 | B32 | B140 |
| H8-1195 | O | C-A1 | C-A3 | C-A1 | C-A3 | B32 | B1 |
| H8-1196 | O | C-A1 | C-A3 | C-A1 | C-A3 | B32 | B6 |
| H8-1197 | O | C-A1 | C-A3 | C-A1 | C-A3 | B32 | B20 |
| H8-1198 | O | C-A1 | C-A3 | C-A1 | C-A3 | B32 | B21 |
| H8-1199 | O | C-A1 | C-A3 | C-A1 | C-A3 | B32 | B22 |
| H8-1200 | O | C-A1 | C-A3 | C-A1 | C-A3 | B32 | B32 |
| H8-1201 | O | C-A1 | C-A3 | C-A1 | C-A3 | B32 | B33 |
| H8-1202 | O | C-A1 | C-A3 | C-A1 | C-A3 | B32 | B34 |
| H8-1203 | O | C-A1 | C-A3 | C-A1 | C-A3 | B32 | B68 |
| H8-1204 | O | C-A1 | C-A3 | C-A1 | C-A3 | B32 | B69 |
| H8-1205 | O | C-A1 | C-A3 | C-A1 | C-A3 | B32 | B70 |
| H8-1206 | O | C-A1 | C-A3 | C-A1 | C-A3 | B32 | B71 |
| H8-1207 | O | C-A1 | C-A3 | C-A1 | C-A3 | B32 | B81 |
| H8-1208 | O | C-A1 | C-A3 | C-A1 | C-A3 | B32 | B90 |
| H8-1209 | O | C-A1 | C-A3 | C-A1 | C-A3 | B32 | B91 |
| H8-1210 | O | C-A1 | C-A3 | C-A1 | C-A3 | B32 | B92 |
| H8-1211 | O | C-A1 | C-A3 | C-A1 | C-A3 | B32 | B93 |
| H8-1212 | O | C-A1 | C-A3 | C-A1 | C-A3 | B32 | B94 |
| H8-1213 | O | C-A1 | C-A3 | C-A1 | C-A3 | B32 | B95 |
| H8-1214 | O | C-A1 | C-A3 | C-A1 | C-A3 | B32 | B96 |
| H8-1215 | O | C-A1 | C-A3 | C-A1 | C-A3 | B32 | B97 |
| H8-1216 | O | C-A1 | C-A3 | C-A1 | C-A3 | B32 | B106 |
| H8-1217 | O | C-A1 | C-A3 | C-A1 | C-A3 | B32 | B107 |
| H8-1218 | O | C-A1 | C-A3 | C-A1 | C-A3 | B32 | B114 |
| H8-1219 | O | C-A1 | C-A3 | C-A1 | C-A3 | B32 | B115 |
| H8-1220 | O | C-A1 | C-A3 | C-A1 | C-A3 | B32 | B116 |
| H8-1221 | O | C-A1 | C-A3 | C-A1 | C-A3 | B32 | B117 |
| H8-1222 | O | C-A1 | C-A3 | C-A1 | C-A3 | B32 | B118 |
| H8-1223 | O | C-A1 | C-A3 | C-A1 | C-A3 | B32 | B119 |
| H8-1224 | O | C-A1 | C-A3 | C-A1 | C-A3 | B32 | B120 |
| H8-1225 | O | C-A1 | C-A3 | C-A1 | C-A3 | B32 | B121 |
| H8-1226 | O | C-A1 | C-A3 | C-A1 | C-A3 | B32 | B122 |
| H8-1227 | O | C-A1 | C-A3 | C-A1 | C-A3 | B32 | B125 |
| H8-1228 | O | C-A1 | C-A3 | C-A1 | C-A3 | B32 | B126 |
| H8-1229 | O | C-A1 | C-A3 | C-A1 | C-A3 | B32 | B127 |
| H8-1230 | O | C-A1 | C-A3 | C-A1 | C-A3 | B32 | B128 |
| H8-1231 | O | C-A1 | C-A3 | C-A1 | C-A3 | B32 | B129 |
| H8-1232 | O | C-A1 | C-A3 | C-A1 | C-A3 | B32 | B130 |
| H8-1233 | O | C-A1 | C-A3 | C-A1 | C-A3 | B32 | B131 |
| H8-1234 | O | C-A1 | C-A3 | C-A1 | C-A3 | B32 | B132 |
| H8-1235 | O | C-A1 | C-A3 | C-A1 | C-A3 | B32 | B133 |
| H8-1236 | O | C-A1 | C-A3 | C-A1 | C-A3 | B32 | B134 |
| H8-1237 | O | C-A1 | C-A3 | C-A1 | C-A3 | B32 | B135 |
| H8-1238 | O | C-A1 | C-A3 | C-A1 | C-A3 | B32 | B136 |
| H8-1239 | O | C-A1 | C-A3 | C-A1 | C-A3 | B32 | B137 |
| H8-1240 | O | C-A1 | C-A3 | C-A1 | C-A3 | B32 | B138 |
| H8-1241 | O | C-A1 | C-A3 | C-A1 | C-A3 | B32 | B139 |
| H8-1242 | O | C-A1 | C-A3 | C-A1 | C-A3 | B32 | B140 |
| H8-1249 | O | C-A3 | C-A1 | C-A3 | C-A1 | B32 | B1 |
| H8-1250 | O | C-A3 | C-A1 | C-A3 | C-A1 | B32 | B6 |
| H8-1251 | O | C-A3 | C-A1 | C-A3 | C-A1 | B32 | B20 |
| H8-1252 | O | C-A3 | C-A1 | C-A3 | C-A1 | B32 | B21 |
| H8-1253 | O | C-A3 | C-A1 | C-A3 | C-A1 | B32 | B22 |
| H8-1254 | O | C-A3 | C-A1 | C-A3 | C-A1 | B32 | B32 |
| H8-1255 | O | C-A3 | C-A1 | C-A3 | C-A1 | B32 | B33 |
| H8-1256 | O | C-A3 | C-A1 | C-A3 | C-A1 | B32 | B34 |
| H8-1257 | O | C-A3 | C-A1 | C-A3 | C-A1 | B32 | B68 |
| H8-1258 | O | C-A3 | C-A1 | C-A3 | C-A1 | B32 | B69 |
| H8-1259 | O | C-A3 | C-A1 | C-A3 | C-A1 | B32 | B70 |
| H8-1260 | O | C-A3 | C-A1 | C-A3 | C-A1 | B32 | B71 |
| H8-1261 | O | C-A3 | C-A1 | C-A3 | C-A1 | B32 | B81 |
| H8-1262 | O | C-A3 | C-A1 | C-A3 | C-A1 | B32 | B90 |
| H8-1263 | O | C-A3 | C-A1 | C-A3 | C-A1 | B32 | B91 |
| H8-1264 | O | C-A3 | C-A1 | C-A3 | C-A1 | B32 | B92 |
| H8-1265 | O | C-A3 | C-A1 | C-A3 | C-A1 | B32 | B93 |
| H8-1266 | O | C-A3 | C-A1 | C-A3 | C-A1 | B32 | B94 |
| H8-1267 | O | C-A3 | C-A1 | C-A3 | C-A1 | B32 | B95 |
| H8-1268 | O | C-A3 | C-A1 | C-A3 | C-A1 | B32 | B96 |
| H8-1269 | O | C-A3 | C-A1 | C-A3 | C-A1 | B32 | B97 |
| H8-1270 | O | C-A3 | C-A1 | C-A3 | C-A1 | B32 | B106 |
| H8-1271 | O | C-A3 | C-A1 | C-A3 | C-A1 | B32 | B107 |
| H8-1272 | O | C-A3 | C-A1 | C-A3 | C-A1 | B32 | B114 |
| H8-1273 | O | C-A3 | C-A1 | C-A3 | C-A1 | B32 | B115 |
| H8-1274 | O | C-A3 | C-A1 | C-A3 | C-A1 | B32 | B116 |
| H8-1275 | O | C-A3 | C-A1 | C-A3 | C-A1 | B32 | B117 |
| H8-1276 | O | C-A3 | C-A1 | C-A3 | C-A1 | B32 | B118 |
| H8-1277 | O | C-A3 | C-A1 | C-A3 | C-A1 | B32 | B119 |
| H8-1278 | O | C-A3 | C-A1 | C-A3 | C-A1 | B32 | B120 |
| H8-1279 | O | C-A3 | C-A1 | C-A3 | C-A1 | B32 | B121 |
| H8-1280 | O | C-A3 | C-A1 | C-A3 | C-A1 | B32 | B122 |
| H8-1281 | O | C-A3 | C-A1 | C-A3 | C-A1 | B32 | B125 |
| H8-1282 | O | C-A3 | C-A1 | C-A3 | C-A1 | B32 | B126 |
| H8-1283 | O | C-A3 | C-A1 | C-A3 | C-A1 | B32 | B127 |
| H8-1284 | O | C-A3 | C-A1 | C-A3 | C-A1 | B32 | B128 |
| H8-1285 | O | C-A3 | C-A1 | C-A3 | C-A1 | B32 | B129 |
| H8-1286 | O | C-A3 | C-A1 | C-A3 | C-A1 | B32 | B130 |
| H8-1287 | O | C-A3 | C-A1 | C-A3 | C-A1 | B32 | B131 |
| H8-1288 | O | C-A3 | C-A1 | C-A3 | C-A1 | B32 | B132 |
| H8-1289 | O | C-A3 | C-A1 | C-A3 | C-A1 | B32 | B133 |
| H8-1290 | O | C-A3 | C-A1 | C-A3 | C-A1 | B32 | B134 |
| H8-1291 | O | C-A3 | C-A1 | C-A3 | C-A1 | B32 | B135 |
| H8-1292 | O | C-A3 | C-A1 | C-A3 | C-A1 | B32 | B136 |
| H8-1293 | O | C-A3 | C-A1 | C-A3 | C-A1 | B32 | B137 |
| H8-1294 | O | C-A3 | C-A1 | C-A3 | C-A1 | B32 | B138 |
| H8-1295 | O | C-A3 | C-A1 | C-A3 | C-A1 | B32 | B139 |
| H8-1296 | O | C-A3 | C-A1 | C-A3 | C-A1 | B32 | B140 |
| H8-1303 | O | C-A1 | C-A1 | C-A3 | C-A1 | B32 | B1 |
| H8-1304 | O | C-A1 | C-A1 | C-A3 | C-A1 | B32 | B6 |
| H8-1305 | O | C-A1 | C-A1 | C-A3 | C-A1 | B32 | B20 |
| H8-1306 | O | C-A1 | C-A1 | C-A3 | C-A1 | B32 | B21 |
| H8-1307 | O | C-A1 | C-A1 | C-A3 | C-A1 | B32 | B22 |
| H8-1308 | O | C-A1 | C-A1 | C-A3 | C-A1 | B32 | B32 |
| H8-1309 | O | C-A1 | C-A1 | C-A3 | C-A1 | B32 | B33 |
| H8-1310 | O | C-A1 | C-A1 | C-A3 | C-A1 | B32 | B34 |
| H8-1311 | O | C-A1 | C-A1 | C-A3 | C-A1 | B32 | B68 |
| H8-1312 | O | C-A1 | C-A1 | C-A3 | C-A1 | B32 | B69 |
| H8-1313 | O | C-A1 | C-A1 | C-A3 | C-A1 | B32 | B70 |
| H8-1314 | O | C-A1 | C-A1 | C-A3 | C-A1 | B32 | B71 |
| H8-1315 | O | C-A1 | C-A1 | C-A3 | C-A1 | B32 | B81 |
| H8-1316 | O | C-A1 | C-A1 | C-A3 | C-A1 | B32 | B90 |
| H8-1317 | O | C-A1 | C-A1 | C-A3 | C-A1 | B32 | B91 |
| H8-1318 | O | C-A1 | C-A1 | C-A3 | C-A1 | B32 | B92 |
| H8-1319 | O | C-A1 | C-A1 | C-A3 | C-A1 | B32 | B93 |
| H8-1320 | O | C-A1 | C-A1 | C-A3 | C-A1 | B32 | B94 |
| H8-1321 | O | C-A1 | C-A1 | C-A3 | C-A1 | B32 | B95 |
| H8-1322 | O | C-A1 | C-A1 | C-A3 | C-A1 | B32 | B96 |
| H8-1323 | O | C-A1 | C-A1 | C-A3 | C-A1 | B32 | B97 |
| H8-1324 | O | C-A1 | C-A1 | C-A3 | C-A1 | B32 | B106 |
| H8-1325 | O | C-A1 | C-A1 | C-A3 | C-A1 | B32 | B107 |
| H8-1326 | O | C-A1 | C-A1 | C-A3 | C-A1 | B32 | B114 |
| H8-1327 | O | C-A1 | C-A1 | C-A3 | C-A1 | B32 | B115 |
| H8-1328 | O | C-A1 | C-A1 | C-A3 | C-A1 | B32 | B116 |
| H8-1329 | O | C-A1 | C-A1 | C-A3 | C-A1 | B32 | B117 |
| H8-1330 | O | C-A1 | C-A1 | C-A3 | C-A1 | B32 | B118 |
| H8-1331 | O | C-A1 | C-A1 | C-A3 | C-A1 | B32 | B119 |
| H8-1332 | O | C-A1 | C-A1 | C-A3 | C-A1 | B32 | B120 |
| H8-1333 | O | C-A1 | C-A1 | C-A3 | C-A1 | B32 | B121 |
| H8-1334 | O | C-A1 | C-A1 | C-A3 | C-A1 | B32 | B122 |
| H8-1335 | O | C-A1 | C-A1 | C-A3 | C-A1 | B32 | B125 |
| H8-1336 | O | C-A1 | C-A1 | C-A3 | C-A1 | B32 | B126 |
| H8-1337 | O | C-A1 | C-A1 | C-A3 | C-A1 | B32 | B127 |
| H8-1338 | O | C-A1 | C-A1 | C-A3 | C-A1 | B32 | B128 |
| H8-1339 | O | C-A1 | C-A1 | C-A3 | C-A1 | B32 | B129 |
| H8-1340 | O | C-A1 | C-A1 | C-A3 | C-A1 | B32 | B130 |
| H8-1341 | O | C-A1 | C-A1 | C-A3 | C-A1 | B32 | B131 |
| H8-1342 | O | C-A1 | C-A1 | C-A3 | C-A1 | B32 | B132 |
| H8-1343 | O | C-A1 | C-A1 | C-A3 | C-A1 | B32 | B133 |
| H8-1344 | O | C-A1 | C-A1 | C-A3 | C-A1 | B32 | B134 |
| H8-1345 | O | C-A1 | C-A1 | C-A3 | C-A1 | B32 | B135 |
| H8-1346 | O | C-A1 | C-A1 | C-A3 | C-A1 | B32 | B136 |
| H8-1347 | O | C-A1 | C-A1 | C-A3 | C-A1 | B32 | B137 |
| H8-1348 | O | C-A1 | C-A1 | C-A3 | C-A1 | B32 | B138 |
| H8-1349 | O | C-A1 | C-A1 | C-A3 | C-A1 | B32 | B139 |
| H8-1350 | O | C-A1 | C-A1 | C-A3 | C-A1 | B32 | B140 |
| H8-1357 | S | C-A1 | C-A1 | C-A1 | C-A1 | B20 | B1 |
| H8-1358 | S | C-A1 | C-A1 | C-A1 | C-A1 | B20 | B6 |
| H8-1359 | S | C-A1 | C-A1 | C-A1 | C-A1 | B20 | B20 |
| H8-1360 | S | C-A1 | C-A1 | C-A1 | C-A1 | B20 | B21 |
| H8-1361 | S | C-A1 | C-A1 | C-A1 | C-A1 | B20 | B22 |
| H8-1362 | S | C-A1 | C-A1 | C-A1 | C-A1 | B20 | B32 |
| H8-1363 | S | C-A1 | C-A1 | C-A1 | C-A1 | B20 | B33 |
| H8-1364 | S | C-A1 | C-A1 | C-A1 | C-A1 | B20 | B34 |
| H8-1365 | S | C-A1 | C-A1 | C-A1 | C-A1 | B20 | B68 |
| H8-1366 | S | C-A1 | C-A1 | C-A1 | C-A1 | B20 | B69 |
| H8-1367 | S | C-A1 | C-A1 | C-A1 | C-A1 | B20 | B70 |
| H8-1368 | S | C-A1 | C-A1 | C-A1 | C-A1 | B20 | B71 |
| H8-1369 | S | C-A1 | C-A1 | C-A1 | C-A1 | B20 | B81 |
| H8-1370 | S | C-A1 | C-A1 | C-A1 | C-A1 | B20 | B90 |
| H8-1371 | S | C-A1 | C-A1 | C-A1 | C-A1 | B20 | B91 |
| H8-1372 | S | C-A1 | C-A1 | C-A1 | C-A1 | B20 | B92 |
| H8-1373 | S | C-A1 | C-A1 | C-A1 | C-A1 | B20 | B93 |
| H8-1374 | S | C-A1 | C-A1 | C-A1 | C-A1 | B20 | B94 |
| H8-1375 | S | C-A1 | C-A1 | C-A1 | C-A1 | B20 | B95 |
| H8-1376 | S | C-A1 | C-A1 | C-A1 | C-A1 | B20 | B96 |
| H8-1377 | S | C-A1 | C-A1 | C-A1 | C-A1 | B20 | B97 |
| H8-1378 | S | C-A1 | C-A1 | C-A1 | C-A1 | B20 | B106 |
| H8-1379 | S | C-A1 | C-A1 | C-A1 | C-A1 | B20 | B107 |
| H8-1380 | S | C-A1 | C-A1 | C-A1 | C-A1 | B20 | B114 |
| H8-1381 | S | C-A1 | C-A1 | C-A1 | C-A1 | B20 | B115 |
| H8-1382 | S | C-A1 | C-A1 | C-A1 | C-A1 | B20 | B116 |
| H8-1383 | S | C-A1 | C-A1 | C-A1 | C-A1 | B20 | B117 |
| H8-1384 | S | C-A1 | C-A1 | C-A1 | C-A1 | B20 | B118 |
| H8-1385 | S | C-A1 | C-A1 | C-A1 | C-A1 | B20 | B119 |
| H8-1386 | S | C-A1 | C-A1 | C-A1 | C-A1 | B20 | B120 |
| H8-1387 | S | C-A1 | C-A1 | C-A1 | C-A1 | B20 | B121 |
| H8-1388 | S | C-A1 | C-A1 | C-A1 | C-A1 | B20 | B122 |
| H8-1389 | S | C-A1 | C-A1 | C-A1 | C-A1 | B20 | B125 |
| H8-1390 | S | C-A1 | C-A1 | C-A1 | C-A1 | B20 | B126 |
| H8-1391 | S | C-A1 | C-A1 | C-A1 | C-A1 | B20 | B127 |
| H8-1392 | S | C-A1 | C-A1 | C-A1 | C-A1 | B20 | B128 |
| H8-1393 | S | C-A1 | C-A1 | C-A1 | C-A1 | B20 | B129 |
| H8-1394 | S | C-A1 | C-A1 | C-A1 | C-A1 | B20 | B130 |
| H8-1395 | S | C-A1 | C-A1 | C-A1 | C-A1 | B20 | B131 |
| H8-1396 | S | C-A1 | C-A1 | C-A1 | C-A1 | B20 | B132 |
| H8-1397 | S | C-A1 | C-A1 | C-A1 | C-A1 | B20 | B133 |
| H8-1398 | S | C-A1 | C-A1 | C-A1 | C-A1 | B20 | B134 |
| H8-1399 | S | C-A1 | C-A1 | C-A1 | C-A1 | B20 | B135 |
| H8-1400 | S | C-A1 | C-A1 | C-A1 | C-A1 | B20 | B136 |
| H8-1401 | S | C-A1 | C-A1 | C-A1 | C-A1 | B20 | B137 |
| H8-1402 | S | C-A1 | C-A1 | C-A1 | C-A1 | B20 | B138 |
| H8-1403 | S | C-A1 | C-A1 | C-A1 | C-A1 | B20 | B139 |
| H8-1404 | S | C-A1 | C-A1 | C-A1 | C-A1 | B20 | B140 |
| H8-1411 | S | C-A1 | C-A1 | C-A1 | C-A1 | B68 | B1 |
| H8-1412 | S | C-A1 | C-A1 | C-A1 | C-A1 | B68 | B6 |
| H8-1413 | S | C-A1 | C-A1 | C-A1 | C-A1 | B68 | B20 |
| H8-1414 | S | C-A1 | C-A1 | C-A1 | C-A1 | B68 | B21 |
| H8-1415 | S | C-A1 | C-A1 | C-A1 | C-A1 | B68 | B22 |
| H8-1416 | S | C-A1 | C-A1 | C-A1 | C-A1 | B68 | B32 |
| H8-1417 | S | C-A1 | C-A1 | C-A1 | C-A1 | B68 | B33 |
| H8-1418 | S | C-A1 | C-A1 | C-A1 | C-A1 | B68 | B34 |
| H8-1419 | S | C-A1 | C-A1 | C-A1 | C-A1 | B68 | B68 |
| H8-1420 | S | C-A1 | C-A1 | C-A1 | C-A1 | B68 | B69 |
| H8-1421 | S | C-A1 | C-A1 | C-A1 | C-A1 | B68 | B70 |
| H8-1422 | S | C-A1 | C-A1 | C-A1 | C-A1 | B68 | B71 |
| H8-1423 | S | C-A1 | C-A1 | C-A1 | C-A1 | B68 | B81 |
| H8-1424 | S | C-A1 | C-A1 | C-A1 | C-A1 | B68 | B90 |
| H8-1425 | S | C-A1 | C-A1 | C-A1 | C-A1 | B68 | B91 |
| H8-1426 | S | C-A1 | C-A1 | C-A1 | C-A1 | B68 | B92 |
| H8-1427 | S | C-A1 | C-A1 | C-A1 | C-A1 | B68 | B93 |
| H8-1428 | S | C-A1 | C-A1 | C-A1 | C-A1 | B68 | B94 |
| H8-1429 | S | C-A1 | C-A1 | C-A1 | C-A1 | B68 | B95 |
| H8-1430 | S | C-A1 | C-A1 | C-A1 | C-A1 | B68 | B96 |
| H8-1431 | S | C-A1 | C-A1 | C-A1 | C-A1 | B68 | B97 |
| H8-1432 | S | C-A1 | C-A1 | C-A1 | C-A1 | B68 | B106 |
| H8-1433 | S | C-A1 | C-A1 | C-A1 | C-A1 | B68 | B107 |
| H8-1434 | S | C-A1 | C-A1 | C-A1 | C-A1 | B68 | B114 |
| H8-1435 | S | C-A1 | C-A1 | C-A1 | C-A1 | B68 | B115 |
| H8-1436 | S | C-A1 | C-A1 | C-A1 | C-A1 | B68 | B116 |
| H8-1437 | S | C-A1 | C-A1 | C-A1 | C-A1 | B68 | B117 |
| H8-1438 | S | C-A1 | C-A1 | C-A1 | C-A1 | B68 | B118 |
| H8-1439 | S | C-A1 | C-A1 | C-A1 | C-A1 | B68 | B119 |
| H8-1440 | S | C-A1 | C-A1 | C-A1 | C-A1 | B68 | B120 |
| H8-1441 | S | C-A1 | C-A1 | C-A1 | C-A1 | B68 | B121 |
| H8-1442 | S | C-A1 | C-A1 | C-A1 | C-A1 | B68 | B122 |
| H8-1443 | S | C-A1 | C-A1 | C-A1 | C-A1 | B68 | B125 |
| H8-1444 | S | C-A1 | C-A1 | C-A1 | C-A1 | B68 | B126 |
| H8-1445 | S | C-A1 | C-A1 | C-A1 | C-A1 | B68 | B127 |
| H8-1446 | S | C-A1 | C-A1 | C-A1 | C-A1 | B68 | B128 |
| H8-1447 | S | C-A1 | C-A1 | C-A1 | C-A1 | B68 | B129 |
| H8-1448 | S | C-A1 | C-A1 | C-A1 | C-A1 | B68 | B130 |
| H8-1449 | S | C-A1 | C-A1 | C-A1 | C-A1 | B68 | B131 |
| H8-1450 | S | C-A1 | C-A1 | C-A1 | C-A1 | B68 | B132 |
| H8-1451 | S | C-A1 | C-A1 | C-A1 | C-A1 | B68 | B133 |
| H8-1452 | S | C-A1 | C-A1 | C-A1 | C-A1 | B68 | B134 |
| H8-1453 | S | C-A1 | C-A1 | C-A1 | C-A1 | B68 | B135 |
| H8-1454 | S | C-A1 | C-A1 | C-A1 | C-A1 | B68 | B136 |
| H8-1455 | S | C-A1 | C-A1 | C-A1 | C-A1 | B68 | B137 |
| H8-1456 | S | C-A1 | C-A1 | C-A1 | C-A1 | B68 | B138 |
| H8-1457 | S | C-A1 | C-A1 | C-A1 | C-A1 | B68 | B139 |
| H8-1458 | S | C-A1 | C-A1 | C-A1 | C-A1 | B68 | B140 |
| H8-1465 | S | C-A1 | C-A1 | C-A1 | C-A1 | B70 | B1 |
| H8-1466 | S | C-A1 | C-A1 | C-A1 | C-A1 | B70 | B6 |
| H8-1467 | S | C-A1 | C-A1 | C-A1 | C-A1 | B70 | B20 |
| H8-1468 | S | C-A1 | C-A1 | C-A1 | C-A1 | B70 | B21 |
| H8-1469 | S | C-A1 | C-A1 | C-A1 | C-A1 | B70 | B22 |
| H8-1470 | S | C-A1 | C-A1 | C-A1 | C-A1 | B70 | B32 |
| H8-1471 | S | C-A1 | C-A1 | C-A1 | C-A1 | B70 | B33 |
| H8-1472 | S | C-A1 | C-A1 | C-A1 | C-A1 | B70 | B34 |
| H8-1473 | S | C-A1 | C-A1 | C-A1 | C-A1 | B70 | B68 |
| H8-1474 | S | C-A1 | C-A1 | C-A1 | C-A1 | B70 | B69 |
| H8-1475 | S | C-A1 | C-A1 | C-A1 | C-A1 | B70 | B70 |
| H8-1476 | S | C-A1 | C-A1 | C-A1 | C-A1 | B70 | B71 |
| H8-1477 | S | C-A1 | C-A1 | C-A1 | C-A1 | B70 | B81 |
| H8-1478 | S | C-A1 | C-A1 | C-A1 | C-A1 | B70 | B90 |
| H8-1479 | S | C-A1 | C-A1 | C-A1 | C-A1 | B70 | B91 |
| H8-1480 | S | C-A1 | C-A1 | C-A1 | C-A1 | B70 | B92 |
| H8-1481 | S | C-A1 | C-A1 | C-A1 | C-A1 | B70 | B93 |
| H8-1482 | S | C-A1 | C-A1 | C-A1 | C-A1 | B70 | B94 |
| H8-1483 | S | C-A1 | C-A1 | C-A1 | C-A1 | B70 | B95 |
| H8-1484 | S | C-A1 | C-A1 | C-A1 | C-A1 | B70 | B96 |
| H8-1485 | S | C-A1 | C-A1 | C-A1 | C-A1 | B70 | B97 |
| H8-1486 | S | C-A1 | C-A1 | C-A1 | C-A1 | B70 | B106 |
| H8-1487 | S | C-A1 | C-A1 | C-A1 | C-A1 | B70 | B107 |
| H8-1488 | S | C-A1 | C-A1 | C-A1 | C-A1 | B70 | B114 |
| H8-1489 | S | C-A1 | C-A1 | C-A1 | C-A1 | B70 | B115 |
| H8-1490 | S | C-A1 | C-A1 | C-A1 | C-A1 | B70 | B116 |
| H8-1491 | S | C-A1 | C-A1 | C-A1 | C-A1 | B70 | B117 |
| H8-1492 | S | C-A1 | C-A1 | C-A1 | C-A1 | B70 | B118 |
| H8-1493 | S | C-A1 | C-A1 | C-A1 | C-A1 | B70 | B119 |
| H8-1494 | S | C-A1 | C-A1 | C-A1 | C-A1 | B70 | B120 |
| H8-1495 | S | C-A1 | C-A1 | C-A1 | C-A1 | B70 | B121 |
| H8-1496 | S | C-A1 | C-A1 | C-A1 | C-A1 | B70 | B122 |
| H8-1497 | S | C-A1 | C-A1 | C-A1 | C-A1 | B70 | B125 |
| H8-1498 | S | C-A1 | C-A1 | C-A1 | C-A1 | B70 | B126 |
| H8-1499 | S | C-A1 | C-A1 | C-A1 | C-A1 | B70 | B127 |
| H8-1500 | S | C-A1 | C-A1 | C-A1 | C-A1 | B70 | B128 |
| H8-1501 | S | C-A1 | C-A1 | C-A1 | C-A1 | B70 | B129 |
| H8-1502 | S | C-A1 | C-A1 | C-A1 | C-A1 | B70 | B130 |
| H8-1503 | S | C-A1 | C-A1 | C-A1 | C-A1 | B70 | B131 |
| H8-1504 | S | C-A1 | C-A1 | C-A1 | C-A1 | B70 | B132 |
| H8-1505 | S | C-A1 | C-A1 | C-A1 | C-A1 | B70 | B133 |
| H8-1506 | S | C-A1 | C-A1 | C-A1 | C-A1 | B70 | B134 |
| H8-1507 | S | C-A1 | C-A1 | C-A1 | C-A1 | B70 | B135 |
| H8-1508 | S | C-A1 | C-A1 | C-A1 | C-A1 | B70 | B136 |
| H8-1509 | S | C-A1 | C-A1 | C-A1 | C-A1 | B70 | B137 |
| H8-1510 | S | C-A1 | C-A1 | C-A1 | C-A1 | B70 | B138 |
| H8-1511 | S | C-A1 | C-A1 | C-A1 | C-A1 | B70 | B139 |
| H8-1512 | S | C-A1 | C-A1 | C-A1 | C-A1 | B70 | B140 |

9. Elektrolumineszenzvorrichtung umfassend:
eine Anode,
eine Kathode und
eine organische Schicht, die zwischen der Anode und der Kathode angeordnet ist, wobei die organische Schicht die Verbindung nach einem der Ansprüche 1 bis 8 umfasst.

10. Elektrolumineszenzvorrichtung nach Anspruch 9, wobei die organische Schicht eine Lochinjektionsschicht oder eine Lochtransportschicht ist und die Lochinjektionsschicht oder die Lochtransportschicht allein durch die Verbindung gebildet ist.

11. Elektrolumineszenzvorrichtung nach Anspruch 9, wobei die organische Schicht eine Lochinjektionsschicht oder eine Lochtransportschicht ist und die Lochinjektionsschicht oder die Lochtransportschicht ferner mindestens ein Lochtransportmaterial umfasst; wobei das molare Dotierungsverhältnis der Verbindung zu dem mindestens einen Lochtransportmaterial 10000: 1 bis 1: 10000 ist;
bevorzugt ist das molare Dotierungsverhältnis der Verbindung zu dem mindestens einen Lochtransportmaterial 10:1 bis 1: 100.

12. Elektrolumineszenzvorrichtung nach Anspruch 9, wobei die Elektrolumineszenzvorrichtung mindestens zwei lichtemittierende Einheiten umfasst und die organische Schicht eine Ladungserzeugungsschicht ist und zwischen den mindestens zwei lichtemittierenden Einheiten angeordnet ist, wobei die Ladungserzeugungsschicht eine p-Typ-Ladungserzeugungsschicht und eine n-Typ-Ladungserzeugungsschicht umfasst;
bevorzugt umfasst die p-Typ-Ladungserzeugungsschicht die Verbindung;
besonders bevorzugt umfasst die p-Typ-Ladungserzeugungsschicht ferner mindestens ein Lochtransportmaterial, wobei ein molares Dotierungsverhältnis der Verbindung zu dem mindestens einen Lochtransportmaterial 10000: 1 bis 1: 10000 ist; am meisten bevorzugt ist das molare Dotierungsverhältnis der Verbindung zu dem mindestens einen Lochtransportmaterial 10:1 bis 1: 100.

13. Elektrolumineszenzvorrichtung nach Anspruch 11 oder 12, wobei das Lochtransportmaterial ausgewählt ist aus einer Verbindung mit einer Triarylamineinheit, einer Spirobifluorenverbindung, einer Pentacenverbindung, einer Oligothiophenverbindung, einer Oligophenylverbindung, einer Oligophenylenvinylenverbindung, einer Oligofluorenverbindung, einem Porphyrinkomplex oder einem Metallphthalocyaninkomplex.

14. Elektrolumineszenzvorrichtung nach Anspruch 12, wobei die Ladungserzeugungsschicht ferner eine Pufferschicht umfasst, die zwischen der p-Typ-Ladungserzeugungsschicht und der n-Typ-Ladungserzeugungsschicht angeordnet ist, und die Pufferschicht die Verbindung umfasst.

15. Verbindungszusammensetzung umfassend die Verbindung nach einem der Ansprüche 1 bis 8.

## Revendications

1. Composé ayant une structure représentée par l'une quelconque des formules 2-1 à 2-10 : 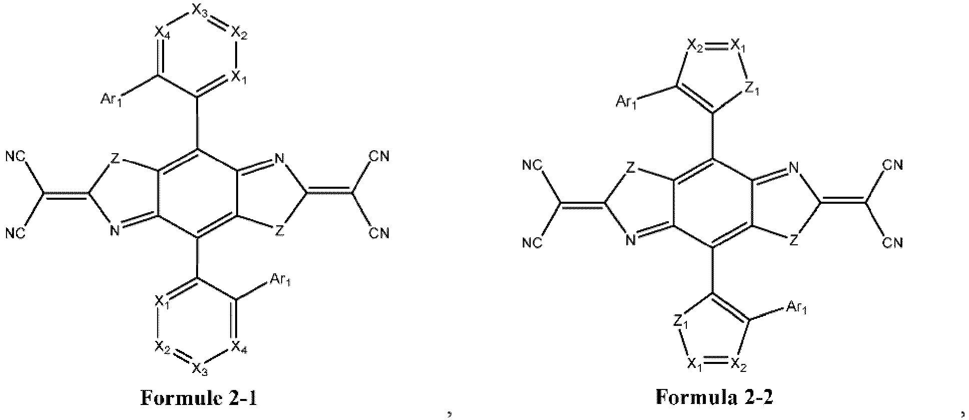 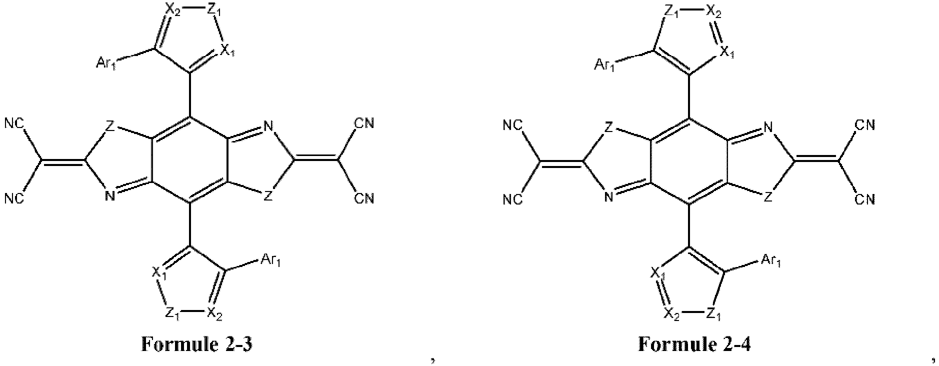 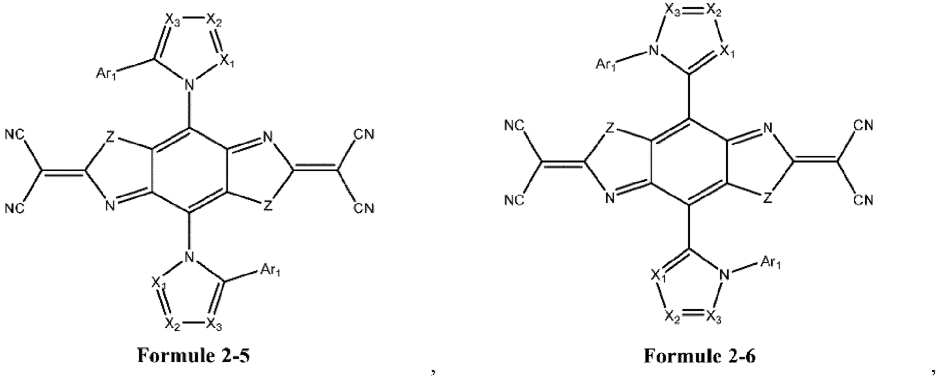 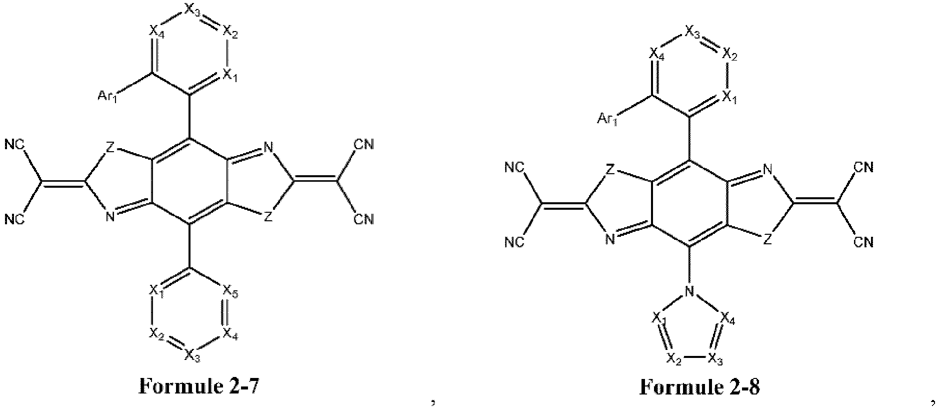 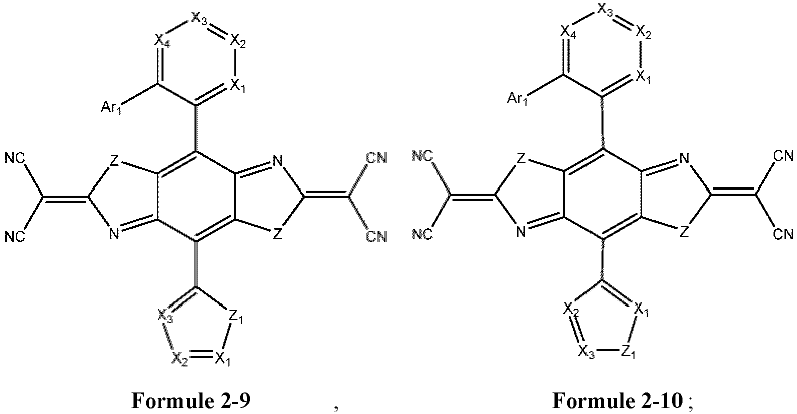
Z est, à chaque occurrence, de manière identique ou différente, choisi parmi O, S ou Se ;
X₁ à X₅ sont, à chaque occurrence, de manière identique ou différente, choisis parmi N ou CR₁ ;
Z₁ est, à chaque occurrence, de manière identique ou différente, choisi parmi O, S, Se ou NR_{N1} ;
Ar₁ est, à chaque occurrence, de manière identique ou différente, choisi parmi le groupe constitué de : un aryle substitué ou non substitué ayant 6 à 20 atomes de carbone, un hétéroaryle substitué ou non substitué ayant 3 à 20 atomes de carbone et des combinaisons de ceux-ci ; et lorsque Ar₁ est choisi parmi un aryle substitué ayant 6 à 20 atomes de carbone ou un hétéroaryle substitué ayant 3 à 20 atomes de carbone, l'aryle ou l'hétéroaryle est substitué par un ou plusieurs R₂ ;
Ri, R₂ et R_{N1} sont, à chaque occurrence, de manière identique ou différente, choisis parmi le groupe constitué de : hydrogène, deutérium, un groupe hydroxyle, un groupe sulfanyle, un halogène, un groupe nitroso, un groupe nitro, un groupe acyle, un groupe carbonyle, un groupe acide carboxylique, un groupe ester, un groupe cyano, un groupe isocyano, SCN, OCN, SF₅, un groupe boranyle, un groupe sulfinyle, un groupe sulfonyle, un groupe phosphoroso, un alkyle substitué ou non substitué ayant 1 à 20 atomes de carbone, un cycloalkyle substitué ou non substitué ayant 3 à 20 atomes de carbone de cycle, un hétéroalkyle substitué ou non substitué ayant 1 à 20 atomes de carbone, un alcoxy substitué ou non substitué ayant 1 à 20 atomes de carbone, un alcényle substitué ou non substitué ayant 2 à 20 atomes de carbone, un alcynyle substitué ou non substitué ayant 2 à 20 atomes de carbone, un aryle substitué ou non substitué ayant 6 à 30 atomes de carbone, un hétéroaryle substitué ou non substitué ayant 3 à 30 atomes de carbone et des combinaisons de ceux-ci ;
au moins l'un parmi R₁ et R₂ est, à chaque occurrence, de manière identique ou différente, choisi parmi le groupe constitué de : un groupe hydroxyle, un groupe sulfanyle, un halogène, un groupe nitroso, un groupe nitro, un groupe acyle, un groupe carbonyle, un groupe acide carboxylique, un groupe ester, un groupe cyano, un groupe isocyano, SCN, OCN, SF₅, un groupe boranyle, un groupe sulfinyle, un groupe sulfonyle, un groupe phosphoroso, un alkyle substitué ou non substitué ayant 1 à 20 atomes de carbone, un cycloalkyle substitué ou non substitué ayant 3 à 20 atomes de carbone de cycle, un hétéroalkyle substitué ou non substitué ayant 1 à 20 atomes de carbone, un alcoxy substitué ou non substitué ayant 1 à 20 atomes de carbone, un alcényle substitué ou non substitué ayant 2 à 20 atomes de carbone, un alcynyle substitué ou non substitué ayant 2 à 20 atomes de carbone, un aryle substitué ou non substitué ayant 6 à 30 atomes de carbone, un hétéroaryle substitué ou non substitué ayant 3 à 30 atomes de carbone et des combinaisons de ceux-ci ; et
les substituants adjacents Ri, R₂ peuvent être éventuellement liés pour former un noyau.

2. Composé selon la revendication 1, dans lequel X₁ est, à chaque occurrence, de manière identique ou différente, choisi parmi CR₁.

3. Composé selon l'une quelconque des revendications 1 à 2, dans lequel le composé a une structure représentée par la formule 3-1 : 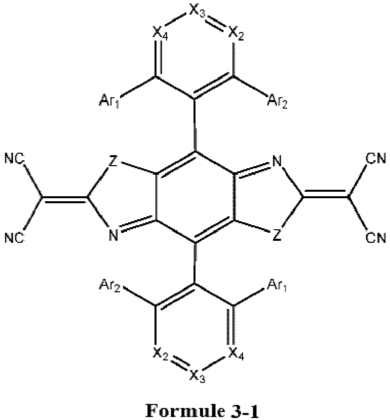 dans lequel dans la formule 3-1,
Z est, à chaque occurrence, de manière identique ou différente, choisi parmi O, S ou Se ;
X₂ à X₄ sont, à chaque occurrence, de manière identique ou différente, choisis parmi N ou CR₁ ;
Ar₁ et Ar₂ sont, à chaque occurrence, de manière identique ou différente, choisis parmi le groupe constitué de : un aryle substitué ou non substitué ayant 6 à 20 atomes de carbone, un hétéroaryle substitué ou non substitué ayant 3 à 20 atomes de carbone et des combinaisons de ceux-ci ; et lorsque Ar₁ et Ar₂ sont choisis parmi un aryle substitué ayant 6 à 20 atomes de carbone ou un hétéroaryle substitué ayant 3 à 20 atomes de carbone, l'aryle ou l'hétéroaryle est substitué par un ou plusieurs R₂ ;
R₁ et R₂ sont, à chaque occurrence, de manière identique ou différente, choisis parmi le groupe constitué de : hydrogène, deutérium, un groupe hydroxyle, un groupe sulfanyle, un halogène, un groupe nitroso, un groupe nitro, un groupe acyle, un groupe carbonyle, un groupe acide carboxylique, un groupe ester, un groupe cyano, un groupe isocyano, SCN, OCN, SF₅, un groupe boranyle, un groupe sulfinyle, un groupe sulfonyle, un groupe phosphoroso, un alkyle substitué ou non substitué ayant 1 à 20 atomes de carbone, un cycloalkyle substitué ou non substitué ayant 3 à 20 atomes de carbone de cycle, un hétéroalkyle substitué ou non substitué ayant 1 à 20 atomes de carbone, un alcoxy substitué ou non substitué ayant 1 à 20 atomes de carbone, un alcényle substitué ou non substitué ayant 2 à 20 atomes de carbone, un alcynyle substitué ou non substitué ayant 2 à 20 atomes de carbone, un aryle substitué ou non substitué ayant 6 à 30 atomes de carbone, un hétéroaryle substitué ou non substitué ayant 3 à 30 atomes de carbone et des combinaisons de ceux-ci ;
au moins l'un parmi R₁ et R₂ est, à chaque occurrence, de manière identique ou différente, choisi parmi le groupe constitué de : un groupe hydroxyle, un groupe sulfanyle, un halogène, un groupe nitroso, un groupe nitro, un groupe acyle, un groupe carbonyle, un groupe acide carboxylique, un groupe ester, un groupe cyano, un groupe isocyano, SCN, OCN, SF₅, un groupe boranyle, un groupe sulfinyle, un groupe sulfonyle, un groupe phosphoroso, un alkyle substitué ou non substitué ayant 1 à 20 atomes de carbone, un cycloalkyle substitué ou non substitué ayant 3 à 20 atomes de carbone de cycle, un hétéroalkyle substitué ou non substitué ayant 1 à 20 atomes de carbone, un alcoxy substitué ou non substitué ayant 1 à 20 atomes de carbone, un alcényle substitué ou non substitué ayant 2 à 20 atomes de carbone, un alcynyle substitué ou non substitué ayant 2 à 20 atomes de carbone, un aryle substitué ou non substitué ayant 6 à 30 atomes de carbone, un hétéroaryle substitué ou non substitué ayant 3 à 30 atomes de carbone et des combinaisons de ceux-ci ; et
les substituants adjacents Ri, R₂ peuvent être éventuellement liés pour former un noyau.

4. Composé selon l'une quelconque des revendications 1 à 3, dans lequel Z est, à chaque occurrence, de manière identique ou différente, choisi parmi O ou S ; préférentiellement, Z est choisi parmi O.

5. Composé selon la revendication 1, dans lequel dans les formules 2-1 à 2-10, au moins l'un parmi X₁ à X₅ est, à chaque occurrence, de manière identique ou différente, choisi parmi CR₁ ; préférentiellement X₁ à X₅ sont, à chaque occurrence, de manière identique ou différente, choisis parmi CR₁.

6. Composé selon l'une quelconque des revendications 1 à 5, dans lequel les R₁ et R₂ sont, à chaque occurrence, de manière identique ou différente, choisis parmi le groupe constitué de : hydrogène, deutérium, halogène, cyano, isocyano, SCN, OCN, SF₅, boranyle, alkyle substitué ou non substitué ayant 1 à 20 atomes de carbone, hétéroalkyle substitué ou non substitué ayant 1 à 20 atomes de carbone, alcoxy substitué ou non substitué ayant 1 à 20 atomes de carbone, aryle substitué ou non substitué ayant 6 à 30 atomes de carbone, hétéroaryle substitué ou non substitué ayant 3 à 30 atomes de carbone et des combinaisons de ceux-ci ;
au moins l'un parmi R₁ et R₂ est, à chaque occurrence de manière identique ou différente, choisi parmi le groupe constitué de : halogène, cyano, isocyano, SCN, OCN, SF₅, boranyle, alkyle substitué ou non substitué ayant 1 à 20 atomes de carbone, hétéroalkyle substitué ou non substitué ayant 1 à 20 atomes de carbone, alcoxy substitué ou non substitué ayant 1 à 20 atomes de carbone, aryle substitué ou non substitué ayant 6 à 30 atomes de carbone, hétéroaryle substitué ou non substitué ayant 3 à 30 atomes de carbone et des combinaisons de ceux-ci ; et
les substituants adjacents Ri, R₂ peuvent être éventuellement liés pour former un noyau ;
préférentiellement, au moins l'un parmi R₁ et R₂ est, à chaque occurrence, de manière identique ou différente, choisi parmi le groupe constitué de : fluor, SF₅, cyano, alkyle substitué ayant 1 à 20 atomes de carbone, hétéroalkyle substitué ayant 1 à 20 atomes de carbone, alcoxy substitué ayant 1 à 20 atomes de carbone, aryle substitué ayant 6 à 30 atomes de carbone, hétéroaryle substitué ayant 3 à 30 atomes de carbone et des combinaisons de ceux-ci ; et un substituant de l'alkyle substitué, de l'hétéroalkyle substitué, de l'alcoxy substitué, de l'aryle substitué ou de l'hétéroaryle substitué comprend au moins un groupe attracteur d'électrons ;
plus préférentiellement, au moins l'un parmi R₁ et R₂ est, à chaque occurrence, de manière identique ou différente, choisi parmi le groupe constitué de : fluor, SF₅, cyano, alkyle substitué ayant 1 à 20 atomes de carbone, hétéroalkyle substitué ayant 1 à 20 atomes de carbone, alcoxy substitué ayant 1 à 20 atomes de carbone, aryle substitué ayant 6 à 30 atomes de carbone, hétéroaryle substitué ayant 3 à 30 atomes de carbone et des combinaisons de ceux-ci ; et un substituant de l'alkyle substitué, de l'hétéroalkyle substitué, de l'alcoxy substitué, de l'aryle substitué ou de l'hétéroaryle substitué comprend au moins un atome de fluor et/ou au moins un groupe cyano.

7. Composé selon l'une quelconque des revendications 1 à 6, dans lequel R₁ et R₂ sont, à chaque occurrence, de manière identique ou différente, choisis parmi le groupe constitué de A1 à A19 et de B1 à B314 : 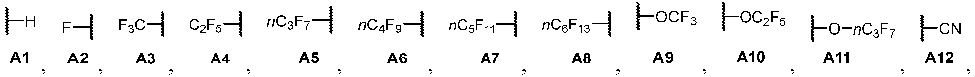 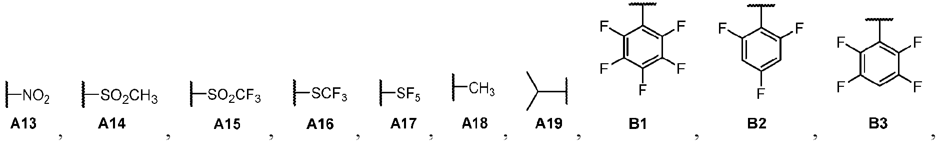 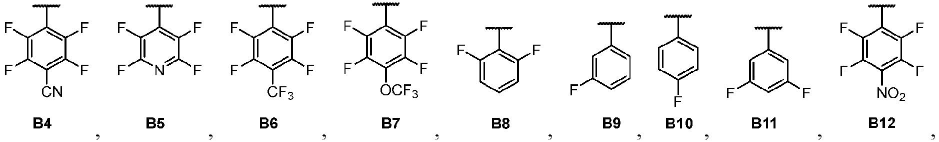 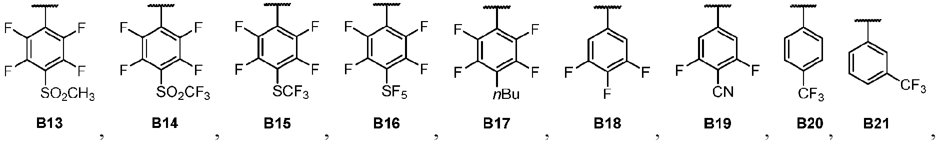 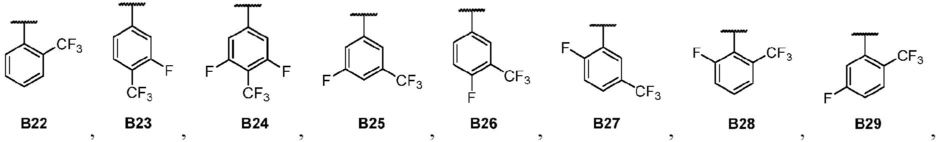 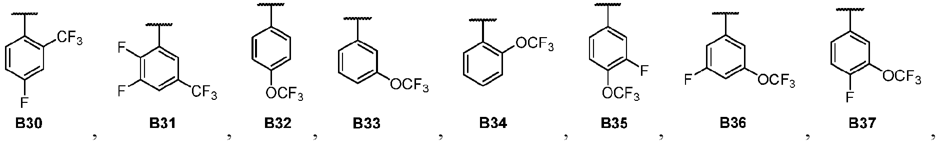 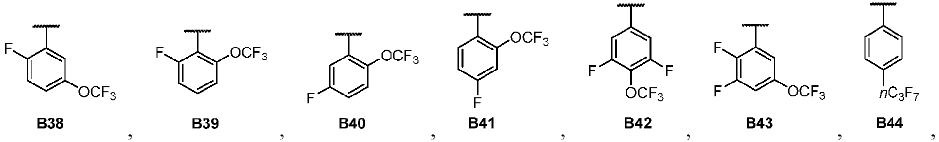 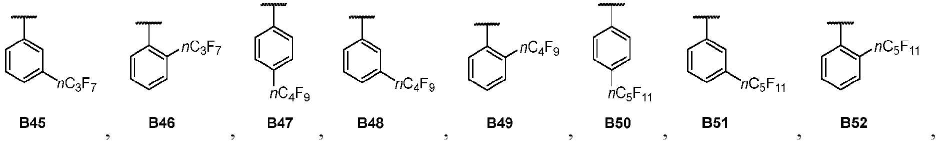 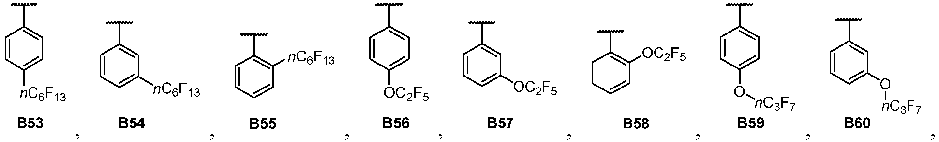 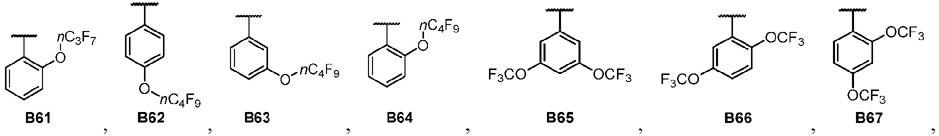 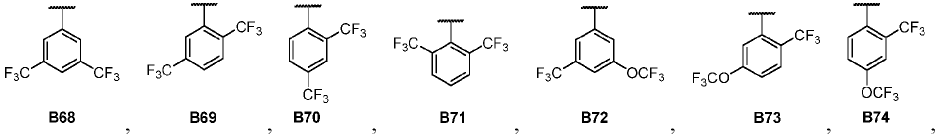 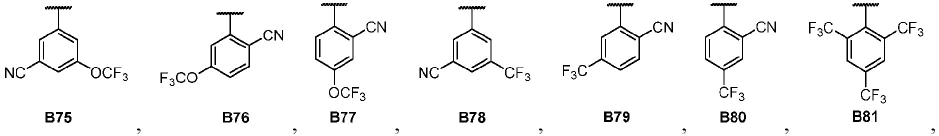 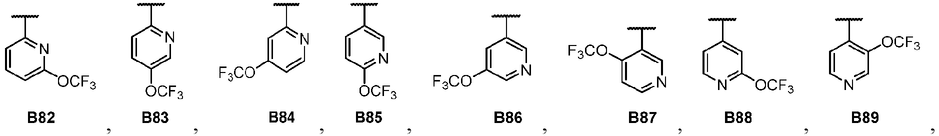 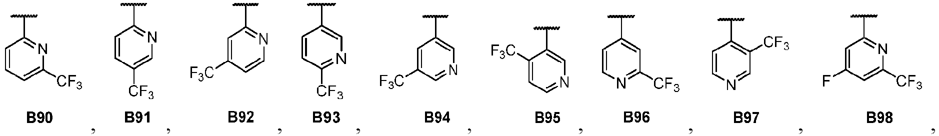 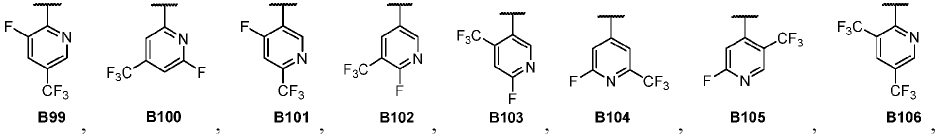 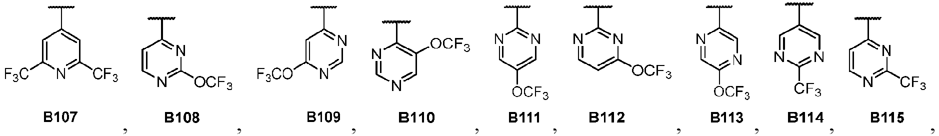 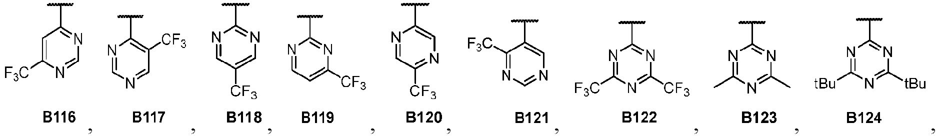 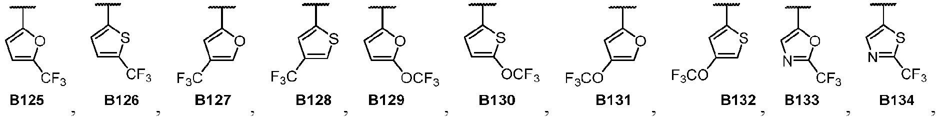 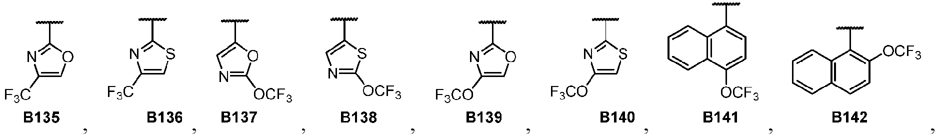 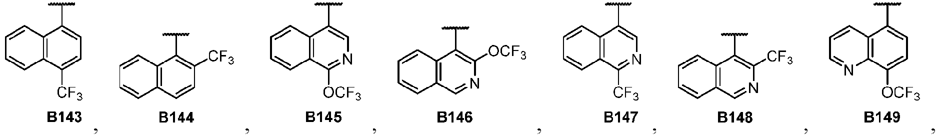 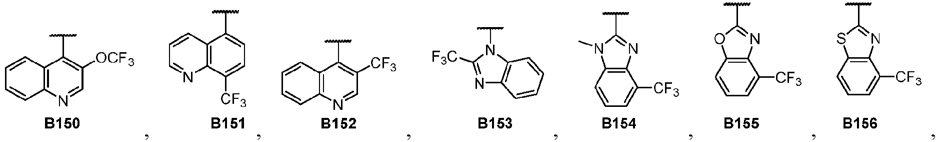 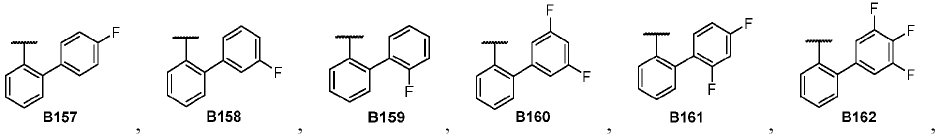 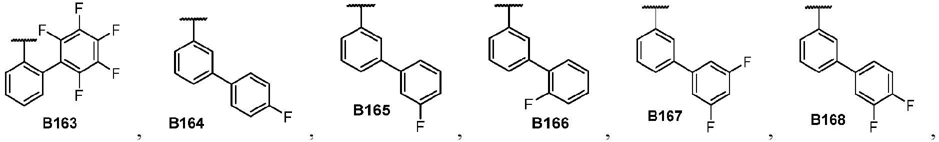 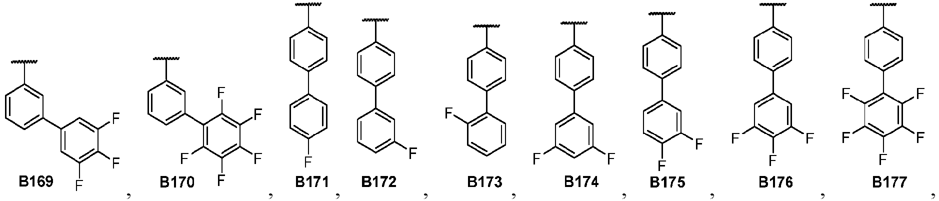 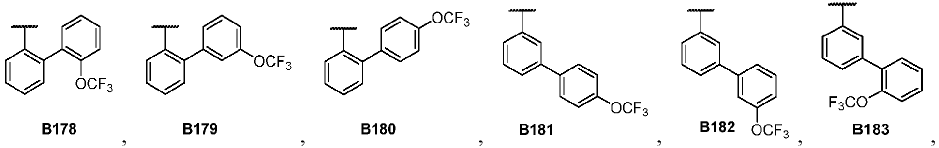 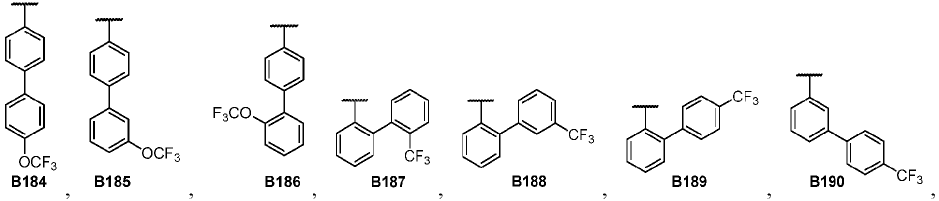 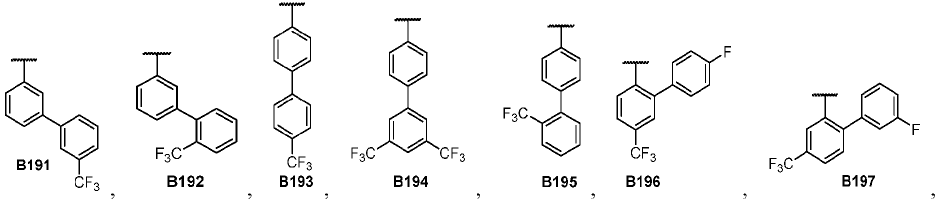 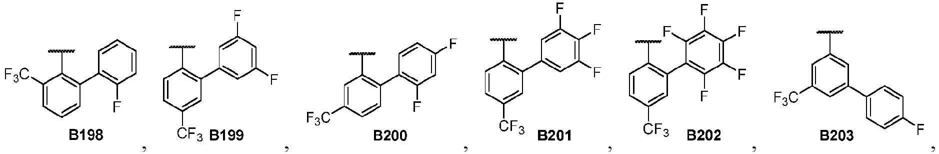 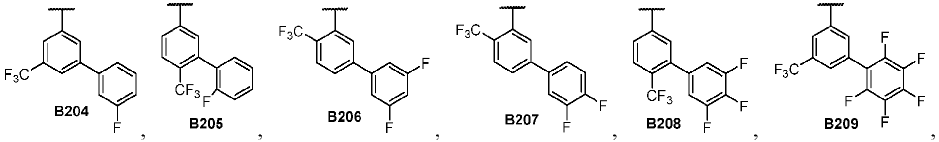 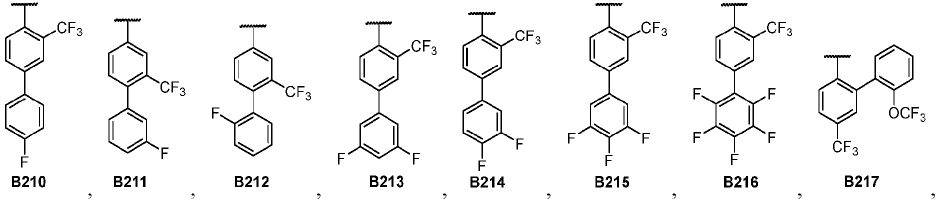 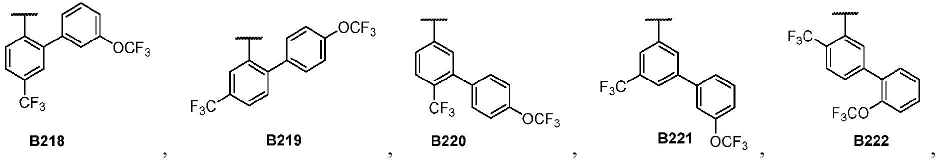 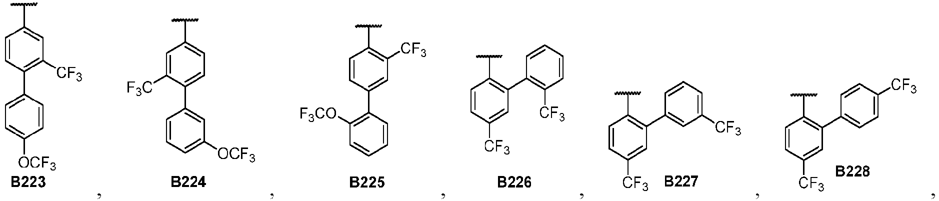 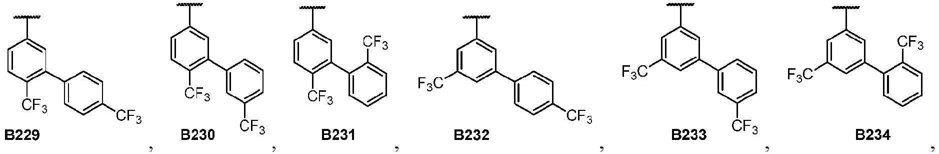 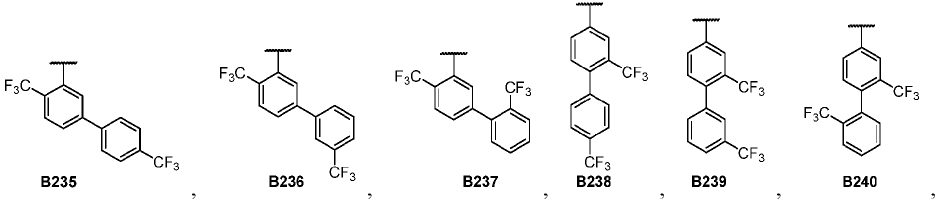 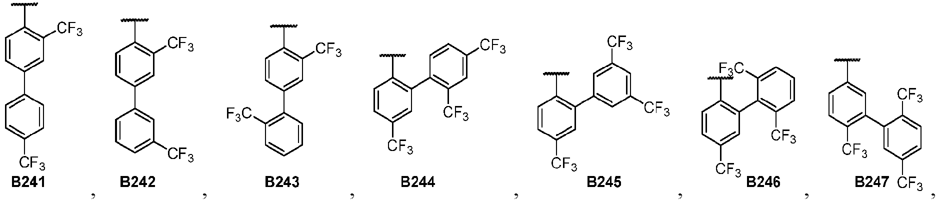 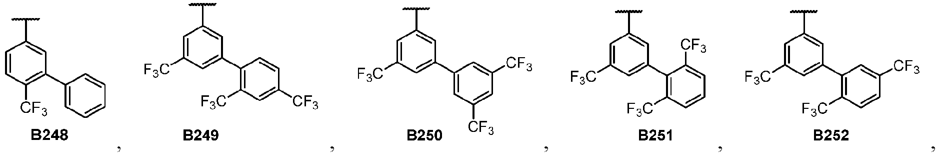 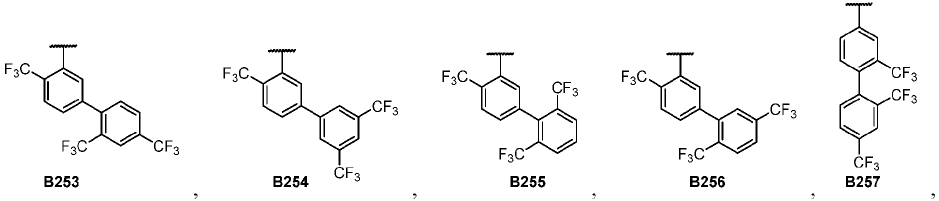 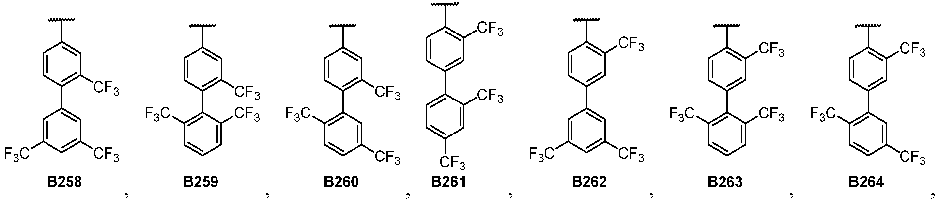 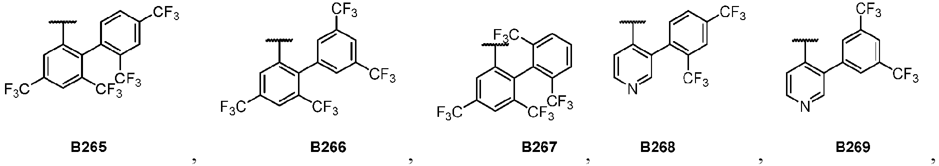 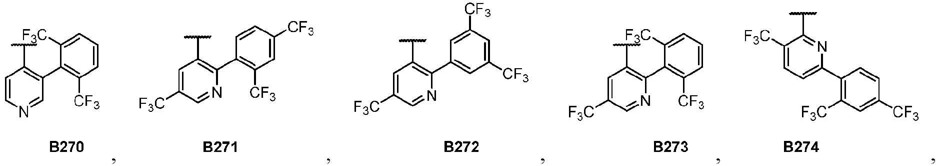 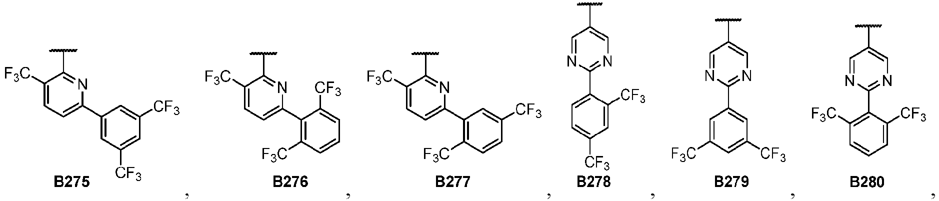 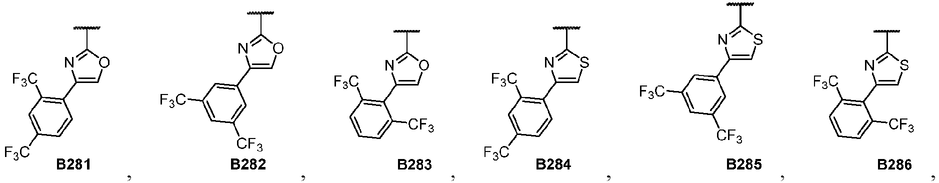 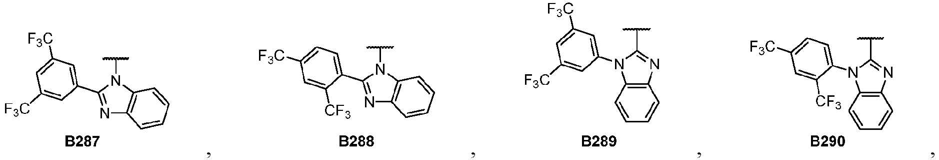 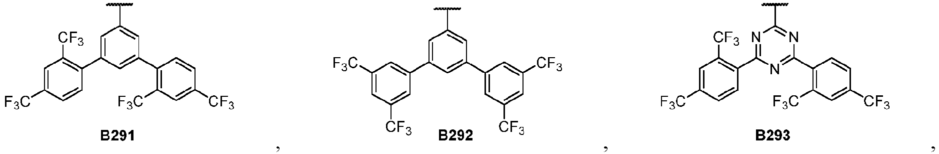 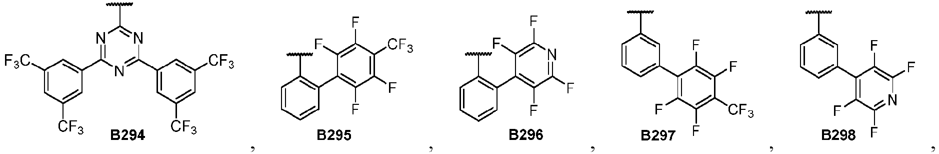 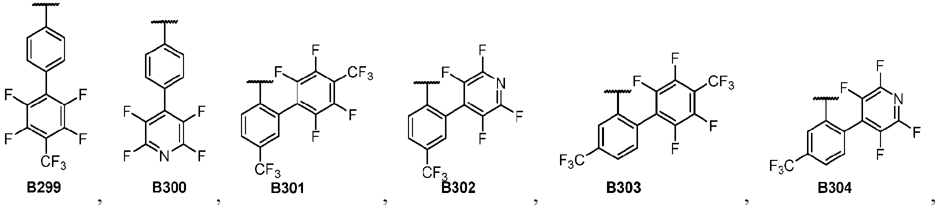 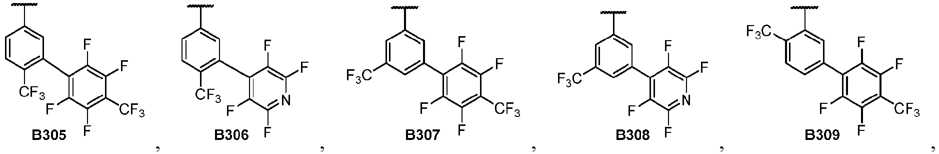 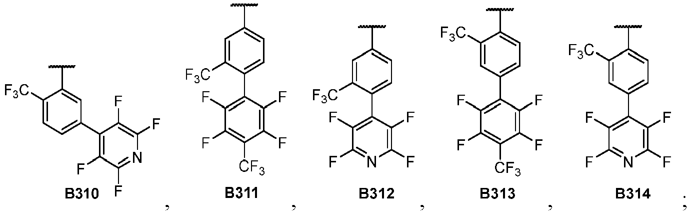 et/ou Ar₁ et Ar₂ sont, à chaque occurrence, de manière identique ou différente, choisis parmi le groupe constitué de B1 à B314.

8. Composé selon la revendication 7, dans lequel le composé est choisi parmi le groupe constitué du composé H1-1 au composé H1-2449, du composé H2-1 au composé H2-138, du composé H3-1 au composé H3-92, du composé H4-1 au composé H4-92, du composé H5-1 au composé H5-115, du composé H6-1 au composé H6-115, du composé H7-1 au composé H7-203 et du composé H8-7 au composé H8-54, du composé H8-61 au composé H8-108, du composé H8-115 au composé H8-162, du composé H8-169 au composé H8-216, du composé H8-223 au composé H8-270, du composé H8-277 au composé H8-324, du composé H8-331 au composé H8-378, du composé H8-385 au composé H8-432, du composé H8-439 au composé H8-486, du composé H8-493 au composé H8-540, du composé H8-547 au composé H8-594, du composé H8-601 au composé H8-648, du composé H8-655 au composé H8-702, du composé H8-709 au composé H8-756, du composé H8-763 au composé H8-810, du composé H8-817 au composé H8-864, du composé H8-871 au composé H8-918, du composé H8-925 au composé H8-972, du composé H8-979 au composé H8-1026, du composé H8-1033 au composé H8-1080, du composé H8-1087 au composé H8-1134, du composé H8-1141 au composé H8-1188, du composé H8-1195 au composé H8-1242, du composé H8-1249 au composé H8-1296, du composé H8-1303 au composé H8-1350, du composé H8-1357 au composé H8-1404, du composé H8-1411 au composé H8-1458, du composé H8-1465 au composé H8-1512 ;
dans lequel les composés H1-1 à H1-2449 ont chacun une structure représentée par la formule 2-1 : 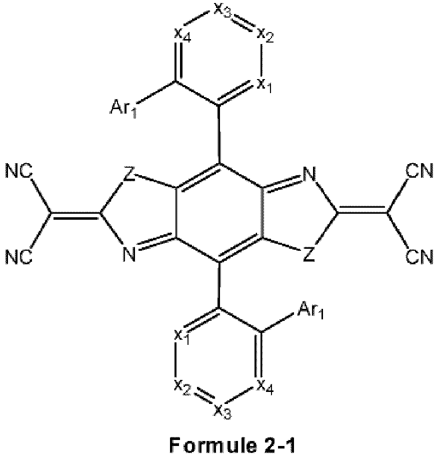
dans la formule 2-1, les deux Z sont identiques, les deux X₁ sont identiques, les deux X₂ sont identiques, les deux X₃ sont identiques, les deux X₄ sont identiques, les deux Ar₁ sont identiques, et Z, X₁, X₂, X₃, X₄ et Ar₁ sont choisis parmi des atomes ou des groupes dans le tableau suivant, respectivement : 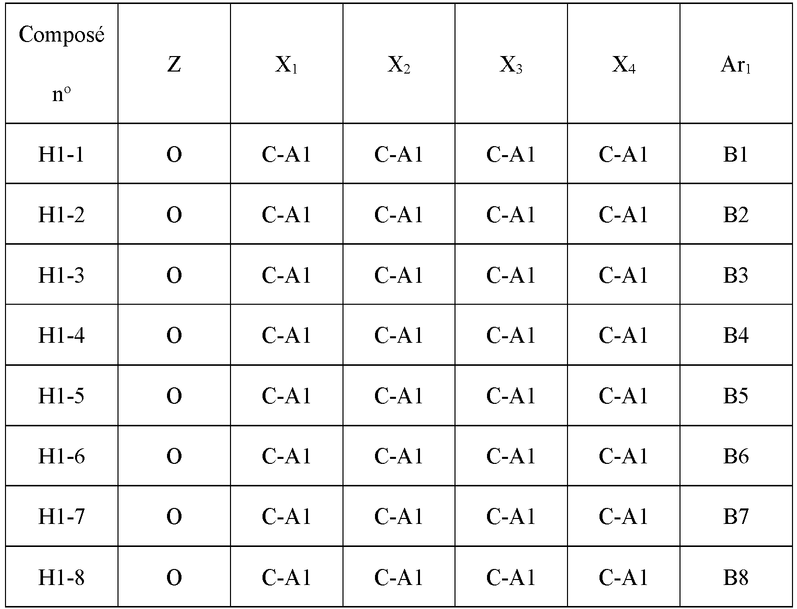 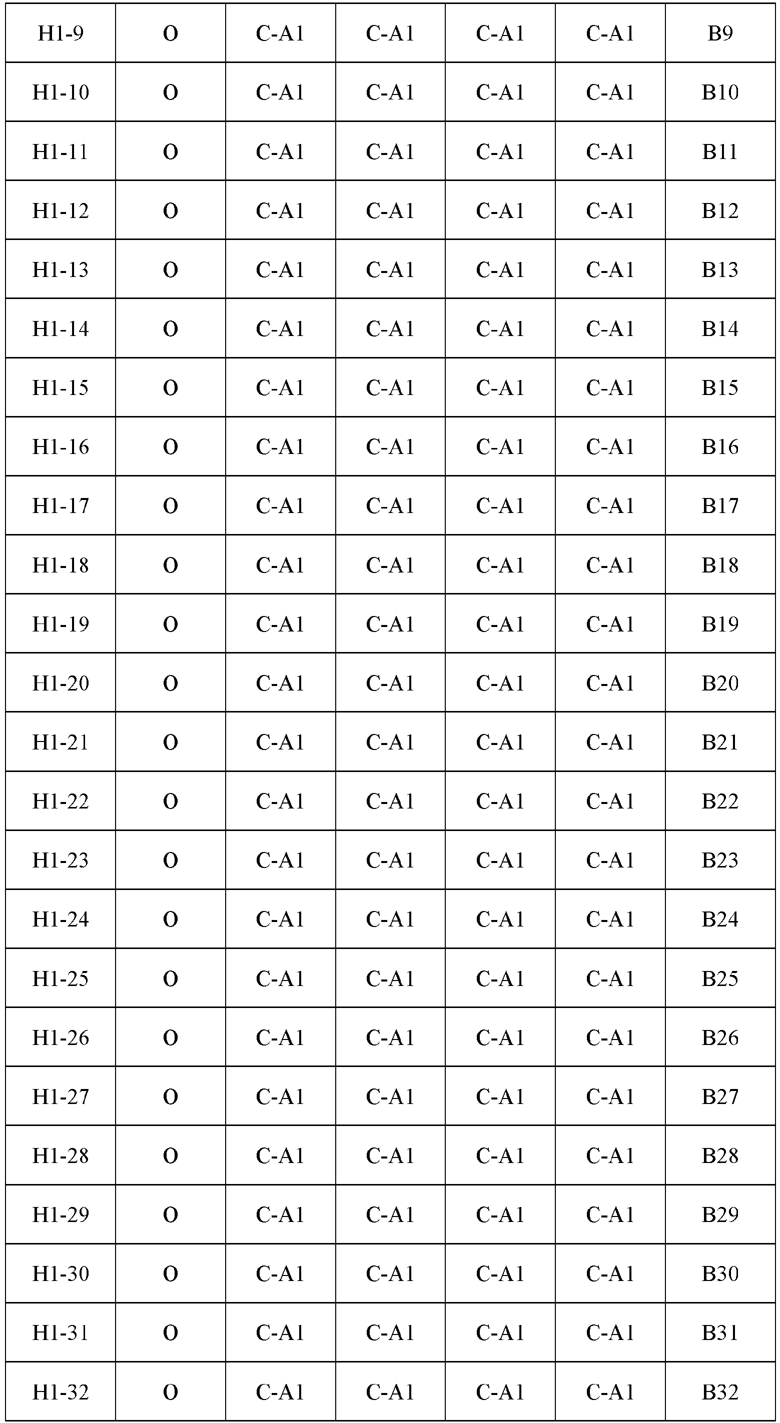 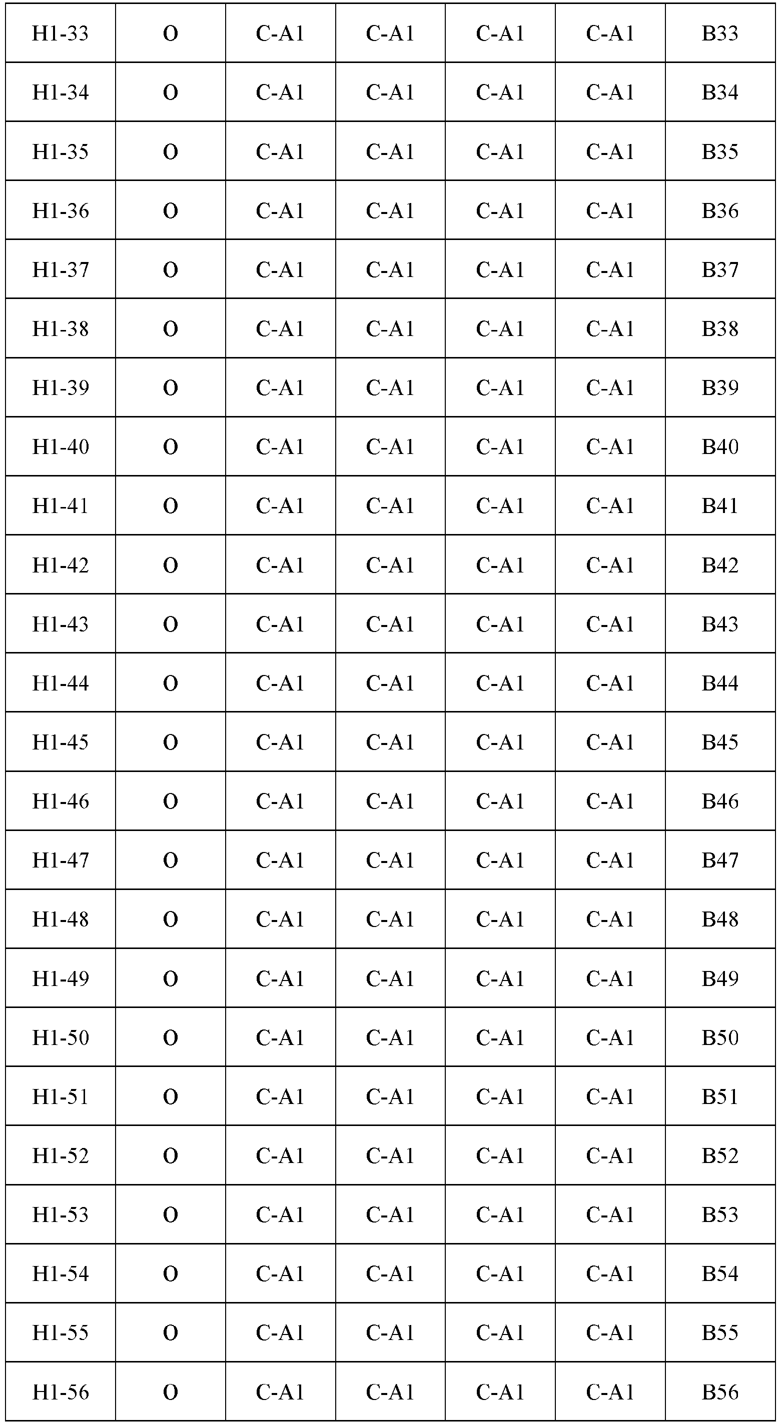 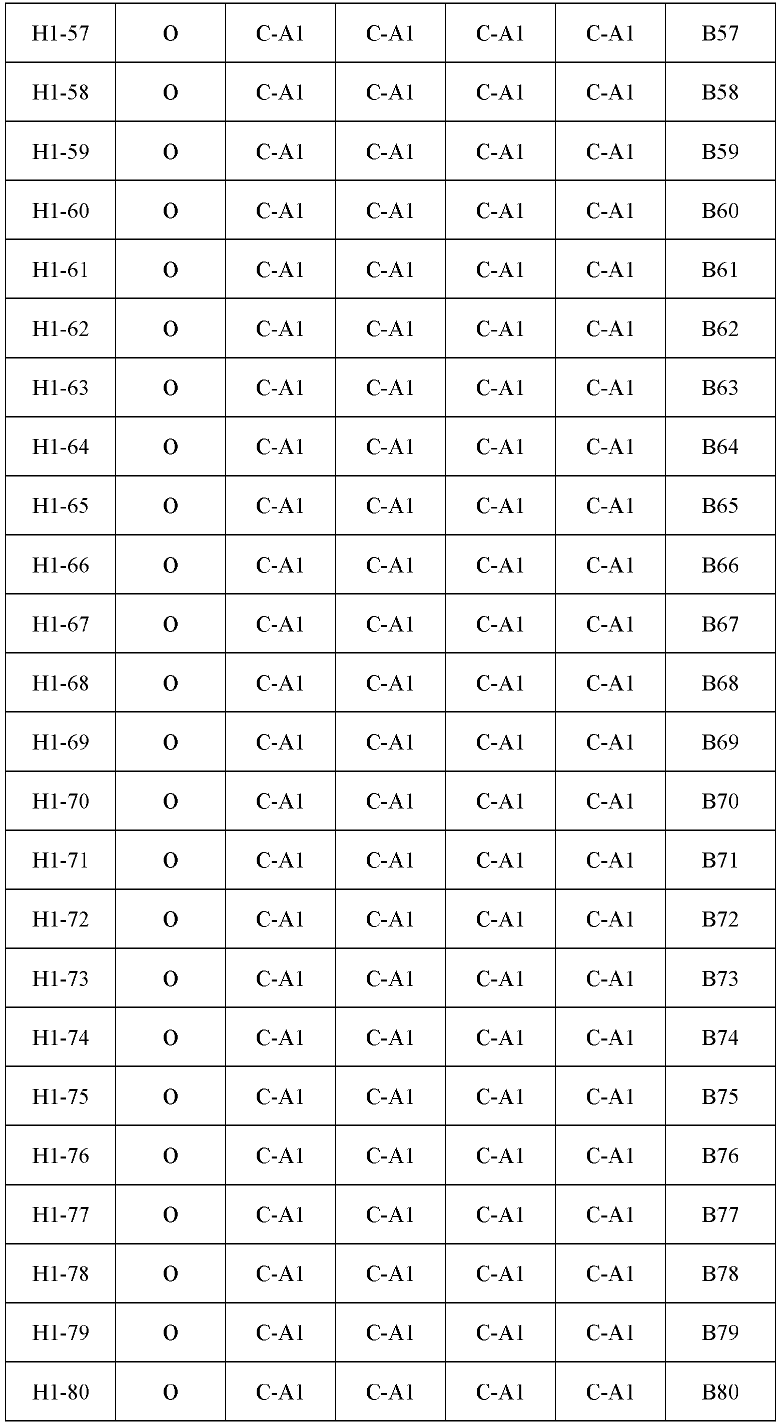 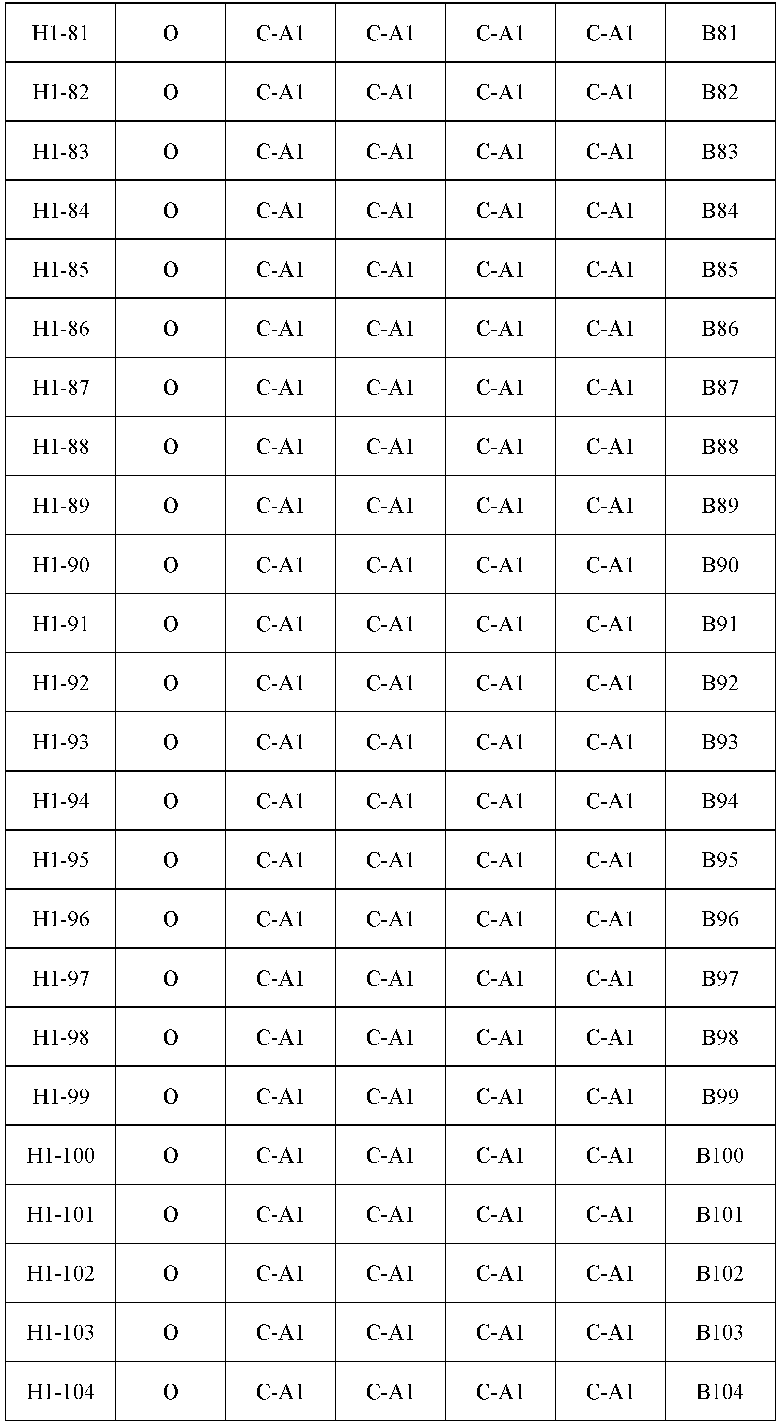 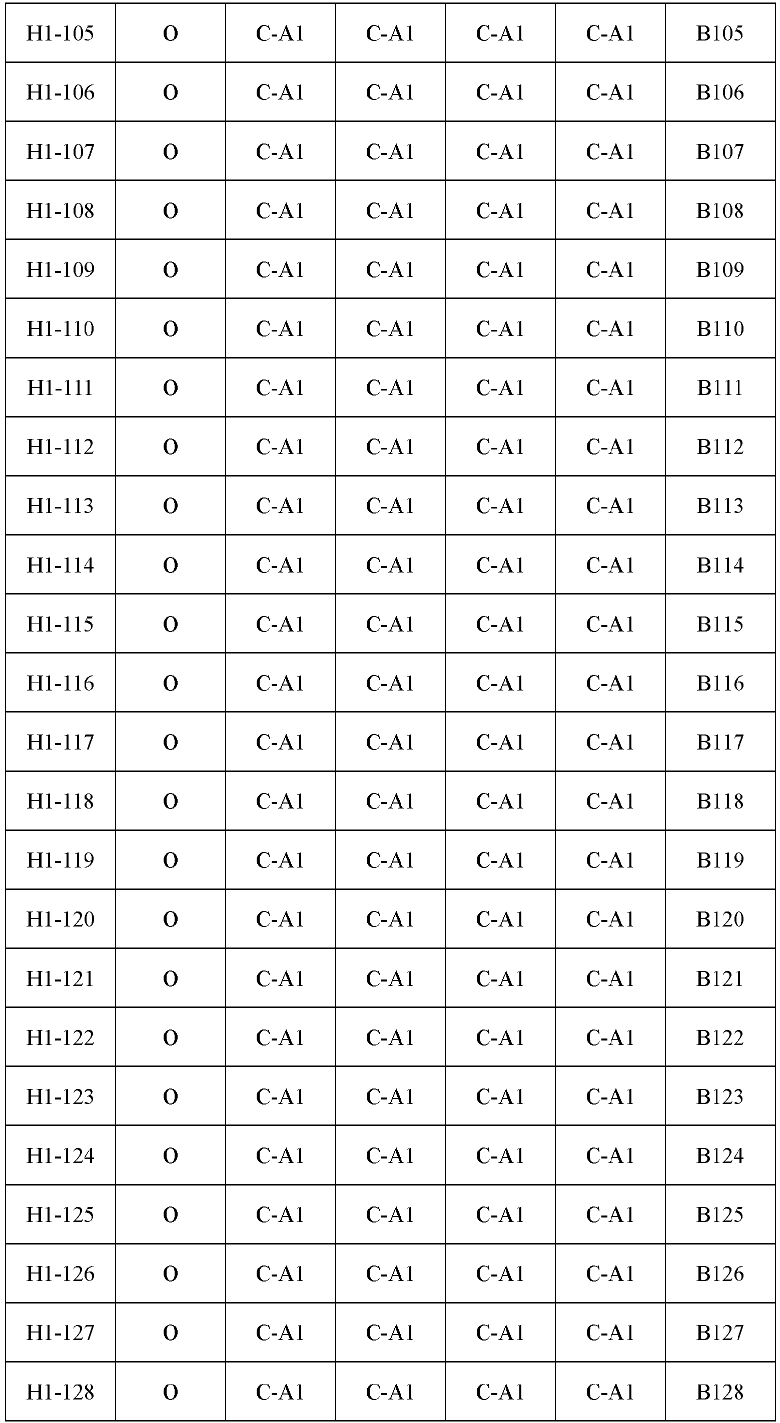 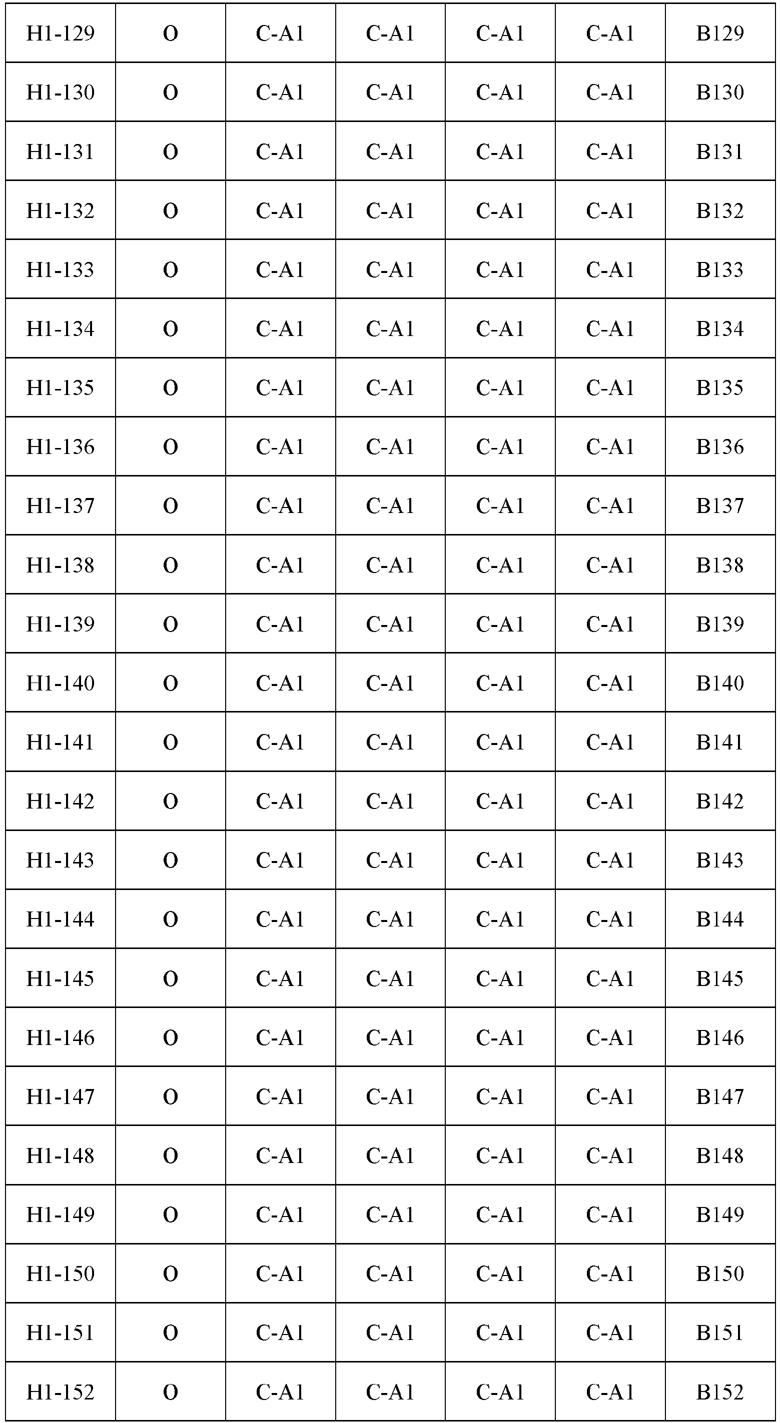 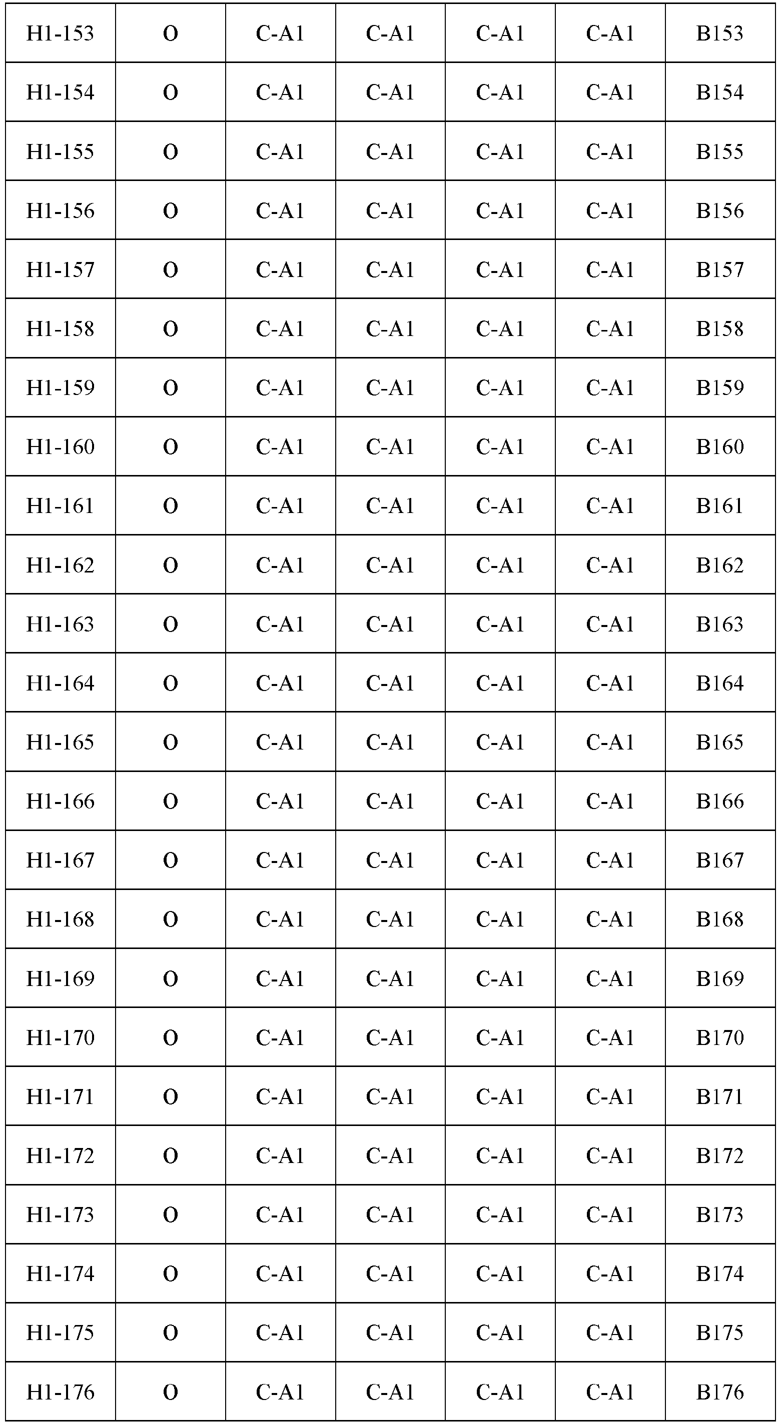 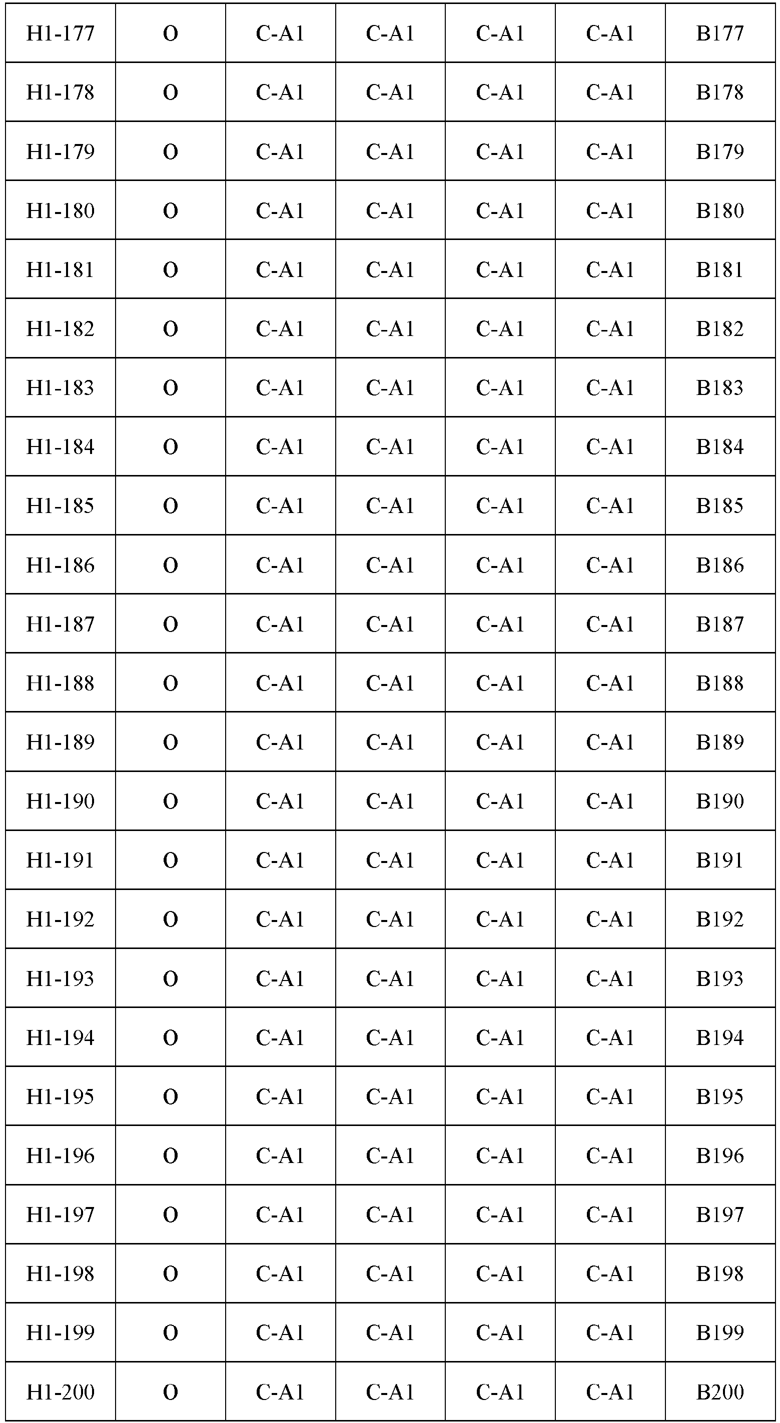 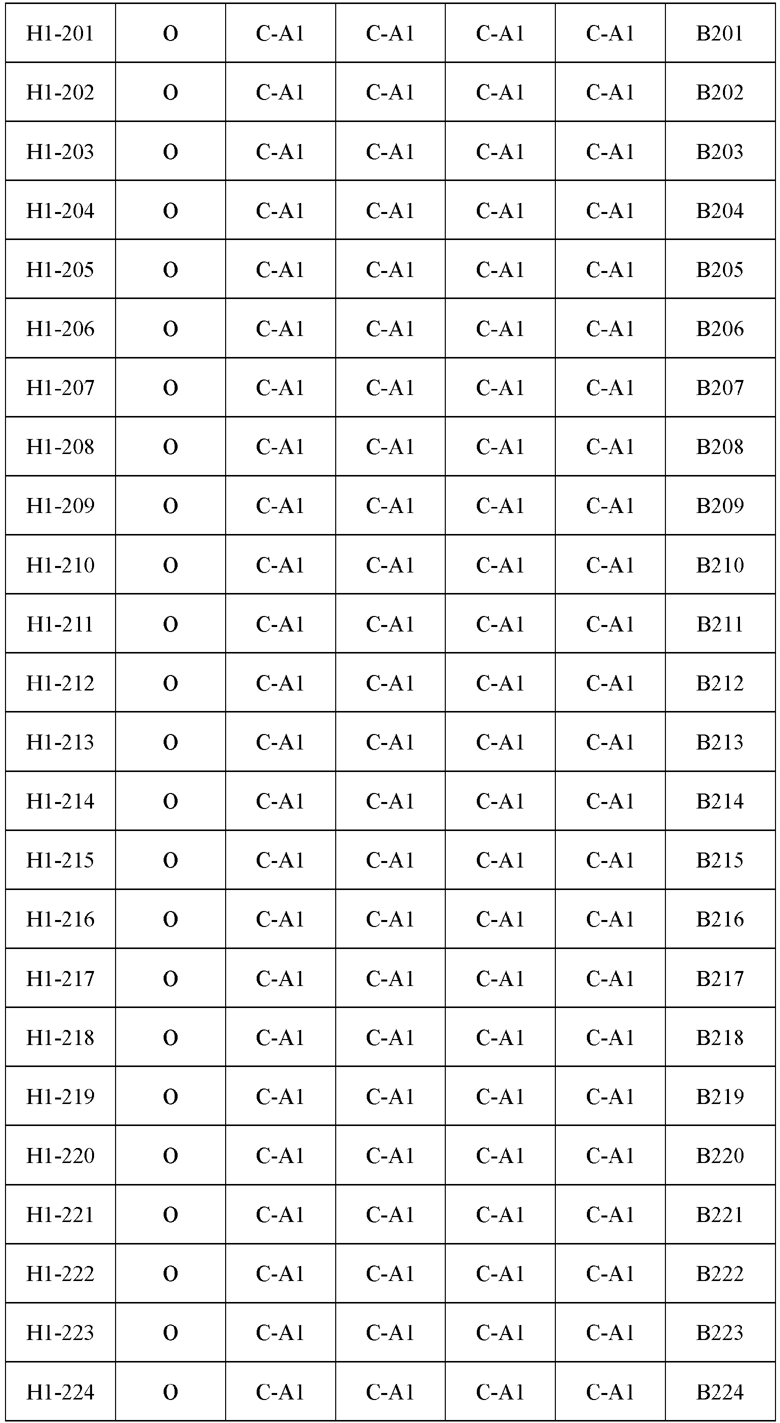 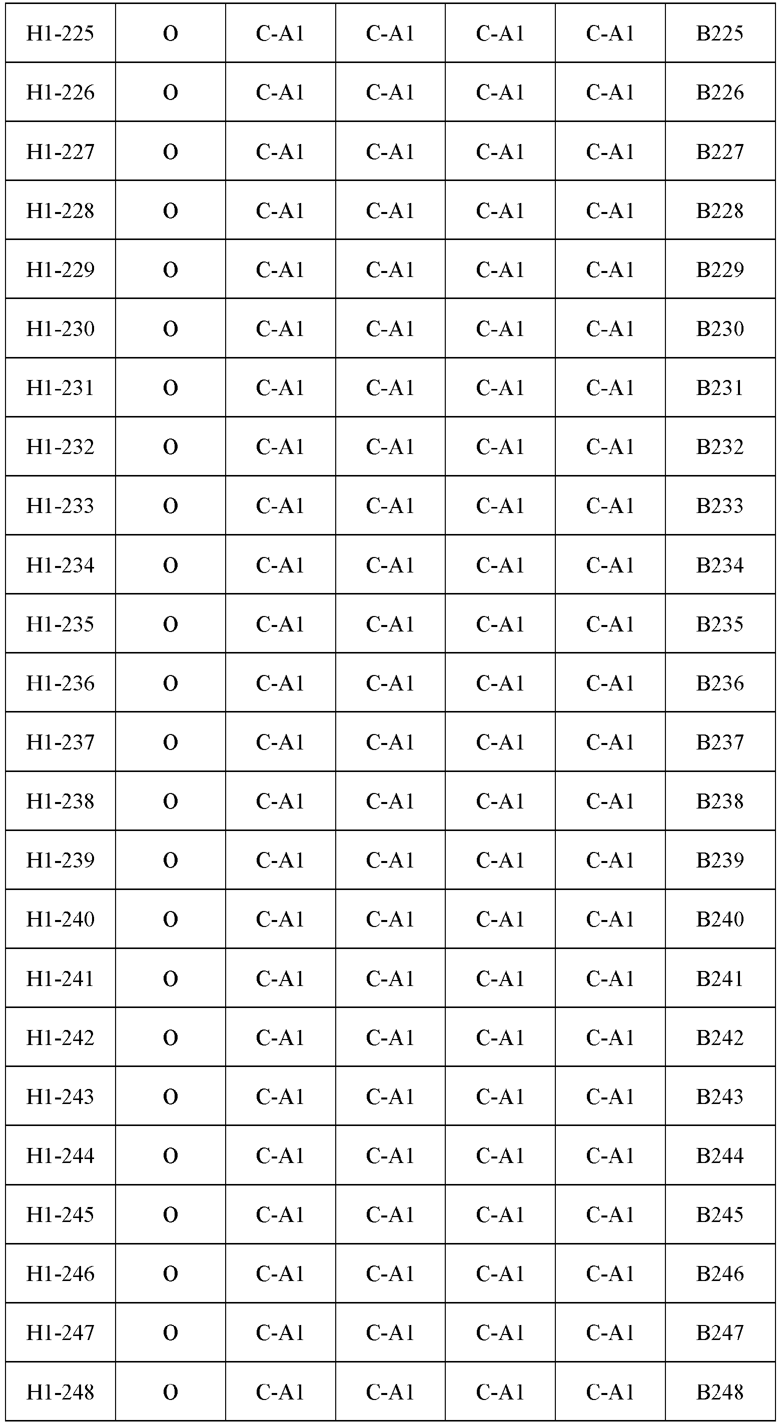 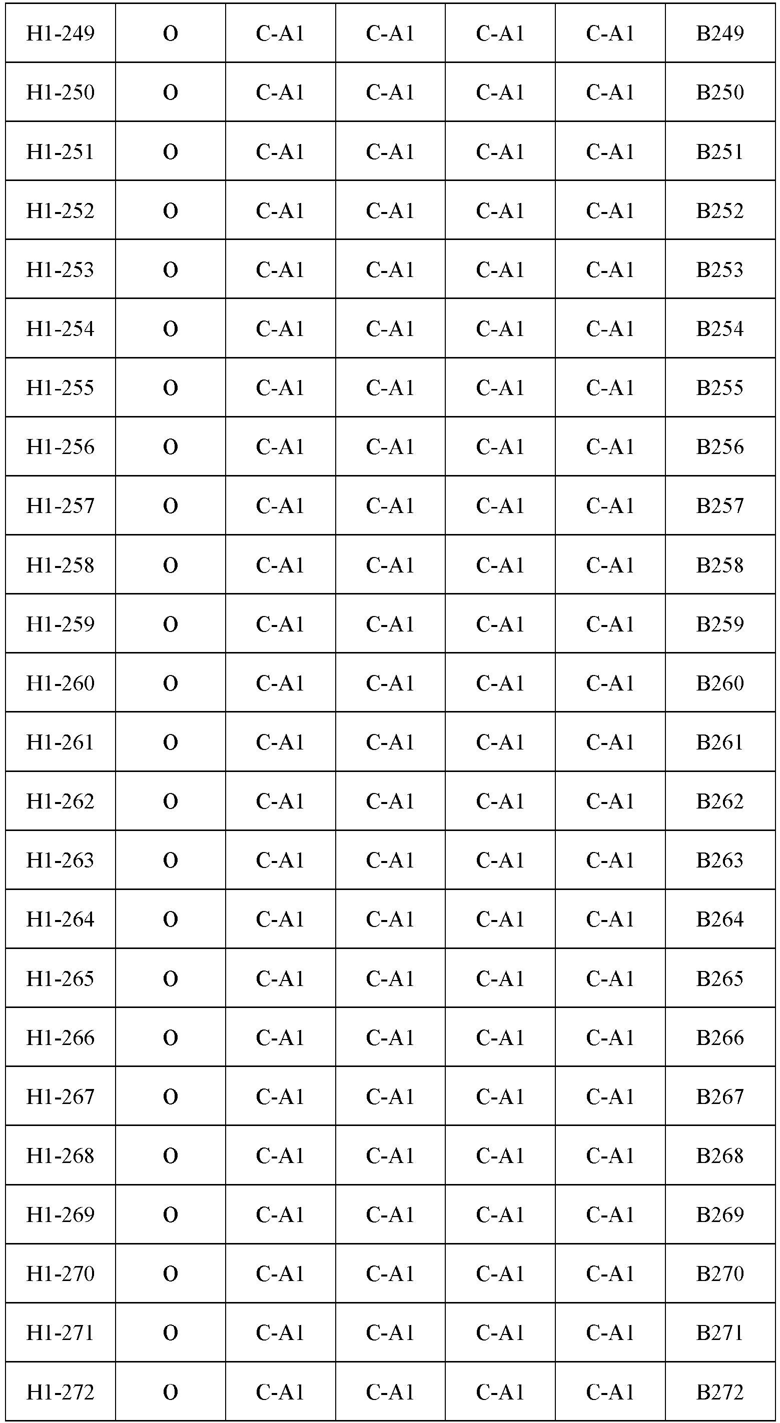 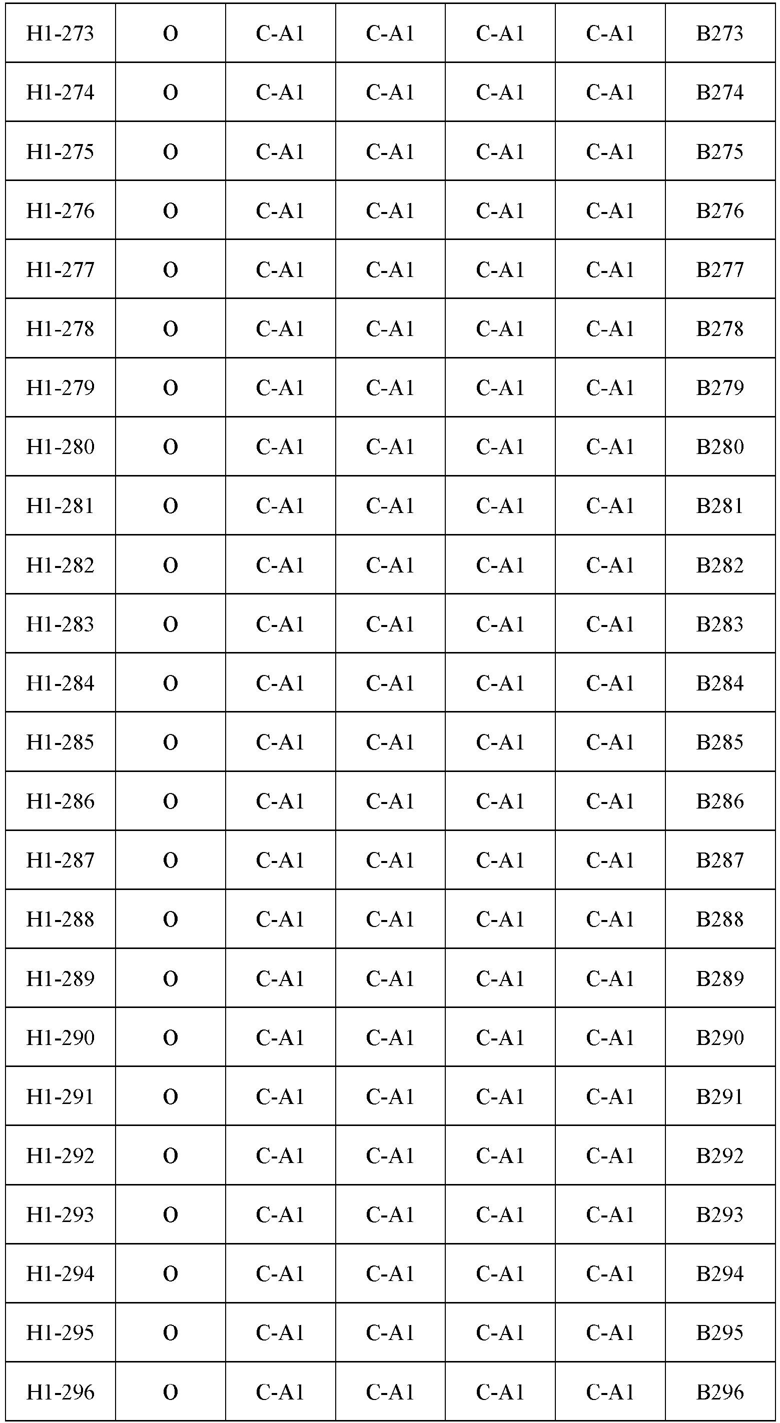 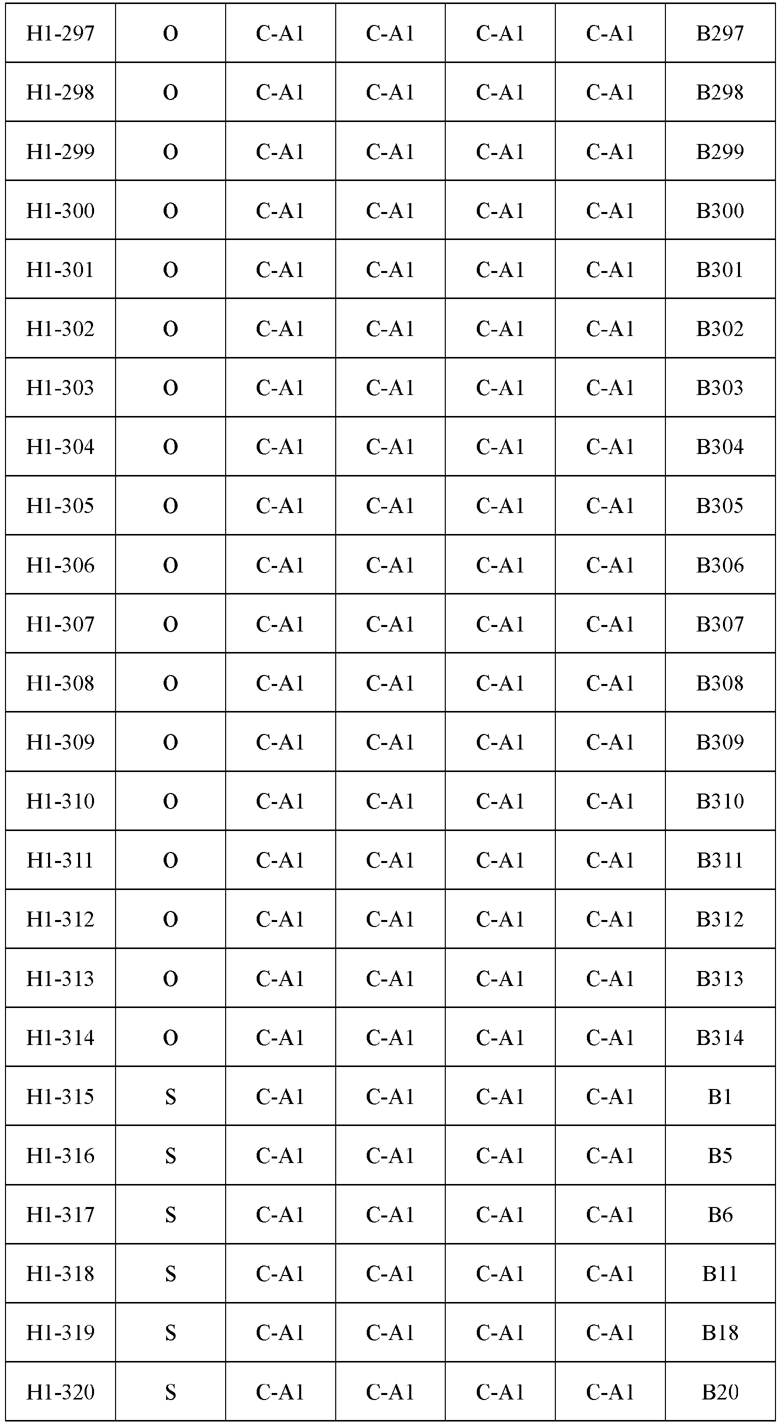 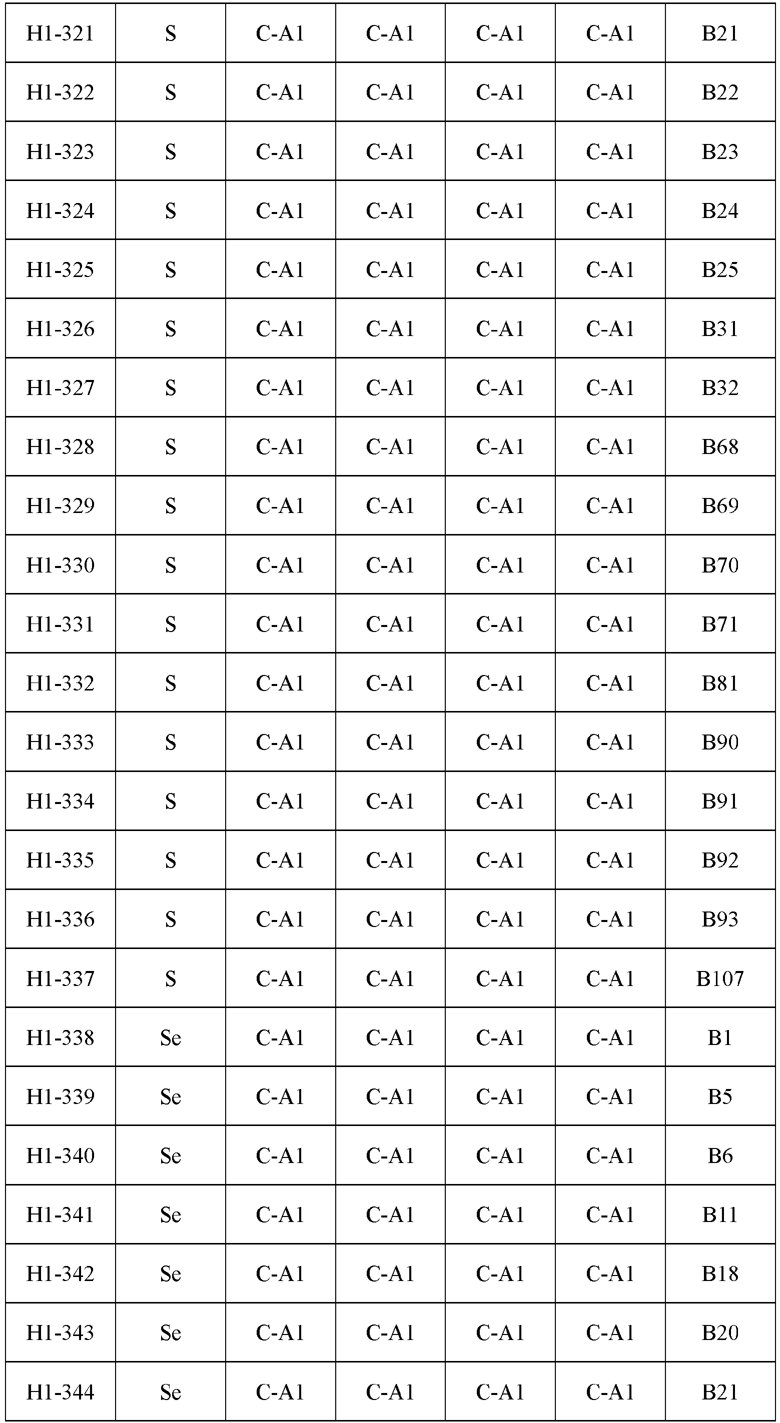 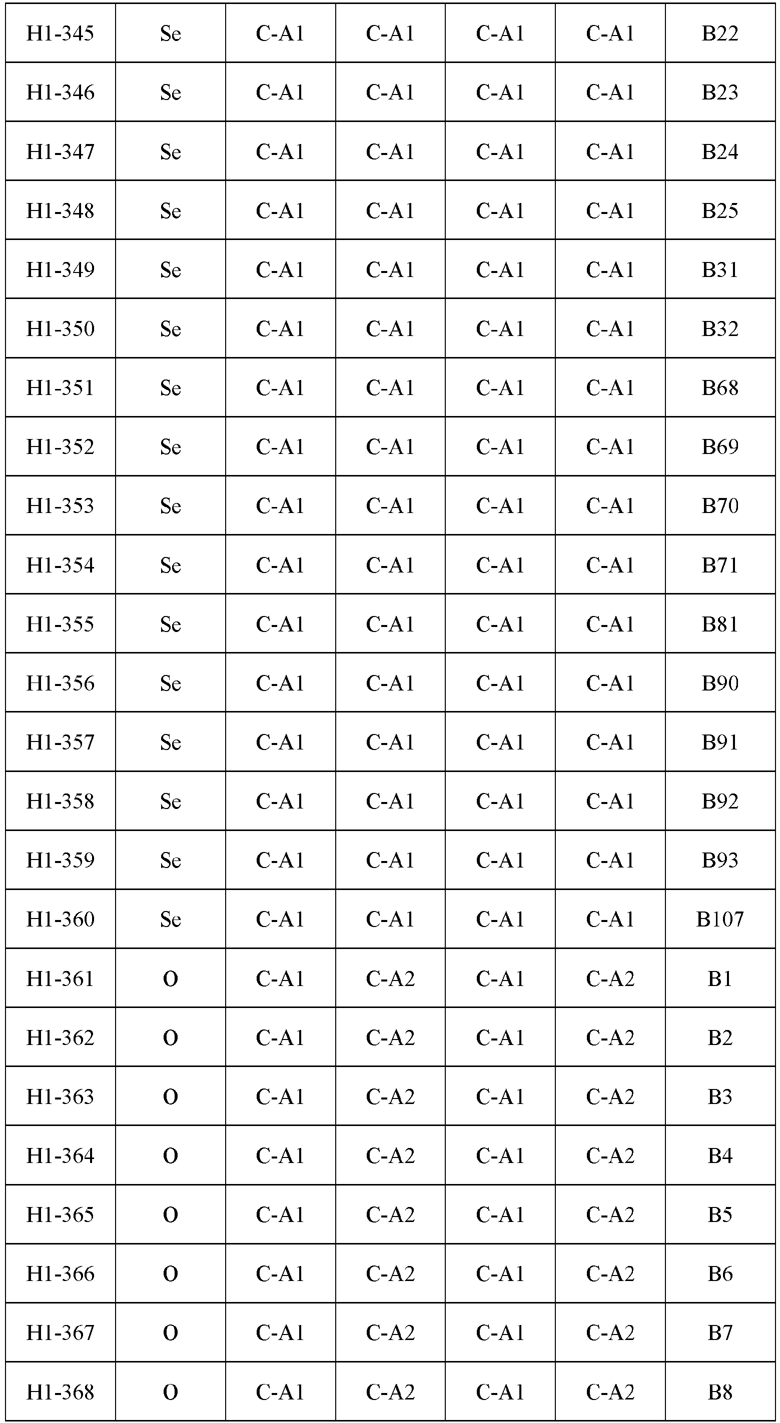 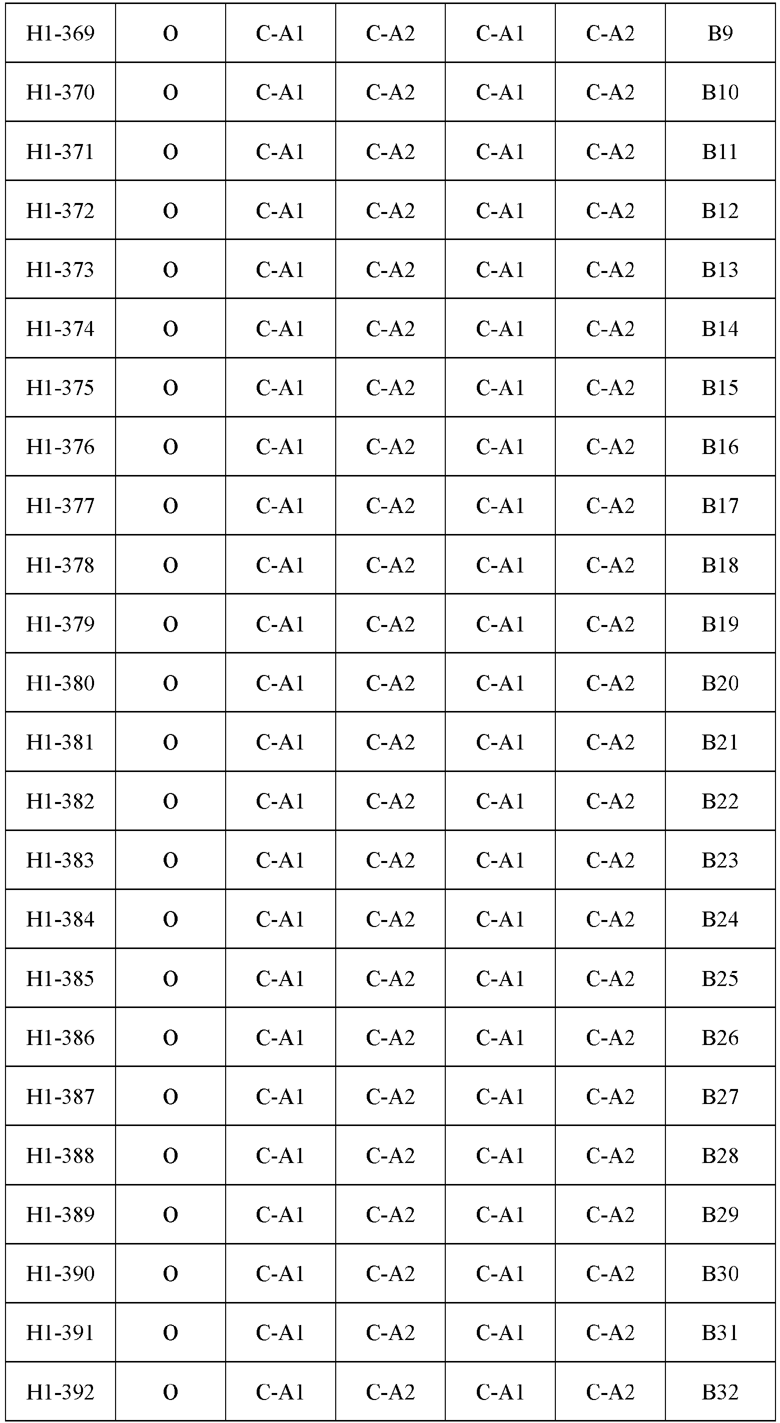 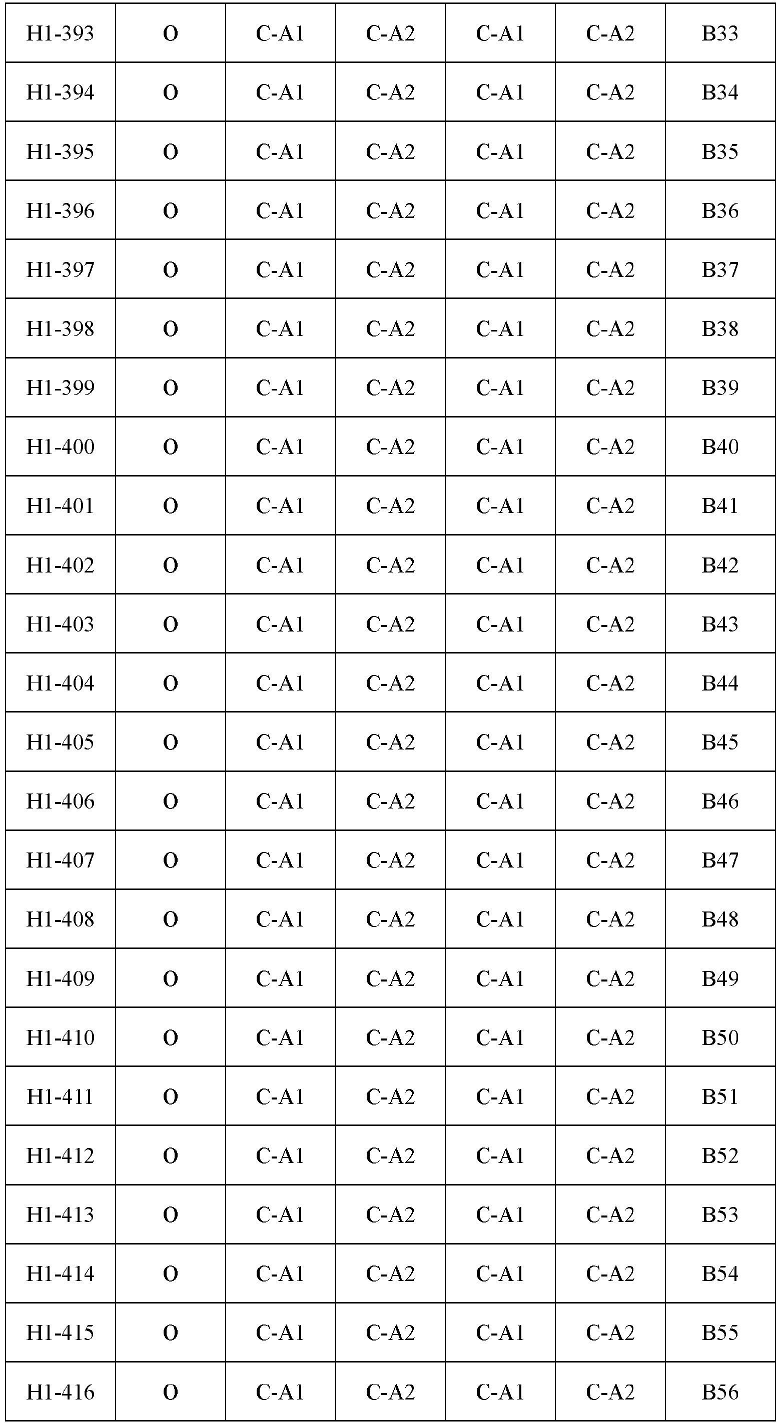 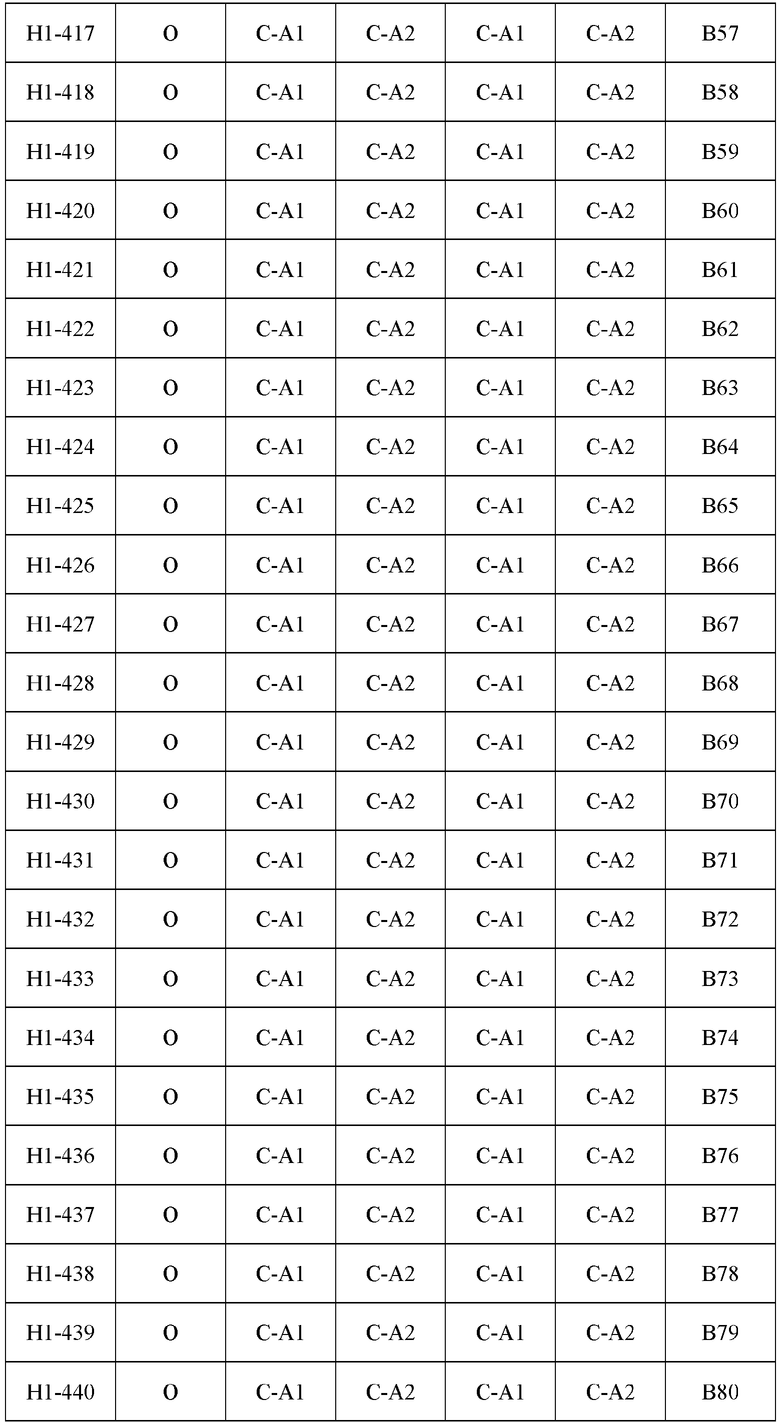 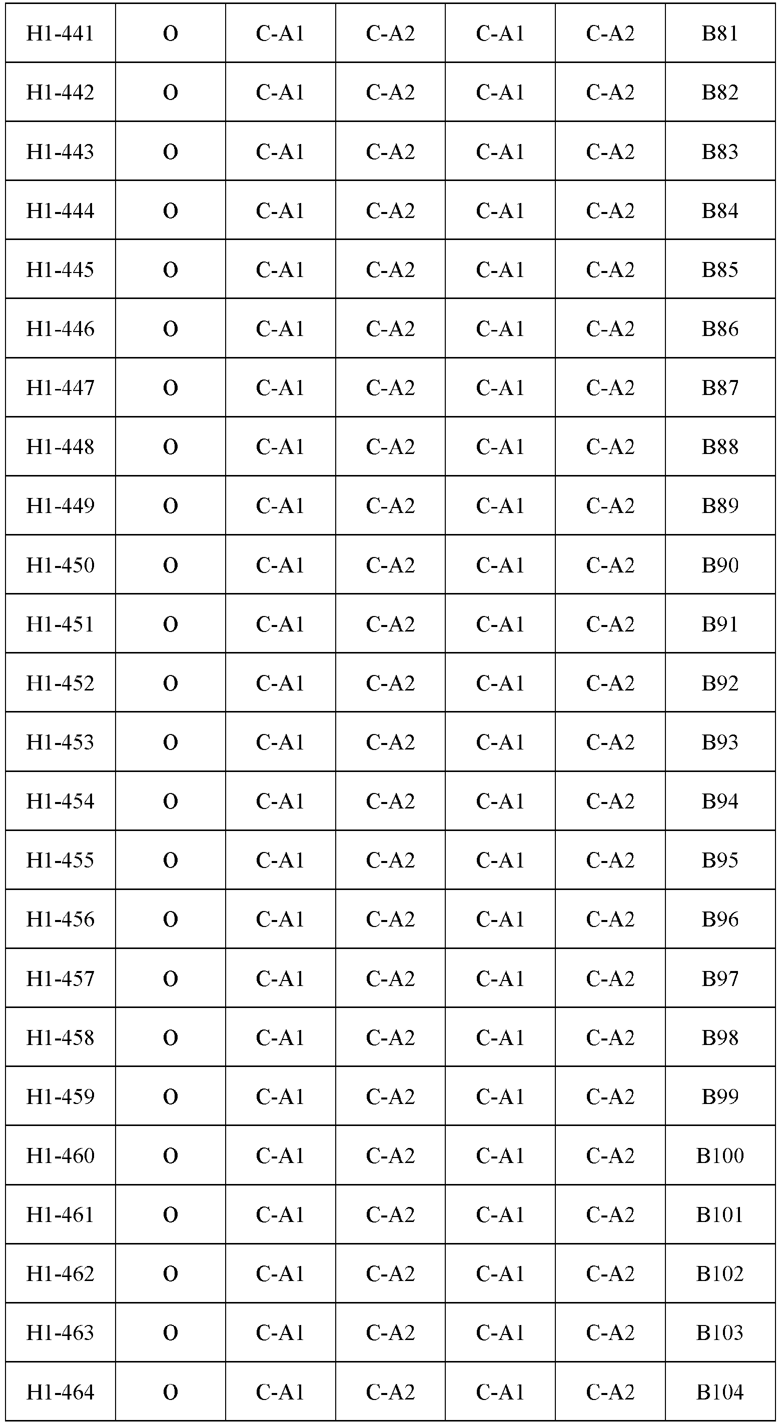 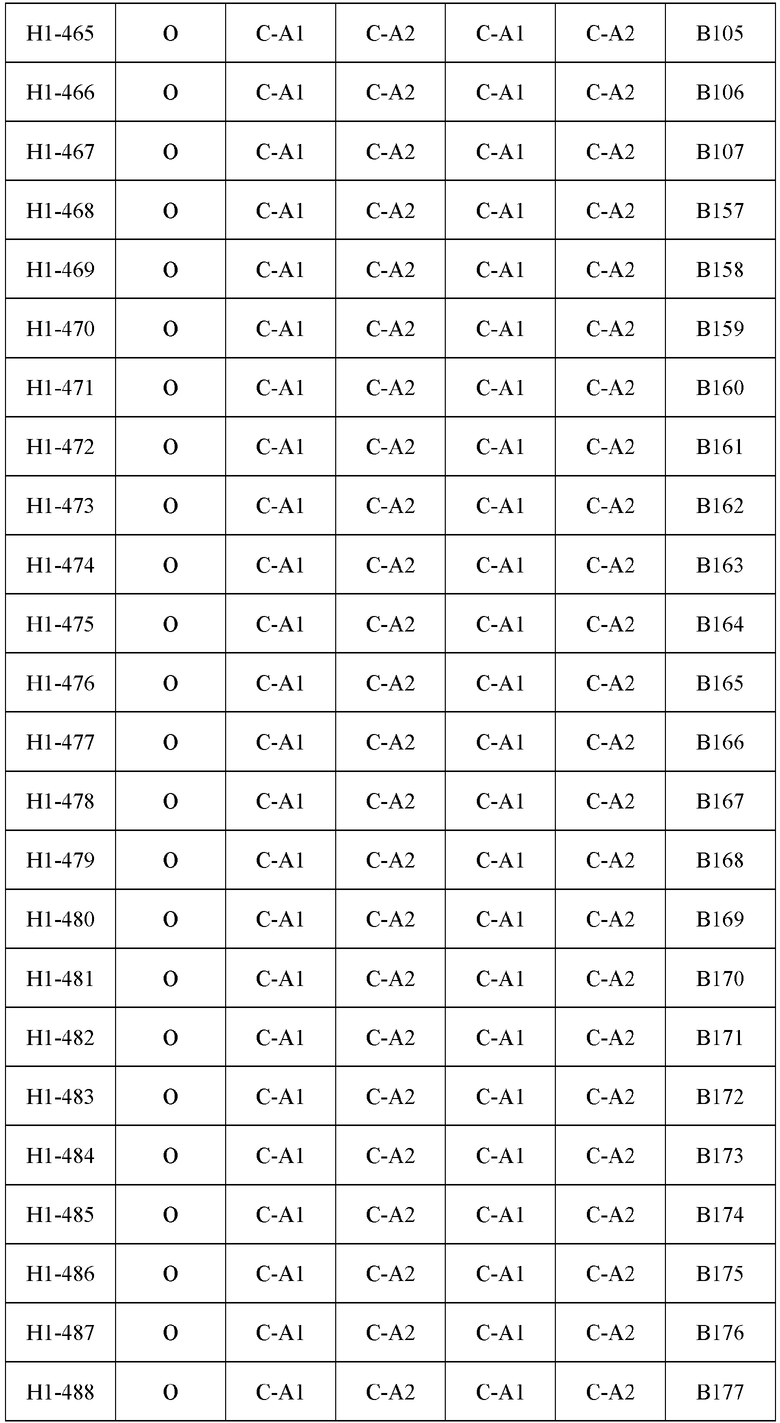 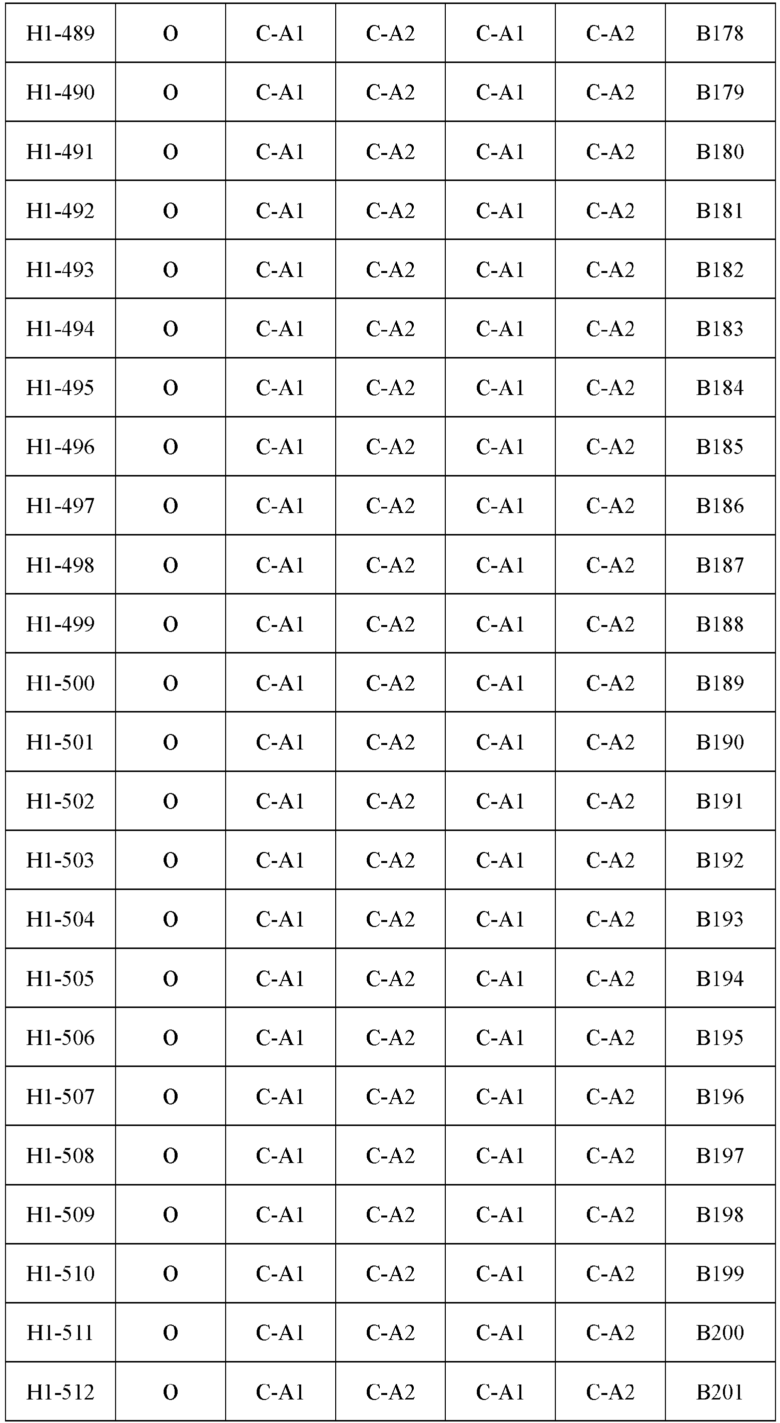 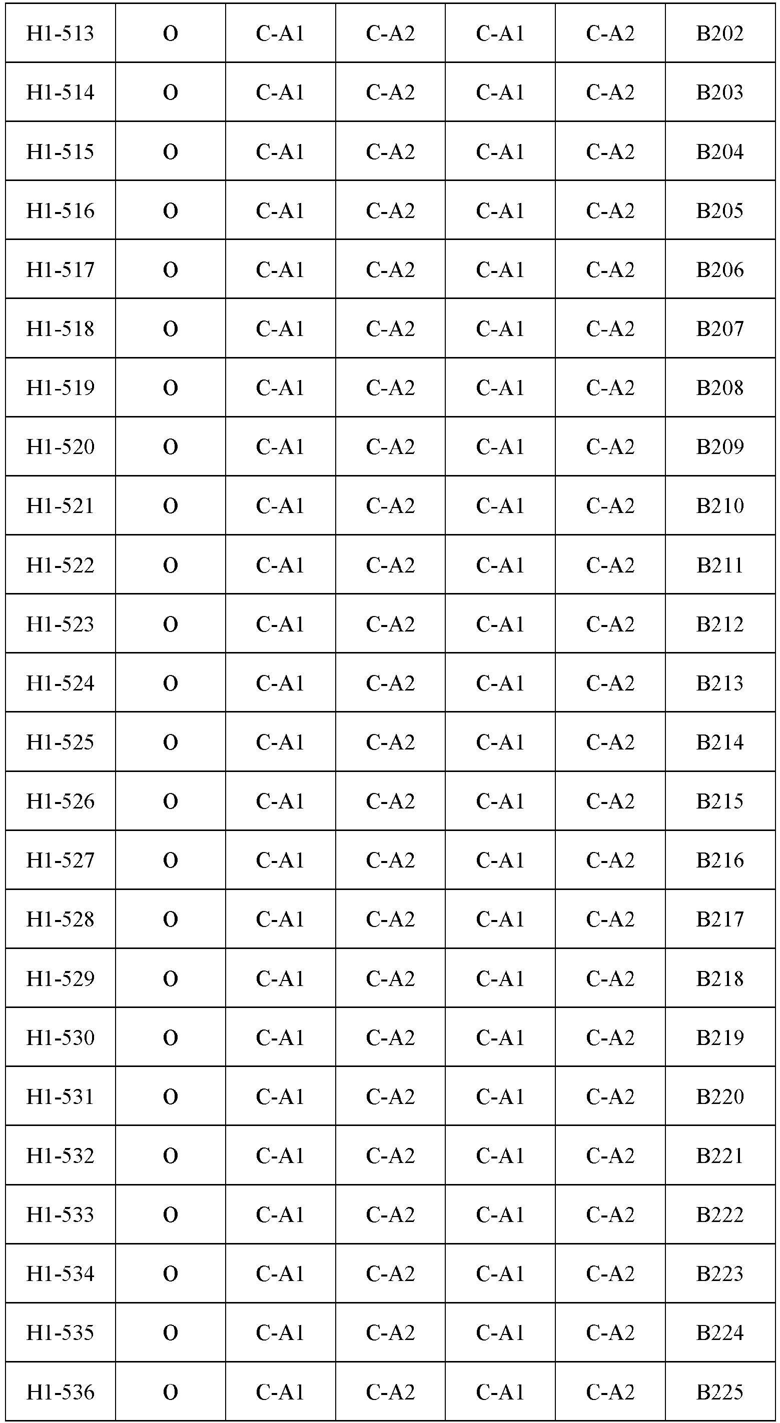 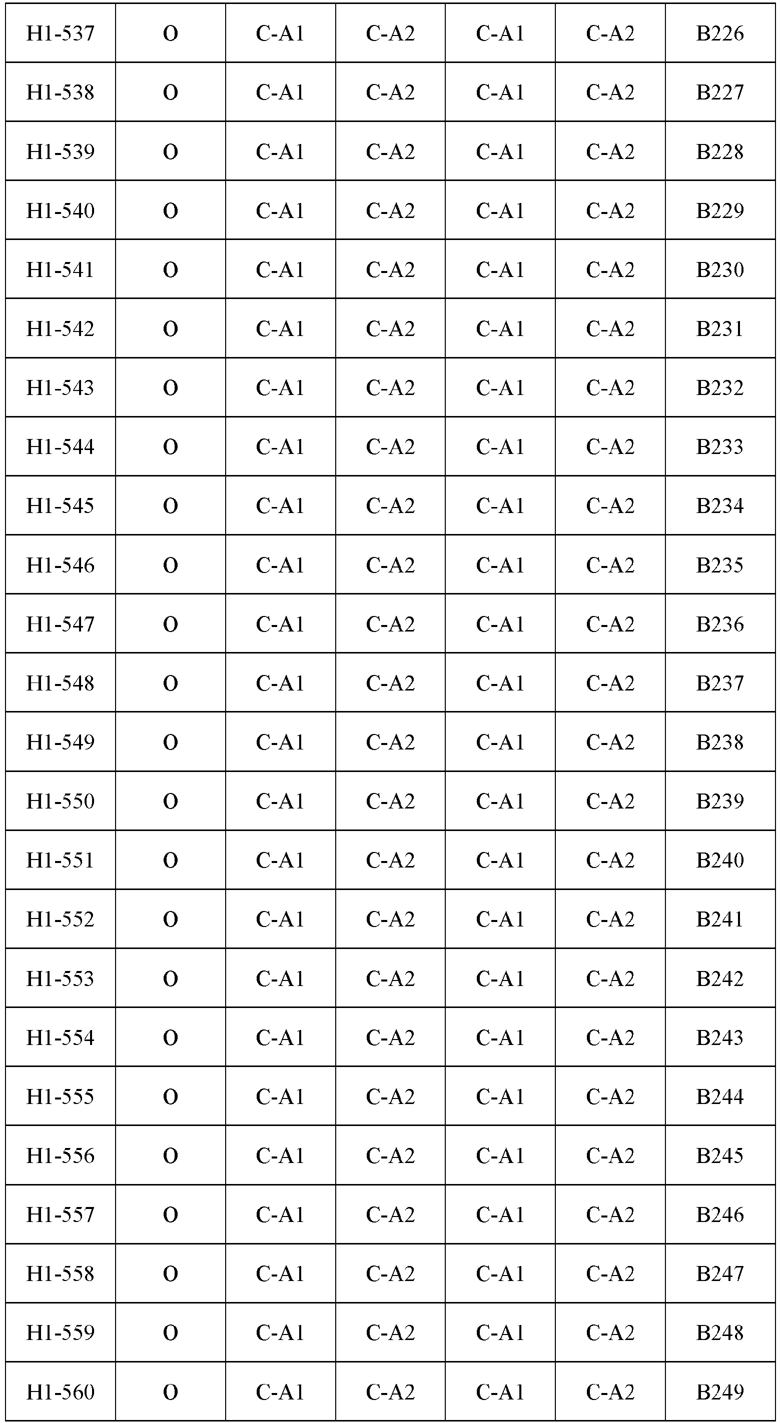 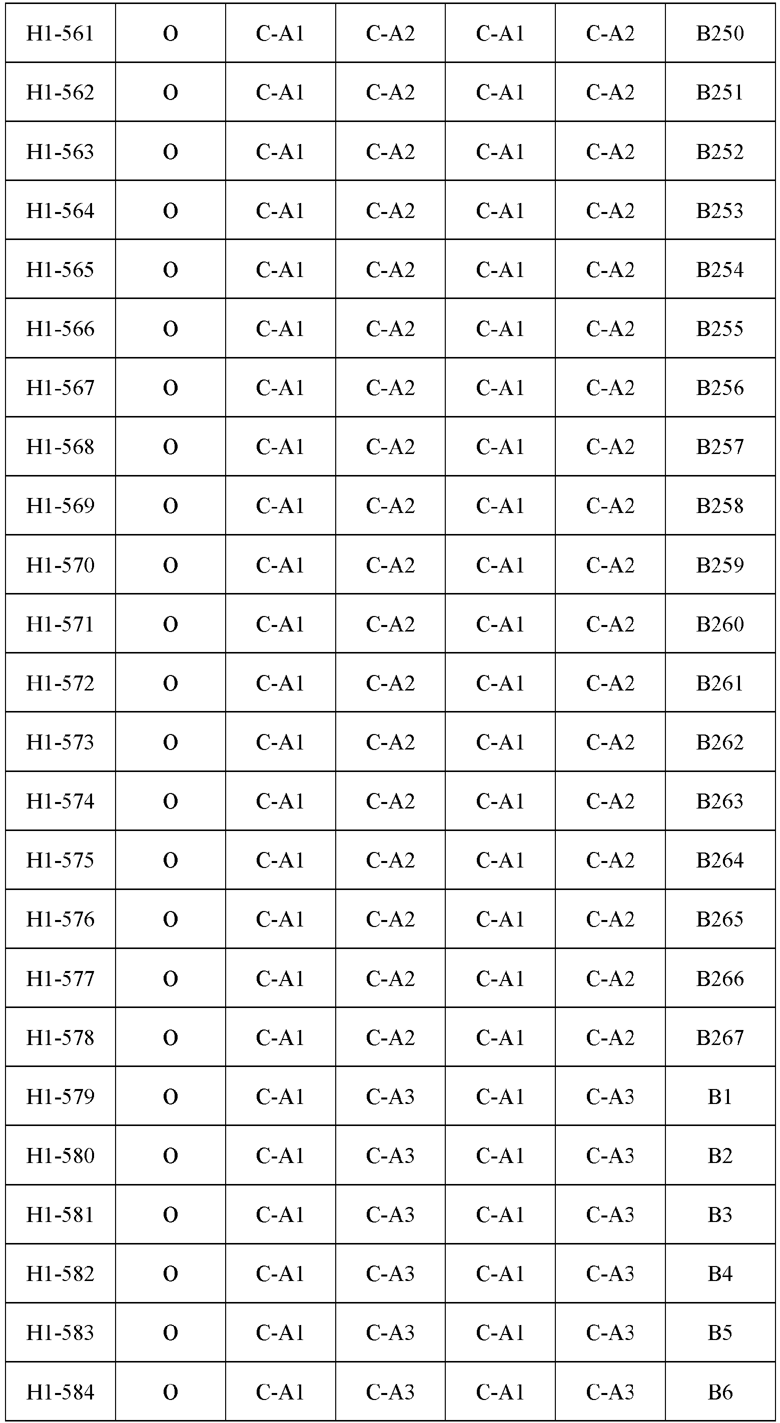 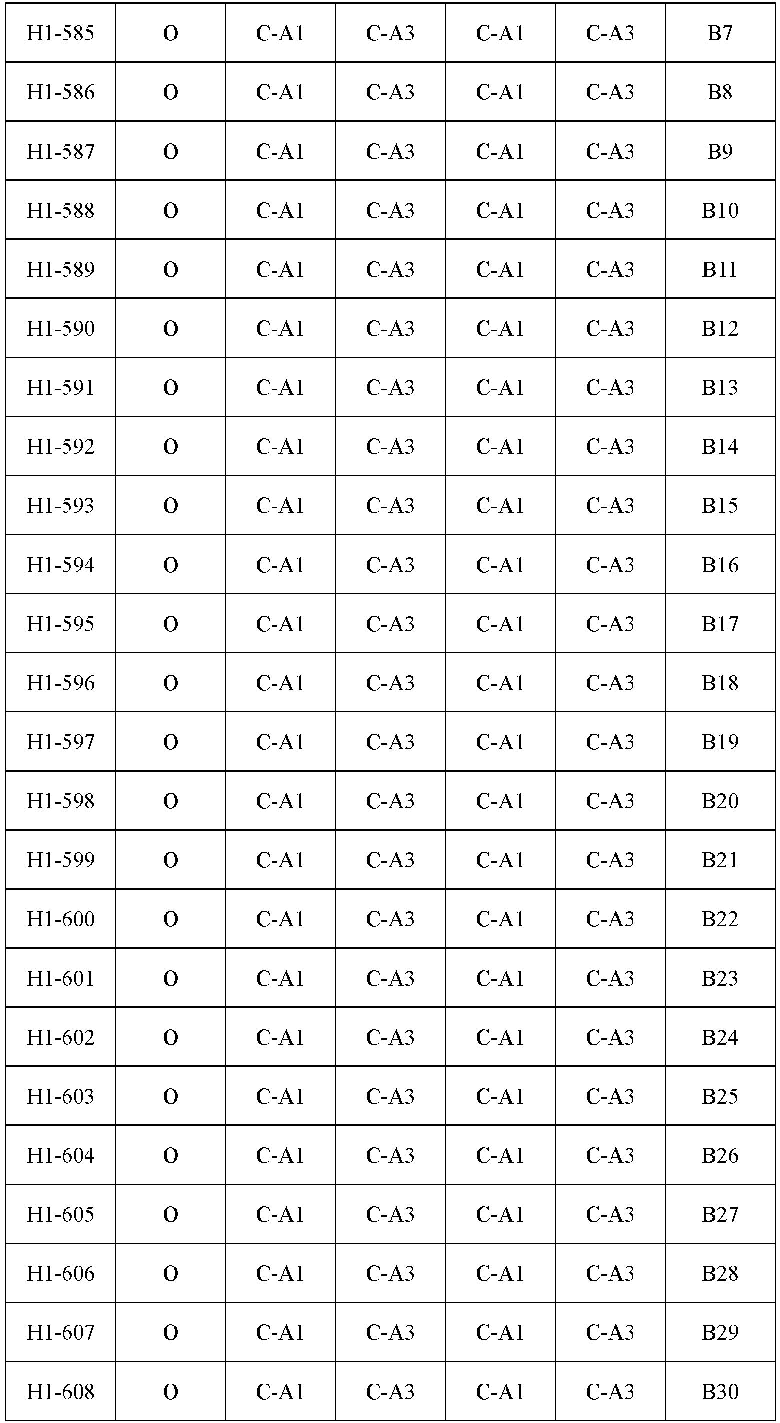 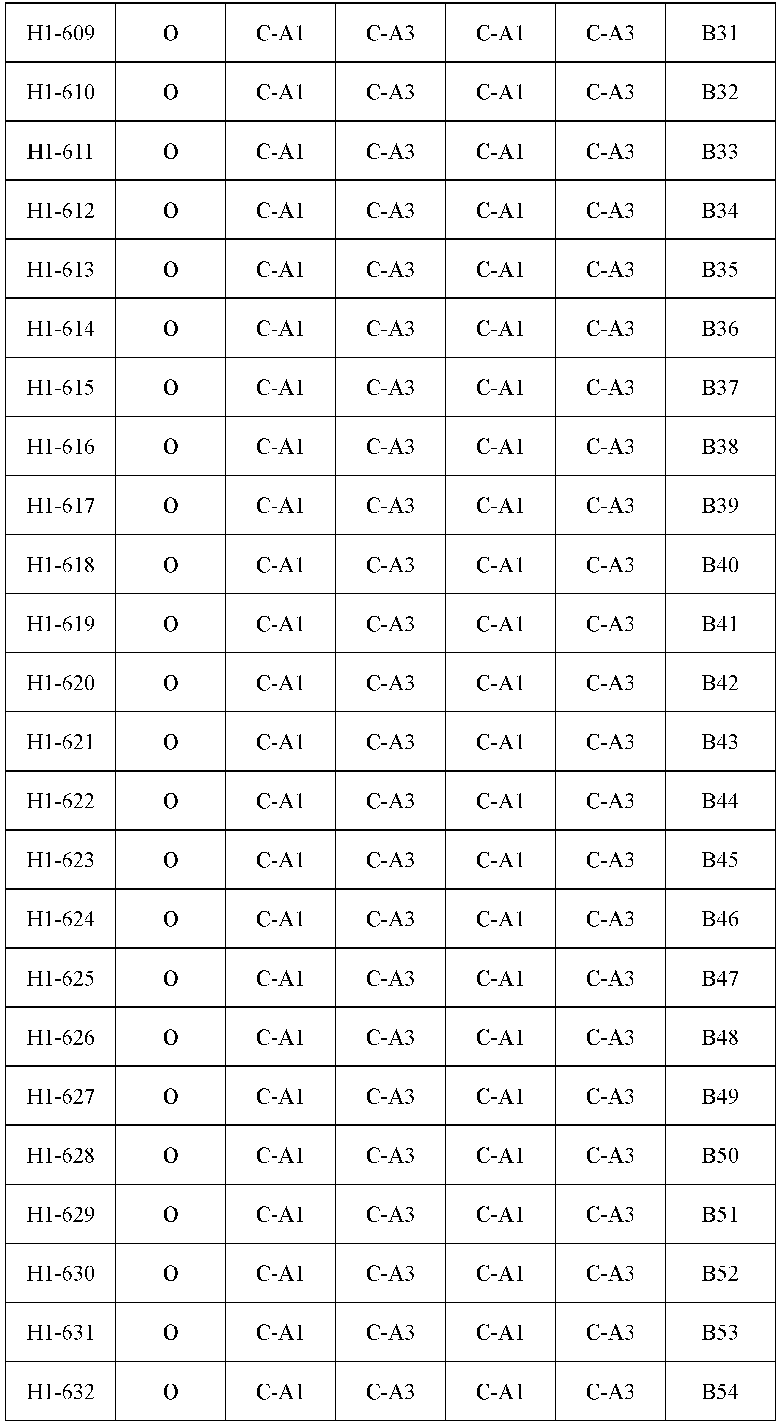 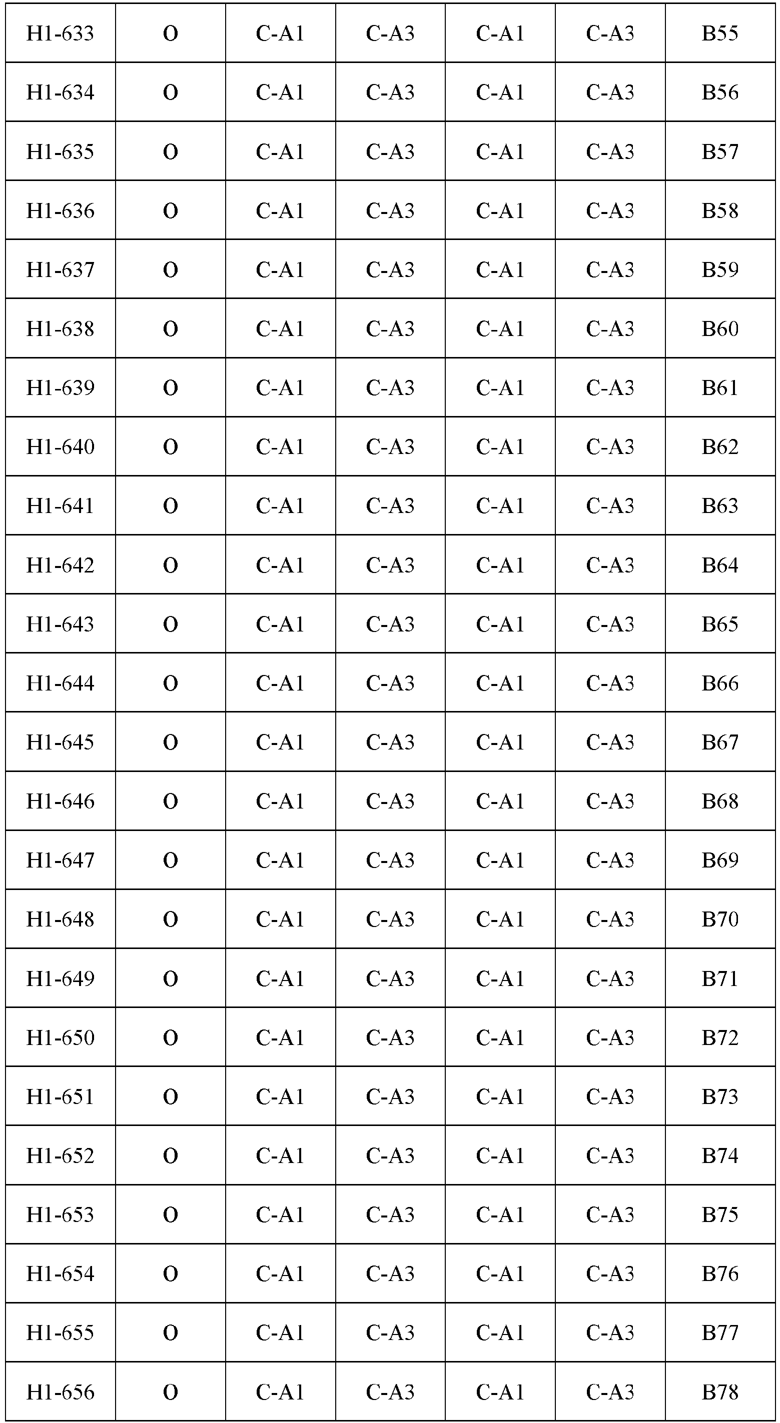 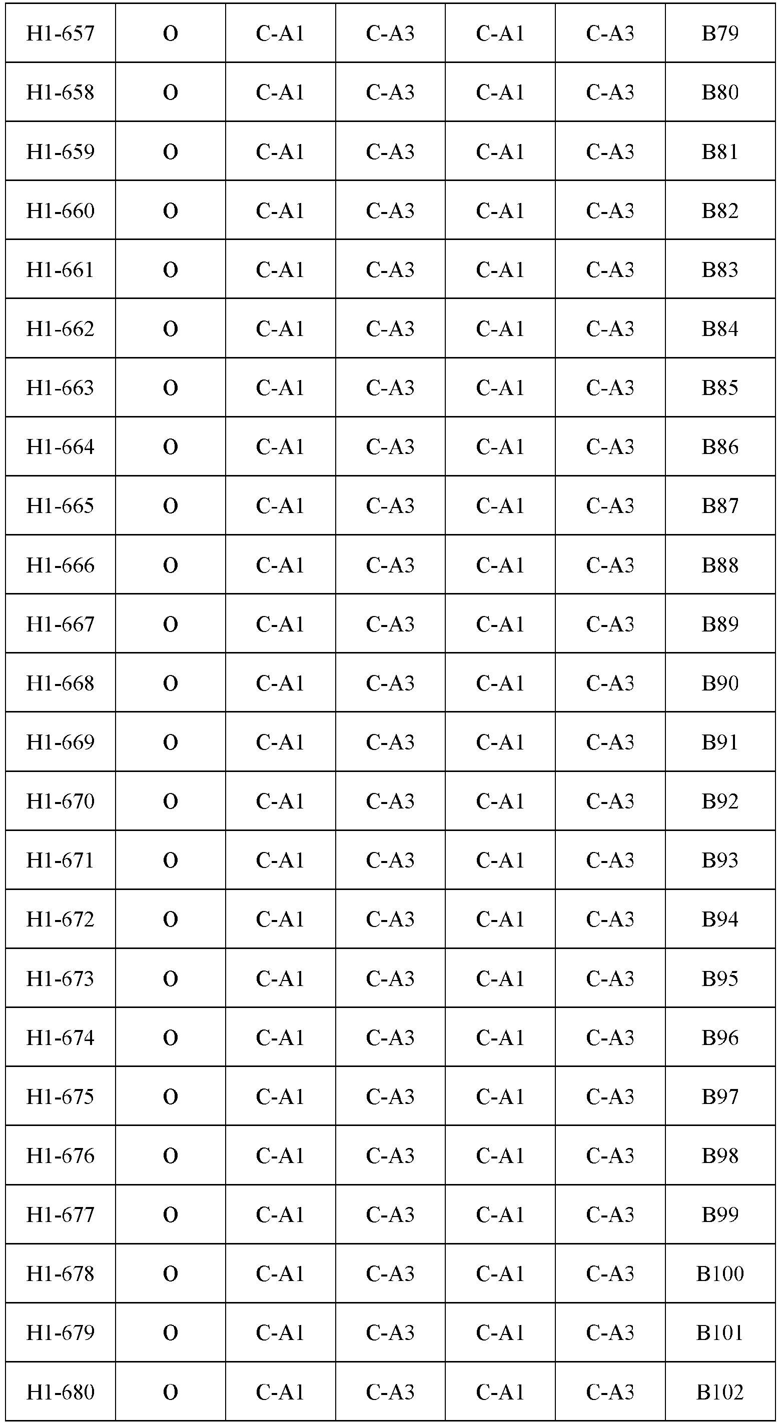 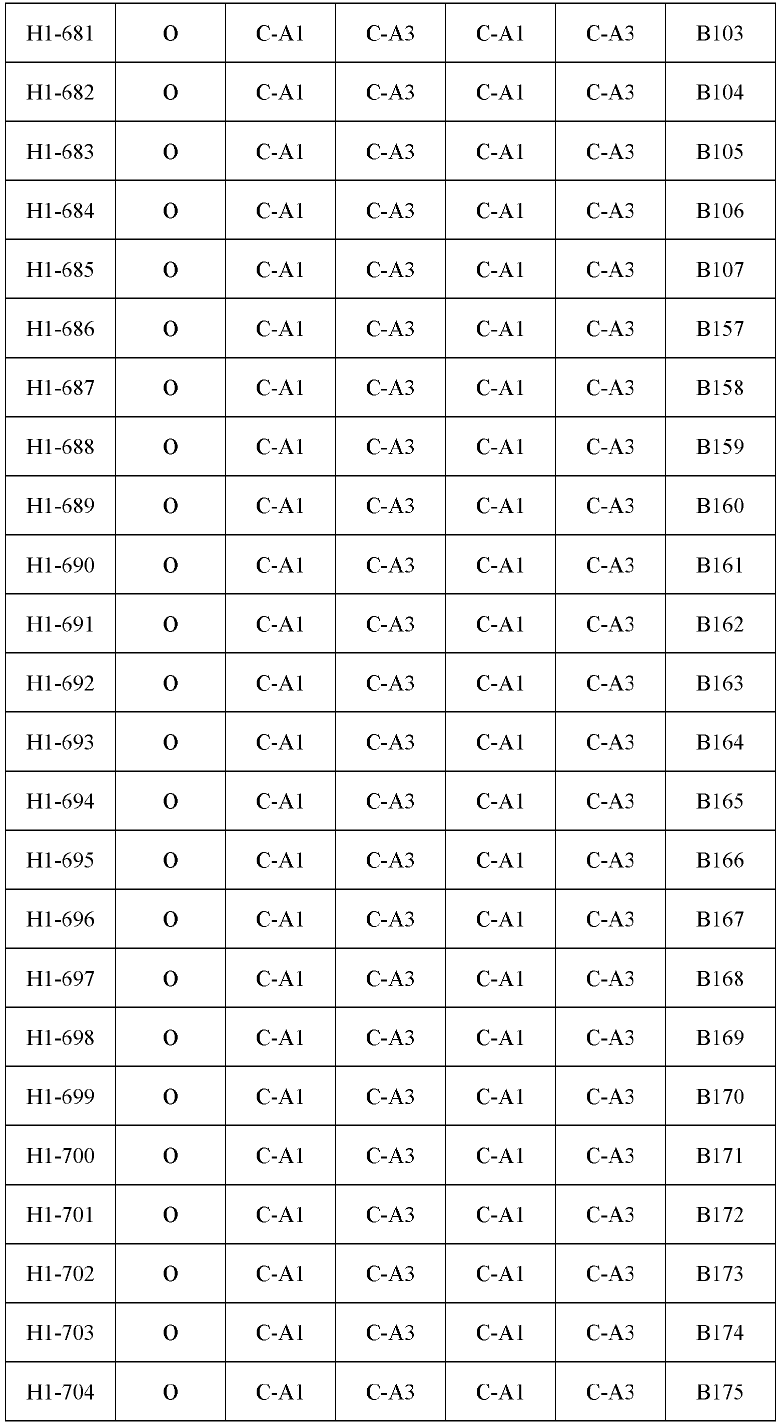 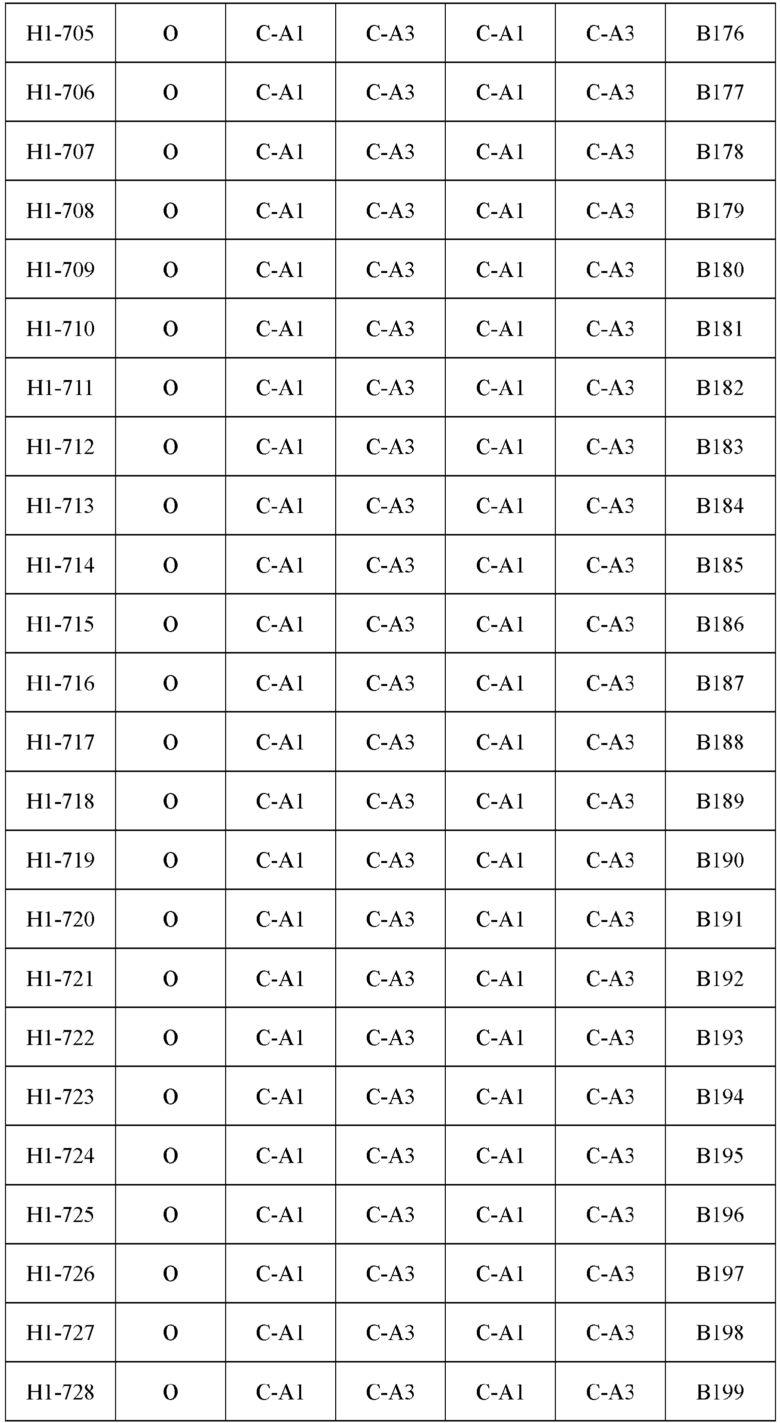 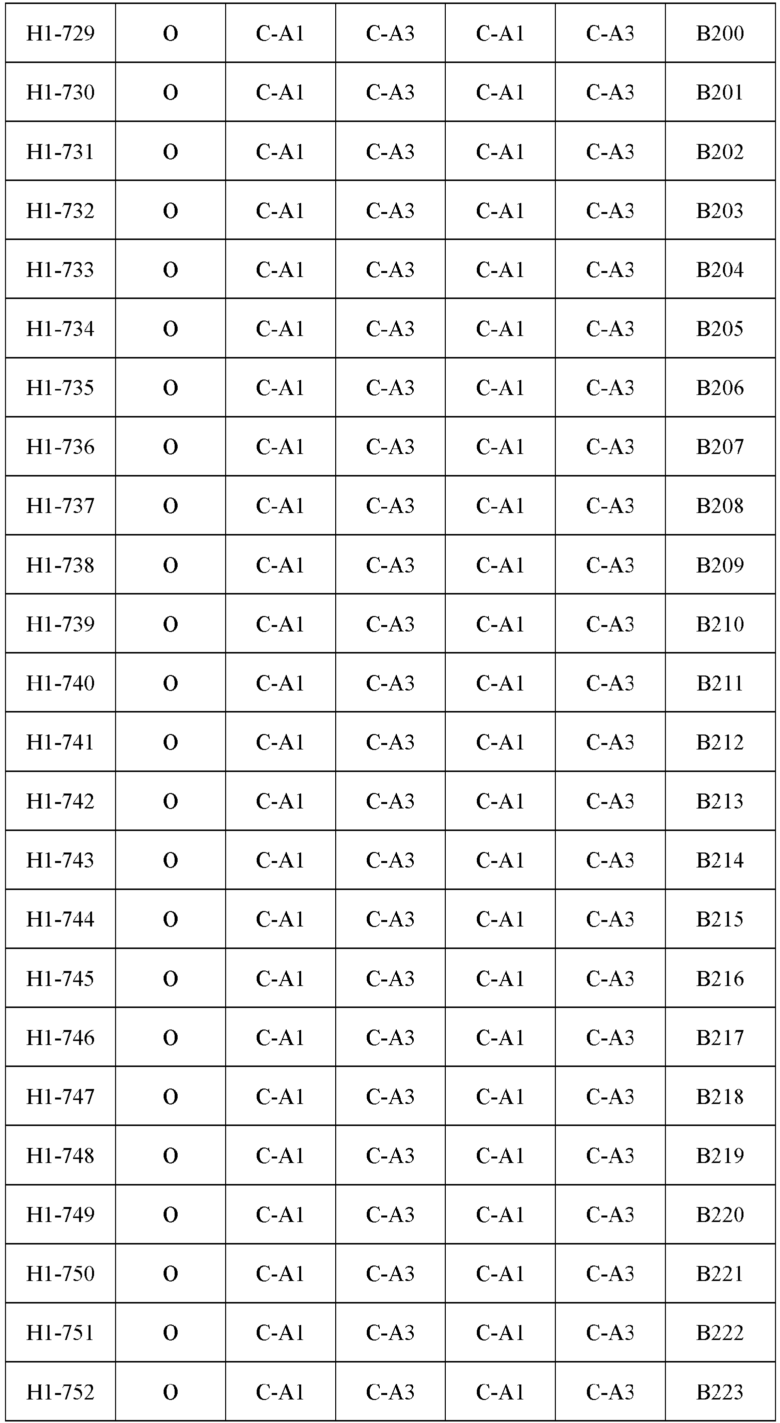 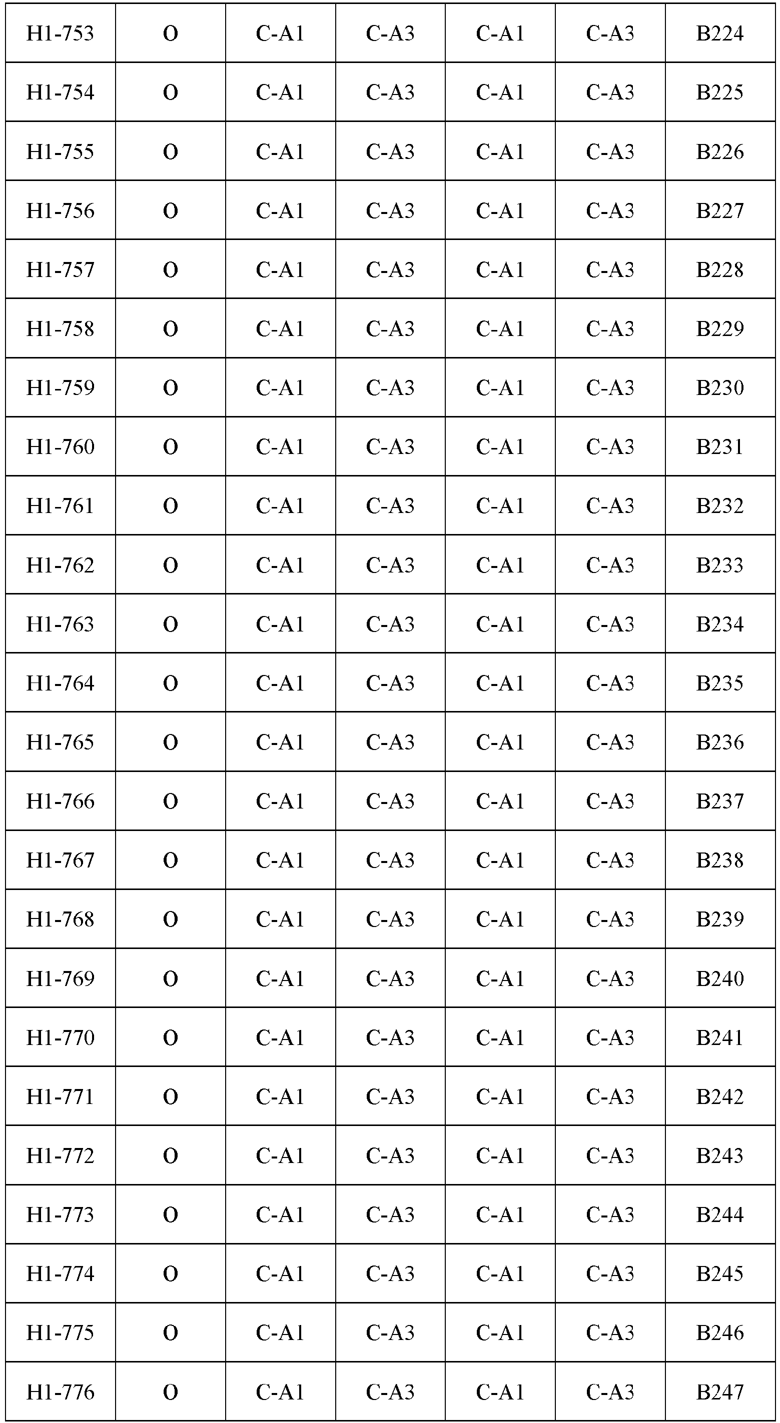 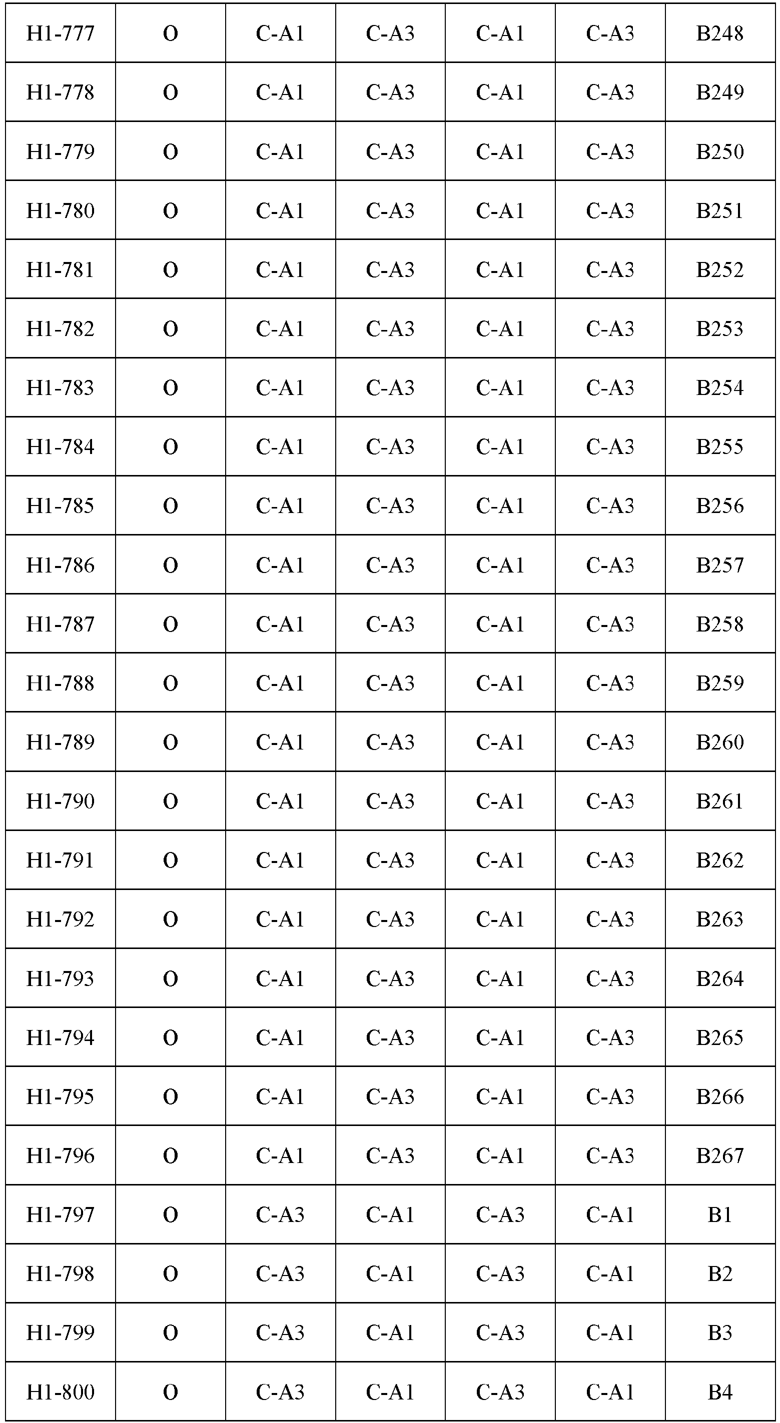 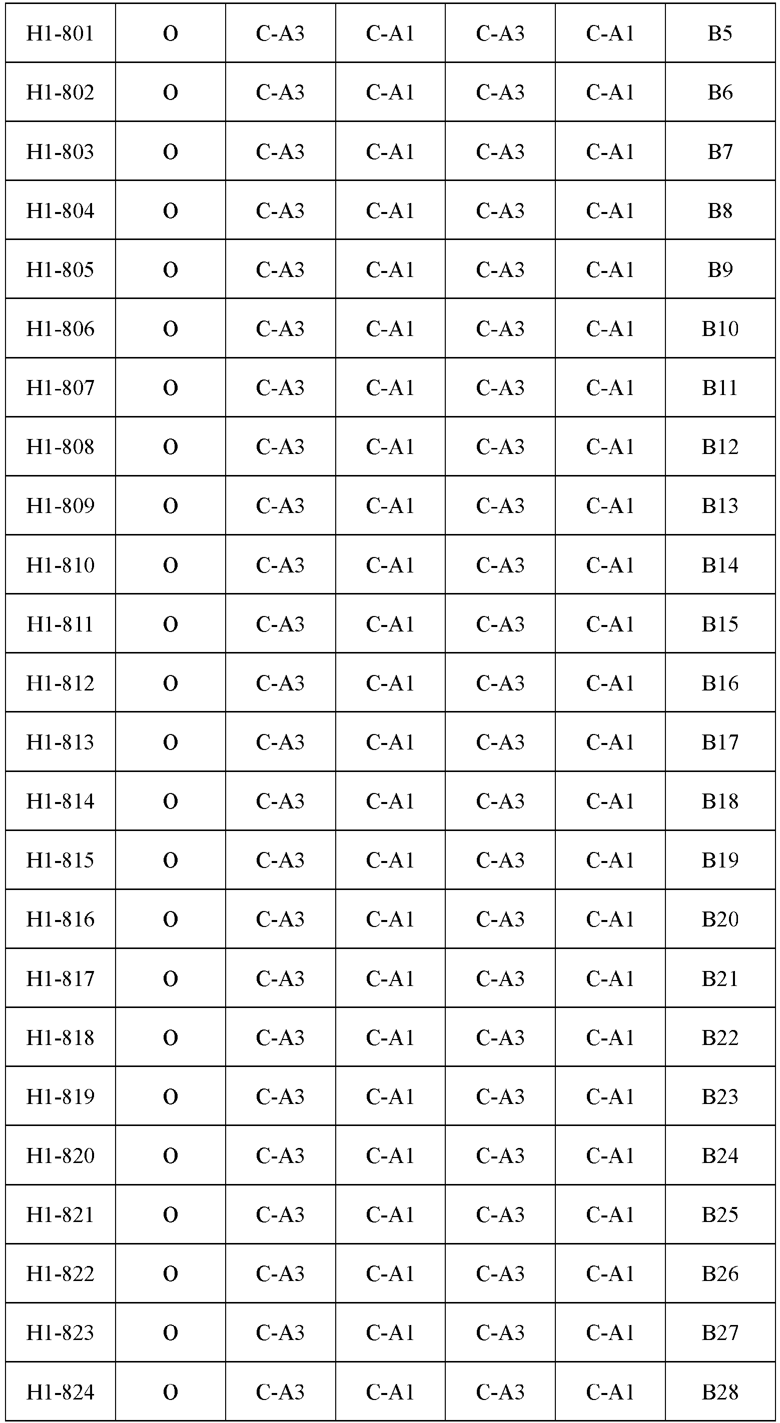 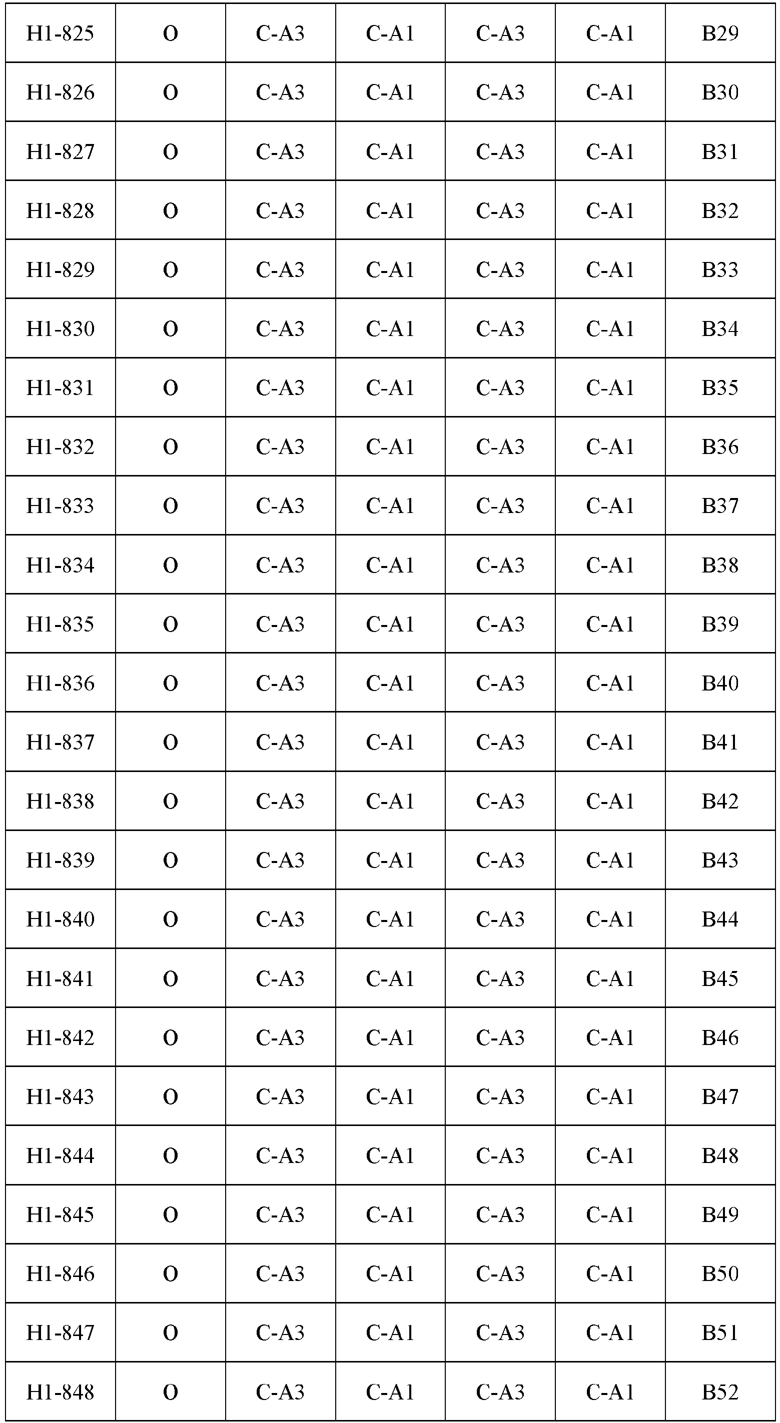 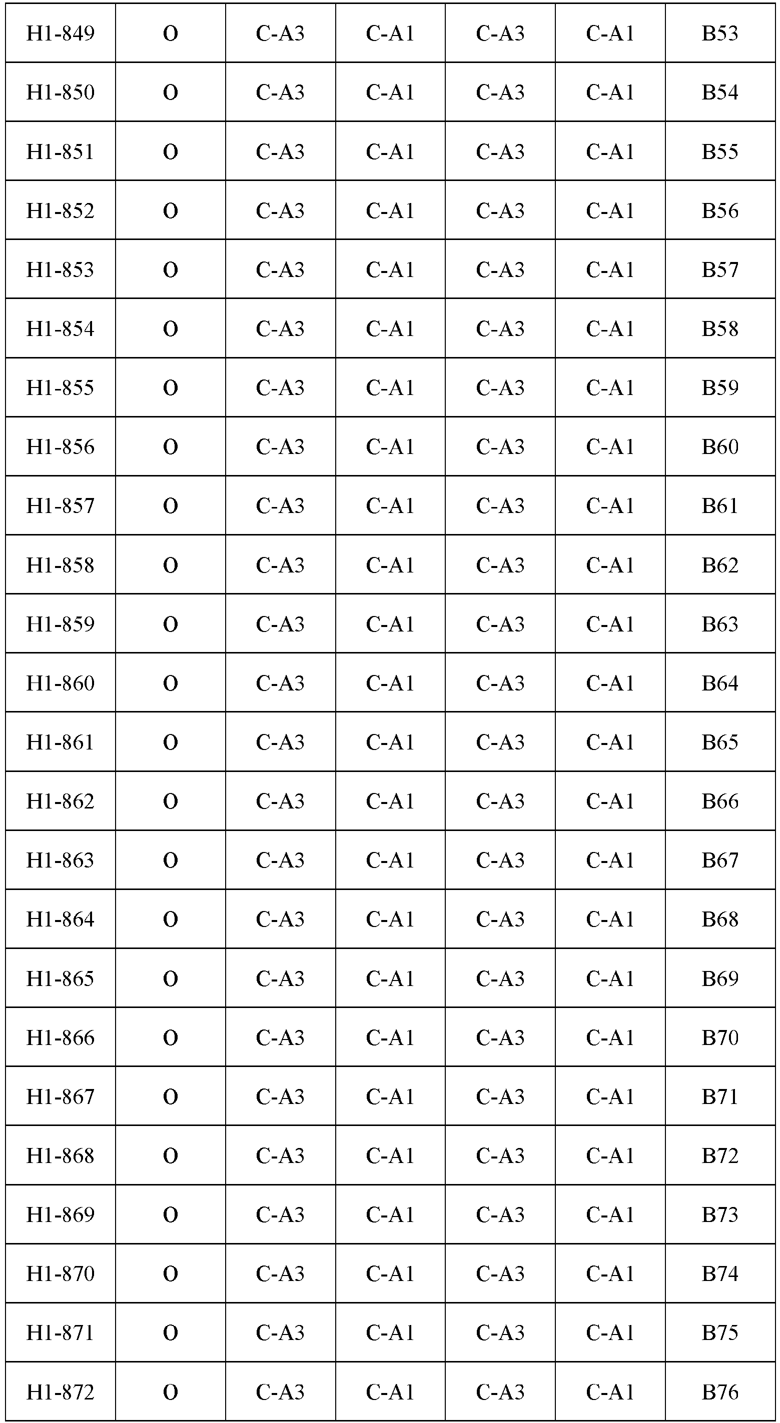 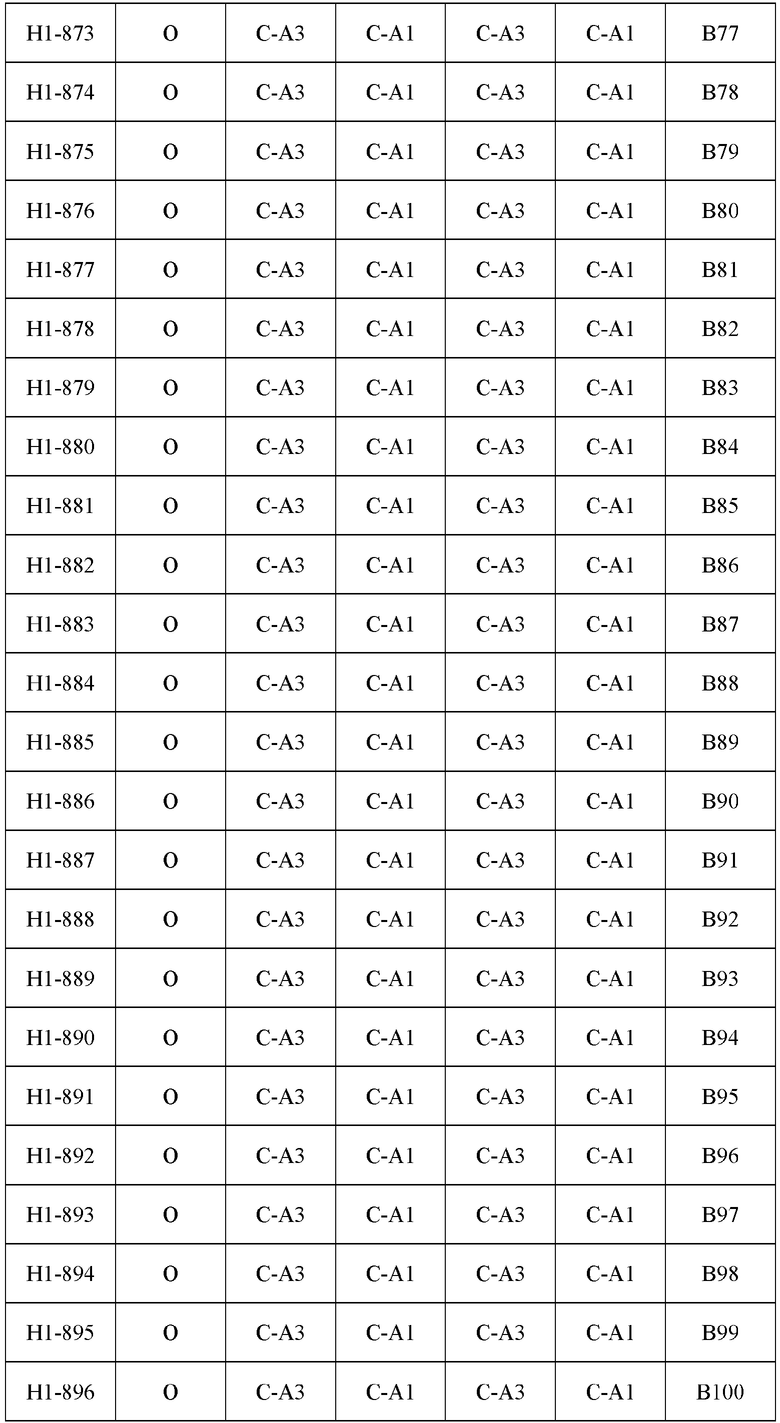 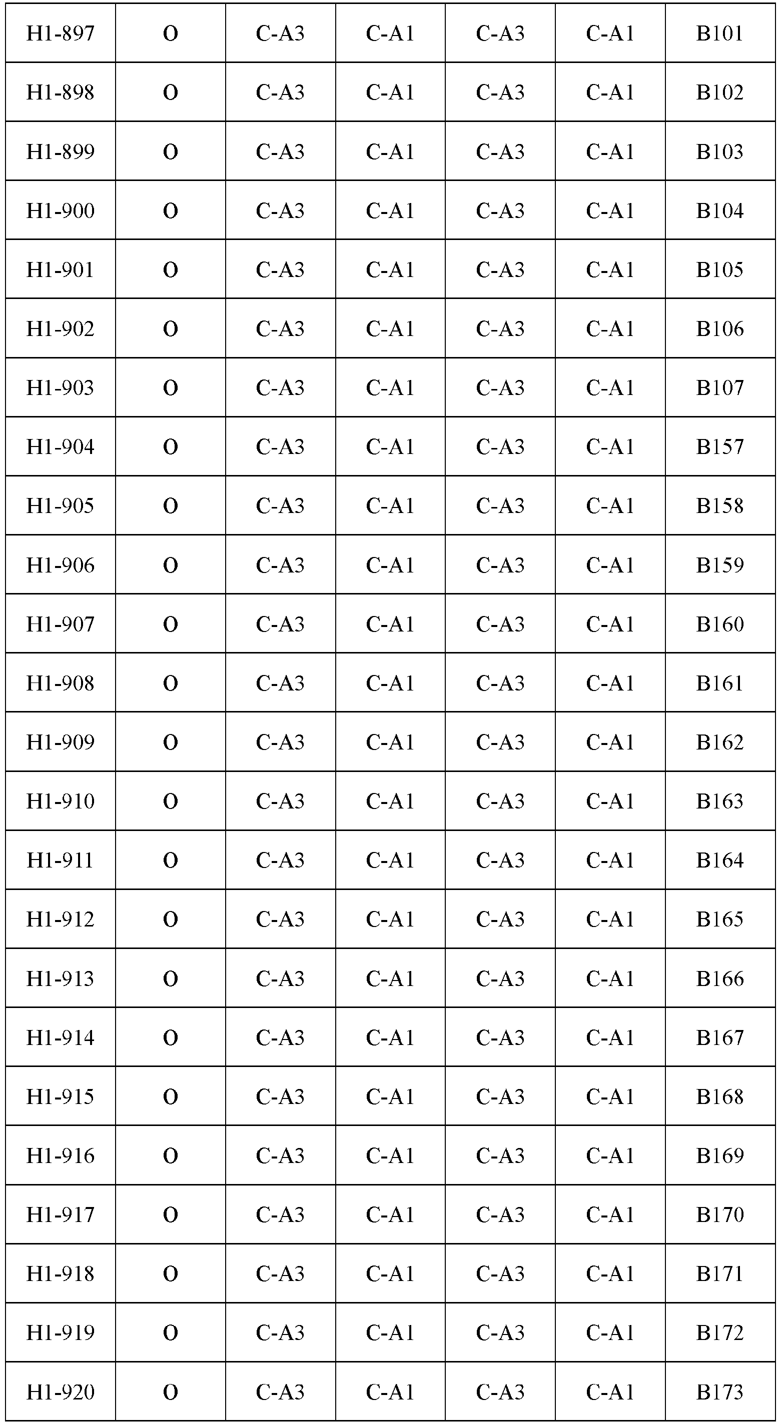 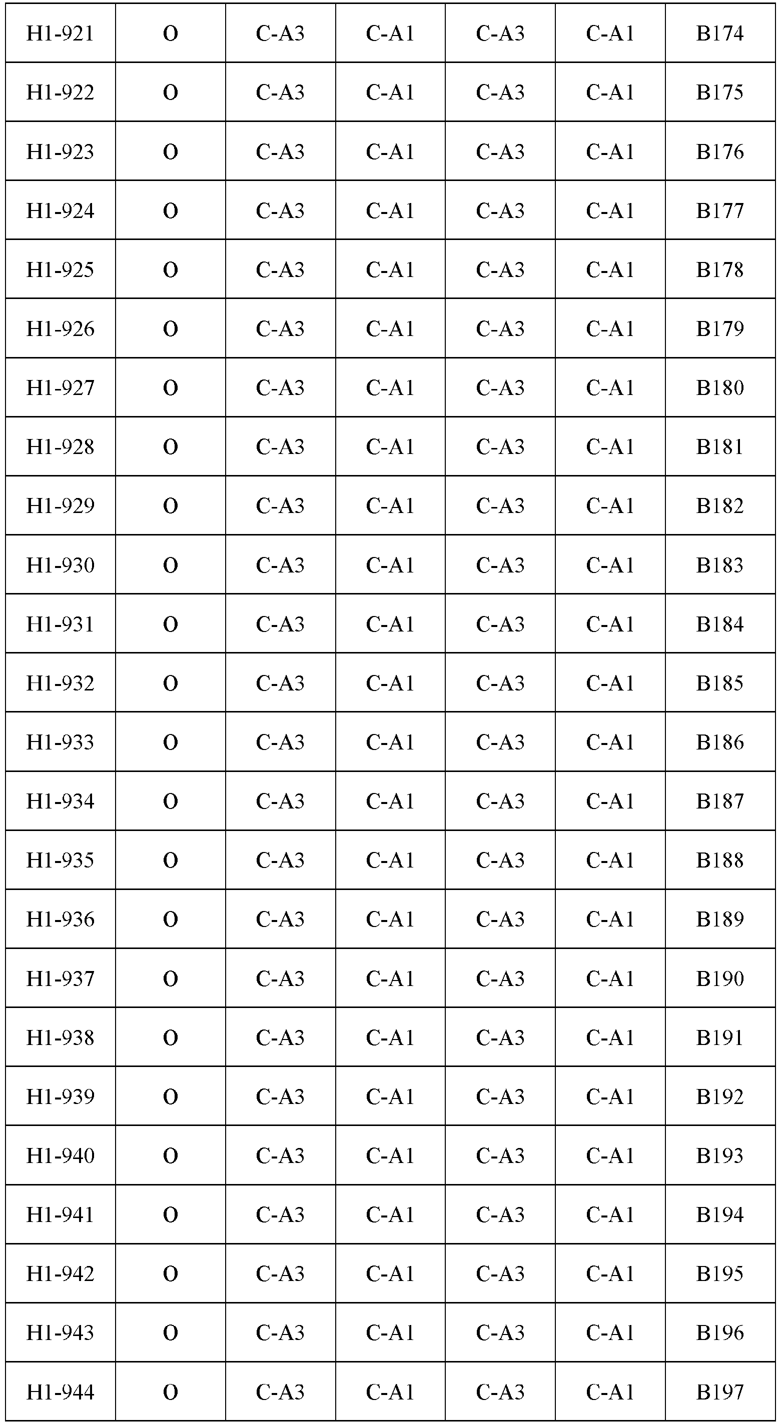 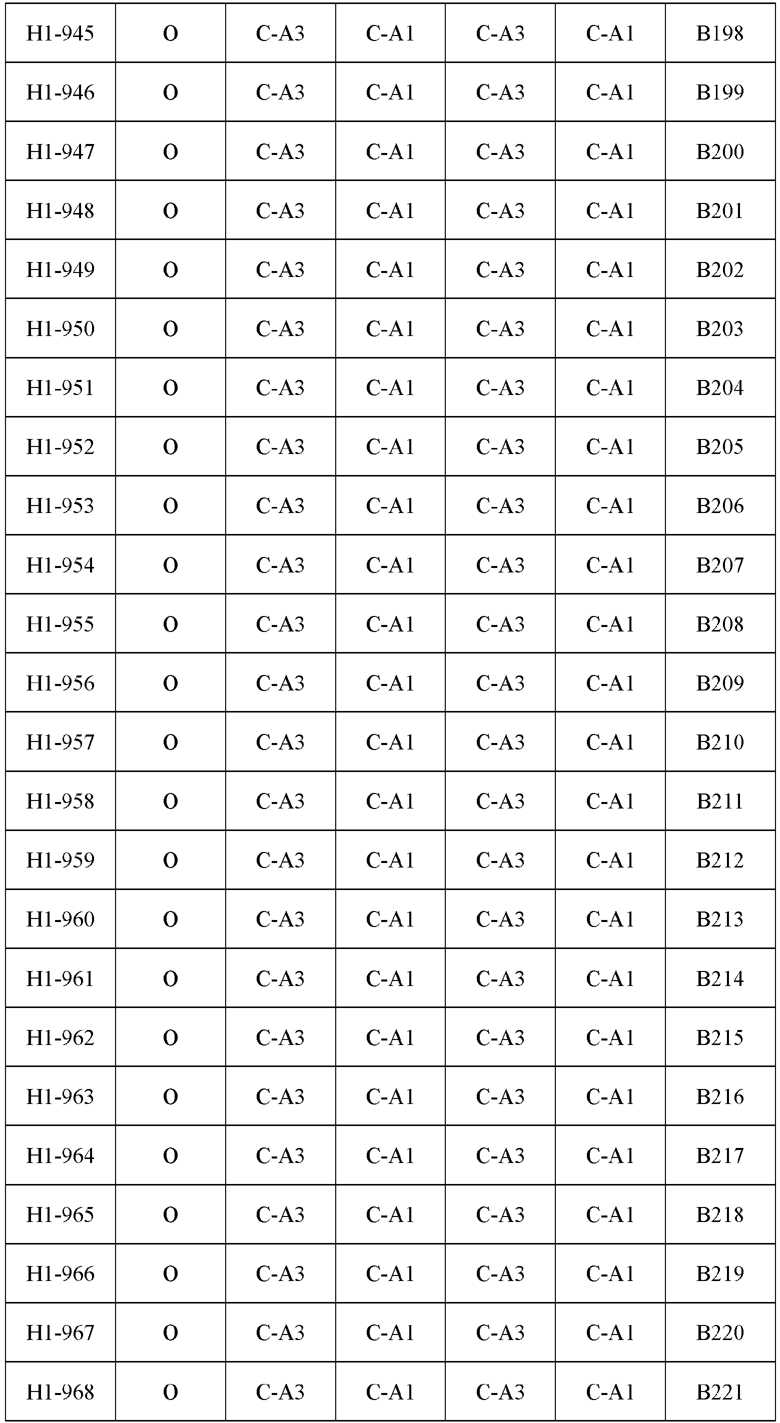 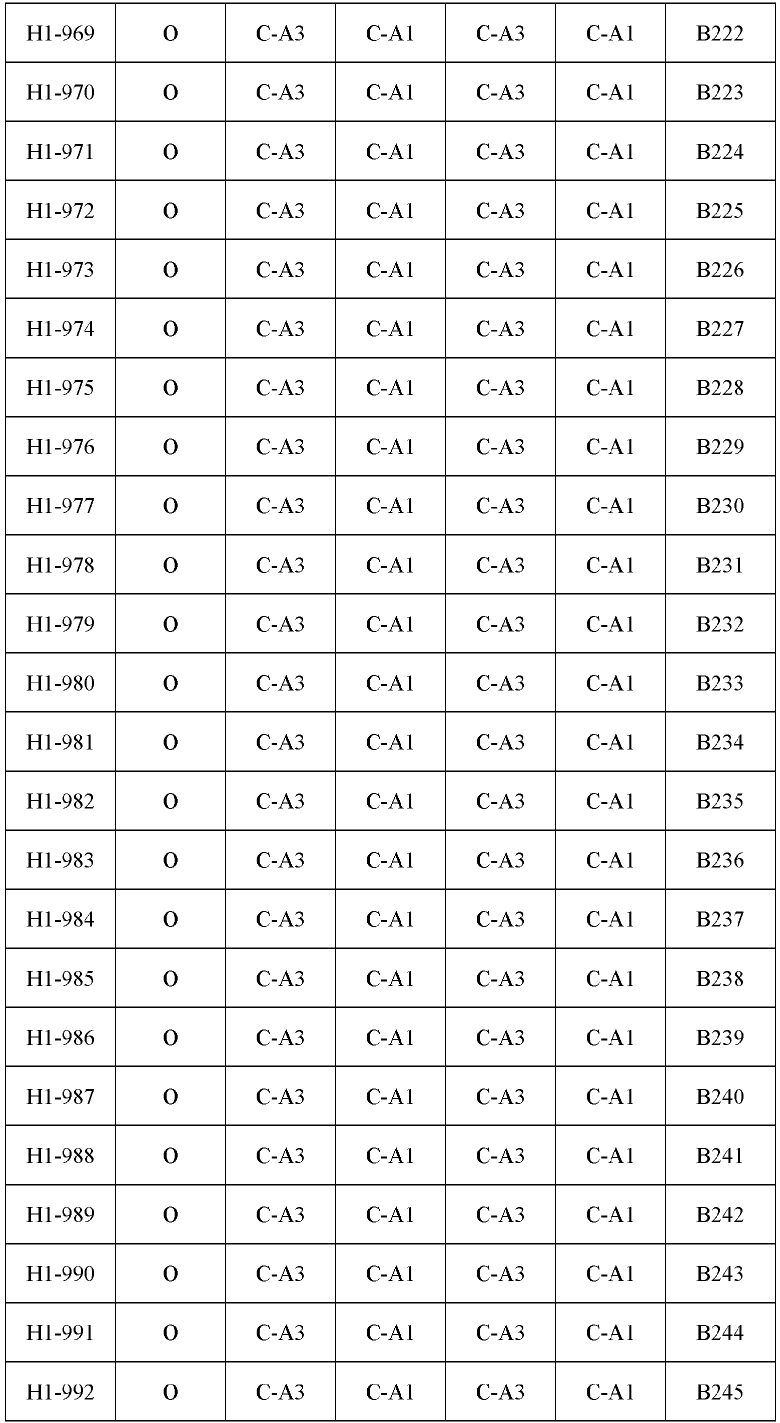 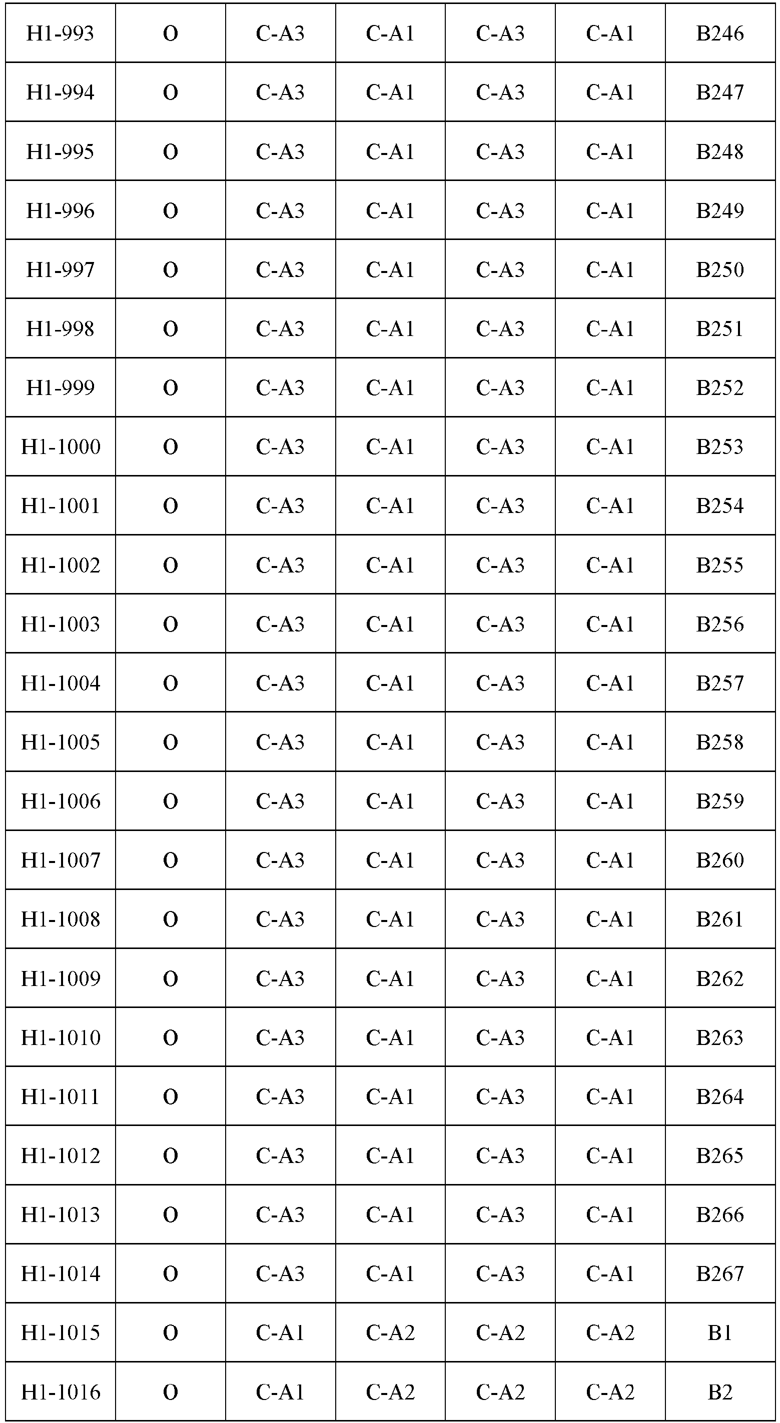 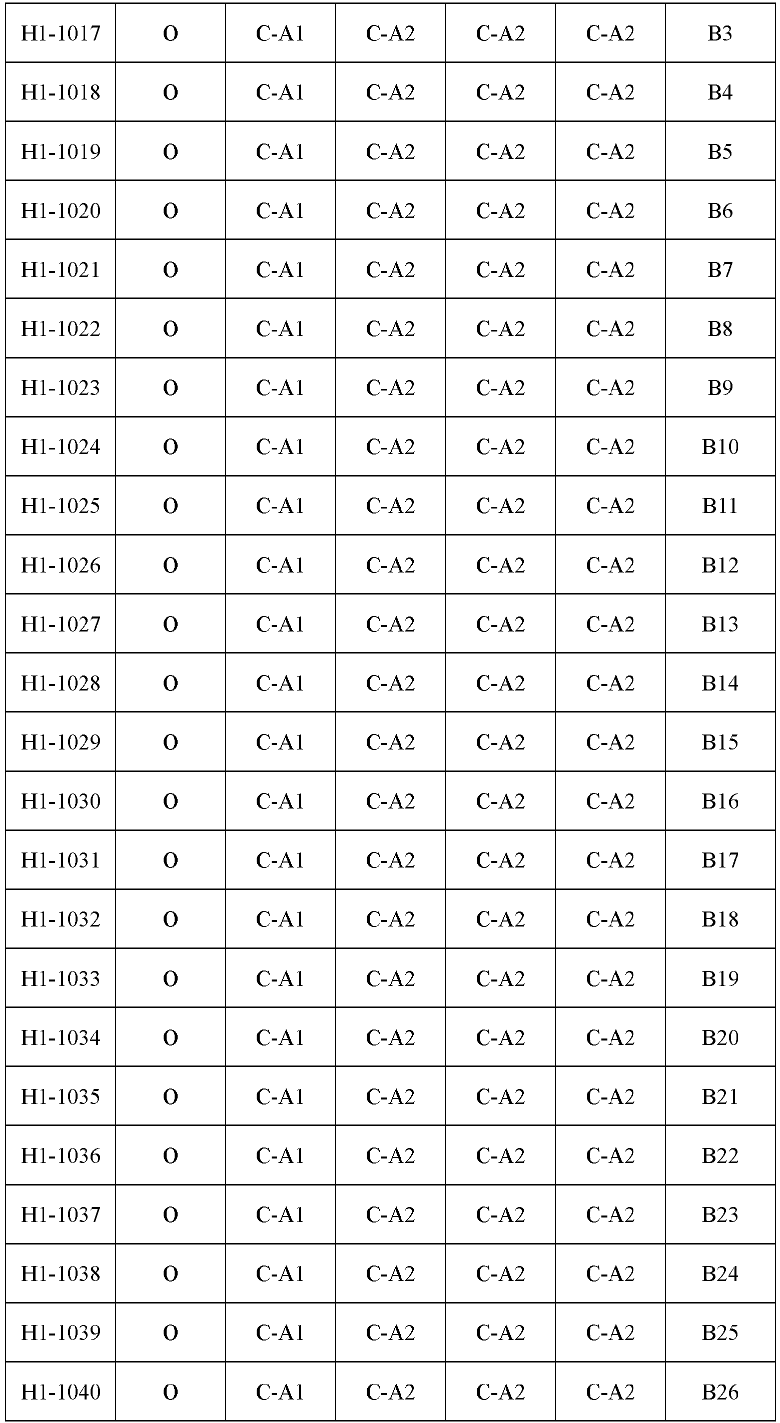 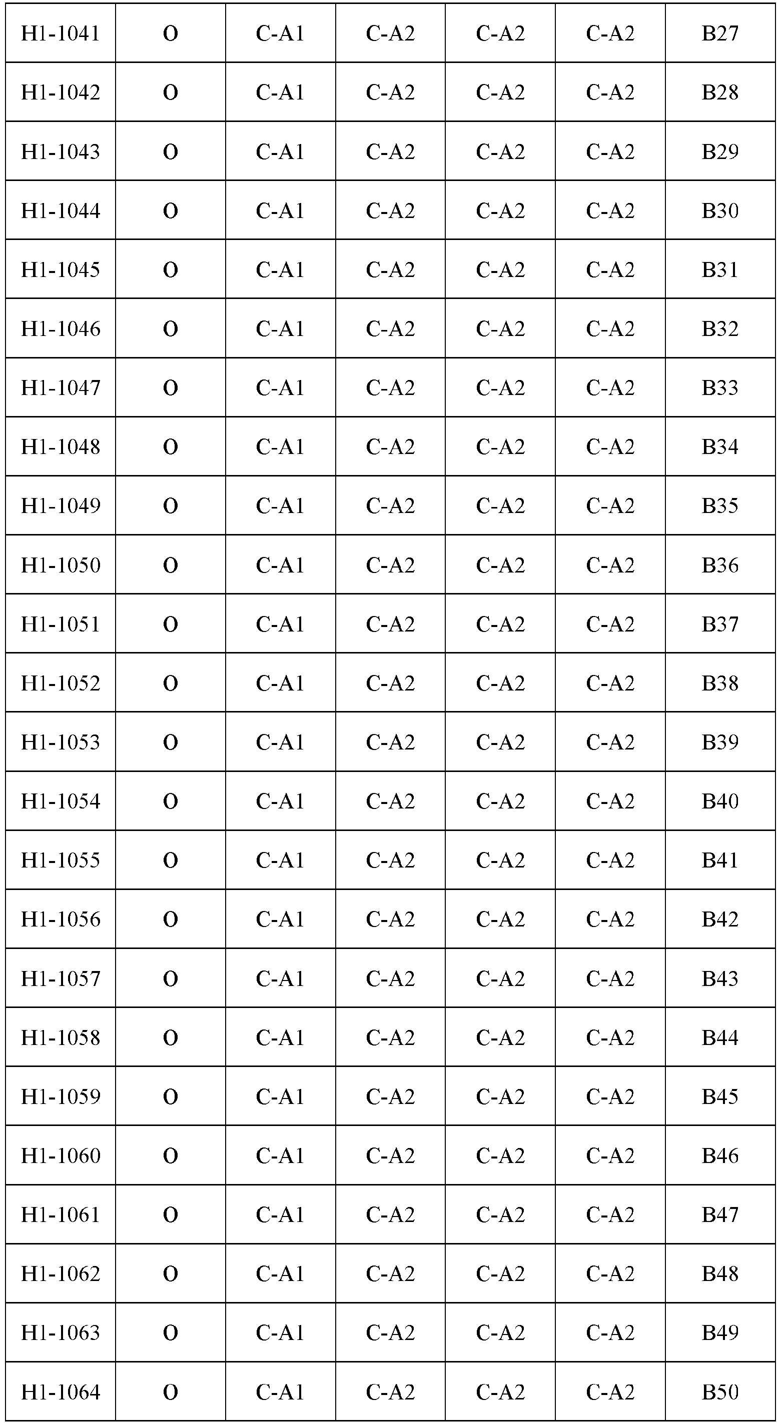 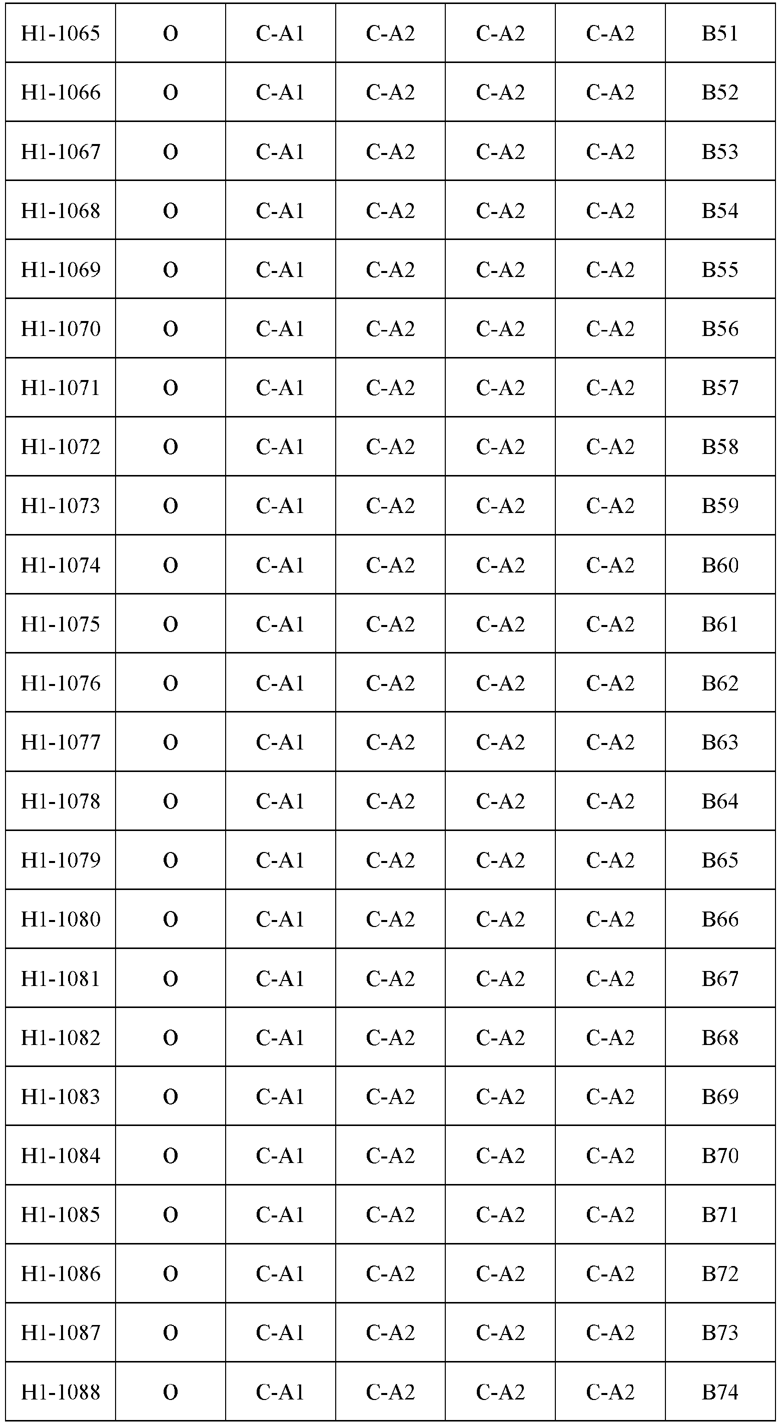 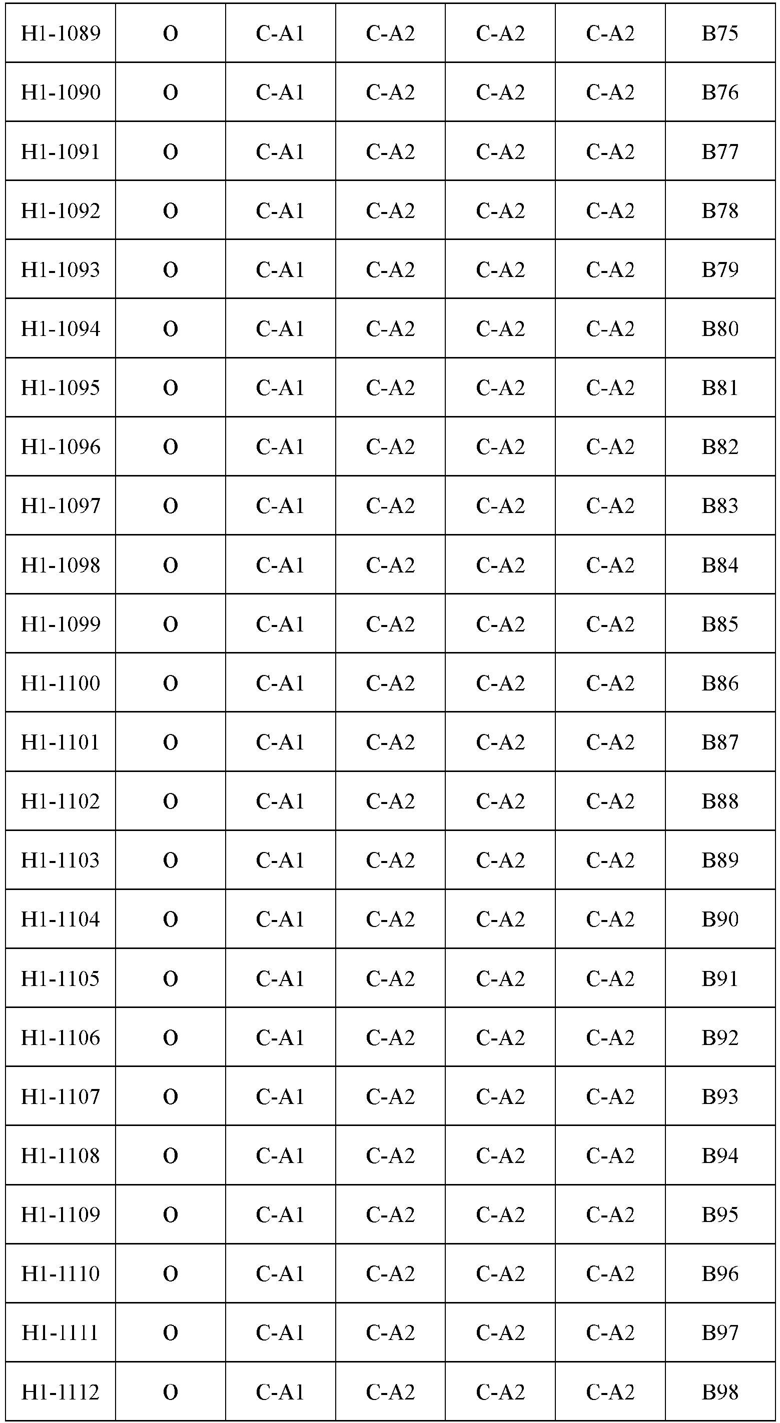 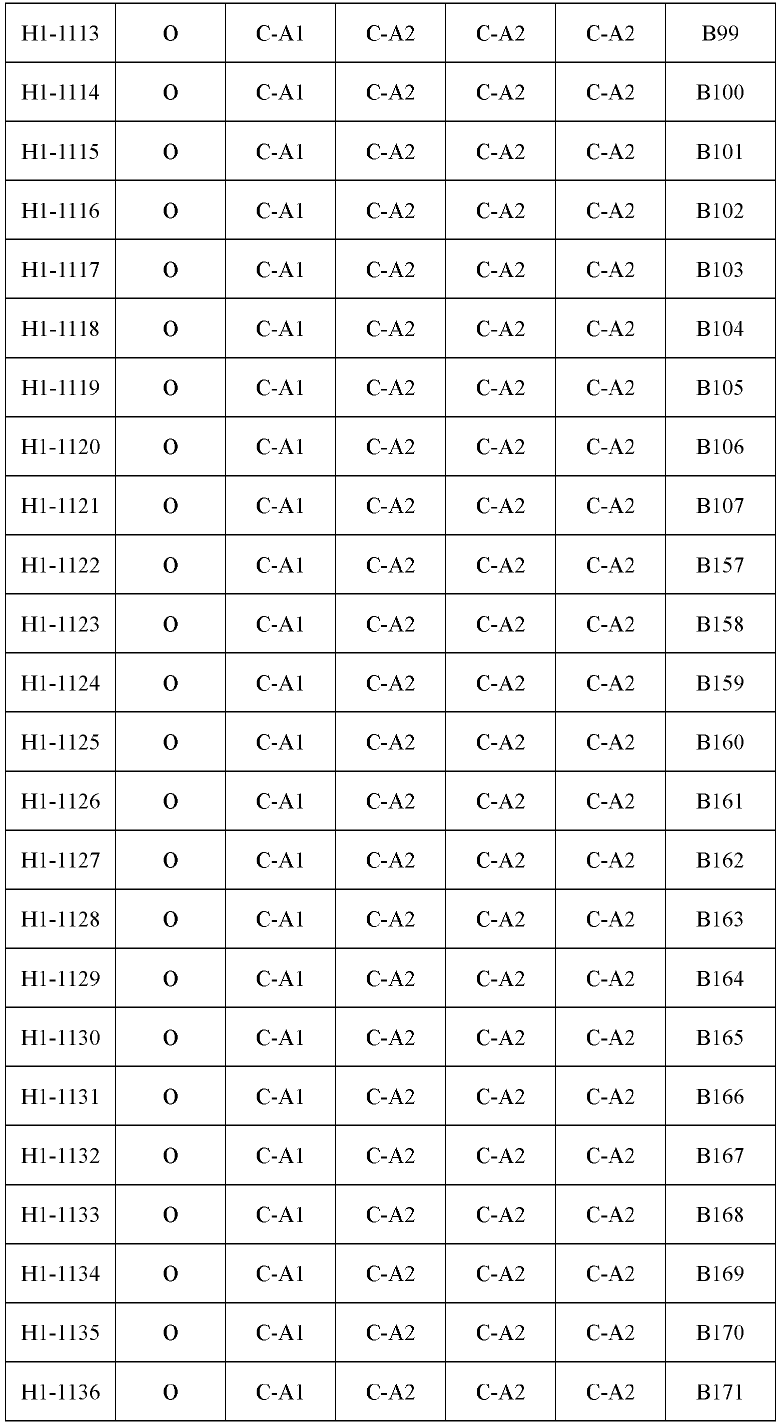 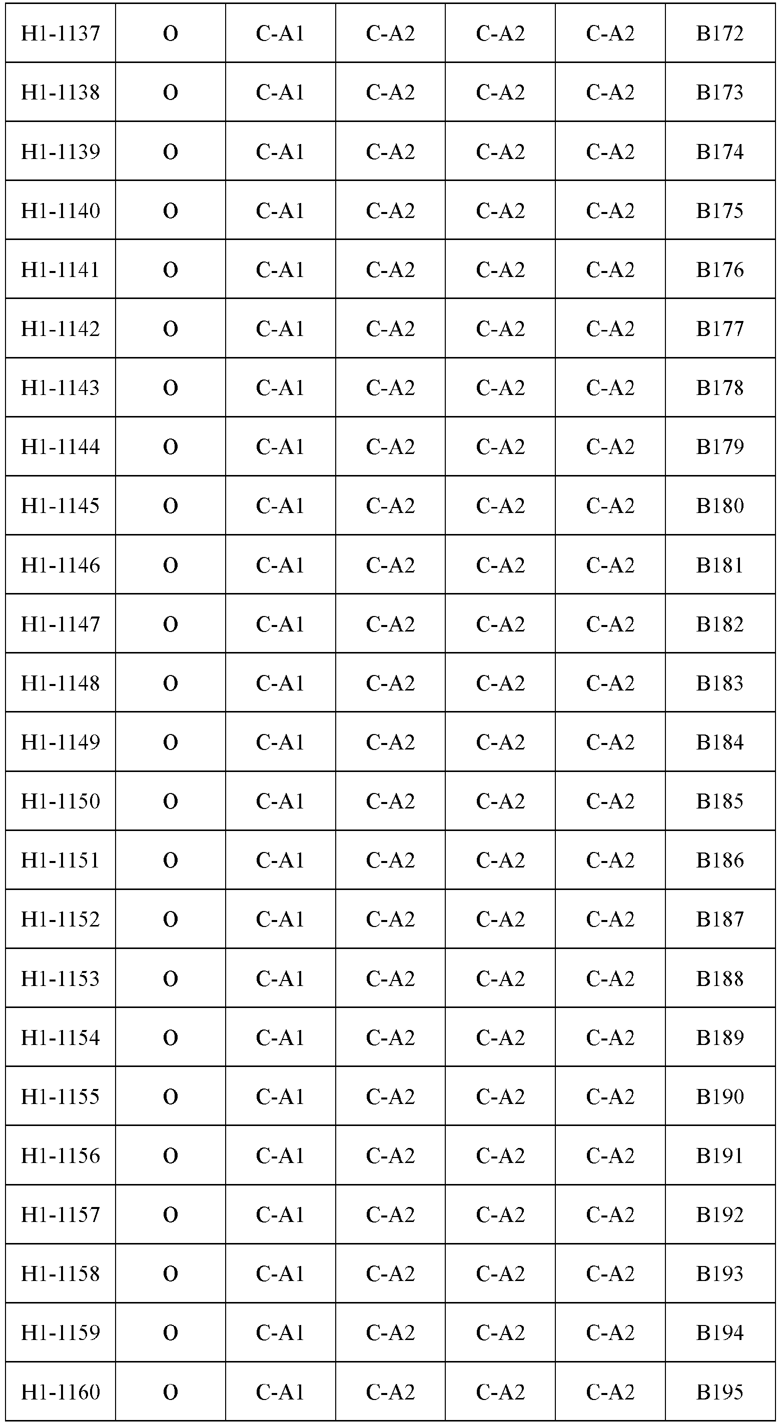 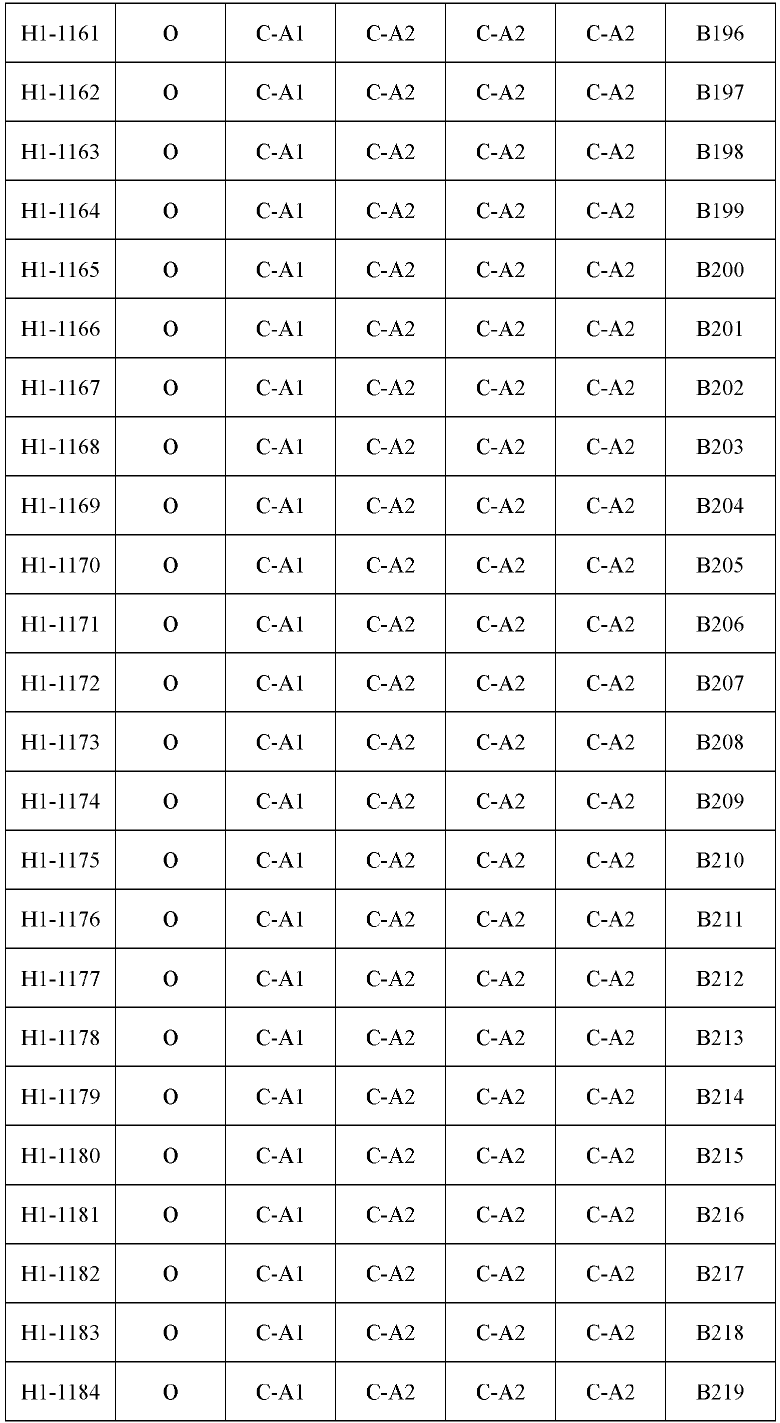 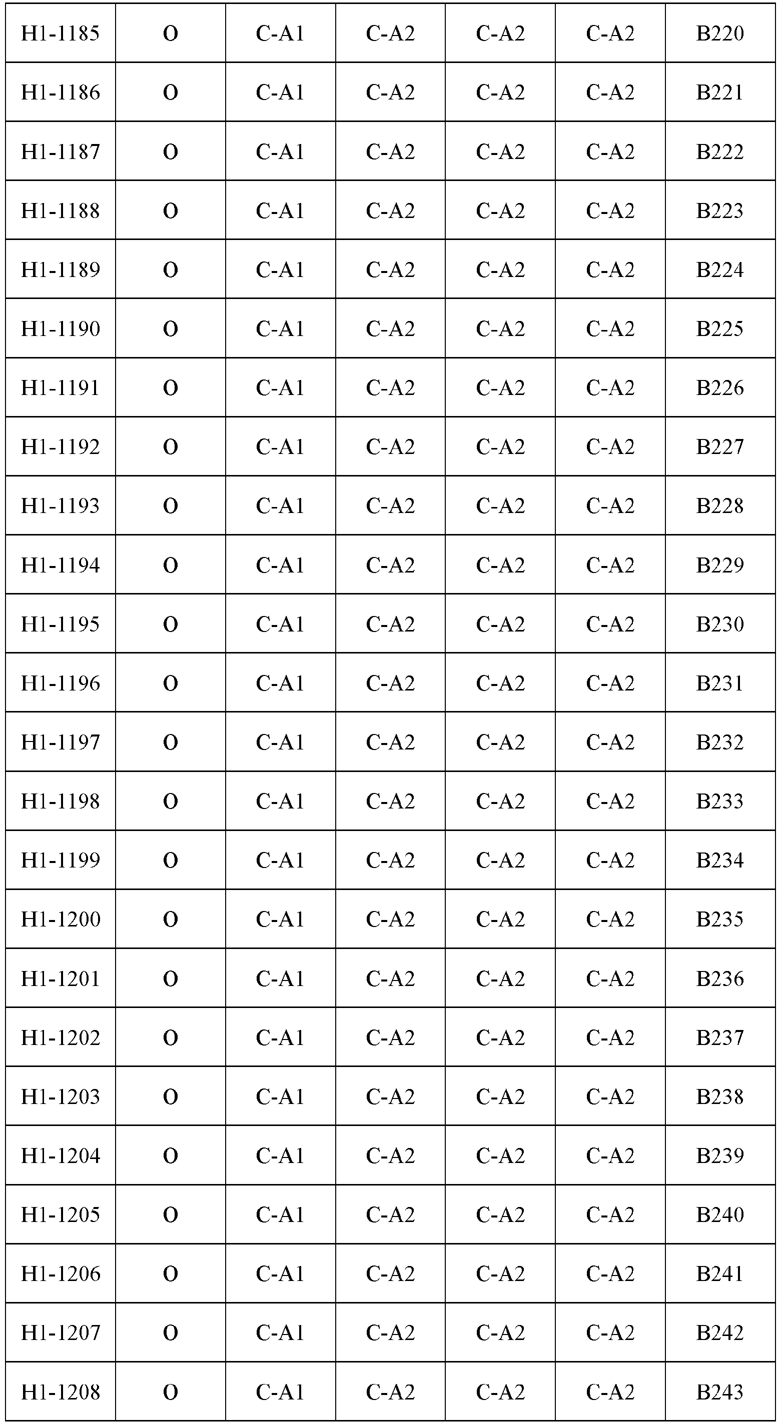 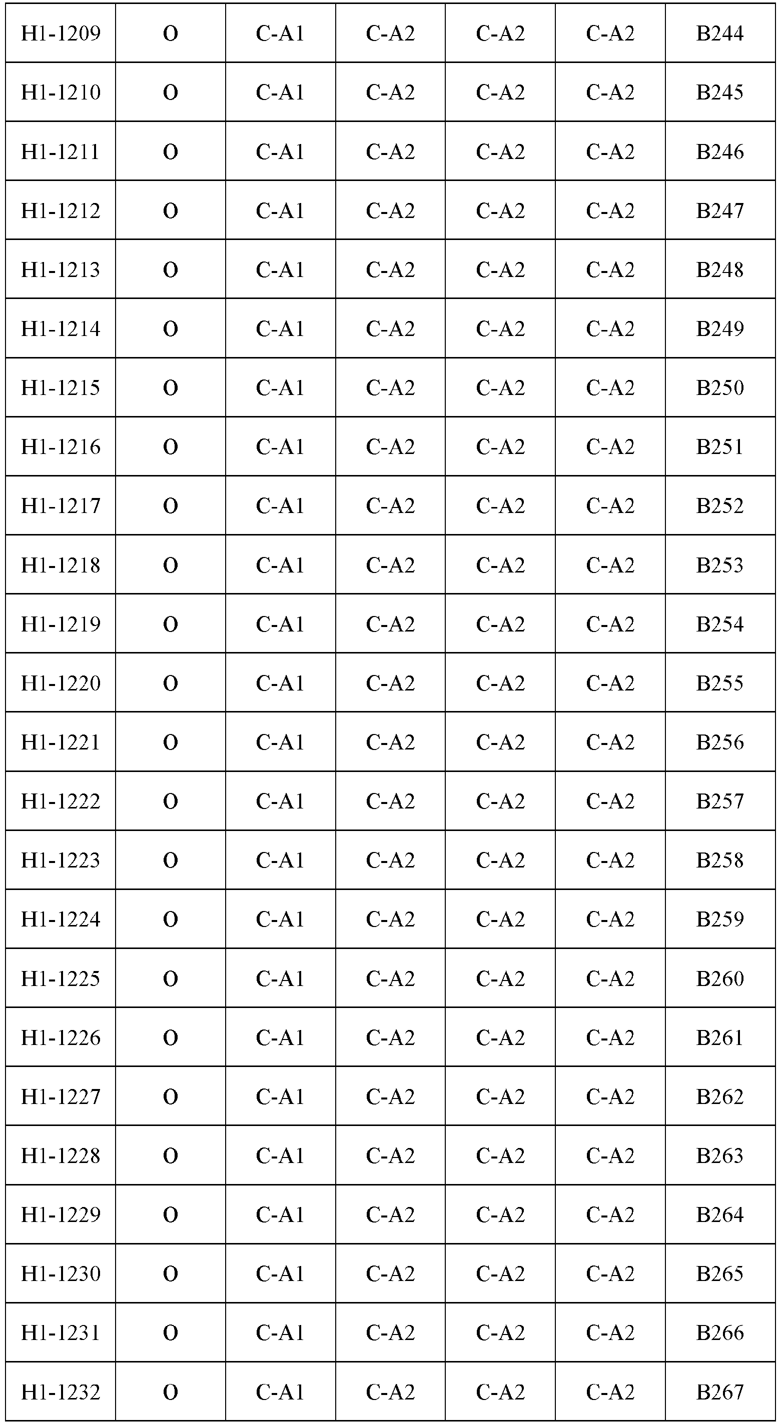 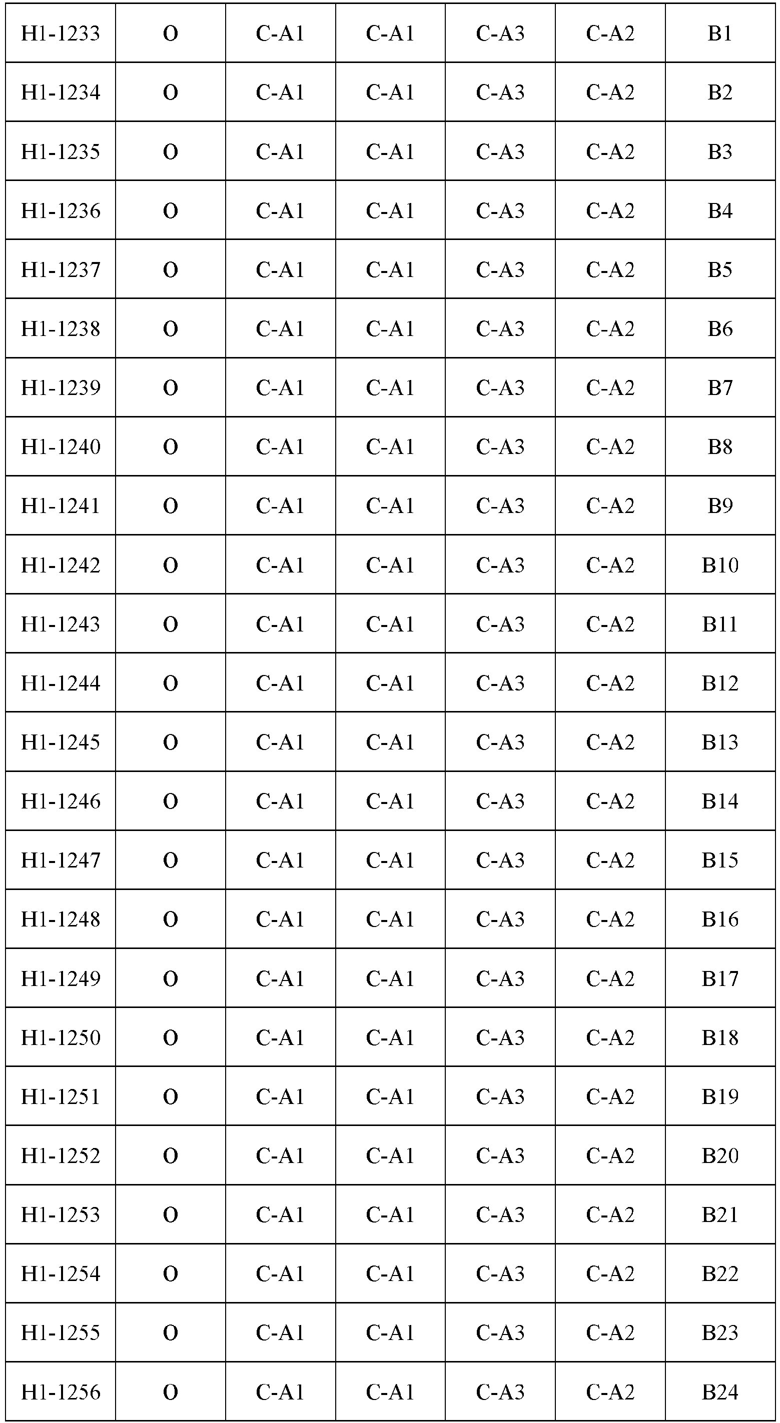 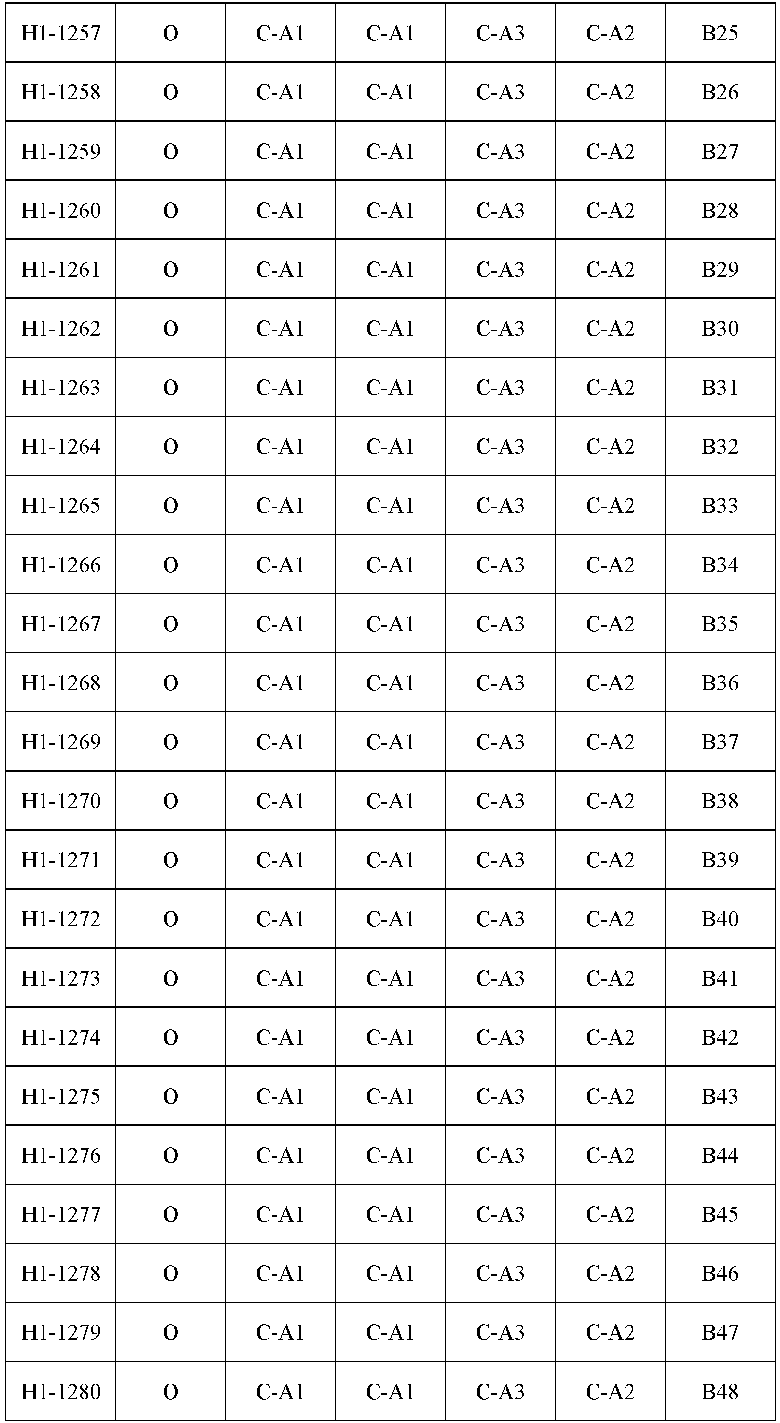 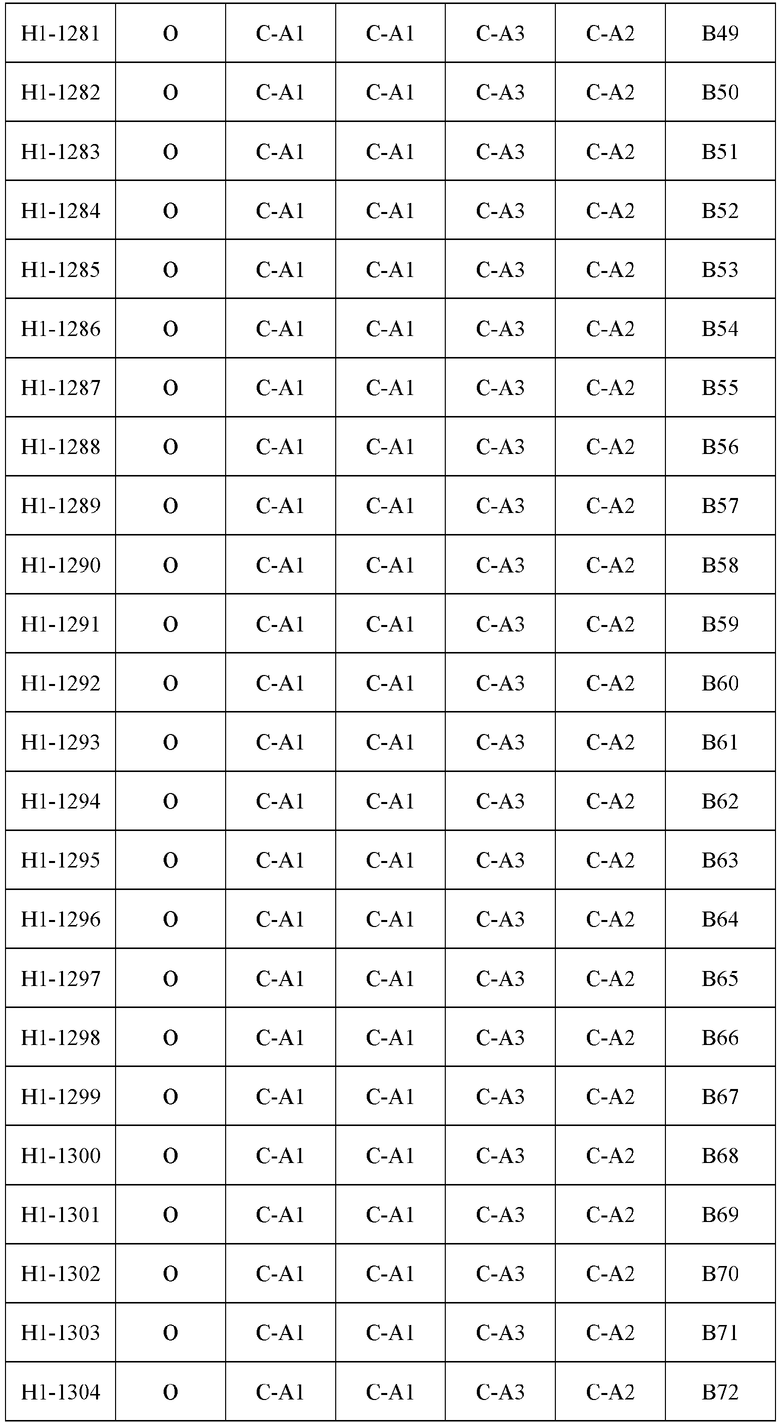 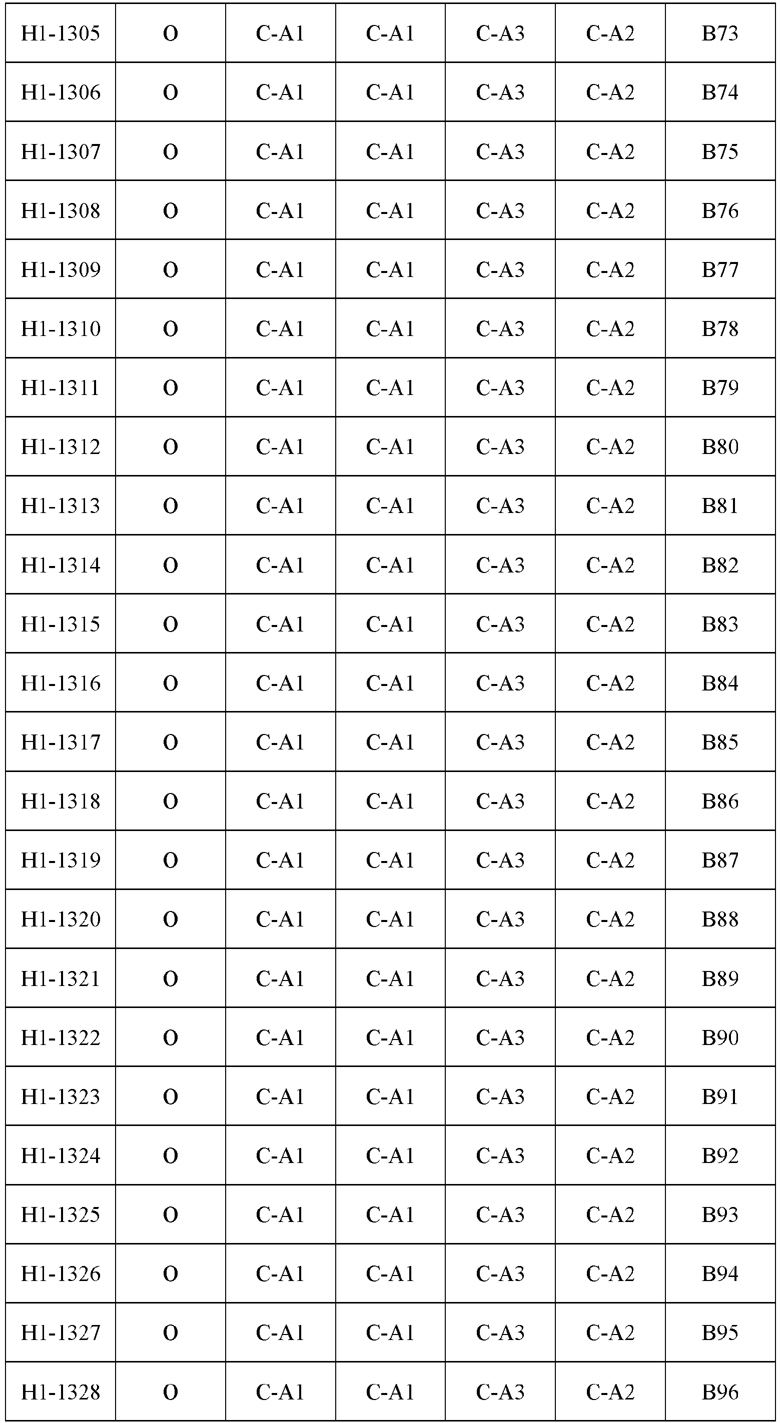 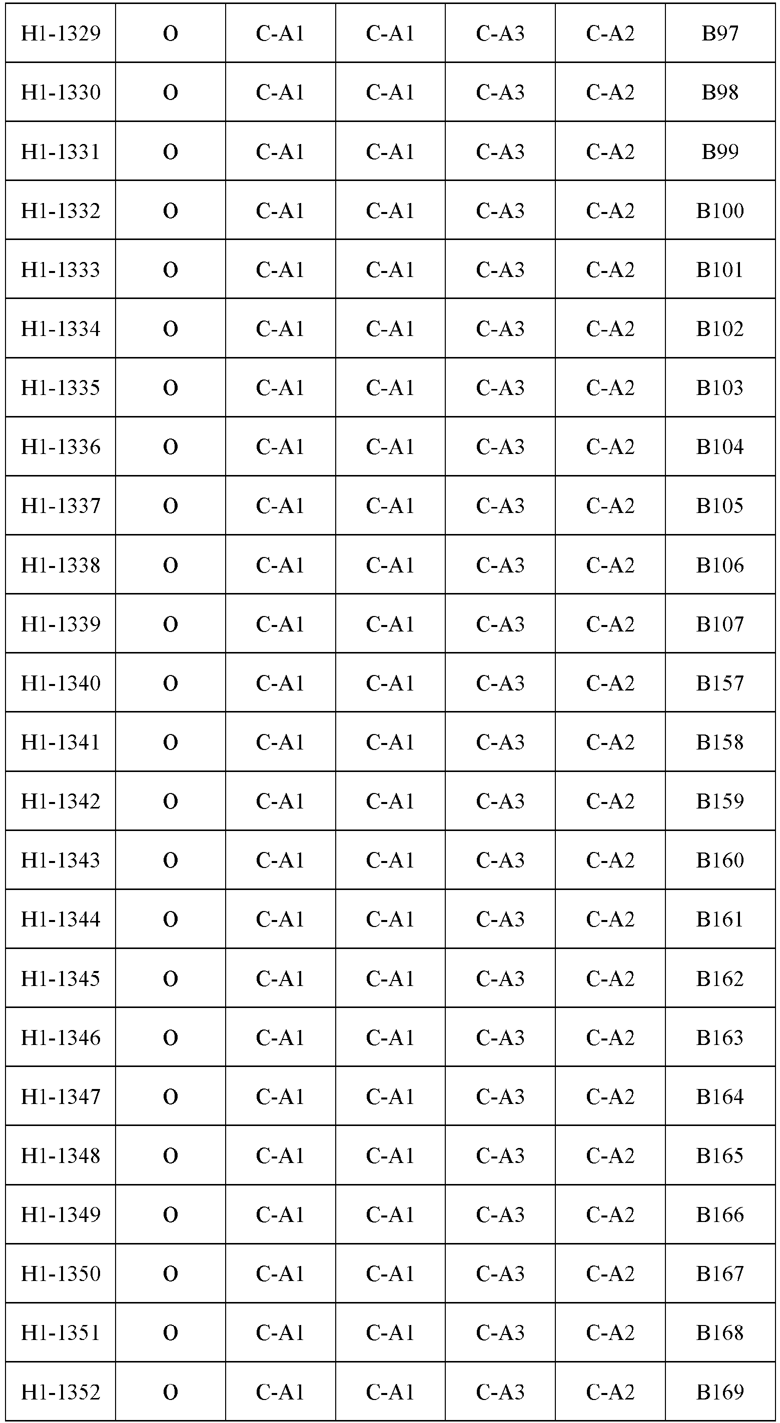 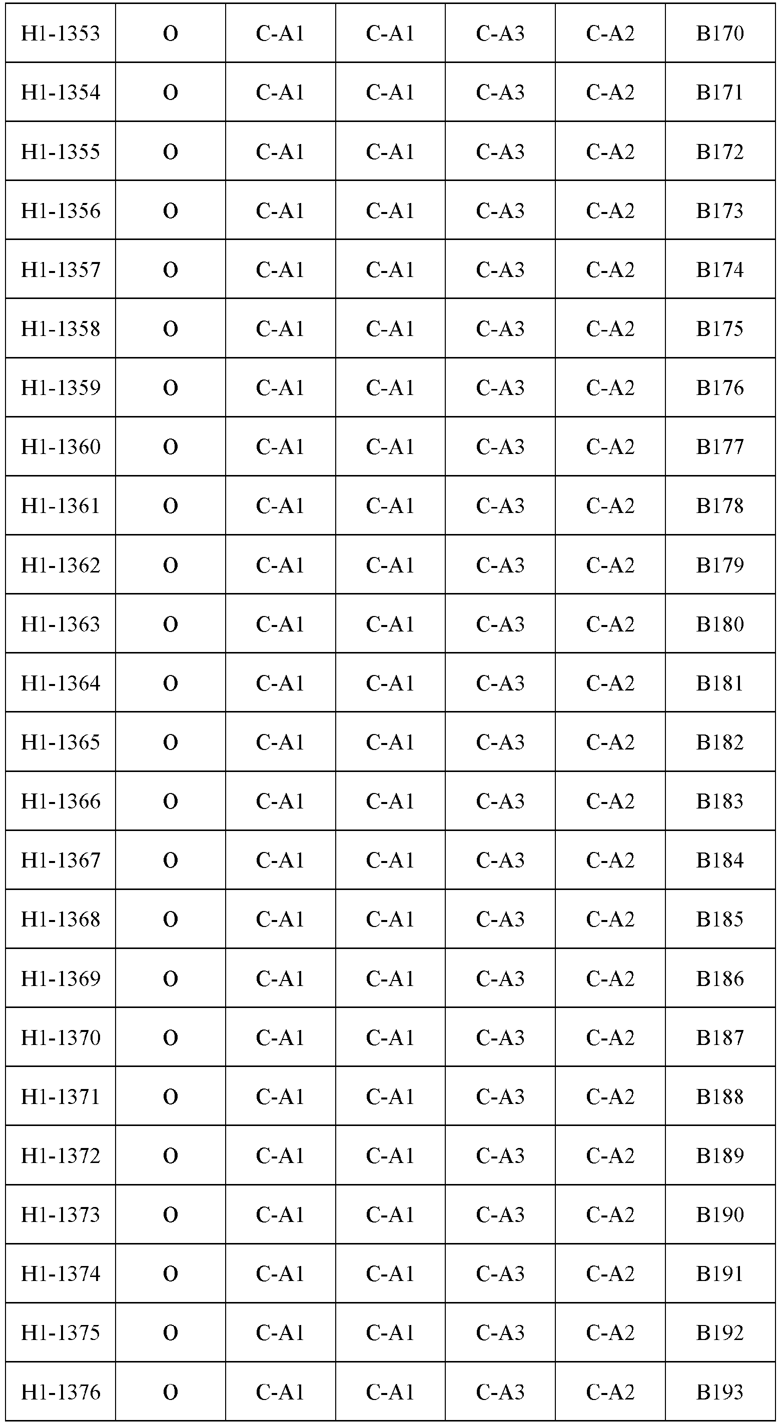 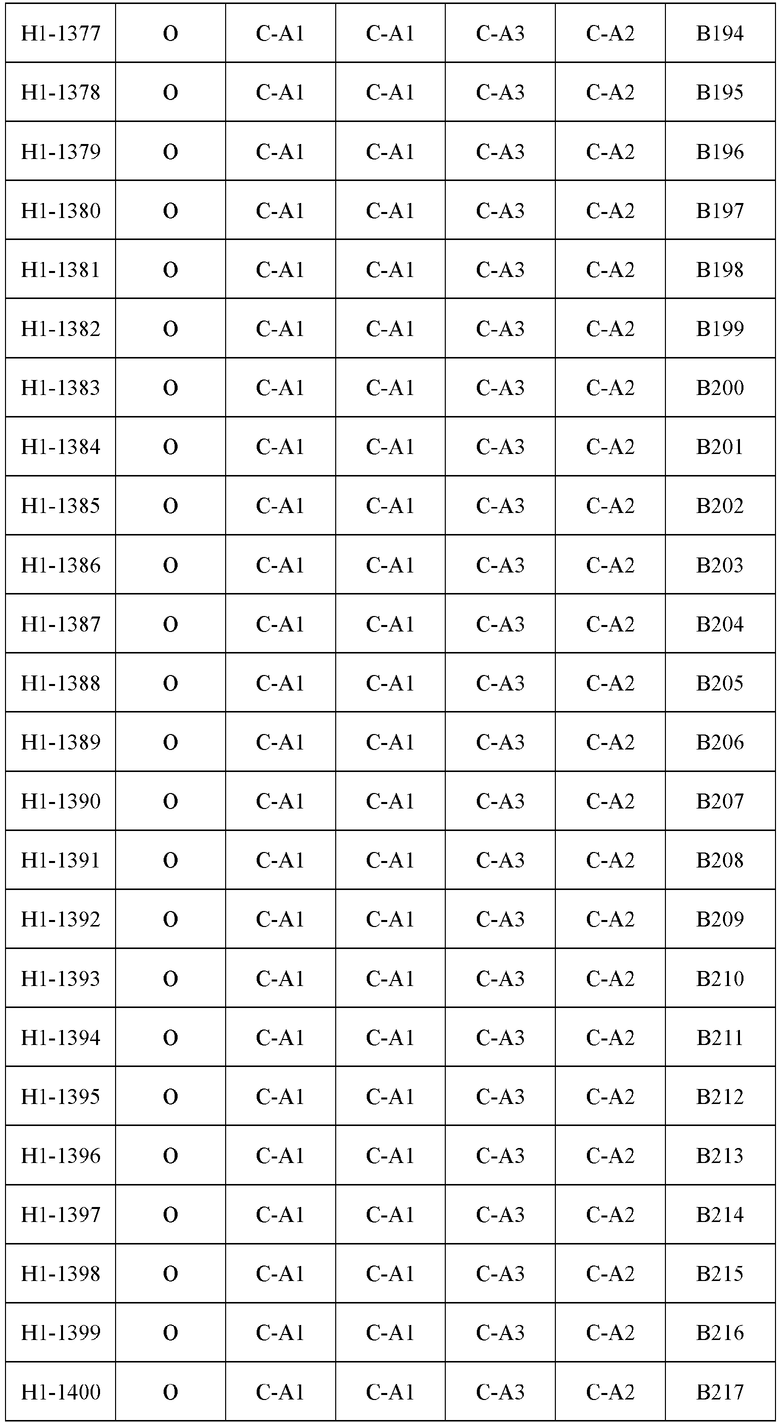 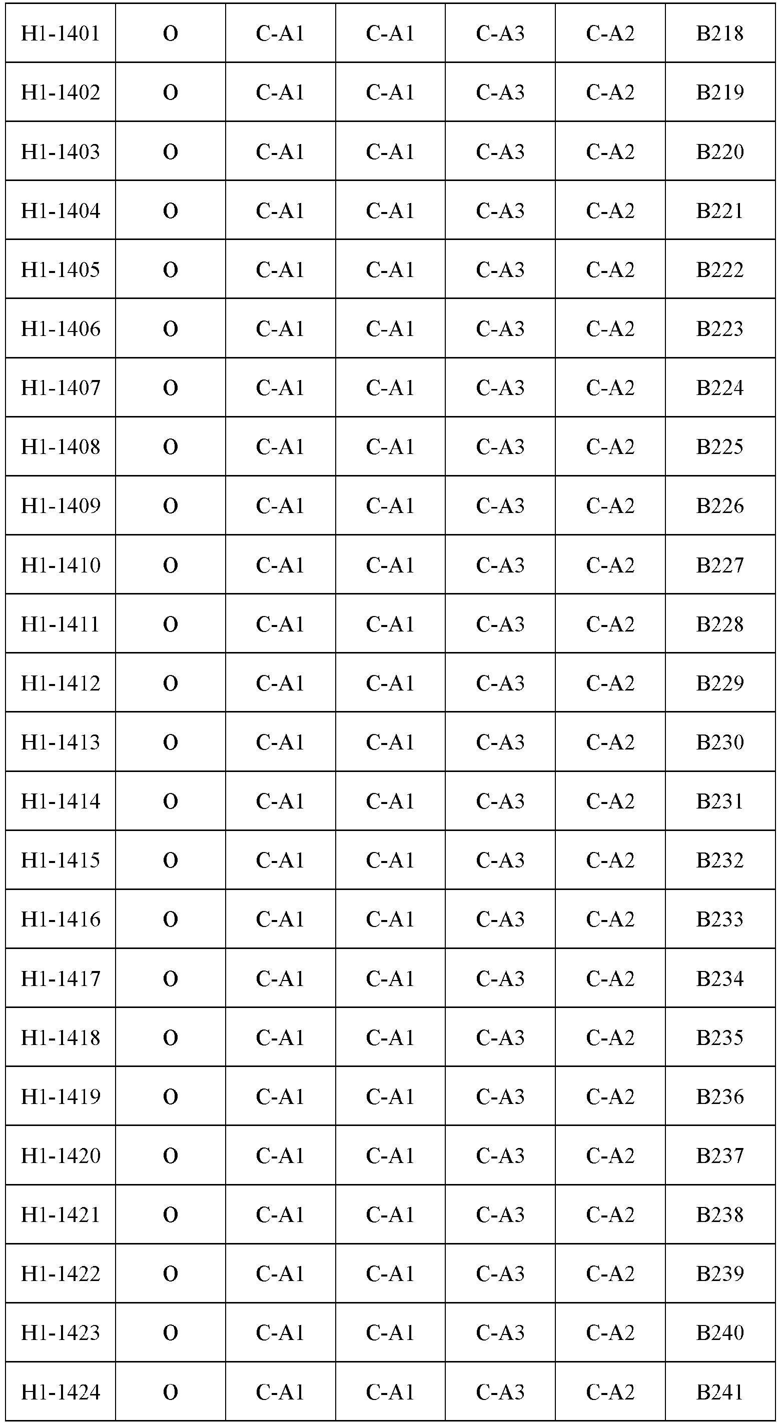 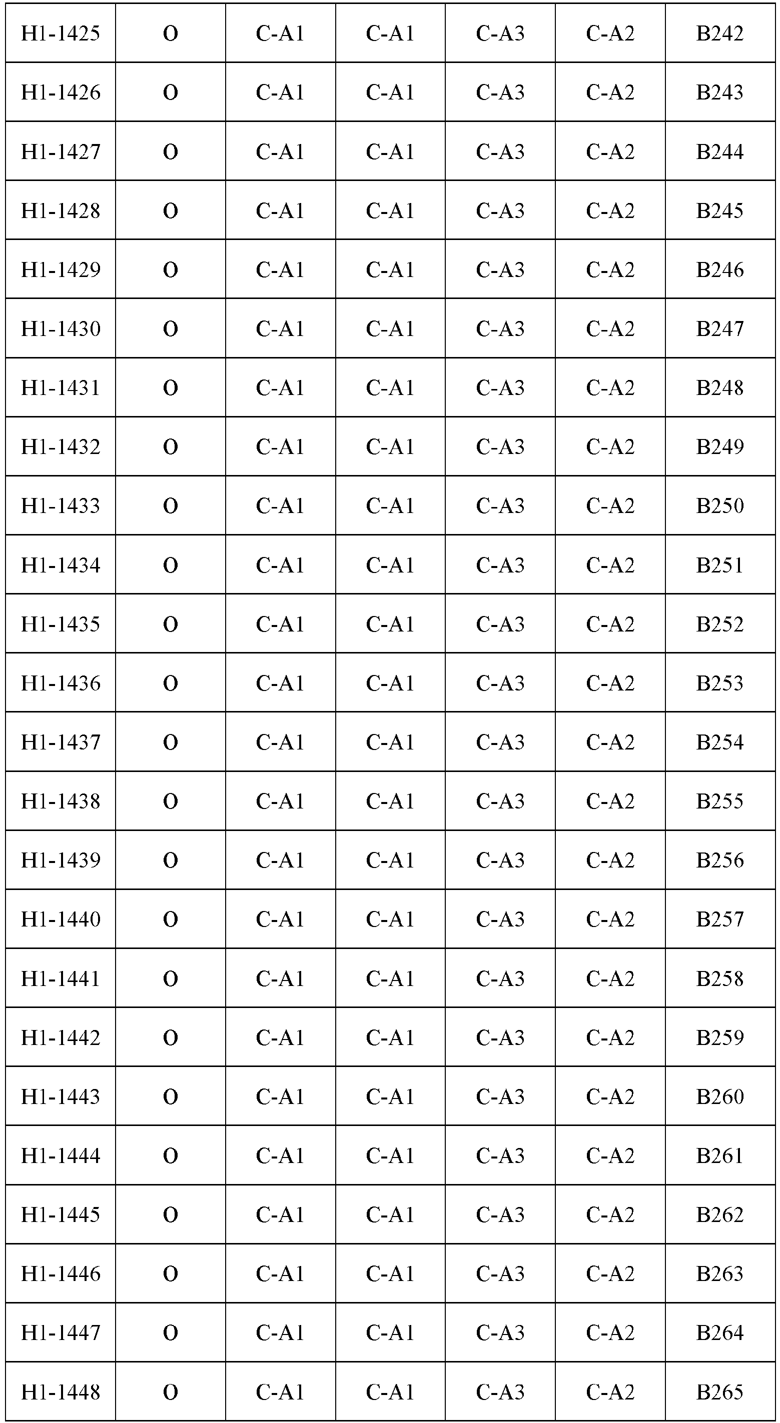 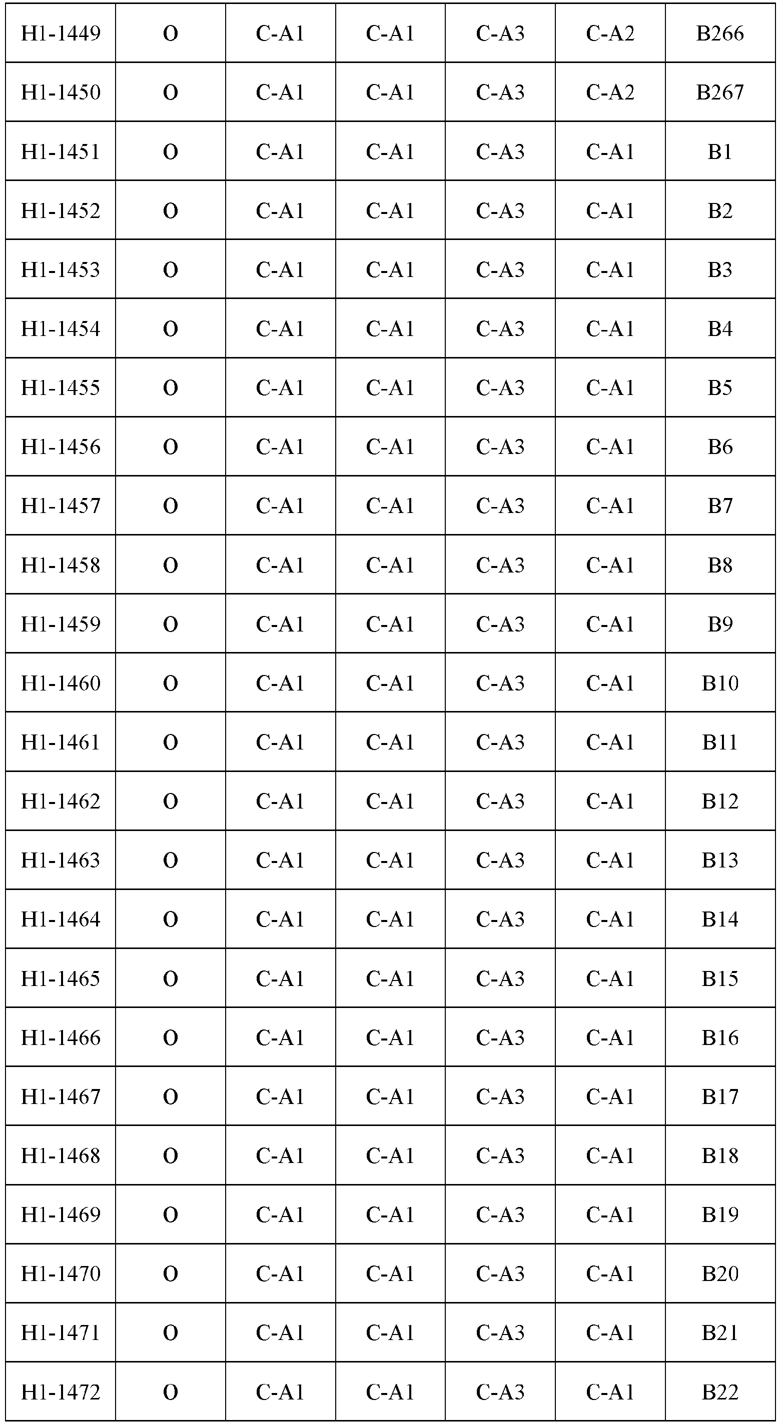 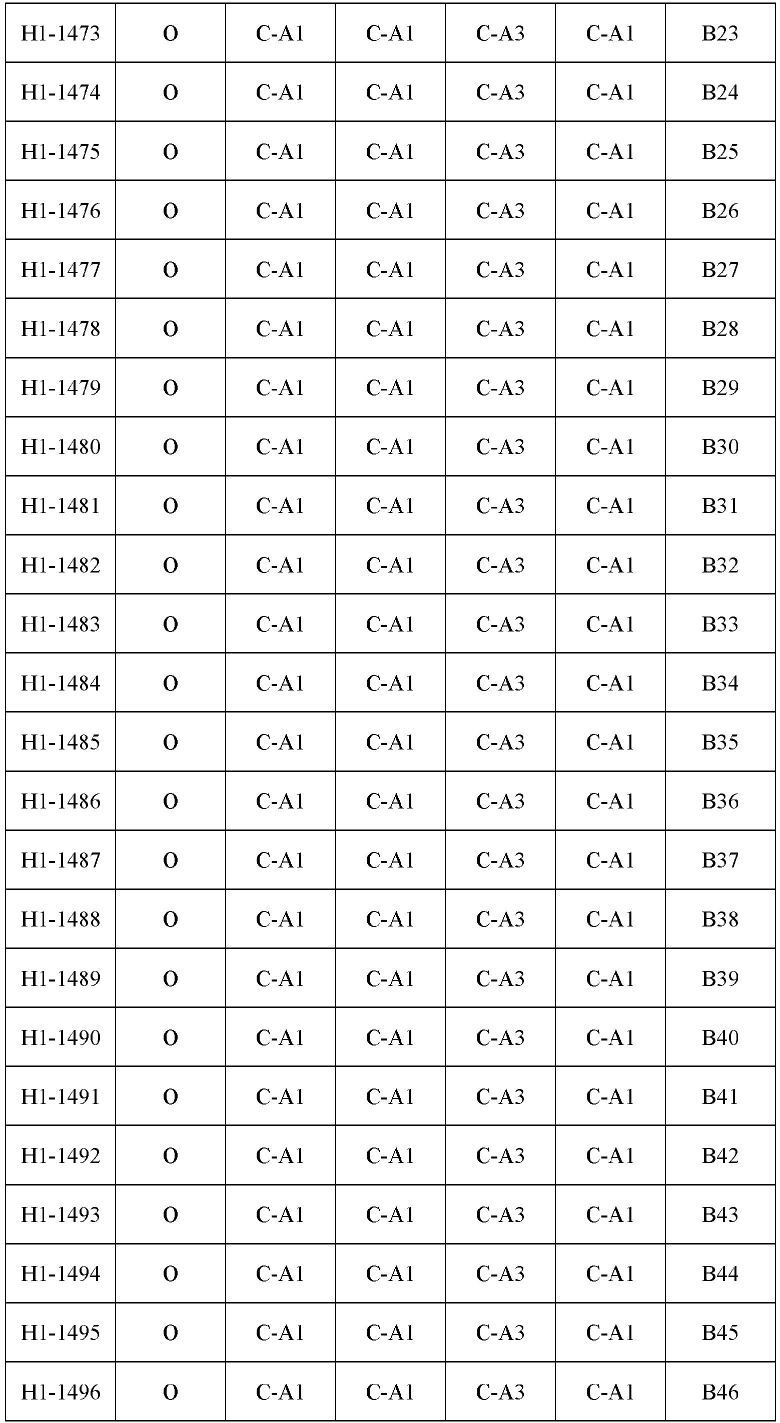 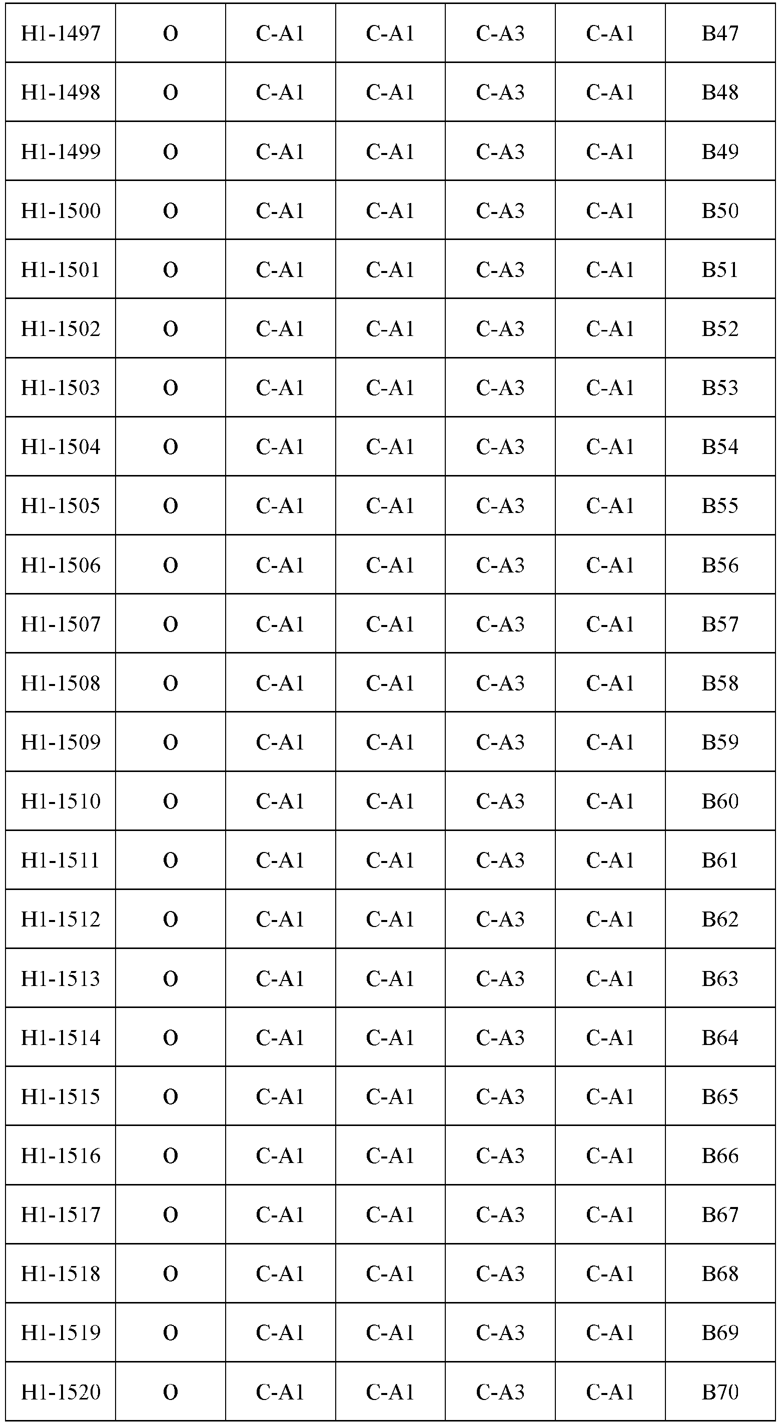 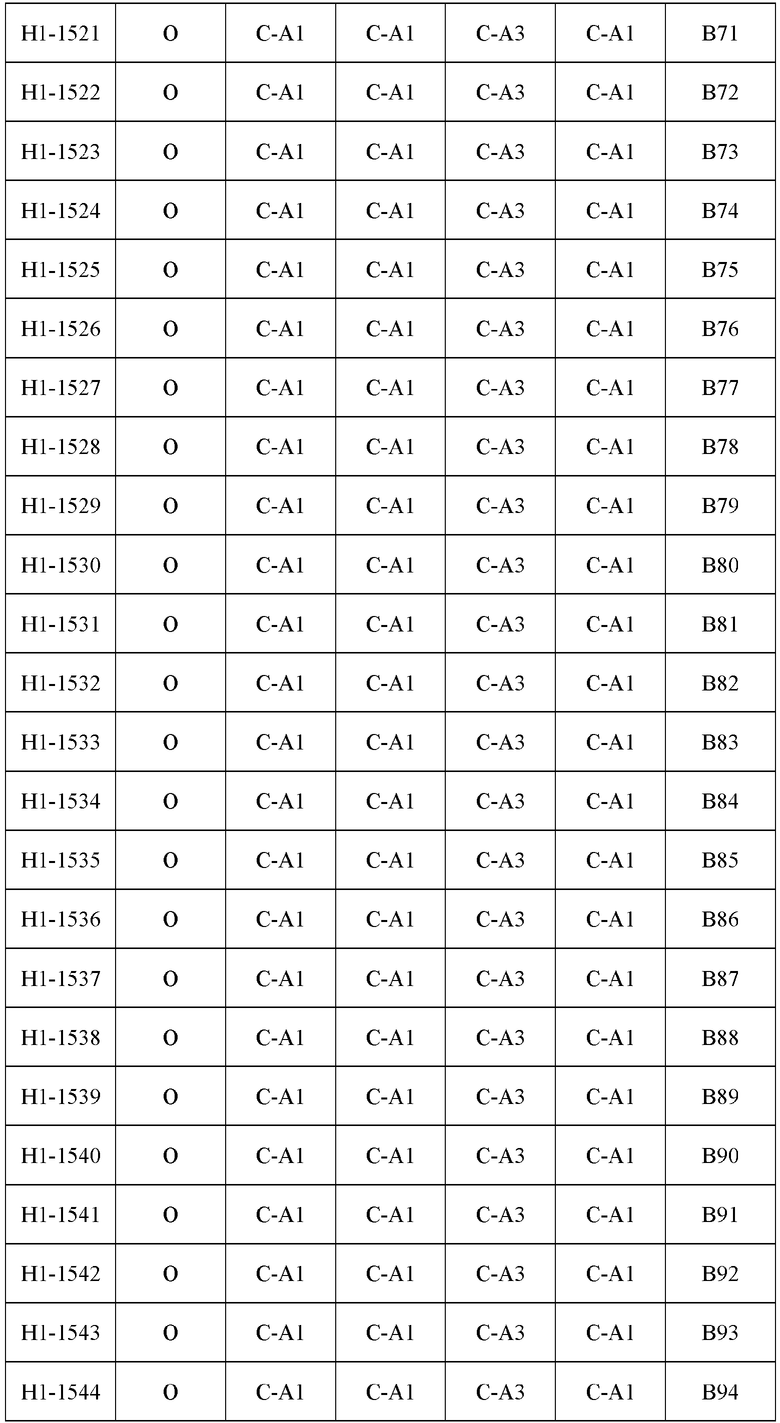 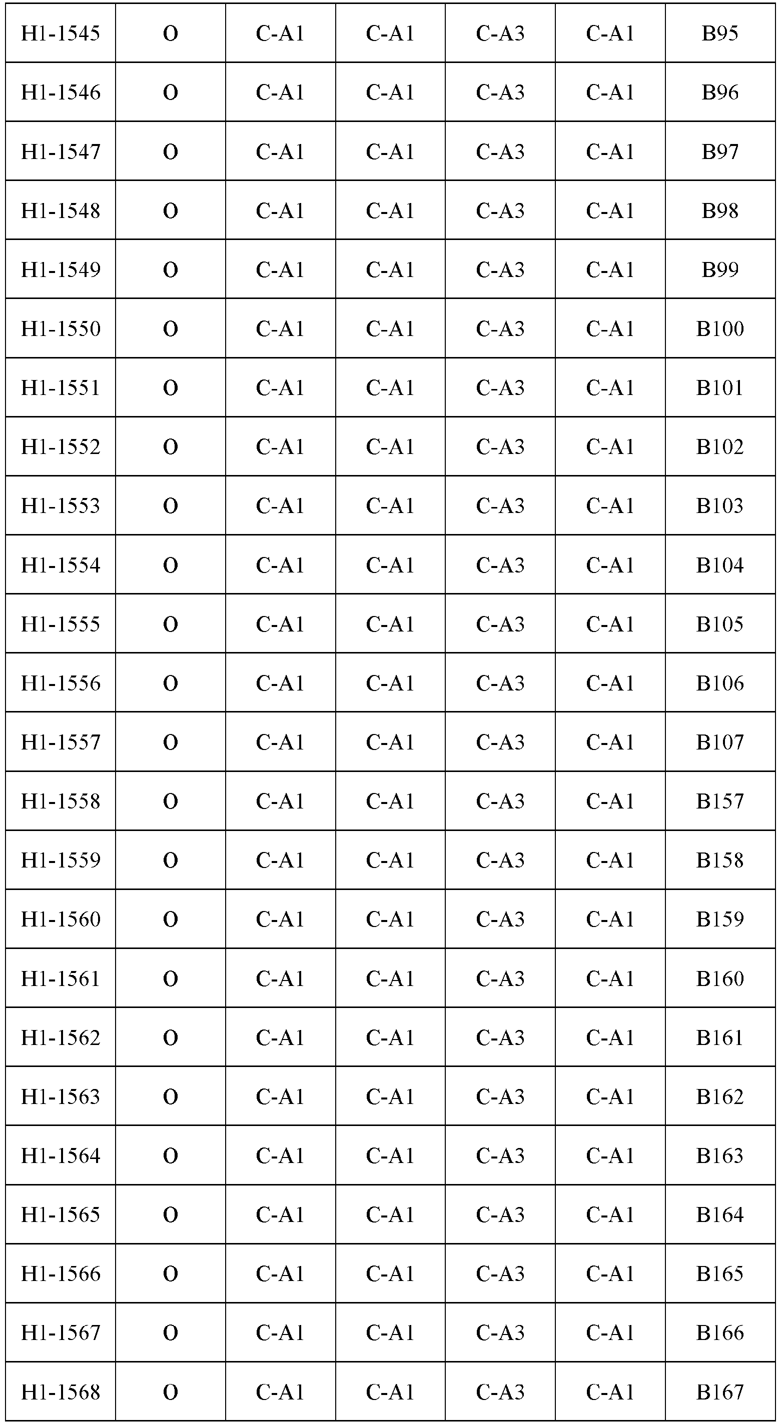 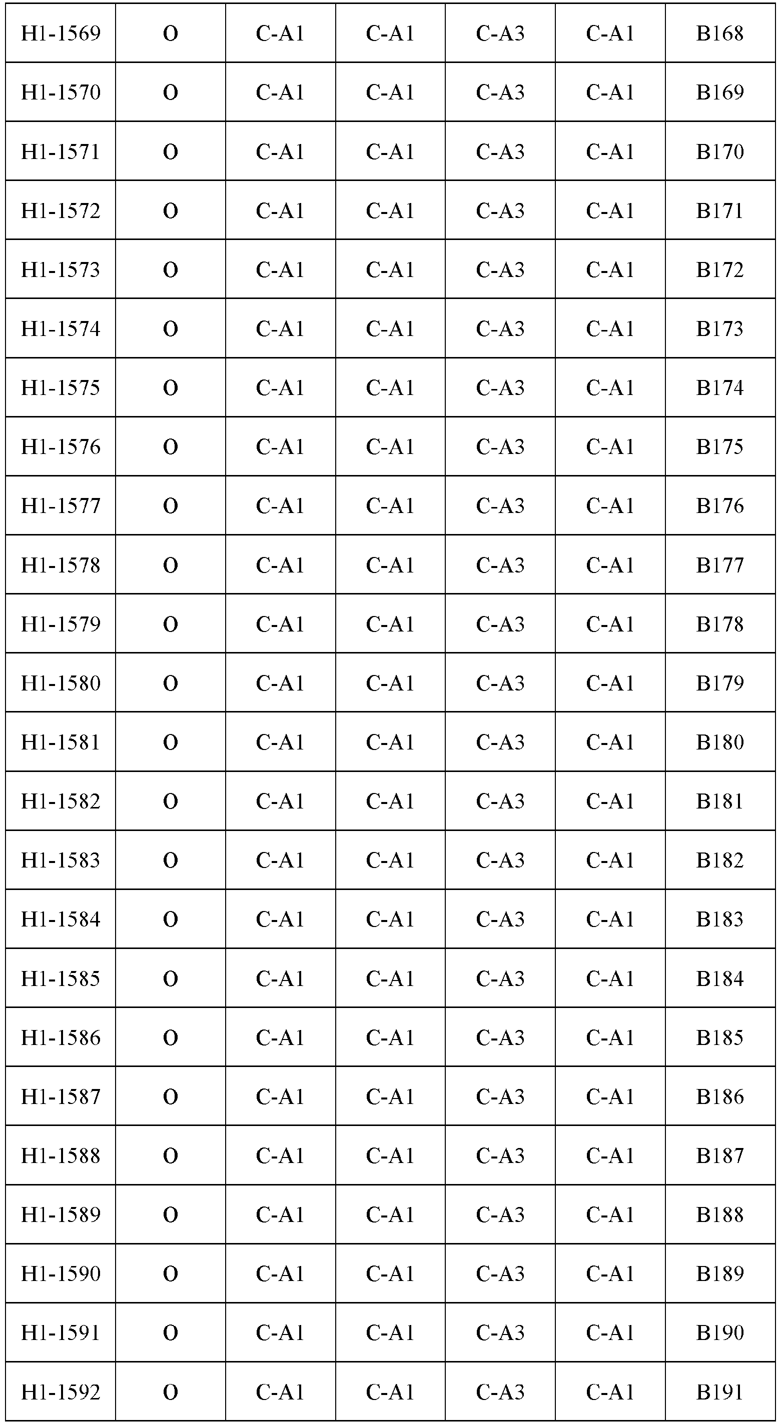 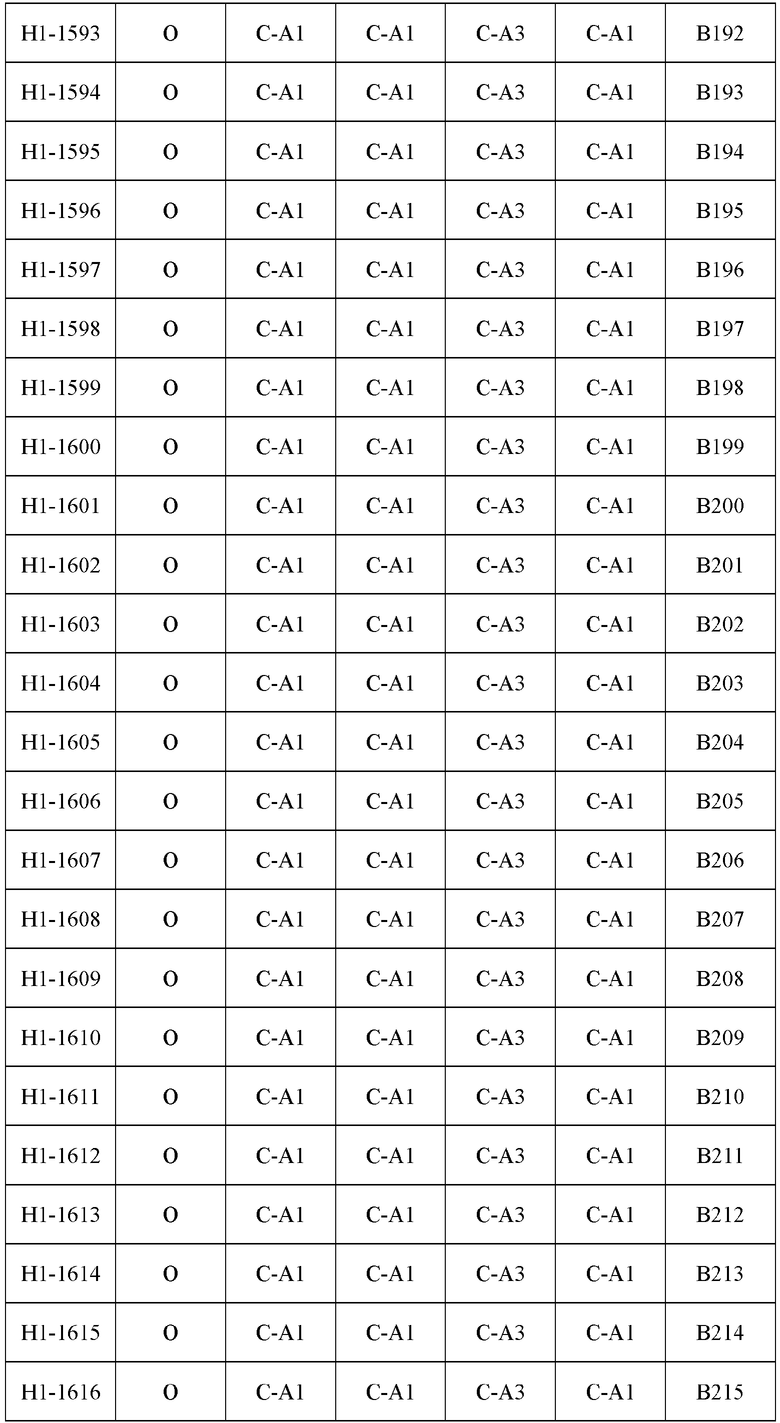 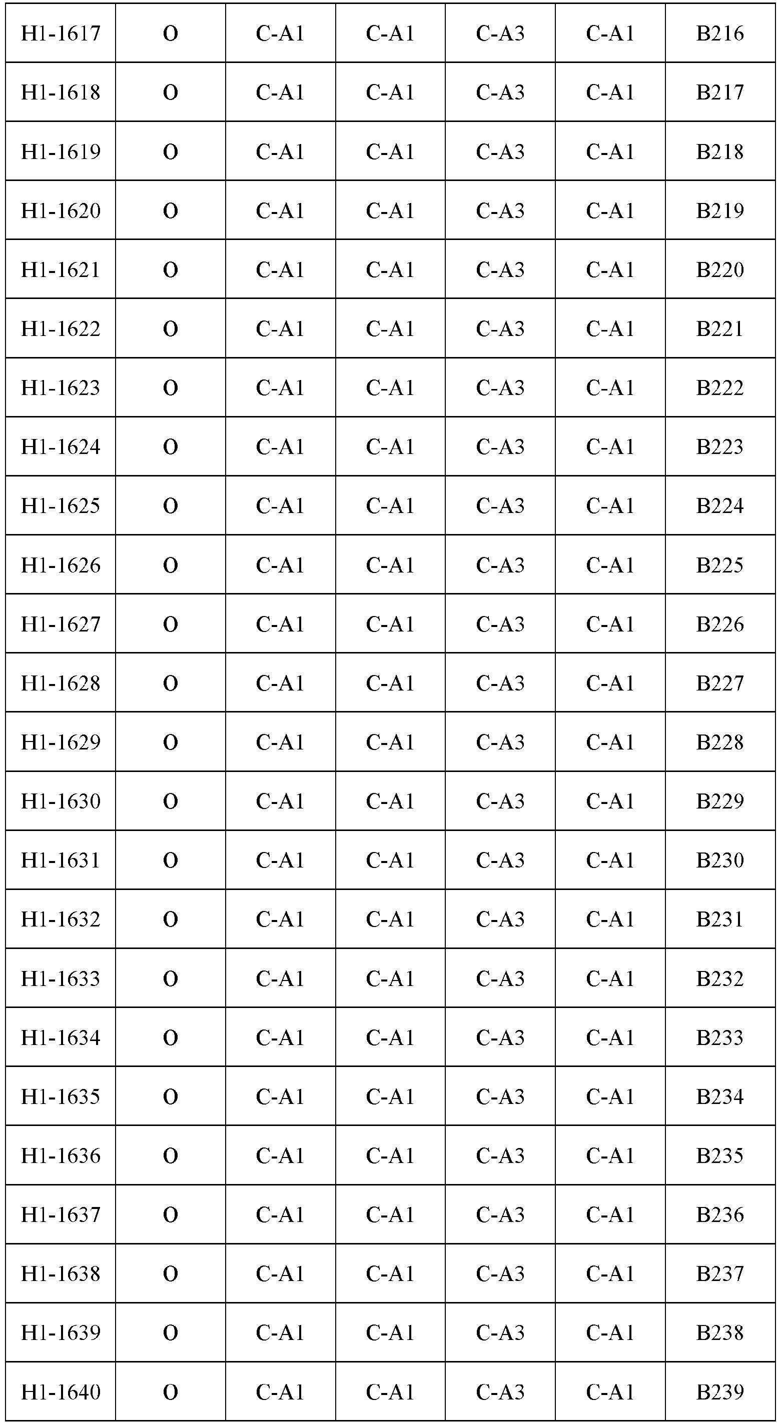 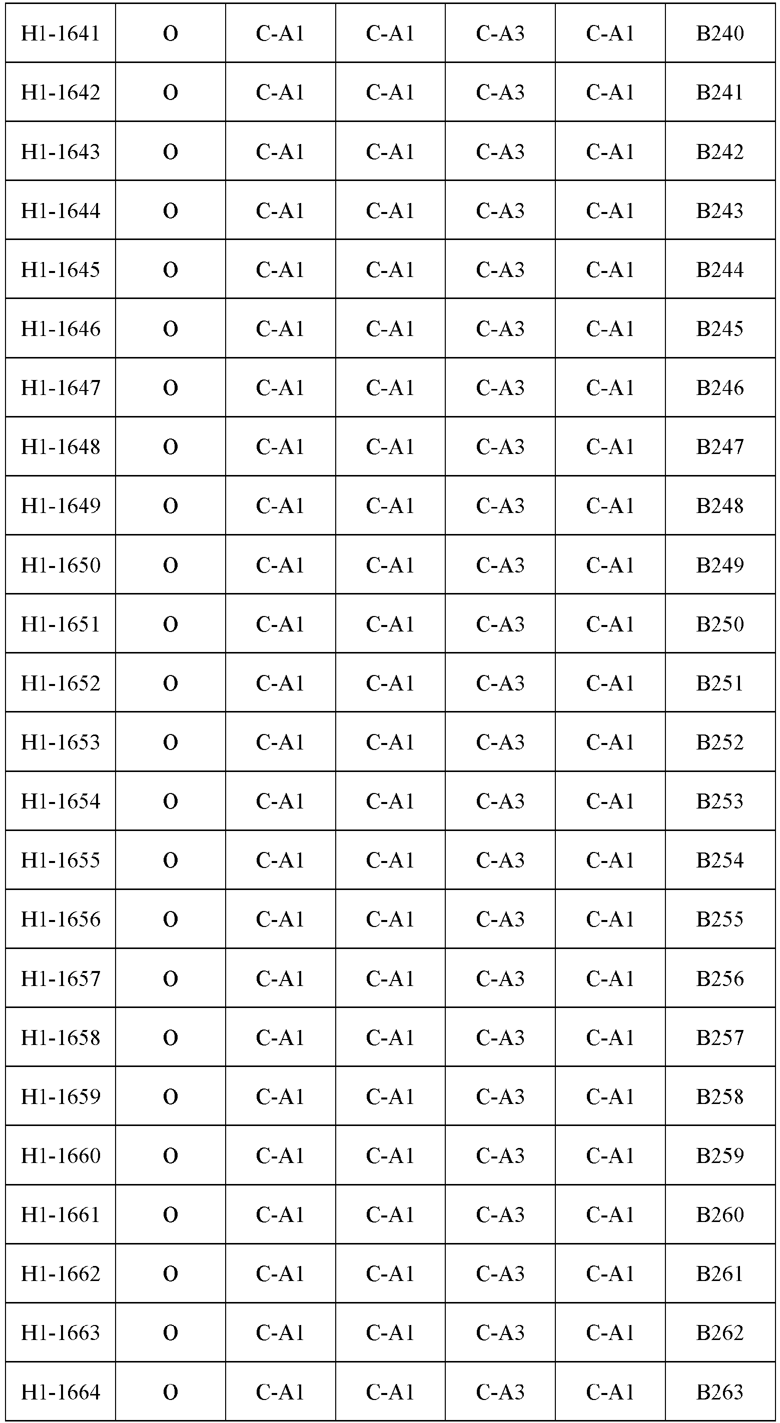 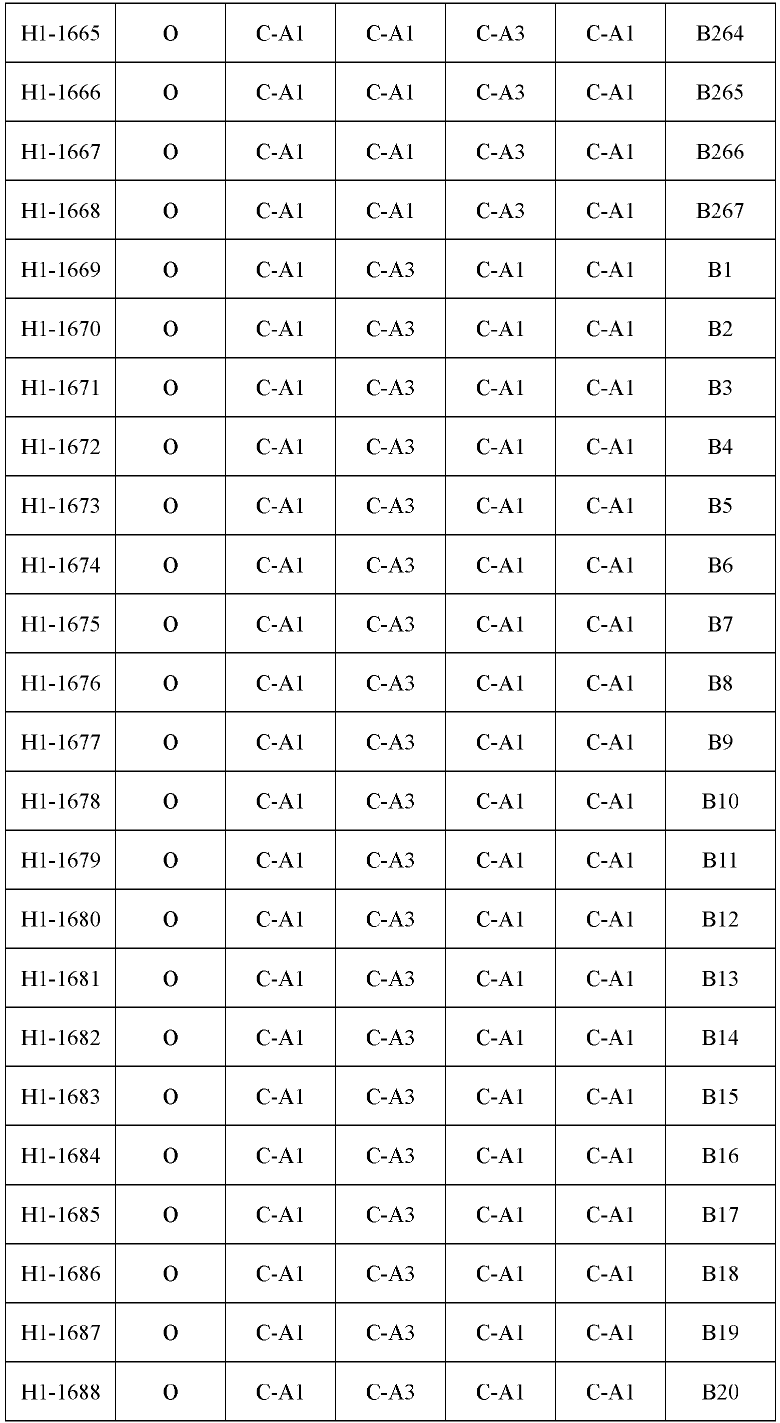 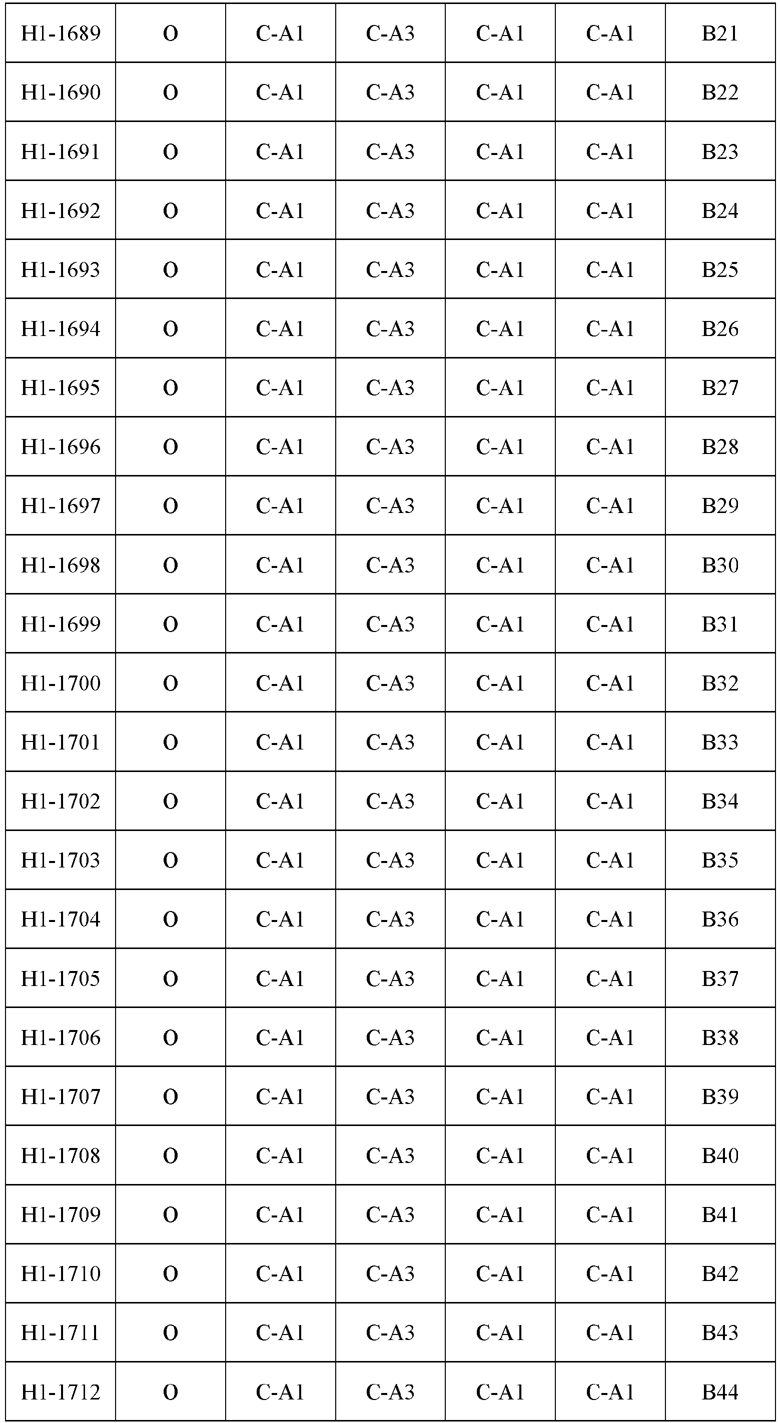 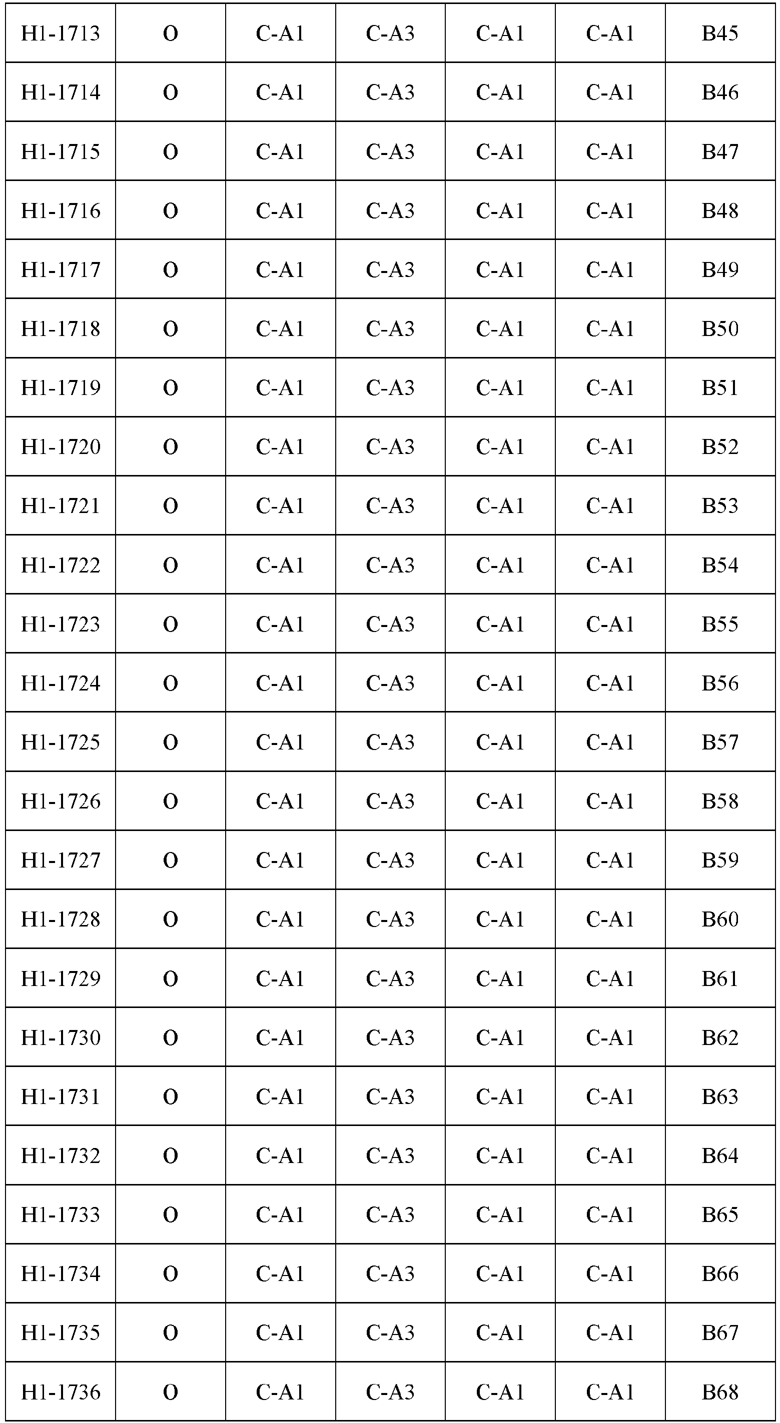 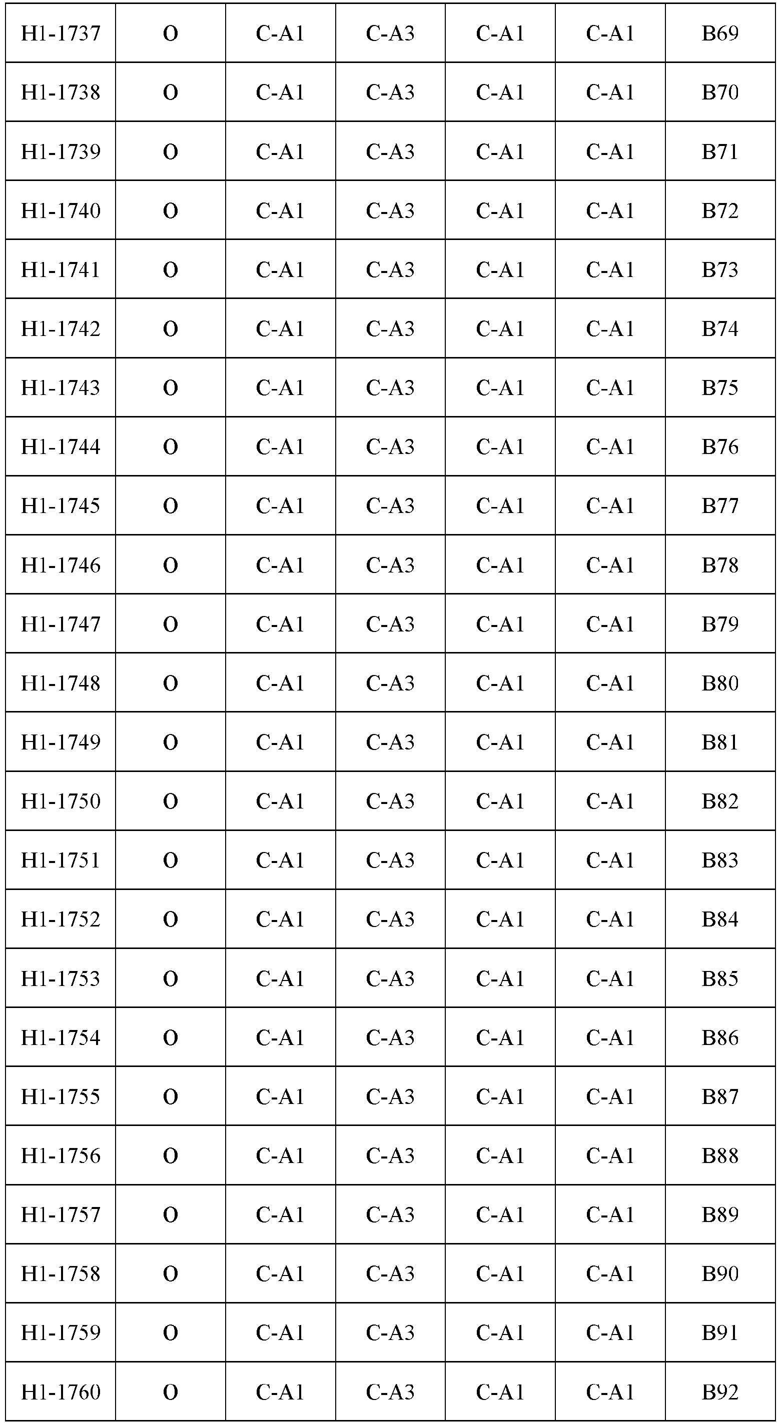 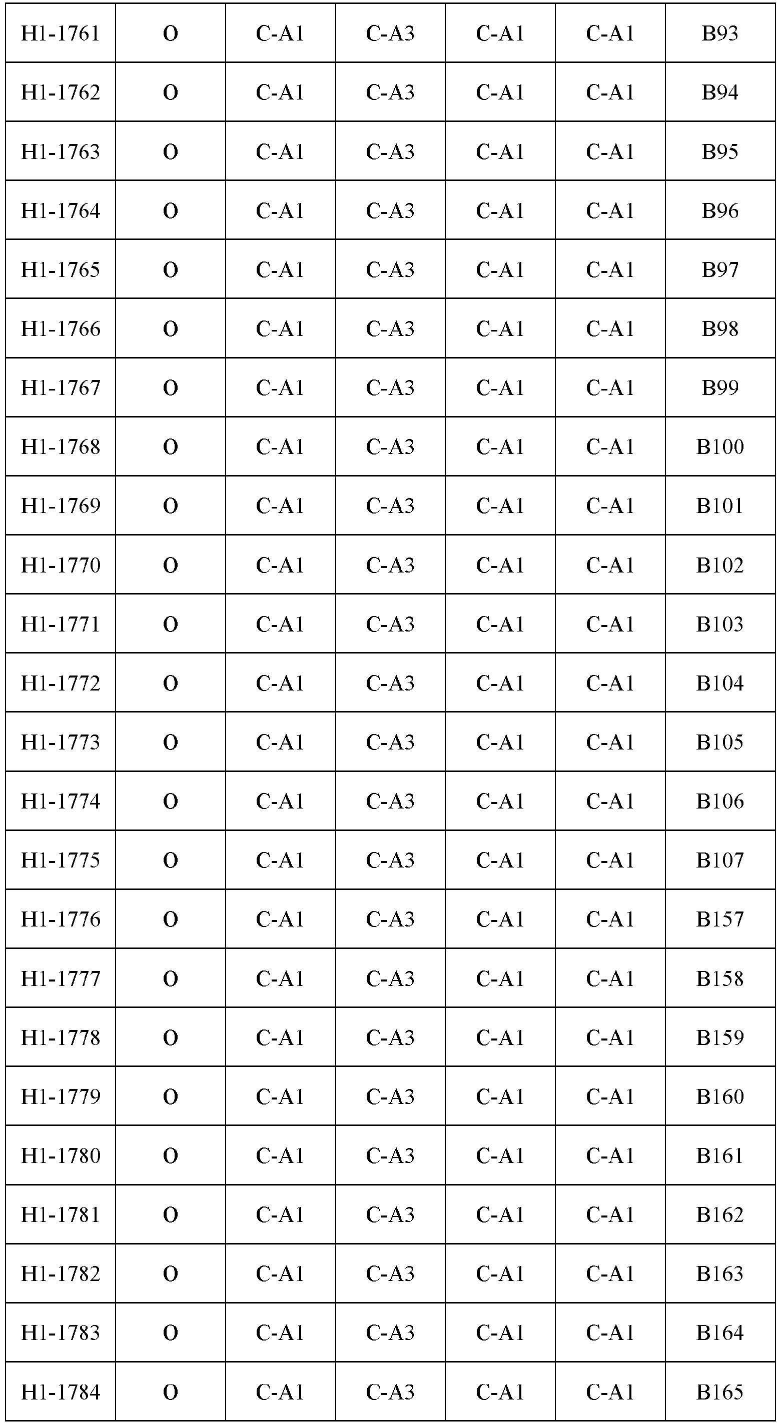 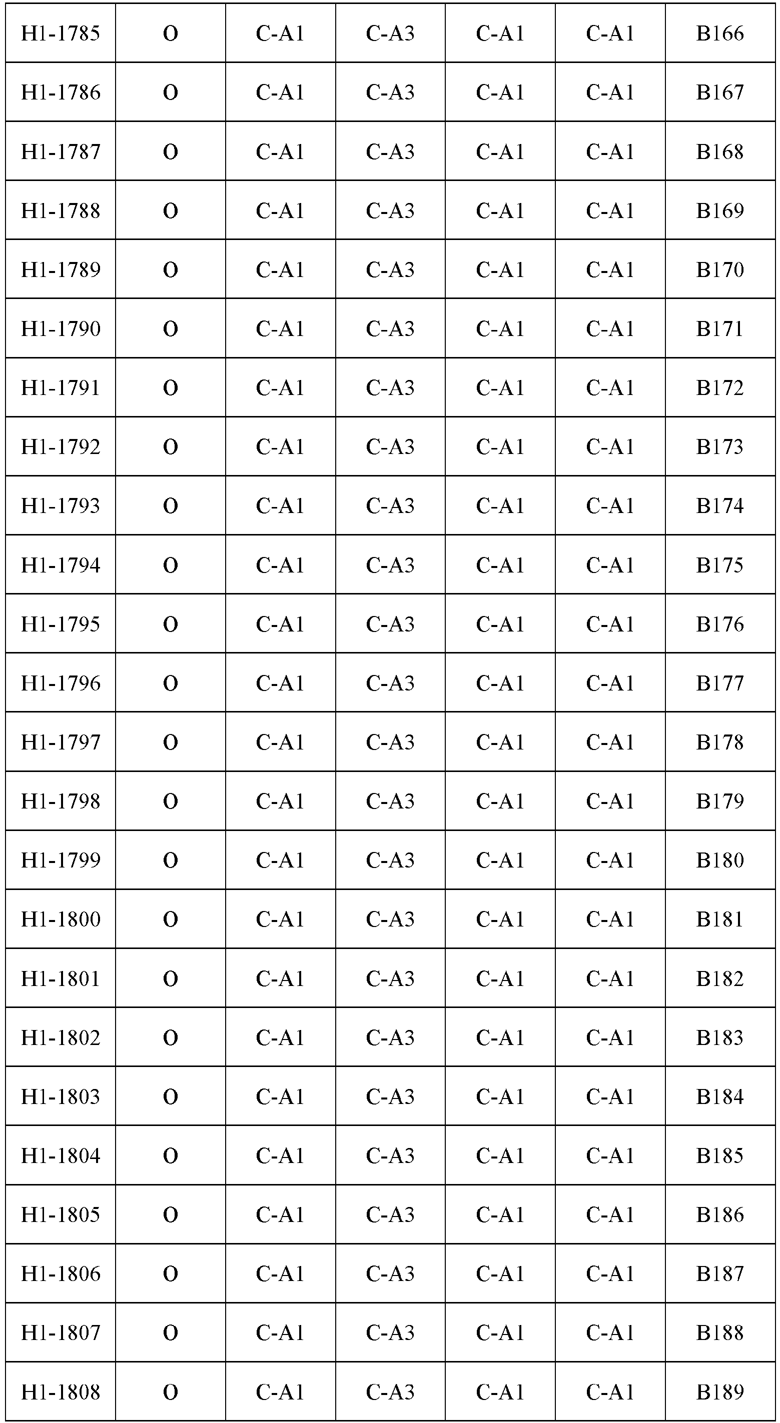 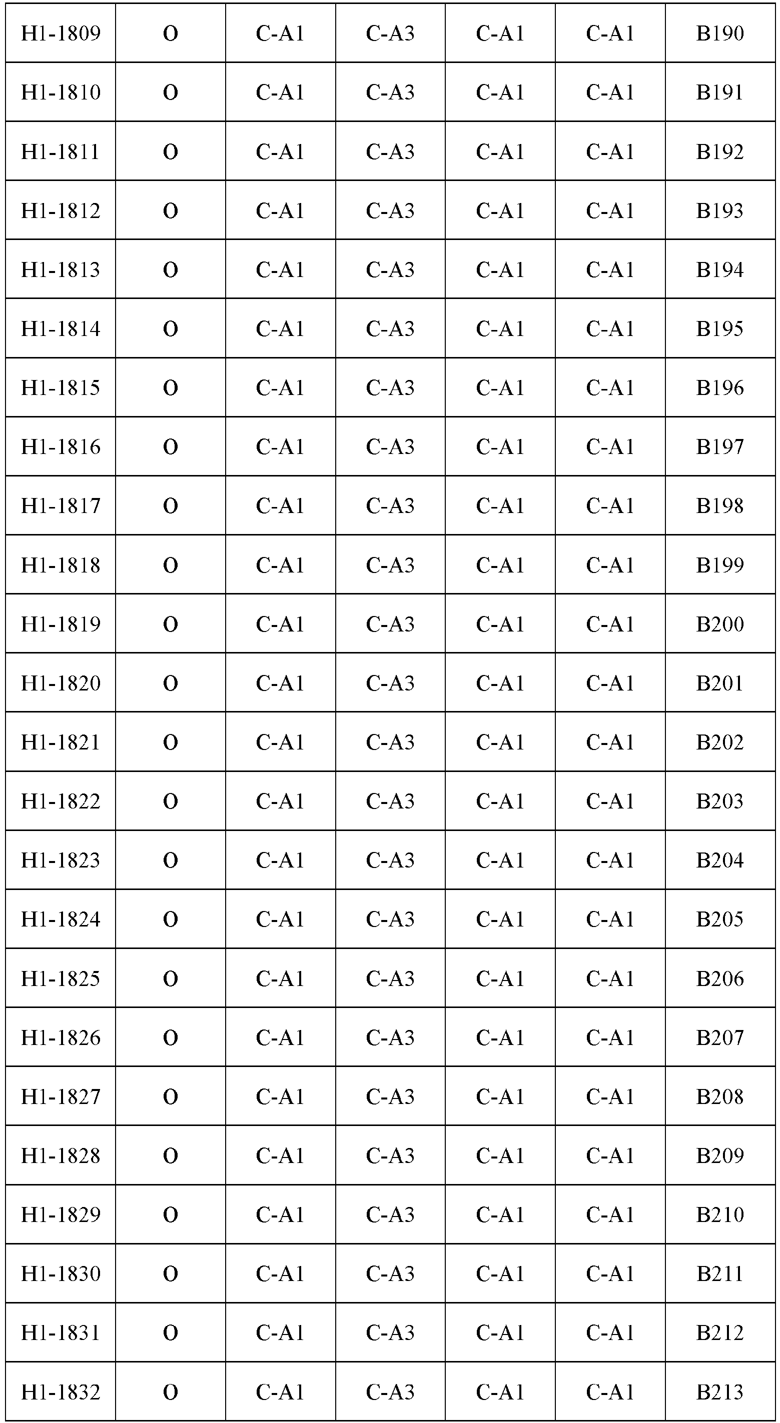 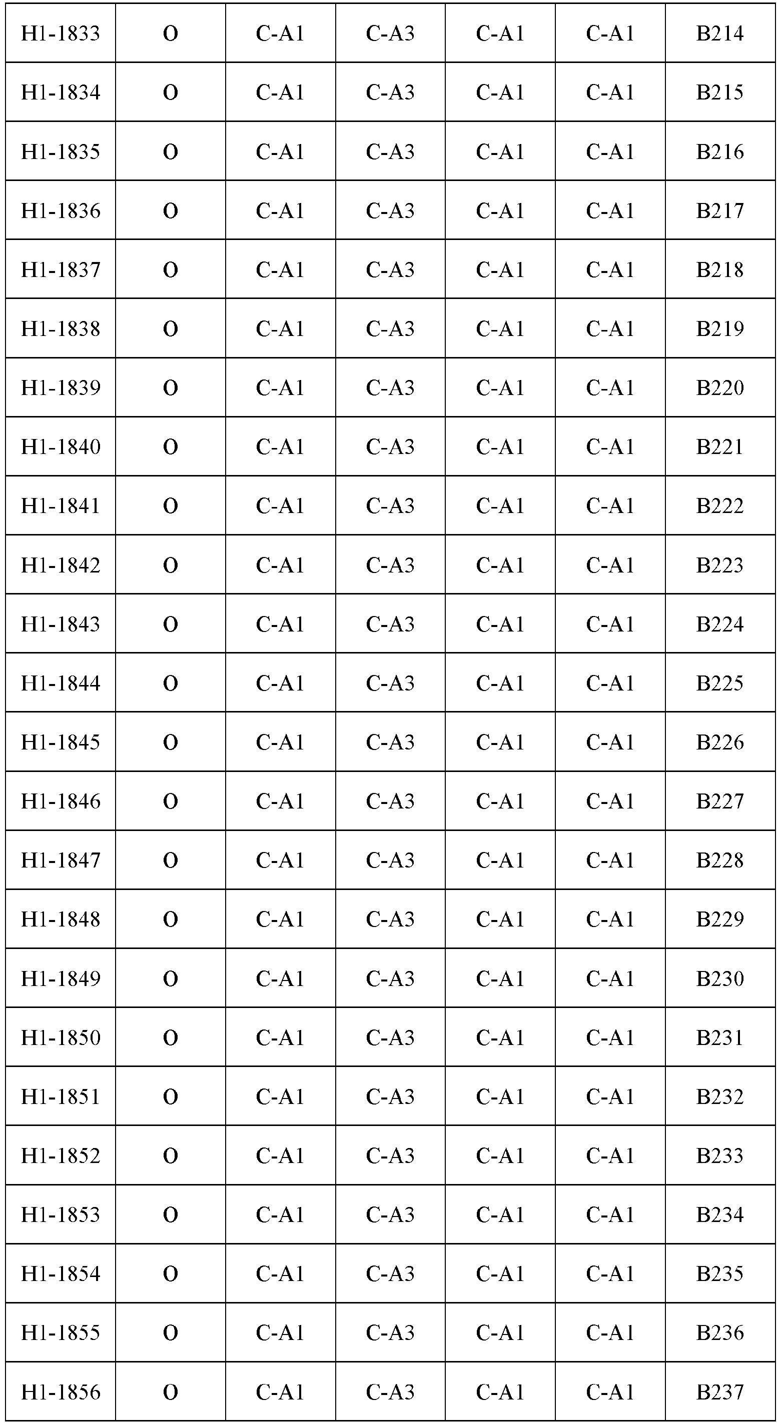 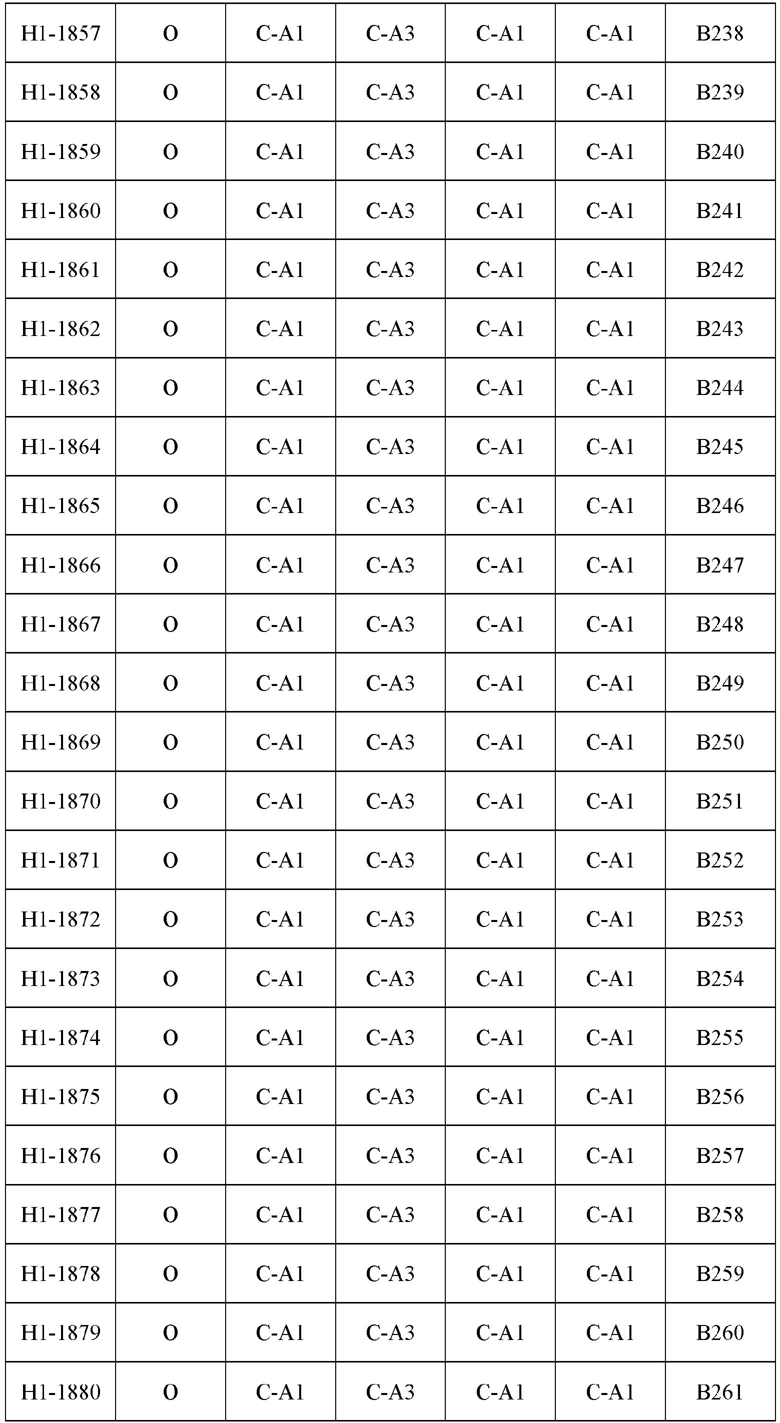 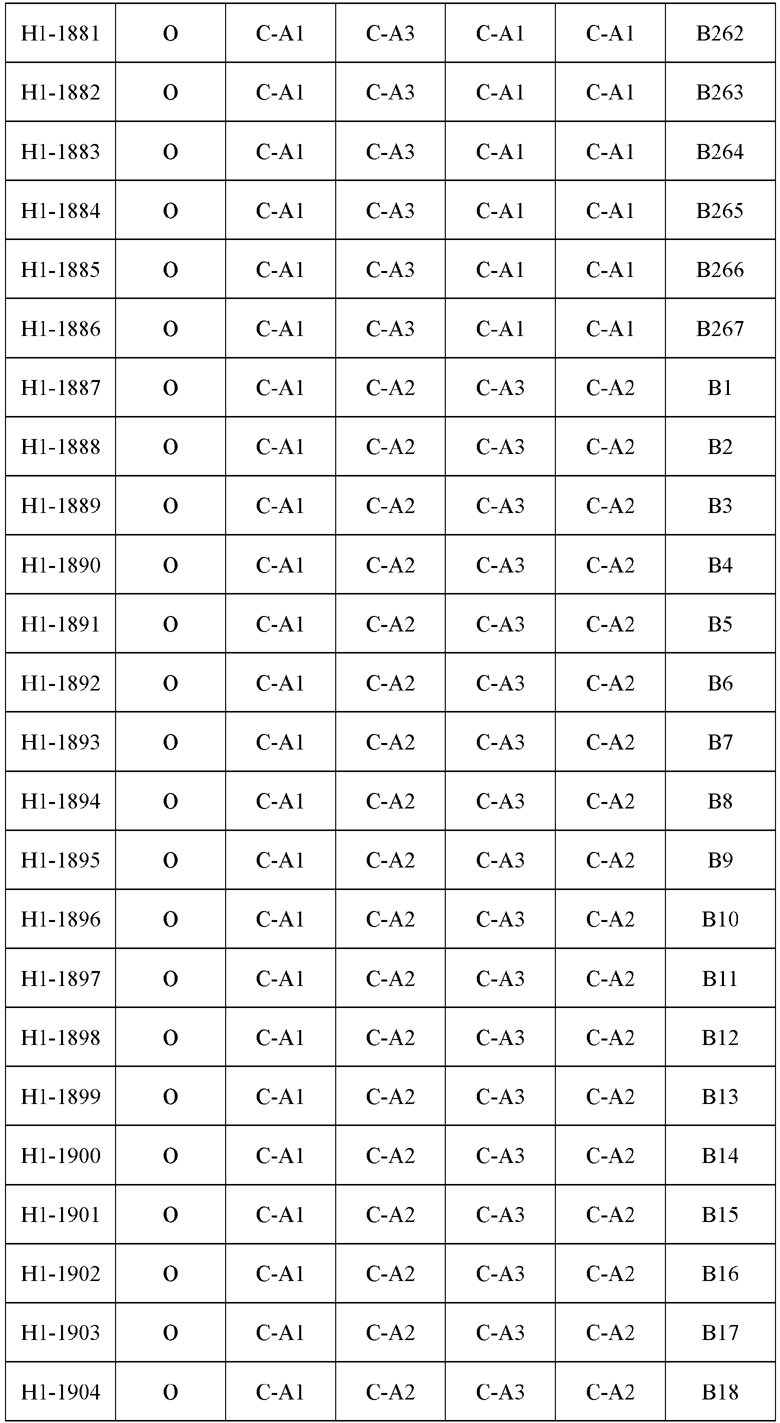 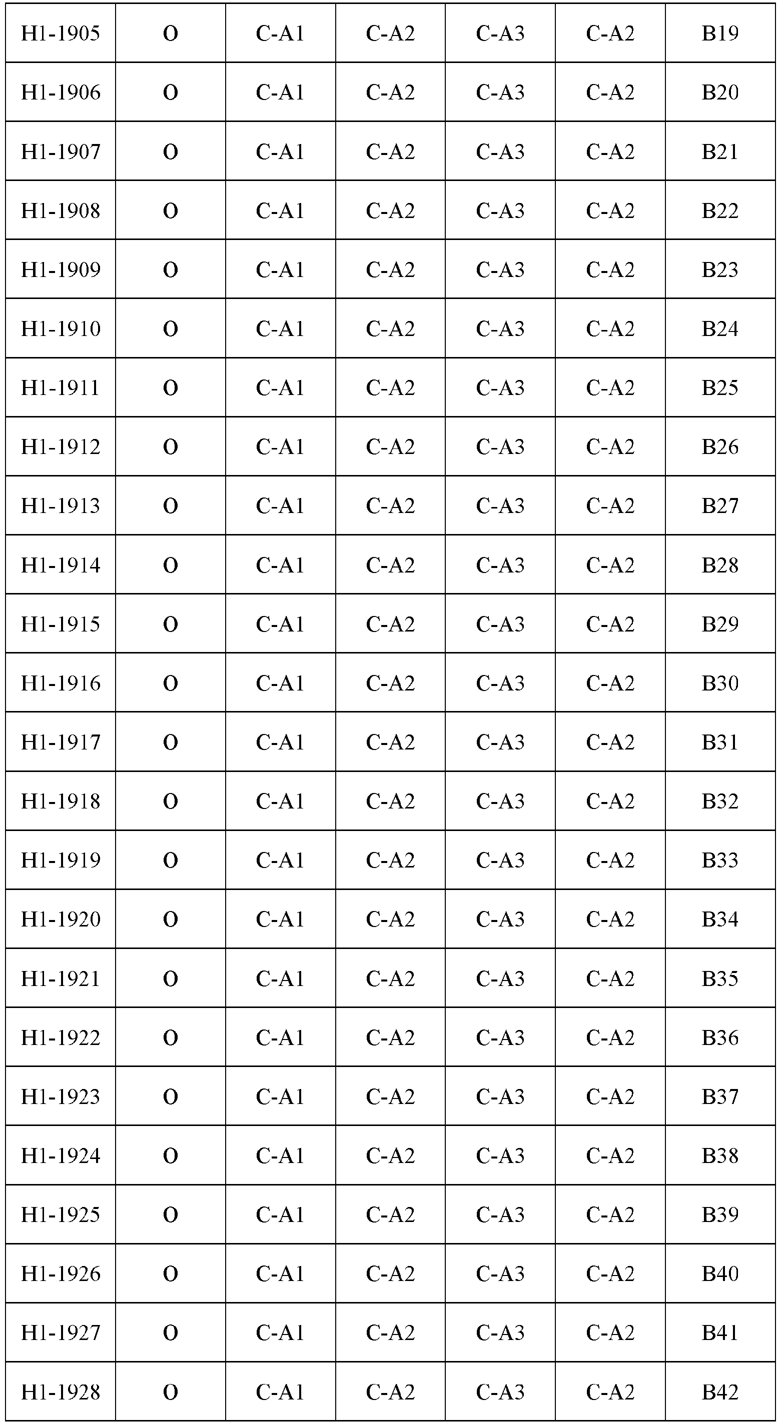 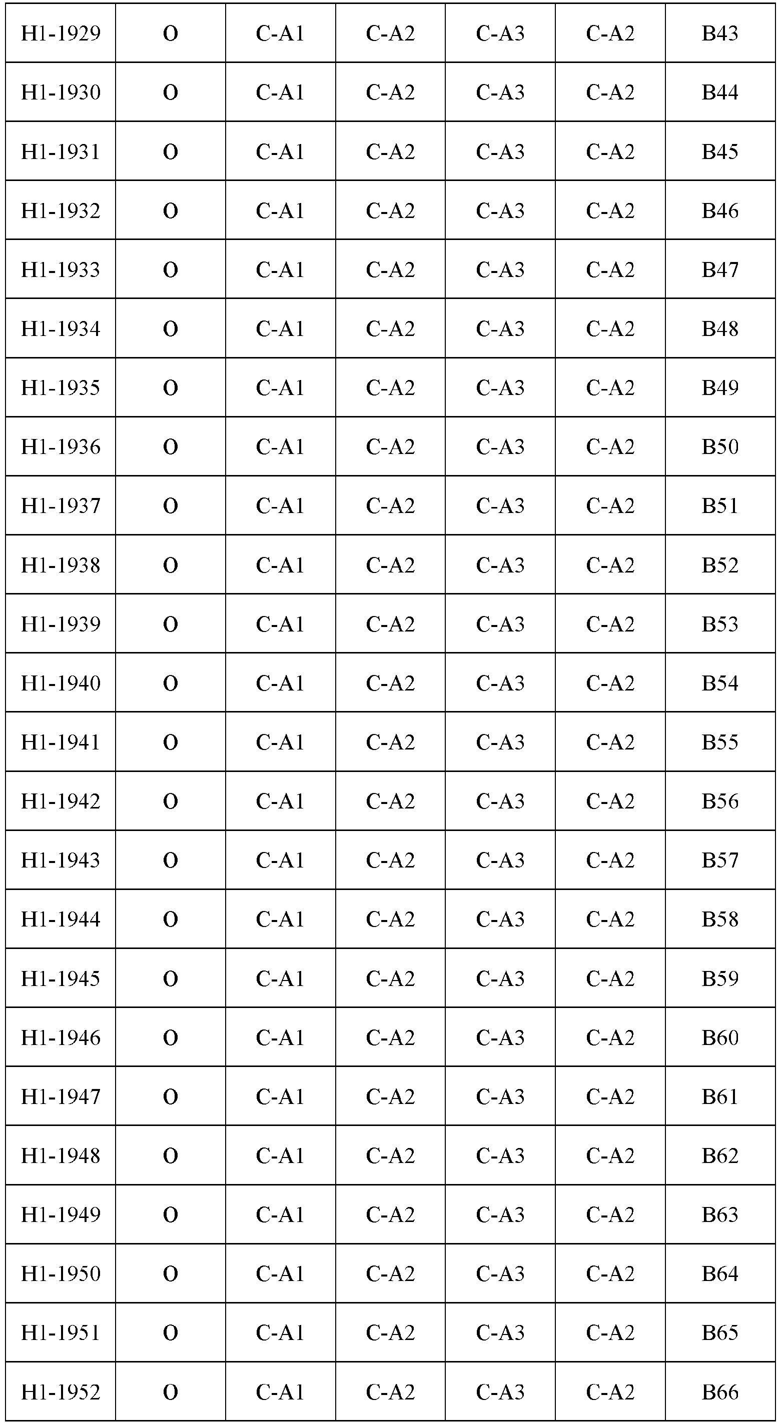 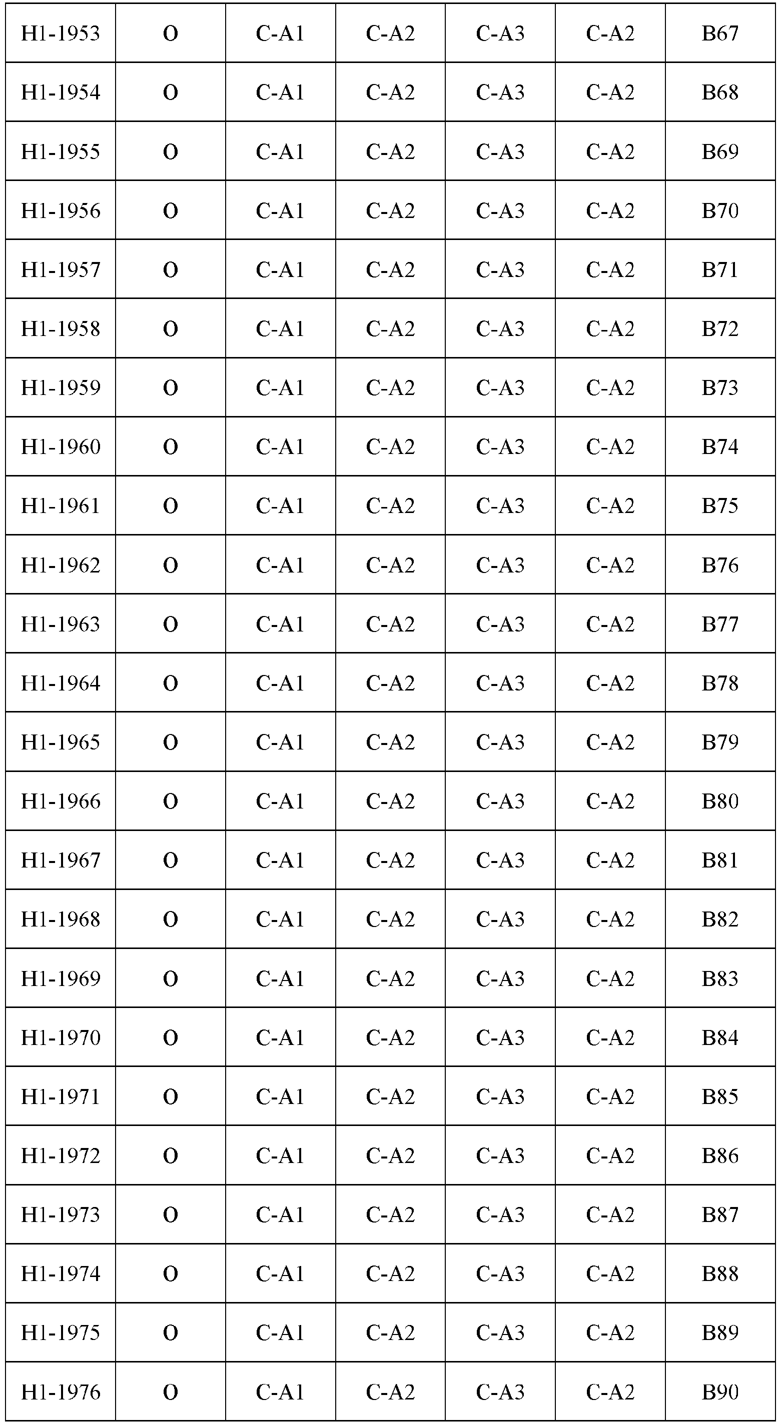 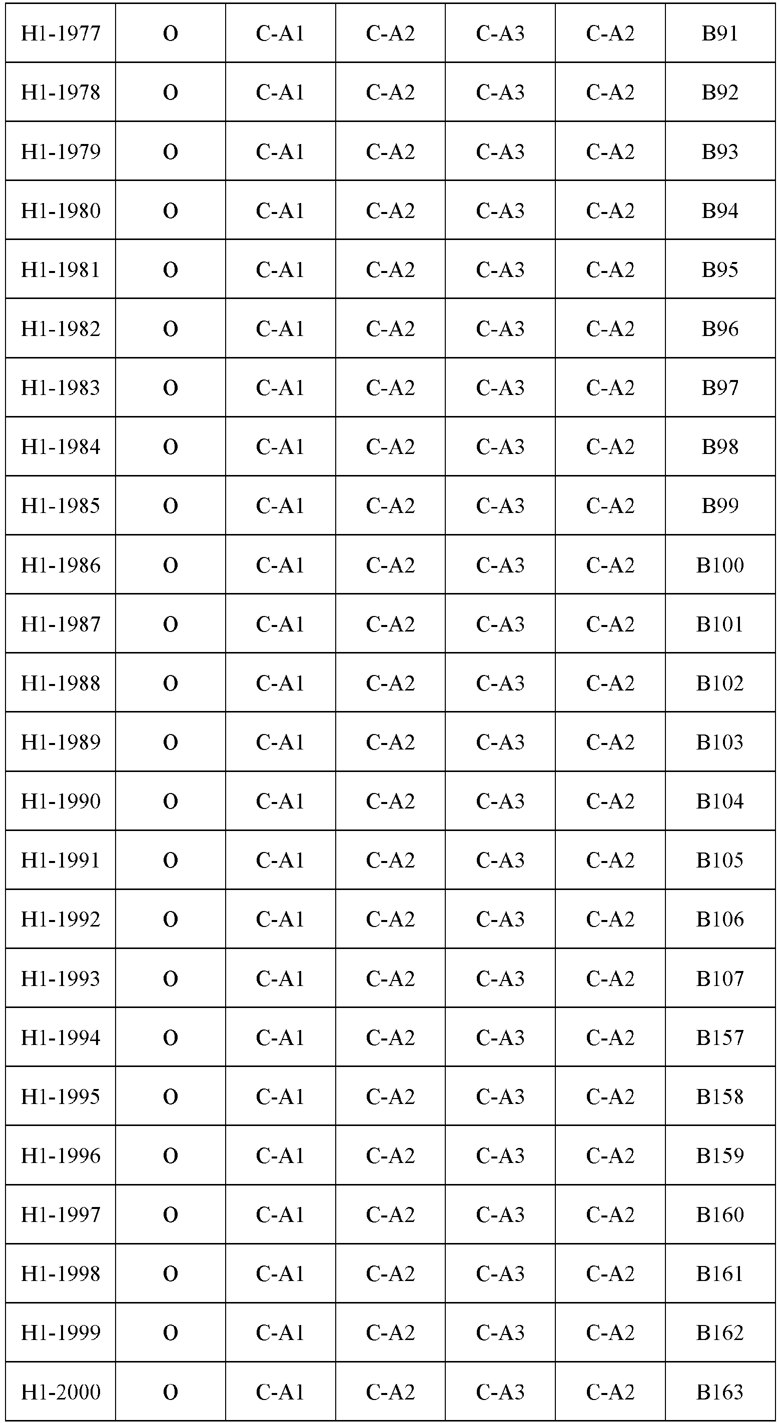 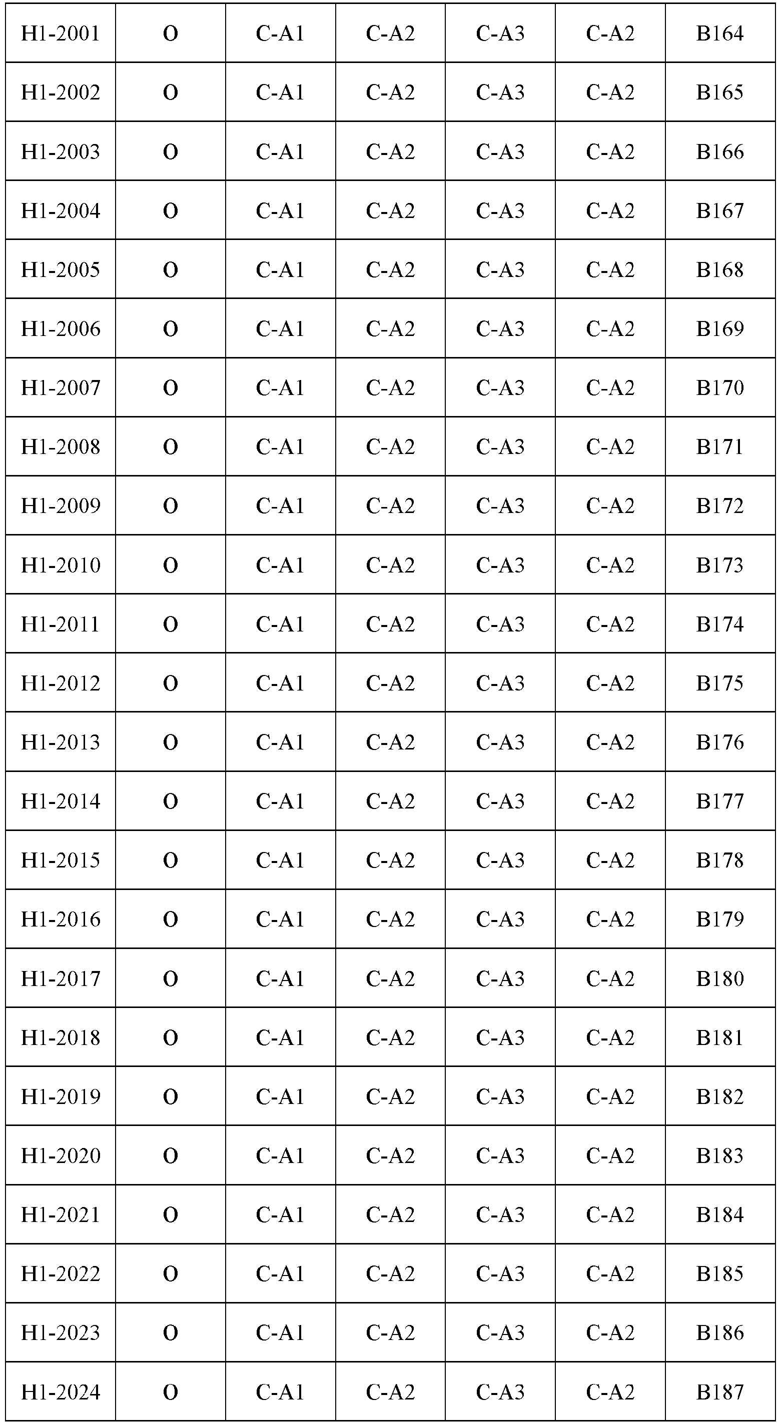 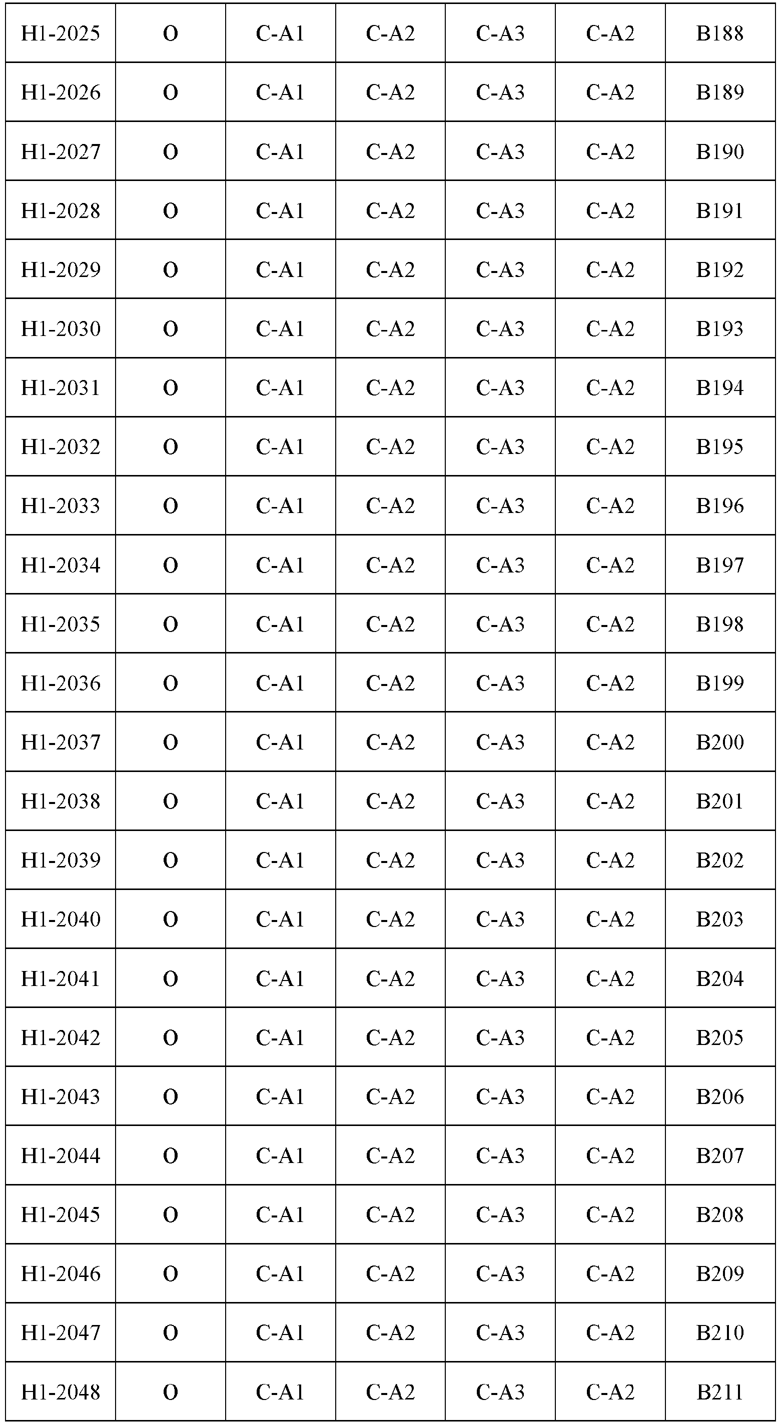 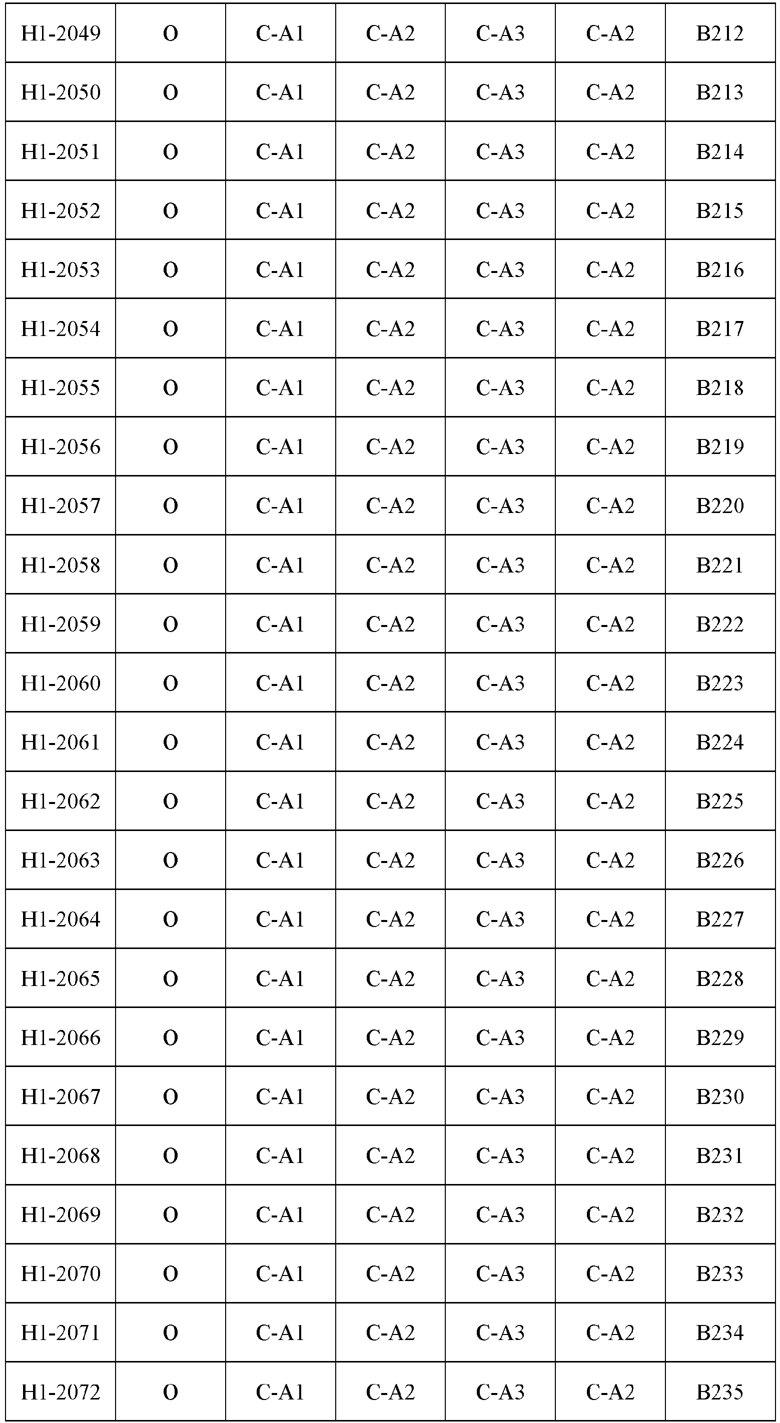 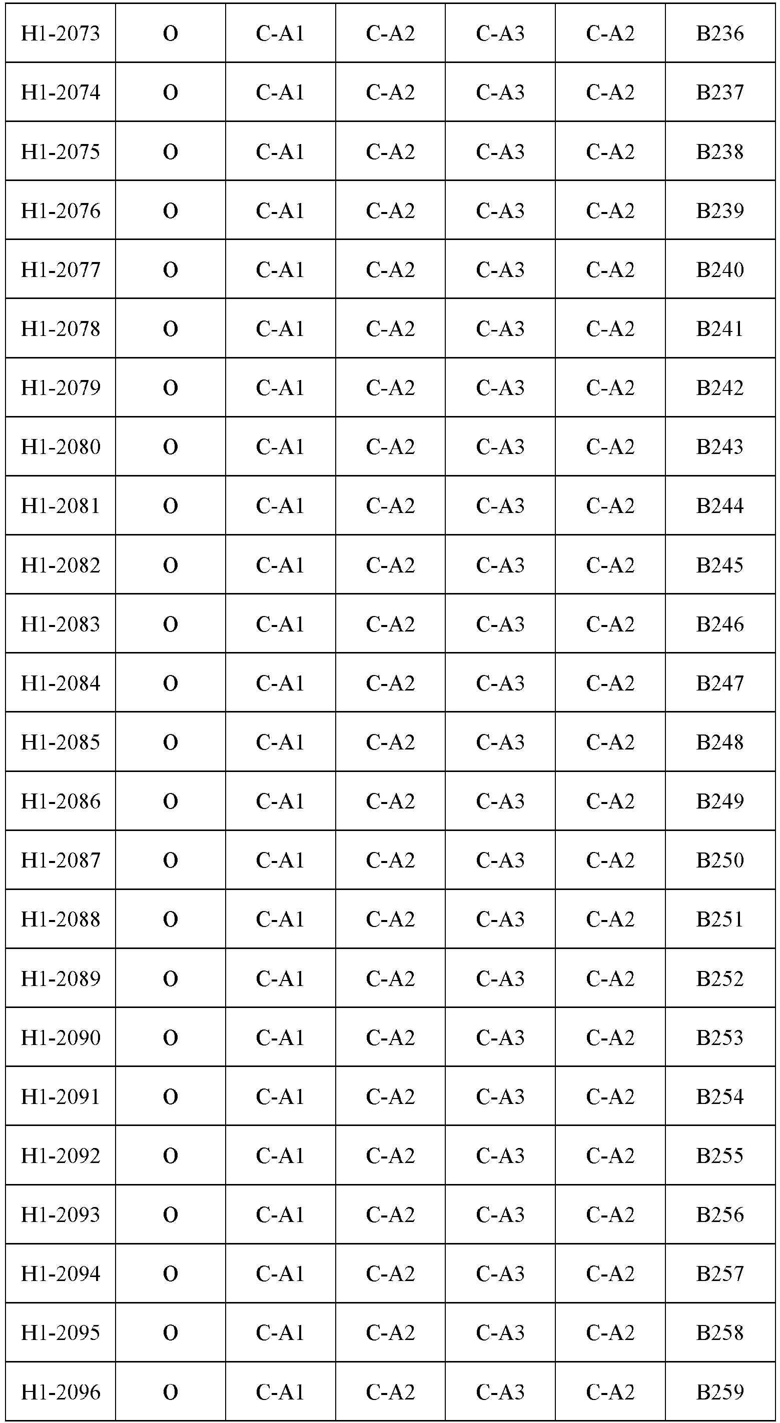 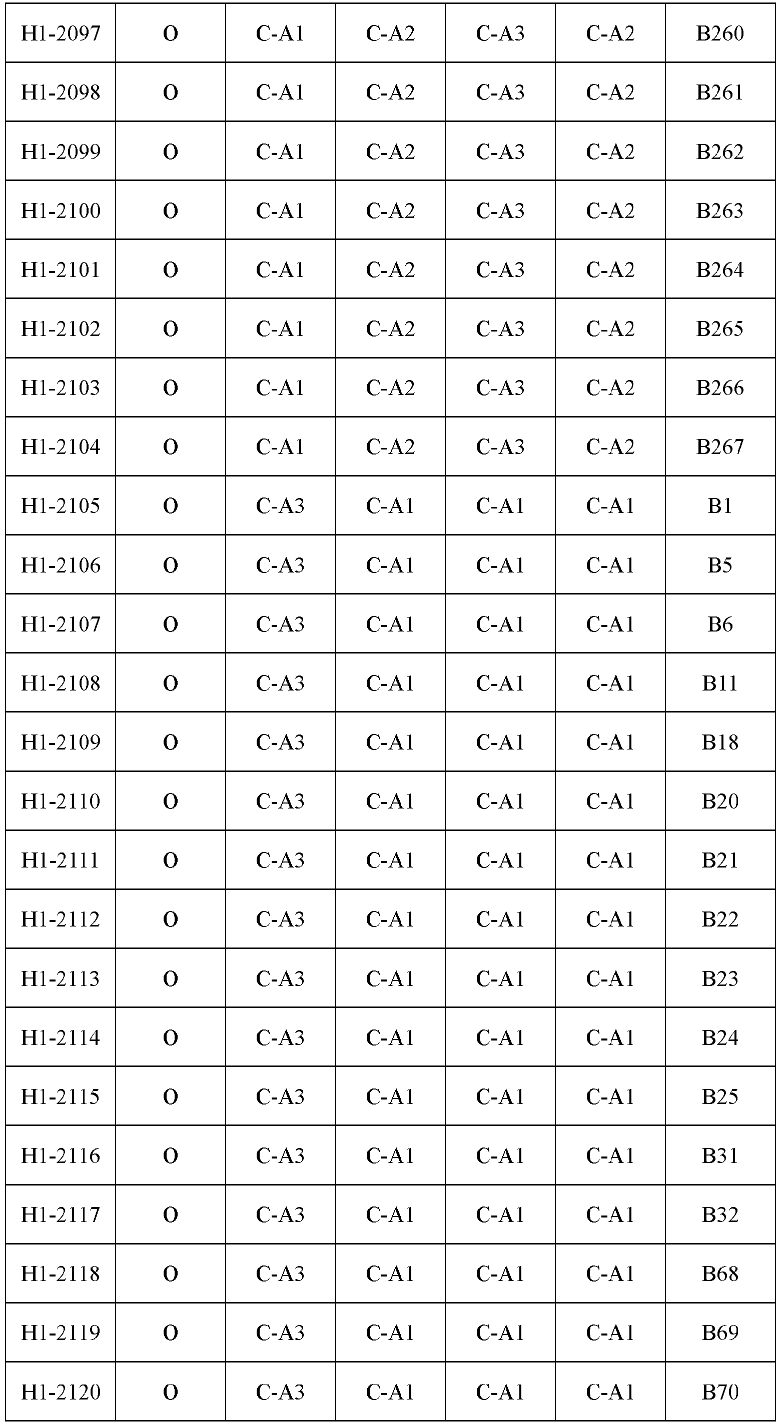 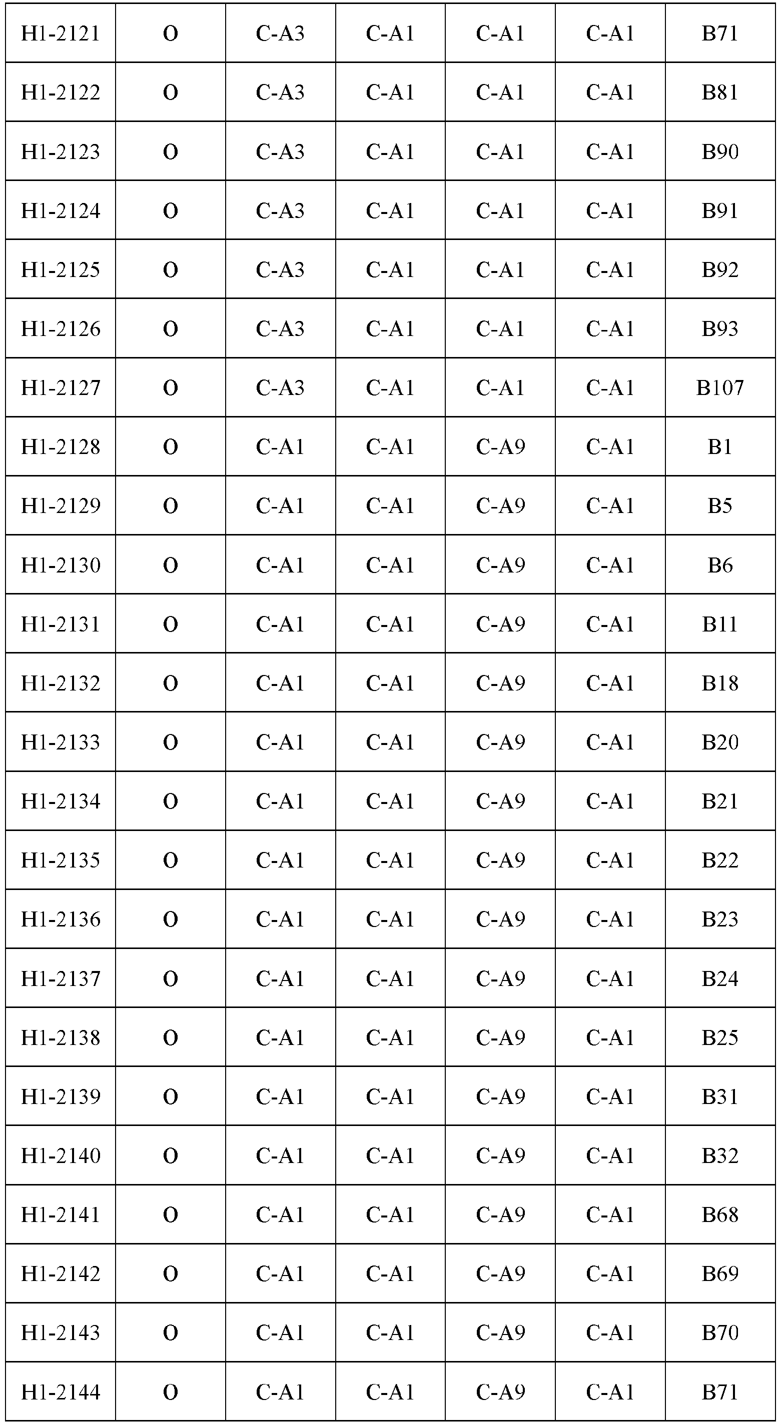 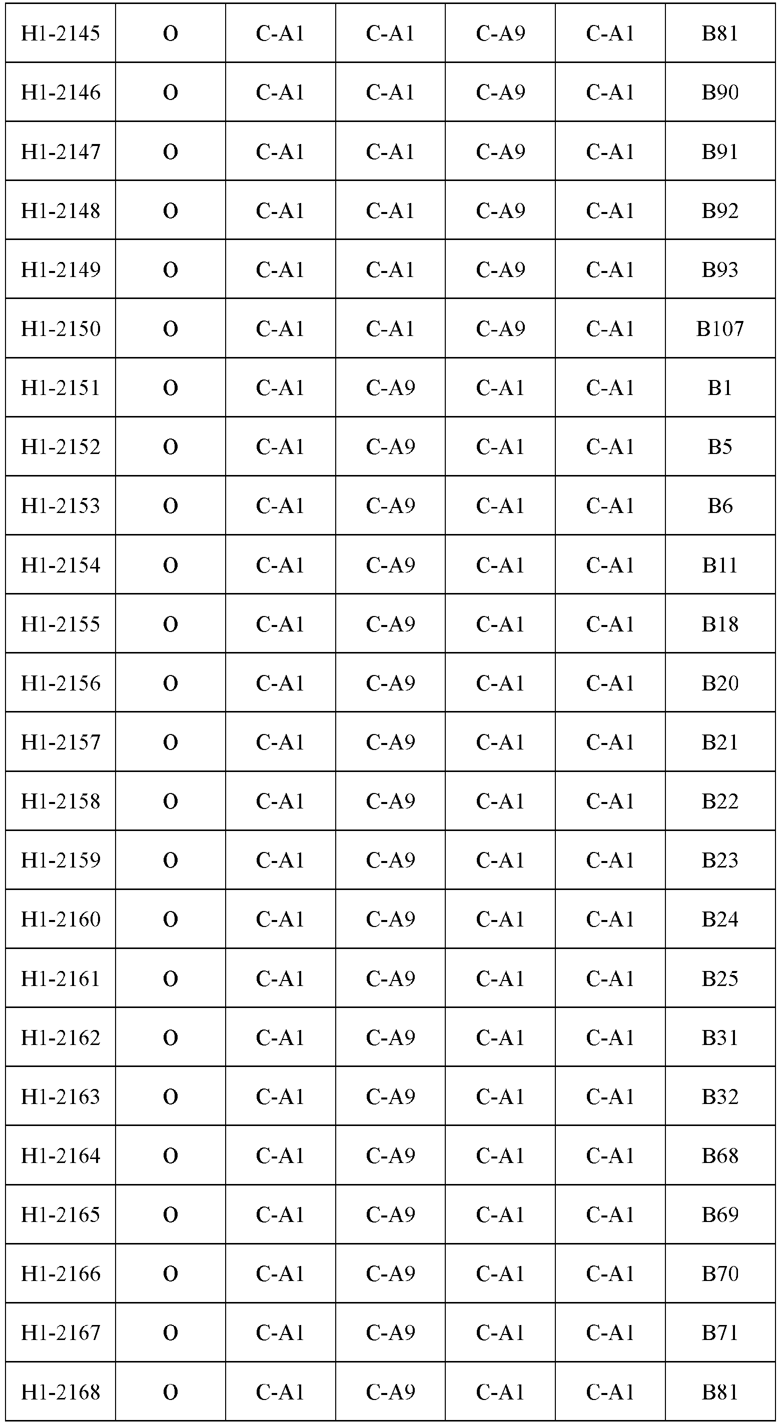 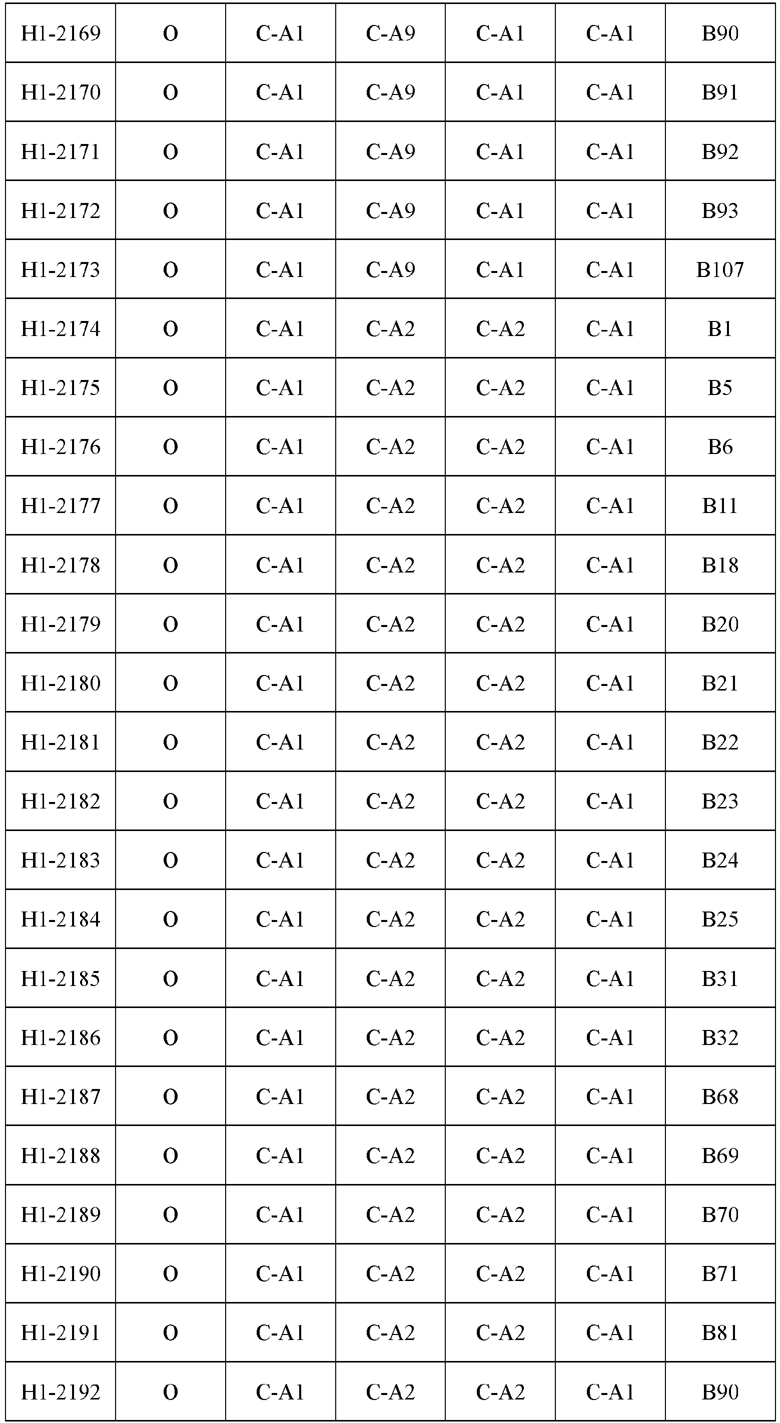 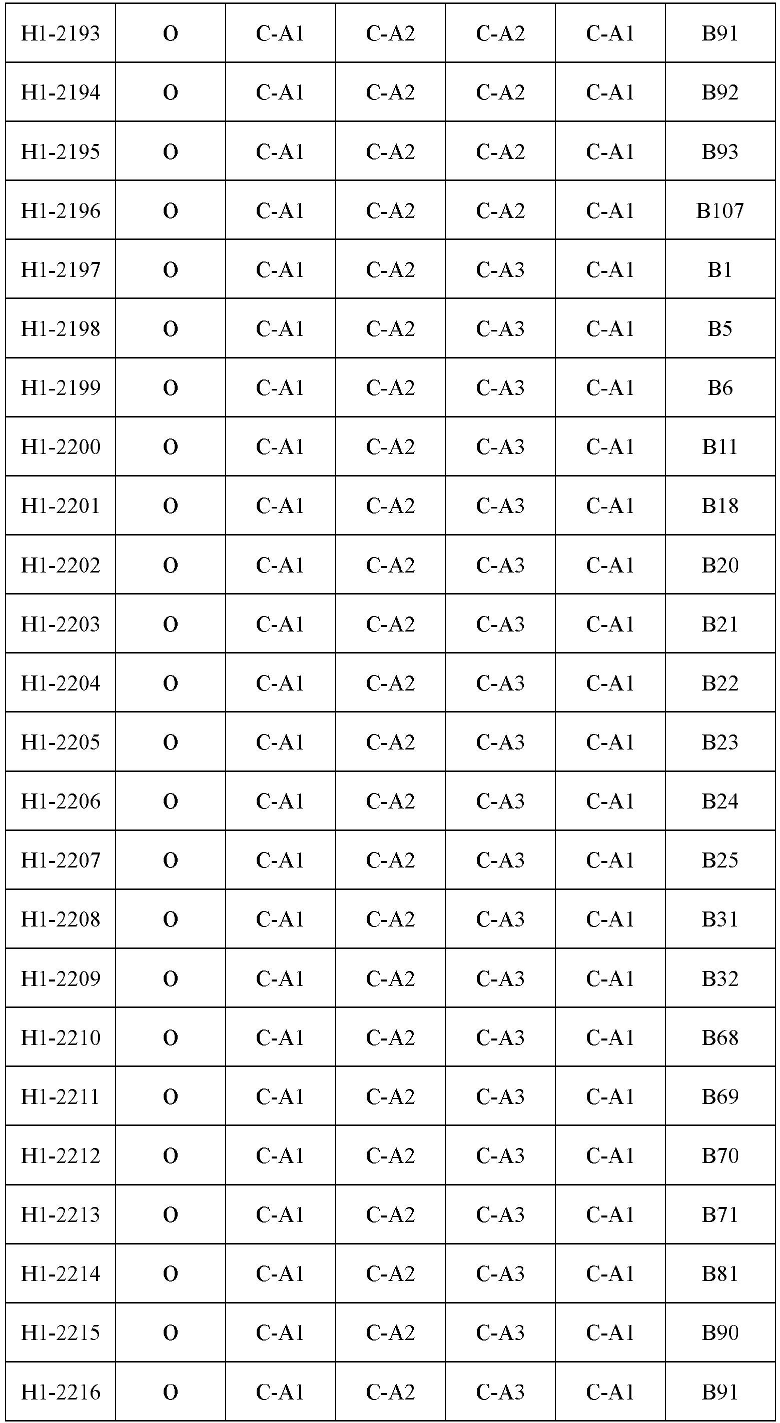 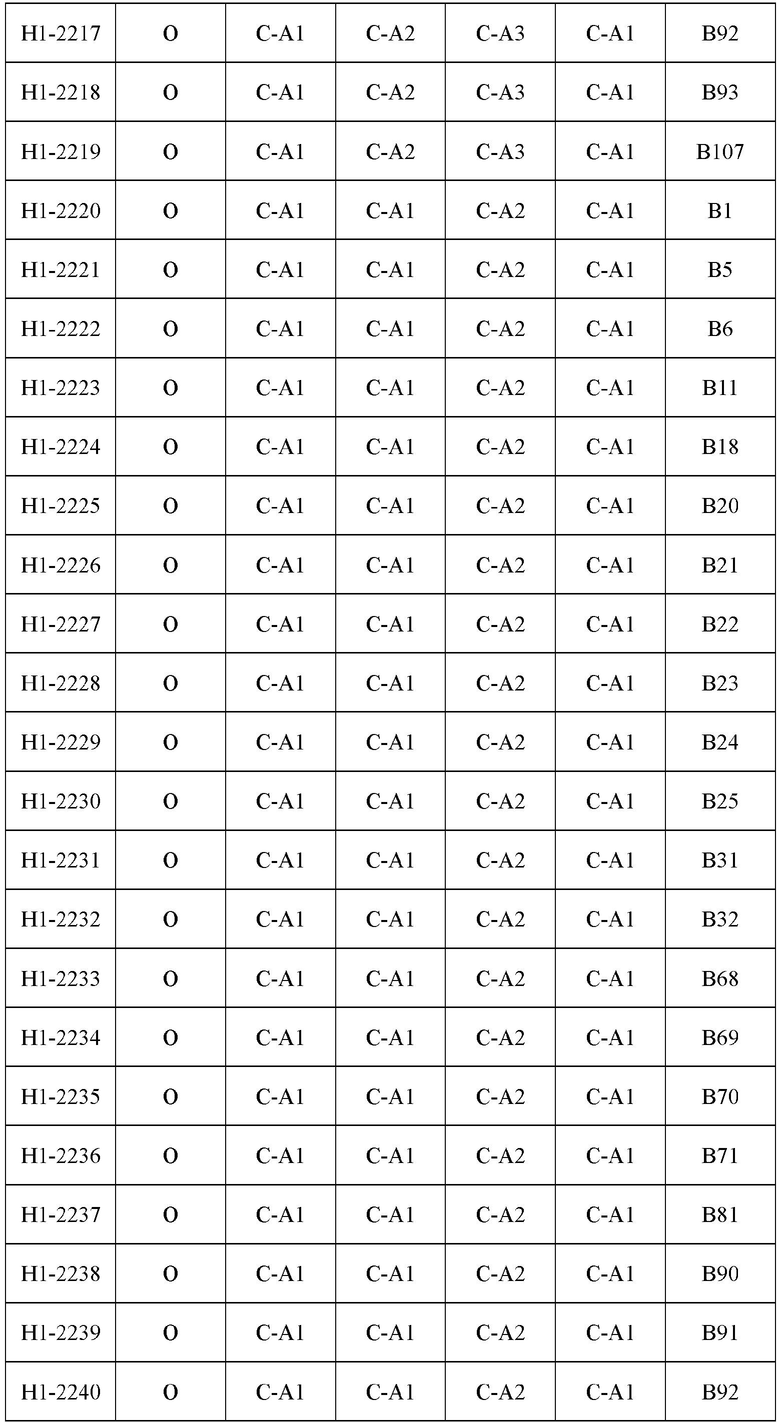 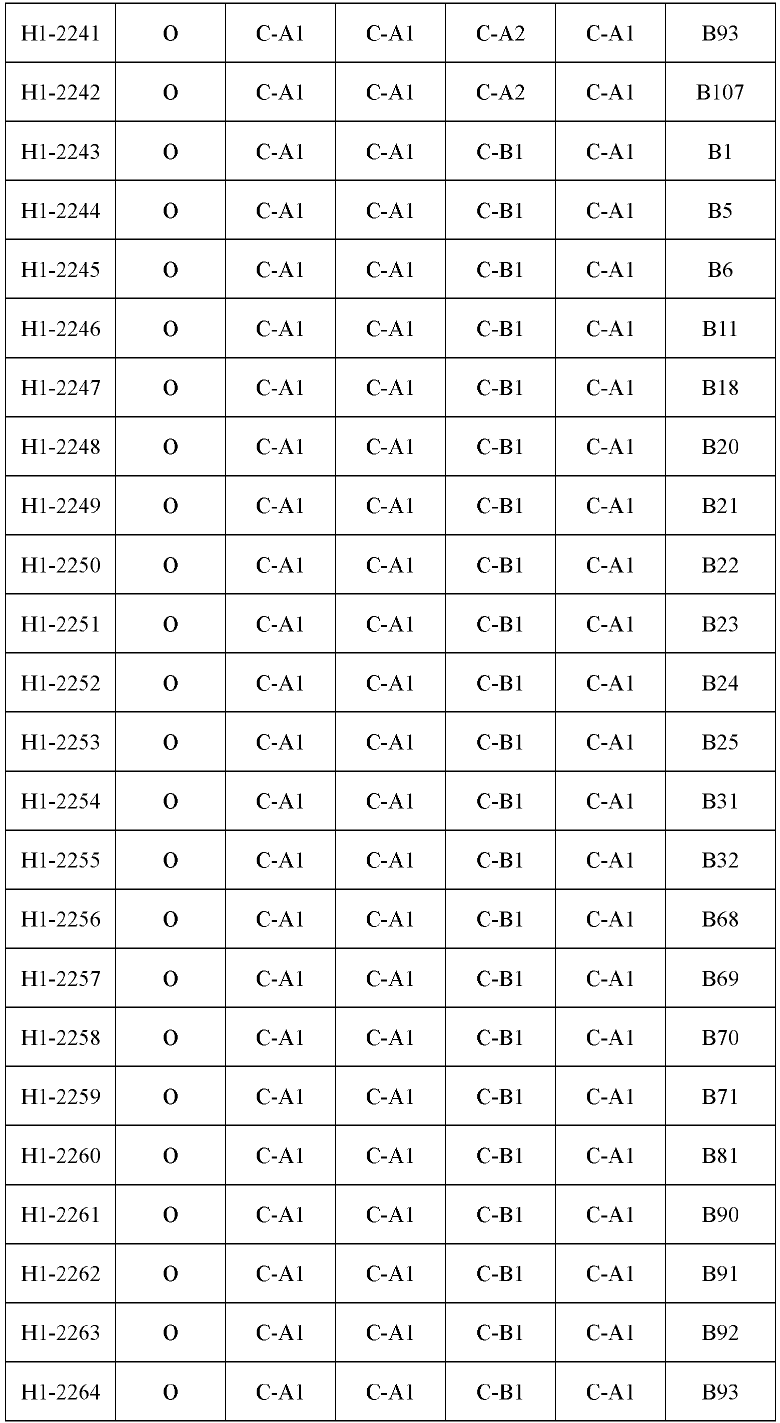 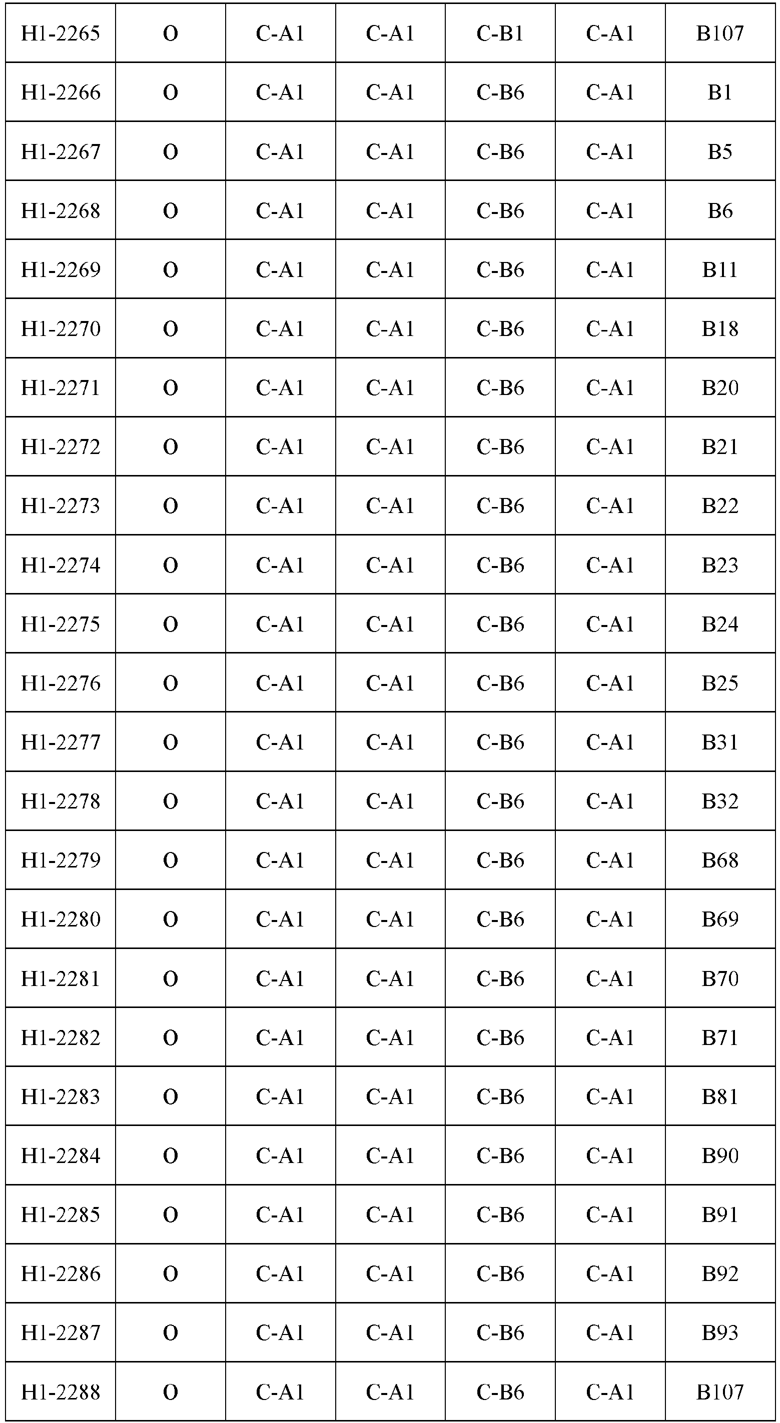 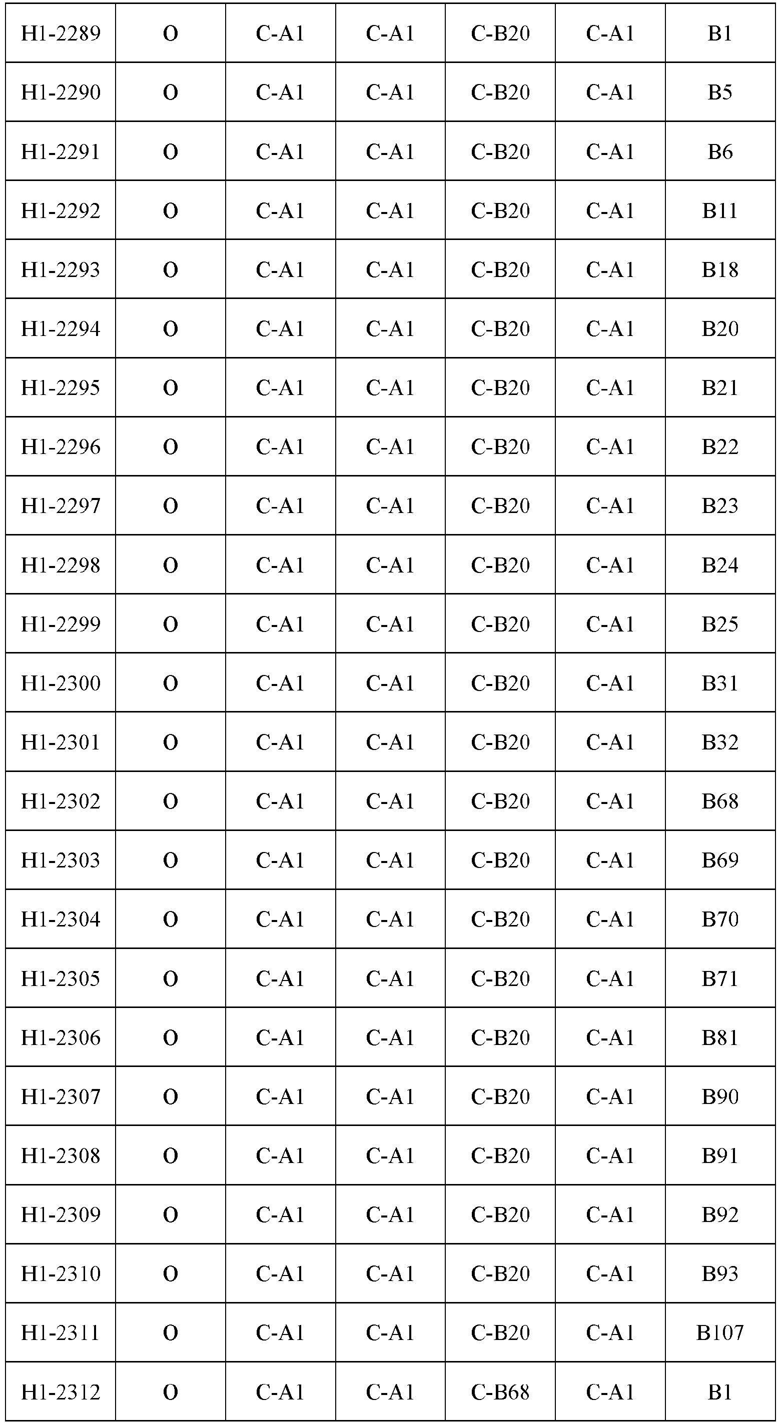 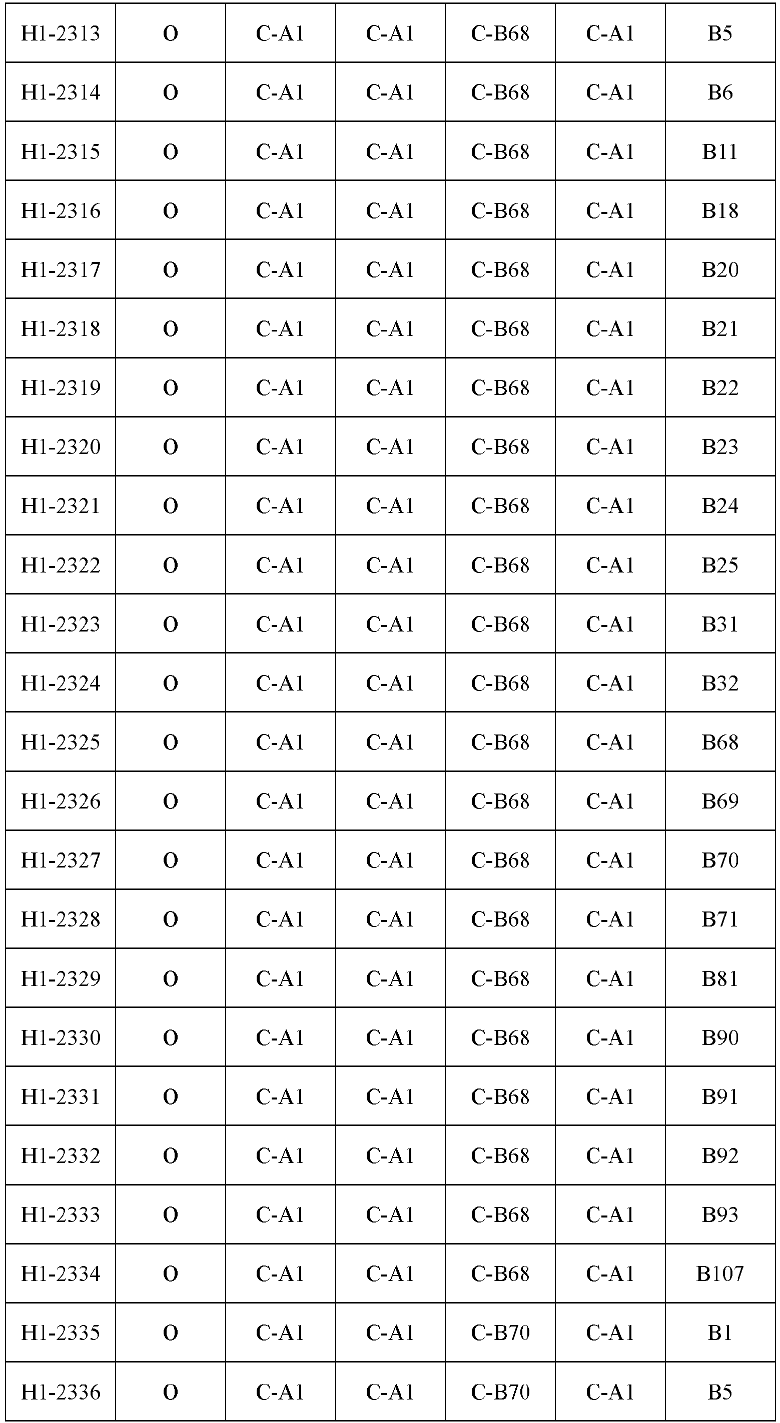 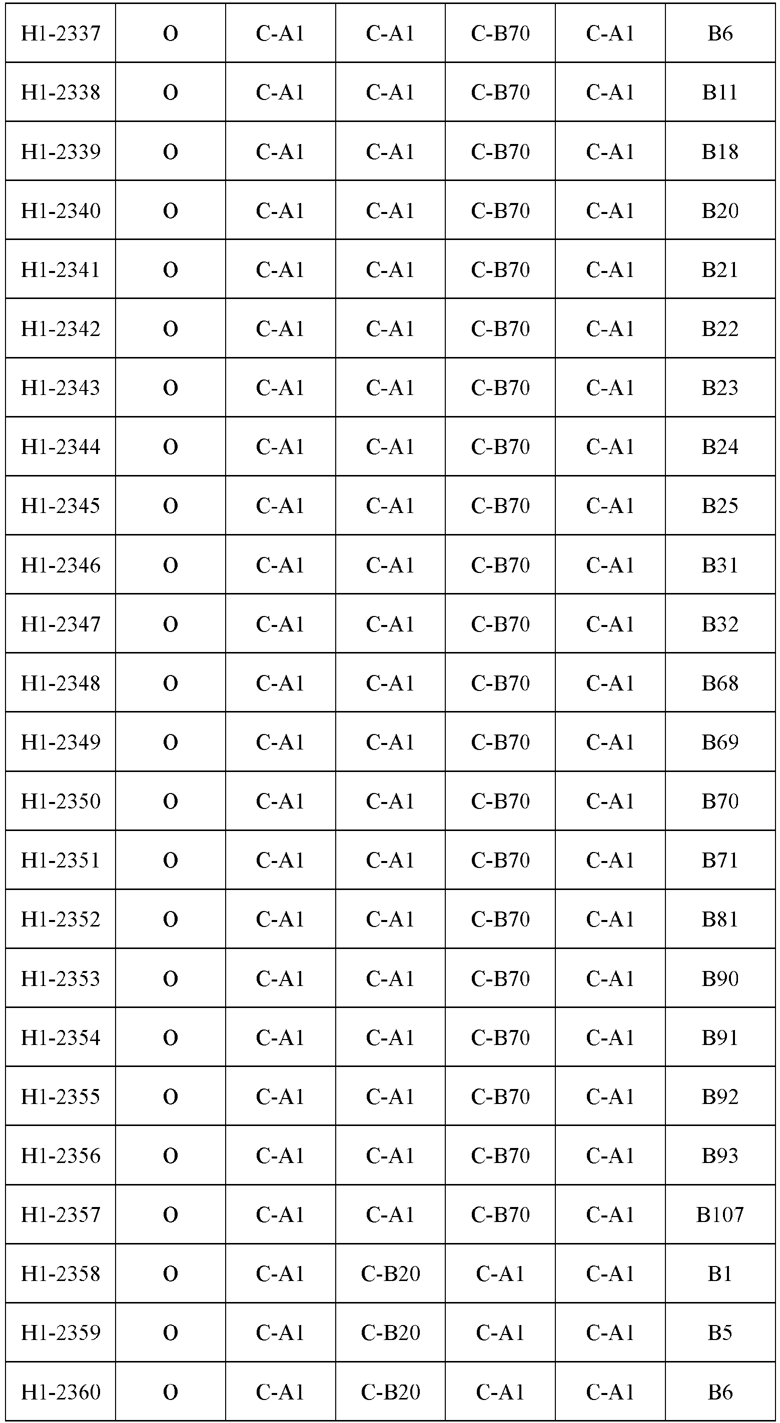 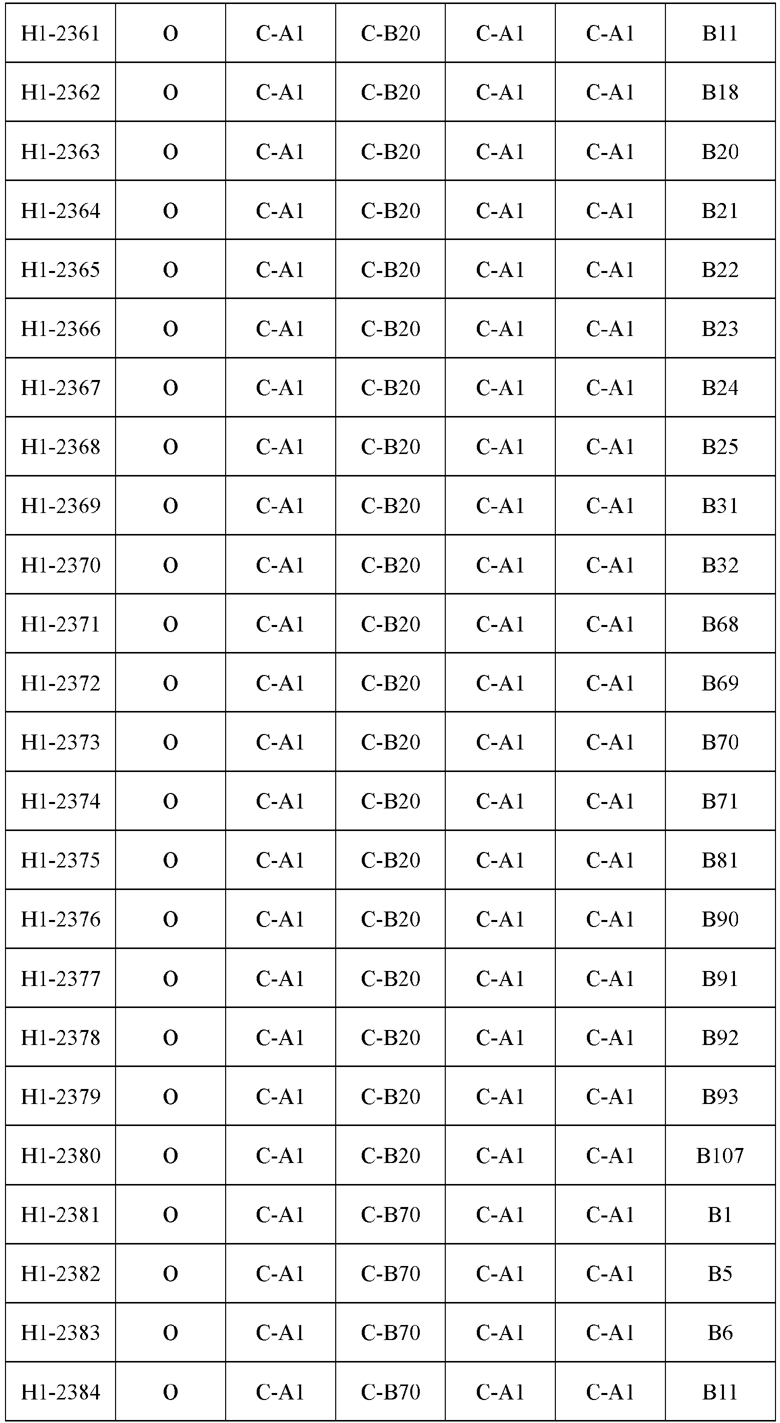 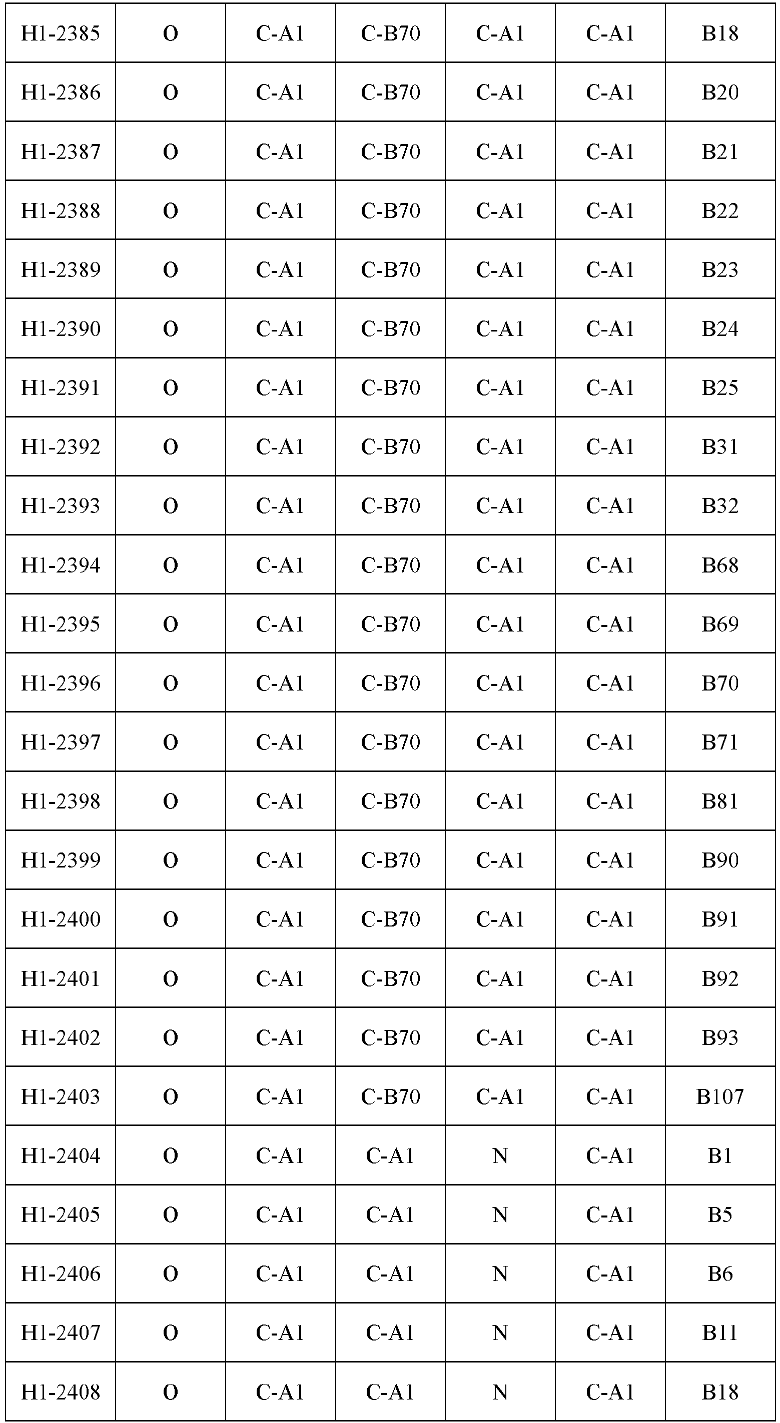 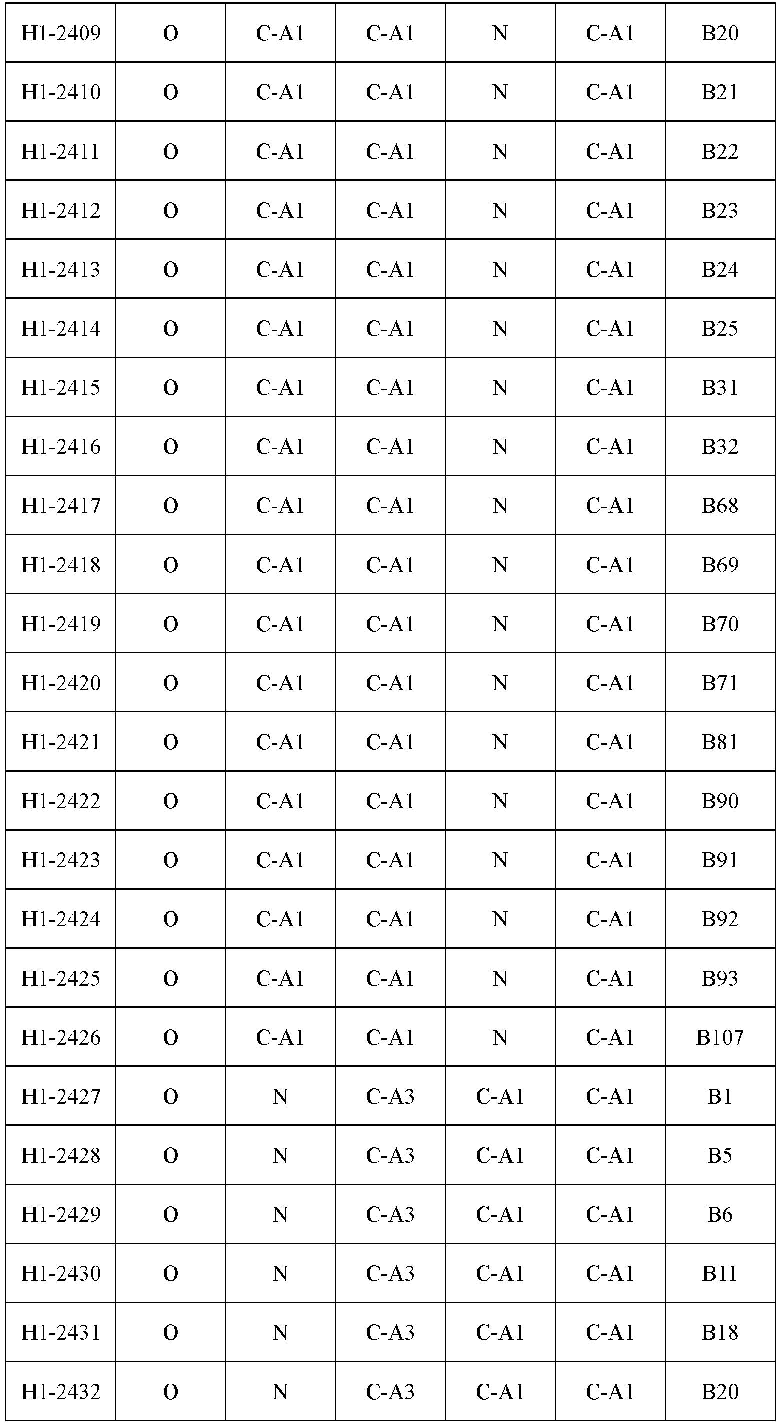 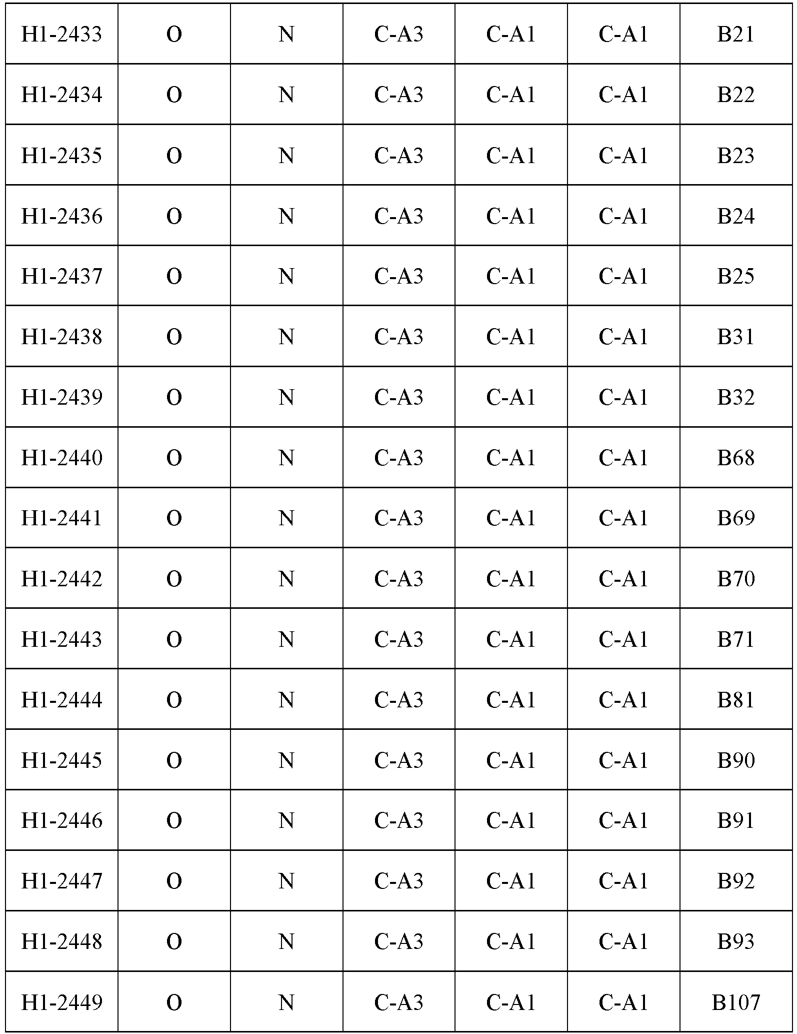
dans lequel les composés H2-1 à H2-138 ont chacun une structure représentée par la formule 2-2-1 : 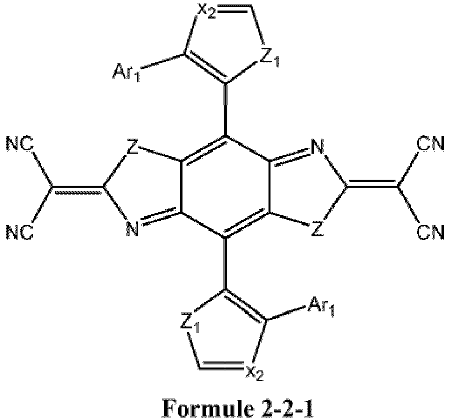
dans la formule 2-2-1, les deux Z sont identiques, les deux Z₁ sont identiques, les deux X₂ sont identiques, les deux Ar₁ sont identiques et Z, Z₁, X₂ et Ar₁ sont choisis parmi des atomes ou des groupes dans le tableau suivant, respectivement :
| Composé n° | Z | Z₁ | X₂ | Ar₁ |
|---|---|---|---|---|
| H2-1 | O | O | C-A1 | B1 |
| H2-2 | O | O | C-A1 | B5 |
| H2-3 | O | O | C-A1 | B6 |
| H2-4 | O | O | C-A1 | B11 |
| H2-5 | O | O | C-A1 | B18 |
| H2-6 | O | O | C-A1 | B20 |
| H2-7 | O | O | C-A1 | B21 |
| H2-8 | O | O | C-A1 | B22 |
| H2-9 | O | O | C-A1 | B23 |
| H2-10 | O | O | C-A1 | B24 |
| H2-11 | O | O | C-A1 | B25 |
| H2-12 | O | O | C-A1 | B31 |
| H2-13 | O | O | C-A1 | B32 |
| H2-14 | O | O | C-A1 | B68 |
| H2-15 | O | O | C-A1 | B69 |
| H2-16 | O | O | C-A1 | B70 |
| H2-17 | O | O | C-A1 | B71 |
| H2-18 | O | O | C-A1 | B81 |
| H2-19 | O | O | C-A1 | B90 |
| H2-20 | O | O | C-A1 | B91 |
| H2-21 | O | O | C-A1 | B92 |
| H2-22 | O | O | C-A1 | B93 |
| H2-23 | O | O | C-A1 | B107 |
| H2-24 | S | O | C-A1 | B1 |
| H2-25 | S | O | C-A1 | B5 |
| H2-26 | S | O | C-A1 | B6 |
| H2-27 | S | O | C-A1 | B11 |
| H2-28 | S | O | C-A1 | B18 |
| H2-29 | S | O | C-A1 | B20 |
| H2-30 | S | O | C-A1 | B21 |
| H2-31 | S | O | C-A1 | B22 |
| H2-32 | S | O | C-A1 | B23 |
| H2-33 | S | O | C-A1 | B24 |
| H2-34 | S | O | C-A1 | B25 |
| H2-35 | S | O | C-A1 | B31 |
| H2-36 | S | O | C-A1 | B32 |
| H2-37 | S | O | C-A1 | B68 |
| H2-38 | S | O | C-A1 | B69 |
| H2-39 | S | O | C-A1 | B70 |
| H2-40 | S | O | C-A1 | B71 |
| H2-41 | S | O | C-A1 | B81 |
| H2-42 | S | O | C-A1 | B90 |
| H2-43 | S | O | C-A1 | B91 |
| H2-44 | S | O | C-A1 | B92 |
| H2-45 | S | O | C-A1 | B93 |
| H2-46 | S | O | C-A1 | B107 |
| H2-47 | Se | O | C-A1 | B1 |
| H2-48 | Se | O | C-A1 | B5 |
| H2-49 | Se | O | C-A1 | B6 |
| H2-50 | Se | O | C-A1 | B11 |
| H2-51 | Se | O | C-A1 | B18 |
| H2-52 | Se | O | C-A1 | B20 |
| H2-53 | Se | O | C-A1 | B21 |
| H2-54 | Se | O | C-A1 | B22 |
| H2-55 | Se | O | C-A1 | B23 |
| H2-56 | Se | O | C-A1 | B24 |
| H2-57 | Se | O | C-A1 | B25 |
| H2-58 | Se | O | C-A1 | B31 |
| H2-59 | Se | O | C-A1 | B32 |
| H2-60 | Se | O | C-A1 | B68 |
| H2-61 | Se | O | C-A1 | B69 |
| H2-62 | Se | O | C-A1 | B70 |
| H2-63 | Se | O | C-A1 | B71 |
| H2-64 | Se | O | C-A1 | B81 |
| H2-65 | Se | O | C-A1 | B90 |
| H2-66 | Se | O | C-A1 | B91 |
| H2-67 | Se | O | C-A1 | B92 |
| H2-68 | Se | O | C-A1 | B93 |
| H2-69 | Se | O | C-A1 | B107 |
| H2-70 | O | S | C-A1 | B1 |
| H2-71 | O | S | C-A1 | B5 |
| H2-72 | O | S | C-A1 | B6 |
| H2-73 | O | S | C-A1 | B11 |
| H2-74 | O | S | C-A1 | B18 |
| H2-75 | O | S | C-A1 | B20 |
| H2-76 | O | S | C-A1 | B21 |
| H2-77 | O | S | C-A1 | B22 |
| H2-78 | O | S | C-A1 | B23 |
| H2-79 | O | S | C-A1 | B24 |
| H2-80 | O | S | C-A1 | B25 |
| H2-81 | O | S | C-A1 | B31 |
| H2-82 | O | S | C-A1 | B32 |
| H2-83 | O | S | C-A1 | B68 |
| H2-84 | O | S | C-A1 | B69 |
| H2-85 | O | S | C-A1 | B70 |
| H2-86 | O | S | C-A1 | B71 |
| H2-87 | O | S | C-A1 | B81 |
| H2-88 | O | S | C-A1 | B90 |
| H2-89 | O | S | C-A1 | B91 |
| H2-90 | O | S | C-A1 | B92 |
| H2-91 | O | S | C-A1 | B93 |
| H2-92 | O | S | C-A1 | B107 |
| H2-93 | O | O | N | B1 |
| H2-94 | O | O | N | B5 |
| H2-95 | O | O | N | B6 |
| H2-96 | O | O | N | B11 |
| H2-97 | O | O | N | B18 |
| H2-98 | O | O | N | B20 |
| H2-99 | O | O | N | B21 |
| H2-100 | O | O | N | B22 |
| H2-101 | O | O | N | B23 |
| H2-102 | O | O | N | B24 |
| H2-103 | O | O | N | B25 |
| H2-104 | O | O | N | B31 |
| H2-105 | O | O | N | B32 |
| H2-106 | O | O | N | B68 |
| H2-107 | O | O | N | B69 |
| H2-108 | O | O | N | B70 |
| H2-109 | O | O | N | B71 |
| H2-110 | O | O | N | B81 |
| H2-111 | O | O | N | B90 |
| H2-112 | O | O | N | B91 |
| H2-113 | O | O | N | B92 |
| H2-114 | O | O | N | B93 |
| H2-115 | O | O | N | B107 |
| H2-116 | O | S | N | B1 |
| H2-117 | O | S | N | B5 |
| H2-118 | O | S | N | B6 |
| H2-119 | O | S | N | B11 |
| H2-120 | O | S | N | B18 |
| H2-121 | O | S | N | B20 |
| H2-122 | O | S | N | B21 |
| H2-123 | O | S | N | B22 |
| H2-124 | O | S | N | B23 |
| H2-125 | O | S | N | B24 |
| H2-126 | O | S | N | B25 |
| H2-127 | O | S | N | B31 |
| H2-128 | O | S | N | B32 |
| H2-129 | O | S | N | B68 |
| H2-130 | O | S | N | B69 |
| H2-131 | O | S | N | B70 |
| H2-132 | O | S | N | B71 |
| H2-133 | O | S | N | B81 |
| H2-134 | O | S | N | B90 |
| H2-135 | O | S | N | B91 |
| H2-136 | O | S | N | B92 |
| H2-137 | O | S | N | B93 |
| H2-138 | O | S | N | B107 |
dans lequel les composés H3-1 à H3-92 ont chacun une structure représentée par la formule 2-3-1 : 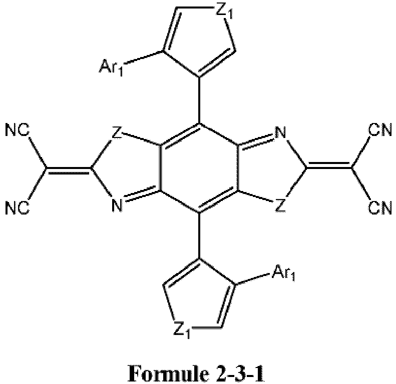
dans la formule 2-3-1, les deux Z sont identiques, les deux Z₁ sont identiques, les deux Ar₁ sont identiques et Z, Z₁ et Ar₁ sont choisis parmi des atomes ou des groupes dans le tableau suivant, respectivement :
| Composé n° | Z | Z₁ | Ar₁ |
|---|---|---|---|
| H3-1 | O | O | B1 |
| H3-2 | O | O | B5 |
| H3-3 | O | O | B6 |
| H3-4 | O | O | B11 |
| H3-5 | O | O | B18 |
| H3-6 | O | O | B20 |
| H3-7 | O | O | B21 |
| H3-8 | O | O | B22 |
| H3-9 | O | O | B23 |
| H3-10 | O | O | B24 |
| H3-11 | O | O | B25 |
| H3-12 | O | O | B31 |
| H3-13 | O | O | B32 |
| H3-14 | O | O | B68 |
| H3-15 | O | O | B69 |
| H3-16 | O | O | B70 |
| H3-17 | O | O | B71 |
| H3-18 | O | O | B81 |
| H3-19 | O | O | B90 |
| H3-20 | O | O | B91 |
| H3-21 | O | O | B92 |
| H3-22 | O | O | B93 |
| H3-23 | O | O | B107 |
| H3-24 | S | O | B1 |
| H3-25 | S | O | B5 |
| H3-26 | S | O | B6 |
| H3-27 | S | O | B11 |
| H3-28 | S | O | B18 |
| H3-29 | S | O | B20 |
| H3-30 | S | O | B21 |
| H3-31 | S | O | B22 |
| H3-32 | S | O | B23 |
| H3-33 | S | O | B24 |
| H3-34 | S | O | B25 |
| H3-35 | S | O | B31 |
| H3-36 | S | O | B32 |
| H3-37 | S | O | B68 |
| H3-38 | S | O | B69 |
| H3-39 | S | O | B70 |
| H3-40 | S | O | B71 |
| H3-41 | S | O | B81 |
| H3-42 | S | O | B90 |
| H3-43 | S | O | B91 |
| H3-44 | S | O | B92 |
| H3-45 | S | O | B93 |
| H3-46 | S | O | B107 |
| H3-47 | Se | O | B1 |
| H3-48 | Se | O | B5 |
| H3-49 | Se | O | B6 |
| H3-50 | Se | O | B11 |
| H3-51 | Se | O | B18 |
| H3-52 | Se | O | B20 |
| H3-53 | Se | O | B21 |
| H3-54 | Se | O | B22 |
| H3-55 | Se | O | B23 |
| H3-56 | Se | O | B24 |
| H3-57 | Se | O | B25 |
| H3-58 | Se | O | B31 |
| H3-59 | Se | O | B32 |
| H3-60 | Se | O | B68 |
| H3-61 | Se | O | B69 |
| H3-62 | Se | O | B70 |
| H3-63 | Se | O | B71 |
| H3-64 | Se | O | B81 |
| H3-65 | Se | O | B90 |
| H3-66 | Se | O | B91 |
| H3-67 | Se | O | B92 |
| H3-68 | Se | O | B93 |
| H3-69 | Se | O | B107 |
| H3-70 | O | S | B1 |
| H3-71 | O | S | B5 |
| H3-72 | O | S | B6 |
| H3-73 | O | S | B11 |
| H3-74 | O | S | B18 |
| H3-75 | O | S | B20 |
| H3-76 | O | S | B21 |
| H3-77 | O | S | B22 |
| H3-78 | O | S | B23 |
| H3-79 | O | S | B24 |
| H3-80 | O | S | B25 |
| H3-81 | O | S | B31 |
| H3-82 | O | S | B32 |
| H3-83 | O | S | B68 |
| H3-84 | O | S | B69 |
| H3-85 | O | S | B70 |
| H3-86 | O | S | B71 |
| H3-87 | O | S | B81 |
| H3-88 | O | S | B90 |
| H3-89 | O | S | B91 |
| H3-90 | O | S | B92 |
| H3-91 | O | S | B93 |
| H3-92 | O | S | B107 |
dans lequel les composés H4-1 à H4-92 ont chacun une structure représentée par la formule 2-4-1 : 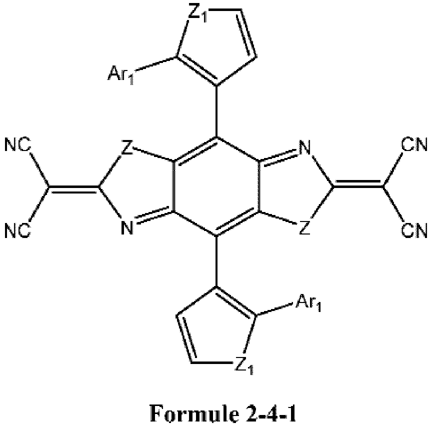
dans la formule 2-4-1, les deux Z sont identiques, les deux Z₁ sont identiques, les deux Ar₁ sont identiques et Z, Z₁ et Ar₁ sont choisis parmi des atomes ou des groupes dans le tableau suivant, respectivement :
| Composé n° | Z | Z₁ | Ar₁ |
|---|---|---|---|
| H4-1 | O | O | B1 |
| H4-2 | O | O | B5 |
| H4-3 | O | O | B6 |
| H4-4 | O | O | B11 |
| H4-5 | O | O | B18 |
| H4-6 | O | O | B20 |
| H4-7 | O | O | B21 |
| H4-8 | O | O | B22 |
| H4-9 | O | O | B23 |
| H4-10 | O | O | B24 |
| H4-11 | O | O | B25 |
| H4-12 | O | O | B31 |
| H4-13 | O | O | B32 |
| H4-14 | O | O | B68 |
| H4-15 | O | O | B69 |
| H4-16 | O | O | B70 |
| H4-17 | O | O | B71 |
| H4-18 | O | O | B81 |
| H4-19 | O | O | B90 |
| H4-20 | O | O | B91 |
| H4-21 | O | O | B92 |
| H4-22 | O | O | B93 |
| H4-23 | O | O | B107 |
| H4-24 | S | O | B1 |
| H4-25 | S | O | B5 |
| H4-26 | S | O | B6 |
| H4-27 | S | O | B11 |
| H4-28 | S | O | B18 |
| H4-29 | S | O | B20 |
| H4-30 | S | O | B21 |
| H4-31 | S | O | B22 |
| H4-32 | S | O | B23 |
| H4-33 | S | O | B24 |
| H4-34 | S | O | B25 |
| H4-35 | S | O | B31 |
| H4-36 | S | O | B32 |
| H4-37 | S | O | B68 |
| H4-38 | S | O | B69 |
| H4-39 | S | O | B70 |
| H4-40 | S | O | B71 |
| H4-41 | S | O | B81 |
| H4-42 | S | O | B90 |
| H4-43 | S | O | B91 |
| H4-44 | S | O | B92 |
| H4-45 | S | O | B93 |
| H4-46 | S | O | B107 |
| H4-47 | Se | O | B1 |
| H4-48 | Se | O | B5 |
| H4-49 | Se | O | B6 |
| H4-50 | Se | O | B11 |
| H4-51 | Se | O | B18 |
| H4-52 | Se | O | B20 |
| H4-53 | Se | O | B21 |
| H4-54 | Se | O | B22 |
| H4-55 | Se | O | B23 |
| H4-56 | Se | O | B24 |
| H4-57 | Se | O | B25 |
| H4-58 | Se | O | B31 |
| H4-59 | Se | O | B32 |
| H4-60 | Se | O | B68 |
| H4-61 | Se | O | B69 |
| H4-62 | Se | O | B70 |
| H4-63 | Se | O | B71 |
| H4-64 | Se | O | B81 |
| H4-65 | Se | O | B90 |
| H4-66 | Se | O | B91 |
| H4-67 | Se | O | B92 |
| H4-68 | Se | O | B93 |
| H4-69 | Se | O | B107 |
| H4-70 | O | S | B1 |
| H4-71 | O | S | B5 |
| H4-72 | O | S | B6 |
| H4-73 | O | S | B11 |
| H4-74 | O | S | B18 |
| H4-75 | O | S | B20 |
| H4-76 | O | S | B21 |
| H4-77 | O | S | B22 |
| H4-78 | O | S | B23 |
| H4-79 | O | S | B24 |
| H4-80 | O | S | B25 |
| H4-81 | O | S | B31 |
| H4-82 | O | S | B32 |
| H4-83 | O | S | B68 |
| H4-84 | O | S | B69 |
| H4-85 | O | S | B70 |
| H4-86 | O | S | B71 |
| H4-87 | O | S | B81 |
| H4-88 | O | S | B90 |
| H4-89 | O | S | B91 |
| H4-90 | O | S | B92 |
| H4-91 | O | S | B93 |
| H4-92 | O | S | B107 |
dans lequel les composés H5-1 à H5-115 ont chacun une structure représentée par la formule 2-5 : 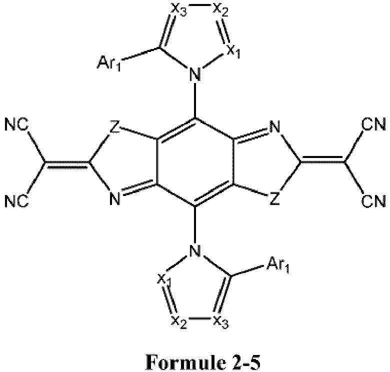
dans la formule 2-5, les deux Z sont identiques, les deux X₁ sont identiques, les deux X₂ sont identiques, les deux X₃ sont identiques, les deux Ar₁ sont identiques, et Z, X₁, X₂, X₃ et Ar₁ sont choisis parmi des atomes ou des groupes dans le tableau suivant, respectivement :
| Composé n° | Z | X₁ | X₂ | X₃ | Ar₁ |
|---|---|---|---|---|---|
| H5-1 | O | C-A1 | C-A1 | C-A1 | B1 |
| H5-2 | O | C-A1 | C-A1 | C-A1 | B5 |
| H5-3 | O | C-A1 | C-A1 | C-A1 | B6 |
| H5-4 | O | C-A1 | C-A1 | C-A1 | B11 |
| H5-5 | O | C-A1 | C-A1 | C-A1 | B18 |
| H5-6 | O | C-A1 | C-A1 | C-A1 | B20 |
| H5-7 | O | C-A1 | C-A1 | C-A1 | B21 |
| H5-8 | O | C-A1 | C-A1 | C-A1 | B22 |
| H5-9 | O | C-A1 | C-A1 | C-A1 | B23 |
| H5-10 | O | C-A1 | C-A1 | C-A1 | B24 |
| H5-11 | O | C-A1 | C-A1 | C-A1 | B25 |
| H5-12 | O | C-A1 | C-A1 | C-A1 | B31 |
| H5-13 | O | C-A1 | C-A1 | C-A1 | B32 |
| H5-14 | O | C-A1 | C-A1 | C-A1 | B68 |
| H5-15 | O | C-A1 | C-A1 | C-A1 | B69 |
| H5-16 | O | C-A1 | C-A1 | C-A1 | B70 |
| H5-17 | O | C-A1 | C-A1 | C-A1 | B71 |
| H5-18 | O | C-A1 | C-A1 | C-A1 | B81 |
| H5-19 | O | C-A1 | C-A1 | C-A1 | B90 |
| H5-20 | O | C-A1 | C-A1 | C-A1 | B91 |
| H5-21 | O | C-A1 | C-A1 | C-A1 | B92 |
| H5-22 | O | C-A1 | C-A1 | C-A1 | B93 |
| H5-23 | O | C-A1 | C-A1 | C-A1 | B107 |
| H5-24 | S | C-A1 | C-A1 | C-A1 | B1 |
| H5-25 | S | C-A1 | C-A1 | C-A1 | B5 |
| H5-26 | S | C-A1 | C-A1 | C-A1 | B6 |
| H5-27 | S | C-A1 | C-A1 | C-A1 | B11 |
| H5-28 | S | C-A1 | C-A1 | C-A1 | B18 |
| H5-29 | S | C-A1 | C-A1 | C-A1 | B20 |
| H5-30 | S | C-A1 | C-A1 | C-A1 | B21 |
| H5-31 | S | C-A1 | C-A1 | C-A1 | B22 |
| H5-32 | S | C-A1 | C-A1 | C-A1 | B23 |
| H5-33 | S | C-A1 | C-A1 | C-A1 | B24 |
| H5-34 | S | C-A1 | C-A1 | C-A1 | B25 |
| H5-35 | S | C-A1 | C-A1 | C-A1 | B31 |
| H5-36 | S | C-A1 | C-A1 | C-A1 | B32 |
| H5-37 | S | C-A1 | C-A1 | C-A1 | B68 |
| H5-38 | S | C-A1 | C-A1 | C-A1 | B69 |
| H5-39 | S | C-A1 | C-A1 | C-A1 | B70 |
| H5-40 | S | C-A1 | C-A1 | C-A1 | B71 |
| H5-41 | S | C-A1 | C-A1 | C-A1 | B81 |
| H5-42 | S | C-A1 | C-A1 | C-A1 | B90 |
| H5-43 | S | C-A1 | C-A1 | C-A1 | B91 |
| H5-44 | S | C-A1 | C-A1 | C-A1 | B92 |
| H5-45 | S | C-A1 | C-A1 | C-A1 | B93 |
| H5-46 | S | C-A1 | C-A1 | C-A1 | B107 |
| H5-47 | Se | C-A1 | C-A1 | C-A1 | B1 |
| H5-48 | Se | C-A1 | C-A1 | C-A1 | B5 |
| H5-49 | Se | C-A1 | C-A1 | C-A1 | B6 |
| H5-50 | Se | C-A1 | C-A1 | C-A1 | B11 |
| H5-51 | Se | C-A1 | C-A1 | C-A1 | B18 |
| H5-52 | Se | C-A1 | C-A1 | C-A1 | B20 |
| H5-53 | Se | C-A1 | C-A1 | C-A1 | B21 |
| H5-54 | Se | C-A1 | C-A1 | C-A1 | B22 |
| H5-55 | Se | C-A1 | C-A1 | C-A1 | B23 |
| H5-56 | Se | C-A1 | C-A1 | C-A1 | B24 |
| H5-57 | Se | C-A1 | C-A1 | C-A1 | B25 |
| H5-58 | Se | C-A1 | C-A1 | C-A1 | B31 |
| H5-59 | Se | C-A1 | C-A1 | C-A1 | B32 |
| H5-60 | Se | C-A1 | C-A1 | C-A1 | B68 |
| H5-61 | Se | C-A1 | C-A1 | C-A1 | B69 |
| H5-62 | Se | C-A1 | C-A1 | C-A1 | B70 |
| H5-63 | Se | C-A1 | C-A1 | C-A1 | B71 |
| H5-64 | Se | C-A1 | C-A1 | C-A1 | B81 |
| H5-65 | Se | C-A1 | C-A1 | C-A1 | B90 |
| H5-66 | Se | C-A1 | C-A1 | C-A1 | B91 |
| H5-67 | Se | C-A1 | C-A1 | C-A1 | B92 |
| H5-68 | Se | C-A1 | C-A1 | C-A1 | B93 |
| H5-69 | Se | C-A1 | C-A1 | C-A1 | B107 |
| H5-70 | O | C-A1 | C-A1 | N | B1 |
| H5-71 | O | C-A1 | C-A1 | N | B5 |
| H5-72 | O | C-A1 | C-A1 | N | B6 |
| H5-73 | O | C-A1 | C-A1 | N | B11 |
| H5-74 | O | C-A1 | C-A1 | N | B18 |
| H5-75 | O | C-A1 | C-A1 | N | B20 |
| H5-76 | O | C-A1 | C-A1 | N | B21 |
| H5-77 | O | C-A1 | C-A1 | N | B22 |
| H5-78 | O | C-A1 | C-A1 | N | B23 |
| H5-79 | O | C-A1 | C-A1 | N | B24 |
| H5-80 | O | C-A1 | C-A1 | N | B25 |
| H5-81 | O | C-A1 | C-A1 | N | B31 |
| H5-82 | O | C-A1 | C-A1 | N | B32 |
| H5-83 | O | C-A1 | C-A1 | N | B68 |
| H5-84 | O | C-A1 | C-A1 | N | B69 |
| H5-85 | O | C-A1 | C-A1 | N | B70 |
| H5-86 | O | C-A1 | C-A1 | N | B71 |
| H5-87 | O | C-A1 | C-A1 | N | B81 |
| H5-88 | O | C-A1 | C-A1 | N | B90 |
| H5-89 | O | C-A1 | C-A1 | N | B91 |
| H5-90 | O | C-A1 | C-A1 | N | B92 |
| H5-91 | O | C-A1 | C-A1 | N | B93 |
| H5-92 | O | C-A1 | C-A1 | N | B107 |
| H5-93 | O | C-A1 | N | C-A1 | B1 |
| H5-94 | O | C-A1 | N | C-A1 | B5 |
| H5-95 | O | C-A1 | N | C-A1 | B6 |
| H5-96 | O | C-A1 | N | C-A1 | B11 |
| H5-97 | O | C-A1 | N | C-A1 | B18 |
| H5-98 | O | C-A1 | N | C-A1 | B20 |
| H5-99 | O | C-A1 | N | C-A1 | B21 |
| H5-100 | O | C-A1 | N | C-A1 | B22 |
| H5-101 | O | C-A1 | N | C-A1 | B23 |
| H5-102 | O | C-A1 | N | C-A1 | B24 |
| H5-103 | O | C-A1 | N | C-A1 | B25 |
| H5-104 | O | C-A1 | N | C-A1 | B31 |
| H5-105 | O | C-A1 | N | C-A1 | B32 |
| H5-106 | O | C-A1 | N | C-A1 | B68 |
| H5-107 | O | C-A1 | N | C-A1 | B69 |
| H5-108 | O | C-A1 | N | C-A1 | B70 |
| H5-109 | O | C-A1 | N | C-A1 | B71 |
| H5-110 | O | C-A1 | N | C-A1 | B81 |
| H5-111 | O | C-A1 | N | C-A1 | B90 |
| H5-112 | O | C-A1 | N | C-A1 | B91 |
| H5-113 | O | C-A1 | N | C-A1 | B92 |
| H5-114 | O | C-A1 | N | C-A1 | B93 |
| H5-115 | O | C-A1 | N | C-A1 | B107 |
dans lequel les composés H6-1 à H6-115 ont chacun une structure représentée par la formule 2-6 : 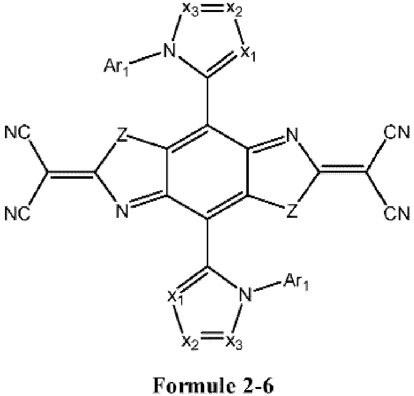
dans la formule 2-6, les deux Z sont identiques, les deux X₁ sont identiques, les deux X₂ sont identiques, les deux X₃ sont identiques, les deux Ar₁ sont identiques, et Z, X₁, X₂, X₃ et Ar₁ sont choisis parmi des atomes ou des groupes dans le tableau suivant, respectivement :
| Composé n° | Z | X₁ | X₂ | X₃ | Ar₁ |
|---|---|---|---|---|---|
| H6-1 | O | C-A1 | C-A1 | C-A1 | B1 |
| H6-2 | O | C-A1 | C-A1 | C-A1 | B5 |
| H6-3 | O | C-A1 | C-A1 | C-A1 | B6 |
| H6-4 | O | C-A1 | C-A1 | C-A1 | B11 |
| H6-5 | O | C-A1 | C-A1 | C-A1 | B18 |
| H6-6 | O | C-A1 | C-A1 | C-A1 | B20 |
| H6-7 | O | C-A1 | C-A1 | C-A1 | B21 |
| H6-8 | O | C-A1 | C-A1 | C-A1 | B22 |
| H6-9 | O | C-A1 | C-A1 | C-A1 | B23 |
| H6-10 | O | C-A1 | C-A1 | C-A1 | B24 |
| H6-11 | O | C-A1 | C-A1 | C-A1 | B25 |
| H6-12 | O | C-A1 | C-A1 | C-A1 | B31 |
| H6-13 | O | C-A1 | C-A1 | C-A1 | B32 |
| H6-14 | O | C-A1 | C-A1 | C-A1 | B68 |
| H6-15 | O | C-A1 | C-A1 | C-A1 | B69 |
| H6-16 | O | C-A1 | C-A1 | C-A1 | B70 |
| H6-17 | O | C-A1 | C-A1 | C-A1 | B71 |
| H6-18 | O | C-A1 | C-A1 | C-A1 | B81 |
| H6-19 | O | C-A1 | C-A1 | C-A1 | B90 |
| H6-20 | O | C-A1 | C-A1 | C-A1 | B91 |
| H6-21 | O | C-A1 | C-A1 | C-A1 | B92 |
| H6-22 | O | C-A1 | C-A1 | C-A1 | B93 |
| H6-23 | O | C-A1 | C-A1 | C-A1 | B107 |
| H6-24 | S | C-A1 | C-A1 | C-A1 | B1 |
| H6-25 | S | C-A1 | C-A1 | C-A1 | B5 |
| H6-26 | S | C-A1 | C-A1 | C-A1 | B6 |
| H6-27 | S | C-A1 | C-A1 | C-A1 | B11 |
| H6-28 | S | C-A1 | C-A1 | C-A1 | B18 |
| H6-29 | S | C-A1 | C-A1 | C-A1 | B20 |
| H6-30 | S | C-A1 | C-A1 | C-A1 | B21 |
| H6-31 | S | C-A1 | C-A1 | C-A1 | B22 |
| H6-32 | S | C-A1 | C-A1 | C-A1 | B23 |
| H6-33 | S | C-A1 | C-A1 | C-A1 | B24 |
| H6-34 | S | C-A1 | C-A1 | C-A1 | B25 |
| H6-35 | S | C-A1 | C-A1 | C-A1 | B31 |
| H6-36 | S | C-A1 | C-A1 | C-A1 | B32 |
| H6-37 | S | C-A1 | C-A1 | C-A1 | B68 |
| H6-38 | S | C-A1 | C-A1 | C-A1 | B69 |
| H6-39 | S | C-A1 | C-A1 | C-A1 | B70 |
| H6-40 | S | C-A1 | C-A1 | C-A1 | B71 |
| H6-41 | S | C-A1 | C-A1 | C-A1 | B81 |
| H6-42 | S | C-A1 | C-A1 | C-A1 | B90 |
| H6-43 | S | C-A1 | C-A1 | C-A1 | B91 |
| H6-44 | S | C-A1 | C-A1 | C-A1 | B92 |
| H6-45 | S | C-A1 | C-A1 | C-A1 | B93 |
| H6-46 | S | C-A1 | C-A1 | C-A1 | B107 |
| H6-47 | Se | C-A1 | C-A1 | C-A1 | B1 |
| H6-48 | Se | C-A1 | C-A1 | C-A1 | B5 |
| H6-49 | Se | C-A1 | C-A1 | C-A1 | B6 |
| H6-50 | Se | C-A1 | C-A1 | C-A1 | B11 |
| H6-51 | Se | C-A1 | C-A1 | C-A1 | B18 |
| H6-52 | Se | C-A1 | C-A1 | C-A1 | B20 |
| H6-53 | Se | C-A1 | C-A1 | C-A1 | B21 |
| H6-54 | Se | C-A1 | C-A1 | C-A1 | B22 |
| H6-55 | Se | C-A1 | C-A1 | C-A1 | B23 |
| H6-56 | Se | C-A1 | C-A1 | C-A1 | B24 |
| H6-57 | Se | C-A1 | C-A1 | C-A1 | B25 |
| H6-58 | Se | C-A1 | C-A1 | C-A1 | B31 |
| H6-59 | Se | C-A1 | C-A1 | C-A1 | B32 |
| H6-60 | Se | C-A1 | C-A1 | C-A1 | B68 |
| H6-61 | Se | C-A1 | C-A1 | C-A1 | B69 |
| H6-62 | Se | C-A1 | C-A1 | C-A1 | B70 |
| H6-63 | Se | C-A1 | C-A1 | C-A1 | B71 |
| H6-64 | Se | C-A1 | C-A1 | C-A1 | B81 |
| H6-65 | Se | C-A1 | C-A1 | C-A1 | B90 |
| H6-66 | Se | C-A1 | C-A1 | C-A1 | B91 |
| H6-67 | Se | C-A1 | C-A1 | C-A1 | B92 |
| H6-68 | Se | C-A1 | C-A1 | C-A1 | B93 |
| H6-69 | Se | C-A1 | C-A1 | C-A1 | B107 |
| H6-70 | O | N | C-A1 | C-A1 | B1 |
| H6-71 | O | N | C-A1 | C-A1 | B5 |
| H6-72 | O | N | C-A1 | C-A1 | B6 |
| H6-73 | O | N | C-A1 | C-A1 | B11 |
| H6-74 | O | N | C-A1 | C-A1 | B18 |
| H6-75 | O | N | C-A1 | C-A1 | B20 |
| H6-76 | O | N | C-A1 | C-A1 | B21 |
| H6-77 | O | N | C-A1 | C-A1 | B22 |
| H6-78 | O | N | C-A1 | C-A1 | B23 |
| H6-79 | O | N | C-A1 | C-A1 | B24 |
| H6-80 | O | N | C-A1 | C-A1 | B25 |
| H6-81 | O | N | C-A1 | C-A1 | B31 |
| H6-82 | O | N | C-A1 | C-A1 | B32 |
| H6-83 | O | N | C-A1 | C-A1 | B68 |
| H6-84 | O | N | C-A1 | C-A1 | B69 |
| H6-85 | O | N | C-A1 | C-A1 | B70 |
| H6-86 | O | N | C-A1 | C-A1 | B71 |
| H6-87 | O | N | C-A1 | C-A1 | B81 |
| H6-88 | O | N | C-A1 | C-A1 | B90 |
| H6-89 | O | N | C-A1 | C-A1 | B91 |
| H6-90 | O | N | C-A1 | C-A1 | B92 |
| H6-91 | O | N | C-A1 | C-A1 | B93 |
| H6-92 | O | N | C-A1 | C-A1 | B107 |
| H6-93 | O | C-A1 | N | C-A1 | B1 |
| H6-94 | O | C-A1 | N | C-A1 | B5 |
| H6-95 | O | C-A1 | N | C-A1 | B6 |
| H6-96 | O | C-A1 | N | C-A1 | B11 |
| H6-97 | O | C-A1 | N | C-A1 | B18 |
| H6-98 | O | C-A1 | N | C-A1 | B20 |
| H6-99 | O | C-A1 | N | C-A1 | B21 |
| H6-100 | O | C-A1 | N | C-A1 | B22 |
| H6-101 | O | C-A1 | N | C-A1 | B23 |
| H6-102 | O | C-A1 | N | C-A1 | B24 |
| H6-103 | O | C-A1 | N | C-A1 | B25 |
| H6-104 | O | C-A1 | N | C-A1 | B31 |
| H6-105 | O | C-A1 | N | C-A1 | B32 |
| H6-106 | O | C-A1 | N | C-A1 | B68 |
| H6-107 | O | C-A1 | N | C-A1 | B69 |
| H6-108 | O | C-A1 | N | C-A1 | B70 |
| H6-109 | O | C-A1 | N | C-A1 | B71 |
| H6-110 | O | C-A1 | N | C-A1 | B81 |
| H6-111 | O | C-A1 | N | C-A1 | B90 |
| H6-112 | O | C-A1 | N | C-A1 | B91 |
| H6-113 | O | C-A1 | N | C-A1 | B92 |
| H6-114 | O | C-A1 | N | C-A1 | B93 |
| H6-115 | O | C-A1 | N | C-A1 | B107 |
dans lequel les composés H7-1 à H7-203 ont chacun une structure représentée par la formule 3-1 : 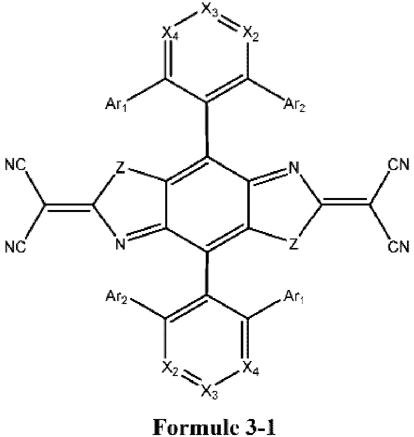
dans la formule 3-1, les deux Z sont identiques, les deux X₂ sont identiques, les deux X₃ sont identiques, les deux X₄ sont identiques, les deux Ar₁ sont identiques, les deux Ar₂ sont identiques, et Z, X₂, X₃, X₄, Ar₁ et Ar₂ sont choisis parmi des atomes ou des groupes dans le tableau suivant, respectivement :
| Composé n° | Z | X₂ | X₃ | X₄ | Ar₁ | Ar₂ |
|---|---|---|---|---|---|---|
| H7-1 | O | C-A1 | C-A1 | C-A1 | B1 | B1 |
| H7-2 | O | C-A1 | C-A1 | C-A1 | B5 | B5 |
| H7-3 | O | C-A1 | C-A1 | C-A1 | B6 | B6 |
| H7-4 | O | C-A1 | C-A1 | C-A1 | B11 | B11 |
| H7-5 | O | C-A1 | C-A1 | C-A1 | B18 | B18 |
| H7-6 | O | C-A1 | C-A1 | C-A1 | B20 | B20 |
| H7-7 | O | C-A1 | C-A1 | C-A1 | B21 | B21 |
| H7-8 | O | C-A1 | C-A1 | C-A1 | B22 | B22 |
| H7-9 | O | C-A1 | C-A1 | C-A1 | B23 | B23 |
| H7-10 | O | C-A1 | C-A1 | C-A1 | B24 | B24 |
| H7-11 | O | C-A1 | C-A1 | C-A1 | B25 | B25 |
| H7-12 | O | C-A1 | C-A1 | C-A1 | B31 | B31 |
| H7-13 | O | C-A1 | C-A1 | C-A1 | B32 | B32 |
| H7-14 | O | C-A1 | C-A1 | C-A1 | B68 | B68 |
| H7-15 | O | C-A1 | C-A1 | C-A1 | B69 | B69 |
| H7-16 | O | C-A1 | C-A1 | C-A1 | B70 | B70 |
| H7-17 | O | C-A1 | C-A1 | C-A1 | B71 | B71 |
| H7-18 | O | C-A1 | C-A1 | C-A1 | B81 | B81 |
| H7-19 | O | C-A1 | C-A1 | C-A1 | B90 | B90 |
| H7-20 | O | C-A1 | C-A1 | C-A1 | B91 | B91 |
| H7-21 | O | C-A1 | C-A1 | C-A1 | B92 | B92 |
| H7-22 | O | C-A1 | C-A1 | C-A1 | B93 | B93 |
| H7-23 | O | C-A1 | C-A1 | C-A1 | B107 | B107 |
| H7-24 | O | C-A1 | C-A3 | C-A1 | B1 | B1 |
| H7-25 | O | C-A1 | C-A3 | C-A1 | B5 | B5 |
| H7-26 | O | C-A1 | C-A3 | C-A1 | B6 | B6 |
| H7-27 | O | C-A1 | C-A3 | C-A1 | B11 | B11 |
| H7-28 | O | C-A1 | C-A3 | C-A1 | B18 | B18 |
| H7-29 | O | C-A1 | C-A3 | C-A1 | B20 | B20 |
| H7-30 | O | C-A1 | C-A3 | C-A1 | B21 | B21 |
| H7-31 | O | C-A1 | C-A3 | C-A1 | B22 | B22 |
| H7-32 | O | C-A1 | C-A3 | C-A1 | B23 | B23 |
| H7-33 | O | C-A1 | C-A3 | C-A1 | B24 | B24 |
| H7-34 | O | C-A1 | C-A3 | C-A1 | B25 | B25 |
| H7-35 | O | C-A1 | C-A3 | C-A1 | B31 | B31 |
| H7-36 | O | C-A1 | C-A3 | C-A1 | B32 | B32 |
| H7-37 | O | C-A1 | C-A3 | C-A1 | B68 | B68 |
| H7-38 | O | C-A1 | C-A3 | C-A1 | B69 | B69 |
| H7-39 | O | C-A1 | C-A3 | C-A1 | B70 | B70 |
| H7-40 | O | C-A1 | C-A3 | C-A1 | B71 | B71 |
| H7-41 | O | C-A1 | C-A3 | C-A1 | B81 | B81 |
| H7-42 | O | C-A1 | C-A3 | C-A1 | B90 | B90 |
| H7-43 | O | C-A1 | C-A3 | C-A1 | B91 | B91 |
| H7-44 | O | C-A1 | C-A3 | C-A1 | B92 | B92 |
| H7-45 | O | C-A1 | C-A3 | C-A1 | B93 | B93 |
| H7-46 | O | C-A1 | C-A3 | C-A1 | B107 | B107 |
| H7-47 | O | C-A1 | C-A9 | C-A1 | B1 | B1 |
| H7-48 | O | C-A1 | C-A9 | C-A1 | B5 | B5 |
| H7-49 | O | C-A1 | C-A9 | C-A1 | B6 | B6 |
| H7-50 | O | C-A1 | C-A9 | C-A1 | B11 | B11 |
| H7-51 | O | C-A1 | C-A9 | C-A1 | B18 | B18 |
| H7-52 | O | C-A1 | C-A9 | C-A1 | B20 | B20 |
| H7-53 | O | C-A1 | C-A9 | C-A1 | B21 | B21 |
| H7-54 | O | C-A1 | C-A9 | C-A1 | B22 | B22 |
| H7-55 | O | C-A1 | C-A9 | C-A1 | B23 | B23 |
| H7-56 | O | C-A1 | C-A9 | C-A1 | B24 | B24 |
| H7-57 | O | C-A1 | C-A9 | C-A1 | B25 | B25 |
| H7-58 | O | C-A1 | C-A9 | C-A1 | B31 | B31 |
| H7-59 | O | C-A1 | C-A9 | C-A1 | B32 | B32 |
| H7-60 | O | C-A1 | C-A9 | C-A1 | B68 | B68 |
| H7-61 | O | C-A1 | C-A9 | C-A1 | B69 | B69 |
| H7-62 | O | C-A1 | C-A9 | C-A1 | B70 | B70 |
| H7-63 | O | C-A1 | C-A9 | C-A1 | B71 | B71 |
| H7-64 | O | C-A1 | C-A9 | C-A1 | B81 | B81 |
| H7-65 | O | C-A1 | C-A9 | C-A1 | B90 | B90 |
| H7-66 | O | C-A1 | C-A9 | C-A1 | B91 | B91 |
| H7-67 | O | C-A1 | C-A9 | C-A1 | B92 | B92 |
| H7-68 | O | C-A1 | C-A9 | C-A1 | B93 | B93 |
| H7-69 | O | C-A1 | C-A9 | C-A1 | B107 | B107 |
| H7-70 | S | C-A1 | C-A1 | C-A1 | B1 | B1 |
| H7-71 | S | C-A1 | C-A1 | C-A1 | B5 | B5 |
| H7-72 | S | C-A1 | C-A1 | C-A1 | B6 | B6 |
| H7-73 | S | C-A1 | C-A1 | C-A1 | B11 | B11 |
| H7-74 | S | C-A1 | C-A1 | C-A1 | B18 | B18 |
| H7-75 | S | C-A1 | C-A1 | C-A1 | B20 | B20 |
| H7-76 | S | C-A1 | C-A1 | C-A1 | B21 | B21 |
| H7-77 | S | C-A1 | C-A1 | C-A1 | B22 | B22 |
| H7-78 | S | C-A1 | C-A1 | C-A1 | B23 | B23 |
| H7-79 | S | C-A1 | C-A1 | C-A1 | B24 | B24 |
| H7-80 | S | C-A1 | C-A1 | C-A1 | B25 | B25 |
| H7-81 | S | C-A1 | C-A1 | C-A1 | B31 | B31 |
| H7-82 | S | C-A1 | C-A1 | C-A1 | B32 | B32 |
| H7-83 | S | C-A1 | C-A1 | C-A1 | B68 | B68 |
| H7-84 | S | C-A1 | C-A1 | C-A1 | B69 | B69 |
| H7-85 | S | C-A1 | C-A1 | C-A1 | B70 | B70 |
| H7-86 | S | C-A1 | C-A1 | C-A1 | B71 | B71 |
| H7-87 | S | C-A1 | C-A1 | C-A1 | B81 | B81 |
| H7-88 | S | C-A1 | C-A1 | C-A1 | B90 | B90 |
| H7-89 | S | C-A1 | C-A1 | C-A1 | B91 | B91 |
| H7-90 | S | C-A1 | C-A1 | C-A1 | B92 | B92 |
| H7-91 | S | C-A1 | C-A1 | C-A1 | B93 | B93 |
| H7-92 | S | C-A1 | C-A1 | C-A1 | B107 | B107 |
| H7-93 | Se | C-A1 | C-A1 | C-A1 | B1 | B1 |
| H7-94 | Se | C-A1 | C-A1 | C-A1 | B5 | B5 |
| H7-95 | Se | C-A1 | C-A1 | C-A1 | B6 | B6 |
| H7-96 | Se | C-A1 | C-A1 | C-A1 | B11 | B11 |
| H7-97 | Se | C-A1 | C-A1 | C-A1 | B18 | B18 |
| H7-98 | Se | C-A1 | C-A1 | C-A1 | B20 | B20 |
| H7-99 | Se | C-A1 | C-A1 | C-A1 | B21 | B21 |
| H7-100 | Se | C-A1 | C-A1 | C-A1 | B22 | B22 |
| H7-101 | Se | C-A1 | C-A1 | C-A1 | B23 | B23 |
| H7-102 | Se | C-A1 | C-A1 | C-A1 | B24 | B24 |
| H7-103 | Se | C-A1 | C-A1 | C-A1 | B25 | B25 |
| H7-104 | Se | C-A1 | C-A1 | C-A1 | B31 | B31 |
| H7-105 | Se | C-A1 | C-A1 | C-A1 | B32 | B32 |
| H7-106 | Se | C-A1 | C-A1 | C-A1 | B68 | B68 |
| H7-107 | Se | C-A1 | C-A1 | C-A1 | B69 | B69 |
| H7-108 | Se | C-A1 | C-A1 | C-A1 | B70 | B70 |
| H7-109 | Se | C-A1 | C-A1 | C-A1 | B71 | B71 |
| H7-110 | Se | C-A1 | C-A1 | C-A1 | B81 | B81 |
| H7-111 | Se | C-A1 | C-A1 | C-A1 | B90 | B90 |
| H7-112 | Se | C-A1 | C-A1 | C-A1 | B91 | B91 |
| H7-113 | Se | C-A1 | C-A1 | C-A1 | B92 | B92 |
| H7-114 | Se | C-A1 | C-A1 | C-A1 | B93 | B93 |
| H7-115 | Se | C-A1 | C-A1 | C-A1 | B107 | B107 |
| H7-116 | O | C-A1 | C-A1 | C-A1 | B1 | B5 |
| H7-117 | O | C-A1 | C-A1 | C-A1 | B1 | B6 |
| H7-118 | O | C-A1 | C-A1 | C-A1 | B1 | B11 |
| H7-119 | O | C-A1 | C-A1 | C-A1 | B1 | B18 |
| H7-120 | O | C-A1 | C-A1 | C-A1 | B1 | B20 |
| H7-121 | O | C-A1 | C-A1 | C-A1 | B1 | B21 |
| H7-122 | O | C-A1 | C-A1 | C-A1 | B1 | B22 |
| H7-123 | O | C-A1 | C-A1 | C-A1 | B1 | B23 |
| H7-124 | O | C-A1 | C-A1 | C-A1 | B1 | B24 |
| H7-125 | O | C-A1 | C-A1 | C-A1 | B1 | B25 |
| H7-126 | O | C-A1 | C-A1 | C-A1 | B1 | B31 |
| H7-127 | O | C-A1 | C-A1 | C-A1 | B1 | B32 |
| H7-128 | O | C-A1 | C-A1 | C-A1 | B1 | B68 |
| H7-129 | O | C-A1 | C-A1 | C-A1 | B1 | B69 |
| H7-130 | O | C-A1 | C-A1 | C-A1 | B1 | B70 |
| H7-131 | O | C-A1 | C-A1 | C-A1 | B1 | B71 |
| H7-132 | O | C-A1 | C-A1 | C-A1 | B1 | B81 |
| H7-133 | O | C-A1 | C-A1 | C-A1 | B1 | B90 |
| H7-134 | O | C-A1 | C-A1 | C-A1 | B1 | B91 |
| H7-135 | O | C-A1 | C-A1 | C-A1 | B1 | B92 |
| H7-136 | O | C-A1 | C-A1 | C-A1 | B1 | B93 |
| H7-137 | O | C-A1 | C-A1 | C-A1 | B1 | B107 |
| H7-138 | O | C-A1 | C-A1 | C-A1 | B20 | B1 |
| H7-139 | O | C-A1 | C-A1 | C-A1 | B20 | B5 |
| H7-140 | O | C-A1 | C-A1 | C-A1 | B20 | B6 |
| H7-141 | O | C-A1 | C-A1 | C-A1 | B20 | B11 |
| H7-142 | O | C-A1 | C-A1 | C-A1 | B20 | B18 |
| H7-143 | O | C-A1 | C-A1 | C-A1 | B20 | B21 |
| H7-144 | O | C-A1 | C-A1 | C-A1 | B20 | B22 |
| H7-145 | O | C-A1 | C-A1 | C-A1 | B20 | B23 |
| H7-146 | O | C-A1 | C-A1 | C-A1 | B20 | B24 |
| H7-147 | O | C-A1 | C-A1 | C-A1 | B20 | B25 |
| H7-148 | O | C-A1 | C-A1 | C-A1 | B20 | B31 |
| H7-149 | O | C-A1 | C-A1 | C-A1 | B20 | B32 |
| H7-150 | O | C-A1 | C-A1 | C-A1 | B20 | B68 |
| H7-151 | O | C-A1 | C-A1 | C-A1 | B20 | B69 |
| H7-152 | O | C-A1 | C-A1 | C-A1 | B20 | B70 |
| H7-153 | O | C-A1 | C-A1 | C-A1 | B20 | B71 |
| H7-154 | O | C-A1 | C-A1 | C-A1 | B20 | B81 |
| H7-155 | O | C-A1 | C-A1 | C-A1 | B20 | B90 |
| H7-156 | O | C-A1 | C-A1 | C-A1 | B20 | B91 |
| H7-157 | O | C-A1 | C-A1 | C-A1 | B20 | B92 |
| H7-158 | O | C-A1 | C-A1 | C-A1 | B20 | B93 |
| H7-159 | O | C-A1 | C-A1 | C-A1 | B20 | B107 |
| H7-160 | O | C-A1 | C-A1 | C-A1 | B68 | B1 |
| H7-161 | O | C-A1 | C-A1 | C-A1 | B68 | B5 |
| H7-162 | O | C-A1 | C-A1 | C-A1 | B68 | B6 |
| H7-163 | O | C-A1 | C-A1 | C-A1 | B68 | B11 |
| H7-164 | O | C-A1 | C-A1 | C-A1 | B68 | B18 |
| H7-165 | O | C-A1 | C-A1 | C-A1 | B68 | B20 |
| H7-166 | O | C-A1 | C-A1 | C-A1 | B68 | B21 |
| H7-167 | O | C-A1 | C-A1 | C-A1 | B68 | B22 |
| H7-168 | O | C-A1 | C-A1 | C-A1 | B68 | B23 |
| H7-169 | O | C-A1 | C-A1 | C-A1 | B68 | B24 |
| H7-170 | O | C-A1 | C-A1 | C-A1 | B68 | B25 |
| H7-171 | O | C-A1 | C-A1 | C-A1 | B68 | B31 |
| H7-172 | O | C-A1 | C-A1 | C-A1 | B68 | B32 |
| H7-173 | O | C-A1 | C-A1 | C-A1 | B68 | B69 |
| H7-174 | O | C-A1 | C-A1 | C-A1 | B68 | B70 |
| H7-175 | O | C-A1 | C-A1 | C-A1 | B68 | B71 |
| H7-176 | O | C-A1 | C-A1 | C-A1 | B68 | B81 |
| H7-177 | O | C-A1 | C-A1 | C-A1 | B68 | B90 |
| H7-178 | O | C-A1 | C-A1 | C-A1 | B68 | B91 |
| H7-179 | O | C-A1 | C-A1 | C-A1 | B68 | B92 |
| H7-180 | O | C-A1 | C-A1 | C-A1 | B68 | B93 |
| H7-181 | O | C-A1 | C-A1 | C-A1 | B68 | B107 |
| H7-182 | O | C-A1 | C-A1 | C-A1 | B70 | B1 |
| H7-183 | O | C-A1 | C-A1 | C-A1 | B70 | B5 |
| H7-184 | O | C-A1 | C-A1 | C-A1 | B70 | B6 |
| H7-185 | O | C-A1 | C-A1 | C-A1 | B70 | B11 |
| H7-186 | O | C-A1 | C-A1 | C-A1 | B70 | B18 |
| H7-187 | O | C-A1 | C-A1 | C-A1 | B70 | B20 |
| H7-188 | O | C-A1 | C-A1 | C-A1 | B70 | B21 |
| H7-189 | O | C-A1 | C-A1 | C-A1 | B70 | B22 |
| H7-190 | O | C-A1 | C-A1 | C-A1 | B70 | B23 |
| H7-191 | O | C-A1 | C-A1 | C-A1 | B70 | B24 |
| H7-192 | O | C-A1 | C-A1 | C-A1 | B70 | B25 |
| H7-193 | O | C-A1 | C-A1 | C-A1 | B70 | B31 |
| H7-194 | O | C-A1 | C-A1 | C-A1 | B70 | B32 |
| H7-195 | O | C-A1 | C-A1 | C-A1 | B70 | B68 |
| H7-196 | O | C-A1 | C-A1 | C-A1 | B70 | B69 |
| H7-197 | O | C-A1 | C-A1 | C-A1 | B70 | B71 |
| H7-198 | O | C-A1 | C-A1 | C-A1 | B70 | B81 |
| H7-199 | O | C-A1 | C-A1 | C-A1 | B70 | B90 |
| H7-200 | O | C-A1 | C-A1 | C-A1 | B70 | B91 |
| H7-201 | O | C-A1 | C-A1 | C-A1 | B70 | B92 |
| H7-202 | O | C-A1 | C-A1 | C-A1 | B70 | B93 |
| H7-203 | O | C-A1 | C-A1 | C-A1 | B70 | B107 |
dans lequel les composés H8-7 à H8-54, les composés H8-61 à H8-108, les composés H8-115 à H8-162, les composés H8-169 à H8-216, les composés H8-223 à H8-270, les composés H8-277 à H8-324, les composés H8-331 à H8-378, les composés H8-385 à H8-432, les composés H8-439 à H8-486, les composés H8-493 à H8-540, les composés H8-547 à H8-594, les composés H8-601 à H8-648, les composés H8-655 à H8-702, les composés H8-709 à H8-756, les composés H8-763 à H8-810, les composés H8-817 à H8-864, les composés H8-871 à H8-918, les composés H8-925 à H8-972, les composés H8-979 à H8-1026, les composés H8-1033 à H8-1080, les composés H8-1087 à H8-1134, les composés H8-1141 à H8-1188, les composés H8-1195 à H8-1242, les composés H8-1249 à H8-1296, les composés H8-1303 à H8-1350, les composés H8-1357 à H8-1404, les composés H8-1411 à H8-1458, les composés H8-1465 à H8-1512 ont chacun une structure représentée par la formule 3-2 : 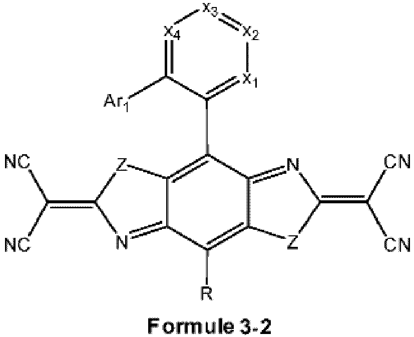
dans la formule 3-2, les deux Z sont identiques et Z, X₁, X₂, X₃, X₄, Ar₁ et R sont choisis parmi des atomes ou des groupes dans le tableau suivant, respectivement :
| Composé n° | Z | X₁ | X₂ | X₃ | X₄ | Ar₁ | R |
|---|---|---|---|---|---|---|---|
| H8-7 | O | C-A1 | C-A1 | C-A1 | C-A1 | B20 | B1 |
| H8-8 | O | C-A1 | C-A1 | C-A1 | C-A1 | B20 | B6 |
| H8-9 | O | C-A1 | C-A1 | C-A1 | C-A1 | B20 | B20 |
| H8-10 | O | C-A1 | C-A1 | C-A1 | C-A1 | B20 | B21 |
| H8-11 | O | C-A1 | C-A1 | C-A1 | C-A1 | B20 | B22 |
| H8-12 | O | C-A1 | C-A1 | C-A1 | C-A1 | B20 | B32 |
| H8-13 | O | C-A1 | C-A1 | C-A1 | C-A1 | B20 | B33 |
| H8-14 | O | C-A1 | C-A1 | C-A1 | C-A1 | B20 | B34 |
| H8-15 | O | C-A1 | C-A1 | C-A1 | C-A1 | B20 | B68 |
| H8-16 | O | C-A1 | C-A1 | C-A1 | C-A1 | B20 | B69 |
| H8-17 | O | C-A1 | C-A1 | C-A1 | C-A1 | B20 | B70 |
| H8-18 | O | C-A1 | C-A1 | C-A1 | C-A1 | B20 | B71 |
| H8-19 | O | C-A1 | C-A1 | C-A1 | C-A1 | B20 | B81 |
| H8-20 | O | C-A1 | C-A1 | C-A1 | C-A1 | B20 | B90 |
| H8-21 | O | C-A1 | C-A1 | C-A1 | C-A1 | B20 | B91 |
| H8-22 | O | C-A1 | C-A1 | C-A1 | C-A1 | B20 | B92 |
| H8-23 | O | C-A1 | C-A1 | C-A1 | C-A1 | B20 | B93 |
| H8-24 | O | C-A1 | C-A1 | C-A1 | C-A1 | B20 | B94 |
| H8-25 | O | C-A1 | C-A1 | C-A1 | C-A1 | B20 | B95 |
| H8-26 | O | C-A1 | C-A1 | C-A1 | C-A1 | B20 | B96 |
| H8-27 | O | C-A1 | C-A1 | C-A1 | C-A1 | B20 | B97 |
| H8-28 | O | C-A1 | C-A1 | C-A1 | C-A1 | B20 | B106 |
| H8-29 | O | C-A1 | C-A1 | C-A1 | C-A1 | B20 | B107 |
| H8-30 | O | C-A1 | C-A1 | C-A1 | C-A1 | B20 | B114 |
| H8-31 | O | C-A1 | C-A1 | C-A1 | C-A1 | B20 | B115 |
| H8-32 | O | C-A1 | C-A1 | C-A1 | C-A1 | B20 | B116 |
| H8-33 | O | C-A1 | C-A1 | C-A1 | C-A1 | B20 | B117 |
| H8-34 | O | C-A1 | C-A1 | C-A1 | C-A1 | B20 | B118 |
| H8-35 | O | C-A1 | C-A1 | C-A1 | C-A1 | B20 | B119 |
| H8-36 | O | C-A1 | C-A1 | C-A1 | C-A1 | B20 | B120 |
| H8-37 | O | C-A1 | C-A1 | C-A1 | C-A1 | B20 | B121 |
| H8-38 | O | C-A1 | C-A1 | C-A1 | C-A1 | B20 | B122 |
| H8-39 | O | C-A1 | C-A1 | C-A1 | C-A1 | B20 | B125 |
| H8-40 | O | C-A1 | C-A1 | C-A1 | C-A1 | B20 | B126 |
| H8-41 | O | C-A1 | C-A1 | C-A1 | C-A1 | B20 | B127 |
| H8-42 | O | C-A1 | C-A1 | C-A1 | C-A1 | B20 | B128 |
| H8-43 | O | C-A1 | C-A1 | C-A1 | C-A1 | B20 | B129 |
| H8-44 | O | C-A1 | C-A1 | C-A1 | C-A1 | B20 | B130 |
| H8-45 | O | C-A1 | C-A1 | C-A1 | C-A1 | B20 | B131 |
| H8-46 | O | C-A1 | C-A1 | C-A1 | C-A1 | B20 | B132 |
| H8-47 | O | C-A1 | C-A1 | C-A1 | C-A1 | B20 | B133 |
| H8-48 | O | C-A1 | C-A1 | C-A1 | C-A1 | B20 | B134 |
| H8-49 | O | C-A1 | C-A1 | C-A1 | C-A1 | B20 | B135 |
| H8-50 | O | C-A1 | C-A1 | C-A1 | C-A1 | B20 | B136 |
| H8-51 | O | C-A1 | C-A1 | C-A1 | C-A1 | B20 | B137 |
| H8-52 | O | C-A1 | C-A1 | C-A1 | C-A1 | B20 | B138 |
| H8-53 | O | C-A1 | C-A1 | C-A1 | C-A1 | B20 | B139 |
| H8-54 | O | C-A1 | C-A1 | C-A1 | C-A1 | B20 | B140 |
| H8-61 | O | C-A1 | C-A2 | C-A1 | C-A2 | B20 | B1 |
| H8-62 | O | C-A1 | C-A2 | C-A1 | C-A2 | B20 | B6 |
| H8-63 | O | C-A1 | C-A2 | C-A1 | C-A2 | B20 | B20 |
| H8-64 | O | C-A1 | C-A2 | C-A1 | C-A2 | B20 | B21 |
| H8-65 | O | C-A1 | C-A2 | C-A1 | C-A2 | B20 | B22 |
| H8-66 | O | C-A1 | C-A2 | C-A1 | C-A2 | B20 | B32 |
| H8-67 | O | C-A1 | C-A2 | C-A1 | C-A2 | B20 | B33 |
| H8-68 | O | C-A1 | C-A2 | C-A1 | C-A2 | B20 | B34 |
| H8-69 | O | C-A1 | C-A2 | C-A1 | C-A2 | B20 | B68 |
| H8-70 | O | C-A1 | C-A2 | C-A1 | C-A2 | B20 | B69 |
| H8-71 | O | C-A1 | C-A2 | C-A1 | C-A2 | B20 | B70 |
| H8-72 | O | C-A1 | C-A2 | C-A1 | C-A2 | B20 | B71 |
| H8-73 | O | C-A1 | C-A2 | C-A1 | C-A2 | B20 | B81 |
| H8-74 | O | C-A1 | C-A2 | C-A1 | C-A2 | B20 | B90 |
| H8-75 | O | C-A1 | C-A2 | C-A1 | C-A2 | B20 | B91 |
| H8-76 | O | C-A1 | C-A2 | C-A1 | C-A2 | B20 | B92 |
| H8-77 | O | C-A1 | C-A2 | C-A1 | C-A2 | B20 | B93 |
| H8-78 | O | C-A1 | C-A2 | C-A1 | C-A2 | B20 | B94 |
| H8-79 | O | C-A1 | C-A2 | C-A1 | C-A2 | B20 | B95 |
| H8-80 | O | C-A1 | C-A2 | C-A1 | C-A2 | B20 | B96 |
| H8-81 | O | C-A1 | C-A2 | C-A1 | C-A2 | B20 | B97 |
| H8-82 | O | C-A1 | C-A2 | C-A1 | C-A2 | B20 | B106 |
| H8-83 | O | C-A1 | C-A2 | C-A1 | C-A2 | B20 | B107 |
| H8-84 | O | C-A1 | C-A2 | C-A1 | C-A2 | B20 | B114 |
| H8-85 | O | C-A1 | C-A2 | C-A1 | C-A2 | B20 | B115 |
| H8-86 | O | C-A1 | C-A2 | C-A1 | C-A2 | B20 | B116 |
| H8-87 | O | C-A1 | C-A2 | C-A1 | C-A2 | B20 | B117 |
| H8-88 | O | C-A1 | C-A2 | C-A1 | C-A2 | B20 | B118 |
| H8-89 | O | C-A1 | C-A2 | C-A1 | C-A2 | B20 | B119 |
| H8-90 | O | C-A1 | C-A2 | C-A1 | C-A2 | B20 | B120 |
| H8-91 | O | C-A1 | C-A2 | C-A1 | C-A2 | B20 | B121 |
| H8-92 | O | C-A1 | C-A2 | C-A1 | C-A2 | B20 | B122 |
| H8-93 | O | C-A1 | C-A2 | C-A1 | C-A2 | B20 | B125 |
| H8-94 | O | C-A1 | C-A2 | C-A1 | C-A2 | B20 | B126 |
| H8-95 | O | C-A1 | C-A2 | C-A1 | C-A2 | B20 | B127 |
| H8-96 | O | C-A1 | C-A2 | C-A1 | C-A2 | B20 | B128 |
| H8-97 | O | C-A1 | C-A2 | C-A1 | C-A2 | B20 | B129 |
| H8-98 | O | C-A1 | C-A2 | C-A1 | C-A2 | B20 | B130 |
| H8-99 | O | C-A1 | C-A2 | C-A1 | C-A2 | B20 | B131 |
| H8-100 | O | C-A1 | C-A2 | C-A1 | C-A2 | B20 | B132 |
| H8-101 | O | C-A1 | C-A2 | C-A1 | C-A2 | B20 | B133 |
| H8-102 | O | C-A1 | C-A2 | C-A1 | C-A2 | B20 | B134 |
| H8-103 | O | C-A1 | C-A2 | C-A1 | C-A2 | B20 | B135 |
| H8-104 | O | C-A1 | C-A2 | C-A1 | C-A2 | B20 | B136 |
| H8-105 | O | C-A1 | C-A2 | C-A1 | C-A2 | B20 | B137 |
| H8-106 | O | C-A1 | C-A2 | C-A1 | C-A2 | B20 | B138 |
| H8-107 | O | C-A1 | C-A2 | C-A1 | C-A2 | B20 | B139 |
| H8-108 | O | C-A1 | C-A2 | C-A1 | C-A2 | B20 | B140 |
| H8-115 | O | C-A1 | C-A3 | C-A1 | C-A3 | B20 | B1 |
| H8-116 | O | C-A1 | C-A3 | C-A1 | C-A3 | B20 | B6 |
| H8-117 | O | C-A1 | C-A3 | C-A1 | C-A3 | B20 | B20 |
| H8-118 | O | C-A1 | C-A3 | C-A1 | C-A3 | B20 | B21 |
| H8-119 | O | C-A1 | C-A3 | C-A1 | C-A3 | B20 | B22 |
| H8-120 | O | C-A1 | C-A3 | C-A1 | C-A3 | B20 | B32 |
| H8-121 | O | C-A1 | C-A3 | C-A1 | C-A3 | B20 | B33 |
| H8-122 | O | C-A1 | C-A3 | C-A1 | C-A3 | B20 | B34 |
| H8-123 | O | C-A1 | C-A3 | C-A1 | C-A3 | B20 | B68 |
| H8-124 | O | C-A1 | C-A3 | C-A1 | C-A3 | B20 | B69 |
| H8-125 | O | C-A1 | C-A3 | C-A1 | C-A3 | B20 | B70 |
| H8-126 | O | C-A1 | C-A3 | C-A1 | C-A3 | B20 | B71 |
| H8-127 | O | C-A1 | C-A3 | C-A1 | C-A3 | B20 | B81 |
| H8-128 | O | C-A1 | C-A3 | C-A1 | C-A3 | B20 | B90 |
| H8-129 | O | C-A1 | C-A3 | C-A1 | C-A3 | B20 | B91 |
| H8-130 | O | C-A1 | C-A3 | C-A1 | C-A3 | B20 | B92 |
| H8-131 | O | C-A1 | C-A3 | C-A1 | C-A3 | B20 | B93 |
| H8-132 | O | C-A1 | C-A3 | C-A1 | C-A3 | B20 | B94 |
| H8-133 | O | C-A1 | C-A3 | C-A1 | C-A3 | B20 | B95 |
| H8-134 | O | C-A1 | C-A3 | C-A1 | C-A3 | B20 | B96 |
| H8-135 | O | C-A1 | C-A3 | C-A1 | C-A3 | B20 | B97 |
| H8-136 | O | C-A1 | C-A3 | C-A1 | C-A3 | B20 | B106 |
| H8-137 | O | C-A1 | C-A3 | C-A1 | C-A3 | B20 | B107 |
| H8-138 | O | C-A1 | C-A3 | C-A1 | C-A3 | B20 | B114 |
| H8-139 | O | C-A1 | C-A3 | C-A1 | C-A3 | B20 | B115 |
| H8-140 | O | C-A1 | C-A3 | C-A1 | C-A3 | B20 | B116 |
| H8-141 | O | C-A1 | C-A3 | C-A1 | C-A3 | B20 | B117 |
| H8-142 | O | C-A1 | C-A3 | C-A1 | C-A3 | B20 | B118 |
| H8-143 | O | C-A1 | C-A3 | C-A1 | C-A3 | B20 | B119 |
| H8-144 | O | C-A1 | C-A3 | C-A1 | C-A3 | B20 | B120 |
| H8-145 | O | C-A1 | C-A3 | C-A1 | C-A3 | B20 | B121 |
| H8-146 | O | C-A1 | C-A3 | C-A1 | C-A3 | B20 | B122 |
| H8-147 | O | C-A1 | C-A3 | C-A1 | C-A3 | B20 | B125 |
| H8-148 | O | C-A1 | C-A3 | C-A1 | C-A3 | B20 | B126 |
| H8-149 | O | C-A1 | C-A3 | C-A1 | C-A3 | B20 | B127 |
| H8-150 | O | C-A1 | C-A3 | C-A1 | C-A3 | B20 | B128 |
| H8-151 | O | C-A1 | C-A3 | C-A1 | C-A3 | B20 | B129 |
| H8-152 | O | C-A1 | C-A3 | C-A1 | C-A3 | B20 | B130 |
| H8-153 | O | C-A1 | C-A3 | C-A1 | C-A3 | B20 | B131 |
| H8-154 | O | C-A1 | C-A3 | C-A1 | C-A3 | B20 | B132 |
| H8-155 | O | C-A1 | C-A3 | C-A1 | C-A3 | B20 | B133 |
| H8-156 | O | C-A1 | C-A3 | C-A1 | C-A3 | B20 | B134 |
| H8-157 | O | C-A1 | C-A3 | C-A1 | C-A3 | B20 | B135 |
| H8-158 | O | C-A1 | C-A3 | C-A1 | C-A3 | B20 | B136 |
| H8-159 | O | C-A1 | C-A3 | C-A1 | C-A3 | B20 | B137 |
| H8-160 | O | C-A1 | C-A3 | C-A1 | C-A3 | B20 | B138 |
| H8-161 | O | C-A1 | C-A3 | C-A1 | C-A3 | B20 | B139 |
| H8-162 | O | C-A1 | C-A3 | C-A1 | C-A3 | B20 | B140 |
| H8-169 | O | C-A3 | C-A1 | C-A3 | C-A1 | B20 | B1 |
| H8-170 | O | C-A3 | C-A1 | C-A3 | C-A1 | B20 | B6 |
| H8-171 | O | C-A3 | C-A1 | C-A3 | C-A1 | B20 | B20 |
| H8-172 | O | C-A3 | C-A1 | C-A3 | C-A1 | B20 | B21 |
| H8-173 | O | C-A3 | C-A1 | C-A3 | C-A1 | B20 | B22 |
| H8-174 | O | C-A3 | C-A1 | C-A3 | C-A1 | B20 | B32 |
| H8-175 | O | C-A3 | C-A1 | C-A3 | C-A1 | B20 | B33 |
| H8-176 | O | C-A3 | C-A1 | C-A3 | C-A1 | B20 | B34 |
| H8-177 | O | C-A3 | C-A1 | C-A3 | C-A1 | B20 | B68 |
| H8-178 | O | C-A3 | C-A1 | C-A3 | C-A1 | B20 | B69 |
| H8-179 | O | C-A3 | C-A1 | C-A3 | C-A1 | B20 | B70 |
| H8-180 | O | C-A3 | C-A1 | C-A3 | C-A1 | B20 | B71 |
| H8-181 | O | C-A3 | C-A1 | C-A3 | C-A1 | B20 | B81 |
| H8-182 | O | C-A3 | C-A1 | C-A3 | C-A1 | B20 | B90 |
| H8-183 | O | C-A3 | C-A1 | C-A3 | C-A1 | B20 | B91 |
| H8-184 | O | C-A3 | C-A1 | C-A3 | C-A1 | B20 | B92 |
| H8-185 | O | C-A3 | C-A1 | C-A3 | C-A1 | B20 | B93 |
| H8-186 | O | C-A3 | C-A1 | C-A3 | C-A1 | B20 | B94 |
| H8-187 | O | C-A3 | C-A1 | C-A3 | C-A1 | B20 | B95 |
| H8-188 | O | C-A3 | C-A1 | C-A3 | C-A1 | B20 | B96 |
| H8-189 | O | C-A3 | C-A1 | C-A3 | C-A1 | B20 | B97 |
| H8-190 | O | C-A3 | C-A1 | C-A3 | C-A1 | B20 | B106 |
| H8-191 | O | C-A3 | C-A1 | C-A3 | C-A1 | B20 | B107 |
| H8-192 | O | C-A3 | C-A1 | C-A3 | C-A1 | B20 | B114 |
| H8-193 | O | C-A3 | C-A1 | C-A3 | C-A1 | B20 | B115 |
| H8-194 | O | C-A3 | C-A1 | C-A3 | C-A1 | B20 | B116 |
| H8-195 | O | C-A3 | C-A1 | C-A3 | C-A1 | B20 | B117 |
| H8-196 | O | C-A3 | C-A1 | C-A3 | C-A1 | B20 | B118 |
| H8-197 | O | C-A3 | C-A1 | C-A3 | C-A1 | B20 | B119 |
| H8-198 | O | C-A3 | C-A1 | C-A3 | C-A1 | B20 | B120 |
| H8-199 | O | C-A3 | C-A1 | C-A3 | C-A1 | B20 | B121 |
| H8-200 | O | C-A3 | C-A1 | C-A3 | C-A1 | B20 | B122 |
| H8-201 | O | C-A3 | C-A1 | C-A3 | C-A1 | B20 | B125 |
| H8-202 | O | C-A3 | C-A1 | C-A3 | C-A1 | B20 | B126 |
| H8-203 | O | C-A3 | C-A1 | C-A3 | C-A1 | B20 | B127 |
| H8-204 | O | C-A3 | C-A1 | C-A3 | C-A1 | B20 | B128 |
| H8-205 | O | C-A3 | C-A1 | C-A3 | C-A1 | B20 | B129 |
| H8-206 | O | C-A3 | C-A1 | C-A3 | C-A1 | B20 | B130 |
| H8-207 | O | C-A3 | C-A1 | C-A3 | C-A1 | B20 | B131 |
| H8-208 | O | C-A3 | C-A1 | C-A3 | C-A1 | B20 | B132 |
| H8-209 | O | C-A3 | C-A1 | C-A3 | C-A1 | B20 | B133 |
| H8-210 | O | C-A3 | C-A1 | C-A3 | C-A1 | B20 | B134 |
| H8-211 | O | C-A3 | C-A1 | C-A3 | C-A1 | B20 | B135 |
| H8-212 | O | C-A3 | C-A1 | C-A3 | C-A1 | B20 | B136 |
| H8-213 | O | C-A3 | C-A1 | C-A3 | C-A1 | B20 | B137 |
| H8-214 | O | C-A3 | C-A1 | C-A3 | C-A1 | B20 | B138 |
| H8-215 | O | C-A3 | C-A1 | C-A3 | C-A1 | B20 | B139 |
| H8-216 | O | C-A3 | C-A1 | C-A3 | C-A1 | B20 | B140 |
| H8-223 | O | C-A1 | C-A1 | C-A3 | C-A1 | B20 | B1 |
| H8-224 | O | C-A1 | C-A1 | C-A3 | C-A1 | B20 | B6 |
| H8-225 | O | C-A1 | C-A1 | C-A3 | C-A1 | B20 | B20 |
| H8-226 | O | C-A1 | C-A1 | C-A3 | C-A1 | B20 | B21 |
| H8-227 | O | C-A1 | C-A1 | C-A3 | C-A1 | B20 | B22 |
| H8-228 | O | C-A1 | C-A1 | C-A3 | C-A1 | B20 | B32 |
| H8-229 | O | C-A1 | C-A1 | C-A3 | C-A1 | B20 | B33 |
| H8-230 | O | C-A1 | C-A1 | C-A3 | C-A1 | B20 | B34 |
| H8-231 | O | C-A1 | C-A1 | C-A3 | C-A1 | B20 | B68 |
| H8-232 | O | C-A1 | C-A1 | C-A3 | C-A1 | B20 | B69 |
| H8-233 | O | C-A1 | C-A1 | C-A3 | C-A1 | B20 | B70 |
| H8-234 | O | C-A1 | C-A1 | C-A3 | C-A1 | B20 | B71 |
| H8-235 | O | C-A1 | C-A1 | C-A3 | C-A1 | B20 | B81 |
| H8-236 | O | C-A1 | C-A1 | C-A3 | C-A1 | B20 | B90 |
| H8-237 | O | C-A1 | C-A1 | C-A3 | C-A1 | B20 | B91 |
| H8-238 | O | C-A1 | C-A1 | C-A3 | C-A1 | B20 | B92 |
| H8-239 | O | C-A1 | C-A1 | C-A3 | C-A1 | B20 | B93 |
| H8-240 | O | C-A1 | C-A1 | C-A3 | C-A1 | B20 | B94 |
| H8-241 | O | C-A1 | C-A1 | C-A3 | C-A1 | B20 | B95 |
| H8-242 | O | C-A1 | C-A1 | C-A3 | C-A1 | B20 | B96 |
| H8-243 | O | C-A1 | C-A1 | C-A3 | C-A1 | B20 | B97 |
| H8-244 | O | C-A1 | C-A1 | C-A3 | C-A1 | B20 | B106 |
| H8-245 | O | C-A1 | C-A1 | C-A3 | C-A1 | B20 | B107 |
| H8-246 | O | C-A1 | C-A1 | C-A3 | C-A1 | B20 | B114 |
| H8-247 | O | C-A1 | C-A1 | C-A3 | C-A1 | B20 | B115 |
| H8-248 | O | C-A1 | C-A1 | C-A3 | C-A1 | B20 | B116 |
| H8-249 | O | C-A1 | C-A1 | C-A3 | C-A1 | B20 | B117 |
| H8-250 | O | C-A1 | C-A1 | C-A3 | C-A1 | B20 | B118 |
| H8-251 | O | C-A1 | C-A1 | C-A3 | C-A1 | B20 | B119 |
| H8-252 | O | C-A1 | C-A1 | C-A3 | C-A1 | B20 | B120 |
| H8-253 | O | C-A1 | C-A1 | C-A3 | C-A1 | B20 | B121 |
| H8-254 | O | C-A1 | C-A1 | C-A3 | C-A1 | B20 | B122 |
| H8-255 | O | C-A1 | C-A1 | C-A3 | C-A1 | B20 | B125 |
| H8-256 | O | C-A1 | C-A1 | C-A3 | C-A1 | B20 | B126 |
| H8-257 | O | C-A1 | C-A1 | C-A3 | C-A1 | B20 | B127 |
| H8-258 | O | C-A1 | C-A1 | C-A3 | C-A1 | B20 | B128 |
| H8-259 | O | C-A1 | C-A1 | C-A3 | C-A1 | B20 | B129 |
| H8-260 | O | C-A1 | C-A1 | C-A3 | C-A1 | B20 | B130 |
| H8-261 | O | C-A1 | C-A1 | C-A3 | C-A1 | B20 | B131 |
| H8-262 | O | C-A1 | C-A1 | C-A3 | C-A1 | B20 | B132 |
| H8-263 | O | C-A1 | C-A1 | C-A3 | C-A1 | B20 | B133 |
| H8-264 | O | C-A1 | C-A1 | C-A3 | C-A1 | B20 | B134 |
| H8-265 | O | C-A1 | C-A1 | C-A3 | C-A1 | B20 | B135 |
| H8-266 | O | C-A1 | C-A1 | C-A3 | C-A1 | B20 | B136 |
| H8-267 | O | C-A1 | C-A1 | C-A3 | C-A1 | B20 | B137 |
| H8-268 | O | C-A1 | C-A1 | C-A3 | C-A1 | B20 | B138 |
| H8-269 | O | C-A1 | C-A1 | C-A3 | C-A1 | B20 | B139 |
| H8-270 | O | C-A1 | C-A1 | C-A3 | C-A1 | B20 | B140 |
| H8-277 | O | C-A1 | C-A1 | C-A1 | C-A1 | B68 | B1 |
| H8-278 | O | C-A1 | C-A1 | C-A1 | C-A1 | B68 | B6 |
| H8-279 | O | C-A1 | C-A1 | C-A1 | C-A1 | B68 | B20 |
| H8-280 | O | C-A1 | C-A1 | C-A1 | C-A1 | B68 | B21 |
| H8-281 | O | C-A1 | C-A1 | C-A1 | C-A1 | B68 | B22 |
| H8-282 | O | C-A1 | C-A1 | C-A1 | C-A1 | B68 | B32 |
| H8-283 | O | C-A1 | C-A1 | C-A1 | C-A1 | B68 | B33 |
| H8-284 | O | C-A1 | C-A1 | C-A1 | C-A1 | B68 | B34 |
| H8-285 | O | C-A1 | C-A1 | C-A1 | C-A1 | B68 | B68 |
| H8-286 | O | C-A1 | C-A1 | C-A1 | C-A1 | B68 | B69 |
| H8-287 | O | C-A1 | C-A1 | C-A1 | C-A1 | B68 | B70 |
| H8-288 | O | C-A1 | C-A1 | C-A1 | C-A1 | B68 | B71 |
| H8-289 | O | C-A1 | C-A1 | C-A1 | C-A1 | B68 | B81 |
| H8-290 | O | C-A1 | C-A1 | C-A1 | C-A1 | B68 | B90 |
| H8-291 | O | C-A1 | C-A1 | C-A1 | C-A1 | B68 | B91 |
| H8-292 | O | C-A1 | C-A1 | C-A1 | C-A1 | B68 | B92 |
| H8-293 | O | C-A1 | C-A1 | C-A1 | C-A1 | B68 | B93 |
| H8-294 | O | C-A1 | C-A1 | C-A1 | C-A1 | B68 | B94 |
| H8-295 | O | C-A1 | C-A1 | C-A1 | C-A1 | B68 | B95 |
| H8-296 | O | C-A1 | C-A1 | C-A1 | C-A1 | B68 | B96 |
| H8-297 | O | C-A1 | C-A1 | C-A1 | C-A1 | B68 | B97 |
| H8-298 | O | C-A1 | C-A1 | C-A1 | C-A1 | B68 | B106 |
| H8-299 | O | C-A1 | C-A1 | C-A1 | C-A1 | B68 | B107 |
| H8-300 | O | C-A1 | C-A1 | C-A1 | C-A1 | B68 | B114 |
| H8-301 | O | C-A1 | C-A1 | C-A1 | C-A1 | B68 | B115 |
| H8-302 | O | C-A1 | C-A1 | C-A1 | C-A1 | B68 | B116 |
| H8-303 | O | C-A1 | C-A1 | C-A1 | C-A1 | B68 | B117 |
| H8-304 | O | C-A1 | C-A1 | C-A1 | C-A1 | B68 | B118 |
| H8-305 | O | C-A1 | C-A1 | C-A1 | C-A1 | B68 | B119 |
| H8-306 | O | C-A1 | C-A1 | C-A1 | C-A1 | B68 | B120 |
| H8-307 | O | C-A1 | C-A1 | C-A1 | C-A1 | B68 | B121 |
| H8-308 | O | C-A1 | C-A1 | C-A1 | C-A1 | B68 | B122 |
| H8-309 | O | C-A1 | C-A1 | C-A1 | C-A1 | B68 | B125 |
| H8-310 | O | C-A1 | C-A1 | C-A1 | C-A1 | B68 | B126 |
| H8-311 | O | C-A1 | C-A1 | C-A1 | C-A1 | B68 | B127 |
| H8-312 | O | C-A1 | C-A1 | C-A1 | C-A1 | B68 | B128 |
| H8-313 | O | C-A1 | C-A1 | C-A1 | C-A1 | B68 | B129 |
| H8-314 | O | C-A1 | C-A1 | C-A1 | C-A1 | B68 | B130 |
| H8-315 | O | C-A1 | C-A1 | C-A1 | C-A1 | B68 | B131 |
| H8-316 | O | C-A1 | C-A1 | C-A1 | C-A1 | B68 | B132 |
| H8-317 | O | C-A1 | C-A1 | C-A1 | C-A1 | B68 | B133 |
| H8-318 | O | C-A1 | C-A1 | C-A1 | C-A1 | B68 | B134 |
| H8-319 | O | C-A1 | C-A1 | C-A1 | C-A1 | B68 | B135 |
| H8-320 | O | C-A1 | C-A1 | C-A1 | C-A1 | B68 | B136 |
| H8-321 | O | C-A1 | C-A1 | C-A1 | C-A1 | B68 | B137 |
| H8-322 | O | C-A1 | C-A1 | C-A1 | C-A1 | B68 | B138 |
| H8-323 | O | C-A1 | C-A1 | C-A1 | C-A1 | B68 | B139 |
| H8-324 | O | C-A1 | C-A1 | C-A1 | C-A1 | B68 | B140 |
| H8-331 | O | C-A1 | C-A2 | C-A1 | C-A2 | B68 | B1 |
| H8-332 | O | C-A1 | C-A2 | C-A1 | C-A2 | B68 | B6 |
| H8-333 | O | C-A1 | C-A2 | C-A1 | C-A2 | B68 | B20 |
| H8-334 | O | C-A1 | C-A2 | C-A1 | C-A2 | B68 | B21 |
| H8-335 | O | C-A1 | C-A2 | C-A1 | C-A2 | B68 | B22 |
| H8-336 | O | C-A1 | C-A2 | C-A1 | C-A2 | B68 | B32 |
| H8-337 | O | C-A1 | C-A2 | C-A1 | C-A2 | B68 | B33 |
| H8-338 | O | C-A1 | C-A2 | C-A1 | C-A2 | B68 | B34 |
| H8-339 | O | C-A1 | C-A2 | C-A1 | C-A2 | B68 | B68 |
| H8-340 | O | C-A1 | C-A2 | C-A1 | C-A2 | B68 | B69 |
| H8-341 | O | C-A1 | C-A2 | C-A1 | C-A2 | B68 | B70 |
| H8-342 | O | C-A1 | C-A2 | C-A1 | C-A2 | B68 | B71 |
| H8-343 | O | C-A1 | C-A2 | C-A1 | C-A2 | B68 | B81 |
| H8-344 | O | C-A1 | C-A2 | C-A1 | C-A2 | B68 | B90 |
| H8-345 | O | C-A1 | C-A2 | C-A1 | C-A2 | B68 | B91 |
| H8-346 | O | C-A1 | C-A2 | C-A1 | C-A2 | B68 | B92 |
| H8-347 | O | C-A1 | C-A2 | C-A1 | C-A2 | B68 | B93 |
| H8-348 | O | C-A1 | C-A2 | C-A1 | C-A2 | B68 | B94 |
| H8-349 | O | C-A1 | C-A2 | C-A1 | C-A2 | B68 | B95 |
| H8-350 | O | C-A1 | C-A2 | C-A1 | C-A2 | B68 | B96 |
| H8-351 | O | C-A1 | C-A2 | C-A1 | C-A2 | B68 | B97 |
| H8-352 | O | C-A1 | C-A2 | C-A1 | C-A2 | B68 | B106 |
| H8-353 | O | C-A1 | C-A2 | C-A1 | C-A2 | B68 | B107 |
| H8-354 | O | C-A1 | C-A2 | C-A1 | C-A2 | B68 | B114 |
| H8-355 | O | C-A1 | C-A2 | C-A1 | C-A2 | B68 | B115 |
| H8-356 | O | C-A1 | C-A2 | C-A1 | C-A2 | B68 | B116 |
| H8-357 | O | C-A1 | C-A2 | C-A1 | C-A2 | B68 | B117 |
| H8-358 | O | C-A1 | C-A2 | C-A1 | C-A2 | B68 | B118 |
| H8-359 | O | C-A1 | C-A2 | C-A1 | C-A2 | B68 | B119 |
| H8-360 | O | C-A1 | C-A2 | C-A1 | C-A2 | B68 | B120 |
| H8-361 | O | C-A1 | C-A2 | C-A1 | C-A2 | B68 | B121 |
| H8-362 | O | C-A1 | C-A2 | C-A1 | C-A2 | B68 | B122 |
| H8-363 | O | C-A1 | C-A2 | C-A1 | C-A2 | B68 | B125 |
| H8-364 | O | C-A1 | C-A2 | C-A1 | C-A2 | B68 | B126 |
| H8-365 | O | C-A1 | C-A2 | C-A1 | C-A2 | B68 | B127 |
| H8-366 | O | C-A1 | C-A2 | C-A1 | C-A2 | B68 | B128 |
| H8-367 | O | C-A1 | C-A2 | C-A1 | C-A2 | B68 | B129 |
| H8-368 | O | C-A1 | C-A2 | C-A1 | C-A2 | B68 | B130 |
| H8-369 | O | C-A1 | C-A2 | C-A1 | C-A2 | B68 | B131 |
| H8-370 | O | C-A1 | C-A2 | C-A1 | C-A2 | B68 | B132 |
| H8-371 | O | C-A1 | C-A2 | C-A1 | C-A2 | B68 | B133 |
| H8-372 | O | C-A1 | C-A2 | C-A1 | C-A2 | B68 | B134 |
| H8-373 | O | C-A1 | C-A2 | C-A1 | C-A2 | B68 | B135 |
| H8-374 | O | C-A1 | C-A2 | C-A1 | C-A2 | B68 | B136 |
| H8-375 | O | C-A1 | C-A2 | C-A1 | C-A2 | B68 | B137 |
| H8-376 | O | C-A1 | C-A2 | C-A1 | C-A2 | B68 | B138 |
| H8-377 | O | C-A1 | C-A2 | C-A1 | C-A2 | B68 | B139 |
| H8-378 | O | C-A1 | C-A2 | C-A1 | C-A2 | B68 | B140 |
| H8-385 | O | C-A1 | C-A3 | C-A1 | C-A3 | B68 | B1 |
| H8-386 | O | C-A1 | C-A3 | C-A1 | C-A3 | B68 | B6 |
| H8-387 | O | C-A1 | C-A3 | C-A1 | C-A3 | B68 | B20 |
| H8-388 | O | C-A1 | C-A3 | C-A1 | C-A3 | B68 | B21 |
| H8-389 | O | C-A1 | C-A3 | C-A1 | C-A3 | B68 | B22 |
| H8-390 | O | C-A1 | C-A3 | C-A1 | C-A3 | B68 | B32 |
| H8-391 | O | C-A1 | C-A3 | C-A1 | C-A3 | B68 | B33 |
| H8-392 | O | C-A1 | C-A3 | C-A1 | C-A3 | B68 | B34 |
| H8-393 | O | C-A1 | C-A3 | C-A1 | C-A3 | B68 | B68 |
| H8-394 | O | C-A1 | C-A3 | C-A1 | C-A3 | B68 | B69 |
| H8-395 | O | C-A1 | C-A3 | C-A1 | C-A3 | B68 | B70 |
| H8-396 | O | C-A1 | C-A3 | C-A1 | C-A3 | B68 | B71 |
| H8-397 | O | C-A1 | C-A3 | C-A1 | C-A3 | B68 | B81 |
| H8-398 | O | C-A1 | C-A3 | C-A1 | C-A3 | B68 | B90 |
| H8-399 | O | C-A1 | C-A3 | C-A1 | C-A3 | B68 | B91 |
| H8-400 | O | C-A1 | C-A3 | C-A1 | C-A3 | B68 | B92 |
| H8-401 | O | C-A1 | C-A3 | C-A1 | C-A3 | B68 | B93 |
| H8-402 | O | C-A1 | C-A3 | C-A1 | C-A3 | B68 | B94 |
| H8-403 | O | C-A1 | C-A3 | C-A1 | C-A3 | B68 | B95 |
| H8-404 | O | C-A1 | C-A3 | C-A1 | C-A3 | B68 | B96 |
| H8-405 | O | C-A1 | C-A3 | C-A1 | C-A3 | B68 | B97 |
| H8-406 | O | C-A1 | C-A3 | C-A1 | C-A3 | B68 | B106 |
| H8-407 | O | C-A1 | C-A3 | C-A1 | C-A3 | B68 | B107 |
| H8-408 | O | C-A1 | C-A3 | C-A1 | C-A3 | B68 | B114 |
| H8-409 | O | C-A1 | C-A3 | C-A1 | C-A3 | B68 | B115 |
| H8-410 | O | C-A1 | C-A3 | C-A1 | C-A3 | B68 | B116 |
| H8-411 | O | C-A1 | C-A3 | C-A1 | C-A3 | B68 | B117 |
| H8-412 | O | C-A1 | C-A3 | C-A1 | C-A3 | B68 | B118 |
| H8-413 | O | C-A1 | C-A3 | C-A1 | C-A3 | B68 | B119 |
| H8-414 | O | C-A1 | C-A3 | C-A1 | C-A3 | B68 | B120 |
| H8-415 | O | C-A1 | C-A3 | C-A1 | C-A3 | B68 | B121 |
| H8-416 | O | C-A1 | C-A3 | C-A1 | C-A3 | B68 | B122 |
| H8-417 | O | C-A1 | C-A3 | C-A1 | C-A3 | B68 | B125 |
| H8-418 | O | C-A1 | C-A3 | C-A1 | C-A3 | B68 | B126 |
| H8-419 | O | C-A1 | C-A3 | C-A1 | C-A3 | B68 | B127 |
| H8-420 | O | C-A1 | C-A3 | C-A1 | C-A3 | B68 | B128 |
| H8-421 | O | C-A1 | C-A3 | C-A1 | C-A3 | B68 | B129 |
| H8-422 | O | C-A1 | C-A3 | C-A1 | C-A3 | B68 | B130 |
| H8-423 | O | C-A1 | C-A3 | C-A1 | C-A3 | B68 | B131 |
| H8-424 | O | C-A1 | C-A3 | C-A1 | C-A3 | B68 | B132 |
| H8-425 | O | C-A1 | C-A3 | C-A1 | C-A3 | B68 | B133 |
| H8-426 | O | C-A1 | C-A3 | C-A1 | C-A3 | B68 | B134 |
| H8-427 | O | C-A1 | C-A3 | C-A1 | C-A3 | B68 | B135 |
| H8-428 | O | C-A1 | C-A3 | C-A1 | C-A3 | B68 | B136 |
| H8-429 | O | C-A1 | C-A3 | C-A1 | C-A3 | B68 | B137 |
| H8-430 | O | C-A1 | C-A3 | C-A1 | C-A3 | B68 | B138 |
| H8-431 | O | C-A1 | C-A3 | C-A1 | C-A3 | B68 | B139 |
| H8-432 | O | C-A1 | C-A3 | C-A1 | C-A3 | B68 | B140 |
| H8-439 | O | C-A3 | C-A1 | C-A3 | C-A1 | B68 | B1 |
| H8-440 | O | C-A3 | C-A1 | C-A3 | C-A1 | B68 | B6 |
| H8-441 | O | C-A3 | C-A1 | C-A3 | C-A1 | B68 | B20 |
| H8-442 | O | C-A3 | C-A1 | C-A3 | C-A1 | B68 | B21 |
| H8-443 | O | C-A3 | C-A1 | C-A3 | C-A1 | B68 | B22 |
| H8-444 | O | C-A3 | C-A1 | C-A3 | C-A1 | B68 | B32 |
| H8-445 | O | C-A3 | C-A1 | C-A3 | C-A1 | B68 | B33 |
| H8-446 | O | C-A3 | C-A1 | C-A3 | C-A1 | B68 | B34 |
| H8-447 | O | C-A3 | C-A1 | C-A3 | C-A1 | B68 | B68 |
| H8-448 | O | C-A3 | C-A1 | C-A3 | C-A1 | B68 | B69 |
| H8-449 | O | C-A3 | C-A1 | C-A3 | C-A1 | B68 | B70 |
| H8-450 | O | C-A3 | C-A1 | C-A3 | C-A1 | B68 | B71 |
| H8-451 | O | C-A3 | C-A1 | C-A3 | C-A1 | B68 | B81 |
| H8-452 | O | C-A3 | C-A1 | C-A3 | C-A1 | B68 | B90 |
| H8-453 | O | C-A3 | C-A1 | C-A3 | C-A1 | B68 | B91 |
| H8-454 | O | C-A3 | C-A1 | C-A3 | C-A1 | B68 | B92 |
| H8-455 | O | C-A3 | C-A1 | C-A3 | C-A1 | B68 | B93 |
| H8-456 | O | C-A3 | C-A1 | C-A3 | C-A1 | B68 | B94 |
| H8-457 | O | C-A3 | C-A1 | C-A3 | C-A1 | B68 | B95 |
| H8-458 | O | C-A3 | C-A1 | C-A3 | C-A1 | B68 | B96 |
| H8-459 | O | C-A3 | C-A1 | C-A3 | C-A1 | B68 | B97 |
| H8-460 | O | C-A3 | C-A1 | C-A3 | C-A1 | B68 | B106 |
| H8-461 | O | C-A3 | C-A1 | C-A3 | C-A1 | B68 | B107 |
| H8-462 | O | C-A3 | C-A1 | C-A3 | C-A1 | B68 | B114 |
| H8-463 | O | C-A3 | C-A1 | C-A3 | C-A1 | B68 | B115 |
| H8-464 | O | C-A3 | C-A1 | C-A3 | C-A1 | B68 | B116 |
| H8-465 | O | C-A3 | C-A1 | C-A3 | C-A1 | B68 | B117 |
| H8-466 | O | C-A3 | C-A1 | C-A3 | C-A1 | B68 | B118 |
| H8-467 | O | C-A3 | C-A1 | C-A3 | C-A1 | B68 | B119 |
| H8-468 | O | C-A3 | C-A1 | C-A3 | C-A1 | B68 | B120 |
| H8-469 | O | C-A3 | C-A1 | C-A3 | C-A1 | B68 | B121 |
| H8-470 | O | C-A3 | C-A1 | C-A3 | C-A1 | B68 | B122 |
| H8-471 | O | C-A3 | C-A1 | C-A3 | C-A1 | B68 | B125 |
| H8-472 | O | C-A3 | C-A1 | C-A3 | C-A1 | B68 | B126 |
| H8-473 | O | C-A3 | C-A1 | C-A3 | C-A1 | B68 | B127 |
| H8-474 | O | C-A3 | C-A1 | C-A3 | C-A1 | B68 | B128 |
| H8-475 | O | C-A3 | C-A1 | C-A3 | C-A1 | B68 | B129 |
| H8-476 | O | C-A3 | C-A1 | C-A3 | C-A1 | B68 | B130 |
| H8-477 | O | C-A3 | C-A1 | C-A3 | C-A1 | B68 | B131 |
| H8-478 | O | C-A3 | C-A1 | C-A3 | C-A1 | B68 | B132 |
| H8-479 | O | C-A3 | C-A1 | C-A3 | C-A1 | B68 | B133 |
| H8-480 | O | C-A3 | C-A1 | C-A3 | C-A1 | B68 | B134 |
| H8-481 | O | C-A3 | C-A1 | C-A3 | C-A1 | B68 | B135 |
| H8-482 | O | C-A3 | C-A1 | C-A3 | C-A1 | B68 | B136 |
| H8-483 | O | C-A3 | C-A1 | C-A3 | C-A1 | B68 | B137 |
| H8-484 | O | C-A3 | C-A1 | C-A3 | C-A1 | B68 | B138 |
| H8-485 | O | C-A3 | C-A1 | C-A3 | C-A1 | B68 | B139 |
| H8-486 | O | C-A3 | C-A1 | C-A3 | C-A1 | B68 | B140 |
| H8-493 | O | C-A1 | C-A1 | C-A3 | C-A1 | B68 | B1 |
| H8-494 | O | C-A1 | C-A1 | C-A3 | C-A1 | B68 | B6 |
| H8-495 | O | C-A1 | C-A1 | C-A3 | C-A1 | B68 | B20 |
| H8-496 | O | C-A1 | C-A1 | C-A3 | C-A1 | B68 | B21 |
| H8-497 | O | C-A1 | C-A1 | C-A3 | C-A1 | B68 | B22 |
| H8-498 | O | C-A1 | C-A1 | C-A3 | C-A1 | B68 | B32 |
| H8-499 | O | C-A1 | C-A1 | C-A3 | C-A1 | B68 | B33 |
| H8-500 | O | C-A1 | C-A1 | C-A3 | C-A1 | B68 | B34 |
| H8-501 | O | C-A1 | C-A1 | C-A3 | C-A1 | B68 | B68 |
| H8-502 | O | C-A1 | C-A1 | C-A3 | C-A1 | B68 | B69 |
| H8-503 | O | C-A1 | C-A1 | C-A3 | C-A1 | B68 | B70 |
| H8-504 | O | C-A1 | C-A1 | C-A3 | C-A1 | B68 | B71 |
| H8-505 | O | C-A1 | C-A1 | C-A3 | C-A1 | B68 | B81 |
| H8-506 | O | C-A1 | C-A1 | C-A3 | C-A1 | B68 | B90 |
| H8-507 | O | C-A1 | C-A1 | C-A3 | C-A1 | B68 | B91 |
| H8-508 | O | C-A1 | C-A1 | C-A3 | C-A1 | B68 | B92 |
| H8-509 | O | C-A1 | C-A1 | C-A3 | C-A1 | B68 | B93 |
| H8-510 | O | C-A1 | C-A1 | C-A3 | C-A1 | B68 | B94 |
| H8-511 | O | C-A1 | C-A1 | C-A3 | C-A1 | B68 | B95 |
| H8-512 | O | C-A1 | C-A1 | C-A3 | C-A1 | B68 | B96 |
| H8-513 | O | C-A1 | C-A1 | C-A3 | C-A1 | B68 | B97 |
| H8-514 | O | C-A1 | C-A1 | C-A3 | C-A1 | B68 | B106 |
| H8-515 | O | C-A1 | C-A1 | C-A3 | C-A1 | B68 | B107 |
| H8-516 | O | C-A1 | C-A1 | C-A3 | C-A1 | B68 | B114 |
| H8-517 | O | C-A1 | C-A1 | C-A3 | C-A1 | B68 | B115 |
| H8-518 | O | C-A1 | C-A1 | C-A3 | C-A1 | B68 | B116 |
| H8-519 | O | C-A1 | C-A1 | C-A3 | C-A1 | B68 | B117 |
| H8-520 | O | C-A1 | C-A1 | C-A3 | C-A1 | B68 | B118 |
| H8-521 | O | C-A1 | C-A1 | C-A3 | C-A1 | B68 | B119 |
| H8-522 | O | C-A1 | C-A1 | C-A3 | C-A1 | B68 | B120 |
| H8-523 | O | C-A1 | C-A1 | C-A3 | C-A1 | B68 | B121 |
| H8-524 | O | C-A1 | C-A1 | C-A3 | C-A1 | B68 | B122 |
| H8-525 | O | C-A1 | C-A1 | C-A3 | C-A1 | B68 | B125 |
| H8-526 | O | C-A1 | C-A1 | C-A3 | C-A1 | B68 | B126 |
| H8-527 | O | C-A1 | C-A1 | C-A3 | C-A1 | B68 | B127 |
| H8-528 | O | C-A1 | C-A1 | C-A3 | C-A1 | B68 | B128 |
| H8-529 | O | C-A1 | C-A1 | C-A3 | C-A1 | B68 | B129 |
| H8-530 | O | C-A1 | C-A1 | C-A3 | C-A1 | B68 | B130 |
| H8-531 | O | C-A1 | C-A1 | C-A3 | C-A1 | B68 | B131 |
| H8-532 | O | C-A1 | C-A1 | C-A3 | C-A1 | B68 | B132 |
| H8-533 | O | C-A1 | C-A1 | C-A3 | C-A1 | B68 | B133 |
| H8-534 | O | C-A1 | C-A1 | C-A3 | C-A1 | B68 | B134 |
| H8-535 | O | C-A1 | C-A1 | C-A3 | C-A1 | B68 | B135 |
| H8-536 | O | C-A1 | C-A1 | C-A3 | C-A1 | B68 | B136 |
| H8-537 | O | C-A1 | C-A1 | C-A3 | C-A1 | B68 | B137 |
| H8-538 | O | C-A1 | C-A1 | C-A3 | C-A1 | B68 | B138 |
| H8-539 | O | C-A1 | C-A1 | C-A3 | C-A1 | B68 | B139 |
| H8-540 | O | C-A1 | C-A1 | C-A3 | C-A1 | B68 | B140 |
| H8-547 | O | C-A1 | C-A1 | C-A1 | C-A1 | B70 | B1 |
| H8-548 | O | C-A1 | C-A1 | C-A1 | C-A1 | B70 | B6 |
| H8-549 | O | C-A1 | C-A1 | C-A1 | C-A1 | B70 | B20 |
| H8-550 | O | C-A1 | C-A1 | C-A1 | C-A1 | B70 | B21 |
| H8-551 | O | C-A1 | C-A1 | C-A1 | C-A1 | B70 | B22 |
| H8-552 | O | C-A1 | C-A1 | C-A1 | C-A1 | B70 | B32 |
| H8-553 | O | C-A1 | C-A1 | C-A1 | C-A1 | B70 | B33 |
| H8-554 | O | C-A1 | C-A1 | C-A1 | C-A1 | B70 | B34 |
| H8-555 | O | C-A1 | C-A1 | C-A1 | C-A1 | B70 | B68 |
| H8-556 | O | C-A1 | C-A1 | C-A1 | C-A1 | B70 | B69 |
| H8-557 | O | C-A1 | C-A1 | C-A1 | C-A1 | B70 | B70 |
| H8-558 | O | C-A1 | C-A1 | C-A1 | C-A1 | B70 | B71 |
| H8-559 | O | C-A1 | C-A1 | C-A1 | C-A1 | B70 | B81 |
| H8-560 | O | C-A1 | C-A1 | C-A1 | C-A1 | B70 | B90 |
| H8-561 | O | C-A1 | C-A1 | C-A1 | C-A1 | B70 | B91 |
| H8-562 | O | C-A1 | C-A1 | C-A1 | C-A1 | B70 | B92 |
| H8-563 | O | C-A1 | C-A1 | C-A1 | C-A1 | B70 | B93 |
| H8-564 | O | C-A1 | C-A1 | C-A1 | C-A1 | B70 | B94 |
| H8-565 | O | C-A1 | C-A1 | C-A1 | C-A1 | B70 | B95 |
| H8-566 | O | C-A1 | C-A1 | C-A1 | C-A1 | B70 | B96 |
| H8-567 | O | C-A1 | C-A1 | C-A1 | C-A1 | B70 | B97 |
| H8-568 | O | C-A1 | C-A1 | C-A1 | C-A1 | B70 | B106 |
| H8-569 | O | C-A1 | C-A1 | C-A1 | C-A1 | B70 | B107 |
| H8-570 | O | C-A1 | C-A1 | C-A1 | C-A1 | B70 | B114 |
| H8-571 | O | C-A1 | C-A1 | C-A1 | C-A1 | B70 | B115 |
| H8-572 | O | C-A1 | C-A1 | C-A1 | C-A1 | B70 | B116 |
| H8-573 | O | C-A1 | C-A1 | C-A1 | C-A1 | B70 | B117 |
| H8-574 | O | C-A1 | C-A1 | C-A1 | C-A1 | B70 | B118 |
| H8-575 | O | C-A1 | C-A1 | C-A1 | C-A1 | B70 | B119 |
| H8-576 | O | C-A1 | C-A1 | C-A1 | C-A1 | B70 | B120 |
| H8-577 | O | C-A1 | C-A1 | C-A1 | C-A1 | B70 | B121 |
| H8-578 | O | C-A1 | C-A1 | C-A1 | C-A1 | B70 | B122 |
| H8-579 | O | C-A1 | C-A1 | C-A1 | C-A1 | B70 | B125 |
| H8-580 | O | C-A1 | C-A1 | C-A1 | C-A1 | B70 | B126 |
| H8-581 | O | C-A1 | C-A1 | C-A1 | C-A1 | B70 | B127 |
| H8-582 | O | C-A1 | C-A1 | C-A1 | C-A1 | B70 | B128 |
| H8-583 | O | C-A1 | C-A1 | C-A1 | C-A1 | B70 | B129 |
| H8-584 | O | C-A1 | C-A1 | C-A1 | C-A1 | B70 | B130 |
| H8-585 | O | C-A1 | C-A1 | C-A1 | C-A1 | B70 | B131 |
| H8-586 | O | C-A1 | C-A1 | C-A1 | C-A1 | B70 | B132 |
| H8-587 | O | C-A1 | C-A1 | C-A1 | C-A1 | B70 | B133 |
| H8-588 | O | C-A1 | C-A1 | C-A1 | C-A1 | B70 | B134 |
| H8-589 | O | C-A1 | C-A1 | C-A1 | C-A1 | B70 | B135 |
| H8-590 | O | C-A1 | C-A1 | C-A1 | C-A1 | B70 | B136 |
| H8-591 | O | C-A1 | C-A1 | C-A1 | C-A1 | B70 | B137 |
| H8-592 | O | C-A1 | C-A1 | C-A1 | C-A1 | B70 | B138 |
| H8-593 | O | C-A1 | C-A1 | C-A1 | C-A1 | B70 | B139 |
| H8-594 | O | C-A1 | C-A1 | C-A1 | C-A1 | B70 | B140 |
| H8-601 | O | C-A1 | C-A2 | C-A1 | C-A2 | B70 | B1 |
| H8-602 | O | C-A1 | C-A2 | C-A1 | C-A2 | B70 | B6 |
| H8-603 | O | C-A1 | C-A2 | C-A1 | C-A2 | B70 | B20 |
| H8-604 | O | C-A1 | C-A2 | C-A1 | C-A2 | B70 | B21 |
| H8-605 | O | C-A1 | C-A2 | C-A1 | C-A2 | B70 | B22 |
| H8-606 | O | C-A1 | C-A2 | C-A1 | C-A2 | B70 | B32 |
| H8-607 | O | C-A1 | C-A2 | C-A1 | C-A2 | B70 | B33 |
| H8-608 | O | C-A1 | C-A2 | C-A1 | C-A2 | B70 | B34 |
| H8-609 | O | C-A1 | C-A2 | C-A1 | C-A2 | B70 | B68 |
| H8-610 | O | C-A1 | C-A2 | C-A1 | C-A2 | B70 | B69 |
| H8-611 | O | C-A1 | C-A2 | C-A1 | C-A2 | B70 | B70 |
| H8-612 | O | C-A1 | C-A2 | C-A1 | C-A2 | B70 | B71 |
| H8-613 | O | C-A1 | C-A2 | C-A1 | C-A2 | B70 | B81 |
| H8-614 | O | C-A1 | C-A2 | C-A1 | C-A2 | B70 | B90 |
| H8-615 | O | C-A1 | C-A2 | C-A1 | C-A2 | B70 | B91 |
| H8-616 | O | C-A1 | C-A2 | C-A1 | C-A2 | B70 | B92 |
| H8-617 | O | C-A1 | C-A2 | C-A1 | C-A2 | B70 | B93 |
| H8-618 | O | C-A1 | C-A2 | C-A1 | C-A2 | B70 | B94 |
| H8-619 | O | C-A1 | C-A2 | C-A1 | C-A2 | B70 | B95 |
| H8-620 | O | C-A1 | C-A2 | C-A1 | C-A2 | B70 | B96 |
| H8-621 | O | C-A1 | C-A2 | C-A1 | C-A2 | B70 | B97 |
| H8-622 | O | C-A1 | C-A2 | C-A1 | C-A2 | B70 | B106 |
| H8-623 | O | C-A1 | C-A2 | C-A1 | C-A2 | B70 | B107 |
| H8-624 | O | C-A1 | C-A2 | C-A1 | C-A2 | B70 | B114 |
| H8-625 | O | C-A1 | C-A2 | C-A1 | C-A2 | B70 | B115 |
| H8-626 | O | C-A1 | C-A2 | C-A1 | C-A2 | B70 | B116 |
| H8-627 | O | C-A1 | C-A2 | C-A1 | C-A2 | B70 | B117 |
| H8-628 | O | C-A1 | C-A2 | C-A1 | C-A2 | B70 | B118 |
| H8-629 | O | C-A1 | C-A2 | C-A1 | C-A2 | B70 | B119 |
| H8-630 | O | C-A1 | C-A2 | C-A1 | C-A2 | B70 | B120 |
| H8-631 | O | C-A1 | C-A2 | C-A1 | C-A2 | B70 | B121 |
| H8-632 | O | C-A1 | C-A2 | C-A1 | C-A2 | B70 | B122 |
| H8-633 | O | C-A1 | C-A2 | C-A1 | C-A2 | B70 | B125 |
| H8-634 | O | C-A1 | C-A2 | C-A1 | C-A2 | B70 | B126 |
| H8-635 | O | C-A1 | C-A2 | C-A1 | C-A2 | B70 | B127 |
| H8-636 | O | C-A1 | C-A2 | C-A1 | C-A2 | B70 | B128 |
| H8-637 | O | C-A1 | C-A2 | C-A1 | C-A2 | B70 | B129 |
| H8-638 | O | C-A1 | C-A2 | C-A1 | C-A2 | B70 | B130 |
| H8-639 | O | C-A1 | C-A2 | C-A1 | C-A2 | B70 | B131 |
| H8-640 | O | C-A1 | C-A2 | C-A1 | C-A2 | B70 | B132 |
| H8-641 | O | C-A1 | C-A2 | C-A1 | C-A2 | B70 | B133 |
| H8-642 | O | C-A1 | C-A2 | C-A1 | C-A2 | B70 | B134 |
| H8-643 | O | C-A1 | C-A2 | C-A1 | C-A2 | B70 | B135 |
| H8-644 | O | C-A1 | C-A2 | C-A1 | C-A2 | B70 | B136 |
| H8-645 | O | C-A1 | C-A2 | C-A1 | C-A2 | B70 | B137 |
| H8-646 | O | C-A1 | C-A2 | C-A1 | C-A2 | B70 | B138 |
| H8-647 | O | C-A1 | C-A2 | C-A1 | C-A2 | B70 | B139 |
| H8-648 | O | C-A1 | C-A2 | C-A1 | C-A2 | B70 | B140 |
| H8-655 | O | C-A1 | C-A3 | C-A1 | C-A3 | B70 | B1 |
| H8-656 | O | C-A1 | C-A3 | C-A1 | C-A3 | B70 | B6 |
| H8-657 | O | C-A1 | C-A3 | C-A1 | C-A3 | B70 | B20 |
| H8-658 | O | C-A1 | C-A3 | C-A1 | C-A3 | B70 | B21 |
| H8-659 | O | C-A1 | C-A3 | C-A1 | C-A3 | B70 | B22 |
| H8-660 | O | C-A1 | C-A3 | C-A1 | C-A3 | B70 | B32 |
| H8-661 | O | C-A1 | C-A3 | C-A1 | C-A3 | B70 | B33 |
| H8-662 | O | C-A1 | C-A3 | C-A1 | C-A3 | B70 | B34 |
| H8-663 | O | C-A1 | C-A3 | C-A1 | C-A3 | B70 | B68 |
| H8-664 | O | C-A1 | C-A3 | C-A1 | C-A3 | B70 | B69 |
| H8-665 | O | C-A1 | C-A3 | C-A1 | C-A3 | B70 | B70 |
| H8-666 | O | C-A1 | C-A3 | C-A1 | C-A3 | B70 | B71 |
| H8-667 | O | C-A1 | C-A3 | C-A1 | C-A3 | B70 | B81 |
| H8-668 | O | C-A1 | C-A3 | C-A1 | C-A3 | B70 | B90 |
| H8-669 | O | C-A1 | C-A3 | C-A1 | C-A3 | B70 | B91 |
| H8-670 | O | C-A1 | C-A3 | C-A1 | C-A3 | B70 | B92 |
| H8-671 | O | C-A1 | C-A3 | C-A1 | C-A3 | B70 | B93 |
| H8-672 | O | C-A1 | C-A3 | C-A1 | C-A3 | B70 | B94 |
| H8-673 | O | C-A1 | C-A3 | C-A1 | C-A3 | B70 | B95 |
| H8-674 | O | C-A1 | C-A3 | C-A1 | C-A3 | B70 | B96 |
| H8-675 | O | C-A1 | C-A3 | C-A1 | C-A3 | B70 | B97 |
| H8-676 | O | C-A1 | C-A3 | C-A1 | C-A3 | B70 | B106 |
| H8-677 | O | C-A1 | C-A3 | C-A1 | C-A3 | B70 | B107 |
| H8-678 | O | C-A1 | C-A3 | C-A1 | C-A3 | B70 | B114 |
| H8-679 | O | C-A1 | C-A3 | C-A1 | C-A3 | B70 | B115 |
| H8-680 | O | C-A1 | C-A3 | C-A1 | C-A3 | B70 | B116 |
| H8-681 | O | C-A1 | C-A3 | C-A1 | C-A3 | B70 | B117 |
| H8-682 | O | C-A1 | C-A3 | C-A1 | C-A3 | B70 | B118 |
| H8-683 | O | C-A1 | C-A3 | C-A1 | C-A3 | B70 | B119 |
| H8-684 | O | C-A1 | C-A3 | C-A1 | C-A3 | B70 | B120 |
| H8-685 | O | C-A1 | C-A3 | C-A1 | C-A3 | B70 | B121 |
| H8-686 | O | C-A1 | C-A3 | C-A1 | C-A3 | B70 | B122 |
| H8-687 | O | C-A1 | C-A3 | C-A1 | C-A3 | B70 | B125 |
| H8-688 | O | C-A1 | C-A3 | C-A1 | C-A3 | B70 | B126 |
| H8-689 | O | C-A1 | C-A3 | C-A1 | C-A3 | B70 | B127 |
| H8-690 | O | C-A1 | C-A3 | C-A1 | C-A3 | B70 | B128 |
| H8-691 | O | C-A1 | C-A3 | C-A1 | C-A3 | B70 | B129 |
| H8-692 | O | C-A1 | C-A3 | C-A1 | C-A3 | B70 | B130 |
| H8-693 | O | C-A1 | C-A3 | C-A1 | C-A3 | B70 | B131 |
| H8-694 | O | C-A1 | C-A3 | C-A1 | C-A3 | B70 | B132 |
| H8-695 | O | C-A1 | C-A3 | C-A1 | C-A3 | B70 | B133 |
| H8-696 | O | C-A1 | C-A3 | C-A1 | C-A3 | B70 | B134 |
| H8-697 | O | C-A1 | C-A3 | C-A1 | C-A3 | B70 | B135 |
| H8-698 | O | C-A1 | C-A3 | C-A1 | C-A3 | B70 | B136 |
| H8-699 | O | C-A1 | C-A3 | C-A1 | C-A3 | B70 | B137 |
| H8-700 | O | C-A1 | C-A3 | C-A1 | C-A3 | B70 | B138 |
| H8-701 | O | C-A1 | C-A3 | C-A1 | C-A3 | B70 | B139 |
| H8-702 | O | C-A1 | C-A3 | C-A1 | C-A3 | B70 | B140 |
| H8-709 | O | C-A3 | C-A1 | C-A3 | C-A1 | B70 | B1 |
| H8-710 | O | C-A3 | C-A1 | C-A3 | C-A1 | B70 | B6 |
| H8-711 | O | C-A3 | C-A1 | C-A3 | C-A1 | B70 | B20 |
| H8-712 | O | C-A3 | C-A1 | C-A3 | C-A1 | B70 | B21 |
| H8-713 | O | C-A3 | C-A1 | C-A3 | C-A1 | B70 | B22 |
| H8-714 | O | C-A3 | C-A1 | C-A3 | C-A1 | B70 | B32 |
| H8-715 | O | C-A3 | C-A1 | C-A3 | C-A1 | B70 | B33 |
| H8-716 | O | C-A3 | C-A1 | C-A3 | C-A1 | B70 | B34 |
| H8-717 | O | C-A3 | C-A1 | C-A3 | C-A1 | B70 | B68 |
| H8-718 | O | C-A3 | C-A1 | C-A3 | C-A1 | B70 | B69 |
| H8-719 | O | C-A3 | C-A1 | C-A3 | C-A1 | B70 | B70 |
| H8-720 | O | C-A3 | C-A1 | C-A3 | C-A1 | B70 | B71 |
| H8-721 | O | C-A3 | C-A1 | C-A3 | C-A1 | B70 | B81 |
| H8-722 | O | C-A3 | C-A1 | C-A3 | C-A1 | B70 | B90 |
| H8-723 | O | C-A3 | C-A1 | C-A3 | C-A1 | B70 | B91 |
| H8-724 | O | C-A3 | C-A1 | C-A3 | C-A1 | B70 | B92 |
| H8-725 | O | C-A3 | C-A1 | C-A3 | C-A1 | B70 | B93 |
| H8-726 | O | C-A3 | C-A1 | C-A3 | C-A1 | B70 | B94 |
| H8-727 | O | C-A3 | C-A1 | C-A3 | C-A1 | B70 | B95 |
| H8-728 | O | C-A3 | C-A1 | C-A3 | C-A1 | B70 | B96 |
| H8-729 | O | C-A3 | C-A1 | C-A3 | C-A1 | B70 | B97 |
| H8-730 | O | C-A3 | C-A1 | C-A3 | C-A1 | B70 | B106 |
| H8-731 | O | C-A3 | C-A1 | C-A3 | C-A1 | B70 | B107 |
| H8-732 | O | C-A3 | C-A1 | C-A3 | C-A1 | B70 | B114 |
| H8-733 | O | C-A3 | C-A1 | C-A3 | C-A1 | B70 | B115 |
| H8-734 | O | C-A3 | C-A1 | C-A3 | C-A1 | B70 | B116 |
| H8-735 | O | C-A3 | C-A1 | C-A3 | C-A1 | B70 | B117 |
| H8-736 | O | C-A3 | C-A1 | C-A3 | C-A1 | B70 | B118 |
| H8-737 | O | C-A3 | C-A1 | C-A3 | C-A1 | B70 | B119 |
| H8-738 | O | C-A3 | C-A1 | C-A3 | C-A1 | B70 | B120 |
| H8-739 | O | C-A3 | C-A1 | C-A3 | C-A1 | B70 | B121 |
| H8-740 | O | C-A3 | C-A1 | C-A3 | C-A1 | B70 | B122 |
| H8-741 | O | C-A3 | C-A1 | C-A3 | C-A1 | B70 | B125 |
| H8-742 | O | C-A3 | C-A1 | C-A3 | C-A1 | B70 | B126 |
| H8-743 | O | C-A3 | C-A1 | C-A3 | C-A1 | B70 | B127 |
| H8-744 | O | C-A3 | C-A1 | C-A3 | C-A1 | B70 | B128 |
| H8-745 | O | C-A3 | C-A1 | C-A3 | C-A1 | B70 | B129 |
| H8-746 | O | C-A3 | C-A1 | C-A3 | C-A1 | B70 | B130 |
| H8-747 | O | C-A3 | C-A1 | C-A3 | C-A1 | B70 | B131 |
| H8-748 | O | C-A3 | C-A1 | C-A3 | C-A1 | B70 | B132 |
| H8-749 | O | C-A3 | C-A1 | C-A3 | C-A1 | B70 | B133 |
| H8-750 | O | C-A3 | C-A1 | C-A3 | C-A1 | B70 | B134 |
| H8-751 | O | C-A3 | C-A1 | C-A3 | C-A1 | B70 | B135 |
| H8-752 | O | C-A3 | C-A1 | C-A3 | C-A1 | B70 | B136 |
| H8-753 | O | C-A3 | C-A1 | C-A3 | C-A1 | B70 | B137 |
| H8-754 | O | C-A3 | C-A1 | C-A3 | C-A1 | B70 | B138 |
| H8-755 | O | C-A3 | C-A1 | C-A3 | C-A1 | B70 | B139 |
| H8-756 | O | C-A3 | C-A1 | C-A3 | C-A1 | B70 | B140 |
| H8-763 | O | C-A1 | C-A1 | C-A3 | C-A1 | B70 | B1 |
| H8-764 | O | C-A1 | C-A1 | C-A3 | C-A1 | B70 | B6 |
| H8-765 | O | C-A1 | C-A1 | C-A3 | C-A1 | B70 | B20 |
| H8-766 | O | C-A1 | C-A1 | C-A3 | C-A1 | B70 | B21 |
| H8-767 | O | C-A1 | C-A1 | C-A3 | C-A1 | B70 | B22 |
| H8-768 | O | C-A1 | C-A1 | C-A3 | C-A1 | B70 | B32 |
| H8-769 | O | C-A1 | C-A1 | C-A3 | C-A1 | B70 | B33 |
| H8-770 | O | C-A1 | C-A1 | C-A3 | C-A1 | B70 | B34 |
| H8-771 | O | C-A1 | C-A1 | C-A3 | C-A1 | B70 | B68 |
| H8-772 | O | C-A1 | C-A1 | C-A3 | C-A1 | B70 | B69 |
| H8-773 | O | C-A1 | C-A1 | C-A3 | C-A1 | B70 | B70 |
| H8-774 | O | C-A1 | C-A1 | C-A3 | C-A1 | B70 | B71 |
| H8-775 | O | C-A1 | C-A1 | C-A3 | C-A1 | B70 | B81 |
| H8-776 | O | C-A1 | C-A1 | C-A3 | C-A1 | B70 | B90 |
| H8-777 | O | C-A1 | C-A1 | C-A3 | C-A1 | B70 | B91 |
| H8-778 | O | C-A1 | C-A1 | C-A3 | C-A1 | B70 | B92 |
| H8-779 | O | C-A1 | C-A1 | C-A3 | C-A1 | B70 | B93 |
| H8-780 | O | C-A1 | C-A1 | C-A3 | C-A1 | B70 | B94 |
| H8-781 | O | C-A1 | C-A1 | C-A3 | C-A1 | B70 | B95 |
| H8-782 | O | C-A1 | C-A1 | C-A3 | C-A1 | B70 | B96 |
| H8-783 | O | C-A1 | C-A1 | C-A3 | C-A1 | B70 | B97 |
| H8-784 | O | C-A1 | C-A1 | C-A3 | C-A1 | B70 | B106 |
| H8-785 | O | C-A1 | C-A1 | C-A3 | C-A1 | B70 | B107 |
| H8-786 | O | C-A1 | C-A1 | C-A3 | C-A1 | B70 | B114 |
| H8-787 | O | C-A1 | C-A1 | C-A3 | C-A1 | B70 | B115 |
| H8-788 | O | C-A1 | C-A1 | C-A3 | C-A1 | B70 | B116 |
| H8-789 | O | C-A1 | C-A1 | C-A3 | C-A1 | B70 | B117 |
| H8-790 | O | C-A1 | C-A1 | C-A3 | C-A1 | B70 | B118 |
| H8-791 | O | C-A1 | C-A1 | C-A3 | C-A1 | B70 | B119 |
| H8-792 | O | C-A1 | C-A1 | C-A3 | C-A1 | B70 | B120 |
| H8-793 | O | C-A1 | C-A1 | C-A3 | C-A1 | B70 | B121 |
| H8-794 | O | C-A1 | C-A1 | C-A3 | C-A1 | B70 | B122 |
| H8-795 | O | C-A1 | C-A1 | C-A3 | C-A1 | B70 | B125 |
| H8-796 | O | C-A1 | C-A1 | C-A3 | C-A1 | B70 | B126 |
| H8-797 | O | C-A1 | C-A1 | C-A3 | C-A1 | B70 | B127 |
| H8-798 | O | C-A1 | C-A1 | C-A3 | C-A1 | B70 | B128 |
| H8-799 | O | C-A1 | C-A1 | C-A3 | C-A1 | B70 | B129 |
| H8-800 | O | C-A1 | C-A1 | C-A3 | C-A1 | B70 | B130 |
| H8-801 | O | C-A1 | C-A1 | C-A3 | C-A1 | B70 | B131 |
| H8-802 | O | C-A1 | C-A1 | C-A3 | C-A1 | B70 | B132 |
| H8-803 | O | C-A1 | C-A1 | C-A3 | C-A1 | B70 | B133 |
| H8-804 | O | C-A1 | C-A1 | C-A3 | C-A1 | B70 | B134 |
| H8-805 | O | C-A1 | C-A1 | C-A3 | C-A1 | B70 | B135 |
| H8-806 | O | C-A1 | C-A1 | C-A3 | C-A1 | B70 | B136 |
| H8-807 | O | C-A1 | C-A1 | C-A3 | C-A1 | B70 | B137 |
| H8-808 | O | C-A1 | C-A1 | C-A3 | C-A1 | B70 | B138 |
| H8-809 | O | C-A1 | C-A1 | C-A3 | C-A1 | B70 | B139 |
| H8-810 | O | C-A1 | C-A1 | C-A3 | C-A1 | B70 | B140 |
| H8-817 | O | C-A1 | C-A1 | C-A1 | C-A1 | B22 | B1 |
| H8-818 | O | C-A1 | C-A1 | C-A1 | C-A1 | B22 | B6 |
| H8-819 | O | C-A1 | C-A1 | C-A1 | C-A1 | B22 | B20 |
| H8-820 | O | C-A1 | C-A1 | C-A1 | C-A1 | B22 | B21 |
| H8-821 | O | C-A1 | C-A1 | C-A1 | C-A1 | B22 | B22 |
| H8-822 | O | C-A1 | C-A1 | C-A1 | C-A1 | B22 | B32 |
| H8-823 | O | C-A1 | C-A1 | C-A1 | C-A1 | B22 | B33 |
| H8-824 | O | C-A1 | C-A1 | C-A1 | C-A1 | B22 | B34 |
| H8-825 | O | C-A1 | C-A1 | C-A1 | C-A1 | B22 | B68 |
| H8-826 | O | C-A1 | C-A1 | C-A1 | C-A1 | B22 | B69 |
| H8-827 | O | C-A1 | C-A1 | C-A1 | C-A1 | B22 | B70 |
| H8-828 | O | C-A1 | C-A1 | C-A1 | C-A1 | B22 | B71 |
| H8-829 | O | C-A1 | C-A1 | C-A1 | C-A1 | B22 | B81 |
| H8-830 | O | C-A1 | C-A1 | C-A1 | C-A1 | B22 | B90 |
| H8-831 | O | C-A1 | C-A1 | C-A1 | C-A1 | B22 | B91 |
| H8-832 | O | C-A1 | C-A1 | C-A1 | C-A1 | B22 | B92 |
| H8-833 | O | C-A1 | C-A1 | C-A1 | C-A1 | B22 | B93 |
| H8-834 | O | C-A1 | C-A1 | C-A1 | C-A1 | B22 | B94 |
| H8-835 | O | C-A1 | C-A1 | C-A1 | C-A1 | B22 | B95 |
| H8-836 | O | C-A1 | C-A1 | C-A1 | C-A1 | B22 | B96 |
| H8-837 | O | C-A1 | C-A1 | C-A1 | C-A1 | B22 | B97 |
| H8-838 | O | C-A1 | C-A1 | C-A1 | C-A1 | B22 | B106 |
| H8-839 | O | C-A1 | C-A1 | C-A1 | C-A1 | B22 | B107 |
| H8-840 | O | C-A1 | C-A1 | C-A1 | C-A1 | B22 | B114 |
| H8-841 | O | C-A1 | C-A1 | C-A1 | C-A1 | B22 | B115 |
| H8-842 | O | C-A1 | C-A1 | C-A1 | C-A1 | B22 | B116 |
| H8-843 | O | C-A1 | C-A1 | C-A1 | C-A1 | B22 | B117 |
| H8-844 | O | C-A1 | C-A1 | C-A1 | C-A1 | B22 | B118 |
| H8-845 | O | C-A1 | C-A1 | C-A1 | C-A1 | B22 | B119 |
| H8-846 | O | C-A1 | C-A1 | C-A1 | C-A1 | B22 | B120 |
| H8-847 | O | C-A1 | C-A1 | C-A1 | C-A1 | B22 | B121 |
| H8-848 | O | C-A1 | C-A1 | C-A1 | C-A1 | B22 | B122 |
| H8-849 | O | C-A1 | C-A1 | C-A1 | C-A1 | B22 | B125 |
| H8-850 | O | C-A1 | C-A1 | C-A1 | C-A1 | B22 | B126 |
| H8-851 | O | C-A1 | C-A1 | C-A1 | C-A1 | B22 | B127 |
| H8-852 | O | C-A1 | C-A1 | C-A1 | C-A1 | B22 | B128 |
| H8-853 | O | C-A1 | C-A1 | C-A1 | C-A1 | B22 | B129 |
| H8-854 | O | C-A1 | C-A1 | C-A1 | C-A1 | B22 | B130 |
| H8-855 | O | C-A1 | C-A1 | C-A1 | C-A1 | B22 | B131 |
| H8-856 | O | C-A1 | C-A1 | C-A1 | C-A1 | B22 | B132 |
| H8-857 | O | C-A1 | C-A1 | C-A1 | C-A1 | B22 | B133 |
| H8-858 | O | C-A1 | C-A1 | C-A1 | C-A1 | B22 | B134 |
| H8-859 | O | C-A1 | C-A1 | C-A1 | C-A1 | B22 | B135 |
| H8-860 | O | C-A1 | C-A1 | C-A1 | C-A1 | B22 | B136 |
| H8-861 | O | C-A1 | C-A1 | C-A1 | C-A1 | B22 | B137 |
| H8-862 | O | C-A1 | C-A1 | C-A1 | C-A1 | B22 | B138 |
| H8-863 | O | C-A1 | C-A1 | C-A1 | C-A1 | B22 | B139 |
| H8-864 | O | C-A1 | C-A1 | C-A1 | C-A1 | B22 | B140 |
| H8-871 | O | C-A1 | C-A2 | C-A1 | C-A2 | B22 | B1 |
| H8-872 | O | C-A1 | C-A2 | C-A1 | C-A2 | B22 | B6 |
| H8-873 | O | C-A1 | C-A2 | C-A1 | C-A2 | B22 | B20 |
| H8-874 | O | C-A1 | C-A2 | C-A1 | C-A2 | B22 | B21 |
| H8-875 | O | C-A1 | C-A2 | C-A1 | C-A2 | B22 | B22 |
| H8-876 | O | C-A1 | C-A2 | C-A1 | C-A2 | B22 | B32 |
| H8-877 | O | C-A1 | C-A2 | C-A1 | C-A2 | B22 | B33 |
| H8-878 | O | C-A1 | C-A2 | C-A1 | C-A2 | B22 | B34 |
| H8-879 | O | C-A1 | C-A2 | C-A1 | C-A2 | B22 | B68 |
| H8-880 | O | C-A1 | C-A2 | C-A1 | C-A2 | B22 | B69 |
| H8-881 | O | C-A1 | C-A2 | C-A1 | C-A2 | B22 | B70 |
| H8-882 | O | C-A1 | C-A2 | C-A1 | C-A2 | B22 | B71 |
| H8-883 | O | C-A1 | C-A2 | C-A1 | C-A2 | B22 | B81 |
| H8-884 | O | C-A1 | C-A2 | C-A1 | C-A2 | B22 | B90 |
| H8-885 | O | C-A1 | C-A2 | C-A1 | C-A2 | B22 | B91 |
| H8-886 | O | C-A1 | C-A2 | C-A1 | C-A2 | B22 | B92 |
| H8-887 | O | C-A1 | C-A2 | C-A1 | C-A2 | B22 | B93 |
| H8-888 | O | C-A1 | C-A2 | C-A1 | C-A2 | B22 | B94 |
| H8-889 | O | C-A1 | C-A2 | C-A1 | C-A2 | B22 | B95 |
| H8-890 | O | C-A1 | C-A2 | C-A1 | C-A2 | B22 | B96 |
| H8-891 | O | C-A1 | C-A2 | C-A1 | C-A2 | B22 | B97 |
| H8-892 | O | C-A1 | C-A2 | C-A1 | C-A2 | B22 | B106 |
| H8-893 | O | C-A1 | C-A2 | C-A1 | C-A2 | B22 | B107 |
| H8-894 | O | C-A1 | C-A2 | C-A1 | C-A2 | B22 | B114 |
| H8-895 | O | C-A1 | C-A2 | C-A1 | C-A2 | B22 | B115 |
| H8-896 | O | C-A1 | C-A2 | C-A1 | C-A2 | B22 | B116 |
| H8-897 | O | C-A1 | C-A2 | C-A1 | C-A2 | B22 | B117 |
| H8-898 | O | C-A1 | C-A2 | C-A1 | C-A2 | B22 | B118 |
| H8-899 | O | C-A1 | C-A2 | C-A1 | C-A2 | B22 | B119 |
| H8-900 | O | C-A1 | C-A2 | C-A1 | C-A2 | B22 | B120 |
| H8-901 | O | C-A1 | C-A2 | C-A1 | C-A2 | B22 | B121 |
| H8-902 | O | C-A1 | C-A2 | C-A1 | C-A2 | B22 | B122 |
| H8-903 | O | C-A1 | C-A2 | C-A1 | C-A2 | B22 | B125 |
| H8-904 | O | C-A1 | C-A2 | C-A1 | C-A2 | B22 | B126 |
| H8-905 | O | C-A1 | C-A2 | C-A1 | C-A2 | B22 | B127 |
| H8-906 | O | C-A1 | C-A2 | C-A1 | C-A2 | B22 | B128 |
| H8-907 | O | C-A1 | C-A2 | C-A1 | C-A2 | B22 | B129 |
| H8-908 | O | C-A1 | C-A2 | C-A1 | C-A2 | B22 | B130 |
| H8-909 | O | C-A1 | C-A2 | C-A1 | C-A2 | B22 | B131 |
| H8-910 | O | C-A1 | C-A2 | C-A1 | C-A2 | B22 | B132 |
| H8-911 | O | C-A1 | C-A2 | C-A1 | C-A2 | B22 | B133 |
| H8-912 | O | C-A1 | C-A2 | C-A1 | C-A2 | B22 | B134 |
| H8-913 | O | C-A1 | C-A2 | C-A1 | C-A2 | B22 | B135 |
| H8-914 | O | C-A1 | C-A2 | C-A1 | C-A2 | B22 | B136 |
| H8-915 | O | C-A1 | C-A2 | C-A1 | C-A2 | B22 | B137 |
| H8-916 | O | C-A1 | C-A2 | C-A1 | C-A2 | B22 | B138 |
| H8-917 | O | C-A1 | C-A2 | C-A1 | C-A2 | B22 | B139 |
| H8-918 | O | C-A1 | C-A2 | C-A1 | C-A2 | B22 | B140 |
| H8-925 | O | C-A1 | C-A3 | C-A1 | C-A3 | B22 | B1 |
| H8-926 | O | C-A1 | C-A3 | C-A1 | C-A3 | B22 | B6 |
| H8-927 | O | C-A1 | C-A3 | C-A1 | C-A3 | B22 | B20 |
| H8-928 | O | C-A1 | C-A3 | C-A1 | C-A3 | B22 | B21 |
| H8-929 | O | C-A1 | C-A3 | C-A1 | C-A3 | B22 | B22 |
| H8-930 | O | C-A1 | C-A3 | C-A1 | C-A3 | B22 | B32 |
| H8-931 | O | C-A1 | C-A3 | C-A1 | C-A3 | B22 | B33 |
| H8-932 | O | C-A1 | C-A3 | C-A1 | C-A3 | B22 | B34 |
| H8-933 | O | C-A1 | C-A3 | C-A1 | C-A3 | B22 | B68 |
| H8-934 | O | C-A1 | C-A3 | C-A1 | C-A3 | B22 | B69 |
| H8-935 | O | C-A1 | C-A3 | C-A1 | C-A3 | B22 | B70 |
| H8-936 | O | C-A1 | C-A3 | C-A1 | C-A3 | B22 | B71 |
| H8-937 | O | C-A1 | C-A3 | C-A1 | C-A3 | B22 | B81 |
| H8-938 | O | C-A1 | C-A3 | C-A1 | C-A3 | B22 | B90 |
| H8-939 | O | C-A1 | C-A3 | C-A1 | C-A3 | B22 | B91 |
| H8-940 | O | C-A1 | C-A3 | C-A1 | C-A3 | B22 | B92 |
| H8-941 | O | C-A1 | C-A3 | C-A1 | C-A3 | B22 | B93 |
| H8-942 | O | C-A1 | C-A3 | C-A1 | C-A3 | B22 | B94 |
| H8-943 | O | C-A1 | C-A3 | C-A1 | C-A3 | B22 | B95 |
| H8-944 | O | C-A1 | C-A3 | C-A1 | C-A3 | B22 | B96 |
| H8-945 | O | C-A1 | C-A3 | C-A1 | C-A3 | B22 | B97 |
| H8-946 | O | C-A1 | C-A3 | C-A1 | C-A3 | B22 | B106 |
| H8-947 | O | C-A1 | C-A3 | C-A1 | C-A3 | B22 | B107 |
| H8-948 | O | C-A1 | C-A3 | C-A1 | C-A3 | B22 | B114 |
| H8-949 | O | C-A1 | C-A3 | C-A1 | C-A3 | B22 | B115 |
| H8-950 | O | C-A1 | C-A3 | C-A1 | C-A3 | B22 | B116 |
| H8-951 | O | C-A1 | C-A3 | C-A1 | C-A3 | B22 | B117 |
| H8-952 | O | C-A1 | C-A3 | C-A1 | C-A3 | B22 | B118 |
| H8-953 | O | C-A1 | C-A3 | C-A1 | C-A3 | B22 | B119 |
| H8-954 | O | C-A1 | C-A3 | C-A1 | C-A3 | B22 | B120 |
| H8-955 | O | C-A1 | C-A3 | C-A1 | C-A3 | B22 | B121 |
| H8-956 | O | C-A1 | C-A3 | C-A1 | C-A3 | B22 | B122 |
| H8-957 | O | C-A1 | C-A3 | C-A1 | C-A3 | B22 | B125 |
| H8-958 | O | C-A1 | C-A3 | C-A1 | C-A3 | B22 | B126 |
| H8-959 | O | C-A1 | C-A3 | C-A1 | C-A3 | B22 | B127 |
| H8-960 | O | C-A1 | C-A3 | C-A1 | C-A3 | B22 | B128 |
| H8-961 | O | C-A1 | C-A3 | C-A1 | C-A3 | B22 | B129 |
| H8-962 | O | C-A1 | C-A3 | C-A1 | C-A3 | B22 | B130 |
| H8-963 | O | C-A1 | C-A3 | C-A1 | C-A3 | B22 | B131 |
| H8-964 | O | C-A1 | C-A3 | C-A1 | C-A3 | B22 | B132 |
| H8-965 | O | C-A1 | C-A3 | C-A1 | C-A3 | B22 | B133 |
| H8-966 | O | C-A1 | C-A3 | C-A1 | C-A3 | B22 | B134 |
| H8-967 | O | C-A1 | C-A3 | C-A1 | C-A3 | B22 | B135 |
| H8-968 | O | C-A1 | C-A3 | C-A1 | C-A3 | B22 | B136 |
| H8-969 | O | C-A1 | C-A3 | C-A1 | C-A3 | B22 | B137 |
| H8-970 | O | C-A1 | C-A3 | C-A1 | C-A3 | B22 | B138 |
| H8-971 | O | C-A1 | C-A3 | C-A1 | C-A3 | B22 | B139 |
| H8-972 | O | C-A1 | C-A3 | C-A1 | C-A3 | B22 | B140 |
| H8-979 | O | C-A3 | C-A1 | C-A3 | C-A1 | B22 | B1 |
| H8-980 | O | C-A3 | C-A1 | C-A3 | C-A1 | B22 | B6 |
| H8-981 | O | C-A3 | C-A1 | C-A3 | C-A1 | B22 | B20 |
| H8-982 | O | C-A3 | C-A1 | C-A3 | C-A1 | B22 | B21 |
| H8-983 | O | C-A3 | C-A1 | C-A3 | C-A1 | B22 | B22 |
| H8-984 | O | C-A3 | C-A1 | C-A3 | C-A1 | B22 | B32 |
| H8-985 | O | C-A3 | C-A1 | C-A3 | C-A1 | B22 | B33 |
| H8-986 | O | C-A3 | C-A1 | C-A3 | C-A1 | B22 | B34 |
| H8-987 | O | C-A3 | C-A1 | C-A3 | C-A1 | B22 | B68 |
| H8-988 | O | C-A3 | C-A1 | C-A3 | C-A1 | B22 | B69 |
| H8-989 | O | C-A3 | C-A1 | C-A3 | C-A1 | B22 | B70 |
| H8-990 | O | C-A3 | C-A1 | C-A3 | C-A1 | B22 | B71 |
| H8-991 | O | C-A3 | C-A1 | C-A3 | C-A1 | B22 | B81 |
| H8-992 | O | C-A3 | C-A1 | C-A3 | C-A1 | B22 | B90 |
| H8-993 | O | C-A3 | C-A1 | C-A3 | C-A1 | B22 | B91 |
| H8-994 | O | C-A3 | C-A1 | C-A3 | C-A1 | B22 | B92 |
| H8-995 | O | C-A3 | C-A1 | C-A3 | C-A1 | B22 | B93 |
| H8-996 | O | C-A3 | C-A1 | C-A3 | C-A1 | B22 | B94 |
| H8-997 | O | C-A3 | C-A1 | C-A3 | C-A1 | B22 | B95 |
| H8-998 | O | C-A3 | C-A1 | C-A3 | C-A1 | B22 | B96 |
| H8-999 | O | C-A3 | C-A1 | C-A3 | C-A1 | B22 | B97 |
| H8-1000 | O | C-A3 | C-A1 | C-A3 | C-A1 | B22 | B106 |
| H8-1001 | O | C-A3 | C-A1 | C-A3 | C-A1 | B22 | B107 |
| H8-1002 | O | C-A3 | C-A1 | C-A3 | C-A1 | B22 | B114 |
| H8-1003 | O | C-A3 | C-A1 | C-A3 | C-A1 | B22 | B115 |
| H8-1004 | O | C-A3 | C-A1 | C-A3 | C-A1 | B22 | B116 |
| H8-1005 | O | C-A3 | C-A1 | C-A3 | C-A1 | B22 | B117 |
| H8-1006 | O | C-A3 | C-A1 | C-A3 | C-A1 | B22 | B118 |
| H8-1007 | O | C-A3 | C-A1 | C-A3 | C-A1 | B22 | B119 |
| H8-1008 | O | C-A3 | C-A1 | C-A3 | C-A1 | B22 | B120 |
| H8-1009 | O | C-A3 | C-A1 | C-A3 | C-A1 | B22 | B121 |
| H8-1010 | O | C-A3 | C-A1 | C-A3 | C-A1 | B22 | B122 |
| H8-1011 | O | C-A3 | C-A1 | C-A3 | C-A1 | B22 | B125 |
| H8-1012 | O | C-A3 | C-A1 | C-A3 | C-A1 | B22 | B126 |
| H8-1013 | O | C-A3 | C-A1 | C-A3 | C-A1 | B22 | B127 |
| H8-1014 | O | C-A3 | C-A1 | C-A3 | C-A1 | B22 | B128 |
| H8-1015 | O | C-A3 | C-A1 | C-A3 | C-A1 | B22 | B129 |
| H8-1016 | O | C-A3 | C-A1 | C-A3 | C-A1 | B22 | B130 |
| H8-1017 | O | C-A3 | C-A1 | C-A3 | C-A1 | B22 | B131 |
| H8-1018 | O | C-A3 | C-A1 | C-A3 | C-A1 | B22 | B132 |
| H8-1019 | O | C-A3 | C-A1 | C-A3 | C-A1 | B22 | B133 |
| H8-1020 | O | C-A3 | C-A1 | C-A3 | C-A1 | B22 | B134 |
| H8-1021 | O | C-A3 | C-A1 | C-A3 | C-A1 | B22 | B135 |
| H8-1022 | O | C-A3 | C-A1 | C-A3 | C-A1 | B22 | B136 |
| H8-1023 | O | C-A3 | C-A1 | C-A3 | C-A1 | B22 | B137 |
| H8-1024 | O | C-A3 | C-A1 | C-A3 | C-A1 | B22 | B138 |
| H8-1025 | O | C-A3 | C-A1 | C-A3 | C-A1 | B22 | B139 |
| H8-1026 | O | C-A3 | C-A1 | C-A3 | C-A1 | B22 | B140 |
| H8-1033 | O | C-A1 | C-A1 | C-A3 | C-A1 | B22 | B1 |
| H8-1034 | O | C-A1 | C-A1 | C-A3 | C-A1 | B22 | B6 |
| H8-1035 | O | C-A1 | C-A1 | C-A3 | C-A1 | B22 | B20 |
| H8-1036 | O | C-A1 | C-A1 | C-A3 | C-A1 | B22 | B21 |
| H8-1037 | O | C-A1 | C-A1 | C-A3 | C-A1 | B22 | B22 |
| H8-1038 | O | C-A1 | C-A1 | C-A3 | C-A1 | B22 | B32 |
| H8-1039 | O | C-A1 | C-A1 | C-A3 | C-A1 | B22 | B33 |
| H8-1040 | O | C-A1 | C-A1 | C-A3 | C-A1 | B22 | B34 |
| H8-1041 | O | C-A1 | C-A1 | C-A3 | C-A1 | B22 | B68 |
| H8-1042 | O | C-A1 | C-A1 | C-A3 | C-A1 | B22 | B69 |
| H8-1043 | O | C-A1 | C-A1 | C-A3 | C-A1 | B22 | B70 |
| H8-1044 | O | C-A1 | C-A1 | C-A3 | C-A1 | B22 | B71 |
| H8-1045 | O | C-A1 | C-A1 | C-A3 | C-A1 | B22 | B81 |
| H8-1046 | O | C-A1 | C-A1 | C-A3 | C-A1 | B22 | B90 |
| H8-1047 | O | C-A1 | C-A1 | C-A3 | C-A1 | B22 | B91 |
| H8-1048 | O | C-A1 | C-A1 | C-A3 | C-A1 | B22 | B92 |
| H8-1049 | O | C-A1 | C-A1 | C-A3 | C-A1 | B22 | B93 |
| H8-1050 | O | C-A1 | C-A1 | C-A3 | C-A1 | B22 | B94 |
| H8-1051 | O | C-A1 | C-A1 | C-A3 | C-A1 | B22 | B95 |
| H8-1052 | O | C-A1 | C-A1 | C-A3 | C-A1 | B22 | B96 |
| H8-1053 | O | C-A1 | C-A1 | C-A3 | C-A1 | B22 | B97 |
| H8-1054 | O | C-A1 | C-A1 | C-A3 | C-A1 | B22 | B106 |
| H8-1055 | O | C-A1 | C-A1 | C-A3 | C-A1 | B22 | B107 |
| H8-1056 | O | C-A1 | C-A1 | C-A3 | C-A1 | B22 | B114 |
| H8-1057 | O | C-A1 | C-A1 | C-A3 | C-A1 | B22 | B115 |
| H8-1058 | O | C-A1 | C-A1 | C-A3 | C-A1 | B22 | B116 |
| H8-1059 | O | C-A1 | C-A1 | C-A3 | C-A1 | B22 | B117 |
| H8-1060 | O | C-A1 | C-A1 | C-A3 | C-A1 | B22 | B118 |
| H8-1061 | O | C-A1 | C-A1 | C-A3 | C-A1 | B22 | B119 |
| H8-1062 | O | C-A1 | C-A1 | C-A3 | C-A1 | B22 | B120 |
| H8-1063 | O | C-A1 | C-A1 | C-A3 | C-A1 | B22 | B121 |
| H8-1064 | O | C-A1 | C-A1 | C-A3 | C-A1 | B22 | B122 |
| H8-1065 | O | C-A1 | C-A1 | C-A3 | C-A1 | B22 | B125 |
| H8-1066 | O | C-A1 | C-A1 | C-A3 | C-A1 | B22 | B126 |
| H8-1067 | O | C-A1 | C-A1 | C-A3 | C-A1 | B22 | B127 |
| H8-1068 | O | C-A1 | C-A1 | C-A3 | C-A1 | B22 | B128 |
| H8-1069 | O | C-A1 | C-A1 | C-A3 | C-A1 | B22 | B129 |
| H8-1070 | O | C-A1 | C-A1 | C-A3 | C-A1 | B22 | B130 |
| H8-1071 | O | C-A1 | C-A1 | C-A3 | C-A1 | B22 | B131 |
| H8-1072 | O | C-A1 | C-A1 | C-A3 | C-A1 | B22 | B132 |
| H8-1073 | O | C-A1 | C-A1 | C-A3 | C-A1 | B22 | B133 |
| H8-1074 | O | C-A1 | C-A1 | C-A3 | C-A1 | B22 | B134 |
| H8-1075 | O | C-A1 | C-A1 | C-A3 | C-A1 | B22 | B135 |
| H8-1076 | O | C-A1 | C-A1 | C-A3 | C-A1 | B22 | B136 |
| H8-1077 | O | C-A1 | C-A1 | C-A3 | C-A1 | B22 | B137 |
| H8-1078 | O | C-A1 | C-A1 | C-A3 | C-A1 | B22 | B138 |
| H8-1079 | O | C-A1 | C-A1 | C-A3 | C-A1 | B22 | B139 |
| H8-1080 | O | C-A1 | C-A1 | C-A3 | C-A1 | B22 | B140 |
| H8-1087 | O | C-A1 | C-A1 | C-A1 | C-A1 | B32 | B1 |
| H8-1088 | O | C-A1 | C-A1 | C-A1 | C-A1 | B32 | B6 |
| H8-1089 | O | C-A1 | C-A1 | C-A1 | C-A1 | B32 | B20 |
| H8-1090 | O | C-A1 | C-A1 | C-A1 | C-A1 | B32 | B21 |
| H8-1091 | O | C-A1 | C-A1 | C-A1 | C-A1 | B32 | B22 |
| H8-1092 | O | C-A1 | C-A1 | C-A1 | C-A1 | B32 | B32 |
| H8-1093 | O | C-A1 | C-A1 | C-A1 | C-A1 | B32 | B33 |
| H8-1094 | O | C-A1 | C-A1 | C-A1 | C-A1 | B32 | B34 |
| H8-1095 | O | C-A1 | C-A1 | C-A1 | C-A1 | B32 | B68 |
| H8-1096 | O | C-A1 | C-A1 | C-A1 | C-A1 | B32 | B69 |
| H8-1097 | O | C-A1 | C-A1 | C-A1 | C-A1 | B32 | B70 |
| H8-1098 | O | C-A1 | C-A1 | C-A1 | C-A1 | B32 | B71 |
| H8-1099 | O | C-A1 | C-A1 | C-A1 | C-A1 | B32 | B81 |
| H8-1100 | O | C-A1 | C-A1 | C-A1 | C-A1 | B32 | B90 |
| H8-1101 | O | C-A1 | C-A1 | C-A1 | C-A1 | B32 | B91 |
| H8-1102 | O | C-A1 | C-A1 | C-A1 | C-A1 | B32 | B92 |
| H8-1103 | O | C-A1 | C-A1 | C-A1 | C-A1 | B32 | B93 |
| H8-1104 | O | C-A1 | C-A1 | C-A1 | C-A1 | B32 | B94 |
| H8-1105 | O | C-A1 | C-A1 | C-A1 | C-A1 | B32 | B95 |
| H8-1106 | O | C-A1 | C-A1 | C-A1 | C-A1 | B32 | B96 |
| H8-1107 | O | C-A1 | C-A1 | C-A1 | C-A1 | B32 | B97 |
| H8-1108 | O | C-A1 | C-A1 | C-A1 | C-A1 | B32 | B106 |
| H8-1109 | O | C-A1 | C-A1 | C-A1 | C-A1 | B32 | B107 |
| H8-1110 | O | C-A1 | C-A1 | C-A1 | C-A1 | B32 | B114 |
| H8-1111 | O | C-A1 | C-A1 | C-A1 | C-A1 | B32 | B115 |
| H8-1112 | O | C-A1 | C-A1 | C-A1 | C-A1 | B32 | B116 |
| H8-1113 | O | C-A1 | C-A1 | C-A1 | C-A1 | B32 | B117 |
| H8-1114 | O | C-A1 | C-A1 | C-A1 | C-A1 | B32 | B118 |
| H8-1115 | O | C-A1 | C-A1 | C-A1 | C-A1 | B32 | B119 |
| H8-1116 | O | C-A1 | C-A1 | C-A1 | C-A1 | B32 | B120 |
| H8-1117 | O | C-A1 | C-A1 | C-A1 | C-A1 | B32 | B121 |
| H8-1118 | O | C-A1 | C-A1 | C-A1 | C-A1 | B32 | B122 |
| H8-1119 | O | C-A1 | C-A1 | C-A1 | C-A1 | B32 | B125 |
| H8-1120 | O | C-A1 | C-A1 | C-A1 | C-A1 | B32 | B126 |
| H8-1121 | O | C-A1 | C-A1 | C-A1 | C-A1 | B32 | B127 |
| H8-1122 | O | C-A1 | C-A1 | C-A1 | C-A1 | B32 | B128 |
| H8-1123 | O | C-A1 | C-A1 | C-A1 | C-A1 | B32 | B129 |
| H8-1124 | O | C-A1 | C-A1 | C-A1 | C-A1 | B32 | B130 |
| H8-1125 | O | C-A1 | C-A1 | C-A1 | C-A1 | B32 | B131 |
| H8-1126 | O | C-A1 | C-A1 | C-A1 | C-A1 | B32 | B132 |
| H8-1127 | O | C-A1 | C-A1 | C-A1 | C-A1 | B32 | B133 |
| H8-1128 | O | C-A1 | C-A1 | C-A1 | C-A1 | B32 | B134 |
| H8-1129 | O | C-A1 | C-A1 | C-A1 | C-A1 | B32 | B135 |
| H8-1130 | O | C-A1 | C-A1 | C-A1 | C-A1 | B32 | B136 |
| H8-1131 | O | C-A1 | C-A1 | C-A1 | C-A1 | B32 | B137 |
| H8-1132 | O | C-A1 | C-A1 | C-A1 | C-A1 | B32 | B138 |
| H8-1133 | O | C-A1 | C-A1 | C-A1 | C-A1 | B32 | B139 |
| H8-1134 | O | C-A1 | C-A1 | C-A1 | C-A1 | B32 | B140 |
| H8-1141 | O | C-A1 | C-A2 | C-A1 | C-A2 | B32 | B1 |
| H8-1142 | O | C-A1 | C-A2 | C-A1 | C-A2 | B32 | B6 |
| H8-1143 | O | C-A1 | C-A2 | C-A1 | C-A2 | B32 | B20 |
| H8-1144 | O | C-A1 | C-A2 | C-A1 | C-A2 | B32 | B21 |
| H8-1145 | O | C-A1 | C-A2 | C-A1 | C-A2 | B32 | B22 |
| H8-1146 | O | C-A1 | C-A2 | C-A1 | C-A2 | B32 | B32 |
| H8-1147 | O | C-A1 | C-A2 | C-A1 | C-A2 | B32 | B33 |
| H8-1148 | O | C-A1 | C-A2 | C-A1 | C-A2 | B32 | B34 |
| H8-1149 | O | C-A1 | C-A2 | C-A1 | C-A2 | B32 | B68 |
| H8-1150 | O | C-A1 | C-A2 | C-A1 | C-A2 | B32 | B69 |
| H8-1151 | O | C-A1 | C-A2 | C-A1 | C-A2 | B32 | B70 |
| H8-1152 | O | C-A1 | C-A2 | C-A1 | C-A2 | B32 | B71 |
| H8-1153 | O | C-A1 | C-A2 | C-A1 | C-A2 | B32 | B81 |
| H8-1154 | O | C-A1 | C-A2 | C-A1 | C-A2 | B32 | B90 |
| H8-1155 | O | C-A1 | C-A2 | C-A1 | C-A2 | B32 | B91 |
| H8-1156 | O | C-A1 | C-A2 | C-A1 | C-A2 | B32 | B92 |
| H8-1157 | O | C-A1 | C-A2 | C-A1 | C-A2 | B32 | B93 |
| H8-1158 | O | C-A1 | C-A2 | C-A1 | C-A2 | B32 | B94 |
| H8-1159 | O | C-A1 | C-A2 | C-A1 | C-A2 | B32 | B95 |
| H8-1160 | O | C-A1 | C-A2 | C-A1 | C-A2 | B32 | B96 |
| H8-1161 | O | C-A1 | C-A2 | C-A1 | C-A2 | B32 | B97 |
| H8-1162 | O | C-A1 | C-A2 | C-A1 | C-A2 | B32 | B106 |
| H8-1163 | O | C-A1 | C-A2 | C-A1 | C-A2 | B32 | B107 |
| H8-1164 | O | C-A1 | C-A2 | C-A1 | C-A2 | B32 | B114 |
| H8-1165 | O | C-A1 | C-A2 | C-A1 | C-A2 | B32 | B115 |
| H8-1166 | O | C-A1 | C-A2 | C-A1 | C-A2 | B32 | B116 |
| H8-1167 | O | C-A1 | C-A2 | C-A1 | C-A2 | B32 | B117 |
| H8-1168 | O | C-A1 | C-A2 | C-A1 | C-A2 | B32 | B118 |
| H8-1169 | O | C-A1 | C-A2 | C-A1 | C-A2 | B32 | B119 |
| H8-1170 | O | C-A1 | C-A2 | C-A1 | C-A2 | B32 | B120 |
| H8-1171 | O | C-A1 | C-A2 | C-A1 | C-A2 | B32 | B121 |
| H8-1172 | O | C-A1 | C-A2 | C-A1 | C-A2 | B32 | B122 |
| H8-1173 | O | C-A1 | C-A2 | C-A1 | C-A2 | B32 | B125 |
| H8-1174 | O | C-A1 | C-A2 | C-A1 | C-A2 | B32 | B126 |
| H8-1175 | O | C-A1 | C-A2 | C-A1 | C-A2 | B32 | B127 |
| H8-1176 | O | C-A1 | C-A2 | C-A1 | C-A2 | B32 | B128 |
| H8-1177 | O | C-A1 | C-A2 | C-A1 | C-A2 | B32 | B129 |
| H8-1178 | O | C-A1 | C-A2 | C-A1 | C-A2 | B32 | B130 |
| H8-1179 | O | C-A1 | C-A2 | C-A1 | C-A2 | B32 | B131 |
| H8-1180 | O | C-A1 | C-A2 | C-A1 | C-A2 | B32 | B132 |
| H8-1181 | O | C-A1 | C-A2 | C-A1 | C-A2 | B32 | B133 |
| H8-1182 | O | C-A1 | C-A2 | C-A1 | C-A2 | B32 | B134 |
| H8-1183 | O | C-A1 | C-A2 | C-A1 | C-A2 | B32 | B135 |
| H8-1184 | O | C-A1 | C-A2 | C-A1 | C-A2 | B32 | B136 |
| H8-1185 | O | C-A1 | C-A2 | C-A1 | C-A2 | B32 | B137 |
| H8-1186 | O | C-A1 | C-A2 | C-A1 | C-A2 | B32 | B138 |
| H8-1187 | O | C-A1 | C-A2 | C-A1 | C-A2 | B32 | B139 |
| H8-1188 | O | C-A1 | C-A2 | C-A1 | C-A2 | B32 | B140 |
| H8-1195 | O | C-A1 | C-A3 | C-A1 | C-A3 | B32 | B1 |
| H8-1196 | O | C-A1 | C-A3 | C-A1 | C-A3 | B32 | B6 |
| H8-1197 | O | C-A1 | C-A3 | C-A1 | C-A3 | B32 | B20 |
| H8-1198 | O | C-A1 | C-A3 | C-A1 | C-A3 | B32 | B21 |
| H8-1199 | O | C-A1 | C-A3 | C-A1 | C-A3 | B32 | B22 |
| H8-1200 | O | C-A1 | C-A3 | C-A1 | C-A3 | B32 | B32 |
| H8-1201 | O | C-A1 | C-A3 | C-A1 | C-A3 | B32 | B33 |
| H8-1202 | O | C-A1 | C-A3 | C-A1 | C-A3 | B32 | B34 |
| H8-1203 | O | C-A1 | C-A3 | C-A1 | C-A3 | B32 | B68 |
| H8-1204 | O | C-A1 | C-A3 | C-A1 | C-A3 | B32 | B69 |
| H8-1205 | O | C-A1 | C-A3 | C-A1 | C-A3 | B32 | B70 |
| H8-1206 | O | C-A1 | C-A3 | C-A1 | C-A3 | B32 | B71 |
| H8-1207 | O | C-A1 | C-A3 | C-A1 | C-A3 | B32 | B81 |
| H8-1208 | O | C-A1 | C-A3 | C-A1 | C-A3 | B32 | B90 |
| H8-1209 | O | C-A1 | C-A3 | C-A1 | C-A3 | B32 | B91 |
| H8-1210 | O | C-A1 | C-A3 | C-A1 | C-A3 | B32 | B92 |
| H8-1211 | O | C-A1 | C-A3 | C-A1 | C-A3 | B32 | B93 |
| H8-1212 | O | C-A1 | C-A3 | C-A1 | C-A3 | B32 | B94 |
| H8-1213 | O | C-A1 | C-A3 | C-A1 | C-A3 | B32 | B95 |
| H8-1214 | O | C-A1 | C-A3 | C-A1 | C-A3 | B32 | B96 |
| H8-1215 | O | C-A1 | C-A3 | C-A1 | C-A3 | B32 | B97 |
| H8-1216 | O | C-A1 | C-A3 | C-A1 | C-A3 | B32 | B106 |
| H8-1217 | O | C-A1 | C-A3 | C-A1 | C-A3 | B32 | B107 |
| H8-1218 | O | C-A1 | C-A3 | C-A1 | C-A3 | B32 | B114 |
| H8-1219 | O | C-A1 | C-A3 | C-A1 | C-A3 | B32 | B115 |
| H8-1220 | O | C-A1 | C-A3 | C-A1 | C-A3 | B32 | B116 |
| H8-1221 | O | C-A1 | C-A3 | C-A1 | C-A3 | B32 | B117 |
| H8-1222 | O | C-A1 | C-A3 | C-A1 | C-A3 | B32 | B118 |
| H8-1223 | O | C-A1 | C-A3 | C-A1 | C-A3 | B32 | B119 |
| H8-1224 | O | C-A1 | C-A3 | C-A1 | C-A3 | B32 | B120 |
| H8-1225 | O | C-A1 | C-A3 | C-A1 | C-A3 | B32 | B121 |
| H8-1226 | O | C-A1 | C-A3 | C-A1 | C-A3 | B32 | B122 |
| H8-1227 | O | C-A1 | C-A3 | C-A1 | C-A3 | B32 | B125 |
| H8-1228 | O | C-A1 | C-A3 | C-A1 | C-A3 | B32 | B126 |
| H8-1229 | O | C-A1 | C-A3 | C-A1 | C-A3 | B32 | B127 |
| H8-1230 | O | C-A1 | C-A3 | C-A1 | C-A3 | B32 | B128 |
| H8-1231 | O | C-A1 | C-A3 | C-A1 | C-A3 | B32 | B129 |
| H8-1232 | O | C-A1 | C-A3 | C-A1 | C-A3 | B32 | B130 |
| H8-1233 | O | C-A1 | C-A3 | C-A1 | C-A3 | B32 | B131 |
| H8-1234 | O | C-A1 | C-A3 | C-A1 | C-A3 | B32 | B132 |
| H8-1235 | O | C-A1 | C-A3 | C-A1 | C-A3 | B32 | B133 |
| H8-1236 | O | C-A1 | C-A3 | C-A1 | C-A3 | B32 | B134 |
| H8-1237 | O | C-A1 | C-A3 | C-A1 | C-A3 | B32 | B135 |
| H8-1238 | O | C-A1 | C-A3 | C-A1 | C-A3 | B32 | B136 |
| H8-1239 | O | C-A1 | C-A3 | C-A1 | C-A3 | B32 | B137 |
| H8-1240 | O | C-A1 | C-A3 | C-A1 | C-A3 | B32 | B138 |
| H8-1241 | O | C-A1 | C-A3 | C-A1 | C-A3 | B32 | B139 |
| H8-1242 | O | C-A1 | C-A3 | C-A1 | C-A3 | B32 | B140 |
| H8-1249 | O | C-A3 | C-A1 | C-A3 | C-A1 | B32 | B1 |
| H8-1250 | O | C-A3 | C-A1 | C-A3 | C-A1 | B32 | B6 |
| H8-1251 | O | C-A3 | C-A1 | C-A3 | C-A1 | B32 | B20 |
| H8-1252 | O | C-A3 | C-A1 | C-A3 | C-A1 | B32 | B21 |
| H8-1253 | O | C-A3 | C-A1 | C-A3 | C-A1 | B32 | B22 |
| H8-1254 | O | C-A3 | C-A1 | C-A3 | C-A1 | B32 | B32 |
| H8-1255 | O | C-A3 | C-A1 | C-A3 | C-A1 | B32 | B33 |
| H8-1256 | O | C-A3 | C-A1 | C-A3 | C-A1 | B32 | B34 |
| H8-1257 | O | C-A3 | C-A1 | C-A3 | C-A1 | B32 | B68 |
| H8-1258 | O | C-A3 | C-A1 | C-A3 | C-A1 | B32 | B69 |
| H8-1259 | O | C-A3 | C-A1 | C-A3 | C-A1 | B32 | B70 |
| H8-1260 | O | C-A3 | C-A1 | C-A3 | C-A1 | B32 | B71 |
| H8-1261 | O | C-A3 | C-A1 | C-A3 | C-A1 | B32 | B81 |
| H8-1262 | O | C-A3 | C-A1 | C-A3 | C-A1 | B32 | B90 |
| H8-1263 | O | C-A3 | C-A1 | C-A3 | C-A1 | B32 | B91 |
| H8-1264 | O | C-A3 | C-A1 | C-A3 | C-A1 | B32 | B92 |
| H8-1265 | O | C-A3 | C-A1 | C-A3 | C-A1 | B32 | B93 |
| H8-1266 | O | C-A3 | C-A1 | C-A3 | C-A1 | B32 | B94 |
| H8-1267 | O | C-A3 | C-A1 | C-A3 | C-A1 | B32 | B95 |
| H8-1268 | O | C-A3 | C-A1 | C-A3 | C-A1 | B32 | B96 |
| H8-1269 | O | C-A3 | C-A1 | C-A3 | C-A1 | B32 | B97 |
| H8-1270 | O | C-A3 | C-A1 | C-A3 | C-A1 | B32 | B106 |
| H8-1271 | O | C-A3 | C-A1 | C-A3 | C-A1 | B32 | B107 |
| H8-1272 | O | C-A3 | C-A1 | C-A3 | C-A1 | B32 | B114 |
| H8-1273 | O | C-A3 | C-A1 | C-A3 | C-A1 | B32 | B115 |
| H8-1274 | O | C-A3 | C-A1 | C-A3 | C-A1 | B32 | B116 |
| H8-1275 | O | C-A3 | C-A1 | C-A3 | C-A1 | B32 | B117 |
| H8-1276 | O | C-A3 | C-A1 | C-A3 | C-A1 | B32 | B118 |
| H8-1277 | O | C-A3 | C-A1 | C-A3 | C-A1 | B32 | B119 |
| H8-1278 | O | C-A3 | C-A1 | C-A3 | C-A1 | B32 | B120 |
| H8-1279 | O | C-A3 | C-A1 | C-A3 | C-A1 | B32 | B121 |
| H8-1280 | O | C-A3 | C-A1 | C-A3 | C-A1 | B32 | B122 |
| H8-1281 | O | C-A3 | C-A1 | C-A3 | C-A1 | B32 | B125 |
| H8-1282 | O | C-A3 | C-A1 | C-A3 | C-A1 | B32 | B126 |
| H8-1283 | O | C-A3 | C-A1 | C-A3 | C-A1 | B32 | B127 |
| H8-1284 | O | C-A3 | C-A1 | C-A3 | C-A1 | B32 | B128 |
| H8-1285 | O | C-A3 | C-A1 | C-A3 | C-A1 | B32 | B129 |
| H8-1286 | O | C-A3 | C-A1 | C-A3 | C-A1 | B32 | B130 |
| H8-1287 | O | C-A3 | C-A1 | C-A3 | C-A1 | B32 | B131 |
| H8-1288 | O | C-A3 | C-A1 | C-A3 | C-A1 | B32 | B132 |
| H8-1289 | O | C-A3 | C-A1 | C-A3 | C-A1 | B32 | B133 |
| H8-1290 | O | C-A3 | C-A1 | C-A3 | C-A1 | B32 | B134 |
| H8-1291 | O | C-A3 | C-A1 | C-A3 | C-A1 | B32 | B135 |
| H8-1292 | O | C-A3 | C-A1 | C-A3 | C-A1 | B32 | B136 |
| H8-1293 | O | C-A3 | C-A1 | C-A3 | C-A1 | B32 | B137 |
| H8-1294 | O | C-A3 | C-A1 | C-A3 | C-A1 | B32 | B138 |
| H8-1295 | O | C-A3 | C-A1 | C-A3 | C-A1 | B32 | B139 |
| H8-1296 | O | C-A3 | C-A1 | C-A3 | C-A1 | B32 | B140 |
| H8-1303 | O | C-A1 | C-A1 | C-A3 | C-A1 | B32 | B1 |
| H8-1304 | O | C-A1 | C-A1 | C-A3 | C-A1 | B32 | B6 |
| H8-1305 | O | C-A1 | C-A1 | C-A3 | C-A1 | B32 | B20 |
| H8-1306 | O | C-A1 | C-A1 | C-A3 | C-A1 | B32 | B21 |
| H8-1307 | O | C-A1 | C-A1 | C-A3 | C-A1 | B32 | B22 |
| H8-1308 | O | C-A1 | C-A1 | C-A3 | C-A1 | B32 | B32 |
| H8-1309 | O | C-A1 | C-A1 | C-A3 | C-A1 | B32 | B33 |
| H8-1310 | O | C-A1 | C-A1 | C-A3 | C-A1 | B32 | B34 |
| H8-1311 | O | C-A1 | C-A1 | C-A3 | C-A1 | B32 | B68 |
| H8-1312 | O | C-A1 | C-A1 | C-A3 | C-A1 | B32 | B69 |
| H8-1313 | O | C-A1 | C-A1 | C-A3 | C-A1 | B32 | B70 |
| H8-1314 | O | C-A1 | C-A1 | C-A3 | C-A1 | B32 | B71 |
| H8-1315 | O | C-A1 | C-A1 | C-A3 | C-A1 | B32 | B81 |
| H8-1316 | O | C-A1 | C-A1 | C-A3 | C-A1 | B32 | B90 |
| H8-1317 | O | C-A1 | C-A1 | C-A3 | C-A1 | B32 | B91 |
| H8-1318 | O | C-A1 | C-A1 | C-A3 | C-A1 | B32 | B92 |
| H8-1319 | O | C-A1 | C-A1 | C-A3 | C-A1 | B32 | B93 |
| H8-1320 | O | C-A1 | C-A1 | C-A3 | C-A1 | B32 | B94 |
| H8-1321 | O | C-A1 | C-A1 | C-A3 | C-A1 | B32 | B95 |
| H8-1322 | O | C-A1 | C-A1 | C-A3 | C-A1 | B32 | B96 |
| H8-1323 | O | C-A1 | C-A1 | C-A3 | C-A1 | B32 | B97 |
| H8-1324 | O | C-A1 | C-A1 | C-A3 | C-A1 | B32 | B106 |
| H8-1325 | O | C-A1 | C-A1 | C-A3 | C-A1 | B32 | B107 |
| H8-1326 | O | C-A1 | C-A1 | C-A3 | C-A1 | B32 | B114 |
| H8-1327 | O | C-A1 | C-A1 | C-A3 | C-A1 | B32 | B115 |
| H8-1328 | O | C-A1 | C-A1 | C-A3 | C-A1 | B32 | B116 |
| H8-1329 | O | C-A1 | C-A1 | C-A3 | C-A1 | B32 | B117 |
| H8-1330 | O | C-A1 | C-A1 | C-A3 | C-A1 | B32 | B118 |
| H8-1331 | O | C-A1 | C-A1 | C-A3 | C-A1 | B32 | B119 |
| H8-1332 | O | C-A1 | C-A1 | C-A3 | C-A1 | B32 | B120 |
| H8-1333 | O | C-A1 | C-A1 | C-A3 | C-A1 | B32 | B121 |
| H8-1334 | O | C-A1 | C-A1 | C-A3 | C-A1 | B32 | B122 |
| H8-1335 | O | C-A1 | C-A1 | C-A3 | C-A1 | B32 | B125 |
| H8-1336 | O | C-A1 | C-A1 | C-A3 | C-A1 | B32 | B126 |
| H8-1337 | O | C-A1 | C-A1 | C-A3 | C-A1 | B32 | B127 |
| H8-1338 | O | C-A1 | C-A1 | C-A3 | C-A1 | B32 | B128 |
| H8-1339 | O | C-A1 | C-A1 | C-A3 | C-A1 | B32 | B129 |
| H8-1340 | O | C-A1 | C-A1 | C-A3 | C-A1 | B32 | B130 |
| H8-1341 | O | C-A1 | C-A1 | C-A3 | C-A1 | B32 | B131 |
| H8-1342 | O | C-A1 | C-A1 | C-A3 | C-A1 | B32 | B132 |
| H8-1343 | O | C-A1 | C-A1 | C-A3 | C-A1 | B32 | B133 |
| H8-1344 | O | C-A1 | C-A1 | C-A3 | C-A1 | B32 | B134 |
| H8-1345 | O | C-A1 | C-A1 | C-A3 | C-A1 | B32 | B135 |
| H8-1346 | O | C-A1 | C-A1 | C-A3 | C-A1 | B32 | B136 |
| H8-1347 | O | C-A1 | C-A1 | C-A3 | C-A1 | B32 | B137 |
| H8-1348 | O | C-A1 | C-A1 | C-A3 | C-A1 | B32 | B138 |
| H8-1349 | O | C-A1 | C-A1 | C-A3 | C-A1 | B32 | B139 |
| H8-1350 | O | C-A1 | C-A1 | C-A3 | C-A1 | B32 | B140 |
| H8-1357 | S | C-A1 | C-A1 | C-A1 | C-A1 | B20 | B1 |
| H8-1358 | S | C-A1 | C-A1 | C-A1 | C-A1 | B20 | B6 |
| H8-1359 | S | C-A1 | C-A1 | C-A1 | C-A1 | B20 | B20 |
| H8-1360 | S | C-A1 | C-A1 | C-A1 | C-A1 | B20 | B21 |
| H8-1361 | S | C-A1 | C-A1 | C-A1 | C-A1 | B20 | B22 |
| H8-1362 | S | C-A1 | C-A1 | C-A1 | C-A1 | B20 | B32 |
| H8-1363 | S | C-A1 | C-A1 | C-A1 | C-A1 | B20 | B33 |
| H8-1364 | S | C-A1 | C-A1 | C-A1 | C-A1 | B20 | B34 |
| H8-1365 | S | C-A1 | C-A1 | C-A1 | C-A1 | B20 | B68 |
| H8-1366 | S | C-A1 | C-A1 | C-A1 | C-A1 | B20 | B69 |
| H8-1367 | S | C-A1 | C-A1 | C-A1 | C-A1 | B20 | B70 |
| H8-1368 | S | C-A1 | C-A1 | C-A1 | C-A1 | B20 | B71 |
| H8-1369 | S | C-A1 | C-A1 | C-A1 | C-A1 | B20 | B81 |
| H8-1370 | S | C-A1 | C-A1 | C-A1 | C-A1 | B20 | B90 |
| H8-1371 | S | C-A1 | C-A1 | C-A1 | C-A1 | B20 | B91 |
| H8-1372 | S | C-A1 | C-A1 | C-A1 | C-A1 | B20 | B92 |
| H8-1373 | S | C-A1 | C-A1 | C-A1 | C-A1 | B20 | B93 |
| H8-1374 | S | C-A1 | C-A1 | C-A1 | C-A1 | B20 | B94 |
| H8-1375 | S | C-A1 | C-A1 | C-A1 | C-A1 | B20 | B95 |
| H8-1376 | S | C-A1 | C-A1 | C-A1 | C-A1 | B20 | B96 |
| H8-1377 | S | C-A1 | C-A1 | C-A1 | C-A1 | B20 | B97 |
| H8-1378 | S | C-A1 | C-A1 | C-A1 | C-A1 | B20 | B106 |
| H8-1379 | S | C-A1 | C-A1 | C-A1 | C-A1 | B20 | B107 |
| H8-1380 | S | C-A1 | C-A1 | C-A1 | C-A1 | B20 | B114 |
| H8-1381 | S | C-A1 | C-A1 | C-A1 | C-A1 | B20 | B115 |
| H8-1382 | S | C-A1 | C-A1 | C-A1 | C-A1 | B20 | B116 |
| H8-1383 | S | C-A1 | C-A1 | C-A1 | C-A1 | B20 | B117 |
| H8-1384 | S | C-A1 | C-A1 | C-A1 | C-A1 | B20 | B118 |
| H8-1385 | S | C-A1 | C-A1 | C-A1 | C-A1 | B20 | B119 |
| H8-1386 | S | C-A1 | C-A1 | C-A1 | C-A1 | B20 | B120 |
| H8-1387 | S | C-A1 | C-A1 | C-A1 | C-A1 | B20 | B121 |
| H8-1388 | S | C-A1 | C-A1 | C-A1 | C-A1 | B20 | B122 |
| H8-1389 | S | C-A1 | C-A1 | C-A1 | C-A1 | B20 | B125 |
| H8-1390 | S | C-A1 | C-A1 | C-A1 | C-A1 | B20 | B126 |
| H8-1391 | S | C-A1 | C-A1 | C-A1 | C-A1 | B20 | B127 |
| H8-1392 | S | C-A1 | C-A1 | C-A1 | C-A1 | B20 | B128 |
| H8-1393 | S | C-A1 | C-A1 | C-A1 | C-A1 | B20 | B129 |
| H8-1394 | S | C-A1 | C-A1 | C-A1 | C-A1 | B20 | B130 |
| H8-1395 | S | C-A1 | C-A1 | C-A1 | C-A1 | B20 | B131 |
| H8-1396 | S | C-A1 | C-A1 | C-A1 | C-A1 | B20 | B132 |
| H8-1397 | S | C-A1 | C-A1 | C-A1 | C-A1 | B20 | B133 |
| H8-1398 | S | C-A1 | C-A1 | C-A1 | C-A1 | B20 | B134 |
| H8-1399 | S | C-A1 | C-A1 | C-A1 | C-A1 | B20 | B135 |
| H8-1400 | S | C-A1 | C-A1 | C-A1 | C-A1 | B20 | B136 |
| H8-1401 | S | C-A1 | C-A1 | C-A1 | C-A1 | B20 | B137 |
| H8-1402 | S | C-A1 | C-A1 | C-A1 | C-A1 | B20 | B138 |
| H8-1403 | S | C-A1 | C-A1 | C-A1 | C-A1 | B20 | B139 |
| H8-1404 | S | C-A1 | C-A1 | C-A1 | C-A1 | B20 | B140 |
| H8-1411 | S | C-A1 | C-A1 | C-A1 | C-A1 | B68 | B1 |
| H8-1412 | S | C-A1 | C-A1 | C-A1 | C-A1 | B68 | B6 |
| H8-1413 | S | C-A1 | C-A1 | C-A1 | C-A1 | B68 | B20 |
| H8-1414 | S | C-A1 | C-A1 | C-A1 | C-A1 | B68 | B21 |
| H8-1415 | S | C-A1 | C-A1 | C-A1 | C-A1 | B68 | B22 |
| H8-1416 | S | C-A1 | C-A1 | C-A1 | C-A1 | B68 | B32 |
| H8-1417 | S | C-A1 | C-A1 | C-A1 | C-A1 | B68 | B33 |
| H8-1418 | S | C-A1 | C-A1 | C-A1 | C-A1 | B68 | B34 |
| H8-1419 | S | C-A1 | C-A1 | C-A1 | C-A1 | B68 | B68 |
| H8-1420 | S | C-A1 | C-A1 | C-A1 | C-A1 | B68 | B69 |
| H8-1421 | S | C-A1 | C-A1 | C-A1 | C-A1 | B68 | B70 |
| H8-1422 | S | C-A1 | C-A1 | C-A1 | C-A1 | B68 | B71 |
| H8-1423 | S | C-A1 | C-A1 | C-A1 | C-A1 | B68 | B81 |
| H8-1424 | S | C-A1 | C-A1 | C-A1 | C-A1 | B68 | B90 |
| H8-1425 | S | C-A1 | C-A1 | C-A1 | C-A1 | B68 | B91 |
| H8-1426 | S | C-A1 | C-A1 | C-A1 | C-A1 | B68 | B92 |
| H8-1427 | S | C-A1 | C-A1 | C-A1 | C-A1 | B68 | B93 |
| H8-1428 | S | C-A1 | C-A1 | C-A1 | C-A1 | B68 | B94 |
| H8-1429 | S | C-A1 | C-A1 | C-A1 | C-A1 | B68 | B95 |
| H8-1430 | S | C-A1 | C-A1 | C-A1 | C-A1 | B68 | B96 |
| H8-1431 | S | C-A1 | C-A1 | C-A1 | C-A1 | B68 | B97 |
| H8-1432 | S | C-A1 | C-A1 | C-A1 | C-A1 | B68 | B106 |
| H8-1433 | S | C-A1 | C-A1 | C-A1 | C-A1 | B68 | B107 |
| H8-1434 | S | C-A1 | C-A1 | C-A1 | C-A1 | B68 | B114 |
| H8-1435 | S | C-A1 | C-A1 | C-A1 | C-A1 | B68 | B115 |
| H8-1436 | S | C-A1 | C-A1 | C-A1 | C-A1 | B68 | B116 |
| H8-1437 | S | C-A1 | C-A1 | C-A1 | C-A1 | B68 | B117 |
| H8-1438 | S | C-A1 | C-A1 | C-A1 | C-A1 | B68 | B118 |
| H8-1439 | S | C-A1 | C-A1 | C-A1 | C-A1 | B68 | B119 |
| H8-1440 | S | C-A1 | C-A1 | C-A1 | C-A1 | B68 | B120 |
| H8-1441 | S | C-A1 | C-A1 | C-A1 | C-A1 | B68 | B121 |
| H8-1442 | S | C-A1 | C-A1 | C-A1 | C-A1 | B68 | B122 |
| H8-1443 | S | C-A1 | C-A1 | C-A1 | C-A1 | B68 | B125 |
| H8-1444 | S | C-A1 | C-A1 | C-A1 | C-A1 | B68 | B126 |
| H8-1445 | S | C-A1 | C-A1 | C-A1 | C-A1 | B68 | B127 |
| H8-1446 | S | C-A1 | C-A1 | C-A1 | C-A1 | B68 | B128 |
| H8-1447 | S | C-A1 | C-A1 | C-A1 | C-A1 | B68 | B129 |
| H8-1448 | S | C-A1 | C-A1 | C-A1 | C-A1 | B68 | B130 |
| H8-1449 | S | C-A1 | C-A1 | C-A1 | C-A1 | B68 | B131 |
| H8-1450 | S | C-A1 | C-A1 | C-A1 | C-A1 | B68 | B132 |
| H8-1451 | S | C-A1 | C-A1 | C-A1 | C-A1 | B68 | B133 |
| H8-1452 | S | C-A1 | C-A1 | C-A1 | C-A1 | B68 | B134 |
| H8-1453 | S | C-A1 | C-A1 | C-A1 | C-A1 | B68 | B135 |
| H8-1454 | S | C-A1 | C-A1 | C-A1 | C-A1 | B68 | B136 |
| H8-1455 | S | C-A1 | C-A1 | C-A1 | C-A1 | B68 | B137 |
| H8-1456 | S | C-A1 | C-A1 | C-A1 | C-A1 | B68 | B138 |
| H8-1457 | S | C-A1 | C-A1 | C-A1 | C-A1 | B68 | B139 |
| H8-1458 | S | C-A1 | C-A1 | C-A1 | C-A1 | B68 | B140 |
| H8-1465 | S | C-A1 | C-A1 | C-A1 | C-A1 | B70 | B1 |
| H8-1466 | S | C-A1 | C-A1 | C-A1 | C-A1 | B70 | B6 |
| H8-1467 | S | C-A1 | C-A1 | C-A1 | C-A1 | B70 | B20 |
| H8-1468 | S | C-A1 | C-A1 | C-A1 | C-A1 | B70 | B21 |
| H8-1469 | S | C-A1 | C-A1 | C-A1 | C-A1 | B70 | B22 |
| H8-1470 | S | C-A1 | C-A1 | C-A1 | C-A1 | B70 | B32 |
| H8-1471 | S | C-A1 | C-A1 | C-A1 | C-A1 | B70 | B33 |
| H8-1472 | S | C-A1 | C-A1 | C-A1 | C-A1 | B70 | B34 |
| H8-1473 | S | C-A1 | C-A1 | C-A1 | C-A1 | B70 | B68 |
| H8-1474 | S | C-A1 | C-A1 | C-A1 | C-A1 | B70 | B69 |
| H8-1475 | S | C-A1 | C-A1 | C-A1 | C-A1 | B70 | B70 |
| H8-1476 | S | C-A1 | C-A1 | C-A1 | C-A1 | B70 | B71 |
| H8-1477 | S | C-A1 | C-A1 | C-A1 | C-A1 | B70 | B81 |
| H8-1478 | S | C-A1 | C-A1 | C-A1 | C-A1 | B70 | B90 |
| H8-1479 | S | C-A1 | C-A1 | C-A1 | C-A1 | B70 | B91 |
| H8-1480 | S | C-A1 | C-A1 | C-A1 | C-A1 | B70 | B92 |
| H8-1481 | S | C-A1 | C-A1 | C-A1 | C-A1 | B70 | B93 |
| H8-1482 | S | C-A1 | C-A1 | C-A1 | C-A1 | B70 | B94 |
| H8-1483 | S | C-A1 | C-A1 | C-A1 | C-A1 | B70 | B95 |
| H8-1484 | S | C-A1 | C-A1 | C-A1 | C-A1 | B70 | B96 |
| H8-1485 | S | C-A1 | C-A1 | C-A1 | C-A1 | B70 | B97 |
| H8-1486 | S | C-A1 | C-A1 | C-A1 | C-A1 | B70 | B106 |
| H8-1487 | S | C-A1 | C-A1 | C-A1 | C-A1 | B70 | B107 |
| H8-1488 | S | C-A1 | C-A1 | C-A1 | C-A1 | B70 | B114 |
| H8-1489 | S | C-A1 | C-A1 | C-A1 | C-A1 | B70 | B115 |
| H8-1490 | S | C-A1 | C-A1 | C-A1 | C-A1 | B70 | B116 |
| H8-1491 | S | C-A1 | C-A1 | C-A1 | C-A1 | B70 | B117 |
| H8-1492 | S | C-A1 | C-A1 | C-A1 | C-A1 | B70 | B118 |
| H8-1493 | S | C-A1 | C-A1 | C-A1 | C-A1 | B70 | B119 |
| H8-1494 | S | C-A1 | C-A1 | C-A1 | C-A1 | B70 | B120 |
| H8-1495 | S | C-A1 | C-A1 | C-A1 | C-A1 | B70 | B121 |
| H8-1496 | S | C-A1 | C-A1 | C-A1 | C-A1 | B70 | B122 |
| H8-1497 | S | C-A1 | C-A1 | C-A1 | C-A1 | B70 | B125 |
| H8-1498 | S | C-A1 | C-A1 | C-A1 | C-A1 | B70 | B126 |
| H8-1499 | S | C-A1 | C-A1 | C-A1 | C-A1 | B70 | B127 |
| H8-1500 | S | C-A1 | C-A1 | C-A1 | C-A1 | B70 | B128 |
| H8-1501 | S | C-A1 | C-A1 | C-A1 | C-A1 | B70 | B129 |
| H8-1502 | S | C-A1 | C-A1 | C-A1 | C-A1 | B70 | B130 |
| H8-1503 | S | C-A1 | C-A1 | C-A1 | C-A1 | B70 | B131 |
| H8-1504 | S | C-A1 | C-A1 | C-A1 | C-A1 | B70 | B132 |
| H8-1505 | S | C-A1 | C-A1 | C-A1 | C-A1 | B70 | B133 |
| H8-1506 | S | C-A1 | C-A1 | C-A1 | C-A1 | B70 | B134 |
| H8-1507 | S | C-A1 | C-A1 | C-A1 | C-A1 | B70 | B135 |
| H8-1508 | S | C-A1 | C-A1 | C-A1 | C-A1 | B70 | B136 |
| H8-1509 | S | C-A1 | C-A1 | C-A1 | C-A1 | B70 | B137 |
| H8-1510 | S | C-A1 | C-A1 | C-A1 | C-A1 | B70 | B138 |
| H8-1511 | S | C-A1 | C-A1 | C-A1 | C-A1 | B70 | B139 |
| H8-1512 | S | C-A1 | C-A1 | C-A1 | C-A1 | B70 | B 140 |

9. Dispositif électroluminescent, comprenant :
une anode,
une cathode et
une couche organique disposée entre l'anode et la cathode, dans lequel la couche organique comprend le composé selon l'une quelconque des revendications 1 à 8.

10. Dispositif électroluminescent selon la revendication 9, dans lequel la couche organique est une couche d'injection de trous ou une couche de transport de trous, et la couche d'injection de trous ou la couche de transport de trous est formée par le composé seul.

11. Dispositif électroluminescent selon la revendication 9, dans lequel la couche organique est une couche d'injection de trous ou une couche de transport de trous, et la couche d'injection de trous ou la couche de transport de trous comprend en outre au moins un matériau de transport de trous ; dans lequel un rapport molaire de dopage du composé par rapport à l'au moins un matériau de transport de trous est compris entre 10000 : 1 et 1 : 10000 ;
préférentiellement, le rapport molaire de dopage du composé par rapport à l'au moins un matériau de transport de trous est compris entre 10 : 1 et 1 : 100 ;

12. Dispositif électroluminescent selon la revendication 9, dans lequel le dispositif électroluminescent comprend au moins deux unités émettrices de lumière et la couche organique est une couche de génération de charge et est disposée entre les au moins deux unités émettrices de lumière, dans lequel la couche de génération de charge comprend une couche de génération de charge de type p et une couche de génération de charge de type n ;
préférentiellement, la couche de génération de charge de type p comprend le composé ;
plus préférentiellement, la couche de génération de charge de type p comprend en outre au moins un matériau de transport de trous, dans lequel un rapport molaire de dopage du composé par rapport à l'au moins un matériau de transport de trous est compris entre 10000 : 1 et 1 : 10000 ; encore plus préférentiellement, le rapport molaire de dopage du composé par rapport à l'au moins un matériau de transport de trous est compris entre 10 : 1 et 1 : 100.

13. Dispositif électroluminescent selon la revendication 11 ou 12, dans lequel le matériau de transport de trous est choisi parmi un composé ayant une unité triarylamine, un composé spirobifluorène, un composé pentacène, un composé oligothiophène, un composé oligophényle, un composé oligophénylènevinylène, un composé oligofluorène, un complexe porphyrine ou un complexe phtalocyanine métallique.

14. Dispositif électroluminescent selon la revendication 12, dans lequel la couche de génération de charge comprend en outre une couche tampon disposée entre la couche de génération de charge de type p et la couche de génération de charge de type n, et la couche tampon comprend le composé.

15. Composition de composé, comprenant le composé selon l'une quelconque des revendications 1 à 8.
